# DEMANDE DE BREVET EUROPEEN

(11) **EP 1 566 387 A2**
(43) Date de publication de la demande: **24.08.2005**
(21) Numéro de dépôt: 05290552.8
(22) Date de dépôt: 04.03.2003
(51) Int. Cl.: C07K 14/47, C12N 15/12, C12Q 1/68, A61K 31/713, A61K 35/12, A61K 39/395, A61K 38/17, C07K 16/18, G01N 33/68, C12N 5/10, A61P 9/00

(54) **Gènes impliqués dans la régulation de l'angiogenese, préparations pharmaceutiques les contenant et leurs applications**

(30) Priorité: 04.03.2002 FR 0202717; 11.04.2002 FR 0204546
(62) Demande divisionnaire de: 03727567.4
(71) Demandeur: Gene Signal, 91000 Evry (FR)
(72) Inventeur: Al-Mahmood, Salman, 75014 Paris (FR); Colin, Sylvie, 75014 Paris (FR); Schneider, Christophe, 51000 Reims (FR)
(74) Mandataire: Galup, Cédric Olivier Nicolas

(57) **Abrégé**

L'invention se rapporte à des compositions thérapeutiques dans le domaine de l'angiogenèse, comportant des séquences nucléotidiques de gènes dont on a mis en évidence leur implication dans le mécanisme de l'angiogénèse y compris leurs séquences complémentaires, les séquences antisens, des séquences polypeptidiques codées par les parties codantes desdits gènes ainsi que des anticorps dirigés contre lesdites séquences polypeptidiques.

L'invention concerne aussi des cellules génétiquement modifiées sous-exprimant ou surexprimant lesdits gènes, des compositions thérapeutiques les contenants, utiles pour le traitement de désordres angiogéniques.

L'invention concerne également des méthodes de diagnostic et/ou pronostique de désordres angiogéniques ainsi que des nouvelles méthodes de criblage des composés actifs dans le traitement desdits désordres.

LISTAGE DE SEQUENCES

<110>  GENE SIGNAL

<120>  Gènes impliqués dans la régulation de l'angiogénèse, préparations pharmaceutiques les
contenant et leurs applications.

<130>  25283/PCT/EP/02

<140>  05XXXXXXXX
<141>  2005-03-11

<150>  03727567.4
<151>  2003-03-04

<150>  PCT/FR03/00695
<151>  2003-03-04

<150>  FR02/02717
<151>  2002-03-04

<150>  FR02/04546
<151>  2002-04-11

<160>  301

<170>  PatentIn version 3.1


<210>  1
<211>  1683
<212>  DNA
<213>  Homo sapiens
<220>
<221>  misc_feature
<223>  séquence appelée GS-N1

<300>
<308>  GENBANK/BC008502
<309>  2001-07-12
<313>  (1)..(1683)
<400>  1
agaaacggca gtggcagcag cgtccggagc agccgcagcc ttctggaagc tccaggcggt     60
ctttctgccg agcctcggtc ccggccccca tcctccccgc cccatcggtt gttgtctggg    120
cggatttaaa cagtcaagta aaatcaagct gggtaatcat ggcagaaggt ggatttgatc    180
cctgtgaatg tgtttgctct catgaacatg caatgagaag actgatcaat ctgttacggc    240
agtcccagtc ctactgcaca gacacagagt gtcttcagga attaccggga ccctctggtg    300
ataatggcat cagtgttaca atgatcttgg tagcctggat ggttattgca ttgatcttgt    360
tcttactgag acctcctaat ctaagaggat ccagcctacc tggaaagcca accagtcctc    420
ataatggaca agatccacca gctcctcctg tggactaact ttgtgatatg ggaagtgaaa    480
atagttaaca ccttgcacga ccaaacgaac gaagatgacc agagtactct taaccccatt    540
agaactgttt ttccttttgt atctgcaata tgggatggta ttgttttcat gagcttctag    600
aaatttcact tgcaagttta tttttgcttc ctgtgttact gccattccta tttacagtat    660
atttgagtga atgattatat ttttaaaaag ttacatgggg cttttttggt tgtcctaaac    720
ttacaaacat tccactcatt ctgtttgtaa ctgtgattat aatttttgtg ataatttctg    780
gcctgattga aggaaatttg agaggtctgc atttatatat tttaaataga tttgataggt    840
ttttaaattg ctttttttca taaggtattt ataaagttat ttggggttgt ctgggattgt    900
gtgaaagaaa attagaacca cgctgtattt acatttacct tggtagttta tttgtggatg    960
gcagttttct gtagttttgg ggactgtggt agctcttgga ttgttttgca aattacagct   1020
gaaatctgtg tcatggatta aactggctta tgtggctaga ataggaagag agaaaaaatg   1080
aaatggttgt ttactaattt tatactccca ttaaaaatct ctaatgttaa gaaaacctta   1140
aataaacatg attgatcaat atggcagatg atttacagtc cattatatga catttgtaag   1200
ccactcaaca ctagccttac aaggttcatg aaaggaggaa aacattaatt ctttctgcct   1260
ttgctgatat aatccacata gtaataattt aagctataat ttatttttaa aatgagtgcc   1320
tcttgggaag cttcttttct gttctaacag ttccctgtta aaatgaaaat cttaggccag   1380
tgcagtggct cacacctgta atctcagcac tttgggaggc tgaggtgggt ggatcacttg   1440
aggtcaggag ttcgagacca gcctggccaa catggtgaaa ccctgtctct actaaaaata   1500
cagaaaaatt agccagggtt ggtgcatgtg cctgtaatcc cagctactca ggaggccaaa   1560
gcaggagaat tgcttgaacc cgggaggtgg aggttgcagt gaaccaagat cgcaccactg   1620
cattccagcc taagcaatag agcgagactc cctcccaaaa aaaaaaaaaa aaaaaaaaaa   1680
aaa                                                                 1683

<210>  2

<211>  1649
<212>  DNA
<213>  Homo sapiens
<220>
<221>  misc_feature
<223>  séquence appelée GS-N2

<300>
<308>  GENBANK/NM_022823
<309>  2001-12-10
<313>  (1)..(1649)
<400>  2
agcgagcggg gccagcgctg cagaaggcgg cggctggctc tccgggacgg tcacatcccg      60
ctgcaggggc gggcggaggc cgccgcactg cctcccgcac cggggaccca ggccagcgtc     120
cgggcaacgc cccctgctcc cggacagact ccgtggcccg ctcgagccct gggggctccg     180
cagacccgcg cccgctccgc ccgcagctcg gccccgcgct gcccgcgtcg ccgggcccgc     240
gccgggatgg ggtaggggca gcgccaccga gtcgggcgat gggccgccct ctgggcaccg     300
agcagccccc cgaggcctga ccaaccgcga ggaccggcgg aggagccccg cctggatgtc     360
aagcggatgc caagcggatg ccacagttcc ccccccagcg gactccgtgg ggacatggct     420
tcgctggtgc ccctttcccc atatctaagc cccacggtcc tcctgctggt cagctgtgac     480
ctgggcttcg tgcgagcaga ccggcctccc tctcctgtga atgtgacggt cactcacctc     540
agagccaact cggccactgt gtcctgggac gtcccagaag gcaacatcgt cattggctac     600
tccatttccc agcaacggca gaatggcccc gggcagcgtg tgattcggga ggtgaacacc     660
accacccggg cctgtgccct ctggggcctg gctgaagaca gtgactacac agtgcaggtc     720
aggagcatcg gccttcgggg agagagtccc ccagggcccc gggtgcactt ccgaactctc     780
aagggttctg accggctacc ttcaaacagt tcaagcccag gtgacatcac agtggaaggt     840
ctggatggag agcggccact gcagactggg gaagtggtca tcattgtggt ggtgttgctc     900
atgtgggctg ctgtaattgg gctgttctgc cgtcagtatg acatcatcaa ggacaatgac     960
tccaacaaca atcccaagga gaagggaaag gggccggaac agagtcctca gggaaggcca    1020
gtggggacaa gacagaaaaa gtcaccatct atcaacacca tcgacgtttg agtgaagaaa    1080
cacacccaga agagagatgc actaacaact ggggataggg atggggtcag gggagcccca    1140
agatggtgat ctgcccgaga ctcccagagg gtaatgccac tcccacaatc tcaggcctgg    1200
tacccatcct ctttccactg tgagcagagc cagaaggtag gtctgttcag agtctgtgcc    1260
cctggacctg gggagtggat atcagatggg atatctcctt ccattccccg gtccagggga    1320
gagtcactag ttgtacccta ctccattagg tcccaaatgg gggccccatt tcacctgtat    1380
caggactctg agcatcccca gctgccccac atcttgcctc tggccctcag agaggggtgt    1440
ttctgtgggt actcctctta ccccagcaaa taaaaggaat tgtctgaccc tagaggcaga    1500
tgctgcactg cactactcca atgtcttcca tggagcctca ggtgctcccc ctctcacctg    1560
gcagcccctt cagctgctag tgatatcact tgttggacat ttttccaata aaggttcttg    1620
gacaaactgg aaaaaaaaaa aaaaaaaaa                                      1649

<210>  3
<211>  5766
<212>  DNA
<213>  Homo sapiens
<220>
<221>  misc_feature
<223>  séquence appelée  GS-N3

<300>
<308>  GENBANK/AB007963
<309>  1998-08-13
<313>  (1)..(5766)
<400>  3
cgccgccgca gccgccgcca ccaacgccaa caacaacgtg tcgggagcag ccgcggccga      60
agccggagca ggggccggag tcgcggctgg agtggggcct gcgcgtgtga gtggccgaga     120
gttgcccgtg ccggagacct caaactccgg gcgggagcgc tttcggtggg ccgagccgcc     180
gtcgcccgcg gcaccgtgtc cccaaggccc agacagcggg gcgggggtct atcaggcgcg     240
ggactggctt ctctcaggcc gacccttcta gccgggctcc ggatcttcct cggcctcggg     300
cccccttcac gttcaggacg gaggccggag aacggtgatc atcacacacc ccagccggct     360
tcctggccag ggcattttct ctgcggccaa gcagtgttgg gattgtagct gctgggagcc     420
ccctcctgcc gccacctctc ctgcaaggaa gggactgacg cgttcccact ttcttactct     480
gcacctcccc actctcttcc cattcaagaa cgagtttctc ttccctctcc attggaaatc     540
ctgctccgga ggtccaaaac acgtatttta agcgtgccct tcctcacctc ctggagcacc     600
cttagaagta ttgcctactt atccgggctg agaatccttc atttttgacc tggctttttt     660
ttcgcccttt gggagataaa ggtccctctc caccctctac taacactctg cacccaaggc     720
cttatccttt ggggtcacca gctccttggc catttctatg tgatttcccc cacccatctg     780
agttccagtt tcctctgggc tccaatctcc agtccctggc ggatctggtc agtcccaccc     840
ctaggtggga gtgaccaggg ggctctggcc caggattccc aaccctggaa ggcagctcca     900
aggcaggaag agaattgggc atcgggtacg aacctggcag ctcaggagtc ggggctccac     960
tcaccccaca caaaaagatg aaaaagcgca aagagctcaa tgcattgatt ggtttggctg    1020
ggacagccgg gagaaagaag cccaagaaag gcccaagcag tcaccgcctg cttcgcactg    1080
agcctcccga ctcagactct gagtccagct ccgaagagga agaggaattc ggtgtggttg    1140

gaaatcgctc tcgctttgcc aagggagact atttacgatg ctgcaagatc tgttatccgc 1200
tctgtggttt tgtcatcctt gctgcctgtg ttgtggcctg tgttggcttg gtgtggatgc 1260
aggttgctct caaggaggat ctggatgccc tcaaggaaaa atttcgaaca atggaatcta 1320
atcagaaaag ctcattccaa gaaatcccca aacttaatga agaactactc agcaagcaaa 1380
aacaacttga gaagattgaa tctggagaga tgggtttgaa caaagtctgg ataaacatca 1440
cagaaatgaa taagcagatt tctctgttga cttctgcagt gaaccacctc aaagccaatg 1500
ttaagtcagc tgcagacttg attagcctgc ctaccactgt agagggactt cagaagagtg 1560
tagcttccat tggcaatact ttaaacagcg tccatcttgc tgtggaagca ctacagaaaa 1620
ctgtggatga acacaagaaa acgatggaat tactgcagag tgatatgaat cagcacttct 1680
tgaaggagac tcctggaagc aaccagatca ttccgtcacc ttcagccaca tcagaacttg 1740
acaataaaac ccacagtgag aatttgaaac aggatatcct gtaccttcac aactctttag 1800
aggaggtaaa cagtgcccta gtggggtacc agagacagaa tgatcttaaa ctcgagggaa 1860
tgaacgagac agtcagtaat cttacccaga gagtcaacct gatagaaagc gatgtggttg 1920
ctatgagcaa ggtagaaaag aaagcaaacc tgtccttcag catgatgggt gatagatctg 1980
ccactctgaa aagacagtct ttggatcaag tcaccaacag aacagataca gtaaaaatcc 2040
aaagcataaa gaaagaagat agttcaaatt ctcaggtatc caagctaaga gagaaactcc 2100
agctgatcag tgctcttaca aacaaacctg agagcaacag gcctccagag accgccgatg 2160
aagagcaagt agagagtttc acatcaaagc catcagcatt gccaaaattt tcacagtttc 2220
ttggagaccc agttgagaaa gctgcccaac taagacctat ctccctacca ggagtttcta 2280
gcactgaaga tcttcaggat ttattccgca agactggcca ggacgtggat gggaagctga 2340
cctaccagga aatctggacc tccctaggtt ctgctatgcc agaaccagag agcttgagag 2400
catttgattc cgatggagat ggaagatact cattcctgga gctaagggta gctttaggta 2460
tctagcttca tcaggcatat tttagaaatg gactgcctaa tatctattta cctaacaaca 2520
aaacaaccct tacttaccca tcagtcctct agtcctccaa actactgtag cagatacttt 2580
gccacctttt aacttgtttg aagaagctat ataaaagtta tttttttaaa gaagaagacc 2640
attttactta tgatgttcag aaatctatga tttcctacaa ccagtaagat cttacatttt 2700
aaaattgcca gaaaaaaaat taaagccctc tttttttctc tttccttttt ttgaggggag 2760
gagaccttat cttttaaagc tgggaaatgt atatagagag agaataagcc acttttatat 2820
ttcacttaaa tttgccttaa attagctgca ctttatagag actcagaaaa tgtcttttct 2880
ttaaaagata ggccttttct gtttgtaaat atttaaatga aagaaagcat tgtgcatatt 2940
gtgtggaaag taggaagaat ggttttgaac aggatatgaa caaatgactt attaaaaatt 3000
gctgatctgg tgtaggtggc agctgaaact acatccatgt ctccataagg catccctcaa 3060
aggcccaggc gctgccaggg ggtttgtcct ggtagctgga ggaaccgatt tcagggaata 3120
gacactggag acaatactga ctccaggcat ggctcatgga agtaggattc tggttctttg 3180
ttcctattcc ctcagctaat cccaacctgg gaatcagaga agtcttgggg atttttctca 3240
tttttagtac tatttcaggg tttatgagca taaaaagtta tccattgggg agctccattt 3300
tccctgctga gtgagctaga ttgccttccc cacccaccca cttaagtctg tcttaaagcc 3360
gtagctggct cccaccacca gtaccatctc catttgaatg gcagggctaa attcccccag 3420
ccattatctc acactgacca cccagagctt tagaagagag ctgtgcttct aattttgacc 3480
cagaaaacca taccccttga gattttacct agaggctaac caagagccta atatgtttct 3540
ctgggggatg actaaagcca aaaaggctgt gagatgaaac atgtgaaata atattcagtt 3600
tccttaccat taccagctca gaagtagcta gaggctttct acccaaagga tgccaaagta 3660
tagcagggca ggcctggagc tagggccttc acatggtggt agcaagtttt tcaaatctaa 3720
tacaatcaag tacaatactt cctttaaatg cttctgtgga cctggcatga aagatcccta 3780
gattgaaagg aataatacct ccatgtctcc tgtatgttga gtctagaatt gctgtgttgt 3840
tcttagaagc agtctttggg caacaacttg aaaggggaaa aaaaaactac aaaaacttaa 3900
ctttggtata ggccaagtca gggagaaagt agagaaagct gtcatgccac agacttcttt 3960
agtggagatc atttcctttt taactttgtt caggttgccc ttcaccatgg atacagtccg 4020
gtacccttaa acatttaagg gctgtttttt tttttcttta tatgatgttc agcttggtat 4080
taaccaaact taaatttttt ttccagaagt attaaaattt agttaaagca agatgaagtc 4140
ttttcccatg aatgtgtccc cttatctcat tatagcttat gctcagtttc actttcttgg 4200
aaaggttaaa acaaattagc ctggcacttt aggtaacttg aaaataactc tcacccttct 4260
gactgccttc catccactct caccccacac ctttttttaa tatatataca cccttacaga 4320
ttttctaaca accaaataaa attctagcaa taaattgaat tatactgcta tatttgtata 4380
tactgtacca taaatagtat gtctgtacct ggaaggtatt tttttgagaa ctgatttata 4440
tgaaatatac ttgagggtaa tgtagcttgt cctttttagt ttcaaattct tattcatagg 4500
cagatacttt gaagacacct taactctttg ttgtgtgtat tttcccttgc atcagataac 4560
tatacatgga gaaatccatt ttgttttttg gtttatcttc tgttcttcct accttgtgct 4620
tgtctgacta gtatcacccc tgagccagta tacagtacca tcctctcccc ctaccaagtt 4680
tggcagaggt gtttatactg cattctcaga tctatgaata tttttgtcat cctgcctgag 4740
aaagtacatc ttccaggcaa aagtagggta tcttgaactc ctttcttctg agttgagcag 4800
actcactggc ttaagcatcc cataagccta ttatttagtg tgaatttggg acttttattt 4860
gtatgttgtc ttaacttact gagcctgtga cttgtattta gctgtagtca agcctcacca 4920
gccatatctc atctctgaga ggaagactgg atagtgagtt tggttttggt tttggtttta 4980
aaccctttta ccttttcagt ctggcagctc ctactgctgc tttcctaagt tactcaaagc 5040
actgcttctt ggtccttgtt ttcttttgcc ttgtactatc ttgtataatg tttactaaaa 5100
gagcctcccc ataaatcaga gaggcaaaaa ggtagcatat aaaaagaaga accaaaagaa 5160
gagaaaacct gacttctaga tgactgtggt gtcatctaaa aggcagaggt gcctgaccct 5220
caggaatgtg tatgatgcaa cagagctgaa gaatgggttc ccttcaccta ctgctggcca 5280
ttgtcccttc tgttggagat tcctttcttt atggttgctg ttcataaccc cttcaacacc 5340
cccccttgct ggtcctgccg gtcacagttg aggattttgg ctgtgatggg ctcatactca 5400
taatggcctg ttgagttgtt tttagtgaca ggttttgttt gctgcacacc tcattggctt 5460
gcttaccaac accagctaga agtggtccct ctttcatatc caaaccagaa cacatttggg 5520

tattcctgag actttataga gcgtgtattg tttgttatac gaacctaacc taccttgttg 5580
ttttctcata ttaagaaatt taaatctact ttgttttagt gaagctattt tggtaatgta 5640
taagcaattt tggttctgtt tagggagaat tcagggtttc catgacttga tttgagcttt 5700
gcatatttca aataagtaac acagccattt taatttagct aaataaaaaa atttcttttt 5760
tctctc 5766

<210> 4
<211> 5242
<212> DNA
<213> Homo sapiens
<220>
<221> misc_feature
<223> séquence appelée GS-N4

<300>
<308> GENBANK/AB037835
<309> 2000-03-14
<313> (1)..(5242)
<400> 4
caggtgtgca gaacggctca acaacttgaa gacctcacca gaagctgtca gtggatatag 60
ttttgcaatg gctgctttgt taggtggagt acatcagtgt cctttgggca ttcctcatgc 120
caaaggaaag atggtagtta gtattgctga agatctttta cgaactgctg cccaaaatag 180
caggctatct ttacagcgca cccaagctgg ctggctttta cttggagcac ttatgacttt 240
aggaccatct gtcgttcgtt accatctgcc caagatgttg ttattgtggc gaaatgtttt 300
cccacgttcc ttaaaggaat tggaagctga gaaggcccga ggcgattctt ttacctggca 360
ggtaactttg gaaggtcgtg ctggagctct atgtgccatg aggagcttcg ttgcacattg 420
tcctgagcta ctaactgaag atgtgattcg aaaattgatg acccctattg aatgtgccat 480
gactatgatg tcacacattc catctgtaat gaaagcccat ggagctcatc tgaaagctag 540
tgctgcaatg gtccgtttaa gactttatga tatcttggct ttgttacctc caaaaactta 600
tgaaggatct tttaatgcac ttcttagaga actggtagcg gaattcactt tgactgacaa 660
ctcagccaac acaactactt ccctcctcag atccctctgc cattatgatg atagtgttct 720
tcttggttct tggcttcagg aaactgatca taaatcaatt gaagaccagc tccagccaaa 780
cagtgcctct ggaagtgggg ctctggagca tgatccttcc tctatttatt tacgcatacc 840
tgctggagaa gcagtacctg gtcccctccc tctcggagtc tcagtcattg atgcttctgt 900
ggcccttttt ggtgtagtgt ttcctcatgt ttcttataaa caccgattac aaatgttgga 960
tcactttgct gaatgtgtta aacaagctaa aggtgtccgc cagcaggctg tgcagcttaa 1020
catatttact gctgttctta gtgcactaaa gggcttagct gaaaacaaaa gtactttagg 1080
acctgaggaa gttcgtaaat ctgccctgac actggttatg ggtcctctgg acaacccaaa 1140
ccccatctta cgttgtgcag cgggggaagc tcttggaaga atggctcagg tggttggaga 1200
agcaactttt attgctagaa tggcccaata tagctttgac aagttgaaat cggctcgaga 1260
tgttgtatct aggactggtc attcattggc tcttggttgt ttgcatcgtt atgttggtgg 1320
aataggctca ggacaacatc tgaagaccag tgtcagcatt ttattggctc tagcacaaga 1380
tgggacatcc cctgaagtcc agacttggtc tcttcattca cttgctttga tagtggattc 1440
tagtggtccg atgtatcgtg gctatgtgga accaacatta tctctagttc ttaccttgct 1500
gttgacagtt ccgccttcac atacagaagt tcatcagtgt ttgggtcgat gcttgggtgc 1560
tataataact actgttggcc ctgaactaca agggaatgga gcaacaactt ctacaattcg 1620
ttcctcttgt ttggtgggtt gtgcaataac acaagaccat tcagattctc ttgttcaggc 1680
agctgccata tcttgccttc agcagcttca catgtttgca ccacgacatg tcaatctatc 1740
tagccttgtt cctagccttt gtgttcactt atgtagttcc catttgttac ttcgacgagc 1800
agctgtggca tgtcttcggc aacttgcaca aagagaagca gcggaagtat gtgaatatgc 1860
catgagcctg gcaaaaaata caggggacaa agagagcagt agtgccaatg tcagtccttt 1920
tgcccctggg gttagttctc gaactgatat ccattgccgg caccaaggtg ttaatataac 1980
agaaactggt cttgagggac ttctttttgg aatgctagac cgggagacag atcgaaaatt 2040
atgttctgat attcatgaca ctttgggaca tatgctttct tcgctggcag tagaaaaact 2100
ttctcattgg ctaatgcttt gtaaagatgt cctggcagct tctagtgata tgagtactgc 2160
aactctctta agtagtggaa aagatgaaga agctgaaaag aaagatgaga tggatgatga 2220
taccatgttt accacgttag gtgaagaaga taaatcaaag ccctttgtgg cccctcgctg 2280
ggccactcga gtatttgctg ccgattgcct gtgtcgaatc atcaatttgt gtgagaatgc 2340
agaccaggct cactttgatc ttgccttggc acgttctgct aaacttcgaa accctacaaa 2400
tgacctcttg gtacttcatc tctctgacct cattcgcatg gcattcatgg ctgcaactga 2460
tcatagcaac cagctgcgaa tggctgggct ccaggcgctt gaagacatta tcaagaagtt 2520
tgcgtctgtg cctgagccag aatttccagg tcatgtgata ctggagcagt atcaggctaa 2580
tgtgggagct gctctaagac cagccttttc acaagataca ccatcagata taatagcgaa 2640
agcttgccag gtatgtagta catggatagg aagtggagtt gtcagtgatc tcaatgatct 2700
ccgtcgagta cacaatcttc ttgtttcttc tctggacaaa gttcaggctg gaaaaggatc 2760
ttccagccag ctgtaccgag agagtgccac gaccatggaa aaactggctg ttctcaaagc 2820
ttgggcagag gtatatgtgg tcgctatgaa tattaaaaag gaagcagagt caaaaccaaa 2880
aagagcaatt aaaaatactg acgatgatga tgacgactgt ggtaccatcg atgaactgcc 2940
accagatagt ttaataacac tggtacaacc tgaactacca acactcagtc gcctgtggtt 3000
agcagcatta aaagattatg cactcttgac tttaccagcc gaattttcta gtcagcttcc 3060
tccagatggt ggagcatttt atacccctga aactattgat acagctagac ttcactatcg 3120
gaattcctgg gccccaattc tccatgcggt ggcactttgg ttaaatagca caggatttac 3180
gtgctcagag tctacagaag cagcagcaat atctggttta caaaaacgtt ctacatctgt 3240

caatttaaac caggcatcag gagcagtggg tagtgctaaa tctttgccag aaattaacaa 3300
agacagaatg catctgattt taggtgtgag tatacagttt ctttgttccc ctagacctga 3360
ggagcccatt gaacatgtta cagcatgcct gcaggcctta cataccttgc tagactcccc 3420
ttatgctcga gtccatattg cagaagatca gctgataggt gttgagttgc tgagtgtttt 3480
gcaccgcctt ctattgacct ggaatccatc atctgtccag ctgttggtta ctggagttgt 3540
acaacagata gtaagagctg ctcaggatta tttgcaagag aaaagaaaca ctctaaatga 3600
agatgatatg gaaaaagagg cctgtaccgt attgggagaa ggaggtgaca gcggtggtct 3660
cattcctgga aaatctcttg tgtttgcaac tatggagctg ctgatgttca ttttagtacg 3720
gcatatgcca catctcagta ccaaggtgtc agactctcca agtcacatag ccactaaaac 3780
tcgactatca gaagaaagtg ctcgtttggt ggcagccaca gttaccatac tctctgattt 3840
accatccctt tgttcacccg ctggatgtat gacaatcctg cccacaattc tgttcttaat 3900
tgcaagaata ttgaaagaca cagcaataaa gtctgcagat aatcaggttc ctccaccagt 3960
cagtgcagct cttcaaggga ttaaaagtat tgtgacactt tcaatggcca aaactgaggc 4020
tggcgttcaa aaacagtgga cagctctgat tcgtagcacg cttgcttgca tcctggaata 4080
ttctcaacca gaagactctg tacctacacc tgatgaagta agcatgctaa cagcaattgc 4140
actcttcctg tggtctgcta gtaatgaaat aataggagtc cagtcattac agaatggctg 4200
catgaacaga tttaaaaatg cattaaattc atgcgaccca tgggttcaag ccaaatgtta 4260
ccagcttctc ctctcagtct tccagcattc caatcgtgcc ctttcaactc cttatattca 4320
ttcattagct ccaatagtgg ttgaaaagct aaaagctgtt gaaagaaaca gaccagccag 4380
taacatagag cttttagcgg ttcaagaagg aataaaagtt cttgaaacac tggttgctct 4440
tggtgaagaa caaaacagag tccagctact tgctctttta gttcccactt tgatatctta 4500
cctgctggat gaaaattctt ttgcctcagc aagttcagct tccaaagatc ttcatgagtt 4560
tgcactccag aatttaatgc atattggacc tctgtatcca catgctttca agacagtaat 4620
gggggctgct cctgagttga aagtgcgtct agaaactgcc gttcgagcaa gccaagcgag 4680
caaagctaaa gcggctgcca gacaaccagc ccccgccata cattctgcac caacaattaa 4740
gctaaaaaca agtttttttt aatttgtaat ttcatttatt tgtatttatg ttgtcaacta 4800
gcccctaatt agtgatggcc actgtatcat tccaggctac ctatgggttc tgttttattt 4860
gtatattggt attatagtgc ttccaagtag gttaagtgaa ataatgctca caccatttat 4920
tgatttttaa ggagtgattt tttttatcat gtggtgaata tgttttcatt ttcctcctaa 4980
taatgtttct cttttgttaa cttatttatt tttcaaacac tgagccatag tcttaaattt 5040
aaatgctaaa aacaattgta tcatgtagtc gggcagccta ttgatactgt caggggaaaa 5100
aaatgggtca gaactcagca tctgtcatga aaaaaatcaa ttgaaaaaca agcagatttt 5160
aaattttgaa gtaatcaaaa taatggaaag tgtctgtttt gtggtgtata tactatgaat 5220
aaaatcacag cattttctaa gc 5242

<210> 5
<211> 2153
<212> DNA
<213> Homo sapiens
<220>
<221> misc_feature
<223> séquence appelée GS-N5

<300>
<308> GENBANK/AK025682
<309> 2000-09-29
<313> (1)..(2153)
<400> 5
caaagagaca aacactgatg aagccgctaa tgacaatatg gcagttcgcc ccaaagagcg 60
cagcagcctg agctctagac agcatccgtt tgtaaggagc agtgtgatag tgcgctcaca 120
gaccttttct ccaggagagc ggaaccagta catctgcagg ttaaatcgga gtgacagtga 180
cagttcaacc ctggctaaaa aatcactgtt tgtgagaaac tccaccgaac gccgcagttt 240
gagggtcaaa aggacggttt gccagtcagt ccttagaaga acaacacagg aatgcccagt 300
gcggacatct ctagacttag aactggacct tcaggcatct ctgacccggc agagccgcct 360
caatgatgag ctgcaggcgc tgagggactt gcggcagaag ctggaggaac tgaaagctca 420
gggagagact gaccttccac caggcgtgct ggaggatgag aggttccaga ggcttctgaa 480
gcaagctgag aagcaggctg aacagtccaa agaagagcag aagcaaggtc tgaatgcaga 540
gaagttgatg aggcaagtct ccaaggacgt gtgtcggctc cgggagcaga gccagaaggt 600
gcctcggcag gtgcagtcct tcagggagaa gattgcctac ttcaccagag caaagataag 660
catcccatcc ctgccagctg atgatgtgtg attacatgac ttaagaaatt attttttcat 720
ctgttcactt tcttagggag ggtaaaagac tgaagatttg tgtttttgtt ttggtgtttg 780
gttttttttg gtaacgtaac tgtcaactct tgaagaactt ttatttcaca tcagattttc 840
aacacattaa tttgtaaagt accttgagtg taatttttat atgcttaaaa aagaaaaaat 900
catataagaa agatgggata acctggtaca caaatgttgc ccagtatgtg cgcattgcca 960
gtggacttca ttttgtaaaa atggaataga ggcagtaccc caccatgtgt actgttgagc 1020
cggctgttga gaaacaactt ggttcagccg gtggttttgc ttctcctttg tgttcctgtg 1080
agtaggctgt gccgatggca gcagcgccac gtggtatctg tgcgctggcg aggagaggag 1140
cgctttccat ttggaggcag tgtgagcaca cgctggagtg cagcgttcca aagtccgccc 1200
gaaatggatg ctgcgtccac ggccatcacc tgtgcgggaa cacctggttt ccttaagaca 1260
atttcctttt tgtcctttta tatttttcta aagaaaacta agatataaac taccaagtgc 1320
tcttaagaat aaaaataaga ataagaatac aaaggagcac tactcttggc tacacgaaag 1380
atcttgggat tcatgacact gaggcagggg agaagaaaga acaccagcca cgcagagata 1440
caaataagct catcaaatga gcagctggtt tgtctcactc agcctgaatt tgcattaagt 1500

gttcaaaagt gagtttctgg cttgcactag tgttttgccc taaatctaag gatggtgcct 1560
agtggtggcc ctgtgttctg aggacagaga gagctggagg ggtggaggga aatgcatcgg 1620
cactcatttg gtgtaaccaa gaagaggggg aaagatgtct tgatagaatc aaagttgcca 1680
aaaacaaaga agagtttatc aagcagattt ctaacattta gtagaaacta cttcatagct 1740
ctcattttta gggggttcta tctataattt atggtaaata gttgggaggc aaagggaaga 1800
atgtgtgtgg gggtattatt aatttgaagt ataagctaaa ttgtttatta aaaagcctat 1860
tttggatgaa caagtttaga gagataaact gactaatgtg tcctccagtt tagaatttca 1920
ccttacccga cccaggcact tcttaaaaac acagatcctc agtgcagctc tgccagcatc 1980
aaggctttta aaatatgtag tatttaggcc atttaaacca tacttattac tcaataaccc 2040
aagaaacaat tttgggttag ccatctaata tagcaaataa aatagatttt ttaaagaaat 2100
ccaaaattgt cttaaaggaa tggacccctt gaagaaaaaa aaaaaaaaaa aaa 2153

<210> 6
<211> 3005
<212> DNA
<213> Homo sapiens
<220>
<221> misc_feature
<223> séquence appelée GS-N6

<300>
<308> GENEBANK/AK023284
<309> 2000-09-29
<313> (1)..(3005)
<400> 6
gacgccgccg ccaccgcctc ctcagagcgg ggcccgggcc cagccgccgc caccgctgcc 60
gccgccgagc tccgccgccg ccgagcacca tgggagacgc tgggagcgag cgcagcaaag 120
cgcccagcct gccgcctcgc tgtccctgcg gcttctgggg gtccagcaag actatgaatc 180
tctgttccaa atgctttgct gattttcaaa agaaacagcc agacgatgat tccgctccaa 240
gtacaagtaa cagccaatca gatttgtttt ccgaagagac caccagtgac aacaacaata 300
cctcgataac cacgccaact cttagtccca gccagcagcc gcttccgaca gaactgaatg 360
taacttcacc gagtaaagag gagtgtgggc catgcacaga cacagctcat gtctcattaa 420
tcacaccaac aaaaagatcc tgtggtacag attcacagtc tgagaatgag gcttcaccag 480
taaaacggcc acgactactt gagaatacgg aacggtccga ggaaaccagt cgatctaaac 540
agaagagtcg acgtcggtgc ttccagtgcc aaaccaaact ggagctggtg cagcaggaat 600
tgggatcgtg tcgctgcggt tatgtgttct gtatgttaca tcgcctcccc gagcagcacg 660
actgcacatt cgaccacatg ggccgtggcc gggaggaagc catcatgaaa atggtgaagc 720
tggaccggaa agtggggcgc tcctgccagc gcatcgggga ggggtgctcc tgaaggccag 780
gcatggccac cacgtgacgc tgttcttagt tcactaatgt tagccttatt taggacaaag 840
tcagccagac accttgtact gggcacgcgt cagactgcag ccagtccgtt tcctttcttt 900
agccagccat cctggtactg tagtttaggg gttgatggtg gttgaaattg atttctggct 960
ggttactaag gtgcctgcta gccattgtat aaaattaaaa catgaagaat attttttttt 1020
ttgagcatgg ctagtggatt taaaacaaca catacctgtc actgctggag tcaaacttat 1080
aaaaagcctt aagtggaaag tgttccagac ggagactctg agttaataga ggagtagaag 1140
ctggtgttaa agttcccacg acgcacatgg ctttgccaga aactctgttt aatgatcggc 1200
ctttcacctc ttcacttatc cttagtccca gtagccagga tacctgatgg ccacgtgtgc 1260
cttggccacg ggaggctgct gagattggcc acgtggctgg actgggtggt ggcctcactc 1320
tcccacagag ctggaaatgg ggggtggggg acagattctt acggaaattt ttttacctga 1380
cttgctatga aaaaactcat cacacaagaa gagaaacagt aacctcactt tgaaaattag 1440
ctccactcaa gactagtcca cgaacgagac ccgccttttc tacacaggat ccaaggtcac 1500
gagaagcagc cagagtgccc cgcctccgcc ggctctggtc tgccattcgc cagtgcaggg 1560
atctggcacg gaccagatgt ggcgaatggc agcacagcgc ggtggctggg tctgcacact 1620
ggcctctgca gccagatttc tatattggga gttttttaaa aagacatttc atagccaaca 1680
agaatcagta gaagtgctgg gagcagcagc tggggaagct gccgcccacg ggctctgccc 1740
cttccagctg gagccgcccg tgcctccagg ggccaagagg atgatgtcgt ggcctccatt 1800
ctcgtttcta tgcagcccca tagtccaagg acacccagtc cacatctacc atatagcaag 1860
tttagtaagg gaaggcagca tacgtcccag ggacagtggg tttggatctg tctagaacag 1920
cggtttgtgg ctgtggccca gctccgagag tgatatttgc tctggtaggt gagggcctga 1980
gggtacattt ctccacctgt gccccctcat gttcacagag gatttcagca gctgcaactg 2040
cgcacgccag gtggggaagg gtgggggtgg gcctggttgc cccatgttag gaaatcacta 2100
ccagtcaggt ggggctgggg ctgggtggac aggatcagga ttcccttgaa agcccaggca 2160
gggtgagcag tcccagtggt cctagtgccg catcagatcc aggtgggtga gggcaggagg 2220
cccctgcgaa ggcagcgtgg atctgcccac acataggcta ctggaatagt ttaacccagc 2280
aactttcctt tttataaaac aacaaatggt tcaactctgt ctgcaaatta acagctgaac 2340
acctgcaact gcaaatgttt tttgatccga cgtactgaaa taggaagtca tgctcttccc 2400
accctccacc caccagagtg gaacccgctg caaaatcccc agccttaatt cttgcttcag 2460
gacccagacc ggtgtcttgc tctagggcaa cccagggcag aggggccagg tctgcccagc 2520
gtttaccact gctgtcaagc cacagccctt ggccaccata cgggccatcc tcagtgaggc 2580
agccccccat aggcttccgc caagctctgg tcccgaagag gctgtgcgag cccttcccgg 2640
ccctccccag ggccccccgc cccctcctct gcctgctgcg tggaggcagc catgggaagg 2700
agcccagggg agctggcctg ggggagcgaa gcccatgttc gcttcctgac ttagagctgg 2760
ggggggtggg gggtggggct tgttcccctg cagtatctgt tctgtgaagt ttgttaaatg 2820
taaggaaagc ttaaattctt gtatctttaa aagagaaaat cttatttaac ccttttgtgt 2880

tctagattta cttacacaca tagcctagag ctcagtttta gttttaacat tgtgaaaata 2940
ttaaaagaat cttgtaactt tattcttttt tctcctgctg aaaaaaaaat taaaccaatc 3000
gtatg 3005

<210> 7
<211> 4397
<212> DNA
<213> Homo sapiens
<220>
<221> misc_feature
<223> séquence GS-N7

<300>
<308> GENEBANK/AB033073
<309> 2001-05-17
<313> (1)..(4397)
<400> 7
gagcgagagt gtgtcgagtg agtgtgcgtc tgtgtgtccc ggcgagggtg cgcgctcggc 60
gccgggagcg cggccagccg agtccggagg catcgggagg tcgagagccg ccgggacccc 120
agctctgcgt tcactgcccc gtccggagct ggacttcggg gccggggccg gggccgtgcg 180
ccggggacag gcagggccgg gtcgcgggcc gcgcgtcccc caggccggag atctgcgagt 240
gaagagggac aagggaaaag aaacaaagcc acagacgcaa cttgagactc ccgcatccca 300
aaagaagcac cagatcagca aaaaaagaag atgggccccc cgagcctcgt gctgtgcttg 360
ctgtccgcaa ctgtgttctc cctgctgggt ggaagctcgg ccttcctgtc gcaccaccgc 420
ctgaaaggca ggtttcagag ggaccgcagg aacatccgcc ccaacatcat cctggtgctg 480
acggacgacc aggatgtgga gctgggttcc atgcaggtga tgaacaagac ccggcgcatc 540
atggagcagg gcgggacgca cttcatcaac gccttcgtga ccacacccat gtgctgcccc 600
tcacgctcct ccatcctcac cggcaagtac gtccacaacc acaacaccta caccaacaat 660
gagaactgct cctcgccctc ctggcaggca cagcacgaga gccgcacctt tgccgtgtac 720
ctcaatagca ctggctaccg gacagctttc ttcgggaagt atcttaatga atacaacggc 780
tcctacgtgc cacccggctg gaaggagtgg gtcggactcc ttaaaaactc ccgcttttat 840
aactacacgc tgtgtcggaa cggggtgaaa gagaagcacg gctccgacta ctccaaggat 900
tacctcacag acctcatcac caatgacagc gtgagcttct tccgcacgtc caagaagatg 960
tacccgcaca ggccagtcct catggtcatc agccatgcag ccccccacgg ccctgaggat 1020
tcagccccac aatattcacg cctcttccca aacgcatctc agcacatcac gccgagctac 1080
aactacgcgc ccaacccgga caaacactgg atcatgcgct acacggggcc catgaagccc 1140
atccacatgg aattcaccaa catgctccag cggaagcgct tgcagaccct catgtcggtg 1200
gacgactcca tggagacgat ttacaacatg ctggttgaga cgggcgagct ggacaacacg 1260
tacatcgtat acaccgccga ccacggttac cacatcggcc agtttggcct ggtgaaaggg 1320
aaatccatgc catatgagtt tgacatcagg gtcccgttct acgtgagggg ccccaacgtg 1380
gaagccggct gtctgaatcc ccacatcgtc ctcaacattg acctggcccc caccatcctg 1440
gacattgcag gcctggacat acctgcggat atggacggga aatccatcct caagctgctg 1500
gacacggagc ggccggtgaa tcggtttcac ttgaaaaaga agatgagggt ctggcgggac 1560
tccttcttgg tggagagagg caagctgcta cacaagagag acaatgacaa ggtggacgcc 1620
caggaggaga actttctgcc caagtaccag cgtgtgaagg acctgtgtca gcgtgctgag 1680
taccagacgg cgtgtgagca gctgggacag aagtggcagt gtgtggagga cgccacgggg 1740
aagctgaagc tgcataagtg caagggcccc atgcggctgg gcggcagcag agccctctcc 1800
aacctcgtgc ccaagtacta cgggcagggc agcgaggcct gcacctgtga cagcggggac 1860
tacaagctca gcctggccgg acgccggaaa aaactcttca agaagaagta caaggccagc 1920
tatgtccgca gtcgctccat ccgctcagtg gccatcgagg tggacggcag ggtgtaccac 1980
gtaggcctgg gtgatgccgc ccagccccga aacctcacca agcggcactg gccaggggcc 2040
cctgaggacc aagatgacaa ggatggtggg gacttcagtg gcactggagg ccttcccgac 2100
tactcagccg ccaaccccat taaagtgaca catcggtgct acatcctaga gaacgacaca 2160
gtccagtgtg acctggacct gtacaagtcc ctgcaggcct ggaaagacca caagctgcac 2220
atcgaccacg agattgaaac cctgcagaac aaaattaaga acctgaggga agtccgaggt 2280
cacctgaaga aaaagcggcc agaagaatgt gactgtcaca aaatcagcta ccacacccag 2340
cacaaaggcc gcctcaagca cagaggctcc agtctgcatc ctttcaggaa gggcctgcaa 2400
gagaaggaca aggtgtggct gttgcgggag cagaagcgca agaagaaact ccgcaagctg 2460
ctcaagcgcc tgcagaacaa cgacacgtgc agcatgccag gcctcacgtg cttcacccac 2520
gacaaccagc actggcagac ggcgcctttc tggacactgg ggcctttctg tgcctgcacc 2580
agcgccaaca ataacacgta ctggtgcatg aggaccatca atgagactca caatttcctc 2640
ttctgtgaat ttgcaactgg cttcctagag tactttgatc tcaacacaga cccctaccag 2700
ctgatgaatg cagtgaacac actggacagg gatgtcctca accagctaca cgtacagctc 2760
atggagctga ggagctgcaa gggttacaag cagtgtaacc cccggactcg aaacatggac 2820
ctgggactta aagatggagg aagctatgag caatacaggc agtttcagcg tcgaaagtgg 2880
ccagaaatga agagaccttc ttccaaatca ctgggacaac tgtgggaagg ctgggaaggt 2940
taagaaacaa cagaggtgga cctccaaaaa catagaggca tcacctgact gcacaggcaa 3000
tgaaaaacca tgtgggtgat ttccagcaga cctgtgctat tggccaggag gcctgagaaa 3060
gcaagcacgc actctcagtc aacatgacag attctggagg ataaccagca ggagcagaga 3120
taacttcagg aagtccattt ttgcccctgc ttttgctttg gattatacct caccagctgc 3180
acaaaatgca ttttttcgta tcaaaaagtc accactaacc ctcccccaga agctcacaaa 3240
ggaaaacgga gagagcgagc gagagagatt tccttggaaa tttctcccaa gggcgaaagt 3300
cattggaatt tttaaatcat aggggaaaag cagtcctgtt ctaaatcctc ttattctttt 3360

ggtttgtcac aaagaaggaa ctaagaagca ggacagaggc aacgtggaga ggctgaaaac 3420
agtgcagaga cgtttgacaa tgagtcagta gcacaaaaga gatgacattt acctagcact 3480
ataaaccctg gttgcctctg aagaaactgc cttcattgta tatatgtgac tatttacatg 3540
taatcaacat gggaactttt aggggaacct aataagaaat cccaattttc aggagtggtg 3600
gtgtcaataa acgctctgtg gccagtgtaa aagaaaatcc ctcgcagttg tggacatttc 3660
tgttcctgtc cagataccat ttctcctagt atttctttgt tatgtcccag aactgatgtt 3720
ttttttttaa ggtactgaaa agaaatgaag ttgatgtatg tcccaagttt tgatgaaact 3780
gtatttgtaa aaaaaatttt gtagtttaag tattgtcata cagtgttcaa aaccccagcc 3840
aatgaccagc agttggtatg aagaaccttt gacattttgt aaaaggccat ttctttcttg 3900
ggagtttttt ggtgtgtctg tttttttaaa gtattcaaga tactaccagt caacatcttt 3960
ttggaagaaa atgccttggg tttagaagat tttcttaaaa ggggagtaga tggttgtaga 4020
ttgactaaaa agtctaccat acttcaaggg actacaggta agtctcatag tataccagct 4080
ttggtacttc attttttaaa aaagtattaa tcaattgcaa agaaattcgc cttggccaac 4140
ccttctttgt gtatcaggta gtctaacctg atacaagtag ttgacagatt tcaactatca 4200
atcaccagtc caacccattt ctcatttaac agatgacgga gataatccct aaaagcaccc 4260
acatttgttt caatgcccca aacaggccaa ggctccctag caactcccta gtggcgtttt 4320
ttaacttctc agaaactgtt accattattt gaaataggct tccttaacct cctttaccct 4380
taacccaaca gggattt 4397

<210> 8
<211> 5844
<212> DNA
<213> Homo sapiens
<220>
<221> misc_feature
<223> séquence appelée GS-N8

<300>
<308> GENEBANK/AB023187
<309> 2001-05-17
<313> (1)..(5844)
<400> 8
gttctctgct gctactgtaa aaacgaaatg agtggtcctg ctcaggttcc aatgatgtcc 60
ccaaatggtt ctgtgcctcc tatctatgtg cctcctggat atgccccaca ggttattgaa 120
gacaatggtg ttcgaagagt tgtcgtggtc cctcaggcac cagagtttca ccctggtagt 180
cacacagttc tccaccgttc tccacatcct cctctacctg gtttcattcc tgtcccaact 240
atgatgccgc ctccaccacg tcatatgtac tcacccgtga ctggagctgg agacatgaca 300
acacagtata tgccacagta tcagtcttca caagtctatg gagatgtaga tgctcactct 360
acacatggaa ggtccaactt tagagatgaa cgatctagta aaacatatga acgtttgcag 420
aaaaaattga aggatcgcca aggaacacag aaagataaaa tgagcagtcc accatcatca 480
ccccagaaat gcccttctcc cattaatgaa cataatggac ttataaaagg acaaattgct 540
ggtggtataa acacaggatc agcaaaaatc aagtctggga aggggaaagg tggtacacaa 600
gttgatacag aaattgaaga aaaagatgaa gaaactaaag catttgaagc acttctttcc 660
aacattgtca aaccagtggc ctccgacatc caggcaagga cagtagtact tacctggtca 720
ccaccttcca gcctcattaa tggtgaaaca gatgaaagta gtgtaccaga gctctatggt 780
tatgaagttc tgatctcaag tactggaaaa gatgggaaat acaaaagtgt atatgtagga 840
gaagaaacaa atatcacttt aaatgatctc aagccagcca tggattacca tgcaaaagtc 900
caggcagaat ataattctat aaagggaact ccttcagagg ctgaaatctt taccaccttg 960
agctgtgaac ctgatatacc taatccacca aggatagcca atcggaccaa aaattcactc 1020
actttgcaat ggaaggcacc tagtgacaat ggttctaaaa tccaaaactt tgtattagaa 1080
tgggatgaag gaaaaggaaa tggagaattt tgtcagtgtt acatgggctc acagaaacaa 1140
tttaaaatta ctaaactttc accagcaatg ggctgtaaat tcagactatc ggccagaaat 1200
gactatggta caagtggttt tagtgaagaa gtcttatatt acacctcagg ctgtgctcct 1260
tctatgccag caagtcctgt attaaccaag gctggaatta cttggttatc cttacaatgg 1320
agtaagccct caggaacacc atcagatgaa ggaatttctt acattttaga gatggaggaa 1380
gaaacttcag gatatggttt taagcctaaa tatgatggag aagatcttgc ttacacagtg 1440
aaaaatctca gacgtagtac taagtataaa tttaaggtta ttgcttacaa ctcagaaggt 1500
aaaagtaatc caagtgaagt agtagaattt actacttgcc ctgataaacc aggcatacct 1560
gtaaagcctt cagtgaaagg aaagatacat tcacacagtt ttaaaataac ctgggatcca 1620
ccaaaagaca atggcggagc aaccatcaat aaatatgtag tggagatggc agaaggttct 1680
aacggaaaca aatgggaaat gatatacagt ggtgctacca gggaacatct ttgtgatcga 1740
ctgaatccag gctgtttcta tcgtttacga gtttactgca tcagtgatgg aggacagagt 1800
gcggtctctg aatctttact tgtgcagact ccagctgtgc ctcctggccc atgcctccct 1860
cccagattac agggtagacc caaagcaaaa gaaatacagt tacgatgggg accccctctg 1920
gttgatggtg gatcacccat ttcctgttac agtgtggaaa tgtctcctat agaaaaagat 1980
gaacctagag aagtttacca aggttctgaa gtagaatgta cagtgagcag ccttcttcct 2040
ggaaagacat acagcttcag actacgtgca gctaacaaaa tggggtttgg accattttca 2100
gaaaaatgtg atattactac agcccctggg ccaccagatc agtgcaagcc ccctcaagtg 2160
acatgtagat ctgcaacttg tgcacaagtg aattgggagg ttcctttgag taatggaaca 2220
gatgtcactg aatatcgact ggagtgggga ggagttgaag gaagtatgca gatatgttac 2280
tgtgggcctg gtctcagtta tgaaataaaa ggactttcac cagcaactac ctattattgc 2340
agggtccagg ctctgagtgt tgtgggtgca ggccctttca gtgaagtagt agcctgtgtg 2400
actccaccat cagttcctgg cattgtgacc tgtcttcaag aaataagcga tgatgagata 2460

gaaaatcccc attattcacc ttctacatgc cttgcaataa gctgggaaaa gccttgtgat 2520
catggttcgg aaatccttgc ctacagcata gactttggag ataaacaatc cctaacagtg 2580
ggaaaggtta caagctatat tatcaacaat ttgcaaccag atacaacata cagaatacga 2640
attcaagcct tgaatagcct tggagctggt cctttcagcc atatgataaa attaaaaact 2700
aagcctctcc ctcctgatcc acctcgtctg gaatgtgttg cctttagcca ccagaacctt 2760
aagctgaaat ggggagaagg aactccaaag acattgtcaa ccgattctat tcagtaccac 2820
cttcagatgg aggataagaa tggacggttt gtatccctat acagaggacc atgtcataca 2880
tacaaagtac aaagacttaa tgagtcaaca tcctataaat tctgtattca agcttgtaat 2940
gaagctgggg aaggtcccct ctcccaagaa tatattttca ctactccaaa atctgtccca 3000
gctgccttga aagcccccaa aatagagaaa gtaaatgatc acatttgtga aattacatgg 3060
gagtgtttac agccaatgaa aggtgatcca gttatttaca gtcttcaagt tatgttggga 3120
aaagattcag aattcaaaca gatttacaag ggtcccgact cttccttccg gtattccagc 3180
cttcagctga actgtgaata tcgcttccgt gtatgtgcca ttcgccagtg ccaagactct 3240
ctgggacacc aggacctcgt aggtccctac agcaccacag tgctcttcat ctctcagagg 3300
actgaaccac cagccagcac caacagagac actgtggaaa gcacaaggac ccgacgggca 3360
ctgagtgacg agcagtgtgc tgccgtcatc cttgtgctgt ttgctttctt ttccattttg 3420
attgccttta tcattcagta ctttgtaatc aagtgaaaat ataactttat tttttaactc 3480
tattacattt tattttgtca tgtactaaaa ttatttctgt attgctttta taaaaaacag 3540
tggcatttag cactggcatt gagactatag cacatcattt ttgccatttt cagtgcttat 3600
attgttaggt agaggctggc actttattag aatgcaagcc acaaaaatat caattttgtt 3660
tttttttgtt agggtgggtc ttcttttttt ctttccctct ctcttttttt aacaaatgcc 3720
ttcttataga aaaactttct aagaggcaac aatttagaat ggatattttg acgaatcggc 3780
atgagtgtaa cagtgataac ctgatctgtt tgttttaaag attattacca agtgaaaaat 3840
tcagaatgaa tagaatttac actaacatgc tatataaaat gttaaagtct gatgctgtga 3900
aagcaatcta gtgctatatt tctacctcct catttgtctt aattatttgg taagtgggat 3960
tatgatgagt aactggaggg gcttagaaac aaaaactgga tgaaagagta tgcatgaaga 4020
aaagcttctt tgataaatgt ggagttcttc attataaata tatattcatg aattcacaga 4080
taagtactta aagaacagac agtttacttg gcctaaaaat attttgatgt ttactcaaaa 4140
agtacctctt caggtcttga gaacatggaa aagaattgag tgcttttaaa tactttttag 4200
aaagtaatca taaaagtaaa ttgaatttca aacctatttg gcttctgttt tgtgaacctt 4260
tgaactatat gtatgtgtat aagggtatac acatacatat atggcatata acaagtgtac 4320
acatatacac ataacaagtg tagaagtata tattacatac atacactcac tctgtctggt 4380
ataggctaat tttgaagaac tcccataagt ttctgctgct tctcccataa ctgctgccac 4440
caccatcaga attcataatc aaacctaacc tttttgtttg gggcaccaaa tctgaagaca 4500
aaattaattt gcaccagtaa acttcaagct gctttctttc ttgaaaacta aacgtttaac 4560
gtataatgtc tgtttggata ctgttccaaa ttgttgattg catgtggtta atgttgcatt 4620
agagcacttt gcaattgcat aattcattaa tgttttgtga gcttgcattt gtgagttatt 4680
ggatgatcag actgaatttt gtcaagtatc acattgtaca tcttgcctag atgtcgatga 4740
ctgcaagtaa taatacagtt tataatgaaa ctatctacaa ttcttgtttt agcacatctg 4800
ttatccgtaa aacacctgta actagctttt ttaatttatt atttgaattt taggatagcg 4860
aatcactaat ttttagttgc tgaggttggc attttagtga ttattaagca cttctgtcag 4920
tctttgaaaa aaagaacgta ttttttgtgc tttgaagatc tctgaagaat ttcttttata 4980
atagaatggg catgtattgt aacagtttta tgtcaaatga tctgtgctgt agaaaaacat 5040
taacccttgt tcaaaaaaga aatggataaa cttggccttt ctaagtggta agaatgacct 5100
gtcactataa tatactgtat gtttacattt tatttaaatt taatctctta tgtatagggt 5160
gataaccttc cccagaaaca acagtgattg cgattgtttt ctagaaactt ctttaaagtg 5220
ccacatttgg cagtacaaat gagtctgagt gtaatagccc agagatttat atatagttga 5280
atgtctaaaa tggtaaaatg tgccactgtg tcaagttaca gtggcttatg tttttcatag 5340
taattcaaat gaacttccta tttttgatag taaatgtcat ttaatagtat acttgccatt 5400
tgagcctcac tgcaaaatta gtgcagagga gaaaacaatt tttaatgtaa tcttgatttt 5460
acctcatata ctgtacattc caaaaactct aaacttttta aagattatag atacactacc 5520
aaacatatca ccttaaaatt gtataaggct gaatgaactt catacaaatg aaaaaaatct 5580
cataaaaata cataaactat gtagcaaaag tatctgtaaa atccatggaa aataaaagtt 5640
gtatcattct ttttgagata cgtttattgt attcatatat attcattatt tgctacctgt 5700
ttaagaaagt gaaatgttat ggtctcccct cttccaatga gcttaaaaca tttttcccaa 5760
cagtatataa atcttcaaca tgagaggatg tatatttatt atataaagcc cagtaaagaa 5820
taaaattaga agttttatcc tagg 5844

<210> 9
<211> 4266
<212> DNA
<213> Homo sapiens
<220>
<221> misc_feature
<223> séquence appelée GS-N9

<300>
<308> GENEBANK/AB014587
<309> 1999-02-06
<313> (1)..(4266)
<400> 9
caagtctata agggtcgaca tgttaaaacg ggtcagttgg cagccatcaa agttatggat 60
gtcactgagg atgaagagga agaaatcaaa ctggagataa atatgctaaa gaaatactct 120

catcacagaa acattgcaac atattatggt gctttcatca aaaagagccc tccaggacat 180
gatgaccaac tctggcttgt tatggagttc tgtggggctg ggtccattac agaccttgtg 240
aagaacacca aagggaacac actcaaagaa gactggatcg cttacatctc cagagaaatc 300
ctgaggggac tggcacatct tcacattcat catgtgattc accgggatat caagggccag 360
aatgtgttgc tgactgagaa tgcagaggtg aaacttgttg actttggtgt gagtgctcag 420
ctggacagga ctgtggggcg gagaaatacg ttcataggca ctccctactg gatggctcct 480
gaggtcatcg cctgtgatga gaacccagat gccacctatg attacagaag tgatctttgg 540
tcttgtggca ttacagccat tgagatggca gaaggtgctc cccctctctg tgacatgcat 600
ccaatgagag cactgtttct cattcccaga aaccctcctc cccggctgaa gtcaaaaaaa 660
tggtcgaaga agttttttag ttttatagaa gggtgcctgg tgaagaatta catgcagcgg 720
ccctctacag agcagctttt gaaacatcct tttataaggg atcagccaaa tgaaaggcaa 780
gttagaatcc agcttaagga tcatatagat cgtaccagga agaagagagg cgagaaagat 840
gaaactgagt atgagtacag tgggagtgag gaagaagagg aggaagtgcc tgaacaggaa 900
ggagagccaa gttccattgt gaacgtgcct ggtgagtcta ctcttcgccg agatttcctg 960
agactgcagc aggagaacaa ggaacgttcc gaggctcttc ggagacaaca gttactacag 1020
gagcaacagc tccgggagca ggaagaatat aaaaggcaac tgctggcaga gagacagaag 1080
cggattgagc agcagaaaga acagaggcga cggctagaag agcaacaaag gagagagcgg 1140
gaagctagaa ggcagcagga acgtgaacag cgaaggagag aacaagaaga aaagaggcgt 1200
ctagaggagt tggagagaag gcgcaaagaa gaagaggaga ggagacgggc agaagaagaa 1260
aagaggagag ttgaaagaga acaggagtat atcaggcgac agctagaaga ggagcagcgg 1320
cacttggaag tccttcagca gcagctgctc caggagcagg ccatgttact gcatgaccat 1380
aggaggccgc acccgcagca ctcgcagcag ccgccaccac cgcagcagga aaggagcaag 1440
ccaagcttcc atgctcccga gcccaaagcc cactacgagc ctgctgaccg agcgcgagag 1500
gtggaagata gatttaggaa aactaaccac agctcccctg aagcccagtc taagcagaca 1560
ggcagagtat tggagccacc agtgccttcc cgatcagagt ctttttccaa tggcaactcc 1620
gagtctgtgc atcccgccct gcagagacca gcggagccac aggttcctgt gagaacaaca 1680
tctcgctccc ctgttctgtc ccgtcgagat tccccactgc agggcagtgg gcagcagaat 1740
agccaggcag gacagagaaa ctccaccagc agtattgagc ccaggcttct gtgggagaga 1800
gtggagaagc tggtgcccag acctggcagt ggcagctcct cagggtccag caactcagga 1860
tcccagcccg ggtctcaccc tgggtctcag agtggctccg gggaacgctt cagagtgaga 1920
tcatcatcca agtctgaagg ctctccatct cagcgcctgg aaaatgcagt gaaaaaacct 1980
gaagataaaa aggaagtttt cagacccctc aagcctgctg gcgaagtgga tctgaccgca 2040
ctggccaaag agcttcgagc agtggaagat gtacggccac ctcacaaagt aacggactac 2100
tcctcatcca gtgaggagtc ggggacgacg gatgaggagg acgacgatgt ggagcaggaa 2160
ggggctgacg agtccacctc aggaccagag gacaccagag cagcgtcatc tctgaatttg 2220
agcaatggtg aaacggaatc tgtgaaaacc atgattgtcc atgatgatgt agaaagtgag 2280
ccggccatga ccccatccaa ggagggcact ctaatcgtcc gccagactca gtccgctagt 2340
agcacactcc agaaacacaa atcttcctcc tcctttacac cttttataga ccccagatta 2400
ctacagattt ctccatctag cggaacaaca gtgacatctg tggtgggatt ttcctgtgat 2460
gggatgagac cagaagccat aaggcaagat cctacccgga aaggctcagt ggtcaatgtg 2520
aatcctacca acactaggcc acagagtgac accccggaga ttcgtaaata caagaagagg 2580
tttaactctg agattctgtg tgctgcctta tggggagtga atttgctagt gggtacagag 2640
agtggcctga tgctgctgga cagaagtggc caagggaagg tctatcctct tatcaaccga 2700
agacgatttc aacaaatgga cgtacttgag ggcttgaatg tcttggtgac aatatctggc 2760
aaaaaggata agttacgtgt ctactatttg tcctggttaa gaaataaaat acttcacaat 2820
gatccagaag ttgagaagaa gcagggatgg acaaccgtag gggatttgga aggatgtgta 2880
cattataaag ttgtaaaata tgaaagaatc aaatttctgg tgattgcttt gaagagttct 2940
gtggaagtct atgcgtgggc accaaagcca tatcacaaat ttatggcctt taagtcattt 3000
ggagaattgg tacataagcc attactggtg gatctcactg ttgaggaagg ccagaggttg 3060
aaagtgatct atggatcctg tgctggattc catgctgttg atgtggattc aggatcagtc 3120
tatgacattt atctaccaac acatatccag tgtagcatca aaccccatgc aatcatcatc 3180
ctccccaata cagatggaat ggagcttctg gtgtgctatg aagatgaggg ggtttatgta 3240
aacacatatg gaaggatcac caaggatgta gttctacagt ggggagagat gcctacatca 3300
gtagcatata ttcgatccaa tcagacaatg ggctggggag agaaggccat agagatccga 3360
tctgtggaaa ctggtcactt ggatggtgtg ttcatgcaca aaagggctca aagactaaaa 3420
ttcttgtgtg aacgcaatga caaggtgttc tttgcctctg ttcggtctgg tggcagcagt 3480
caggtttatt tcatgacctt aggcaggact tctcttctga gctggtagaa gcagtgtgat 3540
ccagggatta ctggcctcca gagtcttcaa gatcctgaga acttggaatt ccttgtaact 3600
ggagctcgga gctgcaccga gggcaaccag gacagctgtg tgtgcagacc tcatgtgttg 3660
ggttctctcc cctccttcct gttcctctta tataccagtt tatccccatt cttttttttt 3720
ttcttactcc aaaataaatc aaggctgcaa tgcagctggt gctgttcaga ttctaccatc 3780
aggtgctata agtgtttggg attgagcatc atactggaaa gcaaacacct ttcctccagc 3840
tccagaattc cttgtctctg aatgactctg tcttgtgggt gtctgacagt ggcgacgatg 3900
aacatgccgt tggttttatt ggcagtgggc acaaggaggt gagaagtggt ggtaaaagga 3960
gcggagtgct gaagcagaga gcagatttaa tatagtaaca ttaacagtgt atttaattga 4020
catttctttt ttgtaatgtg acgatatgtg gacaaagaag aagatgcagg tttaagaagt 4080
taatatttat aaaatgtgaa agacacagtt actaggataa cttttttgtg ggtggggctt 4140
gggagatggg gtggggtggg ttaaggggtc ccattttgtt tctttggatt tggggtgggg 4200
gtcctggcca agaactcagt catttttctg tgtaccaggt tgcctaaatc atgtgcagat 4260
ggttct 4266

<210> 10
<211> 2034

<212>  DNA
<213>  Homo sapiens
<220>
<221>  misc_feature
<223>  séquence appelée GS-N10

<300>
<308>  GENBANK/XM_035658
<309>  2001-12-10
<313>  (1)..(2034)
<400>  10
ccctggtttc ccagtgctgg aatatagaaa atggatggac aagtaaatcc cactcagcac      60
ccatagtcca ggcatgggga cctcaacaca cctgagcccc agacatcacc tttcattctg     120
gacctaccat gaaagacttc tgaatccagg aagagaaact gactgggcaa catgttattc     180
agaaacagga ccttgccctg tcactcagga tggagttcag tggtcctatc atggctcatt     240
atagcctcaa actcccaggc tcaagcaatc ctaccatgtc agccttccca gtagctggga     300
ctacagctgg atctaccatg aaagacttgt gaatccagga agagagactg actgggcaac     360
atgttattca gggtctccct ctgttgtcca aggctggagt gtagtagtgc tatcgcagct     420
gactgcagcc tcaaccttcc aggctgaagc gatcctccca cctcaacctc ccacgtggct     480
gagactacag gtgcttgcca ctatgcccaa ctaacatttg gaattttcgt atacgtggat     540
tccagagggg tgacagcgaa acgtgggacc atccagttgc aggaaaacaa gcttaacacg     600
ccccctgatt ctacattatg ctcctacctc ccggcagcct ctccaggccc agaactttct     660
ccagtcagcc tctacagacc aagctcatga ctcacaatgg cctatttagg cccataccct    720
acgtcacggc agcctccgca gatgagccta ctgcctcaca acagcctcca caggcacagc     780
tccatcgtta caatggcctc tttagaccca gctcctgcct cccagccttc tctccaggcc     840
ctgaactttc tcaagtcgac ctcaccaggc ccagctcatg cttctttgca gcctctccag     900
gcccagctcc tgcatcttgg tggcccctcc aggcccagcc tctgcctccc gtcagcctct     960
acagtcccaa cgtctgcctc acagcagatt cttcaggccc agcatctgcc tcactgtgga    1020
ccccccaagc caagctccca acctttcagc agcttctaca cacccagctc ctgccaccca    1080
gtggcctctt taggccaagc tcatgcttca caagggcctt tccaggccca acttttgtct    1140
catggcaacc ttccctggcc agattcctgc ctgtctccca gcagcctaga caggcccagg    1200
tcttgcctca cactggcctc tctacatcca gcttatgcct cacggtggcc tctccacgcc    1260
caactcctgt cccaggacat catctccggg cccaaaactt actcaagtca gactctctag    1320
tcccaactgc tgcctcctgg tggcctatga aggcccaaaa tctcctcaat ttgacctgtc    1380
caggcccagc tcctgcctcc tgtcagcgtc tacaggccca acctctgcct catgggggct   1440
tctccaggcc cacctcttcc tcttggctgg gtctacaggc acaactgctg cctcacaaca    1500
gccttttttg gcccagttcc tgtccagctc atggcggcca atgtaggccc aaaacttcct   1560
caagtcaaac tctccaggcc caccttctgc ttcccggtgg catcaacagg cccagctttg    1620
acttgagaac agcctctgca ggccctgctc ttgcctctca ggggcttttt ccaggcccag    1680
ctcttgcctc atggcagctg ccccaggcca aatttctgcc tgcctgccag cagcctcaac    1740
aggcacagct cctccctcac agtggcccat ttaggcccaa ctcatgactg tgaggccatt    1800
tccaggccta gtgcctgcct cgtggctgac tcttgaagcc caaaacttcc tcaaatcagg    1860
cttttgccca acttctgtct actgtcggac tctacaggtc agcctctgcc tcacagtgga    1920
ccctccagac ccagatggtg tctcactgtg gcatcctcag gcgaagctcc tgcctttcgg    1980
cagcctctcc aggcccagct cctcctgcct cccagtggcc tctttcggcc cagc          2034

<210>  11
<211>  1817
<212>  DNA
<213>  Homo sapiens
<220>
<221>  misc_feature
<223>  sequence appelée GS-N11

<300>
<308>  GENEBANK/NM_032181
<309>  2001-12-10
<313>  (1)..(1817)
<400>  11
agcataacct gtttgtgaaa ctgcaaaaag gttgacatgg ctgaagcaga agatgggttg      60
gtactgaatg gttgggagag atatgtagcg gctgaccctg aaggacgttg cagcccatgc     120
tatggagacc tgattttatt ttgtaagagg tgggcaaccc ctgaagcatt ttagtgatgc     180
gaacagatgt gtctgttacc aagcaaagcc agttaaagaa ggtcacagag gaggaatgat     240
cacaaagagt ctgagaagcc aactggggcc aaacaagttt tagaacctct actggctgat     300
gccaacatca tcagagatga actgttctta ccagaaactt ctctgctcct gacaccccac     360
ctctccccag aagagaagaa ggagagtggc cacgttgatt cagccaagca cctccaggag     420
gtcccctctg gatgtcccat gaggctgccc ctcagccaca gcccagagca cgtggagatg     480
gctttgctca gcaacatcct agcggcctat tcctttgtct cagaaaatcc tgagcgagca     540
gctctgtact ttgtttctgg cgtgtgcatc gggctggtgc tgaccctggc tgctctggtg     600
ataaggatct cttgccacac agactgcagg cggcgtcccg ggaagaagtt cctgcaggac     660
agagagagca gcagcgacag cagcgacagc gaggatggca gtgaggacac cgtgtccgat     720
ctctccgtgc ggagacaccg ccgcttcgag aggactttga acaagaatgt gttcacctct     780
gcggaggagc tggagcgcgc ccagcggctg gaggagcgcg agcgcatcat cagggagatc     840

tggatgaatg gccagcctga ggtgcccggg accaggagcc tgaatcgcta ctattaggga 900
gcagcaggac cccggaaacc actggaggcc gcctggaaag gagagcgtct gcagggacag 960
tgggcacaag gaactgaacc cagctctgct aatattgtga tttcagagaa aaagcaggac 1020
atgccccttt tctagccagg aggattgctc ctttttggcc aaatgtatgg agaagtagaa 1080
aaatcaaagc agttcatcac cctttccagg tctcggaatg gtgctgaaaa atcctctcca 1140
acactgtgga tggagatcgg agaaacgcga ctgtggtttc tcttgatttc tgaggatctc 1200
agaagtttca gcagacttcc ttgctctgtg ttattccttt gcaaaaggaa agatcattat 1260
agcatgaggg ctgggaatag cagggtgaac ttaacccaat aaatgcaatt tcctaaatga 1320
cttcatgcta tggaggtgat tctgattcaa atagcatgat ccatgcttat tatttaaggc 1380
tgatttttaa aatcttgtgt tgcaagggca acctcgtcca ttttaactgg cacctcaggg 1440
attaaaatcc tactttttag tgtagttccc acctcattca tgaaaatgaa tagaactatt 1500
gtattatggg agatgtgtca gtgaactaga aaatgtcaat aacctcaaag gatgaagggt 1560
ttttatttta aatagtttat aaaatatata ttgacatgca tatttgaaaa tgctgcataa 1620
aaataaaagt ggtgtgtttt ccccctcttt ggagacaaga aaattttatt atgactctag 1680
ataattgtga ttttaaacac tttgtttttt tttttttttt tttaattgga tttcaaagaa 1740
aagaatggaa atgagaggta aggattaaag ccaaagttag gatgggaatt aaaaaaataa 1800
acattacata aaatctt 1817

<210> 12
<211> 4131
<212> DNA
<213> Homo sapiens
<220>
<221> misc_feature
<223> sequence GS-N12

<300>
<308> GENEBANK/AB023233
<309> 1999-06-16
<313> (1)..(4131)
<400> 12
cttcaagacc gagctgccac ggccgcctcc ccgcccgccc cccattctac gcgcctgccc 60
acaccctcct cccctccttc cagcgccttt cggtggagca ctgcggcact cagcccgagc 120
tgccgttttc ccctcgcggg gaacgctgtg acccccccgc aggagcggcg gggcggggtg 180
ggggggcccg ggagaagatg gcgacgccgg gaagcgaacc ccaacctttc gtcccggccc 240
tttcggtagc tactctgcac ccacttcatc atccccacca ccaccaccac caccatcagc 300
accacggagg aaccggcgcc cccggcgggg cgggtggtgg cggcggtggc agcgggggct 360
tcaacctgcc cttgaaccgg ggtctggagc gcgcgcttga ggaggcggcc aactccgggg 420
ggctgaacct gagcgccagg aaattgaagg aatttccccg taccgcagcc cccgggcatg 480
acctctcgga cacggtgcag gcagacttat ctaaaaacag actggttgaa gttccaatgg 540
aattgtgcca ttttgtatca ctggaaattc ttaatctgta tcacaactgt atcagagtca 600
ttcctgaggc catcgttaat ctgcagatgc tgacttacct gaacttgagt cgaaatcagc 660
tgtccgccct gcctgcctgc ctgtgtggtc tgcctctcaa agtcttaatc gcaagtaaca 720
acaaacttgg atcattacca gaagagatag gtcagctcaa acagttaatg gagctggatg 780
tcagctgcaa cgagatcaca gcgttgcccc agcagatagg tcagttgaaa tctctacgag 840
aactgaatgt cagaagaaat taccttaaag ttttaccaca agaactagta gatcttccct 900
tggtaaagtt tgacttttcc tgcaacaaag tgctcgtgat tccaatttgt tttagagaga 960
tgaagcagct gcaagtgtta ctacttgaga ataaccctct gcagtctcct ccagcacaga 1020
tttgcacaaa gggcaaagtt cacatattta agtatctgag catacaagca tgccagatta 1080
agacagctga ctccctttat ctccacacca tggagaggcc acatttacac cagcacgtgg 1140
aagatggcaa gaaggattct gattcgggag ttggaagtga taatggagat aagcgattat 1200
ctgccaccga gccttctgac gaagacactg ttagcctcaa tgtgccaatg tcaaacatca 1260
tggaagaaga acagatcatc aaggaggact cgtgccatcg ccttagcccc gttaaagggg 1320
aatttcatca ggaatttcaa ccggagcctt cccttttggg tgacagcacc aactcaggag 1380
aagaaagaga ccagtttact gatagagcag atggtctcca ttcggaattt atgaactata 1440
aggcaagggc agaagactgt gaagagctgt tacggataga agaggatgtg cactggcaaa 1500
ctgagggcat aataagttca tccaaagatc aggacatgga tatagcaatg atcgagcagc 1560
tgagagaagc agtagatttg ctgcaagatc ccaatggatt aagcacagat attacagaga 1620
gaagtgtttt aaacctatat cctatgggat cagcagaagc cttagaatta caagattctg 1680
cactgaatgg tcaaatacag ctggagacat ctccggtgtg tgaggtgcaa agtgatctaa 1740
cattacagag taacgggagc cagtattctc caaatgagat tagagagaac tcccctgcag 1800
tctctcctac cacaaacagc acagctccat ttggcctgaa gcctcgatca gtgtttctaa 1860
gacctcagag aaatttggaa tctatagacc cgcagtttac aatccggagg aaaatggagc 1920
agatgagaga agagaaagag ctggtggaac aacttcgtga gagcattgag atgagattga 1980
aggtcagtct acacgaagac ctgggggcag ccctcatgga tggtgtcgtc ctctgccatc 2040
tggtcaacca catccgccca cggtcggttg caagcatcca tgtcccatca ccagcggttc 2100
ccaaacttag catggccaaa tgcagaagaa atgtggaaaa ctttttggaa gcgtgccgaa 2160
aattaggagt accagaggct gacctctgct ctccgtgtga catcctgcag ttggattttc 2220
gtcacattcg aaagactgtt gacactctgc tggcactcgg ggagaaagcc ccaccaccaa 2280
cttctgccct ccgctccagg gaccttatag gcttctgtct tgtccatatt ctctttatag 2340
tgctggtcta tatcacttac cactggaatg ctctgtccgc ataacgtctg cacgtgcatc 2400
caaacgctgt gctctgtcgc cctcaacctt tgcagggtcc ttcctacctt tgagcctttg 2460
ccttgcaaac ttccatccct gtcatgtctt cagttatctc tcgagttttg aagctgaaca 2520

gtagcaaatc agattttcca gaagcacaaa ctttgtagaa tacagtttag tataattcct 2580
ctcacttact gaaatacaac gacgacgact gcaaagtgta tgcacaccgc atgcttcctc 2640
atccacatag tgccagcagc agtgccacgc agttcctcct ctccctcccg gtgagctgct 2700
gccctgggca gaggggagga gaattccagg acaagagtgt caaggacagg gatttagcat 2760
atggaagtct ttcctttggg tcagtattga actagaattc taattcggga ctgggcaatt 2820
gagctgtata ggggccacct tgcagggagg acagaaaact aacattttgg cccaacttga 2880
tctatacaaa actttaataa taccactact gaccaagttg gacgtgtaca cgtactcaca 2940
ctgccttgat ggccattcga ttggattcct cccaaatttc ctaaaaaggg agccgcgaag 3000
ggcgctgggc agtgtggccg ccaacttcca ccccggcaag cccctctgtc ctatgcagaa 3060
gggcgctcca gggaaggaag tgtcgttgct gttagagcct cacgtggagg agtcacttaa 3120
acaccagttt tttactgctt aattccttgt taggtcttct cttgaggctc ttagaaaagc 3180
gttttccaga gagatttcta tttttgaaca atggaacgga tcactgcttt tttgccacat 3240
cacatagtaa ctgccggtcc agaatgtgac ggattcgact ctattcattt tcaaataaag 3300
ccatgagccg tggaacattc ttggtcctgg tgcttgggtt atgatggcag gagtcaagaa 3360
gaagattact ttcattctag aagaatgtag tttctctaat tatttgaaat gttcatttag 3420
cctttgattt tcactgatat taactagcaa actgctttaa gtcagctcaa aggattatat 3480
agtaactata tctgcatttg gagcaatgtg atcagtttgc atttaaaagg aaaaaaaaga 3540
attttatctt agccagaatg tccctggatt caggggtgtc tttgtataat atgagagggc 3600
cttgttccaa ggtcaaggca gcctccttat tttacatgct gtttgccaaa tcttgtttct 3660
tagcttgggg agatgatgga cttagcttcc tcaagataaa tttctagttt attaagatgc 3720
aaacagctct catagatggc tactacgaag aaaatcttat ttttctgaac attttcatga 3780
atccagggga cttgaaaata tggaagaccc acatagttag aagaatatat ttataaagat 3840
tccttgctgc taagtcagat cagatttgct aacaggaagc attctttaca tgacagtatc 3900
ttgagttatg tgagtttttt ttcctcctga ctttgtgttg attggtgaaa tgcagggtat 3960
gtggaagtta tctaattaac ctcagttgta tatgaataac ccacagatgt actgaattac 4020
ttttggtgct atcttgtact cttcaatctg taacacaata aaatcccttt gtacgatgtc 4080
taatgagcac cctgagccat aaattgctta ataaacacat tttgggtgat t 4131

<210> 13
<211> 2566
<212> DNA
<213> Homo sapiens
<220>
<221> misc_feature
<223> sequence appelée GS-N13

<300>
<308> GENEBANK/XM_018273
<309> 2001-10-16
<313> (1)..(2566)
<400> 13
tgaaaaaaga tggtgaagaa tgtactaacg aaggcaaagg aatagctgca cgaattcttg 60
ggccatccaa accacctcct tcaacatata atccacataa acctgttcct tatccgatac 120
ctccatgccg accacatgca actattgcac caagtgctta taacaatgca ggtctggtac 180
cattagcgaa tgtcatagct ccacctccac ctccatatac tcctaatcct gtaggaacag 240
agaatgaaga cctttcgaat ccgtcaaaac ctatacagaa tcaaacattt tccaccccag 300
caagtcaact cttttctcct catggttcta atccttcaac acctgctgca actcctgttc 360
ctactgcatc cccagtcaag gcaattaatc atccatcagc atcagcagct gccaccgttt 420
ctggaatgaa cctgctgaat actgtccttc ctgtgttccc agggcaggtc tcctcagccg 480
ttcacacacc tcagccatca ataccaaacc caacagttat cagaacccct tcattgccca 540
ctgcacctgt tacatccatc cacagtacaa ccaccactcc tgttccttcc attttttctg 600
gcctagtgtc actgccaggt ccttctgcca ctcctaccgc agccactcct accccaggac 660
ctacaccacg gtccactctt ggttccagtg aagcatttgc ttctacttct gcacctttca 720
ctagcctccc cttttccacc agctcttctg ctgcttctac cagcaaccca aattctgctt 780
cattgtcatc agtttttgca gggctccctt tgcccttacc accaacatcc caaggcctat 840
ccaacccgac tcctgtaatt gctggtggct ctactcccag cgttgccggt ccacttggtg 900
tgaacagtcc tcttttgtct gcgttaaaag gttttctgac atccaatgac accaatttaa 960
tcaactcctc tgctttatcc tctgctgtca caagtgggct ggcttcacta tcttctctta 1020
ctcttcagaa ctctgactct tctgcttcag cccctaacaa gtgctatgcc ccatcagcca 1080
tccctacccc acagaggact tccactccag ggttggccct gttcccaggc ctgccgtctc 1140
ccgtggctaa ctcaacttcc actcccctga cattgcctgt acagtctcct ttagccactg 1200
ctgcatcagc ttccacgtca gtgccagtta gctgtggctc ctcagcctcc cttttgcgtg 1260
gcccccaccc aggtacctca gatctgcata tttcatctac ccctgctgca acaactcttc 1320
ctgttatgat caaaactgag cccacaagtc ctactccctc ggccttcaaa ggtccatctc 1380
attctgggaa tccctctcat ggcactttag gtttgtcagg gacattgggc cgtgcatata 1440
cttcaacatc cgtgcccatc agtttatctg cttgccttaa tcctgcattg tcaggtctct 1500
ccagcttgag tactccttta aatggttcaa atcctctttc ctctatttcc cttccaccac 1560
atggttcctc cactcccatt gcaccagtat tcactgctct tccttctttt acttctttga 1620
ccaacaattt tcctttaact ggcaacccat ctcttaatcc gtcagtatct ctcccagggt 1680
cattaatagc cacctcatct accgctgcca cctccacatc tctccctcat cctagctcaa 1740
cggcagctgt tctctcaggg ctttctgctt cagcaccagt ctcagcagca cctttccccc 1800
tcaacctgtc cactgctgtt ccctcacttt tctctgttac tcaaggacct ctgtcatctt 1860
caaatccctc ctatccaggc ttttctgtct ctaatacccc aagcgttacc cctgctcttc 1920

cctcattccc ggggctgcag gcgccctcta cagtcgcagc tgtcacacca ctacctgtgg 1980
ctgccacagc cccatcccca gctccagtcc tcccaggatt cgcctcagca ttcagttcca 2040
atttcaactc cgctcttgtt gcacaagccg gtttatcatc tggacttcaa gctgcaggca 2100
gttctgtttt tccaggcctt ttgtccctcc cgggtatccc tgggtttcct cagaatcctt 2160
cacaatcatc cttgcaagaa ttacagcata atgcggctgc gcagtcagca ttgttacagc 2220
aggtccattc agcttcggct ctggaaagct atccagctca gcctgatggg tttcctagtt 2280
atccttcagc gccaggaaca ccattttctt tgcaaccaag cctgtcccag agtgggtggc 2340
agtgaatact tttaactttt attctccttc agagcaacat cagaattgcc tgagaactgc 2400
aatgaacaat ctgacaaatg tgaagctggc caaaagtcgg aaaatgagaa tgagggtaat 2460
cctggagaaa ttgtgacaac aatttgaaaa ttgtggttgc attttaaagt gtgaacactc 2520
ccctatgtaa atatgctgac aataaattgt atggagaatg gtattt 2566

<210> 14
<211> 1830
<212> DNA
<213> Homo sapiens
<220>
<221> misc_feature
<223> sequence appelée GS-N14

<300>
<308> GENEBANK/AK055109
<309> 2001-10-31
<313> (1)..(1830)
<400> 14
attaaaccct ttggtgctag gagctgacaa tttccaaagc agcctgtgaa gtcctagggg 60
ctgggggcca ctcttgcggc aagcagaagg ccatcctact ccgcggagtg atcatggaaa 120
tgtattttag ttaaactctg acagctccca aacggaagac tacagcatga cgtagtatta 180
tgattgcatt gtatgaaaga gcaagtgact ttctaagtag gatgaatcat attcatatgc 240
agatgtctta gcctcttgac gctggaagtg tggatttata gctatgaaac cactgctggc 300
agtgggtggg ccactgggac tgacgggggt taaagggcat tttactaagg cagctaagac 360
atattcagac atcaacgtta tccttctttt tcatatttct acctgagtga agttcatcct 420
tagtattgag taggaagtta cagtaaatgg tagttcattc ttacttacac acatagctaa 480
tctttttttt ttcacttgga attatgttga atgtttcatt ttgacaaaaa agtagactag 540
aaggtatgtt ctttaagttg tcttgcatcc attatataag aaagaaacag gtgagaggaa 600
gagcagaaag ctgagactgg ctgatgttca gagcacttac tcctctagag ggaaagcatg 660
acaccgaaca ctaagcacac agctttttgt tgttttggtt ttttctcccg caaatcttaa 720
agtgattccc atgaccttgg ccaaggacac ttcttaaaga ttaatgactg gcactgacat 780
tgccccaggc gggccactcc tcacactggc tctcagttcc cagccatgcc tggggctcag 840
tcacttctat tccaccctct gagactccat tggtgtcaca caaggtgtct tcttggcttt 900
gattttgaga atcccctatt ttcacttcca gatctgtcag ctgccatgga ggaataatag 960
aaaaccagaa atgcgtgtag agggagattt ctaaaacttc ccttgtgtcg cccatagttg 1020
tagttttggg ttctggcagg tggaacaccc tgaaacctgg aatcattcta tgagaataca 1080
gttcagactt tgcagactcc agcccatact aactgtcatg aagcttgact tcttgtcata 1140
atgcagccat cttggaggaa attggccatt tctgcttaga tggttggcag ggtcgcgctc 1200
agctttgctt tctacactaa ttacatagca ttattcaagt attgttttcc atttcccatc 1260
cctgatttcc agcttcttaa agctgactgt tcttgcaggg gccacttgct tctcctagag 1320
tacaaaagta agggccttcc ttactaactg cagggtctct ctattacacc tcaacataca 1380
cactttgctg ctactgtttg tactgtctac agtagaattt ccttatcttg ctcctggtag 1440
tgcattacag gcaagcatga aatgtaaagt atttatttaa ataaaaagaa aacctctaaa 1500
ttggtaattg aattacctcc ctgtagcttt atagtttgtg acatttcttg accttgctag 1560
ttctttcatt agatctgcgc aagatctagt catctggtta aggattttaa gcagatgcaa 1620
ctataaaccc aagaaactgt attactatta ctgttggtca tactaaacct gtctatttcc 1680
tgaagtatat gacccacaag gatgtggaat aactaggaga aactgttttt gtacactgta 1740
catccttagt atttttacac gtatatgata gggatgaaca tgattttcct tcgtacagac 1800
agcttaaata aagcactatg tcaatctgct 1830

<210> 15
<211> 6253
<212> DNA
<213> Homo sapiens
<220>
<221> misc_feature
<223> sequence appelée GS-N15

<300>
<308> Genbank/NM_014873
<309> 2000-11-02
<313> (1)..(6253)
<400> 15
ctccccgcca gtccgggcga acgcggccgg gcccttgggg accgagtctc ggcgccgccg 60
gggaacgggc gcgggccgcg ccacagccgg agcggggcag ggccccgcca ccgccttctt 120
ccgccggccc cgccgccggc catgcattct tccggctcct ctgactccca aagccccggc 180

tggggcccgg ccagcccgag aaagacagga cggagtccag tgtgagaatg gctataactt 240
tggaagaagc tccgtggctg ggctggctct tggtgaaagc actgatgagg tttgccttca 300
tggtcgtcaa caacctggtt gctattccat cctacatctg ctatgtaatt atacttcagc 360
cccttcgagt gctggacagt aagcggttct ggtatatcga aggaatcatg tataaatggc 420
ttttaggaat ggtagcttcc tggggatggt atgctggata tacagtgatg gaatggggag 480
aagatattaa agcagtttca aaagatgaag cagtgatgtt ggtgaatcat caggcaacag 540
gagatgtgtg cacactgatg atgtgcctcc aggacaaagg actggttgtt gctcagatga 600
tgtggttgat ggatcatatt tttaagtaca caaactttgg aattgtttct ctagttcatg 660
gagacttctt tataagacag ggaagatctt atcgtgacca acagctgctg cttctcaaga 720
agcacttaga aaataattac aggagcagag atcgaaaatg gattgttttg tttccagaag 780
ggggcttcct caggaagagg cgagaaacaa gtcaggcatt tgccaagaaa aataacttgc 840
catttcttac aaatgttact ctgccaaggt ctggggcaac aaaaattatt ttgaatgcac 900
ttgtagcaca acagaaaaat ggaagtccag caggaggaga tgctaaagaa ttagacagca 960
aatcaaaagg cctccagtgg ataatagata caacgatagc ttatcccaaa gctgaaccta 1020
tagatattca aacctggatc cttggataca ggaaaccaac agtcacacat gtacattaca 1080
ggatctttcc aattaaagat gtacccctgg agactgatga ccttaccact tggctctatc 1140
agcggtttgt tgaaaaagaa gacctcttat cacattttta tgaaacagga gcttttccac 1200
cttccaaggg ccataaggaa gctgtttcca gggagatgac cctcagcaac ttgtggatat 1260
ttctcataca gtcttttgca tttttgtcag gctatatgtg gtacaacatc attcagtatt 1320
tttaccattg cctgttttag gaattgacgt ggacttgtca aggtcaccgt aggagttcag 1380
actttctttc ataagtgtgc attattttac atgtgcaaat cagaatatat ttaaaaaaaa 1440
gcaaaagaaa ttcaatggat ggattaatat ttatccccct ttgggatatt ttaaaatcta 1500
ctaaaatgag gattagtaat ataatgacct gctaatatat tttaagaaca tgttttaaaa 1560
agatacactc tatcacatat ttaagcaaac atcacatttg gagaagagga aatcataaaa 1620
tcatcctaga agactatctg agagaaattc tgttgccacc agttatactt gacaatttag 1680
ttgaagctca gaaagttata ttcatccctc ttagctgtag tctatattag ggcagttctc 1740
tagaacgccc acatttccac caactcagta aacttgaggg gagctggttg gcacctcgtg 1800
aagaactctt tccctgtcgt ttgcagtaac aaactccagt ctgttgcagt aacaaactcc 1860
agtctgttgc agtaacaaac tctagaatat tgacattctc tgtgggggaa aagcagtgtc 1920
cactggaccc cttctggtac tggatgtgtt ctttacaaag gctagctcag tccaacattg 1980
tgtttacata cactcgtgct tttccttatc tgacttctca ttttgtatca gaggcatatc 2040
ataaattgat aattttgcaa aatgcacttt tttgagatgc agatatagca aaggattagt 2100
aatatagcct gaaaacaaat gggagcatag cagtgtgtga ggttctcgag aactgtcttg 2160
tctctgtgtg tttatttgcc tgccagtgct ctccagcgcc atcctgccct ggacaccacc 2220
ctgacgtgat gcctctattg cagctcagag gctttatttt ttccattttg acattggcac 2280
taaatgcatt tggggatggt taaaacaaat tactatagaa catttaaatg atcagtttaa 2340
ggggaaatag gctagtttat agaaaaataa gagctagtgg cttataatgg tgacaggttc 2400
tcatgtggca cccctaggac ctgtgcagac agtagtctgt tgaatcatta catcaaggag 2460
ctgcccctgt cagggtgagt gtaattagga acgataccag cacataaggc tccccccaat 2520
ctcttccagt tgctttttct tttttctttt tttttttaga caggtcttac tctgtcaccc 2580
aggctgtagt gcagtggcat aatctcagct cactgcagtc tctgcctccc gggttcgagc 2640
gattctcctg cctcagcctc ccgagtagct gggactacag gcacccacca tcatgcttgg 2700
ctaatttttg tatttttagt agagatgggg tttcaccatg ttggccaggc tggcctcgaa 2760
ctcctgacct caggtgatct gcccacctcg gcttcctaaa gtgctgggat tgcaggcgtg 2820
agccaccacg cccggcctcc agttgctttt gaagagggta aagtcaagtt tctatttcta 2880
gaaaacattt tttagaaatt tgttgcgatg tttgtacaat ttacctcata agagaaccac 2940
acccttctcc aaaagtgctg gtactgcatc agtgagatga gtagggtttt tgtttgggat 3000
tgtaagacta ttaagatcct agtaagtcag tggtagttta ctgtgatgtg agcattgtag 3060
attcccccgt tgtcactaat cacacaacaa ttttgagaag taggctataa aacaaaaaaa 3120
ggttgctgtt ttctattttt aaataaacca aaaaaaaaca gaaaagatgt gaattttccc 3180
cagttatgtg ggaaaggtaa agcaacacca aataaaagcc cacagcagct tcatctttac 3240
gataactcag tgcatatgtg aaagagaatg atgcattaac tgaaatacct cattgaatat 3300
tatactacta ctggtaaaat gcagaagaca gtgttaatgt gtttggtttg ggtactggtt 3360
gttataaaat gcaatttttt tttaaatcta agcatttcat tatgtgttct acagtgtcgg 3420
tgaataaatg aaaccaatct caatttagag gtatggatga tgacagaaag cccaatagaa 3480
gcttaatgat gcttctgttt gaggcccagc aagcaccact aaattactgg atgaaatgaa 3540
attgttcact tgagggatta gtcaaccatc tgggggagaa gttgctcact gtcaaataca 3600
gcatgcacgg tcctagctga tagacctttt cctcattgct acagcaagcc acagggtaga 3660
gtgaccagtt ctcctatcca aaaataaatg cgaacatgca tacataaatg tggctgaggg 3720
ccacttttgc catcactgtg ctccaaagga acatgatatt gttaaatata ctgtacacat 3780
taaactattc tataagatgc tttacttttc aagtacggtg tcattttcac agtctccagg 3840
gtgacaaatg ccaatagata tgtgagttaa tttttttaaa tatctccgtg agttgtaaat 3900
agatgtgtat ttgatctgcc ctatccttct ttgattcttt aacatgttct ctctcttttg 3960
tttgcaaaat atgtttgaaa gcattggtag tgctttcctt atcagtaact ggagttctcc 4020
tgcttgcact agagaaggta aagagagaat cagtattctt atatggcaat ctggggaagc 4080
agcaatatgc cactgtacaa aactgaagaa aagttcctaa tttgtacttt gtgaagggag 4140
atgaaaggac gtttaaagta tatatatttt gtcaagagga aagaagataa aactatgcca 4200
gttttatatc aatagcttgt agaagctcag ctcttcttgg tcttggctag actgcctaga 4260
ttcccacagc agacaaggtt gagaatccat tgctggaatc ttggtattga tgagttacag 4320
tgatggaaca tgtgcttggc cacaggcagg tccagtcact gcaaaagtga ccaagccagc 4380
aggtcaccct taacttcaga aacaattatt ggtggtgaac tgtacttaaa ttgcagagaa 4440
acctgtaagt aatggaaggt aaagaaaaat tacagaatgg aaaataatat tttgggcaag 4500
caaacaaatt cactgagaat tccaaaagta tattaaaaaa gaagatagct atgagttcag 4560

atctatctta ttggtcttta atattacaac caatccttaa ctttccacta taaaggaagg 4620
attactagat tgattacttt ctgggtagat aatctggtaa taaatgatag gtaaatcaaa 4680
aattactttt atttaggagt ttgaattctt actctcatca gacatttttt ttctagggac 4740
gcttactaat taaatgattt aagttgtttc ttaggggttt tttgcctata tatttatgac 4800
tgtgttaatg agtagtgaaa tgatgcggaa agacagctat caggaagagg aaatacagaa 4860
gcctgaataa tctatgggtt agaaaagcat ccctgaataa tcaaaaattg gcagtattgg 4920
cattgttctc aagccttttt atgaaaatga aatctgaaat caccaaatgt aaacctggga 4980
acattattct agtgttgctg tcttggattc atgttaagaa gcgtcttcat tctttgctca 5040
tgttgcccac ttcttgtgga tttgtctgag tgttttttga caatcacttc cttaaagact 5100
cttctgaact agttggacct ggttaatcat agagagtagc ctttaatcat ggatagtctt 5160
cttggattat ttttatattt gaaaagaaaa tgttttattt gcactactga gtaggaagag 5220
ttaattgttt tctttgttct ttttttgaag tcattacaca ggacttcact ccagagttac 5280
cattatgagt gtgttcagct ctggtccaca gaggatggat aaaaatggtt tgttatgttt 5340
ttttgctctg cagtgctatg agccttatat ctgttaatat gaaggacaaa gtcaaaagca 5400
gcagtggata gcaggaaggg tagagactaa tatgtttggg accaaaacca tctaagttag 5460
agatttccag atcacagagg ggctgggcat tctctggagc agtcattggt tggtgcttta 5520
ttgtaatcat tttgcgccaa tccccaacaa ttaggaactg gaccctggga ataagctgag 5580
ggtgctgaac tgttggggaa gggtgactgt agccacatgg aagataaaat atgggttttt 5640
ctgcaaaatt tccatctgag ggtttttaca tttaatattt ttttaagaca gtttaaagag 5700
caaacgtttt ttaagtgtat tctagttgca aagtatgcac acatatcttg aatggcttta 5760
tttttattgt gtaaaactgt tgaacacatg actgtgatgc acaaattctt tacgtgtaag 5820
gagtctatgc attttacagt aacttatttt atgatcgggt gatgagacag ttatactttc 5880
aactgccatt atttttatta agtgctttca ttttctttac agttattata aaattgtatt 5940
tattttatac agatgggttt tcattttcct gatgctgtaa tgtttacttc agcttgttga 6000
cctttctttg tgttatctgc atgttgtaac gtgtgataag aatgaatgta aaggctgtgg 6060
caactgtaat taatttttgt aaagggctgg tcacacgtgg atctggttta tgaatgcatt 6120
tgggatgatt ttggtaacca gatcaccttt tcagaaattt agatgtgaac accaaaagaa 6180
gcattttctc aacaaaaatt aatagctggt tctatttttt ttaaacctag aaaaaataaa 6240
gttgattttt ttc 6253

<210> 16
<211> 4749
<212> DNA
<213> Homo sapiens
<220>
<221> misc_feature
<223> séquence appelée GS-N54

<300>
<308> GENBANK/AK024248
<309> 2000-09-29
<313> (1)..(4749)
<400> 16
gcgggtgttc ggctacgtca ctggggcgct acggttcctg gagctgggca gtcttctcgt 60
cagagtgggg actggtaaga gcgacctccc cgccaggtcc tgtgtgttgc cggctgaaga 120
agggatgatg attattcccc accttctaag agacaaagac caacgagcca ccacagccac 180
cagtcccaga acccgccaat gctggggaat ggaaaatgag ggagttcaac tctggccctc 240
acaatccagt ggaggagacg aaactcatct gcctctgtcc ctctgggcac acctcatgcc 300
aggtgcatct gtggacaggg gccatgctcc tgggcttcca aagttggaga aagctgccag 360
gctcagaccc acctaagctg aagattccct tgagaacaag tactgtcctg tggtttcatg 420
gcctttcttc catttgtggt tcttgcgaag tggaatttaa atgacatctt atcaagatgg 480
ataaaccctg gtttcccagt gctggaatat agaaaatgga tggacaagta aatcccactc 540
agtacccata ggccaggcat ggggacctca acacacctga gccccagaca tcacctttca 600
ttctggacct accatgaaag acttctgaat ccaggaagag aaactgactg ggcaacatgt 660
tattcagaaa caggaccttg ccctgtcact caggatggag ttcagtggtc ctatcatggc 720
tcattatagc ctcaaactcc caggctcaag caatcctacc atgtcagcct tcccagtagc 780
tgggactaca gctggatcta ccatgaaaga cttctgaatc caggaagaga gactgactgg 840
gcaacatgtt attcagggtc tccctctgtt gtccaaggct ggagtgtagt agtgctatca 900
cagctgactg cagcctcaac cttccaggct gaagtgattc tcccatctca acctcccacg 960
tggctgagac tacaggtgct tgccaccatg cccaactaat atttggaatt ttcatataag 1020
tggattccag aggggtgaca gcaaaacgtg ggaccatcca gttgcaggaa aacaagctta 1080
acacgcccac tgattttaca ttatgctcct accacccggc agcctctcca ggcccagaac 1140
tttctccagt cagcctctac agaccaagct catgactcac aatggcctat ttaggcccat 1200
accctacctc acggcagtct ccgcagatga gcctactgcc tcacaacagc ctccacaggc 1260
acagctccat cattacaatg gcctctttag acccagctcc tgcctcccag ccttctctcc 1320
aggccctgaa ctttctgaag tcgacctcac caggcccagc tcatgcttct ttgcagcctc 1380
tccaggccca gctcctgcat cttggtggcc cctccaggcc cagcctctgc ctcccgtcgg 1440
cctctacagt cccaacatct gcctcatagc agattcttga ggcccagcat ctgcctcact 1500
gtggaccccc caagccaagc tcccaacctt tcagcagctt ctacacaccc agctcctgcc 1560
acccagtggc ctctttaggc caagctcatg cttcacaagg gcctttccag gcccaacttt 1620
cgtctcatgg caaccttccc tggccagatt cctgcgtgtc tcccagcagc ctagacaggc 1680
ccaggtcttg cctcacactg gcctctctac atccagctca tgcctcacgg tgacctctcc 1740
aggcacagct cctgtcccag aacgtcatct ccgggcccaa aacttactcg agtcagcctc 1800

tctagtccca actgctgcct cctggtggcc tatgaaggcc caaaatctcc tcaagttgac 1860
ctctcccggc ccagctcctg cctcctgtca gcatctacag gcccaacctc tgcctcatgg 1920
gggcttctcc gggcccagct cttcctattg gctgagtcta caggcacaac tgctgcctca 1980
caacagcctt ttttggccca gttcctgtcc agttcatggc ggccaatgta ggcccaaaac 2040
ttcctcaggt catactctcc aggcccacct tctgcttcct ggtggcatga acaggcccag 2100
ctttgacttg agaacagcct ctgcaggccc tgcacttgcc tcccaggggc ttttccaagc 2160
ccagctcttg cctcatggca gctgccccag gccaaatttc ttcctgcctg ccagcagcct 2220
caacaggcac agctcctccc tcacagtggc ccatttaggc cccactcatg actgtcgggc 2280
catttccagg cctagtgcct gcctcctggc tgactctttc ttgaagccca aaacttcctc 2340
aaatcagcct tttgcctaac ttctgtctac tgtcggactc tacaggccag cctctgcctc 2400
acagtggacc ctccagaccc agatggtgtc tcactgtggc atcctcaggc gaagctcctg 2460
cctttcggca gcctctacag gcccagctcc tgccttgcag tggcctcttt aggccaagct 2520
catgccgcat ggcgactttt ccaggcacag cttttgcctt ttgcagcctg tccaggccca 2580
gaatgtcctt aactcggcat ctccaggacg agcttatcct cccagtgcgt ctacaggccc 2640
gtctcctgcc tcacaacaac ctcttttggc ccaactcctg ctgagctgct ggcagcctct 2700
gtaggccaca gacttcttaa agtaaagctt tccagccacc ttcggcctcc cagcagcctc 2760
agcaatcaaa ctattccctc actgcggcca ccgaaagcca agtttctccc tgcctcatgg 2820
catcctccga aaactgagca tttgcctcac ggtggcctcc ccaggccacg aatctgcctg 2880
cctcccaggc aggtgctgcc tcacaatggt ctctttaggc ccagctcatg ctaaaagatg 2940
gactctccag gtacagctct tgcctcctgg cagcctctgc aggcccaaat tctccaaaag 3000
ttggcctctc ctaactcagc tcctgcctca tgtcggccta cacaggccca gactcttacc 3060
acacagtaga ccctccaggc ccaccacttg cctgatcata gcctcctaag gccaagctcc 3120
tgcctttcag cagcctctac aggcccagct cctgcctcgc aattgccttt gtaggccaag 3180
atcatgccgc gaagtggcct ttcctagcct aacttttgct ttttgacgca tactccagtc 3240
ccaaaacttc ctccagtcag ccggtccagg ccaagctctt cctcccaaag gcttctgcag 3300
gccaaaatca tcctgaagtc accctctgca ggcccagctc ctgcctccaa gtgctgtgta 3360
ggccaagcta atgcctcaca gcacactttc caggctgagc gtttcctttt gtgcatcctc 3420
tccaagccct gaacttactc cacttggcct ctccagacca agctctccct cccagtggcc 3480
tctacaggcc aaaattgtcc tcaggtctgc ctctccaggg ccacctctta gctaccagtg 3540
gcttctgctg gccaaaatcg acctcaagtc agcctcttca cacccagctc ttgcctctga 3600
gtggcctctc caggagcaaa actttctcaa gtcggcctct ccaggcccag cctcctgctt 3660
cccgagggca tgtacaggcc cagcctctgc ctcacagcag actctccaca cccagctcat 3720
ccctgtctgc ggcctctcca gtccaaagct gctgctgcct tttggcagct tgtacaggct 3780
cagctcctcc ctcacggtgg cctctttcag cccaactcat gcctcttgca acctgcccaa 3840
gtgtcagctc ctgtctcaca ctggcctgtt gagggccagc tcatgcctct cgtggcctca 3900
acaggcccat cccctgcctg tcggcggcct ctacaggccc ggcctctacc tcacagtggg 3960
ctctccaggc ccacctcttc ctcaccgtgg cctcctgggg caatgctcct ccctctcggg 4020
agcctctgcg ggcccagctc ctgcctccca gtggcctctg tagaccaagc ccgtgcctca 4080
gggcagcctt tccaggccta gcgtttgctg ctttgcatcc tctccaggcc ctggacttcc 4140
tccagtcggc ctctccaggc ccagctcttc ctctcggcgg cctctgcagg gccagactgt 4200
cgtcaagtcg gcctgtccag ggccagctcc tgcctcccgg cggcctctgc aggcccaagt 4260
cgttctcaag tcggcttccc caggcccagc tccggcctct tggcggcctc tccgggtgca 4320
aaagttcctc gagtcagcct ctccaggccc agctcctcct gcctcccagt ggcctctttc 4380
ggcccagccc agctcttcct cccggctgcg tctccaggcc cgactttggc ctcccaacaa 4440
cgtctttgga ctcagctcct gcctctcaaa ggcctgcacg ggcccggcct cggcctcggc 4500
ctcacagcgg actctccacg cccagctagc tctcgcctca ctgtggcctc cccagtccaa 4560
agctcctgcc tttcggccac ttcagcaggt ccagctcctg cctgccagtg gcctctttag 4620
gcccagctca ttcctcacaa cggccttccc aggccccgtt tttcccttcc ggcagcctct 4680
tggcctctaa tttgtttatc ttttgtgtat aaatcccaaa atatggaatt ttggaatatt 4740
tccaccatt 4749

<210> 17
<211> 3139
<212> DNA
<213> Homo sapiens
<220>
<221> misc_feature
<223> séquence appelée GS-N16

<300>
<308> GENBANK/XM_011833
<309> 2002-12-10
<313> (1)..(3139)
<400> 17
cgcaggaagg ccgcctcatt ggtccgcgct gtcgggcttc ggcggctttt ctctttagcg 60
ctcagggcgc tgacccactg ctcttcctct taagaaagtg ctccattcct tccggcgccc 120
ggagctgctg gcccaaaggg atccggagcg agctagggca gacaccatga ccacccttga 180
tgataagttg ctgggggaga aactgcagta ctactatagc agcagtgagg atgaggacag 240
tgaccacgag gacaaggacc gaggcagatg tgccccagcc agcagttctg tgcctgcaga 300
ggctgagctg gcaggcgaag gcatctcagt taacacaggc ccaaaaggtg tgatcaatga 360
ctggcgccgc ttcaagcagt tggagacaga gcagagggag gagcagtgcc gggagatgga 420
aaggctgatc aagaagctgt caatgacttg caggtcccat ctggatgaag aggaggagca 480
acagaaacag aaagacctcc aggagaagat cagtgggaag atgactctga aggagtttgc 540

cataatgaat gaggaccaag atgatgaaga gtttctgcag cagtaccgga agcagcgaat 600
ggaagagatg cggcagcagc ttcacaaggg gccccaattc aagcaggttt ttgagatctc 660
cagtggagaa gggtttttag acatgattga taaagaacag aaaagcattg tcatcatggt 720
tcatatttat gaggatggca ttccagggac cgaagccatg aatggttgca tgatctgcct 780
tgccgcagag tacccagctg tcaagttctg caaggtgaag agctcagtta ttggcgccag 840
cagtcagttc accaggaatg cccttcctgc cctgctgatc tataaggggg gtgaattgat 900
cggcaatttt gttcgtgtta ctgaccagct gggggatgat ttctttgctg tggaccttga 960
agcttttctc caggaatttg gattactccc agaaaaggaa gtcttggtgc tgacatctgt 1020
gcgtaactct gccacgtgtc acagtgagga tagcgacctg gaaatagatt gaactgatag 1080
tctagttgca tagatttctc attgtttggg ttggaataca cgtcattgtt tatttttgtt 1140
cctttgtctt ctggcttttc agctgttctt tgtagtccct tttattatgc ataaaataaa 1200
gaaattctta gattaaatca gaatgctgaa taaccttgta gctagcaata aggtgactta 1260
cagttgtata aacaggaagc caggcttttg aactgtttac ttaagattct gtggtgtgac 1320
atctctgtta ttgtttccag tcaatattta caaagcatcc taaagacagg gtcttggaaa 1380
ttgtcttcag atgatcttag aggtctctgc caagtctgag agtataattc tgtaggtatt 1440
gtgttatttg caacgtaaat agtgcatttt cttaatcaaa tgattgtaaa ttatatttac 1500
ttgtaatcag ttccatagct ttagacggtg gttagatttt ttttttcccc accagggtct 1560
tgtttaaagg ggtgagccac cgcacccagt cctgaggggt ggcctctgct gctggatttc 1620
atgtcttcct ccagcatgac taagtctgga acagcaggaa gggttgatgc ttactgacct 1680
ggtgatgtta gaagacaagt agtttatgga tttaaacatt agagctggag tggggctgga 1740
aatctttgta aaggaagttc tttcagtaag atgcccctgc ttgtctttgt ctcttttttg 1800
tttaacaagg taactttttg tttaacaagg taactttttg tttaacctag atttttttta 1860
aaactttttt tttttttcat attggaaaag taattcatat tcagtagagg aaaactgacc 1920
aaaacagaag caaaaataag aaaattaaaa taatctctaa tcctactacc tagaataaaa 1980
cactattaat attttggtct gtttcctgcc aaggtgtttt ctgtgtatac atggatattt 2040
tgtttgtttt taaacaaaac gatgggatca ttctgaacat actgttctat agtatggtca 2100
gctaataata tatcagacct ttttttatat tattaaatat tctacaactt tttaaaaatg 2160
tctattaata ttccatcgta tagatgtgat ataatttgct tgatggttgt ctcttaaaaa 2220
gaaagatagc aaatactttt tttaaattac aaaagtgata gatgttcatt gtagaaaatg 2280
taataaacac tgttaagact taaaagccat ataattccac caaccaaaat taatcccttt 2340
tgtcatattt ctagtcattt ttatagcctt tttttctatg tatttataat aattatcatt 2400
tgcgtttttt tccttttttt aactttaaaa atgtatattc tagggtcagg ggaaatgtaa 2460
tctggaatta aatattagcc ttaaaattca caattttgat tttcctggct tttcaggaat 2520
tgactaactg taaaagagtc ttgaaagtat ttagtcaaca aacagagtgc attttttttt 2580
tttttgacta agaaagctcg ttgtagtaga aagggtggaa tgtattgaaa attattagaa 2640
gcagggaagt attgttagtc tagcttattt cctttcagtc ttttttcaat atttttataa 2700
acattgagta cttactgaat ttagttctgt gctcttcctt atttagtgtt gtatcataaa 2760
tactttgatg tttcaaacat tctaaataaa taattttcag tggcttcata atatttcatc 2820
atttggatat ataataattt atttaaccag tctctttatt tttatttatt tattttgttt 2880
gttttctttg agatggagtc tcgctctgtc acccaggctg gagtacaata gcgtgatctc 2940
ggctcactgc aacctccacc tcctgggttc aagcagttct cctgcctcag cctcctgagt 3000
agctgggcac ggtgttgtac ctctagtccc agctacttgg gaggcttagt caggaggatc 3060
tcttgagcct gggaggtcaa ggctgcagtg aaccatgatt gcactccaac ctgggcgaca 3120
gagaccctgt ctcaaaaaa 3139

<210> 18
<211> 2326
<212> DNA
<213> Homo sapiens
<220>
<221> misc_feature
<223> séquence appelée GS-N17

<300>
<308> GENBANK/BC011860
<309> 2001-08-29
<313> (1)..(2326)
<400> 18
ggcacgaggg gagtccggga cgtggagacc cggggtcccg gcagccgggc ggcccgcggg 60
cccagggtgg ggatgcaccg ccgcggggtg ggagctggcg ccatcgccaa gaagaaactt 120
gcagaggcca agtataagga gcgagggacg gtcttggctg aggaccagct agcccagatg 180
tcaaagcagt tggacatgtt caagaccaac ctggaggaat ttgccagcaa acacaagcag 240
gagatccgga agaatcctga gttccgtgtg cagttccagg acatgtgtgc aaccattggc 300
gtggatccgc tggcctctgg aaaaggattt tggtctgaga tgctgggcgt gggggacttc 360
tattacgaac taggtgtcca aattatcgaa gtgtgcctgg cgctgaagca tcggaatgga 420
ggtctgataa ctttggagga actacatcaa caggtgttga agggaagggg caagttcgcc 480
caggatgtca gtcaagatga cctgatcaga gccatcaaga aactaaaggc acttggcact 540
ggcttcggca tcatccctgt gggcggcact tacctcattc agtctgttcc agctgagctc 600
aatatggatc acaccgtggt gctgcagctg gcagagaaga atggctacgt gactgtcagt 660
gagatcaaag ccagtcttaa atgggagacc gagcgagcgc ggcaagtgct ggaacacctg 720
ctaaaggaag ggttggcgtg gctggactta caggccccag gggaggccca ctactggctg 780
ccagctctct tcactgacct ctactcccag gagattacag ctgaggaggc cagagaagcc 840
ctcccctgac tgcatgtgga agggcacaca gcagcaggca gggaggaggc ggaggtggca 900

aataaacctg ggcaattttg tttatacaaa aaataaaaaa aaaaaaaaaa aaaaaaaaaa 960
aaaaaaaaaa aaaaaaaaaa aaaaaaaaaa aaaaaaaaaa aaaaaactcg agactagttc 1020
tctggcgtga tgtctcacag aaagttctcc gctcccagac atgggtccct cggcttcctg 1080
cctcggaagc gcagcagcag gcatcgtggg aaggtgaaga gcttccctaa ggatgaccca 1140
tccaagccgg tccacctcac agccttcctg ggatacaagg ctggcatgac tcacatcgtg 1200
cgggaagtcg acaggccggg atccaaggtg aacaagaagg aggtggtgga ggctgtgacc 1260
attgtagaga caccacccat ggtggttgtg ggcattgtgg gctacgtgga aacccctcga 1320
ggcctccgga ccttcaagac tgtctttgct gagcacatca gtgatgaatg caagaggcgt 1380
ttctataaga attggcataa atctaagaag aaggccttta ccaagtactg caagaaatgg 1440
caggatgagg atggcaagaa gcagctggag aaggacttca gcagcatgaa gaagtactgc 1500
caagtcatcc gtgtcattgc ccacacccag atgcgcctgc ttcctctgcg ccagaagaag 1560
gcccacctga tggagatcca ggtgaacgga ggcactgtgg ccgagaagct ggactgggcc 1620
cgcgagaggc ttgagcagca ggtacctgtg aaccaagtgt ttgggcagga tgagatgatc 1680
gacgtcatcg gggtgaccaa gggcaaaggc tacaaagggg tcaccagtcg ttggcacacc 1740
aagaagctgc cccgcaagac ccaccgaggc ctgcgcaagg tggcctgtat tggggcatgg 1800
catcctgctc gtgtagcctt ctctgtggca cgcgctgggc agaaaggcta ccatcaccgc 1860
actgagatca acaagaagat ttataagatt ggccagggct accttatcaa ggacggcaag 1920
ctgatcaaga acaatgcctc cactgactat gacctatctg acaagagcat caaccctctg 1980
ggtggctttg tccactatgg tgaagtgacc aatgactttg tcatgctgaa aggctgtgtg 2040
gtgggaacca agaagcgggt gctcaccctc cgcaagtcct tgctggtgca gacgaagcgg 2100
cgggctctgg agaagattga ccttaagttc attgacacca cctccaagtt tggccatggc 2160
cgcttccaga ccatggagga gaagaaagca ttcatgggac cactgaagaa agaccgaatt 2220
gcaaaggaag aaggagctta atgccaggaa cagattttgc agttggtggg gtctcaataa 2280
aagttatttt ccactgaaaa aaaaaaaaaa aaaaaaaaaa aaaaaa 2326

<210> 19
<211> 3937
<212> DNA
<213> Homo sapiens
<220>
<221> misc_feature
<223> séquence appelée GS-N18

<300>
<308> GENBANK/XM_042798
<309> 2001-10-16
<313> (1)..(3937)
<400> 19
aagttctgcc ttgtctccgc cgcgggtcag gggtgagagc tggaatctct gcacgggcct 60
tggaaaacga ctgtcttctt ctgccaaaat gtcaggaatt ggaaataaaa gagcagctgg 120
agaacctggc acctccatgc ctcctgagaa gaaggcagct gttgaagatt cagggaccac 180
agtggaaaca attaagctag gaggtgtctc ttcaacggag gaactagaca ttagaacact 240
gcaaaccaaa aatcgcaagc tggcagaaat gttggatcag cggcaggcca ttgaagatga 300
acttcgtgag cacattgaaa aactggaacg acgacaggcc actgatgatg cctcactatt 360
gattgtcaac cgatactgga gtcagtttga tgaaaacatc cgtatcatcc ttaaacgtta 420
tgatctggag cagggcttgg gagacctact cacagaacga aaagcccttg ttgtgcctga 480
accagaacca gactctgata gcaatcagga gcgtaaagat gaccgagaga gaggggaagg 540
gcaagagcca gctttctctt tccttgctac tttggccagc agttccagtg aagagatgga 600
gtctcagctg caggaacgtg tggagtcttc ccgccgagcc gtgtcccaga ttgtgactgt 660
ttatgataaa ttgcaagaaa aagtggagct cttatcccgg aagctaaaca gtggagataa 720
tctgatagtg gaggaagcag tgcaggagct gaactctttc ctcgcacagg agaatatgag 780
gctacaggaa ttgacagatc ttcttcagga aaagcatcgc accatgtctc aggagttctc 840
caagttgcag agtaaagtgg agacagccga atcacgagtg tctgtcctgg agtccatgat 900
tgatgacctg cagtgggata ttgacaaaat tcgaaagagg gaacagcgac tcaaccgaca 960
cttagcagaa gtcctagaac gggtgaattc caaaggttat aaggtgtatg gagcggggag 1020
cagtctgtat ggcggcacaa tcactatcaa tgctcggaag tttgaggaaa tgaatgcaga 1080
gcttgaggag aacaaagagt tggctcagaa ccgtctctgt gagctggaga aacttcggca 1140
agactttgag gaggtcacta cacaaaatga aaagctgaag gtggaattgc ggagtgcagt 1200
ggagcaagtc gttaaggaaa ctccagaata tcgctgcatg cagtcacagt tctccgtctt 1260
gtataatgag agcctacagt tgaaagcaca cttggatgag gctcggaccc tgcttcatgg 1320
caccagagga acccaccagc accaggttga gcttattgag cgagatgagg ttagtcttca 1380
taagaagctg aggactgaag taattcagct agaagataca ttggcccagg tccgcaagga 1440
gtatgaaatg ctgaggatag aatttgagca gacccttgct gccaatgaac aagcaggccc 1500
tataaacagg gagatgcgcc acctcatcag tagcctccag aatcacaatc accagctgaa 1560
aggggaggtc ctgagatata agcggaaatt gagagaagcc cagtctgacc tgaacaagac 1620
acgcctgcgt agtggtagtg ccctcctgca gtcccagtct agtactgagg acccgaagga 1680
tgagcctgcg gagctaaaac cagattctga ggacttatcc tcccagtcct cagcttcaaa 1740
ggcatctcag gaggatgcca atgaaatcaa gtctaaacgg gatgaagaag aacgagaacg 1800
agaaaggagg gagaaggaga gggaacgaga aagagaacgg gagaaggaga aggagagaga 1860
acgagagaag cagaagctaa aagagtcaga aaaagagaga gattctgcta aggataaaga 1920
gaaaggcaaa catgatgatg gacggaaaaa ggaagcagaa attatcaaac aattgaagat 1980
tgaactcaag aaggcacagg agagccaaaa ggagatgaaa ctattgctgg atatgtaccg 2040
ttctgcccca aaggaacaga gagacaaagt tcagctgatg gcagctgaga agaagtctaa 2100

ggcagagttg gaagatctaa ggcaaagact caaggatctg gaagataaag agaagaaaga 2160
gaacaagaaa atggctgatg aggatgcctt gaggaagatc cgggcagtgg aggagcagat 2220
agaataccta cagaagaagc tagccatggc caagcaggaa gaagaagcac tcctctctga 2280
aatggatgtc acaggccagg cctttgaaga catgcaggag caaaatatcc gtttgatgca 2340
gcaattgcgg gagaaggatg atgcaaattt caagctcatg tcagagcgta tcaagtccaa 2400
tcagatccat aagttgctta aagaagagaa ggaggagctg gcagaccagg tgttgactct 2460
gaagactcag gttgatgccc agctacaggt agtaaggaaa ctggaagaga aggagcatct 2520
gttacagagc aacattggca caggggagaa agagctgggt cttaggaccc aagccttaga 2580
gatgaataaa cgcaaggcaa tggaggcagc ccagcttgca gatgacctca aagcacaact 2640
ggagttggct cagaagaagc tacatgattt tcaggatgag atcgtggaga acagtgttac 2700
caaagaaaag gacatgttca atttcaaacg agcccaggag gacatctcta gacttcgcag 2760
gaagctggag accacaaaga aaccagacaa tgtacccaag tgtgatgaga ttctgatgga 2820
agagattaag gattacaagg cacgcttgac ctgtccgtgc tgtaacatgc gtaaaaagga 2880
tgctgttctt actaagtgtt ttcatgtctt ctgctttgag tgtgtgaaga cacgctatga 2940
cacccgccag cgcaaatgtc ccaagtgtaa tgctgctttt ggtgccaatg attttcatcg 3000
catctacatt ggttgatcta agtcaagaga agaagaggag ctggctagtc aggaacttat 3060
tcattaacca ccaaacctct acctcttctc tccttgactg tcacctgtag gacagtttat 3120
cagtcaacta cctttcctcc agactttact tccaggctct cctcttcagt agctggatga 3180
ctttagcaga aaggactggt aaatacaagc cttgggtttc agaatgaatt agaaacaaat 3240
aactcttact gtcttccctc ccagctttgt ttattttgtg cttttagact tttcagtgtt 3300
ttctttttcc agcccactgt ataaacttgg attgtccatt cctcctgaag aaatcaagtt 3360
ggtatttttg atgtggaaaa gggaacaaaa gtggaaacat ggctactttt ggggagtgat 3420
attttaaaaa ataagttgtc tatgggcaca aagttttctt catttgtgta gcaaacttct 3480
tgtgaatgtg gattacaaaa tggtataatt gtgctactct cccctgggtg gtttgcagcc 3540
ctaatgaaat tatgtctagg atgattcagt ctatttccca tttactagca gagtaacttg 3600
ttaagatcag ctggctttct tgttaaagtt atttaagttt tgaatgctct actacttcaa 3660
gtctttaaat ttcttgagac tagaataatt ttaaataata tgaccctttg tcttctaatg 3720
aaataaagat tgaagaggtt gagtcaggac tgagctggtg aagaaatctt gtgggtattc 3780
tggaaatttg atacggagag aacttggtga gctatgaatt actctcagtc tcctttttac 3840
agggttgttg tgatccctct tttccagaaa attctgtgga atgtttctgt aggactttgt 3900
tctccacaag cttgaattaa agcaggattc agtttgc 3937

<210> 20
<211> 2167
<212> DNA
<213> Homo sapiens
<220>
<221> misc_feature
<223> séquence appelée GS-N19

<300>
<308> GENBANK/BC002781
<309> 2001-07-12
<313> (1)..(2167)
<400> 20
ggcacgaggc cgttgccgcc gccgccgctg ccgccgtgct ctcgctttgc ccgccgccgc 60
ctaagggggg ctggggccgg ggccagccat cactgccgtt gccgggatgc cgcgggtgta 120
catcggccgc ctgagctacc aggcccggga gcgcgatgtg gagcgcttct ttaagggcta 180
cgggaagatc ctggaggtgg atctgaagaa cggatatggt tttgtggagt ttgatgatct 240
gcgtgatgca gatgatgctg tttatgaact gaatggcaaa gacctttgtg gtgagcgagt 300
aattgttgag catgcccgcg gcccacggcg agatggcagt tacggttctg gacgcagtgg 360
atatggttat agaagaagtg gccgagataa atatggccct cctactcgca cagagtacag 420
acttattgtg gagaatttgt caagtcggtg cagctggcaa gacctaaagg attatatgcg 480
tcaggcagga gaagtgactt atgcagatgc tcacaaggga cgcaaaaatg aaggggtgat 540
tgaatttgta tcttattctg atatgaaaag agctttggaa aagttggatg gaactgaagt 600
caatgggaga aaaatcagat tagttgaaga caagccaggt tccagacgac gccggtccta 660
ctccagaagc cggagtcatt caaggtctcg ctctcgaagc agacattccc gtaagagcag 720
aagccgaagt ggcagcagca aaagcagtca ttctaagagt agatctcggt ccaggtcggg 780
ctcccgctcc cggagcaaga gccggagccg gagccagagt cggagccgga gcaagaaaga 840
gaaaagcagg agccccagca aggaaaagag ccgcagccgc agccatagcg ctggcaagag 900
ccgcagcaag agcaaagacc aagctgaaga gaagatccaa aacaatgaca atgtcgggaa 960
acccaagagc cggagtccta gcaggcataa aagtaagagc aaaagtcgga gcaggagtca 1020
ggagaggaga gtggaggagg agaagcgagg gagtgtgagc aggggcagga gccaggagaa 1080
gagcctccgc cagagtcgga gccggagcag gagcaaaggg ggcagcagga gccggagcag 1140
gagccgcagc aagagcaagg acaagaggaa gggcaggaag agaagcagag aggagagccg 1200
cagtcgcagt cgcagccgca gcaagagtga gaggagcaga aagcgaggca gcaagcgaga 1260
cagcaaggcg ggcagcagca agaagaagaa gaaggaagac actgaccgct cccagtccag 1320
atctccatcc cgctccgtgt caaaggagcg ggaacatgcc aagtctgaat ccagccagag 1380
ggaaggtcga ggagagagtg agaatgctgg caccaatcag gagacccggt ccaggtcgag 1440
atccaattcc aaatcgaaac caaaccttcc atcagaatca cgctccagat caaagtcagc 1500
ttcaaaaacc cgatctcggt ccaagtctag atccaggtct gcttccagat cgccctcccg 1560
atctagatct aggtcccact caaggtccta actggctatg gccacagctg gaactacccg 1620
agaagtcttt tgtacatgtt tggtagccgt agcacaagtg attggagtag aacatgtcac 1680

tgctgtacat ttttaactcc cctaatggtg tgtctataat tgttaaatct aagtgcttcc 1740
tctcagtaaa gcctcctggc accaggcctt cctgctcgac tgaaaaaaat tttctctttg 1800
aaaatcccct tttactcatg gcccacagta gaatatccaa aacgccttgg ctttcaggcc 1860
tggcctttcc tacagggagc tcagtaacct ggacggctct aaggctggaa tgaccacata 1920
ggtaggtatg gtgagttcaa ccatttttgc tcttgaattg atgcccttcg atgtatgcca 1980
tttagtgaaa gtgctaagtc ttaagtttcc taccactttg gtttcatatt tttggactta 2040
acaaagttgt gaatagcaca gtcgaggaaa attgatacct gcagtaaccc ataggaaata 2100
aactgtagag ttccatattc tggtattgtg attatattgt tttatattaa aaaaaaaaaa 2160
aaaaaaa 2167

<210> 21
<211> 5801
<212> DNA
<213> Homo sapiens
<220>
<221> misc_feature
<223> séquence appelée GS-N20

<300>
<308> GENBANK/U65090
<309> 1997-10-02
<313> (1)..(5801)
<400> 21
gaattcgcgg ccgcgtcgac cggcgctgct ggaagatggc gagcggccgg gacgagcggc 60
cgcattgcgt agggcggctc ctgttgctca tgtgcctgct gctgctgggg agctcggccc 120
gggcggctca catcaagaag gcggaggcga ctaccacaac tacgagcgcg ggcgcgaggg 180
gccgagggca gttcgaccgc tactaccacg aagaggagtt ggagtcggcg ctgagggagg 240
cggcggccgc gggcctcccc ggcctggccc gcctctttag catcggccgc tcggtggaag 300
gccggccgct gtgggtgctt cgcctcaccg ccggcctggg gtcgctaatc cctgagggcg 360
acgcggggcc tgacgctgcc gggcccgacg ctgcggggcc gctgctgccc ggccggcccc 420
aggtgaagct ggtgggcaac atgcatggcg acgagaccgt gtcgcgccag gtgttgatct 480
acttggcccg cgagctggcg gcgctaccgc ccggggaccc gcgcctggtc cgcctgctca 540
acaccaccga cgtgtacctg ctgcccagcc tcaaccccga tggcttcgag cgtgcccgcg 600
agggcgactg tggcttcggc gacggcggcc cgtccggggc cagcggccgc gacaatagtc 660
gcggccgcga cctcaaccga agctttcccg accagtttag caccggcgaa ccccccgccc 720
tggacgaggt gcccgaggtg cgcgccctca tcgagtggat ccgcaggaac aagtttgtgc 780
tttctggaaa tctgcatggt ggctcagtgg tagcaagcta tccttttgat gattctccag 840
aacataaggc cactggaatc tatagcaaaa cctcagatga tgaagtattt aaatacttgg 900
caaaagctta tgcttcaaac caccccataa tgaaaactgg tgagcctcat tgtccaggag 960
atgaagacga gactttcaaa gatggaatca caaacggcgc acattggtat gatgtggaag 1020
gtggtatgca agattacaat tatgtgtggg ccaactgttt tgagatcaca ttagaactgt 1080
cttgttgcaa gtacccacct gcttcacagc ttcgacagga atgggagaac aatcgtgagt 1140
ctttgatcac attgattgaa aaggttcaca ttggagtgaa aggatttgtt aaagattcca 1200
taacaggatc tgggttagag aatgcaacca tctcagtggc tggtattaat cataatatca 1260
caacaggcag atttggtgat ttctaccgat tacttgttcc tggaacttac aaccttacag 1320
tagttttaac tgggtatatg ccattgactg ttactaatgt agtggtgaaa gaaggaccag 1380
ccacagaggt ggatttttct cttaggccaa ctgtaacttc agtaatccct gacacgacag 1440
aggctgtatc aactgctagc acagttgcta tacctaatat tctttctgga acatcatcct 1500
cctgccagcc aattcagcca aaggactttc accaccacca tttccctgat atggaaatct 1560
tcttgagaag gtttgccaat gaatatccta acattacccg gctttattcc ttgggaaaat 1620
cagtagagtc aagagaactt tatgtgatgg agatatctga taatccgggt gtccatgaac 1680
caggtgaacc agaatttaag tacattggaa atatgcatgg aaatgaagtg gttggaagag 1740
aactgctgtt gaacctcata gaataccttt gtaagaactt tggaacagac cctgaagtca 1800
cagatttggt tcataacact agaattcacc ttatgccatc catgaatcct gatgggtatg 1860
aaaagtccca ggaaggagat tcaataagtg taattggcag aaacaacagc aacaactttg 1920
acctgaaccg aaatttccca gaccagtttg ttcagatcac agatcctacg caaccagaaa 1980
ctattgctgt aatgagctgg atgaagtcct atccatttgt actttcagca aacctgcatg 2040
gaggttcttt ggtggttaac tacccttttg atgatgatga acaaggactt gccacatata 2100
gtaaatcacc agatgatgct gtgttccaac aaatagcact ttcttattcc aaggaaaatt 2160
cccagatgtt tcaaggtaga ccttgcaaga atatgtatcc taatgaatat tttcctcatg 2220
gaataacaaa tggagctagt tggtataatg tgccaggagg aatgcaggac tggaactatt 2280
tacaaacaaa ttgctttgaa gtgactattg aactaggttg tgtgaaatat ccacttgaga 2340
aagagctgcc aaacttttgg gaacagaatc gaagatcact aatccagttt atgaaacagg 2400
ttcatcaggg cgtcagagga tttgttctag atgccacaga tggcaggggt atattaaatg 2460
ccaccattag tgttgctgag attaatcacc cagtgactac ttacaaaact ggagattact 2520
ggcgtctctt ggttccagga acttataaaa tcacagcatc tgctcgaggg tataatccag 2580
ttaccaagaa tgtgactgtc aagagtgaag gcgctattca ggtcaacttc acacttgttc 2640
gatcctcaac agattcaaac aatgaatcaa agaaaggaaa aggggctagc agcagcacca 2700
atgatgccag tgatccaact actaaagagt ttgaaacttt aattaaagac ctttcagcgg 2760
agaatggttt ggaaagcctc atgttacgct cctcctcaaa tctggctctg gctctttatc 2820
gataccattc ctacaaagac ttatcagagt ttctgagagg acttgtaatg aactatccac 2880
atattacaaa tcttaccaat ttgggacaga gcactgaata tcgtcacatt tggtcccttg 2940
aaatctccaa taagcccaat gtatctgagc ctgaagaacc aaagattcgt tttgttgctg 3000

gtatccatgg aaatgcgcca gttggaactg aactgctttt ggctctggca gaatttctct 3060
gcctgaacta caaaaagaac ccagctgtta cccaattggt tgacaggact aggattgtga 3120
ttgtcccttc tctaaatcca gatgggcgag agagagctca agagaaagac tgtacttcaa 3180
aaataggaca aacaaatgct cgtggcaaag atttggatac agacttcaca aataatgcct 3240
cccaacctga gaccaaagcc atcattgaaa atttgattca aaaacagaac tttagtcttt 3300
ctgttgcctt agatggtggt tccatgctgg tcacatatcc atatgacaag ccagtacaga 3360
cagtggaaaa taaagagact ctgaagcatt tggcatctct ttatgcaaat aatcatccat 3420
ccatgcacat gggtcagccc agttgcccaa ataaatcaga tgagaatatt ccaggaggag 3480
taatgcgtgg agcagaatgg catagtcacc tgggcagcat gaaggattat agtgtcacct 3540
atggccattg tccggaaatc acagtataca caagctgctg ttactttcct agtgctgcac 3600
gactcccttc cttgtgggca gacaataaga gatctcttct tagtatgtta gtggaggttc 3660
acaagggagt tcatggattt gttaaagata agactggaaa gccaatctct aaagcagtca 3720
ttgtacttaa tgaaggaata aaggtacaaa caaaagaggg aggttatttc catgtactct 3780
tagcgccagg tgtccataac attattgcca tcgctgatgg gtaccagcaa caacattcac 3840
aggtctttgt gcatcatgat gcagctagtt ctgtggtgat agtctttgac acagataacc 3900
ggatatttgg tttgccaagg gagcttgtgg taactgtatc aggtgctact atgtcggcat 3960
tgatcctaac agcttgcatt atttggtgca tctgctcaat caagtctaat agacacaagg 4020
atggctttca tcggctcagg cagcatcatg atgagtatga agatgaaatt cgcatgatgt 4080
ctaccggctc caagaagtcc ctcctaagcc atgagttcca ggatgaaaca gacactgaag 4140
aggaaacatt atattctagc aaacattgaa aaacacattt tgcatatctc ccagcataag 4200
taccaagcaa aattacagtt cctcttggga gaacactgca ttaagaagag agactctctt 4260
gcttcttcaa agagctttgg gaaattaaat tgctaaattt gtattctctg tgaatttcac 4320
tggcagtttt gaacttccct tccttaaagt actctaaacc tttaaaaaaa aatctgattt 4380
atgcagcaga gatgggacag ccactttttc tttttaattt aagatgagct atttggagct 4440
tatgtaataa tggcataaag ccaactagag gatgttgtat tttgcacatc agatgtttac 4500
tagtggcttt agtatttttc tttgttttaa atggccaaaa gaatccagaa acattaaggc 4560
agggacagca gtcagaatcg acataaagct ttaaaaactc aaggtttttt caacctactg 4620
aggagtactt ttctctagtt gttaaatagc tggagttttt cttattcagg tttaatggag 4680
gttgaattga tttttaaaca catataacag taggaaatga ataaatgggc ttctgcattt 4740
ggctttctac ctgttccaag gctagatcgg aactggtaga ctacgctgta agcaggattt 4800
cactacctct cttaaggttt agcaaacttc taaatagccc attttaaggg agaacttact 4860
aactttattg tgaaaggtct aaatgcccac ttgaatgaag ctgagagaga gatctagcaa 4920
aagctaaaac tcatgttgtc tatctttgaa cttggtaaaa acccacaggt gctgctgctt 4980
atatctgtga agcactagct tattctagga atgcctgatt ctttaatatt gcctaaatcg 5040
gaaccttttt ctatgttgca cacatggttt tcagatgacc cagccatcta caagatctga 5100
attctactga aaatatctag aaatgtggaa gagacctact tgcacattct taacctgtat 5160
ttgaacacaa aatatctata cttcatgctc cagcccaagc ctataccctg taatagcata 5220
ctattattga aatcgcttga ccggtcttgt tcacataggc ctctgggagt gatttggttc 5280
tttgccctaa tgtttcattt gacggtctct ttttgatcaa ccaatttttc taaaagttca 5340
gtcgaaagct tttaagtata gcttcctccc ttgaaaaaaa atgtaaacta tgactgctga 5400
gtgataaaac actgtggtgt gaaagtgtca tcttcactgc caatcaggca aagaccggaa 5460
agatttgcat tttattatgt ctgtcttatc atgcaatgga aatgatgctt tttgtaagta 5520
tgcatcttac caatgatgta acggtttaat acctttgaat gttttaataa ccaagttgct 5580
gctgaactta tactaaatca ggggaccaaa aaacttgctc ttatcttctc aaattgtatt 5640
ctatatccat taatgtatca gttatcccaa agccttcagg tggaggggtt taccaccttc 5700
ctaggtcgtt caaccaggtt ttgtgaggaa tgcattcaaa gtggctttat aaaagaagat 5760
tttctttagc aagaaaaaaa aaaaaaaaaa aaaaaaaaaa a 5801

<210> 22
<211> 8171
<212> DNA
<213> Homo sapiens
<220>
<221> misc_feature
<223> séquence appelée GS-N21

<300>
<308> GENBANK/NM_004652
<309> 2001-02-03
<400> 22
cttttctcaa gacaactaca taagcagaca aaattgcaaa gatctgccct gtgtcgagta 60
tgacagccac gactcgtggc tctccggtcg gagggaatga caaccagggc caggctcctg 120
atggacagtc tctgcccccc ctccaacaga atcagacttc atcgcctgat tcttccaatg 180
aaaattcccc ggcaactccc ccagatgagc aaggtcaagg tgatgcccca ccacagcttg 240
aagatgagga acctgcattt ccacatactg acttggccaa gttggatgac atgatcaaca 300
ggcctcgatg ggtggttcca gttttgccga aaggggaatt agaagtgctt ttagaagctg 360
ctattgatct tagtaaaaag ggccttgatg ttaaaagtga agcatgtcag cgatttttcc 420
gtgatgggct aacaatatca ttcactaaaa ttcttacaga tgaagcagtg agtggctgga 480
agtttgaaat tcataggtgt ctggtggagc tatgtgtggc caagttgtcc caagactggt 540
ttccactttt agaacttctt gccatggcct taaatcctca ttgcaaattc catatctaca 600
atggtacacg tccatgtgaa tcagtttcct caagtgttca gttgcctgaa gatgaactct 660
ttgctcgttc tccagatcct cgatcaccaa agggttggct agtggatctt ctcaacaaat 720
ttggcacttt aaatgggttc cagattttgc atgatcgttt tattaatgga tcagcattaa 780

acgttcaaat aattgcagcc cttattaaac catttgggca atgctatgag tttctcactc 840
ttcatacagt gaaaaagtac tttcttccaa taatagaaat ggttccacag tttttagaaa 900
acttaactga tgaagaactg aaaaaagaag caaagaatga agccaaaaat gatgctcttt 960
caatgattat taaatctttg aagaatttag cttcaagggt tccaggacaa gaagaaactg 1020
ttaaaaactt agaaatattt aggttaaaaa tgatacttag attattgcaa atttcttctt 1080
tcaatggaaa gatgaatgca ctgaatgaag ttaataaggt gatatctagt gtatcatact 1140
atactcatcg acatggtaat cctgaggagg aagagtggct cacagctgaa cgaatggcag 1200
aatggataca gcagaacaat atcttatcca tagtgttgcg agatagtctt catcagccac 1260
agtatgtaga aaagttagag aagattcttc gttttgtcat caaagaaaaa gctctgacct 1320
tacaggatct tgataatatc tgggcagcac aggcagggaa acatgaagcc attgtgaaga 1380
atgtacatga tctcctggca aaattggcat gggatttttc tcctgaacaa cttgatcatc 1440
cttttgattg ttttaaggcc agtcggacaa atgcgagtaa aaagcaacgt gaaaagctac 1500
ttgagctgat acgtcgtctt gcagaagatg ataaagatgg tgtgatggca cacagagtgt 1560
tgaaccttct gtggaatctg gctcacagtg atgatgtgcc tgtagatatc atggacctgg 1620
ctctcagtgc ccacataaaa atactagatt acagttgctc ccaggaccgt gatacacaaa 1680
agatccaatg gatagatcgc tttatagaag aacttcgcac aaatgacaaa tgggttattc 1740
ccgcactgaa acaaattaga gaaatttgta gtttgtttgg tgaagcgcct caaaatttga 1800
gtcaaactca gcgaagtccc catgtgtttt atcgccatga cttaatcaat caacttcaac 1860
acaatcatgc cctagttact ttggtagcag aaaaccttgt aacttacatg gaaagcatga 1920
gactatatgc tagagaccat gaagattatg acccacaaac tgtgaggctg ggaagtagat 1980
atagtcatgt tcaagaagtt caagaacggc ttaacttcct tagattttta ttgaaggatg 2040
gtcagctgtg gctatgtgct cctcaggcaa aacaaatatg gaaatgctta gctgagaatg 2100
cagtttacct ttgtgatcgt gaagcctgtt ttaagtggta ttccaagttg atgggggatg 2160
aaccagactt agatcctgat attaataagg acttctttga aagtaatgtg cttcagcttg 2220
atccttctct gttaactgaa aatggaatga agtgttttga gcgattcttc aaagctgtga 2280
attgtcgaga aggaaaacta gtagcaaaaa ggagagccta tatgatggat gacttggagt 2340
taataggatt agattacctt tggagggtcg tgattcagag taatgatgat attgccagca 2400
gagctataga tctcctcaaa gagatataca cgaaccttgg tccaagacta caagtcaatc 2460
aggtggtgat ccatgaagac ttcattcagt cttgttttga tcgtctgaag gcttcctatg 2520
acacattgtg tgttttggat ggtgacaaag acagtgttaa ttgtgcaaga caggaagctg 2580
ttcgaatggt tcgagtatta actgttttaa gggaatatat aaatgaatgt gacagtgatt 2640
atcatgagga aagaacaatt ctccctatgt cgagagcatt ccgcggtaaa cacctctctt 2700
ttgtagttcg atttccaaac cagggcagac aggttgatga cttggaggta tggtctcata 2760
caaatgatac aattggttca gtacgacgat gtattctcaa tcgtattaaa gccaacgtag 2820
cccatacaaa aattgagctc tttgtgggcg gtgagctgat agatcctgca gatgatagaa 2880
agttgattgg acaattaaac ttaaaagata aatcgcttat tacagccaaa cttacacaga 2940
taagttccaa tatgccttca agccctgata gctcttctga ttcctccact ggatctcctg 3000
gaaaccatgg taatcattac agtgatggtc ccaatccaga agtggaaagc tgtttgcctg 3060
gagtgataat gtcactgcat cccagataca tctcttttct ttggcaagtt gcagacttag 3120
gtagcagcct aaatatgcca ccccttagag atggagcaag agtacttatg aaacttatgc 3180
cgccagatag cacaacgata gaaaaattaa gagctatttg tttagaccat gccaaacttg 3240
gagaaagcag ccttagtcca tctcttgact cacttttctt tggtccttca gcctcacaag 3300
tgctatatct aacagaggta gtctatgcct tgttaatgcc tgctggtgca cctctggctg 3360
atgattcctc tgattttcag tttcacttct tgaaaagtgg tggcctaccc cttgtactga 3420
gtatgctaac cagaaataac ttcctaccga atgcagatat ggaaactcga aggggtgcct 3480
acctcaatgc tcttaaaata gccaagcttt tgctaactgc cattggctat ggtcatgttc 3540
gagctgtggc agaagcttgt cagccaggtg tagaaggtgt gaatcccatg acacagatca 3600
accaagttac ccatgatcaa gcagtggtgc tacaaagtgc ccttcagagc attcctaatc 3660
catcatccga gtgcatgctt agaaatgtgt cagttcgtct tgctcagcag atatctgatg 3720
aggcttcaag atatatgcct gatatttgtg taattagagc tatacaaaaa attatctggg 3780
catcaggatg tgggtcgtta cagctagtat ttagcccaaa tgaagaaatc actaaaattt 3840
atgagaagac caatgcaggc aatgagccag acttggaaga cgaacaggtt tgctgtgaag 3900
cattggaagt gatgacctta tgttttgcct tgattccaac agccttagat gctcttagta 3960
aagaaaaggc ttggcagaca ttcatcattg acttactatt gcactgtcac agcaaaactg 4020
ttcgtcaggt ggcacaggag cagttctttt taatgtgcac cagatgttgc atgggacacc 4080
ggcctctact tttcttcatt actctactct ttactgtttt ggggagcaca gcaagagaga 4140
gagctaaaca ctcaggcgac tactttactc ttttaagaca ccttcttaat tacgcttaca 4200
atagtaatat taatgtaccc aatgctgaag ttcttttcaa taatgaaatt gattggctta 4260
aaagaattag ggatgatgtt aaaagaacag gagaaacggg tattgaagag acgatcttag 4320
agggccacct tggagtgaca aaggagttac tggcctttca aacttctgag aaaaaatttc 4380
atattggttg tgaaaaagga ggtgctaatc tcattaaaga attaattgat gatttcatat 4440
ttcctgcatc caatgtttac ctacagtata tgagaaatgg agagcttcca gctgaacagg 4500
ctattccggt ctgtggttca ccacctacaa ttaatgctgg ttttgaatta cttgtagcat 4560
tagctgttgg ctgtgtgagg aatctcaaac aaatagtaga ttctttgact gaaatgtatt 4620
acattggcac agcaataact acttgtgaag cacttactga gtgggaatat ctgccacctg 4680
ttggaccccg cccacccaaa ggattcgtgg ggctgaaaaa tgccggtgct acttgttaca 4740
tgaattctgt gattcagcaa ctctacatga ttccttccat taggaacggt attcttgcca 4800
ttgaaggcac aggtagtgat gtagatgatg atatgtctgg ggatgagaag caggacaatg 4860
agagcaatgt tgatcccagg gatgatgtat ttggatatcc tcaacaattt gaagataaac 4920
cagcattaag taaaactgaa gatagaaaag agtacaacat tggtgtccta agacaccttc 4980
aggtcatctt tggtcattta gctgcttctc gactgcaata ctatgtgccc agaggatttt 5040
ggaaacagtt caggctttgg ggtgagcctg ttaatctgcg tgaacaacac gatgctttag 5100
aattttttaa ttcattggtg gatagtttag atgaagcttt aaaagcttta ggacatccag 5160

ctatgctaag taaagtctta ggaggttcct ttgctgatca gaagatctgc caaggctgcc 5220
cacataggta cgaatgtgaa gaatctttta cgaccctaaa cgtagacatt agaaatcacc 5280
aaaatcttct tgattctttg gaacagtatg tcaaaggaga tttactagaa ggtgcaaatg 5340
catatcattg tgaaaaatgc aataaaaagg ttgataccgt aaagcgcttg ctgattaaaa 5400
aattacctcc tgttcttgct atacaactaa agcgatttga ctatgactgg gaaagagaat 5460
gtgcaatcaa gttcaatgat tattttgaat ttcctcgaga gctggacatg gaaccttaca 5520
cagttgcagg tgtcgcaaag ctggaagggg ataatgtaaa cccagagagt cagttgatac 5580
aacagagtga gcagtctgaa agtgagacag caggaagcac aaaatacaga cttgtgggtg 5640
tgctcgtaca cagtggtcaa gcgagtgggg ggcattatta ttcttacatc atccaaagga 5700
atggtggaga tggtgagaga aatcgctggt ataaatttga tgatggtgat gtaacagaat 5760
gtaaaatgga tgatgacgaa gaaatgaaaa accagtgttt tggtggagag tacatgggag 5820
aagtgtttga tcacatgatg aagcgtatgt catacaggcg ccagaaaagg tggtggaatg 5880
cttatatacc tttttatgaa cgaatggaca caatagacca agatgatgag ttgataagat 5940
atatatcaga gcttgctatc accaccagac ctcatcagat tattatgcca tcagccattg 6000
agagaagtgt acggaaacag aacgtacaat tcatgcataa ccgaatgcag tacagtatgg 6060
agtattttca gtttatgaaa aaactgctta catgtaatgg cgtttactta aaccctcctc 6120
ccgggcaaga tcacctgttg cctgaagcag aagaaatcac tatgatcagt attcaacttg 6180
ctgctaggtt cctctttact acaggatttc acacaaagaa agtagtccgt ggctctgcca 6240
gtgattggta tgatgcattg tgtattctcc ttcgtcacag caagaatgta cgtttttggt 6300
ttgctcataa cgtccttttt aatgtttcaa atcgcttctc cgaatacctt ctggagtgcc 6360
ctagtgcaga agtgaggggt gcgtttgcaa aacttatagt ctttattgca catttttcct 6420
tgcaagatgg gccatgtcct tcaccttttg cctctcctgg accttctagt caggcttatg 6480
acaacttaag cttgagtgat cacttactaa gagcagtact aaatctcttg agaagggaag 6540
tttcagagca tgggcgtcat ttacagcagt atttcaacct gtttgtaatg tatgccaatt 6600
taggtgtggc agagaagaca cagcttctga aattgagtgt acctgctact tttatgcttg 6660
tgtctttaga tgaaggtcca ggtcctccaa tcaaatacca gtatgctgaa ttaggcaaat 6720
tatactcagt agtgtcacag ctgatccgct gttgcaatgt ctcttcaaga atgcagtctt 6780
caatcaatgg taatcctcct cttcccaatc cttttggtga tcctaattta tcacaaccta 6840
taatgccaat tcagcagaat gtggcagaca ttttatttgt gagaacaagt tatgtgaaga 6900
aaatcattga agactgcagt aattcagagg aaaccgtcaa attgcttcgt ttttgctgct 6960
gggagaatcc tcagttctca tctactgtcc tcagtgaact tctctggcag gttgcatatt 7020
cctatcccta tgaactgcgg ccctatttgg atctgctttt gcaaatctta ctgattgagg 7080
actcctggca aactcacaga attcataatg cactgaaagg aattccagat gaccgagatg 7140
ggctgtttga cacaatccag cgctctaaga atcactatca aaaaagagca taccagtgta 7200
taaaatgtat ggtagctcta tttagtaact gtcctgttgc ttaccaaatc ctgcagggca 7260
atggagatct taaaagaaag tggacctggg cagtggaatg gcttggagat gaacttgaaa 7320
gaagaccata tactggcaat cctcagtaca cttacaacaa ttggtctccc ccagtgcaaa 7380
gcaatgaaac gtccaatggt tatttcttgg agagatcaca tagtgctagg atgacacttg 7440
caaaagcttg tgaactctgt ccagaggagg taaaaaaagc caccagtgtg cagcagatag 7500
aaatggaaga gagcaaagag ccagatgacc aagatgctcc agatgaacat gagtcgcctc 7560
cacctgaaga tgccccattg tacccccatt cacctggatc tcagtatcaa cagaataacc 7620
atgtgcatgg acagccatat acaggcccag cagcacatca catgaacaac cctcagagaa 7680
ctggccaacg agcacaagaa aattatgaag gcagtgaaga agtatcccca cctcaaacca 7740
aggatcaatg aaatgcacat aattaactgg ttccatcaag actgtgcacc caggccttac 7800
agtccaacct ttttctgtgt ctggctaata tttaaaacta gaaaaactat tcctaatcaa 7860
catggagtgg agagtttatt cactgtctta tctgcagaaa tttgctgtca atatataacc 7920
cgcctgcagt ggaaagtgta tagtgttttg taataaatgg cctgatgcta atgtgtaaat 7980
ggcaaaggtg tatatagtat attaatgttg actgttaatt cttaagcaag aaactttttt 8040
cttgatgaga ctcacagatc tacacaaact acaaaagtta attttcttgt tacacccact 8100
gcactctgca accagtgttg cctgcctcat ggcagttgga tcagctcctt tacaaaaaag 8160
aaaaaaaaaa a 8171

<210> 23
<211> 3851
<212> DNA
<213> Homo sapiens
<220>
<221> misc_feature
<223> séquence appelée GS-N22

<300>
<308> GENBANK/NM_002525
<309> 2000-10-31
<313> (1)..(3851)
<400> 23
agactggggt gggggagggg ttcaggcctg ttccccgcgg ctgcggcagc accagggccg 60
gccgccaccg cctctagaac gcggaggagg tgggtcctgg gaagcgggat gtccatcgct 120
ccagcttggt ggtgaatgct gaggagagtc actgttgctg cagtctgtgc cacccggagg 180
aagttgtgtg aggccgggcg ggacgtcgcg gcgctctggg gaatcgaaac gcggggtcgg 240
tgcgaagact ctgctgctgc cagacccttt cctattctgg ccatgcctgg aaggaacaag 300
gcgaagtcta cctgcagctg ccctgacctg cagcccaatg gacaggatct gggcgagaac 360
agccgggttg cccgtctagg agcggatgaa tctgaggaag agggacggag ggggtctctc 420
agtaatgctg gggaccctga gatcgtcaag tctcccagcg accccaagca ataccgatac 480

atcaaattac agaatggcct acaggcactt ctgatttcag acctaagtaa tatggaaggt 540
aaaacaggaa atacaacaga tgatgaagaa gaagaggagg tggaggaaga agaagaagat 600
gatgatgaag attctggagc tgaaatagaa gatgacgatg aagagggttt tgatgatgaa 660
gatgagtttg atgatgaaca tgatgatgat cttgatactg aggataatga attggaagaa 720
ttagaagaga gagcagaagc tagaaaaaaa actactgaaa aacagcaatt gcagagcctg 780
tttttgctgt ggtcaaagct gactgataga ctgtggttta agtcaactta ttcaaaaatg 840
tcttcaaccc tgctggtcga gacaagaaat ctttatgggg tagttggagc tgaaagcagg 900
tctgcacctg ttcagcattt ggcaggatgg caagcggagg agcagcaggg tgaaactgac 960
acagttctgt ctgcagcggc tctttgtgtt ggagttggga gtttcgctga tccagatgac 1020
ctgccggggc tggcacactt tttggagcac atggtattca tgggtagttt gaaatatcca 1080
gatgagaatg gatttgatgc cttcctgaag aagcatgggg gtagtgataa tgcctcaact 1140
gattgtgaac gcactgtctt tcagtttgat gtccagagga agtacttcaa ggaagctctt 1200
gatagatggg cgcagttctt catccaccca ctaatgatca gagatgcaat tgaccgtgaa 1260
gttgaagctg ttgatagtga atatcaactt gcaaggcctt ctgatgcaaa cagaaaggaa 1320
atgttgtttg gaagccttgc tagacctggc catcctatgg gaaaattttt ttggggaaat 1380
gctgagacgc tcaagcatga gccaagaaag aataatattg atacacatgc tagattgaga 1440
gaattctgga tgcgttacta ctcttctcat tacatgactt tagtggttca atccaaagaa 1500
acactggata ctttggaaaa gtgggtgact gaaatcttct ctcagatacc aaacaatggg 1560
ttacccagac caaactttgg ccatttaacg gatccatttg acacaccagc atttaacaaa 1620
ctttatagag ttgttccaat cagaaaaatt catgctctga ccatcacatg ggcacttcct 1680
cctcaacagc aacattacag ggtgaagcca cttcattata tatcctggct ggttggacat 1740
gaaggcaaag gcagcattct ttctttcctt aggaaaaaat gctgggctct tgcactgttt 1800
ggtggaaatg gtgagacagg atttgagcaa aattctactt attcagtgtt cagcatttct 1860
attacattga ctgatgaggg ttatgaacat ttttatgagg ttgcttacac tgtctttctg 1920
tatttaaaaa tgctgcagaa gctaggccca gaaaaaagaa tttttgaaga gattcggaaa 1980
attgaggata atgaatttca ttaccaagaa cagacagatc cagttgagta tgtggaaaac 2040
atgtgtgaga acatgcagct gtacccattg caggacattc tcactggaga tcagcttctt 2100
tttgaataca agccagaagt cattggtgaa gccttgaatc agctagttcc tcaaaaagca 2160
aatcttgttt tactgtctgg tgctaatgag ggaaaatgtg acctcaagga gaaatggttt 2220
ggaactcaat atagtataga agatattgaa aactcttggg ctgaactgtg gaatagtaat 2280
ttcgaattaa atccagatct tcatcttcca gctgaaaaca agtacatagc cacggacttt 2340
acgttgaagg ctttcgattg cccggaaaca gaatacccag ttaaaattgt gaatactcca 2400
caaggttgcc tgtggtataa gaaagacaac aaattcaaaa tccccaaagc atatatacgt 2460
ttccatctaa tttcaccgtt gatacagaaa tctgcagcaa atgtggtcct ctttgatatc 2520
tttgtcaata tccttacgca taaccttgcg gaaccagctt atgaagcaga tgtggcacag 2580
ctggagtata aactggcagc tggagaacat ggtttaatta ttcgagtgaa aggatttaac 2640
cacaaactac ctctactgtt tcagctcatt attgactact tagctgagtt caattccaca 2700
ccagctgtct ttacaatgat aactgagcag ttgaagaaga cctactttaa catcctcatc 2760
aagcctgaga ctttggccaa agatgtacgg cttttaatct tggaatatgc ccgttggtct 2820
atgattgaca agtaccaggc tttgatggac ggcctttccc ttgagtctct gctgagcttc 2880
gtcaaagaat tcaaatccca gctctttgtg gagggcctgg tacaagggaa tgtcacaagc 2940
acagaatcta tggatttcct gaaatatgtt gttgacaaac taaacttcaa gcctctggag 3000
caggagatgc ctgtgcagtt ccaggtggta gagctgccca gtggccacca tctatgcaaa 3060
gtgaaagctc tgaacaaggg tgatgccaac tctgaagtca ctgtgtacta ccagtcaggt 3120
accaggagtc taagagaata tacgcttatg gagctgcttg tgatgcacat ggaagaacct 3180
tgttttgact tccttcgaac caagcagacc cttgggtacc atgtctaccc tacctgtagg 3240
aacacatccg ggattctagg attttctgtc actgtgggga ctcaggcaac caaatacaat 3300
tctgaagttg ttgataagaa gatagaagag tttctttcta gctttgagga gaagattgag 3360
aacctcactg aagaggcatt caacacccag gtcacagctc tcatcaagct gaaggagtgt 3420
gaggataccc accttgggga ggaggtggat aggaactgga atgaagtggt tacacagcag 3480
tacctctttg accgccttgc ccacgagatt gaagcactga agtcattctc aaaatcagac 3540
ctggtcaact ggttcaaggc tcatagaggg ccaggaagta aaatgctcag cgttcatgtt 3600
gttgggtatg ggaagtatga actggaagag gatggatccc cttctagtga ggattcaaat 3660
tcttcttgtg aagtgatgca gctgacctac ctgccaacct ctcctctgct ggcagattgt 3720
atcatcccca ttactgatat cagggctttc acaacaacac tcaaccttct cccctaccat 3780
aaaatagtca aataaataaa ctgcagtcac gttggcctga aaaaaaaaaa aaaaaaaaaa 3840
aaaaaaaaaa a 3851

<210> 24
<211> 13107
<212> DNA
<213> Homo sapiens
<220>
<221> misc_feature
<223> séquence appelée GS-N23

<300>
<308> GENBANK/XM_016303
<309> 2001-10-16
<313> (1)..(13107)
<400> 24
ccgaatagta gattcagtgc cccgtcctgt ggatcttctg aaaaatcaag tgcagcttct 60
cagcactcct ctcaaatgtc ttcagactcc tctcgatcta gtagccccag tgttgatacc 120

tccacagact ctcaggcttc tgaggagatt caggtacttc ctgaggagcg gagcgatacc 180
cctgaagttc atcctccact gcccatttcc cagtccccag aaaatgagag taatgatagg 240
agaagcagaa ggtattcagt gtcggagaga agttttggat ctagaacgac gaaaaaatta 300
tcaactctac aaagtgcccc ccagcagcag acctcctcgt ctccacctcc acctctgctg 360
actccaccgc caccactgca gccagcctcc agtatctctg accacacacc ttggcttatg 420
cctccaacaa tccccttagc atcaccattt ttgcctgctt ccactgctcc tatgcaaggg 480
aagcgaaaat ctattttgcg agaaccgaca tttaggtgga cttctttaaa gcattctagg 540
tcagagccac aatacttttc ctcagcaaag tatgccaaag aaggtcttat tcgcaaacca 600
atatttgata atttccgacc ccctccacta actcccgagg acgttggctt tgcatctggt 660
ttttctgcat ctggtaccgc tgcttcagcc cgattgtttt cgccactcca ttctggaaca 720
aggtttgata tgcacaaaag gagccctctt ctgagagctc caagatttac tccaagtgag 780
gctcactcta gaatatttga gtctgtaacc ttgcctagta atcgaacttc tgctggaaca 840
tcttcttcag gagtatccaa tagaaaaagg aaaagaaaag tgtttagtcc tattcgatct 900
gaaccaagat ctccttctca ctccatgagg acaagaagtg gaaggcttag tagttctgag 960
ctctcacctc tcaccccccc gtcttctgtc tcttcctcgt taagcatttc tgttagtcct 1020
cttgccacta gtgccttaaa cccaactttt acttttcctt ctcattccct gactcagtct 1080
ggggaatctg cagagaaaaa tcagagacca aggaagcaga ctagtgctcc ggcagagcca 1140
ttttcatcaa gtagtcctac tcctctcttc ccttggttta ccccaggctc tcagactgaa 1200
agagggagaa ataaagacaa ggcccccgag gagctgtcca aagatcgaga tgctgacaag 1260
agcgtggaga aggacaagag tagagagaga gaccgggaga gagaaaagga gaataagcgg 1320
gagtcaagga aagagaaaag gaaaaaggga tcagaaattc agagtagttc tgctttgtat 1380
cctgtgggta gggtttccaa agagaaggtt gttggtgaag atgttgccac ttcatcttct 1440
gccaaaaaag caacagggcg gaagaagtct tcatcacatg attctgggac tgatattact 1500
tctgtgactc ttggggatac aacagctgtc aaaaccaaaa tacttataaa gaaagggaga 1560
ggaaatctgg aaaaaaccaa cttggacctc ggcccaactg ccccatccct ggagaaggag 1620
aaaaccctct gcctttccac tccttcatct agcactgtta aacattccac ttcctccata 1680
ggctccatgt tggctcaggc agacaagctt ccaatgactg acaagagggt tgccagcctc 1740
ctaaaaaagg ccaaagctca gctctgcaag attgagaaga gtaagagtct taaacaaacc 1800
gaccagccca aagcacaggg tcaagaaagt gactcatcag agacctctgt gcgaggaccc 1860
ccggattaaa catgtctgca gaagagcagc tgttgccctt ggcccgaaaa cgagctgtgt 1920
ttcctgatga catgcccacc ctgagtgcct taccatggga agaacgagaa aagattttgt 1980
cttccatggg gaatgatgac aagtcatcaa ttgctggctc agaagatgct gaacctcttg 2040
ctccacccat caaaccaatt aaacctgtca ctagaaacaa ggcaccccag gaacctccag 2100
taaagaaagg acgtcgatcg aggcggtgtg ggcagtgtcc cggctgccag gtgcctgagg 2160
actgtggtgt ttgtactaat tgcttagata agcccaagtt tggtggtcgc aatataaaga 2220
agcagtgctg caagatgaga aaatgtcaga atctacaatg gatgccttcc aaagcctacc 2280
tgcagaagca agctaaagct gtgaaaaaga aagagaaaaa gtctaagacc agtgaaaaga 2340
aagacagcaa agagagcagt gttgtgaaga acgtggtgga ctctagtcag aaacctaccc 2400
catcagcaag agaggatcct gccccaaaga aaagcagtag tgagcctcct ccacgaaagc 2460
ccgtcgagga aaagagtgaa gaagggaatg tctcggcccc tgggcctgaa tccaaacagg 2520
ccaccactcc agcttccagg aagtcaagca agcaggtctc ccagccagca ctggtcatcc 2580
cgcctcagcc acctactaca ggaccgccaa gaaaagaagt tcccaaaacc actcctagtg 2640
agcccaagaa aaagcagcct ccaccaccag aatcaggtcc agagcagagc aaacagaaaa 2700
aagtggctcc ccgcccaagt atccctgtaa aacaaaaacc aaaagaaaag gaaaaaccac 2760
ctccggtcaa taagcaggag aatgcaggca ctttgaacat cctcagcact ctctccaatg 2820
gcaatagttc taagcaaaaa attccagcag atggagtcca caggatcaga gtggacttta 2880
aggaggattg tgaagcagaa aatgtgtggg agatgggagg cttaggaatc ttgacttctg 2940
ttcctataac acccagggtg gtttgctttc tctgtgccag tagtgggcat gtagagtttg 3000
tgtattgcca agtctgttgt gagcccttcc acaagttttg tttagaggag aacgagcgcc 3060
ctctggagga ccagctggaa aattggtgtt gtcgtcgttg caaattctgt cacgtttgtg 3120
gaaggcaaca tcaggctaca aagcagctgc tggagtgtaa taagtgccga aacagctatc 3180
accctgagtg cctgggacca aactacccca ccaaacccac aaagaagaag aaagtctgga 3240
tctgtaccaa gtgtgttcgc tgtaagagct gtggatccac aactccaggc aaagggtggg 3300
atgcacagtg gtctcatgat ttctcactgt gtcatgattg cgccaagctc tttgctaaag 3360
gaaacttctg ccctctctgt gacaaatgtt atgatgatga tgactatgag agtaagatga 3420
tgcaatgtgg aaagtgtgat cgctgggtcc attccaaatg tgagaatctt tcaggtacag 3480
aagatgagat gtatgagatt ctatctaatc tgccagaaag tgtggcctac acttgtgtga 3540
actgtactga gcggcaccct gcagagtggc gactggccct tgaaaaagag ctgcagattt 3600
ctctgaagca agttctgaca gctttgttga attctcggac taccagccat ttgctacgct 3660
accggcaggc tgccaagcct ccagacttaa atcccgagac agaggagagt ataccttccc 3720
gcagctcccc cgaaggacct gatccaccag ttcttactga ggtcagcaaa caggatgatc 3780
agcagccttt agatctagaa ggagtcaaga ggaagatgga ccaagggaat tacacatctg 3840
tgttggagtt cagtgatgat attgtgaaga tcattcaagc agccattaat tcagatggag 3900
gacagccaga aattaaaaaa gccaacagca tggtcaagtc cttcttcatt cggcaaatgg 3960
aacgtgtttt tccatggttc agtgtcaaaa agtccaggtt ttgggagcca aataaagtat 4020
caagcaacag tgggatgtta ccaaacgcag tgcttccacc ttcacttgac cataattatg 4080
ctcagtggca ggagcgagag gaaaacagcc acactgagca gcctccttta atgaagaaaa 4140
tcattccagc tcccaaaccc aaaggtcctg gagaaccaga ctcaccaact cctctgcatc 4200
ctcctacacc accaattttg agtactgata ggagtcgaga agacagtcca gagctgaacc 4260
cacccccagg catagaagac aatagacagt gtgcgttatg tttgacttat ggtgatgaca 4320
gtgctaatga tgctggtcgt ttactatata ttggccaaaa tgagtggaca catgtaaatt 4380
gtgctttgtg gtcagcggaa gtgtttgaag atgatgacgg atcactaaag aatgtgcata 4440
tggctgtgat caggggcaag cagctgagat gtgaattctg ccaaaagcca ggagccaccg 4500

tgggttgctg tctcacatcc tgcaccagca actatcactt catgtgttcc cgagccaaga 4560
actgtgtctt tctggatgat aaaaaagtat attgccaacg acatcgggat ttgatcaaag 4620
gcgaagtggt tcctgagaat ggatttgaag ttttcagaag agtgtttgtg gactttgaag 4680
gaatcagctt gagaaggaag tttctcaatg gcttggaacc agaaaatatc cacatgatga 4740
ttgggtctat gacaatcgac tgcttaggaa ttctaaatga tctctccgac tgtgaagata 4800
agctctttcc tattggatat cagtgttcca gggtatactg gagcaccaca gatgctcgca 4860
agcgctgtgt atatacatgc aagatagtgg agtgccgtcc tccagtcgta gagccggata 4920
tcaacagcac tgttgaacat gatgaaaaca ggaccattgc ccatagtcca acatctttta 4980
cagaaagttc atcaaaagag agtcaaaaca cagctgaaat tataagtcct ccatcaccag 5040
accgacctcc tcattcacaa acctctggct cctgttatta tcatgtcatc tcaaaggtcc 5100
ccaggattcg aacacccagt tattctccaa cacagagatc ccctggctgt cgaccgttgc 5160
cttctgcagg aagtcctacc ccaaccactc atgaaatagt cacagtaggt gatcctttac 5220
tctcctctgg acttcgaagc attggctcca ggcgtcacag tacctcttcc ttatcacccc 5280
agcggtccaa actccggata atgtctccaa tgagaactgg gaatacttac tctaggaata 5340
atgtttcctc agtctccacc accgggaccg ctactgatct tgaatcaagt gccaaagtag 5400
ttgatcatgt cttagggcca ctgaattcaa gtactagttt agggcaaaac acttccacct 5460
cttcaaattt gcaaaggaca gtggttactg taggcaataa aaacagtcac ttggatggat 5520
cttcatcttc agaaatgaag cagtccagtg cttcagactt ggtgtccaag agctcctctt 5580
taaagggaga gaagaccaaa gtgctgagtt ccaagagctc agagggatct gcacataatg 5640
tggcttaccc tggaattcct aaactggccc cacaggttca taacacaaca tctagagaac 5700
tgaatgttag taaaatcggc tcctttgctg aaccctcttc agtgtcgttt tcttctaaag 5760
aggccctctc cttcccacac ctccatttga gagggcaaag gaatgatcga gaccaacaca 5820
cagattctac ccaatcagca aactcctctc cagatgaaga tactgaagtc aaaaccttga 5880
agctatctgg aatgagcaac agatcatcca ttatcaacga acatatggga tctagttcca 5940
gagataggag acagaaaggg aaaaaatcct gtaaagaaac tttcaaagaa aagcattcca 6000
gtaaatcttt tttggaacct ggtcaggtga caactggtga ggaaggaaac ttgaagccag 6060
agtttatgga tgaggttttg actcctgagt atatgggcca acgaccatgt aacaatgttt 6120
cttctgataa gattggtgat aaaggccttt ctatgccagg agtccccaaa gctccaccca 6180
tgcaagtaga aggatctgcc aaggaattac aggcaccacg gaaacgcaca gtcaaagtga 6240
cactgacacc tctaaaaatg gaaaatgaga gtcaatccaa aaatgccctg aaagaaagta 6300
gtcctgcttc ccctttgcaa atagagtcaa catctcccac agaaccaatt tcagcctctg 6360
aaaatccagg agatggtcca gtggcccaac caagccccaa taatacctca tgccaggatt 6420
ctcaaagtaa caactatcag aatcttccag tacaggacag aaacctaatg cttccagatg 6480
gccccaaacc tcaggaggat ggctctttta aaaggaggta tccccgtcgc agtgcccgtg 6540
cacgttctaa catgtttttt gggcttaccc cactctatgg agtaagatcc tatggtgaag 6600
aagacattcc attctacagc agctcaactg ggaagaagcg aggcaagaga tcagctgaag 6660
gacaggtgga tggggccgat gacttaagca cttcagatga agacgactta tactattaca 6720
acttcactag aacagtgatt tcttcaggtg gagaggaacg actggcatcc cataatttat 6780
ttcgggagga ggaacagtgt gatcttccaa aaatctcaca gttggatggt gttgatgatg 6840
ggacagagag tgatactagt gtcacagcca caacaaggaa aagcagccag attccaaaaa 6900
gaaatggtaa agaaaatgga acagagaact taaagattga tagacctgaa gatgctgggg 6960
agaaagaaca tgtcactaag agttctgttg gccacaaaaa tgagccaaag atggataact 7020
gccattctgt aagcagagtt aaaacacagg gacaagattc cttggaagct cagctcagct 7080
cattggagtc aagccgcaga gtccacacaa gtaccccctc cgacaaaaat ttactggaca 7140
cctataatac tgagctcctg aaatcagatt cagacaataa caacagtgat gactgtggga 7200
atatcctgcc ttcagacatt atggactttg tactaaagaa tactccatcc atgcaggctt 7260
tgggtgagag cccagagtca tcttcatcag aactcctgaa tcttggtgaa ggattgggtc 7320
ttgacagtaa tcgtgaaaaa gacatgggtc tttttgaagt attttctcag cagctgccta 7380
caacagaacc tgtggatagt agtgtctctt cctctatctc agcagaggaa cagtttgagt 7440
tgcctctaga gctaccatct gatctgtctg tcttgaccac ccggagtccc actgtcccca 7500
gccagaatcc cagtagacta gctgttatct cagactcagg ggagaagaga gtaaccatca 7560
cagaaaaatc tgtagcctcc tctgaaagtg acccagcact gctgagccca ggagtagatc 7620
caactcctga aggccacatg actcctgatc attttatcca aggacacatg gatgcagacc 7680
acatctctag ccctccttgt ggttcagtag agcaaggtca tggcaacaat caggatttaa 7740
ctaggaacag tagcaccccc ggccttcagg tacctgtttc cccaactgtt cccatccaga 7800
accagaagta tgtgcccaat tctactgata gtcctggccc gtctcagatt tccaatgcag 7860
ctgtccagac cactccaccc cacctgaagc cagccactga gaaactcata gttgttaacc 7920
agaacatgca gccactttat gttctccaaa ctcttccaaa tggagtgacc caaaaaatcc 7980
aattgacctc ttctgttagt tctacaccca gtgtgatgga gacaaatact tcagtattgg 8040
gacccatggg aggtggtctc acccttacca caggactaaa tccaagcttg ccaacttctc 8100
aatctttgtt cccttctgct agcaaaggat tgctacccat gtctcatcac cagcacttac 8160
attccttccc tgcagctact caaagtagtt tcccaccaaa catcagcaat cctccttcag 8220
gcctgcttat tggggttcag cctcctccgg atccccaact tttggtttca gaatccagcc 8280
agaggacaga cctcagtacc acagtagcca ctccatcctc tggactcaag aaaagaccca 8340
tatctcgtct acagacccga aagaataaaa aacttgctcc ctctagtacc ccttcaaaca 8400
ttgccccttc tgatgtggtt tctaatatga cattgattaa cttcacaccc tcccagcttc 8460
ctaatcatcc aagtctgtta gatttggggt cacttaatac ttcatctcac cgaactgtcc 8520
ccaacatcat aaaaagatct aaatctagca tcatgtattt tgaaccggca cccctgttac 8580
cacagagtgt gggaggaact gctgccacag cggcaggcac atcaacaata agccaggata 8640
ctagccacct cacatcaggg tctgtgtctg gcttggcatc cagttcctct gtcttgaatg 8700
ttgtatccat gcaaactacc acaaccccta caagtagtgc gtcagttcca ggacacgtca 8760
ccttaaccaa cccaaggttg cttggtaccc cagatattgg ctcaataagc aatcttttaa 8820
tcaaagctag ccagcagagc ctggggattc aggaccagcc tgtggcttta ccgccaagtt 8880

caggaatgtt tccacaactg gggacatcac agaccccctc tactgctgca ataacagcgg 8940
catctagcat ctgtgtgctc ccctccactc agactacggg cataacagcc gcttcacctt 9000
ctggggaagc agacgaacac tatcagcttc agcatgtgaa ccagctcctt gccagcaaaa 9060
ctgggattca ttcttcccag cgtgatcttg attctgcttc agggccccag gtatccaact 9120
ttacccagac ggtagacgct cctaatagca tgggactgga gcagaacaag gctttatcct 9180
cagctgtgca agccagcccc acctctcctg ggggttctcc atcctctcca tcttctggac 9240
agcggtcagc aagcccttca gtgccgggtc ccactaaacc caaaccaaaa accaaacggt 9300
ttcagctgcc tctagacaaa gggaatggca agaagcacaa agtttcccat ttgcggacca 9360
gttcttctga agcacacatt ccagaccaag aaacgacatc cctgacctca ggcacaggga 9420
ctccaggagc agaggctgag cagcaggata cagctagcgt ggagcagtcc tcccagaagg 9480
agtgtgggca acctgcaggg caagtcgctg ttcttccgga agttcaggtg acccaaaatc 9540
cagcaaatga acaagaaagt gcagaaccta aaacagtgga agaagaggaa agtaatttca 9600
gctccccact gatgctttgg cttcagcaag aacaaaagcg gaaggaaagc attactgaga 9660
aaaaacccaa gaaaggactt gtttttgaaa tttccagtga tgatggcttt cagatctgtg 9720
cagaaagtat tgaagatgcc tggaagtcat tgacagataa agtccaggaa gctcgatcaa 9780
atgcccgcct aaagcagctc tcatttgcag gtgttaacgg tttgaggatg ctggggattc 9840
tccatgatgc agttgtgttc ctcattgagc agctgtctgg tgccaagcac tgtcgaaatt 9900
acaaattccg tttccacaag ccagaggagg ccaatgaacc ccccttgaac cctcacggct 9960
cagccagggc tgaagtccac ctcagggaag tcagcatttg acatgtttaa cttcctggct 10020
tctaaacatc gtcagcctcc tgaatacaac cccaatgatg aagaagagga ggaggtacag 10080
ctgaagtcag ctcggagggc aactagcatg gatctgccaa tgcccatgcg cttccggcac 10140
ttaaaaaaga cttctaagga ggcagttggt gtctacaggt ctcccatcca tggccggggt 10200
cttttctgta agagaaacat tgatgcaggt gagatggtga ttgagtatgc cggcaacgtc 10260
atccgctcca tccagactga caagcgggaa aagtattacg acagcaaggg cattggttgc 10320
tatatgttcc gaattgatga ctcagaggta gtggatgcca ccatgcatgg aaatgctgca 10380
cgcttcatca atcactcgtg tgagcctaac tgctattctc gggtcatcaa tattgatggg 10440
cagaagcaca ttgtcatctt tgccatgcgt aagatctacc gaggagagga actcacttac 10500
gactataagt tccccattga ggatgccagc aacaagctgc cctgcaactg tggcgccaag 10560
aaatgccgga agttcctaaa ctaaagctgc tcttctcccc cagtgttgga gtgcaaggag 10620
gcggggccat ccaaagcaac gctgaaggcc ttttccagca gctgggagct cccggattgc 10680
gtggcacagc tgaggggcct ctgtgatggc tgagctctct tatgtcctat actcacatca 10740
gacatgtgat catagtccca gagacagagt tgaggtctcg aagaaaagat ccatgatcgg 10800
ctttctcctg gggcccctcc aattgtttac tgttagaaag tgggaatggg gtccctagca 10860
gacttgcctg gaaggagcct attatagagg gttggttatg ttgggagatt gggcctgaat 10920
ttctccacag aaataagttg ccatcctcag gttggccctt tcccaagcac tgtaagtgag 10980
tgggtcaggc aaagccccaa atggagggtt ggttagattc ctgacagttt gccagccagg 11040
ccccacctac agcgtctgtc gaacaaacag aggtctggtg gttttcccta ctatcctccc 11100
actcgagagt tcacttctgg ttgggagaca ggattcctag cacctccggt gtcaaaaggc 11160
tgtcatgggg ttgtgccaat taattaccaa acattgagcc tgcaggcttt gagtgggagt 11220
gttgccccca ggagccttat ctcagccaat tacctttctt gacagtagga gcggcttccc 11280
tctcccattc cctcttcact cccttttctt cctttcccct gtcttcatgc cactgctttc 11340
ccatgcttct ttcgggttgt aggggagact gactgcctgc tcaaggacac tccctgctgg 11400
gcataggatg tgcctgcaaa aagttccctg agcctgtaag cactccaggt ggggaagtgg 11460
acaggagcca ttggtcataa ccagacagaa tttggaaaca ttttcataaa gctccatgga 11520
gagttttaaa gaaacatatg tagcatgatt ttgtaggaga ggaaaaagat tatttaaata 11580
ggatttaaat catgcaacaa cgagagtatc acagccagga tgacccttgg gtcccattcc 11640
taagacatgg ttactttatt ttccccttgt taagacatag gaagacttaa tttttaaacg 11700
gtcagtgtcc agttgaaggc agaacactaa tcagatttca aggcccacaa cttggggact 11760
agaccacctt atgttgaggg aactctgcca cctgcgtgca acccacagct aaagtaaatt 11820
caatgacact actgccctga ttactcctta ggatgtggtc aaaacagcat caaatgtttc 11880
ttctcttcct ttccccaaga cagagtcctg aacctgttaa attaagtcat tggattttac 11940
tctgttctgt ttacagttta ctatttaagg ttttataaat gtaaatatat tttgtatatt 12000
tttctatgag aagcacttca tagggagaag cacttatgac aaggctattt tttaaaccgc 12060
ggtattatcc taatttaaaa gaagatcggt ttttaataat tttttatttt cataggatga 12120
agttagagaa aatattcagc tgtacacaca aagtctggtt tttcctgccc aacttccccc 12180
tggaaggtgt actttttgtt gtttaatgtg tagcttgttt gtgccctgtt gacataaatg 12240
tttcctgggt ttgctctttg acaataaatg gagaaggaag gtcacccaac tccattgggc 12300
cactcccctc cttcccctat tgaagctcct caaaaggcta cagtaatatc ttgatacaac 12360
agattctctt ctttcccgcc tctctccttt ccggcgcaac ttccagagtg gtgggagacg 12420
gcaatcttta catttccctc atctttctta cttcagagtt agcaaacaac aagttgaatg 12480
gcaacttgac atttttgcat caccatctgc ctcataggcc actctttcct ttccctctgc 12540
ccaccaagtc tcatatctgc agagaaccca gtgatcacat tgtgccctct tttggggcag 12600
cctgttgaaa ctgaagcaca gtctgaccac tcacgataaa gcagattttt ctctgcctct 12660
gccacaaggt ttcagagtag tgtagtccaa gtagagggtg gggcaccctt ttctcgccgc 12720
aagaagccca ttcctatgga agtctagcaa agcaatacga ctcagcccag cactctctgc 12780
cccaggactc atggctctgc tgtgccttcc atcctgggct cccttctctc ctgtgacctt 12840
aagaactttg tctggtggct ttgctggaac attgtcactg ttttcactgt catgcaggga 12900
gcccagcact gtggccagga tggcagagac ttccttgtca tcatggagaa gtgccagcag 12960
ggactgggga aaagcactct acccagacct cacctccctt cctccttttg cccatgaaca 13020
agatgcagtg gccctagggg ttccactagt gtctgctttc ctttattatt gcactgtgtg 13080
aggttttttt gtaaatcctt gtattcc 13107

<210> 25

<211>  10330
<212>  DNA
<213>  Homo sapiens
<220>
<221>  misc_feature
<223>  séquence appelée GS-N24

<300>
<308>  GENBANK/U72937
<309>  2000-02-11
<313>  (1)..(10330)
<400>  25
ctcggcccaa caaaatggcg gcggcagcgg tgtcgctttg tttccgcggc tcctgcggcg      60
gtggcagtgg tagcggcctt tgagctgtgg ggaggttcca gcagcagcta cagtgacgac     120
taagactcca gtgcatttct atcgtaaccg ggcgcggggg agcgcagatc ggcgcccagc     180
aatcacagaa gccgacaagg cgttcaagcg aaaacatgac cgctgagccc atgagtgaaa     240
gcaagttgaa tacattggtg cagaagcttc atgacttcct tgcacactca tcagaagaat     300
ctgaagaaac aagttctcct ccacgacttg caatgaatca aaacacagat aaaatcagtg     360
gttctggaag taactctgat atgatggaaa acagcaagga agagggaact agctcttcag     420
aaaaatccaa gtcttcagga tcgtcacgat caaagaggaa accttcaatt gtaacaaagt     480
atgtagaatc agatgatgaa aaacctttgg atgatgaaac tgtaaatgaa gatgcgtcta     540
atgaaaattc agaaaatgat attactatgc agagcttgcc aaaaggtaca gtgattgtac     600
agccagagcc agtgctgaat gaagacaaag atgattttaa agggcctgaa tttagaagca     660
gaagtaaaat gaaaactgaa aatctcaaaa aacgcggaga agatgggctt catgggattg     720
tgagctgcac tgcttgtgga caacaggtca atcattttca aaaagattcc atttatagac     780
acccttcatt gcaagttctt atttgtaaga attgctttaa gtattacatg agtgatgata     840
ttagccgtga ctcagatgga atggatgaac aatgtaggtg gtgtgcggaa ggtggaaact     900
tgatttgttg tgacttttgc cataatgctt tctgcaagaa atgcattcta cgcaaccttg     960
gtcgaaagga gttgtccaca ataatggatg aaaacaacca atggtattgc tacatttgtc    1020
acccagagcc tttgttggac ttggtcactg catgtaacag cgtatttgag aatttagaac    1080
agttgttgca gcaaaataag aagaagataa aagttgacag tgaaaagagt aataaagtat    1140
atgaacatac atccagattt tctccaaaga agactagttc aaattgtaat ggagaagaaa    1200
agaaattaga tgattcctgt tctggctctg taacctactc ttattccgca ctaattgtgc    1260
ccaaagagat gattaagaag gcaaaaaaac tgattgagac cacagccaac atgaactcca    1320
gttatgttaa atttttaaag caggcaacag ataattcaga aatcagttct gctacaaaat    1380
tacgtcagct taaggctttt aagtctgtgt tggctgatat taagaaggct catcttgcat    1440
tggaagaaga cttaaattcc gagtttcgag cgatggatgc tgtaaacaaa gagaaaaata    1500
ccaaagagca taaagtcata gatgctaagt ttgaaacaaa agcacgaaaa ggagaaaaac    1560
cttgtgcttt ggaaaagaag gatatttcaa agtcagaagc taaactttca agaaaacagg    1620
tagatagtga gcacatgcat cagaatgttc caacagagga acaaagaaca aataaaagta    1680
ccggtggtga acataagaaa tctgatagaa aagaagaacc tcaatatgaa cctgccaaca    1740
cttctgaaga tttagacatg gatattgtgt ctgttccttc ctcagttcca gaagacattt    1800
ttgagaatct tgagactgct atggaagttc agagttcagt tgatcatcaa ggggatggca    1860
gcagtggaac tgaacaagaa gtggagagtt catctgtaaa attaaatatt tcttcaaaag    1920
acaacagagg aggtattaaa tcaaaaacta cagctaaagt aacaaaagaa ttatatgtta    1980
aactcactcc tgtttccctt tctaattccc caattaaagg tgctgattgt caggaagttc    2040
cacaagataa agatggctat aaaagttgtg gtctgaaccc caagttagag aaatgtggac    2100
ttggacagga aaacagtgat aatgagcatt tggttgaaaa tgaagtttca ttacttttag    2160
aggaatctga tcttcgaaga tccccacgtg taaagactac acccttgagg cgaccgacag    2220
aaactaaccc tgtaacatct aattcagatg aagaatgtaa tgaaacagtt aaggagaaac    2280
aaaaactatc agttccagtg agaaaaaagg ataagcgtaa ttcttctgac agtgctatag    2340
ataatcctaa gcctaataaa ttgccaaaat ctaagcaatc agagactgtg gatcaaaatt    2400
cagattctga tgaaatgcta gcaatcctca aagaggtgag caggatgagt cacagttctt    2460
cttcagatac tgatattaat gaaattcata caaaccataa gactttgtat gatttaaaga    2520
ctcaggcggg gaaagatgat aaaggaaaaa ggaaacgaaa aagttctaca tctggctcag    2580
attttgatac taaaaagggc aaatcagcta agagctctat aatttctaaa aagaaacgac    2640
aaacccagtc tgagtcttct aattatgact cagaattaga aaaagagata aagagcatga    2700
gtaaaattgg tgctgccaga accaccaaaa aaagaattcc aaatacaaaa gattttgact    2760
cttctgaaga tgagaaacac agcaaaaaag gaatggataa tcaagggcac aaaaatttga    2820
agacctcaca agaaggatca tctgatgatg ctgaaagaaa acaagagaga gagactttct    2880
cttcagcaga aggcacagtt gataaagaca cgaccatcat ggaattaaga gatcgacttc    2940
ctaagaagca gcaagcaagt gcttccactg atggtgtcga taagctttct gggaaagagc    3000
agagttttac ttctttggaa gttagaaaag ttgctgaaac taaagaaaag agcaagcatc    3060
tcaaaaccaa aacatgtaaa aaagtacagg atggcttatc tgatattgca gagaaattcc    3120
taaagaaaga ccagagcgat gaaacttctg aagatgataa aaagcagagc aaaaagggaa    3180
ctgaagaaaa aaagaaacct tcagacttta agaaaaaagt aattaaaatg gaacaacagt    3240
atgaatcttc atctgatggc actgaaaagt tacctgagcg agaagaaatt tgtcattttc    3300
ctaagggcat aaaacaaatt aagaatggaa caactgatgg agaaaagaaa agtaaaaaaa    3360
taagagataa aacttctaaa aagaaggatg aattatctga ttatgctgag aagtcaacag    3420
ggaaaggaga tagttgtgac tcttcagagg ataaaaagag taagaatgga gcatatggta    3480
gagagaagaa aaggtgcaag ttgcttggaa agagttcaag gaagagacaa gattgttcat    3540
catctgatac tgagaaatat tccatgaaag aagatggttg taactcttct gataagagac    3600
tgaaaagaat agaattgagg gaaagaagaa atttaagttc aaagagaaat actaaggaaa    3660

tacaaagtgg ctcatcatca tctgatgctg aggaaagttc tgaagataat aaaaagaaga 3720
agcaaagaac ttcatctaaa aagaaggcag tcattgtcaa ggagaaaaag agaaactccc 3780
taagaacaag cactaaaagg aagcaagctg acattacatc ctcatcttct tctgatatag 3840
aagatgatga tcagaattct ataggtgagg gaagcagcga tgaacagaaa attaagcctg 3900
tgactgaaaa tttagtgctg tcttcacata ctggattttg ccaatcttca ggagatgaag 3960
ccttatctaa atcagtgcct gtcacagtgg atgatgatga tgacgacaat gatcctgaga 4020
atagaattgc caagaagatg cttttagaag aaattaaagc caatctttcc tctgatgagg 4080
atggatcttc agatgatgag ccagaagaag ggaaaaaaag aactggaaaa caaaatgaag 4140
aaaacccagg agatgaggaa gcaaaaaatc aagtcaattc tgaatcagat tcagattctg 4200
aagaatctaa gaagccaaga tacagacata ggcttttgcg gcacaaattg actgtgagtg 4260
acggagaatc tggagaagaa aaaaagacaa agcctaaaga gcataaagaa gtcaaaggca 4320
gaaacagaag aaaggtgagc agtgaagatt cagaagattc tgattttcag gaatcaggag 4380
ttagtgaaga agttagtgaa tccgaagatg aacagcggcc cagaacaagg tctgcaaaga 4440
aagcagagtt ggaagaaaat cagcggagct ataaacagaa aaagaaaagg cgacgtatta 4500
aggttcaaga agattcatcc agtgaaaaca agagtaattc tgaggaagaa gaggaggaaa 4560
aagaagagga ggaggaagag gaggaggagg aggaagagga ggaggaagat gaaaatgatg 4620
attccaagtc tcctggaaaa ggcagaaaga aaattcggaa gattcttaaa gatgataaac 4680
tgagaacaga aacacaaaat gctcttaagg aagaggaaga gagacgaaaa cgtattgctg 4740
agagggagcg tgagcgagaa aaattgagag aggtgataga aattgaagat gcttcaccca 4800
ccaagtgtcc aataacaacc aagttggttt tagatgaaga tgaagaaacc aaagaacctt 4860
tagtgcaggt tcatagaaat atggttatca aattgaaacc ccatcaagta gatggtgttc 4920
agtttatgtg ggattgctgc tgtgagtctg tgaaaaaaac aaagaaatct ccaggttcag 4980
gatgcattct tgcccactgt atgggccttg gtaagacttt acaggtggta agttttcttc 5040
atacagttct tttgtgtgac aaactggatt tcagcacggc gttagtggtt tgtcctctta 5100
atactgcttt gaattggatg aatgaatttg agaagtggca agagggatta aaagatgatg 5160
agaagcttga ggtttctgaa ttagcaactg tgaaacgtcc tcaggagaga agctacatgc 5220
tgcagaggtg gcaagaagat ggtggtgtta tgatcatagg ctatgagatg tatagaaatc 5280
ttgctcaagg aaggaatgtg aagagtcgga aacttaaaga aatatttaac aaagctttgg 5340
ttgatccagg ccctgatttt gttgtttgtg atgaaggcca tattctaaaa aatgaagcat 5400
ctgctgtttc taaagctatg aattctatac gatcaaggag gaggattatt ttaacaggaa 5460
caccacttca aaataaccta attgagtatc attgtatggt taattttatc aaggaaaatt 5520
tacttggatc cattaaggag ttcaggaata gatttataaa tccaattcaa aatggtcagt 5580
gtgcagattc taccatggta gatgtcagag tgatgaaaaa acgtgctcac attctctatg 5640
agatgttagc tggatgtgtt cagaggaaag attatacagc attaacaaaa ttcttgcctc 5700
caaaacacga atatgtgtta gctgtgagaa tgacttctat tcagtgcaag ctctatcagt 5760
actacttaga tcacttaaca ggtgtgggca ataatagtga aggtggaaga ggaaaggcag 5820
gtgcaaagct tttccaagat tttcagatgt taagtagaat atggactcat ccttggtgtt 5880
tgcagctaga ctacattagc aaagaaaata agggttattt tgatgaagac agtatggatg 5940
aatttatagc ctcagattct gatgaaacct ccatgagttt aagctccgat gattatacaa 6000
aaaagaagaa aaaagggaaa aaggggaaaa aagatagtag ctcaagtgga agtggcagtg 6060
acaatgatgt tgaagtgatt aaggtctgga attcaagatc tcggggaggt ggtgaaggaa 6120
atgtggatga aacaggaaac aatccttctg tttctttaaa actggaagaa agtaaagcta 6180
cttcttcttc taatccaagc agcccagctc cagactggta caaagatttt gttacagatg 6240
ctgatgctga ggttttagag cattctggga aaatggtact tctctttgaa attcttcgaa 6300
tggcagagga aattggggat aaagtccttg ttttcagcca gtccctcata tctctggact 6360
tgattgaaga ttttcttgaa ttagctagta gggagaagac agaagataaa gataaacccc 6420
ttatttataa aggtgagggg aagtggcttc gaaacattga ctattaccgt ttagatggtt 6480
ccactactgc acagtcaagg aagaagtggg ctgaagaatt taatgatgaa actaatgtga 6540
gaggacgatt atttatcatt tctactaaag caggatctct aggaattaat ctggtagctg 6600
ctaatcgagt aattatattc gacgcttctt ggaatccatc ttatgacatc cagagtatat 6660
tcagagttta tcgctttgga caaactaagc ctgtttatgt atataggttc ttagctcagg 6720
gaaccatgga agataagatt tatgatcggc aagtaactaa gcagtcactg tcttttcgag 6780
ttgttgatca gcagcaggtg gagcgtcatt ttactatgaa tgagcttact gaactttata 6840
cttttgagcc agacttatta gatgacccta attcagaaaa gaagaagaag agggatactc 6900
ccatgctgcc aaaggatacc atacttgcag agctccttca gatacataaa gaacacattg 6960
taggatacca tgaacatgat tctcttttgg accacaaaga agaagaagag ttgactgaag 7020
aagaaagaaa agcagcttgg gctgagtatg aagcagagaa gaagggactg accatgcgtt 7080
tcaacatacc aactgggacc aatttacccc ctgtcagttt caactctcaa actccttata 7140
ttcctttcaa tttgggagcc ctgtcagcaa tgagtaatca acagctggag gacctcatta 7200
atcaaggaag agaaaaagtt gtagaagcaa caaacagtgt gacagcagtg aggattcaac 7260
ctcttgagga tataatttca gctgtatgga aggagaacat gaatctctca gaggcccaag 7320
tacaggcgtt agcattaagt agacaagcca gccaggagct tgatgttaaa cgaagagaag 7380
caatctacaa tgatgtattg acaaaacaac agatgttaat cagctgtgtt cagcgaatac 7440
ttatgaacag aaggctccag cagcagtaca atcagcagca acagcaacaa atgacttatc 7500
aacaagcaac actgggtcac ctcatgatgc caaagccccc aaatttgatc atgaatcctt 7560
ctaactacca gcagattgat atgagaggaa tgtatcagcc agtggctggt ggtatgcagc 7620
caccaccatt acagcgtgca ccacccccaa tgagaagcaa aaatccagga ccttcccaag 7680
ggaaatcaat gtgattttgc actaaaagct taatggattg ttaaaatcat agaaagatct 7740
tttatttctt taggaatcaa tgacttaaca gaactcaact gtataaatag tttggtcccc 7800
ttaaatgcca atcttccata ttagttttac tttttttttt ttttaaatag ggcataccat 7860
ttcttcctga catttgtcag tgatgttgcc tagaatcttc ttacacacgc tgagtacaga 7920
agatatttca aattgttttc agtgaaaaca agtccttcca taatagtaac aactccacag 7980
atttcctctc taaattttta tgcctgcttt tagcaaccat aaaattgtca taaaattaat 8040

aaatttagga aagaataaag atttatatat tcattcttta catataaaaa cacacagctg 8100
agttcttaga gttgattcct caagttatga aatacttttg tacttaatcc atttcttgat 8160
taaagtgatt gaaatggttt taatgttctt ttgactgaag tctgaaactg ggctcctgct 8220
ttattgtctc tgtgactgaa agttagaaac tgagggttat ctttgacaca gaattgtgtg 8280
caatattctt aaatactact gctctaaaag ttggagaagt cttgcagtta tcttagcatt 8340
gtataaacag ccttaagtat agcctaagaa gagaattcct ttttcttctt tagtccttct 8400
gccatttttt attttcagtt atatgtgctg aaataattac tggtaaaatt tcagggttgt 8460
ggattatctt ccacacatga attttctctc tcctggcacg aatataaagc acatctctta 8520
actgcatggt gccagtgcta atgcttcatc ctgttgctgg cagtgggatg tggacttaga 8580
aaatcaagtt ctagcatttt agtaggttaa cactgaagtt gtggttgtta ggttcacacc 8640
ctgttttata aacaacatca aaatggcaga accattgctg actttaggtt cacatgagga 8700
atgtactttt aacaattccc agtactatca gtattgtgaa ataattcctc tgaaagataa 8760
gaatcactgg cttctatgcg cttcttttct ctcatcatca tgttctttta ccccagtttc 8820
cttacatttt tttaaattgt ttcagagttt gttttttttt tagtttagat tgtgaggcaa 8880
ttattaaatc aaaattaatt catccaatac ccctttacta gaagttttac tagaaaatgt 8940
attacatttt attttttctt aatccagttc tgcaaaaatg acctataaat ttattcatgt 9000
acaattttgg ttacttgaat tgttaaagaa aacattgttt ttgactatgg gagtcaactc 9060
aacatggcag aaccattttt gagatgatga tacaacaggt agtgaaacag cttaagaatt 9120
ccaaaaaaaa aaaaaaaaaa aaaaaaaaag aaaactgggt ttgggctttg ctttaggtat 9180
cactggatta gaatgagttt aacattagct aaaactgctt tgagttgttt ggatgattaa 9240
gagattgcca tttttatctt ggaagaacta gtggtaaaac atccaagagc actaggattg 9300
tgatacagaa tttgtgaggt ttggtggatc cacgcccctc tcccccactt tcccatgatg 9360
aaatatcact aataaatcct gtatatttag atattatgct agccatgtaa tcagatttat 9420
ttaattgggt ggggcaggtg tgtatttact ttagaaaaaa tgaaaaagac aagatttatg 9480
agaaatattt gaaggcagta cactctggcc aactgttacc agttggtatt tctacaagtt 9540
cagaatattt taaacctgat ttactagacc tgggaatttt caacatggtc taattattta 9600
ctcaaagaca tagatgtgaa atttaggcaa ccttctaaat cttttcacca tggatgaaac 9660
tataacttaa agataatact tagaagggtt aattggaaat cagagtttga aataaaactt 9720
ggaccacttt gtatacactc ttctcacttg acattttagc tatataatat gtactttgag 9780
tataacatca agctttaaca aatatttaaa gacaaaaaaa tcacgtcagt aaaatactaa 9840
aaggctcatt tttatatttg ttttagatgt tttaaatagt tgcaatggat taaaaatgat 9900
gatttaaaat gttgcttgta atacagtttt gcctgctaaa ttctccacat tttgtaacct 9960
gttttatttc tttgggtgta aagcgttttt gcttagtatt gtgatattgt atatgttttg 10020
tcccagttgt atagtaatgt ttcagtccat catccagctt tggctgctga aatcatacag 10080
ctgtgaagac ttgcctttgt ttctgttaga ctgcttttca gttctgtatt gagtatctta 10140
agtactgtag aaaagatgtc acttcttcct ttaaggctgt tttgtaatat atataaggac 10200
tggaattgtg tttttaaaga aaagcattca agtatgacaa tatactatct gtgttttcac 10260
cattcaaagt gctgtttagt agttgaaact taaactattt aatgtcattt aataaagtga 10320
ccaaaatgtg 10330

<210> 26
<211> 1777
<212> DNA
<213> Homo sapiens
<220>
<221> misc_feature
<223> séquence appelée GS-N25

<300>
<308> GENBANK/NM_006416
<309> 2001-12-10
<313> (1)..(1777)
<400> 26
agttccgcgg ggggctgtcg gggaaccatg gctgccccga gagacaatgt cactttatta 60
ttcaagttat actgcttggc agtgatgacc ctgatggctg cagtctatac catagcttta 120
agatacacaa ggacatcaga caaagaactc tacttttcaa cgacagccgt gtgtatcaca 180
gaagttataa agttattgct aagtgtggga attttagcta aagaaactgg tagtctgggt 240
agattcaaag catctttaag agaaaatgtc ttggggagcc ccaaggaact gttgaagtta 300
agtgtgccat cgttagtgta tgctgttcag aacaacatgg ctttcctagc tcttagcaat 360
ctggatgcag cagtgtacca ggtgacctac cagttgaaga ttccgtgtac tgctttatgc 420
actgttttaa tgttaaatcg gacactcagc aaattacagt gggtttcagt ttttatgctg 480
tgtgctggag ttacgcttgt acagtggaaa ccagcccaag ctacaaaagt ggtggtggaa 540
caaaatccat tattagggtt tggcgctata gctattgctg tattgtgctc aggatttgca 600
ggagtatatt ttgaaaaagt tttaaagagt tcagatactt ctctttgggt gagaaacatt 660
caaatgtatc tatcagggat tattgtgaca ttagctggcg tctacttgtc agatggagct 720
gaaattaaag aaaaaggatt tttctatggt tacacatatt atgtctggtt tgtcatcttt 780
cttgcaagtg ttggtggcct ctacacttct gttgtggtta agtacacaga caacatcatg 840
aaaggctttt ctgcagcagc ggccattgtc ctttccacca ttgcttcagt aatgctgttt 900
ggattacaga taacactcac ctttgccctg ggtactcttc ttgtatgtgt ttccatatat 960
ctctatggat tacccagaca agacactaca tccatccaac aaggagaaac agcttcaaag 1020
gagagagtta ttggtgtgtg attttagcct cacgtgagac tccttttaag actaaaccat 1080
ttgcattaaa ctagagcctt aagtcaatct cagaaggtag cataaacaaa taaaaattaa 1140
ctgtatggca tgatcagtgc ggttatgtgg aaacaacaac aaacaaacga agctatctga 1200

gtgaactgct aatacagaaa cttaatgtag acctgtttgg ggtctactat tgttttagaa 1260
tgaaggaatt gtattattgt gtgtatatat aatttgtaaa taaaaagtat ggagatgata 1320
cggtgttaaa aaaaatcatg gtaaggctac aatactcaag taacaaggtt tgggacaatg 1380
tctaagggtt aaagtgccaa agccatttct gtactaactg ttctcttgtt ccggtaccgg 1440
ggagaaggat gacccctcct tattctccaa ttcatgtaca gtattttgtc ctagcagcat 1500
aaagacctag ctcttttctt acaagaggca gaaacaagac aggctagttc ataaacaaac 1560
tgtgtaactt ctcaaaatga atctatttca taactcggac aatttctggg tggtgactga 1620
gtaccccttt agtgagtacc cctttagtgc tatatttgtg ccattcatta tctggttcat 1680
atttcttttc tgttagatga tacacatttc ttcaaaaaaa tttctaatgt cacttttgta 1740
cttttttaaa taaagtatgt ttaactgttg ggctctc 1777

<210> 27
<211> 3982
<212> DNA
<213> Homo sapiens
<220>
<221> misc_feature
<223> séquence appelée GS-N26

<300>
<308> GENBANK/U26710
<309> 1996-09-30
<313> (1)..(3982)
<400> 27
ctgggtcctg tgtgtgccac aggggtgggg tgtccagcga gcggtctcct cctcctgcta 60
gtgctgctgc ggcgtcccgc ggcctccccg agtcgggcgg gaggggagag cgggtgtgga 120
tttgtcttga cggtaattgt tgcgtttcca cgtctcggag gcctgcgcgc tgggttgctc 180
cttcttcggg agcgagctgt tctcagcgat cccactccca gccggggctc cccacacaca 240
ctgggctgcg tgcgtgtgga gtgggacccg cgcacacgcg tgtctctgga cagctacggc 300
gccgaaagaa ctaaaattcc agatggcaaa ctcaatgaat ggcagaaacc ctggtggtcg 360
aggaggaaat ccccgaaaag gtcgaatttt gggtattatt gatgctattc aggatgcagt 420
tggaccccct aagcaagctg ccgcagatcg caggaccgtg gagaagactt ggaagctcat 480
ggacaaagtg gtaagactgt gccaaaatcc caaacttcag ttgaaaaata gcccaccata 540
tatacttgat attttgcctg atacatatca gcatttacga cttatattga gtaaatatga 600
tgacaaccag aaacttgccc aactcagtga gaatgagtac tttaaaatct acattgatag 660
ccttatgaaa aagtcaaaac gggcaataag actctttaaa gaaggcaagg agagaatgta 720
tgaagaacag tcacaggaca gacgaaatct cacaaaactg tcccttatct tcagtcacat 780
gctggcagaa atcaaagcaa tctttcccaa tggtcaattc cagggagata actttcgtat 840
cacaaaagca gatgctgctg aattctggag aaagtttttt ggagacaaaa ctatcgtacc 900
atggaaagta ttcagacagt gccttcatga ggtccaccag attagctcta gcctggaagc 960
aatggctcta aaatcaacaa ttgatttaac ttgcaatgat tacatttcag tttttgaatt 1020
tgatattttt accaggctgt ttcagccttg ggctctattt ttgcggaatt ggaatttctt 1080
agctgtgaca catccaggtt acatggcatt tctcacatat gatgaagtta aagcacgact 1140
acagaaatat agcaccaaac ccggaagcta tattttccgg ttaagttgca ctcgattggg 1200
acagtgggcc attggctatg tgactgggga tgggaatatc ttacagacca tacctcataa 1260
caagccctta tttcaagccc tgattgatgg cagcagggaa ggattttatc tttatcctga 1320
tgggaggagt tataatcctg atttaactgg attatgtgaa cctacacctc atgaccatat 1380
aaaagttaca caggaacaat atgaattata ttgtgaaatg ggctccactt ttcagctctg 1440
taagatttgt gcagagaatg acaaagatgt caagattgag ccttgtgggc atttgatgtg 1500
cacctcttgc cttacggcat ggcaggagtc ggatggtcag ggctgccctt tctgtcgttg 1560
tgaaataaaa ggaactgagc ccataatcgt ggaccccttt gatccaagag atgaaggctc 1620
caggtgttgc agcatcattg acccctttgg catgccgatg ctagacttgg acgacgatga 1680
tgatcgtgag gagtccttga tgatgaatcg gttggcaaac gtccgaaagt gcactgacag 1740
gcagaactca ccagtcacat caccaggatc ctctcccctt gcccagagaa gaaagccaca 1800
gcctgaccca ctccagatcc cacatctaag cctgccaccc gtgcctcctc gcctggatct 1860
aattcagaaa ggcatagtta gatctccctg tggcagccca acaggttcac caaagtcttc 1920
tccttgcatg gtgagaaaac aagataaacc actcccagca ccacctcctc ccttaagaga 1980
tcctcctcca ccgccacctg aaagacctcc accaatccca ccagacaata gactgagtag 2040
acacatccat catgtggaaa gcgtgccttc cagagacccg ccaatgcctc ttgaagcatg 2100
gtgccctcgg gatgtgtttg ggactaatca gcttgtggga tgtcgactcc taggggaggg 2160
ctctccaaaa cctggaatca cagcgagttc aaatgtcaat ggaaggcaca gtagagtggg 2220
ctctgaccca gtgcttatgc ggaaacacag acgccatgat ttgcctttag aaggagctaa 2280
ggtcttttcc aatggtcacc ttggaagtga agaatatgat gttcctcccc ggctttctcc 2340
tcctcctcca gttaccaccc tcctccctag cataaagtgt actggtccgt tagcaaattc 2400
tctttcagag aaaacaagag acccagtaga ggaagatgat gatgaataca agattccttc 2460
atcccaccct gtttccctga attcacaacc atctcattgt cataatgtaa aacctcctgt 2520
tcggtcctgt gataatggtc actgtatgct gaatggaaca catggtccat cttcagagaa 2580
gaaatcaaac atccctgact taagcatata tttaaaggga gatgtttttg attcagcctc 2640
tgatcccgtg ccattaccac ctgccaggcc tccaactcgg gacaatccaa agcatggttc 2700
ttcactcaac aggacgccct ctgattatga tcttctcatc cctccattag gtgaagatgc 2760
ttttgatgcc ctccctccat ctctcccacc tcccccacct cctgcaaggc atagtctcat 2820
tgaacattca aaacctcctg gctccagtag ccggccatcc tcaggacagg atctttttct 2880
tcttccttca gatccctttg ttgatctagc aagtggccaa gttcctttgc ctcctgctag 2940

aaggttacca ggtgaaaatg tcaaaactaa cagaacatca caggactatg atcagcttcc 3000
ttcatgttca gatggttcac aggcaccagc cagaccccct aaaccacgac cgcgcaggac 3060
tgcaccagaa attcaccaca gaaaacccca tgggcctgag gcggcattgg aaaatgtcga 3120
tgcaaaaatt gcaaaactca tgggagaggg ttatgccttt gaagaggtga agagagcctt 3180
agagatagcc cagaataatg tcgaagttgc ccggagcatc ctccgagaat ttgccttccc 3240
tcctccagta tccccacgtc taaatctata gcagccagaa ctgtagacac caaaatggaa 3300
agcaatcgat gtattccaag agtgtggaaa taaagagaac tgagatggaa ttcaagagag 3360
aagtgtctcc tcctcgtgta gcagcttgag aagaggcttg ggagtgcagc ttctcaaagg 3420
agaccgatgc ttgctcagga tgtcgacagc tgtggcttcc ttgtttttgc tagccatatt 3480
tttaaatcag ggttgaactg acaaaaataa tttaaagacg tttacttccc ttgaactttg 3540
aacctgtgaa atgctttacc ttgtttacaa tttggcaaag ttgcagtttg ttcttgtttt 3600
tagtttagtt ttgttttggt gttttgatac ctgtactgtg ttcttcacag accctttgta 3660
gcgtggtcag gtctgctgta acatttccca ccaactctct tgctgtccac atcaacagct 3720
aaatcattta ttcatatgga tctctaccat ccccatgcct tgcccaggtc cagttccatt 3780
tctctcattc acaagatgct ttgaaggttc tgattttcaa ctgatcaaac taatgcaaaa 3840
aaaaaaagta tgtattcttc actactgagt ttcttctttg gaaaccatca ctattgagag 3900
atgggaaaaa cctgaatgta taaagcattt atttgtcaat aaactgcctt ttgtaagggg 3960
ttttcacata aaaaaaaaaa aa 3982

<210> 28
<211> 3385
<212> DNA
<213> Homo sapiens
<220>
<221> misc_feature
<223> séquence appelée GS-N27

<300>
<308> GENBANK/XM_039529
<309> 2001-10-16
<313> (1)..(3385)
<400> 28
gtccggctcc ggcggcggcg gtcggtgctg cgagagcggc ggcggcggcg cgggtcggca 60
gcgggagggc gcgcggccga gcggaggcgg agtcggcgcc gagaacatgg ctggaggcaa 120
agctggaaag gacagtggga aggccaaggc taaggcagta tctcgctcac agagagctgg 180
gctacagttt cctgtgggcc gcatccacag acacttgaag actcgcacca caagccatgg 240
aagggtgggt gccactgctg ccgtgtacag tgctgcgatt ctggagtacc tcactgcaga 300
ggtgctggag ctggcaggta atgcttctaa ggatctcaaa gtaaagcgta tcactccgcg 360
tcacttgcag cttgcaatcc gtggtgatga agagttggat tctcttatca aggctaccat 420
agctgggggt gagaagagaa ggtgttctta gatcagaaga tgccattgta ttaggtgacg 480
gcaaggtttt gctgatttag tggaacaaga ttctactggg atgtgtgctg agctgaacca 540
aggaactgcc cgaatgtgct gaacaaggac atttgcttct cagatacgtg aattcaattt 600
taagcaagat tcttgatctg cttcagagca gctttgattt aaagtaattt cagagcgctt 660
ttccttgcat gagtaatttt ctgaatgtat aaaatattct atttatgttt gaccttttgg 720
ataaactgtg ttgctgcagt gttggtccac actaccatcc taagtgaaga cttagtaagt 780
ctgaggatct ccaggcactg gaaagagtga gaacctggga gtgaaagggt tatagttagt 840
gttcattttt cgggttttgt ttcataggga gtcctgagtg gggtaacctg aaatgtttta 900
acagttgaag atcatcctat tcctatcccc agtaaaaata ggcctttcca caatcttcta 960
ccagttaact tagctttttt tttttttttt tcctttaata gagtctcatt cttgtcaccc 1020
agactggagt gcgatggcac gatcttggct cactgcaacc ctccctcccg ggttccagcg 1080
attctcctgc ctcagcctcc caagtagctg ggattacagg cgcacgccac catgcccagc 1140
taatttttgt atttttagta gacgtggggt ttcaccatgt tggccagact ggtctcaaac 1200
tcctgacctc aggcgatccg cccgtctaag ccttccaaag tgctgggatt actggcagga 1260
accaccaggc ccagccaacc agcatttctt aaggtgaaaa tacatggtgt taatttatat 1320
tttgagtgag acccagtcca gctgctataa ctgcttggac acttggcaga ggacagagta 1380
ctgcaaaagt gtctaaggga gacagcattt tgccttgggt gctgtagatg tacaagagag 1440
gttccaggag ggggtgatag gcagaatttt ggtccccatc accttccctg cccagtgtta 1500
tgcctatgaa tgtgttacat tatgtggtaa aagggacttt gcagatgtaa ctaaaatttc 1560
taaaatagag atattatcct ggattacctg ggggaaccca gtgtaattac atgaaccctt 1620
aaaaatggaa gaggatgcag gagtcagatt caaaggaagg cccaaggtgc tattgctgac 1680
ttgaagatag aggggccatg tggaaatcaa gagaaggaag tgaatccttc cagtgagctt 1740
ggaagaaagc accttgaggc acagatgaga agcttggcct tacctgatgc cttgatttta 1800
gcctggtgag accctgagca tataaatttg ctgtgctatg ccacacttct cacctacaga 1860
aacttagttt aaagccacta agtttgtggt aatttgttgg ctttaggccc ctgagggtag 1920
agatttatgg cttgtgttac aagtagagag cagtggagag ttgggctttg taattctttc 1980
aagggtgatt gtagttctgg agtcctatct acctgggttc agatctttgt tggccagtcc 2040
ctggctgtgt gacttcactg ctttaagcct ctgtcttcat ccttaaagtg gtgatgatag 2100
tacctacctc actctgatct ctgaatgaga acacatgtaa gacacttagc acagttcctg 2160
gtgctcaaag agttccaaca cctgaaagca ccatggccct gtttgttttt cagtcaagat 2220
ttctaaaagc cgttattacc agaggttcag tgagaacatg ggcagggtaa actcaaaatg 2280
gagaacataa aaactgggac tcccagacca gtcacttaga agactgctgc aggaagtacc 2340
aaggtcgtta agaatcagta ataataataa gtaatgtgtg aatccattag aatgttgaga 2400
agtagacctt tgacatttca gaaaaatgta tatctcagac acagccatat gtattgttat 2460

ccctgagttg gaagagcatc acagggcata gtttcacaaa agcttcaaaa aatatactaa 2520
ttgctggtaa tatgtgaact aacagaaaaa aatagccatg ttaaaatagg agtgcctgac 2580
aattagctct attcatcttg tctcctagct gtaagcaccc tagattcaaa ttatgcacct 2640
agaaaactaa attactatgt atgttatagg cctttgaaat ggatacatga aaattagtga 2700
aatcagaatg aacgtttcgc ctgccaaaga tatgtatttt tataagtgat acaagggccc 2760
catgagctcc atggccacat ctgtacaaaa caggccctcc tttcaggaac tggtctcgag 2820
agagcaaagg ggtcaactcc ctaaatggca gctgagacag tcctccctgg tggcatggcc 2880
ctgtttcctg ttgatatggt gatagttgga gagtcaaatt tttaccagaa tttgtgaata 2940
tcatctctgg ttaatgatta cacaaaacct tttaaaattt taaaaaatta tttctaattc 3000
tataaataaa ctctgcagtt gtacagccac tgtacagcta acatctcaac actggaaatg 3060
aggctggttt cttccagttt ggctcagatt cttcagggaa gcccagttaa gacaaacagc 3120
tctctatact caatccttac tttgctgaga agatacaggc ctgcccactc gctcccacct 3180
gctcttcacg aggagtcctt tattcctgca tgtttcctgt tctttgggac ctttaggagc 3240
taacctccac agatgctgat agtctgtaga gtcaggaata ccatgtaaca tgttgaagta 3300
gactaaagat tagttctttg gcaatagccc agtctggact tactatgtac ttttgaaaat 3360
aaaactgctt atttgtaaac tctgt 3385

<210> 29
<211> 1128
<212> DNA
<213> Homo sapiens
<220>
<221> misc_feature
<223> séquence appelée GS-N28

<300>
<308> GENBANK/NM_001320
<309> 2000-10-31
<313> (1)..(1128)
<400> 29
gcttctcgtt gtgccccgcc cgcaagcgcc ctcctccggg ccttcgtgac agccaggtcg 60
tgcgcgggtc atcctgggat tggtagttcg ctttctctca tttagccagt ttctttctct 120
accggggact ccgtgtcccg gcatccaccg cggcacctga cccttggcgc ttgcgtgttg 180
ccctcttccc caccctccct aatttccact ccccccaccc cacttcgcct gccgcggtcg 240
ggtccgcggc ctgcgctgta gcggtcgccg ccgttccctg gaagtagcaa cttccctacc 300
ccaccccagt cctggtcccc gtccagccgc tgacgtgaag atgagcagct cagaggaggt 360
gtcctggatt tcctggttct gtgggctccg tggcaatgaa ttcttctgtg aagtggatga 420
agactacatc caggacaaat ttaatcttac tggactcaat gagcaggtcc ctcactatcg 480
acaagctcta gacatgatct tggacctgga gcctgatgaa gaactggaag acaaccccaa 540
ccagagtgac ctgattgagc aggcagccga gatgctttat ggattgatcc acgcccgcta 600
catccttacc aaccgtggca tcgcccagat gttggaaaag taccagcaag gagactttgg 660
ttactgtcct cgtgtgtact gtgagaacca gccaatgctt cccattggcc tttcagacat 720
cccaggtgaa gccatggtga agctctactg ccccaagtgc atggatgtgt acacacccaa 780
gtcatcaaga caccatcaca cggatggcgc ctacttcggc actggtttcc ctcacatgct 840
cttcatggtg catcccgagt accggcccaa gagacctgcc aaccagtttg tgcccaggct 900
ctacggtttc aagatccatc cgatggccta ccagctgcag ctccaagccg ccagcaactt 960
caagagccca gtcaagacga ttcgctgatt ccctccccca cctgtcctgc agtctttgtc 1020
ttttcctttc ttttttgcca ccctttcagg aaccctgtat ggtttttagt ttaaattaaa 1080
ggagtcgtta tcgtggtggg aatatgaaat aaagtagaag aaaaggcc 1128

<210> 30
<211> 18207
<212> DNA
<213> Homo sapiens
<220>
<221> misc_feature
<223> séquence appelée GS-N29

<300>
<308> GENBANK/AF156100
<309> 2001-06-29
<313> (1)..(18207)
<400> 30
gaagccgcat ccagacaaaa gctgccgcat ccctgccctg cccaacccct ggagggattc 60
gagtttggtg cttgtccccg tctgattctc agcgccaaac tttttgctag ttcagagatt 120
ccaagagtct gatgagttac tctgagagga aaccctctgc ctgttgttga ggaggactga 180
gcacagtgct taggcgctga gggggaaaaa gaaggggaaa aaaaagaaaa tgatttcctg 240
ggaagttgtc catacagtat tcctgtttgc tcttctttat tcttccctag ctcaagatgc 300
gagcccccag tcagagatca gagctgagga atttcccgag ggggcctcca cgttggcttt 360
tgtgtttgat gtgactggtt ctatgtatga tgatttagtt caggtgattg aaggggcttc 420
caaaattttg gagacgtctt tgaaaagacc taaaagacct cttttcaact ttgcgttggt 480
gcctttccat gatccagaaa ttggcccagt gacaattacc acagatccca agaaatttca 540
atatgaactc agagaactgt atgttcaggg tggtggtgat tgcccagaaa tgagtattgg 600

agctataaaa attgccttgg aaatttctct tcctggttct ttcatctatg ttttcactga 660
tgctcggtcc aaagattacc ggctcaccca tgaggtgctg caacttatcc aacagaaaca 720
gtcacaagtc gtatttgttc tgactggaga ttgtgatgac aggacccata ttggatataa 780
agtctatgaa gaaattgcct ctacaagttc tggtcaagtg ttccatctgg acaaaaaaca 840
agttaatgag gtattaaaat gggtagaaga agcagtacag gcctccaaag ttcacctttt 900
atccacagat catttggaac aggctgtaaa tacttggaga attccttttg atcccagcct 960
gaaagaggtc actgtgtctt tgagtgggcc ttctccaatg attgaaattc gcaatccttt 1020
agggaagctg ataaaaaagg gatttggcct gcatgagcta ttaaatatcc ataactctgc 1080
caaagtagtg aatgtgaaag agccagaggc tggaatgtgg acagtgaaga cctcaagcag 1140
tggaaggcac tctgttcgca ttactggcct cagtactatt gatttccgag ctggcttttc 1200
tcgaaagccc accctggact tcaaaaaaac agtcagcaga ccagtgcaag gaatacctac 1260
ctatgtactg ctcaatactt ctggaatttc cactccagct agaatagatc ttcttgaact 1320
tttgagtatc tcaggaagtt ctcttaagac tattcctgtt aaatattacc cacatcgaaa 1380
accttatggc atatggaata tttctgactt tgtaccacca aatgaagctt tctttctcaa 1440
agtaacaggc tatgataaag atgattacct cttccagaga gtatcaagtg tttccttttc 1500
tagtattgtc ccagatgctc ccaaagttac gatgcctgag aaaaccccag gatactatct 1560
gcagccgggc caaattccct gctctgttga cagtcttttg ccctttacct tgagctttgt 1620
cagaaatgga gttacacttg gagtagacca gtatttgaaa gaatctgcca gtgtgagctt 1680
agatattgca aaggtcactt tgtctgacga aggtttctat gaatgcattg ctgtcagcag 1740
tgcaggtact ggacgggcac agacattttt tgacgtatca gagccccctc cggtcatcca 1800
agtgcctaac aatgttacag tcactcctgg agagagagca gttttaacat gtctcatcat 1860
cagtgcggtg gattacaatc taacctggca gaggaatgac agagatgtca gactggcaga 1920
gccagcgaga attaggacct tggctaatct gtcattggag ctaaagagtg tgaaattcaa 1980
cgatgctgga gagtatcatt gtatggtttc tagtgaaggt ggatcatcag ccgcttcagt 2040
tttcctcaca gtgcaagaac cacccaaagt cactgtgatg cccaagaatc agtctttcac 2100
aggagggtct gaggtctcca tcatgtgttc tgcaacaggt tatcccaaac caaagattgc 2160
ctggaccgtt aacgatatgt ttatcgtggg ttcacacagg tataggatga cctcagatgg 2220
taccttattt atcaaaaatg cagctcccaa agatgcaggg atctatggtt gcctagcaag 2280
taattcagct ggaacagata aacagaattc tactctcaga tacattgaag cccctaagtt 2340
gatggtagtt cagagtgagc tcttggttgc ccttggggat ataaccgtta tggaatgcaa 2400
aacctctggt attcctccac ctcaagttaa atggttcaaa ggagatcttg agttgaggcc 2460
ctcaacattc ctcattattg accctctctt gggacttttg aagattcaag aaacacaaga 2520
tttggatgct ggcgattata cctgtgtagc catcaatgag gctggaagag caactggcaa 2580
gataactctg gatgttggct cacctccagt tttcatacaa gaacctgctg atgtgtctat 2640
ggaaattggc tcaaatgtga cattaccttg ttatgttcag ggttatccag aaccaacaat 2700
caaatggcga agattagaca acatgccaat tttctcaaga cctttttcag ttagttccat 2760
cagccaacta agaacaggag ctctctttat tttaaactta tgggcaagtg ataaaggaac 2820
ctatatttgt gaagctgaaa accagtttgg aaagatccag tcagagacaa cagtaacagt 2880
gaccggactt gttgctccac ttattggaat cagcccttca gtggccaatg ttattgaagg 2940
acagcagctt actttgccct gtactctgtt agctggaaat cccattccag aacgtcggtg 3000
gattaagaat tcagctatgt tgctccaaaa tccttacatc actgtgcgca gtgatgggag 3060
cctccatatt gaaagagttc agcttcagga tggtggtgaa tatacttgtg tggccagtaa 3120
cgttgctggg accaataaca aaactacctc tgtggttgtg catgttctgc caaccattca 3180
gcatgggcag cagatactca gtacaattga aggcattcca gtaactttac catgcaaagc 3240
aagtggaaat cccaaaccgt ctgtcatctg gtccaagaaa ggagagctga tttcaaccag 3300
cagtgctaag ttttcagcag gagctgatgg tagtctgtat gtggtatcac ctgaaggaga 3360
ggagagtggg gagtatgtct gcactgccac caatacagcc ggctacgcca aaaggaaagt 3420
gcagctaaca gtctatgtaa ggcccagagt gtttggagat ctacgaggac tgtcccaaga 3480
taagcctgtt gagatctccg tccttgcagg ggaagaggta acacttccat gtgaagtgaa 3540
gagcttacct ccacccataa ttacttgggc caaagaaacc cagctcatct caccgttctc 3600
tccaagacac acattcctcc cttctggttc aatgaagatc actgaaaccc gcacttcaga 3660
tagtgggatg tatctttgtg ttgccacaaa tattgctggg aatgtgactc aggctgtcaa 3720
attaaatgtc catgttcctc caaagataca gcgtggacct aaacatctca aagtccaagt 3780
tggtcaaaga gtggatattc catgtaatgc tcaagggact cctcttcctg taatcacctg 3840
gtccaaaggt ggaagcacta tgctggttga tggagagcac catgttagca atccagacgg 3900
aactttaagc atcgaccaag ccacgccctc agatgctggc atatatacat gtgttgctac 3960
taacatagca ggcactgatg aaacagagat aacgctacat gtccaagaac cacccacagt 4020
ggaagatcta gaacctccat ataacactac tttccaagaa agagtggcca atcaacgcat 4080
tgaatttcca tgtcctgcaa aaggtacccc taaaccaacc atcaaatggt tacacaatgg 4140
tagagagttg acaggcagag agcctggcat ttctatcttg gaagaaggca cattgctggt 4200
tattgcttct gttacaccct atgacaatgg ggagtacatc tgtgtggcag tcaatgaagc 4260
tggaaccaca gaaagaaaat ataacctcaa agtccatgtt cctccagtaa ttaaagataa 4320
agaacaagta tcaaatgtgt cggtgttgtt aaatcagctg accaatctct tctgtgaagt 4380
ggaaggcact ccatctccca tcattatgtg gtataaagat aatgtccagg tgactgaaag 4440
cagcactatt cagactgtga acaatgggaa gatactgaag ctcttcagag ccactccaga 4500
ggatgcagga agatattcct gcaaagcaat taatattgca ggcacttctc agaagtactt 4560
taacattgat gtgctagttc cacccaccat aataggtacc aacttcccaa aggaagtctc 4620
agttgtcctc aaccgtgacg tcgcccttga atgccaggtc aaaggcactc cctttcctga 4680
tattcattgg ttcaaagatg gcaagccttt atttttgggc gatcctaatg ttgaacttct 4740
agacagagga caagtcttac atttaaagaa tgcacggaga aatgacaagg ggcgctacca 4800
atgtactgtg tctaatgcag ctggcaaaca agccaaggat ataaaactga ctatctatat 4860
tccacctagt attaaaggag gaaatgtcac cacagacata tcagtattga tcaacagcct 4920
tattaaactg gaatgtaaaa cacggggact tccaatgcct gccattactt ggtataagga 4980

cgggcagcca atcatgtcca gctcacaagc actttatatt gataaaggac aatatcttca 5040
tattcctcga gcacaggtct ctgattcagc aacatatacg tgtcacgtag ccaatgttgc 5100
tggaactgct gaaaaatcat tccatgtgga tgtctatgtt cctccaatga ttgaaggcaa 5160
cttggccacg cctttgaata agcaagtagt tattgctcat tctctgacac tggagtgcaa 5220
tgctgctgga aacccttctc ccattctcac ctggttgaaa gatggtgtac ctgtgaaagc 5280
taatgacaat ttccgcatag aagctggtgg gaagaaactc gaaatcatga gtgcccaaga 5340
aattgatcga ggacagtaca tatgcgtggc taccagtgtg gcaggagaaa aggaaatcaa 5400
atatgaagtt gatgtcttgg tgccaccagc tatagaagga ggagatgaaa catcttactt 5460
cattgtgatg gttaataact tactggagct agattgtcat gtgacaggct ctcccccacc 5520
aactatcatg tggctgaagg atggccagtt aattgatgaa agggatggat tcaagatttt 5580
attaaatgga cgcaaactgg ttattgctca ggctcaagtg tcaaacacag gcctttatcg 5640
gtgcatggca gcaaatactg ctggagacca caagaaggaa tttgaagtga ctgttcatgt 5700
tcctccaaca atcaagtcct caggcctttc tgagagagtt gtggtaaaat acaagcctgt 5760
cgccttgcag tgcatagcca atgggattcc aaatccttcc attacatggt taaaagatga 5820
ccagcctgtg aacactgccc aaggaaacct taaaatacag tcttctggtc gagttctaca 5880
aattgccaaa accctgttgg aagatgctgg cagatacaca tgtgtggcta ccaacgcagc 5940
tggagaaaca caacagcaca ttcaactgca tgttcatgaa ccacctagtc tggaagatgc 6000
tggaaaaatg ctgaatgaga ctgtgttggt gagcaaccct gtacagctgg agtgtaaggc 6060
agctggaaat cctgtgcctg ttattacatg gtacaaagat aattgtttac tctcaggttc 6120
caccagcatg actttcttga acagaggaca gatcattgat attgaaagtg cccagatctc 6180
agatgctggc atatataaat gcgtggccat caactcagct ggagctacag agttatttta 6240
cagtctgcaa gttcatgtgg ccccatcaat ttctggcagc aataacatgg tggcagtggt 6300
ggttaataac ccggtgaggt tagaatgtga agccagaggt attcctgccc caagtctgac 6360
ctggttgaaa gatgggagtc ctgtttctag tttttctaat ggattacagg ttctctctgg 6420
tggtcgaatc ctagcattga ccagtacaca aatcagcgac acaggaaggt acacctgcgt 6480
ggcagtgaat gctgctggag aaaagcaaag ggacattgac ctccgagtat atgttccgcc 6540
aaatattatg ggagaagaac agaatgtctc tgtcctcatt agccaagctg tggaattact 6600
atgtcaaagt gatgctattc ccccacctac tcttacttgg ttaaaagacg gccacccctt 6660
gctgaagaaa ccaggcctca gtatatctga aaatagaagt gtgttaaaga ttgaagatgc 6720
tcaggttcaa gacactggtc gttacacttg tgaagcaaca aatgttgctg gaaaaactga 6780
aaaaaaaaac tacaatgtca acatttgggt ccccccaaat attggtggtt ctgatgaact 6840
tactcaactt acagtcattg aagggaatct cattagtctg ttgtgtgaat caagtggtat 6900
tccaccccca aatctcatct ggaagaagaa aggctctcca gtgctgactg attccatggg 6960
gcgagttaga attttatctg ggggcaggca attacaaatt tcaattgctg aaaagtctga 7020
tgcagcactc tattcatgtg tggcgtcgaa tgttgctggg actgcaaaga aagaatacaa 7080
tctgcaagtt tacattagac caaccataac caacagtggc agccacccta ctgaaattat 7140
tgtgacccga gggaagagta tctccttgga gtgtgaggtg cagggtattc caccaccaac 7200
agtgacctgg atgaaagatg gccacccctt gatcaaggca aagggagtag aaatactgga 7260
tgaaggtcac atccttcagc tgaagaacat tcatgtatct gacacaggcc gttatgtgtg 7320
tgttgctgtg aatgtagcag gaatgactga caaaaaatat gacttaagtg tccatgctcc 7380
tccaagcatc ataggaaacc acaggtcacc tgaaaatatt agtgtggtag aaaagaactc 7440
agtatctttg acttgtgaag cttctggaat tcccctgcct tccacaacct ggttcaaaga 7500
tgggtggcct gtcagcctta gcaattctgt gaggattctt tcaggaggca ggatgctacg 7560
gctgatgcag accacaatgg aagatgctgg ccaatatact tgtgttgtaa ggaatgcagc 7620
tggtgaagaa agaaaaatct ttgggctttc agtattagta ccacctcata ttgtgggtga 7680
aaatacattg gaagatgtga aggtaaaaga gaaacagagt gttacgctga cttgtgaagt 7740
gacagggaat ccagtgccag aaattacatg gcacaaagat gggcagcccc tccaagaaga 7800
tgaagcccat cacattatat ctggtggccg ttttcttcaa attaccaatg tccaggtgcc 7860
acacactgga agatatacat gtttggcttc cagtccagct ggccacaaga gcaggagctt 7920
cagtcttaat gtatttgtat ctcctacaat tgctggtgta ggtagtgatg gcaaccctga 7980
agatgtcact gtcatcctta acagccctac atctttggtc tgtgaagctt attcatatcc 8040
tccagctacc atcacctggt ttaaggatgg cactccttta gaatctaacc gaaatattcg 8100
tattcttcca ggaggcagaa ctctgcagat cctcaatgca caggaggaca atgctggaag 8160
atactcttgt gtagccacga atgaggctgg agaaatgata aagcactatg aagtgaaggt 8220
gtacattcca cccataatca ataaagggga cctttggggg ccaggtcttt cccctaaaga 8280
agtgaagatc aaagtaaaca acactctgac cttggaatgt gaagcgtatg caattccttc 8340
tgcctccctc agctggtaca aggatggaca gccccttaaa tccgatgatc atgttaatat 8400
tgctgcgaat ggacacacac ttcaaataaa ggaggctcaa atatcagaca ccggacgata 8460
tacttgtgta gcatctaaca ttgcaggtga agatgagttg gattttgatg tgaatattca 8520
agttcctcca agttttcaga aactctggga aataggaaac atgctagata ctggcaggaa 8580
tggtgaagcc aaagatgtga tcatcaacaa tcccatttct ctttactgtg agacaaatgc 8640
tgctccccct cctacactga catggtacaa agatggccac cctctgacct caagtgataa 8700
agtattgatt ttgccaggag ggcgagtgtt gcagattcct cgggctaaag tagaagatgc 8760
tgggagatac acatgtgtgg ctgtgaatga ggctggagaa gattcccttc aatatgatgt 8820
ccgtgtactc gtgccgccaa ttatcgaggg agcaaatagt gatctccctg aagaggtcac 8880
cgtgctggtg aacaagagtg cactgataga gtgtttatcc agtggcagcc cagcaccaag 8940
gaattcctgg cagaaagatg gacagccctt gctagaagat gaccatcata aatttctatc 9000
taatggacga attctgcaga ttctgaatac tcaaataaca gatatcggca ggtatgtgtg 9060
tgttgctgag aacacagctg ggagtgccaa aaaatatttt aacctcaatg ttcatgttcc 9120
tccaagtgtc attggtccta aatctgaaaa tcttaccgtc gtggtgaaca atttcatctc 9180
tttgacctgt gaggtctctg gttttccacc tcctgacctc agctggctca agaatgamca 9240
gcccatcaaa ctgaacacaa atactctcat tgtgcctggt ggtcgaactc tacagattat 9300
tcgggccaag gtatcagatg gtggtgaata cacttgtata gctatcaatc ragctggcga 9360

aagcaagaar aagttttccc tgactgttta tgtgccmccm agcattaaag accatgacag 9420
tgaatctctt tctgtagtta atgtaagaga gggaacttct gtgtctttgg agtgtgagtc 9480
gaacgctgtg ccacctccag tcatcacttg gtataagaat gggcggatga taacagagtc 9540
tactcatgtg gagattttag ctgatggaca aatgctacac attaagaaag ctgaggtatc 9600
tgacacaggc cagtatgtat gtagagctat aaatgtagca ggacgggatg ataaaaattt 9660
ccacctcaat gtatatgtgc cacccagtat tgaaggacct gaaagagaag tgattgtgga 9720
gacgatcagc aatcctgtga cattaacatg tgatgccact gggatcccac ctcccacgat 9780
agcatggtta aagaactaca agcgcataga aaattctgac tcactggaag ttcgtatttt 9840
gtctggaggt agcaaactcc agattgcccg gtctcagcat tcagatagtg gaaactatac 9900
atgtattgct tcaaatatgg agggaaaagc ccagaaatat tactttcttt caattcaagt 9960
tcctccaagt gttgctggtg ctgaaattcc aagtgatgtc agtgtccttc taggagaaaa 10020
tgttgagctg gtctgcaatg caaatggcat tcctactcca cttattcaat ggcttaaaga 10080
tggaaagccc atagctagtg gtgaaacaga aagaatccga gtgagtgcaa atggcagcac 10140
attaaacatt tatggagctc ttacatctga cacggggaaa tacacatgtg ttgctactaa 10200
tcccgctgga gaagaagacc gaatttttaa cttgaatgtc tatgttacac ctacaattag 10260
gggtaataaa gatgaagcag agaaactaat gacttatgtg gatacttcaa taaatattga 10320
atgcagagyc acagggacgc ctccaccaca gataaactgg ctgaagaatg gacttcctct 10380
gcctctctcc tcccatatcc ggttactggc agcaggacaa gttatcagga ttgtgagagc 10440
tcaggtgtct gatgtcgctg tgtatacttg tgtggcctcc aacagagctg gggtggataa 10500
taagcattac aatcttcaag tgtttgcacc accaaatatg gacaattcaa tggggacaga 10560
ggaaatcaca gttctcaaag gtagttccac ctctatggca tgcattactg atggaacccc 10620
agctcccagt atggcctggc ttagagatgg ccagcctctg gggcttgatg cccatctgac 10680
agtcagcacc catggaatgg tcctgcagct cctcaaagca gagactgaag attcgggaaa 10740
gtacacctgc attgcctcaa atgaagctgg agaagtcagc aagcacttta tcctcaaggt 10800
cctagaacca cctcacatta atggatctga agaacatgaa gagatatcag taattgttaa 10860
taacccactt gaacttacct gcattgcttc tggaatccca gcccctaaaa tgacctggat 10920
gaaagatggc cggccccttc cacagacgga tcaagtgcaa actctaggag gaggagaggt 10980
tcttcgaatt tctactgctc aggtggagga tacaggaaga tatacatgtc tggcatccag 11040
tcctgcagga gatgatgata aggaatatct agtgagagtg catgtacctc ctaatattgc 11100
tggaactgat gagccccggg atatcactgt gttacggaac agacaagtga cattggaatg 11160
caagtcagat gcagtgcccc cacctgtaat tacttggctc agaaatggag aacggttaca 11220
ggcaacacct cgagtgcgaa tcctatctgg agggagatac ttgcaaatca acaatgctga 11280
cctaggtgat acagccaatt atacctgtgt tgccagcaac attgcaggaa agactacaag 11340
agaatttatt ctcactgtaa atgttcctcc aaacataaag gggggccccc agagccttgt 11400
aattctttta aataagtcaa ctgtattgga atgcatcgct gaaggtgtgc caactccaag 11460
gataacatgg agaaaggatg gagctgttct agctgggaat catgcaagat attccatctt 11520
ggaaaatgga ttccttcata ttcaatcagc acatgtcact gacactggac ggtatttgtg 11580
tatggccacc aatgctgctg gaacagatcg caggcgaata gatttacagg tccatgttcc 11640
tccatctatt gctccgggtc ctaccaacat gactgtaata gtaaatgttc aaactactct 11700
ggcttgtgag gctactggga taccaaaacc atcaatcaat tggagaaaaa atgggcatct 11760
tcttaatgtg gatcaaaatc agaactcata caggctcctt tcttcaggtt cactagtaat 11820
tatttcccct tctgtggatg acactgcaac ctatgaatgt actgtgacaa acggtgctgg 11880
agatgataaa agaactgtgg atctcactgt ccaagttcca ccttccatag ctgatgagcc 11940
tacagatttc ctagtaacca aacatgcccc agcagtaatt acctgcactg cttcgggagt 12000
tccatttccc tcaattcact ggaccaaaaa tggtataaga ctgcttccca ggggagatgg 12060
ctatagaatt ctgtcctcag gagcaattga aatacttgcc acccaattaa accatgctgg 12120
aagatacact tgtgtcgcta ggaatgcggc tggctctgca catcgacacg tgacccttca 12180
tgttcatgag cctccagtca ttcagcccca accaagtgaa ctacacgtca ttctgaacaa 12240
tcctatttta ttaccatgtg aagcaacagg gacacccagt cctttcatta cttggcaaaa 12300
agaaggcatc aatgttaaca cttcaggcag aaaccatgca gttcttccta gtggcggctt 12360
acagatctcc agagctgtcc gagaggatgc tggcacttac atgtgtgtgg cccagaaccc 12420
ggctggtaca gccttgggca aaatcaagtt aaatgtccaa gttcctccag tcattagccc 12480
tcatctaaag gaatatgtta ttgctgtgga caagcccatc acgttatcct gtgaagcaga 12540
tggcctcccc ccgcctgaca ttacatggca taaagatggg cgtgcaattg tggaatctat 12600
ccgccagcgc gtcctcagct ctggctctct gcaaatagca tttgtccagc ctggtgatgc 12660
tggccattac acgtgcatgg cagccaatgt agcaggatca agcagcacaa gcaccaagct 12720
caccgtccat gtaccaccca ggatcagaag tacagaagga cactacacgg tcaatgagaa 12780
ttcacaagcc attcttccat gcgtagctga tggaatcccc acaccagcaa ttaactggaa 12840
aaaagacaat gttcttttag ctaacttgtt aggaaaatac actgctgaac catatggaga 12900
actcatttta gaaaatgttg tgctggagga ttctggcttc tatacctgtg ttgctaacaa 12960
tgctgcaggt gaagatacac acactgtcag cctgactgtg catgttctcc ccacttttac 13020
tgaacttcct ggagacgtgt cattaaataa aggagaacag ctacgattaa gctgtaaagc 13080
tactggtatt ccattgccca aattaacatg gaccttcaat aacaatatta ttccagccca 13140
ctttgacagt gtgaatggac acagtgaact tgttattgaa agagtgtcaa aagaggattc 13200
aggtacttat gtgtgcaccg cagagaacag cgttggcttt gtgaaggcaa ctggatttgt 13260
ttatgtgaaa gaacctccag tcttcaaagg tgattatcct tctaactgga ttgaaccact 13320
tggtgggaat gcaatcctga attgtgaggt gaaaggagac cccaccccaa ccatccagtg 13380
gaacagaaag ggagtggata ttgaaattag ccacagaatc cggcaactgg gcaatggctc 13440
cctggccatc tatggcactg ttaatgaaga tgccggtgac tatacatgtg tagctaccaa 13500
tgaagctggg gtggtggagc gcagcatgag tctgactctg caaagtcctc ctattatcac 13560
tcttgagcca gtggaaactg ttattaatgc tggtggcaaa atcatattga attgtcaggc 13620
aactggagag cctcaaccaa ccattacatg gtcccgtcaa gggcactcta tttcctggga 13680
tgaccgggtt aacgtgttgt ccaacaactc attatatatt gctgatgctc agaaagaaga 13740

tacctctgaa tttgaatgtg ttgctcgaaa cttaatgggt tctgtccttg tcagagtgcc 13800
agtcatagtc caggttcatg gtggattttc ccagtggtct gcatggagag cctgcagtgt 13860
cacctgtgga aaaggcatcc aaaagaggag tcgtctgtgc aaccagcccc ttccagccaa 13920
tggtgggaag ccctgccaag gttcagattt ggaaatgcga aactgtcaaa ataagccttg 13980
tccagtggat ggtagctggt cggaatggag tctttgggaa gaatgcacaa ggagctgtgg 14040
acgcggcaac caaaccagga ccaggacttg caataatcca tcagttcagc atggtgggcg 14100
gccatgtgaa gggaatgctg tggaaataat tatgtgcaac attaggcctt gcccagttca 14160
tggagcatgg agcgcttggc agccttgggg aacatgcagc gaaagttgtg ggaaaggtac 14220
tcagacaaga gcaagacttt gtaataaccc accaccagcg tttggtgggt cctactgtga 14280
tggagcagaa acacagatgc aagtttgcaa tgaaagaaat tgtccaattc atggcaagtg 14340
ggcgacttgg gccagttgga gtgcctgttc tgtgtcatgt ggaggaggtg ccagacagag 14400
aacaaggggc tgctccgacc ctgtgcccca gtatggagga aggaaatgcg aagggagtga 14460
tgtccagagt gatttttgca acagtgaccc ttgcccaacc catggtaact ggagtccttg 14520
gagtggctgg ggaacatgca gccggacgtg taacggaggg cagatgcggc ggtaccgcac 14580
atgtgataac cctcctccct ccaatggggg aagagcttgt ggggggacca actcccagat 14640
ccagaggtgc aacactgaca tgtgtcctgt ggatggaagt tggggaagct ggcatagttg 14700
gagccagtgc tctgcctcct gtggaggagg tgaaaagact cggaagcggc tgtgcgacca 14760
tcctgtgcca gttaaaggtg gccgtccctg tcccggagac actactcagg tgaccaggtg 14820
caatgtacaa gcatgtccag gtgggcccca gcgagccaga ggaagtgtta ttggaaatat 14880
taatgatgtt gaatttggaa ttgctttcct taatgccaca ataactgata gccctaactc 14940
tgatactaga ataatacgtg ccaaaattac caatgtacct cgtagtcttg gttcagcaat 15000
gagaaagata gtttctattc taaatcccat ttattggaca acagcaaagg aaataggaga 15060
agcagtcaat ggctttaccc tcaccaatgc agtcttcaaa agagaaactc aagtggaatt 15120
tgcaactgga gaaatcttgc agatgagtca tattgcccgg ggcttggatt ccgatggttc 15180
tttgctgcta gatatcgttg tgagtggcta tgtcctacag cttcagtcac ctgctgaagt 15240
cactgtaaag gattacacag aggactacat tcaaacaggt cctgggcagc tgtacgccta 15300
ctcaacccgg ctgttcacca ttgatggcat cagcatccca tacacatgga accacaccgt 15360
tttctatgat caggcacagg gaagaatgcc tttcttggtt gaaacacttc atgcatcctc 15420
tgtggaatct gactataacc agatagaaga gacactgggt tttaaaattc atgcttcaat 15480
atccaaagga gatcgcagta atcagtgccc ctccgggttt accttagact cagttggacc 15540
tttttgtgct gatgaggatg aatgtgcagc agggaatccc tgctcccata gctgccacaa 15600
tgccatgggg acttactact gctcctgccc taaaggcctc accatagctg cagatggaag 15660
aacttgtcaa gatattgatg agtgtgcttt gggtaggcat acctgccacg ctggtcagga 15720
ctgtgacaat acgattggat cttatcgctg tgtggtccgt tgtggaagtg gctttcgaag 15780
aacctctgat gggctgagtt gtcaagatat taatgaatgt caagaatcca gcccctgtca 15840
ccagcgctgt ttcaatgcca taggaagttt ccattgtgga tgtgaacctg ggtatcagct 15900
caaaggcaga aaatgcatgg atgtgaacga gtgtagacaa aatgtatgca gaccagatca 15960
gcactgtaag aacacccgtg gtggctataa gtgcattgat ctttgtccaa atggaatgac 16020
caaggcagaa aatggaacct gtattgatat tgatgaatgt aaagatggga cccatcagtg 16080
cagatataac cagatatgtg agaatacaag aagcagctat cgttgtgtat gcccaagagg 16140
ttatcggtct caaggagttg gaagaccctg catggacatt gatgaatgtg aacaagtgcc 16200
taaaccttgt gcacatcagt gctccaacac ccccggcagc ttcaagtgta tctgtccacc 16260
aggacaacat ttattagggg acgggaaatc ttgcgctgga ttggagaggc tgccaaatta 16320
tggcactcaa tacagtagct ataaccttgc acggttctcc cctgtgagaa acaactatca 16380
acctcaacag cattacagac agtactcaca tctctacagc tcctactcag agtatagaaa 16440
cagcagaaca tctctctcca ggactagaag gactattagg aaaacttgcc ctgaaggctc 16500
tgaggcaagc catgacacat gtgtagatat tgatgaatgt gaaaatacag atgcctgcca 16560
gcatgagtgt aagaatacct ttggaagtta tcagtgcatc tgcccacctg gctatcaact 16620
cacacacaat ggaaagacat gccaagatat cgatgaatgt ctggagcaga atgtgcactg 16680
tggacccaat cgcatgtgct tcaacatgag aggaagctac cagtgcatcg atacaccctg 16740
tccacccaac taccaacggg atcctgtttc agggttctgc ctcaagaact gtccacccaa 16800
tgatttggaa tgtgccttga gcccatatgc cttggaatac aaactcgtct ccctcccatt 16860
tggaatagcc accaatcaag atttaatccg gctggttgca tacacacagg atggagtgat 16920
gcatcccagg acaactttcc tcatggtaga tgaggaacag actgttcctt ttgccttgag 16980
ggatgaaaac ctgaaaggag tggtgtatac aacacgacca ctacgagaag cagagaccta 17040
ccgcatgagg gtccgagcct catcctacag tgccaatggg accattgaat atcagaccac 17100
attcatagtt tatatagctg tgtccgccta tccatactaa ggaactctcc aaagcctatt 17160
ccacatattt aaaccgcatt aatcatggca atcaagcccc cttccagatt actgtctctt 17220
gaacagttgc aatcttggca gcttgarart ggtgctacac tctgttttgt gtgccttcct 17280
tggtacttct gaggtatttt catgatccca ccatggtcat atcttgaagt atggtctaga 17340
aaagtccctt attatttatt attacactgg agcagttact tcccaaagat tattctgaac 17400
atctaacagg acatatcagt gatggttaca gtagkgtagt acctaagatc attttcctga 17460
aagcccaacc aracaacgar aaacaagaac aactaattca gaatcaaata gagtttttga 17520
gcatttgact atttttagaa tcataaaatt agttacawag tattttgatc aaagcctatt 17580
aaaataactt acggagattt ttgtaagtat tgatacatta taataggact tgcctatttt 17640
catttttaag aagaaaaaca ccactcattt tataaaatat agtacagcta ctataaggct 17700
tgtttgatcc caaatggtgc ttatcttgat tgaacattca gaacaaggat attattttca 17760
gtgattttgt gagatcagct gaaccactta tgataataat aataaaaaag actgctttgc 17820
cctcacgtca gttgtacatg gcatggaact ttaaaaattt taatataaac tttcatccag 17880
ttagcttcat aacttttacg ttccagaatt ttgtttattt tcctgtcaat gaaagcaatt 17940
tttaaagata ccagtgggac aggtttggtt ttttaaaaat ctcatgtgtt caaattaaca 18000
taaatattac acgtcaatac actgtacatg gtggtaatag actctaagca attgccaaga 18060
tgtattctat ttttatgaag tgtatatata ttaccttagt gtgcattttc tatataatat 18120

cttgatggac tcttttataa aattatttta taaaaaacaa tgttacacta aaatcagcct 18180
aaataaattt tcacaacttt ttttcat 18207

<210> 31
<211> 4325
<212> DNA
<213> Homo sapiens
<220>
<221> misc_feature
<223> séquence appelée GS-N30

<300>
<308> GENBANK/NM_015180
<309> 2001-07-15
<313> (1)..(4325)
<400> 31
ggaggcgaac tacctgtgcc ctaaccttgg aagctggaga aaagttactg ctcacaactg 60
acctgaaaac taaagagtct gtgggtagga gaatcagtca acttcaggac agctggaaag 120
acatggagcc ccagctggca gagatgatta agcagttcca gagcactgta gagacctggg 180
accagtgtga aaagaaaatc aaggagttga aaagcaggct gcaagtttta aaggcacaaa 240
gtgaagatcc tcttccagag cttcacgagg acctccataa cgaaaaagag ctgattaagg 300
aactagaaca gtctttggct agctggactc agaacttgaa agaacttcaa actatgaagg 360
cggacttaac ccggcacgtt ctcgtggaag atgtgatggt tttgaaggag caaatagagc 420
atttgcacag acaatgggag gacctctgct taagggtggc catacgtaaa caggagattg 480
aagacagact caatacatgg gttgtattca atgaaaaaaa taaagagttg tgtgcctggc 540
tggtgcagat ggaaaacaaa gttctacaga cagcggacat tagtattgaa gaaatgattg 600
aaaagttaca gaaggactgc atggaagaaa taaacttgtt tagtgaaaac aagttacagt 660
taaagcagat gggtgaccag ttgatcaagg ccagcaacaa atcaagagca gctgagatcg 720
atgacaagct caacaaaatt aacgatcgtt ggcaacatct ttttgatgtc atcggatcaa 780
gggtgaagaa gctgaaggag acctttgctt ttattcagca gttggacaaa aacatgagca 840
accttcgcac ctggttggct cgaattgagt ctgagctttc caagcctgtt gtttatgatg 900
tctgcgatga tcaagagatc cagaagaggc tcgctgagca gcaggatcta cagcgagata 960
ttgaacaaca cagcgcaggg gtggagtccg tgtttaacat ctgtgacgtc ctactgcacg 1020
actccgatgc ctgtgcaaat gagaccgagt gtgactcgat ccagcagacc accaggagcc 1080
tggacagacg ctggaggaac atttgtgcca tgtccatgga gcggcgcatg aaaatcgagg 1140
agacgtggcg cctgtggcag aagtttttag acgactattc tcgctttgag gactggctca 1200
agtcagctga gaggacggca gcctgcccaa attcctcaga ggtgttgtac acgagtgcca 1260
aagaggaact gaagaggttt gaggcctttc agcggcagat tcatgagcgg ctcactcagc 1320
tggagctcat caacaagcag taccggcggc tggcccggga gaaccgcaca gacacggcca 1380
gcaggctgaa gcagatggtc cacgagggca accagcgctg ggacaacctt cagaggcggg 1440
tcacagccgt cctgcggaga ctcaggcatt tcaccaacca gagggaagaa tttgagggca 1500
ccagggagag cattctggtg tggctcacag agatggacct gcagctgacc aacgtggagc 1560
acttctcaga gagtgacgcc gatgacaaga tgcgccaact gaatggcttc caacaggaaa 1620
ttacattaaa taccaacaag attgatcagc tcattgtgtt tggggagcag ctgattcaga 1680
agagcgagcc cctggatgct gtgctgattg aggatgagct ggaggaactc caccgctact 1740
gccaggaggt gtttggaagg gtctcccggt tccaccggcg gctcacctcc tgcactccgg 1800
gcttggaaga tgaaaaggag gcctctgaga atgaaacaga catggaagac cccagagaaa 1860
tccagactga ttcttggcgt aaacggggag agagcgagga accgtcatct cctcagtccc 1920
tgtgtcatct agtggcccca gggcacgagc ggtctggctg cgagacccct gtcagcgtgg 1980
actccatccc cctggagtgg gaccacacag gcgacgtggg gggctcctcc tctcacgaag 2040
aggacgagga gggcccatac tacagcgcac tgtcaggtaa atccatttcg gatggccact 2100
cgtggcatgt tcccgacagc ccttcctgtc ccgagcatca ctacaagcaa atggaaggtg 2160
acaggaatgt tccacctgtt ccccctgcgt ccagcacccc ttataaacca ccctatggaa 2220
agctactatt acctccaggc acggatggtg gcaaagaagg cccgcgagtc ctgaatggca 2280
acccacagca ggaagacggg ggactggccg gtatcacaga gcagcagtca ggtgccttcg 2340
acagatggga gatgattcaa gcacaggagc ttcacaataa gctcaaaata aaacaaaatt 2400
tgcaacagct gaactctgat atcagcgcca tcactacttg gctgaaaaaa actgaagcag 2460
agctggaaat gttaaagatg gcaaagcctc cctctgatat ccaggaaata gaactgagag 2520
tgaagagact gcaggagata ctgaaagcct ttgacactta caaggcatta gtggtctctg 2580
tcaacgtgag cagcaaggaa tttctgcaaa ccgagagccc cgaatccaca gagctccaaa 2640
gtagactccg ccagctgagc ctgctctggg aagcagcaca gggcgcagtg gacagctgga 2700
gagggggctt acgacagtcg ctcatgcagt gccaggactt ccaccagttg agtcaaaatc 2760
tgctgctgtg gttagcgagt gccaagaacc ggaggcagaa ggctcatgtc accgatccaa 2820
aggcagaccc ccgggctctc ctagagtgtc ggagggaact aatgcaactg gaaaaggagc 2880
tggtagaacg tcaacctcaa gtggacatgt tacaggagat ttcaaacagc cttctcatta 2940
agggacatgg agaagactgt attgaagctg aagaaaaggt gcatgttatt gagaagaaac 3000
tcaaacagtt acgggagcaa gtgtcccaag atttaatggc cttgcaggga acccagaacc 3060
cagcctcacc cctgcccagc ttcgacgagg tagactcggg ggaccagcct cctgcaacat 3120
ccgtgccagc tccccgagca aagcagttca gagcagtgag aactacagaa ggcgaggagg 3180
agacagagag cagggtcccc ggcagcacac ggccacagcg ctccttcctc tcaagggtgg 3240
tccgggcagc cctacccctg cagctgctcc tcctgctgct gctgctcctg gcctgcctgc 3300
tgccctcctc cgaagaagac tacagctgca ctcaggccaa caactttgcc cggtcctttt 3360
accccatgct gaggtacacc aatgggccac cccccacata gagggcatag ctggccacag 3420

tgctacacca cctgcctgat tgccaagggt gcccagcacg tggccccaga ccaatctgag 3480
tgacttagtg ttggcaaggt cccgggacct gtgcagactt cttctgggct tacacagcac 3540
gggctccctg gagcccaggg cagctttcag attgtgttcc tccccaggag cagggaacct 3600
gtgtggcagg tgccccgggt actttggcag aactagttga ttagtttagg gatctctgga 3660
aatgtcagtt tcctgaagag ccaagcactt tgtgaattct ggtttgtttg taaaacagca 3720
ttattataat gtaggtatgg tcaatgagca gtggtgtcca tcacatatat tatagaagca 3780
agcgagcaca ttccacccta gaaatggttc agaaactcat aggcaccctt agctgatgga 3840
aacaatcaat catatttaat acgcttagaa tcagttttac tccaatcagc tggcaatttt 3900
gagctgccgg ttatacacca aaatgttctg ttcagtacct agctctgctc ttttatattg 3960
ctttaaattt ttaaagaaat tatattgcat ggatgtggtt atttgtgcat attttttaac 4020
aatgcccaat ctgtatgaat aatgtaaact tcgatttttt tttaaaaaaa ttagatttta 4080
gctggagctt ttgactaatg taaagtaaat gccaaactac cgacttgata gggatgtttt 4140
tgtaagttaa ttttctaaga ctttttcaca tccaaagtga tgctttgctt tgggttttaa 4200
ctgtttggcc acggcggggg tgggggcggg gggttggtac agaaacttga agctgtttgt 4260
gatatgtaca actcagatgt ttctcattaa aaaacaaaat tagccagaaa aaaaaaaaaa 4320
aaaaa 4325

<210> 32
<211> 4248
<212> DNA
<213> Homo sapiens
<220>
<221> misc_feature
<223> séquence appelée GS-N31

<300>
<308> GENBANK/AF261758
<309> 2000-10-10
<313> (1)..(4248)
<400> 32
cccgggctgt gggctacagg cgcagagcgg gccaggcgcg gagctggcgg cagtgacagg 60
aggcgcgaac ccgcagcgct taccgcgcgg cgccgcacca tggagcccgc cgtgtcgctg 120
gccgtgtgcg cgctgctctt cctgctgtgg gtgcgcctga aggggctgga gttcgtgctc 180
atccaccagc gctgggtgtt cgtgtgcctc ttcctcctgc cgctctcgct tatcttcgat 240
atctactact acgtgcgcgc ctgggtggtg ttcaagctca gcagcgctcc gcgcctgcac 300
gagcagcgcg tgcgggacat ccagaagcag gtgcgggaat ggaaggagca gggtagcaag 360
accttcatgt gcacggggcg ccctggctgg ctcactgtct cactacgtgt cgggaagtac 420
aagaagacac acaaaaacat catgatcaac ctgatggaca ttctggaagt ggacaccaag 480
aaacagattg tccgtgtgga gcccttggtg accatgggcc aggtgactgc cctgctgacc 540
tccattggct ggactctccc cgtgttgcct gagcttgatg acctcacagt ggggggcttg 600
atcatgggca caggcatcga gtcatcatcc cacaagtacg gcctgttcca acacatctgc 660
actgcttacg agctggtcct ggctgatggc agctttgtgc gatgcactcc gtccgaaaac 720
tcagacctgt tctatgccgt accctggtcc tgtgggacgc tgggtttcct ggtggccgct 780
gagatccgca tcatccctgc caagaagtac gtcaagctgc gtttcgagcc agtgcggggc 840
ctggaggcta tctgtgccaa gttcacccac gagtcccagc ggcaggagaa ccacttcgtg 900
gaagggctgc tctactccct ggatgaggct gtcattatga caggggtcat gacagatgag 960
gcagagccca gcaagctgaa tagcattggc aattactaca agccgtggtt ctttaagcat 1020
gtggagaact atctgaagac aaaccgagag ggcctggagt acattccctt gagacactac 1080
taccaccgcc acacgcgcag catcttctgg gagctccagg acatcatccc ctttggcaac 1140
aaccccatct tccgctacct ctttggctgg atggtgcctc ccaagatctc cctcctgaag 1200
ctgacccagg gtgagaccct gcgcaagctg tacgagcagc accacgtggt gcaggacatg 1260
ctggtgccca tgaagtgcct gcagcaggcc ctgcacacct tccaaaacga catccacgtc 1320
taccccatct ggctgtgtcc gttcatcctg cccagccagc caggcctagt gcaccccaaa 1380
ggaaatgagg cagagctcta catcgacatt ggagcatatg gggagccgcg tgtgaaacac 1440
tttgaagcca ggtcctgcat gaggcagctg gagaagtttg tccgcagcgt gcatggcttc 1500
cagatgctgt atgccgactg ctacatgaac cgggaggagt tctgggagat gtttgatggc 1560
tccttgtacc acaagctgcg agagaagctg ggttgccagg acgccttccc cgaggtgtac 1620
gacaagatct gcaaggccgc caggcactga gctggagccc gcctggagag acagacacgt 1680
gtgagtggtc aggcatcttc ccttcactca agcttggctg ctttcctaga tccacacttt 1740
caaagagaaa cccctccaga actcccaccc tgacagccca acaccacctt cctcctggct 1800
tccagggggc agcccagtgg aatggaaaga atgtgggatt tggagtcaga caagcctgag 1860
tccagttccc cgtttagaac tcattagctg tgtgactctg ggtgagtccc ttaacccctc 1920
tgagcccggg tctcttcatt agttgaaagg gatagtaata cctacttgca ggttgttgtc 1980
atctgagttg agcactggtc acattgaagg tgctgggtaa gtggtagctc ttgttgcttc 2040
ccgttcagcg tcacatctgc agtggagcct gaaaaggctc cacattaggt cacctgtgca 2100
cagccatggc tggaatgatg aaggggatac gctggagttg ccctgccatc gcctccatca 2160
gccagacgag gtcctcacag gagaaggaca gctcttcccc accctgggat ctcaggaggg 2220
cagccacgga gtggggaggc cccagatgcg ctgtgccaaa gccaggtccg aggccaaagt 2280
tctccctgcc atccttggtg ccgtcctgcc ccttcctcct tcatgcctgg gcctgcaggc 2340
ccaccccagc caccactgag tccactcgga gtgccctgtg ttcctggaga aggcattcca 2400
gggttgaatc ttgtcccagc ctcagcctgg gacacctagg tggagagagt ggtctccgct 2460
ctgaattgga tccaggggac ctgggctcat tcttcttggc tcaccaaccc tgcaggcctc 2520
atctttccca aaacccactt tgtcttggtg ggagtgggtc cgcgctgctc tgcagcaggg 2580

gctggggagt ggacagcatc aggtgggaaa gtggagtcca ccctcatgtt tctgtaggat 2640
tctcaccgtg gggctggaag aaaagagcat cgacttgatt tctccaacca ctcatccctc 2700
tttttctttc ttccaccact ccccacccca gctgtagtta atttcagtgc cttacaaatc 2760
ctaagctcag agaaagttcc atttccgttc cagagggaag ggaacctccc taggtccttc 2820
cctggcttgt tataacgcaa agcttggttg tttatgcaac tctatcttaa gaactgccca 2880
gcctcagctg aaaacccgaa tctgagaagg aattgcgtca tgtaagggaa gctggaatta 2940
agggagctga gccagtcatg gttgtggcgt gtgagtcagg agacctaggt ttcagcccct 3000
ctctactgtc agcgagctgt gcaacgtggg caagtcattg tcctctgagc tgcagtttcc 3060
tcatctgtca catcgctaca gacaagacct ccctggaacc cttctgattg tcttagacac 3120
tgtggttgca aaacccacgg aaagcctcat ttgtgtggaa agtcagagga aaaatgatcc 3180
agtggacact tggggattat ctgtcattca agatccttcc ttcaacccca aggccagctc 3240
ccatctcatt tccagaaagg ctcatacctg gcttgcaggg aagcatctgt cttgtcattc 3300
caggtgccag aatcctctca gagtcattga agggtgttca cccatcccac ccaaggcttg 3360
gcacactgcc agtgtcttag cagggtcttg tgagggctgg gggcatccag gcactcagaa 3420
ggcaaaggaa ccaccctacc catttggcct ctggaggggg cagaagaaag aaagaaacct 3480
catcctatat tttacaaagc atgtgaattc tggcattagc tctcatagga gacccatgtg 3540
cttccttgct cagtgcaaaa ctgatgattc tacttgctgt agatgaatgg ttaacacgag 3600
ctagttaaac agtgccattg ttttgccagt gaagcctcca accctaagcc actgggacgg 3660
tggccagaga tgccagcagc ctctgtcgcc cttagtcata taaccaaaat ccagacctta 3720
tccacaaccc ggggcttgga aaggaaggta ttttggaatc acaccctccg gttatgttgc 3780
tccagtaaaa tcttgcctgg aaagaggcag tcttcttagc atggtgagct gagttcatgg 3840
cttttttttg tagccagtcc tgtccctggc catccatgtg atggttttgg atggagttaa 3900
acttgatgcc agtgggcagt gcatgtggaa agtatcagag taagcctctc ccctccagag 3960
ccctgagttt cttggctgca tgaaggtttt ctttagaatc agaattgtag ccagtttctt 4020
tggccagaag gatgaatact tggatattac tgaaagggag gggtggagat gggtgtggca 4080
gtgtatggtg tgtgattttt attttcttct ttggtcatgg gggccaagga gaaaggcatg 4140
aatcttccct gtcaggctct tacagccaca ggcactgtgt ctactgtctg gaagacatgt 4200
ccccgtggct gtggggccgc tgcttctgtt taaataaaag tggcctgg 4248

<210> 33
<211> 7764
<212> DNA
<213> Homo sapiens
<220>
<221> misc_feature
<223> séquence appelée GS-N32

<300>
<308> GENBANK/NM_001271
<309> 2000-10-31
<313> (1)..(7764)
<400> 33
ccctgggatg gaggatctgt ctctctctct ctctctcctt tttttttttt tggtggagat 60
gaaggggtgg gtctatggta catcacctga gttgtggggt aaatgtagag agtgtcaatc 120
aaaggcagag ctctcagagc tgggaaggag gctctagatg gcggctgtgc cttagagaga 180
gcgcgctctg ctccctgcct ttgcctcact ttacgcaact ttccctaact ttcgggcagc 240
ctcagggggc ccccgtagcc ccctgccttt cctagggact tactggggtc gattcgaacc 300
tttttttggg agaaaagcag cttttaggag ctttcttttc gtgccttgtt ggaaagaagc 360
agccgtactg agagcccagg tcgttgtttt ttccagctta gaagccatgg cgcacctcca 420
tttttgtgcg ctctcctaat gaggtttttt ttctttcgga cctgttttag tattaattat 480
tgctttattt ttttgaccag ttaacatatt tgagggttat tttatttatt tttcgttttt 540
taacggagga ttttgccttt atttttaatt atttgggatc tgatattttt ctactagtag 600
ataggactct tggtttggac atactacatg gatcagtaaa tacctgggca caggacttca 660
aagcaaacac agattccccc tcccccttaa tatttaagaa ttaaaagatg atgagaaata 720
aggacaaaag ccaagaggag gacagttcgc tacacagcaa tgcatcgagt cactcagcct 780
ctgaagaagc ttcgggttca gactcaggca gtcagtcgga aagtgagcag ggaagtgatc 840
caggaagtgg acatggcagc gagtcgaaca gcagctctga atcttctgag agtcagtcgg 900
aatctgagag cgaatcagca ggttccaaat cccagccagt cctcccagaa gccaaagaga 960
agccagcctc taagaaggaa cggatagctg atgtgaagaa gatgtgggaa gaatatcctg 1020
atgtttatgg ggtcaggcgg tcaaaccgaa gcagacaaga accatcgcga tttaatatta 1080
aggaagaggc aagtagcggg tctgagagtg ggagcccaaa aagaagaggc cagaggcagc 1140
tgaaaaaaca agaaaaatgg aaacaggaac cctcagaaga tgaacaggaa caaggcacca 1200
gtgcagagag tgagccagaa caaaaaaaag taaaagccag aagacctgtc cccagaagaa 1260
cagtgcccaa acctcgtgtt aaaaagcagc cgaagactca gcgtggaaag agaaaaaagc 1320
aagattcttc tgatgaggat gatgatgatg acgaagctcc caaaaggcag actcgtcgaa 1380
gagcggctaa aaacgttagt tacaaagaag atgatgactt tgagactgac tcagatgatc 1440
tcattgaaat gactggagaa ggagttgatg aacagcaaga taatagtgaa actattgaaa 1500
aggtcttaga ttcaagactg ggaaagaaag gagccactgg agcatctact actgtatatg 1560
cgattgaagc taatggcgac cctagtggtg actttgacac tgaaaaggat gaaggtgaaa 1620
tccagtacct catcaagtgg aagggttggt cttacatcca cagcacatgg gagagtgaag 1680
aatccttaca gcaacagaaa gtgaagggcc taaaaaaact agagaacttc aagaaaaaag 1740
aggacgaaat caaacaatgg ttagggaaag tttctcctga agatgtagaa tatttcaatt 1800
gccaacagga gctggcttca gagttgaata aacagtatca gatagtagaa agagtaatag 1860

ctgtgaagac aagtaaatct acattgggtc aaacagattt tccagctcat agtcggaagc 1920
cggcaccctc aaatgagccc gaatatctat gtaaatggat gggactcccc tattcagagt 1980
gtagctggga agatgaagcc ctcattggaa agaaattcca gaattgcatt gacagcttcc 2040
acagtaggaa caactcaaaa accatcccaa caagagaatg caaggccctg aagcagagac 2100
cacgatttgt agctttaaag aaacaacctg catatttagg aggggagaat ctggaacttc 2160
gagattatca gctagaaggt ctaaactggc tagctcattc ctggtgcaaa aataatagtg 2220
taatccttgc tgatgaaatg ggcctaggaa agaccatcca gaccatatca ttcctctcct 2280
acctgttcca ccaacaccag ctgtatggcc cctttcttat agtcgtccct ttatccaccc 2340
tcacctcatg gcagagagag tttgaaatct gggcaccaga gattaacgta gtggtttaca 2400
taggtgacct gatgagcaga aatacgatac gggaatatga atggattcat tcccaaacca 2460
aaagattgaa gttcaacgca cttataacaa catatgagat cctcttgaaa gataagactg 2520
tgctgggcag tattaactgg gcctttctgg gagtggatga agcccatcgg ttgaagaatg 2580
atgactcttt attgtataaa actctgattg atttcaagtc caaccatagg ctcctgatta 2640
cggggacccc tcttcagaat tccctcaaag agctctggtc cttgctgcac tttattatgc 2700
cggagaagtt tgaattttgg gaagattttg aagaagacca tgggaagggg agagagaatg 2760
gctaccagag tcttcataag gtgctagagc ctttccttct ccggagagtc aaaaaagatg 2820
tggagaaatc ccttcctgct aaagtggaac agattctcag ggtggagatg tcagcccttc 2880
agaaacagta ttacaagtgg attctgacca ggaattacaa ggctcttgcc aaaggaacaa 2940
gaggcagcac atctggtttt cttaatattg tgatggaact gaaaaaatgt tgcaaccact 3000
gctatctgat taaaccccct gaagaaaatg aaagggaaaa tggacaggag attcttctgt 3060
ccctcataag gagcagtggg aagttgattt tattagacaa actgttgaca agacttcgag 3120
aaagggggaa tcgagtgctt atcttctctc agatggtgag aatgttggat atcctggctg 3180
aatacctaac tattaaacac tatcctttcc agcgtctgga tggttccatc aagggagaaa 3240
tccgaaaaca ggcactggac cacttcaatg cagatgggtc tgaggacttc tgtttcctgc 3300
tctcgacaag ggctggtggc ctgggaatca atttggcttc agcggacaca gtcgtcatct 3360
ttgactctga ctggaacccc cagaatgact tgcaggcaca agcccgagcg catagaattg 3420
gtcagaagaa gcaggtaaat atttaccgct tagttacaaa ggggactgtg gaggaggaga 3480
tcatagaacg ggccaaaaag aagatggtat tagatcatct ggtgattcag cgcatggaca 3540
ccactggccg gacgatcctg gaaaacaact caggaaggtc caactcaaat ccttttaata 3600
aagaagagct gacagctatt ttgaaatttg gagcagagga tctcttcaaa gaactggaag 3660
gggaggaatc agaacctcag gaaatggata tagatgaaat tttgcggttg gctgaaacga 3720
gagagaatga agtgtcaaca agtgcaacag atgaacttct atcacagttt aaggttgcca 3780
actttgcaac aatggaagat gaagaagagc tagaagagcg tcctcacaag gactgggatg 3840
agatcattcc agaggaacaa aggaaaaaag tagaggagga agagcggcag aaggagctag 3900
aagaaattta tatgctgcct cgaattcgga gttccactaa aaaggctcag acaaatgaca 3960
gtgactctga cactgagtct aagaggcagg cccagagatc ctctgcttct gagagtgaaa 4020
cggaagactc tgatgatgac aagaagccaa agcgcagagg gcgtccgagg agtgtgcgga 4080
aggacctcgt ggagggattt actgatgcag agatccgaag gttcatcaag gcttataaga 4140
agtttggtct ccctcttgaa cggctggagt gcttagcacg tgatgctgag ctggtagata 4200
agtcggtggc agatctgaag cgcctgggtg aactgatcca caacagctgt gtgtcagcaa 4260
tgcaggaata tgaagagcag ctgaaagaaa atgccagcga gggaaaagga ccagggaaaa 4320
ggagaggtcc aacaatcaag atatccggag ttcaggttaa tgtgaaatcc attatccaac 4380
atgaagagga gtttgagatg ctgcataaat ctatccctgt ggaccctgaa gaaaaaaaaa 4440
aatactgctt aacctgtcgt gtcaaagctg cacattttga tgtagagtgg ggggtggaag 4500
atgattctcg cctgttgctg gggatttatg aacatggcta tggaaactgg gagttaatta 4560
aaacagaccc agagcttaaa ttaactgaca aaattctgcc ggtggagaca gataaaaagc 4620
ctcaggggaa gcagctacag acccgagcgg attacttgtt gaagctgctc agaaagggtc 4680
tggagaagaa gggggctgtg acaggtgggg aggaggccaa attaaagaag cggaagcctc 4740
gggtaaagaa ggaaaacaaa gtgcccaggc tgaaagagga gcatggaatt gagctttcat 4800
ctcctaggca ttcagataat ccatcagaag agggagaagt gaaagatgat ggcttggaaa 4860
aaagtccaat gaaaaaaaaa cagaagaaga aagagaacaa ggagaacaag gagaaacaaa 4920
tgagttctag gaaagacaaa gaaggggaca aggaaagaaa gaagtcaaaa gataagaaag 4980
agaagcctaa aagtggtgat gccaaatctt cgagtaaatc aaagcgatct cagggtcctg 5040
tccatattac agcaggaagt gaacctgtcc ccattggaga ggatgaggat gatgatctgg 5100
accaggagac attcagcata tgtaaggaga ggatgaggcc cgtgaaaaag gcactgaaac 5160
agctcgacaa acctgacaag gggctcaacg tgcaagaaca gctggaacac acccggaact 5220
gcctgctgaa aatcggagac cggatagccg agtgccttaa agcctactca gatcaggagc 5280
acatcaaact ctggaggagg aacctatgga tttttgtttc caagtttaca gaatttgatg 5340
ctcgaaaact gcataagtta tacaagatgg ctcataagaa aaggtctcaa gaagaagagg 5400
agcaaaagaa gaaagacgac gtgactgggg gtaagaaacc atttcgtcca gaggcctcag 5460
gctccagccg ggactctctg atatctcagt cccatacctc acacaacctt caccctcaga 5520
agcctcattt gcctgcctcc catggcccac agatgcatgg acacccaaga gataactaca 5580
atcaccccaa caagagacac ttcagtaatg cagatcgagg agactggcag agggaaagaa 5640
agttcaacta tggtggtggc aacaacaatc caccatgggg aagcgacagg caccatcagt 5700
atgagcagca ctggtacaag gaccaccatt atggggaccg gcgacatatg gatgcccacc 5760
gttccggaag ctatcgaccc aacaacatgt ccagaaagag gccttatgac cagtacagca 5820
gtgaccgaga ccaccgggga cacagagatt attatgacag gtatgcaaaa ggctgtgaga 5880
caccaggtgc caacctttgc caggagctgt ttctagggag aaagtgacgt atacatgaat 5940
gtatttatct atcaaattac tgaagatctc atcatgcatg tgtcagccac agcgaatccc 6000
atgtcttggt tataggtttt atgttttgtt ttctgggtca tagggagcac atttcacctg 6060
tgcaggaaaa gagttttctg ccgtcttttg aggaaatcta gtgaagaggt cgccataaaa 6120
tattagagtc aacaaccaaa attattaagc tctgtgcgag gctgtcagcc acactaggta 6180
tcagggatcc cgagatgggt accagcccac agtccttacc tgccacgagc ccataattga 6240

agagtcaaag tcttctgaag ctgcaccctc tttacttcag tacaatgcca ccagtagtac 6300
gatgagccaa agctttacat tgtgagagta gcaagtccag ggagagctaa agaggtttta 6360
tctgtatttc ctaatttcaa atcttggata atttaacctc atagcagctt tggttttccc 6420
tgggctgatg atgtgcgtca tttgcactgt accttgaatt tacagtggga aaatttcata 6480
taaacgtgtc aaagtcgtgc tttgtttttg gaagatctgg taacagcagc ccgcattagc 6540
agagagctgt agctgagtag ctgccacctc gttgggagac tgcccctcgc tcccaccctt 6600
ctctattgtc tggacccagt gggcatcttg ccctgcgttc ttctagtagg tctgtatttc 6660
tatttgatgt cactttcctt ttgcctgaag gactttttct gctggtgata aactctttca 6720
gtgtttgtat atatgcctga aaaagtattt tgccttcatt tttgaaagta gtttttgctg 6780
agtgtataca tttttggctt tacagtttct ttcagtgctt taaagatgta cctctgctat 6840
ttacttgcat tgttttgtga tgaaaaatct gtcatcctta tctttgttcc tctttacata 6900
atgttccttt taaaaaaaat cactgattat gatgtgcctt ggtgtatttt tccttggttt 6960
cttgtgcttg gaaatttttg aacttcttgg atctgtgggt ttattgtttc cataaaattt 7020
ggaaattttt acaatcttct tcaaatattt tttctgatcc cccactctct cttcttcttt 7080
ggagattctc attacaccta tattagcttg cttgaagttg tctcacagct cacttgtatt 7140
ctgttgactt ttaaaaaatt atgctttctg tttcactgtg gatagtttct attgctacct 7200
cttcaagttc actaatactt tccttttcaa tgtcaagact gctgtgaggc ccatccagtg 7260
tactttgcat tttatacatt gtagttctaa aagttcggaa agttgttttt gggtcttttt 7320
atatatgttc tgtgtctaac cttttaaaac ctggaacaca gatataacaa tggttttgat 7380
gtccttgtct gcgaatctta tcacttgggt cagtttcagt tgatacctcc tcactgtggg 7440
tcttgctccc ctggtgcttt ctgtgcctag taatttttgt cagatgccag atgtaacatt 7500
taccttgttg ggtgctggat atttctgtat tcctgtaagt attctggagc tttgttatga 7560
gttgcaggtt atttggaagc agtttccttt ttcaggtctt gctgttaaga ttcgttaggt 7620
agaaccagag cagtgctcag tcaagggcta atgattgccc acccccaagg taaagagcct 7680
cattgcactc tacccaattg cgttagtctg ttttgcagga atacctgagg ctgggtaatt 7740
tatagagaaa agagttttat ttgg 7764

<210> 34
<211> 4693
<212> DNA
<213> Homo sapiens
<220>
<221> misc_feature
<223> séquence appelée GS-N33

<300>
<308> GENBANK/XM_042555
<309> 2001-10-16
<313> (1)..(4693)
<400> 34
ggactgcggg ataggaagct ggggatatgg acaagcagca gcgttatagc gctctgggtt 60
tcgggacata ggcctgggcc atgcggcccc cttggcccct tggcgcgacc cccaggaacg 120
ttcggaaagc tggtcctcgt ggctggggga aaggcggggg gtggggggga agcgggcacg 180
tgaccccggt cagccaatct gggtgctgct gacgtacgcg cgcggccccg atgctctccc 240
caccccccca gcccgttcgg gaagggaggg gctgggggct acgccccatc ccccagcacg 300
gcttcgtttt ctgggggggg gttgacaccc cggattacat accccgtacc aagccgaggg 360
caactttgga ggccccctgg aaggctttag gatccagatt cttcgctgct gctgccttac 420
cgccgagaac caccacccgc caggcgtctt gcggccacac ccctggcggg ttcaggcagg 480
ctacgcccac gcgacccctc ccgtttccct gctttggcca atggaggagc tacgaatggc 540
acgacctgct cgagcttggc agtctccagt tgggctgtgc atggaagctt gggaagactt 600
tgttggaagg ggaggcgggg agagagtgct ggaggctctg gggcgatggc ttccgcacct 660
cttccaacca ccctctttcc ctggagtcgg cggaccacag ctcagccaat tggcttggag 720
atgtggcggg ttgccacttc cctgtgggtc tctgcggcac tcttctgcct ggtgactgac 780
accttggaaa tgaagtttat gacgtcatcg ttgcggctgg ccaatagaaa aagctcccgc 840
ggagaggtgt tccttcccct tcgactcagc ttcttcaccc gcgtgagcga gcgcgcgcgc 900
gcggaggggg tggggaaaat ctcaagcagg gtggcgcgca tgagcggcga agctcctcct 960
ccccgcctat atataaaggg ctggcgcggg gctcggcggc gccatttcgt gctggagtgg 1020
agcagcctct agaacgagct ggaggattct gcctaccgat acagagcctt cgagtcgtcc 1080
ggggccgcca ttacaatcca cctccatccg cttggaaatg gccttcgtcc cggcctatga 1140
ctggtcccag cgggcagtac agacccccta gaagcccctg gagctcccct ttttcgggcc 1200
ccgcccaatc ctcggagtct gtccaccccc tctactccgc cctcaagagg atttcaaaga 1260
tggaggcggc ggctccctaa accacttttc gtgttcatcc gcctccatcc gagatcgaaa 1320
cgggacctcg tcggccccgt aggggcccga caagaagagg gaatccctgc agaccaacag 1380
cgggctatat tgacgacggt gtctgagatc ggggaccgtc ttttgaagag tcagtccctc 1440
cttagttgcc cgcctcagct gaggccgccg ccattttctt gctgtccgcc gtctgcagag 1500
cgcgccaagc tgcccggagc tctccgagag gccccaaaga gactgctttc gtgccggcca 1560
ggcagggggt ttgtcgcctg gaggcccaag aggaacggcc tccccccaac ttagcgggtt 1620
atgctggacc gggcggtgag gggaaccgag gccacccgga ctttccgcgg ctgagggcag 1680
cgccggttcc ttgcggtcaa gatgctgcaa aacgtgactc cccacaataa gctccctggg 1740
gaagggaatg cagggttgct ggggctgggc ccagaagcag cagcaccagg gaaaaggatt 1800
cgaaaaccct ctctcttgta tgagggcttt gagagcccca caatggcttc ggtgcctgct 1860
ttgcaactta cccctgccaa cccaccaccc ccggaggtgt ccaatcccaa aaagccagga 1920
cgagttacca accagctgca atacctacac aaggtagtga tgaaggctct gtggaaacat 1980

cagttcgcat ggccattccg gcagcctgtg gatgctgtca aactgggtct accggattat 2040
cacaaaatta taaaacagcc tatggacatg ggtactatta agaggagact tgaaaacaat 2100
tattattggg ctgcttcaga gtgtatgcaa gattttaata ccatgttcac caactgttac 2160
atttacaaca agcccactga tgatattgtc ctaatggcac aaacgctgga aaagatattc 2220
ctacagaagg ttgcatcaat gccacaagaa gaacaagagc tggtagtgac catccctaag 2280
aacagccaca agaagggggc caagttggca gcgctccagg gcagtgttac cagtgcccat 2340
caggtgcctg ccgtctcttc tgtgtcacac acagccctgt atactcctcc acctgagata 2400
cctaccactg tcttcaacat tccccaccca tcagtcattt cctctccact tctcaagtcc 2460
ttgcactctg ctggaccccc gctccttgct gttactgcag ctcctccagc ccagcccctt 2520
gccaagaaaa aaggcgtaaa gcggaaagca gatactacca cccctacacc tacagccatc 2580
ttggctcctg gttctccagc tagccctcct gggagtcttg agcctaaggc agcacggctt 2640
ccccctatgc gtagagagag tggtcgcccc atcaagcccc cacgcaaaga cttgcctgac 2700
tctcagcaac aacaccagag ctctaagaaa ggaaagcttt cagaacagtt aaaacattgc 2760
aatggcattt tgaaggagtt actctctaag aagcatgctg cctatgcttg gcctttctat 2820
aaaccagtgg atgcttctgc acttggcctg catgactacc atgacatcat taagcacccc 2880
atggacctca gcactgtcaa gcggaagatg gagaaccgtg attaccggga tgcacaggag 2940
tttgctgctg atgtacggct tatgttctcc aactgctata agtacaatcc cccagatcac 3000
gatgttgtgg caatggcacg aaagctacag gatgtatttg agttccgtta tgccaagatg 3060
ccagatgaac cactagaacc agggccttta ccagtctcta ctgccatgcc ccctggcttg 3120
gccaaatcgt cttcagagtc ctccagtgag gaaagtagca gtgagagctc ctctgaggaa 3180
gaggaggagg aagatgagga ggacgaggag gaagaagaga gtgaaagctc agactcagag 3240
gaagaaaggg ctcatcgctt agcagaacta caggaacagc ttcgggcagt acatgaacaa 3300
ctggctgctc tgtcccaggg tccaatatcc aagcccaaga ggaaaagaga gaaaaaagag 3360
aaaaagaaga aacggaaggc agagaagcat cgaggccgag ctggggccga tgaagatgac 3420
aaggggccta gggcaccccg cccacctcaa cctaagaagt ccaagaaagc aagtggcagt 3480
gggggtggca gtgctgcttt aggcccttcc ggctttggac cttctggagg aagtggcacc 3540
aagctcccca aaaaggccac aaagacagcc ccacctgccc tgcctacagg ttatgattca 3600
gaggaggagg aagagagcag gcccatgagt tacgatgaga agcggcagct gagcctggac 3660
atcaacaaat tacctgggga gaagctgggc cgagttgtgc atataatcca agccagggag 3720
ccctctttac gtgattcaaa cccagaagag attgagattg attttgaaac actcaagcca 3780
tccacactta gagagcttga gcgctatgtc ctttcctgcc tacgtaagaa accccggaag 3840
ccctacacca ttaagaagcc tgtgggaaag acaaaggagg aactggcttt ggagaaaaag 3900
cgggaattag aaaagcggtt acaagatgtc agcggacagc tcaattctac taaaaagccc 3960
cccaagaaag cgaatgagaa aacagagtca tcctctgcac agcaagtagc agtgtcacgc 4020
cttagcgctt ccagctccag ctcagattcc agctcctcct cttcctcgtc gtcgtcttca 4080
gacaccagtg attcagactc aggctaaggg gtcaggccag atggggcagg aaggctccgc 4140
aggaccggac ccctagacca ccctgcccca cctgcccctt ccccctttgc tgtgacactt 4200
cttcatctca cccccccctg cccccctcta ggagagctgg ctctgcagtg ggggagggat 4260
gcagggacat ttactgaagg agggacatgg acaaaacaac attgaattcc cagccccatt 4320
ggggagtgat ctcttggaca cagagccccc attcaaaatg gggcagggca agggtgggag 4380
tgtgcaaagc cctgatctgg agttacctga ggccatagct gccctattca cttctaaggg 4440
ccctgttttg agattgtttg ttctaattta ttttaagcta ggtaaggctg gggggagggt 4500
ggggccgtgg tcccctcagc ctccatgggg agggaagaag ggggagctct ttttttacgt 4560
tgattttttt ttttctactc tgttttccct ttttccttcc gctccatttg gggccctggg 4620
ggtttcagtc atctccccat ttggtcccct ggactgtctt tgttgattct aacttgtaaa 4680
taaagaaaat att 4693

<210> 35
<211> 2983
<212> DNA
<213> Homo sapiens
<220>
<221> misc_feature
<223> séquence appelée GS-N34

<300>
<308> GENBANK/BC007429
<309> 2001-07-12
<313> (1)..(2983)
<400> 35
ggcacgaggg cgcggcggag gctcccgtgg cctcggacgc tcctcctagc tagcggccgc 60
cgcccgccgc cgcctgcgcc tccagctcct tcgccccggc gggcccggcc gccgcttccg 120
gcagctcacc tgggaagcgc tcacctggga cgcgctcacc tgggacgcgc tacctgcctc 180
cgggcgcctg ggcttcagga tgaaggaccg tctggagcag ctgaaggcca agcagctgac 240
acaggatgat gatactgatg cggttgagat tgctatcgac aacacggctt ttatggacga 300
gttcttttct gagattgagg aaactcggct taacattgac aagatctcag aacatgtaga 360
ggaggctaag aaactctaca gtatcattct ctctgcaccg attccagagc caaaaaccaa 420
ggatgaccta gagcagctca cgactgagat taagaaaagg gccaacaacg tccggaacaa 480
actgaagagc atggagaagc atattgaaga agatgaggtc aggtcatcgg cagaccttcg 540
gattcggaaa tcccagcact ctgtcctttc tcggaagttt gtggaggtga tgaccaaata 600
caatgaagct caagtggact tccgagaacg cagcaaaggg cgaatccagc ggcagctcga 660
aattactggc aaaaagacaa ccgatgagga gctggaggag atgttggaga gtggcaaccc 720
ggccatcttc acttctggga tcattgactc acagatttcc aagcaagccc tcagtgagat 780

tgagggacga cacaaggaca ttgtgaggct ggagagcagc atcaaggagc ttcacgacat 840
gtttatggac atcgccatgc tggtggagaa tcagggtgag atgttagata acatagagtt 900
gaatgtcatg cacacagtgg accacgtgga gaaggcacga gatgaaacga aaaaagctgt 960
gaaataccag agtcaggccc ggaagaaatt gataattatc attgtgctag tagttgtgtt 1020
gctgggcatt ttagcattga ttattggact ttccgttggg ctgaattaag agtggcctaa 1080
gaggctgctg cactgaaata aactgatttc actccagact ggtgtggcca cccttgtctt 1140
cagatgagaa tggagtctga atggccttcc tgagagcgag tgcgacccgt tcctttgttt 1200
ccttgcaacc acccttggac ctgactcagc taacaatcta gccctggggg aatgtgatct 1260
acctgatgcg accctgagtt ctccccagag cctcctcctg ccccaccagc tctcaagtac 1320
cttttctcct ggactgtgtg gacccaccca gctttcttcc tccctgttgt gtgtcagatt 1380
atgccttgca cttgggaaag ctcttgtgag actctcccaa ggtgctgtat ttttctacct 1440
catggagtat tctcccagaa actgcaatgt atttttttag gggagtatct ttaacaaagc 1500
agaatgattc ttctaagttt ggcaacaaga aggcttggat ctgagtcttc tacctggcag 1560
gatgccaatc ctgtttgttg tccgtatgtc ctgaaaacat gagggactgg cagatgtcat 1620
tttggtctaa agagctgact tgtttgaaat tcagccttaa attaagctct tagttgttca 1680
gcttgggggg caactttgat ttttctctgt gttgtagtct ctcatattta ctcaaggagg 1740
gaccaggatg atacagtcat ctgaggttat gctttgcaaa aggctgacgg tatggaatat 1800
gtttccatgt ctgagtctta gaaactggct gctcattgtt agaaagtgat gctttgtgag 1860
actattgtct tggggccaaa aataatcagg gattttaaat tgggcaaggg acaaggtgct 1920
agaatcctaa gctctggaaa tatttcatga cactggtgta ttcactcatg tgttccagat 1980
gtattctaat tgtgtatgaa atgtatgtac acataagtgt gtgtgtctca ggaagtagga 2040
aataaaaatg gaagctatta tgacctcaaa aaaaaaaagc caactttgag ctaggataaa 2100
aattgggtaa aggacatttg cttacctgca aatgaatcac tgtggaaatg tgatcttccc 2160
atatcatcaa gaaacttgtt ttctggatga atactgggag aataaaatga gaactctgga 2220
gtgagctaaa ttgatcccaa ttaagttttt ctgcttagca gacagaaggt ataatttttt 2280
gacacccttt cccacctggt gcctatgcta ggcttgtcct gagaacatcc ctcagtaact 2340
tgatattcac atgacctaca ggatgtccca tctgcagggc tgagtcagtt ggggaacacc 2400
agaggctaca cagtagctct tcctgctact cggttaatga gcttggcagg ttctttgtct 2460
cactgaattc ttatcatgga aacagcagca gcagccgcta ggaaatcttc aagtgtagtg 2520
tctgtgctaa cccagtggta aatcccttag atcccctgct ggtctctggc agtctccttg 2580
attttgggta ccatgtatat tttccgcttt gactttaacg ctttctagga tagggtaagc 2640
acccttaatt caggcactgt ccattagctt cctttgcaaa ggctacttat ggccggtcac 2700
aatccagcac tcagacagag ccaaggcaat atcctcttgc ccatggctat gatgtcagac 2760
agtggatggg ctccagcaac aagagacaaa ataactaaag gcctttgctc tcctctgaca 2820
ttgaggcctg gggcttacag tttggaatac aacatgtgaa ggtttttgtt gttgtttgta 2880
ttttttagat gtaaacttga ttattttatt gctaatttaa aaataaaaat gactttgtat 2940
tgattgtgaa aaaaaaaaaa aaaaaaaaaa aaaaaaaaaa aaa 2983

<210> 36
<211> 12227
<212> DNA
<213> Homo sapiens
<220>
<221> misc_feature
<223> séquence appelée GS-N35

<300>
<308> GENBANK/NM_015001
<309> 2001-07-12
<313> (1)..(12227)
<400> 36
tgagctgcga ggcccgagag tcagaacctg ggggagaggg atggtctctg cacggggggg 60
agccggagga gccgccgccg ctgccgacgc caccgccgca gccgccgccg ccgccgcccc 120
ggcacccgcc tcccggcgct gacggtctcg tacgaagccg gcgaggggga gccagcagcg 180
gcggtcgccg gcacgccgcc cagcatggtc cgggaaacca ggcatctctg ggtgggcaac 240
ttacccgaga acgtgcggga agagaagatc atcgagcatt tcaaacgata tggccgcgtg 300
gaaagtgtca aaattcttcc caagagggga tctgaaggag gagtggctgc ctttgtggat 360
tttgtggaca tcaaaagtgc acagaaagct cacaactcgg tcaacaaaat gggtgacaga 420
gacctacgca cggattataa tgaaccaggc accatcccga gtgctgctcg gggattggat 480
gatacagttt ccatagcatc tcgtagtaga gaggtttctg ggttcagagg aggtggtgga 540
gggcctgctt atggtccccc accgtcactt catgcacgag aaggacgtta tgagcggaga 600
cttgatgggg cttcagataa cagggagcgt gcttatgaac atagtgccta tggacaccat 660
gaacggggga cgggaggatt tgatcggaca agacattacg atcaggatta ctatagagat 720
cctcgagagc ggactttaca acatgggctc tattacgctt ctcggagtcg aagtccaaat 780
cgctttgatg ctcatgaccc ccgatatgaa cctagggctc gcgagcagtt tacactgccc 840
agtgtggtac acagggatat ctacagggat gatattaccc gggaggtacg aggcagaagg 900
ccagagcgga attaccagca cagcaggagt cggtcaccac attcatccca gtctagaaat 960
cagtctcctc agagactggc tagccaagca tctagaccca caaggtcccc tagcggcagc 1020
ggctctagaa gtagatcctc cagtagtgat tcaatcagca gcagcagtag taccagcagt 1080
gacagcagtg attccagcag tagttcaagt gatgattctc cagctcgatc agttcagtct 1140
gcagcagtcc ctgcacccac ttcccagttg ctttcatctc tggaaaaaga tgagccccgt 1200
aaaagttttg gcatcaaggt ccagaatctt ccagtacgct ctacagatac aagccttaaa 1260
gatggccttt tccatgaatt taagaaattt ggaaaagtaa cttcagtgca gatacatgga 1320

acttcagaag agaggtatgg tctggtattc tttcggcagc aagaggacca agaaaaagcc 1380
ttgactgcat caaaaggaaa acttttcttt ggcatgcaga ttgaagtaac agcatggata 1440
ggtccagaaa cagaaagtga aaatgaattt cgccccttgg atgaaaggat agatgaattt 1500
caccccaaag caacaagaac tctctttatt ggcaaccttg aaaaaaccac tacttaccat 1560
gaccttcgca acatcttcca gcgctttgga gaaattgtgg atattgacat taagaaagta 1620
aatggagttc ctcagtatgc gtttctgcaa tactgtgata ttgctagcgt ttgtaaagct 1680
attaagaaga tggatgggga atatcttgga aataatcgcc tcaagctggg ttttggaaag 1740
agcatgccta caaactgcgt gtggctagat gggctttctt cgaatgtgtc agatcagtat 1800
ttaacacgac atttctgccg atatgggcct gtggtaaagg tggtgtttga ccgcttaaaa 1860
ggcatggccc tggttctcta caatgaaatt gaatatgcac aagcagctgt aaaagagacc 1920
aaagggagga aaatcggtgg gaataaaatt aaggtggatt ttgcaaatcg ggaaagtcag 1980
ctggcttttt atcactgcat ggagaaatct ggtcaagaca tcagagactt ttatgaaatg 2040
ttagccgaaa gaagagagga acgaagggca tcctacgact ataaccaaga tcgtacatat 2100
tatgagagtg ttcgaactcc aggcacttat cctgaggatt ccaggcggga ctatccagct 2160
cgagggagag agttttattc agaatgggaa acttaccaag gagactacta tgaatcacga 2220
tactacgatg atcctcggga atacagggat tacaggaatg atccttatga acaagatatt 2280
agggaatata gttacaggca aagggaacga gaaagagaac gtgaaagatt tgagtctgac 2340
cgggacagag accatgagag gaggccgatt gaacgaagtc aaagtcctgt tcacttgcga 2400
cgtccacaga gtcctggagc gtctccctct caggcagaga ggttgccgag tgattctgag 2460
aggaggcttt acagccgatc ctcagaccgg agtggaagct gtagctcact ctcccctcca 2520
agatatgaga aactggacaa gtctcgtttg gagcgctata caaaaaatga aaagacagat 2580
aaagaacgaa cttttgatcc ggagagagtg gagagagaga gacgcttaat acggaaggaa 2640
aaagtggaaa aggacaaaac tgacaagcag aaacgcaaag gaaaggttca ctcccctagt 2700
tctcagtctt cagaaacgga ccaagaaaat gagcgagagc aaagccctga aaagcccagg 2760
agttgtaata aactgagcag agagaaagct gacaaagagg gaatagcgaa aaaccgcctg 2820
gaactcatgc cttgcgtggt tttgactcga gtgaaagaga aagagggaaa ggtcattgac 2880
cacactcctg tggaaaagtt gaaagccaag cttgataatg acactgtcaa atcttctgcc 2940
ctggaccaga aacttcaggt ctctcagacg gagcctgcaa aatctgactt gtctaaactg 3000
gaatcagtta gaatgaaagt accaaaggaa aaggggcttt caagccatgt tgaagtggtg 3060
gagaaggaag gcaggcttaa agccaggaag cacctcaagc ctgagcagcc tgcagatggg 3120
gtaagtgctg tggatctgga gaagctggaa gccaggaaaa ggcgctttgc agattccaat 3180
ttaaaagcag aaaagcaaaa accagaggtc aagaaaagca gtccagagat ggaggatgct 3240
cgcgtgcttt caaaaaagca gcctgacgtg tcctctagag aggtcattct gctgagggaa 3300
ggagaggctg aaagaaagcc tgtgaggaaa gaaattctta aaagagaatc taaaaaaatc 3360
aaactggaca gacttaatac tgttgccagc cccaaagact gtcaggagct tgccagtatt 3420
tctgttgggt ctggctcaag gcccagctca gacctacaag caagactggg agaactagca 3480
ggtgaatctg tggaaaatca agaagtccaa tcaaaaaagc ccattccctc aaaaccacag 3540
ctcaaacagc tgcaggtatt agatgatcaa ggaccagaga gagaagacgt taggaaaaac 3600
tattgcagtc ttcgtgatga aacacctgaa cgtaaatcag gccaagagaa atcacattca 3660
gtaaatactg aagaaaaaat tggcattgac atcgatcaca cgcagagtta ccgaaaacaa 3720
atggaacaga gtcgtaggaa acagcagatg gaaatggaaa tagccaagtc tgagaagttt 3780
ggcagtccta aaaaagatgt agatgaatat gaaagacgta gcctcgttca cgaggtaggc 3840
aaaccccctc aagatgtcac tgatgactct cctcctagca aaaagaaaag gatggatcat 3900
gtcgattttg atatctgcac caagcgagaa cggaattaca gaagttcacg ccaaatcagc 3960
gaagattctg aaaggactgg tggttctccc agtgtccgac atggttcctt ccatgaagat 4020
gaggatccca taggctcccc taggctactg tcagtaaaag ggtctcctaa agtagatgaa 4080
aaagtcctcc cctattctaa cataacagtc agggaagagt ctttaaaatt taatccttat 4140
gattctagca ggagagaaca gatggcagat atggccaaaa taaaactatc tgtcttgaat 4200
tctgaagatg aactaaatcg ttgggactct cagatgaaac aggatgctgg cagatttgat 4260
gtgagtttcc caaacagcat aattaagaga gatagccttc gaaaaaggtc tgtacgagat 4320
ctggaacctg gtgaggtgcc ttctgattct gacgaagatg gtgaacacaa atcccactca 4380
cccagagcct ctgcattata tgaaagttct cgattgtctt ttttattgag ggacagagaa 4440
gacaagctac gtgagcgaga tgaaagactc tctagttctt tagaaaggaa caaattttac 4500
tcttttgcat tggataagac aatcacacca gacactaaag ctttgcttga aagagctaaa 4560
tccctctctt catctcgtga agaaaattgg tcttttcttg attgggactc ccgatttgca 4620
aattttcgaa acaacaaaga taaagaaaag gttgactctg ctccaagacc tattccatcc 4680
tggtacatga aaaagaagaa aattaggact gattcagaag ggaaaatgga tgataagaaa 4740
gaggaccata aagaagaaga gcaagagagg caggaattgt ttgcttctcg ttttttacac 4800
agctcaatct ttgaacaaga ttccaagcga ttgcagcatc tagagagaaa agaggaagat 4860
tctgacttca tttctggtag gatctatggg aagcagacat ctgagggagc aaacagcaca 4920
actgattcca ttcaagaacc agtagttctg ttccatagca gatttatgga gctcacacgg 4980
atgcaacaga aagaaaaaga aaaagaccag aaacccaaag aggttgagaa acaggaagat 5040
acagagaatc atcccaagac cccagaatct gctcctgaga ataaagattc agaactgaaa 5100
actccacctt ccgttgggcc tccaagtgtc acagtcgtaa ctctagaatc agccccatca 5160
gcactagaga agaccactgg tgacaaaacg gtagaggcgc ctttggtaac agaagagaag 5220
actgtggagc cagctaccgt ctcagaagaa gcaaagcctg catctgaacc tgctcctgcc 5280
cctgtggaac agctggaaca agtagacctg cccccaggag cagaccccga taaagaagct 5340
gccatgatgc ctgcgggtgt tgaggaaggt tcatcaggtg accagccgcc ttatctggat 5400
gccaagcctc caactcccgg ggcctcgttt tcccaggcag agagcaacgt agatccagag 5460
cctgacagta cccagccact ttcaaaacca gctcagaagt ctgaggaagc caatgagcca 5520
aaggccgaaa agccagacgc cactgcagat gctgagcctg atgcaaacca gaaagccgaa 5580
gctgctcctg agtctcagcc cccagcttct gaagatttag aggttgatcc tccagttgct 5640
gcaaaggata aaaagccaaa caaaagcaag cgttcaaaga cccctgttca ggcagctgca 5700

gtgagtatcg tggagaagcc cgtcacaagg aagagtgaga ggatagaccg ggaaaaactc 5760
aagcggtcca attctcctcg gggagaagca cagaagcttt tggaattgaa gatggaggca 5820
gagaagatta caaggactgc ttctaaaaac tctgctgcag accttgaaca tcccgaacca 5880
agtttgcctc tcagccgaac aaggcgccgg aatgtaagga gcgtctatgc aaccatgggt 5940
gaccatgaaa accgctctcc tgtcaaagag cccgttgagc aaccaagagt gaccagaaag 6000
agattggagc gagagcttca ggaggctgca gcggttccca ccacccctcg gaggggaagg 6060
cctccaaaga cacgccggcg agccgatgaa gaggaggaga acgaggccaa ggaacctgca 6120
gaaacactca agccacctga gggatggcgg tcgccaaggt cccagaaaac tgcagctggt 6180
ggtggacccc aagggaaaaa gggaaaaaat gaaccgaagg tggatgctac acgtcctgag 6240
gccaccactg aggtgggccc ccaaataggc gtgaaagaga gctccatgga acccaaggct 6300
gctgaggagg aggcagggag tgaacagaaa cgtgacagaa aagatgctgg cacagacaaa 6360
aacccccctg aaaccgcccc tgttgaagtt gtagagaaaa aaccggcccc tgaaaaaaac 6420
tccaaatcaa agagaggaag atctcgaaac tccaggttag cagtggacaa atctgcaagt 6480
ctgaaaaatg tggatgctgc tgtcagtccc aggggggctg cagcacaggc aggggagagg 6540
gaatctgggg tggtggcagt ctcccctgag aaaagtgaga gtccccaaaa ggaggatggt 6600
ttatcatccc agttgaaaag tgatccagtt gatccagaca aggaaccaga gaaagaagac 6660
gtgtctgcct ctgggccgtc cccagaagcc acccagttag ccaagcagat ggagctggag 6720
caggccgtgg aacacatcgc aaagctcgct gaggcctctg cctctgctgc ctataaggca 6780
gatgcaccag agggccttgc cccagaggac agggacaagc ctgcacacca agcaagtgaa 6840
acagagctgg ctgcggccat cggctccatc atcaatgaca tttctgggga gccagaaaac 6900
ttcccagcac ctccacctta tcctggagaa tcccagacag atctgcaacc ccccgcaggt 6960
gcacaggcgc tgcagccttc tgaggaagga atggagacag atgaggctgt atctggcatc 7020
ctggaaactg aggctgctac agaatcttct aggcctccag tcaatgctcc tgacccctca 7080
gccggcccaa cagataccaa ggaagccaga ggaaatagca gtgaaacctc acactcagtg 7140
ccagaagcca aagggtctaa agaagtggaa gtcactcttg ttcggaaaga caaagggcgc 7200
cagaaaacaa cccgatcacg ccgcaagcga aacacaaaca agaaagtggt ggctcctgta 7260
gagagccatg tccctgaatc caaccaagct caaggtgaga gtcctgctgc aaatgagggg 7320
acaacagtac agcaccccga agccccacag gaagaaaagc agagtgagaa accccattcc 7380
actcctcctc agtcatgtac ttctgaccta agcaagattc cctccacaga gaattcgtcc 7440
caagaaatca gtgttgagga aaggactcca accaaagcat ctgtgccccc agaccttccc 7500
ccacctcccc agccagcacc ggtggatgag gagcctcaag ccaggttcag ggtgcattcc 7560
atcattgaaa gtgacccggt gaccccaccc agcgatccaa gcatccccat acccacactg 7620
ccttctgtaa ctgcagcaaa gctctcacct cctgtcgcct ctggggggat cccacaccag 7680
agccccccta ctaaggtgac agagtggatc acaaggcagg aggagccacg ggctcagtct 7740
actccatctc cagctcttcc cccagacaca aaggcctctg atgttgacac cagctccagc 7800
accctgagga agattctcat ggaccccaag tatgtgtctg ccacaagtgt cacttccaca 7860
agtgtcacca cagccattgc agagcctgtc agtgctgccc cttgcctaca tgaggccccg 7920
cccccgccag ttgactctaa aaagccttta gaagaaaaaa cagcacctcc agtgacaaac 7980
aactctgaga tacaagcctc ggaggtgctg gtagctgctg acaaggaaaa ggtggctcca 8040
gtcattgctc ccaaaattac ctctgttatt agccggatgc ctgtcagcat tgacctggaa 8100
aattcacaga agataacctt ggcaaaacca gctcctcaaa ccctcactgg tctggtgagc 8160
gcactcactg gcctggtgaa cgtctccctg gtcccggtga atgccctgaa aggccccgtg 8220
aagggctcag tgaccacact gaaaagtttg gtgagcaccc ctgctgggcc cgtgaacgtc 8280
ctgaaagggc ctgtgaatgt tcttacgggg ccagtgaatg ttctcaccac tccagtgaac 8340
gccacggtgg gcacagtgaa tgccgcccca ggcacagtca atgccgctgc gagtgcagtg 8400
aatgccacag caagtgcagt gaccgtcaca gcgggtgcgg ttactgctgc atctggtggt 8460
gtaacggcca caacaggcac ggtgacaatg gcaggggcag tgattgcgcc gtcaacaaag 8520
tgcaaacaga gagcgagtgc taatgaaaac agtcggttcc acccagggtc catgcctgtg 8580
atcgacgatc gtccggcaga cgcgggctca ggggcggggc tgcgtgtgaa cacttctgaa 8640
ggggttgtgc tcctgagtta ctcagggcag aagaccgaag gcccacagcg gatcagcgcc 8700
aagatcagcc agatcccccc ggccagtgca atggacattg aatttcagca gtcagtgtcc 8760
aagtcccagg tcaaacctga ttctgtcaca gcatcgcagc ctccatccaa aggccctcaa 8820
gctcctgcag gctatgcgaa cgtggccacc cattccacgt tggtactgac cgcccagaca 8880
tataatgcct ctcctgtgat ttcgtctgtg aaggccgata ggccatcctt ggagaagccc 8940
gagcccattc acctctcggt gtccacgcct gtcacccagg gaggcacagt gaaggttctc 9000
acccagggga tcaacacacc ccctgtgctg gttcacaacc agctggtcct caccccaagc 9060
attgtcacca caaacaagaa gcttgctgac cccgtcaccc ttaaaatcga gaccaaggtc 9120
cttcagccgg ccaacctggg gtccacgctc acgccccacc accctcctgc tctgcccagc 9180
aaactgccta cagaagtcaa ccatgtcccc tcgggcccca gcatcccagc agatcgaact 9240
gtctcccatt tggcagctgc aaagctagat gctcattctc ctcgaccaag tggacccggg 9300
ccatcctcat tcccaagggc aagccacccc agcagtactg catctacggc gctctccacc 9360
aacgccacag tcatgctggc tgcaggcatc ccagtgcccc agttcatctc cagcatccac 9420
ccagagcagt ctgtcatcat gccaccccac agcatcaccc agactgtgtc cctgagccac 9480
ctctcccagg gcgaggtgag aatgaacact cccacgctgc ccagtatcac ctacagcatc 9540
cggccagaag cgcttcactc tcctcgggct ccgctgcagc cccagcaaat agaggtcagg 9600
gccccacagc gtgccagcac cccgcagcca gccccagctg gtgtgcctgc actggcctcc 9660
cagcaccctc ccgaggagga agtgcattat caccttcctg tcgctcgagc cacagcccct 9720
gtgcagtcag aggtactagt catgcagtct gagtaccgac tgcaccccta tactgtgcca 9780
cgggatgtga ggatcatggt gcatccacat gtgacggcag tcagcgagca gcccagggcc 9840
gcggatgggg tggtgaaggt gccaccagcc agcaaggccc ctcagcagcc agggaaggaa 9900
gctgccaaga caccagatgc caaagctgcc cccaccccca cccctgcccc cgtccctgtc 9960
cctgtccccc ttcctgcccc tgctcctgcc cctcatggtg aggcccgtat cctcacagtt 10020
acccccagta accaactcca ggggctgcct ctgacccctc ctgtggtggt gacccatggg 10080

gtgcagattg tgcactccag cggggagctg tttcaagagt accggtacgg cgacatccgc 10140
acctaccacc ccccggccca gctcacacac actcagtttc ccgccgcttc ctctgttggc 10200
ctgccttccc ggaccaagac agctgctcag ggccctcctc ctgaaggtga gcccctgcag 10260
cctcctcagc ctgtgcagtc cacacagcct gcccagcctg caccaccctg cccgccctcc 10320
cagctcggtc agcccggcca gccaccaagc agcaagatgc ctcaagtgtc ccaggaggca 10380
aaggggaccc agacgggagt agagcagcct cgcctcccag ctggacctgc aaacaggcca 10440
cctgagcctc acacccaggt tcagagggca caagcagaaa caggcccgac ttccttcccc 10500
tcccctgtgt ctgtctccat gaagcctgac cttccagtct ctcttcccac tcagactgcc 10560
ccaaaacagc cgttgtttgt cccaacaacc tctggcccca gcaccccacc aggactggtt 10620
ctgccacaca ctgaattcca gccagccccc aaacaagatt cctctccaca cctgacttcc 10680
cagagacccg tggatatggt tcaacttctg aagaagtacc ccatcgtgtg gcagggcctg 10740
ctggccctca agaatgacac agctgctgtg cagctccact tcgtctctgg caacaacgtc 10800
ctggcccatc ggtccctgcc cctttctgaa ggagggcccc cactaaggat cgcccagagg 10860
atgcggctgg aggcaacgca gctggaaggg gttgcccgaa ggatgacggt ggagacagat 10920
tactgtctgc tgctggctct gccctgtggc cgtgaccaag aggatgttgt gagccagacc 10980
gagtccctca aggctgcctt catcacttac ctgcaggcca agcaggcggc agggatcatc 11040
aacgttccca accctggctc caatcagcct gcctacgtgc tgcagatctt cccgccctgt 11100
gagttctctg agagtcacct gtcccgcctg gcccctgacc tccttgccag catctccaac 11160
atctctcccc acctcatgat tgtcattgcc tccgtgtgag ccactgagtg gttatcacct 11220
cagtgaatct tcccagggct ctgcagtaaa aacaaaggac aacccagcca agcagaggaa 11280
gaagctgccg aaggggacag actccactgc cagacggcca gccgtttgct gtcctgccgc 11340
ccggctcagt cggccagact tcctctagga gtggtgctgc taccttgtat gtttacataa 11400
tgctttagcc caaggacaca tcaccaaccc atggactcgc agacaccggg gctgggtttc 11460
tctttcctct ttttggagaa aaggaacagg gcagtggaat gaaaattttt tgtttgtttg 11520
tttttaagaa acaagaaaac agaactgcct ttgcactaaa ttagtgactt ggacttttgc 11580
ccagtgaaga caggctgtga cactctggat gtcttggtgt gtgtagacac acattgcaga 11640
ctcttaacgc aggaaggact tcaaacttct gctgagacct tggggtcaag gaacatttca 11700
ttggtttttt ttgtccaccc ccatctccct tgctcatttg gatgcgtcac cttaattctc 11760
ctgctgccac cgtctttgat tcaccgggat gtacagttta cagttgaaga gcaaacagaa 11820
aggttttctc ttggtgggat atgcagaact tgggatgtgt gtatatataa atatataata 11880
tatataaata tatataatac tgacttaaaa aatcaaatcc cccgacatac gtttttttta 11940
atctgtgcca aaaatgtgtt ttcagaggaa atcttatttt catattcaga ctttgtattg 12000
cccactcatt tgtataagtg cgcttcggta cagcacgggt cctgctcccg cgatgtggaa 12060
gtgtcacacg gcacctgtac aaaaagactg gctaacccct cttcctatta ccttgatctc 12120
ttcccccaac ttcctaacac ttattaattt atgaaactgt ttttctcagc gcagttttgt 12180
tttgtgtgtc cattggatta caaactttat taaaaaatat aaaacac 12227

<210> 37
<211> 5376
<212> DNA
<213> Homo sapiens
<220>
<221> misc_feature
<223> séquence appelée GS-N36

<300>
<308> GENBANK/AF352051
<309> 2001-09-20
<313> (1)..(5376)
<400> 37
ggcacgagga aacctctagg tccaccacct ccatcttaca cgtgtttccg ttgtggtaaa 60
cctggacatt atattaagaa ttgcccaaca aatggggata aaaactttga atctggtcct 120
aggattaaaa agagcactgg aattcccaga agttttcatg atggaagtga aagatcctaa 180
tatgaaaggt gcaatgctta ccaacactgg aaaatatgca ataccaacta tagatgcaga 240
agcatatgca attgggaaga aagagaaacc tcccttctta ccagaggagc catcttcttc 300
ctcagaagaa gatgatccta tcccagatga attgttgtgt ctcatctgca aggatattat 360
gactgatgct gttgtgattc cctgctgtgg aaacagttac tgtgatgaat gtataagaac 420
agcactcctg gaatcagatg agcacacatg tccgacgtgt catcaaaatg atgtttctcc 480
tgatgcttta attgccaata aatttttacg acaggctgta aataacttca aaaatgaaac 540
tggctataca aaaagactac gaaaacagtt acctcctcca ccacccccaa taccacctcc 600
gagaccactg attcagagga acctacaacc tctgatgaga tctccgatat caagacaaca 660
agatcctctt atgattccag tgacatcttc atcaactcac ccagctccgt ctatatcttc 720
attaacttct aatcagtctt ccttggcccc tcctgtgtct ggaaatccgt cttctgctcc 780
agctcctgta cctgatataa ctgcaacagt atccatatca gttcattcag aaaaatcaga 840
tggacctttt cgggattctg ataataaaat attgccagct gcagctcttg catcagagca 900
ctcaaaggga acctcctcaa ttgcaattac cgctcttatg gaagagaagg gttaccaggt 960
gcctgttctt ggaaccccat ctttgcttgg acagtcatta ttgcatggac agttgatccc 1020
cacaactggt ccagtaagaa taaatactgc tcgtccaggt ggtggtcgac caggctggga 1080
acattccaac aaacttggct atctggtttc tccaccacaa caaattagaa gaggggagag 1140
gagctgctac agaagtataa accgtgggcg acaccacagc gaaagatcac agaggactca 1200
aggcccgtca ctaccagcaa ctccagtctt tgtacctgtt ccaccacctc ctttgtatcc 1260
gcctcctccc catacacttc ctctccctcc gggtgttcct cctccacagt tttctcctca 1320
gtttcctcct ggccagccac cacccgctgg gtatagtgtc cctcctccag ggtttcctcc 1380

agctcctgcc aatttatcaa caccttgggt atcatcagga gtgcagacag ctcattcaaa 1440
taccatccca acaacacaag caccaccttt gtccagggaa gaattctata gagagcagcg 1500
acgactaaaa gaagaggaaa agaaaaagtc caagctagat gagtttacaa atgattttgc 1560
taaggaattg atggaataca aaaagattca aaaggagcgt aggcgctcat tttccaggtc 1620
taaatctccc tatagtggtt cttcgtattc aagaagttca tatacttatt ctaaatcaag 1680
atctgggtca acacgttcac gctcttattc tcgatcattc agccgctcac attctcgttc 1740
ctattcacgg tcacctccat accccagaag aggcagaggc aagagccgca attaccgttc 1800
acggtctaga tctcatggat atcatcgatc taggtcaagg tcaccccctt acagacgcta 1860
tcattcacga tcaagatctc ctcaagcgtt taggggacag tctcctaata aacgtaatgt 1920
acctcaaggg gaaacagaac gtgaatattt taatagatac agagaagttc caccaccata 1980
tgacatgaaa gcatattatg ggagaagtgt tgactttaga gacccatttg aaaaagaacg 2040
ctaccgagaa tgggagagaa aatatagaga gtggtatgaa aaatattata aaggttatgc 2100
tgctggagca cagcctagac cctcagcaaa tagagagaac ttttctccag agagattttt 2160
gccacttaac atcaggaatt ctcccttcac aagaggccgc agagaagact atgttggtgg 2220
gcaaagtcat agaagtcgaa acataggtag caactatcca gaaaagcttt cagcaagaga 2280
tggtcacaat cagaaggata atacaaagtc aaaagagaag gagagtgaaa acgctccagg 2340
agatggtaaa ggaaataagc ataagaaaca cagaaaaaga agaaaagggg aggaaagtga 2400
gggttttctg aacccagagt tattagagac ttctaggaaa tcaagagaac ctacaggtgt 2460
tgaagaaaat aaaacagact cattgtttgt tctcccaagt agagatgatg ccacacctgt 2520
tagagatgaa ccaatggatg cagaatcaat cacttttaaa tcagtgtctg aaaaagacaa 2580
gagagaaagg gataaaccaa aagcaaaggg tgataaaacc aaacggaaga atgatggatc 2640
tgctgtgtcc aaaaaagaaa atattgtaaa acctgctaaa ggaccccaag aaaaagtaga 2700
tggagaacgt gagagatctc ctcgatctga acctccaatt aaaaaagcca aagaggagac 2760
tccgaagact gacaatacta aatcatcatc ttcctctcag aaggatgaaa aaatcactgg 2820
aacccccaga aaagctcact ctaaatcagc aaaagaacac caagaaacaa aaccagtcaa 2880
agaggaaaaa gtgaagaagg actattccaa agatgtcaaa tcagaaaagc taacaactaa 2940
ggaagaaaag gccaagaagc ctaatgagaa aaacaaacca cttgataata agggagaaaa 3000
aagaaaaaga aaaactgaag aaaaaggcgt agataaagat tttgagtctt cttcaatgaa 3060
aatctcgaaa ctagaagtga ctgaaatagt gaaaccatca ccaaagcgca aaatggaacc 3120
tgatactgaa aaaatggata ggacccctga aaaggacaaa atttctttaa gtgcgccagc 3180
caaaaaaatc aaactcaaca gagaaactgg gaagaaaatt ggaagtacag aaaatatatc 3240
aaacacaaaa gaaccctctg aaaaattgga gtcaacatct agcaaagtta aacaagaaaa 3300
agtcaaagga aaggtcagac gaaaagtgac tggaactgaa ggatccagct caactctggt 3360
ggattacacc agtacgagct caactggagg cagtcctgtg cggaaatctg aagaaaaaac 3420
agatacaaag cgaactgtga ttaaaacgat ggaagaatat aataatgaca ataccgcgcc 3480
agctgaagat gttatcatta tgattcaggt tcctcaatcc aaatgggata aagatgactt 3540
tgaatctgaa gaagaagatg ttaaatccac acagcctata tcaagtgtag gaaaacctgc 3600
tagtgttata aaaaatgtta gtacaaagcc atcaaatata gtcaagtatc ctgagaaaga 3660
aagtgagcca tccgagaaaa ttcagaaatt caccaaggac gtgagccatg aaatcataca 3720
acatgaggtt aaaagttcaa aaaactctgc atctagtgaa aaagggaaaa ccaaagatcg 3780
agattattca gtgttggaaa aggagaaccc tgaaaagagg aagaacagca ctcagccaga 3840
gaaagagagt aatttggacc gtctgaatga acaaggaaat tttaaaagtc tgtctcaatc 3900
ttccaaagag gctagaacgt cagataaaca tgattccact cgtgcttcct caaataaaga 3960
cttcactccc aatagagaca aaaaaactga ctatgacacc agagagtatt caagttccaa 4020
acgtagagat gaaaagaatg aattaacaag acgaaaagac tctccttctc ggaataaaga 4080
ttctgcatct ggacagaaaa ataaaccaag ggaagagaga gatttgccta aaaaaggaac 4140
aggagattcc aaaaaaagta attctagtcc ctcaagagac agaaaacctc atgatcacaa 4200
agccacttat gatactaaac ggccaaatga agagacaaaa tctgtagata aaaatccttg 4260
taaggatcgt gagaagcatg tattagaagc aaggaacaat aaagagtcaa gtggcaataa 4320
actactttat atacttaacc caccagagac acaggttgaa aaagagcaaa ttactgggca 4380
aattgacaag agtactgtca agcctaaacc ccagttaagt cattcctcta gactttcctc 4440
tgacttaact agagaaactg atgaagctgc ttttgaacca gactataatg aaagtgacag 4500
tgaaagtaat gtttctgtaa aagaagagga atcttcagga aacatttcta aggacctgaa 4560
agataaaata gtggagaaag cgaaagagag cctggacaca gcagcagttg tccaggtggg 4620
cataagcagg aatcagagcc acagcagccc cagcgtcagc cccagcagaa gccacagtcc 4680
ttctggaagc cagacccgaa gccacagtag cagtgccagc tcagcagaaa gtcaggacag 4740
caagaagaag aagaaaaaga aggaaaagaa aaaacacaag aaacataaaa agcataagaa 4800
gcataagaaa catgcaggca ctgaagtgga attggaaaaa agccaaaaac acaaacacaa 4860
gaaaaagaag tcaaagaaga acaaagataa agagaaggag aaggagaaag atgaccaaaa 4920
agtgaaatct gtcactgtgt aaaaagacag attttttaaa ttgacttaat tactaagtca 4980
tctgtattaa attttgttat aatgtaaaga gattcaagcc ttgtaaataa tgacatggaa 5040
gaccctgtgc tgcacttaaa atattgctgc ttgattattt gatttttaca tcagagcttt 5100
ataacacgaa cttttgtaca gaattgtgag ttgtgaccat gtaacatgag aggttttgct 5160
agggcctatt atttttaacc accattaatt agttggggtg gagtttactg taatgtgaaa 5220
ttttcacatt tgaatttttt aattgcctgg caaaagctga tataagttct aaaatatcag 5280
cagaatgatt tgctgaattc attacatccc tgttatgtca ctttttgatt acaataaaag 5340
ttttcagtaa acttttcaaa aaaaaaaaaa aaaaaa 5376

<210> 38
<211> 6626
<212> DNA
<213> Homo sapiens
<220>

<221> misc_feature
<223> séquence appelée GS-N37

<300>
<308> GENBANK/NM_005338
<309> 2001-01-26
<313> (1)..(6626)
<400> 38
cagcatcaat aaggccatta atacgcagga agtggctgta aaggaaaaac acgccagaac 60
gtgcatactg ggcacccacc atgagaaagg ggcacagacc ttctggtctg ttgtcaaccg 120
cctgcctctg tctagcaacg cagtgctctg ctggaagttc tgccatgtgt tccacaaact 180
cctccgagat ggacacccga acgtcctgaa ggactctctg agatacagaa atgaattgag 240
tgacatgagc aggatgtggg gccacctgag cgaggggtat ggccagctgt gcagcatcta 300
cctgaaactg ctaagaacca agatggagta ccacaccaaa aatcccaggt tcccaggcaa 360
cctgcagatg agtgaccgcc agctggacga ggctggagaa agtgacgtga acaacttttt 420
ccagttaaca gtggagatgt ttgactacct ggagtgtgaa ctcaacctct tccaaacagt 480
attcaactcc ctggacatgt cccgctctgt gtccgtgacg gcagcagggc agtgccgcct 540
cgccccgctg atccaggtca tcttggactg cagccacctt tatgactaca ctgtcaagct 600
tctcttcaaa ctccactcct gcctcccagc tgacaccctg caaggccacc gggaccgctt 660
catggagcag tttacaaagt tgaaagatct gttctaccgc tccagcaacc tgcagtactt 720
caagcggctc attcagatcc cccagctgcc tgagaaccca cccaacttcc tgcgagcctc 780
agccctgtca gaacatatca gccctgtggt ggtgatccct gcagaggcct catcccccga 840
cagcgagcca gtcctagaga aggatgacct catggacatg gatgcctctc agcagaattt 900
atttgacaac aagtttgatg acatctttgg cagttcattc agcagtgatc ccttcaattt 960
caacagtcaa aatggtgtga acaaggatga gaaggaccac ttaattgagc gactatacag 1020
agagatcagt ggattgaagg cacagctaga aaacatgaag actgagagcc agcgggttgt 1080
gctgcagctg aagggccacg tcagcgagct ggaagcagat ctggccgagc agcagcacct 1140
gcggcagcag gcggccgacg actgtgaatt cctgcgggca gaactggacg agctcaggag 1200
gcagcgggag gacaccgaga aggctcagcg gagcctgtct gagatagaaa ggaaagctca 1260
agccaatgaa cagcgatata gcaagctaaa ggagaagtac agcgagctgg ttcagaacca 1320
cgctgacctg ctgcggaaga atgcagaggt gaccaaacag gtgtccatgg ccagacaagc 1380
ccaggtagat ttggaacgag agaaaaaaga gctggaggat tcgttggagc gcatcagtga 1440
ccagggccag cggaagactc aagaacagct ggaagttcta gagagcttga agcaggaact 1500
tgccacaagc caacgggagc ttcaggttct gcaaggcagc ctggaaactt ctgcccagtc 1560
agaagcaaac tgggcagccg agttcgccga gctagagaag gagcgggaca gcctggtgag 1620
tggcgcagct catagggagg aggaattatc tgctcttcgg aaagaactgc aggacactca 1680
gctcaaactg gccagcacag aggaatctat gtgccagctt gccaaagacc aacgaaaaat 1740
gcttctggtg gggtccagga aggctgcgga gcaggtgata caagacgccc tgaaccagct 1800
tgaagaacct cctctcatca gctgcgctgg gtctgcagat cacctcctct ccacggtcac 1860
atccatttcc agctgcatcg agcaactgga gaaaagctgg agccagtatc tggcctgccc 1920
agaagacatc agtggacttc tccattccat aaccctgctg gcccacttga ccagcgacgc 1980
cattgctcat ggtgccacca cctgcctcag agccccacct gagcctgccg actcactgac 2040
cgaggcctgt aagcagtatg gcagggaaac cctcgcctac ctggcctccc tggaggaaga 2100
gggaagcctt gagaatgccg acagcacagc catgaggaac tgcctgagca agatcaaggc 2160
catcggcgag gagctcctgc ccaggggact ggacatcaag caggaggagc tgggggacct 2220
ggtggacaag gagatggcgg ccacttcagc tgctattgaa actgccacgg ccagaataga 2280
ggagatgctc agcaaatccc gagcaggaga cacaggagtc aaattggagg tgaatgaaag 2340
gatccttggt tgctgtacca gcctcatgca agctattcag gtgctcatcg tggcctctaa 2400
ggacctccag agagagattg tggagagcgg caggggtaca gcatccccta aagagtttta 2460
tgccaagaac tctcgatgga cagaaggact tatctcagcc tccaaggctg tgggctgggg 2520
agccactgtc atggtggatg cagctgatct ggtggtacaa ggcagaggga aatttgagga 2580
gctaatggtg tgttctcatg aaattgctgc tagcacagcc cagcttgtgg ctgcatccaa 2640
ggtgaaagct gataaggaca gccccaacct agcccagctg cagcaggcct ctcggggagt 2700
gaaccaggcc actgccggcg ttgtggcctc aaccatttcc ggcaaatcac agatcgaaga 2760
gacagacaac atggacttct caagcatgac gctgacacag atcaaacgcc aagagatgga 2820
ttctcaggtt agggtgctag agctagaaaa tgaattgcag aaggagcgtc aaaaactggg 2880
agagcttcgg aaaaagcact acgagcttgc tggtgttgct gagggctggg aagaaggaac 2940
agaggcatct ccacctacac tgcaagaagt ggtaaccgaa aaagaataga gccaaaccaa 3000
caccccatat gtcagtgtaa atccttgtta cctatctcgt gtgtgttatt tccccagcca 3060
caggccaaat ccttggagtc ccaggggcag ccacaccact gccattaccc agtgccgagg 3120
acatgcatga cacttcccaa agactccctc catagcgaca ccctttctgt ttggacccat 3180
ggtcatctct gttcttttcc cgcctcccta gttagcatcc aggctggcca gtgctgccca 3240
tgagcaagcc taggtacgaa gaggggtggt ggggggcagg gccactcaac agagaggacc 3300
aacatccagt cctgctgact atttgacccc cacaacaatg ggtatcctta atagaggagc 3360
tgcttgttgt ttgttgacag cttggaaagg gaagatctta tgccttttct tttctgtttt 3420
cttctcagtc ttttcagttt catcatttgc acaaacttgt gagcatcaga gggctgatgg 3480
attccaaacc aggacactac cctgagatct gcacagtcag aaggacggca ggagtgtcct 3540
ggctgtgaat gccaaagcca ttctccccct ctttgggcag tgccatggat ttccactgct 3600
tcttatggtg gttggttggg ttttttggtt ttgttttttt tttttaagtt tcactcacat 3660
agccaactct cccaaagggc acacccctgg ggctgagtct ccagggcccc ccaactgtgg 3720
tagctccagc gatggtgctg cccaggcctc tcggtgctcc atctccgcct ccacactgac 3780
caagtgctgg cccacccagt ccatgctcca gggtcaggcg gagctgctga gtgacagctt 3840
tcctcaaaaa gcagaaggag agtgagtgcc tttccctcct aaagctgaat cccggcggaa 3900

agcctctgtc cgcctttaca agggagaaga caacagaaag agggacaaga gggttcacac 3960
agcccagttc ccgtgacgag gctcaaaaac ttgatcacat gcttgaatgg agctggtgag 4020
atcaacaaca ctacttccct gccggaatga actgtccgtg aatggtctct gtcaagcggg 4080
ccgtctccct tggcccagag acggagtgtg ggagtgattc ccaactcctt tctgcagacg 4140
tctgccttgg catcctcttg aataggaaga tcgttccact ttctacgcaa ttgacaaacc 4200
cggaagatca gatgcaattg ctcccatcag ggaagaaccc tatacttggt ttgctaccct 4260
tagtatttat tactaacctc ccttaagcag caacagccta caaagagatg cttggagcaa 4320
tcagaacttc aggtgtgact ctagcaaagc tcatctttct gcccggctac atcagccttc 4380
aagaatcaga agaaagccaa ggtgctggac tgttactgac ttggatccca aagcaaggag 4440
atcatttgga gctcttgggt cagagaaaat gagaaaggac agagccagcg gctccaactc 4500
ctttcagcca catgccccag gctctcgctg ccctgtggac aggatgagga cagagggcac 4560
atgaacagct tgccagggat gggcagccca acagcacttt tcctcttcta gatggacccc 4620
agcatttaag tgaccttctg atcttgggaa aacagcgtct tccttcttta tctatagcaa 4680
ctcattggtg gtagccatca agcacttccc aggatctgct ccaacagaat attgctaggt 4740
tttgctacat gacgggttgt gagacttctg tttgatcact gtgaaccaac ccccatctcc 4800
ctagcccacc cccctcccca actccctctc tgtgcatttt ctaagtggga cattcaaaaa 4860
actctctccc aggacctcgg atgaccatac tcagacgtgt gacctccata ctgggttaag 4920
gaagtatcag cactagaaat tgggcagtct taatgttgaa tgctgctttc tgcttagtat 4980
ttttttgatt caaggctcag aaggaatggt gcgtggcttc cctgtcccag ttgtggcaac 5040
taaaccaatc ggtgtgttct tgatgcgggt caacatttcc aaaagtggct agtcctcact 5100
tctagatctc agccattcta actcatatgt tcccaattac caaggggtgg ccgggcacag 5160
tggctcacgc ctgtaatccc agcactttga gaggctgagg tggtaggatc acctgaggtc 5220
aggagttcaa gaccagcctg tccaacatgg tgaaaccccc atctctacta aaaataccaa 5280
aaattagccg agcgtagtga cgggtgcccg taatcccagc tactcaggag gctgagacag 5340
gagaatcacc tgaaccccag aggcagaggt tgcagtgagc tgagatcacg ccattgtact 5400
ccagcctggg caacaagagc aaaactccgt ctcaaaaaaa aaaaaaaatt acaaatgggg 5460
caaacagtct agtgtaatgg atcaaattaa gattctctgc ccagccgggc acagtggcgc 5520
atgcctgtaa tcccagaact ttgggaggcc aagacgggat gattgcttga gctcaggagt 5580
ttgagaccag gctgggcatc atagcaagac ctcatctcta ctaaaattca aaaacaaaat 5640
tagccgggca tgatggtgca tgcctgtagt ctcagctagt tggggagcta aggtgggaga 5700
attgcttgag cttgggaagt cgaggctgca gtcagccctg attgtgccag tgcactccgg 5760
cctgggtgac agagtgagac ccgtgctcaa aaaaaaaaag attctgtgtc agagcccagc 5820
ccaggagttt gaggctgcaa tgagccatga tttcccactg cactccagcc tgagtgacag 5880
agcgagactc catctcttta aaaacaaaca aaaaattatc tgaatgatcc tgtctctaaa 5940
aagaagccac agaaatgttt aaaaacttca tcgacttagc ctgagtcata acggttaaga 6000
aagcacttaa acagaagcag aggctaattc agtgtcacat gaggaagtag ctgtcagatg 6060
tcacataatt actttcgtaa tagctcagat tagaatggct accccattct ctagacaaaa 6120
tcaaattgtc ctattgtgac tcttctaaaa atgaagatga agagctattt aatgacacac 6180
cttggattaa aacgggaatc acatcttaaa gctaaaaatg aacctgcaag ccttctaaat 6240
gagtcactga gcatcactag tgacaagtct cgggtgagcg taaatgggtc atgacaagat 6300
gggacagcaa caaaatcatg gcttaggatc gacaagaagt taaaaaacag ctgcatctgt 6360
tacttaagtt tgtaagacag tgccctgaga cctctagaga aaagatgttt gtttacataa 6420
gagaaagaag gccagacatg gtgtctcaca cgtttaatcc cagcactttg ggaggcaggg 6480
gcgggtggat cacctgaggt caggagttca agactagcct ggccaacatg gtgaaacccc 6540
gtctctacta aaaatacaaa aattagccgg gcatggtggc aggcgcctat aatcccagct 6600
actggggagg ctgaggcagg agaatc 6626

<210> 39
<211> 2366
<212> DNA
<213> Homo sapiens
<220>
<221> misc_feature
<223> séquence appelée GS-N38

<300>
<308> GENBANK/BC000335
<309> 2001-07-12
<313> (1)..(2366)
<400> 39
ggcacgagga gatggcggcc gccgagggac cggtgggcga cggcgagctg tggcagacct 60
ggcttcctaa ccacgtcgtg ttcttgcggc tccgggaggg actgaaaaac cagagtccaa 120
ccgaagctga gaaaccagct tcttcgtcgt tgccttcgtc gccgccgccg cagttgctga 180
cgagaaacgt ggtctttggc ctcggcggag agcttttcct gtgggacgga gaagacagct 240
ccttcttagt cgttcgcctt cggggcccca gcggcggcgg cgaagagccc gccctgtccc 300
agtaccagag attgctttgc ataaatccac ccctgtttga aatctatcaa gtcttgttaa 360
gcccaacaca acatcatgta gcacttatag gaataaaagg acttatggta ttagaattac 420
ctaaaagatg ggggaagaat tctgaatttg aaggtggaaa atcaacagtg aattgtagta 480
ccactccagt tgcggagaga tttttcacca gttccacctc tctgactcta aagcatgctg 540
catggtatcc aagtgaaatc ctggatcccc acgtagtgct gttaacatca gacaacgtaa 600
tcagaattta ctcactacgt gagccgcaga cacccactaa cgtgataata ctttcagaag 660
ccgaagagga aagtctagta ctcaataaag gaagggcgta taccgcatct ctaggagaga 720
cagcagttgc atttgacttt gggccattgg cagcagtccc aaagactcta tttggacaaa 780

acggcaaaga tgaagtagtg gcatacccac tgtacatctt atatgaaaat ggagagactt 840
tcctgacata catcagtctg ttacacagcc ctggaaatat tggaaagctg ttgggtccat 900
tgcccatgca tcctgcggct gaagataact atggttatga tgcgtgtgct gtactctgct 960
taccctgtgt ccccaatatc ttagtgatcg ctactgaatc aggaatgctg tatcactgtg 1020
tcgtgctaga aggggaagaa gaagatgacc acacgtcaga aaagtcctgg gattccagga 1080
ttgacctcat tccttctctg tatgtgtttg aatgtgttga gttggagctt gctttgaaac 1140
tggcatctgg agaggatgac ccttttgatt ctgacttttc ttgtccagtc aaacttcata 1200
gagatcccaa gtgtccttca agatatcact gtactcatga agctggtgta catagtgttg 1260
ggctaacttg gattcataaa cttcacaaat ttcttggatc agatgaagaa gataaggata 1320
gtttacagga actctctaca gaacagaaat gctttgttga acacatcctt tgtacgaagc 1380
cattgccctg caggcagcca gctccaattc gaggattttg gattgtacct gacattctgg 1440
gacccacgat gatctgcatc accagtacct atgaatgcct catatggccg ttattaagta 1500
cagtccatcc agcgtctcct cccctgcttt gtactcgaga agatgttgaa gtggcagagt 1560
ctcccctccg tgttctggct gaaaccccag attcctttga aaagcatatt agaagcattt 1620
tgcaacgtag tgttgccaat ccagcatttt tgaaagcttc tgaaaaggac atagcccctc 1680
ctcctgaaga atgccttcag ctcctcagca gagccaccca ggtgttcaga gagcagtaca 1740
ttctcaaaca ggacttggca aaggaggaga ttcagcggag ggtcaaatta ttatgtgacc 1800
aaaaaaagaa acaactagaa gatctcagtt attgtcgaga agagaggaaa agtctgcggg 1860
aaatggctga gcgtttagct gacaaatatg aggaagctaa agaaaaacaa gaggatatca 1920
tgaacaggat gaaaaaacta cttcacagtt ttcactctga gctcccagtt ctctctgata 1980
gtgagcgaga catgaagaaa gaattacagc tgatacctga tcaacttcga catttgggca 2040
atgccatcaa acaggttact atgaaaaagg attatcaaca gcaaaagatg gagaaggtgt 2100
tgagtcttcc aaaacccacc attattctca gtgcctacca gcgaaagtgc attcagtcca 2160
tcctgaaaga ggagggtgaa catataaggg aaatggtgaa gcaaatcaat gatatccgca 2220
atcatgtaaa cttctgacac caccaggagc tgactcacac ctgaactgaa caccattgaa 2280
ggcttaaacc catattgtaa aacaggtaga attatctaat ttataaaaag gtgttttgat 2340
gacaaaaaaa aaaaaaaaaa aaaaaa 2366

<210> 40
<211> 1543
<212> DNA
<213> Homo sapiens
<220>
<221> misc_feature
<223> séquence appelée GS-N39

<300>
<308> GENBANK/XM_049486
<309> 2001-10-16
<313> (1)..(1543)
<400> 40
aggcagagcc gtggaaccgc cgccaggtcg ctgttggtcc acgccgcccg tcgcgccgcc 60
cgcccgctca gcgtccgccg ccgccatggg agtgcaggtg gaaaccatct ccccaggaga 120
cgggcgcacc ttccccaagc gcggccagac ctgcgtggtg cactacaccg ggatgcttga 180
agatggaaag aaatttgatt cctcccggga cagaaacaag ccctttaagt ttatgctagg 240
caagcaggag gtgatccgag gctgggaaga aggggttgcc cagatgagtg tgggtcagag 300
agccaaactg actatatctc cagattatgc ctatggtgcc actgggcacc caggcatcat 360
cccaccacat gccactctcg tcttcgatgt ggagcttcta aaactggaat gacaggaatg 420
gcctcctccc ttagctccct gttcttggat ctgccatgga gggatctggt gcctccagac 480
atgtgcacat gaatccatat ggagcttttc ctgatgttcc actccacttt gtatagacat 540
ctgccctgac tgaatgtgtt ctgtcactca gctttgcttc cgacacctct gtttcctctt 600
cccctttctc ctcgtatgtg tgtttaccta aactatatgc cataaacctc aagttattca 660
ttttattttg ttttcatttt ggggtgaaga ttcagtttca gtcttttgga tataggtttc 720
caattaagta catggtcaag tattaacagc acaagtggta ggttaacatt agaataggaa 780
ttggtgttgg ggggggggtt tgcaagaata ttttatttta attttttgga tgaaattttt 840
atctattata tattaaacat tcttgctgct gcgctgcaaa gccatagcag atttgaggcg 900
ctgttgagga ctgaattact ctccaagttg agagatgtct ttgggttaaa ttaaaagccc 960
tacctaaaac tgaggtgggg atggggagag cctttgcctc caccattccc acccaccctc 1020
cccttaaacc ctctgccttt gaaagtagat catgttcact gcaatgctgg acactacagg 1080
tatctgtccc tgggccagca gggacctctg aagccttctt tgtggccttt tttttttttc 1140
atcctgtggt ttttctaatg gactttcagg aattttgtaa tctcataact ttccaagctc 1200
caccacttcc taaatcttaa gaactttaat tgacagtttc aattgaaggt gctgtttgta 1260
gacttaacac ccagtgaaag cccagccatc atgacaaatc cttgaatgtt ctcttaagaa 1320
aatgatgctg gtcatcgcag cttcagcatc tcctgttttt tgatgcttgg ctccctctgc 1380
tgatctcagt ttcctggctt ttcctccctc agccccttct cacccctttg ctgtcctgtg 1440
tagtgatttg gtgagaaatc gttgctgcac ccttccccca gcaccattta tgagtctcaa 1500
gttttattat tgcaataaaa gtgctttatg ccggcttttc tca 1543

<210> 41
<211> 3824
<212> DNA
<213> Homo sapiens
<220>

<221> misc_feature
<223> séquence appelée GS-N40

<300>
<308> GENBANK/XM_042827
<309> 2001-10-16
<313> (1)..(3824)
<400> 41
ggactttggg actggacaga cctggtcaca gtctaggttc tacatcttac tggtcgagca 60
actttagagt caacctattt gatttcttga caagaccaca atctgatccc aaagatgtgc 120
tccacaaatc caggcaaatg ggtcaccttt gatgatgatc ctgctgttca atcttctcaa 180
aagtcaaaga attttcctct ggagaatcaa ggtgtctgta gaccaaatgg actgaagctg 240
aaccttcctg gcctcaggga atttcccagt ggatcttcct ccaccagcag cactcctctc 300
tcctccccca ttgtagattt ttatttcagt ccaggacctc caagtaactc tcctctttct 360
acacctacca aagacttccc aggttttcct ggcatcccca aagcagggac tcatgtgctt 420
tatcctattc cagaatcatc ttcagacagc ccactcgcaa tatcaggagg agaatcttcc 480
ttactgccta ccagaccaac atgtttatcc catgccttgt tacccagtga ccactcatgt 540
acacatccaa ctcccaaagt aggtcttcca gatgaagtta atcctcaaca ggctgaaagc 600
ctaggattcc aaagtgatga tctcccccag tttcagtatt ttcgagagga ctgtgctttt 660
tcaagtccat tttggaaaga tgaaggcagt gattcccatt tcacccttga cccaccagga 720
agcaaaaaga tgttctcatc aagaaacaag gagatgccta ttgaccaaaa aagcctaaat 780
aagtgttcac tcaactatat ctgtgagaag cttgaacatc tccagtcagc tgagaaccaa 840
gactcactta gaagtttgtc tatgcactgt ctatgtgctg aagaaaatgc ctcttccttt 900
gtcccccaca cactcttcag gagtcagcca aaatccggat ggtctttcat gctgagaatt 960
cctgagaaga agaatatgat gtcttcccgg caatggggac caatttttct gaaagttttg 1020
cctggaggaa ttttgcagat gtattatgaa cagggattag aaaaaccatt taaagagata 1080
cagcttgatc catattgtag gctttctgaa cccaaggttg agaacttcag tgtagcagga 1140
aaaatccaca ctgtgaagat tgaacatgtg tcttacacag aaaaaaggaa ataccattct 1200
aagacagaag tagttcatga acctgacata gagcagatgc tgaagttggg gtccacatcg 1260
taccatgact tccttgactt tctgactact gtggaggagg agctgatgaa gttgccagct 1320
gtttcaaaac caaaaaagaa ctacgaggag caagaaattt ccttggaaat tgtggacaac 1380
ttttggggta aagtcacaaa agaaggaaaa tttgttgaaa gtgctgtgat aactcaaatt 1440
tattgcctct gctttgtgaa tgggaacctg gaatgctttt taaccttgaa tgaccttgag 1500
ttgccgaagc gagatgaatc ctattatgag aaggactcag aaaaaaaggg gattgatatt 1560
cttgactacc attttcataa gtgtgtgaat gtacaagaat ttgagcaatc aagaatcatt 1620
aagtttgtac ctctggatgc ctgccggttt gagctgatgc gtttcaagac tttgtataat 1680
ggggataatc ttccctttcc cttgaagtct gtagtggttg tccagggagc atacgtggaa 1740
cttcaggctt ttgtcaacat ggcctcattg gcgcagaggt catcctatgc tggttcctta 1800
aggtcctgtg acaatataag gatacacttt cctgtcccat cgcagtggat caaggccctt 1860
tggaccatga acctccagag gcagaagtct ctgaaagcta aaatgaaccg ccgagcatgt 1920
ctggggagtt tacaggaact tgaatctgaa cctgtcattc aagtcactgt ggggtcagca 1980
aaatatgaga gtgcctacca ggcagtggta tggaagatag atcggcttcc agacaaaaat 2040
tcaagtctag atcatcccca ttgtctgtca tacaaattag agcttggatc agaccaagaa 2100
attccctctg attggtatcc atttgctact gttcagtttt ccgtgcctga cacctgtgcc 2160
tcaaggacag aggtcaggtc tctgggagtg gagagtgatg tccagccaca gaaacatgtt 2220
cagcagcgag cttgctacaa catccagcct aaactctaca gatctgtaat tgaagatgta 2280
attgaaggag ttcggaatct atttgctgaa gaaggtatag aggaacaagt tttaaaagac 2340
ttgaagcagc tctgggaaac caaggttttg cagtctaaag caacagaaga cttcttcaga 2400
aatagcatcc aatcacctct gtttactctt cagttgccgc acagcttgca ccaaacattg 2460
caatcgtcaa cagcatcatt agttattcct gctggtagaa ctcttccaag ttttaccaca 2520
gcagaactgg gcacttcaaa ctccagtgca aactttactt ttcctggtta tcccattcat 2580
gtaccagcag gtgtgacact acagactgta tctggtcacc tttataaagt caatgtacca 2640
attatggtga cagagacttc tggaagagca ggtattcttc agcatccaat tcagcaagta 2700
tttcaacagc ttggccagcc ttcagtaata caaactagtg ttccacaatt gaatccatgg 2760
tctcttcaag caactactga aaaatcacag agaattgaaa ccgtgctaca gcaacccgca 2820
attctacctt ctgggccagt agataggaaa cacttagaaa atgccaccag tgatatactt 2880
gtatctcctg gaaatgagca taaaatcgtg cctgaagctt tgttgtgtca tcaggaaagt 2940
tctcactata tcagtcttcc aggtgttgta ttttctccac aggtctctca aacaaattct 3000
aatgtggagt cagtgctcag tggttcagct agcatggctc aaaatctgca tgatgagtcc 3060
ctctccacaa gccctcatgg ggctctccac cagcacgtga ctgatattca gcttcatatt 3120
cttaaaaata ggatgtatgg atgtgattct gtaaagcaac caagaaatat agaggaaccc 3180
agcaacatac ctgtatcaga gaaggattct aattctcagg tggatttaag cattcgggtt 3240
actgatgatg atattggtga aataattcaa gtagatggaa gcggtgatac atcttccaat 3300
gaagaaatag gaagtacaag agatgcagat gagaatgaat ttctagggaa tattgacggg 3360
ggagatctga aggtacctga agaagaagct gacagtattt caaatgagga ttcagccaca 3420
aacagtagtg ataatgaaga ccctcaagta aacattgtag aagaggaccc tttaaattct 3480
ggagatgatg ttagtgaaca ggatgtgcca gacctgtttg acacggataa tgttattgtc 3540
tgtcagtatg ataagattca tcgaagcaag aacaaatgga aattctattt gaaagatggt 3600
gttatgtgtt ttggagggag agactatgta tttgcaaaag ccattggtga tgcagagtgg 3660
taaaccttgt gagctcagta catctatttt gtgaacatca gttggattat attgcatatt 3720
gtgaattcat ttttattttg aatatagtcc agcacagagc tgttcaaatt tttagttcac 3780
tgtatggaat ttaataaaat tataattcag atgcagatac aatt 3824

<210>  42
<211>  1365
<212>  DNA
<213>  Homo sapiens
<220>
<221>  misc_feature
<223>  séquence appelée GS-N41

<300>
<308>  GENBANK/BC010862
<309>  2001-07-17
<313>  (1)..(1365)
<400>  42
ggaagtggga agagagaaag gttgtgatgg cggctatagc tgcatccgag gtgctggtgg      60
acagcgcgga ggaggggtcc ctcgctgcgg cggcggagct ggccgctcag aagcgcgaac     120
agagactgcg caaattccgg gagctgcacc tgatgcggaa tgaagctcgt aaattaaatc     180
accaggaagt tgtggaagaa gataaaagac taaaattacc tgcaaattgg gaagccaaaa     240
aagctcgttt ggagtgggaa ctaaaggaag aggaaaagaa aaaggaatgt gcggcaagag     300
gagaagacta tgagaaagtg aagttgctgg agatcagtgc agaagatgca gaaagatggg     360
agaggaaaaa gaagaggaaa aaccctgatc tgggattttc agattatgct gctgcccagt     420
tacgccagta tcatcggttg accaagcaga tcaaacctga catggaaaca tatgagagac     480
tgagagaaaa acatggagaa gagtttttcc caacatccaa tagtcttctt catggaacac     540
atgtgccttc cacagaggaa attgacagga tggtcataga tctggaaaaa cagattgaaa     600
aacgagacaa atatagccgg agacgtcctt ataatgatga tgcagatatc gactacatta     660
atgaaaggaa tgccaaattc aacaagaaag ctgaaagatt ctatgggaaa tacacagctg     720
aaattaaaca gaatttggaa agaggaacag ctgtctaatc ccttcaagaa ctgtttatag     780
aagcttgaga atggggtaaa aatttctgct agcaaaatca agttcttttt gaaattttat     840
cagtaatcca gaatttagta gtccatgcct tctcactcag catttagaaa taaaaatgtg     900
gtttcttaaa cgtatatcct ttcatgtata tttccacatt tttgtgcttg gatataagat     960
gtatttcttg tagtgaagtt gttttgtaat ctactttgta tacattctaa ttatattatt    1020
tttctatgta ttttaaatgt atatggctgt ttaatctttg aagcattttg ggcttaagat    1080
tgccagcagc acacatcaga tgcagtcatt gttgctatca gtgtggaatt tgatagagtc    1140
tagactcggg ccacttggag ttgtgtactc caaagctaag gacagtgatg aggaagatgg    1200
cagtggccac cggaggactg gagcagtccc tcctcatggc ggcctgtgac caaggtcggg    1260
gaggagtgga gctatccttc catgatctga tcatgtacag ttcccttttt aaaaagcaat    1320
aaatgcttgg gattagaatt tctaaaaaaa aaaaaaaaaa aaaaa                    1365

<210>  43
<211>  6147
<212>  DNA
<213>  Homo sapiens
<220>
<221>  misc_feature
<223>  séquence appelée GS-N42

<300>
<308>  GENBANK/NM_013390
<309>  2000-11-02
<313>  (1)..(6147)
<400>  43
gcgggcgccg gcaatcgagt ggcttcctgg ccatggagag gagatctgga cccgaggacg      60
ccagggactc ctctccgtct ccctggaccg ctgtgtaggc acctgagtta acggacgcta     120
agcggggtag gggaagtaac agtgtatcat gtatgccact gattccaggg gacactcccc     180
tgctttcctc caacctcaga atggaaatag tcgtcaccca tctggctatg ttccagggaa     240
ggttgtccca ttgcgtcccc ctcctcctcc aaagagtcaa gcttcagcca aatttacctc     300
catcagacga gaagaccggg caaccttcgc attctcacct gaagaacagc aagcccagag     360
agaaagtcaa aagcaaaaga gacacaaaaa tactttcatt tgttttgcta ttactagttt     420
ctcatttttt attgcacttg caatcatttt aggaatatcc tcaaaatatg ctccagatga     480
aaattgccca gatcaaaatc ctcgtctcag gaattgggat ccaggacaag attctgcaaa     540
gcaagttgtt atcaaggagg gagatatgct ccgtctgacc tcagacgcca ccgtgcattc     600
tatagtcatt caggatggag gactgcttgt atttggggac aataaagatg gatccagaaa     660
tattactttg aggactcatt acatcctgat ccaggatggt ggggcgcttc atattggagc     720
agaaaaatgc cgctataaat ccaaagcgac aattaccttg tatggcaagt cagatgaagg     780
tgaaagtatg ccaacatttg gcaaaaagtt tattggtgtg gaagctggcg ggacactgga     840
gttacatggg gcacggaagg catcgtggac gttgttggca aggaccctga attcctcagg     900
cttgcccttt gggtcctata cctttgaaaa ggacttttcc cggggcctca atgtgagggt     960
cattgaccaa gacacggcca aaattttgga aagtgagaga tttgataccc atgaataccg    1020
caatgagagc aggcggcttc aggagtttct gagattccag gatccaggtc ggattgttgc    1080
catagctgtc ggggattcag ccgctaaaag tctcttacaa ggaaccatcc agatgatcca    1140
ggaacggttg ggaagtgaac tgatccaagg actgggctac aggcaagctt gggctttagt    1200
tggtgtcatt gatggtggaa gcacttcttg caatgaatcc gtgagaaact atgaaaatca    1260
tagcagtggc gggaaggctc ttgcccaaag agaattttat actgtggatg gccagaagtt    1320
ctctgtgaca gcttatagtg aatggattga aggcgtttct ctttcaggat tccgggtaga    1380

ggttgtagat ggagtgaagc taaatttgct agatgatgtt agtagttgga aacctggaga 1440
ccagattgtg gtcgcaagca cagactattc catgtaccaa gcagaggagt tcactcttct 1500
tccctgttct gaatgcagcc attttcaggt caaagtcaaa gaaacccctc agttcctgca 1560
catgggtgag atcatagacg gtgtagacat gagagctgag gttggaattc ttacccggaa 1620
tattgtgatc caaggagaag tggaggactc atgctacgca gaaaatcagt gccaattttt 1680
tgattatgat acctttgggg gacacattat gataatgaaa aattttactt cagtccatct 1740
ttcttatgtg gaattgaaac acatgggtca gcagcagatg ggcgataccc ctgttcattt 1800
tcacctgtgt ggtgacgtgg attataaagg aggatacaga catgcaacat ttgtggacgg 1860
cctgtctatt catcacagct tctcaaggtg catcactgtg catgggacaa atggcttgct 1920
aataaaagac accattgggt ttgacacact aggtcattgt ttctttttgg aagatggtat 1980
tgaacagagg aatactttgt tccacaatct gggactcctc accaagccgg gtactctcct 2040
gcccaccgat aggaacaact ccatgtgtac caccatgcga gataaagtgt ttggaaatta 2100
cattcctgtg cctgctactg actgtatggc tgtttcaact ttctggattg ctcatcccaa 2160
caataatctg attaataatg cagctgcagg ctcacaggat gctggaatat ggtatttatt 2220
ccacaaggaa ccaactgggg aatccagtgg attgcagctc ttggcaaaac cagaactcac 2280
tccattgggt atattttata acaacagggt ccattcaaat tttaaggctg gcttatttat 2340
tgacaaaggt gtcaaaacaa ccaactctag tgctgctgac ccaagggaat acctctgttt 2400
ggacaatagt gcaagatttc gacctcatca ggatgcaaac cccgaaaaac cacgtgttgc 2460
tgctctaatt gacaggctca ttgcttttaa aaataatgat aatggagctt gggtcagagg 2520
aggagatatt atcgttcaaa attcagcatt tgcagataat ggaataggac tgacctttgc 2580
cagtgatgga agcttcccaa gtgatgaagg ttccagccaa gaggtatctg aatctctctt 2640
tgttggggag agcaggaatt acggctttca gggtggtcag aacaagtatg taggcactgg 2700
aggaatagac cagaagcctc gaacattacc caggaacagg acgttcccaa ttagaggctt 2760
tcagatttat gatgggccca ttcatctcac aaggagcact ttcaaaaaat atgtgccaac 2820
tccagatagg tacagcagtg caattggctt cctcatgaag aattcctggc agataacccc 2880
caggaataat atctccctcg tgaagtttgg tccacatgtc tctctgaatg tcttttttgg 2940
aaagcctggt ccctggtttg aagattgtga gatggatggt gataagaact ccatattcca 3000
tgacattgat ggctctgtga caggatacaa ggatgcttat gtgggaagaa tggacaacta 3060
cctgatccgc catccaagct gtgtaaatgt gtctaagtgg aatgcagtga tctgcagtgg 3120
gacctatgca caggtctatg tacagacatg gagcactcag aatctttcta tgaccattac 3180
acgagatgag tatccgtcca accctatggt gctccgaggt attaatcaga aggctgcctt 3240
tccacagtac cagcctgtcg tcatgctgga gaagggttat accatccact ggaatgggcc 3300
ggcaccacgg actacatttc tatacctcgt caacttcaac aagaatgact ggattcgagt 3360
tggcctttgc tatccatcaa acacaagttt tcaagttacc tttggctatt tgcagcggca 3420
gaatggctca ttatccaaaa tcgaagaata tgagcctgtg cattcactgg aagaactgca 3480
aagaaagcaa tccgagagga aattctattt tgactccagc acggggttac tgtttttgta 3540
tctcaaagcc aaaagccaca ggcatggcca cagttactgt tcatctcagg gatgtgaaag 3600
agtcaagatc caagcagcca cagactcaaa ggacatcagt aactgcatgg ccaaagcata 3660
cccacagtac tacagaaagc cgtcagtggt caagcggatg ccggccatgc tcactggact 3720
ctgtcaaggc tgtggcactc ggcaggtggt gtttactagt gatcctcata aaagttacct 3780
ccctgtgcaa ttccagtcac ctgataaagc agaaacccag cgtggagacc cgtctgttat 3840
ttctgtcaat ggcactgact ttaccttccg aagtgcaggc gtcctcctcc ttgttgtgga 3900
tccgtgcagc gttccattcc gcttgacgga aaaaacggtt tttcctcttg ctgatgtcag 3960
tcgcattgaa gagtatttaa aaacaggcat ccctccaagg tccattgttc tgttgagcac 4020
aagaggagaa ataaagcagt taaacatttc acacttacta gtacctctgg gattagccaa 4080
accagctcat ctttatgaca aagggagtac catatttttg ggattcagtg gaaactttaa 4140
accatcatgg actaagctat ttaccagtcc tgctggacag ggccttgggg tgcttgaaca 4200
attcatacct ttgcagctgg acgaatatgg ttgtcccaga gccaccactg tccgcagaag 4260
agacctggaa ctgctaaagc aagcttcaaa agcacattag agactaactg taacttaagt 4320
gctgggggaa aaaaaatgtg aactaactta tttaatttat ggcattttaa aatgacactg 4380
ttaacccaac ggaaccattt tccagtttga tacagaatgg ggagaaaaga aagcgtttga 4440
aattattgct tggataccag cttcatgcac cttctagttg tacaaaatgt taaagacgtt 4500
gtttgtattt gtaaggctgg tgtattcaga gagcagatct cttattcctc actttccacc 4560
cccgtatttt gtaatgacca tgagcaatgt ttttactttt tgtataatgg ggtggggtgg 4620
agtgggggct tctgagagtc agcctgaggt ctttagagga ccagctattg tagcaccttg 4680
gatacttgaa gtttaatgct cagttgggtc gggtggcagt tgacttggtg gctggcatgt 4740
tcagcagtgc ctggggccct gtttctgggc agcctttgag gattttctat gatattgaat 4800
gacagtttta agtggcaact caggcccagc tcatgccctt ttttgcctgg acatgtgcta 4860
tttttattca cttatatatc aattacttgt aagggttaaa ctttcaaaca ggaagtatat 4920
tgggacaaaa gggctcttgg ggattagata tccctttaat ctgtgaccat ggggcaaaaa 4980
attttcctgc agcaaaagtc tgaggctgtt gggaccattt ttgcagcttt aatccttagc 5040
ctcttttgac tgtatatttg tgtttaaaat gcagagctca actgaatatt tccttttttg 5100
ttttgttttg ttttgtttta agaagtaggt tgttttcctg aaccgtaaac ttgtatcatt 5160
ttaacttgca caaaggaagt ctgttcttgg tattgctctt gcacttgggt tttttgttat 5220
tgttttgtgt ggatttttta aagcttttct gttcaccctc ctgccaggaa aatcccagaa 5280
agcttaatga taccccaaaa tgattacacc cagggaggaa aaaaaggagc gctttctagg 5340
gtcagaatcg tggagagaat actcagaaca tgaacctctt taaagccttg caggaatgag 5400
tcactcttac ttaatgaaat gttaaagcca attaaaaagc atgctgtgat gcccagcttc 5460
cctttccaca gggtgcatgc gtctcctgct ggtgaatcac atgcggcaag aggcaactgg 5520
ctccacagcc tgggatgctg ccgtaccaag aggaaagaag cagcaaaatg cctttacgtt 5580
gttctaaacc cccgacgcat aaagtgtaga ggagggatgg ccaagggtgg gtggtagaaa 5640
gtgtgttcag gctgacactg gcaatgagta cagataattt cactttcctc ttccaggggc 5700
aaaggctgat ggcctctacc tttgtatcca ggagaaactg cagagcagcc ctgtgacttt 5760

acaaaatatg ctacctcaaa gtgctaccga taaacctttc taattgtaag tgcccttact 5820
aagggcacat gtcttaatca aagttagttt tttgttttct ggtttgtttt tttttttttt 5880
gtatattgat gaatgagatc ttacctatta aatatattat tggaaaaaaa aaaaaaaaaa 5940
aaattaaagt ttgagtgtgt gtgtgtgtgt gtgtgtgtgt gtgtgtgtgt gtgttttatg 6000
acttaaatat ctttacgtgt gttttttaga gcttggttct ttaaagattt ggagaagata 6060
tgtaaattac caaggcactt ggtttttctg ttttatatac taataatcag ggcctaagtt 6120
aaataaaaat atgtgtgcat gtatttt 6147

<210> 44
<211> 4357
<212> DNA
<213> Homo sapiens
<220>
<221> misc_feature
<223> séquence appelée GS-N43

<300>
<308> GENBANK/AB029316
<309> 2001-03-14
<313> (1)..(4357)
<400> 44
atagtcacca gaagctggaa gagtcaaagg acacattctc ccctcaagcc ccagtgggag 60
cacggcccag ctggattttg gacttctggc ctccagaact agacagggcc tcacggtgtc 120
acccagggtg gaatacagtg gtgtgatcat agctcactgc agcctggaat tcctgggctc 180
aagcaaccct gccacctcag ccttccaagt agctaggact acagaacatc catgatagca 240
gtcttctgta aatcgaactt ttcaagaatt ctctgaagga accaagtagg atattcttac 300
atcatgactt aatgtgaatg caagaacaag aaataggttt tatctctaaa tataatgaag 360
ggctgtgtgt aaacactgac cctgtctcaa ttctaacaag cattttagac atgagtttac 420
atcggcaaat gggttcagat cgagatcttc agtcctctgc ttcatctgtg agcttgcctt 480
cagtcaaaaa ggcacccaaa aaaagaagaa tttcaatagg ctccctgttt cggaggaaaa 540
aagataacaa acgtaaatca agggagctaa atggcggggt ggatggaatt gcaagtattg 600
aaagtataca ttctgaaatg tgtactgata agaactccat tttctctaca aatacctctt 660
ctgacaatgg attaacttcc atcagcaaac aaattggaga cttcatagag tgccctttgt 720
gccttttgcg gcattctaaa gacagatttc ctgatataat gacttgtcat cacagatctt 780
gtgtggattg cttacgacaa tatttaagga tagaaatctc tgaaagcaga gttaatatta 840
gttgcccaga atgtactgag cggtttaatc cccatgatat tcgcttgata ttaagtgatg 900
atgtcttgat ggaaaaatac gaagaattta tgcttagacg gtggcttgtt gcagatcctg 960
attgtaggtg gtgtccagct ccagactgtg gatatgctgt gatagcattt ggatgtgcca 1020
gctgtccaaa attaacttgt gggcgagagg gctgtggaac agagttttgc taccactgta 1080
aacagatttg gcaccccaac cagacctgtg atgctgctcg acaagagaga gcccagagct 1140
tacgtttgag aactatacgt tcttcatcca ttagttatag tcaagagtct ggagcagcag 1200
ctgatgatat aaagccatgt ccacgatgtg ctgcttatat aataaagatg aatgatggga 1260
gctgcaatca catgacatgt gctgtttgtg gttgtgagtt ttgttggttg tgtatgaaag 1320
aaatctcaga tttgcattat ctaagtccat caggatgtac tttttggggg aagaaaccct 1380
ggagccgaaa gaagaaaata ttgtggcaac tgggaacact ggttggtgct cctgtcggaa 1440
tcgctttaat agctggcatt gctattcctg caatgattat tggcattcct gtgtatgtgg 1500
gccgcaagat tcacaatcgc tatgaaggca aggatgtttc aaagcacaaa cggaatttgg 1560
ccatagcagg tggtgtaacg ttgtctgtaa tcgtgtctcc agtagtagct gcagtgactg 1620
taggtatcgg tgttcctatt atgttagctt atgtctatgg cgtagttcca atttctcttt 1680
gtcgaagcgg aggttgtgga gtctcagcag gcaatggaaa aggagttagg attgaatttg 1740
atgatgaaaa tgatataaat gttggtggaa ctaacacagc tgtagacaca acatcagtag 1800
cagaagcaag acacaaccca agcatagggg agggaagtgt tggtgggctg actggcagtt 1860
tgagtgcaag tggaagccac atggatcgaa taggagccat ccgagacaac ctgagtgaaa 1920
cggccagcac catggcacta gctggagcca gtataacggg gagtctgtca ggaagtgcca 1980
tggtaaactg ttttaacagg ttggaagtac aagcagatgt acagaaagaa cggtacagtc 2040
taagtggaga atctggcaca gtcagcttgg gaacagttag tgataatgcc agcaccaaag 2100
caatggcagg atccattctg aattcctaca tcccattgga caaagaaggc aacagtatgg 2160
aggtgcaagt agatattgag tcaaagccat ccaaattcag gcacaacagt ggaagcagta 2220
gtgtggatga tggcagtgcc acccgaagtt atgctggcgg ttcatccagt ggcttgcctg 2280
aaggtaaatc tagtgccacc aagtggtcca aagaagcaac agcagggaaa aaatcaaaaa 2340
gtggtaaact gaggaaaaag ggtaacatga agataaatga gacgagagag gacatggatg 2400
cacagttgtt agaacaacaa agcacgaact caagtgaatt tgaggctcca tccctcagtg 2460
acagtatgcc ttctgtagca gattctcact ctagtcattt ttctgaattt agttgttctg 2520
acctagaaag catgaaaact tcttgtagtc atggttccag tgattatcac acccgctttg 2580
ctactgttaa cattcttcct gaggtagaaa atgaccgtct ggaaaattcc ccacatcagt 2640
gtagcatttc tgtggttacc caaactgctt cctgttcaga agtttcacag ttgaatcata 2700
ttgctgaaga acatggtaac aatggaataa aacctaatgt tgatttatat tttggcgatg 2760
cactaaaaga aacaaataac aaccactcac atcagacaat ggaattaaaa gttgcaattc 2820
agactgaaat ttaggcccat aaatgctgca gaataattac cactgtacaa ccgtgtttgg 2880
agctggttga actacatgtg actacttaag tttcaggtta ccagcaaaag ccgggtttca 2940
ttatcataat gcagatacat ttctgtgttt cagcaaggca ttgtgtgtca tgtggatctt 3000
agttaccaaa ctatgaagtg aaggctttaa aagtgcatta ttttaaggat aataaatttg 3060
aagagcaaag catgttttgt gtgtttgcca caaaacattg cttgaagcac atacttagat 3120

agaaattggt cttaatttat ataatcaata taaaatacta atgcaattct acagcattca 3180
aatgaagaaa acttgaggct ttagggataa gtggttagtg atattttatt gaaaccacta 3240
aagagataag tttaaaagaa ctgcataggt tactctcagt atatgatact ctgtaacatt 3300
tctatttata tcggcataaa tttcattttt tttcttcata tgcaatgtgg ttatataaag 3360
cttaatgcag ctcatttgct accatttgga tacttagaca ctttgagcaa gattgtggca 3420
gtttttgcac aactttgaaa tagaaatacc tggtactcta tcttgtttat tgttgatgcc 3480
atcttagagg aaaaaatgta aaggtaagta attaagcata tgacagcaac aaataagata 3540
cataaaacta caaaataaag tcccattagg ttataagtat tacaaaaaat ccacctttct 3600
ctaaggggaa gtttgtaccc cattgattct tggtgccttt gggatcgact gggttttaat 3660
ggcctagtta tttgaggatt ttgctgtgtt gttttccatg tcttctctgg tcaccttgga 3720
ttatatataa aaatacagga aatagataaa catgaatgtg attaataatg ctgaaaaagt 3780
attagcctac caaagacaca ctcaggcttt agtgaataac tttacataac ctcagttttt 3840
aacacatgca tatcttctcc aaccatgaaa tcaaagcacg gtgcagaact tgtaccaagt 3900
acaaaaggtc catgtatgat tagcattatt ttcttttgct tttgtttatg gacaatgttc 3960
agctgacata agcagaagtt ggccaaaata ctgcctgtac tgttaatttc ctgtataatt 4020
cacttaaata aaagcaggtt aacctcaatg atagcagtta aaatgttcta tcttatgtat 4080
ttcttttaag tattaccatt atggtgctac tgagcgtttt cttttggtaa aaagaaaaat 4140
gccatgggct gcagtcttct tccatcactt ttccctacca ggtccattaa tatgcttata 4200
acactagtgc cagttatttt atttgataat gcttatggta tttgtatatt tgtttgcatt 4260
ccaattttgt ttaataatga gtgtgtaaac tgcatacgtt aaataaatgt aaatactaat 4320
gtactgctgc aaaaaaaaaa aaaaaaaaaa aaaaaaa 4357

<210> 45
<211> 1801
<212> DNA
<213> Homo sapiens
<220>
<221> misc_feature
<223> séquence appelée GS-N44

<300>
<308> GENBANK/XM_032382
<309> 2001-12-10
<313> (1)..(1801)
<400> 45
cttccccctc ccgcgcgccc gcccgccgcc tgccgccgcc gccgccgccg ccggagctct 60
gtagtatggc atcgaggaga atggagacca aacctgtgat aacctgtctc aaaaccctcc 120
tcatcatcta ctccttcgtc ttctggatca ctggggtgat cctgctggct gttggagtct 180
ggggcaaact tactctgggc acctatatct cccttattgc cgagaactcc acaaatgctc 240
cctatgtgct catcggaact ggcaccacta ttgttgtctt tggcctgttt ggatgctttg 300
ctacatgtcg tggtagccca tggatgctga aactgtatgc catgtttctg tccctggtgt 360
tcctggctga gctcgtagct ggcatttcag ggtttgtgtt tcgtcatgag atcaaggaca 420
ccttcctgag gacttacacg gacgctatgc agacttacaa tggcaatgat gagaggagcc 480
gggcagtgga ccatgtgcag cgcagcctga gctgctgtgg tgtgcagaac tacaccaact 540
ggagcaccag cccctacttc ctggagcatg gcatcccccc cagctgctgc atgaacgaaa 600
ctgattgtaa tccccaggat ctacacaatc tgactgtggc cgccaccaaa gttaaccaga 660
agggttgtta tgatctggta actagtttca tggagactaa catgggaatc atcgctggag 720
tggcgtttgg aatcgcattc tcccagttaa ttggcatgct gctggcctgc tgtctgtccc 780
ggttcatcac ggccaatcag tatgagatgg tgtaaggaga agtctttcaa gaatgacgga 840
ataagagacc tgttttaaaa aggaactgca gcaatctttg aaagacttcc aaagaatgtt 900
agagcacagt acataataca cttgccctgc tccctctccc ccttacccca caacgtgcaa 960
ctgacactcc cacccagtct ctgctccacc tttcagccca cgtcacgtgt agtgtccatt 1020
ttgtgaagcc ctgttgtgcc acagagtgta gccaggtccc cctgcagcta gtcctagtga 1080
acctcacccc gaggccctgc atgggccagc ccctccatct gtacttggtc caactgcaac 1140
tcatcatcgg tgactggtta tcacaccatc gctggcccct ttgggccctg catgtagtgt 1200
gggaggctcc tgttagctcc tcactgtggt aaatgccaca cacctttaag tagataagca 1260
gacgatagtt atctgttctt ttgacttaat ctcatttggt ttgattttcc ctctactaag 1320
gctttcctac cttcttcagg ctgcctaaga catgtaacga aacacttcaa taattgtcca 1380
tgaggagaaa aaaagcatgt gtcatgcatg aaggaaactg aacttgaggt ggcctccttg 1440
cttgttacat acctgggtat gtgtaggcag tttagtgcat ctttgcctct cggttgaaac 1500
ctgtataacc ctgttacaaa gctgtgttgt tgcttcttgt gaaggccatg atattttgtt 1560
tttccccaat taattgctat tgtgttattt tactacttct ctctgtattt tttcttgcat 1620
tgacattata gacattgagg acctcatcca aacaatttaa aaatgagtgt gaagggggaa 1680
caagtcaaaa tatttttaaa agatcttcaa aagtaatgcc tctgtctagc atgccaacaa 1740
gaatgcattg atattgtgaa catttgtgat atatgtatta ataaatagag caattacaag 1800
c 1801

<210> 46
<211> 2081
<212> DNA
<213> Homo sapiens
<220>
<221> misc_feature

<223>  séquence appelée GS-N45

<300>
<308>  GENBANK/AJ133133
<309>  2000-01-19
<313>  (1)..(2081)
<400>  46
cccgggtgta cgtgtaaaat tatgatcaaa taaatttgta tgccttttct cctattaacc      60
tgcctttttt gtcagcgatt gtcagtgaaa cttcagaggg caaaggggaa gttttccttg     120
gcccctccag ttttggtgct gtgaacagga taccaaagct gctctgttct tctggaagct     180
gcaatgaagg gaaccaagga cctgacaagc cagcagaagg agtctaacgt gaagacattt     240
tgctccaaga atatcctagc catccttggc ttctcctcta tcatagctgt gatagctttg     300
cttgctgtgg ggttgaccca gaacaaagca ttgccagaaa acgttaagta tgggattgtg     360
ctggatgcgg gttcttctca cacaagttta tacatctata agtggccagc agaaaaggag     420
aatgacacag gcgtggtgca tcaagtagaa gaatgcaggg ttaaaggtcc tggaatctca     480
aaatttgttc agaaagtaaa tgaaataggc atttacctga ctgattgcat ggaaagagct     540
agggaagtga ttccaaggtc ccagcaccaa gagacacccg tttacctggg agccacggca     600
ggcatgcggt tgctcaggat ggaaagtgaa gagttggcag acagggttct ggatgtggtg     660
gagaggagcc tcagcaacta cccctttgac ttccagggtg ccaggatcat tactggccaa     720
gaggaaggtg cctatggctg gattactatc aactatctgc tgggcaaatt cagtcagaaa     780
acaaggtggt tcagcatagt cccatatgaa accaataatc aggaaacctt tggagctttg     840
gaccttgggg gagcctctac acaagtcact tttgtacccc aaaaccagac tatcgagtcc     900
ccagataatg ctctgcaatt tcgcctctat ggcaaggact acaatgtcta cacacatagc     960
ttcttgtgct atgggaagga tcaggcactc tggcagaaac tggccaagga cattcaggtt    1020
gcaagtaatg aaattctcag ggacccatgc tttcatcctg gatataagaa ggtagtgaac    1080
gtaagtgacc tttacaagac cccctgcacc aagagatttg agatgactct tccattccag    1140
cagtttgaaa tccagggtat tggaaactat caacaatgcc atcaaagcat cctggagctc    1200
ttcaacacca gttactgccc ttactcccag tgtgccttca atgggatttt cttgccacca    1260
ctccaggggg attttggggc attttcagct ttttactttg tgatgaagtt tttaaacttg    1320
acatcagaga aagtctctca ggaaaaggtg actgagatga tgaaaaagtt ctgtgctcag    1380
ccttgggagg agataaaaac atcttacgct ggagtaaagg agaagtacct gagtgaatac    1440
tgcttttctg gtacctacat tctctccctc cttctgcaag gctatcattt cacagctgat    1500
tcctgggagc acatccattt cattggcaag atccagggca gcgacgccgg ctggactttg    1560
ggctacatgc tgaacctgac caacatgatc ccagctgagc aaccattgtc cacacctctc    1620
tcccactcca cctatgtctt cctcatggtt ctattctccc tggtcctttt cacagtggcc    1680
atcataggct tgcttatctt tcacaagcct tcatatttct ggaaagatat ggtatagcaa    1740
aagcagctga aatatgctgg ctggagtgag gaaaaaatcg tccagggagc attttcctcc    1800
atcgcagtgt tcaaggccat ccttccctgt ctgccagggc cagtcttgac gagtgtgaag    1860
cttccttggc ttttactgaa gcctttcttt tggaggtatt caatatcctt tgcctcaagg    1920
acttcggcag atactgtctc tttcatgagt ttttcccagc tacacctttc tcctttgtac    1980
tttgtgcttg tataggtttt aaagacctga cacctttcat aatctttgct ttataaaaga    2040
acaatattga ctttgtctag aaaaaaaaaa aaaaaaaaaa a                        2081

<210>  47
<211>  4332
<212>  DNA
<213>  Homo sapiens
<220>
<221>  misc_feature
<223>  séquence appelée GS-N46

<300>
<308>  GENBANK/AJ306399
<309>  2001-10-15
<313>  (1)..(4332)
<400>  47
ccccgccccg ctctttcgct tcccgggccg ccggcagccg ccgccagccg cagccatggg      60
ccgggcccgg ccgggccaac gcgggccgcc cagccccggc cccgccgcgc agcctcccgc     120
gccaccgcgc cgccgcgccc gttccctggc gctgctcgga gccctgctgg ccgccgccgc     180
tgccgccgcc gtccgggtct gcgcccgcca cgccgaggcc caggcggccg cgcggcagga     240
actggcgctg aagaccctgg ggacagatgg cctttttctc ttttcctcct tggacactga     300
cggggatatg tacatcagcc ctgaggagtt caaacccatt gctgagaagc taacagggtc     360
ttgttctgtc acccagactg gagtgcagtg gtgcagtcac agctcactgc agcctcaact     420
tccctggctc aattgatcct cctgcctcag cctcctgagg tcaactcccg cggccagcta     480
cgaggaggag gagttgcccc ctgaccctag cgaggagacg ctcaccatag aagcccgatt     540
ccagcctctg ctcccggaga ccatgaccaa gagcaaagat ggcttcctag gggtctcccg      600
cctcgccctg tccggcctcc gaaactggac agccgccgcc tcaccaagtg cagtgtttgc     660
cacccgccac ttccagccct tccttccccc gccaggccag gagctgggtg agccctggtg     720
gatcatcccc agtgagctga gcatgttcac tggctacctg tccaacaacc gcttctatcc     780
gccgccgccc aagggcaagg aggtcatcat ccaccggctc ctgagcatgt tccaccctcg     840
gccctttgtg aagacccgct ttgcccctca gggagctgtg gcctgcctga ctgccatcag     900
cgacttctac tacactgtga tgttccggat ccatgccgag ttccagctca gtgagccgcc     960
cgacttcccc ttttggttct cccctgctca gttcaccggc cacatcatcc tctccaaaga    1020

cgccacccac gtccgcgact tccggctctt cgtgcccaac cacaggtctc tgaatgtgga 1080
catggagtgg ctttacgggg ccagtgaaag cagcaacatg gaggtggaca tcggctacat 1140
accccagatg gagctggagg ccacgggccc ctctgtgccc tccgtgatcc tggatgagga 1200
tggcagcatg atcgacagcc acctgccttc aggggagccc ctgcagtttg tgtttgagga 1260
gatcaagtgg cagcaggagc tgagctggga ggaggctgcc cggcgcctgg aggtggccat 1320
gtaccccttc aagaaggtct cctacttgcc gttcactgag gccttcgacc gagccaaggc 1380
tgagaacaag ctggtgcact caatcctgct gtgggggccc ctggatgacc agtcctgctg 1440
aggttcaggg cggactctcc gggagactgt cctggaaagt tcgcccatcc tcaccctgct 1500
caacgagagc ttcatcagca cctggtccct ggtgaaggag ctggaggaac tgcagaacaa 1560
acaggagaac tcgtcccacc agaagctggc tggcctgcac ctggagaagt acagcttccc 1620
cgtggagatg atgatctgcc tgcccaatgg caccgtggtc catcacatca atgccaacta 1680
cttcttggac atcacctccg tgaagcccga ggaaatcgag agcaatctct tcagcttctc 1740
atccaccttt gaagacccgt ccacggccac ctacatgcag ttcctgaagg agggactccg 1800
gcgtggcctg cccctcctcc agccctagag tgcctggacg ggatctgatg cacaggcccc 1860
cacgcctcag agccagagtg gtcctcagcc catttcagac tgcagatgcc gcccactccc 1920
accccactcc taggctgcct tggagggtac aagatccact gagggtggcc accacagcct 1980
tggctccatg gtggcgggta gacaagggat gcctgggctg actgggcaga ggaacctcta 2040
gctctgactg tcactcggct ctccctaccc atttggctct ggaagctgct tggccccccc 2100
agatcagggc ctgggtgaac tccctggacc tttcctagcc agccgcacag tctaggccct 2160
tgtggggtga agaatggagg gaggagcagg ctaggaagac ggggccacca ccctctcctt 2220
gctttcagcc cttcccacag gaaacatcaa gaagccccag ccaggagggg ccaggctgcc 2280
aaggcggctc ccctgtttat ctagagcctt cgttcctggc cataccccgg actgccctcc 2340
tgtgcctgat gtccccagct ggggtcagtc tcaacaggag ccagtcttct ggagcctctg 2400
ggcagaaccc tccatcagag tggaaatcag acgggacccc ctgcagcttc cctgaccacg 2460
ccactgacca gctatctggg gaagtttact gtgaaggggt ttctgccttt agcaatgggg 2520
ttcactaagg gggttcccga ggcccagggc caaggcactc ccaccgccta ccttagcaca 2580
gggtctctgc aggactgcgg gagccagcgc tcctgccgcc cctcttgccc ctcagacctt 2640
gcatccacag aagcacaacc cagccaaaca ccacagcctt ctccagagcc ggcactgtcc 2700
cggcaaccag ggtgccccca ggctagctct tctacctctg ggcaccacgg gactcccctt 2760
ggccactctt gggactttgg tccacgtcct gagccactga ccacggccag tctctctttt 2820
tatatgtgca gaaaagtgtt tttacacaaa ctttctcatg gtttgtaggt atttttttat 2880
aaccccagtg ctgaggagaa aggaggggca gtggcttccc cggcagcagc cccatgatgg 2940
ctgaatccga aatcctcgat gggtccagct tgatgtcttt gcagctgcac ctatgggaag 3000
aagtagtcct ctcttccttc tcctcttcag ctttttaaaa acagtcctca gaggatccat 3060
gatccccagc actgtcccat cctccacaaa ggcccacagg catgcctgta ctctctttca 3120
ttaaggtctt gaagtcaggc tgccccctcc ccagccccca gttctctccc caccccctca 3180
ccccacccgg ggctcactca gcctggcaga ggaagaagga aggcagacat ctccgcagcc 3240
actcctgggc cttttatgtg ccgagttacc ccacttgcct tgggcgtgtc cactgagcct 3300
tccccagcca gtcttgttct caattttgtt ttgttttgtt ttgagacgga gtcttgctct 3360
gtcacccagg ctggagtgct atggctcgat cttggctcac tgcaacctcc acctcccagg 3420
ttcaagcaat tctcttgcct cagcctcccg agtagctggg attacaggtg catgccacca 3480
tggctggcta atttttgtat ttttagtaga gatggggttt caccatattg gtcaggctga 3540
tctggaactt ctgacctcag gtgatccacc tgcctcagcc tcccaaagtg ctgggattac 3600
aggcgtgagc aatcgtgccc agccttgttc ttaattttgt atcatccagt catcgctaat 3660
attacacgca ccttctcact taatcctcac gacaagcctg tgaggcagat gctcattgtt 3720
cccatcttga tgaaacttga gtctcaggga agtgaagtga cttgcccagg gtcactcagg 3780
tagagttgag attcaaaccc acatgtggct ccaaagtctg catctggatt tgggggtgtt 3840
ttttggcatg gcaccctcac ctctctccct gcctgttttc cccaaagtgg aaaggaaggc 3900
ctttcaaacc agagtgtctc actcccctct gacctccaga ccagatgggg catgagccag 3960
ccagctcagc caggctccct gtgtcctggg aggaagtgtc cccatccccc atgcccctta 4020
tggggaggga gggcgtctga tgctctctct ctgcctcccc ccccatcctg tcaggcacag 4080
gtgacggggg cagcccatgc gagcccttct cctgctgctc tgggagggcc agttccacat 4140
tgagccagcc tggtcccatg gaaaatgatg gcctgggctt tctgaggcct tatctgatgc 4200
ctctgcagtt catgtccccc accaggcctc gaggctcagg gtgggagagg gccccgggct 4260
gccctgtcac tcctctaaca cttccctccc ctgtccccaa catgccctgt aataaaatta 4320
gagaagacta ac 4332

<210> 48
<211> 14255
<212> DNA
<213> Homo sapiens
<220>
<221> misc_feature
<223> séquence appelée GS-N47

<300>
<308> GENBANK/L04731
<309> 1993-08-03
<313> (1)..(14255)
<400> 48
gcggcggcgg cggcgggaag cagcggggct ggggttccag ggggagcggc cgccgcctca 60
gcagcctcct cgtcgtccgc ctcgtcttcg tcttcgtcat cgtcctcagc ctcttcaggg 120
ccggccctgc tccgggtggg cccgggcttc gacgcggcgc tgcaggtctc ggccgccatc 180

ggcaccaacc tgcgccggtt ccgggccgtg tttggggaga gcggcggggg aggcggcagc 240
ggagaggatg agcaattctt aggttttggc tcagatgaag aagtcagagt gcgaagtccc 300
acaaggtctc cttcagttaa aactagtcct cgaaaacctc gtgggagacc tagaagtggc 360
tctgaccgaa attcagctat cctctcagat ccatctgtgt tttcccctct aaataaatca 420
gagaccaaat ctggagataa gatcaagaag aaagattcta aaagtataga aaagaagaga 480
ggaagacctc ccaccttccc tggagtaaaa atcaaaataa cacatggaaa ggacatttca 540
gagttaccaa agggaaacaa agaagatagc ctgaaaaaaa ttaaaaggac accttctgct 600
acgtttcagc aagccacaaa gattaaaaaa ttaagagcag gtaaactctc tcctctcaag 660
tctaagttta agacagggaa gcttcaaata ggaaggaagg gggtacaaat tgtacgacgg 720
agaggaaggc ctccatcaac agaaaggata aagacccctt cgggtctcct cattaattct 780
gaactggaaa agccccagaa agtccggaaa gacaaggaag gaacacctcc acttacaaaa 840
gaagataaga cagttgtcag acaaagccct cgaaggatta agccagttag gattattcct 900
tcttcaaaaa ggacagatgc aaccattgct aagcaactct tacagagggc aaaaaagggg 960
gctcaaaaga aaattgaaaa agaagcagct cagctgcagg gaagaaaggt gaagacacag 1020
gtcaaaaata ttcgacagtt catcatgcct gttgtcagtg ctatctcctc gcggatcatt 1080
aagacccctc ggcggtttat agaggatgag gattatgacc ctccaattaa aattgcccga 1140
ttagagtcta caccgaatag tagattcagt gccccgtcct gtggatcttc tgaaaaatca 1200
agtgcagctt ctcagcactc ctctcaaatg tcttcagact cctctcgatc tagtagcccc 1260
agtgttgata cctccacaga ctctcaggct tctgaggaga ttcaggtact tcctgaggag 1320
cggagcgata cccctgaagt tcatcctcca ctgcccattt cccagtcccc agaaaatgag 1380
agtaatgata ggagaagcag aaggtattca gtgtcggaga gaagttttgg atctagaacg 1440
acgaaaaaat tatcaactct acaaagtgcc ccccagcagg agacctcctc gtctccacct 1500
ccacctctgc tgactccacc gccaccactg cagccagcct ccagtatctc tgaccacaca 1560
ccttggctta tgcctccaac aatcccctta gcatcaccat ttttgcctgc ttccactgct 1620
cctatgcaag ggaagcgaaa atctattttg cgagaaccga catttaggtg gacttcttta 1680
aagcattcta ggtcagagcc acaatacttt tcctcagcaa agtatgccaa agaaggtctt 1740
attcgcaaac caatatttga taatttccga ccccctccac taactcccga ggacgttggc 1800
tttgcatctg gtttttctgc atctggtacc gctgcttcag cccgattgtt ttcgccactc 1860
cattctggaa caaggtttga tatgcacaaa aggagccctc ttctgagagc tccaagattt 1920
actccaagtg aggctcactc tagaatattt gagtctgtaa ccttgcctag taatcgaact 1980
tctgctggaa catcttcttc aggagtatcc aatagaaaaa ggaaaagaaa agtgtttagt 2040
cctattcgat ctgaaccaag atctccttct cactccatga ggacaagaag tggaaggctt 2100
agtagttctg agctctcacc tctcaccccc ccgtcttctg tctcttcctc gttaagcatt 2160
tctgttagtc ctcttgccac tagtgcctta aacccaactt ttacttttcc ttctcattcc 2220
ctgactcagt ctggggaatc tgcagagaaa aatcagagac caaggaagca gactagtgct 2280
ccggcagagc cattttcatc aagtagtcct actcctctct tcccttggtt taccccaggc 2340
tctcagactg aaagagggag aaataaagac aaggcccccg aggagctgtc caaagatcga 2400
gatgctgaca agagcgtgga gaaggacaag agtagagaga gagaccggga gagagaaaag 2460
gagaataagc gggagtcaag gaaagagaaa aggaaaaagg gatcagaaat tcagagtagt 2520
tctgctttgt atcctgtggg tagggtttcc aaagagaagg ttgttggtga agatgttgcc 2580
acttcatctt ctgccaaaaa agcaacaggg cggaagaagt cttcatcaca tgattctggg 2640
actgatatta cttctgtgac tcttggggat acaacagctg tcaaaaccaa aatacttata 2700
aagaaaggga gaggaaatct ggaaaaaacc aacttggacc tcggcccaac tgccccatcc 2760
ctggagaagg agaaaaccct ctgcctttcc actccttcat ctagcactgt taaacattcc 2820
acttcctcca taggctccat gttggctcag gcagacaagc ttccaatgac tgacaagagg 2880
gttgccagcc tcctaaaaaa ggccaaagct cagctctgca agattgagaa gagtaagagt 2940
cttaaacaaa ccgaccagcc caaagcacag ggtcaagaaa gtgactcatc agagacctct 3000
gtgcgaggac cccggattaa acatgtctgc agaagagcag ctgttgccct tggccgaaaa 3060
cgagctgtgt ttcctgatga catgcccacc ctgagtgcct taccatggga agaacgagaa 3120
aagattttgt cttccatggg gaatgatgac aagtcatcaa ttgctggctc agaagatgct 3180
gaacctcttg ctccacccat caaaccaatt aaacctgtca ctagaaacaa ggcaccccag 3240
gaacctccag taaagaaagg acgtcgatcg aggcggtgtg ggcagtgtcc cggctgccag 3300
gtgcctgagg actgtggtgt ttgtactaat tgcttagata agcccaagtt tggtggtcgc 3360
aatataaaga agcagtgctg caagatgaga aaatgtcaga atctacaatg gatgccttcc 3420
aaagcctacc tgcagaagca agctaaagct gtgaaaaaga aagagaaaaa gtctaagacc 3480
agtgaaaaga aagacagcaa agagagcagt gttgtgaaga acgtggtgga ctctagtcag 3540
aaacctaccc catcagcaag agaggatcct gccccaaaga aaagcagtag tgagcctcct 3600
ccacgaaagc ccgtcgagga aaagagtgaa gaagggaatg tctcggcccc tgggcctgaa 3660
tccaaacagg ccaccactcc agcttccagg aagtcaagca agcaggtctc ccagccagca 3720
ctggtcatcc cgcctcagcc acctactaca ggaccgccaa gaaaagaagt tcccaaaacc 3780
actcctagtg agcccaagaa aaagcagcct ccaccaccag aatcaggtcc agagcagagc 3840
aaacagaaaa aagtggctcc ccgcccaagt atccctgtaa aacaaaaacc aaaagaaaag 3900
gaaaaaccac ctccggtcaa taagcaggag aatgcaggca ctttgaacat cctcagcact 3960
ctctccaatg gcaatagttc taagcaaaaa attccagcag atggagtcca caggatcaga 4020
gtggacttta aggaggattg tgaagcagaa aatgtgtggg agatgggagg cttaggaatc 4080
ttgacttctg ttcctataac acccagggtg gtttgctttc tctgtgccag tagtgggcat 4140
gtagagtttg tgtattgcca agtctgttgt gagcccttcc acaagttttg tttagaggag 4200
aacgagcgcc ctctggagga ccagctggaa aattggtgtt gtcgtcgttg caaattctgt 4260
cacgtttgtg gaaggcaaca tcaggctaca aagcagctgc tggagtgtaa taagtgccga 4320
aacagctatc accctgagtg cctgggacca aactacccca ccaaacccac aaagaagaag 4380
aaagtctgga tctgtaccaa gtgtgttcgc tgtaagagct gtggatccac aactccaggc 4440
aaagggtggg atgcacagtg gtctcatgat ttctcactgt gtcatgattg cgccaagctc 4500
tttgctaaag gaaacttctg ccctctctgt gacaaatgtt atgatgatga tgactatgag 4560

agtaagatga tgcaatgtgg aaagtgtgat cgctgggtcc attccaaatg tgagaatctt 4620
tcaggtacag aagatgagat gtatgagatt ctatctaatc tgccagaaag tgtggcctac 4680
acttgtgtga actgtactga gcggcaccct gcagagtggc gactggccct tgaaaaagag 4740
ctgcagattt ctctgaagca agttctgaca gctttgttga attctcggac taccagccat 4800
ttgctacgct accggcaggc tgccaagcct ccagacttaa atcccgagac agaggagagt 4860
ataccttccc gcagctcccc cgaaggacct gatccaccag ttcttactga ggtcagcaaa 4920
caggatgatc agcagccttt agatctagaa ggagtcaaga ggaagatgga ccaagggaat 4980
tacacatctg tgttggagtt cagtgatgat attgtgaaga tcattcaagc agccattaat 5040
tcagatggag gacagccaga aattaaaaaa gccaacagca tggtcaagtc cttcttcatt 5100
cggcaaatgg aacgtgtttt tccatggttc agtgtcaaaa agtccaggtt ttgggagcca 5160
aataaagtat caagcaacag tgggatgtta ccaaacgcag tgcttccacc ttcacttgac 5220
cataattatg ctcagtggca ggagcgagag gaaaacagcc acactgagca gcctccttta 5280
atgaagaaaa tcattccagc tcccaaaccc aaaggtcctg gagaaccaga ctcaccaact 5340
cctctgcatc ctcctacacc accaattttg agtactgata ggagtcgaga agacagtcca 5400
gagctgaacc cacccccagg catagaagac aatagacagt gtgcgttatg tttgacttat 5460
ggtgatgaca gtgctaatga tgctggtcgt ttactatata ttggccaaaa tgagtggaca 5520
catgtaaatt gtgctttgtg gtcagcggaa gtgtttgaag atgatgacgg atcactaaag 5580
aatgtgcata tggctgtgat caggggcaag cagctgagat gtgaattctg ccaaaagcca 5640
ggagccaccg tgggttgctg tctcacatcc tgcaccagca actatcactt catgtgttcc 5700
cgagccaaga actgtgtctt tctggatgat aaaaaagtat attgccaacg acatcgggat 5760
ttgatcaaag gcgaagtggt tcctgagaat ggatttgaag ttttcagaag agtgtttgtg 5820
gactttgaag gaatcagctt gagaaggaag tttctcaatg gcttggaacc agaaaatatc 5880
cacatgatga ttgggtctat gacaatcgac tgcttaggaa ttctaaatga tctctccgac 5940
tgtgaagata agctctttcc tattggatat cagtgttcca gggtatactg gagcaccaca 6000
gatgctcgca agcgctgtgt atatacatgc aagatagtgg agtgccgtcc tccagtcgta 6060
gagccggata tcaacagcac tgttgaacat gatgaaaaca ggaccattgc ccatagtcca 6120
acatctttta cagaaagttc atcaaaagag agtcaaaaca cagctgaaat tataagtcct 6180
ccatcaccag accgacctcc tcattcacaa acctctggct cctgttatta tcatgtcatc 6240
tcaaaggtcc ccaggattcg aacacccagt tattctccaa cacagagatc ccctggctgt 6300
cgaccgttgc cttctgcagg aagtcctacc ccaaccactc atgaaatagt cacagtaggt 6360
gatcctttac tctcctctgg acttcgaagc attggctcca ggcgtcacag tacctcttcc 6420
ttatcacccc agcggtccaa actccggata atgtctccaa tgagaactgg gaatacttac 6480
tctaggaata atgtttcctc agtctccacc accgggaccg ctactgatct tgaatcaagt 6540
gccaaagtag ttgatcatgt cttagggcca ctgaattcaa gtactagttt agggcaaaac 6600
acttccacct cttcaaattt gcaaaggaca gtggttactg taggcaataa aaacagtcac 6660
ttggatggat cttcatcttc agaaatgaag cagtccagtg cttcagactt ggtgtccaag 6720
agctcctctt taaagggaga gaagaccaaa gtgctgagtt ccaagagctc agagggatct 6780
gcacataatg tggcttaccc tggaattcct aaactggccc cacaggttca taacacaaca 6840
tctagagaac tgaatgttag taaaatcggc tcctttgctg aaccctcttc agtgtcgttt 6900
tcttctaaag aggccctctc cttcccacac ctccatttga gagggcaaag gaatgatcga 6960
gaccaacaca cagattctac ccaatcagca aactcctctc cagatgaaga tactgaagtc 7020
aaaaccttga agctatctgg aatgagcaac agatcatcca ttatcaacga acatatggga 7080
tctagttcca gagataggag acagaaaggg aaaaaatcct gtaaagaaac tttcaaagaa 7140
aagcattcca gtaaatcttt tttggaacct ggtcaggtga caactggtga ggaaggaaac 7200
ttgaagccag agtttatgga tgaggttttg actcctgagt atatgggcca acgaccatgt 7260
aacaatgttt cttctgataa gattggtgat aaaggccttt ctatgccagg agtccccaaa 7320
gctccaccca tgcaagtaga aggatctgcc aaggaattac aggcaccacg gaaacgcaca 7380
gtcaaagtga cactgacacc tctaaaaatg gaaaatgaga gtcaatccaa aaatgccctg 7440
aaagaaagta gtcctgcttc ccctttgcaa atagagtcaa catctcccac agaaccaatt 7500
tcagcctctg aaaatccagg agatggtcca gtggcccaac caagccccaa taatacctca 7560
tgccaggatt ctcaaagtaa caactatcag aatcttccag tacaggacag aaacctaatg 7620
cttccagatg gccccaaacc tcaggaggat ggctctttta aaaggaggta tccccgtcgc 7680
agtgcccgtg cacgttctaa catgtttttt gggcttaccc cactctatgg agtaagatcc 7740
tatggtgaag aagacattcc attctacagc agctcaactg ggaagaagcg aggcaagaga 7800
tcagctgaag gacaggtgga tggggccgat gacttaagca cttcagatga agacgactta 7860
tactattaca acttcactag aacagtgatt tcttcaggtg gagaggaacg actggcatcc 7920
cataatttat ttcgggagga ggaacagtgt gatcttccaa aaatctcaca gttggatggt 7980
gttgatgatg ggacagagag tgatactagt gtcacagcca caacaaggaa aagcagccag 8040
attccaaaaa gaaatggtaa agaaaatgga acagagaact taaagattga tagacctgaa 8100
gatgctgggg agaaagaaca tgtcactaag agttctgttg gccacaaaaa tgagccaaag 8160
atggataact gccattctgt aagcagagtt aaaacacagg gacaagattc cttggaagct 8220
cagctcagct cattggagtc aagccgcaga gtccacacaa gtaccccctc cgacaaaaat 8280
ttactggaca cctataatac tgagctcctg aaatcagatt cagacaataa caacagtgat 8340
gactgtggga atatcctgcc ttcagacatt atggactttg tactaaagaa tactccatcc 8400
atgcaggctt tgggtgagag cccagagtca tcttcatcag aactcctgaa tcttggtgaa 8460
ggattgggtc ttgacagtaa tcgtgaaaaa gacatgggtc tttttgaagt attttctcag 8520
cagctgccta caacagaacc tgtggatagt agtgtctctt cctctatctc agcagaggaa 8580
cagtttgagt tgcctctaga gctaccatct gatctgtctg tcttgaccac ccggagtccc 8640
actgtcccca gccagaatcc cagtagacta gctgttatct cagactcagg ggagaagaga 8700
gtaaccatca cagaaaaatc tgtagcctcc tctgaaagtg acccagcact gctgagccca 8760
ggagtagatc caactcctga aggccacatg actcctgatc attttatcca aggacacatg 8820
gatgcagacc acatctctag ccctccttgt ggttcagtag agcaaggtca tggcaacaat 8880
caggatttaa ctaggaacag tagcacccct ggccttcagg tacctgtttc cccaactgtt 8940

cccatccaga accagaagta tgtgcccaat tctactgata gtcctggccc gtctcagatt 9000
tccaatgcag ctgtccagac cactccaccc cacctgaagc cagccactga gaaactcata 9060
gttgttaacc agaacatgca gccactttat gttctccaaa ctcttccaaa tggagtgacc 9120
caaaaaatcc aattgacctc ttctgttagt tctacaccca gtgtgatgga gacaaatact 9180
tcagtattgg gacccatggg aggtggtctc acccttacca caggactaaa tccaagcttg 9240
ccaacttctc aatctttgtt cccttctgct agcaaaggat tgctacccat gtctcatcac 9300
cagcacttac attccttccc tgcagctact caaagtagtt tcccaccaaa catcagcaat 9360
cctccttcag gcctgcttat tggggttcag cctcctccgg atccccaact tttggtttca 9420
gaatccagcc agaggacaga cctcagtacc acagtagcca ctccatcctc tggactcaag 9480
aaaagaccca tatctcgtct acagacccga aagaataaaa aacttgctcc ctctagtacc 9540
ccttcaaaca ttgccccttc tgatgtggtt tctaatatga cattgattaa cttcacaccc 9600
tcccagcttc ctaatcatcc aagtctgtta gatttggggt cacttaatac ttcatctcac 9660
cgaactgtcc ccaacatcat aaaaagatct aaatctagca tcatgtattt tgaaccggca 9720
cccctgttac cacagagtgt gggaggaact gctgccacag cggcaggcac atcaacaata 9780
agccaggata ctagccacct cacatcaggg tctgtgtctg gcttggcatc cagttcctct 9840
gtcttgaatg ttgtatccat gcaaactacc acaaccccta caagtagtgc gtcagttcca 9900
ggacacgtca ccttaaccaa cccaaggttg cttggtaccc cagatattgg ctcaataagc 9960
aatcttttaa tcaaagctag ccagcagagc ctggggattc aggaccagcc tgtggcttta 10020
ccgccaagtt caggaatgtt tccacaactg gggacatcac agaccccctc tactgctgca 10080
ataacagcgg catctagcat ctgtgtgctc ccctccactc agactacggg cataacagcc 10140
gcttcacctt ctggggaagc agacgaacac tatcagcttc agcatgtgaa ccagctcctt 10200
gccagcaaaa ctgggattca ttcttcccag cgtgatcttg attctgcttc agggccccag 10260
gtatccaact ttacccagac ggtagacgct cctaatagca tgggactgga gcagaacaag 10320
gctttatcct cagctgtgca agccagcccc acctctcctg ggggttctcc atcctctcca 10380
tcttctggac agcggtcagc aagcccttca gtgccgggtc ccactaaacc caaaccaaaa 10440
accaaacggt ttcagctgcc tctagacaaa gggaatggca agaagcacaa tgtttcccat 10500
ttgcggacca gttcttctga agcacacatt ccagaccaag aaacgacatc cctgacctca 10560
ggcacaggga ctccaggagc agaggctgag cagcaggata cagctagcgt ggagcagtcc 10620
tcccagaagg agtgtgggca acctgcaggg caagtcgctg ttcttccgga agttcaggtg 10680
acccaaaatc cagcaaatga acaagaaagt gcagaaccta aaacagtgga agaagaggaa 10740
agtaatttca gctccccact gatgctttgg cttcagcaag aacaaaagcg gaaggaaagc 10800
attactgaga aaaaacccaa gaaaggactt gtttttgaaa tttccagtga tgatggcttt 10860
cagatctgtg cagaaagtat tgaagatgcc tggaagtcat tgacagataa agtccaggaa 10920
gctcgatcaa atgcccgcct aaagcagctc tcatttgcag gtgttaacgg tttgaggatg 10980
ctggggattc tccatgatgc agttgtgttc ctcattgagc agctgtctgg tgccaagcac 11040
tgtcgaaatt acaaattccg tttccacaag ccagaggagg ccaatgaacc ccccttgaac 11100
cctcacggct cagccagggc tgaagtccac ctcaggaagt cagcatttga catgtttaac 11160
ttcctggctt ctaaacatcg tcagcctcct gaatacaacc ccaatgatga agaagaggag 11220
gaggtacagc tgaagtcagc tcggagggca actagcatgg atctgccaat gcccatgcgc 11280
ttccggcact taaaaaagac ttctaaggag gcagttggtg tctacaggtc tcccatccat 11340
ggccggggtc ttttctgtaa gagaaacatt gatgcaggtg agatggtgat tgagtatgcc 11400
ggcaacgtca tccgctccat ccagactgac aagcgggaaa agtattacga cagcaagggc 11460
attggttgct atatgttccg aattgatgac tcagaggtag tggatgccac catgcatgga 11520
aatgctgcac gcttcatcaa tcactcgtgt gagcctaact gctattctcg ggtcatcaat 11580
attgatgggc agaagcacat tgtcatcttt gccatgcgta agatctaccg aggagaggaa 11640
ctcacttacg actataagtt ccccattgag gatgccagca acaagctgcc ctgcaactgt 11700
ggcgccaaga aatgccggaa gttcctaaac taaagctgct cttctccccc agtgttggag 11760
tgcaaggagg cggggccatc caaagcaacg ctgaaggcct tttccagcag ctgggagctc 11820
ccggattgcg tggcacagct gaggggcctc tgtgatggct gagctctctt atgtcctata 11880
ctcacatcag acatgtgatc atagtcccag agacagagtt gaggtctcga agaaaagatc 11940
catgatcggc tttctcctgg ggcccctcca attgtttact gttagaaagt gggaatgggg 12000
tccctagcag acttgcctgg aaggagccta ttatagaggg ttggttatgt tgggagattg 12060
ggcctgaatt tctccacaga aataagttgc catcctcagg ttggcccttt cccaagcact 12120
gtaagtgagt gggtcagcca aagccccaaa tggagggttg gttagattcc tgacagtttg 12180
ccagccagcc gccacctaca gcgtctgtcg aacaaacaga ggtctggtgg ttttccctac 12240
tgtcctccca ctcgagagtt cacttctggt tgggagacag gattcctagc acctccggtg 12300
tcaaaaggct gtcatggggt tgtgccaatt aattaccaaa cattgagcct gcaggctttg 12360
agtgggagtg ttgcccccag gagccttatc tcagccaatt acctttcttg acagtaggag 12420
cggcttccct ctcccattcc ctcttcactc ccttttcttc ctttcccctg tcttcatgcc 12480
actgctttcc catgcttctt tcggttgtag gggagactga ctgcctgctc aaggacactc 12540
cctgctgggc ataggatgtg cctgcaaaaa gttccctgag cctgtaagca ctccaggtgg 12600
ggaagtggac aggagccatt ggtcataacc agacagaatt tggaaacatt ttcataaagc 12660
tccatggaga gttttaaaga aacatatgta gcatgatttt gtaggagagg aaaaagatta 12720
tttaaatagg atttaaatca tgcaacaacg agagtatcac agccaggatg acccttgggt 12780
cccattccta agacatggtt actttatttt ccccttgtta agacatagga agacttaatt 12840
tttaaacggt cagtgtccag ttgaaggcag aacactaatc agatttcaag gcccacaact 12900
tggggactag accaccttat gttgagggaa ctctgccacc tgcgtgcaac ccacagctaa 12960
agtaaattca atgacactac tgccctgatt actccttagg atgtggtcaa aacagcatca 13020
aatgtttctt ctcttccttt ccccaagaca gagtcctgaa cctgttaaat taagtcattg 13080
gattttactc tgttctgttt acagtttact atttaaggtt ttataaatgt aaatatattt 13140
tgtatatttt tctatgagaa gcacttcata gggagaagca cttatgacaa ggctattttt 13200
taaaccgcgg tattatccta atttaaaaga agatcggttt ttaataattt tttattttca 13260
taggatgaag ttagagaaaa tattcagctg tacacacaaa gtctggtttt tcctgcccaa 13320

cttccccctg gaaggtgtac tttttgttgt ttaatgtgta gcttgtttgt gccctgttga 13380
cataaatgtt tcctgggttt gctctttgac aataaatgga gaaggaaggt cacccaactc 13440
cattgggcca ctcccctcct tcccctattg aagctcctca aaaggctaca gtaatatctt 13500
gatacaacag attctcttct ttcccgcctc tctcctttcc ggcgcaactt ccagagtggt 13560
gggagacggc aatctttaca tttccctcat ctttcttact tcagagttag caaacaacaa 13620
gttgaatggc aacttgacat ttttgcatca ccatctgcct cataggccac tctttccttt 13680
ccctctgccc accaagtcct catatctgca gagaacccat tgatcacctt gtgccctctt 13740
ttggggcagc ctgttgaaac tgaagcacag tctgaccact cacgataaag cagattttct 13800
ctgcctctgc cacaaggttt cagagtagtg tagtccaagt agagggtggg gcaccctttt 13860
ctcgccgcaa gaagcccatt cctatggaag tctagcaaag caatacgact cagcccagca 13920
ctctctgccc caggactcat ggctctgctg tgccttccat cctgggctcc cttctctcct 13980
gtgaccttaa gaactttgtc tggtggcttt gctggaacat tgtcactgtt ttcactgtca 14040
tgcagggagc ccagcactgt ggccaggatg gcagagactt ccttgtcatc atggagaagt 14100
gccagcaggg gactgggaaa agcactctac ccagacctca cctcccttcc tccttttgcc 14160
catgaacaag atgcagtggc cctaggggtt ccactagtgt ctgctttcct ttattattgc 14220
actgtgtgag gtttttttgt aaatccttgt attcc 14255

<210> 49
<211> 11910
<212> DNA
<213> Homo sapiens
<220>
<221> misc_feature
<222> (89)..(89)
<223> n est un résidu inconnu

<220>
<221> misc_feature
<223> séquence appelée GS-N48

<300>
<308> GENBANK/NM_005933
<309> 2001-12-19
<313> (1)..(11910)
<400> 49
atggcgcaca gctgtcggtg gcgcttcccc gcccgacccg ggaccaccgg gggcggcggc 60
ggcggggggc gccggggcct agggggcgnc ccgcggcaac gcgtcccggc cctgctgctt 120
ccccccgggc ccccggtcgg cggtggcggc cccggggcgc ccccctcccc cccggctgtg 180
gcggccgcgg cggcggcggc gggaagcagc ggggctgggg ttccaggggg agcggccgcc 240
gcctcagcag cctcctcgtc gtccgcctcg tcttcgtctt cgtcatcgtc ctcagcctct 300
tcagggccgg ccctgctccg ggtgggcccg ggcttcgacg cggcgctgca ggtctcggcc 360
gccatcggca ccaacctgcg ccggttccgg gccgtgtttg gggagagcgg cgggggaggc 420
ggcagcggag aggatgagca attcttaggt tttggctcag atgaagaagt cagagtgcga 480
agtcccacaa ggtctccttc agttaaaact agtcctcgaa aacctcgtgg gagacctaga 540
agtggctctg accgaaattc agctatcctc tcagatccat ctgtgttttc ccctctaaat 600
aaatcagaga ccaaatctgg agataagatc aagaagaaag attctaaaag tatagaaaag 660
aagagaggaa gacctcccac cttccctgga gtaaaaatca aaataacaca tggaaaggac 720
atttcagagt taccaaaggg aaacaaagaa gatagcctga aaaaaattaa aaggacacct 780
tctgctacgt ttcagcaagc cacaaagatt aaaaaattaa gagcaggtaa actctctcct 840
ctcaagtcta agtttaagac agggaagctt caaataggaa ggaagggggt acaaattgta 900
cgacggagag gaaggcctcc atcaacagaa aggataaaga ccccttcggt ctcctcatta 960
attctgaact ggaaaagccc cagaaagtcc ggaaagacaa ggaaggaaca cctccactta 1020
caaaagaaga taagacagtt gtcagacaaa gccctcgaag gattaagcca gttaggatta 1080
ttccttcttc aaaaaggaca gatgcaacca ttgctaagca actcttacag agggcaaaaa 1140
aagggggctc aaaagaaaat tgaaaaagaa gcagctcagc tgcagggaag aaaggtgaag 1200
acacaggtca aaaatattcg acagttcatc atgcctgttg tcagtgctat ctcctcgcgg 1260
atcattaaga cccctcggcg gtttatagag gatgaggatt atgaccctcc aattaaaatt 1320
gcccgattag agtctacacc gaatagtaga ttcagtgccc cgtcctgtgg atcttctgaa 1380
aaatcaagtg cagcttctca gcactcctct caaatgtctt cagactcctc tcgatctagt 1440
agccccagtg ttgatacctc cacagactct caggcttctg aggagattca ggtacttcct 1500
gaggagcgga gcgatacccc tgaagttcat cctccactgc ccatttccca gtccccagaa 1560
aatgagagta atgataggag aagcagaagg tattcagtgt cggagagaag ttttggatct 1620
agaacgacga aaaaattatc aactctacaa agtgcccccc agcagcagac ctcctcgtct 1680
ccacctccac ctctgctgac tccaccgcca ccactgcagc cagcctccag tatctctgac 1740
cacacacctt ggcttatgcc tccaacaatc cccttagcat caccattttt gcctgcttcc 1800
actgctccta tgcaagggaa gcgaaaatct attttgcgag aaccgacatt taggtggact 1860
tctttaaagc attctaggtc agagccacaa tacttttcct cagcaaagta tgccaaagaa 1920
ggtcttattc gcaaaccaat atttgataat ttccgacccc ctccactaac tcccgaggac 1980
gttggctttg catctggttt ttctgcatct ggtaccgctg cttcagcccg attgttttcg 2040
ccactccatt ctggaacaag gtttgatatg cacaaaagga gccctcttct gagagctcca 2100
agatttactc caagtgaggc tcactctaga atatttgagt ctgtaacctt gcctagtaat 2160
cgaacttctg ctggaacatc ttcttcagga gtatccaata gaaaaaggaa aagaaaagtg 2220
tttagtccta ttcgatctga accaagatct ccttctcact ccatgaggac aagaagtgga 2280

aggcttagta gttctgagct ctcacctctc acccccccgt cttctgtctc ttcctcgtta 2340
agcatttctg ttagtcctct tgccactagt gccttaaacc caacttttac ttttccttct 2400
cattccctga ctcagtctgg ggaatctgca gagaaaaatc agagaccaag gaagcagact 2460
agtgctccgg cagagccatt ttcatcaagt agtcctactc ctctcttccc ttggtttacc 2520
ccaggctctc agactgaaag agggagaaat aaagacaagg cccccgagga gctgtccaaa 2580
gatcgagatg ctgacaagag cgtggagaag gacaagagta gagagagaga ccgggagaga 2640
gaaaaggaga ataagcggga gtcaaggaaa gagaaaagga aaaagggatc agaaattcag 2700
agtagttctg ctttgtatcc tgtgggtagg gtttccaaag agaaggttgt tggtgaagat 2760
gttgccactt catcttctgc caaaaaagca acagggcgga agaagtcttc atcacatgat 2820
tctgggactg atattacttc tgtgactctt ggggatacaa cagctgtcaa aaccaaaata 2880
cttataaaga aagggagagg aaatctggaa aaaaccaact tggacctcgg cccaactgcc 2940
ccatccctgg agaaggagaa aaccctctgc ctttccactc cttcatctag cactgttaaa 3000
cattccactt cctccatagg ctccatgttg gctcaggcag acaagcttcc aatgactgac 3060
aagagggttg ccagcctcct aaaaaaggcc aaagctcagc tctgcaagat tgagaagagt 3120
aagagtctta aacaaaccga ccagcccaaa gcacagggtc aagaaagtga ctcatcagag 3180
acctctgtgc gaggaccccg gattaaacat gtctgcagaa gagcagctgt tgcccttggc 3240
cgaaaacgag ctgtgtttcc tgatgacatg cccaccctga gtgccttacc atgggaagaa 3300
cgagaaaaga ttttgtcttc catggggaat gatgacaagt catcaattgc tggctcagaa 3360
gatgctgaac ctcttgctcc acccatcaaa ccaattaaac ctgtcactag aaacaaggca 3420
ccccaggaac ctccagtaaa gaaaggacgt cgatcgaggc ggtgtgggca gtgtcccggc 3480
tgccaggtgc ctgaggactg tggtgtttgt actaattgct tagataagcc caagtttggt 3540
ggtcgcaata taaagaagca gtgctgcaag atgagaaaat gtcagaatct acaatggatg 3600
ccttccaaag cctacctgca gaagcaagct aaagctgtga aaaagaaaga gaaaaagtct 3660
aagaccagtg aaaagaaaga cagcaaagag agcagtgttg tgaagaacgt ggtggactct 3720
agtcagaaac ctaccccatc agcaagagag gatcctgccc caaagaaaag cagtagtgag 3780
cctcctccac gaaagcccgt cgaggaaaag agtgaagaag ggaatgtctc ggcccctggg 3840
cctgaatcca aacaggccac cactccagct tccaggaagt caagcaagca ggtctcccag 3900
ccagcactgg tcatcccgcc tcagccacct actacaggac cgccaagaaa agaagttccc 3960
aaaaccactc ctagtgagcc caagaaaaag cagcctccac caccagaatc aggtccagag 4020
cagagcaaac agaaaaaagt ggctccccgc ccaagtatcc ctgtaaaaca aaaaccaaaa 4080
gaaaaggaaa aaccacctcc ggtcaataag caggagaatg caggcacttt gaacatcctc 4140
agcactctct ccaatggcaa tagttctaag caaaaaattc cagcagatgg agtccacagg 4200
atcagagtgg actttaagga ggattgtgaa gcagaaaatg tgtgggagat gggaggctta 4260
ggaatcttga cttctgttcc tataacaccc agggtggttt gctttctctg tgccagtagt 4320
gggcatgtag agtttgtgta ttgccaagtc tgttgtgagc ccttccacaa gttttgttta 4380
gaggagaacg agcgccctct ggaggaccag ctggaaaatt ggtgttgtcg tcgttgcaaa 4440
ttctgtcacg tttgtggaag gcaacatcag gctacaaagc agctgctgga gtgtaataag 4500
tgccgaaaca gctatcaccc tgagtgcctg ggaccaaact accccaccaa acccacaaag 4560
aagaagaaag tctggatctg taccaagtgt gttcgctgta agagctgtgg atccacaact 4620
ccaggcaaag ggtgggatgc acagtggtct catgatttct cactgtgtca tgattgcgcc 4680
aagctctttg ctaaaggaaa cttctgccct ctctgtgaca aatgttatga tgatgatgac 4740
tatgagagta agatgatgca atgtggaaag tgtgatcgct gggtccattc caaatgtgag 4800
aatctttcag atgagatgta tgagattcta tctaatctgc cagaaagtgt ggcctacact 4860
tgtgtgaact gtactgagcg gcaccctgca gagtggcgac tggcccttga aaaagagctg 4920
cagatttctc tgaagcaagt tctgacagct ttgttgaatt ctcggactac cagccatttg 4980
ctacgctacc ggcaggctgc caagcctcca gacttaaatc ccgagacaga ggagagtata 5040
ccttcccgca gctcccccga aggacctgat ccaccagttc ttactgaggt cagcaaacag 5100
gatgatcagc agcctttaga tctagaagga gtcaagagga agatggacca agggaattac 5160
acatctgtgt tggagttcag tgatgatatt gtgaagatca ttcaagcagc cattaattca 5220
gatggaggac agccagaaat taaaaaagcc aacagcatgg tcaagtcctt cttcattcgg 5280
caaatggaac gtgtttttcc atggttcagt gtcaaaaagt ccaggttttg ggagccaaat 5340
aaagtatcaa gcaacagtgg gatgttacca aacgcagtgc ttccaccttc acttgaccat 5400
aattatgctc agtggcagga gcgagaggaa aacagccaca ctgagcagcc tcctttaatg 5460
aagaaaatca ttccagctcc caaacccaaa ggtcctggag aaccagactc accaactcct 5520
ctgcatcctc ctacaccacc aattttgagt actgatagga gtcgagaaga cagtccagag 5580
ctgaacccac ccccaggcat agaagacaat agacagtgtg cgttatgttt gacttatggt 5640
gatgacagtg ctaatgatgc tggtcgttta ctatatattg gccaaaatga gtggacacat 5700
gtaaattgtg ctttgtggtc agcggaagtg tttgaagatg atgacggatc actaaagaat 5760
gtgcatatgg ctgtgatcag gggcaagcag ctgagatgtg aattctgcca aaagccagga 5820
gccaccgtgg gttgctgtct cacatcctgc accagcaact atcacttcat gtgttcccga 5880
gccaagaact gtgtctttct ggatgataaa aaagtatatt gccaacgaca tcgggatttg 5940
atcaaaggcg aagtggttcc tgagaatgga tttgaagttt tcagaagagt gtttgtggac 6000
tttgaaggaa tcagcttgag aaggaagttt ctcaatggct tggaaccaga aaatatccac 6060
atgatgattg ggtctatgac aatcgactgc ttaggaattc taaatgatct ctccgactgt 6120
gaagataagc tctttcctat tggatatcag tgttccaggg tatactggag caccacagat 6180
gctcgcaagc gctgtgtata tacatgcaag atagtggagt gccgtcctcc agtcgtagag 6240
ccggatatca acagcactgt tgaacatgat gaaaacagga ccattgccca tagtccaaca 6300
tcttttacag aaagttcatc aaaagagagt caaaacacag ctgaaattat aagtcctcca 6360
tcaccagacc gacctcctca ttcacaaacc tctggctcct gttattatca tgtcatctca 6420
aaggtcccca ggattcgaac acccagttat tctccaacac agagatcccc tggctgtcga 6480
ccgttgcctt ctgcaggaag tcctacccca accactcatg aaatagtcac agtaggtgat 6540
cctttactct cctctggact tcgaagcatt ggctccaggc gtcacagtac ctcttcctta 6600
tcaccccagc ggtccaaact ccggataatg tctccaatga gaactgggaa tacttactct 6660

aggaataatg tttcctcagt ctccaccacc gggaccgcta ctgatcttga atcaagtgcc 6720
aaagtagttg atcatgtctt agggccactg aattcaagta ctagtttagg gcaaaacact 6780
tccacctctt caaatttgca aaggacagtg gttactgtag gcaataaaaa cagtcacttg 6840
gatggatctt catcttcaga aatgaagcag tccagtgctt cagacttggt gtccaagagc 6900
tcctctttaa agggagagaa gaccaaagtg ctgagttcca agagctcaga gggatctgca 6960
cataatgtgg cttaccctgg aattcctaaa ctggccccac aggttcataa cacaacatct 7020
agagaactga atgttagtaa aatcggctcc tttgctgaac cctcttcagt gtcgttttct 7080
tctaaagagg ccctctcctt cccacacctc catttgagag ggcaaaggaa tgatcgagac 7140
caacacacag attctaccca atcagcaaac tcctctccag atgaagatac tgaagtcaaa 7200
accttgaagc tatctggaat gagcaacaga tcatccatta tcaacgaaca tatgggatct 7260
agttccagag ataggagaca gaaagggaaa aaatcctgta aagaaacttt caaagaaaag 7320
cattccagta aatctttttt ggaacctggt caggtgacaa ctggtgagga aggaaacttg 7380
aagccagagt ttatggatga ggttttgact cctgagtata tgggccaacg accatgtaac 7440
aatgtttctt ctgataagat tggtgataaa ggcctttcta tgccaggagt ccccaaagct 7500
ccacccatgc aagtagaagg atctgccaag gaattacagg caccacggaa acgcacagtc 7560
aaagtgacac tgacacctct aaaaatggaa aatgagagtc aatccaaaaa tgccctgaaa 7620
gaaagtagtc ctgcttcccc tttgcaaata gagtcaacat ctcccacaga accaatttca 7680
gcctctgaaa atccaggaga tggtccagtg gcccaaccaa gccccaataa tacctcatgc 7740
caggattctc aaagtaacaa ctatcagaat cttccagtac aggacagaaa cctaatgctt 7800
ccagatggcc ccaaacctca ggaggatggc tcttttaaaa ggaggtatcc ccgtcgcagt 7860
gcccgtgcac gttctaacat gtttttgggg cttaccccac tctatggagt aagatcctat 7920
ggtgaagaag acattccatt ctacagcagc tcaactggga agaagcgagg caagagatca 7980
gctgaaggac aggtggatgg ggccgatgac ttaagcactt cagatgaaga cgacttatac 8040
tattacaact tcactagaac agtgatttct tcaggtggag aggaacgact ggcatcccat 8100
aatttatttc gggaggagga acagtgtgat cttccaaaaa tctcacagtt ggatggtgtt 8160
gatgatggga cagagagtga tactagtgtc acagccacaa caaggaaaag cagccagatt 8220
ccaaaaagaa atggtaaaga aaatggaaca gagaacttaa agattgatag acctgaagat 8280
gctggggaga aagaacatgt cactaagagt tctgttggcc acaaaaatga gccaaagatg 8340
gataactgcc attctgtaag cagagttaaa acacagggac aagattcctt ggaagctcag 8400
ctcagctcat tggagtcaag ccgcagagtc cacacaagta ccccctccga caaaaattta 8460
ctggacacct ataatactga gctcctgaaa tcagattcag acaataacaa cagtgatgac 8520
tgtgggaata tcctgccttc agacattatg gactttgtac taaagaatac tccatccatg 8580
caggctttgg gtgagagccc agagtcatct tcatcagaac tcctgaatct tggtgaagga 8640
ttgggtcttg acagtaatcg tgaaaaagac atgggtcttt ttgaagtatt ttctcagcag 8700
ctgcctacaa cagaacctgt ggatagtagt gtctcttcct ctatctcagc agaggaacag 8760
tttgagttgc ctctagagct accatctgat ctgtctgtct tgaccacccg gagtcccact 8820
gtccccagcc agaatcccag tagactagct gttatctcag actcagggga gaagagagta 8880
accatcacag aaaaatctgt agcctcctct gaaagtgacc cagcactgct gagcccagga 8940
gtagatccaa ctcctgaagg ccacatgact cctgatcatt ttatccaagg acacatggat 9000
gcagaccaca tctctagccc tccttgtggt tcagtagagc aaggtcatgg caacaatcag 9060
gatttaacta ggaacagtag cacccctggc cttcaggtac ctgtttcccc aactgttccc 9120
atccagaacc agaagtatgt gcccaattct actgatagtc ctggcccgtc tcagatttcc 9180
aatgcagctg tccagaccac tccaccccac ctgaagccag ccactgagaa actcatagtt 9240
gttaaccaga acatgcagcc actttatgtt ctccaaactc ttccaaatgg agtgacccaa 9300
aaaatccaat tgacctcttc tgttagttct acacccagtg tgatggagac aaatacttca 9360
gtattgggac ccatgggagg tggtctcacc cttaccacag gactaaatcc aagcttgcca 9420
acttctcaat ctttgttccc ttctgctagc aaaggattgc tacccatgtc tcatcaccag 9480
cacttacatt ccttccctgc agctactcaa agtagtttcc caccaaacat cagcaatcct 9540
ccttcaggcc tgcttattgg ggttcagcct cctccggatc cccaactttt ggtttcagaa 9600
tccagccaga ggacagacct cagtaccaca gtagccactc catcctctgg actcaagaaa 9660
agacccatat ctcgtctaca gacccgaaag aataaaaaac ttgctccctc tagtacccct 9720
tcaaacattg ccccttctga tgtggtttct aatatgacat tgattaactt cacaccctcc 9780
cagcttccta atcatccaag tctgttagat ttggggtcac ttaatacttc atctcaccga 9840
actgtcccca acatcataaa aagatctaaa tctagcatca tgtattttga accggcaccc 9900
ctgttaccac agagtgtggg aggaactgct gccacagcgg caggcacatc aacaataagc 9960
caggatacta gccacctcac atcagggtct gtgtctggct tggcatccag ttcctctgtc 10020
ttgaatgttg tatccatgca aactaccaca acccctacaa gtagtgcgtc agttccagga 10080
cacgtcacct taaccaaccc aaggttgctt ggtaccccag atattggctc aataagcaat 10140
cttttaatca aagctagcca gcagagcctg gggattcagg accagcctgt ggctttaccg 10200
ccaagttcag gaatgtttcc acaactgggg acatcacaga ccccctctac tgctgcaata 10260
acagcggcat ctagcatctg tgtgctcccc tccactcaga ctacgggcat aacagccgct 10320
tcaccttctg gggaagcaga cgaacactat cagcttcagc atgtgaacca gctccttgcc 10380
agcaaaactg ggattcattc ttcccagcgt gatcttgatt ctgcttcagg gccccaggta 10440
tccaacttta cccagacggt agacgctcct aatagcatgg gactggagca gaacaaggct 10500
ttatcctcag ctgtgcaagc cagccccacc tctcctgggg gttctccatc ctctccatct 10560
tctggacagc ggtcagcaag cccttcagtg ccgggtccca ctaaacccaa accaaaaacc 10620
aaacggtttc agctgcctct agacaaaggg aatggcaaga agcacaaagt ttcccatttg 10680
cggaccagtt cttctgaagc acacattcca gaccaagaaa cgacatccct gacctcaggc 10740
acagggactc caggagcaga ggctgagcag caggatacag ctagcgtgga gcagtcctcc 10800
cagaaggagt gtgggcaacc tgcagggcaa gtcgctgttc ttccggaagt tcaggtgacc 10860
caaaatccag caaatgaaca agaaagtgca gaacctaaaa cagtggaaga agaggaaagt 10920
aatttcagct ccccactgat gctttggctt cagcaagaac aaaagcggaa ggaaagcatt 10980
actgagaaaa aacccaagaa aggacttgtt tttgaaattt ccagtgatga tggctttcag 11040

atctgtgcag aaagtattga agatgcctgg aagtcattga cagataaagt ccaggaagct 11100
cgatcaaatg cccgcctaaa gcagctctca tttgcaggtg ttaacggttt gaggatgctg 11160
gggattctcc atgatgcagt tgtgttcctc attgagcagc tgtctggtgc caagcactgt 11220
cgaaattaca aattccgttt ccacaagcca gaggaggcca atgaaccccc cttgaaccct 11280
cacggctcag ccagggctga agtccacctc aggaagtcag catttgacat gtttaacttc 11340
ctggcttcta aacatcgtca gcctcctgaa tacaacccca atgatgaaga agaggaggag 11400
gtacagctga agtcagctcg gagggcaact agcatggatc tgccaatgcc catgcgcttc 11460
cggcacttaa aaaagacttc taaggaggca gttggtgtct acaggtctcc catccatggc 11520
cggggtcttt tctgtaagag aaacattgat gcaggtgaga tggtgattga gtatgccggc 11580
aacgtcatcc gctccatcca gactgacaag cgggaaaagt attacgacag caagggcatt 11640
ggttgctata tgttccgaat tgatgactca gaggtagtgg atgccaccat gcatggaaat 11700
cgtgcacgct tcatcaatca ctcgtgtgag cctaactgct attctcgggt catcaatatt 11760
gatgggcaga agcacattgt catctttgcc atgcgtaaga tctaccgagg agaggaactc 11820
acttacgact ataagttccc cattgaggat gccagcaaca agctgccctg caactgtggc 11880
gccaagaaat gccggaagtt cctaaactaa 11910

<210> 50
<211> 10452
<212> DNA
<213> Homo sapiens
<220>
<221> misc_feature
<223> séquence appelée GS-N49

<300>
<308> GENBANK/U72936
<309> 2000-02-11
<313> (1)..(10452)
<400> 50
ctcggcccaa caaaatggcg gcggcagcgg tgtcgctttg tttccgcggc tcctgcggcg 60
gtggcagtgg tagcggcctt tgagctgtgg ggaggttcca gcagcagcta cagtgacgac 120
taagactcca gtgcatttct atcgtaaccg ggcgcggggg agcgcagatc ggcgcccagc 180
aatcacagaa gccgacaagg cgttcaagcg aaaacatgac cgctgagccc atgagtgaaa 240
gcaagttgaa tacattggtg cagaagcttc atgacttcct tgcacactca tcagaagaat 300
ctgaagaaac aagttctcct ccacgacttg caatgaatca aaacacagat aaaatcagtg 360
gttctggaag taactctgat atgatggaaa acagcaagga agagggaact agctcttcag 420
aaaaatccaa gtcttcagga tcgtcacgat caaagaggaa accttcaatt gtaacaaagt 480
atgtagaatc agatgatgaa aaacctttgg atgatgaaac tgtaaatgaa gatgcgtcta 540
atgaaaattc agaaaatgat attactatgc agagcttgcc aaaaggtaca gtgattgtac 600
agccagagcc agtgctgaat gaagacaaag atgattttaa agggcctgaa tttagaagca 660
gaagtaaaat gaaaactgaa aatctcaaaa aacgcggagt gctgtggtga tatccttata 720
tttgattcct tgtgcacatg tgggagaaaa tctccaaaga attacctagt tttgcttaat 780
tcttgagcga tgtatgcacc aaagcacata gaagatactt aaagatgggc ttcatgggat 840
tgtgagctgc actgcttgtg gacaacaggt caatcatttt caaaaagatt ccatttatag 900
acacccttca ttgcaagttc ttatttgtaa gaattgcttt aagtattaca tgagtgatga 960
tattagccgt gactcagatg gaatggatga acaatgtagg tggtgtgcgg aaggtggaaa 1020
cttgatttgt tgtgactttt gccataatgc tttctgcaag aaatgcattc tacgcaacct 1080
tggtcgaaag gagttgtcca caataatgga tgaaaacaac caatggtatt gctacatttg 1140
tcacccagag cctttgttgg acttggtcac tgcatgtaac agcgtatttg agaatttaga 1200
acagttgttg cagcaaaata agaagaagat aaaagttgac agtgaaaaga gtaataaagt 1260
atatgaacat acatccagat tttctccaaa gaagactagt tcaaattgta atggagaaga 1320
aaagaaatta gatgattcct gttctggctc tgtaacctac tcttattccg cactaattgt 1380
gcccaaagag atgattaaga aggcaaaaaa actgattgag accacagcca acatgaactc 1440
cagttatgtt aaatttttaa agcaggcaac agataattca gaaatcagtt ctgctacaaa 1500
attacgtcag cttaaggctt ttaagtctgt gttggctgat attaagaagg ctcatcttgc 1560
attggaagaa gacttaaatt ccgagtttcg agcgatggat gctgtaaaca aagagaaaaa 1620
taccaaagag cataaagtca tagatgctaa gtttgaaaca aaagcacgaa aaggagaaaa 1680
accttgtgct ttggaaaaga aggatatttc aaagtcagaa gctaaacttt caagaaaaca 1740
ggtagatagt gagcacatgc atcagaatgt tccaacagag gaacaaagaa caaataaaag 1800
taccggtggt gaacataaga aatctgatag aaaagaagaa cctcaatatg aacctgccaa 1860
cacttctgaa gatttagaca tggatattgt gtctgttcct tcctcagttc cagaagacat 1920
ttttgagaat cttgagactg ctatggaagt tcagagttca gttgatcatc aaggggatgg 1980
cagcagtgga actgaacaag aagtggagag ttcatctgta aaattaaata tttcttcaaa 2040
agacaacaga ggaggtatta aatcaaaaac tacagctaaa gtaacaaaag aattatatgt 2100
taaactcact cctgtttccc tttctaattc cccaattaaa ggtgctgatt gtcaggaagt 2160
tccacaagat aaagatggct ataaaagttg tggtctgaac cccaagttag agaaatgtgg 2220
acttggacag gaaaacagtg ataatgagca tttggttgaa aatgaagttt cattactttt 2280
agaggaatct gatcttcgaa gatccccacg tgtaaagact acacccttga ggcgaccgac 2340
agaaactaac cctgtaacat ctaattcaga tgaagaatgt aatgaaacag ttaaggagaa 2400
acaaaaacta tcagttccag tgagaaaaaa ggataagcgt aattcttctg acagtgctat 2460
agataatcct aagcctaata aattgccaaa atctaagcaa tcagagactg tggatcaaaa 2520
ttcagattct gatgaaatgc tagcaatcct caaagaggtg agcaggatga gtcacagttc 2580
ttcttcagat actgatatta atgaaattca tacaaaccat aagactttgt atgatttaaa 2640

gactcaggcg gggaaagatg ataaaggaaa aaggaaacga aaaagttcta catctggctc 2700
agattttgat actaaaaagg gcaaatcagc taagagctct ataatttcta aaaagaaacg 2760
acaaacccag tctgagtctt ctaattatga ctcagaatta gaaaaagaga taaagagcat 2820
gagtaaaatt ggtgctgcca gaaccaccaa aaaaagaatt ccaaatacaa aagattttga 2880
ctcttctgaa gatgagaaac acagcaaaaa aggaatggat aatcaagggc acaaaaattt 2940
gaagacctca caagaaggat catctgatga tgctgaaaga aaacaagaga gagagacttt 3000
ctcttcagca gaaggcacag ttgataaaga cacgaccatc atggaattaa gagatcgact 3060
tcctaagaag cagcaagcaa gtgcttccac tgatggtgtc gataagcttt ctgggaaaga 3120
gcagagtttt acttctttgg aagttagaaa agttgctgaa actaaagaaa agagcaagca 3180
tctcaaaacc aaaacatgta aaaaagtaca ggatggctta tctgatattg cagagaaatt 3240
cctaaagaaa gaccagagcg atgaaacttc tgaagatgat aaaaagcaga gcaaaaaggg 3300
aactgaagaa aaaaagaaac cttcagactt taagaaaaaa gtaattaaaa tggaacaaca 3360
gtatgaatct tcatctgatg gcactgaaaa gttacctgag cgagaagaaa tttgtcattt 3420
tcctaagggc ataaaacaaa ttaagaatgg aacaactgat ggagaaaaga aaagtaaaaa 3480
aataagagat aaaacttcta aaaagaagga tgaattatct gattatgctg agaagtcaac 3540
agggaaagga gatagttgtg actcttcaga ggataaaaag agtaagaatg gagcatatgg 3600
tagagagaag aaaaggtgca agttgcttgg aaagagttca aggaagagac aagattgttc 3660
atcatctgat actgagaaat attccatgaa agaagatggt tgtaactctt ctgataagag 3720
actgaaaaga atagaattga gggaaagaag aaatttaagt tcaaagagaa atactaagga 3780
aatacaaagt ggctcatcat catctgatgc tgaggaaagt tctgaagata ataaaaagaa 3840
gaagcaaaga acttcatcta aaaagaaggc agtcattgtc aaggagaaaa agagaaactc 3900
cctaagaaca agcactaaaa ggaagcaagc tgacattaca tcctcatctt cttctgatat 3960
agaagatgat gatcagaatt ctataggtga gggaagcagc gatgaacaga aaattaagcc 4020
tgtgactgaa aatttagtgc tgtcttcaca tactggattt tgccaatctt caggagatga 4080
agccttatct aaatcagtgc ctgtcacagt ggatgatgat gatgacgaca atgatcctga 4140
gaatagaatt gccaagaaga tgcttttaga agaaattaaa gccaatcttt cctctgatga 4200
ggatggatct tcagatgatg agccagaaga agggaaaaaa agaactggaa aacaaaatga 4260
agaaaaccca ggagatgagg aagcaaaaaa tcaagtcaat tctgaatcag attcagattc 4320
tgaagaatct aagaagccaa gatacagaca taggcttttg cggcacaaat tgactgtgag 4380
tgacggagaa tctggagaag aaaaaaagac aaagcctaaa gagcataaag aagtcaaagg 4440
cagaaacaga agaaaggtga gcagtgaaga ttcagaagat tctgattttc aggaatcagg 4500
agttagtgaa gaagttagtg aatccgaaga tgaacagcgg cccagaacaa ggtctgcaaa 4560
gaaagcagag ttggaagaaa atcagcggag ctataaacag aaaaagaaaa ggcgacgtat 4620
taaggttcaa gaagattcat ccagtgaaaa caagagtaat tctgaggaag aagaggagga 4680
aaaagaagag gaggaggaag aggaggagga ggaggaagag gaggaggaag atgaaaatga 4740
tgattccaag tctcctggaa aaggcagaaa gaaaattcgg aagattctta aagatgataa 4800
actgagaaca gaaacacaaa atgctcttaa ggaagaggaa gagagacgaa aacgtattgc 4860
tgagagggag cgtgagcgag aaaaattgag agaggtgata gaaattgaag atgcttcacc 4920
caccaagtgt ccaataacaa ccaagttggt tttagatgaa gatgaagaaa ccaaagaacc 4980
tttagtgcag gttcatagaa atatggttat caaattgaaa ccccatcaag tagatggtgt 5040
tcagtttatg tgggattgct gctgtgagtc tgtgaaaaaa acaaagaaat ctccaggttc 5100
aggatgcatt cttgcccact gtatgggcct tggtaagact ttacaggtgg taagttttct 5160
tcatacagtt cttttgtgtg acaaactgga tttcagcacg gcgttagtgg tttgtcctct 5220
taatactgct ttgaattgga tgaatgaatt tgagaagtgg caagagggat taaaagatga 5280
tgagaagctt gaggtttctg aattagcaac tgtgaaacgt cctcaggaga gaagctacat 5340
gctgcagagg tggcaagaag atggtggtgt tatgatcata ggctatgaga tgtatagaaa 5400
tcttgctcaa ggaaggaatg tgaagagtcg gaaacttaaa gaaatattta acaaagcttt 5460
ggttgatcca ggccctgatt ttgttgtttg tgatgaaggc catattctaa aaaatgaagc 5520
atctgctgtt tctaaagcta tgaattctat acgatcaagg aggaggatta ttttaacagg 5580
aacaccactt caaaataacc taattgagta tcattgtatg gttaatttta tcaaggaaaa 5640
tttacttgga tccattaagg agttcaggaa tagatttata aatccaattc aaaatggtca 5700
gtgtgcagat tctaccatgg tagatgtcag agtgatgaaa aaacgtgctc acattctcta 5760
tgagatgtta gctggatgtg ttcagaggaa agattataca gcattaacaa aattcttgcc 5820
tccaaaacac gaatatgtgt tagctgtgag aatgacttct attcagtgca agctctatca 5880
gtactactta gatcacttaa caggtgtggg caataatagt gaaggtggaa gaggaaaggc 5940
aggtgcaaag cttttccaag attttcagat gttaagtaga atatggactc atccttggtg 6000
tttgcagcta gactacatta gcaaagaaaa taagggttat tttgatgaag acagtatgga 6060
tgaatttata gcctcagatt ctgatgaaac ctccatgagt ttaagctccg atgattatac 6120
aaaaaagaag aaaaaaggga aaaaggggaa aaaagatagt agctcaagtg gaagtggcag 6180
tgacaatgat gttgaagtga ttaaggtctg gaattcaaga tctcggggag gtggtgaagg 6240
aaatgtggat gaaacaggaa acaatccttc tgtttcttta aaactggaag aaagtaaagc 6300
tacttcttct tctaatccaa gcagcccagc tccagactgg tacaaagatt ttgttacaga 6360
tgctgatgct gaggttttag agcattctgg gaaaatggta cttctctttg aaattcttcg 6420
aatggcagag gaaattgggg ataaagtcct tgttttcagc cagtccctca tatctctgga 6480
cttgattgaa gattttcttg aattagctag tagggagaag acagaagata aagataaacc 6540
ccttatttat aaaggtgagg ggaagtggct tcgaaacatt gactattacc gtttagatgg 6600
ttccactact gcacagtcaa ggaagaagtg ggctgaagaa tttaatgatg aaactaatgt 6660
gagaggacga ttatttatca tttctactaa agcaggatct ctaggaatta atctggtagc 6720
tgctaatcga gtaattatat tcgacgcttc ttggaatcca tcttatgaca tccagagtat 6780
attcagagtt tatcgctttg gacaaactaa gcctgtttat gtatataggt tcttagctca 6840
gggaaccatg gaagataaga tttatgatcg gcaagtaact aagcagtcac tgtcttttcg 6900
agttgttgat cagcagcagg tggagcgtca ttttactatg aatgagctta ctgaacttta 6960
tacttttgag ccagacttat tagatgaccc taattcagaa aagaagaaga agagggatac 7020

tcccatgctg ccaaaggata ccatacttgc agagctcctt cagatacata aagaacacat 7080
tgtaggatac catgaacatg attctctttt ggaccacaaa gaagaagaag agttgactga 7140
agaagaaaga aaagcagctt gggctgagta tgaagcagag aagaagggac tgaccatgcg 7200
tttcaacata ccaactggga ccaatttacc ccctgtcagt ttcaactctc aaactcctta 7260
tattcctttc aatttgggag ccctgtcagc aatgagtaat caacagctgg aggacctcat 7320
taatcaagga agagaaaaag ttgtagaagc aacaaacagt gtgacagcag tgaggattca 7380
acctcttgag gatataattt cagctgtatg gaaggagaac atgaatctct cagaggccca 7440
agtacaggcg ttagcattaa gtagacaagc cagccaggag cttgatgtta aacgaagaga 7500
agcaatctac aatgatgtat tgacaaaaca acagatgtta atcagctgtg ttcagcgaat 7560
acttatgaac agaaggctcc agcagcagta caatcagcag caacagcaac aaatgactta 7620
tcaacaagca acactgggtc acctcatgat gccaaagccc ccaaatttga tcatgaatcc 7680
ttctaactac cagcagattg atatgagagg aatgtatcag ccagtggctg gtggtatgca 7740
gccaccacca ttacagcgtg caccaccccc aatgagaagc aaaaatccag gaccttccca 7800
agggaaatca atgtgatttt gcactaaaag cttaatggat tgttaaaatc atagaaagat 7860
cttttatttc tttaggaatc aatgacttaa cagaactcaa ctgtataaat agtttggtcc 7920
ccttaaatgc caatcttcca tattagtttt actttttttt ttttttaaat agggcatacc 7980
atttcttcct gacatttgtc agtgatgttg cctagaatct tcttacacac gctgagtaca 8040
gaagatattt caaattgttt tcagtgaaaa caagtccttc cataatagta acaactccac 8100
agatttcctc tctaaatttt tatgcctgct tttagcaacc ataaaattgt cataaaatta 8160
ataaatttag gaaagaataa agatttatat attcattctt tacatataaa aacacacagc 8220
tgagttctta gagttgattc ctcaagttat gaaatacttt tgtacttaat ccatttcttg 8280
attaaagtga ttgaaatggt tttaatgttc ttttgactga agtctgaaac tgggctcctg 8340
ctttattgtc tctgtgactg aaagttagaa actgagggtt atctttgaca cagaattgtg 8400
tgcaatattc ttaaatacta ctgctctaaa agttggagaa gtcttgcagt tatcttagca 8460
ttgtataaac agccttaagt atagcctaag aagagaattc ctttttcttc tttagtcctt 8520
ctgccatttt ttattttcag ttatatgtgc tgaaataatt actggtaaaa tttcagggtt 8580
gtggattatc ttccacacat gaattttctc tctcctggca cgaatataaa gcacatctct 8640
taactgcatg gtgccagtgc taatgcttca tcctgttgct ggcagtggga tgtggactta 8700
gaaaatcaag ttctagcatt ttagtaggtt aacactgaag ttgtggttgt taggttcaca 8760
ccctgtttta taaacaacat caaaatggca gaaccattgc tgactttagg ttcacatgag 8820
gaatgtactt ttaacaattc ccagtactat cagtattgtg aaataattcc tctgaaagat 8880
aagaatcact ggcttctatg cgcttctttt ctctcatcat catgttcttt taccccagtt 8940
tccttacatt tttttaaatt gtttcagagt ttgttttttt tttagtttag attgtgaggc 9000
aattattaaa tcaaaattaa ttcatccaat acccctttac tagaagtttt actagaaaat 9060
gtattacatt ttattttttc ttaatccagt tctgcaaaaa tgacctataa atttattcat 9120
gtacaatttt ggttacttga attgttaaag aaaacattgt ttttgactat gggagtcaac 9180
tcaacatggc agaaccattt ttgagatgat gatacaacag gtagtgaaac agcttaagaa 9240
ttccaaaaaa aaaaaaaaaa aaaaaaaaaa agaaaactgg gtttgggctt tgctttaggt 9300
atcactggat tagaatgagt ttaacattag ctaaaactgc tttgagttgt ttggatgatt 9360
aagagattgc catttttatc ttggaagaac tagtggtaaa acatccaaga gcactaggat 9420
tgtgatacag aatttgtgag gtttggtgga tccacgcccc tctcccccac tttcccatga 9480
tgaaatatca ctaataaatc ctgtatattt agatattatg ctagccatgt aatcagattt 9540
atttaattgg gtggggcagg tgtgtattta ctttagaaaa aatgaaaaag acaagattta 9600
tgagaaatat ttgaaggcag tacactctgg ccaactgtta ccagttggta tttctacaag 9660
ttcagaatat tttaaacctg atttactaga cctgggaatt ttcaacatgg tctaattatt 9720
tactcaaaga catagatgtg aaatttaggc aaccttctaa atcttttcac catggatgaa 9780
actataactt aaagataata cttagaaggg ttaattggaa atcagagttt gaaataaaac 9840
ttggaccact ttgtatacac tcttctcact tgacatttta gctatataat atgtactttg 9900
agtataacat caagctttaa caaatattta aagacaaaaa aatcacgtca gtaaaatact 9960
aaaaggctca tttttatatt tgttttagat gttttaaata gttgcaatgg attaaaaatg 10020
atgatttaaa atgttgcttg taatacagtt ttgcctgcta aattctccac attttgtaac 10080
ctgttttatt tctttgggtg taaagcgttt ttgcttagta ttgtgatatt gtatatgttt 10140
tgtcccagtt gtatagtaat gtttcagtcc atcatccagc tttggctgct gaaatcatac 10200
agctgtgaag acttgccttt gtttctgtta gactgctttt cagttctgta ttgagtatct 10260
taagtactgt agaaaagatg tcacttcttc ctttaaggct gttttgtaat atatataagg 10320
actggaattg tgtttttaaa gaaaagcatt caagtatgac aatatactat ctgtgttttc 10380
accattcaaa gtgctgttta gtagttgaaa cttaaactat ttaatgtcat ttaataaagt 10440
gaccaaaatg tg 10452

<210> 51
<211> 4461
<212> DNA
<213> Homo sapiens
<220>
<221> misc_feature
<223> séquence appelée GS-N51

<300>
<308> GENBANK/AL110212
<309> 2000-02-18
<313> (1)..(4461)
<400> 51
gggcttcatg tataatatct taatatgtat ctaaaagata gcttttttat gttttatttt 60

atttttattt tttcagacaa agtattactc tgtcgcccag gctggcatgc agtggcacaa 120
tctcagctca ctgcaacctc cacctcccag gttcaagtga ttcttctgcc tcagcctcct 180
gagtagctgg cattacaggt gcctgccacc atgcctggct aatgtttgtt tgttcttttg 240
tttttgtttc tgagacagga tctcactctg ttgcctaggc tggagtccaa tagcctaatc 300
ttggctcact gcaacctcag cctcccaggc aactgggact acaggcatgc gccaccatgc 360
ccagctaatc tgtgtgtgtt ttttgtttgt ttgtttattt gtgtagtaga gatggggttt 420
caccatgttg gccaggctgg tctcaaactc ctgacctcaa gtgatccacc cgcctcagcc 480
tcccaaagtg ctgggattac aggcatgagc cactgtgcct ggcgtagaaa gtatttctta 540
taattaaaaa cgacacccag tctaactagt ataacaccta aaaaattgtc agatctgtat 600
tttagatagt acgacacagt gaaatgcaat ggcacttaag cagagagaag aaggattttt 660
ctttttcttt ttttaaatag aaggtgttag aacctgaact gggcctggtt tgcatttggg 720
aagagtccag tggggaaaca acacaaataa ggtcttgaga atgaagaatt gcatttatat 780
agggagttgt aacaaatggc ctggagagta tctgactctt tatgaaacat ttaatgagaa 840
atgtgacttg ctttggtgcc tatggttgaa tttgttgcga tgcttctgac attatgaaaa 900
ttttcaaaca tgcaaaaaag ttccagtagt tatccagtga attcttatac tcaccaccta 960
tatctatagt taacatttta cttgtatttt atccagttat cagctcatct atgagatggc 1020
ccttatttat ttgcctgctg tttttattct ctatttgtcc tgttatttct tgttagagaa 1080
gagaaggtgt tcttagatca gaagatgcca ttgtattagg tgacggcaag gttttgctga 1140
tttagtggaa caagattcta ctgggatgtg tgctgagctg aaccaaggaa ctgcccgaac 1200
gtgctgaaca aggacatttg cttctcagat acgtgaattc aattttaagc aagattcttg 1260
atctgcttca gagcagcttt gatttaaagt aatttcagag cacttttcct tgcatgagta 1320
attttctgaa tgtataaaat attctattta tgtttgacct tttggataaa ctgtgttgct 1380
gcagtgttgg tccacactac catcctaagt gaagacttag taagtctgag gatctccagg 1440
cactggaaag agtgagaacc tgggagtgaa agggttatag ttagtgttca tttttcgggt 1500
tttgtttcat agggagtcct gagtggggta acctgaaatg ttttaacagt tgaagatcat 1560
cccattccta tccccagtaa aaataggcct ttccacaatc ttctaccagt taacttagct 1620
tttttttttt ttttccttaa tagagtctca ttcttgtcac ccagactgga gtgcgatggc 1680
acgatcttgg ctcactgcaa ccctccctcc cgggttccag cgattctcct gcctcagcct 1740
cccgggtggc tgggattgca ggcgcacgcc accatgccca gctaattttt gtatttttag 1800
tagacgtggg gtttcaccat gttggccaga ctggtctcaa gctcctgacc tcgggcgatc 1860
cgcccgtcta ggccttccaa agtgctggga ttactggcag gaaccaccag gcccagccaa 1920
ccagcatttc ttaaggtgaa aatacatggt gttaatttat attttgagtg agacccagtc 1980
cagctgctat aactgcttgg acacttggca gaggacagag tactgcaaaa gtgtctaagg 2040
gagacagcat tttgccttgg gtgctgtaga tgtacaagag aggttccagg agggggtgat 2100
gggcagaatt ttggtcccca tcaccttccc tgcccagtgt tatgcctatg aatgtgttac 2160
attatgtggt aaaagggact ttgcagatgt aactaaaatt tctaaaatag agatattatc 2220
ctggattacc tgggggaacc cagtgtaatt acatgaaccc ttaaaaatgg aagaggatgc 2280
aggagtcaga ttcaaaggaa ggcccaaggt gctattgctg acttgaagat agaggggcca 2340
tgtggaaatc aagagaagga agtgaatcct tccagtgagc ttggaagaga gcaccttgag 2400
gcacagatga gaagcttggc cttacctgat gccttgattt tagcctggtg agaccccgag 2460
catataaatt tgctgtgcta tgccacactt ctcacctaca gaaacttagt ttaaagccac 2520
taagtttgtg gtaatttgtt ggctttaggc ccctgagggt agagatttat ggcttgtgtt 2580
acaagtagag agcagtggag agttgggctt tgtaattctt tcaagggtga ttgtagttct 2640
ggagtcctat ctacctgggt tcagatcttt gttggccagt ccctggctgt gtgactccac 2700
tgctttaagc ctctgtcttc atccttaaag tggcgatgat agtacgtacc tcactctgat 2760
ctctgaatga gaacacatgt aagacactta gcacagttcc tggtgctcaa agagttccaa 2820
cacctgaaag caccatggcc ctgtttgctt ttcagtcaag atttctaaaa gccgttatta 2880
ccagaggttc agtgagaaca tgggcagggt aaactcaaaa tggagaacat aaaaactggg 2940
actcccagac cagtcactta gaagactgct gcaggaagta ccaaggtcgt taagaatcag 3000
taataataat aagtaatgtg tgaatccatt agaatgttga gaagtagacc tttgacattt 3060
cagaaaaatg tatatctcag acacagccat atgtattgtt atccctgagt tggaagagca 3120
tcacagggca tagtttcaca aaagcttcaa aaaatatact aattgctggt aatatgtgaa 3180
ctaacagaaa aaaatagcca tgttaaaata ggagtgcctg acaattagct ctattcatct 3240
tgtctcctag ctgtaagcac cctagattca aattatgcac ctagaaaact aaattactat 3300
gtatgttata ggcctttgaa atggatacat gaaaattagt gaaatcagaa tgaacgtttc 3360
gcctgccaaa gatatgtatt tttataagtg atacaagggc cccatgagct ccatggccac 3420
atctgtacaa aacaggccct cctttcagga actggtctcg agagagcaaa ggggtcaact 3480
ccctaaatgg cagctgagac agtcctccct ggtggcatgg ccctgtttcc tgttgatatg 3540
gtgatagttg gagagtcaaa tttttaccag aatttgtgaa tatcatctct ggttaatgat 3600
tacacaaaac cttttaaaat tttaaaaaat tatttctagt tctataaata aactctgcag 3660
ttgtacagcc actgtacagc taacatctca acactggaaa tgaggctggt ttcttccagt 3720
ttggctcaga ttcttcaggg aagcccagtt aagacaaaca gctctctata ctcaatcctt 3780
actttgctga gaagatacag gcctgcccac tcgctcccac ctgctcttca tgaggagtcc 3840
tttattcctg catgtttcct gttctttggg acctttagga gctaacctcc acagatgctg 3900
atagtctgta gagtcaggaa taccatgtaa catgttgaag tagactaaag attagttctt 3960
tggcaatagc ccagtctgga cttactatgt acttttgaaa ataaaactgc ttatttgtaa 4020
actctgtaat taggaccttt tgctatttga gttatattcc tttctaactt caaacgtctg 4080
cattcatgta taatagccat tttatgcaca gatgaggtga ttcccacact tcttttcgca 4140
gatgtttcat tatctggatg agtgtattac ataataaatt tgaataatgg tagtcttcca 4200
aacgacaaaa agcaagctag ttgatttgtc aattttgagt ttaaatgttt tgactgctta 4260
tcagcaaaaa aaatcaaaga atagcatact aatattaatg gaaatgcaga gtatatttaa 4320
ttggcatgat tttttcacgg atgtgtgctt catttgatct attgtatgta gctcgtgatc 4380
acattttctg ttggttaaca ttgttcctag cttatatgat ggaattaaat atattctgtg 4440

caaaaaaaaa aaaaaaaaaa a                                          4461

<210>  52
<211>  8546
<212>  DNA
<213>  Homo sapiens
<220>
<221>  misc_feature
<223>  séquence appelée GS-N52

<220>
<221>  misc_feature
<222>  (162)..(162)
<223>  n est un résidu inconnu

<220>
<221>  misc_feature
<222>  (357)..(357)
<223>  n est un résidu inconnu

<300>
<308>  GENBANK/AJ306906
<309>  2001-04-26
<313>  (1)..(8546)
<400>  52
cctccaagtg tcattggtcc taaatctgaa aatcttaccg tcgtggtgaa caatttcatc      60
tctttgacct gtgaggtctc tggttttcca cctcctgacc tcagctggct caagaatgaa     120
cagcccatca aactgaacac aaatactctc attgtgcctg gnggtcgaac tctacagatt     180
attcgggcca aggtatcaga tggtggtgaa tacacttgta tagctatcaa tcaagctggc     240
gaaagcaaga aaaagttttc cctgactgtt tatgtgcccc caagcattaa agaccatgac      300
agtgaatctc tttctgtagt taatgtaaga gagggaactt ctgtgtcttt ggagtgngag     360
tcgaacgctg tgccacctcc agtcatcact tggtataaga atgggcggat gataacagag     420
tctactcatg tggagatttt agctgatgga caaatgctac acattaagaa agctgaggta     480
tctgacacag gccagtatgt atgtagagct ataaatgtag caggacggga tgataaaaat     540
ttccacctca atgtatatgt gccacccagt attgaaggac ctgaaagaga agtgattgtg     600
gagacgatca gcaatcctgt gacattaaca tgtgatgcca ctgggatccc acctcccacg     660
atagcatggt taaagaacca caagcgcata gaaaattctg actcactgga agttcgtatt     720
ttgtctggag gtagcaaact ccagattgcc cggtctcagc attcagatag tggaaactat     780
acatgtattg cttcaaatat ggagggaaaa gcccagaaat attactttct ttcaattcaa     840
gttcctccaa gtgttgctgg tgctgaaatt ccaagtgatg tcagtgtcct tctaggagaa     900
aatgttgagc tggtctgcaa tgcaaatggc attcctactc cacttattca atggcttaaa     960
gatggaaagc ccatagctag tggtgaaaca gaaagaatcc gagtgagtgc aaatggcagc    1020
acattaaaca tttatggagc tcttacatct gacacgggga aatacacatg tgttgctact    1080
aatcccgctg gagaagaaga ccgaattttt aacttgaatg tctatgttac acctacaatt    1140
aggggtaata aagatgaagc agagaaacta atgactttag tggatacttc aataaatatt    1200
gaatgcagag ccacagggac gcctccacca cagataaact ggctgaagaa tggacttcct    1260
ctgcctctct cctcccatat ccggttactg gcagcaggac aagttatcag gattgtgaga    1320
gctcaggtgt ctgatgtcgc tgtgtatact tgtgtggcct ccaacagagc tggggtggat    1380
aataagcatt acaatcttca agtgtttgca ccaccaaata tggacaattc aatggggaca    1440
gaggaaatca cagttctcaa aggtagttcc acctctatgg catgcattac tgatggaacc    1500
ccagctccca gtatggcctg gcttagagat ggccagcctc tggggcttga tgcccatctg    1560
acagtcagca cccatggaat ggtcctgcag ctcctcaaag cagagactga agattcggga    1620
aagtacacct gcattgcctc aaatgaagct ggagaagtca gcaagcactt tatcctcaag    1680
gtcctagaac cacctcacat taatggatct gaagaacatg aagagatatc agtaattgtt    1740
aataacccac ttgaacttac ctgcattgct tctggaatcc cagcccctaa aatgacctgg    1800
atgaaagatg gccggcccct tccacagacg gatcaagtgc aaactctagg aggaggagag    1860
gttcttcgaa tttctactgc tcaggtggag gatacaggaa gatatacatg tctggcatcc    1920
agtcctgcag gagatgatga taaggaatat ctagtgagag tgcatgtacc tcctaatatt    1980
gctggaactg atgagccccg ggatatcact gtgttacgga acagacaagt gacattggaa    2040
tgcaagtcag atgcagtgcc cccacctgta attacttggc tcagaaatgg agaacggtta    2100
caggcaacac ctcgagtgcg aatcctatct ggagggagat acttgcaaat caacaatgct    2160
gacctaggtg atacagccaa ttatacctgt gttgccagca acattgcagg aaagactaca    2220
agagaattta ttctcactgt aaatgttcct ccaaacataa aggggggccc ccagagcctt    2280
gtaattcttt taaataagtc aactgtattg gaatgcatcg ctgaaggtgt cccaactcca    2340
aggataacat ggagaaagga tggagctgtt ctagctggga atcatgcaag atattccatc    2400
ttggaaaatg gattccttca tattcaatca gcacatgtca ctgacactgg acggtatttg    2460
tgtatggcca ccaatgctgc tggaacagat cgcaggcgaa tagatttaca ggtccatgtt    2520
cctccatcta ttgctccggg tcctaccaac atgactgtaa tagtaaatgt tcaaactact    2580
ctggcttgtg aggctactgg gataccaaaa ccatcaatca attggagaaa aaatgggcat    2640
cttcttaatg tggatcaaaa tcagaactca tacaggctcc tttcttcagg ttcactagta    2700
attatttccc cttctgtgga tgacactgca acctatgaat gtactgtgac aaacggtgct    2760
ggagatgata aaagaactgt ggatctcact gtccaagttc caccttccat agctgatgag    2820
cctacagatt tcctagtaac caaacatgcc ccagcagtaa ttacctgcac tgcttcggga    2880

gttccatttc cctcaattca ctggaccaaa aatggtataa gactgcttcc caggggagat 2940
ggctatagaa ttctgtcctc aggagcaatt gaaatacttg ccacccaatt aaaccatgct 3000
ggaagataca cttgtgtcgc taggaatgcg gctggctctg cacatcgaca cgtgaccctt 3060
catgttcatg agcctccagt cattcagccc caaccaagtg aactacacgt cattctgaac 3120
aatcctattt tattaccatg tgaagcaaca gggacaccca gtcctttcat tacttggcaa 3180
aaagaaggca tcaatgttaa cacttcaggc agaaaccatg cagttcttcc tagtggcggc 3240
ttacagatct ccagagctgt ccgagaggat gctggcactt acatgtgtgt ggcccagaac 3300
ccggctggta cagccttggg caaaatcaag ttaaatgtcc aagttcctcc agtcattagc 3360
cctcatctaa aggaatatgt tattgctgtg gacaagccca tcacgttatc ctgtgaagca 3420
gatggcctcc ctccgcctga cattacatgg cataaagatg ggcgtgcaat tgtggaatct 3480
atccgccagc gcgtcctcag ctctggctct ctgcaaataa catttgtcca gcctggtgat 3540
gctggccatt acacgtgcat ggcagccaat gtagcaggat caagcagcac aagcaccaag 3600
ctcaccgtcc atgtaccacc caggatcaga agtacagaag gacactacac ggtcaatgag 3660
aattcacaag ccattcttcc atgcgtagct gatggaatcc ccacaccagc aattaactgg 3720
aaaaaagaca atgttctttt agctaacttg ttaggaaaat acactgctga accatatgga 3780
gaactcattt tagaaaatgt tgtgctggag gattctggct tctatacctg tgttgctaac 3840
aatgctgcag gtgaagatac acacactgtc agcctgactg tgcatgttct ccccactttt 3900
actgaacttc ctggagacgt gtcattaaat aaaggagaac agctacgatt aagctgtaaa 3960
gctactggta ttccattgcc caaattaaca tggaccttca ataacaatat tattccagcc 4020
cactttgaca gtgtgaatgg acacagtgaa cttgttattg aaagagtgtc aaaagaggat 4080
tcaggtactt atgtgtgcac cgcagagaac agcgttggct ttgtgaaggc aattggattt 4140
gtgtatgtga aagaacctcc agtcttcaaa ggtgattatc cttctcactg gattgaacca 4200
cttggtggga atgcaatcct gaattgtgag gtgaaaggag accccacccc aaccatccag 4260
tggaacagaa agggagtgga tattgaaatt agccacagaa tccggcaact gggcaatggc 4320
tccctggcca tctatggcac tgttaatgaa gatgccggtg actatacatg tgtagctacc 4380
aatgaagctg gggtggtgga gcgcagcatg agtctgactc tgcaaagtcc tcctattatc 4440
actcttgagc cagtggaaac tgttattaat gctggtggca aaatcatatt gaattgtcag 4500
gcaactggag agcctcaacc aaccattaca tggtcccgtc aagggcactc tatttcctgg 4560
gatgaccggg ttaacgtgtt gtccaacaac tcattatata ttgctgatgc tcagaaagaa 4620
gatacctctg aatttgaatg tgttgctcga aacttaatgg gttctgtcct tgtcagagtg 4680
ccagtcatag tccaggttca tggtggattt tcccagtggt ctgcatggag agcctgcagt 4740
gtcacctgtg gaaaaggcat ccaaaagagg agtcgtctgt gcaaccagcc ccttccagcc 4800
aatggtggga agccctgcca aggttcagat ttggaaatgc gaaactgtca aaataagcct 4860
tgtccagtgg atggtagctg gtcggaatgg agtctttggg aagaatgcac aaggagctgt 4920
ggacgcggca accaaaccag gaccaggact tgcaataatc catcagttca gcatggtggg 4980
cggccatgtg aagggaatgc tgtggaaata attatgtgca acattaggcc ttgcccagtt 5040
catggagcat ggagcgcttg gcagccttgg ggaacatgca gcgaaagttg tgggaaaggt 5100
actcagacaa gagcaagact ttgtaataac ccaccaccag cgtttggtgg gtcctactgt 5160
gatggagcag aaacacagat gcaagtttgc aatgaaagaa attgtccagt tcatggcaag 5220
tgggcgactt gggccagttg gagtgcctgt tctgtgtcat gtggaggagg tgccagacag 5280
agaacaaggg gctgctccga ccctgtgccc cagtatggag gaaggaaatg cgaagggagt 5340
gatgtccaga gtgatttttg caacagtgac ccttgcccaa cccatggtaa ctggagtcct 5400
tggagtggct ggggaacatg cagccggacg tgtaacggag ggcagatgcg gcggtaccgc 5460
acatgtgata accctcctcc ctccaatggg ggaagagctt gtgggggacc agactcccag 5520
atccagaggt gcaacactga catgtgtcct gtggatggaa gttggggaag ctggcatagt 5580
tggagccagt gctctgcctc ctgtggagga ggtgaaaaga ctcggaagcg gctgtgcgac 5640
catcctgtgc cagttaaagg tggccgtccc tgtcccggag acactactca ggtgaccagg 5700
tgcaatgtac aagcatgtcc aggtgggccc cagcgagcca gaggaagtgt tattggaaat 5760
attaatgatg ttgaatttgg aattgctttc cttaatgcca caataactga tagccctaac 5820
tctgatacta gaataatacg tgccaaaatt accaatgtac ctcgtagtct tggttcagca 5880
atgagaaaga tagtttctat tctaaatccc atttattgga caacagcaaa ggaaatagga 5940
gaagcagtca atggctttac cctcaccaat gcagtcttca aaagagaaac tcaagtggaa 6000
tttgcaactg gagaaatctt gcagatgagt catattgccc ggggcttgga ttccgatggt 6060
tctttgctgc tagatatcgt tgtgagtggc tatgtcctac agcttcagtc acctgctgaa 6120
gtcactgtaa aggattacac agaggactac attcaaacag gtcctgggca gctgtacgcc 6180
tactcaaccc ggctgttcac cattgatggc atcagcatcc catacacatg gaaccacacc 6240
gttttctatg atcaggcaca gggaagaatg cctttcttgg ttgaaacact tcatgcatcc 6300
tctgtggaat ctgactataa ccagatagaa gagacactgg gttttaaaat tcatgcttca 6360
atatccaaag gagatcgcag taatcagtgc cctctcgggt ttaccttaga ctcagttgga 6420
cctttttgtg ctgatgagga tgaatgtgca gcagggaatc cctgctccca tagctgccac 6480
aatgccatgg ggacttacta ctgctcctgc cctaaaggcc tcaccatagc tgcagatgga 6540
agaacttgtc aagatattga tgagtgtgct ttgggtaggc atacctgcca cgctggtcag 6600
gactgtgaca atacgattgg atcttatcgc tgtgtggtcc gttgtggaag tggctttcga 6660
agaacctctg atgggctgag ttgtcaagat attaatgaat gtcaagaatc cagcccctgt 6720
caccagcgct gtttcaatgc cataggaagt ttccattgtg gatgtgaacc tgggtatcag 6780
ctcaaaggca gaaaatgcat ggatgtgaac gagtgtagac aaaatgtatg cagaccagat 6840
cagcactgta agaacacccg tggtggctat aagtgcattg atctttgtcc aaatggaatg 6900
accaaggcag aaaatggaac ctgtattgat attgatgaat gtaaagatgg gacccatcag 6960
tgcagatata accagatatg tgagaataca agaggcagct atcgttgtgt atgcccaaga 7020
ggttatcggt ctcaaggagt tggaagaccc tgcatggaca ttaatgaatg tgaacaagtg 7080
cctaaacctt gtgcacatca gtgctccaac acccccggca gcttcaagtg tatctgtcca 7140
ccaggacaac atttattagg ggacgggaaa tcttgcgctg gattggagag gctgccaaat 7200
tatggcactc aatacagtag ctataacctt gcacggttct cccctgtgag aaacaactat 7260

caacctcaac agcattacag acagtactca catctctaca gctcctactc agagtataga 7320
aacagcagaa catctctctc caggactaga aggactatta ggaaaacttg ccctgaaggc 7380
tctgaggcaa gccatgacac atgtgtagat attgatgaat gtgaaaatac agatgcctgc 7440
cagcatgagt gtaagaatac ctttggaagt tatcagtgca tctgcccacc tggctatcaa 7500
ctcacacaca atggaaagac atgccaagat atcgatgaat gtctggagca gaatgtgcac 7560
tgtggaccca atcgcatgtg cttcaacatg agaggaagct accagtgcat cgatacaccc 7620
tgtccaccca actaccaacg ggatcctgtt tcagggttct gcctcaagaa ctgtccaccc 7680
aatgatttgg aatgtgcctt gagcccatat gccttggaat acaaactcgt ctccctccca 7740
tttggaatag ccaccaatca agatttaatc cggctggttg catacacaca ggatggagtg 7800
atgcatccca ggacaacttt cctcatggta gatgaggaac agactgttcc ttttgccttg 7860
agggatgaaa acctgaaagg agtggtgtat acaacacgac cactacgaga agcagagacc 7920
taccgcatga gggtccgagc ctcatcctac agtgccaatg ggaccattga atatcagacc 7980
acattcatag tttatatagc tgtgtccgcc tatccatact aaggaactct ccaaagccta 8040
ttccacatat ttaaaccgca ttaatcatgg caatcaagcc cccttccaga ttactgtctc 8100
ttgaacagtt gcaatcttgg cagcttgaaa atggtgctac actctgtttt gtgtgccttc 8160
cttggtactt ctgaggtatt ttcatgatcc caccatggtc atatcttgaa gtatggtcta 8220
gaaaagtccc ttattatttt atttattaca ctggagcagt tacttcccaa agattattct 8280
gaacatctaa caggacatat cagtgatggt ttacagtagt gtagtaccta agatcatttt 8340
cctgaaagcc aaaccaaaca acgaaaaaca agaacaacta attcagaatc aaatagagtt 8400
tttgagcatt tgactatttt tagaatcata aaattagtta ctaagtattt tgatcaaagc 8460
ttataaaata acttacggag atttttgtaa gtattgatac attataatag gacttgccta 8520
ttttcatttt taagaagaaa aacccg 8546

<210> 53
<211> 4187
<212> DNA
<213> Homo sapiens
<220>
<221> misc_feature
<223> séquence appelée GS-N53

<300>
<308> GENBANK/D13643
<309> 2001-10-06
<313> (1)..(4187)
<400> 53
ggcgcgaacc cgcagcgctt accgcgcggc gccgcaccat ggagcccgcc gtgtcgctgg 60
ccgtgtgcgc gctgctcttc ctgctgtggg tgcgcctgaa ggggctggag ttcgtgctca 120
tccaccagcg ctgggtgttc gtgtgcctct tcctcctgcc gctctcgctt atcttcgata 180
tctactacta cgtgcgcgcc tgggtggtgt tcaagctcag cagcgctccg cgcctgcacg 240
agcagcgcgt gcgggacatc cagaagcagg tgcgggaatg gaaggagcag ggtagcaaga 300
ccttcatgtg cacggggcgc cctggctggc tcactgtctc actacgtgtc gggaagtaca 360
agaagacaca caaaaacatc atgatcaacc tgatggacat tctggaagtg gacaccaaga 420
aacagattgt ccgtgtggag cccttggtga ccatgggcca ggtgactgcc ctgctgacct 480
ccattggctg gactctcccc gtgttgcctg agcttgatga cctcacagtg gggggcttga 540
tcatgggcac aggcatcgag tcatcatccc acaagtacgg cctgttccaa cacatctgca 600
ctgcttacga gctggtcctg gctgatggca gctttgtgcg atgcactccg tccgaaaact 660
cagacctgtt ctatgccgta ccctggtcct gtgggacgct gggtttcctg gtggccgctg 720
agatccgcat catccctgcc aagaagtacg tcaagctgcg tttcgagcca gtgcggggcc 780
tggaggctat ctgtgccaag ttcacccacg agtcccagcg gcaggagaac cacttcgtgg 840
aagggctgct ctactccctg gatgaggctg tcattatgac aggggtcatg acagatgagg 900
cagagcccag caagctgaat agcattggca attactacaa gccgtggttc tttaagcatg 960
tggagaacta tctgaagaca aaccgagagg gcctggagta cattcccttg agacactact 1020
accaccgcca cacgcgcagc atcttctggg agctccagga catcatcccc tttggcaaca 1080
accccatctt ccgctacctc tttggctgga tggtgcctcc caagatctcc ctcctgaagc 1140
tgacccaggg tgagaccctg cgcaagctgt acgagcagca ccacgtggtg caggacatgc 1200
tggtgcccat gaagtgcctg cagcaggccc tgcacacctt ccaaaacgac atccacgtct 1260
accccatctg gctgtgtccg ttcatcctgc ccagccagcc aggcctagtg caccccaaag 1320
gaaatgaggc agagctctac atcgacattg gagcatatgg ggagccgcgt gtgaaacact 1380
ttgaagccag gtcctgcatg aggcagctgg agaagtttgt ccgcagcgtg catggcttcc 1440
agatgctgta tgccgactgc tacatgaacc gggaggagtt ctgggagatg tttgatggct 1500
ccttgtacca caagctgcga gagaagctgg gttgccagga cgccttcccc gaggtgtacg 1560
acaagatctg caaggccgcc aggcactgag ctggagcccg cctggagaga cagacacgtg 1620
tgagtggtca ggcatcttcc cttcactcaa gcttggctgc tttcctagat ccacactttc 1680
aaagagaaac ccctccagaa ctcccaccct gacagcccaa caccaccttc ctcctggctt 1740
ccagggggca gcccagtgga atggaaagaa tgtgggattt ggagtcagac aagcctgagt 1800
ccagttcccc gtttagaact cattagctgt gtgactctgg gtgagtccct taacccctct 1860
gagcccgggt ctcttcatta gttgaaaggg atagtaatac ctacttgcag gttgttgtca 1920
tctgagttga gcactggtca cattgaaggt gctgggtaag tggtagctct tgttgcttcc 1980
cgttcagcgt cacatctgca gtggagcctg aaaaggctcc acattaggtc acctgtgcac 2040
agccatggct ggaatgatga aggggatacg ctggagttgc cctgccatcg cctccatcag 2100
ccagacgagg tcctcacagg agaaggacag ctcttcccca ccctgggatc tcaggagggc 2160
agccacggag tggggaggcc ccagatgcgc tgtgccaaag ccaggtccga ggccaaagtt 2220

ctccctgcca tccttggtgc cgtcctgccc cttcctcctt catgcctggg cctgcaggcc 2280
caccccagcc accactgagt ccactcggag tgccctgtgt tcctggagaa ggcattccag 2340
ggttgaatct tgtcccagcc tcagcctggg acacctaggt ggagagagtg gtctccgctc 2400
tgaattggat ccaggggacc tgggctcatt cttcttggct caccaaccct gcaggcctca 2460
tctttcccaa aacccacttt gtcttggtgg gagtgggtcc gcgctgctct gcagcagggg 2520
ctggggagtg gacagcatca ggtgggaaag tggagtccac cctcatgttt ctgtaggatt 2580
ctcaccgtgg ggctggaaga aaagagcatc gacttgattt ctccaaccac tcatccctct 2640
ttttctttct tccaccactc cccaccccag ctgtagttaa tttcagtgcc ttacaaatcc 2700
taagctcaga gaaagttcca tttccgttcc agagggaagg gaacctccct aggtccttcc 2760
ctggcttgtt ataacgcaaa gcttggttgt ttatgcaact ctatcttaag aactgcccag 2820
cctcagctga aaacccgaat ctgagaagga attgcgtcat gtaagggaag ctggaattaa 2880
gggagctgag ccagtcatgg ttgtggcgtg tgagtcagga gacctaggtt tcagcccctc 2940
tctactgtca gcgagctgtg caacgtgggc aagtcattgt cctctgagct gcagtttcct 3000
catctgtcac atcgctacag acaagacctc cctggaaccc ttctgattgt cttagacact 3060
gtggttgcaa aacccacgga aagcctcatt tgtgtggaaa gtcagaggaa aaatgatcca 3120
gtggacactt ggggattatc tgtcattcaa gatccttcct tcaaccccaa ggccagctcc 3180
catctcattt ccagaaaggc tcatacctgg cttgcaggga agcatctgtc ttgtcattcc 3240
aggtgccaga atcctctcag agtcattgaa gggtgttcac ccatcccacc caaggcttgg 3300
cacactgcca gtgtcttagc agggtcttgt gagggctggg ggcatccagg cactcagaag 3360
gcaaaggaac caccctaccc atttggcctc tggagggggc agaagaaaga aagaaacctc 3420
atcctatatt ttacaaagca tgtgaattct ggcattagct ctcataggag acccatgtgc 3480
ttccttgctc agtgcaaaac tgatgattct acttgctgta gatgaatggt taacacgagc 3540
tagttaaaca gtgccattgt tttgccagtg aagcctccaa ccctaagcca ctgggacggt 3600
ggccagagat gccagcagcc tctgtcgccc ttagtcatat aaccaaaatc cagaccttat 3660
ccacaacccg gggcttggaa aggaaggtat tttggaatca caccctccgg ttatgttgct 3720
ccagtaaaat cttgcctgga aagaggcagt cttcttagca tggtgagctg agttcatggc 3780
ttttttttgt agccagtcct gtccctggcc atccatgtga tggttttgga tggagttaaa 3840
cttgatgcca gtgggcagtg catgtggaaa gtatcagagt aagcctctcc cctccagagc 3900
cctgagtttc ttggctgcat gaaggttttc tttagaatca gaattgtagc cagtttcttt 3960
ggccagaagg atgaatactt ggatattact gaaagggagg ggtggagatg ggtgtggcag 4020
tgtatggtgt gtgattttta ttttcttctt tggtcatggg ggccaaggag aaaggcatga 4080
atcttccctg tcaggctctt acagccacag gcactgtgtc tactgtctgg aagacatgtc 4140
cccgtggctg tggggccgct gcttctgttt aaataaaagt ggcctgg 4187

<210> 54
<211> 99
<212> PRT
<213> Homo sapiens
<220>
<221> misc_feature
<223> CDS de la séquence GS-N1: 159...458

<220>
<221> misc_feature
<223> séquence appelée GS-P1: Angioinducine

<400> 54
Met Ala Glu Gly Gly Phe Asp Pro Cys Glu Cys Val Cys Ser His Glu
1               5                   10                  15
His Ala Met Arg Arg Leu Ile Asn Leu Leu Arg Gln Ser Gln Ser Tyr
            20                  25                  30
Cys Thr Asp Thr Glu Cys Leu Gln Glu Leu Pro Gly Pro Ser Gly Asp
        35                  40                  45
Asn Gly Ile Ser Val Thr Met Ile Leu Val Ala Trp Met Val Ile Ala
    50                  55                  60
Leu Ile Leu Phe Leu Leu Arg Pro Pro Asn Leu Arg Gly Ser Ser Leu
65                  70                  75                  80
Pro Gly Lys Pro Thr Ser Pro His Asn Gly Gln Asp Pro Pro Ala Pro
                85                  90                  95
Pro Val Asp

<210> 55
<211> 234
<212> PRT
<213> Homo sapiens
<220>
<221> misc_feature
<223> CDS de la séquence GS-N2: 367...1071

<220>
<221> misc_feature
<223> séquence appelée GS-P2: Angiodockine

<400>  55
Met Pro Ser Gly Cys His Ser Ser Pro Pro Ser Gly Leu Arg Gly Asp
1               5                   10                  15
Met Ala Ser Leu Val Pro Leu Ser Pro Tyr Leu Ser Pro Thr Val Leu
            20                  25                  30
Leu Leu Val Ser Cys Asp Leu Gly Phe Val Arg Ala Asp Arg Pro Pro
        35                  40                  45
Ser Pro Val Asn Val Thr Val Thr His Leu Arg Ala Asn Ser Ala Thr
    50                  55                  60
Val Ser Trp Asp Val Pro Glu Gly Asn Ile Val Ile Gly Tyr Ser Ile
65                  70                  75                  80
Ser Gln Gln Arg Gln Asn Gly Pro Gly Gln Arg Val Ile Arg Glu Val
                85                  90                  95
Asn Thr Thr Thr Arg Ala Cys Ala Leu Trp Gly Leu Ala Glu Asp Ser
            100                 105                 110
Asp Tyr Thr Val Gln Val Arg Ser Ile Gly Leu Arg Gly Glu Ser Pro
        115                 120                 125
Pro Gly Pro Arg Val His Phe Arg Thr Leu Lys Gly Ser Asp Arg Leu
    130                 135                 140
Pro Ser Asn Ser Ser Ser Pro Gly Asp Ile Thr Val Glu Gly Leu Asp
145                 150                 155                 160
Gly Glu Arg Pro Leu Gln Thr Gly Glu Val Val Ile Ile Val Val Val
                165                 170                 175
Leu Leu Met Trp Ala Ala Val Ile Gly Leu Phe Cys Arg Gln Tyr Asp
            180                 185                 190
Ile Ile Lys Asp Asn Asp Ser Asn Asn Asn Pro Lys Glu Lys Gly Lys
        195                 200                 205
Gly Pro Glu Gln Ser Pro Gln Gly Arg Pro Val Gly Thr Arg Gln Lys
    210                 215                 220
Lys Ser Pro Ser Ile Asn Thr Ile Asp Val
225                 230

<210>  56
<211>  495
<212>  PRT
<213>  Homo sapiens
<220>
<221>  misc_feature
<223>  CDS de la séquence GS-N3: 978..2465

<220>
<221>  misc_feature
<223>  séquence appelée GS-P3:Angioblastine

<400>  56
Met Lys Lys Arg Lys Glu Leu Asn Ala Leu Ile Gly Leu Ala Gly Asp
1               5                   10                  15
Ser Arg Arg Lys Lys Pro Lys Lys Gly Pro Ser Ser His Arg Leu Leu
            20                  25                  30
Arg Thr Glu Pro Pro Asp Ser Asp Ser Glu Ser Ser Ser Glu Glu Glu
        35                  40                  45
Glu Glu Phe Gly Val Val Gly Asn Arg Ser Arg Phe Ala Lys Gly Asp
    50                  55                  60
Tyr Leu Arg Cys Cys Lys Ile Cys Tyr Pro Leu Cys Gly Phe Val Ile
65                  70                  75                  80
Leu Ala Ala Cys Val Val Ala Cys Val Gly Leu Val Trp Met Gln Val
                85                  90                  95
Ala Leu Lys Glu Asp Leu Asp Ala Leu Lys Glu Lys Phe Arg Thr Met
            100                 105                 110
Glu Ser Asn Gln Lys Ser Ser Phe Gln Glu Ile Pro Lys Leu Asn Glu
        115                 120                 125
Glu Leu Leu Ser Lys Gln Lys Gln Leu Glu Lys Ile Glu Ser Gly Glu
    130                 135                 140
Met Gly Leu Asn Lys Val Trp Ile Asn Ile Thr Glu Met Asn Lys Gln
145                 150                 155                 160
Ile Ser Leu Leu Thr Ser Ala Val Asn His Leu Lys Ala Asn Val Lys
                165                 170                 175
Ser Ala Ala Asp Leu Ile Ser Leu Pro Thr Thr Val Glu Gly Leu Gln
            180                 185                 190
Lys Ser Val Ala Ser Ile Gly Asn Thr Leu Asn Ser Val His Leu Ala
        195                 200                 205
Val Glu Ala Leu Gln Lys Thr Val Asp Glu His Lys Lys Thr Met Glu

210                 215                 220
Leu Leu Gln Ser Asp Met Asn Gln His Phe Leu Lys Glu Thr Pro Gly
225                 230                 235                 240
Ser Asn Gln Ile Ile Pro Ser Pro Ser Ala Thr Ser Glu Leu Asp Asn
                245                 250                 255
Lys Thr His Ser Glu Asn Leu Lys Gln Asp Ile Leu Tyr Leu His Asn
            260                 265                 270
Ser Leu Glu Glu Val Asn Ser Ala Leu Val Gly Tyr Gln Arg Gln Asn
        275                 280                 285
Asp Leu Lys Leu Glu Gly Met Asn Glu Thr Val Ser Asn Leu Thr Gln
    290                 295                 300
Arg Val Asn Leu Ile Glu Ser Asp Val Val Ala Met Ser Lys Val Glu
305                 310                 315                 320
Lys Lys Ala Asn Leu Ser Phe Ser Met Met Gly Asp Arg Ser Ala Thr
                325                 330                 335
Leu Lys Arg Gln Ser Leu Asp Gln Val Thr Asn Arg Thr Asp Thr Val
            340                 345                 350
Lys Ile Gln Ser Ile Lys Lys Glu Asp Ser Ser Asn Ser Gln Val Ser
        355                 360                 365
Lys Leu Arg Glu Lys Leu Gln Leu Ile Ser Ala Leu Thr Asn Lys Pro
    370                 375                 380
Glu Ser Asn Arg Pro Pro Glu Thr Ala Asp Glu Glu Gln Val Glu Ser
385                 390                 395                 400
Phe Thr Ser Lys Pro Ser Ala Leu Pro Lys Phe Ser Gln Phe Leu Gly
                405                 410                 415
Asp Pro Val Glu Lys Ala Ala Gln Leu Arg Pro Ile Ser Leu Pro Gly
            420                 425                 430
Val Ser Ser Thr Glu Asp Leu Gln Asp Leu Phe Arg Lys Thr Gly Gln
        435                 440                 445
Asp Val Asp Gly Lys Leu Thr Tyr Gln Glu Ile Trp Thr Ser Leu Gly
    450                 455                 460
Ser Ala Met Pro Glu Pro Glu Ser Leu Arg Ala Phe Asp Ser Asp Gly
465                 470                 475                 480
Asp Gly Arg Tyr Ser Phe Leu Glu Leu Arg Val Ala Leu Gly Ile
                485                 490                 495


<210>  57
<211>  1586
<212>  PRT
<213>  Homo sapiens
<220>
<221>  misc_feature
<223>  CDS partielle de la séquence GS-N4:
<1..4762

<220>
<221>  misc_feature
<223>  séquence appelée GS-P4: Angioreceptine

<400>  57
Arg Cys Ala Glu Arg Leu Asn Asn Leu Lys Thr Ser Pro Glu Ala Val
1               5                   10                  15
Ser Gly Tyr Ser Phe Ala Met Ala Ala Leu Leu Gly Gly Val His Gln
            20                  25                  30
Cys Pro Leu Gly Ile Pro His Ala Lys Gly Lys Met Val Val Ser Ile
        35                  40                  45
Ala Glu Asp Leu Leu Arg Thr Ala Ala Gln Asn Ser Arg Leu Ser Leu
    50                  55                  60
Gln Arg Thr Gln Ala Gly Trp Leu Leu Leu Gly Ala Leu Met Thr Leu
65                  70                  75                  80
Gly Pro Ser Val Val Arg Tyr His Leu Pro Lys Met Leu Leu Leu Trp
                85                  90                  95
Arg Asn Val Phe Pro Arg Ser Leu Lys Glu Leu Glu Ala Glu Lys Ala
            100                 105                 110
Arg Gly Asp Ser Phe Thr Trp Gln Val Thr Leu Glu Gly Arg Ala Gly
        115                 120                 125
Ala Leu Cys Ala Met Arg Ser Phe Val Ala His Cys Pro Glu Leu Leu
    130                 135                 140
Thr Glu Asp Val Ile Arg Lys Leu Met Thr Pro Ile Glu Cys Ala Met
145                 150                 155                 160
Thr Met Met Ser His Ile Pro Ser Val Met Lys Ala His Gly Ala His
                165                 170                 175

Leu Lys Ala Ser Ala Ala Met Val Arg Leu Arg Leu Tyr Asp Ile Leu
            180                 185                 190
Ala Leu Leu Pro Pro Lys Thr Tyr Glu Gly Ser Phe Asn Ala Leu Leu
        195                 200                 205
Arg Glu Leu Val Ala Glu Phe Thr Leu Thr Asp Asn Ser Ala Asn Thr
    210                 215                 220
Thr Thr Ser Leu Leu Arg Ser Leu Cys His Tyr Asp Asp Ser Val Leu
225                 230                 235                 240
Leu Gly Ser Trp Leu Gln Glu Thr Asp His Lys Ser Ile Glu Asp Gln
                245                 250                 255
Leu Gln Pro Asn Ser Ala Ser Gly Ser Gly Ala Leu Glu His Asp Pro
            260                 265                 270
Ser Ser Ile Tyr Leu Arg Ile Pro Ala Gly Glu Ala Val Pro Gly Pro
        275                 280                 285
Leu Pro Leu Gly Val Ser Val Ile Asp Ala Ser Val Ala Leu Phe Gly
    290                 295                 300
Val Val Phe Pro His Val Ser Tyr Lys His Arg Leu Gln Met Leu Asp
305                 310                 315                 320
His Phe Ala Glu Cys Val Lys Gln Ala Lys Gly Val Arg Gln Gln Ala
                325                 330                 335
Val Gln Leu Asn Ile Phe Thr Ala Val Leu Ser Ala Leu Lys Gly Leu
            340                 345                 350
Ala Glu Asn Lys Ser Thr Leu Gly Pro Glu Glu Val Arg Lys Ser Ala
        355                 360                 365
Leu Thr Leu Val Met Gly Pro Leu Asp Asn Pro Asn Pro Ile Leu Arg
    370                 375                 380
Cys Ala Ala Gly Glu Ala Leu Gly Arg Met Ala Gln Val Val Gly Glu
385                 390                 395                 400
Ala Thr Phe Ile Ala Arg Met Ala Gln Tyr Ser Phe Asp Lys Leu Lys
                405                 410                 415
Ser Ala Arg Asp Val Val Ser Arg Thr Gly His Ser Leu Ala Leu Gly
            420                 425                 430
Cys Leu His Arg Tyr Val Gly Gly Ile Gly Ser Gly Gln His Leu Lys
        435                 440                 445
Thr Ser Val Ser Ile Leu Leu Ala Leu Ala Gln Asp Gly Thr Ser Pro
    450                 455                 460
Glu Val Gln Thr Trp Ser Leu His Ser Leu Ala Leu Ile Val Asp Ser
465                 470                 475                 480
Ser Gly Pro Met Tyr Arg Gly Tyr Val Glu Pro Thr Leu Ser Leu Val
                485                 490                 495
Leu Thr Leu Leu Leu Thr Val Pro Pro Ser His Thr Glu Val His Gln
            500                 505                 510
Cys Leu Gly Arg Cys Leu Gly Ala Ile Ile Thr Thr Val Gly Pro Glu
        515                 520                 525
Leu Gln Gly Asn Gly Ala Thr Thr Ser Thr Ile Arg Ser Ser Cys Leu
    530                 535                 540
Val Gly Cys Ala Ile Thr Gln Asp His Ser Asp Ser Leu Val Gln Ala
545                 550                 555                 560
Ala Ala Ile Ser Cys Leu Gln Gln Leu His Met Phe Ala Pro Arg His
                565                 570                 575
Val Asn Leu Ser Ser Leu Val Pro Ser Leu Cys Val His Leu Cys Ser
            580                 585                 590
Ser His Leu Leu Leu Arg Arg Ala Ala Val Ala Cys Leu Arg Gln Leu
        595                 600                 605
Ala Gln Arg Glu Ala Ala Glu Val Cys Glu Tyr Ala Met Ser Leu Ala
    610                 615                 620
Lys Asn Thr Gly Asp Lys Glu Ser Ser Ser Ala Asn Val Ser Pro Phe
625                 630                 635                 640
Ala Pro Gly Val Ser Ser Arg Thr Asp Ile His Cys Arg His Gln Gly
                645                 650                 655
Val Asn Ile Thr Glu Thr Gly Leu Glu Gly Leu Leu Phe Gly Met Leu
            660                 665                 670
Asp Arg Glu Thr Asp Arg Lys Leu Cys Ser Asp Ile His Asp Thr Leu
        675                 680                 685
Gly His Met Leu Ser Ser Leu Ala Val Glu Lys Leu Ser His Trp Leu
    690                 695                 700
Met Leu Cys Lys Asp Val Leu Ala Ala Ser Ser Asp Met Ser Thr Ala
705                 710                 715                 720
Thr Leu Leu Ser Ser Gly Lys Asp Glu Glu Ala Glu Lys Lys Asp Glu
                725                 730                 735
Met Asp Asp Asp Thr Met Phe Thr Thr Leu Gly Glu Glu Asp Lys Ser
            740                 745                 750
Lys Pro Phe Val Ala Pro Arg Trp Ala Thr Arg Val Phe Ala Ala Asp

        755                 760                 765
Cys Leu Cys Arg Ile Ile Asn Leu Cys Glu Asn Ala Asp Gln Ala His
    770                 775                 780
Phe Asp Leu Ala Leu Ala Arg Ser Ala Lys Leu Arg Asn Pro Thr Asn
785                 790                 795                 800
Asp Leu Leu Val Leu His Leu Ser Asp Leu Ile Arg Met Ala Phe Met
                805                 810                 815
Ala Ala Thr Asp His Ser Asn Gln Leu Arg Met Ala Gly Leu Gln Ala
            820                 825                 830
Leu Glu Asp Ile Ile Lys Lys Phe Ala Ser Val Pro Glu Pro Glu Phe
        835                 840                 845
Pro Gly His Val Ile Leu Glu Gln Tyr Gln Ala Asn Val Gly Ala Ala
    850                 855                 860
Leu Arg Pro Ala Phe Ser Gln Asp Thr Pro Ser Asp Ile Ile Ala Lys
865                 870                 875                 880
Ala Cys Gln Val Cys Ser Thr Trp Ile Gly Ser Gly Val Val Ser Asp
                885                 890                 895
Leu Asn Asp Leu Arg Arg Val His Asn Leu Leu Val Ser Ser Leu Asp
            900                 905                 910
Lys Val Gln Ala Gly Lys Gly Ser Ser Ser Gln Leu Tyr Arg Glu Ser
        915                 920                 925
Ala Thr Thr Met Glu Lys Leu Ala Val Leu Lys Ala Trp Ala Glu Val
    930                 935                 940
Tyr Val Val Ala Met Asn Ile Lys Lys Glu Ala Glu Ser Lys Pro Lys
945                 950                 955                 960
Arg Ala Ile Lys Asn Thr Asp Asp Asp Asp Asp Cys Gly Thr Ile
                965                 970                 975
Asp Glu Leu Pro Pro Asp Ser Leu Ile Thr Leu Val Gln Pro Glu Leu
                980                 985                 990
Pro Thr Leu Ser Arg Leu Trp Leu Ala Ala Leu Lys Asp Tyr Ala Leu
            995                 1000                1005
Leu Thr Leu Pro Ala Glu Phe Ser Ser Gln Leu Pro Pro Asp Gly
        1010                1015                1020
Gly Ala Phe Tyr Thr Pro Glu Thr Ile Asp Thr Ala Arg Leu His
        1025                1030                1035
Tyr Arg Asn Ser Trp Ala Pro Ile Leu His Ala Val Ala Leu Trp
        1040                1045                1050
Leu Asn Ser Thr Gly Phe Thr Cys Ser Glu Ser Thr Glu Ala Ala
        1055                1060                1065
Ala Ile Ser Gly Leu Gln Lys Arg Ser Thr Ser Val Asn Leu Asn
        1070                1075                1080
Gln Ala Ser Gly Ala Val Gly Ser Ala Lys Ser Leu Pro Glu Ile
        1085                1090                1095
Asn Lys Asp Arg Met His Leu Ile Leu Gly Val Ser Ile Gln Phe
        1100                1105                1110
Leu Cys Ser Pro Arg Pro Glu Glu Pro Ile Glu His Val Thr Ala
        1115                1120                1125
Cys Leu Gln Ala Leu His Thr Leu Leu Asp Ser Pro Tyr Ala Arg
        1130                1135                1140
Val His Ile Ala Glu Asp Gln Leu Ile Gly Val Glu Leu Leu Ser
        1145                1150                1155
Val Leu His Arg Leu Leu Leu Thr Trp Asn Pro Ser Ser Val Gln
        1160                1165                1170
Leu Leu Val Thr Gly Val Val Gln Gln Ile Val Arg Ala Ala Gln
        1175                1180                1185
Asp Tyr Leu Gln Glu Lys Arg Asn Thr Leu Asn Glu Asp Asp Met
        1190                1195                1200
Glu Lys Glu Ala Cys Thr Val Leu Gly Glu Gly Gly Asp Ser Gly
        1205                1210                1215
Gly Leu Ile Pro Gly Lys Ser Leu Val Phe Ala Thr Met Glu Leu
        1220                1225                1230
Leu Met Phe Ile Leu Val Arg His Met Pro His Leu Ser Thr Lys
        1235                1240                1245
Val Ser Asp Ser Pro Ser His Ile Ala Thr Lys Thr Arg Leu Ser
        1250                1255                1260
Glu Glu Ser Ala Arg Leu Val Ala Ala Thr Val Thr Ile Leu Ser
        1265                1270                1275
Asp Leu Pro Ser Leu Cys Ser Pro Ala Gly Cys Met Thr Ile Leu
        1280                1285                1290
Pro Thr Ile Leu Phe Leu Ile Ala Arg Ile Leu Lys Asp Thr Ala
        1295                1300                1305
Ile Lys Ser Ala Asp Asn Gln Val Pro Pro Pro Val Ser Ala Ala
        1310                1315                1320

Leu Gln Gly Ile Lys Ser Ile Val Thr Leu Ser Met Ala Lys Thr
    1325                1330                1335
Glu Ala Gly Val Gln Lys Gln Trp Thr Ala Leu Ile Arg Ser Thr
    1340                1345                1350
Leu Ala Cys Ile Leu Glu Tyr Ser Gln Pro Glu Asp Ser Val Pro
    1355                1360                1365
Thr Pro Asp Glu Val Ser Met Leu Thr Ala Ile Ala Leu Phe Leu
    1370                1375                1380
Trp Ser Ala Ser Asn Glu Ile Ile Gly Val Gln Ser Leu Gln Asn
    1385                1390                1395
Gly Cys Met Asn Arg Phe Lys Asn Ala Leu Asn Ser Cys Asp Pro
    1400                1405                1410
Trp Val Gln Ala Lys Cys Tyr Gln Leu Leu Leu Ser Val Phe Gln
    1415                1420                1425
His Ser Asn Arg Ala Leu Ser Thr Pro Tyr Ile His Ser Leu Ala
    1430                1435                1440
Pro Ile Val Val Glu Lys Leu Lys Ala Val Glu Arg Asn Arg Pro
    1445                1450                1455
Ala Ser Asn Ile Glu Leu Leu Ala Val Gln Glu Gly Ile Lys Val
    1460                1465                1470
Leu Glu Thr Leu Val Ala Leu Gly Glu Glu Gln Asn Arg Val Gln
    1475                1480                1485
Leu Leu Ala Leu Leu Val Pro Thr Leu Ile Ser Tyr Leu Leu Asp
    1490                1495                1500
Glu Asn Ser Phe Ala Ser Ala Ser Ser Ala Ser Lys Asp Leu His
    1505                1510                1515
Glu Phe Ala Leu Gln Asn Leu Met His Ile Gly Pro Leu Tyr Pro
    1520                1525                1530
His Ala Phe Lys Thr Val Met Gly Ala Ala Pro Glu Leu Lys Val
    1535                1540                1545
Arg Leu Glu Thr Ala Val Arg Ala Ser Gln Ala Ser Lys Ala Lys
    1550                1555                1560
Ala Ala Ala Arg Gln Pro Ala Pro Ala Ile His Ser Ala Pro Thr
    1565                1570                1575
Ile Lys Leu Lys Thr Ser Phe Phe
    1580                1585


<210>  58
<211>  217
<212>  PRT
<213>  Homo sapiens
<220>
<221>  misc_feature
<223>  CDS de la séquence GS-N5: 38..691

<220>
<221>  misc_feature
<223>  séquence appelée GS-P5: Angiodensine

<400>  58
Met Ala Val Arg Pro Lys Glu Arg Ser Ser Leu Ser Ser Arg Gln His
1               5                   10                  15
Pro Phe Val Arg Ser Ser Val Ile Val Arg Ser Gln Thr Phe Ser Pro
            20                  25                  30
Gly Glu Arg Asn Gln Tyr Ile Cys Arg Leu Asn Arg Ser Asp Ser Asp
        35                  40                  45
Ser Ser Thr Leu Ala Lys Lys Ser Leu Phe Val Arg Asn Ser Thr Glu
    50                  55                  60
Arg Arg Ser Leu Arg Val Lys Arg Thr Val Cys Gln Ser Val Leu Arg
65                  70                  75                  80
Arg Thr Thr Gln Glu Cys Pro Val Arg Thr Ser Leu Asp Leu Glu Leu
                85                  90                  95
Asp Leu Gln Ala Ser Leu Thr Arg Gln Ser Arg Leu Asn Asp Glu Leu
            100                 105                 110
Gln Ala Leu Arg Asp Leu Arg Gln Lys Leu Glu Glu Leu Lys Ala Gln
        115                 120                 125
Gly Glu Thr Asp Leu Pro Pro Gly Val Leu Glu Asp Glu Arg Phe Gln
    130                 135                 140
Arg Leu Leu Lys Gln Ala Glu Lys Gln Ala Glu Gln Ser Lys Glu Glu
145                 150                 155                 160
Gln Lys Gln Gly Leu Asn Ala Glu Lys Leu Met Arg Gln Val Ser Lys
                165                 170                 175

Asp Val Cys Arg Leu Arg Glu Gln Ser Gln Lys Val Pro Arg Gln Val
            180                 185                 190
Gln Ser Phe Arg Glu Lys Ile Ala Tyr Phe Thr Arg Ala Lys Ile Ser
        195                 200                 205
Ile Pro Ser Leu Pro Ala Asp Asp Val
    210                 215


<210>  59
<211>  227
<212>  PRT
<213>  Homo sapiens
<220>
<221>  misc_feature
<223>  CDS de la séquence GS-N6: 90..773

<220>
<221>  misc_feature
<223>  séquence appelée GS-P6: Vassoserpentine

<400>  59
Met Gly Asp Ala Gly Ser Glu Arg Ser Lys Ala Pro Ser Leu Pro Pro
1               5                   10                  15
Arg Cys Pro Cys Gly Phe Trp Gly Ser Ser Lys Thr Met Asn Leu Cys
            20                  25                  30
Ser Lys Cys Phe Ala Asp Phe Gln Lys Lys Gln Pro Asp Asp Asp Ser
        35                  40                  45
Ala Pro Ser Thr Ser Asn Ser Gln Ser Asp Leu Phe Ser Glu Glu Thr
    50                  55                  60
Thr Ser Asp Asn Asn Asn Thr Ser Ile Thr Thr Pro Thr Leu Ser Pro
65                  70                  75                  80
Ser Gln Gln Pro Leu Pro Thr Glu Leu Asn Val Thr Ser Pro Ser Lys
                85                  90                  95
Glu Glu Cys Gly Pro Cys Thr Asp Thr Ala His Val Ser Leu Ile Thr
            100                 105                 110
Pro Thr Lys Arg Ser Cys Gly Thr Asp Ser Gln Ser Glu Asn Glu Ala
        115                 120                 125
Ser Pro Val Lys Arg Pro Arg Leu Leu Glu Asn Thr Glu Arg Ser Glu
    130                 135                 140
Glu Thr Ser Arg Ser Lys Gln Lys Ser Arg Arg Arg Cys Phe Gln Cys
145                 150                 155                 160
Gln Thr Lys Leu Glu Leu Val Gln Gln Glu Leu Gly Ser Cys Arg Cys
                165                 170                 175
Gly Tyr Val Phe Cys Met Leu His Arg Leu Pro Glu Gln His Asp Cys
            180                 185                 190
Thr Phe Asp His Met Gly Arg Gly Arg Glu Glu Ala Ile Met Lys Met
        195                 200                 205
Val Lys Leu Asp Arg Lys Val Gly Arg Ser Cys Gln Arg Ile Gly Glu
    210                 215                 220
Gly Cys Ser
225


<210>  60
<211>  885
<212>  PRT
<213>  Homo sapiens
<220>
<221>  misc_feature
<223>  CDS partielle  de la séquence GS-N7:
<286..2943

<220>
<221>  misc_feature
<223>  séquence GS-P7: Angiosulfatine

<400>  60
Asp Ser Arg Ile Pro Lys Glu Ala Pro Asp Gln Gln Lys Lys Lys Met
1               5                   10                  15
Gly Pro Pro Ser Leu Val Leu Cys Leu Leu Ser Ala Thr Val Phe Ser
            20                  25                  30
Leu Leu Gly Gly Ser Ser Ala Phe Leu Ser His His Arg Leu Lys Gly
        35                  40                  45

Arg Phe Gln Arg Asp Arg Arg Asn Ile Arg Pro Asn Ile Ile Leu Val
    50                  55                  60
Leu Thr Asp Asp Gln Asp Val Glu Leu Gly Ser Met Gln Val Met Asn
65                  70                  75                  80
Lys Thr Arg Arg Ile Met Glu Gln Gly Gly Thr His Phe Ile Asn Ala
                85                  90                  95
Phe Val Thr Thr Pro Met Cys Cys Pro Ser Arg Ser Ser Ile Leu Thr
            100                 105                 110
Gly Lys Tyr Val His Asn His Asn Thr Tyr Thr Asn Asn Glu Asn Cys
        115                 120                 125
Ser Ser Pro Ser Trp Gln Ala Gln His Glu Ser Arg Thr Phe Ala Val
    130                 135                 140
Tyr Leu Asn Ser Thr Gly Tyr Arg Thr Ala Phe Phe Gly Lys Tyr Leu
145                 150                 155                 160
Asn Glu Tyr Asn Gly Ser Tyr Val Pro Pro Gly Trp Lys Glu Trp Val
                165                 170                 175
Gly Leu Leu Lys Asn Ser Arg Phe Tyr Asn Tyr Thr Leu Cys Arg Asn
            180                 185                 190
Gly Val Lys Glu Lys His Gly Ser Asp Tyr Ser Lys Asp Tyr Leu Thr
        195                 200                 205
Asp Leu Ile Thr Asn Asp Ser Val Ser Phe Phe Arg Thr Ser Lys Lys
    210                 215                 220
Met Tyr Pro His Arg Pro Val Leu Met Val Ile Ser His Ala Ala Pro
225                 230                 235                 240
His Gly Pro Glu Asp Ser Ala Pro Gln Tyr Ser Arg Leu Phe Pro Asn
                245                 250                 255
Ala Ser Gln His Ile Thr Pro Ser Tyr Asn Tyr Ala Pro Asn Pro Asp
            260                 265                 270
Lys His Trp Ile Met Arg Tyr Thr Gly Pro Met Lys Pro Ile His Met
        275                 280                 285
Glu Phe Thr Asn Met Leu Gln Arg Lys Arg Leu Gln Thr Leu Met Ser
    290                 295                 300
Val Asp Asp Ser Met Glu Thr Ile Tyr Asn Met Leu Val Glu Thr Gly
305                 310                 315                 320
Glu Leu Asp Asn Thr Tyr Ile Val Tyr Thr Ala Asp His Gly Tyr His
                325                 330                 335
Ile Gly Gln Phe Gly Leu Val Lys Gly Lys Ser Met Pro Tyr Glu Phe
            340                 345                 350
Asp Ile Arg Val Pro Phe Tyr Val Arg Gly Pro Asn Val Glu Ala Gly
        355                 360                 365
Cys Leu Asn Pro His Ile Val Leu Asn Ile Asp Leu Ala Pro Thr Ile
    370                 375                 380
Leu Asp Ile Ala Gly Leu Asp Ile Pro Ala Asp Met Asp Gly Lys Ser
385                 390                 395                 400
Ile Leu Lys Leu Leu Asp Thr Glu Arg Pro Val Asn Arg Phe His Leu
                405                 410                 415
Lys Lys Lys Met Arg Val Trp Arg Asp Ser Phe Leu Val Glu Arg Gly
            420                 425                 430
Lys Leu Leu His Lys Arg Asp Asn Asp Lys Val Asp Ala Gln Glu Glu
        435                 440                 445
Asn Phe Leu Pro Lys Tyr Gln Arg Val Lys Asp Leu Cys Gln Arg Ala
    450                 455                 460
Glu Tyr Gln Thr Ala Cys Glu Gln Leu Gly Gln Lys Trp Gln Cys Val
465                 470                 475                 480
Glu Asp Ala Thr Gly Lys Leu Lys Leu His Lys Cys Lys Gly Pro Met
                485                 490                 495
Arg Leu Gly Gly Ser Arg Ala Leu Ser Asn Leu Val Pro Lys Tyr Tyr
            500                 505                 510
Gly Gln Gly Ser Glu Ala Cys Thr Cys Asp Ser Gly Asp Tyr Lys Leu
        515                 520                 525
Ser Leu Ala Gly Arg Arg Lys Lys Leu Phe Lys Lys Lys Tyr Lys Ala
    530                 535                 540
Ser Tyr Val Arg Ser Arg Ser Ile Arg Ser Val Ala Ile Glu Val Asp
545                 550                 555                 560
Gly Arg Val Tyr His Val Gly Leu Gly Asp Ala Ala Gln Pro Arg Asn
                565                 570                 575
Leu Thr Lys Arg His Trp Pro Gly Ala Pro Glu Asp Gln Asp Asp Lys
            580                 585                 590
Asp Gly Gly Asp Phe Ser Gly Thr Gly Gly Leu Pro Asp Tyr Ser Ala
        595                 600                 605
Ala Asn Pro Ile Lys Val Thr His Arg Cys Tyr Ile Leu Glu Asn Asp
    610                 615                 620
Thr Val Gln Cys Asp Leu Asp Leu Tyr Lys Ser Leu Gln Ala Trp Lys

625                 630                 635                 640
Asp His Lys Leu His Ile Asp His Glu Ile Glu Thr Leu Gln Asn Lys
                645                 650                 655
Ile Lys Asn Leu Arg Glu Val Arg Gly His Leu Lys Lys Lys Arg Pro
            660                 665                 670
Glu Glu Cys Asp Cys His Lys Ile Ser Tyr His Thr Gln His Lys Gly
        675                 680                 685
Arg Leu Lys His Arg Gly Ser Ser Leu His Pro Phe Arg Lys Gly Leu
    690                 695                 700
Gln Glu Lys Asp Lys Val Trp Leu Leu Arg Glu Gln Lys Arg Lys Lys
705                 710                 715                 720
Lys Leu Arg Lys Leu Leu Lys Arg Leu Gln Asn Asn Asp Thr Cys Ser
                725                 730                 735
Met Pro Gly Leu Thr Cys Phe Thr His Asp Asn Gln His Trp Gln Thr
            740                 745                 750
Ala Pro Phe Trp Thr Leu Gly Pro Phe Cys Ala Cys Thr Ser Ala Asn
        755                 760                 765
Asn Asn Thr Tyr Trp Cys Met Arg Thr Ile Asn Glu Thr His Asn Phe
    770                 775                 780
Leu Phe Cys Glu Phe Ala Thr Gly Phe Leu Glu Tyr Phe Asp Leu Asn
785                 790                 795                 800
Thr Asp Pro Tyr Gln Leu Met Asn Ala Val Asn Thr Leu Asp Arg Asp
                805                 810                 815
Val Leu Asn Gln Leu His Val Gln Leu Met Glu Leu Arg Ser Cys Lys
            820                 825                 830
Gly Tyr Lys Gln Cys Asn Pro Arg Thr Arg Asn Met Asp Leu Gly Leu
        835                 840                 845
Lys Asp Gly Gly Ser Tyr Glu Gln Tyr Arg Gln Phe Gln Arg Arg Lys
    850                 855                 860
Trp Pro Glu Met Lys Arg Pro Ser Ser Lys Ser Leu Gly Gln Leu Trp
865                 870                 875                 880
Glu Gly Trp Glu Gly
                885


<210>  61
<211>  1151
<212>  PRT
<213>  Homo sapiens
<220>
<221>  misc_feature
<223>  CDS partielle de la séquence GS-N8:
<1..3456

<220>
<221>  misc_feature
<223>  séquence appelée GS-P8: Vassoréceptine

<400>  61
Val Leu Cys Cys Tyr Cys Lys Asn Glu Met Ser Gly Pro Ala Gln Val
1               5                   10                  15
Pro Met Met Ser Pro Asn Gly Ser Val Pro Pro Ile Tyr Val Pro Pro
            20                  25                  30
Gly Tyr Ala Pro Gln Val Ile Glu Asp Asn Gly Val Arg Arg Val Val
        35                  40                  45
Val Val Pro Gln Ala Pro Glu Phe His Pro Gly Ser His Thr Val Leu
    50                  55                  60
His Arg Ser Pro His Pro Pro Leu Pro Gly Phe Ile Pro Val Pro Thr
65                  70                  75                  80
Met Met Pro Pro Pro Pro Arg His Met Tyr Ser Pro Val Thr Gly Ala
                85                  90                  95
Gly Asp Met Thr Thr Gln Tyr Met Pro Gln Tyr Gln Ser Ser Gln Val
            100                 105                 110
Tyr Gly Asp Val Asp Ala His Ser Thr His Gly Arg Ser Asn Phe Arg
        115                 120                 125
Asp Glu Arg Ser Ser Lys Thr Tyr Glu Arg Leu Gln Lys Lys Leu Lys
    130                 135                 140
Asp Arg Gln Gly Thr Gln Lys Asp Lys Met Ser Ser Pro Pro Ser Ser
145                 150                 155                 160
Pro Gln Lys Cys Pro Ser Pro Ile Asn Glu His Asn Gly Leu Ile Lys
                165                 170                 175
Gly Gln Ile Ala Gly Gly Ile Asn Thr Gly Ser Ala Lys Ile Lys Ser
            180                 185                 190

Gly Lys Gly Lys Gly Gly Thr Gln Val Asp Thr Glu Ile Glu Glu Lys
        195                 200                 205
Asp Glu Glu Thr Lys Ala Phe Glu Ala Leu Leu Ser Asn Ile Val Lys
    210                 215                 220
Pro Val Ala Ser Asp Ile Gln Ala Arg Thr Val Val Leu Thr Trp Ser
225                 230                 235                 240
Pro Pro Ser Ser Leu Ile Asn Gly Glu Thr Asp Glu Ser Ser Val Pro
                245                 250                 255
Glu Leu Tyr Gly Tyr Glu Val Leu Ile Ser Ser Thr Gly Lys Asp Gly
            260                 265                 270
Lys Tyr Lys Ser Val Tyr Val Gly Glu Glu Thr Asn Ile Thr Leu Asn
        275                 280                 285
Asp Leu Lys Pro Ala Met Asp Tyr His Ala Lys Val Gln Ala Glu Tyr
    290                 295                 300
Asn Ser Ile Lys Gly Thr Pro Ser Glu Ala Glu Ile Phe Thr Thr Leu
305                 310                 315                 320
Ser Cys Glu Pro Asp Ile Pro Asn Pro Pro Arg Ile Ala Asn Arg Thr
                325                 330                 335
Lys Asn Ser Leu Thr Leu Gln Trp Lys Ala Pro Ser Asp Asn Gly Ser
            340                 345                 350
Lys Ile Gln Asn Phe Val Leu Glu Trp Asp Glu Gly Lys Gly Asn Gly
        355                 360                 365
Glu Phe Cys Gln Cys Tyr Met Gly Ser Gln Lys Gln Phe Lys Ile Thr
    370                 375                 380
Lys Leu Ser Pro Ala Met Gly Cys Lys Phe Arg Leu Ser Ala Arg Asn
385                 390                 395                 400
Asp Tyr Gly Thr Ser Gly Phe Ser Glu Glu Val Leu Tyr Tyr Thr Ser
                405                 410                 415
Gly Cys Ala Pro Ser Met Pro Ala Ser Pro Val Leu Thr Lys Ala Gly
            420                 425                 430
Ile Thr Trp Leu Ser Leu Gln Trp Ser Lys Pro Ser Gly Thr Pro Ser
        435                 440                 445
Asp Glu Gly Ile Ser Tyr Ile Leu Glu Met Glu Glu Glu Thr Ser Gly
    450                 455                 460
Tyr Gly Phe Lys Pro Lys Tyr Asp Gly Glu Asp Leu Ala Tyr Thr Val
465                 470                 475                 480
Lys Asn Leu Arg Arg Ser Thr Lys Tyr Lys Phe Lys Val Ile Ala Tyr
                485                 490                 495
Asn Ser Glu Gly Lys Ser Asn Pro Ser Glu Val Val Glu Phe Thr Thr
            500                 505                 510
Cys Pro Asp Lys Pro Gly Ile Pro Val Lys Pro Ser Val Lys Gly Lys
        515                 520                 525
Ile His Ser His Ser Phe Lys Ile Thr Trp Asp Pro Pro Lys Asp Asn
    530                 535                 540
Gly Gly Ala Thr Ile Asn Lys Tyr Val Val Glu Met Ala Glu Gly Ser
545                 550                 555                 560
Asn Gly Asn Lys Trp Glu Met Ile Tyr Ser Gly Ala Thr Arg Glu His
                565                 570                 575
Leu Cys Asp Arg Leu Asn Pro Gly Cys Phe Tyr Arg Leu Arg Val Tyr
            580                 585                 590
Cys Ile Ser Asp Gly Gly Gln Ser Ala Val Ser Glu Ser Leu Leu Val
        595                 600                 605
Gln Thr Pro Ala Val Pro Pro Gly Pro Cys Leu Pro Pro Arg Leu Gln
    610                 615                 620
Gly Arg Pro Lys Ala Lys Glu Ile Gln Leu Arg Trp Gly Pro Pro Leu
625                 630                 635                 640
Val Asp Gly Gly Ser Pro Ile Ser Cys Tyr Ser Val Glu Met Ser Pro
                645                 650                 655
Ile Glu Lys Asp Glu Pro Arg Glu Val Tyr Gln Gly Ser Glu Val Glu
            660                 665                 670
Cys Thr Val Ser Ser Leu Leu Pro Gly Lys Thr Tyr Ser Phe Arg Leu
        675                 680                 685
Arg Ala Ala Asn Lys Met Gly Phe Gly Pro Phe Ser Glu Lys Cys Asp
    690                 695                 700
Ile Thr Thr Ala Pro Gly Pro Pro Asp Gln Cys Lys Pro Pro Gln Val
705                 710                 715                 720
Thr Cys Arg Ser Ala Thr Cys Ala Gln Val Asn Trp Glu Val Pro Leu
                725                 730                 735
Ser Asn Gly Thr Asp Val Thr Glu Tyr Arg Leu Glu Trp Gly Gly Val
            740                 745                 750
Glu Gly Ser Met Gln Ile Cys Tyr Cys Gly Pro Gly Leu Ser Tyr Glu
        755                 760                 765
Ile Lys Gly Leu Ser Pro Ala Thr Thr Tyr Tyr Cys Arg Val Gln Ala

770                 775                 780
Leu Ser Val Val Gly Ala Gly Pro Phe Ser Glu Val Val Ala Cys Val
785                 790                 795                 800
Thr Pro Pro Ser Val Pro Gly Ile Val Thr Cys Leu Gln Glu Ile Ser
                805                 810                 815
Asp Asp Glu Ile Glu Asn Pro His Tyr Ser Pro Ser Thr Cys Leu Ala
            820                 825                 830
Ile Ser Trp Glu Lys Pro Cys Asp His Gly Ser Glu Ile Leu Ala Tyr
        835                 840                 845
Ser Ile Asp Phe Gly Asp Lys Gln Ser Leu Thr Val Gly Lys Val Thr
    850                 855                 860
Ser Tyr Ile Ile Asn Asn Leu Gln Pro Asp Thr Thr Tyr Arg Ile Arg
865                 870                 875                 880
Ile Gln Ala Leu Asn Ser Leu Gly Ala Gly Pro Phe Ser His Met Ile
                885                 890                 895
Lys Leu Lys Thr Lys Pro Leu Pro Pro Asp Pro Pro Arg Leu Glu Cys
            900                 905                 910
Val Ala Phe Ser His Gln Asn Leu Lys Leu Lys Trp Gly Glu Gly Thr
        915                 920                 925
Pro Lys Thr Leu Ser Thr Asp Ser Ile Gln Tyr His Leu Gln Met Glu
    930                 935                 940
Asp Lys Asn Gly Arg Phe Val Ser Leu Tyr Arg Gly Pro Cys His Thr
945                 950                 955                 960
Tyr Lys Val Gln Arg Leu Asn Glu Ser Thr Ser Tyr Lys Phe Cys Ile
                965                 970                 975
Gln Ala Cys Asn Glu Ala Gly Glu Gly Pro Leu Ser Gln Glu Tyr Ile
            980                 985                 990
Phe Thr Thr Pro Lys Ser Val Pro  Ala Ala Leu Lys Ala  Pro Lys Ile
        995                 1000                1005
Glu Lys  Val Asn Asp His Ile  Cys Glu Ile Thr Trp  Glu Cys Leu
    1010                1015                1020
Gln Pro  Met Lys Gly Asp Pro  Val Ile Tyr Ser Leu  Gln Val Met
    1025                1030                1035
Leu Gly  Lys Asp Ser Glu Phe  Lys Gln Ile Tyr Lys  Gly Pro Asp
    1040                1045                1050
Ser Ser  Phe Arg Tyr Ser Ser  Leu Gln Leu Asn Cys  Glu Tyr Arg
    1055                1060                1065
Phe Arg  Val Cys Ala Ile Arg  Gln Cys Gln Asp Ser  Leu Gly His
    1070                1075                1080
Gln Asp  Leu Val Gly Pro Tyr  Ser Thr Thr Val Leu  Phe Ile Ser
    1085                1090                1095
Gln Arg  Thr Glu Pro Pro Ala  Ser Thr Asn Arg Asp  Thr Val Glu
    1100                1105                1110
Ser Thr  Arg Thr Arg Arg Ala  Leu Ser Asp Glu Gln  Cys Ala Ala
    1115                1120                1125
Val Ile  Leu Val Leu Phe Ala  Phe Phe Ser Ile Leu  Ile Ala Phe
    1130                1135                1140
Ile Ile  Gln Tyr Phe Val Ile  Lys
    1145                1150


<210>  62
<211>  1175
<212>  PRT
<213>  Homo sapiens
<220>
<221>  misc_feature
<223>  CDS partielle de la séquence  GS-N9:
<1..3528

<220>
<221>  misc_feature
<223>  séquence appelée GS-P9: Angiokinasine

<400>  62
Gln Val Tyr Lys Gly Arg His Val Lys Thr Gly Gln Leu Ala Ala Ile
1               5                   10                  15
Lys Val Met Asp Val Thr Glu Asp Glu Glu Glu Glu Ile Lys Leu Glu
            20                  25                  30
Ile Asn Met Leu Lys Lys Tyr Ser His His Arg Asn Ile Ala Thr Tyr
        35                  40                  45
Tyr Gly Ala Phe Ile Lys Lys Ser Pro Pro Gly His Asp Asp Gln Leu
    50                  55                  60

Trp Leu Val Met Glu Phe Cys Gly Ala Gly Ser Ile Thr Asp Leu Val
65                  70                  75                  80
Lys Asn Thr Lys Gly Asn Thr Leu Lys Glu Asp Trp Ile Ala Tyr Ile
                85                  90                  95
Ser Arg Glu Ile Leu Arg Gly Leu Ala His Leu His Ile His His Val
            100                 105                 110
Ile His Arg Asp Ile Lys Gly Gln Asn Val Leu Leu Thr Glu Asn Ala
        115                 120                 125
Glu Val Lys Leu Val Asp Phe Gly Val Ser Ala Gln Leu Asp Arg Thr
    130                 135                 140
Val Gly Arg Arg Asn Thr Phe Ile Gly Thr Pro Tyr Trp Met Ala Pro
145                 150                 155                 160
Glu Val Ile Ala Cys Asp Glu Asn Pro Asp Ala Thr Tyr Asp Tyr Arg
                165                 170                 175
Ser Asp Leu Trp Ser Cys Gly Ile Thr Ala Ile Glu Met Ala Glu Gly
            180                 185                 190
Ala Pro Pro Leu Cys Asp Met His Pro Met Arg Ala Leu Phe Leu Ile
        195                 200                 205
Pro Arg Asn Pro Pro Pro Arg Leu Lys Ser Lys Lys Trp Ser Lys Lys
    210                 215                 220
Phe Phe Ser Phe Ile Glu Gly Cys Leu Val Lys Asn Tyr Met Gln Arg
225                 230                 235                 240
Pro Ser Thr Glu Gln Leu Leu Lys His Pro Phe Ile Arg Asp Gln Pro
                245                 250                 255
Asn Glu Arg Gln Val Arg Ile Gln Leu Lys Asp His Ile Asp Arg Thr
            260                 265                 270
Arg Lys Lys Arg Gly Glu Lys Asp Glu Thr Glu Tyr Glu Tyr Ser Gly
        275                 280                 285
Ser Glu Glu Glu Glu Glu Glu Val Pro Glu Gln Glu Gly Glu Pro Ser
    290                 295                 300
Ser Ile Val Asn Val Pro Gly Glu Ser Thr Leu Arg Arg Asp Phe Leu
305                 310                 315                 320
Arg Leu Gln Gln Glu Asn Lys Glu Arg Ser Glu Ala Leu Arg Arg Gln
                325                 330                 335
Gln Leu Leu Gln Glu Gln Gln Leu Arg Glu Gln Glu Glu Tyr Lys Arg
            340                 345                 350
Gln Leu Leu Ala Glu Arg Gln Lys Arg Ile Glu Gln Gln Lys Glu Gln
        355                 360                 365
Arg Arg Arg Leu Glu Glu Gln Gln Arg Arg Glu Arg Glu Ala Arg Arg
    370                 375                 380
Gln Gln Glu Arg Glu Gln Arg Arg Arg Glu Gln Glu Glu Lys Arg Arg
385                 390                 395                 400
Leu Glu Glu Leu Glu Arg Arg Arg Lys Glu Glu Glu Glu Arg Arg Arg
                405                 410                 415
Ala Glu Glu Glu Lys Arg Arg Val Glu Arg Glu Gln Glu Tyr Ile Arg
            420                 425                 430
Arg Gln Leu Glu Glu Glu Gln Arg His Leu Glu Val Leu Gln Gln Gln
        435                 440                 445
Leu Leu Gln Glu Gln Ala Met Leu Leu His Asp His Arg Arg Pro His
    450                 455                 460
Pro Gln His Ser Gln Gln Pro Pro Pro Pro Gln Gln Glu Arg Ser Lys
465                 470                 475                 480
Pro Ser Phe His Ala Pro Glu Pro Lys Ala His Tyr Glu Pro Ala Asp
                485                 490                 495
Arg Ala Arg Glu Val Glu Asp Arg Phe Arg Lys Thr Asn His Ser Ser
            500                 505                 510
Pro Glu Ala Gln Ser Lys Gln Thr Gly Arg Val Leu Glu Pro Pro Val
        515                 520                 525
Pro Ser Arg Ser Glu Ser Phe Ser Asn Gly Asn Ser Glu Ser Val His
    530                 535                 540
Pro Ala Leu Gln Arg Pro Ala Glu Pro Gln Val Pro Val Arg Thr Thr
545                 550                 555                 560
Ser Arg Ser Pro Val Leu Ser Arg Arg Asp Ser Pro Leu Gln Gly Ser
                565                 570                 575
Gly Gln Gln Asn Ser Gln Ala Gly Gln Arg Asn Ser Thr Ser Ser Ile
            580                 585                 590
Glu Pro Arg Leu Leu Trp Glu Arg Val Glu Lys Leu Val Pro Arg Pro
        595                 600                 605
Gly Ser Gly Ser Ser Ser Gly Ser Ser Asn Ser Gly Ser Gln Pro Gly
    610                 615                 620
Ser His Pro Gly Ser Gln Ser Gly Ser Gly Glu Arg Phe Arg Val Arg
625                 630                 635                 640
Ser Ser Ser Lys Ser Glu Gly Ser Pro Ser Gln Arg Leu Glu Asn Ala

                645                 650                 655
Val Lys Lys Pro Glu Asp Lys Lys Glu Val Phe Arg Pro Leu Lys Pro
            660                 665                 670
Ala Gly Glu Val Asp Leu Thr Ala Leu Ala Lys Glu Leu Arg Ala Val
        675                 680                 685
Glu Asp Val Arg Pro Pro His Lys Val Thr Asp Tyr Ser Ser Ser Ser
    690                 695                 700
Glu Glu Ser Gly Thr Thr Asp Glu Glu Asp Asp Asp Val Glu Gln Glu
705                 710                 715                 720
Gly Ala Asp Glu Ser Thr Ser Gly Pro Glu Asp Thr Arg Ala Ala Ser
                725                 730                 735
Ser Leu Asn Leu Ser Asn Gly Glu Thr Glu Ser Val Lys Thr Met Ile
            740                 745                 750
Val His Asp Asp Val Glu Ser Glu Pro Ala Met Thr Pro Ser Lys Glu
        755                 760                 765
Gly Thr Leu Ile Val Arg Gln Thr Gln Ser Ala Ser Ser Thr Leu Gln
    770                 775                 780
Lys His Lys Ser Ser Ser Ser Phe Thr Pro Phe Ile Asp Pro Arg Leu
785                 790                 795                 800
Leu Gln Ile Ser Pro Ser Ser Gly Thr Thr Val Thr Ser Val Val Gly
                805                 810                 815
Phe Ser Cys Asp Gly Met Arg Pro Glu Ala Ile Arg Gln Asp Pro Thr
            820                 825                 830
Arg Lys Gly Ser Val Val Asn Val Asn Pro Thr Asn Thr Arg Pro Gln
        835                 840                 845
Ser Asp Thr Pro Glu Ile Arg Lys Tyr Lys Lys Arg Phe Asn Ser Glu
    850                 855                 860
Ile Leu Cys Ala Ala Leu Trp Gly Val Asn Leu Leu Val Gly Thr Glu
865                 870                 875                 880
Ser Gly Leu Met Leu Leu Asp Arg Ser Gly Gln Gly Lys Val Tyr Pro
                885                 890                 895
Leu Ile Asn Arg Arg Arg Phe Gln Gln Met Asp Val Leu Glu Gly Leu
            900                 905                 910
Asn Val Leu Val Thr Ile Ser Gly Lys Lys Asp Lys Leu Arg Val Tyr
        915                 920                 925
Tyr Leu Ser Trp Leu Arg Asn Lys Ile Leu His Asn Asp Pro Glu Val
    930                 935                 940
Glu Lys Lys Gln Gly Trp Thr Thr Val Gly Asp Leu Glu Gly Cys Val
945                 950                 955                 960
His Tyr Lys Val Val Lys Tyr Glu Arg Ile Lys Phe Leu Val Ile Ala
                965                 970                 975
Leu Lys Ser Ser Val Glu Val Tyr Ala Trp Ala Pro Lys Pro Tyr His
            980                 985                 990
Lys Phe Met Ala Phe Lys Ser Phe Gly Glu Leu Val His Lys Pro Leu
        995                 1000                1005
Leu Val Asp Leu Thr Val Glu Glu Gly Gln Arg Leu Lys Val Ile
    1010                1015                1020
Tyr Gly Ser Cys Ala Gly Phe His Ala Val Asp Val Asp Ser Gly
    1025                1030                1035
Ser Val Tyr Asp Ile Tyr Leu Pro Thr His Ile Gln Cys Ser Ile
    1040                1045                1050
Lys Pro His Ala Ile Ile Ile Leu Pro Asn Thr Asp Gly Met Glu
    1055                1060                1065
Leu Leu Val Cys Tyr Glu Asp Glu Gly Val Tyr Val Asn Thr Tyr
    1070                1075                1080
Gly Arg Ile Thr Lys Asp Val Val Leu Gln Trp Gly Glu Met Pro
    1085                1090                1095
Thr Ser Val Ala Tyr Ile Arg Ser Asn Gln Thr Met Gly Trp Gly
    1100                1105                1110
Glu Lys Ala Ile Glu Ile Arg Ser Val Glu Thr Gly His Leu Asp
    1115                1120                1125
Gly Val Phe Met His Lys Arg Ala Gln Arg Leu Lys Phe Leu Cys
    1130                1135                1140
Glu Arg Asn Asp Lys Val Phe Phe Ala Ser Val Arg Ser Gly Gly
    1145                1150                1155
Ser Ser Gln Val Tyr Phe Met Thr Leu Gly Arg Thr Ser Leu Leu
    1160                1165                1170
Ser Trp
    1175

<210> 63
<211> 389

<212>  PRT
<213>  Homo sapiens
<220>
<221>  misc_feature
<223>  CDS de la séquence GS-N10: 618..1787

<220>
<221>  misc_feature
<223>  séquence appelée GS-P10: Vassosubstratine

<400>  63
Met Leu Leu Pro Pro Gly Ser Leu Ser Arg Pro Arg Thr Phe Ser Ser
1               5                   10                  15
Gln Pro Leu Gln Thr Lys Leu Met Thr His Asn Gly Leu Phe Arg Pro
            20                  25                  30
Ile Pro Tyr Val Thr Ala Ala Ser Ala Asp Glu Pro Thr Ala Ser Gln
        35                  40                  45
Gln Pro Pro Gln Ala Gln Leu His Arg Tyr Asn Gly Leu Phe Arg Pro
    50                  55                  60
Ser Ser Cys Leu Pro Ala Phe Ser Pro Gly Pro Glu Leu Ser Gln Val
65                  70                  75                  80
Asp Leu Thr Arg Pro Ser Ser Cys Phe Phe Ala Ala Ser Pro Gly Pro
                85                  90                  95
Ala Pro Ala Ser Trp Trp Pro Leu Gln Ala Gln Pro Leu Pro Pro Val
            100                 105                 110
Ser Leu Tyr Ser Pro Asn Val Cys Leu Thr Ala Asp Ser Ser Gly Pro
        115                 120                 125
Ala Ser Ala Ser Leu Trp Thr Pro Gln Ala Lys Leu Pro Thr Phe Gln
    130                 135                 140
Gln Leu Leu His Thr Gln Leu Leu Pro Pro Ser Gly Leu Phe Arg Pro
145                 150                 155                 160
Ser Ser Cys Phe Thr Arg Ala Phe Pro Gly Pro Thr Phe Val Ser Trp
                165                 170                 175
Gln Pro Ser Leu Ala Arg Phe Leu Pro Val Ser Gln Gln Pro Arg Gln
            180                 185                 190
Ala Gln Val Leu Pro His Thr Gly Leu Ser Thr Ser Ser Leu Cys Leu
        195                 200                 205
Thr Val Ala Ser Pro Arg Pro Thr Pro Val Pro Gly His His Leu Arg
    210                 215                 220
Ala Gln Asn Leu Leu Lys Ser Asp Ser Leu Val Pro Thr Ala Ala Ser
225                 230                 235                 240
Trp Trp Pro Met Lys Ala Gln Asn Leu Leu Asn Leu Thr Cys Pro Gly
                245                 250                 255
Pro Ala Pro Ala Ser Cys Gln Arg Leu Gln Ala Gln Pro Leu Pro His
            260                 265                 270
Gly Gly Phe Ser Arg Pro Thr Ser Ser Ser Trp Leu Gly Leu Gln Ala
        275                 280                 285
Gln Leu Leu Pro His Asn Ser Leu Phe Trp Pro Ser Ser Cys Pro Ala
    290                 295                 300
His Gly Gly Gln Cys Arg Pro Lys Thr Ser Ser Ser Gln Thr Leu Gln
305                 310                 315                 320
Ala His Leu Leu Leu Pro Gly Gly Ile Asn Arg Pro Ser Phe Asp Leu
                325                 330                 335
Arg Thr Ala Ser Ala Gly Pro Ala Leu Ala Ser Gln Gly Leu Phe Pro
            340                 345                 350
Gly Pro Ala Leu Ala Ser Trp Gln Leu Pro Gln Ala Lys Phe Leu Pro
        355                 360                 365
Ala Cys Gln Gln Pro Gln Gln Ala Gln Leu Leu Pro His Ser Gly Pro
    370                 375                 380
Phe Arg Pro Asn Ser
385


<210>  64
<211>  152
<212>  PRT
<213>  Homo sapiens
<220>
<221>  misc_feature
<223>  CDS de la séquence GS-N11: 439..897

<220>
<221>  misc_feature

<223>  séquence appelée GS-P11: Angiosignaline

<400>  64
Met Arg Leu Pro Leu Ser His Ser Pro Glu His Val Glu Met Ala Leu
1               5                   10                  15
Leu Ser Asn Ile Leu Ala Ala Tyr Ser Phe Val Ser Glu Asn Pro Glu
            20                  25                  30
Arg Ala Ala Leu Tyr Phe Val Ser Gly Val Cys Ile Gly Leu Val Leu
        35                  40                  45
Thr Leu Ala Ala Leu Val Ile Arg Ile Ser Cys His Thr Asp Cys Arg
    50                  55                  60
Arg Arg Pro Gly Lys Lys Phe Leu Gln Asp Arg Glu Ser Ser Ser Asp
65                  70                  75                  80
Ser Ser Asp Ser Glu Asp Gly Ser Glu Asp Thr Val Ser Asp Leu Ser
                85                  90                  95
Val Arg Arg His Arg Arg Phe Glu Arg Thr Leu Asn Lys Asn Val Phe
            100                 105                 110
Thr Ser Ala Glu Glu Leu Glu Arg Ala Gln Arg Leu Glu Glu Arg Glu
        115                 120                 125
Arg Ile Ile Arg Glu Ile Trp Met Asn Gly Gln Pro Glu Val Pro Gly
    130                 135                 140
Thr Arg Ser Leu Asn Arg Tyr Tyr
145                 150


<210>  65
<211>  793
<212>  PRT
<213>  Homo sapiens
<220>
<221>  misc_feature
<223>  CDS partielle de la séquence GS-N12:
<1..2384

<220>
<221>  misc_feature
<223>  séquence appelée GS-P12: Angiofoculine

<400>  65
Ser Arg Pro Ser Cys His Gly Arg Leu Pro Ala Arg Pro Pro Phe Tyr
1               5                   10                  15
Ala Pro Ala His Thr Leu Leu Pro Ser Phe Gln Arg Leu Ser Val Glu
            20                  25                  30
His Cys Gly Thr Gln Pro Glu Leu Pro Phe Ser Pro Arg Gly Glu Arg
        35                  40                  45
Cys Asp Pro Pro Ala Gly Ala Ala Gly Arg Gly Gly Gly Ala Arg Glu
    50                  55                  60
Lys Met Ala Thr Pro Gly Ser Glu Pro Gln Pro Phe Val Pro Ala Leu
65                  70                  75                  80
Ser Val Ala Thr Leu His Pro Leu His His Pro His His His His His
                85                  90                  95
His His Gln His His Gly Gly Thr Gly Ala Pro Gly Gly Ala Gly Gly
            100                 105                 110
Gly Gly Gly Gly Ser Gly Gly Phe Asn Leu Pro Leu Asn Arg Gly Leu
        115                 120                 125
Glu Arg Ala Leu Glu Glu Ala Ala Asn Ser Gly Gly Leu Asn Leu Ser
    130                 135                 140
Ala Arg Lys Leu Lys Glu Phe Pro Arg Thr Ala Ala Pro Gly His Asp
145                 150                 155                 160
Leu Ser Asp Thr Val Gln Ala Asp Leu Ser Lys Asn Arg Leu Val Glu
                165                 170                 175
Val Pro Met Glu Leu Cys His Phe Val Ser Leu Glu Ile Leu Asn Leu
            180                 185                 190
Tyr His Asn Cys Ile Arg Val Ile Pro Glu Ala Ile Val Asn Leu Gln
        195                 200                 205
Met Leu Thr Tyr Leu Asn Leu Ser Arg Asn Gln Leu Ser Ala Leu Pro
    210                 215                 220
Ala Cys Leu Cys Gly Leu Pro Leu Lys Val Leu Ile Ala Ser Asn Asn
225                 230                 235                 240
Lys Leu Gly Ser Leu Pro Glu Glu Ile Gly Gln Leu Lys Gln Leu Met
                245                 250                 255
Glu Leu Asp Val Ser Cys Asn Glu Ile Thr Ala Leu Pro Gln Gln Ile
            260                 265                 270

Gly Gln Leu Lys Ser Leu Arg Glu Leu Asn Val Arg Arg Asn Tyr Leu
        275                 280                 285
Lys Val Leu Pro Gln Glu Leu Val Asp Leu Pro Leu Val Lys Phe Asp
    290                 295                 300
Phe Ser Cys Asn Lys Val Leu Val Ile Pro Ile Cys Phe Arg Glu Met
305                 310                 315                 320
Lys Gln Leu Gln Val Leu Leu Leu Glu Asn Asn Pro Leu Gln Ser Pro
                325                 330                 335
Pro Ala Gln Ile Cys Thr Lys Gly Lys Val His Ile Phe Lys Tyr Leu
            340                 345                 350
Ser Ile Gln Ala Cys Gln Ile Lys Thr Ala Asp Ser Leu Tyr Leu His
        355                 360                 365
Thr Met Glu Arg Pro His Leu His Gln His Val Glu Asp Gly Lys Lys
    370                 375                 380
Asp Ser Asp Ser Gly Val Gly Ser Asp Asn Gly Asp Lys Arg Leu Ser
385                 390                 395                 400
Ala Thr Glu Pro Ser Asp Glu Asp Thr Val Ser Leu Asn Val Pro Met
                405                 410                 415
Ser Asn Ile Met Glu Glu Glu Gln Ile Ile Lys Glu Asp Ser Cys His
            420                 425                 430
Arg Leu Ser Pro Val Lys Gly Glu Phe His Gln Glu Phe Gln Pro Glu
        435                 440                 445
Pro Ser Leu Leu Gly Asp Ser Thr Asn Ser Gly Glu Glu Arg Asp Gln
    450                 455                 460
Phe Thr Asp Arg Ala Asp Gly Leu His Ser Glu Phe Met Asn Tyr Lys
465                 470                 475                 480
Ala Arg Ala Glu Asp Cys Glu Glu Leu Leu Arg Ile Glu Glu Asp Val
                485                 490                 495
His Trp Gln Thr Glu Gly Ile Ile Ser Ser Ser Lys Asp Gln Asp Met
            500                 505                 510
Asp Ile Ala Met Ile Glu Gln Leu Arg Glu Ala Val Asp Leu Leu Gln
        515                 520                 525
Asp Pro Asn Gly Leu Ser Thr Asp Ile Thr Glu Arg Ser Val Leu Asn
    530                 535                 540
Leu Tyr Pro Met Gly Ser Ala Glu Ala Leu Glu Leu Gln Asp Ser Ala
545                 550                 555                 560
Leu Asn Gly Gln Ile Gln Leu Glu Thr Ser Pro Val Cys Glu Val Gln
                565                 570                 575
Ser Asp Leu Thr Leu Gln Ser Asn Gly Ser Gln Tyr Ser Pro Asn Glu
            580                 585                 590
Ile Arg Glu Asn Ser Pro Ala Val Ser Pro Thr Thr Asn Ser Thr Ala
        595                 600                 605
Pro Phe Gly Leu Lys Pro Arg Ser Val Phe Leu Arg Pro Gln Arg Asn
    610                 615                 620
Leu Glu Ser Ile Asp Pro Gln Phe Thr Ile Arg Arg Lys Met Glu Gln
625                 630                 635                 640
Met Arg Glu Glu Lys Glu Leu Val Glu Gln Leu Arg Glu Ser Ile Glu
                645                 650                 655
Met Arg Leu Lys Val Ser Leu His Glu Asp Leu Gly Ala Ala Leu Met
            660                 665                 670
Asp Gly Val Val Leu Cys His Leu Val Asn His Ile Arg Pro Arg Ser
        675                 680                 685
Val Ala Ser Ile His Val Pro Ser Pro Ala Val Pro Lys Leu Ser Met
    690                 695                 700
Ala Lys Cys Arg Arg Asn Val Glu Asn Phe Leu Glu Ala Cys Arg Lys
705                 710                 715                 720
Leu Gly Val Pro Glu Ala Asp Leu Cys Ser Pro Cys Asp Ile Leu Gln
                725                 730                 735
Leu Asp Phe Arg His Ile Arg Lys Thr Val Asp Thr Leu Leu Ala Leu
            740                 745                 750
Gly Glu Lys Ala Pro Pro Pro Thr Ser Ala Leu Arg Ser Arg Asp Leu
        755                 760                 765
Ile Gly Phe Cys Leu Val His Ile Leu Phe Ile Val Leu Val Tyr Ile
    770                 775                 780
Thr Tyr His Trp Asn Ala Leu Ser Ala
785                 790

<210>  66
<211>  639
<212>  PRT
<213>  Homo sapiens
<220>

<221>  misc_feature
<223>  CDS de la séquence GS-N13: 426..2345

<220>
<221>  misc_feature
<223>  séquence appelée GS-P13: Angiohélicine

<400>  66
Met Asn Leu Leu Asn Thr Val Leu Pro Val Phe Pro Gly Gln Val Ser
1               5                   10                  15
Ser Ala Val His Thr Pro Gln Pro Ser Ile Pro Asn Pro Thr Val Ile
            20                  25                  30
Arg Thr Pro Ser Leu Pro Thr Ala Pro Val Thr Ser Ile His Ser Thr
        35                  40                  45
Thr Thr Thr Pro Val Pro Ser Ile Phe Ser Gly Leu Val Ser Leu Pro
    50                  55                  60
Gly Pro Ser Ala Thr Pro Thr Ala Ala Thr Pro Thr Pro Gly Pro Thr
65                  70                  75                  80
Pro Arg Ser Thr Leu Gly Ser Ser Glu Ala Phe Ala Ser Thr Ser Ala
                85                  90                  95
Pro Phe Thr Ser Leu Pro Phe Ser Thr Ser Ser Ser Ala Ala Ser Thr
            100                 105                 110
Ser Asn Pro Asn Ser Ala Ser Leu Ser Ser Val Phe Ala Gly Leu Pro
        115                 120                 125
Leu Pro Leu Pro Pro Thr Ser Gln Gly Leu Ser Asn Pro Thr Pro Val
    130                 135                 140
Ile Ala Gly Gly Ser Thr Pro Ser Val Ala Gly Pro Leu Gly Val Asn
145                 150                 155                 160
Ser Pro Leu Leu Ser Ala Leu Lys Gly Phe Leu Thr Ser Asn Asp Thr
                165                 170                 175
Asn Leu Ile Asn Ser Ser Ala Leu Ser Ser Ala Val Thr Ser Gly Leu
            180                 185                 190
Ala Ser Leu Ser Ser Leu Thr Leu Gln Asn Ser Asp Ser Ser Ala Ser
        195                 200                 205
Ala Pro Asn Lys Cys Tyr Ala Pro Ser Ala Ile Pro Thr Pro Gln Arg
    210                 215                 220
Thr Ser Thr Pro Gly Leu Ala Leu Phe Pro Gly Leu Pro Ser Pro Val
225                 230                 235                 240
Ala Asn Ser Thr Ser Thr Pro Leu Thr Leu Pro Val Gln Ser Pro Leu
                245                 250                 255
Ala Thr Ala Ala Ser Ala Ser Thr Ser Val Pro Val Ser Cys Gly Ser
            260                 265                 270
Ser Ala Ser Leu Leu Arg Gly Pro His Pro Gly Thr Ser Asp Leu His
        275                 280                 285
Ile Ser Ser Thr Pro Ala Ala Thr Thr Leu Pro Val Met Ile Lys Thr
    290                 295                 300
Glu Pro Thr Ser Pro Thr Pro Ser Ala Phe Lys Gly Pro Ser His Ser
305                 310                 315                 320
Gly Asn Pro Ser His Gly Thr Leu Gly Leu Ser Gly Thr Leu Gly Arg
                325                 330                 335
Ala Tyr Thr Ser Thr Ser Val Pro Ile Ser Leu Ser Ala Cys Leu Asn
            340                 345                 350
Pro Ala Leu Ser Gly Leu Ser Ser Leu Ser Thr Pro Leu Asn Gly Ser
        355                 360                 365
Asn Pro Leu Ser Ser Ile Ser Leu Pro Pro His Gly Ser Ser Thr Pro
    370                 375                 380
Ile Ala Pro Val Phe Thr Ala Leu Pro Ser Phe Thr Ser Leu Thr Asn
385                 390                 395                 400
Asn Phe Pro Leu Thr Gly Asn Pro Ser Leu Asn Pro Ser Val Ser Leu
                405                 410                 415
Pro Gly Ser Leu Ile Ala Thr Ser Ser Thr Ala Ala Thr Ser Thr Ser
            420                 425                 430
Leu Pro His Pro Ser Ser Thr Ala Ala Val Leu Ser Gly Leu Ser Ala
        435                 440                 445
Ser Ala Pro Val Ser Ala Ala Pro Phe Pro Leu Asn Leu Ser Thr Ala
    450                 455                 460
Val Pro Ser Leu Phe Ser Val Thr Gln Gly Pro Leu Ser Ser Ser Asn
465                 470                 475                 480
Pro Ser Tyr Pro Gly Phe Ser Val Ser Asn Thr Pro Ser Val Thr Pro
                485                 490                 495
Ala Leu Pro Ser Phe Pro Gly Leu Gln Ala Pro Ser Thr Val Ala Ala
            500                 505                 510
Val Thr Pro Leu Pro Val Ala Ala Thr Ala Pro Ser Pro Ala Pro Val

515                 520                 525
Leu Pro Gly Phe Ala Ser Ala Phe Ser Ser Asn Phe Asn Ser Ala Leu
    530                 535                 540
Val Ala Gln Ala Gly Leu Ser Ser Gly Leu Gln Ala Ala Gly Ser Ser
545                 550                 555                 560
Val Phe Pro Gly Leu Leu Ser Leu Pro Gly Ile Pro Gly Phe Pro Gln
                565                 570                 575
Asn Pro Ser Gln Ser Ser Leu Gln Glu Leu Gln His Asn Ala Ala Ala
            580                 585                 590
Gln Ser Ala Leu Leu Gln Gln Val His Ser Ala Ser Ala Leu Glu Ser
        595                 600                 605
Tyr Pro Ala Gln Pro Asp Gly Phe Pro Ser Tyr Pro Ser Ala Pro Gly
    610                 615                 620
Thr Pro Phe Ser Leu Gln Pro Ser Leu Ser Gln Ser Gly Trp Gln
625                 630                 635


<210>  67
<211>  370
<212>  PRT
<213>  Homo sapiens
<220>
<221>  misc_feature
<223>  CDS de la séquence GS-N15: 228..1340

<220>
<221>  misc_feature
<223>  séquence appelée GS-P15: Angioacyline

<400>  67
Met Ala Ile Thr Leu Glu Glu Ala Pro Trp Leu Gly Trp Leu Leu Val
1               5                   10                  15
Lys Ala Leu Met Arg Phe Ala Phe Met Val Val Asn Asn Leu Val Ala
            20                  25                  30
Ile Pro Ser Tyr Ile Cys Tyr Val Ile Ile Leu Gln Pro Leu Arg Val
        35                  40                  45
Leu Asp Ser Lys Arg Phe Trp Tyr Ile Glu Gly Ile Met Tyr Lys Trp
    50                  55                  60
Leu Leu Gly Met Val Ala Ser Trp Gly Trp Tyr Ala Gly Tyr Thr Val
65                  70                  75                  80
Met Glu Trp Gly Glu Asp Ile Lys Ala Val Ser Lys Asp Glu Ala Val
                85                  90                  95
Met Leu Val Asn His Gln Ala Thr Gly Asp Val Cys Thr Leu Met Met
            100                 105                 110
Cys Leu Gln Asp Lys Gly Leu Val Val Ala Gln Met Met Trp Leu Met
        115                 120                 125
Asp His Ile Phe Lys Tyr Thr Asn Phe Gly Ile Val Ser Leu Val His
    130                 135                 140
Gly Asp Phe Phe Ile Arg Gln Gly Arg Ser Tyr Arg Asp Gln Gln Leu
145                 150                 155                 160
Leu Leu Leu Lys Lys His Leu Glu Asn Asn Tyr Arg Ser Arg Asp Arg
                165                 170                 175
Lys Trp Ile Val Leu Phe Pro Glu Gly Gly Phe Leu Arg Lys Arg Arg
            180                 185                 190
Glu Thr Ser Gln Ala Phe Ala Lys Lys Asn Asn Leu Pro Phe Leu Thr
        195                 200                 205
Asn Val Thr Leu Pro Arg Ser Gly Ala Thr Lys Ile Ile Leu Asn Ala
    210                 215                 220
Leu Val Ala Gln Gln Lys Asn Gly Ser Pro Ala Gly Gly Asp Ala Lys
225                 230                 235                 240
Glu Leu Asp Ser Lys Ser Lys Gly Leu Gln Trp Ile Ile Asp Thr Thr
                245                 250                 255
Ile Ala Tyr Pro Lys Ala Glu Pro Ile Asp Ile Gln Thr Trp Ile Leu
            260                 265                 270
Gly Tyr Arg Lys Pro Thr Val Thr His Val His Tyr Arg Ile Phe Pro
        275                 280                 285
Ile Lys Asp Val Pro Leu Glu Thr Asp Asp Leu Thr Thr Trp Leu Tyr
    290                 295                 300
Gln Arg Phe Val Glu Lys Glu Asp Leu Leu Ser His Phe Tyr Glu Thr
305                 310                 315                 320
Gly Ala Phe Pro Pro Ser Lys Gly His Lys Glu Ala Val Ser Arg Glu
                325                 330                 335
Met Thr Leu Ser Asn Leu Trp Ile Phe Leu Ile Gln Ser Phe Ala Phe

340                 345                 350
Leu Ser Gly Tyr Met Trp Tyr Asn Ile Ile Gln Tyr Phe Tyr His Cys
        355                 360                 365
Leu Phe
    370


<210>  68
<211>  301
<212>  PRT
<213>  Homo sapiens
<220>
<221>  misc_feature
<223>  CDS de la séquence GS-N16: 167...1072 GS-P16

<220>
<221>  misc_feature
<223>  nom: protéine homologue à la phosducine (PDCL)

<400>  68
Met Thr Thr Leu Asp Asp Lys Leu Leu Gly Glu Lys Leu Gln Tyr Tyr
1               5                   10                  15
Tyr Ser Ser Ser Glu Asp Glu Asp Ser Asp His Glu Asp Lys Asp Arg
            20                  25                  30
Gly Arg Cys Ala Pro Ala Ser Ser Ser Val Pro Ala Glu Ala Glu Leu
        35                  40                  45
Ala Gly Glu Gly Ile Ser Val Asn Thr Gly Pro Lys Gly Val Ile Asn
    50                  55                  60
Asp Trp Arg Arg Phe Lys Gln Leu Glu Thr Glu Gln Arg Glu Glu Gln
65                  70                  75                  80
Cys Arg Glu Met Glu Arg Leu Ile Lys Lys Leu Ser Met Thr Cys Arg
                85                  90                  95
Ser His Leu Asp Glu Glu Glu Glu Gln Gln Lys Gln Lys Asp Leu Gln
            100                 105                 110
Glu Lys Ile Ser Gly Lys Met Thr Leu Lys Glu Phe Ala Ile Met Asn
        115                 120                 125
Glu Asp Gln Asp Asp Glu Glu Phe Leu Gln Gln Tyr Arg Lys Gln Arg
    130                 135                 140
Met Glu Glu Met Arg Gln Gln Leu His Lys Gly Pro Gln Phe Lys Gln
145                 150                 155                 160
Val Phe Glu Ile Ser Ser Gly Glu Gly Phe Leu Asp Met Ile Asp Lys
                165                 170                 175
Glu Gln Lys Ser Ile Val Ile Met Val His Ile Tyr Glu Asp Gly Ile
            180                 185                 190
Pro Gly Thr Glu Ala Met Asn Gly Cys Met Ile Cys Leu Ala Ala Glu
        195                 200                 205
Tyr Pro Ala Val Lys Phe Cys Lys Val Lys Ser Ser Val Ile Gly Ala
    210                 215                 220
Ser Ser Gln Phe Thr Arg Asn Ala Leu Pro Ala Leu Leu Ile Tyr Lys
225                 230                 235                 240
Gly Gly Glu Leu Ile Gly Asn Phe Val Arg Val Thr Asp Gln Leu Gly
                245                 250                 255
Asp Asp Phe Phe Ala Val Asp Leu Glu Ala Phe Leu Gln Glu Phe Gly
            260                 265                 270
Leu Leu Pro Glu Lys Glu Val Leu Val Leu Thr Ser Val Arg Asn Ser
        275                 280                 285
Ala Thr Cys His Ser Glu Asp Ser Asp Leu Glu Ile Asp
    290                 295                 300


<210>  69
<211>  403
<212>  PRT
<213>  Homo sapiens
<220>
<221>  misc_feature
<223>  CDS de la séquence GS-N17:1030...2241 (GS-P17)

<220>
<221>  misc_feature
<223>  nom: Protéine similaire à la protéine ribosomale L3 (RPL3)

<400>  69

Met Ser His Arg Lys Phe Ser Ala Pro Arg His Gly Ser Leu Gly Phe
1               5                   10                  15
Leu Pro Arg Lys Arg Ser Ser Arg His Arg Gly Lys Val Lys Ser Phe
            20                  25                  30
Pro Lys Asp Asp Pro Ser Lys Pro Val His Leu Thr Ala Phe Leu Gly
        35                  40                  45
Tyr Lys Ala Gly Met Thr His Ile Val Arg Glu Val Asp Arg Pro Gly
    50                  55                  60
Ser Lys Val Asn Lys Lys Glu Val Val Glu Ala Val Thr Ile Val Glu
65                  70                  75                  80
Thr Pro Pro Met Val Val Val Gly Ile Val Gly Tyr Val Glu Thr Pro
                85                  90                  95
Arg Gly Leu Arg Thr Phe Lys Thr Val Phe Ala Glu His Ile Ser Asp
            100                 105                 110
Glu Cys Lys Arg Arg Phe Tyr Lys Asn Trp His Lys Ser Lys Lys Lys
        115                 120                 125
Ala Phe Thr Lys Tyr Cys Lys Lys Trp Gln Asp Glu Asp Gly Lys Lys
    130                 135                 140
Gln Leu Glu Lys Asp Phe Ser Ser Met Lys Lys Tyr Cys Gln Val Ile
145                 150                 155                 160
Arg Val Ile Ala His Thr Gln Met Arg Leu Leu Pro Leu Arg Gln Lys
                165                 170                 175
Lys Ala His Leu Met Glu Ile Gln Val Asn Gly Gly Thr Val Ala Glu
            180                 185                 190
Lys Leu Asp Trp Ala Arg Glu Arg Leu Glu Gln Gln Val Pro Val Asn
        195                 200                 205
Gln Val Phe Gly Gln Asp Glu Met Ile Asp Val Ile Gly Val Thr Lys
    210                 215                 220
Gly Lys Gly Tyr Lys Gly Val Thr Ser Arg Trp His Thr Lys Lys Leu
225                 230                 235                 240
Pro Arg Lys Thr His Arg Gly Leu Arg Lys Val Ala Cys Ile Gly Ala
                245                 250                 255
Trp His Pro Ala Arg Val Ala Phe Ser Val Ala Arg Ala Gly Gln Lys
            260                 265                 270
Gly Tyr His His Arg Thr Glu Ile Asn Lys Lys Ile Tyr Lys Ile Gly
        275                 280                 285
Gln Gly Tyr Leu Ile Lys Asp Gly Lys Leu Ile Lys Asn Asn Ala Ser
    290                 295                 300
Thr Asp Tyr Asp Leu Ser Asp Lys Ser Ile Asn Pro Leu Gly Gly Phe
305                 310                 315                 320
Val His Tyr Gly Glu Val Thr Asn Asp Phe Val Met Leu Lys Gly Cys
                325                 330                 335
Val Val Gly Thr Lys Lys Arg Val Leu Thr Leu Arg Lys Ser Leu Leu
            340                 345                 350
Val Gln Thr Lys Arg Arg Ala Leu Glu Lys Ile Asp Leu Lys Phe Ile
        355                 360                 365
Asp Thr Thr Ser Lys Phe Gly His Gly Arg Phe Gln Thr Met Glu Glu
    370                 375                 380
Lys Lys Ala Phe Met Gly Pro Leu Lys Lys Asp Arg Ile Ala Lys Glu
385                 390                 395                 400
Glu Gly Ala

<210>  70
<211>  975
<212>  PRT
<213>  Homo sapiens
<220>
<221>  misc_feature
<223>  CDS de la séquence GS-N18: 89...3016 ( GS-P18)

<220>
<221>  misc_feature
<223>  nom: Protéine similaire à la protéine 20 Ring Finger (RNF20)

<400>  70
Met Ser Gly Ile Gly Asn Lys Arg Ala Ala Gly Glu Pro Gly Thr Ser
1               5                   10                  15
Met Pro Pro Glu Lys Lys Ala Ala Val Glu Asp Ser Gly Thr Thr Val
            20                  25                  30
Glu Thr Ile Lys Leu Gly Gly Val Ser Ser Thr Glu Glu Leu Asp Ile
        35                  40                  45

Arg Thr Leu Gln Thr Lys Asn Arg Lys Leu Ala Glu Met Leu Asp Gln
    50                  55                  60
Arg Gln Ala Ile Glu Asp Glu Leu Arg Glu His Ile Glu Lys Leu Glu
65                  70                  75                  80
Arg Arg Gln Ala Thr Asp Asp Ala Ser Leu Leu Ile Val Asn Arg Tyr
                85                  90                  95
Trp Ser Gln Phe Asp Glu Asn Ile Arg Ile Ile Leu Lys Arg Tyr Asp
            100                 105                 110
Leu Glu Gln Gly Leu Gly Asp Leu Leu Thr Glu Arg Lys Ala Leu Val
        115                 120                 125
Val Pro Glu Pro Glu Pro Asp Ser Asp Ser Asn Gln Glu Arg Lys Asp
    130                 135                 140
Asp Arg Glu Arg Gly Glu Gly Gln Glu Pro Ala Phe Ser Phe Leu Ala
145                 150                 155                 160
Thr Leu Ala Ser Ser Ser Ser Glu Glu Met Glu Ser Gln Leu Gln Glu
                165                 170                 175
Arg Val Glu Ser Ser Arg Arg Ala Val Ser Gln Ile Val Thr Val Tyr
            180                 185                 190
Asp Lys Leu Gln Glu Lys Val Glu Leu Leu Ser Arg Lys Leu Asn Ser
        195                 200                 205
Gly Asp Asn Leu Ile Val Glu Glu Ala Val Gln Glu Leu Asn Ser Phe
    210                 215                 220
Leu Ala Gln Glu Asn Met Arg Leu Gln Glu Leu Thr Asp Leu Leu Gln
225                 230                 235                 240
Glu Lys His Arg Thr Met Ser Gln Glu Phe Ser Lys Leu Gln Ser Lys
                245                 250                 255
Val Glu Thr Ala Glu Ser Arg Val Ser Val Leu Glu Ser Met Ile Asp
            260                 265                 270
Asp Leu Gln Trp Asp Ile Asp Lys Ile Arg Lys Arg Glu Gln Arg Leu
        275                 280                 285
Asn Arg His Leu Ala Glu Val Leu Glu Arg Val Asn Ser Lys Gly Tyr
    290                 295                 300
Lys Val Tyr Gly Ala Gly Ser Ser Leu Tyr Gly Gly Thr Ile Thr Ile
305                 310                 315                 320
Asn Ala Arg Lys Phe Glu Glu Met Asn Ala Glu Leu Glu Glu Asn Lys
                325                 330                 335
Glu Leu Ala Gln Asn Arg Leu Cys Glu Leu Glu Lys Leu Arg Gln Asp
            340                 345                 350
Phe Glu Glu Val Thr Thr Gln Asn Glu Lys Leu Lys Val Glu Leu Arg
        355                 360                 365
Ser Ala Val Glu Gln Val Val Lys Glu Thr Pro Glu Tyr Arg Cys Met
    370                 375                 380
Gln Ser Gln Phe Ser Val Leu Tyr Asn Glu Ser Leu Gln Leu Lys Ala
385                 390                 395                 400
His Leu Asp Glu Ala Arg Thr Leu Leu His Gly Thr Arg Gly Thr His
                405                 410                 415
Gln His Gln Val Glu Leu Ile Glu Arg Asp Glu Val Ser Leu His Lys
            420                 425                 430
Lys Leu Arg Thr Glu Val Ile Gln Leu Glu Asp Thr Leu Ala Gln Val
        435                 440                 445
Arg Lys Glu Tyr Glu Met Leu Arg Ile Glu Phe Glu Gln Thr Leu Ala
    450                 455                 460
Ala Asn Glu Gln Ala Gly Pro Ile Asn Arg Glu Met Arg His Leu Ile
465                 470                 475                 480
Ser Ser Leu Gln Asn His Asn His Gln Leu Lys Gly Glu Val Leu Arg
                485                 490                 495
Tyr Lys Arg Lys Leu Arg Glu Ala Gln Ser Asp Leu Asn Lys Thr Arg
            500                 505                 510
Leu Arg Ser Gly Ser Ala Leu Leu Gln Ser Gln Ser Ser Thr Glu Asp
        515                 520                 525
Pro Lys Asp Glu Pro Ala Glu Leu Lys Pro Asp Ser Glu Asp Leu Ser
    530                 535                 540
Ser Gln Ser Ser Ala Ser Lys Ala Ser Gln Glu Asp Ala Asn Glu Ile
545                 550                 555                 560
Lys Ser Lys Arg Asp Glu Glu Glu Arg Glu Arg Glu Arg Arg Glu Lys
                565                 570                 575
Glu Arg Glu Arg Glu Arg Glu Arg Glu Lys Glu Lys Glu Arg Glu Arg
            580                 585                 590
Glu Lys Gln Lys Leu Lys Glu Ser Glu Lys Glu Arg Asp Ser Ala Lys
        595                 600                 605
Asp Lys Glu Lys Gly Lys His Asp Asp Gly Arg Lys Lys Glu Ala Glu
    610                 615                 620
Ile Ile Lys Gln Leu Lys Ile Glu Leu Lys Lys Ala Gln Glu Ser Gln

625                 630                 635                 640
Lys Glu Met Lys Leu Leu Leu Asp Met Tyr Arg Ser Ala Pro Lys Glu
                645                 650                 655
Gln Arg Asp Lys Val Gln Leu Met Ala Ala Glu Lys Lys Ser Lys Ala
            660                 665                 670
Glu Leu Glu Asp Leu Arg Gln Arg Leu Lys Asp Leu Glu Asp Lys Glu
        675                 680                 685
Lys Lys Glu Asn Lys Lys Met Ala Asp Glu Asp Ala Leu Arg Lys Ile
    690                 695                 700
Arg Ala Val Glu Glu Gln Ile Glu Tyr Leu Gln Lys Lys Leu Ala Met
705                 710                 715                 720
Ala Lys Gln Glu Glu Glu Ala Leu Leu Ser Glu Met Asp Val Thr Gly
                725                 730                 735
Gln Ala Phe Glu Asp Met Gln Glu Gln Asn Ile Arg Leu Met Gln Gln
            740                 745                 750
Leu Arg Glu Lys Asp Asp Ala Asn Phe Lys Leu Met Ser Glu Arg Ile
        755                 760                 765
Lys Ser Asn Gln Ile His Lys Leu Leu Lys Glu Glu Lys Glu Glu Leu
    770                 775                 780
Ala Asp Gln Val Leu Thr Leu Lys Thr Gln Val Asp Ala Gln Leu Gln
785                 790                 795                 800
Val Val Arg Lys Leu Glu Glu Lys Glu His Leu Leu Gln Ser Asn Ile
                805                 810                 815
Gly Thr Gly Glu Lys Glu Leu Gly Leu Arg Thr Gln Ala Leu Glu Met
            820                 825                 830
Asn Lys Arg Lys Ala Met Glu Ala Ala Gln Leu Ala Asp Asp Leu Lys
        835                 840                 845
Ala Gln Leu Glu Leu Ala Gln Lys Lys Leu His Asp Phe Gln Asp Glu
    850                 855                 860
Ile Val Glu Asn Ser Val Thr Lys Glu Lys Asp Met Phe Asn Phe Lys
865                 870                 875                 880
Arg Ala Gln Glu Asp Ile Ser Arg Leu Arg Arg Lys Leu Glu Thr Thr
                885                 890                 895
Lys Lys Pro Asp Asn Val Pro Lys Cys Asp Glu Ile Leu Met Glu Glu
            900                 905                 910
Ile Lys Asp Tyr Lys Ala Arg Leu Thr Cys Pro Cys Cys Asn Met Arg
        915                 920                 925
Lys Lys Asp Ala Val Leu Thr Lys Cys Phe His Val Phe Cys Phe Glu
    930                 935                 940
Cys Val Lys Thr Arg Tyr Asp Thr Arg Gln Arg Lys Cys Pro Lys Cys
945                 950                 955                 960
Asn Ala Ala Phe Gly Ala Asn Asp Phe His Arg Ile Tyr Ile Gly
                965                 970                 975


<210>  71
<211>  494
<212>  PRT
<213>  Homo sapiens
<220>
<221>  misc_feature
<223>  CDS de la séquence GS-N19:107...1591( GS-P19)

<220>
<221>  misc_feature
<223>  nom: Protéine similaire au facteur d'épissage, arginine/serine-ri
       ch 4

<400>  71
Met Pro Arg Val Tyr Ile Gly Arg Leu Ser Tyr Gln Ala Arg Glu Arg
1               5                   10                  15
Asp Val Glu Arg Phe Phe Lys Gly Tyr Gly Lys Ile Leu Glu Val Asp
            20                  25                  30
Leu Lys Asn Gly Tyr Gly Phe Val Glu Phe Asp Asp Leu Arg Asp Ala
        35                  40                  45
Asp Asp Ala Val Tyr Glu Leu Asn Gly Lys Asp Leu Cys Gly Glu Arg
    50                  55                  60
Val Ile Val Glu His Ala Arg Gly Pro Arg Arg Asp Gly Ser Tyr Gly
65                  70                  75                  80
Ser Gly Arg Ser Gly Tyr Gly Tyr Arg Arg Ser Gly Arg Asp Lys Tyr
                85                  90                  95
Gly Pro Pro Thr Arg Thr Glu Tyr Arg Leu Ile Val Glu Asn Leu Ser
            100                 105                 110

Ser Arg Cys Ser Trp Gln Asp Leu Lys Asp Tyr Met Arg Gln Ala Gly
        115                 120                 125
Glu Val Thr Tyr Ala Asp Ala His Lys Gly Arg Lys Asn Glu Gly Val
    130                 135                 140
Ile Glu Phe Val Ser Tyr Ser Asp Met Lys Arg Ala Leu Glu Lys Leu
145                 150                 155                 160
Asp Gly Thr Glu Val Asn Gly Arg Lys Ile Arg Leu Val Glu Asp Lys
                165                 170                 175
Pro Gly Ser Arg Arg Arg Arg Ser Tyr Ser Arg Ser Arg Ser His Ser
            180                 185                 190
Arg Ser Arg Ser Arg Ser Arg His Ser Arg Lys Ser Arg Ser Arg Ser
        195                 200                 205
Gly Ser Ser Lys Ser Ser His Ser Lys Ser Arg Ser Arg Ser Arg Ser
    210                 215                 220
Gly Ser Arg Ser Arg Ser Lys Ser Arg Ser Arg Ser Gln Ser Arg Ser
225                 230                 235                 240
Arg Ser Lys Lys Glu Lys Ser Arg Ser Pro Ser Lys Glu Lys Ser Arg
                245                 250                 255
Ser Arg Ser His Ser Ala Gly Lys Ser Arg Ser Lys Ser Lys Asp Gln
            260                 265                 270
Ala Glu Glu Lys Ile Gln Asn Asn Asp Asn Val Gly Lys Pro Lys Ser
        275                 280                 285
Arg Ser Pro Ser Arg His Lys Ser Lys Ser Lys Ser Arg Ser Arg Ser
    290                 295                 300
Gln Glu Arg Arg Val Glu Glu Glu Lys Arg Gly Ser Val Ser Arg Gly
305                 310                 315                 320
Arg Ser Gln Glu Lys Ser Leu Arg Gln Ser Arg Ser Arg Ser Arg Ser
                325                 330                 335
Lys Gly Gly Ser Arg Ser Arg Ser Arg Ser Arg Ser Lys Ser Lys Asp
            340                 345                 350
Lys Arg Lys Gly Arg Lys Arg Ser Arg Glu Glu Ser Arg Ser Arg Ser
        355                 360                 365
Arg Ser Arg Ser Lys Ser Glu Arg Ser Arg Lys Arg Gly Ser Lys Arg
    370                 375                 380
Asp Ser Lys Ala Gly Ser Ser Lys Lys Lys Lys Lys Glu Asp Thr Asp
385                 390                 395                 400
Arg Ser Gln Ser Arg Ser Pro Ser Arg Ser Val Ser Lys Glu Arg Glu
                405                 410                 415
His Ala Lys Ser Glu Ser Ser Gln Arg Glu Gly Arg Gly Glu Ser Glu
            420                 425                 430
Asn Ala Gly Thr Asn Gln Glu Thr Arg Ser Arg Ser Arg Ser Asn Ser
        435                 440                 445
Lys Ser Lys Pro Asn Leu Pro Ser Glu Ser Arg Ser Arg Ser Lys Ser
    450                 455                 460
Ala Ser Lys Thr Arg Ser Arg Ser Lys Ser Arg Ser Arg Ser Ala Ser
465                 470                 475                 480
Arg Ser Pro Ser Arg Ser Arg Ser Arg Ser His Ser Arg Ser
                485                 490


<210>  72
<211>  1377
<212>  PRT
<213>  Homo sapiens
<220>
<221>  misc_feature
<223>  CDS de la séquence GS-N20: 36...4169 ( GS-P20) (CPD)

<220>
<221>  misc_feature
<223>  nom:Carboxypeptidase D

<400>  72
Met Ala Ser Gly Arg Asp Glu Arg Pro His Cys Val Gly Arg Leu Leu
1               5                   10                  15
Leu Leu Met Cys Leu Leu Leu Leu Gly Ser Ser Ala Arg Ala Ala His
            20                  25                  30
Ile Lys Lys Ala Glu Ala Thr Thr Thr Thr Thr Ser Ala Gly Ala Arg
        35                  40                  45
Gly Arg Gly Gln Phe Asp Arg Tyr Tyr His Glu Glu Glu Leu Glu Ser
    50                  55                  60
Ala Leu Arg Glu Ala Ala Ala Ala Gly Leu Pro Gly Leu Ala Arg Leu
65                  70                  75                  80

Phe Ser Ile Gly Arg Ser Val Glu Gly Arg Pro Leu Trp Val Leu Arg
                85                  90                  95
Leu Thr Ala Gly Leu Gly Ser Leu Ile Pro Glu Gly Asp Ala Gly Pro
            100                 105                 110
Asp Ala Ala Gly Pro Asp Ala Ala Gly Pro Leu Leu Pro Gly Arg Pro
        115                 120                 125
Gln Val Lys Leu Val Gly Asn Met His Gly Asp Glu Thr Val Ser Arg
    130                 135                 140
Gln Val Leu Ile Tyr Leu Ala Arg Glu Leu Ala Ala Leu Pro Pro Gly
145                 150                 155                 160
Asp Pro Arg Leu Val Arg Leu Leu Asn Thr Thr Asp Val Tyr Leu Leu
                165                 170                 175
Pro Ser Leu Asn Pro Asp Gly Phe Glu Arg Ala Arg Glu Gly Asp Cys
            180                 185                 190
Gly Phe Gly Asp Gly Gly Pro Ser Gly Ala Ser Gly Arg Asp Asn Ser
        195                 200                 205
Arg Gly Arg Asp Leu Asn Arg Ser Phe Pro Asp Gln Phe Ser Thr Gly
    210                 215                 220
Glu Pro Pro Ala Leu Asp Glu Val Pro Glu Val Arg Ala Leu Ile Glu
225                 230                 235                 240
Trp Ile Arg Arg Asn Lys Phe Val Leu Ser Gly Asn Leu His Gly Gly
                245                 250                 255
Ser Val Val Ala Ser Tyr Pro Phe Asp Asp Ser Pro Glu His Lys Ala
            260                 265                 270
Thr Gly Ile Tyr Ser Lys Thr Ser Asp Asp Glu Val Phe Lys Tyr Leu
        275                 280                 285
Ala Lys Ala Tyr Ala Ser Asn His Pro Ile Met Lys Thr Gly Glu Pro
    290                 295                 300
His Cys Pro Gly Asp Glu Asp Glu Thr Phe Lys Asp Gly Ile Thr Asn
305                 310                 315                 320
Gly Ala His Trp Tyr Asp Val Glu Gly Gly Met Gln Asp Tyr Asn Tyr
                325                 330                 335
Val Trp Ala Asn Cys Phe Glu Ile Thr Leu Glu Leu Ser Cys Cys Lys
            340                 345                 350
Tyr Pro Pro Ala Ser Gln Leu Arg Gln Glu Trp Glu Asn Asn Arg Glu
        355                 360                 365
Ser Leu Ile Thr Leu Ile Glu Lys Val His Ile Gly Val Lys Gly Phe
    370                 375                 380
Val Lys Asp Ser Ile Thr Gly Ser Gly Leu Glu Asn Ala Thr Ile Ser
385                 390                 395                 400
Val Ala Gly Ile Asn His Asn Ile Thr Thr Gly Arg Phe Gly Asp Phe
                405                 410                 415
Tyr Arg Leu Leu Val Pro Gly Thr Tyr Asn Leu Thr Val Val Leu Thr
            420                 425                 430
Gly Tyr Met Pro Leu Thr Val Thr Asn Val Val Val Lys Glu Gly Pro
        435                 440                 445
Ala Thr Glu Val Asp Phe Ser Leu Arg Pro Thr Val Thr Ser Val Ile
    450                 455                 460
Pro Asp Thr Thr Glu Ala Val Ser Thr Ala Ser Thr Val Ala Ile Pro
465                 470                 475                 480
Asn Ile Leu Ser Gly Thr Ser Ser Ser Cys Gln Pro Ile Gln Pro Lys
                485                 490                 495
Asp Phe His His His His Phe Pro Asp Met Glu Ile Phe Leu Arg Arg
            500                 505                 510
Phe Ala Asn Glu Tyr Pro Asn Ile Thr Arg Leu Tyr Ser Leu Gly Lys
        515                 520                 525
Ser Val Glu Ser Arg Glu Leu Tyr Val Met Glu Ile Ser Asp Asn Pro
    530                 535                 540
Gly Val His Glu Pro Gly Glu Pro Glu Phe Lys Tyr Ile Gly Asn Met
545                 550                 555                 560
His Gly Asn Glu Val Val Gly Arg Glu Leu Leu Leu Asn Leu Ile Glu
                565                 570                 575
Tyr Leu Cys Lys Asn Phe Gly Thr Asp Pro Glu Val Thr Asp Leu Val
            580                 585                 590
His Asn Thr Arg Ile His Leu Met Pro Ser Met Asn Pro Asp Gly Tyr
        595                 600                 605
Glu Lys Ser Gln Glu Gly Asp Ser Ile Ser Val Ile Gly Arg Asn Asn
    610                 615                 620
Ser Asn Asn Phe Asp Leu Asn Arg Asn Phe Pro Asp Gln Phe Val Gln
625                 630                 635                 640
Ile Thr Asp Pro Thr Gln Pro Glu Thr Ile Ala Val Met Ser Trp Met
                645                 650                 655
Lys Ser Tyr Pro Phe Val Leu Ser Ala Asn Leu His Gly Gly Ser Leu

            660                 665                 670
Val Val Asn Tyr Pro Phe Asp Asp Asp Glu Gln Gly Leu Ala Thr Tyr
        675                 680                 685
Ser Lys Ser Pro Asp Asp Ala Val Phe Gln Gln Ile Ala Leu Ser Tyr
    690                 695                 700
Ser Lys Glu Asn Ser Gln Met Phe Gln Gly Arg Pro Cys Lys Asn Met
705                 710                 715                 720
Tyr Pro Asn Glu Tyr Phe Pro His Gly Ile Thr Asn Gly Ala Ser Trp
                725                 730                 735
Tyr Asn Val Pro Gly Gly Met Gln Asp Trp Asn Tyr Leu Gln Thr Asn
            740                 745                 750
Cys Phe Glu Val Thr Ile Glu Leu Gly Cys Val Lys Tyr Pro Leu Glu
        755                 760                 765
Lys Glu Leu Pro Asn Phe Trp Glu Gln Asn Arg Arg Ser Leu Ile Gln
    770                 775                 780
Phe Met Lys Gln Val His Gln Gly Val Arg Gly Phe Val Leu Asp Ala
785                 790                 795                 800
Thr Asp Gly Arg Gly Ile Leu Asn Ala Thr Ile Ser Val Ala Glu Ile
                805                 810                 815
Asn His Pro Val Thr Thr Tyr Lys Thr Gly Asp Tyr Trp Arg Leu Leu
            820                 825                 830
Val Pro Gly Thr Tyr Lys Ile Thr Ala Ser Ala Arg Gly Tyr Asn Pro
        835                 840                 845
Val Thr Lys Asn Val Thr Val Lys Ser Glu Gly Ala Ile Gln Val Asn
    850                 855                 860
Phe Thr Leu Val Arg Ser Ser Thr Asp Ser Asn Asn Glu Ser Lys Lys
865                 870                 875                 880
Gly Lys Gly Ala Ser Ser Ser Thr Asn Asp Ala Ser Asp Pro Thr Thr
                885                 890                 895
Lys Glu Phe Glu Thr Leu Ile Lys Asp Leu Ser Ala Glu Asn Gly Leu
            900                 905                 910
Glu Ser Leu Met Leu Arg Ser Ser Ser Asn Leu Ala Leu Ala Leu Tyr
        915                 920                 925
Arg Tyr His Ser Tyr Lys Asp Leu Ser Glu Phe Leu Arg Gly Leu Val
    930                 935                 940
Met Asn Tyr Pro His Ile Thr Asn Leu Thr Asn Leu Gly Gln Ser Thr
945                 950                 955                 960
Glu Tyr Arg His Ile Trp Ser Leu Glu Ile Ser Asn Lys Pro Asn Val
                965                 970                 975
Ser Glu Pro Glu Glu Pro Lys Ile Arg Phe Val Ala Gly Ile His Gly
            980                 985                 990
Asn Ala Pro Val Gly Thr Glu Leu  Leu Leu Ala Leu Ala  Glu Phe Leu
        995                 1000                1005
Cys Leu  Asn Tyr Lys Lys Asn  Pro Ala Val Thr Gln  Leu Val Asp
    1010                1015                1020
Arg Thr  Arg Ile Val Ile Val  Pro Ser Leu Asn Pro  Asp Gly Arg
    1025                1030                1035
Glu Arg  Ala Gln Glu Lys Asp  Cys Thr Ser Lys Ile  Gly Gln Thr
    1040                1045                1050
Asn Ala  Arg Gly Lys Asp Leu  Asp Thr Asp Phe Thr  Asn Asn Ala
    1055                1060                1065
Ser Gln  Pro Glu Thr Lys Ala  Ile Ile Glu Asn Leu  Ile Gln Lys
    1070                1075                1080
Gln Asn  Phe Ser Leu Ser Val  Ala Leu Asp Gly Gly  Ser Met Leu
    1085                1090                1095
Val Thr  Tyr Pro Tyr Asp Lys  Pro Val Gln Thr Val  Glu Asn Lys
    1100                1105                1110
Glu Thr  Leu Lys His Leu Ala  Ser Leu Tyr Ala Asn  Asn His Pro
    1115                1120                1125
Ser Met  His Met Gly Gln Pro  Ser Cys Pro Asn Lys  Ser Asp Glu
    1130                1135                1140
Asn Ile  Pro Gly Gly Val Met  Arg Gly Ala Glu Trp  His Ser His
    1145                1150                1155
Leu Gly  Ser Met Lys Asp Tyr  Ser Val Thr Tyr Gly  His Cys Pro
    1160                1165                1170
Glu Ile  Thr Val Tyr Thr Ser  Cys Cys Tyr Phe Pro  Ser Ala Ala
    1175                1180                1185
Arg Leu  Pro Ser Leu Trp Ala  Asp Asn Lys Arg Ser  Leu Leu Ser
    1190                1195                1200
Met Leu  Val Glu Val His Lys  Gly Val His Gly Phe  Val Lys Asp
    1205                1210                1215
Lys Thr  Gly Lys Pro Ile Ser  Lys Ala Val Ile Val  Leu Asn Glu
    1220                1225                1230

Gly Ile Lys Val Gln Thr Lys Glu Gly Gly Tyr Phe His Val Leu
    1235                1240                1245
Leu Ala Pro Gly Val His Asn Ile Ile Ala Ile Ala Asp Gly Tyr
    1250                1255                1260
Gln Gln Gln His Ser Gln Val Phe Val His His Asp Ala Ala Ser
    1265                1270                1275
Ser Val Val Ile Val Phe Asp Thr Asp Asn Arg Ile Phe Gly Leu
    1280                1285                1290
Pro Arg Glu Leu Val Val Thr Val Ser Gly Ala Thr Met Ser Ala
    1295                1300                1305
Leu Ile Leu Thr Ala Cys Ile Ile Trp Cys Ile Cys Ser Ile Lys
    1310                1315                1320
Ser Asn Arg His Lys Asp Gly Phe His Arg Leu Arg Gln His His
    1325                1330                1335
Asp Glu Tyr Glu Asp Glu Ile Arg Met Met Ser Thr Gly Ser Lys
    1340                1345                1350
Lys Ser Leu Leu Ser His Glu Phe Gln Asp Glu Thr Asp Thr Glu
    1355                1360                1365
Glu Glu Thr Leu Tyr Ser Ser Lys His
    1370                1375

<210>  73
<211>  2563
<212>  PRT
<213>  Homo sapiens
<220>
<221>  misc_feature
<223>  CDS de la séquence GS-N21:60...7751( GS-P21) (USP9X)

<220>
<221>  misc_feature
<223>  nom: Protease 9 spécifique de l'ubiquitine

<400>  73
Met Thr Ala Thr Thr Arg Gly Ser Pro Val Gly Gly Asn Asp Asn Gln
1               5                   10                  15
Gly Gln Ala Pro Asp Gly Gln Ser Leu Pro Pro Leu Gln Gln Asn Gln
            20                  25                  30
Thr Ser Ser Pro Asp Ser Ser Asn Glu Asn Ser Pro Ala Thr Pro Pro
        35                  40                  45
Asp Glu Gln Gly Gln Gly Asp Ala Pro Pro Gln Leu Glu Asp Glu Glu
    50                  55                  60
Pro Ala Phe Pro His Thr Asp Leu Ala Lys Leu Asp Asp Met Ile Asn
65                  70                  75                  80
Arg Pro Arg Trp Val Val Pro Val Leu Pro Lys Gly Glu Leu Glu Val
                85                  90                  95
Leu Leu Glu Ala Ala Ile Asp Leu Ser Lys Lys Gly Leu Asp Val Lys
            100                 105                 110
Ser Glu Ala Cys Gln Arg Phe Phe Arg Asp Gly Leu Thr Ile Ser Phe
        115                 120                 125
Thr Lys Ile Leu Thr Asp Glu Ala Val Ser Gly Trp Lys Phe Glu Ile
    130                 135                 140
His Arg Cys Leu Val Glu Leu Cys Val Ala Lys Leu Ser Gln Asp Trp
145                 150                 155                 160
Phe Pro Leu Leu Glu Leu Leu Ala Met Ala Leu Asn Pro His Cys Lys
                165                 170                 175
Phe His Ile Tyr Asn Gly Thr Arg Pro Cys Glu Ser Val Ser Ser Ser
            180                 185                 190
Val Gln Leu Pro Glu Asp Glu Leu Phe Ala Arg Ser Pro Asp Pro Arg
        195                 200                 205
Ser Pro Lys Gly Trp Leu Val Asp Leu Leu Asn Lys Phe Gly Thr Leu
    210                 215                 220
Asn Gly Phe Gln Ile Leu His Asp Arg Phe Ile Asn Gly Ser Ala Leu
225                 230                 235                 240
Asn Val Gln Ile Ile Ala Ala Leu Ile Lys Pro Phe Gly Gln Cys Tyr
                245                 250                 255
Glu Phe Leu Thr Leu His Thr Val Lys Lys Tyr Phe Leu Pro Ile Ile
            260                 265                 270
Glu Met Val Pro Gln Phe Leu Glu Asn Leu Thr Asp Glu Glu Leu Lys
        275                 280                 285
Lys Glu Ala Lys Asn Glu Ala Lys Asn Asp Ala Leu Ser Met Ile Ile
    290                 295                 300

Lys Ser Leu Lys Asn Leu Ala Ser Arg Val Pro Gly Gln Glu Glu Thr
305                 310                 315                 320
Val Lys Asn Leu Glu Ile Phe Arg Leu Lys Met Ile Leu Arg Leu Leu
                325                 330                 335
Gln Ile Ser Ser Phe Asn Gly Lys Met Asn Ala Leu Asn Glu Val Asn
            340                 345                 350
Lys Val Ile Ser Ser Val Ser Tyr Tyr Thr His Arg His Gly Asn Pro
        355                 360                 365
Glu Glu Glu Glu Trp Leu Thr Ala Glu Arg Met Ala Glu Trp Ile Gln
    370                 375                 380
Gln Asn Asn Ile Leu Ser Ile Val Leu Arg Asp Ser Leu His Gln Pro
385                 390                 395                 400
Gln Tyr Val Glu Lys Leu Glu Lys Ile Leu Arg Phe Val Ile Lys Glu
                405                 410                 415
Lys Ala Leu Thr Leu Gln Asp Leu Asp Asn Ile Trp Ala Ala Gln Ala
            420                 425                 430
Gly Lys His Glu Ala Ile Val Lys Asn Val His Asp Leu Leu Ala Lys
        435                 440                 445
Leu Ala Trp Asp Phe Ser Pro Glu Gln Leu Asp His Pro Phe Asp Cys
    450                 455                 460
Phe Lys Ala Ser Arg Thr Asn Ala Ser Lys Lys Gln Arg Glu Lys Leu
465                 470                 475                 480
Leu Glu Leu Ile Arg Arg Leu Ala Glu Asp Asp Lys Asp Gly Val Met
                485                 490                 495
Ala His Arg Val Leu Asn Leu Leu Trp Asn Leu Ala His Ser Asp Asp
            500                 505                 510
Val Pro Val Asp Ile Met Asp Leu Ala Leu Ser Ala His Ile Lys Ile
        515                 520                 525
Leu Asp Tyr Ser Cys Ser Gln Asp Arg Asp Thr Gln Lys Ile Gln Trp
    530                 535                 540
Ile Asp Arg Phe Ile Glu Glu Leu Arg Thr Asn Asp Lys Trp Val Ile
545                 550                 555                 560
Pro Ala Leu Lys Gln Ile Arg Glu Ile Cys Ser Leu Phe Gly Glu Ala
                565                 570                 575
Pro Gln Asn Leu Ser Gln Thr Gln Arg Ser Pro His Val Phe Tyr Arg
            580                 585                 590
His Asp Leu Ile Asn Gln Leu Gln His Asn His Ala Leu Val Thr Leu
        595                 600                 605
Val Ala Glu Asn Leu Val Thr Tyr Met Glu Ser Met Arg Leu Tyr Ala
    610                 615                 620
Arg Asp His Glu Asp Tyr Asp Pro Gln Thr Val Arg Leu Gly Ser Arg
625                 630                 635                 640
Tyr Ser His Val Gln Glu Val Gln Glu Arg Leu Asn Phe Leu Arg Phe
                645                 650                 655
Leu Leu Lys Asp Gly Gln Leu Trp Leu Cys Ala Pro Gln Ala Lys Gln
            660                 665                 670
Ile Trp Lys Cys Leu Ala Glu Asn Ala Val Tyr Leu Cys Asp Arg Glu
        675                 680                 685
Ala Cys Phe Lys Trp Tyr Ser Lys Leu Met Gly Asp Glu Pro Asp Leu
    690                 695                 700
Asp Pro Asp Ile Asn Lys Asp Phe Phe Glu Ser Asn Val Leu Gln Leu
705                 710                 715                 720
Asp Pro Ser Leu Leu Thr Glu Asn Gly Met Lys Cys Phe Glu Arg Phe
                725                 730                 735
Phe Lys Ala Val Asn Cys Arg Glu Gly Lys Leu Val Ala Lys Arg Arg
            740                 745                 750
Ala Tyr Met Met Asp Asp Leu Glu Leu Ile Gly Leu Asp Tyr Leu Trp
        755                 760                 765
Arg Val Val Ile Gln Ser Asn Asp Asp Ile Ala Ser Arg Ala Ile Asp
    770                 775                 780
Leu Leu Lys Glu Ile Tyr Thr Asn Leu Gly Pro Arg Leu Gln Val Asn
785                 790                 795                 800
Gln Val Val Ile His Glu Asp Phe Ile Gln Ser Cys Phe Asp Arg Leu
                805                 810                 815
Lys Ala Ser Tyr Asp Thr Leu Cys Val Leu Asp Gly Asp Lys Asp Ser
            820                 825                 830
Val Asn Cys Ala Arg Gln Glu Ala Val Arg Met Val Arg Val Leu Thr
        835                 840                 845
Val Leu Arg Glu Tyr Ile Asn Glu Cys Asp Ser Asp Tyr His Glu Glu
    850                 855                 860
Arg Thr Ile Leu Pro Met Ser Arg Ala Phe Arg Gly Lys His Leu Ser
865                 870                 875                 880
Phe Val Val Arg Phe Pro Asn Gln Gly Arg Gln Val Asp Asp Leu Glu

                885                 890                 895
Val Trp Ser His Thr Asn Asp Thr Ile Gly Ser Val Arg Arg Cys Ile
            900                 905                 910
Leu Asn Arg Ile Lys Ala Asn Val Ala His Thr Lys Ile Glu Leu Phe
        915                 920                 925
Val Gly Gly Glu Leu Ile Asp Pro Ala Asp Asp Arg Lys Leu Ile Gly
    930                 935                 940
Gln Leu Asn Leu Lys Asp Lys Ser Leu Ile Thr Ala Lys Leu Thr Gln
945                 950                 955                 960
Ile Ser Ser Asn Met Pro Ser Ser Pro Asp Ser Ser Ser Asp Ser Ser
                965                 970                 975
Thr Gly Ser Pro Gly Asn His Gly Asn His Tyr Ser Asp Gly Pro Asn
            980                 985                 990
Pro Glu Val Glu Ser Cys Leu Pro Gly Val Ile Met Ser Leu His Pro
        995                 1000                1005
Arg Tyr Ile Ser Phe Leu Trp Gln Val Ala Asp Leu Gly Ser Ser
    1010                1015                1020
Leu Asn Met Pro Pro Leu Arg Asp Gly Ala Arg Val Leu Met Lys
    1025                1030                1035
Leu Met Pro Pro Asp Ser Thr Thr Ile Glu Lys Leu Arg Ala Ile
    1040                1045                1050
Cys Leu Asp His Ala Lys Leu Gly Glu Ser Ser Leu Ser Pro Ser
    1055                1060                1065
Leu Asp Ser Leu Phe Phe Gly Pro Ser Ala Ser Gln Val Leu Tyr
    1070                1075                1080
Leu Thr Glu Val Val Tyr Ala Leu Leu Met Pro Ala Gly Ala Pro
    1085                1090                1095
Leu Ala Asp Asp Ser Ser Asp Phe Gln Phe His Phe Leu Lys Ser
    1100                1105                1110
Gly Gly Leu Pro Leu Val Leu Ser Met Leu Thr Arg Asn Asn Phe
    1115                1120                1125
Leu Pro Asn Ala Asp Met Glu Thr Arg Arg Gly Ala Tyr Leu Asn
    1130                1135                1140
Ala Leu Lys Ile Ala Lys Leu Leu Leu Thr Ala Ile Gly Tyr Gly
    1145                1150                1155
His Val Arg Ala Val Ala Glu Ala Cys Gln Pro Gly Val Glu Gly
    1160                1165                1170
Val Asn Pro Met Thr Gln Ile Asn Gln Val Thr His Asp Gln Ala
    1175                1180                1185
Val Val Leu Gln Ser Ala Leu Gln Ser Ile Pro Asn Pro Ser Ser
    1190                1195                1200
Glu Cys Met Leu Arg Asn Val Ser Val Arg Leu Ala Gln Gln Ile
    1205                1210                1215
Ser Asp Glu Ala Ser Arg Tyr Met Pro Asp Ile Cys Val Ile Arg
    1220                1225                1230
Ala Ile Gln Lys Ile Ile Trp Ala Ser Gly Cys Gly Ser Leu Gln
    1235                1240                1245
Leu Val Phe Ser Pro Asn Glu Glu Ile Thr Lys Ile Tyr Glu Lys
    1250                1255                1260
Thr Asn Ala Gly Asn Glu Pro Asp Leu Glu Asp Glu Gln Val Cys
    1265                1270                1275
Cys Glu Ala Leu Glu Val Met Thr Leu Cys Phe Ala Leu Ile Pro
    1280                1285                1290
Thr Ala Leu Asp Ala Leu Ser Lys Glu Lys Ala Trp Gln Thr Phe
    1295                1300                1305
Ile Ile Asp Leu Leu Leu His Cys His Ser Lys Thr Val Arg Gln
    1310                1315                1320
Val Ala Gln Glu Gln Phe Phe Leu Met Cys Thr Arg Cys Cys Met
    1325                1330                1335
Gly His Arg Pro Leu Leu Phe Phe Ile Thr Leu Leu Phe Thr Val
    1340                1345                1350
Leu Gly Ser Thr Ala Arg Glu Arg Ala Lys His Ser Gly Asp Tyr
    1355                1360                1365
Phe Thr Leu Leu Arg His Leu Leu Asn Tyr Ala Tyr Asn Ser Asn
    1370                1375                1380
Ile Asn Val Pro Asn Ala Glu Val Leu Phe Asn Asn Glu Ile Asp
    1385                1390                1395
Trp Leu Lys Arg Ile Arg Asp Asp Val Lys Arg Thr Gly Glu Thr
    1400                1405                1410
Gly Ile Glu Glu Thr Ile Leu Glu Gly His Leu Gly Val Thr Lys
    1415                1420                1425
Glu Leu Leu Ala Phe Gln Thr Ser Glu Lys Lys Phe His Ile Gly
    1430                1435                1440

Cys Glu Lys Gly Gly Ala Asn Leu Ile Lys Glu Leu Ile Asp Asp
    1445                1450                1455
Phe Ile Phe Pro Ala Ser Asn Val Tyr Leu Gln Tyr Met Arg Asn
    1460                1465                1470
Gly Glu Leu Pro Ala Glu Gln Ala Ile Pro Val Cys Gly Ser Pro
    1475                1480                1485
Pro Thr Ile Asn Ala Gly Phe Glu Leu Leu Val Ala Leu Ala Val
    1490                1495                1500
Gly Cys Val Arg Asn Leu Lys Gln Ile Val Asp Ser Leu Thr Glu
    1505                1510                1515
Met Tyr Tyr Ile Gly Thr Ala Ile Thr Thr Cys Glu Ala Leu Thr
    1520                1525                1530
Glu Trp Glu Tyr Leu Pro Pro Val Gly Pro Arg Pro Pro Lys Gly
    1535                1540                1545
Phe Val Gly Leu Lys Asn Ala Gly Ala Thr Cys Tyr Met Asn Ser
    1550                1555                1560
Val Ile Gln Gln Leu Tyr Met Ile Pro Ser Ile Arg Asn Gly Ile
    1565                1570                1575
Leu Ala Ile Glu Gly Thr Gly Ser Asp Val Asp Asp Asp Met Ser
    1580                1585                1590
Gly Asp Glu Lys Gln Asp Asn Glu Ser Asn Val Asp Pro Arg Asp
    1595                1600                1605
Asp Val Phe Gly Tyr Pro Gln Gln Phe Glu Asp Lys Pro Ala Leu
    1610                1615                1620
Ser Lys Thr Glu Asp Arg Lys Glu Tyr Asn Ile Gly Val Leu Arg
    1625                1630                1635
His Leu Gln Val Ile Phe Gly His Leu Ala Ala Ser Arg Leu Gln
    1640                1645                1650
Tyr Tyr Val Pro Arg Gly Phe Trp Lys Gln Phe Arg Leu Trp Gly
    1655                1660                1665
Glu Pro Val Asn Leu Arg Glu Gln His Asp Ala Leu Glu Phe Phe
    1670                1675                1680
Asn Ser Leu Val Asp Ser Leu Asp Glu Ala Leu Lys Ala Leu Gly
    1685                1690                1695
His Pro Ala Met Leu Ser Lys Val Leu Gly Gly Ser Phe Ala Asp
    1700                1705                1710
Gln Lys Ile Cys Gln Gly Cys Pro His Arg Tyr Glu Cys Glu Glu
    1715                1720                1725
Ser Phe Thr Thr Leu Asn Val Asp Ile Arg Asn His Gln Asn Leu
    1730                1735                1740
Leu Asp Ser Leu Glu Gln Tyr Val Lys Gly Asp Leu Leu Glu Gly
    1745                1750                1755
Ala Asn Ala Tyr His Cys Glu Lys Cys Asn Lys Lys Val Asp Thr
    1760                1765                1770
Val Lys Arg Leu Leu Ile Lys Lys Leu Pro Pro Val Leu Ala Ile
    1775                1780                1785
Gln Leu Lys Arg Phe Asp Tyr Asp Trp Glu Arg Glu Cys Ala Ile
    1790                1795                1800
Lys Phe Asn Asp Tyr Phe Glu Phe Pro Arg Glu Leu Asp Met Glu
    1805                1810                1815
Pro Tyr Thr Val Ala Gly Val Ala Lys Leu Glu Gly Asp Asn Val
    1820                1825                1830
Asn Pro Glu Ser Gln Leu Ile Gln Gln Ser Glu Gln Ser Glu Ser
    1835                1840                1845
Glu Thr Ala Gly Ser Thr Lys Tyr Arg Leu Val Gly Val Leu Val
    1850                1855                1860
His Ser Gly Gln Ala Ser Gly Gly His Tyr Tyr Ser Tyr Ile Ile
    1865                1870                1875
Gln Arg Asn Gly Gly Asp Gly Glu Arg Asn Arg Trp Tyr Lys Phe
    1880                1885                1890
Asp Asp Gly Asp Val Thr Glu Cys Lys Met Asp Asp Asp Glu Glu
    1895                1900                1905
Met Lys Asn Gln Cys Phe Gly Gly Glu Tyr Met Gly Glu Val Phe
    1910                1915                1920
Asp His Met Met Lys Arg Met Ser Tyr Arg Arg Gln Lys Arg Trp
    1925                1930                1935
Trp Asn Ala Tyr Ile Pro Phe Tyr Glu Arg Met Asp Thr Ile Asp
    1940                1945                1950
Gln Asp Asp Glu Leu Ile Arg Tyr Ile Ser Glu Leu Ala Ile Thr
    1955                1960                1965
Thr Arg Pro His Gln Ile Ile Met Pro Ser Ala Ile Glu Arg Ser
    1970                1975                1980
Val Arg Lys Gln Asn Val Gln Phe Met His Asn Arg Met Gln Tyr

1985                    1990                    1995
Ser Met  Glu Tyr Phe Gln Phe  Met Lys Lys Leu Leu  Thr Cys Asn
    2000                    2005                    2010
Gly Val  Tyr Leu Asn Pro Pro  Pro Gly Gln Asp His  Leu Leu Pro
    2015                    2020                    2025
Glu Ala  Glu Glu Ile Thr Met  Ile Ser Ile Gln Leu  Ala Ala Arg
    2030                    2035                    2040
Phe Leu  Phe Thr Thr Gly Phe  His Thr Lys Lys Val  Val Arg Gly
    2045                    2050                    2055
Ser Ala  Ser Asp Trp Tyr Asp  Ala Leu Cys Ile Leu  Leu Arg His
    2060                    2065                    2070
Ser Lys  Asn Val Arg Phe Trp  Phe Ala His Asn Val  Leu Phe Asn
    2075                    2080                    2085
Val Ser  Asn Arg Phe Ser Glu  Tyr Leu Leu Glu Cys  Pro Ser Ala
    2090                    2095                    2100
Glu Val  Arg Gly Ala Phe Ala  Lys Leu Ile Val Phe  Ile Ala His
    2105                    2110                    2115
Phe Ser  Leu Gln Asp Gly Pro  Cys Pro Ser Pro Phe  Ala Ser Pro
    2120                    2125                    2130
Gly Pro  Ser Ser Gln Ala Tyr  Asp Asn Leu Ser Leu  Ser Asp His
    2135                    2140                    2145
Leu Leu  Arg Ala Val Leu Asn  Leu Leu Arg Arg Glu  Val Ser Glu
    2150                    2155                    2160
His Gly  Arg His Leu Gln Gln  Tyr Phe Asn Leu Phe  Val Met Tyr
    2165                    2170                    2175
Ala Asn  Leu Gly Val Ala Glu  Lys Thr Gln Leu Leu  Lys Leu Ser
    2180                    2185                    2190
Val Pro  Ala Thr Phe Met Leu  Val Ser Leu Asp Glu  Gly Pro Gly
    2195                    2200                    2205
Pro Pro  Ile Lys Tyr Gln Tyr  Ala Glu Leu Gly Lys  Leu Tyr Ser
    2210                    2215                    2220
Val Val  Ser Gln Leu Ile Arg  Cys Cys Asn Val Ser  Ser Arg Met
    2225                    2230                    2235
Gln Ser  Ser Ile Asn Gly Asn  Pro Pro Leu Pro Asn  Pro Phe Gly
    2240                    2245                    2250
Asp Pro  Asn Leu Ser Gln Pro  Ile Met Pro Ile Gln  Gln Asn Val
    2255                    2260                    2265
Ala Asp  Ile Leu Phe Val Arg  Thr Ser Tyr Val Lys  Lys Ile Ile
    2270                    2275                    2280
Glu Asp  Cys Ser Asn Ser Glu  Glu Thr Val Lys Leu  Leu Arg Phe
    2285                    2290                    2295
Cys Cys  Trp Glu Asn Pro Gln  Phe Ser Ser Thr Val  Leu Ser Glu
    2300                    2305                    2310
Leu Leu  Trp Gln Val Ala Tyr  Ser Tyr Pro Tyr Glu  Leu Arg Pro
    2315                    2320                    2325
Tyr Leu  Asp Leu Leu Leu Gln  Ile Leu Leu Ile Glu  Asp Ser Trp
    2330                    2335                    2340
Gln Thr  His Arg Ile His Asn  Ala Leu Lys Gly Ile  Pro Asp Asp
    2345                    2350                    2355
Arg Asp  Gly Leu Phe Asp Thr  Ile Gln Arg Ser Lys  Asn His Tyr
    2360                    2365                    2370
Gln Lys  Arg Ala Tyr Gln Cys  Ile Lys Cys Met Val  Ala Leu Phe
    2375                    2380                    2385
Ser Asn  Cys Pro Val Ala Tyr  Gln Ile Leu Gln Gly  Asn Gly Asp
    2390                    2395                    2400
Leu Lys  Arg Lys Trp Thr Trp  Ala Val Glu Trp Leu  Gly Asp Glu
    2405                    2410                    2415
Leu Glu  Arg Arg Pro Tyr Thr  Gly Asn Pro Gln Tyr  Thr Tyr Asn
    2420                    2425                    2430
Asn Trp  Ser Pro Pro Val Gln  Ser Asn Glu Thr Ser  Asn Gly Tyr
    2435                    2440                    2445
Phe Leu  Glu Arg Ser His Ser  Ala Arg Met Thr Leu  Ala Lys Ala
    2450                    2455                    2460
Cys Glu  Leu Cys Pro Glu Glu  Val Lys Lys Ala Thr  Ser Val Gln
    2465                    2470                    2475
Gln Ile  Glu Met Glu Glu Ser  Lys Glu Pro Asp Asp  Gln Asp Ala
    2480                    2485                    2490
Pro Asp  Glu His Glu Ser Pro  Pro Pro Glu Asp Ala  Pro Leu Tyr
    2495                    2500                    2505
Pro His  Ser Pro Gly Ser Gln  Tyr Gln Gln Asn Asn  His Val His
    2510                    2515                    2520
Gly Gln  Pro Tyr Thr Gly Pro  Ala Ala His His Met  Asn Asn Pro
    2525                    2530                    2535

Gln Arg Thr Gly Gln Arg Ala Gln Glu Asn Tyr Glu Gly Ser Glu
    2540                2545                2550
Glu Val Ser Pro Pro Gln Thr Lys Asp Gln
    2555                2560

<210> 74
<211> 1219
<212> PRT
<213> Homo sapiens
<220>
<221> misc_feature
<223> CDS de la séquence GS-N22: 136...3795 (GS-P22)

<220>
<221> misc_feature
<223> nom: : Nardilysine (N-arginine dibasic convertase) (NRD1)

<400> 74
   Met Leu Arg Arg Val Thr Val Ala Ala Val Cys Ala Thr Arg Arg Lys
   1 5 10 15
   Leu Cys Glu Ala Gly Arg Asp Val Ala Ala Leu Trp Gly Ile Glu Thr
   20 25 30
   Arg Gly Arg Cys Glu Asp Ser Ala Ala Ala Arg Pro Phe Pro Ile Leu
   35 40 45
   Ala Met Pro Gly Arg Asn Lys Ala Lys Ser Thr Cys Ser Cys Pro Asp
   50 55 60
   Leu Gln Pro Asn Gly Gln Asp Leu Gly Glu Asn Ser Arg Val Ala Arg
   65 70 75 80
   Leu Gly Ala Asp Glu Ser Glu Glu Glu Gly Arg Arg Gly Ser Leu Ser
   85 90 95
   Asn Ala Gly Asp Pro Glu Ile Val Lys Ser Pro Ser Asp Pro Lys Gln
   100 105 110
   Tyr Arg Tyr Ile Lys Leu Gln Asn Gly Leu Gln Ala Leu Leu Ile Ser
   115 120 125
   Asp Leu Ser Asn Met Glu Gly Lys Thr Gly Asn Thr Thr Asp Asp Glu
   130 135 140
   Glu Glu Glu Glu Val Glu Glu Glu Glu Glu Asp Asp Asp Glu Asp Ser
   145 150 155 160
   Gly Ala Glu Ile Glu Asp Asp Asp Glu Glu Gly Phe Asp Asp Glu Asp
   165 170 175
   Glu Phe Asp Asp Glu His Asp Asp Asp Leu Asp Thr Glu Asp Asn Glu
   180 185 190
   Leu Glu Glu Leu Glu Glu Arg Ala Glu Ala Arg Lys Lys Thr Thr Glu
   195 200 205
   Lys Gln Gln Leu Gln Ser Leu Phe Leu Leu Trp Ser Lys Leu Thr Asp
   210 215 220
   Arg Leu Trp Phe Lys Ser Thr Tyr Ser Lys Met Ser Ser Thr Leu Leu
   225 230 235 240
   Val Glu Thr Arg Asn Leu Tyr Gly Val Val Gly Ala Glu Ser Arg Ser
   245 250 255
   Ala Pro Val Gln His Leu Ala Gly Trp Gln Ala Glu Glu Gln Gln Gly
   260 265 270
   Glu Thr Asp Thr Val Leu Ser Ala Ala Ala Leu Cys Val Gly Val Gly
   275 280 285
   Ser Phe Ala Asp Pro Asp Asp Leu Pro Gly Leu Ala His Phe Leu Glu
   290 295 300
   His Met Val Phe Met Gly Ser Leu Lys Tyr Pro Asp Glu Asn Gly Phe
   305 310 315 320
   Asp Ala Phe Leu Lys Lys His Gly Gly Ser Asp Asn Ala Ser Thr Asp
   325 330 335
   Cys Glu Arg Thr Val Phe Gln Phe Asp Val Gln Arg Lys Tyr Phe Lys
   340 345 350
   Glu Ala Leu Asp Arg Trp Ala Gln Phe Phe Ile His Pro Leu Met Ile
   355 360 365
   Arg Asp Ala Ile Asp Arg Glu Val Glu Ala Val Asp Ser Glu Tyr Gln
   370 375 380
   Leu Ala Arg Pro Ser Asp Ala Asn Arg Lys Glu Met Leu Phe Gly Ser
   385 390 395 400
   Leu Ala Arg Pro Gly His Pro Met Gly Lys Phe Phe Trp Gly Asn Ala
   405 410 415
   Glu Thr Leu Lys His Glu Pro Arg Lys Asn Asn Ile Asp Thr His Ala
   420 425 430
   Arg Leu Arg Glu Phe Trp Met Arg Tyr Tyr Ser Ser His Tyr Met Thr
           435                 440                 445
   Leu Val Val Gln Ser Lys Glu Thr Leu Asp Thr Leu Glu Lys Trp Val
       450                 455                 460
   Thr Glu Ile Phe Ser Gln Ile Pro Asn Asn Gly Leu Pro Arg Pro Asn
   465                 470                 475                 480
   Phe Gly His Leu Thr Asp Pro Phe Asp Thr Pro Ala Phe Asn Lys Leu
                   485                 490                 495
   Tyr Arg Val Val Pro Ile Arg Lys Ile His Ala Leu Thr Ile Thr Trp
               500                 505                 510
   Ala Leu Pro Pro Gln Gln Gln His Tyr Arg Val Lys Pro Leu His Tyr
           515                 520                 525
   Ile Ser Trp Leu Val Gly His Glu Gly Lys Gly Ser Ile Leu Ser Phe
       530                 535                 540
   Leu Arg Lys Lys Cys Trp Ala Leu Ala Leu Phe Gly Gly Asn Gly Glu
   545                 550                 555                 560
   Thr Gly Phe Glu Gln Asn Ser Thr Tyr Ser Val Phe Ser Ile Ser Ile
                   565                 570                 575
   Thr Leu Thr Asp Glu Gly Tyr Glu His Phe Tyr Glu Val Ala Tyr Thr
               580                 585                 590
   Val Phe Leu Tyr Leu Lys Met Leu Gln Lys Leu Gly Pro Glu Lys Arg
           595                 600                 605
   Ile Phe Glu Glu Ile Arg Lys Ile Glu Asp Asn Glu Phe His Tyr Gln
       610                 615                 620
   Glu Gln Thr Asp Pro Val Glu Tyr Val Glu Asn Met Cys Glu Asn Met
   625                 630                 635                 640
   Gln Leu Tyr Pro Leu Gln Asp Ile Leu Thr Gly Asp Gln Leu Leu Phe
                   645                 650                 655
   Glu Tyr Lys Pro Glu Val Ile Gly Glu Ala Leu Asn Gln Leu Val Pro
               660                 665                 670
   Gln Lys Ala Asn Leu Val Leu Leu Ser Gly Ala Asn Glu Gly Lys Cys
           675                 680                 685
   Asp Leu Lys Glu Lys Trp Phe Gly Thr Gln Tyr Ser Ile Glu Asp Ile
       690                 695                 700
   Glu Asn Ser Trp Ala Glu Leu Trp Asn Ser Asn Phe Glu Leu Asn Pro
   705                 710                 715                 720
   Asp Leu His Leu Pro Ala Glu Asn Lys Tyr Ile Ala Thr Asp Phe Thr
                   725                 730                 735
   Leu Lys Ala Phe Asp Cys Pro Glu Thr Glu Tyr Pro Val Lys Ile Val
               740                 745                 750
   Asn Thr Pro Gln Gly Cys Leu Trp Tyr Lys Lys Asp Asn Lys Phe Lys
           755                 760                 765
   Ile Pro Lys Ala Tyr Ile Arg Phe His Leu Ile Ser Pro Leu Ile Gln
       770                 775                 780
   Lys Ser Ala Ala Asn Val Val Leu Phe Asp Ile Phe Val Asn Ile Leu
   785                 790                 795                 800
   Thr His Asn Leu Ala Glu Pro Ala Tyr Glu Ala Asp Val Ala Gln Leu
                   805                 810                 815
   Glu Tyr Lys Leu Ala Ala Gly Glu His Gly Leu Ile Ile Arg Val Lys
               820                 825                 830
   Gly Phe Asn His Lys Leu Pro Leu Leu Phe Gln Leu Ile Ile Asp Tyr
           835                 840                 845
   Leu Ala Glu Phe Asn Ser Thr Pro Ala Val Phe Thr Met Ile Thr Glu
       850                 855                 860
   Gln Leu Lys Lys Thr Tyr Phe Asn Ile Leu Ile Lys Pro Glu Thr Leu
   865                 870                 875                 880
   Ala Lys Asp Val Arg Leu Leu Ile Leu Glu Tyr Ala Arg Trp Ser Met
                   885                 890                 895
   Ile Asp Lys Tyr Gln Ala Leu Met Asp Gly Leu Ser Leu Glu Ser Leu
               900                 905                 910
   Leu Ser Phe Val Lys Glu Phe Lys Ser Gln Leu Phe Val Glu Gly Leu
           915                 920                 925
   Val Gln Gly Asn Val Thr Ser Thr Glu Ser Met Asp Phe Leu Lys Tyr
       930                 935                 940
   Val Val Asp Lys Leu Asn Phe Lys Pro Leu Glu Gln Glu Met Pro Val
   945                 950                 955                 960
   Gln Phe Gln Val Val Glu Leu Pro Ser Gly His His Leu Cys Lys Val
                   965                 970                 975
   Lys Ala Leu Asn Lys Gly Asp Ala Asn Ser Glu Val Thr Val Tyr Tyr
               980                 985                 990
   Gln Ser Gly Thr Arg Ser Leu Arg  Glu Tyr Thr Leu Met  Glu Leu Leu
           995                 1000                 1005
   Val Met  His Met Glu Glu Pro  Cys Phe Asp Phe Leu  Arg Thr Lys
   1010                1015                1020
   Gln Thr Leu Gly Tyr His Val Tyr Pro Thr Cys Arg Asn Thr Ser
       1025                1030                1035
   Gly Ile Leu Gly Phe Ser Val Thr Val Gly Thr Gln Ala Thr Lys
       1040                1045                1050
   Tyr Asn Ser Glu Val Val Asp Lys Lys Ile Glu Glu Phe Leu Ser
       1055                1060                1065
   Ser Phe Glu Glu Lys Ile Glu Asn Leu Thr Glu Glu Ala Phe Asn
       1070                1075                1080
   Thr Gln Val Thr Ala Leu Ile Lys Leu Lys Glu Cys Glu Asp Thr
       1085                1090                1095
   His Leu Gly Glu Glu Val Asp Arg Asn Trp Asn Glu Val Val Thr
       1100                1105                1110
   Gln Gln Tyr Leu Phe Asp Arg Leu Ala His Glu Ile Glu Ala Leu
       1115                1120                1125
   Lys Ser Phe Ser Lys Ser Asp Leu Val Asn Trp Phe Lys Ala His
       1130                1135                1140
   Arg Gly Pro Gly Ser Lys Met Leu Ser Val His Val Val Gly Tyr
       1145                1150                1155
   Gly Lys Tyr Glu Leu Glu Glu Asp Gly Ser Pro Ser Ser Glu Asp
       1160                1165                1170
   Ser Asn Ser Ser Cys Glu Val Met Gln Leu Thr Tyr Leu Pro Thr
       1175                1180                1185
   Ser Pro Leu Leu Ala Asp Cys Ile Ile Pro Ile Thr Asp Ile Arg
       1190                1195                1200
   Ala Phe Thr Thr Thr Leu Asn Leu Leu Pro Tyr His Lys Ile Val
       1205                1210                1215
   Lys

<210>  75
<211>  2709
<212>  PRT
<213>  Homo sapiens
<220>
<221>  misc_feature
<223>  CDS de la séquence GS-N23: 1872...10001(GS-P23)

<220>
<221>  misc_feature
<223>  nom: protéine similaire à la protéine HRX Ring Finger HRX (ALL-1)
        ou MLL( Homo sapiens myeloid/lymphoid or mixed-lineage leukemia
        )

<400>  75
Met Ser Ala Glu Glu Gln Leu Leu Pro Leu Ala Arg Lys Arg Ala Val
1               5                   10                  15
Phe Pro Asp Asp Met Pro Thr Leu Ser Ala Leu Pro Trp Glu Glu Arg
            20                  25                  30
Glu Lys Ile Leu Ser Ser Met Gly Asn Asp Asp Lys Ser Ser Ile Ala
        35                  40                  45
Gly Ser Glu Asp Ala Glu Pro Leu Ala Pro Pro Ile Lys Pro Ile Lys
    50                  55                  60
Pro Val Thr Arg Asn Lys Ala Pro Gln Glu Pro Pro Val Lys Lys Gly
65                  70                  75                  80
Arg Arg Ser Arg Arg Cys Gly Gln Cys Pro Gly Cys Gln Val Pro Glu
                85                  90                  95
Asp Cys Gly Val Cys Thr Asn Cys Leu Asp Lys Pro Lys Phe Gly Gly
            100                 105                 110
Arg Asn Ile Lys Lys Gln Cys Cys Lys Met Arg Lys Cys Gln Asn Leu
        115                 120                 125
Gln Trp Met Pro Ser Lys Ala Tyr Leu Gln Lys Gln Ala Lys Ala Val
    130                 135                 140
Lys Lys Lys Glu Lys Lys Ser Lys Thr Ser Glu Lys Lys Asp Ser Lys
145                 150                 155                 160
Glu Ser Ser Val Val Lys Asn Val Val Asp Ser Ser Gln Lys Pro Thr
                165                 170                 175
Pro Ser Ala Arg Glu Asp Pro Ala Pro Lys Lys Ser Ser Ser Glu Pro
            180                 185                 190
Pro Pro Arg Lys Pro Val Glu Glu Lys Ser Glu Glu Gly Asn Val Ser
        195                 200                 205
Ala Pro Gly Pro Glu Ser Lys Gln Ala Thr Thr Pro Ala Ser Arg Lys

210                 215                 220
Ser Ser Lys Gln Val Ser Gln Pro Ala Leu Val Ile Pro Pro Gln Pro
225                 230                 235                 240
Pro Thr Thr Gly Pro Pro Arg Lys Glu Val Pro Lys Thr Thr Pro Ser
                245                 250                 255
Glu Pro Lys Lys Lys Gln Pro Pro Pro Pro Glu Ser Gly Pro Glu Gln
            260                 265                 270
Ser Lys Gln Lys Lys Val Ala Pro Arg Pro Ser Ile Pro Val Lys Gln
        275                 280                 285
Lys Pro Lys Glu Lys Glu Lys Pro Pro Pro Val Asn Lys Gln Glu Asn
    290                 295                 300
Ala Gly Thr Leu Asn Ile Leu Ser Thr Leu Ser Asn Gly Asn Ser Ser
305                 310                 315                 320
Lys Gln Lys Ile Pro Ala Asp Gly Val His Arg Ile Arg Val Asp Phe
                325                 330                 335
Lys Glu Asp Cys Glu Ala Glu Asn Val Trp Glu Met Gly Gly Leu Gly
            340                 345                 350
Ile Leu Thr Ser Val Pro Ile Thr Pro Arg Val Val Cys Phe Leu Cys
        355                 360                 365
Ala Ser Ser Gly His Val Glu Phe Val Tyr Cys Gln Val Cys Cys Glu
    370                 375                 380
Pro Phe His Lys Phe Cys Leu Glu Glu Asn Glu Arg Pro Leu Glu Asp
385                 390                 395                 400
Gln Leu Glu Asn Trp Cys Cys Arg Arg Cys Lys Phe Cys His Val Cys
                405                 410                 415
Gly Arg Gln His Gln Ala Thr Lys Gln Leu Leu Glu Cys Asn Lys Cys
            420                 425                 430
Arg Asn Ser Tyr His Pro Glu Cys Leu Gly Pro Asn Tyr Pro Thr Lys
        435                 440                 445
Pro Thr Lys Lys Lys Lys Val Trp Ile Cys Thr Lys Cys Val Arg Cys
    450                 455                 460
Lys Ser Cys Gly Ser Thr Thr Pro Gly Lys Gly Trp Asp Ala Gln Trp
465                 470                 475                 480
Ser His Asp Phe Ser Leu Cys His Asp Cys Ala Lys Leu Phe Ala Lys
                485                 490                 495
Gly Asn Phe Cys Pro Leu Cys Asp Lys Cys Tyr Asp Asp Asp Asp Tyr
            500                 505                 510
Glu Ser Lys Met Met Gln Cys Gly Lys Cys Asp Arg Trp Val His Ser
        515                 520                 525
Lys Cys Glu Asn Leu Ser Gly Thr Glu Asp Glu Met Tyr Glu Ile Leu
    530                 535                 540
Ser Asn Leu Pro Glu Ser Val Ala Tyr Thr Cys Val Asn Cys Thr Glu
545                 550                 555                 560
Arg His Pro Ala Glu Trp Arg Leu Ala Leu Glu Lys Glu Leu Gln Ile
                565                 570                 575
Ser Leu Lys Gln Val Leu Thr Ala Leu Leu Asn Ser Arg Thr Thr Ser
            580                 585                 590
His Leu Leu Arg Tyr Arg Gln Ala Ala Lys Pro Pro Asp Leu Asn Pro
        595                 600                 605
Glu Thr Glu Glu Ser Ile Pro Ser Arg Ser Ser Pro Glu Gly Pro Asp
    610                 615                 620
Pro Pro Val Leu Thr Glu Val Ser Lys Gln Asp Asp Gln Gln Pro Leu
625                 630                 635                 640
Asp Leu Glu Gly Val Lys Arg Lys Met Asp Gln Gly Asn Tyr Thr Ser
                645                 650                 655
Val Leu Glu Phe Ser Asp Asp Ile Val Lys Ile Ile Gln Ala Ala Ile
            660                 665                 670
Asn Ser Asp Gly Gly Gln Pro Glu Ile Lys Lys Ala Asn Ser Met Val
        675                 680                 685
Lys Ser Phe Phe Ile Arg Gln Met Glu Arg Val Phe Pro Trp Phe Ser
    690                 695                 700
Val Lys Lys Ser Arg Phe Trp Glu Pro Asn Lys Val Ser Ser Asn Ser
705                 710                 715                 720
Gly Met Leu Pro Asn Ala Val Leu Pro Pro Ser Leu Asp His Asn Tyr
                725                 730                 735
Ala Gln Trp Gln Glu Arg Glu Glu Asn Ser His Thr Glu Gln Pro Pro
            740                 745                 750
Leu Met Lys Lys Ile Ile Pro Ala Pro Lys Pro Lys Gly Pro Gly Glu
        755                 760                 765
Pro Asp Ser Pro Thr Pro Leu His Pro Pro Thr Pro Pro Ile Leu Ser
    770                 775                 780
Thr Asp Arg Ser Arg Glu Asp Ser Pro Glu Leu Asn Pro Pro Pro Gly
785                 790                 795                 800

Ile Glu Asp Asn Arg Gln Cys Ala Leu Cys Leu Thr Tyr Gly Asp Asp
                805                 810                 815
Ser Ala Asn Asp Ala Gly Arg Leu Leu Tyr Ile Gly Gln Asn Glu Trp
            820                 825                 830
Thr His Val Asn Cys Ala Leu Trp Ser Ala Glu Val Phe Glu Asp Asp
        835                 840                 845
Asp Gly Ser Leu Lys Asn Val His Met Ala Val Ile Arg Gly Lys Gln
    850                 855                 860
Leu Arg Cys Glu Phe Cys Gln Lys Pro Gly Ala Thr Val Gly Cys Cys
865                 870                 875                 880
Leu Thr Ser Cys Thr Ser Asn Tyr His Phe Met Cys Ser Arg Ala Lys
                885                 890                 895
Asn Cys Val Phe Leu Asp Asp Lys Lys Val Tyr Cys Gln Arg His Arg
            900                 905                 910
Asp Leu Ile Lys Gly Glu Val Val Pro Glu Asn Gly Phe Glu Val Phe
        915                 920                 925
Arg Arg Val Phe Val Asp Phe Glu Gly Ile Ser Leu Arg Arg Lys Phe
    930                 935                 940
Leu Asn Gly Leu Glu Pro Glu Asn Ile His Met Met Ile Gly Ser Met
945                 950                 955                 960
Thr Ile Asp Cys Leu Gly Ile Leu Asn Asp Leu Ser Asp Cys Glu Asp
                965                 970                 975
Lys Leu Phe Pro Ile Gly Tyr Gln Cys Ser Arg Val Tyr Trp Ser Thr
            980                 985                 990
Thr Asp Ala Arg Lys Arg Cys Val  Tyr Thr Cys Lys Ile  Val Glu Cys
        995                 1000                1005
Arg Pro  Pro Val Val Glu Pro  Asp Ile Asn Ser Thr  Val Glu His
    1010                1015                1020
Asp Glu  Asn Arg Thr Ile Ala  His Ser Pro Thr Ser  Phe Thr Glu
    1025                1030                1035
Ser Ser  Ser Lys Glu Ser Gln  Asn Thr Ala Glu Ile  Ile Ser Pro
    1040                1045                1050
Pro Ser  Pro Asp Arg Pro Pro  His Ser Gln Thr Ser  Gly Ser Cys
    1055                1060                1065
Tyr Tyr  His Val Ile Ser Lys  Val Pro Arg Ile Arg  Thr Pro Ser
    1070                1075                1080
Tyr Ser  Pro Thr Gln Arg Ser  Pro Gly Cys Arg Pro  Leu Pro Ser
    1085                1090                1095
Ala Gly  Ser Pro Thr Pro Thr  Thr His Glu Ile Val  Thr Val Gly
    1100                1105                1110
Asp Pro  Leu Leu Ser Ser Gly  Leu Arg Ser Ile Gly  Ser Arg Arg
    1115                1120                1125
His Ser  Thr Ser Ser Leu Ser  Pro Gln Arg Ser Lys  Leu Arg Ile
    1130                1135                1140
Met Ser  Pro Met Arg Thr Gly  Asn Thr Tyr Ser Arg  Asn Asn Val
    1145                1150                1155
Ser Ser  Val Ser Thr Thr Gly  Thr Ala Thr Asp Leu  Glu Ser Ser
    1160                1165                1170
Ala Lys  Val Val Asp His Val  Leu Gly Pro Leu Asn  Ser Ser Thr
    1175                1180                1185
Ser Leu  Gly Gln Asn Thr Ser  Thr Ser Ser Asn Leu  Gln Arg Thr
    1190                1195                1200
Val Val  Thr Val Gly Asn Lys  Asn Ser His Leu Asp  Gly Ser Ser
    1205                1210                1215
Ser Ser  Glu Met Lys Gln Ser  Ser Ala Ser Asp Leu  Val Ser Lys
    1220                1225                1230
Ser Ser  Ser Leu Lys Gly Glu  Lys Thr Lys Val Leu  Ser Ser Lys
    1235                1240                1245
Ser Ser  Glu Gly Ser Ala His  Asn Val Ala Tyr Pro  Gly Ile Pro
    1250                1255                1260
Lys Leu  Ala Pro Gln Val His  Asn Thr Thr Ser Arg  Glu Leu Asn
    1265                1270                1275
Val Ser  Lys Ile Gly Ser Phe  Ala Glu Pro Ser Ser  Val Ser Phe
    1280                1285                1290
Ser Ser  Lys Glu Ala Leu Ser  Phe Pro His Leu His  Leu Arg Gly
    1295                1300                1305
Gln Arg  Asn Asp Arg Asp Gln  His Thr Asp Ser Thr  Gln Ser Ala
    1310                1315                1320
Asn Ser  Ser Pro Asp Glu Asp  Thr Glu Val Lys Thr  Leu Lys Leu
    1325                1330                1335
Ser Gly  Met Ser Asn Arg Ser  Ser Ile Ile Asn Glu  His Met Gly
    1340                1345                1350
Ser Ser  Ser Arg Asp Arg Arg  Gln Lys Gly Lys Lys  Ser Cys Lys

1355                    1360                    1365
Glu Thr  Phe Lys Glu Lys His  Ser Ser Lys Ser Phe  Leu Glu Pro
    1370                    1375                    1380
Gly Gln  Val Thr Thr Gly Glu  Glu Gly Asn Leu Lys  Pro Glu Phe
    1385                    1390                    1395
Met Asp  Glu Val Leu Thr Pro  Glu Tyr Met Gly Gln  Arg Pro Cys
    1400                    1405                    1410
Asn Asn  Val Ser Ser Asp Lys  Ile Gly Asp Lys Gly  Leu Ser Met
    1415                    1420                    1425
Pro Gly  Val Pro Lys Ala Pro  Pro Met Gln Val Glu  Gly Ser Ala
    1430                    1435                    1440
Lys Glu  Leu Gln Ala Pro Arg  Lys Arg Thr Val Lys  Val Thr Leu
    1445                    1450                    1455
Thr Pro  Leu Lys Met Glu Asn  Glu Ser Gln Ser Lys  Asn Ala Leu
    1460                    1465                    1470
Lys Glu  Ser Ser Pro Ala Ser  Pro Leu Gln Ile Glu  Ser Thr Ser
    1475                    1480                    1485
Pro Thr  Glu Pro Ile Ser Ala  Ser Glu Asn Pro Gly  Asp Gly Pro
    1490                    1495                    1500
Val Ala  Gln Pro Ser Pro Asn  Asn Thr Ser Cys Gln  Asp Ser Gln
    1505                    1510                    1515
Ser Asn  Asn Tyr Gln Asn Leu  Pro Val Gln Asp Arg  Asn Leu Met
    1520                    1525                    1530
Leu Pro  Asp Gly Pro Lys Pro  Gln Glu Asp Gly Ser  Phe Lys Arg
    1535                    1540                    1545
Arg Tyr  Pro Arg Arg Ser Ala  Arg Ala Arg Ser Asn  Met Phe Phe
    1550                    1555                    1560
Gly Leu  Thr Pro Leu Tyr Gly  Val Arg Ser Tyr Gly  Glu Glu Asp
    1565                    1570                    1575
Ile Pro  Phe Tyr Ser Ser Ser  Thr Gly Lys Lys Arg  Gly Lys Arg
    1580                    1585                    1590
Ser Ala  Glu Gly Gln Val Asp  Gly Ala Asp Asp Leu  Ser Thr Ser
    1595                    1600                    1605
Asp Glu  Asp Asp Leu Tyr Tyr  Tyr Asn Phe Thr Arg  Thr Val Ile
    1610                    1615                    1620
Ser Ser  Gly Gly Glu Glu Arg  Leu Ala Ser His Asn  Leu Phe Arg
    1625                    1630                    1635
Glu Glu  Glu Gln Cys Asp Leu  Pro Lys Ile Ser Gln  Leu Asp Gly
    1640                    1645                    1650
Val Asp  Asp Gly Thr Glu Ser  Asp Thr Ser Val Thr  Ala Thr Thr
    1655                    1660                    1665
Arg Lys  Ser Ser Gln Ile Pro  Lys Arg Asn Gly Lys  Glu Asn Gly
    1670                    1675                    1680
Thr Glu  Asn Leu Lys Ile Asp  Arg Pro Glu Asp Ala  Gly Glu Lys
    1685                    1690                    1695
Glu His  Val Thr Lys Ser Ser  Val Gly His Lys Asn  Glu Pro Lys
    1700                    1705                    1710
Met Asp  Asn Cys His Ser Val  Ser Arg Val Lys Thr  Gln Gly Gln
    1715                    1720                    1725
Asp Ser  Leu Glu Ala Gln Leu  Ser Ser Leu Glu Ser  Ser Arg Arg
    1730                    1735                    1740
Val His  Thr Ser Thr Pro Ser  Asp Lys Asn Leu Leu  Asp Thr Tyr
    1745                    1750                    1755
Asn Thr  Glu Leu Leu Lys Ser  Asp Ser Asp Asn Asn  Asn Ser Asp
    1760                    1765                    1770
Asp Cys  Gly Asn Ile Leu Pro  Ser Asp Ile Met Asp  Phe Val Leu
    1775                    1780                    1785
Lys Asn  Thr Pro Ser Met Gln  Ala Leu Gly Glu Ser  Pro Glu Ser
    1790                    1795                    1800
Ser Ser  Ser Glu Leu Leu Asn  Leu Gly Glu Gly Leu  Gly Leu Asp
    1805                    1810                    1815
Ser Asn  Arg Glu Lys Asp Met  Gly Leu Phe Glu Val  Phe Ser Gln
    1820                    1825                    1830
Gln Leu  Pro Thr Thr Glu Pro  Val Asp Ser Ser Val  Ser Ser Ser
    1835                    1840                    1845
Ile Ser  Ala Glu Glu Gln Phe  Glu Leu Pro Leu Glu  Leu Pro Ser
    1850                    1855                    1860
Asp Leu  Ser Val Leu Thr Thr  Arg Ser Pro Thr Val  Pro Ser Gln
    1865                    1870                    1875
Asn Pro  Ser Arg Leu Ala Val  Ile Ser Asp Ser Gly  Glu Lys Arg
    1880                    1885                    1890
Val Thr  Ile Thr Glu Lys Ser  Val Ala Ser Ser Glu  Ser Asp Pro
    1895                    1900                    1905

Ala Leu Leu Ser Pro Gly Val Asp Pro Thr Pro Glu Gly His Met
    1910                1915                1920
Thr Pro Asp His Phe Ile Gln Gly His Met Asp Ala Asp His Ile
    1925                1930                1935
Ser Ser Pro Pro Cys Gly Ser Val Glu Gln Gly His Gly Asn Asn
    1940                1945                1950
Gln Asp Leu Thr Arg Asn Ser Ser Thr Pro Gly Leu Gln Val Pro
    1955                1960                1965
Val Ser Pro Thr Val Pro Ile Gln Asn Gln Lys Tyr Val Pro Asn
    1970                1975                1980
Ser Thr Asp Ser Pro Gly Pro Ser Gln Ile Ser Asn Ala Ala Val
    1985                1990                1995
Gln Thr Thr Pro Pro His Leu Lys Pro Ala Thr Glu Lys Leu Ile
    2000                2005                2010
Val Val Asn Gln Asn Met Gln Pro Leu Tyr Val Leu Gln Thr Leu
    2015                2020                2025
Pro Asn Gly Val Thr Gln Lys Ile Gln Leu Thr Ser Ser Val Ser
    2030                2035                2040
Ser Thr Pro Ser Val Met Glu Thr Asn Thr Ser Val Leu Gly Pro
    2045                2050                2055
Met Gly Gly Gly Leu Thr Leu Thr Thr Gly Leu Asn Pro Ser Leu
    2060                2065                2070
Pro Thr Ser Gln Ser Leu Phe Pro Ser Ala Ser Lys Gly Leu Leu
    2075                2080                2085
Pro Met Ser His His Gln His Leu His Ser Phe Pro Ala Ala Thr
    2090                2095                2100
Gln Ser Ser Phe Pro Pro Asn Ile Ser Asn Pro Pro Ser Gly Leu
    2105                2110                2115
Leu Ile Gly Val Gln Pro Pro Pro Asp Pro Gln Leu Leu Val Ser
    2120                2125                2130
Glu Ser Ser Gln Arg Thr Asp Leu Ser Thr Thr Val Ala Thr Pro
    2135                2140                2145
Ser Ser Gly Leu Lys Lys Arg Pro Ile Ser Arg Leu Gln Thr Arg
    2150                2155                2160
Lys Asn Lys Lys Leu Ala Pro Ser Ser Thr Pro Ser Asn Ile Ala
    2165                2170                2175
Pro Ser Asp Val Val Ser Asn Met Thr Leu Ile Asn Phe Thr Pro
    2180                2185                2190
Ser Gln Leu Pro Asn His Pro Ser Leu Leu Asp Leu Gly Ser Leu
    2195                2200                2205
Asn Thr Ser Ser His Arg Thr Val Pro Asn Ile Ile Lys Arg Ser
    2210                2215                2220
Lys Ser Ser Ile Met Tyr Phe Glu Pro Ala Pro Leu Leu Pro Gln
    2225                2230                2235
Ser Val Gly Gly Thr Ala Ala Thr Ala Ala Gly Thr Ser Thr Ile
    2240                2245                2250
Ser Gln Asp Thr Ser His Leu Thr Ser Gly Ser Val Ser Gly Leu
    2255                2260                2265
Ala Ser Ser Ser Ser Val Leu Asn Val Val Ser Met Gln Thr Thr
    2270                2275                2280
Thr Thr Pro Thr Ser Ser Ala Ser Val Pro Gly His Val Thr Leu
    2285                2290                2295
Thr Asn Pro Arg Leu Leu Gly Thr Pro Asp Ile Gly Ser Ile Ser
    2300                2305                2310
Asn Leu Leu Ile Lys Ala Ser Gln Gln Ser Leu Gly Ile Gln Asp
    2315                2320                2325
Gln Pro Val Ala Leu Pro Pro Ser Ser Gly Met Phe Pro Gln Leu
    2330                2335                2340
Gly Thr Ser Gln Thr Pro Ser Thr Ala Ala Ile Thr Ala Ala Ser
    2345                2350                2355
Ser Ile Cys Val Leu Pro Ser Thr Gln Thr Thr Gly Ile Thr Ala
    2360                2365                2370
Ala Ser Pro Ser Gly Glu Ala Asp Glu His Tyr Gln Leu Gln His
    2375                2380                2385
Val Asn Gln Leu Leu Ala Ser Lys Thr Gly Ile His Ser Ser Gln
    2390                2395                2400
Arg Asp Leu Asp Ser Ala Ser Gly Pro Gln Val Ser Asn Phe Thr
    2405                2410                2415
Gln Thr Val Asp Ala Pro Asn Ser Met Gly Leu Glu Gln Asn Lys
    2420                2425                2430
Ala Leu Ser Ser Ala Val Gln Ala Ser Pro Thr Ser Pro Gly Gly
    2435                2440                2445
Ser Pro Ser Ser Pro Ser Ser Gly Gln Arg Ser Ala Ser Pro Ser

2450                2455                2460
Val Pro  Gly Pro Thr Lys Pro  Lys Pro Lys Thr Lys  Arg Phe Gln
    2465                2470                2475
Leu Pro  Leu Asp Lys Gly Asn  Gly Lys Lys His Lys  Val Ser His
    2480                2485                2490
Leu Arg  Thr Ser Ser Ser Glu  Ala His Ile Pro Asp  Gln Glu Thr
    2495                2500                2505
Thr Ser  Leu Thr Ser Gly Thr  Gly Thr Pro Gly Ala  Glu Ala Glu
    2510                2515                2520
Gln Gln  Asp Thr Ala Ser Val  Glu Gln Ser Ser Gln  Lys Glu Cys
    2525                2530                2535
Gly Gln  Pro Ala Gly Gln Val  Ala Val Leu Pro Glu  Val Gln Val
    2540                2545                2550
Thr Gln  Asn Pro Ala Asn Glu  Gln Glu Ser Ala Glu  Pro Lys Thr
    2555                2560                2565
Val Glu  Glu Glu Glu Ser Asn  Phe Ser Ser Pro Leu  Met Leu Trp
    2570                2575                2580
Leu Gln  Gln Glu Gln Lys Arg  Lys Glu Ser Ile Thr  Glu Lys Lys
    2585                2590                2595
Pro Lys  Lys Gly Leu Val Phe  Glu Ile Ser Ser Asp  Asp Gly Phe
    2600                2605                2610
Gln Ile  Cys Ala Glu Ser Ile  Glu Asp Ala Trp Lys  Ser Leu Thr
    2615                2620                2625
Asp Lys  Val Gln Glu Ala Arg  Ser Asn Ala Arg Leu  Lys Gln Leu
    2630                2635                2640
Ser Phe  Ala Gly Val Asn Gly  Leu Arg Met Leu Gly  Ile Leu His
    2645                2650                2655
Asp Ala  Val Val Phe Leu Ile  Glu Gln Leu Ser Gly  Ala Lys His
    2660                2665                2670
Cys Arg  Asn Tyr Lys Phe Arg  Phe His Lys Pro Glu  Glu Ala Asn
    2675                2680                2685
Glu Pro  Pro Leu Asn Pro His  Gly Ser Ala Arg Ala  Glu Val His
    2690                2695                2700
Leu Arg  Glu Val Ser Ile
    2705


<210>  76
<211>  2492
<212>  PRT
<213>  Homo sapiens
<220>
<221>  misc_feature
<223>  CDS de la séquence GS-N24: 216...7694 ( GS-P24)

<220>
<221>  misc_feature
<223>  nom: hélicase et ATPase dépendante de l'ADN (ATRX)

<400>  76
Met Thr Ala Glu Pro Met Ser Glu Ser Lys Leu Asn Thr Leu Val Gln
1               5                   10                  15
Lys Leu His Asp Phe Leu Ala His Ser Ser Glu Glu Ser Glu Glu Thr
            20                  25                  30
Ser Ser Pro Pro Arg Leu Ala Met Asn Gln Asn Thr Asp Lys Ile Ser
        35                  40                  45
Gly Ser Gly Ser Asn Ser Asp Met Met Glu Asn Ser Lys Glu Glu Gly
    50                  55                  60
Thr Ser Ser Ser Glu Lys Ser Lys Ser Ser Gly Ser Ser Arg Ser Lys
65                  70                  75                  80
Arg Lys Pro Ser Ile Val Thr Lys Tyr Val Glu Ser Asp Asp Glu Lys
                85                  90                  95
Pro Leu Asp Asp Glu Thr Val Asn Glu Asp Ala Ser Asn Glu Asn Ser
            100                 105                 110
Glu Asn Asp Ile Thr Met Gln Ser Leu Pro Lys Gly Thr Val Ile Val
        115                 120                 125
Gln Pro Glu Pro Val Leu Asn Glu Asp Lys Asp Asp Phe Lys Gly Pro
    130                 135                 140
Glu Phe Arg Ser Arg Ser Lys Met Lys Thr Glu Asn Leu Lys Lys Arg
145                 150                 155                 160
Gly Glu Asp Gly Leu His Gly Ile Val Ser Cys Thr Ala Cys Gly Gln
                165                 170                 175
Gln Val Asn His Phe Gln Lys Asp Ser Ile Tyr Arg His Pro Ser Leu

180                 185                 190
Gln Val Leu Ile Cys Lys Asn Cys Phe Lys Tyr Tyr Met Ser Asp Asp
        195                 200                 205
Ile Ser Arg Asp Ser Asp Gly Met Asp Glu Gln Cys Arg Trp Cys Ala
    210                 215                 220
Glu Gly Gly Asn Leu Ile Cys Cys Asp Phe Cys His Asn Ala Phe Cys
225                 230                 235                 240
Lys Lys Cys Ile Leu Arg Asn Leu Gly Arg Lys Glu Leu Ser Thr Ile
                245                 250                 255
Met Asp Glu Asn Asn Gln Trp Tyr Cys Tyr Ile Cys His Pro Glu Pro
            260                 265                 270
Leu Leu Asp Leu Val Thr Ala Cys Asn Ser Val Phe Glu Asn Leu Glu
        275                 280                 285
Gln Leu Leu Gln Gln Asn Lys Lys Lys Ile Lys Val Asp Ser Glu Lys
    290                 295                 300
Ser Asn Lys Val Tyr Glu His Thr Ser Arg Phe Ser Pro Lys Lys Thr
305                 310                 315                 320
Ser Ser Asn Cys Asn Gly Glu Glu Lys Lys Leu Asp Asp Ser Cys Ser
                325                 330                 335
Gly Ser Val Thr Tyr Ser Tyr Ser Ala Leu Ile Val Pro Lys Glu Met
            340                 345                 350
Ile Lys Lys Ala Lys Lys Leu Ile Glu Thr Thr Ala Asn Met Asn Ser
        355                 360                 365
Ser Tyr Val Lys Phe Leu Lys Gln Ala Thr Asp Asn Ser Glu Ile Ser
    370                 375                 380
Ser Ala Thr Lys Leu Arg Gln Leu Lys Ala Phe Lys Ser Val Leu Ala
385                 390                 395                 400
Asp Ile Lys Lys Ala His Leu Ala Leu Glu Glu Asp Leu Asn Ser Glu
                405                 410                 415
Phe Arg Ala Met Asp Ala Val Asn Lys Glu Lys Asn Thr Lys Glu His
            420                 425                 430
Lys Val Ile Asp Ala Lys Phe Glu Thr Lys Ala Arg Lys Gly Glu Lys
        435                 440                 445
Pro Cys Ala Leu Glu Lys Lys Asp Ile Ser Lys Ser Glu Ala Lys Leu
    450                 455                 460
Ser Arg Lys Gln Val Asp Ser Glu His Met His Gln Asn Val Pro Thr
465                 470                 475                 480
Glu Glu Gln Arg Thr Asn Lys Ser Thr Gly Gly Glu His Lys Lys Ser
                485                 490                 495
Asp Arg Lys Glu Glu Pro Gln Tyr Glu Pro Ala Asn Thr Ser Glu Asp
            500                 505                 510
Leu Asp Met Asp Ile Val Ser Val Pro Ser Ser Val Pro Glu Asp Ile
        515                 520                 525
Phe Glu Asn Leu Glu Thr Ala Met Glu Val Gln Ser Ser Val Asp His
    530                 535                 540
Gln Gly Asp Gly Ser Ser Gly Thr Glu Gln Glu Val Glu Ser Ser Ser
545                 550                 555                 560
Val Lys Leu Asn Ile Ser Ser Lys Asp Asn Arg Gly Gly Ile Lys Ser
                565                 570                 575
Lys Thr Thr Ala Lys Val Thr Lys Glu Leu Tyr Val Lys Leu Thr Pro
            580                 585                 590
Val Ser Leu Ser Asn Ser Pro Ile Lys Gly Ala Asp Cys Gln Glu Val
        595                 600                 605
Pro Gln Asp Lys Asp Gly Tyr Lys Ser Cys Gly Leu Asn Pro Lys Leu
    610                 615                 620
Glu Lys Cys Gly Leu Gly Gln Glu Asn Ser Asp Asn Glu His Leu Val
625                 630                 635                 640
Glu Asn Glu Val Ser Leu Leu Leu Glu Glu Ser Asp Leu Arg Arg Ser
                645                 650                 655
Pro Arg Val Lys Thr Thr Pro Leu Arg Arg Pro Thr Glu Thr Asn Pro
            660                 665                 670
Val Thr Ser Asn Ser Asp Glu Glu Cys Asn Glu Thr Val Lys Glu Lys
        675                 680                 685
Gln Lys Leu Ser Val Pro Val Arg Lys Lys Asp Lys Arg Asn Ser Ser
    690                 695                 700
Asp Ser Ala Ile Asp Asn Pro Lys Pro Asn Lys Leu Pro Lys Ser Lys
705                 710                 715                 720
Gln Ser Glu Thr Val Asp Gln Asn Ser Asp Ser Asp Glu Met Leu Ala
                725                 730                 735
Ile Leu Lys Glu Val Ser Arg Met Ser His Ser Ser Ser Ser Asp Thr
            740                 745                 750
Asp Ile Asn Glu Ile His Thr Asn His Lys Thr Leu Tyr Asp Leu Lys
        755                 760                 765

Thr Gln Ala Gly Lys Asp Asp Lys Gly Lys Arg Lys Arg Lys Ser Ser
        770                 775                 780
Thr Ser Gly Ser Asp Phe Asp Thr Lys Lys Gly Lys Ser Ala Lys Ser
785                 790                 795                 800
Ser Ile Ile Ser Lys Lys Lys Arg Gln Thr Gln Ser Glu Ser Ser Asn
                805                 810                 815
Tyr Asp Ser Glu Leu Glu Lys Glu Ile Lys Ser Met Ser Lys Ile Gly
            820                 825                 830
Ala Ala Arg Thr Thr Lys Lys Arg Ile Pro Asn Thr Lys Asp Phe Asp
        835                 840                 845
Ser Ser Glu Asp Glu Lys His Ser Lys Lys Gly Met Asp Asn Gln Gly
    850                 855                 860
His Lys Asn Leu Lys Thr Ser Gln Glu Gly Ser Ser Asp Asp Ala Glu
865                 870                 875                 880
Arg Lys Gln Glu Arg Glu Thr Phe Ser Ser Ala Glu Gly Thr Val Asp
                885                 890                 895
Lys Asp Thr Thr Ile Met Glu Leu Arg Asp Arg Leu Pro Lys Lys Gln
            900                 905                 910
Gln Ala Ser Ala Ser Thr Asp Gly Val Asp Lys Leu Ser Gly Lys Glu
        915                 920                 925
Gln Ser Phe Thr Ser Leu Glu Val Arg Lys Val Ala Glu Thr Lys Glu
    930                 935                 940
Lys Ser Lys His Leu Lys Thr Lys Thr Cys Lys Lys Val Gln Asp Gly
945                 950                 955                 960
Leu Ser Asp Ile Ala Glu Lys Phe Leu Lys Lys Asp Gln Ser Asp Glu
                965                 970                 975
Thr Ser Glu Asp Asp Lys Lys Gln Ser Lys Lys Gly Thr Glu Glu Lys
            980                 985                 990
Lys Lys Pro Ser Asp Phe Lys Lys  Lys Val Ile Lys Met  Glu Gln Gln
        995                 1000                 1005
Tyr Glu  Ser Ser Ser Asp Gly  Thr Glu Lys Leu Pro  Glu Arg Glu
    1010                 1015                 1020
Glu Ile  Cys His Phe Pro Lys  Gly Ile Lys Gln Ile  Lys Asn Gly
    1025                 1030                 1035
Thr Thr  Asp Gly Glu Lys Lys  Ser Lys Lys Ile Arg  Asp Lys Thr
    1040                 1045                 1050
Ser Lys  Lys Lys Asp Glu Leu  Ser Asp Tyr Ala Glu  Lys Ser Thr
    1055                 1060                 1065
Gly Lys  Gly Asp Ser Cys Asp  Ser Ser Glu Asp Lys  Lys Ser Lys
    1070                 1075                 1080
Asn Gly  Ala Tyr Gly Arg Glu  Lys Lys Arg Cys Lys  Leu Leu Gly
    1085                 1090                 1095
Lys Ser  Ser Arg Lys Arg Gln  Asp Cys Ser Ser Ser  Asp Thr Glu
    1100                 1105                 1110
Lys Tyr  Ser Met Lys Glu Asp  Gly Cys Asn Ser Ser  Asp Lys Arg
    1115                 1120                 1125
Leu Lys  Arg Ile Glu Leu Arg  Glu Arg Arg Asn Leu  Ser Ser Lys
    1130                 1135                 1140
Arg Asn  Thr Lys Glu Ile Gln  Ser Gly Ser Ser Ser  Ser Asp Ala
    1145                 1150                 1155
Glu Glu  Ser Ser Glu Asp Asn  Lys Lys Lys Lys Gln  Arg Thr Ser
    1160                 1165                 1170
Ser Lys  Lys Lys Ala Val Ile  Val Lys Glu Lys Lys  Arg Asn Ser
    1175                 1180                 1185
Leu Arg  Thr Ser Thr Lys Arg  Lys Gln Ala Asp Ile  Thr Ser Ser
    1190                 1195                 1200
Ser Ser  Ser Asp Ile Glu Asp  Asp Asp Gln Asn Ser  Ile Gly Glu
    1205                 1210                 1215
Gly Ser  Ser Asp Glu Gln Lys  Ile Lys Pro Val Thr  Glu Asn Leu
    1220                 1225                 1230
Val Leu  Ser Ser His Thr Gly  Phe Cys Gln Ser Ser  Gly Asp Glu
    1235                 1240                 1245
Ala Leu  Ser Lys Ser Val Pro  Val Thr Val Asp Asp  Asp Asp Asp
    1250                 1255                 1260
Asp Asn  Asp Pro Glu Asn Arg  Ile Ala Lys Lys Met  Leu Leu Glu
    1265                 1270                 1275
Glu Ile  Lys Ala Asn Leu Ser  Ser Asp Glu Asp Gly  Ser Ser Asp
    1280                 1285                 1290
Asp Glu  Pro Glu Glu Gly Lys  Lys Arg Thr Gly Lys  Gln Asn Glu
    1295                 1300                 1305
Glu Asn  Pro Gly Asp Glu Glu  Ala Lys Asn Gln Val  Asn Ser Glu
    1310                 1315                 1320
Ser Asp  Ser Asp Ser Glu Glu  Ser Lys Lys Pro Arg  Tyr Arg His

1325                    1330                    1335
Arg Leu  Leu Arg His Lys Leu  Thr Val Ser Asp Gly  Glu Ser Gly
    1340                    1345                    1350
Glu Glu  Lys Lys Thr Lys Pro  Lys Glu His Lys Glu  Val Lys Gly
    1355                    1360                    1365
Arg Asn  Arg Arg Lys Val Ser  Ser Glu Asp Ser Glu  Asp Ser Asp
    1370                    1375                    1380
Phe Gln  Glu Ser Gly Val Ser  Glu Glu Val Ser Glu  Ser Glu Asp
    1385                    1390                    1395
Glu Gln  Arg Pro Arg Thr Arg  Ser Ala Lys Lys Ala  Glu Leu Glu
    1400                    1405                    1410
Glu Asn  Gln Arg Ser Tyr Lys  Gln Lys Lys Lys Arg  Arg Arg Ile
    1415                    1420                    1425
Lys Val  Gln Glu Asp Ser Ser  Ser Glu Asn Lys Ser  Asn Ser Glu
    1430                    1435                    1440
Glu Glu  Glu Glu Glu Lys Glu  Glu Glu Glu Glu Glu  Glu Glu Glu
    1445                    1450                    1455
Glu Glu  Glu Glu Glu Glu Asp  Glu Asn Asp Asp Ser  Lys Ser Pro
    1460                    1465                    1470
Gly Lys  Gly Arg Lys Lys Ile  Arg Lys Ile Leu Lys  Asp Asp Lys
    1475                    1480                    1485
Leu Arg  Thr Glu Thr Gln Asn  Ala Leu Lys Glu Glu  Glu Glu Arg
    1490                    1495                    1500
Arg Lys  Arg Ile Ala Glu Arg  Glu Arg Glu Arg Glu  Lys Leu Arg
    1505                    1510                    1515
Glu Val  Ile Glu Ile Glu Asp  Ala Ser Pro Thr Lys  Cys Pro Ile
    1520                    1525                    1530
Thr Thr  Lys Leu Val Leu Asp  Glu Asp Glu Glu Thr  Lys Glu Pro
    1535                    1540                    1545
Leu Val  Gln Val His Arg Asn  Met Val Ile Lys Leu  Lys Pro His
    1550                    1555                    1560
Gln Val  Asp Gly Val Gln Phe  Met Trp Asp Cys Cys  Cys Glu Ser
    1565                    1570                    1575
Val Lys  Lys Thr Lys Lys Ser  Pro Gly Ser Gly Cys  Ile Leu Ala
    1580                    1585                    1590
His Cys  Met Gly Leu Gly Lys  Thr Leu Gln Val Val  Ser Phe Leu
    1595                    1600                    1605
His Thr  Val Leu Leu Cys Asp  Lys Leu Asp Phe Ser  Thr Ala Leu
    1610                    1615                    1620
Val Val  Cys Pro Leu Asn Thr  Ala Leu Asn Trp Met  Asn Glu Phe
    1625                    1630                    1635
Glu Lys  Trp Gln Glu Gly Leu  Lys Asp Asp Glu Lys  Leu Glu Val
    1640                    1645                    1650
Ser Glu  Leu Ala Thr Val Lys  Arg Pro Gln Glu Arg  Ser Tyr Met
    1655                    1660                    1665
Leu Gln  Arg Trp Gln Glu Asp  Gly Gly Val Met Ile  Ile Gly Tyr
    1670                    1675                    1680
Glu Met  Tyr Arg Asn Leu Ala  Gln Gly Arg Asn Val  Lys Ser Arg
    1685                    1690                    1695
Lys Leu  Lys Glu Ile Phe Asn  Lys Ala Leu Val Asp  Pro Gly Pro
    1700                    1705                    1710
Asp Phe  Val Val Cys Asp Glu  Gly His Ile Leu Lys  Asn Glu Ala
    1715                    1720                    1725
Ser Ala  Val Ser Lys Ala Met  Asn Ser Ile Arg Ser  Arg Arg Arg
    1730                    1735                    1740
Ile Ile  Leu Thr Gly Thr Pro  Leu Gln Asn Asn Leu  Ile Glu Tyr
    1745                    1750                    1755
His Cys  Met Val Asn Phe Ile  Lys Glu Asn Leu Leu  Gly Ser Ile
    1760                    1765                    1770
Lys Glu  Phe Arg Asn Arg Phe  Ile Asn Pro Ile Gln  Asn Gly Gln
    1775                    1780                    1785
Cys Ala  Asp Ser Thr Met Val  Asp Val Arg Val Met  Lys Lys Arg
    1790                    1795                    1800
Ala His  Ile Leu Tyr Glu Met  Leu Ala Gly Cys Val  Gln Arg Lys
    1805                    1810                    1815
Asp Tyr  Thr Ala Leu Thr Lys  Phe Leu Pro Pro Lys  His Glu Tyr
    1820                    1825                    1830
Val Leu  Ala Val Arg Met Thr  Ser Ile Gln Cys Lys  Leu Tyr Gln
    1835                    1840                    1845
Tyr Tyr  Leu Asp His Leu Thr  Gly Val Gly Asn Asn  Ser Glu Gly
    1850                    1855                    1860
Gly Arg  Gly Lys Ala Gly Ala  Lys Leu Phe Gln Asp  Phe Gln Met
    1865                    1870                    1875

Leu Ser Arg Ile Trp Thr His Pro Trp Cys Leu Gln Leu Asp Tyr
    1880                1885                1890
Ile Ser Lys Glu Asn Lys Gly Tyr Phe Asp Glu Asp Ser Met Asp
    1895                1900                1905
Glu Phe Ile Ala Ser Asp Ser Asp Glu Thr Ser Met Ser Leu Ser
    1910                1915                1920
Ser Asp Asp Tyr Thr Lys Lys Lys Lys Lys Gly Lys Lys Gly Lys
    1925                1930                1935
Lys Asp Ser Ser Ser Ser Gly Ser Gly Ser Asp Asn Asp Val Glu
    1940                1945                1950
Val Ile Lys Val Trp Asn Ser Arg Ser Arg Gly Gly Gly Glu Gly
    1955                1960                1965
Asn Val Asp Glu Thr Gly Asn Asn Pro Ser Val Ser Leu Lys Leu
    1970                1975                1980
Glu Glu Ser Lys Ala Thr Ser Ser Ser Asn Pro Ser Ser Pro Ala
    1985                1990                1995
Pro Asp Trp Tyr Lys Asp Phe Val Thr Asp Ala Asp Ala Glu Val
    2000                2005                2010
Leu Glu His Ser Gly Lys Met Val Leu Leu Phe Glu Ile Leu Arg
    2015                2020                2025
Met Ala Glu Glu Ile Gly Asp Lys Val Leu Val Phe Ser Gln Ser
    2030                2035                2040
Leu Ile Ser Leu Asp Leu Ile Glu Asp Phe Leu Glu Leu Ala Ser
    2045                2050                2055
Arg Glu Lys Thr Glu Asp Lys Asp Lys Pro Leu Ile Tyr Lys Gly
    2060                2065                2070
Glu Gly Lys Trp Leu Arg Asn Ile Asp Tyr Tyr Arg Leu Asp Gly
    2075                2080                2085
Ser Thr Thr Ala Gln Ser Arg Lys Lys Trp Ala Glu Glu Phe Asn
    2090                2095                2100
Asp Glu Thr Asn Val Arg Gly Arg Leu Phe Ile Ile Ser Thr Lys
    2105                2110                2115
Ala Gly Ser Leu Gly Ile Asn Leu Val Ala Ala Asn Arg Val Ile
    2120                2125                2130
Ile Phe Asp Ala Ser Trp Asn Pro Ser Tyr Asp Ile Gln Ser Ile
    2135                2140                2145
Phe Arg Val Tyr Arg Phe Gly Gln Thr Lys Pro Val Tyr Val Tyr
    2150                2155                2160
Arg Phe Leu Ala Gln Gly Thr Met Glu Asp Lys Ile Tyr Asp Arg
    2165                2170                2175
Gln Val Thr Lys Gln Ser Leu Ser Phe Arg Val Val Asp Gln Gln
    2180                2185                2190
Gln Val Glu Arg His Phe Thr Met Asn Glu Leu Thr Glu Leu Tyr
    2195                2200                2205
Thr Phe Glu Pro Asp Leu Leu Asp Asp Pro Asn Ser Glu Lys Lys
    2210                2215                2220
Lys Lys Arg Asp Thr Pro Met Leu Pro Lys Asp Thr Ile Leu Ala
    2225                2230                2235
Glu Leu Leu Gln Ile His Lys Glu His Ile Val Gly Tyr His Glu
    2240                2245                2250
His Asp Ser Leu Leu Asp His Lys Glu Glu Glu Glu Leu Thr Glu
    2255                2260                2265
Glu Glu Arg Lys Ala Ala Trp Ala Glu Tyr Glu Ala Glu Lys Lys
    2270                2275                2280
Gly Leu Thr Met Arg Phe Asn Ile Pro Thr Gly Thr Asn Leu Pro
    2285                2290                2295
Pro Val Ser Phe Asn Ser Gln Thr Pro Tyr Ile Pro Phe Asn Leu
    2300                2305                2310
Gly Ala Leu Ser Ala Met Ser Asn Gln Gln Leu Glu Asp Leu Ile
    2315                2320                2325
Asn Gln Gly Arg Glu Lys Val Val Glu Ala Thr Asn Ser Val Thr
    2330                2335                2340
Ala Val Arg Ile Gln Pro Leu Glu Asp Ile Ile Ser Ala Val Trp
    2345                2350                2355
Lys Glu Asn Met Asn Leu Ser Glu Ala Gln Val Gln Ala Leu Ala
    2360                2365                2370
Leu Ser Arg Gln Ala Ser Gln Glu Leu Asp Val Lys Arg Arg Glu
    2375                2380                2385
Ala Ile Tyr Asn Asp Val Leu Thr Lys Gln Gln Met Leu Ile Ser
    2390                2395                2400
Cys Val Gln Arg Ile Leu Met Asn Arg Arg Leu Gln Gln Gln Tyr
    2405                2410                2415
Asn Gln Gln Gln Gln Gln Met Thr Tyr Gln Gln Ala Thr Leu

2420                2425                2430
Gly His Leu Met Met Pro Lys Pro Pro Asn Leu Ile Met Asn Pro
    2435                2440                2445
Ser Asn Tyr Gln Gln Ile Asp Met Arg Gly Met Tyr Gln Pro Val
    2450                2455                2460
Ala Gly Gly Met Gln Pro Pro Pro Leu Gln Arg Ala Pro Pro Pro
    2465                2470                2475
Met Arg Ser Lys Asn Pro Gly Pro Ser Gln Gly Lys Ser Met
    2480                2485                2490


<210>  77
<211>  337
<212>  PRT
<213>  Homo sapiens
<220>
<221>  misc_feature
<223>  CDS de la séquence GS-N25:28...1041 ( GS-P25)

<220>
<221>  misc_feature
<223>  nom: transporteur de solutés, famille 35 (transporteur de CMP-aci
       de sialique), membre 1

<400>  77
Met Ala Ala Pro Arg Asp Asn Val Thr Leu Leu Phe Lys Leu Tyr Cys
1               5                   10                  15
Leu Ala Val Met Thr Leu Met Ala Ala Val Tyr Thr Ile Ala Leu Arg
            20                  25                  30
Tyr Thr Arg Thr Ser Asp Lys Glu Leu Tyr Phe Ser Thr Thr Ala Val
        35                  40                  45
Cys Ile Thr Glu Val Ile Lys Leu Leu Leu Ser Val Gly Ile Leu Ala
    50                  55                  60
Lys Glu Thr Gly Ser Leu Gly Arg Phe Lys Ala Ser Leu Arg Glu Asn
65                  70                  75                  80
Val Leu Gly Ser Pro Lys Glu Leu Leu Lys Leu Ser Val Pro Ser Leu
                85                  90                  95
Val Tyr Ala Val Gln Asn Asn Met Ala Phe Leu Ala Leu Ser Asn Leu
            100                 105                 110
Asp Ala Ala Val Tyr Gln Val Thr Tyr Gln Leu Lys Ile Pro Cys Thr
        115                 120                 125
Ala Leu Cys Thr Val Leu Met Leu Asn Arg Thr Leu Ser Lys Leu Gln
    130                 135                 140
Trp Val Ser Val Phe Met Leu Cys Ala Gly Val Thr Leu Val Gln Trp
145                 150                 155                 160
Lys Pro Ala Gln Ala Thr Lys Val Val Val Glu Gln Asn Pro Leu Leu
                165                 170                 175
Gly Phe Gly Ala Ile Ala Ile Ala Val Leu Cys Ser Gly Phe Ala Gly
            180                 185                 190
Val Tyr Phe Glu Lys Val Leu Lys Ser Ser Asp Thr Ser Leu Trp Val
        195                 200                 205
Arg Asn Ile Gln Met Tyr Leu Ser Gly Ile Ile Val Thr Leu Ala Gly
    210                 215                 220
Val Tyr Leu Ser Asp Gly Ala Glu Ile Lys Glu Lys Gly Phe Phe Tyr
225                 230                 235                 240
Gly Tyr Thr Tyr Tyr Val Trp Phe Val Ile Phe Leu Ala Ser Val Gly
                245                 250                 255
Gly Leu Tyr Thr Ser Val Val Val Lys Tyr Thr Asp Asn Ile Met Lys
            260                 265                 270
Gly Phe Ser Ala Ala Ala Ala Ile Val Leu Ser Thr Ile Ala Ser Val
        275                 280                 285
Met Leu Phe Gly Leu Gln Ile Thr Leu Thr Phe Ala Leu Gly Thr Leu
    290                 295                 300
Leu Val Cys Val Ser Ile Tyr Leu Tyr Gly Leu Pro Arg Gln Asp Thr
305                 310                 315                 320
Thr Ser Ile Gln Gln Gly Glu Thr Ala Ser Lys Glu Arg Val Ile Gly
                325                 330                 335
Val


<210>  78
<211>  982

<212>  PRT
<213>  Homo sapiens
<220>
<221>  misc_feature
<223>  CDS de la séquence GS-N26: 323...3271 ( GS-P26)

<220>
<221>  misc_feature
<223>  nom: cbl-b

<400>  78

<210>  79
<211>  114
<212>  PRT
<213>  Homo sapiens
<220>
<221>  misc_feature
<223>  CDS de la séquence GS-N27: 107...451( GS-P27)

<220>

<221>  misc_feature
<223>  nom: histone H2A.F/Z variant (H2AV)

<400>  79
Met Ala Gly Gly Lys Ala Gly Lys Asp Ser Gly Lys Ala Lys Ala Lys
1               5                   10                  15
Ala Val Ser Arg Ser Gln Arg Ala Gly Leu Gln Phe Pro Val Gly Arg
            20                  25                  30
Ile His Arg His Leu Lys Thr Arg Thr Thr Ser His Gly Arg Val Gly
        35                  40                  45
Ala Thr Ala Ala Val Tyr Ser Ala Ala Ile Leu Glu Tyr Leu Thr Ala
    50                  55                  60
Glu Val Leu Glu Leu Ala Gly Asn Ala Ser Lys Asp Leu Lys Val Lys
65                  70                  75                  80
Arg Ile Thr Pro Arg His Leu Gln Leu Ala Ile Arg Gly Asp Glu Glu
                85                  90                  95
Leu Asp Ser Leu Ile Lys Ala Thr Ile Ala Gly Gly Glu Lys Arg Arg
            100                 105                 110
Cys Ser



<210>  80
<211>  215
<212>  PRT
<213>  Homo sapiens
<220>
<221>  misc_feature
<223>  CDS de la séquence GS-N28: 341...1988( GS-P28)

<220>
<221>  misc_feature
<223>  nom: caseine kinase 2,  polypeptide beta (CSNK2B),

<400>  80
Met Ser Ser Ser Glu Glu Val Ser Trp Ile Ser Trp Phe Cys Gly Leu
1               5                   10                  15
Arg Gly Asn Glu Phe Phe Cys Glu Val Asp Glu Asp Tyr Ile Gln Asp
            20                  25                  30
Lys Phe Asn Leu Thr Gly Leu Asn Glu Gln Val Pro His Tyr Arg Gln
        35                  40                  45
Ala Leu Asp Met Ile Leu Asp Leu Glu Pro Asp Glu Glu Leu Glu Asp
    50                  55                  60
Asn Pro Asn Gln Ser Asp Leu Ile Glu Gln Ala Ala Glu Met Leu Tyr
65                  70                  75                  80
Gly Leu Ile His Ala Arg Tyr Ile Leu Thr Asn Arg Gly Ile Ala Gln
                85                  90                  95
Met Leu Glu Lys Tyr Gln Gln Gly Asp Phe Gly Tyr Cys Pro Arg Val
            100                 105                 110
Tyr Cys Glu Asn Gln Pro Met Leu Pro Ile Gly Leu Ser Asp Ile Pro
        115                 120                 125
Gly Glu Ala Met Val Lys Leu Tyr Cys Pro Lys Cys Met Asp Val Tyr
    130                 135                 140
Thr Pro Lys Ser Ser Arg His His His Thr Asp Gly Ala Tyr Phe Gly
145                 150                 155                 160
Thr Gly Phe Pro His Met Leu Phe Met Val His Pro Glu Tyr Arg Pro
                165                 170                 175
Lys Arg Pro Ala Asn Gln Phe Val Pro Arg Leu Tyr Gly Phe Lys Ile
            180                 185                 190
His Pro Met Ala Tyr Gln Leu Gln Leu Gln Ala Ala Ser Asn Phe Lys
        195                 200                 205
Ser Pro Val Lys Thr Ile Arg
    210                 215


<210>  81
<211>  5636
<212>  PRT
<213>  Homo sapiens
<220>
<221>  MISC_FEATURE
<223>  CDS de la séquence GS-N29: 230...17140 ( GS-P29)

<220>
<221>  misc_feature
<223>  nom: hemicentine

<220>
<221>  misc_feature
<222>  (3041)..(3041)
<223>  X est un résidu inconnu

<220>
<221>  misc_feature
<222>  (3003)..(3033)
<223>  X est un résidu inconnu

<220>
<221>  misc_feature
<222>  (3367)..(3367)
<223>  X est un résidu inconnu

<400>  81
Met Ile Ser Trp Glu Val Val His Thr Val Phe Leu Phe Ala Leu Leu
1               5                   10                  15
Tyr Ser Ser Leu Ala Gln Asp Ala Ser Pro Gln Ser Glu Ile Arg Ala
            20                  25                  30
Glu Glu Phe Pro Glu Gly Ala Ser Thr Leu Ala Phe Val Phe Asp Val
        35                  40                  45
Thr Gly Ser Met Tyr Asp Asp Leu Val Gln Val Ile Glu Gly Ala Ser
    50                  55                  60
Lys Ile Leu Glu Thr Ser Leu Lys Arg Pro Lys Arg Pro Leu Phe Asn
65                  70                  75                  80
Phe Ala Leu Val Pro Phe His Asp Pro Glu Ile Gly Pro Val Thr Ile
                85                  90                  95
Thr Thr Asp Pro Lys Lys Phe Gln Tyr Glu Leu Arg Glu Leu Tyr Val
            100                 105                 110
Gln Gly Gly Gly Asp Cys Pro Glu Met Ser Ile Gly Ala Ile Lys Ile
        115                 120                 125
Ala Leu Glu Ile Ser Leu Pro Gly Ser Phe Ile Tyr Val Phe Thr Asp
    130                 135                 140
Ala Arg Ser Lys Asp Tyr Arg Leu Thr His Glu Val Leu Gln Leu Ile
145                 150                 155                 160
Gln Gln Lys Gln Ser Gln Val Val Phe Val Leu Thr Gly Asp Cys Asp
                165                 170                 175
Asp Arg Thr His Ile Gly Tyr Lys Val Tyr Glu Glu Ile Ala Ser Thr
            180                 185                 190
Ser Ser Gly Gln Val Phe His Leu Asp Lys Lys Gln Val Asn Glu Val
        195                 200                 205
Leu Lys Trp Val Glu Glu Ala Val Gln Ala Ser Lys Val His Leu Leu
    210                 215                 220
Ser Thr Asp His Leu Glu Gln Ala Val Asn Thr Trp Arg Ile Pro Phe
225                 230                 235                 240
Asp Pro Ser Leu Lys Glu Val Thr Val Ser Leu Ser Gly Pro Ser Pro
                245                 250                 255
Met Ile Glu Ile Arg Asn Pro Leu Gly Lys Leu Ile Lys Lys Gly Phe
            260                 265                 270
Gly Leu His Glu Leu Leu Asn Ile His Asn Ser Ala Lys Val Val Asn
        275                 280                 285
Val Lys Glu Pro Glu Ala Gly Met Trp Thr Val Lys Thr Ser Ser Ser
    290                 295                 300
Gly Arg His Ser Val Arg Ile Thr Gly Leu Ser Thr Ile Asp Phe Arg
305                 310                 315                 320
Ala Gly Phe Ser Arg Lys Pro Thr Leu Asp Phe Lys Lys Thr Val Ser
                325                 330                 335
Arg Pro Val Gln Gly Ile Pro Thr Tyr Val Leu Leu Asn Thr Ser Gly
            340                 345                 350
Ile Ser Thr Pro Ala Arg Ile Asp Leu Leu Glu Leu Leu Ser Ile Ser
        355                 360                 365
Gly Ser Ser Leu Lys Thr Ile Pro Val Lys Tyr Tyr Pro His Arg Lys
    370                 375                 380
Pro Tyr Gly Ile Trp Asn Ile Ser Asp Phe Val Pro Pro Asn Glu Ala
385                 390                 395                 400
Phe Phe Leu Lys Val Thr Gly Tyr Asp Lys Asp Asp Tyr Leu Phe Gln
                405                 410                 415
Arg Val Ser Ser Val Ser Phe Ser Ser Ile Val Pro Asp Ala Pro Lys

420                 425                 430
Val Thr Met Pro Glu Lys Thr Pro Gly Tyr Tyr Leu Gln Pro Gly Gln
        435                 440                 445
Ile Pro Cys Ser Val Asp Ser Leu Leu Pro Phe Thr Leu Ser Phe Val
    450                 455                 460
Arg Asn Gly Val Thr Leu Gly Val Asp Gln Tyr Leu Lys Glu Ser Ala
465                 470                 475                 480
Ser Val Ser Leu Asp Ile Ala Lys Val Thr Leu Ser Asp Glu Gly Phe
                485                 490                 495
Tyr Glu Cys Ile Ala Val Ser Ser Ala Gly Thr Gly Arg Ala Gln Thr
            500                 505                 510
Phe Phe Asp Val Ser Glu Pro Pro Pro Val Ile Gln Val Pro Asn Asn
        515                 520                 525
Val Thr Val Thr Pro Gly Glu Arg Ala Val Leu Thr Cys Leu Ile Ile
    530                 535                 540
Ser Ala Val Asp Tyr Asn Leu Thr Trp Gln Arg Asn Asp Arg Asp Val
545                 550                 555                 560
Arg Leu Ala Glu Pro Ala Arg Ile Arg Thr Leu Ala Asn Leu Ser Leu
                565                 570                 575
Glu Leu Lys Ser Val Lys Phe Asn Asp Ala Gly Glu Tyr His Cys Met
            580                 585                 590
Val Ser Ser Glu Gly Gly Ser Ser Ala Ala Ser Val Phe Leu Thr Val
        595                 600                 605
Gln Glu Pro Pro Lys Val Thr Val Met Pro Lys Asn Gln Ser Phe Thr
    610                 615                 620
Gly Gly Ser Glu Val Ser Ile Met Cys Ser Ala Thr Gly Tyr Pro Lys
625                 630                 635                 640
Pro Lys Ile Ala Trp Thr Val Asn Asp Met Phe Ile Val Gly Ser His
                645                 650                 655
Arg Tyr Arg Met Thr Ser Asp Gly Thr Leu Phe Ile Lys Asn Ala Ala
            660                 665                 670
Pro Lys Asp Ala Gly Ile Tyr Gly Cys Leu Ala Ser Asn Ser Ala Gly
        675                 680                 685
Thr Asp Lys Gln Asn Ser Thr Leu Arg Tyr Ile Glu Ala Pro Lys Leu
    690                 695                 700
Met Val Val Gln Ser Glu Leu Leu Val Ala Leu Gly Asp Ile Thr Val
705                 710                 715                 720
Met Glu Cys Lys Thr Ser Gly Ile Pro Pro Pro Gln Val Lys Trp Phe
                725                 730                 735
Lys Gly Asp Leu Glu Leu Arg Pro Ser Thr Phe Leu Ile Ile Asp Pro
            740                 745                 750
Leu Leu Gly Leu Leu Lys Ile Gln Glu Thr Gln Asp Leu Asp Ala Gly
        755                 760                 765
Asp Tyr Thr Cys Val Ala Ile Asn Glu Ala Gly Arg Ala Thr Gly Lys
    770                 775                 780
Ile Thr Leu Asp Val Gly Ser Pro Pro Val Phe Ile Gln Glu Pro Ala
785                 790                 795                 800
Asp Val Ser Met Glu Ile Gly Ser Asn Val Thr Leu Pro Cys Tyr Val
                805                 810                 815
Gln Gly Tyr Pro Glu Pro Thr Ile Lys Trp Arg Arg Leu Asp Asn Met
            820                 825                 830
Pro Ile Phe Ser Arg Pro Phe Ser Val Ser Ser Ile Ser Gln Leu Arg
        835                 840                 845
Thr Gly Ala Leu Phe Ile Leu Asn Leu Trp Ala Ser Asp Lys Gly Thr
    850                 855                 860
Tyr Ile Cys Glu Ala Glu Asn Gln Phe Gly Lys Ile Gln Ser Glu Thr
865                 870                 875                 880
Thr Val Thr Val Thr Gly Leu Val Ala Pro Leu Ile Gly Ile Ser Pro
                885                 890                 895
Ser Val Ala Asn Val Ile Glu Gly Gln Gln Leu Thr Leu Pro Cys Thr
            900                 905                 910
Leu Leu Ala Gly Asn Pro Ile Pro Glu Arg Arg Trp Ile Lys Asn Ser
        915                 920                 925
Ala Met Leu Leu Gln Asn Pro Tyr Ile Thr Val Arg Ser Asp Gly Ser
    930                 935                 940
Leu His Ile Glu Arg Val Gln Leu Gln Asp Gly Gly Glu Tyr Thr Cys
945                 950                 955                 960
Val Ala Ser Asn Val Ala Gly Thr Asn Asn Lys Thr Thr Ser Val Val
                965                 970                 975
Val His Val Leu Pro Thr Ile Gln His Gly Gln Gln Ile Leu Ser Thr
            980                 985                 990
Ile Glu Gly Ile Pro Val Thr Leu  Pro Cys Lys Ala Ser  Gly Asn Pro
        995                 1000                1005

Lys Pro Ser Val Ile Trp Ser Lys Lys Gly Glu Leu Ile Ser Thr
    1010                1015                1020
Ser Ser Ala Lys Phe Ser Ala Gly Ala Asp Gly Ser Leu Tyr Val
    1025                1030                1035
Val Ser Pro Glu Gly Glu Glu Ser Gly Glu Tyr Val Cys Thr Ala
    1040                1045                1050
Thr Asn Thr Ala Gly Tyr Ala Lys Arg Lys Val Gln Leu Thr Val
    1055                1060                1065
Tyr Val Arg Pro Arg Val Phe Gly Asp Leu Arg Gly Leu Ser Gln
    1070                1075                1080
Asp Lys Pro Val Glu Ile Ser Val Leu Ala Gly Glu Glu Val Thr
    1085                1090                1095
Leu Pro Cys Glu Val Lys Ser Leu Pro Pro Pro Ile Ile Thr Trp
    1100                1105                1110
Ala Lys Glu Thr Gln Leu Ile Ser Pro Phe Ser Pro Arg His Thr
    1115                1120                1125
Phe Leu Pro Ser Gly Ser Met Lys Ile Thr Glu Thr Arg Thr Ser
    1130                1135                1140
Asp Ser Gly Met Tyr Leu Cys Val Ala Thr Asn Ile Ala Gly Asn
    1145                1150                1155
Val Thr Gln Ala Val Lys Leu Asn Val His Val Pro Pro Lys Ile
    1160                1165                1170
Gln Arg Gly Pro Lys His Leu Lys Val Gln Val Gly Gln Arg Val
    1175                1180                1185
Asp Ile Pro Cys Asn Ala Gln Gly Thr Pro Leu Pro Val Ile Thr
    1190                1195                1200
Trp Ser Lys Gly Gly Ser Thr Met Leu Val Asp Gly Glu His His
    1205                1210                1215
Val Ser Asn Pro Asp Gly Thr Leu Ser Ile Asp Gln Ala Thr Pro
    1220                1225                1230
Ser Asp Ala Gly Ile Tyr Thr Cys Val Ala Thr Asn Ile Ala Gly
    1235                1240                1245
Thr Asp Glu Thr Glu Ile Thr Leu His Val Gln Glu Pro Pro Thr
    1250                1255                1260
Val Glu Asp Leu Glu Pro Pro Tyr Asn Thr Thr Phe Gln Glu Arg
    1265                1270                1275
Val Ala Asn Gln Arg Ile Glu Phe Pro Cys Pro Ala Lys Gly Thr
    1280                1285                1290
Pro Lys Pro Thr Ile Lys Trp Leu His Asn Gly Arg Glu Leu Thr
    1295                1300                1305
Gly Arg Glu Pro Gly Ile Ser Ile Leu Glu Glu Gly Thr Leu Leu
    1310                1315                1320
Val Ile Ala Ser Val Thr Pro Tyr Asp Asn Gly Glu Tyr Ile Cys
    1325                1330                1335
Val Ala Val Asn Glu Ala Gly Thr Thr Glu Arg Lys Tyr Asn Leu
    1340                1345                1350
Lys Val His Val Pro Pro Val Ile Lys Asp Lys Glu Gln Val Ser
    1355                1360                1365
Asn Val Ser Val Leu Leu Asn Gln Leu Thr Asn Leu Phe Cys Glu
    1370                1375                1380
Val Glu Gly Thr Pro Ser Pro Ile Ile Met Trp Tyr Lys Asp Asn
    1385                1390                1395
Val Gln Val Thr Glu Ser Ser Thr Ile Gln Thr Val Asn Asn Gly
    1400                1405                1410
Lys Ile Leu Lys Leu Phe Arg Ala Thr Pro Glu Asp Ala Gly Arg
    1415                1420                1425
Tyr Ser Cys Lys Ala Ile Asn Ile Ala Gly Thr Ser Gln Lys Tyr
    1430                1435                1440
Phe Asn Ile Asp Val Leu Val Pro Pro Thr Ile Ile Gly Thr Asn
    1445                1450                1455
Phe Pro Lys Glu Val Ser Val Val Leu Asn Arg Asp Val Ala Leu
    1460                1465                1470
Glu Cys Gln Val Lys Gly Thr Pro Phe Pro Asp Ile His Trp Phe
    1475                1480                1485
Lys Asp Gly Lys Pro Leu Phe Leu Gly Asp Pro Asn Val Glu Leu
    1490                1495                1500
Leu Asp Arg Gly Gln Val Leu His Leu Lys Asn Ala Arg Arg Asn
    1505                1510                1515
Asp Lys Gly Arg Tyr Gln Cys Thr Val Ser Asn Ala Ala Gly Lys
    1520                1525                1530
Gln Ala Lys Asp Ile Lys Leu Thr Ile Tyr Ile Pro Pro Ser Ile
    1535                1540                1545
Lys Gly Gly Asn Val Thr Thr Asp Ile Ser Val Leu Ile Asn Ser

1550                 1555                 1560
Leu Ile Lys Leu Glu Cys Lys Thr Arg Gly Leu Pro Met Pro Ala
    1565                 1570                 1575
Ile Thr Trp Tyr Lys Asp Gly Gln Pro Ile Met Ser Ser Ser Gln
    1580                 1585                 1590
Ala Leu Tyr Ile Asp Lys Gly Gln Tyr Leu His Ile Pro Arg Ala
    1595                 1600                 1605
Gln Val Ser Asp Ser Ala Thr Tyr Thr Cys His Val Ala Asn Val
    1610                 1615                 1620
Ala Gly Thr Ala Glu Lys Ser Phe His Val Asp Val Tyr Val Pro
    1625                 1630                 1635
Pro Met Ile Glu Gly Asn Leu Ala Thr Pro Leu Asn Lys Gln Val
    1640                 1645                 1650
Val Ile Ala His Ser Leu Thr Leu Glu Cys Asn Ala Ala Gly Asn
    1655                 1660                 1665
Pro Ser Pro Ile Leu Thr Trp Leu Lys Asp Gly Val Pro Val Lys
    1670                 1675                 1680
Ala Asn Asp Asn Phe Arg Ile Glu Ala Gly Gly Lys Lys Leu Glu
    1685                 1690                 1695
Ile Met Ser Ala Gln Glu Ile Asp Arg Gly Gln Tyr Ile Cys Val
    1700                 1705                 1710
Ala Thr Ser Val Ala Gly Glu Lys Glu Ile Lys Tyr Glu Val Asp
    1715                 1720                 1725
Val Leu Val Pro Pro Ala Ile Glu Gly Gly Asp Glu Thr Ser Tyr
    1730                 1735                 1740
Phe Ile Val Met Val Asn Asn Leu Leu Glu Leu Asp Cys His Val
    1745                 1750                 1755
Thr Gly Ser Pro Pro Pro Thr Ile Met Trp Leu Lys Asp Gly Gln
    1760                 1765                 1770
Leu Ile Asp Glu Arg Asp Gly Phe Lys Ile Leu Leu Asn Gly Arg
    1775                 1780                 1785
Lys Leu Val Ile Ala Gln Ala Gln Val Ser Asn Thr Gly Leu Tyr
    1790                 1795                 1800
Arg Cys Met Ala Ala Asn Thr Ala Gly Asp His Lys Lys Glu Phe
    1805                 1810                 1815
Glu Val Thr Val His Val Pro Pro Thr Ile Lys Ser Ser Gly Leu
    1820                 1825                 1830
Ser Glu Arg Val Val Val Lys Tyr Lys Pro Val Ala Leu Gln Cys
    1835                 1840                 1845
Ile Ala Asn Gly Ile Pro Asn Pro Ser Ile Thr Trp Leu Lys Asp
    1850                 1855                 1860
Asp Gln Pro Val Asn Thr Ala Gln Gly Asn Leu Lys Ile Gln Ser
    1865                 1870                 1875
Ser Gly Arg Val Leu Gln Ile Ala Lys Thr Leu Leu Glu Asp Ala
    1880                 1885                 1890
Gly Arg Tyr Thr Cys Val Ala Thr Asn Ala Ala Gly Glu Thr Gln
    1895                 1900                 1905
Gln His Ile Gln Leu His Val His Glu Pro Pro Ser Leu Glu Asp
    1910                 1915                 1920
Ala Gly Lys Met Leu Asn Glu Thr Val Leu Val Ser Asn Pro Val
    1925                 1930                 1935
Gln Leu Glu Cys Lys Ala Ala Gly Asn Pro Val Pro Val Ile Thr
    1940                 1945                 1950
Trp Tyr Lys Asp Asn Cys Leu Leu Ser Gly Ser Thr Ser Met Thr
    1955                 1960                 1965
Phe Leu Asn Arg Gly Gln Ile Ile Asp Ile Glu Ser Ala Gln Ile
    1970                 1975                 1980
Ser Asp Ala Gly Ile Tyr Lys Cys Val Ala Ile Asn Ser Ala Gly
    1985                 1990                 1995
Ala Thr Glu Leu Phe Tyr Ser Leu Gln Val His Val Ala Pro Ser
    2000                 2005                 2010
Ile Ser Gly Ser Asn Asn Met Val Ala Val Val Val Asn Asn Pro
    2015                 2020                 2025
Val Arg Leu Glu Cys Glu Ala Arg Gly Ile Pro Ala Pro Ser Leu
    2030                 2035                 2040
Thr Trp Leu Lys Asp Gly Ser Pro Val Ser Ser Phe Ser Asn Gly
    2045                 2050                 2055
Leu Gln Val Leu Ser Gly Gly Arg Ile Leu Ala Leu Thr Ser Thr
    2060                 2065                 2070
Gln Ile Ser Asp Thr Gly Arg Tyr Thr Cys Val Ala Val Asn Ala
    2075                 2080                 2085
Ala Gly Glu Lys Gln Arg Asp Ile Asp Leu Arg Val Tyr Val Pro
    2090                 2095                 2100

Pro Asn Ile Met Gly Glu Glu Gln Asn Val Ser Val Leu Ile Ser
    2105                2110                2115
Gln Ala Val Glu Leu Leu Cys Gln Ser Asp Ala Ile Pro Pro Pro
    2120                2125                2130
Thr Leu Thr Trp Leu Lys Asp Gly His Pro Leu Leu Lys Lys Pro
    2135                2140                2145
Gly Leu Ser Ile Ser Glu Asn Arg Ser Val Leu Lys Ile Glu Asp
    2150                2155                2160
Ala Gln Val Gln Asp Thr Gly Arg Tyr Thr Cys Glu Ala Thr Asn
    2165                2170                2175
Val Ala Gly Lys Thr Glu Lys Lys Asn Tyr Asn Val Asn Ile Trp
    2180                2185                2190
Val Pro Pro Asn Ile Gly Gly Ser Asp Glu Leu Thr Gln Leu Thr
    2195                2200                2205
Val Ile Glu Gly Asn Leu Ile Ser Leu Leu Cys Glu Ser Ser Gly
    2210                2215                2220
Ile Pro Pro Pro Asn Leu Ile Trp Lys Lys Lys Gly Ser Pro Val
    2225                2230                2235
Leu Thr Asp Ser Met Gly Arg Val Arg Ile Leu Ser Gly Gly Arg
    2240                2245                2250
Gln Leu Gln Ile Ser Ile Ala Glu Lys Ser Asp Ala Ala Leu Tyr
    2255                2260                2265
Ser Cys Val Ala Ser Asn Val Ala Gly Thr Ala Lys Lys Glu Tyr
    2270                2275                2280
Asn Leu Gln Val Tyr Ile Arg Pro Thr Ile Thr Asn Ser Gly Ser
    2285                2290                2295
His Pro Thr Glu Ile Ile Val Thr Arg Gly Lys Ser Ile Ser Leu
    2300                2305                2310
Glu Cys Glu Val Gln Gly Ile Pro Pro Pro Thr Val Thr Trp Met
    2315                2320                2325
Lys Asp Gly His Pro Leu Ile Lys Ala Lys Gly Val Glu Ile Leu
    2330                2335                2340
Asp Glu Gly His Ile Leu Gln Leu Lys Asn Ile His Val Ser Asp
    2345                2350                2355
Thr Gly Arg Tyr Val Cys Val Ala Val Asn Val Ala Gly Met Thr
    2360                2365                2370
Asp Lys Lys Tyr Asp Leu Ser Val His Ala Pro Pro Ser Ile Ile
    2375                2380                2385
Gly Asn His Arg Ser Pro Glu Asn Ile Ser Val Val Glu Lys Asn
    2390                2395                2400
Ser Val Ser Leu Thr Cys Glu Ala Ser Gly Ile Pro Leu Pro Ser
    2405                2410                2415
Thr Thr Trp Phe Lys Asp Gly Trp Pro Val Ser Leu Ser Asn Ser
    2420                2425                2430
Val Arg Ile Leu Ser Gly Gly Arg Met Leu Arg Leu Met Gln Thr
    2435                2440                2445
Thr Met Glu Asp Ala Gly Gln Tyr Thr Cys Val Val Arg Asn Ala
    2450                2455                2460
Ala Gly Glu Glu Arg Lys Ile Phe Gly Leu Ser Val Leu Val Pro
    2465                2470                2475
Pro His Ile Val Gly Glu Asn Thr Leu Glu Asp Val Lys Val Lys
    2480                2485                2490
Glu Lys Gln Ser Val Thr Leu Thr Cys Glu Val Thr Gly Asn Pro
    2495                2500                2505
Val Pro Glu Ile Thr Trp His Lys Asp Gly Gln Pro Leu Gln Glu
    2510                2515                2520
Asp Glu Ala His His Ile Ile Ser Gly Gly Arg Phe Leu Gln Ile
    2525                2530                2535
Thr Asn Val Gln Val Pro His Thr Gly Arg Tyr Thr Cys Leu Ala
    2540                2545                2550
Ser Ser Pro Ala Gly His Lys Ser Arg Ser Phe Ser Leu Asn Val
    2555                2560                2565
Phe Val Ser Pro Thr Ile Ala Gly Val Gly Ser Asp Gly Asn Pro
    2570                2575                2580
Glu Asp Val Thr Val Ile Leu Asn Ser Pro Thr Ser Leu Val Cys
    2585                2590                2595
Glu Ala Tyr Ser Tyr Pro Pro Ala Thr Ile Thr Trp Phe Lys Asp
    2600                2605                2610
Gly Thr Pro Leu Glu Ser Asn Arg Asn Ile Arg Ile Leu Pro Gly
    2615                2620                2625
Gly Arg Thr Leu Gln Ile Leu Asn Ala Gln Glu Asp Asn Ala Gly
    2630                2635                2640
Arg Tyr Ser Cys Val Ala Thr Asn Glu Ala Gly Glu Met Ile Lys

2645                 2650                 2655
His Tyr Glu Val Lys Val Tyr Ile Pro Pro Ile Ile Asn Lys Gly
    2660                 2665                 2670
Asp Leu Trp Gly Pro Gly Leu Ser Pro Lys Glu Val Lys Ile Lys
    2675                 2680                 2685
Val Asn Asn Thr Leu Thr Leu Glu Cys Glu Ala Tyr Ala Ile Pro
    2690                 2695                 2700
Ser Ala Ser Leu Ser Trp Tyr Lys Asp Gly Gln Pro Leu Lys Ser
    2705                 2710                 2715
Asp Asp His Val Asn Ile Ala Ala Asn Gly His Thr Leu Gln Ile
    2720                 2725                 2730
Lys Glu Ala Gln Ile Ser Asp Thr Gly Arg Tyr Thr Cys Val Ala
    2735                 2740                 2745
Ser Asn Ile Ala Gly Glu Asp Glu Leu Asp Phe Asp Val Asn Ile
    2750                 2755                 2760
Gln Val Pro Pro Ser Phe Gln Lys Leu Trp Glu Ile Gly Asn Met
    2765                 2770                 2775
Leu Asp Thr Gly Arg Asn Gly Glu Ala Lys Asp Val Ile Ile Asn
    2780                 2785                 2790
Asn Pro Ile Ser Leu Tyr Cys Glu Thr Asn Ala Ala Pro Pro Pro
    2795                 2800                 2805
Thr Leu Thr Trp Tyr Lys Asp Gly His Pro Leu Thr Ser Ser Asp
    2810                 2815                 2820
Lys Val Leu Ile Leu Pro Gly Gly Arg Val Leu Gln Ile Pro Arg
    2825                 2830                 2835
Ala Lys Val Glu Asp Ala Gly Arg Tyr Thr Cys Val Ala Val Asn
    2840                 2845                 2850
Glu Ala Gly Glu Asp Ser Leu Gln Tyr Asp Val Arg Val Leu Val
    2855                 2860                 2865
Pro Pro Ile Ile Glu Gly Ala Asn Ser Asp Leu Pro Glu Glu Val
    2870                 2875                 2880
Thr Val Leu Val Asn Lys Ser Ala Leu Ile Glu Cys Leu Ser Ser
    2885                 2890                 2895
Gly Ser Pro Ala Pro Arg Asn Ser Trp Gln Lys Asp Gly Gln Pro
    2900                 2905                 2910
Leu Leu Glu Asp Asp His His Lys Phe Leu Ser Asn Gly Arg Ile
    2915                 2920                 2925
Leu Gln Ile Leu Asn Thr Gln Ile Thr Asp Ile Gly Arg Tyr Val
    2930                 2935                 2940
Cys Val Ala Glu Asn Thr Ala Gly Ser Ala Lys Lys Tyr Phe Asn
    2945                 2950                 2955
Leu Asn Val His Val Pro Pro Ser Val Ile Gly Pro Lys Ser Glu
    2960                 2965                 2970
Asn Leu Thr Val Val Val Asn Asn Phe Ile Ser Leu Thr Cys Glu
    2975                 2980                 2985
Val Ser Gly Phe Pro Pro Pro Asp Leu Ser Trp Leu Lys Asn Xaa
    2990                 2995                 3000
Gln Pro Ile Lys Leu Asn Thr Asn Thr Leu Ile Val Pro Gly Gly
    3005                 3010                 3015
Arg Thr Leu Gln Ile Ile Arg Ala Lys Val Ser Asp Gly Gly Glu
    3020                 3025                 3030
Tyr Thr Cys Ile Ala Ile Asn Xaa Ala Gly Glu Ser Lys Lys Lys
    3035                 3040                 3045
Phe Ser Leu Thr Val Tyr Val Pro Pro Ser Ile Lys Asp His Asp
    3050                 3055                 3060
Ser Glu Ser Leu Ser Val Val Asn Val Arg Glu Gly Thr Ser Val
    3065                 3070                 3075
Ser Leu Glu Cys Glu Ser Asn Ala Val Pro Pro Pro Val Ile Thr
    3080                 3085                 3090
Trp Tyr Lys Asn Gly Arg Met Ile Thr Glu Ser Thr His Val Glu
    3095                 3100                 3105
Ile Leu Ala Asp Gly Gln Met Leu His Ile Lys Lys Ala Glu Val
    3110                 3115                 3120
Ser Asp Thr Gly Gln Tyr Val Cys Arg Ala Ile Asn Val Ala Gly
    3125                 3130                 3135
Arg Asp Asp Lys Asn Phe His Leu Asn Val Tyr Val Pro Pro Ser
    3140                 3145                 3150
Ile Glu Gly Pro Glu Arg Glu Val Ile Val Glu Thr Ile Ser Asn
    3155                 3160                 3165
Pro Val Thr Leu Thr Cys Asp Ala Thr Gly Ile Pro Pro Pro Thr
    3170                 3175                 3180
Ile Ala Trp Leu Lys Asn Tyr Lys Arg Ile Glu Asn Ser Asp Ser
    3185                 3190                 3195

Leu Glu Val Arg Ile Leu Ser Gly Gly Ser Lys Leu Gln Ile Ala
    3200                3205                3210
Arg Ser Gln His Ser Asp Ser Gly Asn Tyr Thr Cys Ile Ala Ser
    3215                3220                3225
Asn Met Glu Gly Lys Ala Gln Lys Tyr Tyr Phe Leu Ser Ile Gln
    3230                3235                3240
Val Pro Pro Ser Val Ala Gly Ala Glu Ile Pro Ser Asp Val Ser
    3245                3250                3255
Val Leu Leu Gly Glu Asn Val Glu Leu Val Cys Asn Ala Asn Gly
    3260                3265                3270
Ile Pro Thr Pro Leu Ile Gln Trp Leu Lys Asp Gly Lys Pro Ile
    3275                3280                3285
Ala Ser Gly Glu Thr Glu Arg Ile Arg Val Ser Ala Asn Gly Ser
    3290                3295                3300
Thr Leu Asn Ile Tyr Gly Ala Leu Thr Ser Asp Thr Gly Lys Tyr
    3305                3310                3315
Thr Cys Val Ala Thr Asn Pro Ala Gly Glu Glu Asp Arg Ile Phe
    3320                3325                3330
Asn Leu Asn Val Tyr Val Thr Pro Thr Ile Arg Gly Asn Lys Asp
    3335                3340                3345
Glu Ala Glu Lys Leu Met Thr Tyr Val Asp Thr Ser Ile Asn Ile
    3350                3355                3360
Glu Cys Arg Xaa Thr Gly Thr Pro Pro Pro Gln Ile Asn Trp Leu
    3365                3370                3375
Lys Asn Gly Leu Pro Leu Pro Leu Ser Ser His Ile Arg Leu Leu
    3380                3385                3390
Ala Ala Gly Gln Val Ile Arg Ile Val Arg Ala Gln Val Ser Asp
    3395                3400                3405
Val Ala Val Tyr Thr Cys Val Ala Ser Asn Arg Ala Gly Val Asp
    3410                3415                3420
Asn Lys His Tyr Asn Leu Gln Val Phe Ala Pro Pro Asn Met Asp
    3425                3430                3435
Asn Ser Met Gly Thr Glu Glu Ile Thr Val Leu Lys Gly Ser Ser
    3440                3445                3450
Thr Ser Met Ala Cys Ile Thr Asp Gly Thr Pro Ala Pro Ser Met
    3455                3460                3465
Ala Trp Leu Arg Asp Gly Gln Pro Leu Gly Leu Asp Ala His Leu
    3470                3475                3480
Thr Val Ser Thr His Gly Met Val Leu Gln Leu Leu Lys Ala Glu
    3485                3490                3495
Thr Glu Asp Ser Gly Lys Tyr Thr Cys Ile Ala Ser Asn Glu Ala
    3500                3505                3510
Gly Glu Val Ser Lys His Phe Ile Leu Lys Val Leu Glu Pro Pro
    3515                3520                3525
His Ile Asn Gly Ser Glu Glu His Glu Glu Ile Ser Val Ile Val
    3530                3535                3540
Asn Asn Pro Leu Glu Leu Thr Cys Ile Ala Ser Gly Ile Pro Ala
    3545                3550                3555
Pro Lys Met Thr Trp Met Lys Asp Gly Arg Pro Leu Pro Gln Thr
    3560                3565                3570
Asp Gln Val Gln Thr Leu Gly Gly Gly Glu Val Leu Arg Ile Ser
    3575                3580                3585
Thr Ala Gln Val Glu Asp Thr Gly Arg Tyr Thr Cys Leu Ala Ser
    3590                3595                3600
Ser Pro Ala Gly Asp Asp Asp Lys Glu Tyr Leu Val Arg Val His
    3605                3610                3615
Val Pro Pro Asn Ile Ala Gly Thr Asp Glu Pro Arg Asp Ile Thr
    3620                3625                3630
Val Leu Arg Asn Arg Gln Val Thr Leu Glu Cys Lys Ser Asp Ala
    3635                3640                3645
Val Pro Pro Pro Val Ile Thr Trp Leu Arg Asn Gly Glu Arg Leu
    3650                3655                3660
Gln Ala Thr Pro Arg Val Arg Ile Leu Ser Gly Gly Arg Tyr Leu
    3665                3670                3675
Gln Ile Asn Asn Ala Asp Leu Gly Asp Thr Ala Asn Tyr Thr Cys
    3680                3685                3690
Val Ala Ser Asn Ile Ala Gly Lys Thr Thr Arg Glu Phe Ile Leu
    3695                3700                3705
Thr Val Asn Val Pro Pro Asn Ile Lys Gly Gly Pro Gln Ser Leu
    3710                3715                3720
Val Ile Leu Leu Asn Lys Ser Thr Val Leu Glu Cys Ile Ala Glu
    3725                3730                3735
Gly Val Pro Thr Pro Arg Ile Thr Trp Arg Lys Asp Gly Ala Val

3740                3745                3750
Leu Ala Gly Asn His Ala Arg Tyr Ser Ile Leu Glu Asn Gly Phe
    3755                3760                3765
Leu His Ile Gln Ser Ala His Val Thr Asp Thr Gly Arg Tyr Leu
    3770                3775                3780
Cys Met Ala Thr Asn Ala Ala Gly Thr Asp Arg Arg Arg Ile Asp
    3785                3790                3795
Leu Gln Val His Val Pro Pro Ser Ile Ala Pro Gly Pro Thr Asn
    3800                3805                3810
Met Thr Val Ile Val Asn Val Gln Thr Thr Leu Ala Cys Glu Ala
    3815                3820                3825
Thr Gly Ile Pro Lys Pro Ser Ile Asn Trp Arg Lys Asn Gly His
    3830                3835                3840
Leu Leu Asn Val Asp Gln Asn Gln Asn Ser Tyr Arg Leu Leu Ser
    3845                3850                3855
Ser Gly Ser Leu Val Ile Ile Ser Pro Ser Val Asp Asp Thr Ala
    3860                3865                3870
Thr Tyr Glu Cys Thr Val Thr Asn Gly Ala Gly Asp Asp Lys Arg
    3875                3880                3885
Thr Val Asp Leu Thr Val Gln Val Pro Pro Ser Ile Ala Asp Glu
    3890                3895                3900
Pro Thr Asp Phe Leu Val Thr Lys His Ala Pro Ala Val Ile Thr
    3905                3910                3915
Cys Thr Ala Ser Gly Val Pro Phe Pro Ser Ile His Trp Thr Lys
    3920                3925                3930
Asn Gly Ile Arg Leu Leu Pro Arg Gly Asp Gly Tyr Arg Ile Leu
    3935                3940                3945
Ser Ser Gly Ala Ile Glu Ile Leu Ala Thr Gln Leu Asn His Ala
    3950                3955                3960
Gly Arg Tyr Thr Cys Val Ala Arg Asn Ala Ala Gly Ser Ala His
    3965                3970                3975
Arg His Val Thr Leu His Val His Glu Pro Pro Val Ile Gln Pro
    3980                3985                3990
Gln Pro Ser Glu Leu His Val Ile Leu Asn Asn Pro Ile Leu Leu
    3995                4000                4005
Pro Cys Glu Ala Thr Gly Thr Pro Ser Pro Phe Ile Thr Trp Gln
    4010                4015                4020
Lys Glu Gly Ile Asn Val Asn Thr Ser Gly Arg Asn His Ala Val
    4025                4030                4035
Leu Pro Ser Gly Gly Leu Gln Ile Ser Arg Ala Val Arg Glu Asp
    4040                4045                4050
Ala Gly Thr Tyr Met Cys Val Ala Gln Asn Pro Ala Gly Thr Ala
    4055                4060                4065
Leu Gly Lys Ile Lys Leu Asn Val Gln Val Pro Pro Val Ile Ser
    4070                4075                4080
Pro His Leu Lys Glu Tyr Val Ile Ala Val Asp Lys Pro Ile Thr
    4085                4090                4095
Leu Ser Cys Glu Ala Asp Gly Leu Pro Pro Pro Asp Ile Thr Trp
    4100                4105                4110
His Lys Asp Gly Arg Ala Ile Val Glu Ser Ile Arg Gln Arg Val
    4115                4120                4125
Leu Ser Ser Gly Ser Leu Gln Ile Ala Phe Val Gln Pro Gly Asp
    4130                4135                4140
Ala Gly His Tyr Thr Cys Met Ala Ala Asn Val Ala Gly Ser Ser
    4145                4150                4155
Ser Thr Ser Thr Lys Leu Thr Val His Val Pro Pro Arg Ile Arg
    4160                4165                4170
Ser Thr Glu Gly His Tyr Thr Val Asn Glu Asn Ser Gln Ala Ile
    4175                4180                4185
Leu Pro Cys Val Ala Asp Gly Ile Pro Thr Pro Ala Ile Asn Trp
    4190                4195                4200
Lys Lys Asp Asn Val Leu Leu Ala Asn Leu Leu Gly Lys Tyr Thr
    4205                4210                4215
Ala Glu Pro Tyr Gly Glu Leu Ile Leu Glu Asn Val Val Leu Glu
    4220                4225                4230
Asp Ser Gly Phe Tyr Thr Cys Val Ala Asn Asn Ala Ala Gly Glu
    4235                4240                4245
Asp Thr His Thr Val Ser Leu Thr Val His Val Leu Pro Thr Phe
    4250                4255                4260
Thr Glu Leu Pro Gly Asp Val Ser Leu Asn Lys Gly Glu Gln Leu
    4265                4270                4275
Arg Leu Ser Cys Lys Ala Thr Gly Ile Pro Leu Pro Lys Leu Thr
    4280                4285                4290

Trp Thr Phe Asn Asn Asn Ile Ile Pro Ala His Phe Asp Ser Val
    4295                4300                4305
Asn Gly His Ser Glu Leu Val Ile Glu Arg Val Ser Lys Glu Asp
    4310                4315                4320
Ser Gly Thr Tyr Val Cys Thr Ala Glu Asn Ser Val Gly Phe Val
    4325                4330                4335
Lys Ala Thr Gly Phe Val Tyr Val Lys Glu Pro Pro Val Phe Lys
    4340                4345                4350
Gly Asp Tyr Pro Ser Asn Trp Ile Glu Pro Leu Gly Gly Asn Ala
    4355                4360                4365
Ile Leu Asn Cys Glu Val Lys Gly Asp Pro Thr Pro Thr Ile Gln
    4370                4375                4380
Trp Asn Arg Lys Gly Val Asp Ile Glu Ile Ser His Arg Ile Arg
    4385                4390                4395
Gln Leu Gly Asn Gly Ser Leu Ala Ile Tyr Gly Thr Val Asn Glu
    4400                4405                4410
Asp Ala Gly Asp Tyr Thr Cys Val Ala Thr Asn Glu Ala Gly Val
    4415                4420                4425
Val Glu Arg Ser Met Ser Leu Thr Leu Gln Ser Pro Pro Ile Ile
    4430                4435                4440
Thr Leu Glu Pro Val Glu Thr Val Ile Asn Ala Gly Gly Lys Ile
    4445                4450                4455
Ile Leu Asn Cys Gln Ala Thr Gly Glu Pro Gln Pro Thr Ile Thr
    4460                4465                4470
Trp Ser Arg Gln Gly His Ser Ile Ser Trp Asp Asp Arg Val Asn
    4475                4480                4485
Val Leu Ser Asn Asn Ser Leu Tyr Ile Ala Asp Ala Gln Lys Glu
    4490                4495                4500
Asp Thr Ser Glu Phe Glu Cys Val Ala Arg Asn Leu Met Gly Ser
    4505                4510                4515
Val Leu Val Arg Val Pro Val Ile Val Gln Val His Gly Gly Phe
    4520                4525                4530
Ser Gln Trp Ser Ala Trp Arg Ala Cys Ser Val Thr Cys Gly Lys
    4535                4540                4545
Gly Ile Gln Lys Arg Ser Arg Leu Cys Asn Gln Pro Leu Pro Ala
    4550                4555                4560
Asn Gly Gly Lys Pro Cys Gln Gly Ser Asp Leu Glu Met Arg Asn
    4565                4570                4575
Cys Gln Asn Lys Pro Cys Pro Val Asp Gly Ser Trp Ser Glu Trp
    4580                4585                4590
Ser Leu Trp Glu Glu Cys Thr Arg Ser Cys Gly Arg Gly Asn Gln
    4595                4600                4605
Thr Arg Thr Arg Thr Cys Asn Asn Pro Ser Val Gln His Gly Gly
    4610                4615                4620
Arg Pro Cys Glu Gly Asn Ala Val Glu Ile Ile Met Cys Asn Ile
    4625                4630                4635
Arg Pro Cys Pro Val His Gly Ala Trp Ser Ala Trp Gln Pro Trp
    4640                4645                4650
Gly Thr Cys Ser Glu Ser Cys Gly Lys Gly Thr Gln Thr Arg Ala
    4655                4660                4665
Arg Leu Cys Asn Asn Pro Pro Pro Ala Phe Gly Gly Ser Tyr Cys
    4670                4675                4680
Asp Gly Ala Glu Thr Gln Met Gln Val Cys Asn Glu Arg Asn Cys
    4685                4690                4695
Pro Ile His Gly Lys Trp Ala Thr Trp Ala Ser Trp Ser Ala Cys
    4700                4705                4710
Ser Val Ser Cys Gly Gly Gly Ala Arg Gln Arg Thr Arg Gly Cys
    4715                4720                4725
Ser Asp Pro Val Pro Gln Tyr Gly Gly Arg Lys Cys Glu Gly Ser
    4730                4735                4740
Asp Val Gln Ser Asp Phe Cys Asn Ser Asp Pro Cys Pro Thr His
    4745                4750                4755
Gly Asn Trp Ser Pro Trp Ser Gly Trp Gly Thr Cys Ser Arg Thr
    4760                4765                4770
Cys Asn Gly Gly Gln Met Arg Arg Tyr Arg Thr Cys Asp Asn Pro
    4775                4780                4785
Pro Pro Ser Asn Gly Gly Arg Ala Cys Gly Gly Pro Asp Ser Gln
    4790                4795                4800
Ile Gln Arg Cys Asn Thr Asp Met Cys Pro Val Asp Gly Ser Trp
    4805                4810                4815
Gly Ser Trp His Ser Trp Ser Gln Cys Ser Ala Ser Cys Gly Gly
    4820                4825                4830
Gly Glu Lys Thr Arg Lys Arg Leu Cys Asp His Pro Val Pro Val

4835                    4840                    4845
Lys Gly Gly Arg Pro Cys Pro Gly Asp Thr Thr Gln Val Thr Arg
    4850                    4855                    4860
Cys Asn Val Gln Ala Cys Pro Gly Gly Pro Gln Arg Ala Arg Gly
    4865                    4870                    4875
Ser Val Ile Gly Asn Ile Asn Asp Val Glu Phe Gly Ile Ala Phe
    4880                    4885                    4890
Leu Asn Ala Thr Ile Thr Asp Ser Pro Asn Ser Asp Thr Arg Ile
    4895                    4900                    4905
Ile Arg Ala Lys Ile Thr Asn Val Pro Arg Ser Leu Gly Ser Ala
    4910                    4915                    4920
Met Arg Lys Ile Val Ser Ile Leu Asn Pro Ile Tyr Trp Thr Thr
    4925                    4930                    4935
Ala Lys Glu Ile Gly Glu Ala Val Asn Gly Phe Thr Leu Thr Asn
    4940                    4945                    4950
Ala Val Phe Lys Arg Glu Thr Gln Val Glu Phe Ala Thr Gly Glu
    4955                    4960                    4965
Ile Leu Gln Met Ser His Ile Ala Arg Gly Leu Asp Ser Asp Gly
    4970                    4975                    4980
Ser Leu Leu Leu Asp Ile Val Val Ser Gly Tyr Val Leu Gln Leu
    4985                    4990                    4995
Gln Ser Pro Ala Glu Val Thr Val Lys Asp Tyr Thr Glu Asp Tyr
    5000                    5005                    5010
Ile Gln Thr Gly Pro Gly Gln Leu Tyr Ala Tyr Ser Thr Arg Leu
    5015                    5020                    5025
Phe Thr Ile Asp Gly Ile Ser Ile Pro Tyr Thr Trp Asn His Thr
    5030                    5035                    5040
Val Phe Tyr Asp Gln Ala Gln Gly Arg Met Pro Phe Leu Val Glu
    5045                    5050                    5055
Thr Leu His Ala Ser Ser Val Glu Ser Asp Tyr Asn Gln Ile Glu
    5060                    5065                    5070
Glu Thr Leu Gly Phe Lys Ile His Ala Ser Ile Ser Lys Gly Asp
    5075                    5080                    5085
Arg Ser Asn Gln Cys Pro Ser Gly Phe Thr Leu Asp Ser Val Gly
    5090                    5095                    5100
Pro Phe Cys Ala Asp Glu Asp Glu Cys Ala Ala Gly Asn Pro Cys
    5105                    5110                    5115
Ser His Ser Cys His Asn Ala Met Gly Thr Tyr Tyr Cys Ser Cys
    5120                    5125                    5130
Pro Lys Gly Leu Thr Ile Ala Ala Asp Gly Arg Thr Cys Gln Asp
    5135                    5140                    5145
Ile Asp Glu Cys Ala Leu Gly Arg His Thr Cys His Ala Gly Gln
    5150                    5155                    5160
Asp Cys Asp Asn Thr Ile Gly Ser Tyr Arg Cys Val Val Arg Cys
    5165                    5170                    5175
Gly Ser Gly Phe Arg Arg Thr Ser Asp Gly Leu Ser Cys Gln Asp
    5180                    5185                    5190
Ile Asn Glu Cys Gln Glu Ser Ser Pro Cys His Gln Arg Cys Phe
    5195                    5200                    5205
Asn Ala Ile Gly Ser Phe His Cys Gly Cys Glu Pro Gly Tyr Gln
    5210                    5215                    5220
Leu Lys Gly Arg Lys Cys Met Asp Val Asn Glu Cys Arg Gln Asn
    5225                    5230                    5235
Val Cys Arg Pro Asp Gln His Cys Lys Asn Thr Arg Gly Gly Tyr
    5240                    5245                    5250
Lys Cys Ile Asp Leu Cys Pro Asn Gly Met Thr Lys Ala Glu Asn
    5255                    5260                    5265
Gly Thr Cys Ile Asp Ile Asp Glu Cys Lys Asp Gly Thr His Gln
    5270                    5275                    5280
Cys Arg Tyr Asn Gln Ile Cys Glu Asn Thr Arg Ser Ser Tyr Arg
    5285                    5290                    5295
Cys Val Cys Pro Arg Gly Tyr Arg Ser Gln Gly Val Gly Arg Pro
    5300                    5305                    5310
Cys Met Asp Ile Asp Glu Cys Glu Gln Val Pro Lys Pro Cys Ala
    5315                    5320                    5325
His Gln Cys Ser Asn Thr Pro Gly Ser Phe Lys Cys Ile Cys Pro
    5330                    5335                    5340
Pro Gly Gln His Leu Leu Gly Asp Gly Lys Ser Cys Ala Gly Leu
    5345                    5350                    5355
Glu Arg Leu Pro Asn Tyr Gly Thr Gln Tyr Ser Ser Tyr Asn Leu
    5360                    5365                    5370
Ala Arg Phe Ser Pro Val Arg Asn Asn Tyr Gln Pro Gln Gln His
    5375                    5380                    5385

Tyr Arg Gln Tyr Ser His Leu Tyr Ser Ser Tyr Ser Glu Tyr Arg
    5390                5395                5400
Asn Ser Arg Thr Ser Leu Ser Arg Thr Arg Arg Thr Ile Arg Lys
    5405                5410                5415
Thr Cys Pro Glu Gly Ser Glu Ala Ser His Asp Thr Cys Val Asp
    5420                5425                5430
Ile Asp Glu Cys Glu Asn Thr Asp Ala Cys Gln His Glu Cys Lys
    5435                5440                5445
Asn Thr Phe Gly Ser Tyr Gln Cys Ile Cys Pro Pro Gly Tyr Gln
    5450                5455                5460
Leu Thr His Asn Gly Lys Thr Cys Gln Asp Ile Asp Glu Cys Leu
    5465                5470                5475
Glu Gln Asn Val His Cys Gly Pro Asn Arg Met Cys Phe Asn Met
    5480                5485                5490
Arg Gly Ser Tyr Gln Cys Ile Asp Thr Pro Cys Pro Pro Asn Tyr
    5495                5500                5505
Gln Arg Asp Pro Val Ser Gly Phe Cys Leu Lys Asn Cys Pro Pro
    5510                5515                5520
Asn Asp Leu Glu Cys Ala Leu Ser Pro Tyr Ala Leu Glu Tyr Lys
    5525                5530                5535
Leu Val Ser Leu Pro Phe Gly Ile Ala Thr Asn Gln Asp Leu Ile
    5540                5545                5550
Arg Leu Val Ala Tyr Thr Gln Asp Gly Val Met His Pro Arg Thr
    5555                5560                5565
Thr Phe Leu Met Val Asp Glu Glu Gln Thr Val Pro Phe Ala Leu
    5570                5575                5580
Arg Asp Glu Asn Leu Lys Gly Val Val Tyr Thr Thr Arg Pro Leu
    5585                5590                5595
Arg Glu Ala Glu Thr Tyr Arg Met Arg Val Arg Ala Ser Ser Tyr
    5600                5605                5610
Ser Ala Asn Gly Thr Ile Glu Tyr Gln Thr Thr Phe Ile Val Tyr
    5615                5620                5625
Ile Ala Val Ser Ala Tyr Pro Tyr
    5630                5635

<210> 82
<211> 1092
<212> PRT
<213> Homo sapiens
<220>
<221> misc_feature
<223> CDS de la séquence GS-N30: 123...3401 ( GS-P30)

<220>
<221> misc_feature
<223> nom: SYNE-2 ("Homo sapiens synaptic nuclei expressed gene 2")

<400> 82
Met Glu Pro Gln Leu Ala Glu Met Ile Lys Gln Phe Gln Ser Thr Val
1               5                   10                  15
Glu Thr Trp Asp Gln Cys Glu Lys Lys Ile Lys Glu Leu Lys Ser Arg
            20                  25                  30
Leu Gln Val Leu Lys Ala Gln Ser Glu Asp Pro Leu Pro Glu Leu His
        35                  40                  45
Glu Asp Leu His Asn Glu Lys Glu Leu Ile Lys Glu Leu Glu Gln Ser
    50                  55                  60
Leu Ala Ser Trp Thr Gln Asn Leu Lys Glu Leu Gln Thr Met Lys Ala
65                  70                  75                  80
Asp Leu Thr Arg His Val Leu Val Glu Asp Val Met Val Leu Lys Glu
                85                  90                  95
Gln Ile Glu His Leu His Arg Gln Trp Glu Asp Leu Cys Leu Arg Val
            100                 105                 110
Ala Ile Arg Lys Gln Glu Ile Glu Asp Arg Leu Asn Thr Trp Val Val
        115                 120                 125
Phe Asn Glu Lys Asn Lys Glu Leu Cys Ala Trp Leu Val Gln Met Glu
    130                 135                 140
Asn Lys Val Leu Gln Thr Ala Asp Ile Ser Ile Glu Glu Met Ile Glu
145                 150                 155                 160
Lys Leu Gln Lys Asp Cys Met Glu Glu Ile Asn Leu Phe Ser Glu Asn
                165                 170                 175
Lys Leu Gln Leu Lys Gln Met Gly Asp Gln Leu Ile Lys Ala Ser Asn
            180                 185                 190

Lys Ser Arg Ala Ala Glu Ile Asp Asp Lys Leu Asn Lys Ile Asn Asp
        195                 200                 205
Arg Trp Gln His Leu Phe Asp Val Ile Gly Ser Arg Val Lys Lys Leu
    210                 215                 220
Lys Glu Thr Phe Ala Phe Ile Gln Gln Leu Asp Lys Asn Met Ser Asn
225                 230                 235                 240
Leu Arg Thr Trp Leu Ala Arg Ile Glu Ser Glu Leu Ser Lys Pro Val
                245                 250                 255
Val Tyr Asp Val Cys Asp Asp Gln Glu Ile Gln Lys Arg Leu Ala Glu
            260                 265                 270
Gln Gln Asp Leu Gln Arg Asp Ile Glu Gln His Ser Ala Gly Val Glu
        275                 280                 285
Ser Val Phe Asn Ile Cys Asp Val Leu Leu His Asp Ser Asp Ala Cys
    290                 295                 300
Ala Asn Glu Thr Glu Cys Asp Ser Ile Gln Gln Thr Thr Arg Ser Leu
305                 310                 315                 320
Asp Arg Arg Trp Arg Asn Ile Cys Ala Met Ser Met Glu Arg Arg Met
                325                 330                 335
Lys Ile Glu Glu Thr Trp Arg Leu Trp Gln Lys Phe Leu Asp Asp Tyr
            340                 345                 350
Ser Arg Phe Glu Asp Trp Leu Lys Ser Ala Glu Arg Thr Ala Ala Cys
        355                 360                 365
Pro Asn Ser Ser Glu Val Leu Tyr Thr Ser Ala Lys Glu Glu Leu Lys
    370                 375                 380
Arg Phe Glu Ala Phe Gln Arg Gln Ile His Glu Arg Leu Thr Gln Leu
385                 390                 395                 400
Glu Leu Ile Asn Lys Gln Tyr Arg Arg Leu Ala Arg Glu Asn Arg Thr
                405                 410                 415
Asp Thr Ala Ser Arg Leu Lys Gln Met Val His Glu Gly Asn Gln Arg
            420                 425                 430
Trp Asp Asn Leu Gln Arg Arg Val Thr Ala Val Leu Arg Arg Leu Arg
        435                 440                 445
His Phe Thr Asn Gln Arg Glu Glu Phe Glu Gly Thr Arg Glu Ser Ile
    450                 455                 460
Leu Val Trp Leu Thr Glu Met Asp Leu Gln Leu Thr Asn Val Glu His
465                 470                 475                 480
Phe Ser Glu Ser Asp Ala Asp Asp Lys Met Arg Gln Leu Asn Gly Phe
                485                 490                 495
Gln Gln Glu Ile Thr Leu Asn Thr Asn Lys Ile Asp Gln Leu Ile Val
            500                 505                 510
Phe Gly Glu Gln Leu Ile Gln Lys Ser Glu Pro Leu Asp Ala Val Leu
        515                 520                 525
Ile Glu Asp Glu Leu Glu Glu Leu His Arg Tyr Cys Gln Glu Val Phe
    530                 535                 540
Gly Arg Val Ser Arg Phe His Arg Arg Leu Thr Ser Cys Thr Pro Gly
545                 550                 555                 560
Leu Glu Asp Glu Lys Glu Ala Ser Glu Asn Glu Thr Asp Met Glu Asp
                565                 570                 575
Pro Arg Glu Ile Gln Thr Asp Ser Trp Arg Lys Arg Gly Glu Ser Glu
            580                 585                 590
Glu Pro Ser Ser Pro Gln Ser Leu Cys His Leu Val Ala Pro Gly His
        595                 600                 605
Glu Arg Ser Gly Cys Glu Thr Pro Val Ser Val Asp Ser Ile Pro Leu
    610                 615                 620
Glu Trp Asp His Thr Gly Asp Val Gly Gly Ser Ser Ser His Glu Glu
625                 630                 635                 640
Asp Glu Glu Gly Pro Tyr Tyr Ser Ala Leu Ser Gly Lys Ser Ile Ser
                645                 650                 655
Asp Gly His Ser Trp His Val Pro Asp Ser Pro Ser Cys Pro Glu His
            660                 665                 670
His Tyr Lys Gln Met Glu Gly Asp Arg Asn Val Pro Pro Val Pro Pro
        675                 680                 685
Ala Ser Ser Thr Pro Tyr Lys Pro Pro Tyr Gly Lys Leu Leu Leu Pro
    690                 695                 700
Pro Gly Thr Asp Gly Gly Lys Glu Gly Pro Arg Val Leu Asn Gly Asn
705                 710                 715                 720
Pro Gln Gln Glu Asp Gly Gly Leu Ala Gly Ile Thr Glu Gln Gln Ser
                725                 730                 735
Gly Ala Phe Asp Arg Trp Glu Met Ile Gln Ala Gln Glu Leu His Asn
            740                 745                 750
Lys Leu Lys Ile Lys Gln Asn Leu Gln Gln Leu Asn Ser Asp Ile Ser
        755                 760                 765
Ala Ile Thr Thr Trp Leu Lys Lys Thr Glu Ala Glu Leu Glu Met Leu

770                 775                 780
Lys Met Ala Lys Pro Pro Ser Asp Ile Gln Glu Ile Glu Leu Arg Val
785                 790                 795                 800
Lys Arg Leu Gln Glu Ile Leu Lys Ala Phe Asp Thr Tyr Lys Ala Leu
                805                 810                 815
Val Val Ser Val Asn Val Ser Ser Lys Glu Phe Leu Gln Thr Glu Ser
            820                 825                 830
Pro Glu Ser Thr Glu Leu Gln Ser Arg Leu Arg Gln Leu Ser Leu Leu
        835                 840                 845
Trp Glu Ala Ala Gln Gly Ala Val Asp Ser Trp Arg Gly Gly Leu Arg
    850                 855                 860
Gln Ser Leu Met Gln Cys Gln Asp Phe His Gln Leu Ser Gln Asn Leu
865                 870                 875                 880
Leu Leu Trp Leu Ala Ser Ala Lys Asn Arg Arg Gln Lys Ala His Val
                885                 890                 895
Thr Asp Pro Lys Ala Asp Pro Arg Ala Leu Leu Glu Cys Arg Arg Glu
            900                 905                 910
Leu Met Gln Leu Glu Lys Glu Leu Val Glu Arg Gln Pro Gln Val Asp
        915                 920                 925
Met Leu Gln Glu Ile Ser Asn Ser Leu Leu Ile Lys Gly His Gly Glu
    930                 935                 940
Asp Cys Ile Glu Ala Glu Glu Lys Val His Val Ile Glu Lys Lys Leu
945                 950                 955                 960
Lys Gln Leu Arg Glu Gln Val Ser Gln Asp Leu Met Ala Leu Gln Gly
                965                 970                 975
Thr Gln Asn Pro Ala Ser Pro Leu Pro Ser Phe Asp Glu Val Asp Ser
            980                 985                 990
Gly Asp Gln Pro Pro Ala Thr Ser  Val Pro Ala Pro Arg  Ala Lys Gln
        995                 1000                1005
Phe Arg  Ala Val Arg Thr Thr  Glu Gly Glu Glu Glu  Thr Glu Ser
    1010                1015                1020
Arg Val  Pro Gly Ser Thr Arg  Pro Gln Arg Ser Phe  Leu Ser Arg
    1025                1030                1035
Val Val  Arg Ala Ala Leu Pro  Leu Gln Leu Leu Leu  Leu Leu Leu
    1040                1045                1050
Leu Leu  Leu Ala Cys Leu Leu  Pro Ser Ser Glu Glu  Asp Tyr Ser
    1055                1060                1065
Cys Thr  Gln Ala Asn Asn Phe  Ala Arg Ser Phe Tyr  Pro Met Leu
    1070                1075                1080
Arg Tyr  Thr Asn Gly Pro Pro  Pro Thr
    1085                1090


<210>  83
<211>  516
<212>  PRT
<213>  Homo sapiens
<220>
<221>  misc_feature
<223>  CDS de la séquence GS-N31: 100...1650 (GS-P31)

<220>
<221>  misc_feature
<223>  nom: seladine-1

<400>  83


<210>  84
<211>  1739
<212>  PRT
<213>  Homo sapiens
<220>
<221>  misc_feature
<223>  CDS de la séquence GS-N32: 708...5927 ( GS-P32)

<220>
<221>  misc_feature
<223>  nom: CHD2 ( "chromodomain helicase DNA binding protein 2" )

<400>  84
Met Met Arg Asn Lys Asp Lys Ser Gln Glu Glu Asp Ser Ser Leu His
1               5                   10                  15
Ser Asn Ala Ser Ser His Ser Ala Ser Glu Glu Ala Ser Gly Ser Asp
            20                  25                  30
Ser Gly Ser Gln Ser Glu Ser Glu Gln Gly Ser Asp Pro Gly Ser Gly
        35                  40                  45
His Gly Ser Glu Ser Asn Ser Ser Ser Glu Ser Ser Glu Ser Gln Ser
    50                  55                  60
Glu Ser Glu Ser Glu Ser Ala Gly Ser Lys Ser Gln Pro Val Leu Pro

65                  70                  75                  80
Glu Ala Lys Glu Lys Pro Ala Ser Lys Lys Glu Arg Ile Ala Asp Val
                85                  90                  95
Lys Lys Met Trp Glu Glu Tyr Pro Asp Val Tyr Gly Val Arg Arg Ser
            100                 105                 110
Asn Arg Ser Arg Gln Glu Pro Ser Arg Phe Asn Ile Lys Glu Glu Ala
        115                 120                 125
Ser Ser Gly Ser Glu Ser Gly Ser Pro Lys Arg Arg Gly Gln Arg Gln
    130                 135                 140
Leu Lys Lys Gln Glu Lys Trp Lys Gln Glu Pro Ser Glu Asp Glu Gln
145                 150                 155                 160
Glu Gln Gly Thr Ser Ala Glu Ser Glu Pro Glu Gln Lys Lys Val Lys
                165                 170                 175
Ala Arg Arg Pro Val Pro Arg Arg Thr Val Pro Lys Pro Arg Val Lys
            180                 185                 190
Lys Gln Pro Lys Thr Gln Arg Gly Lys Arg Lys Lys Gln Asp Ser Ser
        195                 200                 205
Asp Glu Asp Asp Asp Asp Asp Glu Ala Pro Lys Arg Gln Thr Arg Arg
    210                 215                 220
Arg Ala Ala Lys Asn Val Ser Tyr Lys Glu Asp Asp Asp Phe Glu Thr
225                 230                 235                 240
Asp Ser Asp Asp Leu Ile Glu Met Thr Gly Glu Gly Val Asp Glu Gln
                245                 250                 255
Gln Asp Asn Ser Glu Thr Ile Glu Lys Val Leu Asp Ser Arg Leu Gly
            260                 265                 270
Lys Lys Gly Ala Thr Gly Ala Ser Thr Thr Val Tyr Ala Ile Glu Ala
        275                 280                 285
Asn Gly Asp Pro Ser Gly Asp Phe Asp Thr Glu Lys Asp Glu Gly Glu
    290                 295                 300
Ile Gln Tyr Leu Ile Lys Trp Lys Gly Trp Ser Tyr Ile His Ser Thr
305                 310                 315                 320
Trp Glu Ser Glu Glu Ser Leu Gln Gln Gln Lys Val Lys Gly Leu Lys
                325                 330                 335
Lys Leu Glu Asn Phe Lys Lys Lys Glu Asp Glu Ile Lys Gln Trp Leu
            340                 345                 350
Gly Lys Val Ser Pro Glu Asp Val Glu Tyr Phe Asn Cys Gln Gln Glu
        355                 360                 365
Leu Ala Ser Glu Leu Asn Lys Gln Tyr Gln Ile Val Glu Arg Val Ile
    370                 375                 380
Ala Val Lys Thr Ser Lys Ser Thr Leu Gly Gln Thr Asp Phe Pro Ala
385                 390                 395                 400
His Ser Arg Lys Pro Ala Pro Ser Asn Glu Pro Glu Tyr Leu Cys Lys
                405                 410                 415
Trp Met Gly Leu Pro Tyr Ser Glu Cys Ser Trp Glu Asp Glu Ala Leu
            420                 425                 430
Ile Gly Lys Lys Phe Gln Asn Cys Ile Asp Ser Phe His Ser Arg Asn
        435                 440                 445
Asn Ser Lys Thr Ile Pro Thr Arg Glu Cys Lys Ala Leu Lys Gln Arg
    450                 455                 460
Pro Arg Phe Val Ala Leu Lys Lys Gln Pro Ala Tyr Leu Gly Gly Glu
465                 470                 475                 480
Asn Leu Glu Leu Arg Asp Tyr Gln Leu Glu Gly Leu Asn Trp Leu Ala
                485                 490                 495
His Ser Trp Cys Lys Asn Asn Ser Val Ile Leu Ala Asp Glu Met Gly
            500                 505                 510
Leu Gly Lys Thr Ile Gln Thr Ile Ser Phe Leu Ser Tyr Leu Phe His
        515                 520                 525
Gln His Gln Leu Tyr Gly Pro Phe Leu Ile Val Val Pro Leu Ser Thr
    530                 535                 540
Leu Thr Ser Trp Gln Arg Glu Phe Glu Ile Trp Ala Pro Glu Ile Asn
545                 550                 555                 560
Val Val Val Tyr Ile Gly Asp Leu Met Ser Arg Asn Thr Ile Arg Glu
                565                 570                 575
Tyr Glu Trp Ile His Ser Gln Thr Lys Arg Leu Lys Phe Asn Ala Leu
            580                 585                 590
Ile Thr Thr Tyr Glu Ile Leu Leu Lys Asp Lys Thr Val Leu Gly Ser
        595                 600                 605
Ile Asn Trp Ala Phe Leu Gly Val Asp Glu Ala His Arg Leu Lys Asn
    610                 615                 620
Asp Asp Ser Leu Leu Tyr Lys Thr Leu Ile Asp Phe Lys Ser Asn His
625                 630                 635                 640
Arg Leu Leu Ile Thr Gly Thr Pro Leu Gln Asn Ser Leu Lys Glu Leu
                645                 650                 655

Trp Ser Leu Leu His Phe Ile Met Pro Glu Lys Phe Glu Phe Trp Glu
            660                 665                 670
Asp Phe Glu Glu Asp His Gly Lys Gly Arg Glu Asn Gly Tyr Gln Ser
        675                 680                 685
Leu His Lys Val Leu Glu Pro Phe Leu Leu Arg Arg Val Lys Lys Asp
    690                 695                 700
Val Glu Lys Ser Leu Pro Ala Lys Val Glu Gln Ile Leu Arg Val Glu
705                 710                 715                 720
Met Ser Ala Leu Gln Lys Gln Tyr Tyr Lys Trp Ile Leu Thr Arg Asn
                725                 730                 735
Tyr Lys Ala Leu Ala Lys Gly Thr Arg Gly Ser Thr Ser Gly Phe Leu
            740                 745                 750
Asn Ile Val Met Glu Leu Lys Lys Cys Cys Asn His Cys Tyr Leu Ile
        755                 760                 765
Lys Pro Pro Glu Glu Asn Glu Arg Glu Asn Gly Gln Glu Ile Leu Leu
    770                 775                 780
Ser Leu Ile Arg Ser Ser Gly Lys Leu Ile Leu Leu Asp Lys Leu Leu
785                 790                 795                 800
Thr Arg Leu Arg Glu Arg Gly Asn Arg Val Leu Ile Phe Ser Gln Met
                805                 810                 815
Val Arg Met Leu Asp Ile Leu Ala Glu Tyr Leu Thr Ile Lys His Tyr
            820                 825                 830
Pro Phe Gln Arg Leu Asp Gly Ser Ile Lys Gly Glu Ile Arg Lys Gln
        835                 840                 845
Ala Leu Asp His Phe Asn Ala Asp Gly Ser Glu Asp Phe Cys Phe Leu
    850                 855                 860
Leu Ser Thr Arg Ala Gly Gly Leu Gly Ile Asn Leu Ala Ser Ala Asp
865                 870                 875                 880
Thr Val Val Ile Phe Asp Ser Asp Trp Asn Pro Gln Asn Asp Leu Gln
                885                 890                 895
Ala Gln Ala Arg Ala His Arg Ile Gly Gln Lys Lys Gln Val Asn Ile
            900                 905                 910
Tyr Arg Leu Val Thr Lys Gly Thr Val Glu Glu Glu Ile Ile Glu Arg
        915                 920                 925
Ala Lys Lys Lys Met Val Leu Asp His Leu Val Ile Gln Arg Met Asp
    930                 935                 940
Thr Thr Gly Arg Thr Ile Leu Glu Asn Asn Ser Gly Arg Ser Asn Ser
945                 950                 955                 960
Asn Pro Phe Asn Lys Glu Glu Leu Thr Ala Ile Leu Lys Phe Gly Ala
                965                 970                 975
Glu Asp Leu Phe Lys Glu Leu Glu Gly Glu Glu Ser Glu Pro Gln Glu
            980                 985                 990
Met Asp Ile Asp Glu Ile Leu Arg  Leu Ala Glu Thr Arg  Glu Asn Glu
        995                 1000                1005
Val Ser  Thr Ser Ala Thr Asp  Glu Leu Leu Ser Gln  Phe Lys Val
    1010                1015                1020
Ala Asn  Phe Ala Thr Met Glu  Asp Glu Glu Glu Leu  Glu Glu Arg
    1025                1030                1035
Pro His  Lys Asp Trp Asp Glu  Ile Ile Pro Glu Glu  Gln Arg Lys
    1040                1045                1050
Lys Val  Glu Glu Glu Glu Arg  Gln Lys Glu Leu Glu  Glu Ile Tyr
    1055                1060                1065
Met Leu  Pro Arg Ile Arg Ser  Ser Thr Lys Lys Ala  Gln Thr Asn
    1070                1075                1080
Asp Ser  Asp Ser Asp Thr Glu  Ser Lys Arg Gln Ala  Gln Arg Ser
    1085                1090                1095
Ser Ala  Ser Glu Ser Glu Thr  Glu Asp Ser Asp Asp  Asp Lys Lys
    1100                1105                1110
Pro Lys  Arg Arg Gly Arg Pro  Arg Ser Val Arg Lys  Asp Leu Val
    1115                1120                1125
Glu Gly  Phe Thr Asp Ala Glu  Ile Arg Arg Phe Ile  Lys Ala Tyr
    1130                1135                1140
Lys Lys  Phe Gly Leu Pro Leu  Glu Arg Leu Glu Cys  Leu Ala Arg
    1145                1150                1155
Asp Ala  Glu Leu Val Asp Lys  Ser Val Ala Asp Leu  Lys Arg Leu
    1160                1165                1170
Gly Glu  Leu Ile His Asn Ser  Cys Val Ser Ala Met  Gln Glu Tyr
    1175                1180                1185
Glu Glu  Gln Leu Lys Glu Asn  Ala Ser Glu Gly Lys  Gly Pro Gly
    1190                1195                1200
Lys Arg  Arg Gly Pro Thr Ile  Lys Ile Ser Gly Val  Gln Val Asn
    1205                1210                1215
Val Lys  Ser Ile Ile Gln His  Glu Glu Glu Phe Glu  Met Leu His

1220                 1225                 1230
Lys Ser Ile Pro Val Asp Pro Glu Glu Lys Lys Lys Tyr Cys Leu
    1235                 1240                 1245
Thr Cys Arg Val Lys Ala Ala His Phe Asp Val Glu Trp Gly Val
    1250                 1255                 1260
Glu Asp Asp Ser Arg Leu Leu Leu Gly Ile Tyr Glu His Gly Tyr
    1265                 1270                 1275
Gly Asn Trp Glu Leu Ile Lys Thr Asp Pro Glu Leu Lys Leu Thr
    1280                 1285                 1290
Asp Lys Ile Leu Pro Val Glu Thr Asp Lys Lys Pro Gln Gly Lys
    1295                 1300                 1305
Gln Leu Gln Thr Arg Ala Asp Tyr Leu Leu Lys Leu Leu Arg Lys
    1310                 1315                 1320
Gly Leu Glu Lys Lys Gly Ala Val Thr Gly Gly Glu Glu Ala Lys
    1325                 1330                 1335
Leu Lys Lys Arg Lys Pro Arg Val Lys Lys Glu Asn Lys Val Pro
    1340                 1345                 1350
Arg Leu Lys Glu Glu His Gly Ile Glu Leu Ser Ser Pro Arg His
    1355                 1360                 1365
Ser Asp Asn Pro Ser Glu Glu Gly Glu Val Lys Asp Asp Gly Leu
    1370                 1375                 1380
Glu Lys Ser Pro Met Lys Lys Lys Gln Lys Lys Lys Glu Asn Lys
    1385                 1390                 1395
Glu Asn Lys Glu Lys Gln Met Ser Ser Arg Lys Asp Lys Glu Gly
    1400                 1405                 1410
Asp Lys Glu Arg Lys Lys Ser Lys Asp Lys Lys Glu Lys Pro Lys
    1415                 1420                 1425
Ser Gly Asp Ala Lys Ser Ser Ser Lys Ser Lys Arg Ser Gln Gly
    1430                 1435                 1440
Pro Val His Ile Thr Ala Gly Ser Glu Pro Val Pro Ile Gly Glu
    1445                 1450                 1455
Asp Glu Asp Asp Asp Leu Asp Gln Glu Thr Phe Ser Ile Cys Lys
    1460                 1465                 1470
Glu Arg Met Arg Pro Val Lys Lys Ala Leu Lys Gln Leu Asp Lys
    1475                 1480                 1485
Pro Asp Lys Gly Leu Asn Val Gln Glu Gln Leu Glu His Thr Arg
    1490                 1495                 1500
Asn Cys Leu Leu Lys Ile Gly Asp Arg Ile Ala Glu Cys Leu Lys
    1505                 1510                 1515
Ala Tyr Ser Asp Gln Glu His Ile Lys Leu Trp Arg Arg Asn Leu
    1520                 1525                 1530
Trp Ile Phe Val Ser Lys Phe Thr Glu Phe Asp Ala Arg Lys Leu
    1535                 1540                 1545
His Lys Leu Tyr Lys Met Ala His Lys Lys Arg Ser Gln Glu Glu
    1550                 1555                 1560
Glu Glu Gln Lys Lys Lys Asp Asp Val Thr Gly Gly Lys Lys Pro
    1565                 1570                 1575
Phe Arg Pro Glu Ala Ser Gly Ser Ser Arg Asp Ser Leu Ile Ser
    1580                 1585                 1590
Gln Ser His Thr Ser His Asn Leu His Pro Gln Lys Pro His Leu
    1595                 1600                 1605
Pro Ala Ser His Gly Pro Gln Met His Gly His Pro Arg Asp Asn
    1610                 1615                 1620
Tyr Asn His Pro Asn Lys Arg His Phe Ser Asn Ala Asp Arg Gly
    1625                 1630                 1635
Asp Trp Gln Arg Glu Arg Lys Phe Asn Tyr Gly Gly Gly Asn Asn
    1640                 1645                 1650
Asn Pro Pro Trp Gly Ser Asp Arg His His Gln Tyr Glu Gln His
    1655                 1660                 1665
Trp Tyr Lys Asp His His Tyr Gly Asp Arg Arg His Met Asp Ala
    1670                 1675                 1680
His Arg Ser Gly Ser Tyr Arg Pro Asn Asn Met Ser Arg Lys Arg
    1685                 1690                 1695
Pro Tyr Asp Gln Tyr Ser Ser Asp Arg Asp His Arg Gly His Arg
    1700                 1705                 1710
Asp Tyr Tyr Asp Arg Tyr Ala Lys Gly Cys Glu Thr Pro Gly Ala
    1715                 1720                 1725
Asn Leu Cys Gln Glu Leu Phe Leu Gly Arg Lys
    1730                 1735

<210>  85
<211>  801

<212>  PRT
<213>  Homo sapiens
<220>
<221>  misc_feature
<223>  CDS de la séquence GS-N33: 1702...4107 ( GS-P33)

<220>
<221>  misc_feature
<223>  nom:Protéine 2 contenant un bromodomaine ( BRD2)

<400>  85

<210>  86
<211>  289
<212>  PRT
<213>  Homo sapiens
<220>
<221>  misc_feature
<223>  CDS de la séquence GS-N34: 200...1069 ( GS-P34)

<220>
<221>  misc_feature
<223>  nom: Syntaxine 3A

<400>  86
Met Lys Asp Arg Leu Glu Gln Leu Lys Ala Lys Gln Leu Thr Gln Asp
1               5                   10                  15
Asp Asp Thr Asp Ala Val Glu Ile Ala Ile Asp Asn Thr Ala Phe Met
            20                  25                  30
Asp Glu Phe Phe Ser Glu Ile Glu Glu Thr Arg Leu Asn Ile Asp Lys
        35                  40                  45
Ile Ser Glu His Val Glu Glu Ala Lys Lys Leu Tyr Ser Ile Ile Leu
    50                  55                  60
Ser Ala Pro Ile Pro Glu Pro Lys Thr Lys Asp Asp Leu Glu Gln Leu
65                  70                  75                  80
Thr Thr Glu Ile Lys Lys Arg Ala Asn Asn Val Arg Asn Lys Leu Lys
                85                  90                  95
Ser Met Glu Lys His Ile Glu Glu Asp Glu Val Arg Ser Ser Ala Asp
            100                 105                 110
Leu Arg Ile Arg Lys Ser Gln His Ser Val Leu Ser Arg Lys Phe Val
        115                 120                 125
Glu Val Met Thr Lys Tyr Asn Glu Ala Gln Val Asp Phe Arg Glu Arg
    130                 135                 140

Ser Lys Gly Arg Ile Gln Arg Gln Leu Glu Ile Thr Gly Lys Lys Thr
145                 150                 155                 160
Thr Asp Glu Glu Leu Glu Glu Met Leu Glu Ser Gly Asn Pro Ala Ile
                165                 170                 175
Phe Thr Ser Gly Ile Ile Asp Ser Gln Ile Ser Lys Gln Ala Leu Ser
            180                 185                 190
Glu Ile Glu Gly Arg His Lys Asp Ile Val Arg Leu Glu Ser Ser Ile
        195                 200                 205
Lys Glu Leu His Asp Met Phe Met Asp Ile Ala Met Leu Val Glu Asn
    210                 215                 220
Gln Gly Glu Met Leu Asp Asn Ile Glu Leu Asn Val Met His Thr Val
225                 230                 235                 240
Asp His Val Glu Lys Ala Arg Asp Glu Thr Lys Lys Ala Val Lys Tyr
                245                 250                 255
Gln Ser Gln Ala Arg Lys Lys Leu Ile Ile Ile Ile Val Leu Val Val
            260                 265                 270
Val Leu Leu Gly Ile Leu Ala Leu Ile Ile Gly Leu Ser Val Gly Leu
        275                 280                 285
Asn

<210>  87
<211>  3664
<212>  PRT
<213>  Homo sapiens
<220>
<221>  misc_feature
<223>  CDS de la séquence GS-N35: 205...11199 ( GS-P35)

<220>
<221>  misc_feature
<223>  nom: Protéine represseur associée à SMART/HDAC1(SHARP

<400>  87
Met Val Arg Glu Thr Arg His Leu Trp Val Gly Asn Leu Pro Glu Asn
1               5                   10                  15
Val Arg Glu Glu Lys Ile Ile Glu His Phe Lys Arg Tyr Gly Arg Val
            20                  25                  30
Glu Ser Val Lys Ile Leu Pro Lys Arg Gly Ser Glu Gly Gly Val Ala
        35                  40                  45
Ala Phe Val Asp Phe Val Asp Ile Lys Ser Ala Gln Lys Ala His Asn
    50                  55                  60
Ser Val Asn Lys Met Gly Asp Arg Asp Leu Arg Thr Asp Tyr Asn Glu
65                  70                  75                  80
Pro Gly Thr Ile Pro Ser Ala Ala Arg Gly Leu Asp Asp Thr Val Ser
                85                  90                  95
Ile Ala Ser Arg Ser Arg Glu Val Ser Gly Phe Arg Gly Gly Gly Gly
            100                 105                 110
Gly Pro Ala Tyr Gly Pro Pro Pro Ser Leu His Ala Arg Glu Gly Arg
        115                 120                 125
Tyr Glu Arg Arg Leu Asp Gly Ala Ser Asp Asn Arg Glu Arg Ala Tyr
    130                 135                 140
Glu His Ser Ala Tyr Gly His His Glu Arg Gly Thr Gly Gly Phe Asp
145                 150                 155                 160
Arg Thr Arg His Tyr Asp Gln Asp Tyr Tyr Arg Asp Pro Arg Glu Arg
                165                 170                 175
Thr Leu Gln His Gly Leu Tyr Tyr Ala Ser Arg Ser Arg Ser Pro Asn
            180                 185                 190
Arg Phe Asp Ala His Asp Pro Arg Tyr Glu Pro Arg Ala Arg Glu Gln
        195                 200                 205
Phe Thr Leu Pro Ser Val Val His Arg Asp Ile Tyr Arg Asp Asp Ile
    210                 215                 220
Thr Arg Glu Val Arg Gly Arg Arg Pro Glu Arg Asn Tyr Gln His Ser
225                 230                 235                 240
Arg Ser Arg Ser Pro His Ser Ser Gln Ser Arg Asn Gln Ser Pro Gln
                245                 250                 255
Arg Leu Ala Ser Gln Ala Ser Arg Pro Thr Arg Ser Pro Ser Gly Ser
            260                 265                 270
Gly Ser Arg Ser Arg Ser Ser Ser Ser Asp Ser Ile Ser Ser Ser Ser
        275                 280                 285
Ser Thr Ser Ser Asp Ser Ser Asp Ser Ser Ser Ser Ser Ser Asp Asp

290                 295                 300
Ser Pro Ala Arg Ser Val Gln Ser Ala Ala Val Pro Ala Pro Thr Ser
305                 310                 315                 320
Gln Leu Leu Ser Ser Leu Glu Lys Asp Glu Pro Arg Lys Ser Phe Gly
                325                 330                 335
Ile Lys Val Gln Asn Leu Pro Val Arg Ser Thr Asp Thr Ser Leu Lys
            340                 345                 350
Asp Gly Leu Phe His Glu Phe Lys Lys Phe Gly Lys Val Thr Ser Val
        355                 360                 365
Gln Ile His Gly Thr Ser Glu Glu Arg Tyr Gly Leu Val Phe Phe Arg
    370                 375                 380
Gln Gln Glu Asp Gln Glu Lys Ala Leu Thr Ala Ser Lys Gly Lys Leu
385                 390                 395                 400
Phe Phe Gly Met Gln Ile Glu Val Thr Ala Trp Ile Gly Pro Glu Thr
                405                 410                 415
Glu Ser Glu Asn Glu Phe Arg Pro Leu Asp Glu Arg Ile Asp Glu Phe
            420                 425                 430
His Pro Lys Ala Thr Arg Thr Leu Phe Ile Gly Asn Leu Glu Lys Thr
        435                 440                 445
Thr Thr Tyr His Asp Leu Arg Asn Ile Phe Gln Arg Phe Gly Glu Ile
    450                 455                 460
Val Asp Ile Asp Ile Lys Lys Val Asn Gly Val Pro Gln Tyr Ala Phe
465                 470                 475                 480
Leu Gln Tyr Cys Asp Ile Ala Ser Val Cys Lys Ala Ile Lys Lys Met
                485                 490                 495
Asp Gly Glu Tyr Leu Gly Asn Asn Arg Leu Lys Leu Gly Phe Gly Lys
            500                 505                 510
Ser Met Pro Thr Asn Cys Val Trp Leu Asp Gly Leu Ser Ser Asn Val
        515                 520                 525
Ser Asp Gln Tyr Leu Thr Arg His Phe Cys Arg Tyr Gly Pro Val Val
    530                 535                 540
Lys Val Val Phe Asp Arg Leu Lys Gly Met Ala Leu Val Leu Tyr Asn
545                 550                 555                 560
Glu Ile Glu Tyr Ala Gln Ala Ala Val Lys Glu Thr Lys Gly Arg Lys
                565                 570                 575
Ile Gly Gly Asn Lys Ile Lys Val Asp Phe Ala Asn Arg Glu Ser Gln
            580                 585                 590
Leu Ala Phe Tyr His Cys Met Glu Lys Ser Gly Gln Asp Ile Arg Asp
        595                 600                 605
Phe Tyr Glu Met Leu Ala Glu Arg Arg Glu Glu Arg Arg Ala Ser Tyr
    610                 615                 620
Asp Tyr Asn Gln Asp Arg Thr Tyr Tyr Glu Ser Val Arg Thr Pro Gly
625                 630                 635                 640
Thr Tyr Pro Glu Asp Ser Arg Arg Asp Tyr Pro Ala Arg Gly Arg Glu
                645                 650                 655
Phe Tyr Ser Glu Trp Glu Thr Tyr Gln Gly Asp Tyr Tyr Glu Ser Arg
            660                 665                 670
Tyr Tyr Asp Asp Pro Arg Glu Tyr Arg Asp Tyr Arg Asn Asp Pro Tyr
        675                 680                 685
Glu Gln Asp Ile Arg Glu Tyr Ser Tyr Arg Gln Arg Glu Arg Glu Arg
    690                 695                 700
Glu Arg Glu Arg Phe Glu Ser Asp Arg Asp Arg Asp His Glu Arg Arg
705                 710                 715                 720
Pro Ile Glu Arg Ser Gln Ser Pro Val His Leu Arg Arg Pro Gln Ser
                725                 730                 735
Pro Gly Ala Ser Pro Ser Gln Ala Glu Arg Leu Pro Ser Asp Ser Glu
            740                 745                 750
Arg Arg Leu Tyr Ser Arg Ser Ser Asp Arg Ser Gly Ser Cys Ser Ser
        755                 760                 765
Leu Ser Pro Pro Arg Tyr Glu Lys Leu Asp Lys Ser Arg Leu Glu Arg
    770                 775                 780
Tyr Thr Lys Asn Glu Lys Thr Asp Lys Glu Arg Thr Phe Asp Pro Glu
785                 790                 795                 800
Arg Val Glu Arg Glu Arg Arg Leu Ile Arg Lys Glu Lys Val Glu Lys
                805                 810                 815
Asp Lys Thr Asp Lys Gln Lys Arg Lys Gly Lys Val His Ser Pro Ser
            820                 825                 830
Ser Gln Ser Ser Glu Thr Asp Gln Glu Asn Glu Arg Glu Gln Ser Pro
        835                 840                 845
Glu Lys Pro Arg Ser Cys Asn Lys Leu Ser Arg Glu Lys Ala Asp Lys
    850                 855                 860
Glu Gly Ile Ala Lys Asn Arg Leu Glu Leu Met Pro Cys Val Val Leu
865                 870                 875                 880

Thr Arg Val Lys Glu Lys Glu Gly Lys Val Ile Asp His Thr Pro Val
                885                 890                 895
Glu Lys Leu Lys Ala Lys Leu Asp Asn Asp Thr Val Lys Ser Ser Ala
            900                 905                 910
Leu Asp Gln Lys Leu Gln Val Ser Gln Thr Glu Pro Ala Lys Ser Asp
        915                 920                 925
Leu Ser Lys Leu Glu Ser Val Arg Met Lys Val Pro Lys Glu Lys Gly
    930                 935                 940
Leu Ser Ser His Val Glu Val Val Glu Lys Glu Gly Arg Leu Lys Ala
945                 950                 955                 960
Arg Lys His Leu Lys Pro Glu Gln Pro Ala Asp Gly Val Ser Ala Val
                965                 970                 975
Asp Leu Glu Lys Leu Glu Ala Arg Lys Arg Arg Phe Ala Asp Ser Asn
            980                 985                 990
Leu Lys Ala Glu Lys Gln Lys Pro  Glu Val Lys Lys Ser  Ser Pro Glu
        995                 1000                1005
Met Glu  Asp Ala Arg Val Leu  Ser Lys Lys Gln Pro  Asp Val Ser
    1010                1015                1020
Ser Arg  Glu Val Ile Leu Leu  Arg Glu Gly Glu Ala  Glu Arg Lys
    1025                1030                1035
Pro Val  Arg Lys Glu Ile Leu  Lys Arg Glu Ser Lys  Lys Ile Lys
    1040                1045                1050
Leu Asp  Arg Leu Asn Thr Val  Ala Ser Pro Lys Asp  Cys Gln Glu
    1055                1060                1065
Leu Ala  Ser Ile Ser Val Gly  Ser Gly Ser Arg Pro  Ser Ser Asp
    1070                1075                1080
Leu Gln  Ala Arg Leu Gly Glu  Leu Ala Gly Glu Ser  Val Glu Asn
    1085                1090                1095
Gln Glu  Val Gln Ser Lys Lys  Pro Ile Pro Ser Lys  Pro Gln Leu
    1100                1105                1110
Lys Gln  Leu Gln Val Leu Asp  Asp Gln Gly Pro Glu  Arg Glu Asp
    1115                1120                1125
Val Arg  Lys Asn Tyr Cys Ser  Leu Arg Asp Glu Thr  Pro Glu Arg
    1130                1135                1140
Lys Ser  Gly Gln Glu Lys Ser  His Ser Val Asn Thr  Glu Glu Lys
    1145                1150                1155
Ile Gly  Ile Asp Ile Asp His  Thr Gln Ser Tyr Arg  Lys Gln Met
    1160                1165                1170
Glu Gln  Ser Arg Arg Lys Gln  Gln Met Glu Met Glu  Ile Ala Lys
    1175                1180                1185
Ser Glu  Lys Phe Gly Ser Pro  Lys Lys Asp Val Asp  Glu Tyr Glu
    1190                1195                1200
Arg Arg  Ser Leu Val His Glu  Val Gly Lys Pro Pro  Gln Asp Val
    1205                1210                1215
Thr Asp  Asp Ser Pro Pro Ser  Lys Lys Lys Arg Met  Asp His Val
    1220                1225                1230
Asp Phe  Asp Ile Cys Thr Lys  Arg Glu Arg Asn Tyr  Arg Ser Ser
    1235                1240                1245
Arg Gln  Ile Ser Glu Asp Ser  Glu Arg Thr Gly Gly  Ser Pro Ser
    1250                1255                1260
Val Arg  His Gly Ser Phe His  Glu Asp Glu Asp Pro  Ile Gly Ser
    1265                1270                1275
Pro Arg  Leu Leu Ser Val Lys  Gly Ser Pro Lys Val  Asp Glu Lys
    1280                1285                1290
Val Leu  Pro Tyr Ser Asn Ile  Thr Val Arg Glu Glu  Ser Leu Lys
    1295                1300                1305
Phe Asn  Pro Tyr Asp Ser Ser  Arg Arg Glu Gln Met  Ala Asp Met
    1310                1315                1320
Ala Lys  Ile Lys Leu Ser Val  Leu Asn Ser Glu Asp  Glu Leu Asn
    1325                1330                1335
Arg Trp  Asp Ser Gln Met Lys  Gln Asp Ala Gly Arg  Phe Asp Val
    1340                1345                1350
Ser Phe  Pro Asn Ser Ile Ile  Lys Arg Asp Ser Leu  Arg Lys Arg
    1355                1360                1365
Ser Val  Arg Asp Leu Glu Pro  Gly Glu Val Pro Ser  Asp Ser Asp
    1370                1375                1380
Glu Asp  Gly Glu His Lys Ser  His Ser Pro Arg Ala  Ser Ala Leu
    1385                1390                1395
Tyr Glu  Ser Ser Arg Leu Ser  Phe Leu Leu Arg Asp  Arg Glu Asp
    1400                1405                1410
Lys Leu  Arg Glu Arg Asp Glu  Arg Leu Ser Ser Ser  Leu Glu Arg
    1415                1420                1425
Asn Lys  Phe Tyr Ser Phe Ala  Leu Asp Lys Thr Ile  Thr Pro Asp

1430                 1435                 1440
Thr Lys  Ala Leu Leu Glu Arg  Ala Lys Ser Leu Ser  Ser Ser Arg
    1445                 1450                 1455
Glu Glu  Asn Trp Ser Phe Leu  Asp Trp Asp Ser Arg  Phe Ala Asn
    1460                 1465                 1470
Phe Arg  Asn Asn Lys Asp Lys  Glu Lys Val Asp Ser  Ala Pro Arg
    1475                 1480                 1485
Pro Ile  Pro Ser Trp Tyr Met  Lys Lys Lys Lys Ile  Arg Thr Asp
    1490                 1495                 1500
Ser Glu  Gly Lys Met Asp Asp  Lys Lys Glu Asp His  Lys Glu Glu
    1505                 1510                 1515
Glu Gln  Glu Arg Gln Glu Leu  Phe Ala Ser Arg Phe  Leu His Ser
    1520                 1525                 1530
Ser Ile  Phe Glu Gln Asp Ser  Lys Arg Leu Gln His  Leu Glu Arg
    1535                 1540                 1545
Lys Glu  Glu Asp Ser Asp Phe  Ile Ser Gly Arg Ile  Tyr Gly Lys
    1550                 1555                 1560
Gln Thr  Ser Glu Gly Ala Asn  Ser Thr Thr Asp Ser  Ile Gln Glu
    1565                 1570                 1575
Pro Val  Val Leu Phe His Ser  Arg Phe Met Glu Leu  Thr Arg Met
    1580                 1585                 1590
Gln Gln  Lys Glu Lys Glu Lys  Asp Gln Lys Pro Lys  Glu Val Glu
    1595                 1600                 1605
Lys Gln  Glu Asp Thr Glu Asn  His Pro Lys Thr Pro  Glu Ser Ala
    1610                 1615                 1620
Pro Glu  Asn Lys Asp Ser Glu  Leu Lys Thr Pro Pro  Ser Val Gly
    1625                 1630                 1635
Pro Pro  Ser Val Thr Val Val  Thr Leu Glu Ser Ala  Pro Ser Ala
    1640                 1645                 1650
Leu Glu  Lys Thr Thr Gly Asp  Lys Thr Val Glu Ala  Pro Leu Val
    1655                 1660                 1665
Thr Glu  Glu Lys Thr Val Glu  Pro Ala Thr Val Ser  Glu Glu Ala
    1670                 1675                 1680
Lys Pro  Ala Ser Glu Pro Ala  Pro Ala Pro Val Glu  Gln Leu Glu
    1685                 1690                 1695
Gln Val  Asp Leu Pro Pro Gly  Ala Asp Pro Asp Lys  Glu Ala Ala
    1700                 1705                 1710
Met Met  Pro Ala Gly Val Glu  Glu Gly Ser Ser Gly  Asp Gln Pro
    1715                 1720                 1725
Pro Tyr  Leu Asp Ala Lys Pro  Pro Thr Pro Gly Ala  Ser Phe Ser
    1730                 1735                 1740
Gln Ala  Glu Ser Asn Val Asp  Pro Glu Pro Asp Ser  Thr Gln Pro
    1745                 1750                 1755
Leu Ser  Lys Pro Ala Gln Lys  Ser Glu Glu Ala Asn  Glu Pro Lys
    1760                 1765                 1770
Ala Glu  Lys Pro Asp Ala Thr  Ala Asp Ala Glu Pro  Asp Ala Asn
    1775                 1780                 1785
Gln Lys  Ala Glu Ala Ala Pro  Glu Ser Gln Pro Pro  Ala Ser Glu
    1790                 1795                 1800
Asp Leu  Glu Val Asp Pro Pro  Val Ala Ala Lys Asp  Lys Lys Pro
    1805                 1810                 1815
Asn Lys  Ser Lys Arg Ser Lys  Thr Pro Val Gln Ala  Ala Ala Val
    1820                 1825                 1830
Ser Ile  Val Glu Lys Pro Val  Thr Arg Lys Ser Glu  Arg Ile Asp
    1835                 1840                 1845
Arg Glu  Lys Leu Lys Arg Ser  Asn Ser Pro Arg Gly  Glu Ala Gln
    1850                 1855                 1860
Lys Leu  Leu Glu Leu Lys Met  Glu Ala Glu Lys Ile  Thr Arg Thr
    1865                 1870                 1875
Ala Ser  Lys Asn Ser Ala Ala  Asp Leu Glu His Pro  Glu Pro Ser
    1880                 1885                 1890
Leu Pro  Leu Ser Arg Thr Arg  Arg Arg Asn Val Arg  Ser Val Tyr
    1895                 1900                 1905
Ala Thr  Met Gly Asp His Glu  Asn Arg Ser Pro Val  Lys Glu Pro
    1910                 1915                 1920
Val Glu  Gln Pro Arg Val Thr  Arg Lys Arg Leu Glu  Arg Glu Leu
    1925                 1930                 1935
Gln Glu  Ala Ala Ala Val Pro  Thr Thr Pro Arg Arg  Gly Arg Pro
    1940                 1945                 1950
Pro Lys  Thr Arg Arg Arg Ala  Asp Glu Glu Glu Glu  Asn Glu Ala
    1955                 1960                 1965
Lys Glu  Pro Ala Glu Thr Leu  Lys Pro Pro Glu Gly  Trp Arg Ser
    1970                 1975                 1980

Pro Arg Ser Gln Lys Thr Ala Ala Gly Gly Gly Pro Gln Gly Lys
    1985                1990                1995
Lys Gly Lys Asn Glu Pro Lys Val Asp Ala Thr Arg Pro Glu Ala
    2000                2005                2010
Thr Thr Glu Val Gly Pro Gln Ile Gly Val Lys Glu Ser Ser Met
    2015                2020                2025
Glu Pro Lys Ala Ala Glu Glu Glu Ala Gly Ser Glu Gln Lys Arg
    2030                2035                2040
Asp Arg Lys Asp Ala Gly Thr Asp Lys Asn Pro Pro Glu Thr Ala
    2045                2050                2055
Pro Val Glu Val Val Glu Lys Lys Pro Ala Pro Glu Lys Asn Ser
    2060                2065                2070
Lys Ser Lys Arg Gly Arg Ser Arg Asn Ser Arg Leu Ala Val Asp
    2075                2080                2085
Lys Ser Ala Ser Leu Lys Asn Val Asp Ala Ala Val Ser Pro Arg
    2090                2095                2100
Gly Ala Ala Ala Gln Ala Gly Glu Arg Glu Ser Gly Val Val Ala
    2105                2110                2115
Val Ser Pro Glu Lys Ser Glu Ser Pro Gln Lys Glu Asp Gly Leu
    2120                2125                2130
Ser Ser Gln Leu Lys Ser Asp Pro Val Asp Pro Asp Lys Glu Pro
    2135                2140                2145
Glu Lys Glu Asp Val Ser Ala Ser Gly Pro Ser Pro Glu Ala Thr
    2150                2155                2160
Gln Leu Ala Lys Gln Met Glu Leu Glu Gln Ala Val Glu His Ile
    2165                2170                2175
Ala Lys Leu Ala Glu Ala Ser Ala Ser Ala Ala Tyr Lys Ala Asp
    2180                2185                2190
Ala Pro Glu Gly Leu Ala Pro Glu Asp Arg Asp Lys Pro Ala His
    2195                2200                2205
Gln Ala Ser Glu Thr Glu Leu Ala Ala Ala Ile Gly Ser Ile Ile
    2210                2215                2220
Asn Asp Ile Ser Gly Glu Pro Glu Asn Phe Pro Ala Pro Pro Pro
    2225                2230                2235
Tyr Pro Gly Glu Ser Gln Thr Asp Leu Gln Pro Pro Ala Gly Ala
    2240                2245                2250
Gln Ala Leu Gln Pro Ser Glu Glu Gly Met Glu Thr Asp Glu Ala
    2255                2260                2265
Val Ser Gly Ile Leu Glu Thr Glu Ala Ala Thr Glu Ser Ser Arg
    2270                2275                2280
Pro Pro Val Asn Ala Pro Asp Pro Ser Ala Gly Pro Thr Asp Thr
    2285                2290                2295
Lys Glu Ala Arg Gly Asn Ser Ser Glu Thr Ser His Ser Val Pro
    2300                2305                2310
Glu Ala Lys Gly Ser Lys Glu Val Glu Val Thr Leu Val Arg Lys
    2315                2320                2325
Asp Lys Gly Arg Gln Lys Thr Thr Arg Ser Arg Arg Lys Arg Asn
    2330                2335                2340
Thr Asn Lys Lys Val Val Ala Pro Val Glu Ser His Val Pro Glu
    2345                2350                2355
Ser Asn Gln Ala Gln Gly Glu Ser Pro Ala Ala Asn Glu Gly Thr
    2360                2365                2370
Thr Val Gln His Pro Glu Ala Pro Gln Glu Glu Lys Gln Ser Glu
    2375                2380                2385
Lys Pro His Ser Thr Pro Pro Gln Ser Cys Thr Ser Asp Leu Ser
    2390                2395                2400
Lys Ile Pro Ser Thr Glu Asn Ser Ser Gln Glu Ile Ser Val Glu
    2405                2410                2415
Glu Arg Thr Pro Thr Lys Ala Ser Val Pro Pro Asp Leu Pro Pro
    2420                2425                2430
Pro Pro Gln Pro Ala Pro Val Asp Glu Glu Pro Gln Ala Arg Phe
    2435                2440                2445
Arg Val His Ser Ile Ile Glu Ser Asp Pro Val Thr Pro Pro Ser
    2450                2455                2460
Asp Pro Ser Ile Pro Ile Pro Thr Leu Pro Ser Val Thr Ala Ala
    2465                2470                2475
Lys Leu Ser Pro Pro Val Ala Ser Gly Gly Ile Pro His Gln Ser
    2480                2485                2490
Pro Pro Thr Lys Val Thr Glu Trp Ile Thr Arg Gln Glu Glu Pro
    2495                2500                2505
Arg Ala Gln Ser Thr Pro Ser Pro Ala Leu Pro Pro Asp Thr Lys
    2510                2515                2520
Ala Ser Asp Val Asp Thr Ser Ser Ser Thr Leu Arg Lys Ile Leu

2525                    2530                    2535
Met Asp Pro Lys Tyr Val Ser Ala Thr Ser Val Thr Ser Thr Ser
    2540                    2545                    2550
Val Thr Thr Ala Ile Ala Glu Pro Val Ser Ala Ala Pro Cys Leu
    2555                    2560                    2565
His Glu Ala Pro Pro Pro Pro Val Asp Ser Lys Lys Pro Leu Glu
    2570                    2575                    2580
Glu Lys Thr Ala Pro Pro Val Thr Asn Asn Ser Glu Ile Gln Ala
    2585                    2590                    2595
Ser Glu Val Leu Val Ala Ala Asp Lys Glu Lys Val Ala Pro Val
    2600                    2605                    2610
Ile Ala Pro Lys Ile Thr Ser Val Ile Ser Arg Met Pro Val Ser
    2615                    2620                    2625
Ile Asp Leu Glu Asn Ser Gln Lys Ile Thr Leu Ala Lys Pro Ala
    2630                    2635                    2640
Pro Gln Thr Leu Thr Gly Leu Val Ser Ala Leu Thr Gly Leu Val
    2645                    2650                    2655
Asn Val Ser Leu Val Pro Val Asn Ala Leu Lys Gly Pro Val Lys
    2660                    2665                    2670
Gly Ser Val Thr Thr Leu Lys Ser Leu Val Ser Thr Pro Ala Gly
    2675                    2680                    2685
Pro Val Asn Val Leu Lys Gly Pro Val Asn Val Leu Thr Gly Pro
    2690                    2695                    2700
Val Asn Val Leu Thr Thr Pro Val Asn Ala Thr Val Gly Thr Val
    2705                    2710                    2715
Asn Ala Ala Pro Gly Thr Val Asn Ala Ala Ala Ser Ala Val Asn
    2720                    2725                    2730
Ala Thr Ala Ser Ala Val Thr Val Thr Ala Gly Ala Val Thr Ala
    2735                    2740                    2745
Ala Ser Gly Gly Val Thr Ala Thr Thr Gly Thr Val Thr Met Ala
    2750                    2755                    2760
Gly Ala Val Ile Ala Pro Ser Thr Lys Cys Lys Gln Arg Ala Ser
    2765                    2770                    2775
Ala Asn Glu Asn Ser Arg Phe His Pro Gly Ser Met Pro Val Ile
    2780                    2785                    2790
Asp Asp Arg Pro Ala Asp Ala Gly Ser Gly Ala Gly Leu Arg Val
    2795                    2800                    2805
Asn Thr Ser Glu Gly Val Val Leu Leu Ser Tyr Ser Gly Gln Lys
    2810                    2815                    2820
Thr Glu Gly Pro Gln Arg Ile Ser Ala Lys Ile Ser Gln Ile Pro
    2825                    2830                    2835
Pro Ala Ser Ala Met Asp Ile Glu Phe Gln Gln Ser Val Ser Lys
    2840                    2845                    2850
Ser Gln Val Lys Pro Asp Ser Val Thr Ala Ser Gln Pro Pro Ser
    2855                    2860                    2865
Lys Gly Pro Gln Ala Pro Ala Gly Tyr Ala Asn Val Ala Thr His
    2870                    2875                    2880
Ser Thr Leu Val Leu Thr Ala Gln Thr Tyr Asn Ala Ser Pro Val
    2885                    2890                    2895
Ile Ser Ser Val Lys Ala Asp Arg Pro Ser Leu Glu Lys Pro Glu
    2900                    2905                    2910
Pro Ile His Leu Ser Val Ser Thr Pro Val Thr Gln Gly Gly Thr
    2915                    2920                    2925
Val Lys Val Leu Thr Gln Gly Ile Asn Thr Pro Pro Val Leu Val
    2930                    2935                    2940
His Asn Gln Leu Val Leu Thr Pro Ser Ile Val Thr Thr Asn Lys
    2945                    2950                    2955
Lys Leu Ala Asp Pro Val Thr Leu Lys Ile Glu Thr Lys Val Leu
    2960                    2965                    2970
Gln Pro Ala Asn Leu Gly Ser Thr Leu Thr Pro His His Pro Pro
    2975                    2980                    2985
Ala Leu Pro Ser Lys Leu Pro Thr Glu Val Asn His Val Pro Ser
    2990                    2995                    3000
Gly Pro Ser Ile Pro Ala Asp Arg Thr Val Ser His Leu Ala Ala
    3005                    3010                    3015
Ala Lys Leu Asp Ala His Ser Pro Arg Pro Ser Gly Pro Gly Pro
    3020                    3025                    3030
Ser Ser Phe Pro Arg Ala Ser His Pro Ser Ser Thr Ala Ser Thr
    3035                    3040                    3045
Ala Leu Ser Thr Asn Ala Thr Val Met Leu Ala Ala Gly Ile Pro
    3050                    3055                    3060
Val Pro Gln Phe Ile Ser Ser Ile His Pro Glu Gln Ser Val Ile
    3065                    3070                    3075

Met Pro Pro His Ser Ile Thr Gln Thr Val Ser Leu Ser His Leu
    3080                3085                3090
Ser Gln Gly Glu Val Arg Met Asn Thr Pro Thr Leu Pro Ser Ile
    3095                3100                3105
Thr Tyr Ser Ile Arg Pro Glu Ala Leu His Ser Pro Arg Ala Pro
    3110                3115                3120
Leu Gln Pro Gln Gln Ile Glu Val Arg Ala Pro Gln Arg Ala Ser
    3125                3130                3135
Thr Pro Gln Pro Ala Pro Ala Gly Val Pro Ala Leu Ala Ser Gln
    3140                3145                3150
His Pro Pro Glu Glu Glu Val His Tyr His Leu Pro Val Ala Arg
    3155                3160                3165
Ala Thr Ala Pro Val Gln Ser Glu Val Leu Val Met Gln Ser Glu
    3170                3175                3180
Tyr Arg Leu His Pro Tyr Thr Val Pro Arg Asp Val Arg Ile Met
    3185                3190                3195
Val His Pro His Val Thr Ala Val Ser Glu Gln Pro Arg Ala Ala
    3200                3205                3210
Asp Gly Val Val Lys Val Pro Pro Ala Ser Lys Ala Pro Gln Gln
    3215                3220                3225
Pro Gly Lys Glu Ala Ala Lys Thr Pro Asp Ala Lys Ala Ala Pro
    3230                3235                3240
Thr Pro Thr Pro Ala Pro Val Pro Val Pro Val Pro Leu Pro Ala
    3245                3250                3255
Pro Ala Pro Ala Pro His Gly Glu Ala Arg Ile Leu Thr Val Thr
    3260                3265                3270
Pro Ser Asn Gln Leu Gln Gly Leu Pro Leu Thr Pro Pro Val Val
    3275                3280                3285
Val Thr His Gly Val Gln Ile Val His Ser Ser Gly Glu Leu Phe
    3290                3295                3300
Gln Glu Tyr Arg Tyr Gly Asp Ile Arg Thr Tyr His Pro Pro Ala
    3305                3310                3315
Gln Leu Thr His Thr Gln Phe Pro Ala Ala Ser Ser Val Gly Leu
    3320                3325                3330
Pro Ser Arg Thr Lys Thr Ala Ala Gln Gly Pro Pro Pro Glu Gly
    3335                3340                3345
Glu Pro Leu Gln Pro Pro Gln Pro Val Gln Ser Thr Gln Pro Ala
    3350                3355                3360
Gln Pro Ala Pro Pro Cys Pro Pro Ser Gln Leu Gly Gln Pro Gly
    3365                3370                3375
Gln Pro Pro Ser Ser Lys Met Pro Gln Val Ser Gln Glu Ala Lys
    3380                3385                3390
Gly Thr Gln Thr Gly Val Glu Gln Pro Arg Leu Pro Ala Gly Pro
    3395                3400                3405
Ala Asn Arg Pro Pro Glu Pro His Thr Gln Val Gln Arg Ala Gln
    3410                3415                3420
Ala Glu Thr Gly Pro Thr Ser Phe Pro Ser Pro Val Ser Val Ser
    3425                3430                3435
Met Lys Pro Asp Leu Pro Val Ser Leu Pro Thr Gln Thr Ala Pro
    3440                3445                3450
Lys Gln Pro Leu Phe Val Pro Thr Thr Ser Gly Pro Ser Thr Pro
    3455                3460                3465
Pro Gly Leu Val Leu Pro His Thr Glu Phe Gln Pro Ala Pro Lys
    3470                3475                3480
Gln Asp Ser Ser Pro His Leu Thr Ser Gln Arg Pro Val Asp Met
    3485                3490                3495
Val Gln Leu Leu Lys Lys Tyr Pro Ile Val Trp Gln Gly Leu Leu
    3500                3505                3510
Ala Leu Lys Asn Asp Thr Ala Ala Val Gln Leu His Phe Val Ser
    3515                3520                3525
Gly Asn Asn Val Leu Ala His Arg Ser Leu Pro Leu Ser Glu Gly
    3530                3535                3540
Gly Pro Pro Leu Arg Ile Ala Gln Arg Met Arg Leu Glu Ala Thr
    3545                3550                3555
Gln Leu Glu Gly Val Ala Arg Arg Met Thr Val Glu Thr Asp Tyr
    3560                3565                3570
Cys Leu Leu Leu Ala Leu Pro Cys Gly Arg Asp Gln Glu Asp Val
    3575                3580                3585
Val Ser Gln Thr Glu Ser Leu Lys Ala Ala Phe Ile Thr Tyr Leu
    3590                3595                3600
Gln Ala Lys Gln Ala Ala Gly Ile Ile Asn Val Pro Asn Pro Gly
    3605                3610                3615
Ser Asn Gln Pro Ala Tyr Val Leu Gln Ile Phe Pro Pro Cys Glu

3620                 3625                 3630
Phe Ser  Glu Ser His Leu Ser  Arg Leu Ala Pro Asp  Leu Leu Ala
    3635                 3640                 3645
Ser Ile  Ser Asn Ile Ser Pro  His Leu Met Ile Val  Ile Ala Ser
    3650                 3655                 3660
Val

<210>  88
<211>  1616
<212>  PRT
<213>  Homo sapiens
<220>
<221>  misc_feature
<223>  CDS de la séquence GS-N36: 92...4942 ( GS-P36) (PLPP)

<220>
<221>  misc_feature
<223>  nom: Protéine liée au potentiel de prolifération

<400>  88
Met Gly Ile Lys Thr Leu Asn Leu Val Leu Gly Leu Lys Arg Ala Leu
1               5                   10                  15
Glu Phe Pro Glu Val Phe Met Met Glu Val Lys Asp Pro Asn Met Lys
            20                  25                  30
Gly Ala Met Leu Thr Asn Thr Gly Lys Tyr Ala Ile Pro Thr Ile Asp
        35                  40                  45
Ala Glu Ala Tyr Ala Ile Gly Lys Lys Glu Lys Pro Pro Phe Leu Pro
    50                  55                  60
Glu Glu Pro Ser Ser Ser Ser Glu Glu Asp Asp Pro Ile Pro Asp Glu
65                  70                  75                  80
Leu Leu Cys Leu Ile Cys Lys Asp Ile Met Thr Asp Ala Val Val Ile
                85                  90                  95
Pro Cys Cys Gly Asn Ser Tyr Cys Asp Glu Cys Ile Arg Thr Ala Leu
            100                 105                 110
Leu Glu Ser Asp Glu His Thr Cys Pro Thr Cys His Gln Asn Asp Val
        115                 120                 125
Ser Pro Asp Ala Leu Ile Ala Asn Lys Phe Leu Arg Gln Ala Val Asn
    130                 135                 140
Asn Phe Lys Asn Glu Thr Gly Tyr Thr Lys Arg Leu Arg Lys Gln Leu
145                 150                 155                 160
Pro Pro Pro Pro Pro Pro Ile Pro Pro Pro Arg Pro Leu Ile Gln Arg
                165                 170                 175
Asn Leu Gln Pro Leu Met Arg Ser Pro Ile Ser Arg Gln Gln Asp Pro
            180                 185                 190
Leu Met Ile Pro Val Thr Ser Ser Ser Thr His Pro Ala Pro Ser Ile
        195                 200                 205
Ser Ser Leu Thr Ser Asn Gln Ser Ser Leu Ala Pro Pro Val Ser Gly
    210                 215                 220
Asn Pro Ser Ser Ala Pro Ala Pro Val Pro Asp Ile Thr Ala Thr Val
225                 230                 235                 240
Ser Ile Ser Val His Ser Glu Lys Ser Asp Gly Pro Phe Arg Asp Ser
                245                 250                 255
Asp Asn Lys Ile Leu Pro Ala Ala Ala Leu Ala Ser Glu His Ser Lys
            260                 265                 270
Gly Thr Ser Ser Ile Ala Ile Thr Ala Leu Met Glu Glu Lys Gly Tyr
        275                 280                 285
Gln Val Pro Val Leu Gly Thr Pro Ser Leu Leu Gly Gln Ser Leu Leu
    290                 295                 300
His Gly Gln Leu Ile Pro Thr Thr Gly Pro Val Arg Ile Asn Thr Ala
305                 310                 315                 320
Arg Pro Gly Gly Gly Arg Pro Gly Trp Glu His Ser Asn Lys Leu Gly
                325                 330                 335
Tyr Leu Val Ser Pro Pro Gln Gln Ile Arg Arg Gly Glu Arg Ser Cys
            340                 345                 350
Tyr Arg Ser Ile Asn Arg Gly Arg His His Ser Glu Arg Ser Gln Arg
        355                 360                 365
Thr Gln Gly Pro Ser Leu Pro Ala Thr Pro Val Phe Val Pro Val Pro
    370                 375                 380
Pro Pro Pro Leu Tyr Pro Pro Pro Pro His Thr Leu Pro Leu Pro Pro
385                 390                 395                 400
Gly Val Pro Pro Pro Gln Phe Ser Pro Gln Phe Pro Pro Gly Gln Pro

                405                 410                 415
Pro Pro Ala Gly Tyr Ser Val Pro Pro Pro Gly Phe Pro Pro Ala Pro
            420                 425                 430
Ala Asn Leu Ser Thr Pro Trp Val Ser Ser Gly Val Gln Thr Ala His
        435                 440                 445
Ser Asn Thr Ile Pro Thr Thr Gln Ala Pro Pro Leu Ser Arg Glu Glu
    450                 455                 460
Phe Tyr Arg Glu Gln Arg Arg Leu Lys Glu Glu Glu Lys Lys Lys Ser
465                 470                 475                 480
Lys Leu Asp Glu Phe Thr Asn Asp Phe Ala Lys Glu Leu Met Glu Tyr
                485                 490                 495
Lys Lys Ile Gln Lys Glu Arg Arg Arg Ser Phe Ser Arg Ser Lys Ser
            500                 505                 510
Pro Tyr Ser Gly Ser Ser Tyr Ser Arg Ser Ser Tyr Thr Tyr Ser Lys
        515                 520                 525
Ser Arg Ser Gly Ser Thr Arg Ser Arg Ser Tyr Ser Arg Ser Phe Ser
    530                 535                 540
Arg Ser His Ser Arg Ser Tyr Ser Arg Ser Pro Pro Tyr Pro Arg Arg
545                 550                 555                 560
Gly Arg Gly Lys Ser Arg Asn Tyr Arg Ser Arg Ser Arg Ser His Gly
                565                 570                 575
Tyr His Arg Ser Arg Ser Arg Ser Pro Pro Tyr Arg Arg Tyr His Ser
            580                 585                 590
Arg Ser Arg Ser Pro Gln Ala Phe Arg Gly Gln Ser Pro Asn Lys Arg
        595                 600                 605
Asn Val Pro Gln Gly Glu Thr Glu Arg Glu Tyr Phe Asn Arg Tyr Arg
    610                 615                 620
Glu Val Pro Pro Pro Tyr Asp Met Lys Ala Tyr Tyr Gly Arg Ser Val
625                 630                 635                 640
Asp Phe Arg Asp Pro Phe Glu Lys Glu Arg Tyr Arg Glu Trp Glu Arg
                645                 650                 655
Lys Tyr Arg Glu Trp Tyr Glu Lys Tyr Tyr Lys Gly Tyr Ala Ala Gly
            660                 665                 670
Ala Gln Pro Arg Pro Ser Ala Asn Arg Glu Asn Phe Ser Pro Glu Arg
        675                 680                 685
Phe Leu Pro Leu Asn Ile Arg Asn Ser Pro Phe Thr Arg Gly Arg Arg
    690                 695                 700
Glu Asp Tyr Val Gly Gly Gln Ser His Arg Ser Arg Asn Ile Gly Ser
705                 710                 715                 720
Asn Tyr Pro Glu Lys Leu Ser Ala Arg Asp Gly His Asn Gln Lys Asp
                725                 730                 735
Asn Thr Lys Ser Lys Glu Lys Glu Ser Glu Asn Ala Pro Gly Asp Gly
            740                 745                 750
Lys Gly Asn Lys His Lys Lys His Arg Lys Arg Arg Lys Gly Glu Glu
        755                 760                 765
Ser Glu Gly Phe Leu Asn Pro Glu Leu Leu Glu Thr Ser Arg Lys Ser
    770                 775                 780
Arg Glu Pro Thr Gly Val Glu Glu Asn Lys Thr Asp Ser Leu Phe Val
785                 790                 795                 800
Leu Pro Ser Arg Asp Asp Ala Thr Pro Val Arg Asp Glu Pro Met Asp
                805                 810                 815
Ala Glu Ser Ile Thr Phe Lys Ser Val Ser Glu Lys Asp Lys Arg Glu
            820                 825                 830
Arg Asp Lys Pro Lys Ala Lys Gly Asp Lys Thr Lys Arg Lys Asn Asp
        835                 840                 845
Gly Ser Ala Val Ser Lys Lys Glu Asn Ile Val Lys Pro Ala Lys Gly
    850                 855                 860
Pro Gln Glu Lys Val Asp Gly Glu Arg Glu Arg Ser Pro Arg Ser Glu
865                 870                 875                 880
Pro Pro Ile Lys Lys Ala Lys Glu Glu Thr Pro Lys Thr Asp Asn Thr
                885                 890                 895
Lys Ser Ser Ser Ser Ser Gln Lys Asp Glu Lys Ile Thr Gly Thr Pro
            900                 905                 910
Arg Lys Ala His Ser Lys Ser Ala Lys Glu His Gln Glu Thr Lys Pro
        915                 920                 925
Val Lys Glu Glu Lys Val Lys Lys Asp Tyr Ser Lys Asp Val Lys Ser
    930                 935                 940
Glu Lys Leu Thr Thr Lys Glu Glu Lys Ala Lys Lys Pro Asn Glu Lys
945                 950                 955                 960
Asn Lys Pro Leu Asp Asn Lys Gly Glu Lys Arg Lys Arg Lys Thr Glu
                965                 970                 975
Glu Lys Gly Val Asp Lys Asp Phe Glu Ser Ser Ser Met Lys Ile Ser
            980                 985                 990

Lys Leu Glu Val Thr Glu Ile Val Lys Pro Ser Pro Lys Arg Lys Met
        995                 1000                1005
Glu Pro Asp Thr Glu Lys Met Asp Arg Thr Pro Glu Lys Asp Lys
    1010                1015                1020
Ile Ser Leu Ser Ala Pro Ala Lys Lys Ile Lys Leu Asn Arg Glu
    1025                1030                1035
Thr Gly Lys Lys Ile Gly Ser Thr Glu Asn Ile Ser Asn Thr Lys
    1040                1045                1050
Glu Pro Ser Glu Lys Leu Glu Ser Thr Ser Ser Lys Val Lys Gln
    1055                1060                1065
Glu Lys Val Lys Gly Lys Val Arg Arg Lys Val Thr Gly Thr Glu
    1070                1075                1080
Gly Ser Ser Ser Thr Leu Val Asp Tyr Thr Ser Thr Ser Ser Thr
    1085                1090                1095
Gly Gly Ser Pro Val Arg Lys Ser Glu Glu Lys Thr Asp Thr Lys
    1100                1105                1110
Arg Thr Val Ile Lys Thr Met Glu Glu Tyr Asn Asn Asp Asn Thr
    1115                1120                1125
Ala Pro Ala Glu Asp Val Ile Ile Met Ile Gln Val Pro Gln Ser
    1130                1135                1140
Lys Trp Asp Lys Asp Asp Phe Glu Ser Glu Glu Glu Asp Val Lys
    1145                1150                1155
Ser Thr Gln Pro Ile Ser Ser Val Gly Lys Pro Ala Ser Val Ile
    1160                1165                1170
Lys Asn Val Ser Thr Lys Pro Ser Asn Ile Val Lys Tyr Pro Glu
    1175                1180                1185
Lys Glu Ser Glu Pro Ser Glu Lys Ile Gln Lys Phe Thr Lys Asp
    1190                1195                1200
Val Ser His Glu Ile Ile Gln His Glu Val Lys Ser Ser Lys Asn
    1205                1210                1215
Ser Ala Ser Ser Glu Lys Gly Lys Thr Lys Asp Arg Asp Tyr Ser
    1220                1225                1230
Val Leu Glu Lys Glu Asn Pro Glu Lys Arg Lys Asn Ser Thr Gln
    1235                1240                1245
Pro Glu Lys Glu Ser Asn Leu Asp Arg Leu Asn Glu Gln Gly Asn
    1250                1255                1260
Phe Lys Ser Leu Ser Gln Ser Ser Lys Glu Ala Arg Thr Ser Asp
    1265                1270                1275
Lys His Asp Ser Thr Arg Ala Ser Ser Asn Lys Asp Phe Thr Pro
    1280                1285                1290
Asn Arg Asp Lys Lys Thr Asp Tyr Asp Thr Arg Glu Tyr Ser Ser
    1295                1300                1305
Ser Lys Arg Arg Asp Glu Lys Asn Glu Leu Thr Arg Arg Lys Asp
    1310                1315                1320
Ser Pro Ser Arg Asn Lys Asp Ser Ala Ser Gly Gln Lys Asn Lys
    1325                1330                1335
Pro Arg Glu Glu Arg Asp Leu Pro Lys Lys Gly Thr Gly Asp Ser
    1340                1345                1350
Lys Lys Ser Asn Ser Ser Pro Ser Arg Asp Arg Lys Pro His Asp
    1355                1360                1365
His Lys Ala Thr Tyr Asp Thr Lys Arg Pro Asn Glu Glu Thr Lys
    1370                1375                1380
Ser Val Asp Lys Asn Pro Cys Lys Asp Arg Glu Lys His Val Leu
    1385                1390                1395
Glu Ala Arg Asn Asn Lys Glu Ser Ser Gly Asn Lys Leu Leu Tyr
    1400                1405                1410
Ile Leu Asn Pro Pro Glu Thr Gln Val Glu Lys Glu Gln Ile Thr
    1415                1420                1425
Gly Gln Ile Asp Lys Ser Thr Val Lys Pro Lys Pro Gln Leu Ser
    1430                1435                1440
His Ser Ser Arg Leu Ser Ser Asp Leu Thr Arg Glu Thr Asp Glu
    1445                1450                1455
Ala Ala Phe Glu Pro Asp Tyr Asn Glu Ser Asp Ser Glu Ser Asn
    1460                1465                1470
Val Ser Val Lys Glu Glu Glu Ser Ser Gly Asn Ile Ser Lys Asp
    1475                1480                1485
Leu Lys Asp Lys Ile Val Glu Lys Ala Lys Glu Ser Leu Asp Thr
    1490                1495                1500
Ala Ala Val Val Gln Val Gly Ile Ser Arg Asn Gln Ser His Ser
    1505                1510                1515
Ser Pro Ser Val Ser Pro Ser Arg Ser His Ser Pro Ser Gly Ser
    1520                1525                1530
Gln Thr Arg Ser His Ser Ser Ser Ala Ser Ser Ala Glu Ser Gln

    1535                1540                1545
Asp Ser  Lys Lys Lys Lys Lys  Lys Lys Glu Lys Lys  Lys His Lys
    1550                1555                1560
Lys His  Lys Lys His Lys Lys  His Lys Lys His Ala  Gly Thr Glu
    1565                1570                1575
Val Glu  Leu Glu Lys Ser Gln  Lys His Lys His Lys  Lys Lys Lys
    1580                1585                1590
Ser Lys  Lys Asn Lys Asp Lys  Glu Lys Glu Lys Glu  Lys Asp Asp
    1595                1600                1605
Gln Lys  Val Lys Ser Val Thr  Val
    1610                1615


<210>  89
<211>  914
<212>  PRT
<213>  Homo sapiens
<220>
<221>  misc_feature
<223>  CDS de la séquence GS-N37: 245...2989 ( GS-P37)

<220>
<221>  misc_feature
<223>  nom:protéine 1 interagissant avecla protéine  huntingtine (HIP1)

<400>  89
Met Ser Arg Met Trp Gly His Leu Ser Glu Gly Tyr Gly Gln Leu Cys
1               5                   10                  15
Ser Ile Tyr Leu Lys Leu Leu Arg Thr Lys Met Glu Tyr His Thr Lys
            20                  25                  30
Asn Pro Arg Phe Pro Gly Asn Leu Gln Met Ser Asp Arg Gln Leu Asp
        35                  40                  45
Glu Ala Gly Glu Ser Asp Val Asn Asn Phe Phe Gln Leu Thr Val Glu
    50                  55                  60
Met Phe Asp Tyr Leu Glu Cys Glu Leu Asn Leu Phe Gln Thr Val Phe
65                  70                  75                  80
Asn Ser Leu Asp Met Ser Arg Ser Val Ser Val Thr Ala Ala Gly Gln
                85                  90                  95
Cys Arg Leu Ala Pro Leu Ile Gln Val Ile Leu Asp Cys Ser His Leu
            100                 105                 110
Tyr Asp Tyr Thr Val Lys Leu Leu Phe Lys Leu His Ser Cys Leu Pro
        115                 120                 125
Ala Asp Thr Leu Gln Gly His Arg Asp Arg Phe Met Glu Gln Phe Thr
    130                 135                 140
Lys Leu Lys Asp Leu Phe Tyr Arg Ser Ser Asn Leu Gln Tyr Phe Lys
145                 150                 155                 160
Arg Leu Ile Gln Ile Pro Gln Leu Pro Glu Asn Pro Pro Asn Phe Leu
                165                 170                 175
Arg Ala Ser Ala Leu Ser Glu His Ile Ser Pro Val Val Val Ile Pro
            180                 185                 190
Ala Glu Ala Ser Ser Pro Asp Ser Glu Pro Val Leu Glu Lys Asp Asp
        195                 200                 205
Leu Met Asp Met Asp Ala Ser Gln Gln Asn Leu Phe Asp Asn Lys Phe
    210                 215                 220
Asp Asp Ile Phe Gly Ser Ser Phe Ser Ser Asp Pro Phe Asn Phe Asn
225                 230                 235                 240
Ser Gln Asn Gly Val Asn Lys Asp Glu Lys Asp His Leu Ile Glu Arg
                245                 250                 255
Leu Tyr Arg Glu Ile Ser Gly Leu Lys Ala Gln Leu Glu Asn Met Lys
            260                 265                 270
Thr Glu Ser Gln Arg Val Val Leu Gln Leu Lys Gly His Val Ser Glu
        275                 280                 285
Leu Glu Ala Asp Leu Ala Glu Gln Gln His Leu Arg Gln Gln Ala Ala
    290                 295                 300
Asp Asp Cys Glu Phe Leu Arg Ala Glu Leu Asp Glu Leu Arg Arg Gln
305                 310                 315                 320
Arg Glu Asp Thr Glu Lys Ala Gln Arg Ser Leu Ser Glu Ile Glu Arg
                325                 330                 335
Lys Ala Gln Ala Asn Glu Gln Arg Tyr Ser Lys Leu Lys Glu Lys Tyr
            340                 345                 350
Ser Glu Leu Val Gln Asn His Ala Asp Leu Leu Arg Lys Asn Ala Glu
        355                 360                 365
Val Thr Lys Gln Val Ser Met Ala Arg Gln Ala Gln Val Asp Leu Glu

370                 375                 380
Arg Glu Lys Lys Glu Leu Glu Asp Ser Leu Glu Arg Ile Ser Asp Gln
385                 390                 395                 400
Gly Gln Arg Lys Thr Gln Glu Gln Leu Glu Val Leu Glu Ser Leu Lys
                405                 410                 415
Gln Glu Leu Ala Thr Ser Gln Arg Glu Leu Gln Val Leu Gln Gly Ser
            420                 425                 430
Leu Glu Thr Ser Ala Gln Ser Glu Ala Asn Trp Ala Ala Glu Phe Ala
        435                 440                 445
Glu Leu Glu Lys Glu Arg Asp Ser Leu Val Ser Gly Ala Ala His Arg
    450                 455                 460
Glu Glu Glu Leu Ser Ala Leu Arg Lys Glu Leu Gln Asp Thr Gln Leu
465                 470                 475                 480
Lys Leu Ala Ser Thr Glu Glu Ser Met Cys Gln Leu Ala Lys Asp Gln
                485                 490                 495
Arg Lys Met Leu Leu Val Gly Ser Arg Lys Ala Ala Glu Gln Val Ile
            500                 505                 510
Gln Asp Ala Leu Asn Gln Leu Glu Glu Pro Pro Leu Ile Ser Cys Ala
        515                 520                 525
Gly Ser Ala Asp His Leu Leu Ser Thr Val Thr Ser Ile Ser Ser Cys
    530                 535                 540
Ile Glu Gln Leu Glu Lys Ser Trp Ser Gln Tyr Leu Ala Cys Pro Glu
545                 550                 555                 560
Asp Ile Ser Gly Leu Leu His Ser Ile Thr Leu Leu Ala His Leu Thr
                565                 570                 575
Ser Asp Ala Ile Ala His Gly Ala Thr Thr Cys Leu Arg Ala Pro Pro
            580                 585                 590
Glu Pro Ala Asp Ser Leu Thr Glu Ala Cys Lys Gln Tyr Gly Arg Glu
        595                 600                 605
Thr Leu Ala Tyr Leu Ala Ser Leu Glu Glu Glu Gly Ser Leu Glu Asn
    610                 615                 620
Ala Asp Ser Thr Ala Met Arg Asn Cys Leu Ser Lys Ile Lys Ala Ile
625                 630                 635                 640
Gly Glu Glu Leu Leu Pro Arg Gly Leu Asp Ile Lys Gln Glu Glu Leu
                645                 650                 655
Gly Asp Leu Val Asp Lys Glu Met Ala Ala Thr Ser Ala Ala Ile Glu
            660                 665                 670
Thr Ala Thr Ala Arg Ile Glu Glu Met Leu Ser Lys Ser Arg Ala Gly
        675                 680                 685
Asp Thr Gly Val Lys Leu Glu Val Asn Glu Arg Ile Leu Gly Cys Cys
    690                 695                 700
Thr Ser Leu Met Gln Ala Ile Gln Val Leu Ile Val Ala Ser Lys Asp
705                 710                 715                 720
Leu Gln Arg Glu Ile Val Glu Ser Gly Arg Gly Thr Ala Ser Pro Lys
                725                 730                 735
Glu Phe Tyr Ala Lys Asn Ser Arg Trp Thr Glu Gly Leu Ile Ser Ala
            740                 745                 750
Ser Lys Ala Val Gly Trp Gly Ala Thr Val Met Val Asp Ala Ala Asp
        755                 760                 765
Leu Val Val Gln Gly Arg Gly Lys Phe Glu Glu Leu Met Val Cys Ser
    770                 775                 780
His Glu Ile Ala Ala Ser Thr Ala Gln Leu Val Ala Ala Ser Lys Val
785                 790                 795                 800
Lys Ala Asp Lys Asp Ser Pro Asn Leu Ala Gln Leu Gln Gln Ala Ser
                805                 810                 815
Arg Gly Val Asn Gln Ala Thr Ala Gly Val Val Ala Ser Thr Ile Ser
            820                 825                 830
Gly Lys Ser Gln Ile Glu Glu Thr Asp Asn Met Asp Phe Ser Ser Met
        835                 840                 845
Thr Leu Thr Gln Ile Lys Arg Gln Glu Met Asp Ser Gln Val Arg Val
    850                 855                 860
Leu Glu Leu Glu Asn Glu Leu Gln Lys Glu Arg Gln Lys Leu Gly Glu
865                 870                 875                 880
Leu Arg Lys Lys His Tyr Glu Leu Ala Gly Val Ala Glu Gly Trp Glu
                885                 890                 895
Glu Gly Thr Glu Ala Ser Pro Pro Thr Leu Gln Glu Val Val Thr Glu
            900                 905                 910
Lys Glu

<210>  90
<211>  741

<212>  PRT
<213>  Homo sapiens
<220>
<221>  misc_feature
<223>  CDS de la séquence GS-N38: 12..2237 ( GS-P38) (NUP88)

<220>
<221>  misc_feature
<223>  nom: nucleoporine 88kD

<400>  90
Met Ala Ala Ala Glu Gly Pro Val Gly Asp Gly Glu Leu Trp Gln Thr
1               5                   10                  15
Trp Leu Pro Asn His Val Val Phe Leu Arg Leu Arg Glu Gly Leu Lys
            20                  25                  30
Asn Gln Ser Pro Thr Glu Ala Glu Lys Pro Ala Ser Ser Ser Leu Pro
        35                  40                  45
Ser Ser Pro Pro Pro Gln Leu Leu Thr Arg Asn Val Val Phe Gly Leu
    50                  55                  60
Gly Gly Glu Leu Phe Leu Trp Asp Gly Glu Asp Ser Ser Phe Leu Val
65                  70                  75                  80
Val Arg Leu Arg Gly Pro Ser Gly Gly Gly Glu Glu Pro Ala Leu Ser
                85                  90                  95
Gln Tyr Gln Arg Leu Leu Cys Ile Asn Pro Pro Leu Phe Glu Ile Tyr
            100                 105                 110
Gln Val Leu Leu Ser Pro Thr Gln His His Val Ala Leu Ile Gly Ile
        115                 120                 125
Lys Gly Leu Met Val Leu Glu Leu Pro Lys Arg Trp Gly Lys Asn Ser
    130                 135                 140
Glu Phe Glu Gly Gly Lys Ser Thr Val Asn Cys Ser Thr Thr Pro Val
145                 150                 155                 160
Ala Glu Arg Phe Phe Thr Ser Ser Thr Ser Leu Thr Leu Lys His Ala
                165                 170                 175
Ala Trp Tyr Pro Ser Glu Ile Leu Asp Pro His Val Val Leu Leu Thr
            180                 185                 190
Ser Asp Asn Val Ile Arg Ile Tyr Ser Leu Arg Glu Pro Gln Thr Pro
        195                 200                 205
Thr Asn Val Ile Ile Leu Ser Glu Ala Glu Glu Glu Ser Leu Val Leu
    210                 215                 220
Asn Lys Gly Arg Ala Tyr Thr Ala Ser Leu Gly Glu Thr Ala Val Ala
225                 230                 235                 240
Phe Asp Phe Gly Pro Leu Ala Ala Val Pro Lys Thr Leu Phe Gly Gln
                245                 250                 255
Asn Gly Lys Asp Glu Val Val Ala Tyr Pro Leu Tyr Ile Leu Tyr Glu
            260                 265                 270
Asn Gly Glu Thr Phe Leu Thr Tyr Ile Ser Leu Leu His Ser Pro Gly
        275                 280                 285
Asn Ile Gly Lys Leu Leu Gly Pro Leu Pro Met His Pro Ala Ala Glu
    290                 295                 300
Asp Asn Tyr Gly Tyr Asp Ala Cys Ala Val Leu Cys Leu Pro Cys Val
305                 310                 315                 320
Pro Asn Ile Leu Val Ile Ala Thr Glu Ser Gly Met Leu Tyr His Cys
                325                 330                 335
Val Val Leu Glu Gly Glu Glu Glu Asp Asp His Thr Ser Glu Lys Ser
            340                 345                 350
Trp Asp Ser Arg Ile Asp Leu Ile Pro Ser Leu Tyr Val Phe Glu Cys
        355                 360                 365
Val Glu Leu Glu Leu Ala Leu Lys Leu Ala Ser Gly Glu Asp Asp Pro
    370                 375                 380
Phe Asp Ser Asp Phe Ser Cys Pro Val Lys Leu His Arg Asp Pro Lys
385                 390                 395                 400
Cys Pro Ser Arg Tyr His Cys Thr His Glu Ala Gly Val His Ser Val
                405                 410                 415
Gly Leu Thr Trp Ile His Lys Leu His Lys Phe Leu Gly Ser Asp Glu
            420                 425                 430
Glu Asp Lys Asp Ser Leu Gln Glu Leu Ser Thr Glu Gln Lys Cys Phe
        435                 440                 445
Val Glu His Ile Leu Cys Thr Lys Pro Leu Pro Cys Arg Gln Pro Ala
    450                 455                 460
Pro Ile Arg Gly Phe Trp Ile Val Pro Asp Ile Leu Gly Pro Thr Met
465                 470                 475                 480
Ile Cys Ile Thr Ser Thr Tyr Glu Cys Leu Ile Trp Pro Leu Leu Ser
                485                 490                 495

Thr Val His Pro Ala Ser Pro Pro Leu Leu Cys Thr Arg Glu Asp Val
            500                 505                 510
Glu Val Ala Glu Ser Pro Leu Arg Val Leu Ala Glu Thr Pro Asp Ser
        515                 520                 525
Phe Glu Lys His Ile Arg Ser Ile Leu Gln Arg Ser Val Ala Asn Pro
    530                 535                 540
Ala Phe Leu Lys Ala Ser Glu Lys Asp Ile Ala Pro Pro Pro Glu Glu
545                 550                 555                 560
Cys Leu Gln Leu Leu Ser Arg Ala Thr Gln Val Phe Arg Glu Gln Tyr
                565                 570                 575
Ile Leu Lys Gln Asp Leu Ala Lys Glu Glu Ile Gln Arg Arg Val Lys
            580                 585                 590
Leu Leu Cys Asp Gln Lys Lys Lys Gln Leu Glu Asp Leu Ser Tyr Cys
        595                 600                 605
Arg Glu Glu Arg Lys Ser Leu Arg Glu Met Ala Glu Arg Leu Ala Asp
    610                 615                 620
Lys Tyr Glu Glu Ala Lys Glu Lys Gln Glu Asp Ile Met Asn Arg Met
625                 630                 635                 640
Lys Lys Leu Leu His Ser Phe His Ser Glu Leu Pro Val Leu Ser Asp
                645                 650                 655
Ser Glu Arg Asp Met Lys Lys Glu Leu Gln Leu Ile Pro Asp Gln Leu
            660                 665                 670
Arg His Leu Gly Asn Ala Ile Lys Gln Val Thr Met Lys Lys Asp Tyr
        675                 680                 685
Gln Gln Gln Lys Met Glu Lys Val Leu Ser Leu Pro Lys Pro Thr Ile
    690                 695                 700
Ile Leu Ser Ala Tyr Gln Arg Lys Cys Ile Gln Ser Ile Leu Lys Glu
705                 710                 715                 720
Glu Gly Glu His Ile Arg Glu Met Val Lys Gln Ile Asn Asp Ile Arg
                725                 730                 735
Asn His Val Asn Phe
            740


<210>  91
<211>  108
<212>  PRT
<213>  Homo sapiens
<220>
<221>  misc_feature
<223>  CDS de la séquence GS-N39: 86...412 ( GS-P39)

<220>
<221>  misc_feature
<223>  nom: Protein 1A liant FK506, (FKPB1A),

<400>  91
Met Gly Val Gln Val Glu Thr Ile Ser Pro Gly Asp Gly Arg Thr Phe
1               5                   10                  15
Pro Lys Arg Gly Gln Thr Cys Val Val His Tyr Thr Gly Met Leu Glu
            20                  25                  30
Asp Gly Lys Lys Phe Asp Ser Ser Arg Asp Arg Asn Lys Pro Phe Lys
        35                  40                  45
Phe Met Leu Gly Lys Gln Glu Val Ile Arg Gly Trp Glu Glu Gly Val
    50                  55                  60
Ala Gln Met Ser Val Gly Gln Arg Ala Lys Leu Thr Ile Ser Pro Asp
65                  70                  75                  80
Tyr Ala Tyr Gly Ala Thr Gly His Pro Gly Ile Ile Pro Pro His Ala
                85                  90                  95
Thr Leu Val Phe Asp Val Glu Leu Leu Lys Leu Glu
            100                 105


<210>  92
<211>  1182
<212>  PRT
<213>  Homo sapiens
<220>
<221>  misc_feature
<223>  CDS de la séquence GS-N40: 115...3663 ( GS-P40) (SALF)

<220>
<221>  misc_feature

<223>  nom: facteur similaire au facteur "stoned B/TFIIA-alpha/beta"

<400>  92
Met Cys Ser Thr Asn Pro Gly Lys Trp Val Thr Phe Asp Asp Asp Pro
1               5                   10                  15
Ala Val Gln Ser Ser Gln Lys Ser Lys Asn Phe Pro Leu Glu Asn Gln
            20                  25                  30
Gly Val Cys Arg Pro Asn Gly Leu Lys Leu Asn Leu Pro Gly Leu Arg
        35                  40                  45
Glu Phe Pro Ser Gly Ser Ser Ser Thr Ser Ser Thr Pro Leu Ser Ser
    50                  55                  60
Pro Ile Val Asp Phe Tyr Phe Ser Pro Gly Pro Pro Ser Asn Ser Pro
65                  70                  75                  80
Leu Ser Thr Pro Thr Lys Asp Phe Pro Gly Phe Pro Gly Ile Pro Lys
                85                  90                  95
Ala Gly Thr His Val Leu Tyr Pro Ile Pro Glu Ser Ser Ser Asp Ser
            100                 105                 110
Pro Leu Ala Ile Ser Gly Gly Glu Ser Ser Leu Leu Pro Thr Arg Pro
        115                 120                 125
Thr Cys Leu Ser His Ala Leu Leu Pro Ser Asp His Ser Cys Thr His
    130                 135                 140
Pro Thr Pro Lys Val Gly Leu Pro Asp Glu Val Asn Pro Gln Gln Ala
145                 150                 155                 160
Glu Ser Leu Gly Phe Gln Ser Asp Asp Leu Pro Gln Phe Gln Tyr Phe
                165                 170                 175
Arg Glu Asp Cys Ala Phe Ser Ser Pro Phe Trp Lys Asp Glu Gly Ser
            180                 185                 190
Asp Ser His Phe Thr Leu Asp Pro Pro Gly Ser Lys Lys Met Phe Ser
        195                 200                 205
Ser Arg Asn Lys Glu Met Pro Ile Asp Gln Lys Ser Leu Asn Lys Cys
    210                 215                 220
Ser Leu Asn Tyr Ile Cys Glu Lys Leu Glu His Leu Gln Ser Ala Glu
225                 230                 235                 240
Asn Gln Asp Ser Leu Arg Ser Leu Ser Met His Cys Leu Cys Ala Glu
                245                 250                 255
Glu Asn Ala Ser Ser Phe Val Pro His Thr Leu Phe Arg Ser Gln Pro
            260                 265                 270
Lys Ser Gly Trp Ser Phe Met Leu Arg Ile Pro Glu Lys Lys Asn Met
        275                 280                 285
Met Ser Ser Arg Gln Trp Gly Pro Ile Phe Leu Lys Val Leu Pro Gly
    290                 295                 300
Gly Ile Leu Gln Met Tyr Tyr Glu Gln Gly Leu Glu Lys Pro Phe Lys
305                 310                 315                 320
Glu Ile Gln Leu Asp Pro Tyr Cys Arg Leu Ser Glu Pro Lys Val Glu
                325                 330                 335
Asn Phe Ser Val Ala Gly Lys Ile His Thr Val Lys Ile Glu His Val
            340                 345                 350
Ser Tyr Thr Glu Lys Arg Lys Tyr His Ser Lys Thr Glu Val Val His
        355                 360                 365
Glu Pro Asp Ile Glu Gln Met Leu Lys Leu Gly Ser Thr Ser Tyr His
    370                 375                 380
Asp Phe Leu Asp Phe Leu Thr Thr Val Glu Glu Glu Leu Met Lys Leu
385                 390                 395                 400
Pro Ala Val Ser Lys Pro Lys Lys Asn Tyr Glu Glu Gln Glu Ile Ser
                405                 410                 415
Leu Glu Ile Val Asp Asn Phe Trp Gly Lys Val Thr Lys Glu Gly Lys
            420                 425                 430
Phe Val Glu Ser Ala Val Ile Thr Gln Ile Tyr Cys Leu Cys Phe Val
        435                 440                 445
Asn Gly Asn Leu Glu Cys Phe Leu Thr Leu Asn Asp Leu Glu Leu Pro
    450                 455                 460
Lys Arg Asp Glu Ser Tyr Tyr Glu Lys Asp Ser Glu Lys Lys Gly Ile
465                 470                 475                 480
Asp Ile Leu Asp Tyr His Phe His Lys Cys Val Asn Val Gln Glu Phe
                485                 490                 495
Glu Gln Ser Arg Ile Ile Lys Phe Val Pro Leu Asp Ala Cys Arg Phe
            500                 505                 510
Glu Leu Met Arg Phe Lys Thr Leu Tyr Asn Gly Asp Asn Leu Pro Phe
        515                 520                 525
Ser Leu Lys Ser Val Val Val Val Gln Gly Ala Tyr Val Glu Leu Gln
    530                 535                 540
Ala Phe Val Asn Met Ala Ser Leu Ala Gln Arg Ser Ser Tyr Ala Gly
545                 550                 555                 560

Ser Leu Arg Ser Cys Asp Asn Ile Arg Ile His Phe Pro Val Pro Ser
                565                 570                 575
Gln Trp Ile Lys Ala Leu Trp Thr Met Asn Leu Gln Arg Gln Lys Ser
            580                 585                 590
Leu Lys Ala Lys Met Asn Arg Arg Ala Cys Leu Gly Ser Leu Gln Glu
        595                 600                 605
Leu Glu Ser Glu Pro Val Ile Gln Val Thr Val Gly Ser Ala Lys Tyr
    610                 615                 620
Glu Ser Ala Tyr Gln Ala Val Val Trp Lys Ile Asp Arg Leu Pro Asp
625                 630                 635                 640
Lys Asn Ser Ser Leu Asp His Pro His Cys Leu Ser Tyr Lys Leu Glu
                645                 650                 655
Leu Gly Ser Asp Gln Glu Ile Pro Ser Asp Trp Tyr Pro Phe Ala Thr
            660                 665                 670
Val Gln Phe Ser Val Pro Asp Thr Cys Ala Ser Arg Thr Glu Val Arg
        675                 680                 685
Ser Leu Gly Val Glu Ser Asp Val Gln Pro Gln Lys His Val Gln Gln
    690                 695                 700
Arg Ala Cys Tyr Asn Ile Gln Pro Lys Leu Tyr Arg Ser Val Ile Glu
705                 710                 715                 720
Asp Val Ile Glu Gly Val Arg Asn Leu Phe Ala Glu Glu Gly Ile Glu
                725                 730                 735
Glu Gln Val Leu Lys Asp Leu Lys Gln Leu Trp Glu Thr Lys Val Leu
            740                 745                 750
Gln Ser Lys Ala Thr Glu Asp Phe Phe Arg Asn Ser Ile Gln Ser Pro
        755                 760                 765
Leu Phe Thr Leu Gln Leu Pro His Ser Leu His Gln Thr Leu Gln Ser
    770                 775                 780
Ser Thr Ala Ser Leu Val Ile Pro Ala Gly Arg Thr Leu Pro Ser Phe
785                 790                 795                 800
Thr Thr Ala Glu Leu Gly Thr Ser Asn Ser Ser Ala Asn Phe Thr Phe
                805                 810                 815
Pro Gly Tyr Pro Ile His Val Pro Ala Gly Val Thr Leu Gln Thr Val
            820                 825                 830
Ser Gly His Leu Tyr Lys Val Asn Val Pro Ile Met Val Thr Glu Thr
        835                 840                 845
Ser Gly Arg Ala Gly Ile Leu Gln His Pro Ile Gln Gln Val Phe Gln
    850                 855                 860
Gln Leu Gly Gln Pro Ser Val Ile Gln Thr Ser Val Pro Gln Leu Asn
865                 870                 875                 880
Pro Trp Ser Leu Gln Ala Thr Thr Glu Lys Ser Gln Arg Ile Glu Thr
                885                 890                 895
Val Leu Gln Gln Pro Ala Ile Leu Pro Ser Gly Pro Val Asp Arg Lys
            900                 905                 910
His Leu Glu Asn Ala Thr Ser Asp Ile Leu Val Ser Pro Gly Asn Glu
        915                 920                 925
His Lys Ile Val Pro Glu Ala Leu Leu Cys His Gln Glu Ser Ser His
    930                 935                 940
Tyr Ile Ser Leu Pro Gly Val Val Phe Ser Pro Gln Val Ser Gln Thr
945                 950                 955                 960
Asn Ser Asn Val Glu Ser Val Leu Ser Gly Ser Ala Ser Met Ala Gln
                965                 970                 975
Asn Leu His Asp Glu Ser Leu Ser Thr Ser Pro His Gly Ala Leu His
            980                 985                 990
Gln His Val Thr Asp Ile Gln Leu  His Ile Leu Lys Asn  Arg Met Tyr
        995                 1000                1005
Gly Cys  Asp Ser Val Lys Gln  Pro Arg Asn Ile Glu  Glu Pro Ser
    1010                1015                1020
Asn Ile  Pro Val Ser Glu Lys  Asp Ser Asn Ser Gln  Val Asp Leu
    1025                1030                1035
Ser Ile  Arg Val Thr Asp Asp  Asp Ile Gly Glu Ile  Ile Gln Val
    1040                1045                1050
Asp Gly  Ser Gly Asp Thr Ser  Ser Asn Glu Glu Ile  Gly Ser Thr
    1055                1060                1065
Arg Asp  Ala Asp Glu Asn Glu  Phe Leu Gly Asn Ile  Asp Gly Gly
    1070                1075                1080
Asp Leu  Lys Val Pro Glu Glu  Glu Ala Asp Ser Ile  Ser Asn Glu
    1085                1090                1095
Asp Ser  Ala Thr Asn Ser Ser  Asp Asn Glu Asp Pro  Gln Val Asn
    1100                1105                1110
Ile Val  Glu Glu Asp Pro Leu  Asn Ser Gly Asp Asp  Val Ser Glu
    1115                1120                1125
Gln Asp  Val Pro Asp Leu Phe  Asp Thr Asp Asn Val  Ile Val Cys

1130                1135                1140
Gln Tyr Asp Lys Ile His Arg Ser Lys Asn Lys Trp Lys Phe Tyr
    1145                1150                1155
Leu Lys Asp Gly Val Met Cys Phe Gly Gly Arg Asp Tyr Val Phe
    1160                1165                1170
Ala Lys Ala Ile Gly Asp Ala Glu Trp
    1175                1180

<210> 93
<211> 243
<212> PRT
<213> Homo sapiens
<220>
<221> misc_feature
<223> CDS de la séquence GS-N41: 27...758 ( GS-P41)

<220>
<221> misc_feature
<223> nom: Protéine similaire à la protéine P29 interagissant avec GCIP

<400> 93
Met Ala Ala Ile Ala Ala Ser Glu Val Leu Val Asp Ser Ala Glu Glu
1               5                   10                  15
Gly Ser Leu Ala Ala Ala Ala Glu Leu Ala Ala Gln Lys Arg Glu Gln
            20                  25                  30
Arg Leu Arg Lys Phe Arg Glu Leu His Leu Met Arg Asn Glu Ala Arg
        35                  40                  45
Lys Leu Asn His Gln Glu Val Val Glu Glu Asp Lys Arg Leu Lys Leu
    50                  55                  60
Pro Ala Asn Trp Glu Ala Lys Lys Ala Arg Leu Glu Trp Glu Leu Lys
65                  70                  75                  80
Glu Glu Glu Lys Lys Lys Glu Cys Ala Ala Arg Gly Glu Asp Tyr Glu
                85                  90                  95
Lys Val Lys Leu Leu Glu Ile Ser Ala Glu Asp Ala Glu Arg Trp Glu
            100                 105                 110
Arg Lys Lys Lys Arg Lys Asn Pro Asp Leu Gly Phe Ser Asp Tyr Ala
        115                 120                 125
Ala Ala Gln Leu Arg Gln Tyr His Arg Leu Thr Lys Gln Ile Lys Pro
    130                 135                 140
Asp Met Glu Thr Tyr Glu Arg Leu Arg Glu Lys His Gly Glu Glu Phe
145                 150                 155                 160
Phe Pro Thr Ser Asn Ser Leu Leu His Gly Thr His Val Pro Ser Thr
                165                 170                 175
Glu Glu Ile Asp Arg Met Val Ile Asp Leu Glu Lys Gln Ile Glu Lys
            180                 185                 190
Arg Asp Lys Tyr Ser Arg Arg Arg Pro Tyr Asn Asp Asp Ala Asp Ile
        195                 200                 205
Asp Tyr Ile Asn Glu Arg Asn Ala Lys Phe Asn Lys Lys Ala Glu Arg
    210                 215                 220
Phe Tyr Gly Lys Tyr Thr Ala Glu Ile Lys Gln Asn Leu Glu Arg Gly
225                 230                 235                 240
Thr Ala Val

<210> 94
<211> 1383
<212> PRT
<213> Homo sapiens
<220>
<221> misc_feature
<223> CDS de la séquence GS-N42: 149...4300 ( GS-P42)

<220>
<221> misc_feature
<223> nom: Protéine transmembranaire 2(TMEM2)

<400> 94
Met Tyr Ala Thr Asp Ser Arg Gly His Ser Pro Ala Phe Leu Gln Pro
1               5                   10                  15
Gln Asn Gly Asn Ser Arg His Pro Ser Gly Tyr Val Pro Gly Lys Val
            20                  25                  30

Val Pro Leu Arg Pro Pro Pro Pro Pro Lys Ser Gln Ala Ser Ala Lys
        35                  40                  45
Phe Thr Ser Ile Arg Arg Glu Asp Arg Ala Thr Phe Ala Phe Ser Pro
    50                  55                  60
Glu Glu Gln Gln Ala Gln Arg Glu Ser Gln Lys Gln Lys Arg His Lys
65                  70                  75                  80
Asn Thr Phe Ile Cys Phe Ala Ile Thr Ser Phe Ser Phe Phe Ile Ala
                85                  90                  95
Leu Ala Ile Ile Leu Gly Ile Ser Ser Lys Tyr Ala Pro Asp Glu Asn
            100                 105                 110
Cys Pro Asp Gln Asn Pro Arg Leu Arg Asn Trp Asp Pro Gly Gln Asp
        115                 120                 125
Ser Ala Lys Gln Val Val Ile Lys Glu Gly Asp Met Leu Arg Leu Thr
    130                 135                 140
Ser Asp Ala Thr Val His Ser Ile Val Ile Gln Asp Gly Gly Leu Leu
145                 150                 155                 160
Val Phe Gly Asp Asn Lys Asp Gly Ser Arg Asn Ile Thr Leu Arg Thr
                165                 170                 175
His Tyr Ile Leu Ile Gln Asp Gly Gly Ala Leu His Ile Gly Ala Glu
            180                 185                 190
Lys Cys Arg Tyr Lys Ser Lys Ala Thr Ile Thr Leu Tyr Gly Lys Ser
        195                 200                 205
Asp Glu Gly Glu Ser Met Pro Thr Phe Gly Lys Lys Phe Ile Gly Val
    210                 215                 220
Glu Ala Gly Gly Thr Leu Glu Leu His Gly Ala Arg Lys Ala Ser Trp
225                 230                 235                 240
Thr Leu Leu Ala Arg Thr Leu Asn Ser Ser Gly Leu Pro Phe Gly Ser
                245                 250                 255
Tyr Thr Phe Glu Lys Asp Phe Ser Arg Gly Leu Asn Val Arg Val Ile
            260                 265                 270
Asp Gln Asp Thr Ala Lys Ile Leu Glu Ser Glu Arg Phe Asp Thr His
        275                 280                 285
Glu Tyr Arg Asn Glu Ser Arg Arg Leu Gln Glu Phe Leu Arg Phe Gln
    290                 295                 300
Asp Pro Gly Arg Ile Val Ala Ile Ala Val Gly Asp Ser Ala Ala Lys
305                 310                 315                 320
Ser Leu Leu Gln Gly Thr Ile Gln Met Ile Gln Glu Arg Leu Gly Ser
                325                 330                 335
Glu Leu Ile Gln Gly Leu Gly Tyr Arg Gln Ala Trp Ala Leu Val Gly
            340                 345                 350
Val Ile Asp Gly Gly Ser Thr Ser Cys Asn Glu Ser Val Arg Asn Tyr
        355                 360                 365
Glu Asn His Ser Ser Gly Gly Lys Ala Leu Ala Gln Arg Glu Phe Tyr
    370                 375                 380
Thr Val Asp Gly Gln Lys Phe Ser Val Thr Ala Tyr Ser Glu Trp Ile
385                 390                 395                 400
Glu Gly Val Ser Leu Ser Gly Phe Arg Val Glu Val Val Asp Gly Val
                405                 410                 415
Lys Leu Asn Leu Leu Asp Asp Val Ser Ser Trp Lys Pro Gly Asp Gln
            420                 425                 430
Ile Val Val Ala Ser Thr Asp Tyr Ser Met Tyr Gln Ala Glu Glu Phe
        435                 440                 445
Thr Leu Leu Pro Cys Ser Glu Cys Ser His Phe Gln Val Lys Val Lys
    450                 455                 460
Glu Thr Pro Gln Phe Leu His Met Gly Glu Ile Ile Asp Gly Val Asp
465                 470                 475                 480
Met Arg Ala Glu Val Gly Ile Leu Thr Arg Asn Ile Val Ile Gln Gly
                485                 490                 495
Glu Val Glu Asp Ser Cys Tyr Ala Glu Asn Gln Cys Gln Phe Phe Asp
            500                 505                 510
Tyr Asp Thr Phe Gly Gly His Ile Met Ile Met Lys Asn Phe Thr Ser
        515                 520                 525
Val His Leu Ser Tyr Val Glu Leu Lys His Met Gly Gln Gln Gln Met
    530                 535                 540
Gly Arg Tyr Pro Val His Phe His Leu Cys Gly Asp Val Asp Tyr Lys
545                 550                 555                 560
Gly Gly Tyr Arg His Ala Thr Phe Val Asp Gly Leu Ser Ile His His
                565                 570                 575
Ser Phe Ser Arg Cys Ile Thr Val His Gly Thr Asn Gly Leu Leu Ile
            580                 585                 590
Lys Asp Thr Ile Gly Phe Asp Thr Leu Gly His Cys Phe Phe Leu Glu
        595                 600                 605
Asp Gly Ile Glu Gln Arg Asn Thr Leu Phe His Asn Leu Gly Leu Leu

610                 615                 620
Thr Lys Pro Gly Thr Leu Leu Pro Thr Asp Arg Asn Asn Ser Met Cys
625                 630                 635                 640
Thr Thr Met Arg Asp Lys Val Phe Gly Asn Tyr Ile Pro Val Pro Ala
                645                 650                 655
Thr Asp Cys Met Ala Val Ser Thr Phe Trp Ile Ala His Pro Asn Asn
            660                 665                 670
Asn Leu Ile Asn Asn Ala Ala Ala Gly Ser Gln Asp Ala Gly Ile Trp
        675                 680                 685
Tyr Leu Phe His Lys Glu Pro Thr Gly Glu Ser Ser Gly Leu Gln Leu
    690                 695                 700
Leu Ala Lys Pro Glu Leu Thr Pro Leu Gly Ile Phe Tyr Asn Asn Arg
705                 710                 715                 720
Val His Ser Asn Phe Lys Ala Gly Leu Phe Ile Asp Lys Gly Val Lys
                725                 730                 735
Thr Thr Asn Ser Ser Ala Ala Asp Pro Arg Glu Tyr Leu Cys Leu Asp
            740                 745                 750
Asn Ser Ala Arg Phe Arg Pro His Gln Asp Ala Asn Pro Glu Lys Pro
        755                 760                 765
Arg Val Ala Ala Leu Ile Asp Arg Leu Ile Ala Phe Lys Asn Asn Asp
    770                 775                 780
Asn Gly Ala Trp Val Arg Gly Gly Asp Ile Ile Val Gln Asn Ser Ala
785                 790                 795                 800
Phe Ala Asp Asn Gly Ile Gly Leu Thr Phe Ala Ser Asp Gly Ser Phe
                805                 810                 815
Pro Ser Asp Glu Gly Ser Ser Gln Glu Val Ser Glu Ser Leu Phe Val
            820                 825                 830
Gly Glu Ser Arg Asn Tyr Gly Phe Gln Gly Gly Gln Asn Lys Tyr Val
        835                 840                 845
Gly Thr Gly Gly Ile Asp Gln Lys Pro Arg Thr Leu Pro Arg Asn Arg
    850                 855                 860
Thr Phe Pro Ile Arg Gly Phe Gln Ile Tyr Asp Gly Pro Ile His Leu
865                 870                 875                 880
Thr Arg Ser Thr Phe Lys Lys Tyr Val Pro Thr Pro Asp Arg Tyr Ser
                885                 890                 895
Ser Ala Ile Gly Phe Leu Met Lys Asn Ser Trp Gln Ile Thr Pro Arg
            900                 905                 910
Asn Asn Ile Ser Leu Val Lys Phe Gly Pro His Val Ser Leu Asn Val
        915                 920                 925
Phe Phe Gly Lys Pro Gly Pro Trp Phe Glu Asp Cys Glu Met Asp Gly
    930                 935                 940
Asp Lys Asn Ser Ile Phe His Asp Ile Asp Gly Ser Val Thr Gly Tyr
945                 950                 955                 960
Lys Asp Ala Tyr Val Gly Arg Met Asp Asn Tyr Leu Ile Arg His Pro
                965                 970                 975
Ser Cys Val Asn Val Ser Lys Trp Asn Ala Val Ile Cys Ser Gly Thr
            980                 985                 990
Tyr Ala Gln Val Tyr Val Gln Thr  Trp Ser Thr Gln Asn  Leu Ser Met
        995                 1000                1005
Thr Ile  Thr Arg Asp Glu Tyr  Pro Ser Asn Pro Met  Val Leu Arg
    1010                1015                1020
Gly Ile  Asn Gln Lys Ala Ala  Phe Pro Gln Tyr Gln  Pro Val Val
    1025                1030                1035
Met Leu  Glu Lys Gly Tyr Thr  Ile His Trp Asn Gly  Pro Ala Pro
    1040                1045                1050
Arg Thr  Thr Phe Leu Tyr Leu  Val Asn Phe Asn Lys  Asn Asp Trp
    1055                1060                1065
Ile Arg  Val Gly Leu Cys Tyr  Pro Ser Asn Thr Ser  Phe Gln Val
    1070                1075                1080
Thr Phe  Gly Tyr Leu Gln Arg  Gln Asn Gly Ser Leu  Ser Lys Ile
    1085                1090                1095
Glu Glu  Tyr Glu Pro Val His  Ser Leu Glu Glu Leu  Gln Arg Lys
    1100                1105                1110
Gln Ser  Glu Arg Lys Phe Tyr  Phe Asp Ser Ser Thr  Gly Leu Leu
    1115                1120                1125
Phe Leu  Tyr Leu Lys Ala Lys  Ser His Arg His Gly  His Ser Tyr
    1130                1135                1140
Cys Ser  Ser Gln Gly Cys Glu  Arg Val Lys Ile Gln  Ala Ala Thr
    1145                1150                1155
Asp Ser  Lys Asp Ile Ser Asn  Cys Met Ala Lys Ala  Tyr Pro Gln
    1160                1165                1170
Tyr Tyr  Arg Lys Pro Ser Val  Val Lys Arg Met Pro  Ala Met Leu
    1175                1180                1185

Thr Gly Leu Cys Gln Gly Cys Gly Thr Arg Gln Val Val Phe Thr
    1190                1195                1200
Ser Asp Pro His Lys Ser Tyr Leu Pro Val Gln Phe Gln Ser Pro
    1205                1210                1215
Asp Lys Ala Glu Thr Gln Arg Gly Asp Pro Ser Val Ile Ser Val
    1220                1225                1230
Asn Gly Thr Asp Phe Thr Phe Arg Ser Ala Gly Val Leu Leu Leu
    1235                1240                1245
Val Val Asp Pro Cys Ser Val Pro Phe Arg Leu Thr Glu Lys Thr
    1250                1255                1260
Val Phe Pro Leu Ala Asp Val Ser Arg Ile Glu Glu Tyr Leu Lys
    1265                1270                1275
Thr Gly Ile Pro Pro Arg Ser Ile Val Leu Leu Ser Thr Arg Gly
    1280                1285                1290
Glu Ile Lys Gln Leu Asn Ile Ser His Leu Leu Val Pro Leu Gly
    1295                1300                1305
Leu Ala Lys Pro Ala His Leu Tyr Asp Lys Gly Ser Thr Ile Phe
    1310                1315                1320
Leu Gly Phe Ser Gly Asn Phe Lys Pro Ser Trp Thr Lys Leu Phe
    1325                1330                1335
Thr Ser Pro Ala Gly Gln Gly Leu Gly Val Leu Glu Gln Phe Ile
    1340                1345                1350
Pro Leu Gln Leu Asp Glu Tyr Gly Cys Pro Arg Ala Thr Thr Val
    1355                1360                1365
Arg Arg Arg Asp Leu Glu Leu Leu Lys Gln Ala Ser Lys Ala His
    1370                1375                1380

<210>  95
<211>  838
<212>  PRT
<213>  Homo sapiens
<220>
<221>  misc_feature
<223>  CDS de la séquence GS-N43: 318...2834 ( GS-P43)

<220>
<221>  misc_feature
<223>  nom: protéine Dorfine contenant le domaine ring-IBR-ring

<400>  95
Met Gln Glu Gln Glu Ile Gly Phe Ile Ser Lys Tyr Asn Glu Gly Leu
1               5                   10                  15
Cys Val Asn Thr Asp Pro Val Ser Ile Leu Thr Ser Ile Leu Asp Met
            20                  25                  30
Ser Leu His Arg Gln Met Gly Ser Asp Arg Asp Leu Gln Ser Ser Ala
        35                  40                  45
Ser Ser Val Ser Leu Pro Ser Val Lys Lys Ala Pro Lys Lys Arg Arg
    50                  55                  60
Ile Ser Ile Gly Ser Leu Phe Arg Arg Lys Lys Asp Asn Lys Arg Lys
65                  70                  75                  80
Ser Arg Glu Leu Asn Gly Gly Val Asp Gly Ile Ala Ser Ile Glu Ser
                85                  90                  95
Ile His Ser Glu Met Cys Thr Asp Lys Asn Ser Ile Phe Ser Thr Asn
            100                 105                 110
Thr Ser Ser Asp Asn Gly Leu Thr Ser Ile Ser Lys Gln Ile Gly Asp
        115                 120                 125
Phe Ile Glu Cys Pro Leu Cys Leu Leu Arg His Ser Lys Asp Arg Phe
    130                 135                 140
Pro Asp Ile Met Thr Cys His His Arg Ser Cys Val Asp Cys Leu Arg
145                 150                 155                 160
Gln Tyr Leu Arg Ile Glu Ile Ser Glu Ser Arg Val Asn Ile Ser Cys
                165                 170                 175
Pro Glu Cys Thr Glu Arg Phe Asn Pro His Asp Ile Arg Leu Ile Leu
            180                 185                 190
Ser Asp Asp Val Leu Met Glu Lys Tyr Glu Glu Phe Met Leu Arg Arg
        195                 200                 205
Trp Leu Val Ala Asp Pro Asp Cys Arg Trp Cys Pro Ala Pro Asp Cys
    210                 215                 220
Gly Tyr Ala Val Ile Ala Phe Gly Cys Ala Ser Cys Pro Lys Leu Thr
225                 230                 235                 240
Cys Gly Arg Glu Gly Cys Gly Thr Glu Phe Cys Tyr His Cys Lys Gln
                245                 250                 255

Ile Trp His Pro Asn Gln Thr Cys Asp Ala Ala Arg Gln Glu Arg Ala
            260                 265                 270
Gln Ser Leu Arg Leu Arg Thr Ile Arg Ser Ser Ser Ile Ser Tyr Ser
        275                 280                 285
Gln Glu Ser Gly Ala Ala Ala Asp Asp Ile Lys Pro Cys Pro Arg Cys
    290                 295                 300
Ala Ala Tyr Ile Ile Lys Met Asn Asp Gly Ser Cys Asn His Met Thr
305                 310                 315                 320
Cys Ala Val Cys Gly Cys Glu Phe Cys Trp Leu Cys Met Lys Glu Ile
                325                 330                 335
Ser Asp Leu His Tyr Leu Ser Pro Ser Gly Cys Thr Phe Trp Gly Lys
            340                 345                 350
Lys Pro Trp Ser Arg Lys Lys Lys Ile Leu Trp Gln Leu Gly Thr Leu
        355                 360                 365
Val Gly Ala Pro Val Gly Ile Ala Leu Ile Ala Gly Ile Ala Ile Pro
    370                 375                 380
Ala Met Ile Ile Gly Ile Pro Val Tyr Val Gly Arg Lys Ile His Asn
385                 390                 395                 400
Arg Tyr Glu Gly Lys Asp Val Ser Lys His Lys Arg Asn Leu Ala Ile
                405                 410                 415
Ala Gly Gly Val Thr Leu Ser Val Ile Val Ser Pro Val Val Ala Ala
            420                 425                 430
Val Thr Val Gly Ile Gly Val Pro Ile Met Leu Ala Tyr Val Tyr Gly
        435                 440                 445
Val Val Pro Ile Ser Leu Cys Arg Ser Gly Gly Cys Gly Val Ser Ala
    450                 455                 460
Gly Asn Gly Lys Gly Val Arg Ile Glu Phe Asp Asp Glu Asn Asp Ile
465                 470                 475                 480
Asn Val Gly Gly Thr Asn Thr Ala Val Asp Thr Thr Ser Val Ala Glu
                485                 490                 495
Ala Arg His Asn Pro Ser Ile Gly Glu Gly Ser Val Gly Gly Leu Thr
            500                 505                 510
Gly Ser Leu Ser Ala Ser Gly Ser His Met Asp Arg Ile Gly Ala Ile
        515                 520                 525
Arg Asp Asn Leu Ser Glu Thr Ala Ser Thr Met Ala Leu Ala Gly Ala
    530                 535                 540
Ser Ile Thr Gly Ser Leu Ser Gly Ser Ala Met Val Asn Cys Phe Asn
545                 550                 555                 560
Arg Leu Glu Val Gln Ala Asp Val Gln Lys Glu Arg Tyr Ser Leu Ser
                565                 570                 575
Gly Glu Ser Gly Thr Val Ser Leu Gly Thr Val Ser Asp Asn Ala Ser
            580                 585                 590
Thr Lys Ala Met Ala Gly Ser Ile Leu Asn Ser Tyr Ile Pro Leu Asp
        595                 600                 605
Lys Glu Gly Asn Ser Met Glu Val Gln Val Asp Ile Glu Ser Lys Pro
    610                 615                 620
Ser Lys Phe Arg His Asn Ser Gly Ser Ser Ser Val Asp Asp Gly Ser
625                 630                 635                 640
Ala Thr Arg Ser Tyr Ala Gly Gly Ser Ser Ser Gly Leu Pro Glu Gly
                645                 650                 655
Lys Ser Ser Ala Thr Lys Trp Ser Lys Glu Ala Thr Ala Gly Lys Lys
            660                 665                 670
Ser Lys Ser Gly Lys Leu Arg Lys Lys Gly Asn Met Lys Ile Asn Glu
        675                 680                 685
Thr Arg Glu Asp Met Asp Ala Gln Leu Leu Glu Gln Gln Ser Thr Asn
    690                 695                 700
Ser Ser Glu Phe Glu Ala Pro Ser Leu Ser Asp Ser Met Pro Ser Val
705                 710                 715                 720
Ala Asp Ser His Ser Ser His Phe Ser Glu Phe Ser Cys Ser Asp Leu
                725                 730                 735
Glu Ser Met Lys Thr Ser Cys Ser His Gly Ser Ser Asp Tyr His Thr
            740                 745                 750
Arg Phe Ala Thr Val Asn Ile Leu Pro Glu Val Glu Asn Asp Arg Leu
        755                 760                 765
Glu Asn Ser Pro His Gln Cys Ser Ile Ser Val Val Thr Gln Thr Ala
    770                 775                 780
Ser Cys Ser Glu Val Ser Gln Leu Asn His Ile Ala Glu Glu His Gly
785                 790                 795                 800
Asn Asn Gly Ile Lys Pro Asn Val Asp Leu Tyr Phe Gly Asp Ala Leu
                805                 810                 815
Lys Glu Thr Asn Asn Asn His Ser His Gln Thr Met Glu Leu Lys Val
            820                 825                 830
Ala Ile Gln Thr Glu Ile

        835

<210> 96
<211> 244
<212> PRT
<213> Homo sapiens
<220>
<221> misc_feature
<223> CDS de la séquence GS-N44: 81...815 ( GS-P44)

<220>
<221> misc_feature
<223> nom: membre 2 de la superfamille de transmembranaire 4 (TM4SF2)

<400> 96
Met Glu Thr Lys Pro Val Ile Thr Cys Leu Lys Thr Leu Leu Ile Ile
1               5                   10                  15
Tyr Ser Phe Val Phe Trp Ile Thr Gly Val Ile Leu Leu Ala Val Gly
            20                  25                  30
Val Trp Gly Lys Leu Thr Leu Gly Thr Tyr Ile Ser Leu Ile Ala Glu
        35                  40                  45
Asn Ser Thr Asn Ala Pro Tyr Val Leu Ile Gly Thr Gly Thr Thr Ile
    50                  55                  60
Val Val Phe Gly Leu Phe Gly Cys Phe Ala Thr Cys Arg Gly Ser Pro
65                  70                  75                  80
Trp Met Leu Lys Leu Tyr Ala Met Phe Leu Ser Leu Val Phe Leu Ala
                85                  90                  95
Glu Leu Val Ala Gly Ile Ser Gly Phe Val Phe Arg His Glu Ile Lys
            100                 105                 110
Asp Thr Phe Leu Arg Thr Tyr Thr Asp Ala Met Gln Thr Tyr Asn Gly
        115                 120                 125
Asn Asp Glu Arg Ser Arg Ala Val Asp His Val Gln Arg Ser Leu Ser
    130                 135                 140
Cys Cys Gly Val Gln Asn Tyr Thr Asn Trp Ser Thr Ser Pro Tyr Phe
145                 150                 155                 160
Leu Glu His Gly Ile Pro Pro Ser Cys Cys Met Asn Glu Thr Asp Cys
                165                 170                 175
Asn Pro Gln Asp Leu His Asn Leu Thr Val Ala Ala Thr Lys Val Asn
            180                 185                 190
Gln Lys Gly Cys Tyr Asp Leu Val Thr Ser Phe Met Glu Thr Asn Met
        195                 200                 205
Gly Ile Ile Ala Gly Val Ala Phe Gly Ile Ala Phe Ser Gln Leu Ile
    210                 215                 220
Gly Met Leu Leu Ala Cys Cys Leu Ser Arg Phe Ile Thr Ala Asn Gln
225                 230                 235                 240
Tyr Glu Met Val

<210> 97
<211> 517
<212> PRT
<213> Homo sapiens
<220>
<221> misc_feature
<223> CDS de la séquence GS-N45: 184...1737 ( GS-P45)

<220>
<221> misc_feature
<223> nom: Ecto-ATP diphosphohydrolase I

<400> 97
Met Lys Gly Thr Lys Asp Leu Thr Ser Gln Gln Lys Glu Ser Asn Val
1               5                   10                  15
Lys Thr Phe Cys Ser Lys Asn Ile Leu Ala Ile Leu Gly Phe Ser Ser
            20                  25                  30
Ile Ile Ala Val Ile Ala Leu Leu Ala Val Gly Leu Thr Gln Asn Lys
        35                  40                  45
Ala Leu Pro Glu Asn Val Lys Tyr Gly Ile Val Leu Asp Ala Gly Ser
    50                  55                  60
Ser His Thr Ser Leu Tyr Ile Tyr Lys Trp Pro Ala Glu Lys Glu Asn
65                  70                  75                  80

Asp Thr Gly Val Val His Gln Val Glu Glu Cys Arg Val Lys Gly Pro
                85                  90                  95
Gly Ile Ser Lys Phe Val Gln Lys Val Asn Glu Ile Gly Ile Tyr Leu
            100                 105                 110
Thr Asp Cys Met Glu Arg Ala Arg Glu Val Ile Pro Arg Ser Gln His
        115                 120                 125
Gln Glu Thr Pro Val Tyr Leu Gly Ala Thr Ala Gly Met Arg Leu Leu
    130                 135                 140
Arg Met Glu Ser Glu Glu Leu Ala Asp Arg Val Leu Asp Val Val Glu
145                 150                 155                 160
Arg Ser Leu Ser Asn Tyr Pro Phe Asp Phe Gln Gly Ala Arg Ile Ile
                165                 170                 175
Thr Gly Gln Glu Glu Gly Ala Tyr Gly Trp Ile Thr Ile Asn Tyr Leu
            180                 185                 190
Leu Gly Lys Phe Ser Gln Lys Thr Arg Trp Phe Ser Ile Val Pro Tyr
        195                 200                 205
Glu Thr Asn Asn Gln Glu Thr Phe Gly Ala Leu Asp Leu Gly Gly Ala
    210                 215                 220
Ser Thr Gln Val Thr Phe Val Pro Gln Asn Gln Thr Ile Glu Ser Pro
225                 230                 235                 240
Asp Asn Ala Leu Gln Phe Arg Leu Tyr Gly Lys Asp Tyr Asn Val Tyr
                245                 250                 255
Thr His Ser Phe Leu Cys Tyr Gly Lys Asp Gln Ala Leu Trp Gln Lys
            260                 265                 270
Leu Ala Lys Asp Ile Gln Val Ala Ser Asn Glu Ile Leu Arg Asp Pro
        275                 280                 285
Cys Phe His Pro Gly Tyr Lys Lys Val Val Asn Val Ser Asp Leu Tyr
    290                 295                 300
Lys Thr Pro Cys Thr Lys Arg Phe Glu Met Thr Leu Pro Phe Gln Gln
305                 310                 315                 320
Phe Glu Ile Gln Gly Ile Gly Asn Tyr Gln Gln Cys His Gln Ser Ile
                325                 330                 335
Leu Glu Leu Phe Asn Thr Ser Tyr Cys Pro Tyr Ser Gln Cys Ala Phe
            340                 345                 350
Asn Gly Ile Phe Leu Pro Pro Leu Gln Gly Asp Phe Gly Ala Phe Ser
        355                 360                 365
Ala Phe Tyr Phe Val Met Lys Phe Leu Asn Leu Thr Ser Glu Lys Val
    370                 375                 380
Ser Gln Glu Lys Val Thr Glu Met Met Lys Lys Phe Cys Ala Gln Pro
385                 390                 395                 400
Trp Glu Glu Ile Lys Thr Ser Tyr Ala Gly Val Lys Glu Lys Tyr Leu
                405                 410                 415
Ser Glu Tyr Cys Phe Ser Gly Thr Tyr Ile Leu Ser Leu Leu Leu Gln
            420                 425                 430
Gly Tyr His Phe Thr Ala Asp Ser Trp Glu His Ile His Phe Ile Gly
        435                 440                 445
Lys Ile Gln Gly Ser Asp Ala Gly Trp Thr Leu Gly Tyr Met Leu Asn
    450                 455                 460
Leu Thr Asn Met Ile Pro Ala Glu Gln Pro Leu Ser Thr Pro Leu Ser
465                 470                 475                 480
His Ser Thr Tyr Val Phe Leu Met Val Leu Phe Ser Leu Val Leu Phe
                485                 490                 495
Thr Val Ala Ile Ile Gly Leu Leu Ile Phe His Lys Pro Ser Tyr Phe
            500                 505                 510
Trp Lys Asp Met Val
        515


<210>  98
<211>  590
<212>  PRT
<213>  Homo sapiens
<220>
<221>  misc_feature
<223>  CDS de la séquence GS-N46: 56...1828 ( GS-P46)

<220>
<221>  misc_feature
<223>  nom: selenoproteine N

<220>
<221>  misc_feature
<222>  (127)..(127)

<223>  X est un résidu inconnu

<220>
<221>  misc_feature
<222>  (462)..(462)
<223>  X est un résidu inconnu

<400>  98
Met Gly Arg Ala Arg Pro Gly Gln Arg Gly Pro Pro Ser Pro Gly Pro
1               5                   10                  15
Ala Ala Gln Pro Pro Ala Pro Pro Arg Arg Arg Ala Arg Ser Leu Ala
            20                  25                  30
Leu Leu Gly Ala Leu Leu Ala Ala Ala Ala Ala Ala Ala Val Arg Val
        35                  40                  45
Cys Ala Arg His Ala Glu Ala Gln Ala Ala Ala Arg Gln Glu Leu Ala
    50                  55                  60
Leu Lys Thr Leu Gly Thr Asp Gly Leu Phe Leu Phe Ser Ser Leu Asp
65                  70                  75                  80
Thr Asp Gly Asp Met Tyr Ile Ser Pro Glu Glu Phe Lys Pro Ile Ala
                85                  90                  95
Glu Lys Leu Thr Gly Ser Cys Ser Val Thr Gln Thr Gly Val Gln Trp
            100                 105                 110
Cys Ser His Ser Ser Leu Gln Pro Gln Leu Pro Trp Leu Asn Xaa Ser
        115                 120                 125
Ser Cys Leu Ser Leu Leu Arg Ser Thr Pro Ala Ala Ser Tyr Glu Glu
    130                 135                 140
Glu Glu Leu Pro Pro Asp Pro Ser Glu Glu Thr Leu Thr Ile Glu Ala
145                 150                 155                 160
Arg Phe Gln Pro Leu Leu Pro Glu Thr Met Thr Lys Ser Lys Asp Gly
                165                 170                 175
Phe Leu Gly Val Ser Arg Leu Ala Leu Ser Gly Leu Arg Asn Trp Thr
            180                 185                 190
Ala Ala Ala Ser Pro Ser Ala Val Phe Ala Thr Arg His Phe Gln Pro
        195                 200                 205
Phe Leu Pro Pro Pro Gly Gln Glu Leu Gly Glu Pro Trp Trp Ile Ile
    210                 215                 220
Pro Ser Glu Leu Ser Met Phe Thr Gly Tyr Leu Ser Asn Asn Arg Phe
225                 230                 235                 240
Tyr Pro Pro Pro Pro Lys Gly Lys Glu Val Ile Ile His Arg Leu Leu
                245                 250                 255
Ser Met Phe His Pro Arg Pro Phe Val Lys Thr Arg Phe Ala Pro Gln
            260                 265                 270
Gly Ala Val Ala Cys Leu Thr Ala Ile Ser Asp Phe Tyr Tyr Thr Val
        275                 280                 285
Met Phe Arg Ile His Ala Glu Phe Gln Leu Ser Glu Pro Pro Asp Phe
    290                 295                 300
Pro Phe Trp Phe Ser Pro Ala Gln Phe Thr Gly His Ile Ile Leu Ser
305                 310                 315                 320
Lys Asp Ala Thr His Val Arg Asp Phe Arg Leu Phe Val Pro Asn His
                325                 330                 335
Arg Ser Leu Asn Val Asp Met Glu Trp Leu Tyr Gly Ala Ser Glu Ser
            340                 345                 350
Ser Asn Met Glu Val Asp Ile Gly Tyr Ile Pro Gln Met Glu Leu Glu
        355                 360                 365
Ala Thr Gly Pro Ser Val Pro Ser Val Ile Leu Asp Glu Asp Gly Ser
    370                 375                 380
Met Ile Asp Ser His Leu Pro Ser Gly Glu Pro Leu Gln Phe Val Phe
385                 390                 395                 400
Glu Glu Ile Lys Trp Gln Gln Glu Leu Ser Trp Glu Glu Ala Ala Arg
                405                 410                 415
Arg Leu Glu Val Ala Met Tyr Pro Phe Lys Lys Val Ser Tyr Leu Pro
            420                 425                 430
Phe Thr Glu Ala Phe Asp Arg Ala Lys Ala Glu Asn Lys Leu Val His
        435                 440                 445
Ser Ile Leu Leu Trp Gly Ala Leu Asp Asp Gln Ser Cys Xaa Gly Ser
    450                 455                 460
Gly Arg Thr Leu Arg Glu Thr Val Leu Glu Ser Ser Pro Ile Leu Thr
465                 470                 475                 480
Leu Leu Asn Glu Ser Phe Ile Ser Thr Trp Ser Leu Val Lys Glu Leu
                485                 490                 495
Glu Glu Leu Gln Asn Lys Gln Glu Asn Ser Ser His Gln Lys Leu Ala
            500                 505                 510
Gly Leu His Leu Glu Lys Tyr Ser Phe Pro Val Glu Met Met Ile Cys

515                 520                 525
Leu Pro Asn Gly Thr Val Val His His Ile Asn Ala Asn Tyr Phe Leu
    530                 535                 540
Asp Ile Thr Ser Val Lys Pro Glu Glu Ile Glu Ser Asn Leu Phe Ser
545                 550                 555                 560
Phe Ser Ser Thr Phe Glu Asp Pro Ser Thr Ala Thr Tyr Met Gln Phe
                565                 570                 575
Leu Lys Glu Gly Leu Arg Arg Gly Leu Pro Leu Leu Gln Pro
            580                 585                 590


<210>  99
<211>  3969
<212>  PRT
<213>  Homo sapiens
<220>
<221>  misc_feature
<222>  (30)..(30)
<223>  X est un résidu inconnu

<220>
<221>  misc_feature
<223>  CDS de la séquence GS-N48: 1..11910

<220>
<221>  misc_feature
<223>  nom:MLL( " myeloid/lymphoid or mixed-lineage leukemia") (GS-P48)

<400>  99
Met Ala His Ser Cys Arg Trp Arg Phe Pro Ala Arg Pro Gly Thr Thr
1               5                   10                  15
Gly Gly Gly Gly Gly Gly Gly Arg Arg Gly Leu Gly Gly Xaa Pro Arg
            20                  25                  30
Gln Arg Val Pro Ala Leu Leu Leu Pro Pro Gly Pro Pro Val Gly Gly
        35                  40                  45
Gly Gly Pro Gly Ala Pro Pro Ser Pro Pro Ala Val Ala Ala Ala Ala
    50                  55                  60
Ala Ala Ala Gly Ser Ser Gly Ala Gly Val Pro Gly Gly Ala Ala Ala
65                  70                  75                  80
Ala Ser Ala Ala Ser Ser Ser Ser Ala Ser Ser Ser Ser Ser Ser Ser
                85                  90                  95
Ser Ser Ala Ser Ser Gly Pro Ala Leu Leu Arg Val Gly Pro Gly Phe
            100                 105                 110
Asp Ala Ala Leu Gln Val Ser Ala Ala Ile Gly Thr Asn Leu Arg Arg
        115                 120                 125
Phe Arg Ala Val Phe Gly Glu Ser Gly Gly Gly Gly Gly Ser Gly Glu
    130                 135                 140
Asp Glu Gln Phe Leu Gly Phe Gly Ser Asp Glu Glu Val Arg Val Arg
145                 150                 155                 160
Ser Pro Thr Arg Ser Pro Ser Val Lys Thr Ser Pro Arg Lys Pro Arg
                165                 170                 175
Gly Arg Pro Arg Ser Gly Ser Asp Arg Asn Ser Ala Ile Leu Ser Asp
            180                 185                 190
Pro Ser Val Phe Ser Pro Leu Asn Lys Ser Glu Thr Lys Ser Gly Asp
        195                 200                 205
Lys Ile Lys Lys Lys Asp Ser Lys Ser Ile Glu Lys Lys Arg Gly Arg
    210                 215                 220
Pro Pro Thr Phe Pro Gly Val Lys Ile Lys Ile Thr His Gly Lys Asp
225                 230                 235                 240
Ile Ser Glu Leu Pro Lys Gly Asn Lys Glu Asp Ser Leu Lys Lys Ile
                245                 250                 255
Lys Arg Thr Pro Ser Ala Thr Phe Gln Gln Ala Thr Lys Ile Lys Lys
            260                 265                 270
Leu Arg Ala Gly Lys Leu Ser Pro Leu Lys Ser Lys Phe Lys Thr Gly
        275                 280                 285
Lys Leu Gln Ile Gly Arg Lys Gly Val Gln Ile Val Arg Arg Arg Gly
    290                 295                 300
Arg Pro Pro Ser Thr Glu Arg Ile Lys Thr Pro Ser Val Ser Ser Leu
305                 310                 315                 320
Ile Leu Asn Trp Lys Ser Pro Arg Lys Ser Gly Lys Thr Arg Lys Glu
                325                 330                 335
His Leu His Leu Gln Lys Lys Ile Arg Gln Leu Ser Asp Lys Ala Leu
            340                 345                 350

Glu Gly Leu Ser Gln Leu Gly Leu Phe Leu Leu Gln Lys Gly Gln Met
        355                 360                 365
Gln Pro Leu Leu Ser Asn Ser Tyr Arg Gly Gln Lys Lys Gly Ala Gln
    370                 375                 380
Lys Lys Ile Glu Lys Glu Ala Ala Gln Leu Gln Gly Arg Lys Val Lys
385                 390                 395                 400
Thr Gln Val Lys Asn Ile Arg Gln Phe Ile Met Pro Val Val Ser Ala
                405                 410                 415
Ile Ser Ser Arg Ile Ile Lys Thr Pro Arg Arg Phe Ile Glu Asp Glu
            420                 425                 430
Asp Tyr Asp Pro Pro Ile Lys Ile Ala Arg Leu Glu Ser Thr Pro Asn
        435                 440                 445
Ser Arg Phe Ser Ala Pro Ser Cys Gly Ser Ser Glu Lys Ser Ser Ala
    450                 455                 460
Ala Ser Gln His Ser Ser Gln Met Ser Ser Asp Ser Ser Arg Ser Ser
465                 470                 475                 480
Ser Pro Ser Val Asp Thr Ser Thr Asp Ser Gln Ala Ser Glu Glu Ile
                485                 490                 495
Gln Val Leu Pro Glu Glu Arg Ser Asp Thr Pro Glu Val His Pro Pro
            500                 505                 510
Leu Pro Ile Ser Gln Ser Pro Glu Asn Glu Ser Asn Asp Arg Arg Ser
        515                 520                 525
Arg Arg Tyr Ser Val Ser Glu Arg Ser Phe Gly Ser Arg Thr Thr Lys
    530                 535                 540
Lys Leu Ser Thr Leu Gln Ser Ala Pro Gln Gln Gln Thr Ser Ser Ser
545                 550                 555                 560
Pro Pro Pro Pro Leu Leu Thr Pro Pro Pro Pro Leu Gln Pro Ala Ser
                565                 570                 575
Ser Ile Ser Asp His Thr Pro Trp Leu Met Pro Pro Thr Ile Pro Leu
            580                 585                 590
Ala Ser Pro Phe Leu Pro Ala Ser Thr Ala Pro Met Gln Gly Lys Arg
        595                 600                 605
Lys Ser Ile Leu Arg Glu Pro Thr Phe Arg Trp Thr Ser Leu Lys His
    610                 615                 620
Ser Arg Ser Glu Pro Gln Tyr Phe Ser Ser Ala Lys Tyr Ala Lys Glu
625                 630                 635                 640
Gly Leu Ile Arg Lys Pro Ile Phe Asp Asn Phe Arg Pro Pro Pro Leu
                645                 650                 655
Thr Pro Glu Asp Val Gly Phe Ala Ser Gly Phe Ser Ala Ser Gly Thr
            660                 665                 670
Ala Ala Ser Ala Arg Leu Phe Ser Pro Leu His Ser Gly Thr Arg Phe
        675                 680                 685
Asp Met His Lys Arg Ser Pro Leu Leu Arg Ala Pro Arg Phe Thr Pro
    690                 695                 700
Ser Glu Ala His Ser Arg Ile Phe Glu Ser Val Thr Leu Pro Ser Asn
705                 710                 715                 720
Arg Thr Ser Ala Gly Thr Ser Ser Ser Gly Val Ser Asn Arg Lys Arg
                725                 730                 735
Lys Arg Lys Val Phe Ser Pro Ile Arg Ser Glu Pro Arg Ser Pro Ser
            740                 745                 750
His Ser Met Arg Thr Arg Ser Gly Arg Leu Ser Ser Ser Glu Leu Ser
        755                 760                 765
Pro Leu Thr Pro Pro Ser Ser Val Ser Ser Ser Leu Ser Ile Ser Val
    770                 775                 780
Ser Pro Leu Ala Thr Ser Ala Leu Asn Pro Thr Phe Thr Phe Pro Ser
785                 790                 795                 800
His Ser Leu Thr Gln Ser Gly Glu Ser Ala Glu Lys Asn Gln Arg Pro
                805                 810                 815
Arg Lys Gln Thr Ser Ala Pro Ala Glu Pro Phe Ser Ser Ser Ser Pro
            820                 825                 830
Thr Pro Leu Phe Pro Trp Phe Thr Pro Gly Ser Gln Thr Glu Arg Gly
        835                 840                 845
Arg Asn Lys Asp Lys Ala Pro Glu Glu Leu Ser Lys Asp Arg Asp Ala
    850                 855                 860
Asp Lys Ser Val Glu Lys Asp Lys Ser Arg Glu Arg Asp Arg Glu Arg
865                 870                 875                 880
Glu Lys Glu Asn Lys Arg Glu Ser Arg Lys Glu Lys Arg Lys Lys Gly
                885                 890                 895
Ser Glu Ile Gln Ser Ser Ser Ala Leu Tyr Pro Val Gly Arg Val Ser
            900                 905                 910
Lys Glu Lys Val Val Gly Glu Asp Val Ala Thr Ser Ser Ser Ala Lys
        915                 920                 925
Lys Ala Thr Gly Arg Lys Lys Ser Ser Ser His Asp Ser Gly Thr Asp

930                 935                 940
Ile Thr Ser Val Thr Leu Gly Asp Thr Thr Ala Val Lys Thr Lys Ile
945                 950                 955                 960
Leu Ile Lys Lys Gly Arg Gly Asn Leu Glu Lys Thr Asn Leu Asp Leu
                965                 970                 975
Gly Pro Thr Ala Pro Ser Leu Glu Lys Glu Lys Thr Leu Cys Leu Ser
            980                 985                 990
Thr Pro Ser Ser Ser Thr Val Lys  His Ser Thr Ser Ser  Ile Gly Ser
        995                 1000                1005
Met Leu  Ala Gln Ala Asp Lys  Leu Pro Met Thr Asp  Lys Arg Val
    1010                1015                1020
Ala Ser  Leu Leu Lys Lys Ala  Lys Ala Gln Leu Cys  Lys Ile Glu
    1025                1030                1035
Lys Ser  Lys Ser Leu Lys Gln  Thr Asp Gln Pro Lys  Ala Gln Gly
    1040                1045                1050
Gln Glu  Ser Asp Ser Ser Glu  Thr Ser Val Arg Gly  Pro Arg Ile
    1055                1060                1065
Lys His  Val Cys Arg Arg Ala  Ala Val Ala Leu Gly  Arg Lys Arg
    1070                1075                1080
Ala Val  Phe Pro Asp Asp Met  Pro Thr Leu Ser Ala  Leu Pro Trp
    1085                1090                1095
Glu Glu  Arg Glu Lys Ile Leu  Ser Ser Met Gly Asn  Asp Asp Lys
    1100                1105                1110
Ser Ser  Ile Ala Gly Ser Glu  Asp Ala Glu Pro Leu  Ala Pro Pro
    1115                1120                1125
Ile Lys  Pro Ile Lys Pro Val  Thr Arg Asn Lys Ala  Pro Gln Glu
    1130                1135                1140
Pro Pro  Val Lys Lys Gly Arg  Arg Ser Arg Arg Cys  Gly Gln Cys
    1145                1150                1155
Pro Gly  Cys Gln Val Pro Glu  Asp Cys Gly Val Cys  Thr Asn Cys
    1160                1165                1170
Leu Asp  Lys Pro Lys Phe Gly  Gly Arg Asn Ile Lys  Lys Gln Cys
    1175                1180                1185
Cys Lys  Met Arg Lys Cys Gln  Asn Leu Gln Trp Met  Pro Ser Lys
    1190                1195                1200
Ala Tyr  Leu Gln Lys Gln Ala  Lys Ala Val Lys Lys  Lys Glu Lys
    1205                1210                1215
Lys Ser  Lys Thr Ser Glu Lys  Lys Asp Ser Lys Glu  Ser Ser Val
    1220                1225                1230
Val Lys  Asn Val Val Asp Ser  Ser Gln Lys Pro Thr  Pro Ser Ala
    1235                1240                1245
Arg Glu  Asp Pro Ala Pro Lys  Lys Ser Ser Ser Glu  Pro Pro Pro
    1250                1255                1260
Arg Lys  Pro Val Glu Glu Lys  Ser Glu Glu Gly Asn  Val Ser Ala
    1265                1270                1275
Pro Gly  Pro Glu Ser Lys Gln  Ala Thr Thr Pro Ala  Ser Arg Lys
    1280                1285                1290
Ser Ser  Lys Gln Val Ser Gln  Pro Ala Leu Val Ile  Pro Pro Gln
    1295                1300                1305
Pro Pro  Thr Thr Gly Pro Pro  Arg Lys Glu Val Pro  Lys Thr Thr
    1310                1315                1320
Pro Ser  Glu Pro Lys Lys Lys  Gln Pro Pro Pro Pro  Glu Ser Gly
    1325                1330                1335
Pro Glu  Gln Ser Lys Gln Lys  Lys Val Ala Pro Arg  Pro Ser Ile
    1340                1345                1350
Pro Val  Lys Gln Lys Pro Lys  Glu Lys Glu Lys Pro  Pro Pro Val
    1355                1360                1365
Asn Lys  Gln Glu Asn Ala Gly  Thr Leu Asn Ile Leu  Ser Thr Leu
    1370                1375                1380
Ser Asn  Gly Asn Ser Ser Lys  Gln Lys Ile Pro Ala  Asp Gly Val
    1385                1390                1395
His Arg  Ile Arg Val Asp Phe  Lys Glu Asp Cys Glu  Ala Glu Asn
    1400                1405                1410
Val Trp  Glu Met Gly Gly Leu  Gly Ile Leu Thr Ser  Val Pro Ile
    1415                1420                1425
Thr Pro  Arg Val Val Cys Phe  Leu Cys Ala Ser Ser  Gly His Val
    1430                1435                1440
Glu Phe  Val Tyr Cys Gln Val  Cys Cys Glu Pro Phe  His Lys Phe
    1445                1450                1455
Cys Leu  Glu Glu Asn Glu Arg  Pro Leu Glu Asp Gln  Leu Glu Asn
    1460                1465                1470
Trp Cys  Cys Arg Arg Cys Lys  Phe Cys His Val Cys  Gly Arg Gln
    1475                1480                1485

His Gln Ala Thr Lys Gln Leu Leu Glu Cys Asn Lys Cys Arg Asn
    1490                1495                1500
Ser Tyr His Pro Glu Cys Leu Gly Pro Asn Tyr Pro Thr Lys Pro
    1505                1510                1515
Thr Lys Lys Lys Lys Val Trp Ile Cys Thr Lys Cys Val Arg Cys
    1520                1525                1530
Lys Ser Cys Gly Ser Thr Thr Pro Gly Lys Gly Trp Asp Ala Gln
    1535                1540                1545
Trp Ser His Asp Phe Ser Leu Cys His Asp Cys Ala Lys Leu Phe
    1550                1555                1560
Ala Lys Gly Asn Phe Cys Pro Leu Cys Asp Lys Cys Tyr Asp Asp
    1565                1570                1575
Asp Asp Tyr Glu Ser Lys Met Met Gln Cys Gly Lys Cys Asp Arg
    1580                1585                1590
Trp Val His Ser Lys Cys Glu Asn Leu Ser Asp Glu Met Tyr Glu
    1595                1600                1605
Ile Leu Ser Asn Leu Pro Glu Ser Val Ala Tyr Thr Cys Val Asn
    1610                1615                1620
Cys Thr Glu Arg His Pro Ala Glu Trp Arg Leu Ala Leu Glu Lys
    1625                1630                1635
Glu Leu Gln Ile Ser Leu Lys Gln Val Leu Thr Ala Leu Leu Asn
    1640                1645                1650
Ser Arg Thr Thr Ser His Leu Leu Arg Tyr Arg Gln Ala Ala Lys
    1655                1660                1665
Pro Pro Asp Leu Asn Pro Glu Thr Glu Glu Ser Ile Pro Ser Arg
    1670                1675                1680
Ser Ser Pro Glu Gly Pro Asp Pro Pro Val Leu Thr Glu Val Ser
    1685                1690                1695
Lys Gln Asp Asp Gln Gln Pro Leu Asp Leu Glu Gly Val Lys Arg
    1700                1705                1710
Lys Met Asp Gln Gly Asn Tyr Thr Ser Val Leu Glu Phe Ser Asp
    1715                1720                1725
Asp Ile Val Lys Ile Ile Gln Ala Ala Ile Asn Ser Asp Gly Gly
    1730                1735                1740
Gln Pro Glu Ile Lys Lys Ala Asn Ser Met Val Lys Ser Phe Phe
    1745                1750                1755
Ile Arg Gln Met Glu Arg Val Phe Pro Trp Phe Ser Val Lys Lys
    1760                1765                1770
Ser Arg Phe Trp Glu Pro Asn Lys Val Ser Ser Asn Ser Gly Met
    1775                1780                1785
Leu Pro Asn Ala Val Leu Pro Pro Ser Leu Asp His Asn Tyr Ala
    1790                1795                1800
Gln Trp Gln Glu Arg Glu Glu Asn Ser His Thr Glu Gln Pro Pro
    1805                1810                1815
Leu Met Lys Lys Ile Ile Pro Ala Pro Lys Pro Lys Gly Pro Gly
    1820                1825                1830
Glu Pro Asp Ser Pro Thr Pro Leu His Pro Pro Thr Pro Pro Ile
    1835                1840                1845
Leu Ser Thr Asp Arg Ser Arg Glu Asp Ser Pro Glu Leu Asn Pro
    1850                1855                1860
Pro Pro Gly Ile Glu Asp Asn Arg Gln Cys Ala Leu Cys Leu Thr
    1865                1870                1875
Tyr Gly Asp Asp Ser Ala Asn Asp Ala Gly Arg Leu Leu Tyr Ile
    1880                1885                1890
Gly Gln Asn Glu Trp Thr His Val Asn Cys Ala Leu Trp Ser Ala
    1895                1900                1905
Glu Val Phe Glu Asp Asp Asp Gly Ser Leu Lys Asn Val His Met
    1910                1915                1920
Ala Val Ile Arg Gly Lys Gln Leu Arg Cys Glu Phe Cys Gln Lys
    1925                1930                1935
Pro Gly Ala Thr Val Gly Cys Cys Leu Thr Ser Cys Thr Ser Asn
    1940                1945                1950
Tyr His Phe Met Cys Ser Arg Ala Lys Asn Cys Val Phe Leu Asp
    1955                1960                1965
Asp Lys Lys Val Tyr Cys Gln Arg His Arg Asp Leu Ile Lys Gly
    1970                1975                1980
Glu Val Val Pro Glu Asn Gly Phe Glu Val Phe Arg Arg Val Phe
    1985                1990                1995
Val Asp Phe Glu Gly Ile Ser Leu Arg Arg Lys Phe Leu Asn Gly
    2000                2005                2010
Leu Glu Pro Glu Asn Ile His Met Met Ile Gly Ser Met Thr Ile
    2015                2020                2025
Asp Cys Leu Gly Ile Leu Asn Asp Leu Ser Asp Cys Glu Asp Lys

2030                2035                2040
Leu Phe Pro Ile Gly Tyr Gln Cys Ser Arg Val Tyr Trp Ser Thr
    2045                2050                2055
Thr Asp Ala Arg Lys Arg Cys Val Tyr Thr Cys Lys Ile Val Glu
    2060                2065                2070
Cys Arg Pro Pro Val Val Glu Pro Asp Ile Asn Ser Thr Val Glu
    2075                2080                2085
His Asp Glu Asn Arg Thr Ile Ala His Ser Pro Thr Ser Phe Thr
    2090                2095                2100
Glu Ser Ser Ser Lys Glu Ser Gln Asn Thr Ala Glu Ile Ile Ser
    2105                2110                2115
Pro Pro Ser Pro Asp Arg Pro Pro His Ser Gln Thr Ser Gly Ser
    2120                2125                2130
Cys Tyr Tyr His Val Ile Ser Lys Val Pro Arg Ile Arg Thr Pro
    2135                2140                2145
Ser Tyr Ser Pro Thr Gln Arg Ser Pro Gly Cys Arg Pro Leu Pro
    2150                2155                2160
Ser Ala Gly Ser Pro Thr Pro Thr Thr His Glu Ile Val Thr Val
    2165                2170                2175
Gly Asp Pro Leu Leu Ser Ser Gly Leu Arg Ser Ile Gly Ser Arg
    2180                2185                2190
Arg His Ser Thr Ser Ser Leu Ser Pro Gln Arg Ser Lys Leu Arg
    2195                2200                2205
Ile Met Ser Pro Met Arg Thr Gly Asn Thr Tyr Ser Arg Asn Asn
    2210                2215                2220
Val Ser Ser Val Ser Thr Thr Gly Thr Ala Thr Asp Leu Glu Ser
    2225                2230                2235
Ser Ala Lys Val Val Asp His Val Leu Gly Pro Leu Asn Ser Ser
    2240                2245                2250
Thr Ser Leu Gly Gln Asn Thr Ser Thr Ser Ser Asn Leu Gln Arg
    2255                2260                2265
Thr Val Val Thr Val Gly Asn Lys Asn Ser His Leu Asp Gly Ser
    2270                2275                2280
Ser Ser Ser Glu Met Lys Gln Ser Ser Ala Ser Asp Leu Val Ser
    2285                2290                2295
Lys Ser Ser Ser Leu Lys Gly Glu Lys Thr Lys Val Leu Ser Ser
    2300                2305                2310
Lys Ser Ser Glu Gly Ser Ala His Asn Val Ala Tyr Pro Gly Ile
    2315                2320                2325
Pro Lys Leu Ala Pro Gln Val His Asn Thr Thr Ser Arg Glu Leu
    2330                2335                2340
Asn Val Ser Lys Ile Gly Ser Phe Ala Glu Pro Ser Ser Val Ser
    2345                2350                2355
Phe Ser Ser Lys Glu Ala Leu Ser Phe Pro His Leu His Leu Arg
    2360                2365                2370
Gly Gln Arg Asn Asp Arg Asp Gln His Thr Asp Ser Thr Gln Ser
    2375                2380                2385
Ala Asn Ser Ser Pro Asp Glu Asp Thr Glu Val Lys Thr Leu Lys
    2390                2395                2400
Leu Ser Gly Met Ser Asn Arg Ser Ser Ile Ile Asn Glu His Met
    2405                2410                2415
Gly Ser Ser Ser Arg Asp Arg Arg Gln Lys Gly Lys Lys Ser Cys
    2420                2425                2430
Lys Glu Thr Phe Lys Glu Lys His Ser Ser Lys Ser Phe Leu Glu
    2435                2440                2445
Pro Gly Gln Val Thr Thr Gly Glu Glu Gly Asn Leu Lys Pro Glu
    2450                2455                2460
Phe Met Asp Glu Val Leu Thr Pro Glu Tyr Met Gly Gln Arg Pro
    2465                2470                2475
Cys Asn Asn Val Ser Ser Asp Lys Ile Gly Asp Lys Gly Leu Ser
    2480                2485                2490
Met Pro Gly Val Pro Lys Ala Pro Pro Met Gln Val Glu Gly Ser
    2495                2500                2505
Ala Lys Glu Leu Gln Ala Pro Arg Lys Arg Thr Val Lys Val Thr
    2510                2515                2520
Leu Thr Pro Leu Lys Met Glu Asn Glu Ser Gln Ser Lys Asn Ala
    2525                2530                2535
Leu Lys Glu Ser Ser Pro Ala Ser Pro Leu Gln Ile Glu Ser Thr
    2540                2545                2550
Ser Pro Thr Glu Pro Ile Ser Ala Ser Glu Asn Pro Gly Asp Gly
    2555                2560                2565
Pro Val Ala Gln Pro Ser Pro Asn Asn Thr Ser Cys Gln Asp Ser
    2570                2575                2580

Gln Ser Asn Asn Tyr Gln Asn Leu Pro Val Gln Asp Arg Asn Leu
    2585                2590                2595
Met Leu Pro Asp Gly Pro Lys Pro Gln Glu Asp Gly Ser Phe Lys
    2600                2605                2610
Arg Arg Tyr Pro Arg Arg Ser Ala Arg Ala Arg Ser Asn Met Phe
    2615                2620                2625
Phe Gly Leu Thr Pro Leu Tyr Gly Val Arg Ser Tyr Gly Glu Glu
    2630                2635                2640
Asp Ile Pro Phe Tyr Ser Ser Ser Thr Gly Lys Lys Arg Gly Lys
    2645                2650                2655
Arg Ser Ala Glu Gly Gln Val Asp Gly Ala Asp Asp Leu Ser Thr
    2660                2665                2670
Ser Asp Glu Asp Asp Leu Tyr Tyr Tyr Asn Phe Thr Arg Thr Val
    2675                2680                2685
Ile Ser Ser Gly Gly Glu Glu Arg Leu Ala Ser His Asn Leu Phe
    2690                2695                2700
Arg Glu Glu Glu Gln Cys Asp Leu Pro Lys Ile Ser Gln Leu Asp
    2705                2710                2715
Gly Val Asp Asp Gly Thr Glu Ser Asp Thr Ser Val Thr Ala Thr
    2720                2725                2730
Thr Arg Lys Ser Ser Gln Ile Pro Lys Arg Asn Gly Lys Glu Asn
    2735                2740                2745
Gly Thr Glu Asn Leu Lys Ile Asp Arg Pro Glu Asp Ala Gly Glu
    2750                2755                2760
Lys Glu His Val Thr Lys Ser Ser Val Gly His Lys Asn Glu Pro
    2765                2770                2775
Lys Met Asp Asn Cys His Ser Val Ser Arg Val Lys Thr Gln Gly
    2780                2785                2790
Gln Asp Ser Leu Glu Ala Gln Leu Ser Ser Leu Glu Ser Ser Arg
    2795                2800                2805
Arg Val His Thr Ser Thr Pro Ser Asp Lys Asn Leu Leu Asp Thr
    2810                2815                2820
Tyr Asn Thr Glu Leu Leu Lys Ser Asp Ser Asp Asn Asn Asn Ser
    2825                2830                2835
Asp Asp Cys Gly Asn Ile Leu Pro Ser Asp Ile Met Asp Phe Val
    2840                2845                2850
Leu Lys Asn Thr Pro Ser Met Gln Ala Leu Gly Glu Ser Pro Glu
    2855                2860                2865
Ser Ser Ser Ser Glu Leu Leu Asn Leu Gly Glu Gly Leu Gly Leu
    2870                2875                2880
Asp Ser Asn Arg Glu Lys Asp Met Gly Leu Phe Glu Val Phe Ser
    2885                2890                2895
Gln Gln Leu Pro Thr Thr Glu Pro Val Asp Ser Ser Val Ser Ser
    2900                2905                2910
Ser Ile Ser Ala Glu Glu Gln Phe Glu Leu Pro Leu Glu Leu Pro
    2915                2920                2925
Ser Asp Leu Ser Val Leu Thr Thr Arg Ser Pro Thr Val Pro Ser
    2930                2935                2940
Gln Asn Pro Ser Arg Leu Ala Val Ile Ser Asp Ser Gly Glu Lys
    2945                2950                2955
Arg Val Thr Ile Thr Glu Lys Ser Val Ala Ser Ser Glu Ser Asp
    2960                2965                2970
Pro Ala Leu Leu Ser Pro Gly Val Asp Pro Thr Pro Glu Gly His
    2975                2980                2985
Met Thr Pro Asp His Phe Ile Gln Gly His Met Asp Ala Asp His
    2990                2995                3000
Ile Ser Ser Pro Pro Cys Gly Ser Val Glu Gln Gly His Gly Asn
    3005                3010                3015
Asn Gln Asp Leu Thr Arg Asn Ser Ser Thr Pro Gly Leu Gln Val
    3020                3025                3030
Pro Val Ser Pro Thr Val Pro Ile Gln Asn Gln Lys Tyr Val Pro
    3035                3040                3045
Asn Ser Thr Asp Ser Pro Gly Pro Ser Gln Ile Ser Asn Ala Ala
    3050                3055                3060
Val Gln Thr Thr Pro Pro His Leu Lys Pro Ala Thr Glu Lys Leu
    3065                3070                3075
Ile Val Val Asn Gln Asn Met Gln Pro Leu Tyr Val Leu Gln Thr
    3080                3085                3090
Leu Pro Asn Gly Val Thr Gln Lys Ile Gln Leu Thr Ser Ser Val
    3095                3100                3105
Ser Ser Thr Pro Ser Val Met Glu Thr Asn Thr Ser Val Leu Gly
    3110                3115                3120
Pro Met Gly Gly Gly Leu Thr Leu Thr Thr Gly Leu Asn Pro Ser

3125                3130                3135
Leu Pro  Thr Ser Gln Ser Leu  Phe Pro Ser Ala Ser  Lys Gly Leu
    3140                3145                3150
Leu Pro  Met Ser His His Gln  His Leu His Ser Phe  Pro Ala Ala
    3155                3160                3165
Thr Gln  Ser Ser Phe Pro Pro  Asn Ile Ser Asn Pro  Pro Ser Gly
    3170                3175                3180
Leu Leu  Ile Gly Val Gln Pro  Pro Pro Asp Pro Gln  Leu Leu Val
    3185                3190                3195
Ser Glu  Ser Ser Gln Arg Thr  Asp Leu Ser Thr Thr  Val Ala Thr
    3200                3205                3210
Pro Ser  Ser Gly Leu Lys Lys  Arg Pro Ile Ser Arg  Leu Gln Thr
    3215                3220                3225
Arg Lys  Asn Lys Lys Leu Ala  Pro Ser Ser Thr Pro  Ser Asn Ile
    3230                3235                3240
Ala Pro  Ser Asp Val Val Ser  Asn Met Thr Leu Ile  Asn Phe Thr
    3245                3250                3255
Pro Ser  Gln Leu Pro Asn His  Pro Ser Leu Leu Asp  Leu Gly Ser
    3260                3265                3270
Leu Asn  Thr Ser Ser His Arg  Thr Val Pro Asn Ile  Ile Lys Arg
    3275                3280                3285
Ser Lys  Ser Ser Ile Met Tyr  Phe Glu Pro Ala Pro  Leu Leu Pro
    3290                3295                3300
Gln Ser  Val Gly Gly Thr Ala  Ala Thr Ala Ala Gly  Thr Ser Thr
    3305                3310                3315
Ile Ser  Gln Asp Thr Ser His  Leu Thr Ser Gly Ser  Val Ser Gly
    3320                3325                3330
Leu Ala  Ser Ser Ser Ser Val  Leu Asn Val Val Ser  Met Gln Thr
    3335                3340                3345
Thr Thr  Thr Pro Thr Ser Ser  Ala Ser Val Pro Gly  His Val Thr
    3350                3355                3360
Leu Thr  Asn Pro Arg Leu Leu  Gly Thr Pro Asp Ile  Gly Ser Ile
    3365                3370                3375
Ser Asn  Leu Leu Ile Lys Ala  Ser Gln Gln Ser Leu  Gly Ile Gln
    3380                3385                3390
Asp Gln  Pro Val Ala Leu Pro  Pro Ser Ser Gly Met  Phe Pro Gln
    3395                3400                3405
Leu Gly  Thr Ser Gln Thr Pro  Ser Thr Ala Ala Ile  Thr Ala Ala
    3410                3415                3420
Ser Ser  Ile Cys Val Leu Pro  Ser Thr Gln Thr Thr  Gly Ile Thr
    3425                3430                3435
Ala Ala  Ser Pro Ser Gly Glu  Ala Asp Glu His Tyr  Gln Leu Gln
    3440                3445                3450
His Val  Asn Gln Leu Leu Ala  Ser Lys Thr Gly Ile  His Ser Ser
    3455                3460                3465
Gln Arg  Asp Leu Asp Ser Ala  Ser Gly Pro Gln Val  Ser Asn Phe
    3470                3475                3480
Thr Gln  Thr Val Asp Ala Pro  Asn Ser Met Gly Leu  Glu Gln Asn
    3485                3490                3495
Lys Ala  Leu Ser Ser Ala Val  Gln Ala Ser Pro Thr  Ser Pro Gly
    3500                3505                3510
Gly Ser  Pro Ser Ser Pro Ser  Ser Gly Gln Arg Ser  Ala Ser Pro
    3515                3520                3525
Ser Val  Pro Gly Pro Thr Lys  Pro Lys Pro Lys Thr  Lys Arg Phe
    3530                3535                3540
Gln Leu  Pro Leu Asp Lys Gly  Asn Gly Lys Lys His  Lys Val Ser
    3545                3550                3555
His Leu  Arg Thr Ser Ser Ser  Glu Ala His Ile Pro  Asp Gln Glu
    3560                3565                3570
Thr Thr  Ser Leu Thr Ser Gly  Thr Gly Thr Pro Gly  Ala Glu Ala
    3575                3580                3585
Glu Gln  Gln Asp Thr Ala Ser  Val Glu Gln Ser Ser  Gln Lys Glu
    3590                3595                3600
Cys Gly  Gln Pro Ala Gly Gln  Val Ala Val Leu Pro  Glu Val Gln
    3605                3610                3615
Val Thr  Gln Asn Pro Ala Asn  Glu Gln Glu Ser Ala  Glu Pro Lys
    3620                3625                3630
Thr Val  Glu Glu Glu Glu Ser  Asn Phe Ser Ser Pro  Leu Met Leu
    3635                3640                3645
Trp Leu  Gln Gln Glu Gln Lys  Arg Lys Glu Ser Ile  Thr Glu Lys
    3650                3655                3660
Lys Pro  Lys Lys Gly Leu Val  Phe Glu Ile Ser Ser  Asp Asp Gly
    3665                3670                3675

Phe Gln Ile Cys Ala Glu Ser Ile Glu Asp Ala Trp Lys Ser Leu
    3680                3685                3690
Thr Asp Lys Val Gln Glu Ala Arg Ser Asn Ala Arg Leu Lys Gln
    3695                3700                3705
Leu Ser Phe Ala Gly Val Asn Gly Leu Arg Met Leu Gly Ile Leu
    3710                3715                3720
His Asp Ala Val Val Phe Leu Ile Glu Gln Leu Ser Gly Ala Lys
    3725                3730                3735
His Cys Arg Asn Tyr Lys Phe Arg Phe His Lys Pro Glu Glu Ala
    3740                3745                3750
Asn Glu Pro Pro Leu Asn Pro His Gly Ser Ala Arg Ala Glu Val
    3755                3760                3765
His Leu Arg Lys Ser Ala Phe Asp Met Phe Asn Phe Leu Ala Ser
    3770                3775                3780
Lys His Arg Gln Pro Pro Glu Tyr Asn Pro Asn Asp Glu Glu Glu
    3785                3790                3795
Glu Glu Val Gln Leu Lys Ser Ala Arg Arg Ala Thr Ser Met Asp
    3800                3805                3810
Leu Pro Met Pro Met Arg Phe Arg His Leu Lys Lys Thr Ser Lys
    3815                3820                3825
Glu Ala Val Gly Val Tyr Arg Ser Pro Ile His Gly Arg Gly Leu
    3830                3835                3840
Phe Cys Lys Arg Asn Ile Asp Ala Gly Glu Met Val Ile Glu Tyr
    3845                3850                3855
Ala Gly Asn Val Ile Arg Ser Ile Gln Thr Asp Lys Arg Glu Lys
    3860                3865                3870
Tyr Tyr Asp Ser Lys Gly Ile Gly Cys Tyr Met Phe Arg Ile Asp
    3875                3880                3885
Asp Ser Glu Val Val Asp Ala Thr Met His Gly Asn Arg Ala Arg
    3890                3895                3900
Phe Ile Asn His Ser Cys Glu Pro Asn Cys Tyr Ser Arg Val Ile
    3905                3910                3915
Asn Ile Asp Gly Gln Lys His Ile Val Ile Phe Ala Met Arg Lys
    3920                3925                3930
Ile Tyr Arg Gly Glu Glu Leu Thr Tyr Asp Tyr Lys Phe Pro Ile
    3935                3940                3945
Glu Asp Ala Ser Asn Lys Leu Pro Cys Asn Cys Gly Ala Lys Lys
    3950                3955                3960
Cys Arg Lys Phe Leu Asn
    3965


<210>  100
<211>  2288
<212>  PRT
<213>  Homo sapiens
<220>
<221>  misc_feature
<223>  CDS de la séquence GS-N49: 950..7816 (GS-P49)

<220>
<221>  misc_feature
<223>  nom: nom: hélicase et ATPase dépendante de l'ADN (ATRX)

<400>  100
Met Ser Asp Asp Ile Ser Arg Asp Ser Asp Gly Met Asp Glu Gln Cys
1               5                   10                  15
Arg Trp Cys Ala Glu Gly Gly Asn Leu Ile Cys Cys Asp Phe Cys His
            20                  25                  30
Asn Ala Phe Cys Lys Lys Cys Ile Leu Arg Asn Leu Gly Arg Lys Glu
        35                  40                  45
Leu Ser Thr Ile Met Asp Glu Asn Asn Gln Trp Tyr Cys Tyr Ile Cys
    50                  55                  60
His Pro Glu Pro Leu Leu Asp Leu Val Thr Ala Cys Asn Ser Val Phe
65                  70                  75                  80
Glu Asn Leu Glu Gln Leu Leu Gln Gln Asn Lys Lys Lys Ile Lys Val
                85                  90                  95
Asp Ser Glu Lys Ser Asn Lys Val Tyr Glu His Thr Ser Arg Phe Ser
            100                 105                 110
Pro Lys Lys Thr Ser Ser Asn Cys Asn Gly Glu Glu Lys Lys Leu Asp
        115                 120                 125
Asp Ser Cys Ser Gly Ser Val Thr Tyr Ser Tyr Ser Ala Leu Ile Val
    130                 135                 140

Pro Lys Glu Met Ile Lys Lys Ala Lys Lys Leu Ile Glu Thr Thr Ala
145                 150                 155                 160
Asn Met Asn Ser Ser Tyr Val Lys Phe Leu Lys Gln Ala Thr Asp Asn
                165                 170                 175
Ser Glu Ile Ser Ser Ala Thr Lys Leu Arg Gln Leu Lys Ala Phe Lys
            180                 185                 190
Ser Val Leu Ala Asp Ile Lys Lys Ala His Leu Ala Leu Glu Glu Asp
        195                 200                 205
Leu Asn Ser Glu Phe Arg Ala Met Asp Ala Val Asn Lys Glu Lys Asn
    210                 215                 220
Thr Lys Glu His Lys Val Ile Asp Ala Lys Phe Glu Thr Lys Ala Arg
225                 230                 235                 240
Lys Gly Glu Lys Pro Cys Ala Leu Glu Lys Lys Asp Ile Ser Lys Ser
                245                 250                 255
Glu Ala Lys Leu Ser Arg Lys Gln Val Asp Ser Glu His Met His Gln
            260                 265                 270
Asn Val Pro Thr Glu Glu Gln Arg Thr Asn Lys Ser Thr Gly Gly Glu
        275                 280                 285
His Lys Lys Ser Asp Arg Lys Glu Glu Pro Gln Tyr Glu Pro Ala Asn
    290                 295                 300
Thr Ser Glu Asp Leu Asp Met Asp Ile Val Ser Val Pro Ser Ser Val
305                 310                 315                 320
Pro Glu Asp Ile Phe Glu Asn Leu Glu Thr Ala Met Glu Val Gln Ser
                325                 330                 335
Ser Val Asp His Gln Gly Asp Gly Ser Ser Gly Thr Glu Gln Glu Val
            340                 345                 350
Glu Ser Ser Ser Val Lys Leu Asn Ile Ser Ser Lys Asp Asn Arg Gly
        355                 360                 365
Gly Ile Lys Ser Lys Thr Thr Ala Lys Val Thr Lys Glu Leu Tyr Val
    370                 375                 380
Lys Leu Thr Pro Val Ser Leu Ser Asn Ser Pro Ile Lys Gly Ala Asp
385                 390                 395                 400
Cys Gln Glu Val Pro Gln Asp Lys Asp Gly Tyr Lys Ser Cys Gly Leu
                405                 410                 415
Asn Pro Lys Leu Glu Lys Cys Gly Leu Gly Gln Glu Asn Ser Asp Asn
            420                 425                 430
Glu His Leu Val Glu Asn Glu Val Ser Leu Leu Leu Glu Glu Ser Asp
        435                 440                 445
Leu Arg Arg Ser Pro Arg Val Lys Thr Thr Pro Leu Arg Arg Pro Thr
    450                 455                 460
Glu Thr Asn Pro Val Thr Ser Asn Ser Asp Glu Glu Cys Asn Glu Thr
465                 470                 475                 480
Val Lys Glu Lys Gln Lys Leu Ser Val Pro Val Arg Lys Lys Asp Lys
                485                 490                 495
Arg Asn Ser Ser Asp Ser Ala Ile Asp Asn Pro Lys Pro Asn Lys Leu
            500                 505                 510
Pro Lys Ser Lys Gln Ser Glu Thr Val Asp Gln Asn Ser Asp Ser Asp
        515                 520                 525
Glu Met Leu Ala Ile Leu Lys Glu Val Ser Arg Met Ser His Ser Ser
    530                 535                 540
Ser Ser Asp Thr Asp Ile Asn Glu Ile His Thr Asn His Lys Thr Leu
545                 550                 555                 560
Tyr Asp Leu Lys Thr Gln Ala Gly Lys Asp Asp Lys Gly Lys Arg Lys
                565                 570                 575
Arg Lys Ser Ser Thr Ser Gly Ser Asp Phe Asp Thr Lys Lys Gly Lys
            580                 585                 590
Ser Ala Lys Ser Ser Ile Ile Ser Lys Lys Lys Arg Gln Thr Gln Ser
        595                 600                 605
Glu Ser Ser Asn Tyr Asp Ser Glu Leu Glu Lys Glu Ile Lys Ser Met
    610                 615                 620
Ser Lys Ile Gly Ala Ala Arg Thr Thr Lys Lys Arg Ile Pro Asn Thr
625                 630                 635                 640
Lys Asp Phe Asp Ser Ser Glu Asp Glu Lys His Ser Lys Lys Gly Met
                645                 650                 655
Asp Asn Gln Gly His Lys Asn Leu Lys Thr Ser Gln Glu Gly Ser Ser
            660                 665                 670
Asp Asp Ala Glu Arg Lys Gln Glu Arg Glu Thr Phe Ser Ser Ala Glu
        675                 680                 685
Gly Thr Val Asp Lys Asp Thr Thr Ile Met Glu Leu Arg Asp Arg Leu
    690                 695                 700
Pro Lys Lys Gln Gln Ala Ser Ala Ser Thr Asp Gly Val Asp Lys Leu
705                 710                 715                 720
Ser Gly Lys Glu Gln Ser Phe Thr Ser Leu Glu Val Arg Lys Val Ala

                725                 730                 735
Glu Thr Lys Glu Lys Ser Lys His Leu Lys Thr Lys Thr Cys Lys Lys
            740                 745                 750
Val Gln Asp Gly Leu Ser Asp Ile Ala Glu Lys Phe Leu Lys Lys Asp
        755                 760                 765
Gln Ser Asp Glu Thr Ser Glu Asp Asp Lys Lys Gln Ser Lys Lys Gly
    770                 775                 780
Thr Glu Glu Lys Lys Lys Pro Ser Asp Phe Lys Lys Lys Val Ile Lys
785                 790                 795                 800
Met Glu Gln Gln Tyr Glu Ser Ser Ser Asp Gly Thr Glu Lys Leu Pro
                805                 810                 815
Glu Arg Glu Glu Ile Cys His Phe Pro Lys Gly Ile Lys Gln Ile Lys
            820                 825                 830
Asn Gly Thr Thr Asp Gly Glu Lys Lys Ser Lys Lys Ile Arg Asp Lys
        835                 840                 845
Thr Ser Lys Lys Lys Asp Glu Leu Ser Asp Tyr Ala Glu Lys Ser Thr
    850                 855                 860
Gly Lys Gly Asp Ser Cys Asp Ser Ser Glu Asp Lys Lys Ser Lys Asn
865                 870                 875                 880
Gly Ala Tyr Gly Arg Glu Lys Lys Arg Cys Lys Leu Leu Gly Lys Ser
                885                 890                 895
Ser Arg Lys Arg Gln Asp Cys Ser Ser Ser Asp Thr Glu Lys Tyr Ser
            900                 905                 910
Met Lys Glu Asp Gly Cys Asn Ser Ser Asp Lys Arg Leu Lys Arg Ile
        915                 920                 925
Glu Leu Arg Glu Arg Arg Asn Leu Ser Ser Lys Arg Asn Thr Lys Glu
    930                 935                 940
Ile Gln Ser Gly Ser Ser Ser Ser Asp Ala Glu Glu Ser Ser Glu Asp
945                 950                 955                 960
Asn Lys Lys Lys Lys Gln Arg Thr Ser Ser Lys Lys Lys Ala Val Ile
                965                 970                 975
Val Lys Glu Lys Lys Arg Asn Ser Leu Arg Thr Ser Thr Lys Arg Lys
            980                 985                 990
Gln Ala Asp Ile Thr Ser Ser Ser Ser Ser Asp Ile Glu Asp Asp Asp
        995                 1000                1005
Gln Asn Ser Ile Gly Glu Gly Ser Ser Asp Glu Gln Lys Ile Lys
    1010                1015                1020
Pro Val Thr Glu Asn Leu Val Leu Ser Ser His Thr Gly Phe Cys
    1025                1030                1035
Gln Ser Ser Gly Asp Glu Ala Leu Ser Lys Ser Val Pro Val Thr
    1040                1045                1050
Val Asp Asp Asp Asp Asp Asp Asn Asp Pro Glu Asn Arg Ile Ala
    1055                1060                1065
Lys Lys Met Leu Leu Glu Glu Ile Lys Ala Asn Leu Ser Ser Asp
    1070                1075                1080
Glu Asp Gly Ser Ser Asp Asp Glu Pro Glu Glu Gly Lys Lys Arg
    1085                1090                1095
Thr Gly Lys Gln Asn Glu Glu Asn Pro Gly Asp Glu Glu Ala Lys
    1100                1105                1110
Asn Gln Val Asn Ser Glu Ser Asp Ser Asp Ser Glu Glu Ser Lys
    1115                1120                1125
Lys Pro Arg Tyr Arg His Arg Leu Leu Arg His Lys Leu Thr Val
    1130                1135                1140
Ser Asp Gly Glu Ser Gly Glu Glu Lys Lys Thr Lys Pro Lys Glu
    1145                1150                1155
His Lys Glu Val Lys Gly Arg Asn Arg Arg Lys Val Ser Ser Glu
    1160                1165                1170
Asp Ser Glu Asp Ser Asp Phe Gln Glu Ser Gly Val Ser Glu Glu
    1175                1180                1185
Val Ser Glu Ser Glu Asp Glu Gln Arg Pro Arg Thr Arg Ser Ala
    1190                1195                1200
Lys Lys Ala Glu Leu Glu Glu Asn Gln Arg Ser Tyr Lys Gln Lys
    1205                1210                1215
Lys Lys Arg Arg Arg Ile Lys Val Gln Glu Asp Ser Ser Ser Glu
    1220                1225                1230
Asn Lys Ser Asn Ser Glu Glu Glu Glu Glu Glu Lys Glu Glu Glu
    1235                1240                1245
Glu Glu Glu Glu Glu Glu Glu Glu Glu Glu Glu Glu Asp Glu Asn
    1250                1255                1260
Asp Asp Ser Lys Ser Pro Gly Lys Gly Arg Lys Lys Ile Arg Lys
    1265                1270                1275
Ile Leu Lys Asp Asp Lys Leu Arg Thr Glu Thr Gln Asn Ala Leu
    1280                1285                1290

Lys Glu Glu Glu Glu Arg Arg Lys Arg Ile Ala Glu Arg Glu Arg
    1295                1300                1305
Glu Arg Glu Lys Leu Arg Glu Val Ile Glu Ile Glu Asp Ala Ser
    1310                1315                1320
Pro Thr Lys Cys Pro Ile Thr Thr Lys Leu Val Leu Asp Glu Asp
    1325                1330                1335
Glu Glu Thr Lys Glu Pro Leu Val Gln Val His Arg Asn Met Val
    1340                1345                1350
Ile Lys Leu Lys Pro His Gln Val Asp Gly Val Gln Phe Met Trp
    1355                1360                1365
Asp Cys Cys Cys Glu Ser Val Lys Lys Thr Lys Lys Ser Pro Gly
    1370                1375                1380
Ser Gly Cys Ile Leu Ala His Cys Met Gly Leu Gly Lys Thr Leu
    1385                1390                1395
Gln Val Val Ser Phe Leu His Thr Val Leu Leu Cys Asp Lys Leu
    1400                1405                1410
Asp Phe Ser Thr Ala Leu Val Val Cys Pro Leu Asn Thr Ala Leu
    1415                1420                1425
Asn Trp Met Asn Glu Phe Glu Lys Trp Gln Glu Gly Leu Lys Asp
    1430                1435                1440
Asp Glu Lys Leu Glu Val Ser Glu Leu Ala Thr Val Lys Arg Pro
    1445                1450                1455
Gln Glu Arg Ser Tyr Met Leu Gln Arg Trp Gln Glu Asp Gly Gly
    1460                1465                1470
Val Met Ile Ile Gly Tyr Glu Met Tyr Arg Asn Leu Ala Gln Gly
    1475                1480                1485
Arg Asn Val Lys Ser Arg Lys Leu Lys Glu Ile Phe Asn Lys Ala
    1490                1495                1500
Leu Val Asp Pro Gly Pro Asp Phe Val Val Cys Asp Glu Gly His
    1505                1510                1515
Ile Leu Lys Asn Glu Ala Ser Ala Val Ser Lys Ala Met Asn Ser
    1520                1525                1530
Ile Arg Ser Arg Arg Arg Ile Ile Leu Thr Gly Thr Pro Leu Gln
    1535                1540                1545
Asn Asn Leu Ile Glu Tyr His Cys Met Val Asn Phe Ile Lys Glu
    1550                1555                1560
Asn Leu Leu Gly Ser Ile Lys Glu Phe Arg Asn Arg Phe Ile Asn
    1565                1570                1575
Pro Ile Gln Asn Gly Gln Cys Ala Asp Ser Thr Met Val Asp Val
    1580                1585                1590
Arg Val Met Lys Lys Arg Ala His Ile Leu Tyr Glu Met Leu Ala
    1595                1600                1605
Gly Cys Val Gln Arg Lys Asp Tyr Thr Ala Leu Thr Lys Phe Leu
    1610                1615                1620
Pro Pro Lys His Glu Tyr Val Leu Ala Val Arg Met Thr Ser Ile
    1625                1630                1635
Gln Cys Lys Leu Tyr Gln Tyr Tyr Leu Asp His Leu Thr Gly Val
    1640                1645                1650
Gly Asn Asn Ser Glu Gly Gly Arg Gly Lys Ala Gly Ala Lys Leu
    1655                1660                1665
Phe Gln Asp Phe Gln Met Leu Ser Arg Ile Trp Thr His Pro Trp
    1670                1675                1680
Cys Leu Gln Leu Asp Tyr Ile Ser Lys Glu Asn Lys Gly Tyr Phe
    1685                1690                1695
Asp Glu Asp Ser Met Asp Glu Phe Ile Ala Ser Asp Ser Asp Glu
    1700                1705                1710
Thr Ser Met Ser Leu Ser Ser Asp Asp Tyr Thr Lys Lys Lys Lys
    1715                1720                1725
Lys Gly Lys Lys Gly Lys Lys Asp Ser Ser Ser Ser Gly Ser Gly
    1730                1735                1740
Ser Asp Asn Asp Val Glu Val Ile Lys Val Trp Asn Ser Arg Ser
    1745                1750                1755
Arg Gly Gly Gly Glu Gly Asn Val Asp Glu Thr Gly Asn Asn Pro
    1760                1765                1770
Ser Val Ser Leu Lys Leu Glu Glu Ser Lys Ala Thr Ser Ser Ser
    1775                1780                1785
Asn Pro Ser Ser Pro Ala Pro Asp Trp Tyr Lys Asp Phe Val Thr
    1790                1795                1800
Asp Ala Asp Ala Glu Val Leu Glu His Ser Gly Lys Met Val Leu
    1805                1810                1815
Leu Phe Glu Ile Leu Arg Met Ala Glu Glu Ile Gly Asp Lys Val
    1820                1825                1830
Leu Val Phe Ser Gln Ser Leu Ile Ser Leu Asp Leu Ile Glu Asp

1835                 1840                 1845
Phe Leu Glu Leu Ala Ser Arg Glu Lys Thr Glu Asp Lys Asp Lys
    1850                 1855                 1860
Pro Leu Ile Tyr Lys Gly Glu Gly Lys Trp Leu Arg Asn Ile Asp
    1865                 1870                 1875
Tyr Tyr Arg Leu Asp Gly Ser Thr Thr Ala Gln Ser Arg Lys Lys
    1880                 1885                 1890
Trp Ala Glu Glu Phe Asn Asp Glu Thr Asn Val Arg Gly Arg Leu
    1895                 1900                 1905
Phe Ile Ile Ser Thr Lys Ala Gly Ser Leu Gly Ile Asn Leu Val
    1910                 1915                 1920
Ala Ala Asn Arg Val Ile Ile Phe Asp Ala Ser Trp Asn Pro Ser
    1925                 1930                 1935
Tyr Asp Ile Gln Ser Ile Phe Arg Val Tyr Arg Phe Gly Gln Thr
    1940                 1945                 1950
Lys Pro Val Tyr Val Tyr Arg Phe Leu Ala Gln Gly Thr Met Glu
    1955                 1960                 1965
Asp Lys Ile Tyr Asp Arg Gln Val Thr Lys Gln Ser Leu Ser Phe
    1970                 1975                 1980
Arg Val Val Asp Gln Gln Gln Val Glu Arg His Phe Thr Met Asn
    1985                 1990                 1995
Glu Leu Thr Glu Leu Tyr Thr Phe Glu Pro Asp Leu Leu Asp Asp
    2000                 2005                 2010
Pro Asn Ser Glu Lys Lys Lys Lys Arg Asp Thr Pro Met Leu Pro
    2015                 2020                 2025
Lys Asp Thr Ile Leu Ala Glu Leu Leu Gln Ile His Lys Glu His
    2030                 2035                 2040
Ile Val Gly Tyr His Glu His Asp Ser Leu Leu Asp His Lys Glu
    2045                 2050                 2055
Glu Glu Glu Leu Thr Glu Glu Glu Arg Lys Ala Ala Trp Ala Glu
    2060                 2065                 2070
Tyr Glu Ala Glu Lys Lys Gly Leu Thr Met Arg Phe Asn Ile Pro
    2075                 2080                 2085
Thr Gly Thr Asn Leu Pro Pro Val Ser Phe Asn Ser Gln Thr Pro
    2090                 2095                 2100
Tyr Ile Pro Phe Asn Leu Gly Ala Leu Ser Ala Met Ser Asn Gln
    2105                 2110                 2115
Gln Leu Glu Asp Leu Ile Asn Gln Gly Arg Glu Lys Val Val Glu
    2120                 2125                 2130
Ala Thr Asn Ser Val Thr Ala Val Arg Ile Gln Pro Leu Glu Asp
    2135                 2140                 2145
Ile Ile Ser Ala Val Trp Lys Glu Asn Met Asn Leu Ser Glu Ala
    2150                 2155                 2160
Gln Val Gln Ala Leu Ala Leu Ser Arg Gln Ala Ser Gln Glu Leu
    2165                 2170                 2175
Asp Val Lys Arg Arg Glu Ala Ile Tyr Asn Asp Val Leu Thr Lys
    2180                 2185                 2190
Gln Gln Met Leu Ile Ser Cys Val Gln Arg Ile Leu Met Asn Arg
    2195                 2200                 2205
Arg Leu Gln Gln Gln Tyr Asn Gln Gln Gln Gln Gln Gln Met Thr
    2210                 2215                 2220
Tyr Gln Gln Ala Thr Leu Gly His Leu Met Met Pro Lys Pro Pro
    2225                 2230                 2235
Asn Leu Ile Met Asn Pro Ser Asn Tyr Gln Gln Ile Asp Met Arg
    2240                 2245                 2250
Gly Met Tyr Gln Pro Val Ala Gly Gly Met Gln Pro Pro Pro Leu
    2255                 2260                 2265
Gln Arg Ala Pro Pro Pro Met Arg Ser Lys Asn Pro Gly Pro Ser
    2270                 2275                 2280
Gln Gly Lys Ser Met
    2285


<210>  101
<211>  2673
<212>  PRT
<213>  Homo sapiens
<220>
<221>  misc_feature
<222>  (119)..(119)
<223>  X est un résidu inconnu

<220>

<221>  misc_feature
<223>  NOM:fibuline 6 ( GS-P52)

<220>
<221>  misc_feature
<223>  CDS partielle de la séquence GS-N52:
<1..8022

<400>  101
Pro Pro Ser Val Ile Gly Pro Lys Ser Glu Asn Leu Thr Val Val Val
1               5                   10                  15
Asn Asn Phe Ile Ser Leu Thr Cys Glu Val Ser Gly Phe Pro Pro Pro
            20                  25                  30
Asp Leu Ser Trp Leu Lys Asn Glu Gln Pro Ile Lys Leu Asn Thr Asn
        35                  40                  45
Thr Leu Ile Val Pro Gly Gly Arg Thr Leu Gln Ile Ile Arg Ala Lys
    50                  55                  60
Val Ser Asp Gly Gly Glu Tyr Thr Cys Ile Ala Ile Asn Gln Ala Gly
65                  70                  75                  80
Glu Ser Lys Lys Lys Phe Ser Leu Thr Val Tyr Val Pro Pro Ser Ile
                85                  90                  95
Lys Asp His Asp Ser Glu Ser Leu Ser Val Val Asn Val Arg Glu Gly
            100                 105                 110
Thr Ser Val Ser Leu Glu Xaa Glu Ser Asn Ala Val Pro Pro Pro Val
        115                 120                 125
Ile Thr Trp Tyr Lys Asn Gly Arg Met Ile Thr Glu Ser Thr His Val
    130                 135                 140
Glu Ile Leu Ala Asp Gly Gln Met Leu His Ile Lys Lys Ala Glu Val
145                 150                 155                 160
Ser Asp Thr Gly Gln Tyr Val Cys Arg Ala Ile Asn Val Ala Gly Arg
                165                 170                 175
Asp Asp Lys Asn Phe His Leu Asn Val Tyr Val Pro Pro Ser Ile Glu
            180                 185                 190
Gly Pro Glu Arg Glu Val Ile Val Glu Thr Ile Ser Asn Pro Val Thr
        195                 200                 205
Leu Thr Cys Asp Ala Thr Gly Ile Pro Pro Pro Thr Ile Ala Trp Leu
    210                 215                 220
Lys Asn His Lys Arg Ile Glu Asn Ser Asp Ser Leu Glu Val Arg Ile
225                 230                 235                 240
Leu Ser Gly Gly Ser Lys Leu Gln Ile Ala Arg Ser Gln His Ser Asp
                245                 250                 255
Ser Gly Asn Tyr Thr Cys Ile Ala Ser Asn Met Glu Gly Lys Ala Gln
            260                 265                 270
Lys Tyr Tyr Phe Leu Ser Ile Gln Val Pro Pro Ser Val Ala Gly Ala
        275                 280                 285
Glu Ile Pro Ser Asp Val Ser Val Leu Leu Gly Glu Asn Val Glu Leu
    290                 295                 300
Val Cys Asn Ala Asn Gly Ile Pro Thr Pro Leu Ile Gln Trp Leu Lys
305                 310                 315                 320
Asp Gly Lys Pro Ile Ala Ser Gly Glu Thr Glu Arg Ile Arg Val Ser
                325                 330                 335
Ala Asn Gly Ser Thr Leu Asn Ile Tyr Gly Ala Leu Thr Ser Asp Thr
            340                 345                 350
Gly Lys Tyr Thr Cys Val Ala Thr Asn Pro Ala Gly Glu Glu Asp Arg
        355                 360                 365
Ile Phe Asn Leu Asn Val Tyr Val Thr Pro Thr Ile Arg Gly Asn Lys
    370                 375                 380
Asp Glu Ala Glu Lys Leu Met Thr Leu Val Asp Thr Ser Ile Asn Ile
385                 390                 395                 400
Glu Cys Arg Ala Thr Gly Thr Pro Pro Pro Gln Ile Asn Trp Leu Lys
                405                 410                 415
Asn Gly Leu Pro Leu Pro Leu Ser Ser His Ile Arg Leu Leu Ala Ala
            420                 425                 430
Gly Gln Val Ile Arg Ile Val Arg Ala Gln Val Ser Asp Val Ala Val
        435                 440                 445
Tyr Thr Cys Val Ala Ser Asn Arg Ala Gly Val Asp Asn Lys His Tyr
    450                 455                 460
Asn Leu Gln Val Phe Ala Pro Pro Asn Met Asp Asn Ser Met Gly Thr
465                 470                 475                 480
Glu Glu Ile Thr Val Leu Lys Gly Ser Ser Thr Ser Met Ala Cys Ile
                485                 490                 495
Thr Asp Gly Thr Pro Ala Pro Ser Met Ala Trp Leu Arg Asp Gly Gln
            500                 505                 510

Pro Leu Gly Leu Asp Ala His Leu Thr Val Ser Thr His Gly Met Val
        515                 520                 525
Leu Gln Leu Leu Lys Ala Glu Thr Glu Asp Ser Gly Lys Tyr Thr Cys
    530                 535                 540
Ile Ala Ser Asn Glu Ala Gly Glu Val Ser Lys His Phe Ile Leu Lys
545                 550                 555                 560
Val Leu Glu Pro Pro His Ile Asn Gly Ser Glu Glu His Glu Glu Ile
                565                 570                 575
Ser Val Ile Val Asn Asn Pro Leu Glu Leu Thr Cys Ile Ala Ser Gly
            580                 585                 590
Ile Pro Ala Pro Lys Met Thr Trp Met Lys Asp Gly Arg Pro Leu Pro
        595                 600                 605
Gln Thr Asp Gln Val Gln Thr Leu Gly Gly Gly Glu Val Leu Arg Ile
    610                 615                 620
Ser Thr Ala Gln Val Glu Asp Thr Gly Arg Tyr Thr Cys Leu Ala Ser
625                 630                 635                 640
Ser Pro Ala Gly Asp Asp Asp Lys Glu Tyr Leu Val Arg Val His Val
                645                 650                 655
Pro Pro Asn Ile Ala Gly Thr Asp Glu Pro Arg Asp Ile Thr Val Leu
            660                 665                 670
Arg Asn Arg Gln Val Thr Leu Glu Cys Lys Ser Asp Ala Val Pro Pro
        675                 680                 685
Pro Val Ile Thr Trp Leu Arg Asn Gly Glu Arg Leu Gln Ala Thr Pro
    690                 695                 700
Arg Val Arg Ile Leu Ser Gly Gly Arg Tyr Leu Gln Ile Asn Asn Ala
705                 710                 715                 720
Asp Leu Gly Asp Thr Ala Asn Tyr Thr Cys Val Ala Ser Asn Ile Ala
                725                 730                 735
Gly Lys Thr Thr Arg Glu Phe Ile Leu Thr Val Asn Val Pro Pro Asn
            740                 745                 750
Ile Lys Gly Gly Pro Gln Ser Leu Val Ile Leu Leu Asn Lys Ser Thr
        755                 760                 765
Val Leu Glu Cys Ile Ala Glu Gly Val Pro Thr Pro Arg Ile Thr Trp
    770                 775                 780
Arg Lys Asp Gly Ala Val Leu Ala Gly Asn His Ala Arg Tyr Ser Ile
785                 790                 795                 800
Leu Glu Asn Gly Phe Leu His Ile Gln Ser Ala His Val Thr Asp Thr
                805                 810                 815
Gly Arg Tyr Leu Cys Met Ala Thr Asn Ala Ala Gly Thr Asp Arg Arg
            820                 825                 830
Arg Ile Asp Leu Gln Val His Val Pro Pro Ser Ile Ala Pro Gly Pro
        835                 840                 845
Thr Asn Met Thr Val Ile Val Asn Val Gln Thr Thr Leu Ala Cys Glu
    850                 855                 860
Ala Thr Gly Ile Pro Lys Pro Ser Ile Asn Trp Arg Lys Asn Gly His
865                 870                 875                 880
Leu Leu Asn Val Asp Gln Asn Gln Asn Ser Tyr Arg Leu Leu Ser Ser
                885                 890                 895
Gly Ser Leu Val Ile Ile Ser Pro Ser Val Asp Asp Thr Ala Thr Tyr
            900                 905                 910
Glu Cys Thr Val Thr Asn Gly Ala Gly Asp Asp Lys Arg Thr Val Asp
        915                 920                 925
Leu Thr Val Gln Val Pro Pro Ser Ile Ala Asp Glu Pro Thr Asp Phe
    930                 935                 940
Leu Val Thr Lys His Ala Pro Ala Val Ile Thr Cys Thr Ala Ser Gly
945                 950                 955                 960
Val Pro Phe Pro Ser Ile His Trp Thr Lys Asn Gly Ile Arg Leu Leu
                965                 970                 975
Pro Arg Gly Asp Gly Tyr Arg Ile Leu Ser Ser Gly Ala Ile Glu Ile
            980                 985                 990
Leu Ala Thr Gln Leu Asn His Ala  Gly Arg Tyr Thr Cys  Val Ala Arg
        995                 1000                1005
Asn Ala  Ala Gly Ser Ala His  Arg His Val Thr Leu  His Val His
    1010                1015                1020
Glu Pro  Pro Val Ile Gln Pro  Gln Pro Ser Glu Leu  His Val Ile
    1025                1030                1035
Leu Asn  Asn Pro Ile Leu Leu  Pro Cys Glu Ala Thr  Gly Thr Pro
    1040                1045                1050
Ser Pro  Phe Ile Thr Trp Gln  Lys Glu Gly Ile Asn  Val Asn Thr
    1055                1060                1065
Ser Gly  Arg Asn His Ala Val  Leu Pro Ser Gly Gly  Leu Gln Ile
    1070                1075                1080
Ser Arg  Ala Val Arg Glu Asp  Ala Gly Thr Tyr Met  Cys Val Ala

1085                 1090                 1095
Gln Asn  Pro Ala Gly Thr Ala  Leu Gly Lys Ile Lys  Leu Asn Val
    1100                 1105                 1110
Gln Val  Pro Pro Val Ile Ser  Pro His Leu Lys Glu  Tyr Val Ile
    1115                 1120                 1125
Ala Val  Asp Lys Pro Ile Thr  Leu Ser Cys Glu Ala  Asp Gly Leu
    1130                 1135                 1140
Pro Pro  Pro Asp Ile Thr Trp  His Lys Asp Gly Arg  Ala Ile Val
    1145                 1150                 1155
Glu Ser  Ile Arg Gln Arg Val  Leu Ser Ser Gly Ser  Leu Gln Ile
    1160                 1165                 1170
Thr Phe  Val Gln Pro Gly Asp  Ala Gly His Tyr Thr  Cys Met Ala
    1175                 1180                 1185
Ala Asn  Val Ala Gly Ser Ser  Ser Thr Ser Thr Lys  Leu Thr Val
    1190                 1195                 1200
His Val  Pro Pro Arg Ile Arg  Ser Thr Glu Gly His  Tyr Thr Val
    1205                 1210                 1215
Asn Glu  Asn Ser Gln Ala Ile  Leu Pro Cys Val Ala  Asp Gly Ile
    1220                 1225                 1230
Pro Thr  Pro Ala Ile Asn Trp  Lys Lys Asp Asn Val  Leu Leu Ala
    1235                 1240                 1245
Asn Leu  Leu Gly Lys Tyr Thr  Ala Glu Pro Tyr Gly  Glu Leu Ile
    1250                 1255                 1260
Leu Glu  Asn Val Val Leu Glu  Asp Ser Gly Phe Tyr  Thr Cys Val
    1265                 1270                 1275
Ala Asn  Asn Ala Ala Gly Glu  Asp Thr His Thr Val  Ser Leu Thr
    1280                 1285                 1290
Val His  Val Leu Pro Thr Phe  Thr Glu Leu Pro Gly  Asp Val Ser
    1295                 1300                 1305
Leu Asn  Lys Gly Glu Gln Leu  Arg Leu Ser Cys Lys  Ala Thr Gly
    1310                 1315                 1320
Ile Pro  Leu Pro Lys Leu Thr  Trp Thr Phe Asn Asn  Asn Ile Ile
    1325                 1330                 1335
Pro Ala  His Phe Asp Ser Val  Asn Gly His Ser Glu  Leu Val Ile
    1340                 1345                 1350
Glu Arg  Val Ser Lys Glu Asp  Ser Gly Thr Tyr Val  Cys Thr Ala
    1355                 1360                 1365
Glu Asn  Ser Val Gly Phe Val  Lys Ala Ile Gly Phe  Val Tyr Val
    1370                 1375                 1380
Lys Glu  Pro Pro Val Phe Lys  Gly Asp Tyr Pro Ser  His Trp Ile
    1385                 1390                 1395
Glu Pro  Leu Gly Gly Asn Ala  Ile Leu Asn Cys Glu  Val Lys Gly
    1400                 1405                 1410
Asp Pro  Thr Pro Thr Ile Gln  Trp Asn Arg Lys Gly  Val Asp Ile
    1415                 1420                 1425
Glu Ile  Ser His Arg Ile Arg  Gln Leu Gly Asn Gly  Ser Leu Ala
    1430                 1435                 1440
Ile Tyr  Gly Thr Val Asn Glu  Asp Ala Gly Asp Tyr  Thr Cys Val
    1445                 1450                 1455
Ala Thr  Asn Glu Ala Gly Val  Val Glu Arg Ser Met  Ser Leu Thr
    1460                 1465                 1470
Leu Gln  Ser Pro Pro Ile Ile  Thr Leu Glu Pro Val  Glu Thr Val
    1475                 1480                 1485
Ile Asn  Ala Gly Gly Lys Ile  Ile Leu Asn Cys Gln  Ala Thr Gly
    1490                 1495                 1500
Glu Pro  Gln Pro Thr Ile Thr  Trp Ser Arg Gln Gly  His Ser Ile
    1505                 1510                 1515
Ser Trp  Asp Asp Arg Val Asn  Val Leu Ser Asn Asn  Ser Leu Tyr
    1520                 1525                 1530
Ile Ala  Asp Ala Gln Lys Glu  Asp Thr Ser Glu Phe  Glu Cys Val
    1535                 1540                 1545
Ala Arg  Asn Leu Met Gly Ser  Val Leu Val Arg Val  Pro Val Ile
    1550                 1555                 1560
Val Gln  Val His Gly Gly Phe  Ser Gln Trp Ser Ala  Trp Arg Ala
    1565                 1570                 1575
Cys Ser  Val Thr Cys Gly Lys  Gly Ile Gln Lys Arg  Ser Arg Leu
    1580                 1585                 1590
Cys Asn  Gln Pro Leu Pro Ala  Asn Gly Gly Lys Pro  Cys Gln Gly
    1595                 1600                 1605
Ser Asp  Leu Glu Met Arg Asn  Cys Gln Asn Lys Pro  Cys Pro Val
    1610                 1615                 1620
Asp Gly  Ser Trp Ser Glu Trp  Ser Leu Trp Glu Glu  Cys Thr Arg
    1625                 1630                 1635

Ser Cys Gly Arg Gly Asn Gln Thr Arg Thr Arg Thr Cys Asn Asn
    1640                1645                1650
Pro Ser Val Gln His Gly Gly Arg Pro Cys Glu Gly Asn Ala Val
    1655                1660                1665
Glu Ile Ile Met Cys Asn Ile Arg Pro Cys Pro Val His Gly Ala
    1670                1675                1680
Trp Ser Ala Trp Gln Pro Trp Gly Thr Cys Ser Glu Ser Cys Gly
    1685                1690                1695
Lys Gly Thr Gln Thr Arg Ala Arg Leu Cys Asn Asn Pro Pro Pro
    1700                1705                1710
Ala Phe Gly Gly Ser Tyr Cys Asp Gly Ala Glu Thr Gln Met Gln
    1715                1720                1725
Val Cys Asn Glu Arg Asn Cys Pro Val His Gly Lys Trp Ala Thr
    1730                1735                1740
Trp Ala Ser Trp Ser Ala Cys Ser Val Ser Cys Gly Gly Gly Ala
    1745                1750                1755
Arg Gln Arg Thr Arg Gly Cys Ser Asp Pro Val Pro Gln Tyr Gly
    1760                1765                1770
Gly Arg Lys Cys Glu Gly Ser Asp Val Gln Ser Asp Phe Cys Asn
    1775                1780                1785
Ser Asp Pro Cys Pro Thr His Gly Asn Trp Ser Pro Trp Ser Gly
    1790                1795                1800
Trp Gly Thr Cys Ser Arg Thr Cys Asn Gly Gly Gln Met Arg Arg
    1805                1810                1815
Tyr Arg Thr Cys Asp Asn Pro Pro Pro Ser Asn Gly Gly Arg Ala
    1820                1825                1830
Cys Gly Gly Pro Asp Ser Gln Ile Gln Arg Cys Asn Thr Asp Met
    1835                1840                1845
Cys Pro Val Asp Gly Ser Trp Gly Ser Trp His Ser Trp Ser Gln
    1850                1855                1860
Cys Ser Ala Ser Cys Gly Gly Gly Glu Lys Thr Arg Lys Arg Leu
    1865                1870                1875
Cys Asp His Pro Val Pro Val Lys Gly Gly Arg Pro Cys Pro Gly
    1880                1885                1890
Asp Thr Thr Gln Val Thr Arg Cys Asn Val Gln Ala Cys Pro Gly
    1895                1900                1905
Gly Pro Gln Arg Ala Arg Gly Ser Val Ile Gly Asn Ile Asn Asp
    1910                1915                1920
Val Glu Phe Gly Ile Ala Phe Leu Asn Ala Thr Ile Thr Asp Ser
    1925                1930                1935
Pro Asn Ser Asp Thr Arg Ile Ile Arg Ala Lys Ile Thr Asn Val
    1940                1945                1950
Pro Arg Ser Leu Gly Ser Ala Met Arg Lys Ile Val Ser Ile Leu
    1955                1960                1965
Asn Pro Ile Tyr Trp Thr Thr Ala Lys Glu Ile Gly Glu Ala Val
    1970                1975                1980
Asn Gly Phe Thr Leu Thr Asn Ala Val Phe Lys Arg Glu Thr Gln
    1985                1990                1995
Val Glu Phe Ala Thr Gly Glu Ile Leu Gln Met Ser His Ile Ala
    2000                2005                2010
Arg Gly Leu Asp Ser Asp Gly Ser Leu Leu Leu Asp Ile Val Val
    2015                2020                2025
Ser Gly Tyr Val Leu Gln Leu Gln Ser Pro Ala Glu Val Thr Val
    2030                2035                2040
Lys Asp Tyr Thr Glu Asp Tyr Ile Gln Thr Gly Pro Gly Gln Leu
    2045                2050                2055
Tyr Ala Tyr Ser Thr Arg Leu Phe Thr Ile Asp Gly Ile Ser Ile
    2060                2065                2070
Pro Tyr Thr Trp Asn His Thr Val Phe Tyr Asp Gln Ala Gln Gly
    2075                2080                2085
Arg Met Pro Phe Leu Val Glu Thr Leu His Ala Ser Ser Val Glu
    2090                2095                2100
Ser Asp Tyr Asn Gln Ile Glu Glu Thr Leu Gly Phe Lys Ile His
    2105                2110                2115
Ala Ser Ile Ser Lys Gly Asp Arg Ser Asn Gln Cys Pro Ser Gly
    2120                2125                2130
Phe Thr Leu Asp Ser Val Gly Pro Phe Cys Ala Asp Glu Asp Glu
    2135                2140                2145
Cys Ala Ala Gly Asn Pro Cys Ser His Ser Cys His Asn Ala Met
    2150                2155                2160
Gly Thr Tyr Tyr Cys Ser Cys Pro Lys Gly Leu Thr Ile Ala Ala
    2165                2170                2175
Asp Gly Arg Thr Cys Gln Asp Ile Asp Glu Cys Ala Leu Gly Arg

2180                    2185                    2190
His Thr  Cys His Ala Gly Gln  Asp Cys Asp Asn Thr  Ile Gly Ser
    2195                    2200                    2205
Tyr Arg  Cys Val Val Arg Cys  Gly Ser Gly Phe Arg  Arg Thr Ser
    2210                    2215                    2220
Asp Gly  Leu Ser Cys Gln Asp  Ile Asn Glu Cys Gln  Glu Ser Ser
    2225                    2230                    2235
Pro Cys  His Gln Arg Cys Phe  Asn Ala Ile Gly Ser  Phe His Cys
    2240                    2245                    2250
Gly Cys  Glu Pro Gly Tyr Gln  Leu Lys Gly Arg Lys  Cys Met Asp
    2255                    2260                    2265
Val Asn  Glu Cys Arg Gln Asn  Val Cys Arg Pro Asp  Gln His Cys
    2270                    2275                    2280
Lys Asn  Thr Arg Gly Gly Tyr  Lys Cys Ile Asp Leu  Cys Pro Asn
    2285                    2290                    2295
Gly Met  Thr Lys Ala Glu Asn  Gly Thr Cys Ile Asp  Ile Asp Glu
    2300                    2305                    2310
Cys Lys  Asp Gly Thr His Gln  Cys Arg Tyr Asn Gln  Ile Cys Glu
    2315                    2320                    2325
Asn Thr  Arg Gly Ser Tyr Arg  Cys Val Cys Pro Arg  Gly Tyr Arg
    2330                    2335                    2340
Ser Gln  Gly Val Gly Arg Pro  Cys Met Asp Ile Asn  Glu Cys Glu
    2345                    2350                    2355
Gln Val  Pro Lys Pro Cys Ala  His Gln Cys Ser Asn  Thr Pro Gly
    2360                    2365                    2370
Ser Phe  Lys Cys Ile Cys Pro  Pro Gly Gln His Leu  Leu Gly Asp
    2375                    2380                    2385
Gly Lys  Ser Cys Ala Gly Leu  Glu Arg Leu Pro Asn  Tyr Gly Thr
    2390                    2395                    2400
Gln Tyr  Ser Ser Tyr Asn Leu  Ala Arg Phe Ser Pro  Val Arg Asn
    2405                    2410                    2415
Asn Tyr  Gln Pro Gln Gln His  Tyr Arg Gln Tyr Ser  His Leu Tyr
    2420                    2425                    2430
Ser Ser  Tyr Ser Glu Tyr Arg  Asn Ser Arg Thr Ser  Leu Ser Arg
    2435                    2440                    2445
Thr Arg  Arg Thr Ile Arg Lys  Thr Cys Pro Glu Gly  Ser Glu Ala
    2450                    2455                    2460
Ser His  Asp Thr Cys Val Asp  Ile Asp Glu Cys Glu  Asn Thr Asp
    2465                    2470                    2475
Ala Cys  Gln His Glu Cys Lys  Asn Thr Phe Gly Ser  Tyr Gln Cys
    2480                    2485                    2490
Ile Cys  Pro Pro Gly Tyr Gln  Leu Thr His Asn Gly  Lys Thr Cys
    2495                    2500                    2505
Gln Asp  Ile Asp Glu Cys Leu  Glu Gln Asn Val His  Cys Gly Pro
    2510                    2515                    2520
Asn Arg  Met Cys Phe Asn Met  Arg Gly Ser Tyr Gln  Cys Ile Asp
    2525                    2530                    2535
Thr Pro  Cys Pro Pro Asn Tyr  Gln Arg Asp Pro Val  Ser Gly Phe
    2540                    2545                    2550
Cys Leu  Lys Asn Cys Pro Pro  Asn Asp Leu Glu Cys  Ala Leu Ser
    2555                    2560                    2565
Pro Tyr  Ala Leu Glu Tyr Lys  Leu Val Ser Leu Pro  Phe Gly Ile
    2570                    2575                    2580
Ala Thr  Asn Gln Asp Leu Ile  Arg Leu Val Ala Tyr  Thr Gln Asp
    2585                    2590                    2595
Gly Val  Met His Pro Arg Thr  Thr Phe Leu Met Val  Asp Glu Glu
    2600                    2605                    2610
Gln Thr  Val Pro Phe Ala Leu  Arg Asp Glu Asn Leu  Lys Gly Val
    2615                    2620                    2625
Val Tyr  Thr Thr Arg Pro Leu  Arg Glu Ala Glu Thr  Tyr Arg Met
    2630                    2635                    2640
Arg Val  Arg Ala Ser Ser Tyr  Ser Ala Asn Gly Thr  Ile Glu Tyr
    2645                    2650                    2655
Gln Thr  Thr Phe Ile Val Tyr  Ile Ala Val Ser Ala  Tyr Pro Tyr
    2660                    2665                    2670

<210>  102
<211>  528
<212>  PRT
<213>  Homo sapiens
<220>
<221>  misc_feature

<223>  CDS partielle de la séquence GS-N53:
<1..1589

<220>
<221>  misc_feature
<223>  nom: séladine-1 ( GS-P53)

<400>  102
Arg Glu Pro Ala Ala Leu Thr Ala Arg Arg Arg Thr Met Glu Pro Ala
1               5                   10                  15
Val Ser Leu Ala Val Cys Ala Leu Leu Phe Leu Leu Trp Val Arg Leu
            20                  25                  30
Lys Gly Leu Glu Phe Val Leu Ile His Gln Arg Trp Val Phe Val Cys
        35                  40                  45
Leu Phe Leu Leu Pro Leu Ser Leu Ile Phe Asp Ile Tyr Tyr Tyr Val
    50                  55                  60
Arg Ala Trp Val Val Phe Lys Leu Ser Ser Ala Pro Arg Leu His Glu
65                  70                  75                  80
Gln Arg Val Arg Asp Ile Gln Lys Gln Val Arg Glu Trp Lys Glu Gln
                85                  90                  95
Gly Ser Lys Thr Phe Met Cys Thr Gly Arg Pro Gly Trp Leu Thr Val
            100                 105                 110
Ser Leu Arg Val Gly Lys Tyr Lys Lys Thr His Lys Asn Ile Met Ile
        115                 120                 125
Asn Leu Met Asp Ile Leu Glu Val Asp Thr Lys Lys Gln Ile Val Arg
    130                 135                 140
Val Glu Pro Leu Val Thr Met Gly Gln Val Thr Ala Leu Leu Thr Ser
145                 150                 155                 160
Ile Gly Trp Thr Leu Pro Val Leu Pro Glu Leu Asp Asp Leu Thr Val
                165                 170                 175
Gly Gly Leu Ile Met Gly Thr Gly Ile Glu Ser Ser Ser His Lys Tyr
            180                 185                 190
Gly Leu Phe Gln His Ile Cys Thr Ala Tyr Glu Leu Val Leu Ala Asp
        195                 200                 205
Gly Ser Phe Val Arg Cys Thr Pro Ser Glu Asn Ser Asp Leu Phe Tyr
    210                 215                 220
Ala Val Pro Trp Ser Cys Gly Thr Leu Gly Phe Leu Val Ala Ala Glu
225                 230                 235                 240
Ile Arg Ile Ile Pro Ala Lys Lys Tyr Val Lys Leu Arg Phe Glu Pro
                245                 250                 255
Val Arg Gly Leu Glu Ala Ile Cys Ala Lys Phe Thr His Glu Ser Gln
            260                 265                 270
Arg Gln Glu Asn His Phe Val Glu Gly Leu Leu Tyr Ser Leu Asp Glu
        275                 280                 285
Ala Val Ile Met Thr Gly Val Met Thr Asp Glu Ala Glu Pro Ser Lys
    290                 295                 300
Leu Asn Ser Ile Gly Asn Tyr Tyr Lys Pro Trp Phe Phe Lys His Val
305                 310                 315                 320
Glu Asn Tyr Leu Lys Thr Asn Arg Glu Gly Leu Glu Tyr Ile Pro Leu
                325                 330                 335
Arg His Tyr Tyr His Arg His Thr Arg Ser Ile Phe Trp Glu Leu Gln
            340                 345                 350
Asp Ile Ile Pro Phe Gly Asn Asn Pro Ile Phe Arg Tyr Leu Phe Gly
        355                 360                 365
Trp Met Val Pro Pro Lys Ile Ser Leu Leu Lys Leu Thr Gln Gly Glu
    370                 375                 380
Thr Leu Arg Lys Leu Tyr Glu Gln His His Val Val Gln Asp Met Leu
385                 390                 395                 400
Val Pro Met Lys Cys Leu Gln Gln Ala Leu His Thr Phe Gln Asn Asp
                405                 410                 415
Ile His Val Tyr Pro Ile Trp Leu Cys Pro Phe Ile Leu Pro Ser Gln
            420                 425                 430
Pro Gly Leu Val His Pro Lys Gly Asn Glu Ala Glu Leu Tyr Ile Asp
        435                 440                 445
Ile Gly Ala Tyr Gly Glu Pro Arg Val Lys His Phe Glu Ala Arg Ser
    450                 455                 460
Cys Met Arg Gln Leu Glu Lys Phe Val Arg Ser Val His Gly Phe Gln
465                 470                 475                 480
Met Leu Tyr Ala Asp Cys Tyr Met Asn Arg Glu Glu Phe Trp Glu Met
                485                 490                 495
Phe Asp Gly Ser Leu Tyr His Lys Leu Arg Glu Lys Leu Gly Cys Gln
            500                 505                 510
Asp Ala Phe Pro Glu Val Tyr Asp Lys Ile Cys Lys Ala Ala Arg His

515                    520                    525

<210>  103
<211>  257
<212>  DNA
<213>  Homo sapiens
<220>
<221>  misc_feature
<223>  séquence nucléique antisens de GS-N1 :415...158

<400>  103
ctggttggct ttccaggtag gctggatcct cttagattag gaggtctcag taagaacaag     60
atcaatgcaa taaccatcca ggctaccaag atcattgtaa cactgatgcc attatcacca    120
gagggtcccg gtaattcctg aagacactct gtgtctgtgc agtaggactg ggactgccgt    180
aacagattga tcagtcttct cattgcatgt tcatgagagc aaacacattc acagggatca    240
aatccacctt ctgccat                                                   257

<210>  104
<211>  202
<212>  DNA
<213>  Homo sapiens
<220>
<221>  misc_feature
<223>  séquence nucléique antisens de GS-N2:1313...1112

<400>  104
gaccggggaa tggaaggaga tatcccatct gatatccact ccccaggtcc aggggcacag     60
actctgaaca gacctacctt ctggctctgc tcacagtgga aagaggatgg gtaccaggcc    120
tgagattgtg ggagtggcaa taccctctgg gagtctcggg cagatcacca tcttgggctc    180
cccctgaccc catccctatc cc                                             202

<210>  105
<211>  242
<212>  DNA
<213>  Homo sapiens
<220>
<221>  misc_feature
<223>  séquence nucléique antisens de GS-N3 :3336....3095

<400>  105
tgggtgggga aggcaatcta gctcactcag cagggaaaat ggagctcccc aatggataac     60
tttttatgct cataaaccct gaaatagtac taaaaatgag aaaaatcccc aagacttctc    120
tgattcccag gttgggatta gctgagggaa taggaacaaa ggaccagaat cctacttcca    180
tgagccatgc ctggagtcag tattgtctcc agtgtctact ccctgaaatc ggttcctcca    240
gc                                                                   242

<210>  106
<211>  211
<212>  DNA
<213>  Homo sapiens
<220>
<221>  misc_feature
<223>  séquence nucléique antisens de GS-N4 :1392...1182

<400>  106
ggggatgtcc catcttgtgc tagagccaat aaaatgctga cactggtctt cagatgttgt     60
cctgagccta ttccaccaac ataacgatgc aaacaaccaa gagccaatga atgaccagtc    120
ctagatacaa catctcgagc cgatttcaac ttgtcaaagc tatattgggc cattctagca    180
ataaaagttg cttctccaac cacctgagcc a                                   211

<210>  107
<211>  191
<212>  DNA
<213>  Homo sapiens
<220>
<221>  misc_feature
<223>  séquence nucléique antisens de GS-N5 :577...387

<400>  107
ccgacacacg tccttggaga cttgcctcat caacttctct gcattcagac cttgcttctg     60
ctcttctttg gactgttcag cctgcttctc agcttgcttc agaagcctct ggaacctctc    120
atcctccagc acacctggtg gaaggtcagt ctctccctga gctttcagtt cctccagctt    180
ctgccgcaag t                                                         191


<210>  108
<211>  238
<212>  DNA
<213>  Homo sapiens
<220>
<221>  misc_feature
<223>  séquence nucléique antisens de GS-N6 :1805...1568

<400>  108
acgagaatgg aggccacgac atcatcctct tggcccctgg aggcacgggc ggctccagct     60
ggaaggggca gagcccgtgg gcggcagctt ccccagctgc tgctcccagc acttctactg    120
attcttgttg gctatgaaat gtctttttaa aaaactccca atatagaaat ctggctgcag    180
aggccagtgt gcagacccag ccaccgcgct gtgctgccat tcgccacatc tggtccgt      238


<210>  109
<211>  205
<212>  DNA
<213>  Homo sapiens
<220>
<221>  misc_feature
<223>  séquence nucléique antisens de GS-N7: 3071...2867

<400>  109
tgcgtgcttg ctttctcagg cctcctggtc aatagcacag gtctgctgga aatcacccac     60
atggtttttc attgcctgtg cagtcaggtg atgcctctat gtttttggag gtccacctct    120
gttgtttctt aaccttccca gccttcccac agttgtccca gtgatttgga agaaggtctc    180
ttcatttctg gccactttcg acgct                                          205


<210>  110
<211>  186
<212>  DNA
<213>  Homo sapiens
<220>
<221>  misc_feature
<223>  séquence nucléique antisens de GS-N8 :2847...2662

<400>  110
ccgtccattc ttatcctcca tctgaaggtg gtactgaata gaatcggttg acaatgtctt     60
tggagttcct tctccccatt tcagcttaag gttctggtgg ctaaaggcaa cacattccag    120
acgaggtgga tcaggaggga gaggcttagt ttttaatttt atcgtatggc tgaaaggacc    180
agctcc                                                               186


<210>  111
<211>  223
<212>  DNA
<213>  Homo sapiens
<220>
<221>  misc_feature
<223>  séquence nucléique antisens de GS-N9: 3675...3453

<400>  111
ggaggggaga gaacccaaca catgaggtct gcacacacag ctgtcctggt tgccctcggt     60
gcagctccga gctccagtta caaggaattc caagttctca ggatcttgaa gactctggag    120
gccagtaatc cctggatcac actgcttcta ccagctcaga agagaagtcc tgcctaaggt    180
catgaaataa acctgactgc tgccaccaga ccgaacagag gca                      223


<210>  112
<211>  247
<212>  DNA
<213>  Homo sapiens
<220>

<221>  misc_feature
<223>  séquence nucléique antisens de GS-N10 :1271...1025

<220>
<221>  misc_feature
<223>  séquence nucléique antisens de GS-N54 :786...540

<400>  112
gatggagctg tgcctgtgga ggctgttgtg aggcagtagg ctcatctgcg gagactgccg     60
tgaggtaggg tatgggccta aataggccat tgtgagtcat gagcttggtc tgtagaggct    120
gactggagaa agttctgggc ctggagaggc tgccgggtgg taggagcata atgtaaaatc    180
agtgggcgtg ttaagcttgt tttcctgcaa ctggatggtc ccacgttttg ctgtcacccc    240
tctggaa                                                              247


<210>  113
<211>  162
<212>  DNA
<213>  Homo sapiens
<220>
<221>  misc_feature
<223>  séquence nucléique antisens de GS-N11 :984...823

<400>  113
gctgggttca gttccttgtg cccactgtcc ctgcagacgc tctcctttcc aggtggcctc     60
cagtggtttc ccgggtcctg ctgctcccta atagtagcga ttcaggctcc tggtcccggg    120
cacctcaggc tggccattca tccagatctc cctgatgatg cg                       162


<210>  114
<211>  166
<212>  DNA
<213>  Homo sapiens
<220>
<221>  misc_feature
<223>  séquence nucléique antisens de GS-N12 :2108...1943

<400>  114
aagtttggga accgctggtg atgggacatg gatgcttgca accgaccgtg ggcggatgtg     60
gttgaccaga tggcagagga cgacaccatc catgagggct gcccccaggt cttcgtgtag    120
actgaccttc aatctcatct caatgctctc acgaagttgt tccacc                   166


<210>  115
<211>  135
<212>  DNA
<213>  Homo sapiens
<220>
<221>  misc_feature
<223>  séquence nucléique antisens de GS-N13 :1061...930

<400>  115
gcgcttgtta ggggctgaag cagaagagtc agagttctga agagtaagag aagatagtga     60
agccagccca cttgtgacag cagaggataa agcagaggag ttgattaaat tggtgtcatt    120
ggatgtcaga aaacc                                                     135


<210>  116
<211>  136
<212>  DNA
<213>  Homo sapiens
<220>
<221>  misc_feature
<223>  séquence nucléique antisens de GS-N14 :1709...1574

<400>  116
ttccacatcc ttgtgggtca tatacttcag gaaatagaca ggtttagtat gaccaacagt     60
aatagtaata cagttccttg ggtttatagt tgcatctgct taaaatcctt aaccagatga    120
ctagatcttg cgcaga                                                    136


<210>  117
<211>  552

<212>  DNA
<213>  Homo sapiens
<220>
<221>  misc_feature
<223>  séquence nucléique antisens de GS-N15 :4534...4071

<400>  117
gcggccgcga attcactagt gattcattat gctgagtgat atcttttttt ttaatatact      60
tttggaattc tcagtgaatt tgtttgcttg cccaaaatat tattttccat tctgtaattt     120
ttctttacct tccattactt acaggtttct ctgcaattta agtacagttc accaccaata     180
attgtttctg aagttaaggg tgacctgctg gcttggtcac ttttgcagtg actggacctg     240
cctgtggcca agcacatgtt ccatcactgt aactcatcaa taccaagatt ccagcaatgg     300
attctcaacc ttgtctgctg tgggaatcta ggcagtctag ccaagaccaa gaagagctga     360
gcttctgcaa gctattgata taaaactggc atagttttat cttctttcct cttgacaaaa     420
tatatatact ttaaacgtcc tttcatctcc cttcacgaag tacaaattag gaacttttct     480
tcagttttgt acagtggcat gttgctgctt ccccagcatt tagtgagggt taataatcga     540
attcccgcgg cc                                                         552


<210>  118
<211>  417
<212>  DNA
<213>  Homo sapiens
<220>
<221>  misc_feature
<223>  séquence nucléique antisens de GS-N16 :748...332

<400>  118
ccctggaatg ccatcctcat aaatatgaac catgatgaca atgcttttct gttctttatc      60
aatcatgtct aaaaaccctt ctccactgga gatctcaaaa acctgcttga attggggccc    120
cttgtgaagc tgctgccgca tctcttccat tcgctgcttc cggtactgct gcaggaactc     180
ttcatcatct tggtcctcat tcattatggc aaactccttc agagtcatct tcccactgat     240
cttctcctgg aggtctttct gtttctgttg ctcctcctct tcatccagat gggacctgca     300
agtcattgac agcctcttga tcagcctttc catctcccgg cactgctcct ccctctgctc     360
tgtctccaac tgcttgaagc ggcgccagtc attgatcaca ccttttgggc ctgtgtt        417


<210>  119
<211>  244
<212>  DNA
<213>  Homo sapiens
<220>
<221>  misc_feature
<223>  séquence nucléique antisens de GS-N17 :2276...2033

<400>  119
tgagacccca ccaactgcaa aatctgttcc tggcattaag ctccttcttc ctttgcaatt      60
cggtctttct tcagcggtcc catgaatgct ttcttctcct ccatggtctg gaagtggcca     120
tggccaaact tggaggtggt gtcaatgaac ttaaggtcaa tcttctccag agcccgccgc     180
ttcgtctgca ccagcaagga cttgcggagg gtgagcaccc gcttcttggt tcccaccaca     240
cagc                                                                  244


<210>  120
<211>  311
<212>  DNA
<213>  Homo sapiens
<220>
<221>  misc_feature
<223>  séquence nucléique antisens de GS-N18 :1340...1030

<400>  120
gctggtgggt tcctctggtg ccatgaagca gggtccgagc ctcacccaag tgtgctttca      60
actgtaggct ctcattatac aagacggaga actgtgactg catgcagcga tattctggag     120
tttccttaac gacttgctcc actgcactcc gcaattccac cttcagcttt tcattttgtg     180
tagtgacctc ctcaaagtct tgccgaagtt tctccagctc acagagacgg ttctgagcca     240
actctttgtt ctcctcaagc tctgcattca tttcctcaaa cttccgagca ttgatagtga     300
ttgtgccgcc a                                                          311

<210>  121
<211>  246
<212>  DNA
<213>  Homo sapiens

<220>
<221> misc_feature
<223> séquence nucléique antisens de GS-N19 :718...473

<400> 121
gctcttacgg gaatgtctgc ttcgagagcg agaccttgaa tgactccggc ttctggagta 60
ggaccggcgt cgtctggaac ctggcttgtc ttcaactaat ctgatttttc tcccattgac 120
ttcagttcca tccaactttt ccaaagctct tttcatatca gaataagata caaattcaat 180
caccccttca tttttgcgtc ccttgtgagc atctgcataa gtcacttctc ctgcctgacg 240
catata 246

<210> 122
<211> 203
<212> DNA
<213> Homo sapiens
<220>
<221> misc_feature
<223> séquence nucléique antisens de GS-N20 :5099...4899

<400> 122
cagatcttgt agatggctgg gtcatctgaa aaccatgtgt gcaacataga aaaaggttcc 60
gatttaggca atattaaaga atcaggcatt cctagaataa gctagtgctt cacagatata 120
agcagcagca cctgtgggtt tttaccaagt tcaaagatag acaacatgag ttttagcttt 180
tgctagatct ctctcccagc tcc 203

<210> 123
<211> 253
<212> DNA
<213> Homo sapiens
<220>
<221> misc_feature
<223> séquence nucléique antisens de GS-N21:7934...7682

<400> 123
ttccactgca ggcgggttat atattgacag caaatttctg cagataagac agtgaataaa 60
ctctccactc catgttgatt aggaatagtt tttctagttt taaatattag ccagacacag 120
aaaaaggttg gactgtaagg cctgggtgca cagtcttgat ggaaccagtt aattatgtgc 180
atttcattga tccttggttt gaggtgggga tacttcttca ctgccttcat aattttcttg 240
tgctcgttgg cca 253

<210> 124
<211> 173
<212> DNA
<213> Homo sapiens
<220>
<221> misc_feature
<223> séquence nucléique antisens de GS-N22 :2858...2686

<400> 124
gaaaggccgt ccatcaaagc ctggtacttg tcaatcatag accaacgggc atattccagg 60
attaaaagcc gtacatcttt ggccaaagtc tcgggcttga tgaggatgtt aaagtaggtc 120
ttcttcaact gctcagttat cattgtaaag acagctggtg tggaattgaa ctc 173

<210> 125
<211> 228
<212> DNA
<213> Homo sapiens
<220>
<221> misc_feature
<223> séquence nucléique antisens de GS-N23 :7484...7257

<220>
<221> misc_feature
<223> séquence nucléique antisens de GS-N47 :8634...8407

<220>
<221> misc_feature
<223> séquence nucléique antisens de GS-N48 :8811...8584

<400> 125
ccgggtggtc aagacagaca gatcagatgg tagctctaga ggcaactcaa actgttcctc 60
tgctgagata gaggaagaga cactactatc cacaggttct gttgtaggca gctgctgaga 120
aaatacttca aaaagacatg ggtctttttc acgattactg tcaagaccca atccttcacc 180
aagattcagg agttctgatg aagatgactc tgggctctca cccaaagc 228

<210> 126
<211> 381
<212> DNA
<213> Homo sapiens
<220>
<221> misc_feature
<223> séquence nucléique antisens de GS-N24 :6812...6432

<220>
<221> misc_feature
<223> séquence nucléique antisens de GS-N49 :6934...6554

<400> 126
aaaatgacgc tccacctgct gctgatcaac aactcgaaaa gacagtgact gcttagttac 60
ttgccgatca taaatcttat cttccatggt tccctgagct aagaacctat atacataaac 120
aggcttagtt tgtccaaagc gataaactct gaatatactc tggatgtcat aagatggatt 180
ccaagaagcg tcgaatataa ttactcgatt agcagctacc agattaattc ctagagatcc 240
tgctttagta gaaatgataa atgatcgtcc tctcacatta gtttcatcat taaattcttc 300
agcccacttc ttccttgact gtgcagtagt ggaaccatct aaacggtaat agtcaatgtt 360
tcgaagccac ttcccctcac c 381

<210> 127
<211> 395
<212> DNA
<213> Homo sapiens
<220>
<221> misc_feature
<223> séquence nucléique antisens de GS-N25 :1388..992

<220>
<221> misc_feature
<223> séquence nucléique antisens de GS-N50 :1387..991

<400> 127
cccttagaca ttgtcccaaa ccttgttact tgagtattgt agccttacca tgattttttt 60
taacaccgta tcatctccat actttttatt tacaaattat atatacacac aataatacaa 120
ttccttcatt ctaaaacaat agtagacccc aaacaggtct acattaagtt tctgtactag 180
cagttcactc agatagcttc gtttgtttgt tgttgtttcc acataaccgc actgatcatg 240
ccatacagtt aatttttatt tgtttatgct accttctgag attaacttaa ggctctagtt 300
taatgcaaat ggtttagtct taaaaggagt ctcacgtgag gctaaaatca cacaccaata 360
actctccttt gaagctgttt ctccttggtg gatgg 395

<210> 128
<211> 381
<212> DNA
<213> Homo sapiens
<220>
<221> misc_feature
<223> séquence nucléique antisens de GS-N26 :1300...920

<400> 128
ttccctgctg ccatcaatca gggcttgaaa taagggcttg ttatgaggta tggtctgtaa 60
gatattccca tccccagtca catagccaat ggcccactgt cccaatcgag tgcaacttaa 120
ccggaaaata tagcttccgg gtttggtgct atatttctgt agtcgtgcat taacttcatc 180
atatgtgaga aatgccatgt aacctggatg tgtcacagct aagaaattcc aattccgcaa 240
aatagagccc caaggctgaa acagcctggt aaaaatatca aattcaaaaa ctgaaatgta 300
atcattgcaa gttaaatcaa ttgttgattt tagagccatt gcttccaggc cagagctaat 360
ctggtggacc tcatgaaggc a 381

<210> 129
<211> 298
<212> DNA
<213> Homo sapiens

<220>
<221> misc_feature
<223> séquence nucléique antisens de GS-N27: 3171...2813

<220>
<221> misc_feature
<223> séquence nucléique antisens de GS-N51: 3813...3515

<400> 129
cgagtgggca ggcctgtatc ttctcagcaa agtaaggatt gagtatagag agctgtttgt 60
cttaactggg cttccctgaa gaatctgagc caaactggaa gaaaccagcc tcatttccag 120
tgttgagatg ttagctgtac agtggctgta caactgcaga gtttatttat agaattagaa 180
ataattttta aaattttaaa aggttttgtg taatcattaa ccagagatga tattcacaaa 240
ttctggtaaa aatttgactc tccaactatc accatatcaa caggaaacag ggccatgc 298

<210> 130
<211> 413
<212> DNA
<213> Homo sapiens
<220>
<221> misc_feature
<223> séquence nucléique antisens de GS-N28: 704...292

<400> 130
tgggaagcat tggctggttc tcacagtaca cacgaggaca gtaaccaaag tctccttgct 60
ggtacttttc caacatctgg gcgatgccac ggttggtaag gatgtagcgg gcgtggatca 120
atccataaag catctcggct gcctgctcaa tcaggtcact ctggttgggg ttgtcttcca 180
gttcttcatc aggctccagg tccaagatca tgtctagagc ttgtcgatag tgagggacct 240
gctcattgag tccagtaaga ttaaatttgt cctggatgta gtcttcatcc acttcacaga 300
agaattcatt gccacggagc ccacagaacc aggaaatcca ggacacctcc tctgagctgc 360
tcatcttcac gtcagcggct ggacggggac caggactggg gtggggtagg gaa 413

<210> 131
<211> 564
<212> DNA
<213> Homo sapiens
<220>
<221> misc_feature
<223> séquence nucléique antisens de GS-N29 :14213...13650

<220>
<221> misc_feature
<223> séquence nucléique antisens de GS-N52 :5095...4532

<400> 131
tcccacaact ttcgctgcat gttccccaag gctgccaagc gctccatgct ccatgaactg 60
ggcaaggcct aatgttgcac ataattattt ccacagcatt cccttcacat ggccgcccac 120
catgctgaac tgatggatta ttgcaagtcc tggtcctggt ttggttgccg cgtccacagc 180
tccttgtgca ttcttcccaa agactccatt ccgaccagct accatccact ggacaaggct 240
tattttgaca gtttcgcatt tccaaatctg aaccttggca gggcttccca ccattggctg 300
gaaggggctg gttgcacaga cgactcctct tttggatgcc ttttccacag gtgacactgc 360
aggctctcca tgcagaccac tgggaaaatc caccatgaac ctggactatg actggcactc 420
tgacaaggac agaacccatt aagtttcgag caacacattc aaattcagag atatcttctt 480
tctgagcatc agcaatatat aatgagttgt tggacaacac gttaacccgg tcatcccagg 540
aaatagagtg cccttgacgg gacc 564

<210> 132
<211> 414
<212> DNA
<213> Homo sapiens
<220>
<221> misc_feature
<223> séquence nucléique antisens de GS-N30 :3068...2655

<400> 132
tgaggctggg ttctgggttc cctgcaaggc cattaaatct tgggacactt gctcccgtaa 60
ctgtttgagt ttcttctcaa taacatgcac cttttcttca gcttcaatac ggtcttctcc 120
atgtccctta atgagaaggc tgtttgaaat ctcctgtaac atgtccactt gaggttgacg 180
ttctaccagc tccttttcca gttgcattag ttccctccga cactctagga gagcccgggg 240
gtctgccttt ggatcggtga catgagcctt ctgcctccgg ttcttggcac tcgctaacca 300

cagcagcaga ttttgactca actggtggaa gtcctggcac tgcatgagcg actgtcgtaa    360
gccccctctc cagctgtcca ctgcgccctg tgctgcttcc cagagcaggc tcag          414

<210>  133
<211>  298
<212>  DNA
<213>  Homo sapiens
<220>
<221>  misc_feature
<223>  séquence nucléique antisens de GS-N31 :3239...2942

<220>
<221>  misc_feature
<223>  séquence nucléique antisens de GS-N53 :3178...2881

<400>  133
agctggcctt ggggttgaag gaaggatctt gaatgacaga taatccccaa gtgtccactg     60
gatcattttt cctctgactt tccacacaaa tgaggctttc catgggcttt gcaaccacag    120
tgtctaagac aatcagaagg gttccaggga ggtcttgtct gtagcgatgt gacagatgag    180
gaaactgcag ctcagaggac aatgacttgc ccacgttgca cagctcgctg acagtagaga    240
ggggctgaaa cctaggtctc ctgactcaca cgccacaacc atgactggct cagctccc      298

<210>  134
<211>  365
<212>  DNA
<213>  Homo sapiens
<220>
<221>  misc_feature
<223>  séquence nucléique antisens de GS-N32:4779...4443

<400>  134
cctctttcag cctgggcact ttgttttcct tctttacccg aggcttccgc ttctttaatt     60
tggcctcttc cccacctgtc acagccccct tcttctccag accctttctg agcagcttca    120
acaagtattc cgctcgggtc tgtagctgct tcccctgagg cttcttatct gtctccaccg    180
gcaggatttt gtcagttaat ttaagctctg ggtctgtttt aattaactcc cagtttccat    240
agccatgttc ataaatcccc agcaacaggc gagaatcatc ttccaccctc cactctacat    300
caaaatgtgc agctttgaca cgacaggtta agcagtattt ttttttcttt cagggtccac    360
aggga                                                                365

<210>  135
<211>  270
<212>  DNA
<213>  Homo sapiens
<220>
<221>  misc_feature
<223>  séquence nucléique antisens de GS-N33 :4033...3764

<400>  135
tggaagcgct aaggcgtgac actgctactt gctgtgcaga ggatgactct gttttctcat     60
tcgctttctt ggggggcttt ttagtagaat tgagctgtcc gctgacatct tgtaaccgct    120
tttctaattc ccgctttttc tccaaagcca gttcctcctt tgtctttccc acaggcttct    180
taatggtgta gggcttccgg ggtttcttac gtaggcagga aaggacatag cgctcaagct    240
ctctaagtgt ggatggcttg agtgcttcaa                                     270

<210>  136
<211>  298
<212>  DNA
<213>  Homo sapiens
<220>
<221>  misc_feature
<223>  séquence nucléique antisens de GS-N34 :984..688

<400>  136
ttccgggcct gactctggta tttcacagct tttttcgttt catctcgtgc cttctccacg     60
tggtccactg tgtgcatgac attcaactct atgttatcta acatctcacc ctgattctcc    120
accagcatgg cgatgtccat aaacatgtcg tgaagctcct tgatgctgct ctccagcctc    180
acaatgtcct tgtgtcgtcc ctcaatctca ctgagggctt gcttggaaat ctgtgagtca    240
atgatcccag aagtgaagat ggccgggttg ccactctcca acatctcctc cagctcca      298

<210> 137
<211> 117
<212> DNA
<213> Homo sapiens
<220>
<221> misc_feature
<223> séquence nucléique antisens de GS-N35 6834..6718

<400> 137
tgcttggtgt gcaggcttgt ccctgtcctc tggggcaagg ccctctggtg catctgcctt 60
ataggcagca gaggcagagg cctcagcgag ctttgcgatg tgttccacgg cctgctc 117


<210> 138
<211> 96
<212> DNA
<213> Homo sapiens
<220>
<221> misc_feature
<223> séquence nucléique antisens de GS-N36 :708...614

<400> 138
tggagctggg tgagttgatg aagatgccac tggaatcata agaggatctt gttgtcttga 60
tatcggagat ctcatcagag gttgtaggtt cctctg 96


<210> 139
<211> 393
<212> DNA
<213> Homo sapiens
<220>
<221> misc_feature
<223> séquence nucléique antisens de GS-N37 : 3076...2685

<400> 139
tccaaggatt tggcctgtgg ctggggaaat aacacacacg agataggtaa caaggattta 60
cactgacata tggggtgttg gtttggctct attctttttc ggttaccact tcttgcagtg 120
taggtggaga tgcctctgtt ccttcttccc agccctcagc aacaccagca agctcgtagt 180
gctttttccg aagctctccc agtttttgac gctccttctg caattcattt tctagctcta 240
gcaccctaac ctgagaatcc atctcttggc gtttgatctg tgtcagcgtc atgcttgaga 300
agtccatgtt gtctgtctct tcgatctgtg atttgccgga aatggttgag gccacaacgc 360
cggcagtggc ctggttcact ccccgagagg cct 393


<210> 140
<211> 100
<212> DNA
<213> Homo sapiens
<220>
<221> misc_feature
<223> séquence nucléique antisens de GS-N38 :1341...1242

<400> 140
ctgtagagag ttcctgtaaa ctatccttat cttcttcatc tgatccaaga aatttgtgaa 60
gtttatgaat ccaagttagc ccaacactat gtacaccagc 100


<210> 141
<211> 90
<212> DNA
<213> Homo sapiens
<220>
<221> misc_feature
<223> séquence nucléique antisens de GS-N39 :1482...1393

<400> 141
gctgggggaa gggtgcagca acgatttctc accaaatcac tacacaggac agcaaagggg 60
tgagaagggg ctgagggagg aaaagccagg 90


<210> 142
<211> 144

<212>  DNA
<213>  Homo sapiens
<220>
<221>  misc_feature
<223>  séquence nucléique antisens de GS-N40 :283...140

<400>  142
tggaggaaga tccactggga aattccctga ggccaggaag gttcagcttc agtccatttg     60
gtctacagac accttgattc tccagaggaa aattctttga cttttgagaa gattgaacag    120
caggatcatc atcaaaggtg accc                                           144


<210>  143
<211>  113
<212>  DNA
<213>  Homo sapiens
<220>
<221>  misc_feature
<223>  séquence nucléique antisens de GS-N41 169..57

<400>  143
cgagcttcat tccgcatcag gtgcagctcc cggaatttgc gcagtctctg ttcgcgcttc     60
tgagcggcca gctccgccgc cgcagcgagg gacccctcct ccgcgctgtc cac           113


<210>  144
<211>  180
<212>  DNA
<213>  Homo sapiens
<220>
<221>  misc_feature
<223>  séquence nucléique antisens de GS-N42 :4397...4218

<400>  144
tggttccgtt gggttaacag tgtcatttta aaatgccata aattaaataa gttagttcac     60
attttttttc ccccagcact taagttacag ttagtctcta atgtgctttt gaagcttgct    120
ttagctgttc caggtctctt ctgcggacag tggtggctct gggacaacca tattcgtcca    180


<210>  145
<211>  507
<212>  DNA
<213>  Homo sapiens
<220>
<221>  misc_feature
<223>  séquence nucléique antisens de GS-N43 3428...2922

<400>  145
gcaaaaactg ccacaatctt gctcaaagtg tctaagtatc caaatggtag caaatgagct     60
gcattaagct ttatataacc acattgcata tgaagaaaaa aaatgaaatt tatgccgata    120
taaatagaaa tgttacagag tatcatatac tgagagtaac ctatgcagtt cttttaaact    180
tatctcttta gtggtttcaa taaaatatca ctaaccactt atccctaaag cctcaagttt    240
tcttcatttg aatgctgtag aattgcatta gtattttata ttgattatat aaattaagac    300
caatttctat ctaagtatgt gcttcaagca atgttttgtg gcaaacacac aaaacatgct    360
ttgctcttca aatttattat ccttaaaata atgcactttt aaagccttca cttcatagtt    420
tggtaactaa gatccacatg atacacaatg ccttgctgaa cacagaaaat gtatctgcat    480
tatgataatg aaacccggct tttgctg                                        507

<210>  146
<211>  632
<212>  DNA
<213>  Homo sapiens
<220>
<221>  misc_feature
<223>  séquence nucléique antisens de GS-N44 1345...2714

<400>  146
ggcagcctga agaaggtagg aaagccttag tagagggaaa atcaaaccaa atgagattag     60
gtcaaaagaa cagataacta tcgtctgctt atctacttaa aggtgtgtgg catttaccac    120
agtgaggagc taacaggagc ctcccacact acatgcaggg cccaaagggg ccagcgatgg    180
tgtgataacc agtcaccgat gatgagttgc agttggacca cgtacagatg gaggggctgg    240
cccatgcagg gcctcggggt gaggttcact aggactagct gcagggggac ctggctacac    300
tctgtggcac aacagggctt cacaaaatgg acactacacg tgacgtgggc tgaaaggtgg    360

agcagagact gggtgggagt gtcagttgta cgttgtgggg taagggggag agggagcagg    420
gcaagtgtat tatgtactgt gctctaacat tctttggaag tctttcaaag attgctgcag    480
ttccttttta aaacaggtct cttattccgt cattcttgaa agacttctcc ttacaccatc    540
tcatactgat tggccgtgat gaaccgggac agacagcagg ccagcagcat gccaattaac    600
tgggagaatg cgattccaaa cgccactcca gc                                  632

<210>  147
<211>  704
<212>  DNA
<213>  Homo sapiens
<220>
<221>  misc_feature
<223>  séquence nucléique antisens de GS-N45 :1693...1990

<400>  147
gcaagcctat gatggccact gtgaaaagga ccagggagaa tagaaccatg aggaagacat     60
aggtggagtg ggaggaaggt gtggacaatg gttgctcagc tgggatcatg ttggtcaggt    120
tcagcatgta gcccaaagtc cagccggcgt cgctgccctg gatcttgcca atgaaatgga    180
tgtgctccca ggaatcagct gtgaaatgat agccttgcag aaggagggag agaatgtagg    240
taccagaaaa gcagtattca ctcaggtact tctcctttac tccagcgtaa gatgttttta    300
tctcctctca aggctgagca cagaactttt tcatcatctc agtcaccttt tcctgagaga    360
ctttctctga tgtcaagttt aaaaacttca tcacaaagta aaaagctgaa aatgccccaa    420
aatccccctg gagtggtggc aagaaaatcc cattgaaggc acactgggag taagggcagt    480
aactggtgtt gaagagctcc aggatgcttt gatggcattg ttgatagttt ccaataccct    540
ggatttcaaa ctgctggaat ggaagagtca tctcaaatct cttggtgcag ggggtcttgt    600
aaaggtcact tacgttcact accttcttat atccaggatg aaagcatggg tccctgagaa    660
tttcattact tgcaacctga atgtccttgg ccagtttctg ccag                     704

<210>  148
<211>  257
<212>  DNA
<213>  Homo sapiens
<220>
<221>  misc_feature
<223>  séquence nucléique antisens de GS-N46 :3071...2889

<400>  148
ggccgcggga attcgattat taaccctcac taaatgctgg ggatcatgga tcctctgagg     60
actgttttta aaaagctgaa gaggagaagg aagagaggac tacttcttcc cataggtgca    120
gctgcaaaga catcaagctg gacccatcga ggatttcgga ttcagccatc atggggctgc    180
tgccggggaa gccactgccc ctcctttctc cccagcattt agtgagggtt aataatcact    240
agtgaattcg cggccgc                                                   257

<210>  149
<211>  5717
<212>  DNA
<213>  Homo sapiens
<220>
<221>  misc_feature
<223>  vecteur d'expression appelé GS-V1 contenant la séquence antisens
       de GS-N1 ;
       antisens: 1108...1364

<400>  149
tcaatattgg ccattagcca tattattcat tggttatata gcataaatca atattggcta     60
ttggccattg catacgttgt atctatatca taatatgtac atttatattg gctcatgtcc    120
aatatgaccg ccatgttggc attgattatt gactagttat taatagtaat caattacggg    180
gtcattagtt catagcccat atatggagtt ccgcgttaca taacttacgg taaatggccc    240
gcctggctga ccgcccaacg acccccgccc attgacgtca ataatgacgt atgttcccat    300
agtaacgcca atagggactt tccattgacg tcaatgggtg gagtatttac ggtaaactgc    360
ccacttggca gtacatcaag tgtatcatat gccaagtccg ccccctattg acgtcaatga    420
cggtaaatgg cccgcctggc attatgccca gtacatgacc ttacgggact ttcctacttg    480
gcagtacatc tacgtattag tcatcgctat taccatggtg atgcggtttt ggcagtacac    540
caatgggcgt ggatagcggt ttgactcacg gggatttcca agtctccacc ccattgacgt    600
caatgggagt ttgttttggc accaaaatca acgggacttt ccaaaatgtc gtaacaactg    660
cgatcgcccg ccccgttgac gcaaatgggc ggtaggcgtg tacggtggga ggtctatata    720
agcagagctc gtttagtgaa ccgtcagatc actagaagct ttattgcggt agtttatcac    780
agttaaattg ctaacgcagt cagtgcttct gacacaacag tctcgaactt aagctgcagt    840
gactctctta aggtagcctt gcagaagttg gtcgtgaggc actgggcagg taagtatcaa    900
ggttacaaga caggtttaag gagaccaata gaaactgggc ttgtcgagac agagaagact    960

cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtctg gttggctttc caggtaggct ggatcctctt 1140
agattaggag gtctcagtaa gaacaagatc aatgcaataa ccatccaggc taccaagatc 1200
attgtaacac tgatgccatt atcaccagag ggtcccggta attcctgaag acactctgtg 1260
tctgtgcagt aggactggga ctgccgtaac agattgatca gtcttctcat tgcatgttca 1320
tgagagcaaa cacattcaca gggatcaaat ccaccttctg ccatgtcgac ccgggcggcc 1380
gcttcccttt agtgagggtt aatgcttcga gcagacatga taagatacat tgatgagttt 1440
ggacaaacca caactagaat gcagtgaaaa aaatgcttta tttgtgaaat ttgtgatgct 1500
attgctttat ttgtaaccat tataagctgc aataaacaag ttaacaacaa caattgcatt 1560
cattttatgt ttcaggttca gggggagatg tgggaggttt tttaaagcaa gtaaaacctc 1620
tacaaatgtg gtaaaatccg ataaggatcg atccgggctg gcgtaatagc gaagaggccc 1680
gcaccgatcg cccttcccaa cagttgcgca gcctgaatgg cgaatggacg cgccctgtag 1740
cggcgcatta agcgcggcgg gtgtggtggt tacgcgcagc gtgaccgcta cacttgccag 1800
cgccctagcg cccgctcctt tcgctttctt cccttccttt ctcgccacgt tcgccggctt 1860
tccccgtcaa gctctaaatc gggggctccc tttagggttc cgatttagtg ctttacggca 1920
cctcgacccc aaaaaacttg attagggtga tggttcacgt agtgggccat cgccctgata 1980
gacggttttt cgccctttga cgttggagtc cacgttcttt aatagtggac tcttgttcca 2040
aactggaaca acactcaacc ctatctcggt ctattctttt gatttataag ggattttgcc 2100
gatttcggcc tattggttaa aaaatgagct gatttaacaa aaatttaacg cgaattttaa 2160
caaaatatta acgcttacaa tttcctgatg cggtattttc tccttacgca tctgtgcggt 2220
atttcacacc gcatacgcgg atctgcgcag caccatggcc tgaaataacc tctgaaagag 2280
gaacttggtt aggtaccttc tgaggcggaa agaaccagct gtggaatgtg tgtcagttag 2340
ggtgtggaaa gtccccaggc tccccagcag gcagaagtat gcaaagcatg catctcaatt 2400
agtcagcaac caggtgtgga aagtccccag gctccccagc aggcagaagt atgcaaagca 2460
tgcatctcaa ttagtcagca accatagtcc cgcccctaac tccgcccatc ccgcccctaa 2520
ctccgcccag ttccgcccat tctccgcccc atggctgact aatttttttt atttatgcag 2580
aggccgaggc cgcctcggcc tctgagctat tccagaagta gtgaggaggc ttttttggag 2640
gcctaggctt ttgcaaaaag cttgattctt ctgacacaac agtctcgaac ttaaggctag 2700
agccaccatg attgaacaag atggattgca cgcaggttct ccggccgctt gggtggagag 2760
gctattcggc tatgactggg cacaacagac aatcggctgc tctgatgccg ccgtgttccg 2820
gctgtcagcg caggggcgcc cggttctttt tgtcaagacc gacctgtccg gtgccctgaa 2880
tgaactgcag gacgaggcag cgcggctatc gtggctggcc acgacgggcg ttccttgcgc 2940
agctgtgctc gacgttgtca ctgaagcggg aagggactgg ctgctattgg gcgaagtgcc 3000
ggggcaggat ctcctgtcat ctcaccttgc tcctgccgag aaagtatcca tcatggctga 3060
tgcaatgcgg cggctgcata cgcttgatcc ggctacctgc ccattcgacc accaagcgaa 3120
acatcgcatc gagcgagcac gtactcggat ggaagccggt cttgtcgatc aggatgatct 3180
ggacgaagag catcaggggc tcgcgccagc cgaactgttc gccaggctca aggcgcgcat 3240
gcccgacggc gaggatctcg tcgtgaccca tggcgatgcc tgcttgccga atatcatggt 3300
ggaaaatggc cgcttttctg gattcatcga ctgtggccgg ctgggtgtgg cggaccgcta 3360
tcaggacata gcgttggcta cccgtgatat tgctgaagag cttggcggcg aatgggctga 3420
ccgcttcctc gtgctttacg gtatcgccgc tcccgattcg cagcgcatcg ccttctatcg 3480
ccttcttgac gagttcttct gagcgggact ctggggttcg aaatgaccga ccaagcgacg 3540
cccaacctgc catcacgatg gccgcaataa aatatcttta ttttcattac atctgtgtgt 3600
tggttttttg tgtgaatcga tagcgataag gatccgcgta tggtgcactc tcagtacaat 3660
ctgctctgat gccgcatagt taagccagcc ccgacacccg ccaacacccg ctgacgcgcc 3720
ctgacgggct tgtctgctcc cggcatccgc ttacagacaa gctgtgaccg tctccgggag 3780
ctgcatgtgt cagaggtttt caccgtcatc accgaaacgc gcgagacgaa agggcctcgt 3840
gatacgccta tttttatagg ttaatgtcat gataataatg gtttcttaga cgtcaggtgg 3900
cacttttcgg ggaaatgtgc gcggaacccc tatttgttta tttttctaaa tacattcaaa 3960
tatgtatccg ctcatgagac aataaccctg ataaatgctt caataatatt gaaaaaggaa 4020
gagtatgagt attcaacatt tccgtgtcgc ccttattccc ttttttgcgg cattttgcct 4080
tcctgttttt gctcacccag aaacgctggt gaaagtaaaa gatgctgaag atcagttggg 4140
tgcacgagtg ggttacatcg aactggatct caacagcggt aagatccttg agagttttcg 4200
ccccgaagaa cgttttccaa tgatgagcac ttttaaagtt ctgctatgtg gcgcggtatt 4260
atcccgtatt gacgccgggc aagagcaact cggtcgccgc atacactatt ctcagaatga 4320
cttggttgag tactcaccag tcacagaaaa gcatcttacg gatggcatga cagtaagaga 4380
attatgcagt gctgccataa ccatgagtga taacactgcg gccaacttac ttctgacaac 4440
gatcggagga ccgaaggagc taaccgcttt tttgcacaac atgggggatc atgtaactcg 4500
ccttgatcgt tgggaaccgg agctgaatga agccatacca aacgacgagc gtgacaccac 4560
gatgcctgta gcaatggcaa caacgttgcg caaactatta actggcgaac tacttactct 4620
agcttcccgg caacaattaa tagactggat ggaggcggat aaagttgcag gaccacttct 4680
gcgctcggcc cttccggctg gctggtttat tgctgataaa tctggagccg gtgagcgtgg 4740
gtctcgcggt atcattgcag cactggggcc agatggtaag ccctcccgta tcgtagttat 4800
ctacacgacg gggagtcagg caactatgga tgaacgaaat agacagatcg ctgagatagg 4860
tgcctcactg attaagcatt ggtaactgtc agaccaagtt tactcatata tactttagat 4920
tgatttaaaa cttcattttt aatttaaaag gatctaggtg aagatccttt ttgataatct 4980
catgaccaaa atcccttaac gtgagttttc gttccactga gcgtcagacc ccgtagaaaa 5040
gatcaaagga tcttcttgag atcctttttt tctgcgcgta atctgctgct tgcaaacaaa 5100
aaaaccaccg ctaccagcgg tggtttgttt gccggatcaa gagctaccaa ctctttttcc 5160
gaaggtaact ggcttcagca gagcgcagat accaaatact gttcttctag tgtagccgta 5220
gttaggccac cacttcaaga actctgtagc accgcctaca tacctcgctc tgctaatcct 5280
gttaccagtg gctgctgcca gtggcgataa gtcgtgtctt accgggttgg actcaagacg 5340

atagttaccg gataaggcgc agcggtcggg ctgaacgggg ggttcgtgca cacagcccag 5400
cttggagcga acgacctaca ccgaactgag atacctacag cgtgagctat gagaaagcgc 5460
cacgcttccc gaagggagaa aggcggacag gtatccggta agcggcaggg tcggaacagg 5520
agagcgcacg agggagcttc cagggggaaa cgcctggtat ctttatagtc ctgtcgggtt 5580
tcgccacctc tgacttgagc gtcgattttt gtgatgctcg tcaggggggc ggagcctatg 5640
gaaaaacgcc agcaacgcgg cctttttacg gttcctggcc ttttgctggc cttttgctca 5700
catggctcga cagatct 5717

<210> 150
<211> 5662
<212> DNA
<213> Homo sapiens
<220>
<221> misc_feature
<223> vecteur d'expression appelé GS-V2 contenant la séquence antisens
de GS-N2;
antisens: 1108...1309

<400> 150
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtgac cggggaatgg aaggagatat cccatctgat 1140
atccactccc caggtccagg ggcacagact ctgaacagac ctaccttctg gctctgctca 1200
cagtggaaag aggatgggta ccaggcctga gattgtggga gtggcaatac cctctgggag 1260
tctcgggcag atcaccatct tgggctcccc ctgaccccat ccctatcccg tcgacccggg 1320
cggccgcttc cctttagtga gggttaatgc ttcgagcaga catgataaga tacattgatg 1380
agtttggaca aaccacaact agaatgcagt gaaaaaaatg ctttatttgt gaaatttgtg 1440
atgctattgc tttatttgta accattataa gctgcaataa acaagttaac aacaacaatt 1500
gcattcattt tatgtttcag gttcaggggg agatgtggga ggttttttaa agcaagtaaa 1560
acctctacaa atgtggtaaa atccgataag gatcgatccg ggctggcgta atagcgaaga 1620
ggcccgcacc gatcgccctt cccaacagtt gcgcagcctg aatggcgaat ggacgcgccc 1680
tgtagcggcg cattaagcgc ggcgggtgtg gtggttacgc gcagcgtgac cgctacactt 1740
gccagcgccc tagcgcccgc tcctttcgct ttcttccctt cctttctcgc cacgttcgcc 1800
ggctttcccc gtcaagctct aaatcggggg ctccctttag ggttccgatt tagtgcttta 1860
cggcacctcg accccaaaaa acttgattag ggtgatggtt cacgtagtgg gccatcgccc 1920
tgatagacgg tttttcgccc tttgacgttg gagtccacgt tctttaatag tggactcttg 1980
ttccaaactg gaacaacact caaccctatc tcggtctatt cttttgattt ataagggatt 2040
ttgccgattt cggcctattg gttaaaaaat gagctgattt aacaaaaatt taacgcgaat 2100
tttaacaaaa tattaacgct tacaatttcc tgatgcggta ttttctcctt acgcatctgt 2160
gcggtatttc acaccgcata cgcggatctg cgcagcacca tggcctgaaa taacctctga 2220
aagaggaact tggttaggta ccttctgagg cggaaagaac cagctgtgga atgtgtgtca 2280
gttagggtgt ggaaagtccc caggctcccc agcaggcaga agtatgcaaa gcatgcatct 2340
caattagtca gcaaccaggt gtggaaagtc cccaggctcc ccagcaggca gaagtatgca 2400
aagcatgcat ctcaattagt cagcaaccat agtcccgccc ctaactccgc ccatcccgcc 2460
cctaactccg cccagttccg cccattctcc gccccatggc tgactaattt tttttattta 2520
tgcagaggcc gaggccgcct cggcctctga gctattccag aagtagtgag gaggcttttt 2580
tggaggccta ggcttttgca aaaagcttga ttcttctgac acaacagtct cgaacttaag 2640
gctagagcca ccatgattga acaagatgga ttgcacgcag gttctccggc cgcttgggtg 2700
gagaggctat tcggctatga ctgggcacaa cagacaatcg gctgctctga tgccgccgtg 2760
ttccggctgt cagcgcaggg gcgcccggtt ctttttgtca agaccgacct gtccggtgcc 2820
ctgaatgaac tgcaggacga ggcagcgcgg ctatcgtggc tggccacgac gggcgttcct 2880
tgcgcagctg tgctcgacgt tgtcactgaa gcgggaaggg actggctgct attgggcgaa 2940
gtgccggggc aggatctcct gtcatctcac cttgctcctg ccgagaaagt atccatcatg 3000
gctgatgcaa tgcggcggct gcatacgctt gatccggcta cctgcccatt cgaccaccaa 3060
gcgaaacatc gcatcgagcg agcacgtact cggatggaag ccggtcttgt cgatcaggat 3120
gatctggacg aagagcatca ggggctcgcg ccagccgaac tgttcgccag gctcaaggcg 3180

cgcatgcccg acggcgagga tctcgtcgtg acccatggcg atgcctgctt gccgaatatc 3240
atggtggaaa atggccgctt ttctggattc atcgactgtg gccggctggg tgtggcggac 3300
cgctatcagg acatagcgtt ggctacccgt gatattgctg aagagcttgg cggcgaatgg 3360
gctgaccgct tcctcgtgct ttacggtatc gccgctcccg attcgcagcg catcgccttc 3420
tatcgccttc ttgacgagtt cttctgagcg ggactctggg gttcgaaatg accgaccaag 3480
cgacgcccaa cctgccatca cgatggccgc aataaaatat ctttattttc attacatctg 3540
tgtgttggtt ttttgtgtga atcgatagcg ataaggatcc gcgtatggtg cactctcagt 3600
acaatctgct ctgatgccgc atagttaagc cagccccgac acccgccaac acccgctgac 3660
gcgccctgac gggcttgtct gctcccggca tccgcttaca gacaagctgt gaccgtctcc 3720
gggagctgca tgtgtcagag gttttcaccg tcatcaccga aacgcgcgag acgaaagggc 3780
ctcgtgatac gcctattttt ataggttaat gtcatgataa taatggtttc ttagacgtca 3840
ggtggcactt ttcggggaaa tgtgcgcgga acccctattt gtttattttt ctaaatacat 3900
tcaaatatgt atccgctcat gagacaataa ccctgataaa tgcttcaata atattgaaaa 3960
aggaagagta tgagtattca acatttccgt gtcgccctta ttcccttttt tgcggcattt 4020
tgccttcctg tttttgctca cccagaaacg ctggtgaaag taaaagatgc tgaagatcag 4080
ttgggtgcac gagtgggtta catcgaactg gatctcaaca gcggtaagat ccttgagagt 4140
tttcgccccg aagaacgttt tccaatgatg agcactttta aagttctgct atgtggcgcg 4200
gtattatccc gtattgacgc cgggcaagag caactcggtc gccgcataca ctattctcag 4260
aatgacttgg ttgagtactc accagtcaca gaaaagcatc ttacggatgg catgacagta 4320
agagaattat gcagtgctgc cataaccatg agtgataaca ctgcggccaa cttacttctg 4380
acaacgatcg gaggaccgaa ggagctaacc gcttttttgc acaacatggg ggatcatgta 4440
actcgccttg atcgttggga accggagctg aatgaagcca taccaaacga cgagcgtgac 4500
accacgatgc ctgtagcaat ggcaacaacg ttgcgcaaac tattaactgg cgaactactt 4560
actctagctt cccggcaaca attaatagac tggatggagg cggataaagt tgcaggacca 4620
cttctgcgct cggcccttcc ggctggctgg tttattgctg ataaatctgg agccggtgag 4680
cgtgggtctc gcggtatcat tgcagcactg gggccagatg gtaagccctc ccgtatcgta 4740
gttatctaca cgacggggag tcaggcaact atggatgaac gaaatagaca gatcgctgag 4800
ataggtgcct cactgattaa gcattggtaa ctgtcagacc aagtttactc atatatactt 4860
tagattgatt taaaacttca tttttaattt aaaaggatct aggtgaagat cctttttgat 4920
aatctcatga ccaaaatccc ttaacgtgag ttttcgttcc actgagcgtc agaccccgta 4980
gaaaagatca aaggatcttc ttgagatcct ttttttctgc gcgtaatctg ctgcttgcaa 5040
acaaaaaaac caccgctacc agcggtggtt tgtttgccgg atcaagagct accaactctt 5100
tttccgaagg taactggctt cagcagagcg cagataccaa atactgttct tctagtgtag 5160
ccgtagttag gccaccactt caagaactct gtagcaccgc ctacatacct cgctctgcta 5220
atcctgttac cagtggctgc tgccagtggc gataagtcgt gtcttaccgg gttggactca 5280
agacgatagt taccggataa ggcgcagcgg tcgggctgaa cggggggttc gtgcacacag 5340
cccagcttgg agcgaacgac ctacaccgaa ctgagatacc tacagcgtga gctatgagaa 5400
agcgccacgc ttcccgaagg gagaaaggcg gacaggtatc cggtaagcgg cagggtcgga 5460
acaggagagc gcacgaggga gcttccaggg ggaaacgcct ggtatcttta tagtcctgtc 5520
gggtttcgcc acctctgact tgagcgtcga tttttgtgat gctcgtcagg ggggcggagc 5580
ctatggaaaa acgccagcaa cgcggccttt ttacggttcc tggccttttg ctggcctttt 5640
gctcacatgg ctcgacagat ct 5662

<210> 151
<211> 5702
<212> DNA
<213> Homo sapiens
<220>
<221> misc_feature
<223> vecteur d'expression appelé GS-V3 contenant la séquence antisens
de GS-N3 ;
antisens: 1108...1349

<400> 151
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080

ataggctagc ctcgagaatt cacgcgttgg gtggggaagg caatctagct cactcagcag 1140
ggaaaatgga gctccccaat ggataacttt ttatgctcat aaaccctgaa atagtactaa 1200
aaatgagaaa aatccccaag acttctctga ttcccaggtt gggattagct gagggaatag 1260
gaacaaagga ccagaatcct acttccatga gccatgcctg gagtcagtat tgtctccagt 1320
gtctactccc tgaaatcggt tcctccagcg tcgacccggg cggccgcttc cctttagtga 1380
gggttaatgc ttcgagcaga catgataaga tacattgatg agtttggaca aaccacaact 1440
agaatgcagt gaaaaaaatg ctttatttgt gaaatttgtg atgctattgc tttatttgta 1500
accattataa gctgcaataa acaagttaac aacaacaatt gcattcattt tatgtttcag 1560
gttcaggggg agatgtggga ggttttttaa agcaagtaaa acctctacaa atgtggtaaa 1620
atccgataag gatcgatccg ggctggcgta atagcgaaga ggcccgcacc gatcgccctt 1680
cccaacagtt gcgcagcctg aatggcgaat ggacgcgccc tgtagcggcg cattaagcgc 1740
ggcgggtgtg gtggttacgc gcagcgtgac cgctacactt gccagcgccc tagcgcccgc 1800
tcctttcgct ttcttccctt cctttctcgc cacgttcgcc ggctttcccc gtcaagctct 1860
aaatcggggg ctccctttag ggttccgatt tagtgcttta cggcacctcg accccaaaaa 1920
acttgattag ggtgatggtt cacgtagtgg gccatcgccc tgatagacgg tttttcgccc 1980
tttgacgttg gagtccacgt tctttaatag tggactcttg ttccaaactg gaacaacact 2040
caaccctatc tcggtctatt cttttgattt ataagggatt ttgccgattt cggcctattg 2100
gttaaaaaat gagctgattt aacaaaaatt taacgcgaat tttaacaaaa tattaacgct 2160
tacaatttcc tgatgcggta ttttctcctt acgcatctgt gcggtatttc acaccgcata 2220
cgcggatctg cgcagcacca tggcctgaaa taacctctga aagaggaact tggttaggta 2280
ccttctgagg cggaaagaac cagctgtgga atgtgtgtca gttagggtgt ggaaagtccc 2340
caggctcccc agcaggcaga agtatgcaaa gcatgcatct caattagtca gcaaccaggt 2400
gtggaaagtc cccaggctcc ccagcaggca gaagtatgca aagcatgcat ctcaattagt 2460
cagcaaccat agtcccgccc ctaactccgc ccatcccgcc cctaactccg cccagttccg 2520
cccattctcc gccccatggc tgactaattt tttttattta tgcagaggcc gaggccgcct 2580
cggcctctga gctattccag aagtagtgag gaggcttttt tggaggccta ggcttttgca 2640
aaaagcttga ttcttctgac acaacagtct cgaacttaag gctagagcca ccatgattga 2700
acaagatgga ttgcacgcag gttctccggc cgcttgggtg gagaggctat tcggctatga 2760
ctgggcacaa cagacaatcg gctgctctga tgccgccgtg ttccggctgt cagcgcaggg 2820
gcgcccggtt ctttttgtca agaccgacct gtccggtgcc ctgaatgaac tgcaggacga 2880
ggcagcgcgg ctatcgtggc tggccacgac gggcgttcct tgcgcagctg tgctcgacgt 2940
tgtcactgaa gcgggaaggg actggctgct attgggcgaa gtgccggggc aggatctcct 3000
gtcatctcac cttgctcctg ccgagaaagt atccatcatg gctgatgcaa tgcggcggct 3060
gcatacgctt gatccggcta cctgcccatt cgaccaccaa gcgaaacatc gcatcgagcg 3120
agcacgtact cggatggaag ccggtcttgt cgatcaggat gatctggacg aagagcatca 3180
ggggctcgcg ccagccgaac tgttcgccag gctcaaggcg cgcatgcccg acggcgagga 3240
tctcgtcgtg acccatggcg atgcctgctt gccgaatatc atggtggaaa atggccgctt 3300
ttctggattc atcgactgtg gccggctggg tgtggcggac cgctatcagg acatagcgtt 3360
ggctacccgt gatattgctg aagagcttgg cggcgaatgg gctgaccgct tcctcgtgct 3420
ttacggtatc gccgctcccg attcgcagcg catcgccttc tatcgccttc ttgacgagtt 3480
cttctgagcg ggactctggg gttcgaaatg accgaccaag cgacgcccaa cctgccatca 3540
cgatggccgc aataaaatat ctttattttc attacatctg tgtgttggtt ttttgtgtga 3600
atcgatagcg ataaggatcc gcgtatggtg cactctcagt acaatctgct ctgatgccgc 3660
atagttaagc cagccccgac acccgccaac acccgctgac gcgccctgac gggcttgtct 3720
gctcccggca tccgcttaca gacaagctgt gaccgtctcc gggagctgca tgtgtcagag 3780
gttttcaccg tcatcaccga aacgcgcgag acgaaagggc ctcgtgatac gcctattttt 3840
ataggttaat gtcatgataa taatggtttc ttagacgtca ggtggcactt ttcggggaaa 3900
tgtgcgcgga acccctattt gtttattttt ctaaatacat tcaaatatgt atccgctcat 3960
gagacaataa ccctgataaa tgcttcaata atattgaaaa aggaagagta tgagtattca 4020
acatttccgt gtcgccctta ttcccttttt tgcggcattt tgccttcctg tttttgctca 4080
cccagaaacg ctggtgaaag taaaagatgc tgaagatcag ttgggtgcac gagtgggtta 4140
catcgaactg gatctcaaca gcggtaagat ccttgagagt tttcgccccg aagaacgttt 4200
tccaatgatg agcactttta aagttctgct atgtggcgcg gtattatccc gtattgacgc 4260
cgggcaagag caactcggtc gccgcataca ctattctcag aatgacttgg ttgagtactc 4320
accagtcaca gaaaagcatc ttacggatgg catgacagta agagaattat gcagtgctgc 4380
cataaccatg agtgataaca ctgcggccaa cttacttctg acaacgatcg gaggaccgaa 4440
ggagctaacc gcttttttgc acaacatggg ggatcatgta actcgccttg atcgttggga 4500
accggagctg aatgaagcca taccaaacga cgagcgtgac accacgatgc ctgtagcaat 4560
ggcaacaacg ttgcgcaaac tattaactgg cgaactactt actctagctt cccggcaaca 4620
attaatagac tggatggagg cggataaagt tgcaggacca cttctgcgct cggcccttcc 4680
ggctggctgg tttattgctg ataaatctgg agccggtgag cgtgggtctc gcggtatcat 4740
tgcagcactg gggccagatg gtaagccctc ccgtatcgta gttatctaca cgacggggag 4800
tcaggcaact atggatgaac gaaatagaca gatcgctgag ataggtgcct cactgattaa 4860
gcattggtaa ctgtcagacc aagtttactc atatatactt tagattgatt taaaacttca 4920
tttttaattt aaaaggatct aggtgaagat cctttttgat aatctcatga ccaaaatccc 4980
ttaacgtgag ttttcgttcc actgagcgtc agaccccgta gaaaagatca aaggatcttc 5040
ttgagatcct ttttttctgc gcgtaatctg ctgcttgcaa acaaaaaaac caccgctacc 5100
agcggtggtt tgtttgccgg atcaagagct accaactctt tttccgaagg taactggctt 5160
cagcagagcg cagataccaa atactgttct tctagtgtag ccgtagttag gccaccactt 5220
caagaactct gtagcaccgc ctacatacct cgctctgcta atcctgttac cagtggctgc 5280
tgccagtggc gataagtcgt gtcttaccgg gttggactca agacgatagt taccggataa 5340
ggcgcagcgg tcgggctgaa cggggggttc gtgcacacag cccagcttgg agcgaacgac 5400
ctacaccgaa ctgagatacc tacagcgtga gctatgagaa agcgccacgc ttcccgaagg 5460

gagaaaggcg gacaggtatc cggtaagcgg cagggtcgga acaggagagc gcacgaggga 5520
gcttccaggg ggaaacgcct ggtatcttta tagtcctgtc gggtttcgcc acctctgact 5580
tgagcgtcga tttttgtgat gctcgtcagg ggggcggagc ctatggaaaa acgccagcaa 5640
cgcggccttt ttacggttcc tggccttttg ctggcctttt gctcacatgg ctcgacagat 5700
ct 5702

<210> 152
<211> 5671
<212> DNA
<213> Homo sapiens
<220>
<221> misc_feature
<223> vecteur d'expression appelé GS-V4 contenant la séquence antisens
de GS-N4 appelé GS-V4; antisens: 1108...1318

<400> 152
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtggg gatgtcccat cttgtgctag agccaataaa 1140
atgctgacac tggtcttcag atgttgtcct gagcctattc caccaacata acgatgcaaa 1200
caaccaagag ccaatgaatg accagtccta gatacaacat ctcgagccga tttcaacttg 1260
tcaaagctat attgggccat tctagcaata aaagttgctt ctccaaccac ctgagccagt 1320
cgacccgggc ggccgcttcc ctttagtgag ggttaatgct tcgagcagac atgataagat 1380
acattgatga gtttggacaa accacaacta gaatgcagtg aaaaaaatgc tttatttgtg 1440
aaatttgtga tgctattgct ttatttgtaa ccattataag ctgcaataaa caagttaaca 1500
acaacaattg cattcatttt atgtttcagg ttcaggggga gatgtgggag gttttttaaa 1560
gcaagtaaaa cctctacaaa tgtggtaaaa tccgataagg atcgatccgg gctggcgtaa 1620
tagcgaagag gcccgcaccg atcgcccttc ccaacagttg cgcagcctga atggcgaatg 1680
gacgcgccct gtagcggcgc attaagcgcg gcgggtgtgg tggttacgcg cagcgtgacc 1740
gctacacttg ccagcgccct agcgcccgct cctttcgctt tcttcccttc ctttctcgcc 1800
acgttcgccg gctttccccg tcaagctcta aatcgggggc tccctttagg gttccgattt 1860
agtgctttac ggcacctcga ccccaaaaaa cttgattagg gtgatggttc acgtagtggg 1920
ccatcgccct gatagacggt ttttcgccct ttgacgttgg agtccacgtt ctttaatagt 1980
ggactcttgt tccaaactgg aacaacactc aaccctatct cggtctattc ttttgattta 2040
taagggattt tgccgatttc ggcctattgg ttaaaaaatg agctgattta acaaaaattt 2100
aacgcgaatt ttaacaaaat attaacgctt acaatttcct gatgcggtat tttctcctta 2160
cgcatctgtg cggtatttca caccgcatac gcggatctgc gcagcaccat ggcctgaaat 2220
aacctctgaa agaggaactt ggttaggtac cttctgaggc ggaaagaacc agctgtggaa 2280
tgtgtgtcag ttagggtgtg gaaagtcccc aggctcccca gcaggcagaa gtatgcaaag 2340
catgcatctc aattagtcag caaccaggtg tggaaagtcc ccaggctccc cagcaggcag 2400
aagtatgcaa agcatgcatc tcaattagtc agcaaccata gtcccgcccc taactccgcc 2460
catcccgccc ctaactccgc ccagttccgc ccattctccg ccccatggct gactaatttt 2520
ttttatttat gcagaggccg aggccgcctc ggcctctgag ctattccaga agtagtgagg 2580
aggctttttt ggaggcctag gcttttgcaa aaagcttgat tcttctgaca caacagtctc 2640
gaacttaagg ctagagccac catgattgaa caagatggat tgcacgcagg ttctccggcc 2700
gcttgggtgg agaggctatt cggctatgac tgggcacaac agacaatcgg ctgctctgat 2760
gccgccgtgt tccggctgtc agcgcagggg cgcccggttc tttttgtcaa gaccgacctg 2820
tccggtgccc tgaatgaact gcaggacgag gcagcgcggc tatcgtggct ggccacgacg 2880
ggcgttcctt gcgcagctgt gctcgacgtt gtcactgaag cgggaaggga ctggctgcta 2940
ttgggcgaag tgccggggca ggatctcctg tcatctcacc ttgctcctgc cgagaaagta 3000
tccatcatgg ctgatgcaat gcggcggctg catacgcttg atccggctac ctgcccattc 3060
gaccaccaag cgaaacatcg catcgagcga gcacgtactc ggatggaagc cggtcttgtc 3120
gatcaggatg atctggacga agagcatcag gggctcgcgc cagccgaact gttcgccagg 3180
ctcaaggcgc gcatgcccga cggcgaggat ctcgtcgtga cccatggcga tgcctgcttg 3240
ccgaatatca tggtggaaaa tggccgcttt tctggattca tcgactgtgg ccggctgggt 3300
gtggcggacc gctatcagga catagcgttg gctacccgtg atattgctga agagcttggc 3360

ggcgaatggg ctgaccgctt cctcgtgctt tacggtatcg ccgctcccga ttcgcagcgc 3420
atcgccttct atcgccttct tgacgagttc ttctgagcgg gactctgggg ttcgaaatga 3480
ccgaccaagc gacgcccaac ctgccatcac gatggccgca ataaaatatc tttattttca 3540
ttacatctgt gtgttggttt tttgtgtgaa tcgatagcga taaggatccg cgtatggtgc 3600
actctcagta caatctgctc tgatgccgca tagttaagcc agccccgaca cccgccaaca 3660
cccgctgacg cgccctgacg ggcttgtctg ctcccggcat ccgcttacag acaagctgtg 3720
accgtctccg ggagctgcat gtgtcagagg ttttcaccgt catcaccgaa acgcgcgaga 3780
cgaaagggcc tcgtgatacg cctattttta taggttaatg tcatgataat aatggtttct 3840
tagacgtcag gtggcacttt tcggggaaat gtgcgcggaa cccctatttg tttatttttc 3900
taaatacatt caaatatgta tccgctcatg agacaataac cctgataaat gcttcaataa 3960
tattgaaaaa ggaagagtat gagtattcaa catttccgtg tcgcccttat tccctttttt 4020
gcggcatttt gccttcctgt ttttgctcac ccagaaacgc tggtgaaagt aaaagatgct 4080
gaagatcagt tgggtgcacg agtgggttac atcgaactgg atctcaacag cggtaagatc 4140
cttgagagtt ttcgccccga agaacgtttt ccaatgatga gcacttttaa agttctgcta 4200
tgtggcgcgg tattatcccg tattgacgcc gggcaagagc aactcggtcg ccgcatacac 4260
tattctcaga atgacttggt tgagtactca ccagtcacag aaaagcatct tacggatggc 4320
atgacagtaa gagaattatg cagtgctgcc ataaccatga gtgataacac tgcggccaac 4380
ttacttctga caacgatcgg aggaccgaag gagctaaccg cttttttgca caacatgggg 4440
gatcatgtaa ctcgccttga tcgttgggaa ccggagctga atgaagccat accaaacgac 4500
gagcgtgaca ccacgatgcc tgtagcaatg gcaacaacgt tgcgcaaact attaactggc 4560
gaactactta ctctagcttc ccggcaacaa ttaatagact ggatggaggc ggataaagtt 4620
gcaggaccac ttctgcgctc ggcccttccg gctggctggt ttattgctga taaatctgga 4680
gccggtgagc gtgggtctcg cggtatcatt gcagcactgg ggccagatgg taagccctcc 4740
cgtatcgtag ttatctacac gacggggagt caggcaacta tggatgaacg aaatagacag 4800
atcgctgaga taggtgcctc actgattaag cattggtaac tgtcagacca agtttactca 4860
tatatacttt agattgattt aaaacttcat ttttaattta aaaggatcta ggtgaagatc 4920
ctttttgata atctcatgac caaaatccct taacgtgagt tttcgttcca ctgagcgtca 4980
gaccccgtag aaaagatcaa aggatcttct tgagatcctt tttttctgcg cgtaatctgc 5040
tgcttgcaaa caaaaaaacc accgctacca gcggtggttt gtttgccgga tcaagagcta 5100
ccaactcttt ttccgaaggt aactggcttc agcagagcgc agataccaaa tactgttctt 5160
ctagtgtagc cgtagttagg ccaccacttc aagaactctg tagcaccgcc tacatacctc 5220
gctctgctaa tcctgttacc agtggctgct gccagtggcg ataagtcgtg tcttaccggg 5280
ttggactcaa gacgatagtt accggataag gcgcagcggt cgggctgaac ggggggttcg 5340
tgcacacagc ccagcttgga gcgaacgacc tacaccgaac tgagatacct acagcgtgag 5400
ctatgagaaa gcgccacgct tcccgaaggg agaaaggcgg acaggtatcc ggtaagcggc 5460
agggtcggaa caggagagcg cacgagggag cttccagggg gaaacgcctg gtatctttat 5520
agtcctgtcg ggtttcgcca cctctgactt gagcgtcgat ttttgtgatg ctcgtcaggg 5580
gggcggagcc tatggaaaaa cgccagcaac gcggcctttt tacggttcct ggccttttgc 5640
tggccttttg ctcacatggc tcgacagatc t 5671

<210> 153
<211> 5651
<212> DNA
<213> Homo sapiens
<220>
<221> misc_feature
<223> vecteur d'expression appelé GS-V5 contenant la séquence antisens
      de GS-N5 ; antisens: 1108...1298

<400> 153
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtccg acacacgtcc ttggagactt gcctcatcaa 1140
cttctctgca ttcagacctt gcttctgctc ttctttggac tgttcagcct gcttctcagc 1200
ttgcttcaga agcctctgga acctctcatc ctccagcaca cctggtggaa ggtcagtctc 1260
tccctgagct ttcagttcct ccagcttctg ccgcaagtgt cgacccgggc ggccgcttcc 1320

ctttagtgag ggttaatgct tcgagcagac atgataagat acattgatga gtttggacaa 1380
accacaacta gaatgcagtg aaaaaaatgc tttatttgtg aaatttgtga tgctattgct 1440
ttatttgtaa ccattataag ctgcaataaa caagttaaca acaacaattg cattcatttt 1500
atgtttcagg ttcaggggga gatgtgggag gttttttaaa gcaagtaaaa cctctacaaa 1560
tgtggtaaaa tccgataagg atcgatccgg gctggcgtaa tagcgaagag gcccgcaccg 1620
atcgcccttc ccaacagttg cgcagcctga atggcgaatg gacgcgccct gtagcggcgc 1680
attaagcgcg gcgggtgtgg tggttacgcg cagcgtgacc gctacacttg ccagcgccct 1740
agcgcccgct cctttcgctt tcttcccttc ctttctcgcc acgttcgccg gctttccccg 1800
tcaagctcta aatcgggggc tccctttagg gttccgattt agtgctttac ggcacctcga 1860
ccccaaaaaa cttgattagg gtgatggttc acgtagtggg ccatcgccct gatagacggt 1920
ttttcgccct ttgacgttgg agtccacgtt ctttaatagt ggactcttgt tccaaactgg 1980
aacaacactc aaccctatct cggtctattc ttttgattta taagggattt tgccgatttc 2040
ggcctattgg ttaaaaaatg agctgattta acaaaaattt aacgcgaatt ttaacaaaat 2100
attaacgctt acaatttcct gatgcggtat tttctcctta cgcatctgtg cggtatttca 2160
caccgcatac gcggatctgc gcagcaccat ggcctgaaat aacctctgaa agaggaactt 2220
ggttaggtac cttctgaggc ggaaagaacc agctgtggaa tgtgtgtcag ttagggtgtg 2280
gaaagtcccc aggctcccca gcaggcagaa gtatgcaaag catgcatctc aattagtcag 2340
caaccaggtg tggaaagtcc ccaggctccc cagcaggcag aagtatgcaa agcatgcatc 2400
tcaattagtc agcaaccata gtcccgcccc taactccgcc catcccgccc ctaactccgc 2460
ccagttccgc ccattctccg ccccatggct gactaatttt ttttatttat gcagaggccg 2520
aggccgcctc ggcctctgag ctattccaga agtagtgagg aggctttttt ggaggcctag 2580
gcttttgcaa aaagcttgat tcttctgaca caacagtctc gaacttaagg ctagagccac 2640
catgattgaa caagatggat tgcacgcagg ttctccggcc gcttgggtgg agaggctatt 2700
cggctatgac tgggcacaac agacaatcgg ctgctctgat gccgccgtgt tccggctgtc 2760
agcgcagggg cgcccggttc tttttgtcaa gaccgacctg tccggtgccc tgaatgaact 2820
gcaggacgag gcagcgcggc tatcgtggct ggccacgacg ggcgttcctt gcgcagctgt 2880
gctcgacgtt gtcactgaag cgggaaggga ctggctgcta ttgggcgaag tgccggggca 2940
ggatctcctg tcatctcacc ttgctcctgc cgagaaagta tccatcatgg ctgatgcaat 3000
gcggcggctg catacgcttg atccggctac ctgcccattc gaccaccaag cgaaacatcg 3060
catcgagcga gcacgtactc ggatggaagc cggtcttgtc gatcaggatg atctggacga 3120
agagcatcag gggctcgcgc cagccgaact gttcgccagg ctcaaggcgc gcatgcccga 3180
cggcgaggat ctcgtcgtga cccatggcga tgcctgcttg ccgaatatca tggtggaaaa 3240
tggccgcttt tctggattca tcgactgtgg ccggctgggt gtggcggacc gctatcagga 3300
catagcgttg gctacccgtg atattgctga agagcttggc ggcgaatggg ctgaccgctt 3360
cctcgtgctt tacggtatcg ccgctcccga ttcgcagcgc atcgccttct atcgccttct 3420
tgacgagttc ttctgagcgg gactctgggg ttcgaaatga ccgaccaagc gacgcccaac 3480
ctgccatcac gatggccgca ataaaatatc tttattttca ttacatctgt gtgttggttt 3540
tttgtgtgaa tcgatagcga taaggatccg cgtatggtgc actctcagta caatctgctc 3600
tgatgccgca tagttaagcc agccccgaca cccgccaaca cccgctgacg cgccctgacg 3660
ggcttgtctg ctcccggcat ccgcttacag acaagctgtg accgtctccg ggagctgcat 3720
gtgtcagagg ttttcaccgt catcaccgaa acgcgcgaga cgaaagggcc tcgtgatacg 3780
cctattttta taggttaatg tcatgataat aatggtttct tagacgtcag gtggcacttt 3840
tcggggaaat gtgcgcggaa cccctatttg tttatttttc taaatacatt caaatatgta 3900
tccgctcatg agacaataac cctgataaat gcttcaataa tattgaaaaa ggaagagtat 3960
gagtattcaa catttccgtg tcgcccttat tccctttttt gcggcatttt gccttcctgt 4020
ttttgctcac ccagaaacgc tggtgaaagt aaaagatgct gaagatcagt tgggtgcacg 4080
agtgggttac atcgaactgg atctcaacag cggtaagatc cttgagagtt ttcgccccga 4140
agaacgtttt ccaatgatga gcacttttaa agttctgcta tgtggcgcgg tattatcccg 4200
tattgacgcc gggcaagagc aactcggtcg ccgcatacac tattctcaga atgacttggt 4260
tgagtactca ccagtcacag aaaagcatct tacggatggc atgacagtaa gagaattatg 4320
cagtgctgcc ataaccatga gtgataacac tgcggccaac ttacttctga caacgatcgg 4380
aggaccgaag gagctaaccg cttttttgca caacatgggg gatcatgtaa ctcgccttga 4440
tcgttgggaa ccggagctga atgaagccat accaaacgac gagcgtgaca ccacgatgcc 4500
tgtagcaatg gcaacaacgt tgcgcaaact attaactggc gaactactta ctctagcttc 4560
ccggcaacaa ttaatagact ggatggaggc ggataaagtt gcaggaccac ttctgcgctc 4620
ggcccttccg gctggctggt ttattgctga taaatctgga gccggtgagc gtgggtctcg 4680
cggtatcatt gcagcactgg ggccagatgg taagccctcc cgtatcgtag ttatctacac 4740
gacggggagt caggcaacta tggatgaacg aaatagacag atcgctgaga taggtgcctc 4800
actgattaag cattggtaac tgtcagacca agtttactca tatatacttt agattgattt 4860
aaaacttcat ttttaattta aaaggatcta ggtgaagatc ctttttgata atctcatgac 4920
caaaatccct taacgtgagt tttcgttcca ctgagcgtca gaccccgtag aaaagatcaa 4980
aggatcttct tgagatcctt tttttctgcg cgtaatctgc tgcttgcaaa caaaaaaacc 5040
accgctacca gcggtggttt gtttgccgga tcaagagcta ccaactcttt ttccgaaggt 5100
aactggcttc agcagagcgc agataccaaa tactgttctt ctagtgtagc cgtagttagg 5160
ccaccacttc aagaactctg tagcaccgcc tacatacctc gctctgctaa tcctgttacc 5220
agtggctgct gccagtggcg ataagtcgtg tcttaccggg ttggactcaa gacgatagtt 5280
accggataag gcgcagcggt cgggctgaac ggggggttcg tgcacacagc ccagcttgga 5340
gcgaacgacc tacaccgaac tgagatacct acagcgtgag ctatgagaaa gcgccacgct 5400
tcccgaaggg agaaaggcgg acaggtatcc ggtaagcggc agggtcggaa caggagagcg 5460
cacgagggag cttccagggg gaaacgcctg gtatctttat agtcctgtcg ggtttcgcca 5520
cctctgactt gagcgtcgat ttttgtgatg ctcgtcaggg gggcggagcc tatggaaaaa 5580
cgccagcaac gcggcctttt tacggttcct ggccttttgc tggccttttg ctcacatggc 5640
tcgacagatc t 5651

<210> 154
<211> 5698
<212> DNA
<213> Homo sapiens
<220>
<221> misc_feature
<223> vecteur d'expression appelé GS-V6 contenant la séquence antisens
de GS-N6;
antisens: 1108...1345

<400> 154
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtacg agaatggagg ccacgacatc atcctcttgg 1140
cccctggagg cacgggcggc tccagctgga aggggcagag cccgtgggcg gcagcttccc 1200
cagctgctgc tcccagcact tctactgatt cttgttggct atgaaatgtc tttttaaaaa 1260
actcccaata tagaaatctg gctgcagagg ccagtgtgca gacccagcca ccgcgctgtg 1320
ctgccattcg ccacatctgg tccgtgtcga cccgggcggc cgcttccctt tagtgagggt 1380
taatgcttcg agcagacatg ataagataca ttgatgagtt tggacaaacc acaactagaa 1440
tgcagtgaaa aaaatgcttt atttgtgaaa tttgtgatgc tattgcttta tttgtaacca 1500
ttataagctg caataaacaa gttaacaaca acaattgcat tcattttatg tttcaggttc 1560
agggggagat gtgggaggtt ttttaaagca agtaaaacct ctacaaatgt ggtaaaatcc 1620
gataaggatc gatccgggct ggcgtaatag cgaagaggcc cgcaccgatc gcccttccca 1680
acagttgcgc agcctgaatg gcgaatggac gcgccctgta gcggcgcatt aagcgcggcg 1740
ggtgtggtgg ttacgcgcag cgtgaccgct acacttgcca gcgccctagc gcccgctcct 1800
ttcgctttct tcccttcctt tctcgccacg ttcgccggct ttccccgtca agctctaaat 1860
cgggggctcc ctttagggtt ccgatttagt gctttacggc acctcgaccc caaaaaactt 1920
gattagggtg atggttcacg tagtgggcca tcgccctgat agacggtttt tcgccctttg 1980
acgttggagt ccacgttctt taatagtgga ctcttgttcc aaactggaac aacactcaac 2040
cctatctcgg tctattcttt tgatttataa gggattttgc cgatttcggc ctattggtta 2100
aaaaatgagc tgatttaaca aaaatttaac gcgaatttta acaaaatatt aacgcttaca 2160
atttcctgat gcggtatttt ctccttacgc atctgtgcgg tatttcacac cgcatacgcg 2220
gatctgcgca gcaccatggc ctgaaataac ctctgaaaga ggaacttggt taggtacctt 2280
ctgaggcgga aagaaccagc tgtggaatgt gtgtcagtta gggtgtggaa agtccccagg 2340
ctccccagca ggcagaagta tgcaaagcat gcatctcaat tagtcagcaa ccaggtgtgg 2400
aaagtcccca ggctccccag caggcagaag tatgcaaagc atgcatctca attagtcagc 2460
aaccatagtc ccgcccctaa ctccgcccat cccgccccta actccgccca gttccgccca 2520
ttctccgccc catggctgac taattttttt tatttatgca gaggccgagg ccgcctcggc 2580
ctctgagcta ttccagaagt agtgaggagg cttttttgga ggcctaggct tttgcaaaaa 2640
gcttgattct tctgacacaa cagtctcgaa cttaaggcta gagccaccat gattgaacaa 2700
gatggattgc acgcaggttc tccggccgct tgggtggaga ggctattcgg ctatgactgg 2760
gcacaacaga caatcggctg ctctgatgcc gccgtgttcc ggctgtcagc gcaggggcgc 2820
ccggttcttt ttgtcaagac cgacctgtcc ggtgccctga atgaactgca ggacgaggca 2880
gcgcggctat cgtggctggc cacgacgggc gttccttgcg cagctgtgct cgacgttgtc 2940
actgaagcgg gaagggactg gctgctattg ggcgaagtgc cggggcagga tctcctgtca 3000
tctcaccttg ctcctgccga gaaagtatcc atcatggctg atgcaatgcg gcggctgcat 3060
acgcttgatc cggctacctg cccattcgac caccaagcga aacatcgcat cgagcgagca 3120
cgtactcgga tggaagccgg tcttgtcgat caggatgatc tggacgaaga gcatcagggg 3180
ctcgcgccag ccgaactgtt cgccaggctc aaggcgcgca tgcccgacgg cgaggatctc 3240
gtcgtgaccc atggcgatgc ctgcttgccg aatatcatgg tggaaaatgg ccgcttttct 3300
ggattcatcg actgtggccg gctgggtgtg gcggaccgct atcaggacat agcgttggct 3360
acccgtgata ttgctgaaga gcttggcggc gaatgggctg accgcttcct cgtgctttac 3420
ggtatcgccg ctcccgattc gcagcgcatc gccttctatc gccttcttga cgagttcttc 3480
tgagcgggac tctggggttc gaaatgaccg accaagcgac gcccaacctg ccatcacgat 3540
ggccgcaata aaatatcttt attttcatta catctgtgtg ttggtttttt gtgtgaatcg 3600

atagcgataa ggatccgcgt atggtgcact ctcagtacaa tctgctctga tgccgcatag 3660
ttaagccagc cccgacaccc gccaacaccc gctgacgcgc cctgacgggc ttgtctgctc 3720
ccggcatccg cttacagaca agctgtgacc gtctccggga gctgcatgtg tcagaggttt 3780
tcaccgtcat caccgaaacg cgcgagacga aagggcctcg tgatacgcct atttttatag 3840
gttaatgtca tgataataat ggtttcttag acgtcaggtg gcacttttcg gggaaatgtg 3900
cgcggaaccc ctatttgttt atttttctaa atacattcaa atatgtatcc gctcatgaga 3960
caataaccct gataaatgct tcaataatat tgaaaaagga agagtatgag tattcaacat 4020
ttccgtgtcg cccttattcc cttttttgcg gcattttgcc ttcctgtttt tgctcaccca 4080
gaaacgctgg tgaaagtaaa agatgctgaa gatcagttgg gtgcacgagt gggttacatc 4140
gaactggatc tcaacagcgg taagatcctt gagagttttc gccccgaaga acgttttcca 4200
atgatgagca cttttaaagt tctgctatgt ggcgcggtat tatcccgtat tgacgccggg 4260
caagagcaac tcggtcgccg catacactat tctcagaatg acttggttga gtactcacca 4320
gtcacagaaa agcatcttac ggatggcatg acagtaagag aattatgcag tgctgccata 4380
accatgagtg ataacactgc ggccaactta cttctgacaa cgatcggagg accgaaggag 4440
ctaaccgctt ttttgcacaa catgggggat catgtaactc gccttgatcg ttgggaaccg 4500
gagctgaatg aagccatacc aaacgacgag cgtgacacca cgatgcctgt agcaatggca 4560
acaacgttgc gcaaactatt aactggcgaa ctacttactc tagcttcccg gcaacaatta 4620
atagactgga tggaggcgga taaagttgca ggaccacttc tgcgctcggc ccttccggct 4680
ggctggttta ttgctgataa atctggagcc ggtgagcgtg ggtctcgcgg tatcattgca 4740
gcactggggc cagatggtaa gccctcccgt atcgtagtta tctacacgac ggggagtcag 4800
gcaactatgg atgaacgaaa tagacagatc gctgagatag gtgcctcact gattaagcat 4860
tggtaactgt cagaccaagt ttactcatat atactttaga ttgatttaaa acttcatttt 4920
taatttaaaa ggatctaggt gaagatcctt tttgataatc tcatgaccaa aatcccttaa 4980
cgtgagtttt cgttccactg agcgtcagac cccgtagaaa agatcaaagg atcttcttga 5040
gatccttttt ttctgcgcgt aatctgctgc ttgcaaacaa aaaaaccacc gctaccagcg 5100
gtggtttgtt tgccggatca agagctacca actctttttc cgaaggtaac tggcttcagc 5160
agagcgcaga taccaaatac tgttcttcta gtgtagccgt agttaggcca ccacttcaag 5220
aactctgtag caccgcctac atacctcgct ctgctaatcc tgttaccagt ggctgctgcc 5280
agtggcgata agtcgtgtct taccgggttg gactcaagac gatagttacc ggataaggcg 5340
cagcggtcgg gctgaacggg gggttcgtgc acacagccca gcttggagcg aacgacctac 5400
accgaactga gatacctaca gcgtgagcta tgagaaagcg ccacgcttcc cgaagggaga 5460
aaggcggaca ggtatccggt aagcggcagg gtcggaacag gagagcgcac gagggagctt 5520
ccagggggaa acgcctggta tctttatagt cctgtcgggt ttcgccacct ctgacttgag 5580
cgtcgatttt tgtgatgctc gtcagggggg cggagcctat ggaaaaacgc cagcaacgcg 5640
gcctttttac ggttcctggc cttttgctgg ccttttgctc acatggctcg acagatct 5698

<210> 155
<211> 5665
<212> DNA
<213> Homo sapiens
<220>
<221> misc_feature
<223> vecteur d'expression appelé GS-V7 contenant la séquence antisens
de GS-N7 ;
antisens: 1108...1312

<400> 155
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgttgc gtgcttgctt tctcaggcct cctggtcaat 1140
agcacaggtc tgctggaaat cacccacatg gtttttcatt gcctgtgcag tcaggtgatg 1200
cctctatgtt tttggaggtc cacctctgtt gtttcttaac cttcccagcc ttcccacagt 1260
tgtcccagtg atttggaaga aggtctcttc atttctggcc actttcgacg ctgtcgaccc 1320
gggcggccgc ttccctttag tgagggttaa tgcttcgagc agacatgata agatacattg 1380
atgagtttgg acaaaccaca actagaatgc agtgaaaaaa atgctttatt tgtgaaattt 1440
gtgatgctat tgctttattt gtaaccatta taagctgcaa taaacaagtt aacaacaaca 1500

attgcattca ttttatgttt caggttcagg gggagatgtg ggaggttttt taaagcaagt 1560
aaaacctcta caaatgtggt aaaatccgat aaggatcgat ccgggctggc gtaatagcga 1620
agaggcccgc accgatcgcc cttcccaaca gttgcgcagc ctgaatggcg aatggacgcg 1680
ccctgtagcg gcgcattaag cgcggcgggt gtggtggtta cgcgcagcgt gaccgctaca 1740
cttgccagcg ccctagcgcc cgctcctttc gctttcttcc cttcctttct cgccacgttc 1800
gccggctttc cccgtcaagc tctaaatcgg gggctccctt tagggttccg atttagtgct 1860
ttacggcacc tcgaccccaa aaaacttgat tagggtgatg gttcacgtag tgggccatcg 1920
ccctgataga cggtttttcg ccctttgacg ttggagtcca cgttctttaa tagtggactc 1980
ttgttccaaa ctggaacaac actcaaccct atctcggtct attcttttga tttataaggg 2040
attttgccga tttcggccta ttggttaaaa aatgagctga tttaacaaaa atttaacgcg 2100
aattttaaca aaatattaac gcttacaatt tcctgatgcg gtattttctc cttacgcatc 2160
tgtgcggtat ttcacaccgc atacgcggat ctgcgcagca ccatggcctg aaataacctc 2220
tgaaagagga acttggttag gtaccttctg aggcggaaag aaccagctgt ggaatgtgtg 2280
tcagttaggg tgtggaaagt ccccaggctc cccagcaggc agaagtatgc aaagcatgca 2340
tctcaattag tcagcaacca ggtgtggaaa gtccccaggc tccccagcag gcagaagtat 2400
gcaaagcatg catctcaatt agtcagcaac catagtcccg cccctaactc cgcccatccc 2460
gcccctaact ccgcccagtt ccgcccattc tccgccccat ggctgactaa ttttttttat 2520
ttatgcagag gccgaggccg cctcggcctc tgagctattc cagaagtagt gaggaggctt 2580
ttttggaggc ctaggctttt gcaaaaagct tgattcttct gacacaacag tctcgaactt 2640
aaggctagag ccaccatgat tgaacaagat ggattgcacg caggttctcc ggccgcttgg 2700
gtggagaggc tattcggcta tgactgggca caacagacaa tcggctgctc tgatgccgcc 2760
gtgttccggc tgtcagcgca ggggcgcccg gttctttttg tcaagaccga cctgtccggt 2820
gccctgaatg aactgcagga cgaggcagcg cggctatcgt ggctggccac gacgggcgtt 2880
ccttgcgcag ctgtgctcga cgttgtcact gaagcgggaa gggactggct gctattgggc 2940
gaagtgccgg ggcaggatct cctgtcatct caccttgctc ctgccgagaa agtatccatc 3000
atggctgatg caatgcggcg gctgcatacg cttgatccgg ctacctgccc attcgaccac 3060
caagcgaaac atcgcatcga gcgagcacgt actcggatgg aagccggtct tgtcgatcag 3120
gatgatctgg acgaagagca tcaggggctc gcgccagccg aactgttcgc caggctcaag 3180
gcgcgcatgc ccgacggcga ggatctcgtc gtgacccatg gcgatgcctg cttgccgaat 3240
atcatggtgg aaaatggccg cttttctgga ttcatcgact gtggccggct gggtgtggcg 3300
gaccgctatc aggacatagc gttggctacc cgtgatattg ctgaagagct tggcggcgaa 3360
tgggctgacc gcttcctcgt gctttacggt atcgccgctc ccgattcgca gcgcatcgcc 3420
ttctatcgcc ttcttgacga gttcttctga gcgggactct ggggttcgaa atgaccgacc 3480
aagcgacgcc caacctgcca tcacgatggc cgcaataaaa tatctttatt ttcattacat 3540
ctgtgtgttg gttttttgtg tgaatcgata gcgataagga tccgcgtatg gtgcactctc 3600
agtacaatct gctctgatgc cgcatagtta agccagcccc gacacccgcc aacacccgct 3660
gacgcgccct gacgggcttg tctgctcccg gcatccgctt acagacaagc tgtgaccgtc 3720
tccgggagct gcatgtgtca gaggttttca ccgtcatcac cgaaacgcgc gagacgaaag 3780
ggcctcgtga tacgcctatt tttataggtt aatgtcatga taataatggt ttcttagacg 3840
tcaggtggca cttttcgggg aaatgtgcgc ggaaccccta tttgtttatt tttctaaata 3900
cattcaaata tgtatccgct catgagacaa taaccctgat aaatgcttca ataatattga 3960
aaaaggaaga gtatgagtat tcaacatttc cgtgtcgccc ttattccctt ttttgcggca 4020
ttttgccttc ctgtttttgc tcacccagaa acgctggtga aagtaaaaga tgctgaagat 4080
cagttgggtg cacgagtggg ttacatcgaa ctggatctca acagcggtaa gatccttgag 4140
agttttcgcc ccgaagaacg ttttccaatg atgagcactt ttaaagttct gctatgtggc 4200
gcggtattat cccgtattga cgccgggcaa gagcaactcg gtcgccgcat acactattct 4260
cagaatgact tggttgagta ctcaccagtc acagaaaagc atcttacgga tggcatgaca 4320
gtaagagaat tatgcagtgc tgccataacc atgagtgata acactgcggc caacttactt 4380
ctgacaacga tcggaggacc gaaggagcta accgcttttt tgcacaacat gggggatcat 4440
gtaactcgcc ttgatcgttg ggaaccggag ctgaatgaag ccataccaaa cgacgagcgt 4500
gacaccacga tgcctgtagc aatggcaaca acgttgcgca aactattaac tggcgaacta 4560
cttactctag cttcccggca acaattaata gactggatgg aggcggataa agttgcagga 4620
ccacttctgc gctcggccct tccggctggc tggtttattg ctgataaatc tggagccggt 4680
gagcgtgggt ctcgcggtat cattgcagca ctggggccag atggtaagcc ctcccgtatc 4740
gtagttatct acacgacggg gagtcaggca actatggatg aacgaaatag acagatcgct 4800
gagataggtg cctcactgat taagcattgg taactgtcag accaagttta ctcatatata 4860
ctttagattg atttaaaact tcatttttaa tttaaaagga tctaggtgaa gatccttttt 4920
gataatctca tgaccaaaat cccttaacgt gagttttcgt tccactgagc gtcagacccc 4980
gtagaaaaga tcaaaggatc ttcttgagat cctttttttc tgcgcgtaat ctgctgcttg 5040
caaacaaaaa aaccaccgct accagcggtg gtttgtttgc cggatcaaga gctaccaact 5100
ctttttccga aggtaactgg cttcagcaga gcgcagatac caaatactgt tcttctagtg 5160
tagccgtagt taggccacca cttcaagaac tctgtagcac cgcctacata cctcgctctg 5220
ctaatcctgt taccagtggc tgctgccagt ggcgataagt cgtgtcttac cgggttggac 5280
tcaagacgat agttaccgga taaggcgcag cggtcgggct gaacgggggg ttcgtgcaca 5340
cagcccagct tggagcgaac gacctacacc gaactgagat acctacagcg tgagctatga 5400
gaaagcgcca cgcttcccga agggagaaag gcggacaggt atccggtaag cggcagggtc 5460
ggaacaggag agcgcacgag ggagcttcca gggggaaacg cctggtatct ttatagtcct 5520
gtcgggtttc gccacctctg acttgagcgt cgatttttgt gatgctcgtc aggggggcgg 5580
agcctatgga aaaacgccag caacgcggcc tttttacggt tcctggcctt ttgctggcct 5640
tttgctcaca tggctcgaca gatct 5665

<210> 156

<211> 5646
<212> DNA
<213> Homo sapiens
<220>
<221> misc_feature
<223> vecteur d'expression appelé GS-V8 contenant la séquence antisens
      de GS-N8 ; antisens: 1108...1293

<400> 156
tcaatattgg ccattagcca tattattcat tggttatata gcataaatca atattggcta      60
ttggccattg catacgttgt atctatatca taatatgtac atttatattg gctcatgtcc     120
aatatgaccg ccatgttggc attgattatt gactagttat taatagtaat caattacggg     180
gtcattagtt catagcccat atatggagtt ccgcgttaca taacttacgg taaatggccc     240
gcctggctga ccgcccaacg acccccgccc attgacgtca ataatgacgt atgttcccat     300
agtaacgcca atagggactt tccattgacg tcaatgggtg gagtatttac ggtaaactgc     360
ccacttggca gtacatcaag tgtatcatat gccaagtccg ccccctattg acgtcaatga     420
cggtaaatgg cccgcctggc attatgccca gtacatgacc ttacgggact ttcctacttg     480
gcagtacatc tacgtattag tcatcgctat taccatggtg atgcggtttt ggcagtacac     540
caatgggcgt ggatagcggt ttgactcacg gggatttcca agtctccacc ccattgacgt     600
caatgggagt ttgttttggc accaaaatca acgggacttt ccaaaatgtc gtaacaactg     660
cgatcgcccg ccccgttgac gcaaatgggc ggtaggcgtg tacggtggga ggtctatata     720
agcagagctc gtttagtgaa ccgtcagatc actagaagct ttattgcggt agtttatcac     780
agttaaattg ctaacgcagt cagtgcttct gacacaacag tctcgaactt aagctgcagt     840
gactctctta aggtagcctt gcagaagttg gtcgtgaggc actgggcagg taagtatcaa     900
ggttacaaga caggtttaag gagaccaata gaaactgggc ttgtcgagac agagaagact     960
cttgcgtttc tgataggcac ctattggtct tactgacatc cactttgcct ttctctccac    1020
aggtgtccac tcccagttca attacagctc ttaaggctag agtacttaat acgactcact    1080
ataggctagc ctcgagaatt cacgcgtccg tccattctta tcctccatct gaaggtggta    1140
ctgaatagaa tcggttgaca atgtctttgg agttccttct ccccatttca gcttaaggtt    1200
ctggtggcta aaggcaacac attccagacg aggtggatca ggagggagag gcttagtttt    1260
taattttatc gtatggctga aaggaccagc tccgtcgacc cgggcggccg cttcccttta    1320
gtgagggtta atgcttcgag cagacatgat aagatacatt gatgagtttg gacaaaccac    1380
aactagaatg cagtgaaaaa aatgctttat ttgtgaaatt tgtgatgcta ttgctttatt    1440
tgtaaccatt ataagctgca ataaacaagt taacaacaac aattgcattc attttatgtt    1500
tcaggttcag ggggagatgt gggaggtttt ttaaagcaag taaaacctct acaaatgtgg    1560
taaaatccga taaggatcga tccgggctgg cgtaatagcg aagaggcccg caccgatcgc    1620
ccttcccaac agttgcgcag cctgaatggc gaatggacgc gccctgtagc ggcgcattaa    1680
gcgcggcggg tgtggtggtt acgcgcagcg tgaccgctac acttgccagc gccctagcgc    1740
ccgctccttt cgctttcttc ccttcctttc tcgccacgtt cgccggcttt ccccgtcaag    1800
ctctaaatcg ggggctccct ttagggttcc gatttagtgc tttacggcac ctcgacccca    1860
aaaaacttga ttagggtgat ggttcacgta gtgggccatc gccctgatag acggtttttc    1920
gccctttgac gttggagtcc acgttcttta atagtggact cttgttccaa actggaacaa    1980
cactcaaccc tatctcggtc tattcttttg atttataagg gattttgccg atttcggcct    2040
attggttaaa aaatgagctg atttaacaaa aatttaacgc gaattttaac aaaatattaa    2100
cgcttacaat ttcctgatgc ggtattttct ccttacgcat ctgtgcggta tttcacaccg    2160
catacgcgga tctgcgcagc accatggcct gaaataacct ctgaaagagg aacttggtta    2220
ggtaccttct gaggcggaaa gaaccagctg tggaatgtgt gtcagttagg gtgtggaaag    2280
tccccaggct ccccagcagg cagaagtatg caaagcatgc atctcaatta gtcagcaacc    2340
aggtgtggaa agtccccagg ctccccagca ggcagaagta tgcaaagcat gcatctcaat    2400
tagtcagcaa ccatagtccc gcccctaact ccgcccatcc cgcccctaac tccgcccagt    2460
tccgcccatt ctccgcccca tggctgacta atttttttta tttatgcaga ggccgaggcc    2520
gcctcggcct ctgagctatt ccagaagtag tgaggaggct tttttggagg cctaggcttt    2580
tgcaaaaagc ttgattcttc tgacacaaca gtctcgaact taaggctaga gccaccatga    2640
ttgaacaaga tggattgcac gcaggttctc cggccgcttg ggtggagagg ctattcggct    2700
atgactgggc acaacagaca atcggctgct ctgatgccgc cgtgttccgg ctgtcagcgc    2760
aggggcgccc ggttcttttt gtcaagaccg acctgtccgg tgccctgaat gaactgcagg    2820
acgaggcagc gcggctatcg tggctggcca cgacgggcgt tccttgcgca gctgtgctcg    2880
acgttgtcac tgaagcggga agggactggc tgctattggg cgaagtgccg gggcaggatc    2940
tcctgtcatc tcaccttgct cctgccgaga aagtatccat catggctgat gcaatgcggc    3000
ggctgcatac gcttgatccg gctacctgcc cattcgacca ccaagcgaaa catcgcatcg    3060
agcgagcacg tactcggatg gaagccggtc ttgtcgatca ggatgatctg gacgaagagc    3120
atcaggggct cgcgccagcc gaactgttcg ccaggctcaa ggcgcgcatg cccgacggcg    3180
aggatctcgt cgtgacccat ggcgatgcct gcttgccgaa tatcatggtg gaaaatggcc    3240
gcttttctgg attcatcgac tgtggccggc tgggtgtggc ggaccgctat caggacatag    3300
cgttggctac ccgtgatatt gctgaagagc ttggcggcga atgggctgac cgcttcctcg    3360
tgctttacgg tatcgccgct cccgattcgc agcgcatcgc cttctatcgc cttcttgacg    3420
agttcttctg agcgggactc tggggttcga aatgaccgac caagcgacgc ccaacctgcc    3480
atcacgatgg ccgcaataaa atatctttat tttcattaca tctgtgtgtt ggttttttgt    3540
gtgaatcgat agcgataagg atccgcgtat ggtgcactct cagtacaatc tgctctgatg    3600
ccgcatagtt aagccagccc cgacacccgc caacacccgc tgacgcgccc tgacgggctt    3660
gtctgctccc ggcatccgct tacagacaag ctgtgaccgt ctccgggagc tgcatgtgtc    3720
agaggttttc accgtcatca ccgaaacgcg cgagacgaaa gggcctcgtg atacgcctat    3780
ttttataggt taatgtcatg ataataatgg tttcttagac gtcaggtggc acttttcggg    3840

gaaatgtgcg cggaacccct atttgtttat ttttctaaat acattcaaat atgtatccgc 3900
tcatgagaca ataaccctga taaatgcttc aataatattg aaaaaggaag agtatgagta 3960
ttcaacattt ccgtgtcgcc cttattccct tttttgcggc attttgcctt cctgtttttg 4020
ctcacccaga aacgctggtg aaagtaaaag atgctgaaga tcagttgggt gcacgagtgg 4080
gttacatcga actggatctc aacagcggta agatccttga gagttttcgc cccgaagaac 4140
gttttccaat gatgagcact tttaaagttc tgctatgtgg cgcggtatta tcccgtattg 4200
acgccgggca agagcaactc ggtcgccgca tacactattc tcagaatgac ttggttgagt 4260
actcaccagt cacagaaaag catcttacgg atggcatgac agtaagagaa ttatgcagtg 4320
ctgccataac catgagtgat aacactgcgg ccaacttact tctgacaacg atcggaggac 4380
cgaaggagct aaccgctttt ttgcacaaca tgggggatca tgtaactcgc cttgatcgtt 4440
gggaaccgga gctgaatgaa gccataccaa acgacgagcg tgacaccacg atgcctgtag 4500
caatggcaac aacgttgcgc aaactattaa ctggcgaact acttactcta gcttcccggc 4560
aacaattaat agactggatg gaggcggata aagttgcagg accacttctg cgctcggccc 4620
ttccggctgg ctggtttatt gctgataaat ctggagccgg tgagcgtggg tctcgcggta 4680
tcattgcagc actggggcca gatggtaagc cctcccgtat cgtagttatc tacacgacgg 4740
ggagtcaggc aactatggat gaacgaaata gacagatcgc tgagataggt gcctcactga 4800
ttaagcattg gtaactgtca gaccaagttt actcatatat actttagatt gatttaaaac 4860
ttcattttta atttaaaagg atctaggtga agatcctttt tgataatctc atgaccaaaa 4920
tcccttaacg tgagttttcg ttccactgag cgtcagaccc cgtagaaaag atcaaaggat 4980
cttcttgaga tccttttttt ctgcgcgtaa tctgctgctt gcaaacaaaa aaaccaccgc 5040
taccagcggt ggtttgtttg ccggatcaag agctaccaac tctttttccg aaggtaactg 5100
gcttcagcag agcgcagata ccaaatactg ttcttctagt gtagccgtag ttaggccacc 5160
acttcaagaa ctctgtagca ccgcctacat acctcgctct gctaatcctg ttaccagtgg 5220
ctgctgccag tggcgataag tcgtgtctta ccgggttgga ctcaagacga tagttaccgg 5280
ataaggcgca gcggtcgggc tgaacggggg gttcgtgcac acagcccagc ttggagcgaa 5340
cgacctacac cgaactgaga tacctacagc gtgagctatg agaaagcgcc acgcttcccg 5400
aagggagaaa ggcggacagg tatccggtaa gcggcagggt cggaacagga gagcgcacga 5460
gggagcttcc agggggaaac gcctggtatc tttatagtcc tgtcgggttt cgccacctct 5520
gacttgagcg tcgatttttg tgatgctcgt caggggggcg gagcctatgg aaaaacgcca 5580
gcaacgcggc ctttttacgg ttcctggcct tttgctggcc ttttgctcac atggctcgac 5640
agatct 5646

<210> 157
<211> 5683
<212> DNA
<213> Homo sapiens
<220>
<221> misc_feature
<223> vecteur d'expression appelé GS-V9, contenant la séquence antisens
 de GS-N9
 antisens: 1108..1330

<400> 157
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtgga ggggagagaa cccaacacat gaggtctgca 1140
cacacagctg tcctggttgc cctcggtgca gctccgagct ccagttacaa ggaattccaa 1200
gttctcagga tcttgaagac tctggaggcc agtaatccct ggatcacact gcttctacca 1260
gctcagaaga gaagtcctgc ctaaggtcat gaaataaacc tgactgctgc caccagaccg 1320
aacagaggca gtcgacccgg gcggccgctt ccctttagtg agggttaatg cttcgagcag 1380
acatgataag atacattgat gagtttggac aaaccacaac tagaatgcag tgaaaaaaat 1440
gctttatttg tgaaatttgt gatgctattg ctttatttgt aaccattata agctgcaata 1500
aacaagttaa caacaacaat tgcattcatt ttatgtttca ggttcagggg gagatgtggg 1560
aggtttttta aagcaagtaa aacctctaca aatgtggtaa aatccgataa ggatcgatcc 1620
gggctggcgt aatagcgaag aggcccgcac cgatcgccct tcccaacagt tgcgcagcct 1680
gaatggcgaa tggacgcgcc ctgtagcggc gcattaagcg cggcgggtgt ggtggttacg 1740

cgcagcgtga ccgctacact tgccagcgcc ctagcgcccg ctcctttcgc tttcttccct 1800
tcctttctcg ccacgttcgc cggctttccc cgtcaagctc taaatcgggg gctcccttta 1860
gggttccgat ttagtgcttt acggcacctc gaccccaaaa aacttgatta gggtgatggt 1920
tcacgtagtg ggccatcgcc ctgatagacg gtttttcgcc ctttgacgtt ggagtccacg 1980
ttctttaata gtggactctt gttccaaact ggaacaacac tcaaccctat ctcggtctat 2040
tcttttgatt tataagggat tttgccgatt tcggcctatt ggttaaaaaa tgagctgatt 2100
taacaaaaat ttaacgcgaa ttttaacaaa atattaacgc ttacaatttc ctgatgcggt 2160
attttctcct tacgcatctg tgcggtattt cacaccgcat acgcggatct gcgcagcacc 2220
atggcctgaa ataacctctg aaagaggaac ttggttaggt accttctgag gcggaaagaa 2280
ccagctgtgg aatgtgtgtc agttagggtg tggaaagtcc ccaggctccc cagcaggcag 2340
aagtatgcaa agcatgcatc tcaattagtc agcaaccagg tgtggaaagt ccccaggctc 2400
cccagcaggc agaagtatgc aaagcatgca tctcaattag tcagcaacca tagtcccgcc 2460
cctaactccg cccatcccgc ccctaactcc gcccagttcc gcccattctc cgccccatgg 2520
ctgactaatt ttttttattt atgcagaggc cgaggccgcc tcggcctctg agctattcca 2580
gaagtagtga ggaggctttt ttggaggcct aggcttttgc aaaaagcttg attcttctga 2640
cacaacagtc tcgaacttaa ggctagagcc accatgattg aacaagatgg attgcacgca 2700
ggttctccgg ccgcttgggt ggagaggcta ttcggctatg actgggcaca acagacaatc 2760
ggctgctctg atgccgccgt gttccggctg tcagcgcagg ggcgcccggt tctttttgtc 2820
aagaccgacc tgtccggtgc cctgaatgaa ctgcaggacg aggcagcgcg gctatcgtgg 2880
ctggccacga cgggcgttcc ttgcgcagct gtgctcgacg ttgtcactga agcgggaagg 2940
gactggctgc tattgggcga agtgccgggg caggatctcc tgtcatctca ccttgctcct 3000
gccgagaaag tatccatcat ggctgatgca atgcggcggc tgcatacgct tgatccggct 3060
acctgcccat tcgaccacca agcgaaacat cgcatcgagc gagcacgtac tcggatggaa 3120
gccggtcttg tcgatcagga tgatctggac gaagagcatc aggggctcgc gccagccgaa 3180
ctgttcgcca ggctcaaggc gcgcatgccc gacggcgagg atctcgtcgt gacccatggc 3240
gatgcctgct tgccgaatat catggtggaa aatggccgct tttctggatt catcgactgt 3300
ggccggctgg gtgtggcgga ccgctatcag gacatagcgt tggctacccg tgatattgct 3360
gaagagcttg gcggcgaatg ggctgaccgc ttcctcgtgc tttacggtat cgccgctccc 3420
gattcgcagc gcatcgcctt ctatcgcctt cttgacgagt tcttctgagc gggactctgg 3480
ggttcgaaat gaccgaccaa gcgacgccca acctgccatc acgatggccg caataaaata 3540
tctttatttt cattacatct gtgtgttggt tttttgtgtg aatcgatagc gataaggatc 3600
cgcgtatggt gcactctcag tacaatctgc tctgatgccg catagttaag ccagccccga 3660
cacccgccaa cacccgctga cgcgccctga cgggcttgtc tgctcccggc atccgcttac 3720
agacaagctg tgaccgtctc cgggagctgc atgtgtcaga ggttttcacc gtcatcaccg 3780
aaacgcgcga gacgaaaggg cctcgtgata cgcctatttt tataggttaa tgtcatgata 3840
ataatggttt cttagacgtc aggtggcact tttcggggaa atgtgcgcgg aacccctatt 3900
tgtttatttt tctaaataca ttcaaatatg tatccgctca tgagacaata accctgataa 3960
atgcttcaat aatattgaaa aaggaagagt atgagtattc aacatttccg tgtcgccctt 4020
attccctttt ttgcggcatt ttgccttcct gtttttgctc acccagaaac gctggtgaaa 4080
gtaaaagatg ctgaagatca gttgggtgca cgagtgggtt acatcgaact ggatctcaac 4140
agcggtaaga tccttgagag ttttcgcccc gaagaacgtt ttccaatgat gagcactttt 4200
aaagttctgc tatgtggcgc ggtattatcc cgtattgacg ccgggcaaga gcaactcggt 4260
cgccgcatac actattctca gaatgacttg gttgagtact caccagtcac agaaaagcat 4320
cttacggatg gcatgacagt aagagaatta tgcagtgctg ccataaccat gagtgataac 4380
actgcggcca acttacttct gacaacgatc ggaggaccga aggagctaac cgcttttttg 4440
cacaacatgg gggatcatgt aactcgcctt gatcgttggg aaccggagct gaatgaagcc 4500
ataccaaacg acgagcgtga caccacgatg cctgtagcaa tggcaacaac gttgcgcaaa 4560
ctattaactg gcgaactact tactctagct tcccggcaac aattaataga ctggatggag 4620
gcggataaag ttgcaggacc acttctgcgc tcggcccttc cggctggctg gtttattgct 4680
gataaatctg gagccggtga gcgtgggtct cgcggtatca ttgcagcact ggggccagat 4740
ggtaagccct cccgtatcgt agttatctac acgacgggga gtcaggcaac tatggatgaa 4800
cgaaatagac agatcgctga gataggtgcc tcactgatta agcattggta actgtcagac 4860
caagtttact catatatact ttagattgat ttaaaacttc atttttaatt taaaaggatc 4920
taggtgaaga tcctttttga taatctcatg accaaaatcc cttaacgtga gttttcgttc 4980
cactgagcgt cagaccccgt agaaaagatc aaaggatctt cttgagatcc tttttttctg 5040
cgcgtaatct gctgcttgca aacaaaaaaa ccaccgctac cagcggtggt ttgtttgccg 5100
gatcaagagc taccaactct ttttccgaag gtaactggct tcagcagagc gcagatacca 5160
aatactgttc ttctagtgta gccgtagtta ggccaccact tcaagaactc tgtagcaccg 5220
cctacatacc tcgctctgct aatcctgtta ccagtggctg ctgccagtgg cgataagtcg 5280
tgtcttaccg ggttggactc aagacgatag ttaccggata aggcgcagcg gtcgggctga 5340
acggggggtt cgtgcacaca gcccagcttg gagcgaacga cctacaccga actgagatac 5400
ctacagcgtg agctatgaga aagcgccacg cttcccgaag ggagaaaggc ggacaggtat 5460
ccggtaagcg gcagggtcgg aacaggagag cgcacgaggg agcttccagg gggaaacgcc 5520
tggtatcttt atagtcctgt cgggtttcgc cacctctgac ttgagcgtcg atttttgtga 5580
tgctcgtcag gggggcggag cctatggaaa aacgccagca acgcggcctt tttacggttc 5640
ctggcctttt gctggccttt tgctcacatg gctcgacaga tct 5683

<210> 158
<211> 5707
<212> DNA
<213> Homo sapiens
<220>

<221> misc_feature
<223> vecteur d'expression appelé GS-V10 contenant la séquence antisens
       de GS-N10
      antisens: 1108...1354

<220>
<221> misc_feature
<223> vecteur d'expression appelé GS-V10 contenant la séquence antisens
       de GS-N54 ;
      antisens: 1108...1354

<400> 158
tcaatattgg ccattagcca tattattcat tggttatata gcataaatca atattggcta      60
ttggccattg catacgttgt atctatatca taatatgtac atttatattg gctcatgtcc     120
aatatgaccg ccatgttggc attgattatt gactagttat taatagtaat caattacggg     180
gtcattagtt catagcccat atatggagtt ccgcgttaca taacttacgg taaatggccc     240
gcctggctga ccgcccaacg acccccgccc attgacgtca ataatgacgt atgttcccat     300
agtaacgcca atagggactt tccattgacg tcaatgggtg gagtatttac ggtaaactgc     360
ccacttggca gtacatcaag tgtatcatat gccaagtccg ccccctattg acgtcaatga     420
cggtaaatgg cccgcctggc attatgccca gtacatgacc ttacgggact ttcctacttg     480
gcagtacatc tacgtattag tcatcgctat taccatggtg atgcggtttt ggcagtacac     540
caatgggcgt ggatagcggt ttgactcacg gggatttcca agtctccacc ccattgacgt     600
caatgggagt ttgttttggc accaaaatca acgggacttt ccaaaatgtc gtaacaactg     660
cgatcgcccg ccccgttgac gcaaatgggc ggtaggcgtg tacggtggga ggtctatata     720
agcagagctc gtttagtgaa ccgtcagatc actagaagct ttattgcggt agtttatcac     780
agttaaattg ctaacgcagt cagtgcttct gacacaacag tctcgaactt aagctgcagt     840
gactctctta aggtagcctt gcagaagttg gtcgtgaggc actgggcagg taagtatcaa     900
ggttacaaga caggtttaag gagaccaata gaaactgggc ttgtcgagac agagaagact     960
cttgcgtttc tgataggcac ctattggtct tactgacatc cactttgcct ttctctccac    1020
aggtgtccac tcccagttca attacagctc ttaaggctag agtacttaat acgactcact    1080
ataggctagc ctcgagaatt cacgcgtgat ggagctgtgc ctgtggaggc tgttgtgagg    1140
cagtaggctc atctgcggag actgccgtga ggtagggtat gggcctaaat aggccattgt    1200
gagtcatgag cttggtctgt agaggctgac tggagaaagt tctgggcctg gagaggctgc    1260
cgggtggtag gagcataatg taaaatcagt gggcgtgtta agcttgtttt cctgcaactg    1320
gatggtccca cgttttgctg tcacccctct ggaagtcgac ccgggcggcc gcttcccttt    1380
agtgagggtt aatgcttcga gcagacatga taagatacat tgatgagttt ggacaaacca    1440
caactagaat gcagtgaaaa aaatgcttta tttgtgaaat ttgtgatgct attgctttat    1500
ttgtaaccat tataagctgc aataaacaag ttaacaacaa caattgcatt cattttatgt    1560
ttcaggttca gggggagatg tgggaggttt tttaaagcaa gtaaaacctc tacaaatgtg    1620
gtaaaatccg ataaggatcg atccgggctg gcgtaatagc gaagaggccc gcaccgatcg    1680
cccttcccaa cagttgcgca gcctgaatgg cgaatggacg cgccctgtag cggcgcatta    1740
agcgcggcgg gtgtggtggt tacgcgcagc gtgaccgcta cacttgccag cgccctagcg    1800
cccgctcctt tcgctttctt cccttccttt ctcgccacgt tcgccggctt tccccgtcaa    1860
gctctaaatc gggggctccc tttagggttc cgatttagtg ctttacggca cctcgacccc    1920
aaaaaacttg attagggtga tggttcacgt agtgggccat cgccctgata gacggttttt    1980
cgccctttga cgttggagtc cacgttcttt aatagtggac tcttgttcca aactggaaca    2040
acactcaacc ctatctcggt ctattctttt gatttataag ggattttgcc gatttcggcc    2100
tattggttaa aaaatgagct gatttaacaa aaatttaacg cgaattttaa caaaatatta    2160
acgcttacaa tttcctgatg cggtattttc tccttacgca tctgtgcggt atttcacacc    2220
gcatacgcgg atctgcgcag caccatggcc tgaaataacc tctgaaagag gaacttggtt    2280
aggtaccttc tgaggcggaa agaaccagct gtggaatgtg tgtcagttag ggtgtggaaa    2340
gtccccaggc tccccagcag gcagaagtat gcaaagcatg catctcaatt agtcagcaac    2400
caggtgtgga aagtccccag gctccccagc aggcagaagt atgcaaagca tgcatctcaa    2460
ttagtcagca accatagtcc cgcccctaac tccgcccatc ccgcccctaa ctccgcccag    2520
ttccgcccat tctccgcccc atggctgact aatttttttt atttatgcag aggccgaggc    2580
cgcctcggcc tctgagctat tccagaagta gtgaggaggc ttttttggag gcctaggctt    2640
ttgcaaaaag cttgattctt ctgacacaac agtctcgaac ttaaggctag agccaccatg    2700
attgaacaag atggattgca cgcaggttct ccggccgctt gggtggagag gctattcggc    2760
tatgactggg cacaacagac aatcggctgc tctgatgccg ccgtgttccg gctgtcagcg    2820
caggggcgcc cggttctttt tgtcaagacc gacctgtccg gtgccctgaa tgaactgcag    2880
gacgaggcag cgcggctatc gtggctggcc acgacgggcg ttccttgcgc agctgtgctc    2940
gacgttgtca ctgaagcggg aagggactgg ctgctattgg gcgaagtgcc ggggcaggat    3000
ctcctgtcat ctcaccttgc tcctgccgag aaagtatcca tcatggctga tgcaatgcgg    3060
cggctgcata cgcttgatcc ggctacctgc ccattcgacc accaagcgaa acatcgcatc    3120
gagcgagcac gtactcggat ggaagccggt cttgtcgatc aggatgatct ggacgaagag    3180
catcaggggc tcgcgccagc cgaactgttc gccaggctca aggcgcgcat gcccgacggc    3240
gaggatctcg tcgtgaccca tggcgatgcc tgcttgccga atatcatggt ggaaaatggc    3300
cgcttttctg gattcatcga ctgtggccgg ctgggtgtgg cggaccgcta tcaggacata    3360
gcgttggcta cccgtgatat tgctgaagag cttggcggcg aatgggctga ccgcttcctc    3420
gtgctttacg gtatcgccgc tcccgattcg cagcgcatcg ccttctatcg ccttcttgac    3480
gagttcttct gagcgggact ctggggttcg aaatgaccga ccaagcgacg cccaacctgc    3540
catcacgatg gccgcaataa aatatcttta ttttcattac atctgtgtgt tggttttttg    3600
tgtgaatcga tagcgataag gatccgcgta tggtgcactc tcagtacaat ctgctctgat    3660

gccgcatagt taagccagcc ccgacacccg ccaacacccg ctgacgcgcc ctgacgggct 3720
tgtctgctcc cggcatccgc ttacagacaa gctgtgaccg tctccgggag ctgcatgtgt 3780
cagaggtttt caccgtcatc accgaaacgc gcgagacgaa agggcctcgt gatacgccta 3840
tttttatagg ttaatgtcat gataataatg gtttcttaga cgtcaggtgg cacttttcgg 3900
ggaaatgtgc gcggaacccc tatttgttta tttttctaaa tacattcaaa tatgtatccg 3960
ctcatgagac aataaccctg ataaatgctt caataatatt gaaaaaggaa gagtatgagt 4020
attcaacatt tccgtgtcgc ccttattccc ttttttgcgg cattttgcct tcctgttttt 4080
gctcacccag aaacgctggt gaaagtaaaa gatgctgaag atcagttggg tgcacgagtg 4140
ggttacatcg aactggatct caacagcggt aagatccttg agagttttcg ccccgaagaa 4200
cgttttccaa tgatgagcac ttttaaagtt ctgctatgtg gcgcggtatt atcccgtatt 4260
gacgccgggc aagagcaact cggtcgccgc atacactatt ctcagaatga cttggttgag 4320
tactcaccag tcacagaaaa gcatcttacg gatggcatga cagtaagaga attatgcagt 4380
gctgccataa ccatgagtga taacactgcg gccaacttac ttctgacaac gatcggagga 4440
ccgaaggagc taaccgcttt tttgcacaac atgggggatc atgtaactcg ccttgatcgt 4500
tgggaaccgg agctgaatga agccatacca aacgacgagc gtgacaccac gatgcctgta 4560
gcaatggcaa caacgttgcg caaactatta actggcgaac tacttactct agcttcccgg 4620
caacaattaa tagactggat ggaggcggat aaagttgcag gaccacttct gcgctcggcc 4680
cttccggctg gctggtttat tgctgataaa tctggagccg gtgagcgtgg gtctcgcggt 4740
atcattgcag cactggggcc agatggtaag ccctcccgta tcgtagttat ctacacgacg 4800
gggagtcagg caactatgga tgaacgaaat agacagatcg ctgagatagg tgcctcactg 4860
attaagcatt ggtaactgtc agaccaagtt tactcatata tactttagat tgatttaaaa 4920
cttcattttt aatttaaaag gatctaggtg aagatccttt ttgataatct catgaccaaa 4980
atcccttaac gtgagttttc gttccactga gcgtcagacc ccgtagaaaa gatcaaagga 5040
tcttcttgag atcctttttt tctgcgcgta atctgctgct tgcaaacaaa aaaaccaccg 5100
ctaccagcgg tggtttgttt gccggatcaa gagctaccaa ctctttttcc gaaggtaact 5160
ggcttcagca gagcgcagat accaaatact gttcttctag tgtagccgta gttaggccac 5220
cacttcaaga actctgtagc accgcctaca tacctcgctc tgctaatcct gttaccagtg 5280
gctgctgcca gtggcgataa gtcgtgtctt accgggttgg actcaagacg atagttaccg 5340
gataaggcgc agcggtcggg ctgaacgggg ggttcgtgca cacagcccag cttggagcga 5400
acgacctaca ccgaactgag atacctacag cgtgagctat gagaaagcgc cacgcttccc 5460
gaagggagaa aggcggacag gtatccggta agcggcaggg tcggaacagg agagcgcacg 5520
agggagcttc cagggggaaa cgcctggtat ctttatagtc ctgtcgggtt tcgccacctc 5580
tgacttgagc gtcgattttt gtgatgctcg tcaggggggc ggagcctatg gaaaaacgcc 5640
agcaacgcgg cctttttacg gttcctggcc ttttgctggc cttttgctca catggctcga 5700
cagatct 5707

<210> 159
<211> 5622
<212> DNA
<213> Homo sapiens
<220>
<221> misc_feature
<223> vecteur d'expression appelé GS-V11 contenant la séquence antisens
 de GS-N11 ;
 antisens: 1108...1269

<400> 159
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtgct gggttcagtt ccttgtgccc actgtccctg 1140
cagacgctct cctttccagg tggcctccag tggtttcccg ggtcctgctg ctccctaata 1200
gtagcgattc aggctcctgg tcccgggcac ctcaggctgg ccattcatcc agatctccct 1260
gatgatgcgg tcgacccggg cggccgcttc cctttagtga gggttaatgc ttcgagcaga 1320
catgataaga tacattgatg agtttggaca aaccacaact agaatgcagt gaaaaaaatg 1380
ctttatttgt gaaatttgtg atgctattgc tttatttgta accattataa gctgcaataa 1440
acaagttaac aacaacaatt gcattcattt tatgtttcag gttcaggggg agatgtggga 1500

ggttttttaa agcaagtaaa acctctacaa atgtggtaaa atccgataag gatcgatccg 1560
ggctggcgta atagcgaaga ggcccgcacc gatcgccctt cccaacagtt gcgcagcctg 1620
aatggcgaat ggacgcgccc tgtagcggcg cattaagcgc ggcgggtgtg gtggttacgc 1680
gcagcgtgac cgctacactt gccagcgccc tagcgcccgc tcctttcgct ttcttccctt 1740
cctttctcgc cacgttcgcc ggctttcccc gtcaagctct aaatcggggg ctccctttag 1800
ggttccgatt tagtgcttta cggcacctcg accccaaaaa acttgattag ggtgatggtt 1860
cacgtagtgg gccatcgccc tgatagacgg tttttcgccc tttgacgttg gagtccacgt 1920
tctttaatag tggactcttg ttccaaactg gaacaacact caaccctatc tcggtctatt 1980
cttttgattt ataagggatt ttgccgattt cggcctattg gttaaaaaat gagctgattt 2040
aacaaaaatt taacgcgaat tttaacaaaa tattaacgct tacaatttcc tgatgcggta 2100
ttttctcctt acgcatctgt gcggtatttc acaccgcata cgcggatctg cgcagcacca 2160
tggcctgaaa taacctctga aagaggaact tggttaggta ccttctgagg cggaaagaac 2220
cagctgtgga atgtgtgtca gttagggtgt ggaaagtccc caggctcccc agcaggcaga 2280
agtatgcaaa gcatgcatct caattagtca gcaaccaggt gtggaaagtc cccaggctcc 2340
ccagcaggca gaagtatgca aagcatgcat ctcaattagt cagcaaccat agtcccgccc 2400
ctaactccgc ccatcccgcc cctaactccg cccagttccg cccattctcc gccccatggc 2460
tgactaattt tttttattta tgcagaggcc gaggccgcct cggcctctga gctattccag 2520
aagtagtgag gaggcttttt tggaggccta ggcttttgca aaaagcttga ttcttctgac 2580
acaacagtct cgaacttaag gctagagcca ccatgattga acaagatgga ttgcacgcag 2640
gttctccggc cgcttgggtg gagaggctat tcggctatga ctgggcacaa cagacaatcg 2700
gctgctctga tgccgccgtg ttccggctgt cagcgcaggg gcgcccggtt ctttttgtca 2760
agaccgacct gtccggtgcc ctgaatgaac tgcaggacga ggcagcgcgg ctatcgtggc 2820
tggccacgac gggcgttcct tgcgcagctg tgctcgacgt tgtcactgaa gcgggaaggg 2880
actggctgct attgggcgaa gtgccggggc aggatctcct gtcatctcac cttgctcctg 2940
ccgagaaagt atccatcatg gctgatgcaa tgcggcggct gcatacgctt gatccggcta 3000
cctgcccatt cgaccaccaa gcgaaacatc gcatcgagcg agcacgtact cggatggaag 3060
ccggtcttgt cgatcaggat gatctggacg aagagcatca ggggctcgcg ccagccgaac 3120
tgttcgccag gctcaaggcg cgcatgcccg acggcgagga tctcgtcgtg acccatggcg 3180
atgcctgctt gccgaatatc atggtggaaa atggccgctt ttctggattc atcgactgtg 3240
gccggctggg tgtggcggac cgctatcagg acatagcgtt ggctacccgt gatattgctg 3300
aagagcttgg cggcgaatgg gctgaccgct tcctcgtgct ttacggtatc gccgctcccg 3360
attcgcagcg catcgccttc tatcgccttc ttgacgagtt cttctgagcg ggactctggg 3420
gttcgaaatg accgaccaag cgacgcccaa cctgccatca cgatggccgc aataaaatat 3480
ctttattttc attacatctg tgtgttggtt ttttgtgtga atcgatagcg ataaggatcc 3540
gcgtatggtg cactctcagt acaatctgct ctgatgccgc atagttaagc cagccccgac 3600
acccgccaac acccgctgac gcgccctgac gggcttgtct gctcccggca tccgcttaca 3660
gacaagctgt gaccgtctcc gggagctgca tgtgtcagag gttttcaccg tcatcaccga 3720
aacgcgcgag acgaaagggc ctcgtgatac gcctattttt ataggttaat gtcatgataa 3780
taatggtttc ttagacgtca ggtggcactt ttcggggaaa tgtgcgcgga acccctattt 3840
gtttattttt ctaaatacat tcaaatatgt atccgctcat gagacaataa ccctgataaa 3900
tgcttcaata atattgaaaa aggaagagta tgagtattca acatttccgt gtcgccctta 3960
ttcccttttt tgcggcattt tgccttcctg tttttgctca cccagaaacg ctggtgaaag 4020
taaaagatgc tgaagatcag ttgggtgcac gagtgggtta catcgaactg gatctcaaca 4080
gcggtaagat ccttgagagt tttcgccccg aagaacgttt tccaatgatg agcactttta 4140
aagttctgct atgtggcgcg gtattatccc gtattgacgc cgggcaagag caactcggtc 4200
gccgcataca ctattctcag aatgacttgg ttgagtactc accagtcaca gaaaagcatc 4260
ttacggatgg catgacagta agagaattat gcagtgctgc cataaccatg agtgataaca 4320
ctgcggccaa cttacttctg acaacgatcg gaggaccgaa ggagctaacc gcttttttgc 4380
acaacatggg ggatcatgta actcgccttg atcgttggga accggagctg aatgaagcca 4440
taccaaacga cgagcgtgac accacgatgc ctgtagcaat ggcaacaacg ttgcgcaaac 4500
tattaactgg cgaactactt actctagctt cccggcaaca attaatagac tggatggagg 4560
cggataaagt tgcaggacca cttctgcgct cggcccttcc ggctggctgg tttattgctg 4620
ataaatctgg agccggtgag cgtgggtctc gcggtatcat tgcagcactg gggccagatg 4680
gtaagccctc ccgtatcgta gttatctaca cgacggggag tcaggcaact atggatgaac 4740
gaaatagaca gatcgctgag ataggtgcct cactgattaa gcattggtaa ctgtcagacc 4800
aagtttactc atatatactt tagattgatt taaaacttca tttttaattt aaaaggatct 4860
aggtgaagat cctttttgat aatctcatga ccaaaatccc ttaacgtgag ttttcgttcc 4920
actgagcgtc agaccccgta gaaaagatca aaggatcttc ttgagatcct ttttttctgc 4980
gcgtaatctg ctgcttgcaa acaaaaaaac caccgctacc agcggtggtt tgtttgccgg 5040
atcaagagct accaactctt tttccgaagg taactggctt cagcagagcg cagataccaa 5100
atactgttct tctagtgtag ccgtagttag gccaccactt caagaactct gtagcaccgc 5160
ctacatacct cgctctgcta atcctgttac cagtggctgc tgccagtggc gataagtcgt 5220
gtcttaccgg gttggactca agacgatagt taccggataa ggcgcagcgg tcgggctgaa 5280
cggggggttc gtgcacacag cccagcttgg agcgaacgac ctacaccgaa ctgagatacc 5340
tacagcgtga gctatgagaa agcgccacgc ttcccgaagg gagaaaggcg gacaggtatc 5400
cggtaagcgg cagggtcgga acaggagagc gcacgaggga gcttccaggg ggaaacgcct 5460
ggtatcttta tagtcctgtc gggtttcgcc acctctgact tgagcgtcga tttttgtgat 5520
gctcgtcagg ggggcggagc ctatggaaaa acgccagcaa cgcggccttt ttacggttcc 5580
tggccttttg ctggcctttt gctcacatgg ctcgacagat ct 5622

<210> 160
<211> 5626

<212>  DNA
<213>  Homo sapiens
<220>
<221>  misc_feature
<223>  vecteur d'expression appelé GS-V12 contenant la séquence antisens
        de GS-N12 ;
       antisens: 1108...1273

<400>  160
tcaatattgg ccattagcca tattattcat tggttatata gcataaatca atattggcta     60
ttggccattg catacgttgt atctatatca taatatgtac atttatattg gctcatgtcc    120
aatatgaccg ccatgttggc attgattatt gactagttat taatagtaat caattacggg    180
gtcattagtt catagcccat atatggagtt ccgcgttaca taacttacgg taaatggccc    240
gcctggctga ccgcccaacg acccccgccc attgacgtca ataatgacgt atgttcccat    300
agtaacgcca atagggactt tccattgacg tcaatgggtg gagtatttac ggtaaactgc    360
ccacttggca gtacatcaag tgtatcatat gccaagtccg ccccctattg acgtcaatga    420
cggtaaatgg cccgcctggc attatgccca gtacatgacc ttacgggact ttcctacttg    480
gcagtacatc tacgtattag tcatcgctat taccatggtg atgcggtttt ggcagtacac    540
caatgggcgt ggatagcggt ttgactcacg gggatttcca agtctccacc ccattgacgt    600
caatgggagt ttgttttggc accaaaatca acgggacttt ccaaaatgtc gtaacaactg    660
cgatcgcccg ccccgttgac gcaaatgggc ggtaggcgtg tacggtggga ggtctatata    720
agcagagctc gtttagtgaa ccgtcagatc actagaagct ttattgcggt agtttatcac    780
agttaaattg ctaacgcagt cagtgcttct gacacaacag tctcgaactt aagctgcagt    840
gactctctta aggtagcctt gcagaagttg gtcgtgaggc actgggcagg taagtatcaa    900
ggttacaaga caggtttaag gagaccaata gaaactgggc ttgtcgagac agagaagact    960
cttgcgtttc tgataggcac ctattggtct tactgacatc cactttgcct ttctctccac   1020
aggtgtccac tcccagttca attacagctc ttaaggctag agtacttaat acgactcact   1080
ataggctagc ctcgagaatt cacgcgtaag tttgggaacc gctggtgatg ggacatggat   1140
gcttgcaacc gaccgtgggc ggatgtggtt gaccagatgg cagaggacga caccatccat   1200
gagggctgcc cccaggtctt cgtgtagact gaccttcaat ctcatctcaa tgctctcacg   1260
aagttgttcc accgtcgacc cgggcggccg cttcccttta gtgagggtta atgcttcgag   1320
cagacatgat aagatacatt gatgagtttg gacaaaccac aactagaatg cagtgaaaaa   1380
aatgctttat ttgtgaaatt tgtgatgcta ttgctttatt tgtaaccatt ataagctgca   1440
ataaacaagt taacaacaac aattgcattc attttatgtt tcaggttcag ggggagatgt   1500
gggaggtttt ttaaagcaag taaaacctct acaaatgtgg taaaatccga taaggatcga   1560
tccgggctgg cgtaatagcg aagaggcccg caccgatcgc ccttcccaac agttgcgcag   1620
cctgaatggc gaatggacgc gccctgtagc ggcgcattaa gcgcggcggg tgtggtggtt   1680
acgcgcagcg tgaccgctac acttgccagc gccctagcgc ccgctccttt cgctttcttc   1740
ccttcctttc tcgccacgtt cgccggcttt ccccgtcaag ctctaaatcg ggggctccct   1800
ttagggttcc gatttagtgc tttacggcac ctcgacccca aaaaacttga ttagggtgat   1860
ggttcacgta gtgggccatc gccctgatag acggtttttc gccctttgac gttggagtcc   1920
acgttcttta atagtggact cttgttccaa actggaacaa cactcaaccc tatctcggtc   1980
tattcttttg atttataagg gattttgccg atttcggcct attggttaaa aaatgagctg   2040
atttaacaaa aatttaacgc gaattttaac aaaatattaa cgcttacaat ttcctgatgc   2100
ggtattttct ccttacgcat ctgtgcggta tttcacaccg catacgcgga tctgcgcagc   2160
accatggcct gaaataacct ctgaaagagg aacttggtta ggtaccttct gaggcggaaa   2220
gaaccagctg tggaatgtgt gtcagttagg gtgtggaaag tccccaggct ccccagcagg   2280
cagaagtatg caaagcatgc atctcaatta gtcagcaacc aggtgtggaa agtccccagg   2340
ctccccagca ggcagaagta tgcaaagcat gcatctcaat tagtcagcaa ccatagtccc   2400
gcccctaact ccgcccatcc cgcccctaac tccgcccagt tccgcccatt ctccgcccca   2460
tggctgacta atttttttta tttatgcaga ggccgaggcc gcctcggcct ctgagctatt   2520
ccagaagtag tgaggaggct tttttggagg cctaggcttt tgcaaaaagc ttgattcttc   2580
tgacacaaca gtctcgaact taaggctaga gccaccatga ttgaacaaga tggattgcac   2640
gcaggttctc cggccgcttg ggtggagagg ctattcggct atgactgggc acaacagaca   2700
atcggctgct ctgatgccgc cgtgttccgg ctgtcagcgc aggggcgccc ggttcttttt   2760
gtcaagaccg acctgtccgg tgccctgaat gaactgcagg acgaggcagc gcggctatcg   2820
tggctggcca cgacgggcgt tccttgcgca gctgtgctcg acgttgtcac tgaagcggga   2880
agggactggc tgctattggg cgaagtgccg gggcaggatc tcctgtcatc tcaccttgct   2940
cctgccgaga aagtatccat catggctgat gcaatgcggc ggctgcatac gcttgatccg   3000
gctacctgcc cattcgacca ccaagcgaaa catcgcatcg agcgagcacg tactcggatg   3060
gaagccggtc ttgtcgatca ggatgatctg gacgaagagc atcaggggct cgcgccagcc   3120
gaactgttcg ccaggctcaa ggcgcgcatg cccgacggcg aggatctcgt cgtgacccat   3180
ggcgatgcct gcttgccgaa tatcatggtg gaaaatggcc gcttttctgg attcatcgac   3240
tgtggccggc tgggtgtggc ggaccgctat caggacatag cgttggctac ccgtgatatt   3300
gctgaagagc ttggcggcga atgggctgac cgcttcctcg tgctttacgg tatcgccgct   3360
cccgattcgc agcgcatcgc cttctatcgc cttcttgacg agttcttctg agcgggactc   3420
tggggttcga aatgaccgac caagcgacgc ccaacctgcc atcacgatgg ccgcaataaa   3480
atatctttat tttcattaca tctgtgtgtt ggttttttgt gtgaatcgat agcgataagg   3540
atccgcgtat ggtgcactct cagtacaatc tgctctgatg ccgcatagtt aagccagccc   3600
cgacacccgc caacacccgc tgacgcgccc tgacgggctt gtctgctccc ggcatccgct   3660
tacagacaag ctgtgaccgt ctccgggagc tgcatgtgtc agaggttttc accgtcatca   3720
ccgaaacgcg cgagacgaaa gggcctcgtg atacgcctat ttttataggt taatgtcatg   3780
ataataatgg tttcttagac gtcaggtggc acttttcggg gaaatgtgcg cggaacccct   3840

atttgtttat ttttctaaat acattcaaat atgtatccgc tcatgagaca ataaccctga 3900
taaatgcttc aataatattg aaaaaggaag agtatgagta ttcaacattt ccgtgtcgcc 3960
cttattccct tttttgcggc attttgcctt cctgtttttg ctcacccaga aacgctggtg 4020
aaagtaaaag atgctgaaga tcagttgggt gcacgagtgg gttacatcga actggatctc 4080
aacagcggta agatccttga gagttttcgc cccgaagaac gttttccaat gatgagcact 4140
tttaaagttc tgctatgtgg cgcggtatta tcccgtattg acgccgggca agagcaactc 4200
ggtcgccgca tacactattc tcagaatgac ttggttgagt actcaccagt cacagaaaag 4260
catcttacgg atggcatgac agtaagagaa ttatgcagtg ctgccataac catgagtgat 4320
aacactgcgg ccaacttact tctgacaacg atcggaggac cgaaggagct aaccgctttt 4380
ttgcacaaca tgggggatca tgtaactcgc cttgatcgtt gggaaccgga gctgaatgaa 4440
gccataccaa acgacgagcg tgacaccacg atgcctgtag caatggcaac aacgttgcgc 4500
aaactattaa ctggcgaact acttactcta gcttcccggc aacaattaat agactggatg 4560
gaggcggata aagttgcagg accacttctg cgctcggccc ttccggctgg ctggtttatt 4620
gctgataaat ctggagccgg tgagcgtggg tctcgcggta tcattgcagc actggggcca 4680
gatggtaagc cctcccgtat cgtagttatc tacacgacgg ggagtcaggc aactatggat 4740
gaacgaaata gacagatcgc tgagataggt gcctcactga ttaagcattg gtaactgtca 4800
gaccaagttt actcatatat actttagatt gatttaaaac ttcattttta atttaaaagg 4860
atctaggtga agatcctttt tgataatctc atgaccaaaa tcccttaacg tgagttttcg 4920
ttccactgag cgtcagaccc cgtagaaaag atcaaaggat cttcttgaga tccttttttt 4980
ctgcgcgtaa tctgctgctt gcaaacaaaa aaaccaccgc taccagcggt ggtttgtttg 5040
ccggatcaag agctaccaac tctttttccg aaggtaactg gcttcagcag agcgcagata 5100
ccaaatactg ttcttctagt gtagccgtag ttaggccacc acttcaagaa ctctgtagca 5160
ccgcctacat acctcgctct gctaatcctg ttaccagtgg ctgctgccag tggcgataag 5220
tcgtgtctta ccgggttgga ctcaagacga tagttaccgg ataaggcgca gcggtcgggc 5280
tgaacggggg gttcgtgcac acagcccagc ttggagcgaa cgacctacac cgaactgaga 5340
tacctacagc gtgagctatg agaaagcgcc acgcttcccg aagggagaaa ggcggacagg 5400
tatccggtaa gcggcagggt cggaacagga gagcgcacga gggagcttcc agggggaaac 5460
gcctggtatc tttatagtcc tgtcgggttt cgccacctct gacttgagcg tcgatttttg 5520
tgatgctcgt caggggggcg gagcctatgg aaaaacgcca gcaacgcggc ctttttacgg 5580
ttcctggcct tttgctggcc ttttgctcac atggctcgac agatct 5626

<210> 161
<211> 5595
<212> DNA
<213> Homo sapiens
<220>
<221> misc_feature
<223> vecteur d'expression appelé GS-V13 contenant la séquence antisens
de GS-N13 ;
antisens: 1108...1239

<400> 161
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtgcg cttgttaggg gctgaagcag aagagtcaga 1140
gttctgaaga gtaagagaag atagtgaagc cagcccactt gtgacagcag aggataaagc 1200
agaggagttg attaaattgg tgtcattgga tgtcagaaaa ccgtcgaccc gggcggccgc 1260
ttccctttag tgagggttaa tgcttcgagc agacatgata agatacattg atgagtttgg 1320
acaaaccaca actagaatgc agtgaaaaaa atgctttatt tgtgaaattt gtgatgctat 1380
tgctttattt gtaaccatta taagctgcaa taaacaagtt aacaacaaca attgcattca 1440
ttttatgttt caggttcagg gggagatgtg ggaggttttt taaagcaagt aaaacctcta 1500
caaatgtggt aaaatccgat aaggatcgat ccgggctggc gtaatagcga agaggcccgc 1560
accgatcgcc cttcccaaca gttgcgcagc ctgaatggcg aatggacgcg ccctgtagcg 1620
gcgcattaag cgcggcgggt gtggtggtta cgcgcagcgt gaccgctaca cttgccagcg 1680
ccctagcgcc cgctcctttc gctttcttcc cttcctttct cgccacgttc gccggctttc 1740
cccgtcaagc tctaaatcgg gggctccctt tagggttccg atttagtgct ttacggcacc 1800

tcgaccccaa aaaacttgat tagggtgatg gttcacgtag tgggccatcg ccctgataga 1860
cggtttttcg ccctttgacg ttggagtcca cgttctttaa tagtggactc ttgttccaaa 1920
ctggaacaac actcaaccct atctcggtct attcttttga tttataaggg attttgccga 1980
tttcggccta ttggttaaaa aatgagctga tttaacaaaa atttaacgcg aattttaaca 2040
aaatattaac gcttacaatt tcctgatgcg gtattttctc cttacgcatc tgtgcggtat 2100
ttcacaccgc atacgcggat ctgcgcagca ccatggcctg aaataacctc tgaaagagga 2160
acttggttag gtaccttctg aggcggaaag aaccagctgt ggaatgtgtg tcagttaggg 2220
tgtggaaagt ccccaggctc cccagcaggc agaagtatgc aaagcatgca tctcaattag 2280
tcagcaacca ggtgtggaaa gtccccaggc tccccagcag gcagaagtat gcaaagcatg 2340
catctcaatt agtcagcaac catagtcccg cccctaactc cgcccatccc gcccctaact 2400
ccgcccagtt ccgcccattc tccgccccat ggctgactaa ttttttttat ttatgcagag 2460
gccgaggccg cctcggcctc tgagctattc cagaagtagt gaggaggctt ttttggaggc 2520
ctaggctttt gcaaaaagct tgattcttct gacacaacag tctcgaactt aaggctagag 2580
ccaccatgat tgaacaagat ggattgcacg caggttctcc ggccgcttgg gtggagaggc 2640
tattcggcta tgactgggca caacagacaa tcggctgctc tgatgccgcc gtgttccggc 2700
tgtcagcgca ggggcgcccg gttctttttg tcaagaccga cctgtccggt gccctgaatg 2760
aactgcagga cgaggcagcg cggctatcgt ggctggccac gacgggcgtt ccttgcgcag 2820
ctgtgctcga cgttgtcact gaagcgggaa gggactggct gctattgggc gaagtgccgg 2880
ggcaggatct cctgtcatct caccttgctc ctgccgagaa agtatccatc atggctgatg 2940
caatgcggcg gctgcatacg cttgatccgg ctacctgccc attcgaccac caagcgaaac 3000
atcgcatcga gcgagcacgt actcggatgg aagccggtct tgtcgatcag gatgatctgg 3060
acgaagagca tcaggggctc gcgccagccg aactgttcgc caggctcaag gcgcgcatgc 3120
ccgacggcga ggatctcgtc gtgacccatg gcgatgcctg cttgccgaat atcatggtgg 3180
aaaatggccg cttttctgga ttcatcgact gtggccggct gggtgtggcg gaccgctatc 3240
aggacatagc gttggctacc cgtgatattg ctgaagagct tggcggcgaa tgggctgacc 3300
gcttcctcgt gctttacggt atcgccgctc ccgattcgca gcgcatcgcc ttctatcgcc 3360
ttcttgacga gttcttctga gcgggactct ggggttcgaa atgaccgacc aagcgacgcc 3420
caacctgcca tcacgatggc cgcaataaaa tatctttatt ttcattacat ctgtgtgttg 3480
gttttttgtg tgaatcgata gcgataagga tccgcgtatg gtgcactctc agtacaatct 3540
gctctgatgc cgcatagtta agccagcccc gacacccgcc aacacccgct gacgcgccct 3600
gacgggcttg tctgctcccg gcatccgctt acagacaagc tgtgaccgtc tccgggagct 3660
gcatgtgtca gaggttttca ccgtcatcac cgaaacgcgc gagacgaaag ggcctcgtga 3720
tacgcctatt tttataggtt aatgtcatga taataatggt ttcttagacg tcaggtggca 3780
cttttcgggg aaatgtgcgc ggaaccccta tttgtttatt tttctaaata cattcaaata 3840
tgtatccgct catgagacaa taaccctgat aaatgcttca ataatattga aaaaggaaga 3900
gtatgagtat tcaacatttc cgtgtcgccc ttattccctt ttttgcggca ttttgccttc 3960
ctgtttttgc tcacccagaa acgctggtga aagtaaaaga tgctgaagat cagttgggtg 4020
cacgagtggg ttacatcgaa ctggatctca acagcggtaa gatccttgag agttttcgcc 4080
ccgaagaacg ttttccaatg atgagcactt ttaaagttct gctatgtggc gcggtattat 4140
cccgtattga cgccgggcaa gagcaactcg gtcgccgcat acactattct cagaatgact 4200
tggttgagta ctcaccagtc acagaaaagc atcttacgga tggcatgaca gtaagagaat 4260
tatgcagtgc tgccataacc atgagtgata acactgcggc caacttactt ctgacaacga 4320
tcggaggacc gaaggagcta accgcttttt tgcacaacat gggggatcat gtaactcgcc 4380
ttgatcgttg ggaaccggag ctgaatgaag ccataccaaa cgacgagcgt gacaccacga 4440
tgcctgtagc aatggcaaca acgttgcgca aactattaac tggcgaacta cttactctag 4500
cttcccggca acaattaata gactggatgg aggcggataa agttgcagga ccacttctgc 4560
gctcggccct tccggctggc tggtttattg ctgataaatc tggagccggt gagcgtgggt 4620
ctcgcggtat cattgcagca ctggggccag atggtaagcc ctcccgtatc gtagttatct 4680
acacgacggg gagtcaggca actatggatg aacgaaatag acagatcgct gagataggtg 4740
cctcactgat taagcattgg taactgtcag accaagttta ctcatatata ctttagattg 4800
atttaaaact tcatttttaa tttaaaagga tctaggtgaa gatccttttt gataatctca 4860
tgaccaaaat cccttaacgt gagttttcgt tccactgagc gtcagacccc gtagaaaaga 4920
tcaaaggatc ttcttgagat cctttttttc tgcgcgtaat ctgctgcttg caaacaaaaa 4980
aaccaccgct accagcggtg gtttgtttgc cggatcaaga gctaccaact ctttttccga 5040
aggtaactgg cttcagcaga gcgcagatac caaatactgt tcttctagtg tagccgtagt 5100
taggccacca cttcaagaac tctgtagcac cgcctacata cctcgctctg ctaatcctgt 5160
taccagtggc tgctgccagt ggcgataagt cgtgtcttac cgggttggac tcaagacgat 5220
agttaccgga taaggcgcag cggtcgggct gaacgggggg ttcgtgcaca cagcccagct 5280
tggagcgaac gacctacacc gaactgagat acctacagcg tgagctatga gaaagcgcca 5340
cgcttcccga agggagaaag gcggacaggt atccggtaag cggcagggtc ggaacaggag 5400
agcgcacgag ggagcttcca gggggaaacg cctggtatct ttatagtcct gtcgggtttc 5460
gccacctctg acttgagcgt cgatttttgt gatgctcgtc aggggggcgg agcctatgga 5520
aaaacgccag caacgcggcc tttttacggt tcctggcctt ttgctggcct tttgctcaca 5580
tggctcgaca gatct 5595

<210> 162
<211> 5596
<212> DNA
<213> Homo sapiens
<220>
<221> misc_feature
<223> vecteur d'expression appelé GS-V14 contenant la séquence antisens

de GS-N14 ;
antisens: 1108...1243

<400> 162
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtttc cacatccttg tgggtcatat acttcaggaa 1140
atagacaggt ttagtatgac caacagtaat agtaatacag ttccttgggt ttatagttgc 1200
atctgcttaa aatccttaac cagatgacta gatcttgcgc agagtcgacc cgggcggccg 1260
cttcccttta gtgagggtta atgcttcgag cagacatgat aagatacatt gatgagtttg 1320
gacaaaccac aactagaatg cagtgaaaaa aatgctttat ttgtgaaatt tgtgatgcta 1380
ttgctttatt tgtaaccatt ataagctgca ataaacaagt taacaacaac aattgcattc 1440
attttatgtt tcaggttcag ggggagatgt gggaggtttt ttaaagcaag taaaacctct 1500
acaaatgtgg taaaatccga taaggatcga tccgggctgg cgtaatagcg aagaggcccg 1560
caccgatcgc ccttcccaac agttgcgcag cctgaatggc gaatggacgc gccctgtagc 1620
ggcgcattaa gcgcggcggg tgtggtggtt acgcgcagcg tgaccgctac acttgccagc 1680
gccctagcgc ccgctccttt cgctttcttc ccttcctttc tcgccacgtt cgccggcttt 1740
ccccgtcaag ctctaaatcg ggggctccct ttagggttcc gatttagtgc tttacggcac 1800
ctcgacccca aaaaacttga ttagggtgat ggttcacgta gtgggccatc gccctgatag 1860
acggtttttc gccctttgac gttggagtcc acgttcttta atagtggact cttgttccaa 1920
actggaacaa cactcaaccc tatctcggtc tattcttttg atttataagg gattttgccg 1980
atttcggcct attggttaaa aaatgagctg atttaacaaa aatttaacgc gaattttaac 2040
aaaatattaa cgcttacaat ttcctgatgc ggtattttct ccttacgcat ctgtgcggta 2100
tttcacaccg catacgcgga tctgcgcagc accatggcct gaaataacct ctgaaagagg 2160
aacttggtta ggtaccttct gaggcggaaa gaaccagctg tggaatgtgt gtcagttagg 2220
gtgtggaaag tccccaggct ccccagcagg cagaagtatg caaagcatgc atctcaatta 2280
gtcagcaacc aggtgtggaa agtccccagg ctccccagca ggcagaagta tgcaaagcat 2340
gcatctcaat tagtcagcaa ccatagtccc gcccctaact ccgcccatcc cgcccctaac 2400
tccgcccagt tccgcccatt ctccgcccca tggctgacta atttttttta tttatgcaga 2460
ggccgaggcc gcctcggcct ctgagctatt ccagaagtag tgaggaggct tttttggagg 2520
cctaggcttt tgcaaaaagc ttgattcttc tgacacaaca gtctcgaact taaggctaga 2580
gccaccatga ttgaacaaga tggattgcac gcaggttctc cggccgcttg ggtggagagg 2640
ctattcggct atgactgggc acaacagaca atcggctgct ctgatgccgc cgtgttccgg 2700
ctgtcagcgc aggggcgccc ggttcttttt gtcaagaccg acctgtccgg tgccctgaat 2760
gaactgcagg acgaggcagc gcggctatcg tggctggcca cgacgggcgt tccttgcgca 2820
gctgtgctcg acgttgtcac tgaagcggga agggactggc tgctattggg cgaagtgccg 2880
gggcaggatc tcctgtcatc tcaccttgct cctgccgaga aagtatccat catggctgat 2940
gcaatgcggc ggctgcatac gcttgatccg gctacctgcc cattcgacca ccaagcgaaa 3000
catcgcatcg agcgagcacg tactcggatg gaagccggtc ttgtcgatca ggatgatctg 3060
gacgaagagc atcaggggct cgcgccagcc gaactgttcg ccaggctcaa ggcgcgcatg 3120
cccgacggcg aggatctcgt cgtgacccat ggcgatgcct gcttgccgaa tatcatggtg 3180
gaaaatggcc gcttttctgg attcatcgac tgtggccggc tgggtgtggc ggaccgctat 3240
caggacatag cgttggctac ccgtgatatt gctgaagagc ttggcggcga atgggctgac 3300
cgcttcctcg tgctttacgg tatcgccgct cccgattcgc agcgcatcgc cttctatcgc 3360
cttcttgacg agttcttctg agcgggactc tggggttcga aatgaccgac caagcgacgc 3420
ccaacctgcc atcacgatgg ccgcaataaa atatctttat tttcattaca tctgtgtgtt 3480
ggttttttgt gtgaatcgat agcgataagg atccgcgtat ggtgcactct cagtacaatc 3540
tgctctgatg ccgcatagtt aagccagccc cgacacccgc caacacccgc tgacgcgccc 3600
tgacgggctt gtctgctccc ggcatccgct tacagacaag ctgtgaccgt ctccgggagc 3660
tgcatgtgtc agaggttttc accgtcatca ccgaaacgcg cgagacgaaa gggcctcgtg 3720
atacgcctat ttttataggt taatgtcatg ataataatgg tttcttagac gtcaggtggc 3780
acttttcggg gaaatgtgcg cggaacccct atttgtttat ttttctaaat acattcaaat 3840
atgtatccgc tcatgagaca ataaccctga taaatgcttc aataatattg aaaaaggaag 3900
agtatgagta ttcaacattt ccgtgtcgcc cttattccct tttttgcggc attttgcctt 3960
cctgtttttg ctcacccaga aacgctggtg aaagtaaaag atgctgaaga tcagttgggt 4020
gcacgagtgg gttacatcga actggatctc aacagcggta agatccttga gagttttcgc 4080
cccgaagaac gttttccaat gatgagcact tttaaagttc tgctatgtgg cgcggtatta 4140

tcccgtattg acgccgggca agagcaactc ggtcgccgca tacactattc tcagaatgac 4200
ttggttgagt actcaccagt cacagaaaag catcttacgg atggcatgac agtaagagaa 4260
ttatgcagtg ctgccataac catgagtgat aacactgcgg ccaacttact tctgacaacg 4320
atcggaggac cgaaggagct aaccgctttt ttgcacaaca tgggggatca tgtaactcgc 4380
cttgatcgtt gggaaccgga gctgaatgaa gccataccaa acgacgagcg tgacaccacg 4440
atgcctgtag caatggcaac aacgttgcgc aaactattaa ctggcgaact acttactcta 4500
gcttcccggc aacaattaat agactggatg gaggcggata aagttgcagg accacttctg 4560
cgctcggccc ttccggctgg ctggtttatt gctgataaat ctggagccgg tgagcgtggg 4620
tctcgcggta tcattgcagc actggggcca gatggtaagc cctcccgtat cgtagttatc 4680
tacacgacgg ggagtcaggc aactatggat gaacgaaata gacagatcgc tgagataggt 4740
gcctcactga ttaagcattg gtaactgtca gaccaagttt actcatatat actttagatt 4800
gatttaaaac ttcattttta atttaaaagg atctaggtga agatcctttt tgataatctc 4860
atgaccaaaa tcccttaacg tgagttttcg ttccactgag cgtcagaccc cgtagaaaag 4920
atcaaaggat cttcttgaga tccttttttt ctgcgcgtaa tctgctgctt gcaaacaaaa 4980
aaaccaccgc taccagcggt ggtttgtttg ccggatcaag agctaccaac tctttttccg 5040
aaggtaactg gcttcagcag agcgcagata ccaaatactg ttcttctagt gtagccgtag 5100
ttaggccacc acttcaagaa ctctgtagca ccgcctacat acctcgctct gctaatcctg 5160
ttaccagtgg ctgctgccag tggcgataag tcgtgtctta ccgggttgga ctcaagacga 5220
tagttaccgg ataaggcgca gcggtcgggc tgaacggggg gttcgtgcac acagcccagc 5280
ttggagcgaa cgacctacac cgaactgaga tacctacagc gtgagctatg agaaagcgcc 5340
acgcttcccg aagggagaaa ggcggacagg tatccggtaa gcggcagggt cggaacagga 5400
gagcgcacga gggagcttcc agggggaaac gcctggtatc tttatagtcc tgtcgggttt 5460
cgccacctct gacttgagcg tcgatttttg tgatgctcgt caggggggcg gagcctatgg 5520
aaaaacgcca gcaacgcggc ctttttacgg ttcctggcct tttgctggcc ttttgctcac 5580
atggctcgac agatct 5596

<210> 163
<211> 6012
<212> DNA
<213> Homo sapiens
<220>
<221> misc_feature
<223> vecteur d'expression appelé GS-V15 contenant la séquence antisens
 de GS-N15 ;
 antisens: 1108...1659

<400> 163
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtgcg gccgcgaatt cactagtgat tcattatgct 1140
gagtgatatc ttttttttta atatactttt ggaattctca gtgaatttgt ttgcttgccc 1200
aaaatattat tttccattct gtaatttttc tttaccttcc attacttaca ggtttctctg 1260
caatttaagt acagttcacc accaataatt gtttctgaag ttaagggtga cctgctggct 1320
tggtcacttt tgcagtgact ggacctgcct gtggccaagc acatgttcca tcactgtaac 1380
tcatcaatac caagattcca gcaatggatt ctcaaccttg tctgctgtgg gaatctaggc 1440
agtctagcca agaccaagaa gagctgagct tctgcaagct attgatataa aactggcata 1500
gttttatctt ctttcctctt gacaaaatat atatacttta aacgtccttt catctccctt 1560
cacgaagtac aaattaggaa cttttcttca gttttgtaca gtggcatgtt gctgcttccc 1620
cagcatttag tgagggttaa taatcgaatt cccgcggccg tcgacccggg cggccgcttc 1680
cctttagtga gggttaatgc ttcgagcaga catgataaga tacattgatg agtttggaca 1740
aaccacaact agaatgcagt gaaaaaaatg ctttatttgt gaaatttgtg atgctattgc 1800
tttatttgta accattataa gctgcaataa acaagttaac aacaacaatt gcattcattt 1860
tatgtttcag gttcaggggg agatgtggga ggttttttaa agcaagtaaa acctctacaa 1920
atgtggtaaa atccgataag gatcgatccg ggctggcgta atagcgaaga ggcccgcacc 1980
gatcgccctt cccaacagtt gcgcagcctg aatggcgaat ggacgcgccc tgtagcggcg 2040
cattaagcgc ggcgggtgtg gtggttacgc gcagcgtgac cgctacactt gccagcgccc 2100

tagcgcccgc tcctttcgct ttcttccctt cctttctcgc cacgttcgcc ggctttcccc 2160
gtcaagctct aaatcggggg ctccctttag ggttccgatt tagtgcttta cggcacctcg 2220
accccaaaaa acttgattag ggtgatggtt cacgtagtgg gccatcgccc tgatagacgg 2280
tttttcgccc tttgacgttg gagtccacgt tctttaatag tggactcttg ttccaaactg 2340
gaacaacact caaccctatc tcggtctatt cttttgattt ataagggatt ttgccgattt 2400
cggcctattg gttaaaaaat gagctgattt aacaaaaatt taacgcgaat tttaacaaaa 2460
tattaacgct tacaatttcc tgatgcggta ttttctcctt acgcatctgt gcggtatttc 2520
acaccgcata cgcggatctg cgcagcacca tggcctgaaa taacctctga aagaggaact 2580
tggttaggta ccttctgagg cggaaagaac cagctgtgga atgtgtgtca gttagggtgt 2640
ggaaagtccc caggctcccc agcaggcaga agtatgcaaa gcatgcatct caattagtca 2700
gcaaccaggt gtggaaagtc cccaggctcc ccagcaggca gaagtatgca aagcatgcat 2760
ctcaattagt cagcaaccat agtcccgccc ctaactccgc ccatcccgcc cctaactccg 2820
cccagttccg cccattctcc gccccatggc tgactaattt tttttattta tgcagaggcc 2880
gaggccgcct cggcctctga gctattccag aagtagtgag gaggcttttt tggaggccta 2940
ggcttttgca aaaagcttga ttcttctgac acaacagtct cgaacttaag gctagagcca 3000
ccatgattga acaagatgga ttgcacgcag gttctccggc cgcttgggtg gagaggctat 3060
tcggctatga ctgggcacaa cagacaatcg gctgctctga tgccgccgtg ttccggctgt 3120
cagcgcaggg gcgcccggtt ctttttgtca agaccgacct gtccggtgcc ctgaatgaac 3180
tgcaggacga ggcagcgcgg ctatcgtggc tggccacgac gggcgttcct tgcgcagctg 3240
tgctcgacgt tgtcactgaa gcgggaaggg actggctgct attgggcgaa gtgccggggc 3300
aggatctcct gtcatctcac cttgctcctg ccgagaaagt atccatcatg gctgatgcaa 3360
tgcggcggct gcatacgctt gatccggcta cctgcccatt cgaccaccaa gcgaaacatc 3420
gcatcgagcg agcacgtact cggatggaag ccggtcttgt cgatcaggat gatctggacg 3480
aagagcatca ggggctcgcg ccagccgaac tgttcgccag gctcaaggcg cgcatgcccg 3540
acggcgagga tctcgtcgtg acccatggcg atgcctgctt gccgaatatc atggtggaaa 3600
atggccgctt ttctggattc atcgactgtg gccggctggg tgtggcggac cgctatcagg 3660
acatagcgtt ggctacccgt gatattgctg aagagcttgg cggcgaatgg gctgaccgct 3720
tcctcgtgct ttacggtatc gccgctcccg attcgcagcg catcgccttc tatcgccttc 3780
ttgacgagtt cttctgagcg ggactctggg gttcgaaatg accgaccaag cgacgcccaa 3840
cctgccatca cgatggccgc aataaaatat ctttattttc attacatctg tgtgttggtt 3900
ttttgtgtga atcgatagcg ataaggatcc gcgtatggtg cactctcagt acaatctgct 3960
ctgatgccgc atagttaagc cagccccgac acccgccaac acccgctgac gcgccctgac 4020
gggcttgtct gctcccggca tccgcttaca gacaagctgt gaccgtctcc gggagctgca 4080
tgtgtcagag gttttcaccg tcatcaccga aacgcgcgag acgaaagggc ctcgtgatac 4140
gcctattttt ataggttaat gtcatgataa taatggtttc ttagacgtca ggtggcactt 4200
ttcggggaaa tgtgcgcgga acccctattt gtttattttt ctaaatacat tcaaatatgt 4260
atccgctcat gagacaataa ccctgataaa tgcttcaata atattgaaaa aggaagagta 4320
tgagtattca acatttccgt gtcgccctta ttcccttttt tgcggcattt tgccttcctg 4380
tttttgctca cccagaaacg ctggtgaaag taaaagatgc tgaagatcag ttgggtgcac 4440
gagtgggtta catcgaactg gatctcaaca gcggtaagat ccttgagagt tttcgccccg 4500
aagaacgttt tccaatgatg agcactttta aagttctgct atgtggcgcg gtattatccc 4560
gtattgacgc cgggcaagag caactcggtc gccgcataca ctattctcag aatgacttgg 4620
ttgagtactc accagtcaca gaaaagcatc ttacggatgg catgacagta agagaattat 4680
gcagtgctgc cataaccatg agtgataaca ctgcggccaa cttacttctg acaacgatcg 4740
gaggaccgaa ggagctaacc gcttttttgc acaacatggg ggatcatgta actcgccttg 4800
atcgttggga accggagctg aatgaagcca taccaaacga cgagcgtgac accacgatgc 4860
ctgtagcaat ggcaacaacg ttgcgcaaac tattaactgg cgaactactt actctagctt 4920
cccggcaaca attaatagac tggatggagg cggataaagt tgcaggacca cttctgcgct 4980
cggcccttcc ggctggctgg tttattgctg ataaatctgg agccggtgag cgtgggtctc 5040
gcggtatcat tgcagcactg gggccagatg gtaagccctc ccgtatcgta gttatctaca 5100
cgacggggag tcaggcaact atggatgaac gaaatagaca gatcgctgag ataggtgcct 5160
cactgattaa gcattggtaa ctgtcagacc aagtttactc atatatactt tagattgatt 5220
taaaacttca tttttaattt aaaaggatct aggtgaagat cctttttgat aatctcatga 5280
ccaaaatccc ttaacgtgag ttttcgttcc actgagcgtc agaccccgta gaaaagatca 5340
aaggatcttc ttgagatcct ttttttctgc gcgtaatctg ctgcttgcaa acaaaaaaac 5400
caccgctacc agcggtggtt tgtttgccgg atcaagagct accaactctt tttccgaagg 5460
taactggctt cagcagagcg cagataccaa atactgttct tctagtgtag ccgtagttag 5520
gccaccactt caagaactct gtagcaccgc ctacatacct cgctctgcta atcctgttac 5580
cagtggctgc tgccagtggc gataagtcgt gtcttaccgg gttggactca agacgatagt 5640
taccggataa ggcgcagcgg tcgggctgaa cggggggttc gtgcacacag cccagcttgg 5700
agcgaacgac ctacaccgaa ctgagatacc tacagcgtga gctatgagaa agcgccacgc 5760
ttcccgaagg gagaaaggcg gacaggtatc cggtaagcgg cagggtcgga acaggagagc 5820
gcacgaggga gcttccaggg ggaaacgcct ggtatcttta tagtcctgtc gggtttcgcc 5880
acctctgact tgagcgtcga tttttgtgat gctcgtcagg ggggcggagc ctatggaaaa 5940
acgccagcaa cgcggccttt ttacggttcc tggccttttg ctggcctttt gctcacatgg 6000
ctcgacagat ct 6012

<210> 164
<211> 5877
<212> DNA
<213> Homo sapiens
<220>
<221> misc_feature

<223>  vecteur d'expression appelé GS-V16 contenant la séquence antisens
       de GS-N16 ; antisens: 1108...1524

<400>  164
tcaatattgg ccattagcca tattattcat tggttatata gcataaatca atattggcta      60
ttggccattg catacgttgt atctatatca taatatgtac atttatattg gctcatgtcc     120
aatatgaccg ccatgttggc attgattatt gactagttat taatagtaat caattacggg     180
gtcattagtt catagcccat atatggagtt ccgcgttaca taacttacgg taaatggccc     240
gcctggctga ccgcccaacg acccccgccc attgacgtca ataatgacgt atgttcccat     300
agtaacgcca atagggactt tccattgacg tcaatgggtg gagtatttac ggtaaactgc     360
ccacttggca gtacatcaag tgtatcatat gccaagtccg ccccctattg acgtcaatga     420
cggtaaatgg cccgcctggc attatgccca gtacatgacc ttacgggact ttcctacttg     480
gcagtacatc tacgtattag tcatcgctat taccatggtg atgcggtttt ggcagtacac     540
caatgggcgt ggatagcggt ttgactcacg gggatttcca agtctccacc ccattgacgt     600
caatgggagt ttgttttggc accaaaatca acgggacttt ccaaaatgtc gtaacaactg     660
cgatcgcccg ccccgttgac gcaaatgggc ggtaggcgtg tacggtggga ggtctatata     720
agcagagctc gtttagtgaa ccgtcagatc actagaagct ttattgcggt agtttatcac     780
agttaaattg ctaacgcagt cagtgcttct gacacaacag tctcgaactt aagctgcagt     840
gactctctta aggtagcctt gcagaagttg gtcgtgaggc actgggcagg taagtatcaa     900
ggttacaaga caggtttaag gagaccaata gaaactgggc ttgtcgagac agagaagact     960
cttgcgtttc tgataggcac ctattggtct tactgacatc cactttgcct ttctctccac    1020
aggtgtccac tcccagttca attacagctc ttaaggctag agtacttaat acgactcact    1080
ataggctagc ctcgagaatt cacgcgtccc tggaatgcca tcctcataaa tatgaaccat    1140
gatgacaatg cttttctgtt ctttatcaat catgtctaaa aacccttctc cactggagat    1200
ctcaaaaacc tgcttgaatt ggggcccctt gtgaagctgc tgccgcatct cttccattcg    1260
ctgcttccgg tactgctgca ggaactcttc atcatcttgg tcctcattca ttatggcaaa    1320
ctccttcaga gtcatcttcc cactgatctt ctcctggagg tctttctgtt tctgttgctc    1380
ctcctcttca tccagatggg acctgcaagt cattgacagc ctcttgatca gcctttccat    1440
ctcccggcac tgctcctccc tctgctctgt ctccaactgc ttgaagcggc gccagtcatt    1500
gatcacacct tttgggcctg tgttgtcgac ccgggcggcc gcttcccttt agtgagggtt    1560
aatgcttcga gcagacatga taagatacat tgatgagttt ggacaaacca caactagaat    1620
gcagtgaaaa aaatgcttta tttgtgaaat ttgtgatgct attgctttat ttgtaaccat    1680
tataagctgc aataaacaag ttaacaacaa caattgcatt cattttatgt ttcaggttca    1740
gggggagatg tgggaggttt tttaaagcaa gtaaaacctc tacaaatgtg gtaaaatccg    1800
ataaggatcg atccgggctg gcgtaatagc gaagaggccc gcaccgatcg cccttcccaa    1860
cagttgcgca gcctgaatgg cgaatggacg cgccctgtag cggcgcatta agcgcggcgg    1920
gtgtggtggt tacgcgcagc gtgaccgcta cacttgccag cgccctagcg cccgctcctt    1980
tcgctttctt cccttccttt ctcgccacgt tcgccggctt tccccgtcaa gctctaaatc    2040
gggggctccc tttagggttc cgatttagtg ctttacggca cctcgacccc aaaaaacttg    2100
attagggtga tggttcacgt agtgggccat cgccctgata gacggttttt cgccctttga    2160
cgttggagtc cacgttcttt aatagtggac tcttgttcca aactggaaca acactcaacc    2220
ctatctcggt ctattctttt gatttataag ggattttgcc gatttcggcc tattggttaa    2280
aaaatgagct gatttaacaa aaatttaacg cgaattttaa caaaatatta acgcttacaa    2340
tttcctgatg cggtattttc tccttacgca tctgtgcggt atttcacacc gcatacgcgg    2400
atctgcgcag caccatggcc tgaaataacc tctgaaagag gaacttggtt aggtaccttc    2460
tgaggcggaa agaaccagct gtggaatgtg tgtcagttag ggtgtggaaa gtccccaggc    2520
tccccagcag gcagaagtat gcaaagcatg catctcaatt agtcagcaac caggtgtgga    2580
aagtccccag gctccccagc aggcagaagt atgcaaagca tgcatctcaa ttagtcagca    2640
accatagtcc cgcccctaac tccgcccatc ccgcccctaa ctccgcccag ttccgcccat    2700
tctccgcccc atggctgact aatttttttt atttatgcag aggccgaggc cgcctcggcc    2760
tctgagctat tccagaagta gtgaggaggc ttttttggag gcctaggctt ttgcaaaaag    2820
cttgattctt ctgacacaac agtctcgaac ttaaggctag agccaccatg attgaacaag    2880
atggattgca cgcaggttct ccggccgctt gggtggagag gctattcggc tatgactggg    2940
cacaacagac aatcggctgc tctgatgccg ccgtgttccg gctgtcagcg caggggcgcc    3000
cggttctttt tgtcaagacc gacctgtccg gtgccctgaa tgaactgcag gacgaggcag    3060
cgcggctatc gtggctggcc acgacgggcg ttccttgcgc agctgtgctc gacgttgtca    3120
ctgaagcggg aagggactgg ctgctattgg gcgaagtgcc ggggcaggat ctcctgtcat    3180
ctcaccttgc tcctgccgag aaagtatcca tcatggctga tgcaatgcgg cggctgcata    3240
cgcttgatcc ggctacctgc ccattcgacc accaagcgaa acatcgcatc gagcgagcac    3300
gtactcggat ggaagccggt cttgtcgatc aggatgatct ggacgaagag catcaggggc    3360
tcgcgccagc cgaactgttc gccaggctca aggcgcgcat gcccgacggc gaggatctcg    3420
tcgtgaccca tggcgatgcc tgcttgccga atatcatggt ggaaaatggc cgcttttctg    3480
gattcatcga ctgtggccgg ctgggtgtgg cggaccgcta tcaggacata gcgttggcta    3540
cccgtgatat tgctgaagag cttggcggcg aatgggctga ccgcttcctc gtgctttacg    3600
gtatcgccgc tcccgattcg cagcgcatcg ccttctatcg ccttcttgac gagttcttct    3660
gagcgggact ctggggttcg aaatgaccga ccaagcgacg cccaacctgc catcacgatg    3720
gccgcaataa aatatcttta ttttcattac atctgtgtgt tggttttttg tgtgaatcga    3780
tagcgataag gatccgcgta tggtgcactc tcagtacaat ctgctctgat gccgcatagt    3840
taagccagcc ccgacacccg ccaacacccg ctgacgcgcc ctgacgggct tgtctgctcc    3900
cggcatccgc ttacagacaa gctgtgaccg tctccgggag ctgcatgtgt cagaggtttt    3960
caccgtcatc accgaaacgc gcgagacgaa agggcctcgt gatacgccta tttttatagg    4020
ttaatgtcat gataataatg gtttcttaga cgtcaggtgg cacttttcgg ggaaatgtgc    4080

gcggaacccc tatttgttta tttttctaaa tacattcaaa tatgtatccg ctcatgagac 4140
aataaccctg ataaatgctt caataatatt gaaaaaggaa gagtatgagt attcaacatt 4200
tccgtgtcgc ccttattccc ttttttgcgg cattttgcct tcctgttttt gctcacccag 4260
aaacgctggt gaaagtaaaa gatgctgaag atcagttggg tgcacgagtg ggttacatcg 4320
aactggatct caacagcggt aagatccttg agagttttcg ccccgaagaa cgttttccaa 4380
tgatgagcac ttttaaagtt ctgctatgtg gcgcggtatt atcccgtatt gacgccgggc 4440
aagagcaact cggtcgccgc atacactatt ctcagaatga cttggttgag tactcaccag 4500
tcacagaaaa gcatcttacg gatggcatga cagtaagaga attatgcagt gctgccataa 4560
ccatgagtga taacactgcg gccaacttac ttctgacaac gatcggagga ccgaaggagc 4620
taaccgcttt tttgcacaac atgggggatc atgtaactcg ccttgatcgt tgggaaccgg 4680
agctgaatga agccatacca aacgacgagc gtgacaccac gatgcctgta gcaatggcaa 4740
caacgttgcg caaactatta actggcgaac tacttactct agcttcccgg caacaattaa 4800
tagactggat ggaggcggat aaagttgcag gaccacttct gcgctcggcc cttccggctg 4860
gctggtttat tgctgataaa tctggagccg gtgagcgtgg gtctcgcggt atcattgcag 4920
cactggggcc agatggtaag ccctcccgta tcgtagttat ctacacgacg gggagtcagg 4980
caactatgga tgaacgaaat agacagatcg ctgagatagg tgcctcactg attaagcatt 5040
ggtaactgtc agaccaagtt tactcatata tactttagat tgatttaaaa cttcattttt 5100
aatttaaaag gatctaggtg aagatccttt ttgataatct catgaccaaa atcccttaac 5160
gtgagttttc gttccactga gcgtcagacc ccgtagaaaa gatcaaagga tcttcttgag 5220
atcctttttt tctgcgcgta atctgctgct tgcaaacaaa aaaaccaccg ctaccagcgg 5280
tggtttgttt gccggatcaa gagctaccaa ctctttttcc gaaggtaact ggcttcagca 5340
gagcgcagat accaaatact gttcttctag tgtagccgta gttaggccac cacttcaaga 5400
actctgtagc accgcctaca tacctcgctc tgctaatcct gttaccagtg gctgctgcca 5460
gtggcgataa gtcgtgtctt accgggttgg actcaagacg atagttaccg gataaggcgc 5520
agcggtcggg ctgaacgggg ggttcgtgca cacagcccag cttggagcga acgacctaca 5580
ccgaactgag atacctacag cgtgagctat gagaaagcgc cacgcttccc gaagggagaa 5640
aggcggacag gtatccggta agcggcaggg tcggaacagg agagcgcacg agggagcttc 5700
cagggggaaa cgcctggtat ctttatagtc ctgtcgggtt tcgccacctc tgacttgagc 5760
gtcgattttt gtgatgctcg tcaggggggc ggagcctatg gaaaaacgcc agcaacgcgg 5820
cctttttacg gttcctggcc ttttgctggc cttttgctca catggctcga cagatct 5877

<210> 165
<211> 5704
<212> DNA
<213> Homo sapiens
<220>
<221> misc_feature
<223> vecteur d'expression appelé GS-V17 contenant la séquence antisens
de GS-N17 ; antisens: 1108...1351

<400> 165
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgttga gaccccacca actgcaaaat ctgttcctgg 1140
cattaagctc cttcttcctt tgcaattcgg tctttcttca gcggtcccat gaatgctttc 1200
ttctcctcca tggtctggaa gtggccatgg ccaaacttgg aggtggtgtc aatgaactta 1260
aggtcaatct tctccagagc ccgccgcttc gtctgcacca gcaaggactt gcggagggtg 1320
agcacccgct tcttggttcc caccacacag cgtcgacccg ggcggccgct tccctttagt 1380
gagggttaat gcttcgagca gacatgataa gatacattga tgagtttgga caaaccacaa 1440
ctagaatgca gtgaaaaaaa tgctttattt gtgaaatttg tgatgctatt gctttatttg 1500
taaccattat aagctgcaat aaacaagtta acaacaacaa ttgcattcat tttatgtttc 1560
aggttcaggg ggagatgtgg gaggtttttt aaagcaagta aaacctctac aaatgtggta 1620
aaatccgata aggatcgatc cgggctggcg taatagcgaa gaggcccgca ccgatcgccc 1680
ttcccaacag ttgcgcagcc tgaatggcga atggacgcgc cctgtagcgg cgcattaagc 1740
gcggcgggtg tggtggttac gcgcagcgtg accgctacac ttgccagcgc cctagcgccc 1800
gctcctttcg ctttcttccc ttcctttctc gccacgttcg ccggctttcc ccgtcaagct 1860

ctaaatcggg ggctcccttt agggttccga tttagtgctt tacggcacct cgaccccaaa 1920
aaacttgatt agggtgatgg ttcacgtagt gggccatcgc cctgatagac ggtttttcgc 1980
cctttgacgt tggagtccac gttctttaat agtggactct tgttccaaac tggaacaaca 2040
ctcaacccta tctcggtcta ttcttttgat ttataaggga ttttgccgat ttcggcctat 2100
tggttaaaaa atgagctgat ttaacaaaaa tttaacgcga attttaacaa aatattaacg 2160
cttacaattt cctgatgcgg tattttctcc ttacgcatct gtgcggtatt tcacaccgca 2220
tacgcggatc tgcgcagcac catggcctga aataacctct gaaagaggaa cttggttagg 2280
taccttctga ggcggaaaga accagctgtg gaatgtgtgt cagttagggt gtggaaagtc 2340
cccaggctcc ccagcaggca gaagtatgca aagcatgcat ctcaattagt cagcaaccag 2400
gtgtggaaag tccccaggct ccccagcagg cagaagtatg caaagcatgc atctcaatta 2460
gtcagcaacc atagtcccgc ccctaactcc gcccatcccg cccctaactc cgcccagttc 2520
cgcccattct ccgccccatg gctgactaat tttttttatt tatgcagagg ccgaggccgc 2580
ctcggcctct gagctattcc agaagtagtg aggaggcttt tttggaggcc taggcttttg 2640
caaaaagctt gattcttctg acacaacagt ctcgaactta aggctagagc caccatgatt 2700
gaacaagatg gattgcacgc aggttctccg gccgcttggg tggagaggct attcggctat 2760
gactgggcac aacagacaat cggctgctct gatgccgccg tgttccggct gtcagcgcag 2820
gggcgcccgg ttctttttgt caagaccgac ctgtccggtg ccctgaatga actgcaggac 2880
gaggcagcgc ggctatcgtg gctggccacg acgggcgttc cttgcgcagc tgtgctcgac 2940
gttgtcactg aagcgggaag ggactggctg ctattgggcg aagtgccggg gcaggatctc 3000
ctgtcatctc accttgctcc tgccgagaaa gtatccatca tggctgatgc aatgcggcgg 3060
ctgcatacgc ttgatccggc tacctgccca ttcgaccacc aagcgaaaca tcgcatcgag 3120
cgagcacgta ctcggatgga agccggtctt gtcgatcagg atgatctgga cgaagagcat 3180
caggggctcg cgccagccga actgttcgcc aggctcaagg cgcgcatgcc cgacggcgag 3240
gatctcgtcg tgacccatgg cgatgcctgc ttgccgaata tcatggtgga aaatggccgc 3300
ttttctggat tcatcgactg tggccggctg ggtgtggcgg accgctatca ggacatagcg 3360
ttggctaccc gtgatattgc tgaagagctt ggcggcgaat gggctgaccg cttcctcgtg 3420
ctttacggta tcgccgctcc cgattcgcag cgcatcgcct tctatcgcct tcttgacgag 3480
ttcttctgag cgggactctg gggttcgaaa tgaccgacca agcgacgccc aacctgccat 3540
cacgatggcc gcaataaaat atctttattt tcattacatc tgtgtgttgg ttttttgtgt 3600
gaatcgatag cgataaggat ccgcgtatgg tgcactctca gtacaatctg ctctgatgcc 3660
gcatagttaa gccagccccg acacccgcca acacccgctg acgcgccctg acgggcttgt 3720
ctgctcccgg catccgctta cagacaagct gtgaccgtct ccgggagctg catgtgtcag 3780
aggttttcac cgtcatcacc gaaacgcgcg agacgaaagg gcctcgtgat acgcctattt 3840
ttataggtta atgtcatgat aataatggtt tcttagacgt caggtggcac ttttcgggga 3900
aatgtgcgcg gaacccctat ttgtttattt ttctaaatac attcaaatat gtatccgctc 3960
atgagacaat aaccctgata aatgcttcaa taatattgaa aaaggaagag tatgagtatt 4020
caacatttcc gtgtcgccct tattcccttt tttgcggcat tttgccttcc tgtttttgct 4080
cacccagaaa cgctggtgaa agtaaaagat gctgaagatc agttgggtgc acgagtgggt 4140
tacatcgaac tggatctcaa cagcggtaag atccttgaga gttttcgccc cgaagaacgt 4200
tttccaatga tgagcacttt taaagttctg ctatgtggcg cggtattatc ccgtattgac 4260
gccgggcaag agcaactcgg tcgccgcata cactattctc agaatgactt ggttgagtac 4320
tcaccagtca cagaaaagca tcttacggat ggcatgacag taagagaatt atgcagtgct 4380
gccataacca tgagtgataa cactgcggcc aacttacttc tgacaacgat cggaggaccg 4440
aaggagctaa ccgctttttt gcacaacatg ggggatcatg taactcgcct tgatcgttgg 4500
gaaccggagc tgaatgaagc cataccaaac gacgagcgtg acaccacgat gcctgtagca 4560
atggcaacaa cgttgcgcaa actattaact ggcgaactac ttactctagc ttcccggcaa 4620
caattaatag actggatgga ggcggataaa gttgcaggac cacttctgcg ctcggccctt 4680
ccggctggct ggtttattgc tgataaatct ggagccggtg agcgtgggtc tcgcggtatc 4740
attgcagcac tggggccaga tggtaagccc tcccgtatcg tagttatcta cacgacgggg 4800
agtcaggcaa ctatggatga acgaaataga cagatcgctg agataggtgc ctcactgatt 4860
aagcattggt aactgtcaga ccaagtttac tcatatatac tttagattga tttaaaactt 4920
catttttaat ttaaaaggat ctaggtgaag atcctttttg ataatctcat gaccaaaatc 4980
ccttaacgtg agttttcgtt ccactgagcg tcagaccccg tagaaaagat caaaggatct 5040
tcttgagatc ctttttttct gcgcgtaatc tgctgcttgc aaacaaaaaa accaccgcta 5100
ccagcggtgg tttgtttgcc ggatcaagag ctaccaactc tttttccgaa ggtaactggc 5160
ttcagcagag cgcagatacc aaatactgtt cttctagtgt agccgtagtt aggccaccac 5220
ttcaagaact ctgtagcacc gcctacatac ctcgctctgc taatcctgtt accagtggct 5280
gctgccagtg gcgataagtc gtgtcttacc gggttggact caagacgata gttaccggat 5340
aaggcgcagc ggtcgggctg aacggggggt tcgtgcacac agcccagctt ggagcgaacg 5400
acctacaccg aactgagata cctacagcgt gagctatgag aaagcgccac gcttcccgaa 5460
gggagaaagg cggacaggta tccggtaagc ggcagggtcg gaacaggaga gcgcacgagg 5520
gagcttccag ggggaaacgc ctggtatctt tatagtcctg tcgggtttcg ccacctctga 5580
cttgagcgtc gatttttgtg atgctcgtca ggggggcgga gcctatggaa aaacgccagc 5640
aacgcggcct ttttacggtt cctggccttt tgctggcctt ttgctcacat ggctcgacag 5700
atct 5704

<210> 166
<211> 5771
<212> DNA
<213> Homo sapiens
<220>
<221> misc_feature
<223> vecteur d'expression appelé GS-V18 contenant la séquence antisens

de GS-N18 ; antisens: 1108...1418

<400> 166
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtgct ggtgggttcc tctggtgcca tgaagcaggg 1140
tccgagcctc acccaagtgt gctttcaact gtaggctctc attatacaag acggagaact 1200
gtgactgcat gcagcgatat tctggagttt ccttaacgac ttgctccact gcactccgca 1260
attccacctt cagcttttca ttttgtgtag tgacctcctc aaagtcttgc cgaagtttct 1320
ccagctcaca gagacggttc tgagccaact ctttgttctc ctcaagctct gcattcattt 1380
cctcaaactt ccgagcattg atagtgattg tgccgccagt cgacccgggc ggccgcttcc 1440
ctttagtgag ggttaatgct tcgagcagac atgataagat acattgatga gtttggacaa 1500
accacaacta gaatgcagtg aaaaaaatgc tttatttgtg aaatttgtga tgctattgct 1560
ttatttgtaa ccattataag ctgcaataaa caagttaaca acaacaattg cattcatttt 1620
atgtttcagg ttcaggggga gatgtgggag gttttttaaa gcaagtaaaa cctctacaaa 1680
tgtggtaaaa tccgataagg atcgatccgg gctggcgtaa tagcgaagag gcccgcaccg 1740
atcgcccttc ccaacagttg cgcagcctga atggcgaatg gacgcgccct gtagcggcgc 1800
attaagcgcg gcgggtgtgg tggttacgcg cagcgtgacc gctacacttg ccagcgccct 1860
agcgcccgct cctttcgctt tcttcccttc ctttctcgcc acgttcgccg gctttccccg 1920
tcaagctcta aatcgggggc tccctttagg gttccgattt agtgctttac ggcacctcga 1980
ccccaaaaaa cttgattagg gtgatggttc acgtagtggg ccatcgccct gatagacggt 2040
ttttcgccct ttgacgttgg agtccacgtt ctttaatagt ggactcttgt tccaaactgg 2100
aacaacactc aaccctatct cggtctattc ttttgattta taagggattt tgccgatttc 2160
ggcctattgg ttaaaaaatg agctgattta acaaaaattt aacgcgaatt ttaacaaaat 2220
attaacgctt acaatttcct gatgcggtat tttctcctta cgcatctgtg cggtatttca 2280
caccgcatac gcggatctgc gcagcaccat ggcctgaaat aacctctgaa agaggaactt 2340
ggttaggtac cttctgaggc ggaaagaacc agctgtggaa tgtgtgtcag ttagggtgtg 2400
gaaagtcccc aggctcccca gcaggcagaa gtatgcaaag catgcatctc aattagtcag 2460
caaccaggtg tggaaagtcc ccaggctccc cagcaggcag aagtatgcaa agcatgcatc 2520
tcaattagtc agcaaccata gtcccgcccc taactccgcc catcccgccc ctaactccgc 2580
ccagttccgc ccattctccg ccccatggct gactaatttt ttttatttat gcagaggccg 2640
aggccgcctc ggcctctgag ctattccaga agtagtgagg aggctttttt ggaggcctag 2700
gcttttgcaa aaagcttgat tcttctgaca caacagtctc gaacttaagg ctagagccac 2760
catgattgaa caagatggat tgcacgcagg ttctccggcc gcttgggtgg agaggctatt 2820
cggctatgac tgggcacaac agacaatcgg ctgctctgat gccgccgtgt tccggctgtc 2880
agcgcagggg cgcccggttc tttttgtcaa gaccgacctg tccggtgccc tgaatgaact 2940
gcaggacgag gcagcgcggc tatcgtggct ggccacgacg ggcgttcctt gcgcagctgt 3000
gctcgacgtt gtcactgaag cgggaaggga ctggctgcta ttgggcgaag tgccggggca 3060
ggatctcctg tcatctcacc ttgctcctgc cgagaaagta tccatcatgg ctgatgcaat 3120
gcggcggctg catacgcttg atccggctac ctgcccattc gaccaccaag cgaaacatcg 3180
catcgagcga gcacgtactc ggatggaagc cggtcttgtc gatcaggatg atctggacga 3240
agagcatcag gggctcgcgc cagccgaact gttcgccagg ctcaaggcgc gcatgcccga 3300
cggcgaggat ctcgtcgtga cccatggcga tgcctgcttg ccgaatatca tggtggaaaa 3360
tggccgcttt tctggattca tcgactgtgg ccggctgggt gtggcggacc gctatcagga 3420
catagcgttg gctacccgtg atattgctga agagcttggc ggcgaatggg ctgaccgctt 3480
cctcgtgctt tacggtatcg ccgctcccga ttcgcagcgc atcgccttct atcgccttct 3540
tgacgagttc ttctgagcgg gactctgggg ttcgaaatga ccgaccaagc gacgcccaac 3600
ctgccatcac gatggccgca ataaaatatc tttattttca ttacatctgt gtgttggttt 3660
tttgtgtgaa tcgatagcga taaggatccg cgtatggtgc actctcagta caatctgctc 3720
tgatgccgca tagttaagcc agccccgaca cccgccaaca cccgctgacg cgccctgacg 3780
ggcttgtctg ctcccggcat ccgcttacag acaagctgtg accgtctccg ggagctgcat 3840
gtgtcagagg ttttcaccgt catcaccgaa acgcgcgaga cgaaagggcc tcgtgatacg 3900
cctattttta taggttaatg tcatgataat aatggtttct tagacgtcag gtggcacttt 3960
tcggggaaat gtgcgcggaa cccctatttg tttatttttc taaatacatt caaatatgta 4020
tccgctcatg agacaataac cctgataaat gcttcaataa tattgaaaaa ggaagagtat 4080
gagtattcaa catttccgtg tcgcccttat tccctttttt gcggcatttt gccttcctgt 4140

ttttgctcac ccagaaacgc tggtgaaagt aaaagatgct gaagatcagt tgggtgcacg 4200
agtgggttac atcgaactgg atctcaacag cggtaagatc cttgagagtt ttcgccccga 4260
agaacgtttt ccaatgatga gcacttttaa agttctgcta tgtggcgcgg tattatcccg 4320
tattgacgcc gggcaagagc aactcggtcg ccgcatacac tattctcaga atgacttggt 4380
tgagtactca ccagtcacag aaaagcatct tacggatggc atgacagtaa gagaattatg 4440
cagtgctgcc ataaccatga gtgataacac tgcggccaac ttacttctga caacgatcgg 4500
aggaccgaag gagctaaccg cttttttgca caacatgggg gatcatgtaa ctcgccttga 4560
tcgttgggaa ccggagctga atgaagccat accaaacgac gagcgtgaca ccacgatgcc 4620
tgtagcaatg gcaacaacgt tgcgcaaact attaactggc gaactactta ctctagcttc 4680
ccggcaacaa ttaatagact ggatggaggc ggataaagtt gcaggaccac ttctgcgctc 4740
ggcccttccg gctggctggt ttattgctga taaatctgga gccggtgagc gtgggtctcg 4800
cggtatcatt gcagcactgg ggccagatgg taagccctcc cgtatcgtag ttatctacac 4860
gacggggagt caggcaacta tggatgaacg aaatagacag atcgctgaga taggtgcctc 4920
actgattaag cattggtaac tgtcagacca agtttactca tatatacttt agattgattt 4980
aaaacttcat ttttaattta aaaggatcta ggtgaagatc ctttttgata atctcatgac 5040
caaaatccct taacgtgagt tttcgttcca ctgagcgtca gaccccgtag aaaagatcaa 5100
aggatcttct tgagatcctt tttttctgcg cgtaatctgc tgcttgcaaa caaaaaaacc 5160
accgctacca gcggtggttt gtttgccgga tcaagagcta ccaactcttt ttccgaaggt 5220
aactggcttc agcagagcgc agataccaaa tactgttctt ctagtgtagc cgtagttagg 5280
ccaccacttc aagaactctg tagcaccgcc tacatacctc gctctgctaa tcctgttacc 5340
agtggctgct gccagtggcg ataagtcgtg tcttaccggg ttggactcaa gacgatagtt 5400
accggataag gcgcagcggt cgggctgaac ggggggttcg tgcacacagc ccagcttgga 5460
gcgaacgacc tacaccgaac tgagatacct acagcgtgag ctatgagaaa gcgccacgct 5520
tcccgaaggg agaaaggcgg acaggtatcc ggtaagcggc agggtcggaa caggagagcg 5580
cacgagggag cttccagggg gaaacgcctg gtatctttat agtcctgtcg ggtttcgcca 5640
cctctgactt gagcgtcgat ttttgtgatg ctcgtcaggg gggcggagcc tatggaaaaa 5700
cgccagcaac gcggcctttt tacggttcct ggccttttgc tggccttttg ctcacatggc 5760
tcgacagatc t 5771

<210> 167
<211> 5706
<212> DNA
<213> Homo sapiens
<220>
<221> misc_feature
<223> vecteur d'expression appelé GS-V19 contenant la séquence antisens
de GS-N19 ; antisens: 1108...1353

<400> 167
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtgct cttacgggaa tgtctgcttc gagagcgaga 1140
ccttgaatga ctccggcttc tggagtagga ccggcgtcgt ctggaacctg gcttgtcttc 1200
aactaatctg atttttctcc cattgacttc agttccatcc aacttttcca aagctctttt 1260
catatcagaa taagatacaa attcaatcac cccttcattt ttgcgtccct tgtgagcatc 1320
tgcataagtc acttctcctg cctgacgcat atagtcgacc cgggcggccg cttcccttta 1380
gtgagggtta atgcttcgag cagacatgat aagatacatt gatgagtttg gacaaaccac 1440
aactagaatg cagtgaaaaa aatgctttat ttgtgaaatt tgtgatgcta ttgctttatt 1500
tgtaaccatt ataagctgca ataaacaagt taacaacaac aattgcattc attttatgtt 1560
tcaggttcag ggggagatgt gggaggtttt ttaaagcaag taaaacctct acaaatgtgg 1620
taaaatccga taaggatcga tccgggctgg cgtaatagcg aagaggcccg caccgatcgc 1680
ccttcccaac agttgcgcag cctgaatggc gaatggacgc gccctgtagc ggcgcattaa 1740
gcgcggcggg tgtggtggtt acgcgcagcg tgaccgctac acttgccagc gccctagcgc 1800
ccgctccttt cgctttcttc ccttcctttc tcgccacgtt cgccggcttt ccccgtcaag 1860
ctctaaatcg ggggctccct ttagggttcc gatttagtgc tttacggcac ctcgacccca 1920
aaaaacttga ttagggtgat ggttcacgta gtgggccatc gccctgatag acggtttttc 1980

gccctttgac gttggagtcc acgttcttta atagtggact cttgttccaa actggaacaa 2040
cactcaaccc tatctcggtc tattcttttg atttataagg gattttgccg atttcggcct 2100
attggttaaa aaatgagctg atttaacaaa aatttaacgc gaattttaac aaaatattaa 2160
cgcttacaat ttcctgatgc ggtattttct ccttacgcat ctgtgcggta tttcacaccg 2220
catacgcgga tctgcgcagc accatggcct gaaataacct ctgaaagagg aacttggtta 2280
ggtaccttct gaggcggaaa gaaccagctg tggaatgtgt gtcagttagg gtgtggaaag 2340
tccccaggct ccccagcagg cagaagtatg caaagcatgc atctcaatta gtcagcaacc 2400
aggtgtggaa agtccccagg ctccccagca ggcagaagta tgcaaagcat gcatctcaat 2460
tagtcagcaa ccatagtccc gcccctaact ccgcccatcc cgcccctaac tccgcccagt 2520
tccgcccatt ctccgcccca tggctgacta atttttttta tttatgcaga ggccgaggcc 2580
gcctcggcct ctgagctatt ccagaagtag tgaggaggct tttttggagg cctaggcttt 2640
tgcaaaaagc ttgattcttc tgacacaaca gtctcgaact taaggctaga gccaccatga 2700
ttgaacaaga tggattgcac gcaggttctc cggccgcttg ggtggagagg ctattcggct 2760
atgactgggc acaacagaca atcggctgct ctgatgccgc cgtgttccgg ctgtcagcgc 2820
aggggcgccc ggttcttttt gtcaagaccg acctgtccgg tgccctgaat gaactgcagg 2880
acgaggcagc gcggctatcg tggctggcca cgacgggcgt tccttgcgca gctgtgctcg 2940
acgttgtcac tgaagcggga agggactggc tgctattggg cgaagtgccg gggcaggatc 3000
tcctgtcatc tcaccttgct cctgccgaga aagtatccat catggctgat gcaatgcggc 3060
ggctgcatac gcttgatccg gctacctgcc cattcgacca ccaagcgaaa catcgcatcg 3120
agcgagcacg tactcggatg gaagccggtc ttgtcgatca ggatgatctg gacgaagagc 3180
atcaggggct cgcgccagcc gaactgttcg ccaggctcaa ggcgcgcatg cccgacggcg 3240
aggatctcgt cgtgacccat ggcgatgcct gcttgccgaa tatcatggtg gaaaatggcc 3300
gcttttctgg attcatcgac tgtggccggc tgggtgtggc ggaccgctat caggacatag 3360
cgttggctac ccgtgatatt gctgaagagc ttggcggcga atgggctgac cgcttcctcg 3420
tgctttacgg tatcgccgct cccgattcgc agcgcatcgc cttctatcgc cttcttgacg 3480
agttcttctg agcgggactc tggggttcga aatgaccgac caagcgacgc ccaacctgcc 3540
atcacgatgg ccgcaataaa atatctttat tttcattaca tctgtgtgtt ggttttttgt 3600
gtgaatcgat agcgataagg atccgcgtat ggtgcactct cagtacaatc tgctctgatg 3660
ccgcatagtt aagccagccc cgacacccgc caacacccgc tgacgcgccc tgacgggctt 3720
gtctgctccc ggcatccgct tacagacaag ctgtgaccgt ctccgggagc tgcatgtgtc 3780
agaggttttc accgtcatca ccgaaacgcg cgagacgaaa gggcctcgtg atacgcctat 3840
ttttataggt taatgtcatg ataataatgg tttcttagac gtcaggtggc acttttcggg 3900
gaaatgtgcg cggaacccct atttgtttat ttttctaaat acattcaaat atgtatccgc 3960
tcatgagaca ataaccctga taaatgcttc aataatattg aaaaaggaag agtatgagta 4020
ttcaacattt ccgtgtcgcc cttattccct tttttgcggc attttgcctt cctgtttttg 4080
ctcacccaga aacgctggtg aaagtaaaag atgctgaaga tcagttgggt gcacgagtgg 4140
gttacatcga actggatctc aacagcggta agatccttga gagttttcgc cccgaagaac 4200
gttttccaat gatgagcact tttaaagttc tgctatgtgg cgcggtatta tcccgtattg 4260
acgccgggca agagcaactc ggtcgccgca tacactattc tcagaatgac ttggttgagt 4320
actcaccagt cacagaaaag catcttacgg atggcatgac agtaagagaa ttatgcagtg 4380
ctgccataac catgagtgat aacactgcgg ccaacttact tctgacaacg atcggaggac 4440
cgaaggagct aaccgctttt ttgcacaaca tgggggatca tgtaactcgc cttgatcgtt 4500
gggaaccgga gctgaatgaa gccataccaa acgacgagcg tgacaccacg atgcctgtag 4560
caatggcaac aacgttgcgc aaactattaa ctggcgaact acttactcta gcttcccggc 4620
aacaattaat agactggatg gaggcggata aagttgcagg accacttctg cgctcggccc 4680
ttccggctgg ctggtttatt gctgataaat ctggagccgg tgagcgtggg tctcgcggta 4740
tcattgcagc actggggcca gatggtaagc cctcccgtat cgtagttatc tacacgacgg 4800
ggagtcaggc aactatggat gaacgaaata gacagatcgc tgagataggt gcctcactga 4860
ttaagcattg gtaactgtca gaccaagttt actcatatat actttagatt gatttaaaac 4920
ttcattttta atttaaaagg atctaggtga agatcctttt tgataatctc atgaccaaaa 4980
tcccttaacg tgagttttcg ttccactgag cgtcagaccc cgtagaaaag atcaaaggat 5040
cttcttgaga tccttttttt ctgcgcgtaa tctgctgctt gcaaacaaaa aaaccaccgc 5100
taccagcggt ggtttgtttg ccggatcaag agctaccaac tctttttccg aaggtaactg 5160
gcttcagcag agcgcagata ccaaatactg ttcttctagt gtagccgtag ttaggccacc 5220
acttcaagaa ctctgtagca ccgcctacat acctcgctct gctaatcctg ttaccagtgg 5280
ctgctgccag tggcgataag tcgtgtctta ccgggttgga ctcaagacga tagttaccgg 5340
ataaggcgca gcggtcgggc tgaacggggg gttcgtgcac acagcccagc ttggagcgaa 5400
cgacctacac cgaactgaga tacctacagc gtgagctatg agaaagcgcc acgcttcccg 5460
aagggagaaa ggcggacagg tatccggtaa gcggcagggt cggaacagga gagcgcacga 5520
gggagcttcc agggggaaac gcctggtatc tttatagtcc tgtcgggttt cgccacctct 5580
gacttgagcg tcgatttttg tgatgctcgt caggggggcg gagcctatgg aaaaacgcca 5640
gcaacgcggc ctttttacgg ttcctggcct tttgctggcc ttttgctcac atggctcgac 5700
agatct 5706

<210> 168
<211> 5663
<212> DNA
<213> Homo sapiens
<220>
<221> misc_feature
<223> vecteur d'expression appelé GS-V20 contenant la séquence antisens
de GS-N20 ; antisens: 1108...1310

<400> 168
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtcag atcttgtaga tggctgggtc atctgaaaac 1140
catgtgtgca acatagaaaa aggttccgat ttaggcaata ttaaagaatc aggcattcct 1200
agaataagct agtgcttcac agatataagc agcagcacct gtgggttttt accaagttca 1260
aagatagaca acatgagttt tagcttttgc tagatctctc tcccagctcc gtcgacccgg 1320
gcggccgctt ccctttagtg agggttaatg cttcgagcag acatgataag atacattgat 1380
gagtttggac aaaccacaac tagaatgcag tgaaaaaaat gctttatttg tgaaatttgt 1440
gatgctattg ctttatttgt aaccattata agctgcaata aacaagttaa caacaacaat 1500
tgcattcatt ttatgtttca ggttcagggg gagatgtggg aggtttttta aagcaagtaa 1560
aacctctaca aatgtggtaa aatccgataa ggatcgatcc gggctggcgt aatagcgaag 1620
aggcccgcac cgatcgccct tcccaacagt tgcgcagcct gaatggcgaa tggacgcgcc 1680
ctgtagcggc gcattaagcg cggcgggtgt ggtggttacg cgcagcgtga ccgctacact 1740
tgccagcgcc ctagcgcccg ctcctttcgc tttcttccct tcctttctcg ccacgttcgc 1800
cggctttccc cgtcaagctc taaatcgggg gctcccttta gggttccgat ttagtgcttt 1860
acggcacctc gaccccaaaa aacttgatta gggtgatggt tcacgtagtg ggccatcgcc 1920
ctgatagacg gtttttcgcc ctttgacgtt ggagtccacg ttctttaata gtggactctt 1980
gttccaaact ggaacaacac tcaaccctat ctcggtctat tcttttgatt tataagggat 2040
tttgccgatt tcggcctatt ggttaaaaaa tgagctgatt taacaaaaat ttaacgcgaa 2100
ttttaacaaa atattaacgc ttacaatttc ctgatgcggt attttctcct tacgcatctg 2160
tgcggtattt cacaccgcat acgcggatct gcgcagcacc atggcctgaa ataacctctg 2220
aaagaggaac ttggttaggt accttctgag gcggaaagaa ccagctgtgg aatgtgtgtc 2280
agttagggtg tggaaagtcc ccaggctccc cagcaggcag aagtatgcaa agcatgcatc 2340
tcaattagtc agcaaccagg tgtggaaagt ccccaggctc cccagcaggc agaagtatgc 2400
aaagcatgca tctcaattag tcagcaacca tagtcccgcc cctaactccg cccatcccgc 2460
ccctaactcc gcccagttcc gcccattctc cgccccatgg ctgactaatt ttttttattt 2520
atgcagaggc cgaggccgcc tcggcctctg agctattcca gaagtagtga ggaggctttt 2580
ttggaggcct aggcttttgc aaaaagcttg attcttctga cacaacagtc tcgaacttaa 2640
ggctagagcc accatgattg aacaagatgg attgcacgca ggttctccgg ccgcttgggt 2700
ggagaggcta ttcggctatg actgggcaca acagacaatc ggctgctctg atgccgccgt 2760
gttccggctg tcagcgcagg ggcgcccggt tctttttgtc aagaccgacc tgtccggtgc 2820
cctgaatgaa ctgcaggacg aggcagcgcg gctatcgtgg ctggccacga cgggcgttcc 2880
ttgcgcagct gtgctcgacg ttgtcactga agcgggaagg gactggctgc tattgggcga 2940
agtgccgggg caggatctcc tgtcatctca ccttgctcct gccgagaaag tatccatcat 3000
ggctgatgca atgcggcggc tgcatacgct tgatccggct acctgcccat tcgaccacca 3060
agcgaaacat cgcatcgagc gagcacgtac tcggatggaa gccggtcttg tcgatcagga 3120
tgatctggac gaagagcatc aggggctcgc gccagccgaa ctgttcgcca ggctcaaggc 3180
gcgcatgccc gacggcgagg atctcgtcgt gacccatggc gatgcctgct tgccgaatat 3240
catggtggaa aatggccgct tttctggatt catcgactgt ggccggctgg gtgtggcgga 3300
ccgctatcag gacatagcgt tggctacccg tgatattgct gaagagcttg gcggcgaatg 3360
ggctgaccgc ttcctcgtgc tttacggtat cgccgctccc gattcgcagc gcatcgcctt 3420
ctatcgcctt cttgacgagt tcttctgagc gggactctgg ggttcgaaat gaccgaccaa 3480
gcgacgccca acctgccatc acgatggccg caataaaata tctttatttt cattacatct 3540
gtgtgttggt tttttgtgtg aatcgatagc gataaggatc cgcgtatggt gcactctcag 3600
tacaatctgc tctgatgccg catagttaag ccagccccga cacccgccaa cacccgctga 3660
cgcgccctga cgggcttgtc tgctcccggc atccgcttac agacaagctg tgaccgtctc 3720
cgggagctgc atgtgtcaga ggttttcacc gtcatcaccg aaacgcgcga gacgaaaggg 3780
cctcgtgata cgcctatttt tataggttaa tgtcatgata ataatggttt cttagacgtc 3840
aggtggcact tttcggggaa atgtgcgcgg aacccctatt tgtttatttt tctaaataca 3900
ttcaaatatg tatccgctca tgagacaata accctgataa atgcttcaat aatattgaaa 3960
aaggaagagt atgagtattc aacatttccg tgtcgccctt attccctttt ttgcggcatt 4020
ttgccttcct gtttttgctc acccagaaac gctggtgaaa gtaaaagatg ctgaagatca 4080
gttgggtgca cgagtgggtt acatcgaact ggatctcaac agcggtaaga tccttgagag 4140
ttttcgcccc gaagaacgtt ttccaatgat gagcactttt aaagttctgc tatgtggcgc 4200
ggtattatcc cgtattgacg ccgggcaaga gcaactcggt cgccgcatac actattctca 4260

gaatgacttg gttgagtact caccagtcac agaaaagcat cttacggatg gcatgacagt 4320
aagagaatta tgcagtgctg ccataaccat gagtgataac actgcggcca acttacttct 4380
gacaacgatc ggaggaccga aggagctaac cgcttttttg cacaacatgg gggatcatgt 4440
aactcgcctt gatcgttggg aaccggagct gaatgaagcc ataccaaacg acgagcgtga 4500
caccacgatg cctgtagcaa tggcaacaac gttgcgcaaa ctattaactg gcgaactact 4560
tactctagct tcccggcaac aattaataga ctggatggag gcggataaag ttgcaggacc 4620
acttctgcgc tcggcccttc cggctggctg gtttattgct gataaatctg gagccggtga 4680
gcgtgggtct cgcggtatca ttgcagcact ggggccagat ggtaagccct cccgtatcgt 4740
agttatctac acgacgggga gtcaggcaac tatggatgaa cgaaatagac agatcgctga 4800
gataggtgcc tcactgatta agcattggta actgtcagac caagtttact catatatact 4860
ttagattgat ttaaaacttc atttttaatt taaaaggatc taggtgaaga tcctttttga 4920
taatctcatg accaaaatcc cttaacgtga gttttcgttc cactgagcgt cagaccccgt 4980
agaaaagatc aaaggatctt cttgagatcc tttttttctg cgcgtaatct gctgcttgca 5040
aacaaaaaaa ccaccgctac cagcggtggt ttgtttgccg gatcaagagc taccaactct 5100
ttttccgaag gtaactggct tcagcagagc gcagatacca aatactgttc ttctagtgta 5160
gccgtagtta ggccaccact tcaagaactc tgtagcaccg cctacatacc tcgctctgct 5220
aatcctgtta ccagtggctg ctgccagtgg cgataagtcg tgtcttaccg ggttggactc 5280
aagacgatag ttaccggata aggcgcagcg gtcgggctga acggggggtt cgtgcacaca 5340
gcccagcttg gagcgaacga cctacaccga actgagatac ctacagcgtg agctatgaga 5400
aagcgccacg cttcccgaag ggagaaaggc ggacaggtat ccggtaagcg gcagggtcgg 5460
aacaggagag cgcacgaggg agcttccagg gggaaacgcc tggtatcttt atagtcctgt 5520
cgggtttcgc cacctctgac ttgagcgtcg atttttgtga tgctcgtcag gggggcggag 5580
cctatggaaa aacgccagca acgcggcctt tttacggttc ctggcctttt gctggccttt 5640
tgctcacatg gctcgacaga tct 5663

<210> 169
<211> 5713
<212> DNA
<213> Homo sapiens
<220>
<221> misc_feature
<223> vecteur d'expression appelé GS-V21 contenant la séquence antisens
de GS-N21 ; antisens: 1108...1360

<400> 169
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtttc cactgcaggc gggttatata ttgacagcaa 1140
atttctgcag ataagacagt gaataaactc tccactccat gttgattagg aatagttttt 1200
ctagttttaa atattagcca gacacagaaa aaggttggac tgtaaggcct gggtgcacag 1260
tcttgatgga accagttaat tatgtgcatt tcattgatcc ttggtttgag gtggggatac 1320
ttcttcactg ccttcataat tttcttgtgc tcgttggcca gtcgacccgg gcggccgctt 1380
ccctttagtg agggttaatg cttcgagcag acatgataag atacattgat gagtttggac 1440
aaaccacaac tagaatgcag tgaaaaaaat gctttatttg tgaaatttgt gatgctattg 1500
ctttatttgt aaccattata agctgcaata aacaagttaa caacaacaat tgcattcatt 1560
ttatgtttca ggttcagggg gagatgtggg aggtttttta aagcaagtaa aacctctaca 1620
aatgtggtaa aatccgataa ggatcgatcc gggctggcgt aatagcgaag aggcccgcac 1680
cgatcgccct tcccaacagt tgcgcagcct gaatggcgaa tggacgcgcc ctgtagcggc 1740
gcattaagcg cggcgggtgt ggtggttacg cgcagcgtga ccgctacact tgccagcgcc 1800
ctagcgcccg ctcctttcgc tttcttccct tcctttctcg ccacgttcgc cggctttccc 1860
cgtcaagctc taaatcgggg gctcccttta gggttccgat ttagtgcttt acggcacctc 1920
gaccccaaaa aacttgatta gggtgatggt tcacgtagtg ggccatcgcc ctgatagacg 1980
gtttttcgcc ctttgacgtt ggagtccacg ttctttaata gtggactctt gttccaaact 2040
ggaacaacac tcaaccctat ctcggtctat tcttttgatt tataagggat tttgccgatt 2100
tcggcctatt ggttaaaaaa tgagctgatt taacaaaaat ttaacgcgaa ttttaacaaa 2160
atattaacgc ttacaatttc ctgatgcggt attttctcct tacgcatctg tgcggtattt 2220

cacaccgcat acgcggatct gcgcagcacc atggcctgaa ataacctctg aaagaggaac 2280
ttggttaggt accttctgag gcggaaagaa ccagctgtgg aatgtgtgtc agttagggtg 2340
tggaaagtcc ccaggctccc cagcaggcag aagtatgcaa agcatgcatc tcaattagtc 2400
agcaaccagg tgtggaaagt ccccaggctc cccagcaggc agaagtatgc aaagcatgca 2460
tctcaattag tcagcaacca tagtcccgcc cctaactccg cccatcccgc ccctaactcc 2520
gcccagttcc gcccattctc cgccccatgg ctgactaatt ttttttattt atgcagaggc 2580
cgaggccgcc tcggcctctg agctattcca gaagtagtga ggaggctttt ttggaggcct 2640
aggcttttgc aaaaagcttg attcttctga cacaacagtc tcgaacttaa ggctagagcc 2700
accatgattg aacaagatgg attgcacgca ggttctccgg ccgcttgggt ggagaggcta 2760
ttcggctatg actgggcaca acagacaatc ggctgctctg atgccgccgt gttccggctg 2820
tcagcgcagg ggcgcccggt tctttttgtc aagaccgacc tgtccggtgc cctgaatgaa 2880
ctgcaggacg aggcagcgcg gctatcgtgg ctggccacga cgggcgttcc ttgcgcagct 2940
gtgctcgacg ttgtcactga agcgggaagg gactggctgc tattgggcga agtgccgggg 3000
caggatctcc tgtcatctca ccttgctcct gccgagaaag tatccatcat ggctgatgca 3060
atgcggcggc tgcatacgct tgatccggct acctgcccat tcgaccacca agcgaaacat 3120
cgcatcgagc gagcacgtac tcggatggaa gccggtcttg tcgatcagga tgatctggac 3180
gaagagcatc aggggctcgc gccagccgaa ctgttcgcca ggctcaaggc gcgcatgccc 3240
gacggcgagg atctcgtcgt gacccatggc gatgcctgct tgccgaatat catggtggaa 3300
aatggccgct tttctggatt catcgactgt ggccggctgg gtgtggcgga ccgctatcag 3360
gacatagcgt tggctacccg tgatattgct gaagagcttg gcggcgaatg ggctgaccgc 3420
ttcctcgtgc tttacggtat cgccgctccc gattcgcagc gcatcgcctt ctatcgcctt 3480
cttgacgagt tcttctgagc gggactctgg ggttcgaaat gaccgaccaa gcgacgccca 3540
acctgccatc acgatggccg caataaaata tctttatttt cattacatct gtgtgttggt 3600
tttttgtgtg aatcgatagc gataaggatc cgcgtatggt gcactctcag tacaatctgc 3660
tctgatgccg catagttaag ccagccccga cacccgccaa cacccgctga cgcgccctga 3720
cgggcttgtc tgctcccggc atccgcttac agacaagctg tgaccgtctc cgggagctgc 3780
atgtgtcaga ggttttcacc gtcatcaccg aaacgcgcga gacgaaaggg cctcgtgata 3840
cgcctatttt tataggttaa tgtcatgata ataatggttt cttagacgtc aggtggcact 3900
tttcggggaa atgtgcgcgg aacccctatt tgtttatttt tctaaataca ttcaaatatg 3960
tatccgctca tgagacaata accctgataa atgcttcaat aatattgaaa aaggaagagt 4020
atgagtattc aacatttccg tgtcgccctt attccctttt ttgcggcatt ttgccttcct 4080
gtttttgctc acccagaaac gctggtgaaa gtaaaagatg ctgaagatca gttgggtgca 4140
cgagtgggtt acatcgaact ggatctcaac agcggtaaga tccttgagag ttttcgcccc 4200
gaagaacgtt ttccaatgat gagcactttt aaagttctgc tatgtggcgc ggtattatcc 4260
cgtattgacg ccgggcaaga gcaactcggt cgccgcatac actattctca gaatgacttg 4320
gttgagtact caccagtcac agaaaagcat cttacggatg gcatgacagt aagagaatta 4380
tgcagtgctg ccataaccat gagtgataac actgcggcca acttacttct gacaacgatc 4440
ggaggaccga aggagctaac cgcttttttg cacaacatgg gggatcatgt aactcgcctt 4500
gatcgttggg aaccggagct gaatgaagcc ataccaaacg acgagcgtga caccacgatg 4560
cctgtagcaa tggcaacaac gttgcgcaaa ctattaactg gcgaactact tactctagct 4620
tcccggcaac aattaataga ctggatggag gcggataaag ttgcaggacc acttctgcgc 4680
tcggcccttc cggctggctg gtttattgct gataaatctg gagccggtga gcgtgggtct 4740
cgcggtatca ttgcagcact ggggccagat ggtaagccct cccgtatcgt agttatctac 4800
acgacgggga gtcaggcaac tatggatgaa cgaaatagac agatcgctga gataggtgcc 4860
tcactgatta agcattggta actgtcagac caagtttact catatatact ttagattgat 4920
ttaaaacttc atttttaatt taaaaggatc taggtgaaga tcctttttga taatctcatg 4980
accaaaatcc cttaacgtga gttttcgttc cactgagcgt cagaccccgt agaaaagatc 5040
aaaggatctt cttgagatcc tttttttctg cgcgtaatct gctgcttgca aacaaaaaaa 5100
ccaccgctac cagcggtggt ttgtttgccg gatcaagagc taccaactct ttttccgaag 5160
gtaactggct tcagcagagc gcagatacca aatactgttc ttctagtgta gccgtagtta 5220
ggccaccact tcaagaactc tgtagcaccg cctacatacc tcgctctgct aatcctgtta 5280
ccagtggctg ctgccagtgg cgataagtcg tgtcttaccg ggttggactc aagacgatag 5340
ttaccggata aggcgcagcg gtcgggctga acggggggtt cgtgcacaca gcccagcttg 5400
gagcgaacga cctacaccga actgagatac ctacagcgtg agctatgaga aagcgccacg 5460
cttcccgaag ggagaaaggc ggacaggtat ccggtaagcg gcagggtcgg aacaggagag 5520
cgcacgaggg agcttccagg gggaaacgcc tggtatcttt atagtcctgt cgggtttcgc 5580
cacctctgac ttgagcgtcg atttttgtga tgctcgtcag gggggcggag cctatggaaa 5640
aacgccagca acgcggcctt tttacggttc ctggcctttt gctggccttt tgctcacatg 5700
gctcgacaga tct 5713

<210> 170
<211> 5633
<212> DNA
<213> Homo sapiens
<220>
<221> misc_feature
<223> vecteur d'expression appelé GS-V22 contenant la séquence antisens
de GS-N22 ; antisens: 1108...1280

<400> 170
tcaatattgg ccattagcca tattattcat tggttatata gcataaatca atattggcta 60
ttggccattg catacgttgt atctatatca taatatgtac atttatattg gctcatgtcc 120

aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtgaa aggccgtcca tcaaagcctg gtacttgtca 1140
atcatagacc aacgggcata ttccaggatt aaaagccgta catctttggc caaagtctcg 1200
ggcttgatga ggatgttaaa gtaggtcttc ttcaactgct cagttatcat tgtaaagaca 1260
gctggtgtgg aattgaactc gtcgacccgg gcggccgctt ccctttagtg agggttaatg 1320
cttcgagcag acatgataag atacattgat gagtttggac aaaccacaac tagaatgcag 1380
tgaaaaaaat gctttatttg tgaaatttgt gatgctattg ctttatttgt aaccattata 1440
agctgcaata aacaagttaa caacaacaat tgcattcatt ttatgtttca ggttcagggg 1500
gagatgtggg aggtttttta aagcaagtaa aacctctaca aatgtggtaa aatccgataa 1560
ggatcgatcc gggctggcgt aatagcgaag aggcccgcac cgatcgccct tcccaacagt 1620
tgcgcagcct gaatggcgaa tggacgcgcc ctgtagcggc gcattaagcg cggcgggtgt 1680
ggtggttacg cgcagcgtga ccgctacact tgccagcgcc ctagcgcccg ctcctttcgc 1740
tttcttccct tcctttctcg ccacgttcgc cggctttccc cgtcaagctc taaatcgggg 1800
gctcccttta gggttccgat ttagtgcttt acggcacctc gaccccaaaa aacttgatta 1860
gggtgatggt tcacgtagtg ggccatcgcc ctgatagacg gtttttcgcc ctttgacgtt 1920
ggagtccacg ttctttaata gtggactctt gttccaaact ggaacaacac tcaaccctat 1980
ctcggtctat tcttttgatt tataagggat tttgccgatt tcggcctatt ggttaaaaaa 2040
tgagctgatt taacaaaaat ttaacgcgaa ttttaacaaa atattaacgc ttacaatttc 2100
ctgatgcggt attttctcct tacgcatctg tgcggtattt cacaccgcat acgcggatct 2160
gcgcagcacc atggcctgaa ataacctctg aaagaggaac ttggttaggt accttctgag 2220
gcggaaagaa ccagctgtgg aatgtgtgtc agttagggtg tggaaagtcc ccaggctccc 2280
cagcaggcag aagtatgcaa agcatgcatc tcaattagtc agcaaccagg tgtggaaagt 2340
ccccaggctc cccagcaggc agaagtatgc aaagcatgca tctcaattag tcagcaacca 2400
tagtcccgcc cctaactccg cccatcccgc ccctaactcc gcccagttcc gcccattctc 2460
cgccccatgg ctgactaatt ttttttattt atgcagaggc cgaggccgcc tcggcctctg 2520
agctattcca gaagtagtga ggaggctttt ttggaggcct aggcttttgc aaaaagcttg 2580
attcttctga cacaacagtc tcgaacttaa ggctagagcc accatgattg aacaagatgg 2640
attgcacgca ggttctccgg ccgcttgggt ggagaggcta ttcggctatg actgggcaca 2700
acagacaatc ggctgctctg atgccgccgt gttccggctg tcagcgcagg ggcgcccggt 2760
tctttttgtc aagaccgacc tgtccggtgc cctgaatgaa ctgcaggacg aggcagcgcg 2820
gctatcgtgg ctggccacga cgggcgttcc ttgcgcagct gtgctcgacg ttgtcactga 2880
agcgggaagg gactggctgc tattgggcga agtgccgggg caggatctcc tgtcatctca 2940
ccttgctcct gccgagaaag tatccatcat ggctgatgca atgcggcggc tgcatacgct 3000
tgatccggct acctgcccat tcgaccacca agcgaaacat cgcatcgagc gagcacgtac 3060
tcggatggaa gccggtcttg tcgatcagga tgatctggac gaagagcatc aggggctcgc 3120
gccagccgaa ctgttcgcca ggctcaaggc gcgcatgccc gacggcgagg atctcgtcgt 3180
gacccatggc gatgcctgct tgccgaatat catggtggaa aatggccgct tttctggatt 3240
catcgactgt ggccggctgg gtgtggcgga ccgctatcag gacatagcgt tggctacccg 3300
tgatattgct gaagagcttg gcggcgaatg ggctgaccgc ttcctcgtgc tttacggtat 3360
cgccgctccc gattcgcagc gcatcgcctt ctatcgcctt cttgacgagt tcttctgagc 3420
gggactctgg ggttcgaaat gaccgaccaa gcgacgccca acctgccatc acgatggccg 3480
caataaaata tctttatttt cattacatct gtgtgttggt tttttgtgtg aatcgatagc 3540
gataaggatc cgcgtatggt gcactctcag tacaatctgc tctgatgccg catagttaag 3600
ccagccccga cacccgccaa cacccgctga cgcgccctga cgggcttgtc tgctcccggc 3660
atccgcttac agacaagctg tgaccgtctc cgggagctgc atgtgtcaga ggttttcacc 3720
gtcatcaccg aaacgcgcga gacgaaaggg cctcgtgata cgcctatttt tataggttaa 3780
tgtcatgata ataatggttt cttagacgtc aggtggcact tttcggggaa atgtgcgcgg 3840
aacccctatt tgtttatttt tctaaataca ttcaaatatg tatccgctca tgagacaata 3900
accctgataa atgcttcaat aatattgaaa aaggaagagt atgagtattc aacatttccg 3960
tgtcgccctt attccctttt ttgcggcatt ttgccttcct gtttttgctc acccagaaac 4020
gctggtgaaa gtaaaagatg ctgaagatca gttgggtgca cgagtgggtt acatcgaact 4080
ggatctcaac agcggtaaga tccttgagag ttttcgcccc gaagaacgtt ttccaatgat 4140
gagcactttt aaagttctgc tatgtggcgc ggtattatcc cgtattgacg ccgggcaaga 4200
gcaactcggt cgccgcatac actattctca gaatgacttg gttgagtact caccagtcac 4260
agaaaagcat cttacggatg gcatgacagt aagagaatta tgcagtgctg ccataaccat 4320
gagtgataac actgcggcca acttacttct gacaacgatc ggaggaccga aggagctaac 4380
cgcttttttg cacaacatgg gggatcatgt aactcgcctt gatcgttggg aaccggagct 4440
gaatgaagcc ataccaaacg acgagcgtga caccacgatg cctgtagcaa tggcaacaac 4500

gttgcgcaaa ctattaactg gcgaactact tactctagct tcccggcaac aattaataga 4560
ctggatggag gcggataaag ttgcaggacc acttctgcgc tcggcccttc cggctggctg 4620
gtttattgct gataaatctg gagccggtga gcgtgggtct cgcggtatca ttgcagcact 4680
ggggccagat ggtaagccct cccgtatcgt agttatctac acgacgggga gtcaggcaac 4740
tatggatgaa cgaaatagac agatcgctga gataggtgcc tcactgatta agcattggta 4800
actgtcagac caagtttact catatatact ttagattgat ttaaaacttc atttttaatt 4860
taaaaggatc taggtgaaga tcctttttga taatctcatg accaaaatcc cttaacgtga 4920
gttttcgttc cactgagcgt cagaccccgt agaaaagatc aaaggatctt cttgagatcc 4980
tttttttctg cgcgtaatct gctgcttgca aacaaaaaaa ccaccgctac cagcggtggt 5040
ttgtttgccg gatcaagagc taccaactct ttttccgaag gtaactggct tcagcagagc 5100
gcagatacca aatactgttc ttctagtgta gccgtagtta ggccaccact tcaagaactc 5160
tgtagcaccg cctacatacc tcgctctgct aatcctgtta ccagtggctg ctgccagtgg 5220
cgataagtcg tgtcttaccg ggttggactc aagacgatag ttaccggata aggcgcagcg 5280
gtcgggctga acggggggtt cgtgcacaca gcccagcttg gagcgaacga cctacaccga 5340
actgagatac ctacagcgtg agctatgaga aagcgccacg cttcccgaag ggagaaaggc 5400
ggacaggtat ccggtaagcg gcagggtcgg aacaggagag cgcacgaggg agcttccagg 5460
gggaaacgcc tggtatcttt atagtcctgt cgggtttcgc cacctctgac ttgagcgtcg 5520
atttttgtga tgctcgtcag gggggcggag cctatggaaa aacgccagca acgcggcctt 5580
tttacggttc ctggcctttt gctggccttt tgctcacatg gctcgacaga tct 5633

<210> 171
<211> 5688
<212> DNA
<213> Homo sapiens
<220>
<221> misc_feature
<223> vecteur d'expression appelé GS-V23 contenant la séquence antisens
de GS-N23 ; antisens: 1108...1335

<220>
<221> misc_feature
<223> vecteur d'expression appelé GS-V23 contenant la séquence antisens
de GS-N47 ; antisens: 1108...1335

<220>
<221> misc_feature
<223> vecteur d'expression appelé GS-V23 contenant la séquence antisens
de GS-N48 ; antisens: 1108...1335

<400> 171
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtccg ggtggtcaag acagacagat cagatggtag 1140
ctctagaggc aactcaaact gttcctctgc tgagatagag gaagagacac tactatccac 1200
aggttctgtt gtaggcagct gctgagaaaa tacttcaaaa agacatgggt ctttttcacg 1260
attactgtca agacccaatc cttcaccaag attcaggagt tctgatgaag atgactctgg 1320
gctctcaccc aaagcgtcga cccgggcggc cgcttccctt tagtgagggt taatgcttcg 1380
agcagacatg ataagataca ttgatgagtt tggacaaacc acaactagaa tgcagtgaaa 1440
aaaatgcttt atttgtgaaa tttgtgatgc tattgcttta tttgtaacca ttataagctg 1500
caataaacaa gttaacaaca acaattgcat tcattttatg tttcaggttc agggggagat 1560
gtgggaggtt ttttaaagca agtaaaacct ctacaaatgt ggtaaaatcc gataaggatc 1620
gatccgggct ggcgtaatag cgaagaggcc cgcaccgatc gcccttccca acagttgcgc 1680
agcctgaatg gcgaatggac gcgccctgta gcggcgcatt aagcgcggcg ggtgtggtgg 1740
ttacgcgcag cgtgaccgct acacttgcca gcgccctagc gcccgctcct ttcgctttct 1800
tcccttcctt tctcgccacg ttcgccggct ttccccgtca agctctaaat cgggggctcc 1860
ctttagggtt ccgatttagt gctttacggc acctcgaccc caaaaaactt gattagggtg 1920

atggttcacg tagtgggcca tcgccctgat agacggtttt tcgccctttg acgttggagt 1980
ccacgttctt taatagtgga ctcttgttcc aaactggaac aacactcaac cctatctcgg 2040
tctattcttt tgatttataa gggattttgc cgatttcggc ctattggtta aaaaatgagc 2100
tgatttaaca aaaatttaac gcgaatttta acaaaatatt aacgcttaca atttcctgat 2160
gcggtatttt ctccttacgc atctgtgcgg tatttcacac cgcatacgcg gatctgcgca 2220
gcaccatggc ctgaaataac ctctgaaaga ggaacttggt taggtacctt ctgaggcgga 2280
aagaaccagc tgtggaatgt gtgtcagtta gggtgtggaa agtccccagg ctccccagca 2340
ggcagaagta tgcaaagcat gcatctcaat tagtcagcaa ccaggtgtgg aaagtcccca 2400
ggctccccag caggcagaag tatgcaaagc atgcatctca attagtcagc aaccatagtc 2460
ccgcccctaa ctccgcccat cccgccccta actccgccca gttccgccca ttctccgccc 2520
catggctgac taattttttt tatttatgca gaggccgagg ccgcctcggc ctctgagcta 2580
ttccagaagt agtgaggagg cttttttgga ggcctaggct tttgcaaaaa gcttgattct 2640
tctgacacaa cagtctcgaa cttaaggcta gagccaccat gattgaacaa gatggattgc 2700
acgcaggttc tccggccgct tgggtggaga ggctattcgg ctatgactgg gcacaacaga 2760
caatcggctg ctctgatgcc gccgtgttcc ggctgtcagc gcaggggcgc ccggttcttt 2820
ttgtcaagac cgacctgtcc ggtgccctga atgaactgca ggacgaggca gcgcggctat 2880
cgtggctggc cacgacgggc gttccttgcg cagctgtgct cgacgttgtc actgaagcgg 2940
gaagggactg gctgctattg ggcgaagtgc cggggcagga tctcctgtca tctcaccttg 3000
ctcctgccga gaaagtatcc atcatggctg atgcaatgcg gcggctgcat acgcttgatc 3060
cggctacctg cccattcgac caccaagcga aacatcgcat cgagcgagca cgtactcgga 3120
tggaagccgg tcttgtcgat caggatgatc tggacgaaga gcatcagggg ctcgcgccag 3180
ccgaactgtt cgccaggctc aaggcgcgca tgcccgacgg cgaggatctc gtcgtgaccc 3240
atggcgatgc ctgcttgccg aatatcatgg tggaaaatgg ccgcttttct ggattcatcg 3300
actgtggccg gctgggtgtg gcggaccgct atcaggacat agcgttggct acccgtgata 3360
ttgctgaaga gcttggcggc gaatgggctg accgcttcct cgtgctttac ggtatcgccg 3420
ctcccgattc gcagcgcatc gccttctatc gccttcttga cgagttcttc tgagcgggac 3480
tctggggttc gaaatgaccg accaagcgac gcccaacctg ccatcacgat ggccgcaata 3540
aaatatcttt attttcatta catctgtgtg ttggtttttt gtgtgaatcg atagcgataa 3600
ggatccgcgt atggtgcact ctcagtacaa tctgctctga tgccgcatag ttaagccagc 3660
cccgacaccc gccaacaccc gctgacgcgc cctgacgggc ttgtctgctc ccggcatccg 3720
cttacagaca agctgtgacc gtctccggga gctgcatgtg tcagaggttt tcaccgtcat 3780
caccgaaacg cgcgagacga aagggcctcg tgatacgcct atttttatag gttaatgtca 3840
tgataataat ggtttcttag acgtcaggtg gcacttttcg gggaaatgtg cgcggaaccc 3900
ctatttgttt atttttctaa atacattcaa atatgtatcc gctcatgaga caataaccct 3960
gataaatgct tcaataatat tgaaaaagga agagtatgag tattcaacat ttccgtgtcg 4020
cccttattcc cttttttgcg gcattttgcc ttcctgtttt tgctcaccca gaaacgctgg 4080
tgaaagtaaa agatgctgaa gatcagttgg gtgcacgagt gggttacatc gaactggatc 4140
tcaacagcgg taagatcctt gagagttttc gccccgaaga acgttttcca atgatgagca 4200
cttttaaagt tctgctatgt ggcgcggtat tatcccgtat tgacgccggg caagagcaac 4260
tcggtcgccg catacactat tctcagaatg acttggttga gtactcacca gtcacagaaa 4320
agcatcttac ggatggcatg acagtaagag aattatgcag tgctgccata accatgagtg 4380
ataacactgc ggccaactta cttctgacaa cgatcggagg accgaaggag ctaaccgctt 4440
ttttgcacaa catgggggat catgtaactc gccttgatcg ttgggaaccg gagctgaatg 4500
aagccatacc aaacgacgag cgtgacacca cgatgcctgt agcaatggca acaacgttgc 4560
gcaaactatt aactggcgaa ctacttactc tagcttcccg gcaacaatta atagactgga 4620
tggaggcgga taaagttgca ggaccacttc tgcgctcggc ccttccggct ggctggttta 4680
ttgctgataa atctggagcc ggtgagcgtg ggtctcgcgg tatcattgca gcactggggc 4740
cagatggtaa gccctcccgt atcgtagtta tctacacgac ggggagtcag gcaactatgg 4800
atgaacgaaa tagacagatc gctgagatag gtgcctcact gattaagcat tggtaactgt 4860
cagaccaagt ttactcatat atactttaga ttgatttaaa acttcatttt taatttaaaa 4920
ggatctaggt gaagatcctt tttgataatc tcatgaccaa aatcccttaa cgtgagtttt 4980
cgttccactg agcgtcagac cccgtagaaa agatcaaagg atcttcttga gatccttttt 5040
ttctgcgcgt aatctgctgc ttgcaaacaa aaaaaccacc gctaccagcg gtggtttgtt 5100
tgccggatca agagctacca actctttttc cgaaggtaac tggcttcagc agagcgcaga 5160
taccaaatac tgttcttcta gtgtagccgt agttaggcca ccacttcaag aactctgtag 5220
caccgcctac atacctcgct ctgctaatcc tgttaccagt ggctgctgcc agtggcgata 5280
agtcgtgtct taccgggttg gactcaagac gatagttacc ggataaggcg cagcggtcgg 5340
gctgaacggg gggttcgtgc acacagccca gcttggagcg aacgacctac accgaactga 5400
gatacctaca gcgtgagcta tgagaaagcg ccacgcttcc cgaagggaga aaggcggaca 5460
ggtatccggt aagcggcagg gtcggaacag gagagcgcac gagggagctt ccagggggaa 5520
acgcctggta tctttatagt cctgtcgggt ttcgccacct ctgacttgag cgtcgatttt 5580
tgtgatgctc gtcagggggg cggagcctat ggaaaaacgc cagcaacgcg gcctttttac 5640
ggttcctggc cttttgctgg ccttttgctc acatggctcg acagatct 5688

<210> 172
<211> 5841
<212> DNA
<213> Homo sapiens
<220>
<221> misc_feature
<223> vecteur d'expression appelé GS-V24 contenant la séquence antisens
de GS-N24 ; antisens: 1108...1488

<220>
<221> misc_feature
<223> vecteur d'expression appelé GS-V24 contenant la séquence antisens
       de GS-N49 ; antisens: 1108...1488

<400> 172
tcaatattgg ccattagcca tattattcat tggttatata gcataaatca atattggcta      60
ttggccattg catacgttgt atctatatca taatatgtac atttatattg gctcatgtcc     120
aatatgaccg ccatgttggc attgattatt gactagttat taatagtaat caattacggg     180
gtcattagtt catagcccat atatggagtt ccgcgttaca taacttacgg taaatggccc     240
gcctggctga ccgcccaacg acccccgccc attgacgtca ataatgacgt atgttcccat     300
agtaacgcca atagggactt tccattgacg tcaatgggtg gagtatttac ggtaaactgc     360
ccacttggca gtacatcaag tgtatcatat gccaagtccg ccccctattg acgtcaatga     420
cggtaaatgg cccgcctggc attatgccca gtacatgacc ttacgggact ttcctacttg     480
gcagtacatc tacgtattag tcatcgctat taccatggtg atgcggtttt ggcagtacac     540
caatgggcgt ggatagcggt ttgactcacg gggatttcca agtctccacc ccattgacgt     600
caatgggagt ttgttttggc accaaaatca acgggacttt ccaaaatgtc gtaacaactg     660
cgatcgcccg ccccgttgac gcaaatgggc ggtaggcgtg tacggtggga ggtctatata     720
agcagagctc gtttagtgaa ccgtcagatc actagaagct ttattgcggt agtttatcac     780
agttaaattg ctaacgcagt cagtgcttct gacacaacag tctcgaactt aagctgcagt     840
gactctctta aggtagcctt gcagaagttg gtcgtgaggc actgggcagg taagtatcaa     900
ggttacaaga caggtttaag gagaccaata gaaactgggc ttgtcgagac agagaagact     960
cttgcgtttc tgataggcac ctattggtct tactgacatc cactttgcct ttctctccac    1020
aggtgtccac tcccagttca attacagctc ttaaggctag agtacttaat acgactcact    1080
ataggctagc ctcgagaatt cacgcgtaaa atgacgctcc acctgctgct gatcaacaac    1140
tcgaaaagac agtgactgct tagttacttg ccgatcataa atcttatctt ccatggttcc    1200
ctgagctaag aacctatata cataaacagg cttagtttgt ccaaagcgat aaactctgaa    1260
tatactctgg atgtcataag atggattcca agaagcgtcg aatataatta ctcgattagc    1320
agctaccaga ttaattccta gagatcctgc tttagtagaa atgataaatg atcgtcctct    1380
cacattagtt tcatcattaa attcttcagc ccacttcttc cttgactgtg cagtagtgga    1440
accatctaaa cggtaatagt caatgtttcg aagccacttc ccctcaccgt cgacccgggc    1500
ggccgcttcc ctttagtgag ggttaatgct tcgagcagac atgataagat acattgatga    1560
gtttggacaa accacaacta gaatgcagtg aaaaaaatgc tttatttgtg aaatttgtga    1620
tgctattgct ttatttgtaa ccattataag ctgcaataaa caagttaaca acaacaattg    1680
cattcatttt atgtttcagg ttcaggggga gatgtgggag gttttttaaa gcaagtaaaa    1740
cctctacaaa tgtggtaaaa tccgataagg atcgatccgg gctggcgtaa tagcgaagag    1800
gcccgcaccg atcgcccttc ccaacagttg cgcagcctga atggcgaatg gacgcgccct    1860
gtagcggcgc attaagcgcg gcgggtgtgg tggttacgcg cagcgtgacc gctacacttg    1920
ccagcgccct agcgcccgct cctttcgctt tcttcccttc ctttctcgcc acgttcgccg    1980
gctttccccg tcaagctcta aatcgggggc tccctttagg gttccgattt agtgctttac    2040
ggcacctcga ccccaaaaaa cttgattagg gtgatggttc acgtagtggg ccatcgccct    2100
gatagacggt ttttcgccct ttgacgttgg agtccacgtt ctttaatagt ggactcttgt    2160
tccaaactgg aacaacactc aaccctatct cggtctattc ttttgattta taagggattt    2220
tgccgatttc ggcctattgg ttaaaaaatg agctgattta acaaaaattt aacgcgaatt    2280
ttaacaaaat attaacgctt acaatttcct gatgcggtat tttctcctta cgcatctgtg    2340
cggtatttca caccgcatac gcggatctgc gcagcaccat ggcctgaaat aacctctgaa    2400
agaggaactt ggttaggtac cttctgaggc ggaaagaacc agctgtggaa tgtgtgtcag    2460
ttagggtgtg gaaagtcccc aggctcccca gcaggcagaa gtatgcaaag catgcatctc    2520
aattagtcag caaccaggtg tggaaagtcc ccaggctccc cagcaggcag aagtatgcaa    2580
agcatgcatc tcaattagtc agcaaccata gtcccgcccc taactccgcc catcccgccc    2640
ctaactccgc ccagttccgc ccattctccg ccccatggct gactaatttt ttttatttat    2700
gcagaggccg aggccgcctc ggcctctgag ctattccaga agtagtgagg aggctttttt    2760
ggaggcctag gcttttgcaa aaagcttgat tcttctgaca caacagtctc gaacttaagg    2820
ctagagccac catgattgaa caagatggat tgcacgcagg ttctccggcc gcttgggtgg    2880
agaggctatt cggctatgac tgggcacaac agacaatcgg ctgctctgat gccgccgtgt    2940
tccggctgtc agcgcagggg cgcccggttc tttttgtcaa gaccgacctg tccggtgccc    3000
tgaatgaact gcaggacgag gcagcgcggc tatcgtggct ggccacgacg ggcgttcctt    3060
gcgcagctgt gctcgacgtt gtcactgaag cgggaaggga ctggctgcta ttgggcgaag    3120
tgccggggca ggatctcctg tcatctcacc ttgctcctgc cgagaaagta tccatcatgg    3180
ctgatgcaat gcggcggctg catacgcttg atccggctac ctgcccattc gaccaccaag    3240
cgaaacatcg catcgagcga gcacgtactc ggatggaagc cggtcttgtc gatcaggatg    3300
atctggacga agagcatcag gggctcgcgc cagccgaact gttcgccagg ctcaaggcgc    3360
gcatgcccga cggcgaggat ctcgtcgtga cccatggcga tgcctgcttg ccgaatatca    3420
tggtggaaaa tggccgcttt tctggattca tcgactgtgg ccggctgggt gtggcggacc    3480
gctatcagga catagcgttg gctacccgtg atattgctga agagcttggc ggcgaatggg    3540
ctgaccgctt cctcgtgctt tacggtatcg ccgctcccga ttcgcagcgc atcgccttct    3600
atcgccttct tgacgagttc ttctgagcgg gactctgggg ttcgaaatga ccgaccaagc    3660
gacgcccaac ctgccatcac gatggccgca ataaaatatc tttattttca ttacatctgt    3720
gtgttggttt tttgtgtgaa tcgatagcga taaggatccg cgtatggtgc actctcagta    3780
caatctgctc tgatgccgca tagttaagcc agccccgaca cccgccaaca cccgctgacg    3840
cgccctgacg ggcttgtctg ctcccggcat ccgcttacag acaagctgtg accgtctccg    3900
ggagctgcat gtgtcagagg ttttcaccgt catcaccgaa acgcgcgaga cgaaagggcc    3960

tcgtgatacg cctattttta taggttaatg tcatgataat aatggtttct tagacgtcag 4020
gtggcacttt tcggggaaat gtgcgcggaa cccctatttg tttatttttc taaatacatt 4080
caaatatgta tccgctcatg agacaataac cctgataaat gcttcaataa tattgaaaaa 4140
ggaagagtat gagtattcaa catttccgtg tcgcccttat tccctttttt gcggcatttt 4200
gccttcctgt ttttgctcac ccagaaacgc tggtgaaagt aaaagatgct gaagatcagt 4260
tgggtgcacg agtgggttac atcgaactgg atctcaacag cggtaagatc cttgagagtt 4320
ttcgccccga agaacgtttt ccaatgatga gcacttttaa agttctgcta tgtggcgcgg 4380
tattatcccg tattgacgcc gggcaagagc aactcggtcg ccgcatacac tattctcaga 4440
atgacttggt tgagtactca ccagtcacag aaaagcatct tacggatggc atgacagtaa 4500
gagaattatg cagtgctgcc ataaccatga gtgataacac tgcggccaac ttacttctga 4560
caacgatcgg aggaccgaag gagctaaccg cttttttgca caacatgggg gatcatgtaa 4620
ctcgccttga tcgttgggaa ccggagctga atgaagccat accaaacgac gagcgtgaca 4680
ccacgatgcc tgtagcaatg gcaacaacgt tgcgcaaact attaactggc gaactactta 4740
ctctagcttc ccggcaacaa ttaatagact ggatggaggc ggataaagtt gcaggaccac 4800
ttctgcgctc ggcccttccg gctggctggt ttattgctga taaatctgga gccggtgagc 4860
gtgggtctcg cggtatcatt gcagcactgg ggccagatgg taagccctcc cgtatcgtag 4920
ttatctacac gacggggagt caggcaacta tggatgaacg aaatagacag atcgctgaga 4980
taggtgcctc actgattaag cattggtaac tgtcagacca agtttactca tatatacttt 5040
agattgattt aaaacttcat ttttaattta aaaggatcta ggtgaagatc ctttttgata 5100
atctcatgac caaaatccct taacgtgagt tttcgttcca ctgagcgtca gaccccgtag 5160
aaaagatcaa aggatcttct tgagatcctt tttttctgcg cgtaatctgc tgcttgcaaa 5220
caaaaaaacc accgctacca gcggtggttt gtttgccgga tcaagagcta ccaactcttt 5280
ttccgaaggt aactggcttc agcagagcgc agataccaaa tactgttctt ctagtgtagc 5340
cgtagttagg ccaccacttc aagaactctg tagcaccgcc tacatacctc gctctgctaa 5400
tcctgttacc agtggctgct gccagtggcg ataagtcgtg tcttaccggg ttggactcaa 5460
gacgatagtt accggataag gcgcagcggt cgggctgaac ggggggttcg tgcacacagc 5520
ccagcttgga gcgaacgacc tacaccgaac tgagatacct acagcgtgag ctatgagaaa 5580
gcgccacgct tcccgaaggg agaaaggcgg acaggtatcc ggtaagcggc agggtcggaa 5640
caggagagcg cacgagggag cttccagggg gaaacgcctg gtatctttat agtcctgtcg 5700
ggtttcgcca cctctgactt gagcgtcgat ttttgtgatg ctcgtcaggg gggcggagcc 5760
tatggaaaaa cgccagcaac gcggcctttt tacggttcct ggccttttgc tggccttttg 5820
ctcacatggc tcgacagatc t 5841

<210> 173
<211> 5855
<212> DNA
<213> Homo sapiens
<220>
<221> misc_feature
<223> vecteur d'expression appelé GS-V25 contenant la séquence antisens
de GS-N25 ; antisens: 1108...1502

<220>
<221> misc_feature
<223> vecteur d'expression appelé GS-V25 contenant la séquence antisens
de GS-N50 ; antisens: 1108...1502


<400> 173
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtccc ttagacattg tcccaaacct tgttacttga 1140
gtattgtagc cttaccatga ttttttttaa caccgtatca tctccatact ttttatttac 1200
aaattatata tacacacaat aatacaattc cttcattcta aaacaatagt agaccccaaa 1260
caggtctaca ttaagtttct gtactagcag ttcactcaga tagcttcgtt tgtttgttgt 1320
tgtttccaca taaccgcact gatcatgcca tacagttaat ttttatttgt ttatgctacc 1380
ttctgagatt aacttaaggc tctagtttaa tgcaaatggt ttagtcttaa aaggagtctc 1440

acgtgaggct aaaatcacac accaataact ctcctttgaa gctgtttctc cttggtggat 1500
gggtcgaccc gggcggccgc ttccctttag tgagggttaa tgcttcgagc agacatgata 1560
agatacattg atgagtttgg acaaaccaca actagaatgc agtgaaaaaa atgctttatt 1620
tgtgaaattt gtgatgctat tgctttattt gtaaccatta taagctgcaa taaacaagtt 1680
aacaacaaca attgcattca ttttatgttt caggttcagg gggagatgtg ggaggttttt 1740
taaagcaagt aaaacctcta caaatgtggt aaaatccgat aaggatcgat ccgggctggc 1800
gtaatagcga agaggcccgc accgatcgcc cttcccaaca gttgcgcagc ctgaatggcg 1860
aatggacgcg ccctgtagcg gcgcattaag cgcggcgggt gtggtggtta cgcgcagcgt 1920
gaccgctaca cttgccagcg ccctagcgcc cgctcctttc gctttcttcc cttcctttct 1980
cgccacgttc gccggctttc cccgtcaagc tctaaatcgg gggctccctt tagggttccg 2040
atttagtgct ttacggcacc tcgaccccaa aaaacttgat tagggtgatg gttcacgtag 2100
tgggccatcg ccctgataga cggtttttcg ccctttgacg ttggagtcca cgttctttaa 2160
tagtggactc ttgttccaaa ctggaacaac actcaaccct atctcggtct attcttttga 2220
tttataaggg attttgccga tttcggccta ttggttaaaa aatgagctga tttaacaaaa 2280
atttaacgcg aattttaaca aaatattaac gcttacaatt tcctgatgcg gtattttctc 2340
cttacgcatc tgtgcggtat ttcacaccgc atacgcggat ctgcgcagca ccatggcctg 2400
aaataacctc tgaaagagga acttggttag gtaccttctg aggcggaaag aaccagctgt 2460
ggaatgtgtg tcagttaggg tgtggaaagt ccccaggctc cccagcaggc agaagtatgc 2520
aaagcatgca tctcaattag tcagcaacca ggtgtggaaa gtccccaggc tccccagcag 2580
gcagaagtat gcaaagcatg catctcaatt agtcagcaac catagtcccg cccctaactc 2640
cgcccatccc gcccctaact ccgcccagtt ccgcccattc tccgccccat ggctgactaa 2700
ttttttttat ttatgcagag gccgaggccg cctcggcctc tgagctattc cagaagtagt 2760
gaggaggctt ttttggaggc ctaggctttt gcaaaaagct tgattcttct gacacaacag 2820
tctcgaactt aaggctagag ccaccatgat tgaacaagat ggattgcacg caggttctcc 2880
ggccgcttgg gtggagaggc tattcggcta tgactgggca caacagacaa tcggctgctc 2940
tgatgccgcc gtgttccggc tgtcagcgca ggggcgcccg gttctttttg tcaagaccga 3000
cctgtccggt gccctgaatg aactgcagga cgaggcagcg cggctatcgt ggctggccac 3060
gacgggcgtt ccttgcgcag ctgtgctcga cgttgtcact gaagcgggaa gggactggct 3120
gctattgggc gaagtgccgg ggcaggatct cctgtcatct caccttgctc ctgccgagaa 3180
agtatccatc atggctgatg caatgcggcg gctgcatacg cttgatccgg ctacctgccc 3240
attcgaccac caagcgaaac atcgcatcga gcgagcacgt actcggatgg aagccggtct 3300
tgtcgatcag gatgatctgg acgaagagca tcaggggctc gcgccagccg aactgttcgc 3360
caggctcaag gcgcgcatgc ccgacggcga ggatctcgtc gtgacccatg gcgatgcctg 3420
cttgccgaat atcatggtgg aaaatggccg cttttctgga ttcatcgact gtggccggct 3480
gggtgtggcg gaccgctatc aggacatagc gttggctacc cgtgatattg ctgaagagct 3540
tggcggcgaa tgggctgacc gcttcctcgt gctttacggt atcgccgctc ccgattcgca 3600
gcgcatcgcc ttctatcgcc ttcttgacga gttcttctga gcgggactct ggggttcgaa 3660
atgaccgacc aagcgacgcc caacctgcca tcacgatggc cgcaataaaa tatctttatt 3720
ttcattacat ctgtgtgttg gttttttgtg tgaatcgata gcgataagga tccgcgtatg 3780
gtgcactctc agtacaatct gctctgatgc cgcatagtta agccagcccc gacacccgcc 3840
aacacccgct gacgcgccct gacgggcttg tctgctcccg gcatccgctt acagacaagc 3900
tgtgaccgtc tccgggagct gcatgtgtca gaggttttca ccgtcatcac cgaaacgcgc 3960
gagacgaaag ggcctcgtga tacgcctatt tttataggtt aatgtcatga taataatggt 4020
ttcttagacg tcaggtggca cttttcgggg aaatgtgcgc ggaaccccta tttgtttatt 4080
tttctaaata cattcaaata tgtatccgct catgagacaa taaccctgat aaatgcttca 4140
ataatattga aaaaggaaga gtatgagtat tcaacatttc cgtgtcgccc ttattccctt 4200
ttttgcggca ttttgccttc ctgtttttgc tcacccagaa acgctggtga aagtaaaaga 4260
tgctgaagat cagttgggtg cacgagtggg ttacatcgaa ctggatctca acagcggtaa 4320
gatccttgag agttttcgcc ccgaagaacg ttttccaatg atgagcactt ttaaagttct 4380
gctatgtggc gcggtattat cccgtattga cgccgggcaa gagcaactcg gtcgccgcat 4440
acactattct cagaatgact tggttgagta ctcaccagtc acagaaaagc atcttacgga 4500
tggcatgaca gtaagagaat tatgcagtgc tgccataacc atgagtgata acactgcggc 4560
caacttactt ctgacaacga tcggaggacc gaaggagcta accgcttttt tgcacaacat 4620
gggggatcat gtaactcgcc ttgatcgttg ggaaccggag ctgaatgaag ccataccaaa 4680
cgacgagcgt gacaccacga tgcctgtagc aatggcaaca acgttgcgca aactattaac 4740
tggcgaacta cttactctag cttcccggca acaattaata gactggatgg aggcggataa 4800
agttgcagga ccacttctgc gctcggccct tccggctggc tggtttattg ctgataaatc 4860
tggagccggt gagcgtgggt ctcgcggtat cattgcagca ctggggccag atggtaagcc 4920
ctcccgtatc gtagttatct acacgacggg gagtcaggca actatggatg aacgaaatag 4980
acagatcgct gagataggtg cctcactgat taagcattgg taactgtcag accaagttta 5040
ctcatatata ctttagattg atttaaaact tcatttttaa tttaaaagga tctaggtgaa 5100
gatccttttt gataatctca tgaccaaaat cccttaacgt gagttttcgt tccactgagc 5160
gtcagacccc gtagaaaaga tcaaaggatc ttcttgagat cctttttttc tgcgcgtaat 5220
ctgctgcttg caaacaaaaa aaccaccgct accagcggtg gtttgtttgc cggatcaaga 5280
gctaccaact ctttttccga aggtaactgg cttcagcaga gcgcagatac caaatactgt 5340
tcttctagtg tagccgtagt taggccacca cttcaagaac tctgtagcac cgcctacata 5400
cctcgctctg ctaatcctgt taccagtggc tgctgccagt ggcgataagt cgtgtcttac 5460
cgggttggac tcaagacgat agttaccgga taaggcgcag cggtcgggct gaacgggggg 5520
ttcgtgcaca cagcccagct tggagcgaac gacctacacc gaactgagat acctacagcg 5580
tgagctatga gaaagcgcca cgcttcccga agggagaaag gcggacaggt atccggtaag 5640
cggcagggtc ggaacaggag agcgcacgag ggagcttcca gggggaaacg cctggtatct 5700
ttatagtcct gtcgggtttc gccacctctg acttgagcgt cgatttttgt gatgctcgtc 5760
aggggggcgg agcctatgga aaaacgccag caacgcggcc tttttacggt tcctggcctt 5820

ttgctggcct tttgctcaca tggctcgaca gatct                              5855

<210>  174
<211>  5841
<212>  DNA
<213>  Homo sapiens
<220>
<221>  misc_feature
<223>  vecteur d'expression appelé GS-V26 contenant la séquence antisens
        de GS-N26 ; antisens: 1108...1488

<400>  174
tcaatattgg ccattagcca tattattcat tggttatata gcataaatca atattggcta      60
ttggccattg catacgttgt atctatatca taatatgtac atttatattg gctcatgtcc     120
aatatgaccg ccatgttggc attgattatt gactagttat taatagtaat caattacggg     180
gtcattagtt catagcccat atatggagtt ccgcgttaca taacttacgg taaatggccc     240
gcctggctga ccgcccaacg acccccgccc attgacgtca ataatgacgt atgttcccat     300
agtaacgcca atagggactt tccattgacg tcaatgggtg gagtatttac ggtaaactgc     360
ccacttggca gtacatcaag tgtatcatat gccaagtccg ccccctattg acgtcaatga     420
cggtaaatgg cccgcctggc attatgccca gtacatgacc ttacgggact ttcctacttg     480
gcagtacatc tacgtattag tcatcgctat taccatggtg atgcggtttt ggcagtacac     540
caatgggcgt ggatagcggt ttgactcacg gggatttcca agtctccacc ccattgacgt     600
caatgggagt ttgttttggc accaaaatca acgggacttt ccaaaatgtc gtaacaactg     660
cgatcgcccg ccccgttgac gcaaatgggc ggtaggcgtg tacggtggga ggtctatata     720
agcagagctc gtttagtgaa ccgtcagatc actagaagct ttattgcggt agtttatcac     780
agttaaattg ctaacgcagt cagtgcttct gacacaacag tctcgaactt aagctgcagt     840
gactctctta aggtagcctt gcagaagttg gtcgtgaggc actgggcagg taagtatcaa     900
ggttacaaga caggtttaag gagaccaata gaaactgggc ttgtcgagac agagaagact     960
cttgcgtttc tgataggcac ctattggtct tactgacatc cactttgcct ttctctccac    1020
aggtgtccac tcccagttca attacagctc ttaaggctag agtacttaat acgactcact    1080
ataggctagc ctcgagaatt cacgcgtttc cctgctgcca tcaatcaggg cttgaaataa    1140
gggcttgtta tgaggtatgg tctgtaagat attcccatcc ccagtcacat agccaatggc    1200
ccactgtccc aatcgagtgc aacttaaccg gaaaatatag cttccgggtt tggtgctata    1260
tttctgtagt cgtgcattaa cttcatcata tgtgagaaat gccatgtaac ctggatgtgt    1320
cacagctaag aaattccaat tccgcaaaat agagccccaa ggctgaaaca gcctggtaaa    1380
aatatcaaat tcaaaaactg aaatgtaatc attgcaagtt aaatcaattg ttgattttag    1440
agccattgct tccaggccag agctaatctg gtggacctca tgaaggcagt cgacccgggc    1500
ggccgcttcc ctttagtgag ggttaatgct tcgagcagac atgataagat acattgatga    1560
gtttggacaa accacaacta gaatgcagtg aaaaaaatgc tttatttgtg aaatttgtga    1620
tgctattgct ttatttgtaa ccattataag ctgcaataaa caagttaaca acaacaattg    1680
cattcatttt atgtttcagg ttcaggggga gatgtgggag gttttttaaa gcaagtaaaa    1740
cctctacaaa tgtggtaaaa tccgataagg atcgatccgg gctggcgtaa tagcgaagag    1800
gcccgcaccg atcgcccttc ccaacagttg cgcagcctga atggcgaatg gacgcgccct    1860
gtagcggcgc attaagcgcg gcgggtgtgg tggttacgcg cagcgtgacc gctacacttg    1920
ccagcgccct agcgcccgct cctttcgctt tcttcccttc ctttctcgcc acgttcgccg    1980
gctttccccg tcaagctcta aatcgggggc tccctttagg gttccgattt agtgctttac    2040
ggcacctcga ccccaaaaaa cttgattagg gtgatggttc acgtagtggg ccatcgccct    2100
gatagacggt ttttcgccct ttgacgttgg agtccacgtt ctttaatagt ggactcttgt    2160
tccaaactgg aacaacactc aaccctatct cggtctattc ttttgattta taagggattt    2220
tgccgatttc ggcctattgg ttaaaaaatg agctgattta acaaaaattt aacgcgaatt    2280
ttaacaaaat attaacgctt acaatttcct gatgcggtat tttctcctta cgcatctgtg    2340
cggtatttca caccgcatac gcggatctgc gcagcaccat ggcctgaaat aacctctgaa    2400
agaggaactt ggttaggtac cttctgaggc ggaaagaacc agctgtggaa tgtgtgtcag    2460
ttagggtgtg gaaagtcccc aggctcccca gcaggcagaa gtatgcaaag catgcatctc    2520
aattagtcag caaccaggtg tggaaagtcc ccaggctccc cagcaggcag aagtatgcaa    2580
agcatgcatc tcaattagtc agcaaccata gtcccgcccc taactccgcc catcccgccc    2640
ctaactccgc ccagttccgc ccattctccg ccccatggct gactaatttt ttttatttat    2700
gcagaggccg aggccgcctc ggcctctgag ctattccaga agtagtgagg aggctttttt    2760
ggaggcctag gcttttgcaa aaagcttgat tcttctgaca caacagtctc gaacttaagg    2820
ctagagccac catgattgaa caagatggat tgcacgcagg ttctccggcc gcttgggtgg    2880
agaggctatt cggctatgac tgggcacaac agacaatcgg ctgctctgat gccgccgtgt    2940
tccggctgtc agcgcagggg cgcccggttc tttttgtcaa gaccgacctg tccggtgccc    3000
tgaatgaact gcaggacgag gcagcgcggc tatcgtggct ggccacgacg ggcgttcctt    3060
gcgcagctgt gctcgacgtt gtcactgaag cgggaaggga ctggctgcta ttgggcgaag    3120
tgccggggca ggatctcctg tcatctcacc ttgctcctgc cgagaaagta tccatcatgg    3180
ctgatgcaat gcggcggctg catacgcttg atccggctac ctgcccattc gaccaccaag    3240
cgaaacatcg catcgagcga gcacgtactc ggatggaagc cggtcttgtc gatcaggatg    3300
atctggacga agagcatcag gggctcgcgc cagccgaact gttcgccagg ctcaaggcgc    3360
gcatgcccga cggcgaggat ctcgtcgtga cccatggcga tgcctgcttg ccgaatatca    3420
tggtggaaaa tggccgcttt tctggattca tcgactgtgg ccggctgggt gtggcggacc    3480
gctatcagga catagcgttg gctacccgtg atattgctga agagcttggc ggcgaatggg    3540
ctgaccgctt cctcgtgctt tacggtatcg ccgctcccga ttcgcagcgc atcgccttct    3600

atcgccttct tgacgagttc ttctgagcgg gactctgggg ttcgaaatga ccgaccaagc 3660
gacgcccaac ctgccatcac gatggccgca ataaaatatc tttattttca ttacatctgt 3720
gtgttggttt tttgtgtgaa tcgatagcga taaggatccg cgtatggtgc actctcagta 3780
caatctgctc tgatgccgca tagttaagcc agccccgaca cccgccaaca cccgctgacg 3840
cgccctgacg ggcttgtctg ctcccggcat ccgcttacag acaagctgtg accgtctccg 3900
ggagctgcat gtgtcagagg ttttcaccgt catcaccgaa acgcgcgaga cgaaagggcc 3960
tcgtgatacg cctattttta taggttaatg tcatgataat aatggtttct tagacgtcag 4020
gtggcacttt tcggggaaat gtgcgcggaa cccctatttg tttatttttc taaatacatt 4080
caaatatgta tccgctcatg agacaataac cctgataaat gcttcaataa tattgaaaaa 4140
ggaagagtat gagtattcaa catttccgtg tcgcccttat tccctttttt gcggcatttt 4200
gccttcctgt ttttgctcac ccagaaacgc tggtgaaagt aaaagatgct gaagatcagt 4260
tgggtgcacg agtgggttac atcgaactgg atctcaacag cggtaagatc cttgagagtt 4320
ttcgccccga agaacgtttt ccaatgatga gcacttttaa agttctgcta tgtggcgcgg 4380
tattatcccg tattgacgcc gggcaagagc aactcggtcg ccgcatacac tattctcaga 4440
atgacttggt tgagtactca ccagtcacag aaaagcatct tacggatggc atgacagtaa 4500
gagaattatg cagtgctgcc ataaccatga gtgataacac tgcggccaac ttacttctga 4560
caacgatcgg aggaccgaag gagctaaccg cttttttgca caacatgggg gatcatgtaa 4620
ctcgccttga tcgttgggaa ccggagctga atgaagccat accaaacgac gagcgtgaca 4680
ccacgatgcc tgtagcaatg gcaacaacgt tgcgcaaact attaactggc gaactactta 4740
ctctagcttc ccggcaacaa ttaatagact ggatggaggc ggataaagtt gcaggaccac 4800
ttctgcgctc ggcccttccg gctggctggt ttattgctga taaatctgga gccggtgagc 4860
gtgggtctcg cggtatcatt gcagcactgg ggccagatgg taagccctcc cgtatcgtag 4920
ttatctacac gacggggagt caggcaacta tggatgaacg aaatagacag atcgctgaga 4980
taggtgcctc actgattaag cattggtaac tgtcagacca agtttactca tatatacttt 5040
agattgattt aaaacttcat ttttaattta aaaggatcta ggtgaagatc ctttttgata 5100
atctcatgac caaaatccct taacgtgagt tttcgttcca ctgagcgtca gaccccgtag 5160
aaaagatcaa aggatcttct tgagatcctt tttttctgcg cgtaatctgc tgcttgcaaa 5220
caaaaaaacc accgctacca gcggtggttt gtttgccgga tcaagagcta ccaactcttt 5280
ttccgaaggt aactggcttc agcagagcgc agataccaaa tactgttctt ctagtgtagc 5340
cgtagttagg ccaccacttc aagaactctg tagcaccgcc tacatacctc gctctgctaa 5400
tcctgttacc agtggctgct gccagtggcg ataagtcgtg tcttaccggg ttggactcaa 5460
gacgatagtt accggataag gcgcagcggt cgggctgaac ggggggttcg tgcacacagc 5520
ccagcttgga gcgaacgacc tacaccgaac tgagatacct acagcgtgag ctatgagaaa 5580
gcgccacgct tcccgaaggg agaaaggcgg acaggtatcc ggtaagcggc agggtcggaa 5640
caggagagcg cacgagggag cttccagggg gaaacgcctg gtatctttat agtcctgtcg 5700
ggtttcgcca cctctgactt gagcgtcgat ttttgtgatg ctcgtcaggg gggcggagcc 5760
tatggaaaaa cgccagcaac gcggcctttt tacggttcct ggccttttgc tggccttttg 5820
ctcacatggc tcgacagatc t 5841

<210> 175
<211> 5758
<212> DNA
<213> Homo sapiens
<220>
<221> misc_feature
<223> vecteur d'expression appelé GS-V27 contenant la séquence antisens
de GS-N27 ; antisens: 1108...1405

<400> 175
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtcga gtgggcaggc ctgtatcttc tcagcaaagt 1140
aaggattgag tatagagagc tgtttgtctt aactgggctt ccctgaagaa tctgagccaa 1200
actggaagaa accagcctca tttccagtgt tgagatgtta gctgtacagt ggctgtacaa 1260
ctgcagagtt tatttataga attagaaata atttttaaaa ttttaaaagg ttttgtgtaa 1320
tcattaacca gagatgatat tcacaaattc tggtaaaaat ttgactctcc aactatcacc 1380

atatcaacag gaaacagggc catgcgtcga cccgggcggc cgcttccctt tagtgagggt 1440
taatgcttcg agcagacatg ataagataca ttgatgagtt tggacaaacc acaactagaa 1500
tgcagtgaaa aaaatgcttt atttgtgaaa tttgtgatgc tattgcttta tttgtaacca 1560
ttataagctg caataaacaa gttaacaaca acaattgcat tcattttatg tttcaggttc 1620
agggggagat gtgggaggtt ttttaaagca agtaaaacct ctacaaatgt ggtaaaatcc 1680
gataaggatc gatccgggct ggcgtaatag cgaagaggcc cgcaccgatc gcccttccca 1740
acagttgcgc agcctgaatg gcgaatggac gcgccctgta gcggcgcatt aagcgcggcg 1800
ggtgtggtgg ttacgcgcag cgtgaccgct acacttgcca gcgccctagc gcccgctcct 1860
ttcgctttct tcccttcctt tctcgccacg ttcgccggct ttccccgtca agctctaaat 1920
cgggggctcc ctttagggtt ccgatttagt gctttacggc acctcgaccc caaaaaactt 1980
gattagggtg atggttcacg tagtgggcca tcgccctgat agacggtttt tcgccctttg 2040
acgttggagt ccacgttctt taatagtgga ctcttgttcc aaactggaac aacactcaac 2100
cctatctcgg tctattcttt tgatttataa gggattttgc cgatttcggc ctattggtta 2160
aaaaatgagc tgatttaaca aaaatttaac gcgaatttta acaaaatatt aacgcttaca 2220
atttcctgat gcggtatttt ctccttacgc atctgtgcgg tatttcacac cgcatacgcg 2280
gatctgcgca gcaccatggc ctgaaataac ctctgaaaga ggaacttggt taggtacctt 2340
ctgaggcgga aagaaccagc tgtggaatgt gtgtcagtta gggtgtggaa agtccccagg 2400
ctccccagca ggcagaagta tgcaaagcat gcatctcaat tagtcagcaa ccaggtgtgg 2460
aaagtcccca ggctccccag caggcagaag tatgcaaagc atgcatctca attagtcagc 2520
aaccatagtc ccgcccctaa ctccgcccat cccgccccta actccgccca gttccgccca 2580
ttctccgccc catggctgac taattttttt tatttatgca gaggccgagg ccgcctcggc 2640
ctctgagcta ttccagaagt agtgaggagg cttttttgga ggcctaggct tttgcaaaaa 2700
gcttgattct tctgacacaa cagtctcgaa cttaaggcta gagccaccat gattgaacaa 2760
gatggattgc acgcaggttc tccggccgct tgggtggaga ggctattcgg ctatgactgg 2820
gcacaacaga caatcggctg ctctgatgcc gccgtgttcc ggctgtcagc gcaggggcgc 2880
ccggttcttt ttgtcaagac cgacctgtcc ggtgccctga atgaactgca ggacgaggca 2940
gcgcggctat cgtggctggc cacgacgggc gttccttgcg cagctgtgct cgacgttgtc 3000
actgaagcgg gaagggactg gctgctattg ggcgaagtgc cggggcagga tctcctgtca 3060
tctcaccttg ctcctgccga gaaagtatcc atcatggctg atgcaatgcg gcggctgcat 3120
acgcttgatc cggctacctg cccattcgac caccaagcga aacatcgcat cgagcgagca 3180
cgtactcgga tggaagccgg tcttgtcgat caggatgatc tggacgaaga gcatcagggg 3240
ctcgcgccag ccgaactgtt cgccaggctc aaggcgcgca tgcccgacgg cgaggatctc 3300
gtcgtgaccc atggcgatgc ctgcttgccg aatatcatgg tggaaaatgg ccgcttttct 3360
ggattcatcg actgtggccg gctgggtgtg gcggaccgct atcaggacat agcgttggct 3420
acccgtgata ttgctgaaga gcttggcggc gaatgggctg accgcttcct cgtgctttac 3480
ggtatcgccg ctcccgattc gcagcgcatc gccttctatc gccttcttga cgagttcttc 3540
tgagcgggac tctggggttc gaaatgaccg accaagcgac gcccaacctg ccatcacgat 3600
ggccgcaata aaatatcttt attttcatta catctgtgtg ttggtttttt gtgtgaatcg 3660
atagcgataa ggatccgcgt atggtgcact ctcagtacaa tctgctctga tgccgcatag 3720
ttaagccagc cccgacaccc gccaacaccc gctgacgcgc cctgacgggc ttgtctgctc 3780
ccggcatccg cttacagaca agctgtgacc gtctccggga gctgcatgtg tcagaggttt 3840
tcaccgtcat caccgaaacg cgcgagacga aagggcctcg tgatacgcct atttttatag 3900
gttaatgtca tgataataat ggtttcttag acgtcaggtg gcacttttcg gggaaatgtg 3960
cgcggaaccc ctatttgttt atttttctaa atacattcaa atatgtatcc gctcatgaga 4020
caataaccct gataaatgct tcaataatat tgaaaaagga agagtatgag tattcaacat 4080
ttccgtgtcg cccttattcc cttttttgcg gcattttgcc ttcctgtttt tgctcaccca 4140
gaaacgctgg tgaaagtaaa agatgctgaa gatcagttgg gtgcacgagt gggttacatc 4200
gaactggatc tcaacagcgg taagatcctt gagagttttc gccccgaaga acgttttcca 4260
atgatgagca cttttaaagt tctgctatgt ggcgcggtat tatcccgtat tgacgccggg 4320
caagagcaac tcggtcgccg catacactat tctcagaatg acttggttga gtactcacca 4380
gtcacagaaa agcatcttac ggatggcatg acagtaagag aattatgcag tgctgccata 4440
accatgagtg ataacactgc ggccaactta cttctgacaa cgatcggagg accgaaggag 4500
ctaaccgctt ttttgcacaa catgggggat catgtaactc gccttgatcg ttgggaaccg 4560
gagctgaatg aagccatacc aaacgacgag cgtgacacca cgatgcctgt agcaatggca 4620
acaacgttgc gcaaactatt aactggcgaa ctacttactc tagcttcccg gcaacaatta 4680
atagactgga tggaggcgga taaagttgca ggaccacttc tgcgctcggc ccttccggct 4740
ggctggttta ttgctgataa atctggagcc ggtgagcgtg ggtctcgcgg tatcattgca 4800
gcactggggc cagatggtaa gccctcccgt atcgtagtta tctacacgac ggggagtcag 4860
gcaactatgg atgaacgaaa tagacagatc gctgagatag gtgcctcact gattaagcat 4920
tggtaactgt cagaccaagt ttactcatat atactttaga ttgatttaaa acttcatttt 4980
taatttaaaa ggatctaggt gaagatcctt tttgataatc tcatgaccaa aatcccttaa 5040
cgtgagtttt cgttccactg agcgtcagac cccgtagaaa agatcaaagg atcttcttga 5100
gatccttttt ttctgcgcgt aatctgctgc ttgcaaacaa aaaaaccacc gctaccagcg 5160
gtggtttgtt tgccggatca agagctacca actctttttc cgaaggtaac tggcttcagc 5220
agagcgcaga taccaaatac tgttcttcta gtgtagccgt agttaggcca ccacttcaag 5280
aactctgtag caccgcctac atacctcgct ctgctaatcc tgttaccagt ggctgctgcc 5340
agtggcgata agtcgtgtct taccgggttg gactcaagac gatagttacc ggataaggcg 5400
cagcggtcgg gctgaacggg gggttcgtgc acacagccca gcttggagcg aacgacctac 5460
accgaactga gatacctaca gcgtgagcta tgagaaagcg ccacgcttcc cgaagggaga 5520
aaggcggaca ggtatccggt aagcggcagg gtcggaacag gagagcgcac gagggagctt 5580
ccagggggaa acgcctggta tctttatagt cctgtcgggt ttcgccacct ctgacttgag 5640
cgtcgatttt tgtgatgctc gtcagggggg cggagcctat ggaaaaacgc cagcaacgcg 5700
gcctttttac ggttcctggc cttttgctgg ccttttgctc acatggctcg acagatct 5758

<210> 176
<211> 5873
<212> DNA
<213> Homo sapiens
<220>
<221> misc_feature
<223> vecteur d'expression appelé GS-V28 contenant la séquence antisens
de GS-N28 ; antisens: 1108...1520

<400> 176
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgttgg gaagcattgg ctggttctca cagtacacac 1140
gaggacagta accaaagtct ccttgctggt acttttccaa catctgggcg atgccacggt 1200
tggtaaggat gtagcgggcg tggatcaatc cataaagcat ctcggctgcc tgctcaatca 1260
ggtcactctg gttggggttg tcttccagtt cttcatcagg ctccaggtcc aagatcatgt 1320
ctagagcttg tcgatagtga gggacctgct cattgagtcc agtaagatta aatttgtcct 1380
ggatgtagtc ttcatccact tcacagaaga attcattgcc acggagccca cagaaccagg 1440
aaatccagga cacctcctct gagctgctca tcttcacgtc agcggctgga cggggaccag 1500
gactggggtg gggtagggaa gtcgacccgg gcggccgctt ccctttagtg agggttaatg 1560
cttcgagcag acatgataag atacattgat gagtttggac aaaccacaac tagaatgcag 1620
tgaaaaaaat gctttatttg tgaaatttgt gatgctattg ctttatttgt aaccattata 1680
agctgcaata aacaagttaa caacaacaat tgcattcatt ttatgtttca ggttcagggg 1740
gagatgtggg aggtttttta aagcaagtaa aacctctaca aatgtggtaa aatccgataa 1800
ggatcgatcc gggctggcgt aatagcgaag aggcccgcac cgatcgccct tcccaacagt 1860
tgcgcagcct gaatggcgaa tggacgcgcc ctgtagcggc gcattaagcg cggcgggtgt 1920
ggtggttacg cgcagcgtga ccgctacact tgccagcgcc ctagcgcccg ctcctttcgc 1980
tttcttccct tcctttctcg ccacgttcgc cggctttccc cgtcaagctc taaatcgggg 2040
gctcccttta gggttccgat ttagtgcttt acggcacctc gaccccaaaa aacttgatta 2100
gggtgatggt tcacgtagtg ggccatcgcc ctgatagacg gtttttcgcc ctttgacgtt 2160
ggagtccacg ttctttaata gtggactctt gttccaaact ggaacaacac tcaaccctat 2220
ctcggtctat tcttttgatt tataagggat tttgccgatt tcggcctatt ggttaaaaaa 2280
tgagctgatt taacaaaaat ttaacgcgaa ttttaacaaa atattaacgc ttacaatttc 2340
ctgatgcggt attttctcct tacgcatctg tgcggtattt cacaccgcat acgcggatct 2400
gcgcagcacc atggcctgaa ataacctctg aaagaggaac ttggttaggt accttctgag 2460
gcggaaagaa ccagctgtgg aatgtgtgtc agttagggtg tggaaagtcc ccaggctccc 2520
cagcaggcag aagtatgcaa agcatgcatc tcaattagtc agcaaccagg tgtggaaagt 2580
ccccaggctc cccagcaggc agaagtatgc aaagcatgca tctcaattag tcagcaacca 2640
tagtcccgcc cctaactccg cccatcccgc ccctaactcc gcccagttcc gcccattctc 2700
cgccccatgg ctgactaatt ttttttattt atgcagaggc cgaggccgcc tcggcctctg 2760
agctattcca gaagtagtga ggaggctttt ttggaggcct aggcttttgc aaaaagcttg 2820
attcttctga cacaacagtc tcgaacttaa ggctagagcc accatgattg aacaagatgg 2880
attgcacgca ggttctccgg ccgcttgggt ggagaggcta ttcggctatg actgggcaca 2940
acagacaatc ggctgctctg atgccgccgt gttccggctg tcagcgcagg ggcgcccggt 3000
tctttttgtc aagaccgacc tgtccggtgc cctgaatgaa ctgcaggacg aggcagcgcg 3060
gctatcgtgg ctggccacga cgggcgttcc ttgcgcagct gtgctcgacg ttgtcactga 3120
agcgggaagg gactggctgc tattgggcga agtgccgggg caggatctcc tgtcatctca 3180
ccttgctcct gccgagaaag tatccatcat ggctgatgca atgcggcggc tgcatacgct 3240
tgatccggct acctgcccat tcgaccacca agcgaaacat cgcatcgagc gagcacgtac 3300
tcggatggaa gccggtcttg tcgatcagga tgatctggac gaagagcatc aggggctcgc 3360
gccagccgaa ctgttcgcca ggctcaaggc gcgcatgccc gacggcgagg atctcgtcgt 3420
gacccatggc gatgcctgct tgccgaatat catggtggaa aatggccgct tttctggatt 3480
catcgactgt ggccggctgg gtgtggcgga ccgctatcag gacatagcgt tggctacccg 3540
tgatattgct gaagagcttg gcggcgaatg ggctgaccgc ttcctcgtgc tttacggtat 3600
cgccgctccc gattcgcagc gcatcgcctt ctatcgcctt cttgacgagt tcttctgagc 3660

gggactctgg ggttcgaaat gaccgaccaa gcgacgccca acctgccatc acgatggccg 3720
caataaaata tctttatttt cattacatct gtgtgttggt tttttgtgtg aatcgatagc 3780
gataaggatc cgcgtatggt gcactctcag tacaatctgc tctgatgccg catagttaag 3840
ccagccccga cacccgccaa cacccgctga cgcgccctga cgggcttgtc tgctcccggc 3900
atccgcttac agacaagctg tgaccgtctc cgggagctgc atgtgtcaga ggttttcacc 3960
gtcatcaccg aaacgcgcga gacgaaaggg cctcgtgata cgcctatttt tataggttaa 4020
tgtcatgata ataatggttt cttagacgtc aggtggcact tttcggggaa atgtgcgcgg 4080
aacccctatt tgtttatttt tctaaataca ttcaaatatg tatccgctca tgagacaata 4140
accctgataa atgcttcaat aatattgaaa aaggaagagt atgagtattc aacatttccg 4200
tgtcgccctt attccctttt ttgcggcatt ttgccttcct gtttttgctc acccagaaac 4260
gctggtgaaa gtaaaagatg ctgaagatca gttgggtgca cgagtgggtt acatcgaact 4320
ggatctcaac agcggtaaga tccttgagag ttttcgcccc gaagaacgtt ttccaatgat 4380
gagcactttt aaagttctgc tatgtggcgc ggtattatcc cgtattgacg ccgggcaaga 4440
gcaactcggt cgccgcatac actattctca gaatgacttg gttgagtact caccagtcac 4500
agaaaagcat cttacggatg gcatgacagt aagagaatta tgcagtgctg ccataaccat 4560
gagtgataac actgcggcca acttacttct gacaacgatc ggaggaccga aggagctaac 4620
cgcttttttg cacaacatgg gggatcatgt aactcgcctt gatcgttggg aaccggagct 4680
gaatgaagcc ataccaaacg acgagcgtga caccacgatg cctgtagcaa tggcaacaac 4740
gttgcgcaaa ctattaactg gcgaactact tactctagct tcccggcaac aattaataga 4800
ctggatggag gcggataaag ttgcaggacc acttctgcgc tcggcccttc cggctggctg 4860
gtttattgct gataaatctg gagccggtga gcgtgggtct cgcggtatca ttgcagcact 4920
ggggccagat ggtaagccct cccgtatcgt agttatctac acgacgggga gtcaggcaac 4980
tatggatgaa cgaaatagac agatcgctga gataggtgcc tcactgatta agcattggta 5040
actgtcagac caagtttact catatatact ttagattgat ttaaaacttc atttttaatt 5100
taaaaggatc taggtgaaga tcctttttga taatctcatg accaaaatcc cttaacgtga 5160
gttttcgttc cactgagcgt cagaccccgt agaaaagatc aaaggatctt cttgagatcc 5220
tttttttctg cgcgtaatct gctgcttgca aacaaaaaaa ccaccgctac cagcggtggt 5280
ttgtttgccg gatcaagagc taccaactct ttttccgaag gtaactggct tcagcagagc 5340
gcagatacca aatactgttc ttctagtgta gccgtagtta ggccaccact tcaagaactc 5400
tgtagcaccg cctacatacc tcgctctgct aatcctgtta ccagtggctg ctgccagtgg 5460
cgataagtcg tgtcttaccg ggttggactc aagacgatag ttaccggata aggcgcagcg 5520
gtcgggctga acggggggtt cgtgcacaca gcccagcttg gagcgaacga cctacaccga 5580
actgagatac ctacagcgtg agctatgaga aagcgccacg cttcccgaag ggagaaaggc 5640
ggacaggtat ccggtaagcg gcagggtcgg aacaggagag cgcacgaggg agcttccagg 5700
gggaaacgcc tggtatcttt atagtcctgt cgggtttcgc cacctctgac ttgagcgtcg 5760
atttttgtga tgctcgtcag gggggcggag cctatggaaa aacgccagca acgcggcctt 5820
tttacggttc ctggcctttt gctggccttt tgctcacatg gctcgacaga tct 5873

<210> 177
<211> 6024
<212> DNA
<213> Homo sapiens
<220>
<221> misc_feature
<223> vecteur d'expression appelé GS-V29 contenant la séquence antisens
de GS-N29 ; antisens: 1108...1671

<220>
<221> misc_feature
<223> vecteur d'expression appelé GS-V29 contenant la séquence antisens
de GS-N52 ; antisens: 1108...1671

<400> 177
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgttcc cacaactttc gctgcatgtt ccccaaggct 1140

gccaagcgct ccatgctcca tgaactgggc aaggcctaat gttgcacata attatttcca 1200
cagcattccc ttcacatggc cgcccaccat gctgaactga tggattattg caagtcctgg 1260
tcctggtttg gttgccgcgt ccacagctcc ttgtgcattc ttcccaaaga ctccattccg 1320
accagctacc atccactgga caaggcttat tttgacagtt tcgcatttcc aaatctgaac 1380
cttggcaggg cttcccacca ttggctggaa ggggctggtt gcacagacga ctcctctttt 1440
ggatgccttt tccacaggtg acactgcagg ctctccatgc agaccactgg gaaaatccac 1500
catgaacctg gactatgact ggcactctga caaggacaga acccattaag tttcgagcaa 1560
cacattcaaa ttcagagata tcttctttct gagcatcagc aatatataat gagttgttgg 1620
acaacacgtt aacccggtca tcccaggaaa tagagtgccc ttgacgggac cgtcgacccg 1680
ggcggccgct tccctttagt gagggttaat gcttcgagca gacatgataa gatacattga 1740
tgagtttgga caaaccacaa ctagaatgca gtgaaaaaaa tgctttattt gtgaaatttg 1800
tgatgctatt gctttatttg taaccattat aagctgcaat aaacaagtta acaacaacaa 1860
ttgcattcat tttatgtttc aggttcaggg ggagatgtgg gaggtttttt aaagcaagta 1920
aaacctctac aaatgtggta aaatccgata aggatcgatc cgggctggcg taatagcgaa 1980
gaggcccgca ccgatcgccc ttcccaacag ttgcgcagcc tgaatggcga atggacgcgc 2040
cctgtagcgg cgcattaagc gcggcgggtg tggtggttac gcgcagcgtg accgctacac 2100
ttgccagcgc cctagcgccc gctcctttcg ctttcttccc ttcctttctc gccacgttcg 2160
ccggctttcc ccgtcaagct ctaaatcggg ggctcccttt agggttccga tttagtgctt 2220
tacggcacct cgaccccaaa aaacttgatt agggtgatgg ttcacgtagt gggccatcgc 2280
cctgatagac ggtttttcgc cctttgacgt tggagtccac gttctttaat agtggactct 2340
tgttccaaac tggaacaaca ctcaacccta tctcggtcta ttcttttgat ttataaggga 2400
ttttgccgat ttcggcctat tggttaaaaa atgagctgat ttaacaaaaa tttaacgcga 2460
attttaacaa aatattaacg cttacaattt cctgatgcgg tattttctcc ttacgcatct 2520
gtgcggtatt tcacaccgca tacgcggatc tgcgcagcac catggcctga aataacctct 2580
gaaagaggaa cttggttagg taccttctga ggcggaaaga accagctgtg gaatgtgtgt 2640
cagttagggt gtggaaagtc cccaggctcc ccagcaggca gaagtatgca aagcatgcat 2700
ctcaattagt cagcaaccag gtgtggaaag tccccaggct ccccagcagg cagaagtatg 2760
caaagcatgc atctcaatta gtcagcaacc atagtcccgc ccctaactcc gcccatcccg 2820
cccctaactc cgcccagttc cgcccattct ccgccccatg gctgactaat tttttttatt 2880
tatgcagagg ccgaggccgc ctcggcctct gagctattcc agaagtagtg aggaggcttt 2940
tttggaggcc taggcttttg caaaaagctt gattcttctg acacaacagt ctcgaactta 3000
aggctagagc caccatgatt gaacaagatg gattgcacgc aggttctccg gccgcttggg 3060
tggagaggct attcggctat gactgggcac aacagacaat cggctgctct gatgccgccg 3120
tgttccggct gtcagcgcag gggcgcccgg ttctttttgt caagaccgac ctgtccggtg 3180
ccctgaatga actgcaggac gaggcagcgc ggctatcgtg gctggccacg acgggcgttc 3240
cttgcgcagc tgtgctcgac gttgtcactg aagcgggaag ggactggctg ctattgggcg 3300
aagtgccggg gcaggatctc ctgtcatctc accttgctcc tgccgagaaa gtatccatca 3360
tggctgatgc aatgcggcgg ctgcatacgc ttgatccggc tacctgccca ttcgaccacc 3420
aagcgaaaca tcgcatcgag cgagcacgta ctcggatgga agccggtctt gtcgatcagg 3480
atgatctgga cgaagagcat caggggctcg cgccagccga actgttcgcc aggctcaagg 3540
cgcgcatgcc cgacggcgag gatctcgtcg tgacccatgg cgatgcctgc ttgccgaata 3600
tcatggtgga aaatggccgc ttttctggat tcatcgactg tggccggctg ggtgtggcgg 3660
accgctatca ggacatagcg ttggctaccc gtgatattgc tgaagagctt ggcggcgaat 3720
gggctgaccg cttcctcgtg ctttacggta tcgccgctcc cgattcgcag cgcatcgcct 3780
tctatcgcct tcttgacgag ttcttctgag cgggactctg gggttcgaaa tgaccgacca 3840
agcgacgccc aacctgccat cacgatggcc gcaataaaat atctttattt tcattacatc 3900
tgtgtgttgg ttttttgtgt gaatcgatag cgataaggat ccgcgtatgg tgcactctca 3960
gtacaatctg ctctgatgcc gcatagttaa gccagccccg acacccgcca acacccgctg 4020
acgcgccctg acgggcttgt ctgctcccgg catccgctta cagacaagct gtgaccgtct 4080
ccgggagctg catgtgtcag aggttttcac cgtcatcacc gaaacgcgcg agacgaaagg 4140
gcctcgtgat acgcctattt ttataggtta atgtcatgat aataatggtt tcttagacgt 4200
caggtggcac ttttcgggga aatgtgcgcg gaacccctat ttgtttattt ttctaaatac 4260
attcaaatat gtatccgctc atgagacaat aaccctgata aatgcttcaa taatattgaa 4320
aaaggaagag tatgagtatt caacatttcc gtgtcgccct tattcccttt tttgcggcat 4380
tttgccttcc tgtttttgct cacccagaaa cgctggtgaa agtaaaagat gctgaagatc 4440
agttgggtgc acgagtgggt tacatcgaac tggatctcaa cagcggtaag atccttgaga 4500
gttttcgccc cgaagaacgt tttccaatga tgagcacttt taaagttctg ctatgtggcg 4560
cggtattatc ccgtattgac gccgggcaag agcaactcgg tcgccgcata cactattctc 4620
agaatgactt ggttgagtac tcaccagtca cagaaaagca tcttacggat ggcatgacag 4680
taagagaatt atgcagtgct gccataacca tgagtgataa cactgcggcc aacttacttc 4740
tgacaacgat cggaggaccg aaggagctaa ccgctttttt gcacaacatg ggggatcatg 4800
taactcgcct tgatcgttgg gaaccggagc tgaatgaagc cataccaaac gacgagcgtg 4860
acaccacgat gcctgtagca atggcaacaa cgttgcgcaa actattaact ggcgaactac 4920
ttactctagc ttcccggcaa caattaatag actggatgga ggcggataaa gttgcaggac 4980
cacttctgcg ctcggccctt ccggctggct ggtttattgc tgataaatct ggagccggtg 5040
agcgtgggtc tcgcggtatc attgcagcac tggggccaga tggtaagccc tcccgtatcg 5100
tagttatcta cacgacgggg agtcaggcaa ctatggatga acgaaataga cagatcgctg 5160
agataggtgc ctcactgatt aagcattggt aactgtcaga ccaagtttac tcatatatac 5220
tttagattga tttaaaactt catttttaat ttaaaaggat ctaggtgaag atcctttttg 5280
ataatctcat gaccaaaatc ccttaacgtg agttttcgtt ccactgagcg tcagaccccg 5340
tagaaaagat caaaggatct tcttgagatc ctttttttct gcgcgtaatc tgctgcttgc 5400
aaacaaaaaa accaccgcta ccagcggtgg tttgtttgcc ggatcaagag ctaccaactc 5460
tttttccgaa ggtaactggc ttcagcagag cgcagatacc aaatactgtt cttctagtgt 5520

agccgtagtt aggccaccac ttcaagaact ctgtagcacc gcctacatac ctcgctctgc 5580
taatcctgtt accagtggct gctgccagtg gcgataagtc gtgtcttacc gggttggact 5640
caagacgata gttaccggat aaggcgcagc ggtcgggctg aacggggggt tcgtgcacac 5700
agcccagctt ggagcgaacg acctacaccg aactgagata cctacagcgt gagctatgag 5760
aaagcgccac gcttcccgaa gggagaaagg cggacaggta tccggtaagc ggcagggtcg 5820
gaacaggaga gcgcacgagg gagcttccag ggggaaacgc ctggtatctt tatagtcctg 5880
tcgggtttcg ccacctctga cttgagcgtc gatttttgtg atgctcgtca ggggggcgga 5940
gcctatggaa aaacgccagc aacgcggcct ttttacggtt cctggccttt tgctggcctt 6000
ttgctcacat ggctcgacag atct 6024

<210> 178
<211> 5874
<212> DNA
<213> Homo sapiens
<220>
<221> misc_feature
<223> vecteur d'expression appelé GS-V30 contenant la séquence antisens
de GS-N30 ; antisens: 1108...1521

<400> 178
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgttga ggctgggttc tgggttccct gcaaggccat 1140
taaatcttgg gacacttgct cccgtaactg tttgagtttc ttctcaataa catgcacctt 1200
ttcttcagct tcaatacggt cttctccatg tcccttaatg agaaggctgt ttgaaatctc 1260
ctgtaacatg tccacttgag gttgacgttc taccagctcc ttttccagtt gcattagttc 1320
cctccgacac tctaggagag cccgggggtc tgcctttgga tcggtgacat gagccttctg 1380
cctccggttc ttggcactcg ctaaccacag cagcagattt tgactcaact ggtggaagtc 1440
ctggcactgc atgagcgact gtcgtaagcc ccctctccag ctgtccactg cgccctgtgc 1500
tgcttcccag agcaggctca ggtcgacccg ggcggccgct tccctttagt gagggttaat 1560
gcttcgagca gacatgataa gatacattga tgagtttgga caaaccacaa ctagaatgca 1620
gtgaaaaaaa tgctttattt gtgaaatttg tgatgctatt gctttatttg taaccattat 1680
aagctgcaat aaacaagtta acaacaacaa ttgcattcat tttatgtttc aggttcaggg 1740
ggagatgtgg gaggtttttt aaagcaagta aaacctctac aaatgtggta aaatccgata 1800
aggatcgatc cgggctggcg taatagcgaa gaggcccgca ccgatcgccc ttcccaacag 1860
ttgcgcagcc tgaatggcga atggacgcgc cctgtagcgg cgcattaagc gcggcgggtg 1920
tggtggttac gcgcagcgtg accgctacac ttgccagcgc cctagcgccc gctcctttcg 1980
ctttcttccc ttcctttctc gccacgttcg ccggctttcc ccgtcaagct ctaaatcggg 2040
ggctcccttt agggttccga tttagtgctt tacggcacct cgaccccaaa aaacttgatt 2100
agggtgatgg ttcacgtagt gggccatcgc cctgatagac ggtttttcgc cctttgacgt 2160
tggagtccac gttctttaat agtggactct tgttccaaac tggaacaaca ctcaacccta 2220
tctcggtcta ttcttttgat ttataaggga ttttgccgat ttcggcctat tggttaaaaa 2280
atgagctgat ttaacaaaaa tttaacgcga attttaacaa aatattaacg cttacaattt 2340
cctgatgcgg tattttctcc ttacgcatct gtgcggtatt tcacaccgca tacgcggatc 2400
tgcgcagcac catggcctga aataacctct gaaagaggaa cttggttagg taccttctga 2460
ggcggaaaga accagctgtg gaatgtgtgt cagttagggt gtggaaagtc cccaggctcc 2520
ccagcaggca gaagtatgca aagcatgcat ctcaattagt cagcaaccag gtgtggaaag 2580
tccccaggct ccccagcagg cagaagtatg caaagcatgc atctcaatta gtcagcaacc 2640
atagtcccgc ccctaactcc gcccatcccg cccctaactc cgcccagttc cgcccattct 2700
ccgccccatg gctgactaat tttttttatt tatgcagagg ccgaggccgc ctcggcctct 2760
gagctattcc agaagtagtg aggaggcttt tttggaggcc taggcttttg caaaaagctt 2820
gattcttctg acacaacagt ctcgaactta aggctagagc caccatgatt gaacaagatg 2880
gattgcacgc aggttctccg gccgcttggg tggagaggct attcggctat gactgggcac 2940
aacagacaat cggctgctct gatgccgccg tgttccggct gtcagcgcag gggcgcccgg 3000
ttctttttgt caagaccgac ctgtccggtg ccctgaatga actgcaggac gaggcagcgc 3060
ggctatcgtg gctggccacg acgggcgttc cttgcgcagc tgtgctcgac gttgtcactg 3120

aagcgggaag ggactggctg ctattgggcg aagtgccggg gcaggatctc ctgtcatctc 3180
accttgctcc tgccgagaaa gtatccatca tggctgatgc aatgcggcgg ctgcatacgc 3240
ttgatccggc tacctgccca ttcgaccacc aagcgaaaca tcgcatcgag cgagcacgta 3300
ctcggatgga agccggtctt gtcgatcagg atgatctgga cgaagagcat caggggctcg 3360
cgccagccga actgttcgcc aggctcaagg cgcgcatgcc cgacggcgag gatctcgtcg 3420
tgacccatgg cgatgcctgc ttgccgaata tcatggtgga aaatggccgc ttttctggat 3480
tcatcgactg tggccggctg ggtgtggcgg accgctatca ggacatagcg ttggctaccc 3540
gtgatattgc tgaagagctt ggcggcgaat gggctgaccg cttcctcgtg ctttacggta 3600
tcgccgctcc cgattcgcag cgcatcgcct tctatcgcct tcttgacgag ttcttctgag 3660
cgggactctg gggttcgaaa tgaccgacca agcgacgccc aacctgccat cacgatggcc 3720
gcaataaaat atctttattt tcattacatc tgtgtgttgg ttttttgtgt gaatcgatag 3780
cgataaggat ccgcgtatgg tgcactctca gtacaatctg ctctgatgcc gcatagttaa 3840
gccagccccg acacccgcca acacccgctg acgcgccctg acgggcttgt ctgctcccgg 3900
catccgctta cagacaagct gtgaccgtct ccgggagctg catgtgtcag aggttttcac 3960
cgtcatcacc gaaacgcgcg agacgaaagg gcctcgtgat acgcctattt ttataggtta 4020
atgtcatgat aataatggtt tcttagacgt caggtggcac ttttcgggga aatgtgcgcg 4080
gaacccctat ttgtttattt ttctaaatac attcaaatat gtatccgctc atgagacaat 4140
aaccctgata aatgcttcaa taatattgaa aaaggaagag tatgagtatt caacatttcc 4200
gtgtcgccct tattcccttt tttgcggcat tttgccttcc tgtttttgct cacccagaaa 4260
cgctggtgaa agtaaaagat gctgaagatc agttgggtgc acgagtgggt tacatcgaac 4320
tggatctcaa cagcggtaag atccttgaga gttttcgccc cgaagaacgt tttccaatga 4380
tgagcacttt taaagttctg ctatgtggcg cggtattatc ccgtattgac gccgggcaag 4440
agcaactcgg tcgccgcata cactattctc agaatgactt ggttgagtac tcaccagtca 4500
cagaaaagca tcttacggat ggcatgacag taagagaatt atgcagtgct gccataacca 4560
tgagtgataa cactgcggcc aacttacttc tgacaacgat cggaggaccg aaggagctaa 4620
ccgctttttt gcacaacatg ggggatcatg taactcgcct tgatcgttgg gaaccggagc 4680
tgaatgaagc cataccaaac gacgagcgtg acaccacgat gcctgtagca atggcaacaa 4740
cgttgcgcaa actattaact ggcgaactac ttactctagc ttcccggcaa caattaatag 4800
actggatgga ggcggataaa gttgcaggac cacttctgcg ctcggccctt ccggctggct 4860
ggtttattgc tgataaatct ggagccggtg agcgtgggtc tcgcggtatc attgcagcac 4920
tggggccaga tggtaagccc tcccgtatcg tagttatcta cacgacgggg agtcaggcaa 4980
ctatggatga acgaaataga cagatcgctg agataggtgc ctcactgatt aagcattggt 5040
aactgtcaga ccaagtttac tcatatatac tttagattga tttaaaactt catttttaat 5100
ttaaaaggat ctaggtgaag atcctttttg ataatctcat gaccaaaatc ccttaacgtg 5160
agttttcgtt ccactgagcg tcagaccccg tagaaaagat caaaggatct tcttgagatc 5220
ctttttttct gcgcgtaatc tgctgcttgc aaacaaaaaa accaccgcta ccagcggtgg 5280
tttgtttgcc ggatcaagag ctaccaactc tttttccgaa ggtaactggc ttcagcagag 5340
cgcagatacc aaatactgtt cttctagtgt agccgtagtt aggccaccac ttcaagaact 5400
ctgtagcacc gcctacatac ctcgctctgc taatcctgtt accagtggct gctgccagtg 5460
gcgataagtc gtgtcttacc gggttggact caagacgata gttaccggat aaggcgcagc 5520
ggtcgggctg aacggggggt tcgtgcacac agcccagctt ggagcgaacg acctacaccg 5580
aactgagata cctacagcgt gagctatgag aaagcgccac gcttcccgaa gggagaaagg 5640
cggacaggta tccggtaagc ggcagggtcg gaacaggaga gcgcacgagg gagcttccag 5700
ggggaaacgc ctggtatctt tatagtcctg tcgggtttcg ccacctctga cttgagcgtc 5760
gatttttgtg atgctcgtca ggggggcgga gcctatggaa aaacgccagc aacgcggcct 5820
ttttacggtt cctggccttt tgctggcctt ttgctcacat ggctcgacag atct 5874

<210> 179
<211> 5758
<212> DNA
<213> Homo sapiens
<220>
<221> misc_feature
<223> vecteur d'expression appelé GS-V31 contenant la séquence antisens
de GS-N31 ; antisens: 1108...1405

<220>
<221> misc_feature
<223> vecteur d'expression appelé GS-V31 contenant la séquence antisens
de GS-N53 ; antisens: 1108...1405


<400> 179
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600

caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtagc tggccttggg gttgaaggaa ggatcttgaa 1140
tgacagataa tccccaagtg tccactggat catttttcct ctgactttcc acacaaatga 1200
ggctttccat gggctttgca accacagtgt ctaagacaat cagaagggtt ccagggaggt 1260
cttgtctgta gcgatgtgac agatgaggaa actgcagctc agaggacaat gacttgccca 1320
cgttgcacag ctcgctgaca gtagagaggg gctgaaacct aggtctcctg actcacacgc 1380
cacaaccatg actggctcag ctcccgtcga cccgggcggc cgcttccctt tagtgagggt 1440
taatgcttcg agcagacatg ataagataca ttgatgagtt tggacaaacc acaactagaa 1500
tgcagtgaaa aaaatgcttt atttgtgaaa tttgtgatgc tattgcttta tttgtaacca 1560
ttataagctg caataaacaa gttaacaaca acaattgcat tcattttatg tttcaggttc 1620
agggggagat gtgggaggtt ttttaaagca agtaaaacct ctacaaatgt ggtaaaatcc 1680
gataaggatc gatccgggct ggcgtaatag cgaagaggcc cgcaccgatc gcccttccca 1740
acagttgcgc agcctgaatg gcgaatggac gcgccctgta gcggcgcatt aagcgcggcg 1800
ggtgtggtgg ttacgcgcag cgtgaccgct acacttgcca gcgccctagc gcccgctcct 1860
ttcgctttct tcccttcctt tctcgccacg ttcgccggct ttccccgtca agctctaaat 1920
cgggggctcc ctttagggtt ccgatttagt gctttacggc acctcgaccc caaaaaactt 1980
gattagggtg atggttcacg tagtgggcca tcgccctgat agacggtttt tcgccctttg 2040
acgttggagt ccacgttctt taatagtgga ctcttgttcc aaactggaac aacactcaac 2100
cctatctcgg tctattcttt tgatttataa gggattttgc cgatttcggc ctattggtta 2160
aaaaatgagc tgatttaaca aaaatttaac gcgaatttta acaaaatatt aacgcttaca 2220
atttcctgat gcggtatttt ctccttacgc atctgtgcgg tatttcacac cgcatacgcg 2280
gatctgcgca gcaccatggc ctgaaataac ctctgaaaga ggaacttggt taggtacctt 2340
ctgaggcgga aagaaccagc tgtggaatgt gtgtcagtta gggtgtggaa agtccccagg 2400
ctccccagca ggcagaagta tgcaaagcat gcatctcaat tagtcagcaa ccaggtgtgg 2460
aaagtcccca ggctccccag caggcagaag tatgcaaagc atgcatctca attagtcagc 2520
aaccatagtc ccgcccctaa ctccgcccat cccgccccta actccgccca gttccgccca 2580
ttctccgccc catggctgac taattttttt tatttatgca gaggccgagg ccgcctcggc 2640
ctctgagcta ttccagaagt agtgaggagg cttttttgga ggcctaggct tttgcaaaaa 2700
gcttgattct tctgacacaa cagtctcgaa cttaaggcta gagccaccat gattgaacaa 2760
gatggattgc acgcaggttc tccggccgct tgggtggaga ggctattcgg ctatgactgg 2820
gcacaacaga caatcggctg ctctgatgcc gccgtgttcc ggctgtcagc gcaggggcgc 2880
ccggttcttt ttgtcaagac cgacctgtcc ggtgccctga atgaactgca ggacgaggca 2940
gcgcggctat cgtggctggc cacgacgggc gttccttgcg cagctgtgct cgacgttgtc 3000
actgaagcgg gaagggactg gctgctattg ggcgaagtgc cggggcagga tctcctgtca 3060
tctcaccttg ctcctgccga gaaagtatcc atcatggctg atgcaatgcg gcggctgcat 3120
acgcttgatc cggctacctg cccattcgac caccaagcga aacatcgcat cgagcgagca 3180
cgtactcgga tggaagccgg tcttgtcgat caggatgatc tggacgaaga gcatcagggg 3240
ctcgcgccag ccgaactgtt cgccaggctc aaggcgcgca tgcccgacgg cgaggatctc 3300
gtcgtgaccc atggcgatgc ctgcttgccg aatatcatgg tggaaaatgg ccgcttttct 3360
ggattcatcg actgtggccg gctgggtgtg gcggaccgct atcaggacat agcgttggct 3420
acccgtgata ttgctgaaga gcttggcggc gaatgggctg accgcttcct cgtgctttac 3480
ggtatcgccg ctcccgattc gcagcgcatc gccttctatc gccttcttga cgagttcttc 3540
tgagcgggac tctggggttc gaaatgaccg accaagcgac gcccaacctg ccatcacgat 3600
ggccgcaata aaatatcttt attttcatta catctgtgtg ttggtttttt gtgtgaatcg 3660
atagcgataa ggatccgcgt atggtgcact ctcagtacaa tctgctctga tgccgcatag 3720
ttaagccagc cccgacaccc gccaacaccc gctgacgcgc cctgacgggc ttgtctgctc 3780
ccggcatccg cttacagaca agctgtgacc gtctccggga gctgcatgtg tcagaggttt 3840
tcaccgtcat caccgaaacg cgcgagacga aagggcctcg tgatacgcct atttttatag 3900
gttaatgtca tgataataat ggtttcttag acgtcaggtg gcacttttcg gggaaatgtg 3960
cgcggaaccc ctatttgttt atttttctaa atacattcaa atatgtatcc gctcatgaga 4020
caataaccct gataaatgct tcaataatat tgaaaaagga agagtatgag tattcaacat 4080
ttccgtgtcg cccttattcc cttttttgcg gcattttgcc ttcctgtttt tgctcaccca 4140
gaaacgctgg tgaaagtaaa agatgctgaa gatcagttgg gtgcacgagt gggttacatc 4200
gaactggatc tcaacagcgg taagatcctt gagagttttc gccccgaaga acgttttcca 4260
atgatgagca cttttaaagt tctgctatgt ggcgcggtat tatcccgtat tgacgccggg 4320
caagagcaac tcggtcgccg catacactat tctcagaatg acttggttga gtactcacca 4380
gtcacagaaa agcatcttac ggatggcatg acagtaagag aattatgcag tgctgccata 4440
accatgagtg ataacactgc ggccaactta cttctgacaa cgatcggagg accgaaggag 4500
ctaaccgctt ttttgcacaa catgggggat catgtaactc gccttgatcg ttgggaaccg 4560
gagctgaatg aagccatacc aaacgacgag cgtgacacca cgatgcctgt agcaatggca 4620
acaacgttgc gcaaactatt aactggcgaa ctacttactc tagcttcccg gcaacaatta 4680
atagactgga tggaggcgga taaagttgca ggaccacttc tgcgctcggc ccttccggct 4740
ggctggttta ttgctgataa atctggagcc ggtgagcgtg ggtctcgcgg tatcattgca 4800
gcactggggc cagatggtaa gccctcccgt atcgtagtta tctacacgac ggggagtcag 4860
gcaactatgg atgaacgaaa tagacagatc gctgagatag gtgcctcact gattaagcat 4920
tggtaactgt cagaccaagt ttactcatat atactttaga ttgatttaaa acttcatttt 4980

taatttaaaa ggatctaggt gaagatcctt tttgataatc tcatgaccaa aatcccttaa 5040
cgtgagtttt cgttccactg agcgtcagac cccgtagaaa agatcaaagg atcttcttga 5100
gatccttttt ttctgcgcgt aatctgctgc ttgcaaacaa aaaaaccacc gctaccagcg 5160
gtggtttgtt tgccggatca agagctacca actctttttc cgaaggtaac tggcttcagc 5220
agagcgcaga taccaaatac tgttcttcta gtgtagccgt agttaggcca ccacttcaag 5280
aactctgtag caccgcctac atacctcgct ctgctaatcc tgttaccagt ggctgctgcc 5340
agtggcgata agtcgtgtct taccgggttg gactcaagac gatagttacc ggataaggcg 5400
cagcggtcgg gctgaacggg gggttcgtgc acacagccca gcttggagcg aacgacctac 5460
accgaactga gatacctaca gcgtgagcta tgagaaagcg ccacgcttcc cgaagggaga 5520
aaggcggaca ggtatccggt aagcggcagg gtcggaacag gagagcgcac gagggagctt 5580
ccagggggaa acgcctggta tctttatagt cctgtcgggt ttcgccacct ctgacttgag 5640
cgtcgatttt tgtgatgctc gtcagggggg cggagcctat ggaaaaacgc cagcaacgcg 5700
gcctttttac ggttcctggc cttttgctgg ccttttgctc acatggctcg acagatct 5758

<210> 180
<211> 5825
<212> DNA
<213> Homo sapiens
<220>
<221> misc_feature
<223> vecteur d'expression appelé GS-V32 contenant la séquence antisens
de GS-N32 ; antisens: 1108...1472

<400> 180
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtcct ctttcagcct gggcactttg ttttccttct 1140
ttacccgagg cttccgcttc tttaatttgg cctcttcccc acctgtcaca gcccccttct 1200
tctccagacc ctttctgagc agcttcaaca agtattccgc tcgggtctgt agctgcttcc 1260
cctgaggctt cttatctgtc tccaccggca ggattttgtc agttaattta agctctgggt 1320
ctgttttaat taactcccag tttccatagc catgttcata aatccccagc aacaggcgag 1380
aatcatcttc caccctccac tctacatcaa aatgtgcagc tttgacacga caggttaagc 1440
agtatttttt ttttcttcag ggtccacagg gagtcgaccc gggcggccgc ttccctttag 1500
tgagggttaa tgcttcgagc agacatgata agatacattg atgagtttgg acaaaccaca 1560
actagaatgc agtgaaaaaa atgctttatt tgtgaaattt gtgatgctat tgctttattt 1620
gtaaccatta taagctgcaa taaacaagtt aacaacaaca attgcattca ttttatgttt 1680
caggttcagg gggagatgtg ggaggttttt taaagcaagt aaaacctcta caaatgtggt 1740
aaaatccgat aaggatcgat ccgggctggc gtaatagcga agaggcccgc accgatcgcc 1800
cttcccaaca gttgcgcagc ctgaatggcg aatggacgcg ccctgtagcg gcgcattaag 1860
cgcggcgggt gtggtggtta cgcgcagcgt gaccgctaca cttgccagcg ccctagcgcc 1920
cgctcctttc gctttcttcc cttcctttct cgccacgttc gccggctttc cccgtcaagc 1980
tctaaatcgg gggctccctt tagggttccg atttagtgct ttacggcacc tcgaccccaa 2040
aaaacttgat tagggtgatg gttcacgtag tgggccatcg ccctgataga cggtttttcg 2100
ccctttgacg ttggagtcca cgttctttaa tagtggactc ttgttccaaa ctggaacaac 2160
actcaaccct atctcggtct attcttttga tttataaggg attttgccga tttcggccta 2220
ttggttaaaa aatgagctga tttaacaaaa atttaacgcg aattttaaca aaatattaac 2280
gcttacaatt tcctgatgcg gtattttctc cttacgcatc tgtgcggtat ttcacaccgc 2340
atacgcggat ctgcgcagca ccatggcctg aaataacctc tgaaagagga acttggttag 2400
gtaccttctg aggcggaaag aaccagctgt ggaatgtgtg tcagttaggg tgtggaaagt 2460
ccccaggctc cccagcaggc agaagtatgc aaagcatgca tctcaattag tcagcaacca 2520
ggtgtggaaa gtccccaggc tccccagcag gcagaagtat gcaaagcatg catctcaatt 2580
agtcagcaac catagtcccg cccctaactc cgcccatccc gcccctaact ccgcccagtt 2640
ccgcccattc tccgccccat ggctgactaa ttttttttat ttatgcagag gccgaggccg 2700
cctcggcctc tgagctattc cagaagtagt gaggaggctt ttttggaggc ctaggctttt 2760
gcaaaaagct tgattcttct gacacaacag tctcgaactt aaggctagag ccaccatgat 2820
tgaacaagat ggattgcacg caggttctcc ggccgcttgg gtggagaggc tattcggcta 2880

tgactgggca caacagacaa tcggctgctc tgatgccgcc gtgttccggc tgtcagcgca 2940
ggggcgcccg gttctttttg tcaagaccga cctgtccggt gccctgaatg aactgcagga 3000
cgaggcagcg cggctatcgt ggctggccac gacgggcgtt ccttgcgcag ctgtgctcga 3060
cgttgtcact gaagcgggaa gggactggct gctattgggc gaagtgccgg ggcaggatct 3120
cctgtcatct caccttgctc ctgccgagaa agtatccatc atggctgatg caatgcggcg 3180
gctgcatacg cttgatccgg ctacctgccc attcgaccac caagcgaaac atcgcatcga 3240
gcgagcacgt actcggatgg aagccggtct tgtcgatcag gatgatctgg acgaagagca 3300
tcaggggctc gcgccagccg aactgttcgc caggctcaag gcgcgcatgc ccgacggcga 3360
ggatctcgtc gtgacccatg gcgatgcctg cttgccgaat atcatggtgg aaaatggccg 3420
cttttctgga ttcatcgact gtggccggct gggtgtggcg gaccgctatc aggacatagc 3480
gttggctacc cgtgatattg ctgaagagct tggcggcgaa tgggctgacc gcttcctcgt 3540
gctttacggt atcgccgctc ccgattcgca gcgcatcgcc ttctatcgcc ttcttgacga 3600
gttcttctga gcgggactct ggggttcgaa atgaccgacc aagcgacgcc caacctgcca 3660
tcacgatggc cgcaataaaa tatctttatt ttcattacat ctgtgtgttg gttttttgtg 3720
tgaatcgata gcgataagga tccgcgtatg gtgcactctc agtacaatct gctctgatgc 3780
cgcatagtta agccagcccc gacacccgcc aacacccgct gacgcgccct gacgggcttg 3840
tctgctcccg gcatccgctt acagacaagc tgtgaccgtc tccgggagct gcatgtgtca 3900
gaggttttca ccgtcatcac cgaaacgcgc gagacgaaag ggcctcgtga tacgcctatt 3960
tttataggtt aatgtcatga taataatggt ttcttagacg tcaggtggca cttttcgggg 4020
aaatgtgcgc ggaaccccta tttgtttatt tttctaaata cattcaaata tgtatccgct 4080
catgagacaa taaccctgat aaatgcttca ataatattga aaaaggaaga gtatgagtat 4140
tcaacatttc cgtgtcgccc ttattccctt ttttgcggca ttttgccttc ctgtttttgc 4200
tcacccagaa acgctggtga aagtaaaaga tgctgaagat cagttgggtg cacgagtggg 4260
ttacatcgaa ctggatctca acagcggtaa gatccttgag agttttcgcc ccgaagaacg 4320
ttttccaatg atgagcactt ttaaagttct gctatgtggc gcggtattat cccgtattga 4380
cgccgggcaa gagcaactcg gtcgccgcat acactattct cagaatgact tggttgagta 4440
ctcaccagtc acagaaaagc atcttacgga tggcatgaca gtaagagaat tatgcagtgc 4500
tgccataacc atgagtgata acactgcggc caacttactt ctgacaacga tcggaggacc 4560
gaaggagcta accgcttttt tgcacaacat gggggatcat gtaactcgcc ttgatcgttg 4620
ggaaccggag ctgaatgaag ccataccaaa cgacgagcgt gacaccacga tgcctgtagc 4680
aatggcaaca acgttgcgca aactattaac tggcgaacta cttactctag cttcccggca 4740
acaattaata gactggatgg aggcggataa agttgcagga ccacttctgc gctcggccct 4800
tccggctggc tggtttattg ctgataaatc tggagccggt gagcgtgggt ctcgcggtat 4860
cattgcagca ctggggccag atggtaagcc ctcccgtatc gtagttatct acacgacggg 4920
gagtcaggca actatggatg aacgaaatag acagatcgct gagataggtg cctcactgat 4980
taagcattgg taactgtcag accaagttta ctcatatata ctttagattg atttaaaact 5040
tcatttttaa tttaaaagga tctaggtgaa gatccttttt gataatctca tgaccaaaat 5100
cccttaacgt gagttttcgt tccactgagc gtcagacccc gtagaaaaga tcaaaggatc 5160
ttcttgagat cctttttttc tgcgcgtaat ctgctgcttg caaacaaaaa aaccaccgct 5220
accagcggtg gtttgtttgc cggatcaaga gctaccaact ctttttccga aggtaactgg 5280
cttcagcaga gcgcagatac caaatactgt tcttctagtg tagccgtagt taggccacca 5340
cttcaagaac tctgtagcac cgcctacata cctcgctctg ctaatcctgt taccagtggc 5400
tgctgccagt ggcgataagt cgtgtcttac cgggttggac tcaagacgat agttaccgga 5460
taaggcgcag cggtcgggct gaacgggggg ttcgtgcaca cagcccagct tggagcgaac 5520
gacctacacc gaactgagat acctacagcg tgagctatga gaaagcgcca cgcttcccga 5580
agggagaaag gcggacaggt atccggtaag cggcagggtc ggaacaggag agcgcacgag 5640
ggagcttcca gggggaaacg cctggtatct ttatagtcct gtcgggtttc gccacctctg 5700
acttgagcgt cgatttttgt gatgctcgtc aggggggcgg agcctatgga aaaacgccag 5760
caacgcggcc tttttacggt tcctggcctt ttgctggcct tttgctcaca tggctcgaca 5820
gatct 5825

<210> 181
<211> 5730
<212> DNA
<213> Homo sapiens
<220>
<221> misc_feature
<223> vecteur d'expression appelé GS-V33 contenant la séquence antisens
de GS-N33 ; antisens: 1108...1377

<400> 181
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660

cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgttgg aagcgctaag gcgtgacact gctacttgct 1140
gtgcagagga tgactctgtt ttctcattcg ctttcttggg gggcttttta gtagaattga 1200
gctgtccgct gacatcttgt aaccgctttt ctaattcccg ctttttctcc aaagccagtt 1260
cctcctttgt ctttcccaca ggcttcttaa tggtgtaggg cttccggggt ttcttacgta 1320
ggcaggaaag gacatagcgc tcaagctctc taagtgtgga tggcttgagt gcttcaagtc 1380
gacccgggcg gccgcttccc tttagtgagg gttaatgctt cgagcagaca tgataagata 1440
cattgatgag tttggacaaa ccacaactag aatgcagtga aaaaaatgct ttatttgtga 1500
aatttgtgat gctattgctt tatttgtaac cattataagc tgcaataaac aagttaacaa 1560
caacaattgc attcatttta tgtttcaggt tcagggggag atgtgggagg ttttttaaag 1620
caagtaaaac ctctacaaat gtggtaaaat ccgataagga tcgatccggg ctggcgtaat 1680
agcgaagagg cccgcaccga tcgcccttcc caacagttgc gcagcctgaa tggcgaatgg 1740
acgcgccctg tagcggcgca ttaagcgcgg cgggtgtggt ggttacgcgc agcgtgaccg 1800
ctacacttgc cagcgcccta gcgcccgctc ctttcgcttt cttcccttcc tttctcgcca 1860
cgttcgccgg ctttccccgt caagctctaa atcgggggct ccctttaggg ttccgattta 1920
gtgctttacg gcacctcgac cccaaaaaac ttgattaggg tgatggttca cgtagtgggc 1980
catcgccctg atagacggtt tttcgccctt tgacgttgga gtccacgttc tttaatagtg 2040
gactcttgtt ccaaactgga acaacactca accctatctc ggtctattct tttgatttat 2100
aagggatttt gccgatttcg gcctattggt taaaaaatga gctgatttaa caaaaattta 2160
acgcgaattt taacaaaata ttaacgctta caatttcctg atgcggtatt ttctccttac 2220
gcatctgtgc ggtatttcac accgcatacg cggatctgcg cagcaccatg gcctgaaata 2280
acctctgaaa gaggaacttg gttaggtacc ttctgaggcg gaaagaacca gctgtggaat 2340
gtgtgtcagt tagggtgtgg aaagtcccca ggctccccag caggcagaag tatgcaaagc 2400
atgcatctca attagtcagc aaccaggtgt ggaaagtccc caggctcccc agcaggcaga 2460
agtatgcaaa gcatgcatct caattagtca gcaaccatag tcccgcccct aactccgccc 2520
atcccgcccc taactccgcc cagttccgcc cattctccgc cccatggctg actaattttt 2580
tttatttatg cagaggccga ggccgcctcg gcctctgagc tattccagaa gtagtgagga 2640
ggcttttttg gaggcctagg cttttgcaaa aagcttgatt cttctgacac aacagtctcg 2700
aacttaaggc tagagccacc atgattgaac aagatggatt gcacgcaggt tctccggccg 2760
cttgggtgga gaggctattc ggctatgact gggcacaaca gacaatcggc tgctctgatg 2820
ccgccgtgtt ccggctgtca gcgcaggggc gcccggttct ttttgtcaag accgacctgt 2880
ccggtgccct gaatgaactg caggacgagg cagcgcggct atcgtggctg gccacgacgg 2940
gcgttccttg cgcagctgtg ctcgacgttg tcactgaagc gggaagggac tggctgctat 3000
tgggcgaagt gccggggcag gatctcctgt catctcacct tgctcctgcc gagaaagtat 3060
ccatcatggc tgatgcaatg cggcggctgc atacgcttga tccggctacc tgcccattcg 3120
accaccaagc gaaacatcgc atcgagcgag cacgtactcg gatggaagcc ggtcttgtcg 3180
atcaggatga tctggacgaa gagcatcagg ggctcgcgcc agccgaactg ttcgccaggc 3240
tcaaggcgcg catgcccgac ggcgaggatc tcgtcgtgac ccatggcgat gcctgcttgc 3300
cgaatatcat ggtggaaaat ggccgctttt ctggattcat cgactgtggc cggctgggtg 3360
tggcggaccg ctatcaggac atagcgttgg ctacccgtga tattgctgaa gagcttggcg 3420
gcgaatgggc tgaccgcttc ctcgtgcttt acggtatcgc cgctcccgat tcgcagcgca 3480
tcgccttcta tcgccttctt gacgagttct tctgagcggg actctggggt tcgaaatgac 3540
cgaccaagcg acgcccaacc tgccatcacg atggccgcaa taaaatatct ttattttcat 3600
tacatctgtg tgttggtttt ttgtgtgaat cgatagcgat aaggatccgc gtatggtgca 3660
ctctcagtac aatctgctct gatgccgcat agttaagcca gccccgacac ccgccaacac 3720
ccgctgacgc gccctgacgg gcttgtctgc tcccggcatc cgcttacaga caagctgtga 3780
ccgtctccgg gagctgcatg tgtcagaggt tttcaccgtc atcaccgaaa cgcgcgagac 3840
gaaagggcct cgtgatacgc ctatttttat aggttaatgt catgataata atggtttctt 3900
agacgtcagg tggcactttt cggggaaatg tgcgcggaac ccctatttgt ttatttttct 3960
aaatacattc aaatatgtat ccgctcatga gacaataacc ctgataaatg cttcaataat 4020
attgaaaaag gaagagtatg agtattcaac atttccgtgt cgcccttatt cccttttttg 4080
cggcattttg ccttcctgtt tttgctcacc cagaaacgct ggtgaaagta aaagatgctg 4140
aagatcagtt gggtgcacga gtgggttaca tcgaactgga tctcaacagc ggtaagatcc 4200
ttgagagttt tcgccccgaa gaacgttttc caatgatgag cacttttaaa gttctgctat 4260
gtggcgcggt attatcccgt attgacgccg ggcaagagca actcggtcgc cgcatacact 4320
attctcagaa tgacttggtt gagtactcac cagtcacaga aaagcatctt acggatggca 4380
tgacagtaag agaattatgc agtgctgcca taaccatgag tgataacact gcggccaact 4440
tacttctgac aacgatcgga ggaccgaagg agctaaccgc ttttttgcac aacatggggg 4500
atcatgtaac tcgccttgat cgttgggaac cggagctgaa tgaagccata ccaaacgacg 4560
agcgtgacac cacgatgcct gtagcaatgg caacaacgtt gcgcaaacta ttaactggcg 4620
aactacttac tctagcttcc cggcaacaat taatagactg gatggaggcg gataaagttg 4680
caggaccact tctgcgctcg gcccttccgg ctggctggtt tattgctgat aaatctggag 4740
ccggtgagcg tgggtctcgc ggtatcattg cagcactggg gccagatggt aagccctccc 4800
gtatcgtagt tatctacacg acggggagtc aggcaactat ggatgaacga aatagacaga 4860
tcgctgagat aggtgcctca ctgattaagc attggtaact gtcagaccaa gtttactcat 4920
atatacttta gattgattta aaacttcatt tttaatttaa aaggatctag gtgaagatcc 4980
tttttgataa tctcatgacc aaaatccctt aacgtgagtt ttcgttccac tgagcgtcag 5040

accccgtaga aaagatcaaa ggatcttctt gagatccttt ttttctgcgc gtaatctgct 5100
gcttgcaaac aaaaaaacca ccgctaccag cggtggtttg tttgccggat caagagctac 5160
caactctttt tccgaaggta actggcttca gcagagcgca gataccaaat actgttcttc 5220
tagtgtagcc gtagttaggc caccacttca agaactctgt agcaccgcct acatacctcg 5280
ctctgctaat cctgttacca gtggctgctg ccagtggcga taagtcgtgt cttaccgggt 5340
tggactcaag acgatagtta ccggataagg cgcagcggtc gggctgaacg gggggttcgt 5400
gcacacagcc cagcttggag cgaacgacct acaccgaact gagataccta cagcgtgagc 5460
tatgagaaag cgccacgctt cccgaaggga gaaaggcgga caggtatccg gtaagcggca 5520
gggtcggaac aggagagcgc acgagggagc ttccaggggg aaacgcctgg tatctttata 5580
gtcctgtcgg gtttcgccac ctctgacttg agcgtcgatt tttgtgatgc tcgtcagggg 5640
ggcggagcct atggaaaaac gccagcaacg cggccttttt acggttcctg gccttttgct 5700
ggccttttgc tcacatggct cgacagatct 5730

<210> 182
<211> 5758
<212> DNA
<213> Homo sapiens
<220>
<221> misc_feature
<223> vecteur d'expression appelé GS-V34 contenant la séquence antisens
de GS-N34 ; antisens: 1108...1405

<400> 182
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtttc cgggcctgac tctggtattt cacagctttt 1140
ttcgtttcat ctcgtgcctt ctccacgtgg tccactgtgt gcatgacatt caactctatg 1200
ttatctaaca tctcaccctg attctccacc agcatggcga tgtccataaa catgtcgtga 1260
agctccttga tgctgctctc cagcctcaca atgtccttgt gtcgtccctc aatctcactg 1320
agggcttgct tggaaatctg tgagtcaatg atcccagaag tgaagatggc cgggttgcca 1380
ctctccaaca tctcctccag ctccagtcga cccgggcggc cgcttccctt tagtgagggt 1440
taatgcttcg agcagacatg ataagataca ttgatgagtt tggacaaacc acaactagaa 1500
tgcagtgaaa aaaatgcttt atttgtgaaa tttgtgatgc tattgcttta tttgtaacca 1560
ttataagctg caataaacaa gttaacaaca acaattgcat tcattttatg tttcaggttc 1620
agggggagat gtgggaggtt ttttaaagca agtaaaacct ctacaaatgt ggtaaaatcc 1680
gataaggatc gatccgggct ggcgtaatag cgaagaggcc cgcaccgatc gcccttccca 1740
acagttgcgc agcctgaatg gcgaatggac gcgccctgta gcggcgcatt aagcgcggcg 1800
ggtgtggtgg ttacgcgcag cgtgaccgct acacttgcca gcgccctagc gcccgctcct 1860
ttcgctttct tcccttcctt tctcgccacg ttcgccggct ttccccgtca agctctaaat 1920
cgggggctcc ctttagggtt ccgatttagt gctttacggc acctcgaccc caaaaaactt 1980
gattagggtg atggttcacg tagtgggcca tcgccctgat agacggtttt tcgccctttg 2040
acgttggagt ccacgttctt taatagtgga ctcttgttcc aaactggaac aacactcaac 2100
cctatctcgg tctattcttt tgatttataa gggattttgc cgatttcggc ctattggtta 2160
aaaaatgagc tgatttaaca aaaatttaac gcgaatttta acaaaatatt aacgcttaca 2220
atttcctgat gcggtatttt ctccttacgc atctgtgcgg tatttcacac cgcatacgcg 2280
gatctgcgca gcaccatggc ctgaaataac ctctgaaaga ggaacttggt taggtacctt 2340
ctgaggcgga aagaaccagc tgtggaatgt gtgtcagtta gggtgtggaa agtccccagg 2400
ctccccagca ggcagaagta tgcaaagcat gcatctcaat tagtcagcaa ccaggtgtgg 2460
aaagtcccca ggctccccag caggcagaag tatgcaaagc atgcatctca attagtcagc 2520
aaccatagtc ccgcccctaa ctccgcccat cccgccccta actccgccca gttccgccca 2580
ttctccgccc catggctgac taattttttt tatttatgca gaggccgagg ccgcctcggc 2640
ctctgagcta ttccagaagt agtgaggagg cttttttgga ggcctaggct tttgcaaaaa 2700
gcttgattct tctgacacaa cagtctcgaa cttaaggcta gagccaccat gattgaacaa 2760
gatggattgc acgcaggttc tccggccgct tgggtggaga ggctattcgg ctatgactgg 2820
gcacaacaga caatcggctg ctctgatgcc gccgtgttcc ggctgtcagc gcaggggcgc 2880
ccggttcttt ttgtcaagac cgacctgtcc ggtgccctga atgaactgca ggacgaggca 2940

gcgcggctat cgtggctggc cacgacgggc gttccttgcg cagctgtgct cgacgttgtc 3000
actgaagcgg gaagggactg gctgctattg ggcgaagtgc cggggcagga tctcctgtca 3060
tctcaccttg ctcctgccga gaaagtatcc atcatggctg atgcaatgcg gcggctgcat 3120
acgcttgatc cggctacctg cccattcgac caccaagcga aacatcgcat cgagcgagca 3180
cgtactcgga tggaagccgg tcttgtcgat caggatgatc tggacgaaga gcatcagggg 3240
ctcgcgccag ccgaactgtt cgccaggctc aaggcgcgca tgcccgacgg cgaggatctc 3300
gtcgtgaccc atggcgatgc ctgcttgccg aatatcatgg tggaaaatgg ccgcttttct 3360
ggattcatcg actgtggccg gctgggtgtg gcggaccgct atcaggacat agcgttggct 3420
acccgtgata ttgctgaaga gcttggcggc gaatgggctg accgcttcct cgtgctttac 3480
ggtatcgccg ctcccgattc gcagcgcatc gccttctatc gccttcttga cgagttcttc 3540
tgagcgggac tctggggttc gaaatgaccg accaagcgac gcccaacctg ccatcacgat 3600
ggccgcaata aaatatcttt attttcatta catctgtgtg ttggtttttt gtgtgaatcg 3660
atagcgataa ggatccgcgt atggtgcact ctcagtacaa tctgctctga tgccgcatag 3720
ttaagccagc cccgacaccc gccaacaccc gctgacgcgc cctgacgggc ttgtctgctc 3780
ccggcatccg cttacagaca agctgtgacc gtctccggga gctgcatgtg tcagaggttt 3840
tcaccgtcat caccgaaacg cgcgagacga aagggcctcg tgatacgcct atttttatag 3900
gttaatgtca tgataataat ggtttcttag acgtcaggtg gcacttttcg gggaaatgtg 3960
cgcggaaccc ctatttgttt atttttctaa atacattcaa atatgtatcc gctcatgaga 4020
caataaccct gataaatgct tcaataatat tgaaaaagga agagtatgag tattcaacat 4080
ttccgtgtcg cccttattcc cttttttgcg gcattttgcc ttcctgtttt tgctcaccca 4140
gaaacgctgg tgaaagtaaa agatgctgaa gatcagttgg gtgcacgagt gggttacatc 4200
gaactggatc tcaacagcgg taagatcctt gagagttttc gccccgaaga acgttttcca 4260
atgatgagca cttttaaagt tctgctatgt ggcgcggtat tatcccgtat tgacgccggg 4320
caagagcaac tcggtcgccg catacactat tctcagaatg acttggttga gtactcacca 4380
gtcacagaaa agcatcttac ggatggcatg acagtaagag aattatgcag tgctgccata 4440
accatgagtg ataacactgc ggccaactta cttctgacaa cgatcggagg accgaaggag 4500
ctaaccgctt ttttgcacaa catgggggat catgtaactc gccttgatcg ttgggaaccg 4560
gagctgaatg aagccatacc aaacgacgag cgtgacacca cgatgcctgt agcaatggca 4620
acaacgttgc gcaaactatt aactggcgaa ctacttactc tagcttcccg gcaacaatta 4680
atagactgga tggaggcgga taaagttgca ggaccacttc tgcgctcggc ccttccggct 4740
ggctggttta ttgctgataa atctggagcc ggtgagcgtg ggtctcgcgg tatcattgca 4800
gcactggggc cagatggtaa gccctcccgt atcgtagtta tctacacgac ggggagtcag 4860
gcaactatgg atgaacgaaa tagacagatc gctgagatag gtgcctcact gattaagcat 4920
tggtaactgt cagaccaagt ttactcatat atactttaga ttgatttaaa acttcatttt 4980
taatttaaaa ggatctaggt gaagatcctt tttgataatc tcatgaccaa aatcccttaa 5040
cgtgagtttt cgttccactg agcgtcagac cccgtagaaa agatcaaagg atcttcttga 5100
gatccttttt ttctgcgcgt aatctgctgc ttgcaaacaa aaaaaccacc gctaccagcg 5160
gtggtttgtt tgccggatca agagctacca actctttttc cgaaggtaac tggcttcagc 5220
agagcgcaga taccaaatac tgttcttcta gtgtagccgt agttaggcca ccacttcaag 5280
aactctgtag caccgcctac atacctcgct ctgctaatcc tgttaccagt ggctgctgcc 5340
agtggcgata agtcgtgtct taccgggttg gactcaagac gatagttacc ggataaggcg 5400
cagcggtcgg gctgaacggg gggttcgtgc acacagccca gcttggagcg aacgacctac 5460
accgaactga gatacctaca gcgtgagcta tgagaaagcg ccacgcttcc cgaagggaga 5520
aaggcggaca ggtatccggt aagcggcagg gtcggaacag gagagcgcac gagggagctt 5580
ccagggggaa acgcctggta tctttatagt cctgtcgggt ttcgccacct ctgacttgag 5640
cgtcgatttt tgtgatgctc gtcagggggg cggagcctat ggaaaaacgc cagcaacgcg 5700
gcctttttac ggttcctggc cttttgctgg ccttttgctc acatggctcg acagatct 5758

<210> 183
<211> 5577
<212> DNA
<213> Homo sapiens
<220>
<221> misc_feature
<223> vecteur d'expression appelé GS-V35 contenant la séquence antisens
de GS-N35; antisens: 1108...1224

<400> 183
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840

gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgttgc ttggtgtgca ggcttgtccc tgtcctctgg 1140
ggcaaggccc tctggtgcat ctgccttata ggcagcagag gcagaggcct cagcgagctt 1200
tgcgatgtgt tccacggcct gctcgtcgac ccgggcggcc gcttcccttt agtgagggtt 1260
aatgcttcga gcagacatga taagatacat tgatgagttt ggacaaacca caactagaat 1320
gcagtgaaaa aaatgcttta tttgtgaaat ttgtgatgct attgctttat ttgtaaccat 1380
tataagctgc aataaacaag ttaacaacaa caattgcatt cattttatgt ttcaggttca 1440
gggggagatg tgggaggttt tttaaagcaa gtaaaacctc tacaaatgtg gtaaaatccg 1500
ataaggatcg atccgggctg gcgtaatagc gaagaggccc gcaccgatcg cccttcccaa 1560
cagttgcgca gcctgaatgg cgaatggacg cgccctgtag cggcgcatta agcgcggcgg 1620
gtgtggtggt tacgcgcagc gtgaccgcta cacttgccag cgccctagcg cccgctcctt 1680
tcgctttctt cccttccttt ctcgccacgt tcgccggctt tccccgtcaa gctctaaatc 1740
gggggctccc tttagggttc cgatttagtg ctttacggca cctcgacccc aaaaaacttg 1800
attagggtga tggttcacgt agtgggccat cgccctgata gacggttttt cgccctttga 1860
cgttggagtc cacgttcttt aatagtggac tcttgttcca aactggaaca acactcaacc 1920
ctatctcggt ctattctttt gatttataag ggattttgcc gatttcggcc tattggttaa 1980
aaaatgagct gatttaacaa aaatttaacg cgaattttaa caaaatatta acgcttacaa 2040
tttcctgatg cggtattttc tccttacgca tctgtgcggt atttcacacc gcatacgcgg 2100
atctgcgcag caccatggcc tgaaataacc tctgaaagag gaacttggtt aggtaccttc 2160
tgaggcggaa agaaccagct gtggaatgtg tgtcagttag ggtgtggaaa gtccccaggc 2220
tccccagcag gcagaagtat gcaaagcatg catctcaatt agtcagcaac caggtgtgga 2280
aagtccccag gctccccagc aggcagaagt atgcaaagca tgcatctcaa ttagtcagca 2340
accatagtcc cgcccctaac tccgcccatc ccgcccctaa ctccgcccag ttccgcccat 2400
tctccgcccc atggctgact aatttttttt atttatgcag aggccgaggc cgcctcggcc 2460
tctgagctat tccagaagta gtgaggaggc ttttttggag gcctaggctt ttgcaaaaag 2520
cttgattctt ctgacacaac agtctcgaac ttaaggctag agccaccatg attgaacaag 2580
atggattgca cgcaggttct ccggccgctt gggtggagag gctattcggc tatgactggg 2640
cacaacagac aatcggctgc tctgatgccg ccgtgttccg gctgtcagcg caggggcgcc 2700
cggttctttt tgtcaagacc gacctgtccg gtgccctgaa tgaactgcag gacgaggcag 2760
cgcggctatc gtggctggcc acgacgggcg ttccttgcgc agctgtgctc gacgttgtca 2820
ctgaagcggg aagggactgg ctgctattgg gcgaagtgcc ggggcaggat ctcctgtcat 2880
ctcaccttgc tcctgccgag aaagtatcca tcatggctga tgcaatgcgg cggctgcata 2940
cgcttgatcc ggctacctgc ccattcgacc accaagcgaa acatcgcatc gagcgagcac 3000
gtactcggat ggaagccggt cttgtcgatc aggatgatct ggacgaagag catcaggggc 3060
tcgcgccagc cgaactgttc gccaggctca aggcgcgcat gcccgacggc gaggatctcg 3120
tcgtgaccca tggcgatgcc tgcttgccga atatcatggt ggaaaatggc cgcttttctg 3180
gattcatcga ctgtggccgg ctgggtgtgg cggaccgcta tcaggacata gcgttggcta 3240
cccgtgatat tgctgaagag cttggcggcg aatgggctga ccgcttcctc gtgctttacg 3300
gtatcgccgc tcccgattcg cagcgcatcg ccttctatcg ccttcttgac gagttcttct 3360
gagcgggact ctggggttcg aaatgaccga ccaagcgacg cccaacctgc catcacgatg 3420
gccgcaataa aatatcttta ttttcattac atctgtgtgt tggttttttg tgtgaatcga 3480
tagcgataag gatccgcgta tggtgcactc tcagtacaat ctgctctgat gccgcatagt 3540
taagccagcc ccgacacccg ccaacacccg ctgacgcgcc ctgacgggct tgtctgctcc 3600
cggcatccgc ttacagacaa gctgtgaccg tctccgggag ctgcatgtgt cagaggtttt 3660
caccgtcatc accgaaacgc gcgagacgaa agggcctcgt gatacgccta tttttatagg 3720
ttaatgtcat gataataatg gtttcttaga cgtcaggtgg cacttttcgg ggaaatgtgc 3780
gcggaacccc tatttgttta tttttctaaa tacattcaaa tatgtatccg ctcatgagac 3840
aataaccctg ataaatgctt caataatatt gaaaaaggaa gagtatgagt attcaacatt 3900
tccgtgtcgc ccttattccc ttttttgcgg cattttgcct tcctgttttt gctcacccag 3960
aaacgctggt gaaagtaaaa gatgctgaag atcagttggg tgcacgagtg ggttacatcg 4020
aactggatct caacagcggt aagatccttg agagttttcg ccccgaagaa cgttttccaa 4080
tgatgagcac ttttaaagtt ctgctatgtg gcgcggtatt atcccgtatt gacgccgggc 4140
aagagcaact cggtcgccgc atacactatt ctcagaatga cttggttgag tactcaccag 4200
tcacagaaaa gcatcttacg gatggcatga cagtaagaga attatgcagt gctgccataa 4260
ccatgagtga taacactgcg gccaacttac ttctgacaac gatcggagga ccgaaggagc 4320
taaccgcttt tttgcacaac atgggggatc atgtaactcg ccttgatcgt tgggaaccgg 4380
agctgaatga agccatacca aacgacgagc gtgacaccac gatgcctgta gcaatggcaa 4440
caacgttgcg caaactatta actggcgaac tacttactct agcttcccgg caacaattaa 4500
tagactggat ggaggcggat aaagttgcag gaccacttct gcgctcggcc cttccggctg 4560
gctggtttat tgctgataaa tctggagccg gtgagcgtgg gtctcgcggt atcattgcag 4620
cactggggcc agatggtaag ccctcccgta tcgtagttat ctacacgacg gggagtcagg 4680
caactatgga tgaacgaaat agacagatcg ctgagatagg tgcctcactg attaagcatt 4740
ggtaactgtc agaccaagtt tactcatata tactttagat tgatttaaaa cttcattttt 4800
aatttaaaag gatctaggtg aagatccttt ttgataatct catgaccaaa atcccttaac 4860
gtgagttttc gttccactga gcgtcagacc ccgtagaaaa gatcaaagga tcttcttgag 4920
atcctttttt tctgcgcgta atctgctgct tgcaaacaaa aaaaccaccg ctaccagcgg 4980
tggtttgttt gccggatcaa gagctaccaa ctctttttcc gaaggtaact ggcttcagca 5040
gagcgcagat accaaatact gttcttctag tgtagccgta gttaggccac cacttcaaga 5100
actctgtagc accgcctaca tacctcgctc tgctaatcct gttaccagtg gctgctgcca 5160
gtggcgataa gtcgtgtctt accgggttgg actcaagacg atagttaccg gataaggcgc 5220

agcggtcggg ctgaacgggg ggttcgtgca cacagcccag cttggagcga acgacctaca 5280
ccgaactgag atacctacag cgtgagctat gagaaagcgc cacgcttccc gaagggagaa 5340
aggcggacag gtatccggta agcggcaggg tcggaacagg agagcgcacg agggagcttc 5400
cagggggaaa cgcctggtat ctttatagtc ctgtcgggtt tcgccacctc tgacttgagc 5460
gtcgattttt gtgatgctcg tcaggggggc ggagcctatg gaaaaacgcc agcaacgcgg 5520
cctttttacg gttcctggcc ttttgctggc cttttgctca catggctcga cagatct 5577

<210> 184
<211> 5556
<212> DNA
<213> Homo sapiens
<220>
<221> misc_feature
<223> vecteur d'expression appelé GS-V36 contenant la séquence antisens
      de GS-N36 ; antisens: 1108...1203

<400> 184
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgttgg agctgggtga gttgatgaag atgccactgg 1140
aatcataaga ggatcttgtt gtcttgatat cggagatctc atcagaggtt gtaggttcct 1200
ctggtcgacc cgggcggccg cttcccttta gtgagggtta atgcttcgag cagacatgat 1260
aagatacatt gatgagtttg gacaaaccac aactagaatg cagtgaaaaa aatgctttat 1320
ttgtgaaatt tgtgatgcta ttgctttatt tgtaaccatt ataagctgca ataaacaagt 1380
taacaacaac aattgcattc attttatgtt tcaggttcag ggggagatgt gggaggtttt 1440
ttaaagcaag taaaacctct acaaatgtgg taaaatccga taaggatcga tccgggctgg 1500
cgtaatagcg aagaggcccg caccgatcgc ccttcccaac agttgcgcag cctgaatggc 1560
gaatggacgc gccctgtagc ggcgcattaa gcgcggcggg tgtggtggtt acgcgcagcg 1620
tgaccgctac acttgccagc gccctagcgc ccgctccttt cgctttcttc ccttcctttc 1680
tcgccacgtt cgccggcttt ccccgtcaag ctctaaatcg ggggctccct ttagggttcc 1740
gatttagtgc tttacggcac ctcgacccca aaaaacttga ttagggtgat ggttcacgta 1800
gtgggccatc gccctgatag acggtttttc gccctttgac gttggagtcc acgttcttta 1860
atagtggact cttgttccaa actggaacaa cactcaaccc tatctcggtc tattcttttg 1920
atttataagg gattttgccg atttcggcct attggttaaa aaatgagctg atttaacaaa 1980
aatttaacgc gaattttaac aaaatattaa cgcttacaat ttcctgatgc ggtattttct 2040
ccttacgcat ctgtgcggta tttcacaccg catacgcgga tctgcgcagc accatggcct 2100
gaaataacct ctgaaagagg aacttggtta ggtaccttct gaggcggaaa gaaccagctg 2160
tggaatgtgt gtcagttagg gtgtggaaag tccccaggct ccccagcagg cagaagtatg 2220
caaagcatgc atctcaatta gtcagcaacc aggtgtggaa agtccccagg ctccccagca 2280
ggcagaagta tgcaaagcat gcatctcaat tagtcagcaa ccatagtccc gcccctaact 2340
ccgcccatcc cgcccctaac tccgcccagt tccgcccatt ctccgcccca tggctgacta 2400
atttttttta tttatgcaga ggccgaggcc gcctcggcct ctgagctatt ccagaagtag 2460
tgaggaggct tttttggagg cctaggcttt tgcaaaaagc ttgattcttc tgacacaaca 2520
gtctcgaact taaggctaga gccaccatga ttgaacaaga tggattgcac gcaggttctc 2580
cggccgcttg ggtggagagg ctattcggct atgactgggc acaacagaca atcggctgct 2640
ctgatgccgc cgtgttccgg ctgtcagcgc aggggcgccc ggttcttttt gtcaagaccg 2700
acctgtccgg tgccctgaat gaactgcagg acgaggcagc gcggctatcg tggctggcca 2760
cgacgggcgt tccttgcgca gctgtgctcg acgttgtcac tgaagcggga agggactggc 2820
tgctattggg cgaagtgccg gggcaggatc tcctgtcatc tcaccttgct cctgccgaga 2880
aagtatccat catggctgat gcaatgcggc ggctgcatac gcttgatccg gctacctgcc 2940
cattcgacca ccaagcgaaa catcgcatcg agcgagcacg tactcggatg gaagccggtc 3000
ttgtcgatca ggatgatctg gacgaagagc atcaggggct cgcgccagcc gaactgttcg 3060
ccaggctcaa ggcgcgcatg cccgacggcg aggatctcgt cgtgacccat ggcgatgcct 3120
gcttgccgaa tatcatggtg gaaaatggcc gcttttctgg attcatcgac tgtggccggc 3180
tgggtgtggc ggaccgctat caggacatag cgttggctac ccgtgatatt gctgaagagc 3240
ttggcggcga atgggctgac cgcttcctcg tgctttacgg tatcgccgct cccgattcgc 3300

agcgcatcgc cttctatcgc cttcttgacg agttcttctg agcgggactc tggggttcga 3360
aatgaccgac caagcgacgc ccaacctgcc atcacgatgg ccgcaataaa atatctttat 3420
tttcattaca tctgtgtgtt ggttttttgt gtgaatcgat agcgataagg atccgcgtat 3480
ggtgcactct cagtacaatc tgctctgatg ccgcatagtt aagccagccc cgacacccgc 3540
caacacccgc tgacgcgccc tgacgggctt gtctgctccc ggcatccgct tacagacaag 3600
ctgtgaccgt ctccgggagc tgcatgtgtc agaggttttc accgtcatca ccgaaacgcg 3660
cgagacgaaa gggcctcgtg atacgcctat ttttataggt taatgtcatg ataataatgg 3720
tttcttagac gtcaggtggc acttttcggg gaaatgtgcg cggaacccct atttgtttat 3780
ttttctaaat acattcaaat atgtatccgc tcatgagaca ataaccctga taaatgcttc 3840
aataatattg aaaaaggaag agtatgagta ttcaacattt ccgtgtcgcc cttattccct 3900
tttttgcggc attttgcctt cctgtttttg ctcacccaga aacgctggtg aaagtaaaag 3960
atgctgaaga tcagttgggt gcacgagtgg gttacatcga actggatctc aacagcggta 4020
agatccttga gagttttcgc cccgaagaac gttttccaat gatgagcact tttaaagttc 4080
tgctatgtgg cgcggtatta tcccgtattg acgccgggca agagcaactc ggtcgccgca 4140
tacactattc tcagaatgac ttggttgagt actcaccagt cacagaaaag catcttacgg 4200
atggcatgac agtaagagaa ttatgcagtg ctgccataac catgagtgat aacactgcgg 4260
ccaacttact tctgacaacg atcggaggac cgaaggagct aaccgctttt ttgcacaaca 4320
tgggggatca tgtaactcgc cttgatcgtt gggaaccgga gctgaatgaa gccataccaa 4380
acgacgagcg tgacaccacg atgcctgtag caatggcaac aacgttgcgc aaactattaa 4440
ctggcgaact acttactcta gcttcccggc aacaattaat agactggatg gaggcggata 4500
aagttgcagg accacttctg cgctcggccc ttccggctgg ctggtttatt gctgataaat 4560
ctggagccgg tgagcgtggg tctcgcggta tcattgcagc actggggcca gatggtaagc 4620
cctcccgtat cgtagttatc tacacgacgg ggagtcaggc aactatggat gaacgaaata 4680
gacagatcgc tgagataggt gcctcactga ttaagcattg gtaactgtca gaccaagttt 4740
actcatatat actttagatt gatttaaaac ttcattttta atttaaaagg atctaggtga 4800
agatcctttt tgataatctc atgaccaaaa tcccttaacg tgagttttcg ttccactgag 4860
cgtcagaccc cgtagaaaag atcaaaggat cttcttgaga tccttttttt ctgcgcgtaa 4920
tctgctgctt gcaaacaaaa aaaccaccgc taccagcggt ggtttgtttg ccggatcaag 4980
agctaccaac tctttttccg aaggtaactg gcttcagcag agcgcagata ccaaatactg 5040
ttcttctagt gtagccgtag ttaggccacc acttcaagaa ctctgtagca ccgcctacat 5100
acctcgctct gctaatcctg ttaccagtgg ctgctgccag tggcgataag tcgtgtctta 5160
ccgggttgga ctcaagacga tagttaccgg ataaggcgca gcggtcgggc tgaacggggg 5220
gttcgtgcac acagcccagc ttggagcgaa cgacctacac cgaactgaga tacctacagc 5280
gtgagctatg agaaagcgcc acgcttcccg aagggagaaa ggcggacagg tatccggtaa 5340
gcggcagggt cggaacagga gagcgcacga gggagcttcc agggggaaac gcctggtatc 5400
tttatagtcc tgtcgggttt cgccacctct gacttgagcg tcgatttttg tgatgctcgt 5460
caggggggcg gagcctatgg aaaaacgcca gcaacgcggc ctttttacgg ttcctggcct 5520
tttgctggcc ttttgctcac atggctcgac agatct 5556

<210> 185
<211> 5853
<212> DNA
<213> Homo sapiens
<220>
<221> misc_feature
<223> vecteur d'expression appelé GS-V37 contenant la séquence antisens
de GS-N37 ; antisens: 1108...1500

<400> 185
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgttcc aaggatttgg cctgtggctg gggaaataac 1140
acacacgaga taggtaacaa ggatttacac tgacatatgg ggtgttggtt tggctctatt 1200
ctttttcggt taccacttct tgcagtgtag gtggagatgc ctctgttcct tcttcccagc 1260
cctcagcaac accagcaagc tcgtagtgct ttttccgaag ctctcccagt ttttgacgct 1320
ccttctgcaa ttcattttct agctctagca ccctaacctg agaatccatc tcttggcgtt 1380

tgatctgtgt cagcgtcatg cttgagaagt ccatgttgtc tgtctcttcg atctgtgatt 1440
tgccggaaat ggttgaggcc acaacgccgg cagtggcctg gttcactccc cgagaggcct 1500
gtcgacccgg gcggccgctt ccctttagtg agggttaatg cttcgagcag acatgataag 1560
atacattgat gagtttggac aaaccacaac tagaatgcag tgaaaaaaat gctttatttg 1620
tgaaatttgt gatgctattg ctttatttgt aaccattata agctgcaata aacaagttaa 1680
caacaacaat tgcattcatt ttatgtttca ggttcagggg gagatgtggg aggtttttta 1740
aagcaagtaa aacctctaca aatgtggtaa aatccgataa ggatcgatcc gggctggcgt 1800
aatagcgaag aggcccgcac cgatcgccct tcccaacagt tgcgcagcct gaatggcgaa 1860
tggacgcgcc ctgtagcggc gcattaagcg cggcgggtgt ggtggttacg cgcagcgtga 1920
ccgctacact tgccagcgcc ctagcgcccg ctcctttcgc tttcttccct tcctttctcg 1980
ccacgttcgc cggctttccc cgtcaagctc taaatcgggg gctcccttta gggttccgat 2040
ttagtgcttt acggcacctc gaccccaaaa aacttgatta gggtgatggt tcacgtagtg 2100
ggccatcgcc ctgatagacg gtttttcgcc ctttgacgtt ggagtccacg ttctttaata 2160
gtggactctt gttccaaact ggaacaacac tcaaccctat ctcggtctat tcttttgatt 2220
tataagggat tttgccgatt tcggcctatt ggttaaaaaa tgagctgatt taacaaaaat 2280
ttaacgcgaa ttttaacaaa atattaacgc ttacaatttc ctgatgcggt attttctcct 2340
tacgcatctg tgcggtattt cacaccgcat acgcggatct gcgcagcacc atggcctgaa 2400
ataacctctg aaagaggaac ttggttaggt accttctgag gcggaaagaa ccagctgtgg 2460
aatgtgtgtc agttagggtg tggaaagtcc ccaggctccc cagcaggcag aagtatgcaa 2520
agcatgcatc tcaattagtc agcaaccagg tgtggaaagt ccccaggctc cccagcaggc 2580
agaagtatgc aaagcatgca tctcaattag tcagcaacca tagtcccgcc cctaactccg 2640
cccatcccgc ccctaactcc gcccagttcc gcccattctc cgccccatgg ctgactaatt 2700
ttttttattt atgcagaggc cgaggccgcc tcggcctctg agctattcca gaagtagtga 2760
ggaggctttt ttggaggcct aggcttttgc aaaaagcttg attcttctga cacaacagtc 2820
tcgaacttaa ggctagagcc accatgattg aacaagatgg attgcacgca ggttctccgg 2880
ccgcttgggt ggagaggcta ttcggctatg actgggcaca acagacaatc ggctgctctg 2940
atgccgccgt gttccggctg tcagcgcagg ggcgcccggt tctttttgtc aagaccgacc 3000
tgtccggtgc cctgaatgaa ctgcaggacg aggcagcgcg gctatcgtgg ctggccacga 3060
cgggcgttcc ttgcgcagct gtgctcgacg ttgtcactga agcgggaagg gactggctgc 3120
tattgggcga agtgccgggg caggatctcc tgtcatctca ccttgctcct gccgagaaag 3180
tatccatcat ggctgatgca atgcggcggc tgcatacgct tgatccggct acctgcccat 3240
tcgaccacca agcgaaacat cgcatcgagc gagcacgtac tcggatggaa gccggtcttg 3300
tcgatcagga tgatctggac gaagagcatc aggggctcgc gccagccgaa ctgttcgcca 3360
ggctcaaggc gcgcatgccc gacggcgagg atctcgtcgt gacccatggc gatgcctgct 3420
tgccgaatat catggtggaa aatggccgct tttctggatt catcgactgt ggccggctgg 3480
gtgtggcgga ccgctatcag gacatagcgt tggctacccg tgatattgct gaagagcttg 3540
gcggcgaatg ggctgaccgc ttcctcgtgc tttacggtat cgccgctccc gattcgcagc 3600
gcatcgcctt ctatcgcctt cttgacgagt tcttctgagc gggactctgg ggttcgaaat 3660
gaccgaccaa gcgacgccca acctgccatc acgatggccg caataaaata tctttatttt 3720
cattacatct gtgtgttggt tttttgtgtg aatcgatagc gataaggatc cgcgtatggt 3780
gcactctcag tacaatctgc tctgatgccg catagttaag ccagccccga cacccgccaa 3840
cacccgctga cgcgccctga cgggcttgtc tgctcccggc atccgcttac agacaagctg 3900
tgaccgtctc cgggagctgc atgtgtcaga ggttttcacc gtcatcaccg aaacgcgcga 3960
gacgaaaggg cctcgtgata cgcctatttt tataggttaa tgtcatgata ataatggttt 4020
cttagacgtc aggtggcact tttcggggaa atgtgcgcgg aacccctatt tgtttatttt 4080
tctaaataca ttcaaatatg tatccgctca tgagacaata accctgataa atgcttcaat 4140
aatattgaaa aaggaagagt atgagtattc aacatttccg tgtcgccctt attccctttt 4200
ttgcggcatt ttgccttcct gtttttgctc acccagaaac gctggtgaaa gtaaaagatg 4260
ctgaagatca gttgggtgca cgagtgggtt acatcgaact ggatctcaac agcggtaaga 4320
tccttgagag ttttcgcccc gaagaacgtt ttccaatgat gagcactttt aaagttctgc 4380
tatgtggcgc ggtattatcc cgtattgacg ccgggcaaga gcaactcggt cgccgcatac 4440
actattctca gaatgacttg gttgagtact caccagtcac agaaaagcat cttacggatg 4500
gcatgacagt aagagaatta tgcagtgctg ccataaccat gagtgataac actgcggcca 4560
acttacttct gacaacgatc ggaggaccga aggagctaac cgcttttttg cacaacatgg 4620
gggatcatgt aactcgcctt gatcgttggg aaccggagct gaatgaagcc ataccaaacg 4680
acgagcgtga caccacgatg cctgtagcaa tggcaacaac gttgcgcaaa ctattaactg 4740
gcgaactact tactctagct tcccggcaac aattaataga ctggatggag gcggataaag 4800
ttgcaggacc acttctgcgc tcggcccttc cggctggctg gtttattgct gataaatctg 4860
gagccggtga gcgtgggtct cgcggtatca ttgcagcact ggggccagat ggtaagccct 4920
cccgtatcgt agttatctac acgacgggga gtcaggcaac tatggatgaa cgaaatagac 4980
agatcgctga gataggtgcc tcactgatta agcattggta actgtcagac caagtttact 5040
catatatact ttagattgat ttaaaacttc atttttaatt taaaaggatc taggtgaaga 5100
tcctttttga taatctcatg accaaaatcc cttaacgtga gttttcgttc cactgagcgt 5160
cagaccccgt agaaaagatc aaaggatctt cttgagatcc tttttttctg cgcgtaatct 5220
gctgcttgca aacaaaaaaa ccaccgctac cagcggtggt ttgtttgccg gatcaagagc 5280
taccaactct ttttccgaag gtaactggct tcagcagagc gcagatacca aatactgttc 5340
ttctagtgta gccgtagtta ggccaccact tcaagaactc tgtagcaccg cctacatacc 5400
tcgctctgct aatcctgtta ccagtggctg ctgccagtgg cgataagtcg tgtcttaccg 5460
ggttggactc aagacgatag ttaccggata aggcgcagcg gtcgggctga acggggggtt 5520
cgtgcacaca gcccagcttg gagcgaacga cctacaccga actgagatac ctacagcgtg 5580
agctatgaga aagcgccacg cttcccgaag ggagaaaggc ggacaggtat ccggtaagcg 5640
gcagggtcgg aacaggagag cgcacgaggg agcttccagg gggaaacgcc tggtatcttt 5700
atagtcctgt cgggtttcgc cacctctgac ttgagcgtcg atttttgtga tgctcgtcag 5760

gggggcggag cctatggaaa aacgccagca acgcggcctt tttacggttc ctggcctttt 5820
gctggccttt tgctcacatg gctcgacaga tct 5853

<210> 186
<211> 5560
<212> DNA
<213> Homo sapiens
<220>
<221> misc_feature
<223> vecteur d'expression appelé GS-V38 contenant la séquence antisens
de GS-N38 ; antisens: 1108..1207

<400> 186
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtctg tagagagttc ctgtaaacta tccttatctt 1140
cttcatctga tccaagaaat ttgtgaagtt tatgaatcca agttagccca acactatgta 1200
caccagcgtc gacccgggcg gccgcttccc tttagtgagg gttaatgctt cgagcagaca 1260
tgataagata cattgatgag tttggacaaa ccacaactag aatgcagtga aaaaaatgct 1320
ttatttgtga aatttgtgat gctattgctt tatttgtaac cattataagc tgcaataaac 1380
aagttaacaa caacaattgc attcatttta tgtttcaggt tcagggggag atgtgggagg 1440
ttttttaaag caagtaaaac ctctacaaat gtggtaaaat ccgataagga tcgatccggg 1500
ctggcgtaat agcgaagagg cccgcaccga tcgcccttcc caacagttgc gcagcctgaa 1560
tggcgaatgg acgcgccctg tagcggcgca ttaagcgcgg cgggtgtggt ggttacgcgc 1620
agcgtgaccg ctacacttgc cagcgcccta gcgcccgctc ctttcgcttt cttcccttcc 1680
tttctcgcca cgttcgccgg ctttccccgt caagctctaa atcgggggct ccctttaggg 1740
ttccgattta gtgctttacg gcacctcgac cccaaaaaac ttgattaggg tgatggttca 1800
cgtagtgggc catcgccctg atagacggtt tttcgccctt tgacgttgga gtccacgttc 1860
tttaatagtg gactcttgtt ccaaactgga acaacactca accctatctc ggtctattct 1920
tttgatttat aagggatttt gccgatttcg gcctattggt taaaaaatga gctgatttaa 1980
caaaaattta acgcgaattt taacaaaata ttaacgctta caatttcctg atgcggtatt 2040
ttctccttac gcatctgtgc ggtatttcac accgcatacg cggatctgcg cagcaccatg 2100
gcctgaaata acctctgaaa gaggaacttg gttaggtacc ttctgaggcg gaaagaacca 2160
gctgtggaat gtgtgtcagt tagggtgtgg aaagtcccca ggctccccag caggcagaag 2220
tatgcaaagc atgcatctca attagtcagc aaccaggtgt ggaaagtccc caggctcccc 2280
agcaggcaga agtatgcaaa gcatgcatct caattagtca gcaaccatag tcccgcccct 2340
aactccgccc atcccgcccc taactccgcc cagttccgcc cattctccgc cccatggctg 2400
actaattttt tttatttatg cagaggccga ggccgcctcg gcctctgagc tattccagaa 2460
gtagtgagga ggcttttttg gaggcctagg cttttgcaaa aagcttgatt cttctgacac 2520
aacagtctcg aacttaaggc tagagccacc atgattgaac aagatggatt gcacgcaggt 2580
tctccggccg cttgggtgga gaggctattc ggctatgact gggcacaaca gacaatcggc 2640
tgctctgatg ccgccgtgtt ccggctgtca gcgcaggggc gcccggttct ttttgtcaag 2700
accgacctgt ccggtgccct gaatgaactg caggacgagg cagcgcggct atcgtggctg 2760
gccacgacgg gcgttccttg cgcagctgtg ctcgacgttg tcactgaagc gggaagggac 2820
tggctgctat tgggcgaagt gccggggcag gatctcctgt catctcacct tgctcctgcc 2880
gagaaagtat ccatcatggc tgatgcaatg cggcggctgc atacgcttga tccggctacc 2940
tgcccattcg accaccaagc gaaacatcgc atcgagcgag cacgtactcg gatggaagcc 3000
ggtcttgtcg atcaggatga tctggacgaa gagcatcagg ggctcgcgcc agccgaactg 3060
ttcgccaggc tcaaggcgcg catgcccgac ggcgaggatc tcgtcgtgac ccatggcgat 3120
gcctgcttgc cgaatatcat ggtggaaaat ggccgctttt ctggattcat cgactgtggc 3180
cggctgggtg tggcggaccg ctatcaggac atagcgttgg ctacccgtga tattgctgaa 3240
gagcttggcg gcgaatgggc tgaccgcttc ctcgtgcttt acggtatcgc cgctcccgat 3300
tcgcagcgca tcgccttcta tcgccttctt gacgagttct tctgagcggg actctggggt 3360
tcgaaatgac cgaccaagcg acgcccaacc tgccatcacg atggccgcaa taaaatatct 3420
ttattttcat tacatctgtg tgttggtttt ttgtgtgaat cgatagcgat aaggatccgc 3480
gtatggtgca ctctcagtac aatctgctct gatgccgcat agttaagcca gccccgacac 3540

ccgccaacac ccgctgacgc gccctgacgg gcttgtctgc tcccggcatc cgcttacaga 3600
caagctgtga ccgtctccgg gagctgcatg tgtcagaggt tttcaccgtc atcaccgaaa 3660
cgcgcgagac gaaagggcct cgtgatacgc ctatttttat aggttaatgt catgataata 3720
atggtttctt agacgtcagg tggcactttt cggggaaatg tgcgcggaac ccctatttgt 3780
ttatttttct aaatacattc aaatatgtat ccgctcatga gacaataacc ctgataaatg 3840
cttcaataat attgaaaaag gaagagtatg agtattcaac atttccgtgt cgcccttatt 3900
cccttttttg cggcattttg ccttcctgtt tttgctcacc cagaaacgct ggtgaaagta 3960
aaagatgctg aagatcagtt gggtgcacga gtgggttaca tcgaactgga tctcaacagc 4020
ggtaagatcc ttgagagttt tcgccccgaa gaacgttttc caatgatgag cacttttaaa 4080
gttctgctat gtggcgcggt attatcccgt attgacgccg ggcaagagca actcggtcgc 4140
cgcatacact attctcagaa tgacttggtt gagtactcac cagtcacaga aaagcatctt 4200
acggatggca tgacagtaag agaattatgc agtgctgcca taaccatgag tgataacact 4260
gcggccaact tacttctgac aacgatcgga ggaccgaagg agctaaccgc ttttttgcac 4320
aacatggggg atcatgtaac tcgccttgat cgttgggaac cggagctgaa tgaagccata 4380
ccaaacgacg agcgtgacac cacgatgcct gtagcaatgg caacaacgtt gcgcaaacta 4440
ttaactggcg aactacttac tctagcttcc cggcaacaat taatagactg gatggaggcg 4500
gataaagttg caggaccact tctgcgctcg gcccttccgg ctggctggtt tattgctgat 4560
aaatctggag ccggtgagcg tgggtctcgc ggtatcattg cagcactggg gccagatggt 4620
aagccctccc gtatcgtagt tatctacacg acggggagtc aggcaactat ggatgaacga 4680
aatagacaga tcgctgagat aggtgcctca ctgattaagc attggtaact gtcagaccaa 4740
gtttactcat atatacttta gattgattta aaacttcatt tttaatttaa aaggatctag 4800
gtgaagatcc tttttgataa tctcatgacc aaaatccctt aacgtgagtt ttcgttccac 4860
tgagcgtcag accccgtaga aaagatcaaa ggatcttctt gagatccttt ttttctgcgc 4920
gtaatctgct gcttgcaaac aaaaaaacca ccgctaccag cggtggtttg tttgccggat 4980
caagagctac caactctttt tccgaaggta actggcttca gcagagcgca gataccaaat 5040
actgttcttc tagtgtagcc gtagttaggc caccacttca agaactctgt agcaccgcct 5100
acatacctcg ctctgctaat cctgttacca gtggctgctg ccagtggcga taagtcgtgt 5160
cttaccgggt tggactcaag acgatagtta ccggataagg cgcagcggtc gggctgaacg 5220
gggggttcgt gcacacagcc cagcttggag cgaacgacct acaccgaact gagataccta 5280
cagcgtgagc tatgagaaag cgccacgctt cccgaaggga gaaaggcgga caggtatccg 5340
gtaagcggca gggtcggaac aggagagcgc acgagggagc ttccaggggg aaacgcctgg 5400
tatctttata gtcctgtcgg gtttcgccac ctctgacttg agcgtcgatt tttgtgatgc 5460
tcgtcagggg ggcggagcct atggaaaaac gccagcaacg cggccttttt acggttcctg 5520
gccttttgct ggccttttgc tcacatggct cgacagatct 5560

<210> 187
<211> 5550
<212> DNA
<213> Homo sapiens
<220>
<221> misc_feature
<223> vecteur d'expression appelé GS-V39 contenant la séquence antisens
 de GS-N39 ; antisens: 1108...1197

<400> 187
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtgct gggggaaggg tgcagcaacg atttctcacc 1140
aaatcactac acaggacagc aaaggggtga gaaggggctg agggaggaaa agccagggtc 1200
gacccgggcg gccgcttccc tttagtgagg gttaatgctt cgagcagaca tgataagata 1260
cattgatgag tttggacaaa ccacaactag aatgcagtga aaaaaatgct ttatttgtga 1320
aatttgtgat gctattgctt tatttgtaac cattataagc tgcaataaac aagttaacaa 1380
caacaattgc attcatttta tgtttcaggt tcagggggag atgtgggagg ttttttaaag 1440
caagtaaaac ctctacaaat gtggtaaaat ccgataagga tcgatccggg ctggcgtaat 1500
agcgaagagg cccgcaccga tcgcccttcc caacagttgc gcagcctgaa tggcgaatgg 1560
acgcgccctg tagcggcgca ttaagcgcgg cgggtgtggt ggttacgcgc agcgtgaccg 1620

ctacacttgc cagcgcccta gcgcccgctc ctttcgcttt cttcccttcc tttctcgcca 1680
cgttcgccgg ctttccccgt caagctctaa atcgggggct ccctttaggg ttccgattta 1740
gtgctttacg gcacctcgac cccaaaaaac ttgattaggg tgatggttca cgtagtgggc 1800
catcgccctg atagacggtt tttcgccctt tgacgttgga gtccacgttc tttaatagtg 1860
gactcttgtt ccaaactgga acaacactca accctatctc ggtctattct tttgatttat 1920
aagggatttt gccgatttcg gcctattggt taaaaaatga gctgatttaa caaaaattta 1980
acgcgaattt taacaaaata ttaacgctta caatttcctg atgcggtatt ttctccttac 2040
gcatctgtgc ggtatttcac accgcatacg cggatctgcg cagcaccatg gcctgaaata 2100
acctctgaaa gaggaacttg gttaggtacc ttctgaggcg gaaagaacca gctgtggaat 2160
gtgtgtcagt tagggtgtgg aaagtcccca ggctccccag caggcagaag tatgcaaagc 2220
atgcatctca attagtcagc aaccaggtgt ggaaagtccc caggctcccc agcaggcaga 2280
agtatgcaaa gcatgcatct caattagtca gcaaccatag tcccgcccct aactccgccc 2340
atcccgcccc taactccgcc cagttccgcc cattctccgc cccatggctg actaattttt 2400
tttatttatg cagaggccga ggccgcctcg gcctctgagc tattccagaa gtagtgagga 2460
ggcttttttg gaggcctagg cttttgcaaa aagcttgatt cttctgacac aacagtctcg 2520
aacttaaggc tagagccacc atgattgaac aagatggatt gcacgcaggt tctccggccg 2580
cttgggtgga gaggctattc ggctatgact gggcacaaca gacaatcggc tgctctgatg 2640
ccgccgtgtt ccggctgtca gcgcaggggc gcccggttct ttttgtcaag accgacctgt 2700
ccggtgccct gaatgaactg caggacgagg cagcgcggct atcgtggctg gccacgacgg 2760
gcgttccttg cgcagctgtg ctcgacgttg tcactgaagc gggaagggac tggctgctat 2820
tgggcgaagt gccggggcag gatctcctgt catctcacct tgctcctgcc gagaaagtat 2880
ccatcatggc tgatgcaatg cggcggctgc atacgcttga tccggctacc tgcccattcg 2940
accaccaagc gaaacatcgc atcgagcgag cacgtactcg gatggaagcc ggtcttgtcg 3000
atcaggatga tctggacgaa gagcatcagg ggctcgcgcc agccgaactg ttcgccaggc 3060
tcaaggcgcg catgcccgac ggcgaggatc tcgtcgtgac ccatggcgat gcctgcttgc 3120
cgaatatcat ggtggaaaat ggccgctttt ctggattcat cgactgtggc cggctgggtg 3180
tggcggaccg ctatcaggac atagcgttgg ctacccgtga tattgctgaa gagcttggcg 3240
gcgaatgggc tgaccgcttc ctcgtgcttt acggtatcgc cgctcccgat tcgcagcgca 3300
tcgccttcta tcgccttctt gacgagttct tctgagcggg actctggggt tcgaaatgac 3360
cgaccaagcg acgcccaacc tgccatcacg atggccgcaa taaaatatct ttattttcat 3420
tacatctgtg tgttggtttt ttgtgtgaat cgatagcgat aaggatccgc gtatggtgca 3480
ctctcagtac aatctgctct gatgccgcat agttaagcca gccccgacac ccgccaacac 3540
ccgctgacgc gccctgacgg gcttgtctgc tcccggcatc cgcttacaga caagctgtga 3600
ccgtctccgg gagctgcatg tgtcagaggt tttcaccgtc atcaccgaaa cgcgcgagac 3660
gaaagggcct cgtgatacgc ctatttttat aggttaatgt catgataata atggtttctt 3720
agacgtcagg tggcactttt cggggaaatg tgcgcggaac ccctatttgt ttatttttct 3780
aaatacattc aaatatgtat ccgctcatga gacaataacc ctgataaatg cttcaataat 3840
attgaaaaag gaagagtatg agtattcaac atttccgtgt cgcccttatt cccttttttg 3900
cggcattttg ccttcctgtt tttgctcacc cagaaacgct ggtgaaagta aaagatgctg 3960
aagatcagtt gggtgcacga gtgggttaca tcgaactgga tctcaacagc ggtaagatcc 4020
ttgagagttt tcgccccgaa gaacgttttc caatgatgag cacttttaaa gttctgctat 4080
gtggcgcggt attatcccgt attgacgccg ggcaagagca actcggtcgc cgcatacact 4140
attctcagaa tgacttggtt gagtactcac cagtcacaga aaagcatctt acggatggca 4200
tgacagtaag agaattatgc agtgctgcca taaccatgag tgataacact gcggccaact 4260
tacttctgac aacgatcgga ggaccgaagg agctaaccgc ttttttgcac aacatggggg 4320
atcatgtaac tcgccttgat cgttgggaac cggagctgaa tgaagccata ccaaacgacg 4380
agcgtgacac cacgatgcct gtagcaatgg caacaacgtt gcgcaaacta ttaactggcg 4440
aactacttac tctagcttcc cggcaacaat taatagactg gatggaggcg gataaagttg 4500
caggaccact tctgcgctcg gcccttccgg ctggctggtt tattgctgat aaatctggag 4560
ccggtgagcg tgggtctcgc ggtatcattg cagcactggg gccagatggt aagccctccc 4620
gtatcgtagt tatctacacg acggggagtc aggcaactat ggatgaacga aatagacaga 4680
tcgctgagat aggtgcctca ctgattaagc attggtaact gtcagaccaa gtttactcat 4740
atatacttta gattgattta aaacttcatt tttaatttaa aaggatctag gtgaagatcc 4800
tttttgataa tctcatgacc aaaatccctt aacgtgagtt ttcgttccac tgagcgtcag 4860
accccgtaga aaagatcaaa ggatcttctt gagatccttt ttttctgcgc gtaatctgct 4920
gcttgcaaac aaaaaaacca ccgctaccag cggtggtttg tttgccggat caagagctac 4980
caactctttt tccgaaggta actggcttca gcagagcgca gataccaaat actgttcttc 5040
tagtgtagcc gtagttaggc caccacttca agaactctgt agcaccgcct acatacctcg 5100
ctctgctaat cctgttacca gtggctgctg ccagtggcga taagtcgtgt cttaccgggt 5160
tggactcaag acgatagtta ccggataagg cgcagcggtc gggctgaacg gggggttcgt 5220
gcacacagcc cagcttggag cgaacgacct acaccgaact gagataccta cagcgtgagc 5280
tatgagaaag cgccacgctt cccgaaggga gaaaggcgga caggtatccg gtaagcggca 5340
gggtcggaac aggagagcgc acgagggagc ttccaggggg aaacgcctgg tatctttata 5400
gtcctgtcgg gtttcgccac ctctgacttg agcgtcgatt tttgtgatgc tcgtcagggg 5460
ggcggagcct atggaaaaac gccagcaacg cggccttttt acggttcctg gccttttgct 5520
ggccttttgc tcacatggct cgacagatct 5550

<210> 188
<211> 5604
<212> DNA
<213> Homo sapiens
<220>
<221> misc_feature

<223>  vecteur d'expression appelé GS-V40 contenant la séquence antisens
       de GS-N40 ; antisens: 1108...1251

<400>  188
tcaatattgg ccattagcca tattattcat tggttatata gcataaatca atattggcta      60
ttggccattg catacgttgt atctatatca taatatgtac atttatattg gctcatgtcc     120
aatatgaccg ccatgttggc attgattatt gactagttat taatagtaat caattacggg     180
gtcattagtt catagcccat atatggagtt ccgcgttaca taacttacgg taaatggccc     240
gcctggctga ccgcccaacg acccccgccc attgacgtca ataatgacgt atgttcccat     300
agtaacgcca atagggactt tccattgacg tcaatgggtg gagtatttac ggtaaactgc     360
ccacttggca gtacatcaag tgtatcatat gccaagtccg ccccctattg acgtcaatga     420
cggtaaatgg cccgcctggc attatgccca gtacatgacc ttacgggact ttcctacttg     480
gcagtacatc tacgtattag tcatcgctat taccatggtg atgcggtttt ggcagtacac     540
caatgggcgt ggatagcggt ttgactcacg gggatttcca agtctccacc ccattgacgt     600
caatgggagt ttgttttggc accaaaatca acgggacttt ccaaaatgtc gtaacaactg     660
cgatcgcccg ccccgttgac gcaaatgggc ggtaggcgtg tacggtggga ggtctatata     720
agcagagctc gtttagtgaa ccgtcagatc actagaagct ttattgcggt agtttatcac     780
agttaaattg ctaacgcagt cagtgcttct gacacaacag tctcgaactt aagctgcagt     840
gactctctta aggtagcctt gcagaagttg gtcgtgaggc actgggcagg taagtatcaa     900
ggttacaaga caggtttaag gagaccaata gaaactgggc ttgtcgagac agagaagact     960
cttgcgtttc tgataggcac ctattggtct tactgacatc cactttgcct ttctctccac    1020
aggtgtccac tcccagttca attacagctc ttaaggctag agtacttaat acgactcact    1080
ataggctagc ctcgagaatt cacgcgttgg aggaagatcc actgggaaat tccctgaggc    1140
caggaaggtt cagcttcagt ccatttggtc tacagacacc ttgattctcc agaggaaaat    1200
tctttgactt ttgagaagat tgaacagcag gatcatcatc aaaggtgacc cgtcgacccg    1260
ggcggccgct tccctttagt gagggttaat gcttcgagca gacatgataa gatacattga    1320
tgagtttgga caaaccacaa ctagaatgca gtgaaaaaaa tgctttattt gtgaaatttg    1380
tgatgctatt gctttatttg taaccattat aagctgcaat aaacaagtta acaacaacaa    1440
ttgcattcat tttatgtttc aggttcaggg ggagatgtgg gaggtttttt aaagcaagta    1500
aaacctctac aaatgtggta aaatccgata aggatcgatc cgggctggcg taatagcgaa    1560
gaggcccgca ccgatcgccc ttcccaacag ttgcgcagcc tgaatggcga atggacgcgc    1620
cctgtagcgg cgcattaagc gcggcgggtg tggtggttac gcgcagcgtg accgctacac    1680
ttgccagcgc cctagcgccc gctcctttcg ctttcttccc ttcctttctc gccacgttcg    1740
ccggctttcc ccgtcaagct ctaaatcggg ggctcccttt agggttccga tttagtgctt    1800
tacggcacct cgaccccaaa aaacttgatt agggtgatgg ttcacgtagt gggccatcgc    1860
cctgatagac ggtttttcgc cctttgacgt tggagtccac gttctttaat agtggactct    1920
tgttccaaac tggaacaaca ctcaacccta tctcggtcta ttcttttgat ttataaggga    1980
ttttgccgat ttcggcctat tggttaaaaa atgagctgat ttaacaaaaa tttaacgcga    2040
attttaacaa aatattaacg cttacaattt cctgatgcgg tattttctcc ttacgcatct    2100
gtgcggtatt tcacaccgca tacgcggatc tgcgcagcac catggcctga aataacctct    2160
gaaagaggaa cttggttagg taccttctga ggcggaaaga accagctgtg gaatgtgtgt    2220
cagttagggt gtggaaagtc cccaggctcc ccagcaggca gaagtatgca aagcatgcat    2280
ctcaattagt cagcaaccag gtgtggaaag tccccaggct ccccagcagg cagaagtatg    2340
caaagcatgc atctcaatta gtcagcaacc atagtcccgc ccctaactcc gcccatcccg    2400
cccctaactc cgcccagttc cgcccattct ccgccccatg gctgactaat tttttttatt    2460
tatgcagagg ccgaggccgc ctcggcctct gagctattcc agaagtagtg aggaggcttt    2520
tttggaggcc taggcttttg caaaaagctt gattcttctg acacaacagt ctcgaactta    2580
aggctagagc caccatgatt gaacaagatg gattgcacgc aggttctccg gccgcttggg    2640
tggagaggct attcggctat gactgggcac aacagacaat cggctgctct gatgccgccg    2700
tgttccggct gtcagcgcag gggcgcccgg ttctttttgt caagaccgac ctgtccggtg    2760
ccctgaatga actgcaggac gaggcagcgc ggctatcgtg gctggccacg acgggcgttc    2820
cttgcgcagc tgtgctcgac gttgtcactg aagcgggaag ggactggctg ctattgggcg    2880
aagtgccggg gcaggatctc ctgtcatctc accttgctcc tgccgagaaa gtatccatca    2940
tggctgatgc aatgcggcgg ctgcatacgc ttgatccggc tacctgccca ttcgaccacc    3000
aagcgaaaca tcgcatcgag cgagcacgta ctcggatgga agccggtctt gtcgatcagg    3060
atgatctgga cgaagagcat caggggctcg cgccagccga actgttcgcc aggctcaagg    3120
cgcgcatgcc cgacggcgag gatctcgtcg tgacccatgg cgatgcctgc ttgccgaata    3180
tcatggtgga aaatggccgc ttttctggat tcatcgactg tggccggctg ggtgtggcgg    3240
accgctatca ggacatagcg ttggctaccc gtgatattgc tgaagagctt ggcggcgaat    3300
gggctgaccg cttcctcgtg ctttacggta tcgccgctcc cgattcgcag cgcatcgcct    3360
tctatcgcct tcttgacgag ttcttctgag cgggactctg gggttcgaaa tgaccgacca    3420
agcgacgccc aacctgccat cacgatggcc gcaataaaat atctttattt tcattacatc    3480
tgtgtgttgg ttttttgtgt gaatcgatag cgataaggat ccgcgtatgg tgcactctca    3540
gtacaatctg ctctgatgcc gcatagttaa gccagccccg acacccgcca acacccgctg    3600
acgcgccctg acgggcttgt ctgctcccgg catccgctta cagacaagct gtgaccgtct    3660
ccgggagctg catgtgtcag aggttttcac cgtcatcacc gaaacgcgcg agacgaaagg    3720
gcctcgtgat acgcctattt ttataggtta atgtcatgat aataatggtt tcttagacgt    3780
caggtggcac ttttcgggga aatgtgcgcg gaacccctat ttgtttattt ttctaaatac    3840
attcaaatat gtatccgctc atgagacaat aaccctgata aatgcttcaa taatattgaa    3900
aaaggaagag tatgagtatt caacatttcc gtgtcgccct tattcccttt tttgcggcat    3960
tttgccttcc tgtttttgct cacccagaaa cgctggtgaa agtaaaagat gctgaagatc    4020
agttgggtgc acgagtgggt tacatcgaac tggatctcaa cagcggtaag atccttgaga    4080

gttttcgccc cgaagaacgt tttccaatga tgagcacttt taaagttctg ctatgtggcg 4140
cggtattatc ccgtattgac gccgggcaag agcaactcgg tcgccgcata cactattctc 4200
agaatgactt ggttgagtac tcaccagtca cagaaaagca tcttacggat ggcatgacag 4260
taagagaatt atgcagtgct gccataacca tgagtgataa cactgcggcc aacttacttc 4320
tgacaacgat cggaggaccg aaggagctaa ccgctttttt gcacaacatg ggggatcatg 4380
taactcgcct tgatcgttgg gaaccggagc tgaatgaagc cataccaaac gacgagcgtg 4440
acaccacgat gcctgtagca atggcaacaa cgttgcgcaa actattaact ggcgaactac 4500
ttactctagc ttcccggcaa caattaatag actggatgga ggcggataaa gttgcaggac 4560
cacttctgcg ctcggccctt ccggctggct ggtttattgc tgataaatct ggagccggtg 4620
agcgtgggtc tcgcggtatc attgcagcac tggggccaga tggtaagccc tcccgtatcg 4680
tagttatcta cacgacgggg agtcaggcaa ctatggatga acgaaataga cagatcgctg 4740
agataggtgc ctcactgatt aagcattggt aactgtcaga ccaagtttac tcatatatac 4800
tttagattga tttaaaactt catttttaat ttaaaaggat ctaggtgaag atcctttttg 4860
ataatctcat gaccaaaatc ccttaacgtg agttttcgtt ccactgagcg tcagaccccg 4920
tagaaaagat caaaggatct tcttgagatc ctttttttct gcgcgtaatc tgctgcttgc 4980
aaacaaaaaa accaccgcta ccagcggtgg tttgtttgcc ggatcaagag ctaccaactc 5040
tttttccgaa ggtaactggc ttcagcagag cgcagatacc aaatactgtt cttctagtgt 5100
agccgtagtt aggccaccac ttcaagaact ctgtagcacc gcctacatac ctcgctctgc 5160
taatcctgtt accagtggct gctgccagtg gcgataagtc gtgtcttacc gggttggact 5220
caagacgata gttaccggat aaggcgcagc ggtcgggctg aacggggggt tcgtgcacac 5280
agcccagctt ggagcgaacg acctacaccg aactgagata cctacagcgt gagctatgag 5340
aaagcgccac gcttcccgaa gggagaaagg cggacaggta tccggtaagc ggcagggtcg 5400
gaacaggaga gcgcacgagg gagcttccag ggggaaacgc ctggtatctt tatagtcctg 5460
tcgggtttcg ccacctctga cttgagcgtc gatttttgtg atgctcgtca ggggggcgga 5520
gcctatggaa aaacgccagc aacgcggcct ttttacggtt cctggccttt tgctggcctt 5580
ttgctcacat ggctcgacag atct 5604

<210> 189
<211> 5573
<212> DNA
<213> Homo sapiens
<220>
<221> misc_feature
<223> vecteur d'expression appelé GS-V41 contenant la séquence antisens
de GS-N41 ; antisens: 1108...1220

<400> 189
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtcga gcttcattcc gcatcaggtg cagctcccgg 1140
aatttgcgca gtctctgttc gcgcttctga gcggccagct ccgccgccgc agcgagggac 1200
ccctcctccg cgctgtccac gtcgacccgg gcggccgctt ccctttagtg agggttaatg 1260
cttcgagcag acatgataag atacattgat gagtttggac aaaccacaac tagaatgcag 1320
tgaaaaaaat gctttatttg tgaaatttgt gatgctattg ctttatttgt aaccattata 1380
agctgcaata aacaagttaa caacaacaat tgcattcatt ttatgtttca ggttcagggg 1440
gagatgtggg aggtttttta aagcaagtaa aacctctaca aatgtggtaa aatccgataa 1500
ggatcgatcc gggctggcgt aatagcgaag aggcccgcac cgatcgccct tcccaacagt 1560
tgcgcagcct gaatggcgaa tggacgcgcc ctgtagcggc gcattaagcg cggcgggtgt 1620
ggtggttacg cgcagcgtga ccgctacact tgccagcgcc ctagcgcccg ctcctttcgc 1680
tttcttccct tcctttctcg ccacgttcgc cggctttccc cgtcaagctc taaatcgggg 1740
gctcccttta gggttccgat ttagtgcttt acggcacctc gaccccaaaa aacttgatta 1800
gggtgatggt tcacgtagtg ggccatcgcc ctgatagacg gtttttcgcc ctttgacgtt 1860
ggagtccacg ttctttaata gtggactctt gttccaaact ggaacaacac tcaaccctat 1920
ctcggtctat tcttttgatt tataagggat tttgccgatt tcggcctatt ggttaaaaaa 1980
tgagctgatt taacaaaaat ttaacgcgaa ttttaacaaa atattaacgc ttacaatttc 2040
ctgatgcggt attttctcct tacgcatctg tgcggtattt cacaccgcat acgcggatct 2100

gcgcagcacc atggcctgaa ataacctctg aaagaggaac ttggttaggt accttctgag 2160
gcggaaagaa ccagctgtgg aatgtgtgtc agttagggtg tggaaagtcc ccaggctccc 2220
cagcaggcag aagtatgcaa agcatgcatc tcaattagtc agcaaccagg tgtggaaagt 2280
ccccaggctc cccagcaggc agaagtatgc aaagcatgca tctcaattag tcagcaacca 2340
tagtcccgcc cctaactccg cccatcccgc ccctaactcc gcccagttcc gcccattctc 2400
cgccccatgg ctgactaatt ttttttattt atgcagaggc cgaggccgcc tcggcctctg 2460
agctattcca gaagtagtga ggaggctttt ttggaggcct aggcttttgc aaaaagcttg 2520
attcttctga cacaacagtc tcgaacttaa ggctagagcc accatgattg aacaagatgg 2580
attgcacgca ggttctccgg ccgcttgggt ggagaggcta ttcggctatg actgggcaca 2640
acagacaatc ggctgctctg atgccgccgt gttccggctg tcagcgcagg ggcgcccggt 2700
tctttttgtc aagaccgacc tgtccggtgc cctgaatgaa ctgcaggacg aggcagcgcg 2760
gctatcgtgg ctggccacga cgggcgttcc ttgcgcagct gtgctcgacg ttgtcactga 2820
agcgggaagg gactggctgc tattgggcga agtgccgggg caggatctcc tgtcatctca 2880
ccttgctcct gccgagaaag tatccatcat ggctgatgca atgcggcggc tgcatacgct 2940
tgatccggct acctgcccat tcgaccacca agcgaaacat cgcatcgagc gagcacgtac 3000
tcggatggaa gccggtcttg tcgatcagga tgatctggac gaagagcatc aggggctcgc 3060
gccagccgaa ctgttcgcca ggctcaaggc gcgcatgccc gacggcgagg atctcgtcgt 3120
gacccatggc gatgcctgct tgccgaatat catggtggaa aatggccgct tttctggatt 3180
catcgactgt ggccggctgg gtgtggcgga ccgctatcag gacatagcgt tggctacccg 3240
tgatattgct gaagagcttg gcggcgaatg ggctgaccgc ttcctcgtgc tttacggtat 3300
cgccgctccc gattcgcagc gcatcgcctt ctatcgcctt cttgacgagt tcttctgagc 3360
gggactctgg ggttcgaaat gaccgaccaa gcgacgccca acctgccatc acgatggccg 3420
caataaaata tctttatttt cattacatct gtgtgttggt tttttgtgtg aatcgatagc 3480
gataaggatc cgcgtatggt gcactctcag tacaatctgc tctgatgccg catagttaag 3540
ccagccccga cacccgccaa cacccgctga cgcgccctga cgggcttgtc tgctcccggc 3600
atccgcttac agacaagctg tgaccgtctc cgggagctgc atgtgtcaga ggttttcacc 3660
gtcatcaccg aaacgcgcga gacgaaaggg cctcgtgata cgcctatttt tataggttaa 3720
tgtcatgata ataatggttt cttagacgtc aggtggcact tttcggggaa atgtgcgcgg 3780
aacccctatt tgtttatttt tctaaataca ttcaaatatg tatccgctca tgagacaata 3840
accctgataa atgcttcaat aatattgaaa aaggaagagt atgagtattc aacatttccg 3900
tgtcgccctt attccctttt ttgcggcatt ttgccttcct gtttttgctc acccagaaac 3960
gctggtgaaa gtaaaagatg ctgaagatca gttgggtgca cgagtgggtt acatcgaact 4020
ggatctcaac agcggtaaga tccttgagag ttttcgcccc gaagaacgtt ttccaatgat 4080
gagcactttt aaagttctgc tatgtggcgc ggtattatcc cgtattgacg ccgggcaaga 4140
gcaactcggt cgccgcatac actattctca gaatgacttg gttgagtact caccagtcac 4200
agaaaagcat cttacggatg gcatgacagt aagagaatta tgcagtgctg ccataaccat 4260
gagtgataac actgcggcca acttacttct gacaacgatc ggaggaccga aggagctaac 4320
cgcttttttg cacaacatgg gggatcatgt aactcgcctt gatcgttggg aaccggagct 4380
gaatgaagcc ataccaaacg acgagcgtga caccacgatg cctgtagcaa tggcaacaac 4440
gttgcgcaaa ctattaactg gcgaactact tactctagct tcccggcaac aattaataga 4500
ctggatggag gcggataaag ttgcaggacc acttctgcgc tcggcccttc cggctggctg 4560
gtttattgct gataaatctg gagccggtga gcgtgggtct cgcggtatca ttgcagcact 4620
ggggccagat ggtaagccct cccgtatcgt agttatctac acgacgggga gtcaggcaac 4680
tatggatgaa cgaaatagac agatcgctga gataggtgcc tcactgatta agcattggta 4740
actgtcagac caagtttact catatatact ttagattgat ttaaaacttc atttttaatt 4800
taaaaggatc taggtgaaga tcctttttga taatctcatg accaaaatcc cttaacgtga 4860
gttttcgttc cactgagcgt cagaccccgt agaaaagatc aaaggatctt cttgagatcc 4920
tttttttctg cgcgtaatct gctgcttgca aacaaaaaaa ccaccgctac cagcggtggt 4980
ttgtttgccg gatcaagagc taccaactct ttttccgaag gtaactggct tcagcagagc 5040
gcagatacca aatactgttc ttctagtgta gccgtagtta ggccaccact tcaagaactc 5100
tgtagcaccg cctacatacc tcgctctgct aatcctgtta ccagtggctg ctgccagtgg 5160
cgataagtcg tgtcttaccg ggttggactc aagacgatag ttaccggata aggcgcagcg 5220
gtcgggctga acggggggtt cgtgcacaca gcccagcttg gagcgaacga cctacaccga 5280
actgagatac ctacagcgtg agctatgaga aagcgccacg cttcccgaag ggagaaaggc 5340
ggacaggtat ccggtaagcg gcagggtcgg aacaggagag cgcacgaggg agcttccagg 5400
gggaaacgcc tggtatcttt atagtcctgt cgggtttcgc cacctctgac ttgagcgtcg 5460
atttttgtga tgctcgtcag gggggcggag cctatggaaa aacgccagca acgcggcctt 5520
tttacggttc ctggcctttt gctggccttt tgctcacatg gctcgacaga tct 5573

<210> 190
<211> 5640
<212>. DNA
<213> Homo sapiens
<220>
<221> misc_feature
<223> vecteur d'expression appelé GS-V42 contenant la séquence antisens
de GS-N42 ; antisens: 1108...1287

<400> 190
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180

gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgttgg ttccgttggg ttaacagtgt cattttaaaa 1140
tgccataaat taaataagtt agttcacatt ttttttcccc cagcacttaa gttacagtta 1200
gtctctaatg tgcttttgaa gcttgcttta gctgttccag gtctcttctg cggacagtgg 1260
tggctctggg acaaccatat tcgtccagtc gacccgggcg gccgcttccc tttagtgagg 1320
gttaatgctt cgagcagaca tgataagata cattgatgag tttggacaaa ccacaactag 1380
aatgcagtga aaaaaatgct ttatttgtga aatttgtgat gctattgctt tatttgtaac 1440
cattataagc tgcaataaac aagttaacaa caacaattgc attcatttta tgtttcaggt 1500
tcagggggag atgtgggagg ttttttaaag caagtaaaac ctctacaaat gtggtaaaat 1560
ccgataagga tcgatccggg ctggcgtaat agcgaagagg cccgcaccga tcgcccttcc 1620
caacagttgc gcagcctgaa tggcgaatgg acgcgccctg tagcggcgca ttaagcgcgg 1680
cgggtgtggt ggttacgcgc agcgtgaccg ctacacttgc cagcgcccta gcgcccgctc 1740
ctttcgcttt cttcccttcc tttctcgcca cgttcgccgg ctttccccgt caagctctaa 1800
atcgggggct ccctttaggg ttccgattta gtgctttacg gcacctcgac cccaaaaaac 1860
ttgattaggg tgatggttca cgtagtgggc catcgccctg atagacggtt tttcgccctt 1920
tgacgttgga gtccacgttc tttaatagtg gactcttgtt ccaaactgga acaacactca 1980
accctatctc ggtctattct tttgatttat aagggatttt gccgatttcg gcctattggt 2040
taaaaaatga gctgatttaa caaaaattta acgcgaattt taacaaaata ttaacgctta 2100
caatttcctg atgcggtatt ttctccttac gcatctgtgc ggtatttcac accgcatacg 2160
cggatctgcg cagcaccatg gcctgaaata acctctgaaa gaggaacttg gttaggtacc 2220
ttctgaggcg gaaagaacca gctgtggaat gtgtgtcagt tagggtgtgg aaagtcccca 2280
ggctccccag caggcagaag tatgcaaagc atgcatctca attagtcagc aaccaggtgt 2340
ggaaagtccc caggctcccc agcaggcaga agtatgcaaa gcatgcatct caattagtca 2400
gcaaccatag tcccgcccct aactccgccc atcccgcccc taactccgcc cagttccgcc 2460
cattctccgc cccatggctg actaattttt tttatttatg cagaggccga ggccgcctcg 2520
gcctctgagc tattccagaa gtagtgagga ggcttttttg gaggcctagg cttttgcaaa 2580
aagcttgatt cttctgacac aacagtctcg aacttaaggc tagagccacc atgattgaac 2640
aagatggatt gcacgcaggt tctccggccg cttgggtgga gaggctattc ggctatgact 2700
gggcacaaca gacaatcggc tgctctgatg ccgccgtgtt ccggctgtca gcgcaggggc 2760
gcccggttct ttttgtcaag accgacctgt ccggtgccct gaatgaactg caggacgagg 2820
cagcgcggct atcgtggctg gccacgacgg gcgttccttg cgcagctgtg ctcgacgttg 2880
tcactgaagc gggaagggac tggctgctat tgggcgaagt gccggggcag gatctcctgt 2940
catctcacct tgctcctgcc gagaaagtat ccatcatggc tgatgcaatg cggcggctgc 3000
atacgcttga tccggctacc tgcccattcg accaccaagc gaaacatcgc atcgagcgag 3060
cacgtactcg gatggaagcc ggtcttgtcg atcaggatga tctggacgaa gagcatcagg 3120
ggctcgcgcc agccgaactg ttcgccaggc tcaaggcgcg catgcccgac ggcgaggatc 3180
tcgtcgtgac ccatggcgat gcctgcttgc cgaatatcat ggtggaaaat ggccgctttt 3240
ctggattcat cgactgtggc cggctgggtg tggcggaccg ctatcaggac atagcgttgg 3300
ctacccgtga tattgctgaa gagcttggcg gcgaatgggc tgaccgcttc ctcgtgcttt 3360
acggtatcgc cgctcccgat tcgcagcgca tcgccttcta tcgccttctt gacgagttct 3420
tctgagcggg actctggggt tcgaaatgac cgaccaagcg acgcccaacc tgccatcacg 3480
atggccgcaa taaaatatct ttattttcat tacatctgtg tgttggtttt ttgtgtgaat 3540
cgatagcgat aaggatccgc gtatggtgca ctctcagtac aatctgctct gatgccgcat 3600
agttaagcca gccccgacac ccgccaacac ccgctgacgc gccctgacgg gcttgtctgc 3660
tcccggcatc cgcttacaga caagctgtga ccgtctccgg gagctgcatg tgtcagaggt 3720
tttcaccgtc atcaccgaaa cgcgcgagac gaaagggcct cgtgatacgc ctatttttat 3780
aggttaatgt catgataata atggtttctt agacgtcagg tggcactttt cggggaaatg 3840
tgcgcggaac ccctatttgt ttatttttct aaatacattc aaatatgtat ccgctcatga 3900
gacaataacc ctgataaatg cttcaataat attgaaaaag gaagagtatg agtattcaac 3960
atttccgtgt cgcccttatt cccttttttg cggcattttg ccttcctgtt tttgctcacc 4020
cagaaacgct ggtgaaagta aaagatgctg aagatcagtt gggtgcacga gtgggttaca 4080
tcgaactgga tctcaacagc ggtaagatcc ttgagagttt tcgccccgaa gaacgttttc 4140
caatgatgag cacttttaaa gttctgctat gtggcgcggt attatcccgt attgacgccg 4200
ggcaagagca actcggtcgc cgcatacact attctcagaa tgacttggtt gagtactcac 4260
cagtcacaga aaagcatctt acggatggca tgacagtaag agaattatgc agtgctgcca 4320
taaccatgag tgataacact gcggccaact tacttctgac aacgatcgga ggaccgaagg 4380
agctaaccgc ttttttgcac aacatggggg atcatgtaac tcgccttgat cgttgggaac 4440
cggagctgaa tgaagccata ccaaacgacg agcgtgacac cacgatgcct gtagcaatgg 4500
caacaacgtt gcgcaaacta ttaactggcg aactacttac tctagcttcc cggcaacaat 4560

taatagactg gatggaggcg gataaagttg caggaccact tctgcgctcg gcccttccgg 4620
ctggctggtt tattgctgat aaatctggag ccggtgagcg tgggtctcgc ggtatcattg 4680
cagcactggg gccagatggt aagccctccc gtatcgtagt tatctacacg acggggagtc 4740
aggcaactat ggatgaacga aatagacaga tcgctgagat aggtgcctca ctgattaagc 4800
attggtaact gtcagaccaa gtttactcat atatacttta gattgattta aaacttcatt 4860
tttaatttaa aaggatctag gtgaagatcc tttttgataa tctcatgacc aaaatccctt 4920
aacgtgagtt ttcgttccac tgagcgtcag accccgtaga aaagatcaaa ggatcttctt 4980
gagatccttt ttttctgcgc gtaatctgct gcttgcaaac aaaaaaacca ccgctaccag 5040
cggtggtttg tttgccggat caagagctac caactctttt tccgaaggta actggcttca 5100
gcagagcgca gataccaaat actgttcttc tagtgtagcc gtagttaggc caccacttca 5160
agaactctgt agcaccgcct acatacctcg ctctgctaat cctgttacca gtggctgctg 5220
ccagtggcga taagtcgtgt cttaccgggt tggactcaag acgatagtta ccggataagg 5280
cgcagcggtc gggctgaacg gggggttcgt gcacacagcc cagcttggag cgaacgacct 5340
acaccgaact gagataccta cagcgtgagc tatgagaaag cgccacgctt cccgaaggga 5400
gaaaggcgga caggtatccg gtaagcggca gggtcggaac aggagagcgc acgagggagc 5460
ttccaggggg aaacgcctgg tatctttata gtcctgtcgg gtttcgccac ctctgacttg 5520
agcgtcgatt tttgtgatgc tcgtcagggg ggcggagcct atggaaaaac gccagcaacg 5580
cggccttttt acggttcctg gccttttgct ggccttttgc tcacatggct cgacagatct 5640

<210> 191
<211> 5967
<212> DNA
<213> Homo sapiens
<220>
<221> misc_feature
<223> vecteur d'expression appelé GS-V43 contenant la séquence antisens
de GS-N43 ; antisens: 1108...1614

<400> 191
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtgca aaaactgcca caatcttgct caaagtgtct 1140
aagtatccaa atggtagcaa atgagctgca ttaagcttta tataaccaca ttgcatatga 1200
agaaaaaaaa tgaaatttat gccgatataa atagaaatgt tacagagtat catatactga 1260
gagtaaccta tgcagttctt ttaaacttat ctctttagtg gtttcaataa aatatcacta 1320
accacttatc cctaaagcct caagttttct tcatttgaat gctgtagaat tgcattagta 1380
ttttatattg attatataaa ttaagaccaa tttctatcta agtatgtgct tcaagcaatg 1440
ttttgtggca aacacacaaa acatgctttg ctcttcaaat ttattatcct taaaataatg 1500
cacttttaaa gccttcactt catagtttgg taactaagat ccacatgata cacaatgcct 1560
tgctgaacac agaaaatgta tctgcattat gataatgaaa cccggctttt gctggtcgac 1620
ccgggcggcc gcttcccttt agtgagggtt aatgcttcga gcagacatga taagatacat 1680
tgatgagttt ggacaaacca caactagaat gcagtgaaaa aaatgcttta tttgtgaaat 1740
ttgtgatgct attgctttat ttgtaaccat tataagctgc aataaacaag ttaacaacaa 1800
caattgcatt cattttatgt ttcaggttca gggggagatg tgggaggttt tttaaagcaa 1860
gtaaaacctc tacaaatgtg gtaaaatccg ataaggatcg atccgggctg gcgtaatagc 1920
gaagaggccc gcaccgatcg cccttcccaa cagttgcgca gcctgaatgg cgaatggacg 1980
cgccctgtag cggcgcatta agcgcggcgg gtgtggtggt tacgcgcagc gtgaccgcta 2040
cacttgccag cgccctagcg cccgctcctt tcgctttctt cccttccttt ctcgccacgt 2100
tcgccggctt tccccgtcaa gctctaaatc gggggctccc tttagggttc cgatttagtg 2160
ctttacggca cctcgacccc aaaaaacttg attagggtga tggttcacgt agtgggccat 2220
cgccctgata gacggttttt cgccctttga cgttggagtc cacgttcttt aatagtggac 2280
tcttgttcca aactggaaca acactcaacc ctatctcggt ctattctttt gatttataag 2340
ggattttgcc gatttcggcc tattggttaa aaaatgagct gatttaacaa aaatttaacg 2400
cgaattttaa caaaatatta acgcttacaa tttcctgatg cggtattttc tccttacgca 2460
tctgtgcggt atttcacacc gcatacgcgg atctgcgcag caccatggcc tgaaataacc 2520
tctgaaagag gaacttggtt aggtaccttc tgaggcggaa agaaccagct gtggaatgtg 2580

tgtcagttag ggtgtggaaa gtccccaggc tccccagcag gcagaagtat gcaaagcatg 2640
catctcaatt agtcagcaac caggtgtgga aagtccccag gctccccagc aggcagaagt 2700
atgcaaagca tgcatctcaa ttagtcagca accatagtcc cgcccctaac tccgcccatc 2760
ccgcccctaa ctccgcccag ttccgcccat tctccgcccc atggctgact aatttttttt 2820
atttatgcag aggccgaggc cgcctcggcc tctgagctat tccagaagta gtgaggaggc 2880
ttttttggag gcctaggctt ttgcaaaaag cttgattctt ctgacacaac agtctcgaac 2940
ttaaggctag agccaccatg attgaacaag atggattgca cgcaggttct ccggccgctt 3000
gggtggagag gctattcggc tatgactggg cacaacagac aatcggctgc tctgatgccg 3060
ccgtgttccg gctgtcagcg caggggcgcc cggttctttt tgtcaagacc gacctgtccg 3120
gtgccctgaa tgaactgcag gacgaggcag cgcggctatc gtggctggcc acgacgggcg 3180
ttccttgcgc agctgtgctc gacgttgtca ctgaagcggg aagggactgg ctgctattgg 3240
gcgaagtgcc ggggcaggat ctcctgtcat ctcaccttgc tcctgccgag aaagtatcca 3300
tcatggctga tgcaatgcgg cggctgcata cgcttgatcc ggctacctgc ccattcgacc 3360
accaagcgaa acatcgcatc gagcgagcac gtactcggat ggaagccggt cttgtcgatc 3420
aggatgatct ggacgaagag catcaggggc tcgcgccagc cgaactgttc gccaggctca 3480
aggcgcgcat gcccgacggc gaggatctcg tcgtgaccca tggcgatgcc tgcttgccga 3540
atatcatggt ggaaaatggc cgcttttctg gattcatcga ctgtggccgg ctgggtgtgg 3600
cggaccgcta tcaggacata gcgttggcta cccgtgatat tgctgaagag cttggcggcg 3660
aatgggctga ccgcttcctc gtgctttacg gtatcgccgc tcccgattcg cagcgcatcg 3720
ccttctatcg ccttcttgac gagttcttct gagcgggact ctggggttcg aaatgaccga 3780
ccaagcgacg cccaacctgc catcacgatg gccgcaataa aatatcttta ttttcattac 3840
atctgtgtgt tggttttttg tgtgaatcga tagcgataag gatccgcgta tggtgcactc 3900
tcagtacaat ctgctctgat gccgcatagt taagccagcc ccgacacccg ccaacacccg 3960
ctgacgcgcc ctgacgggct tgtctgctcc cggcatccgc ttacagacaa gctgtgaccg 4020
tctccgggag ctgcatgtgt cagaggtttt caccgtcatc accgaaacgc gcgagacgaa 4080
agggcctcgt gatacgccta tttttatagg ttaatgtcat gataataatg gtttcttaga 4140
cgtcaggtgg cacttttcgg ggaaatgtgc gcggaacccc tatttgttta tttttctaaa 4200
tacattcaaa tatgtatccg ctcatgagac aataaccctg ataaatgctt caataatatt 4260
gaaaaaggaa gagtatgagt attcaacatt tccgtgtcgc ccttattccc ttttttgcgg 4320
cattttgcct tcctgttttt gctcacccag aaacgctggt gaaagtaaaa gatgctgaag 4380
atcagttggg tgcacgagtg ggttacatcg aactggatct caacagcggt aagatccttg 4440
agagttttcg ccccgaagaa cgttttccaa tgatgagcac ttttaaagtt ctgctatgtg 4500
gcgcggtatt atcccgtatt gacgccgggc aagagcaact cggtcgccgc atacactatt 4560
ctcagaatga cttggttgag tactcaccag tcacagaaaa gcatcttacg gatggcatga 4620
cagtaagaga attatgcagt gctgccataa ccatgagtga taacactgcg gccaacttac 4680
ttctgacaac gatcggagga ccgaaggagc taaccgcttt tttgcacaac atgggggatc 4740
atgtaactcg ccttgatcgt tgggaaccgg agctgaatga agccatacca aacgacgagc 4800
gtgacaccac gatgcctgta gcaatggcaa caacgttgcg caaactatta actggcgaac 4860
tacttactct agcttcccgg caacaattaa tagactggat ggaggcggat aaagttgcag 4920
gaccacttct gcgctcggcc cttccggctg gctggtttat tgctgataaa tctggagccg 4980
gtgagcgtgg gtctcgcggt atcattgcag cactggggcc agatggtaag ccctcccgta 5040
tcgtagttat ctacacgacg gggagtcagg caactatgga tgaacgaaat agacagatcg 5100
ctgagatagg tgcctcactg attaagcatt ggtaactgtc agaccaagtt tactcatata 5160
tactttagat tgatttaaaa cttcattttt aatttaaaag gatctaggtg aagatccttt 5220
ttgataatct catgaccaaa atcccttaac gtgagttttc gttccactga gcgtcagacc 5280
ccgtagaaaa gatcaaagga tcttcttgag atcctttttt tctgcgcgta atctgctgct 5340
tgcaaacaaa aaaaccaccg ctaccagcgg tggtttgttt gccggatcaa gagctaccaa 5400
ctctttttcc gaaggtaact ggcttcagca gagcgcagat accaaatact gttcttctag 5460
tgtagccgta gttaggccac cacttcaaga actctgtagc accgcctaca tacctcgctc 5520
tgctaatcct gttaccagtg gctgctgcca gtggcgataa gtcgtgtctt accgggttgg 5580
actcaagacg atagttaccg gataaggcgc agcggtcggg ctgaacgggg ggttcgtgca 5640
cacagcccag cttggagcga acgacctaca ccgaactgag atacctacag cgtgagctat 5700
gagaaagcgc cacgcttccc gaagggagaa aggcggacag gtatccggta agcggcaggg 5760
tcggaacagg agagcgcacg agggagcttc cagggggaaa cgcctggtat ctttatagtc 5820
ctgtcgggtt tcgccacctc tgacttgagc gtcgattttt gtgatgctcg tcaggggggc 5880
ggagcctatg gaaaaacgcc agcaacgcgg cctttttacg gttcctggcc ttttgctggc 5940
cttttgctca catggctcga cagatct 5967

<210> 192
<211> 6092
<212> DNA
<213> Homo sapiens
<220>
<221> misc_feature
<223> vecteur d'expression appelé GS-V44 contenant la séquence antisens
de GS-N43 ; antisens: 1108...1739

<400> 192
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240

gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtggc agcctgaaga aggtaggaaa gccttagtag 1140
agggaaaatc aaaccaaatg agattaggtc aaaagaacag ataactatcg tctgcttatc 1200
tacttaaagg tgtgtggcat ttaccacagt gaggagctaa caggagcctc ccacactaca 1260
tgcagggccc aaaggggcca gcgatggtgt gataaccagt caccgatgat gagttgcagt 1320
tggaccacgt acagatggag gggctggccc atgcagggcc tcggggtgag gttcactagg 1380
actagctgca gggggacctg gctacactct gtggcacaac agggcttcac aaaatggaca 1440
ctacacgtga cgtgggctga aaggtggagc agagactggg tgggagtgtc agttgtacgt 1500
tgtggggtaa gggggagagg gagcagggca agtgtattat gtactgtgct ctaacattct 1560
ttggaagtct ttcaaagatt gctgcagttc ctttttaaaa caggtctctt attccgtcat 1620
tcttgaaaga cttctcctta caccatctca tactgattgg ccgtgatgaa ccgggacaga 1680
cagcaggcca gcagcatgcc aattaactgg gagaatgcga ttccaaacgc cactccagcg 1740
tcgacccggg cggccgcttc cctttagtga gggttaatgc ttcgagcaga catgataaga 1800
tacattgatg agtttggaca aaccacaact agaatgcagt gaaaaaaatg ctttatttgt 1860
gaaatttgtg atgctattgc tttatttgta accattataa gctgcaataa acaagttaac 1920
aacaacaatt gcattcattt tatgtttcag gttcaggggg agatgtggga ggttttttaa 1980
agcaagtaaa acctctacaa atgtggtaaa atccgataag gatcgatccg ggctggcgta 2040
atagcgaaga ggcccgcacc gatcgccctt cccaacagtt gcgcagcctg aatggcgaat 2100
ggacgcgccc tgtagcggcg cattaagcgc ggcgggtgtg gtggttacgc gcagcgtgac 2160
cgctacactt gccagcgccc tagcgcccgc tcctttcgct ttcttccctt cctttctcgc 2220
cacgttcgcc ggctttcccc gtcaagctct aaatcggggg ctccctttag ggttccgatt 2280
tagtgcttta cggcacctcg accccaaaaa acttgattag ggtgatggtt cacgtagtgg 2340
gccatcgccc tgatagacgg tttttcgccc tttgacgttg gagtccacgt tctttaatag 2400
tggactcttg ttccaaactg gaacaacact caaccctatc tcggtctatt cttttgattt 2460
ataagggatt ttgccgattt cggcctattg gttaaaaaat gagctgattt aacaaaaatt 2520
taacgcgaat tttaacaaaa tattaacgct tacaatttcc tgatgcggta ttttctcctt 2580
acgcatctgt gcggtatttc acaccgcata cgcggatctg cgcagcacca tggcctgaaa 2640
taacctctga aagaggaact tggttaggta ccttctgagg cggaaagaac cagctgtgga 2700
atgtgtgtca gttagggtgt ggaaagtccc caggctcccc agcaggcaga agtatgcaaa 2760
gcatgcatct caattagtca gcaaccaggt gtggaaagtc cccaggctcc ccagcaggca 2820
gaagtatgca aagcatgcat ctcaattagt cagcaaccat agtcccgccc ctaactccgc 2880
ccatcccgcc cctaactccg cccagttccg cccattctcc gccccatggc tgactaattt 2940
tttttattta tgcagaggcc gaggccgcct cggcctctga gctattccag aagtagtgag 3000
gaggcttttt tggaggccta ggcttttgca aaaagcttga ttcttctgac acaacagtct 3060
cgaacttaag gctagagcca ccatgattga acaagatgga ttgcacgcag gttctccggc 3120
cgcttgggtg gagaggctat tcggctatga ctgggcacaa cagacaatcg gctgctctga 3180
tgccgccgtg ttccggctgt cagcgcaggg gcgcccggtt ctttttgtca agaccgacct 3240
gtccggtgcc ctgaatgaac tgcaggacga ggcagcgcgg ctatcgtggc tggccacgac 3300
gggcgttcct tgcgcagctg tgctcgacgt tgtcactgaa gcgggaaggg actggctgct 3360
attgggcgaa gtgccggggc aggatctcct gtcatctcac cttgctcctg ccgagaaagt 3420
atccatcatg gctgatgcaa tgcggcggct gcatacgctt gatccggcta cctgcccatt 3480
cgaccaccaa gcgaaacatc gcatcgagcg agcacgtact cggatggaag ccggtcttgt 3540
cgatcaggat gatctggacg aagagcatca ggggctcgcg ccagccgaac tgttcgccag 3600
gctcaaggcg cgcatgcccg acggcgagga tctcgtcgtg acccatggcg atgcctgctt 3660
gccgaatatc atggtggaaa atggccgctt ttctggattc atcgactgtg gccggctggg 3720
tgtggcggac cgctatcagg acatagcgtt ggctacccgt gatattgctg aagagcttgg 3780
cggcgaatgg gctgaccgct tcctcgtgct ttacggtatc gccgctcccg attcgcagcg 3840
catcgccttc tatcgccttc ttgacgagtt cttctgagcg ggactctggg gttcgaaatg 3900
accgaccaag cgacgcccaa cctgccatca cgatggccgc aataaaatat ctttattttc 3960
attacatctg tgtgttggtt ttttgtgtga atcgatagcg ataaggatcc gcgtatggtg 4020
cactctcagt acaatctgct ctgatgccgc atagttaagc cagccccgac acccgccaac 4080
acccgctgac gcgccctgac gggcttgtct gctcccggca tccgcttaca gacaagctgt 4140
gaccgtctcc gggagctgca tgtgtcagag gttttcaccg tcatcaccga aacgcgcgag 4200
acgaaagggc ctcgtgatac gcctattttt ataggttaat gtcatgataa taatggtttc 4260
ttagacgtca ggtggcactt ttcggggaaa tgtgcgcgga acccctattt gtttattttt 4320
ctaaatacat tcaaatatgt atccgctcat gagacaataa ccctgataaa tgcttcaata 4380
atattgaaaa aggaagagta tgagtattca acatttccgt gtcgccctta ttcccttttt 4440
tgcggcattt tgccttcctg tttttgctca cccagaaacg ctggtgaaag taaaagatgc 4500
tgaagatcag ttgggtgcac gagtgggtta catcgaactg gatctcaaca gcggtaagat 4560
ccttgagagt tttcgccccg aagaacgttt tccaatgatg agcactttta aagttctgct 4620

atgtggcgcg gtattatccc gtattgacgc cgggcaagag caactcggtc gccgcataca 4680
ctattctcag aatgacttgg ttgagtactc accagtcaca gaaaagcatc ttacggatgg 4740
catgacagta agagaattat gcagtgctgc cataaccatg agtgataaca ctgcggccaa 4800
cttacttctg acaacgatcg gaggaccgaa ggagctaacc gcttttttgc acaacatggg 4860
ggatcatgta actcgccttg atcgttggga accggagctg aatgaagcca taccaaacga 4920
cgagcgtgac accacgatgc ctgtagcaat ggcaacaacg ttgcgcaaac tattaactgg 4980
cgaactactt actctagctt cccggcaaca attaatagac tggatggagg cggataaagt 5040
tgcaggacca cttctgcgct cggcccttcc ggctggctgg tttattgctg ataaatctgg 5100
agccggtgag cgtgggtctc gcggtatcat tgcagcactg gggccagatg gtaagccctc 5160
ccgtatcgta gttatctaca cgacggggag tcaggcaact atggatgaac gaaatagaca 5220
gatcgctgag ataggtgcct cactgattaa gcattggtaa ctgtcagacc aagtttactc 5280
atatatactt tagattgatt taaaacttca tttttaattt aaaaggatct aggtgaagat 5340
cctttttgat aatctcatga ccaaaatccc ttaacgtgag ttttcgttcc actgagcgtc 5400
agaccccgta gaaaagatca aaggatcttc ttgagatcct ttttttctgc gcgtaatctg 5460
ctgcttgcaa acaaaaaaac caccgctacc agcggtggtt tgtttgccgg atcaagagct 5520
accaactctt tttccgaagg taactggctt cagcagagcg cagataccaa atactgttct 5580
tctagtgtag ccgtagttag gccaccactt caagaactct gtagcaccgc ctacatacct 5640
cgctctgcta atcctgttac cagtggctgc tgccagtggc gataagtcgt gtcttaccgg 5700
gttggactca agacgatagt taccggataa ggcgcagcgg tcgggctgaa cggggggttc 5760
gtgcacacag cccagcttgg agcgaacgac ctacaccgaa ctgagatacc tacagcgtga 5820
gctatgagaa agcgccacgc ttcccgaagg gagaaaggcg gacaggtatc cggtaagcgg 5880
cagggtcgga acaggagagc gcacgaggga gcttccaggg ggaaacgcct ggtatcttta 5940
tagtcctgtc gggtttcgcc acctctgact tgagcgtcga tttttgtgat gctcgtcagg 6000
ggggcggagc ctatggaaaa acgccagcaa cgcggccttt ttacggttcc tggccttttg 6060
ctggcctttt gctcacatgg ctcgacagat ct 6092

<210> 193
<211> 6164
<212> DNA
<213> Homo sapiens
<220>
<221> misc_feature
<223> vecteur d'expression appelé GS-V45 contenant la séquence antisens
de GS-N45 ; antisens: 1108...1811

<400> 193
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtgca agcctatgat ggccactgtg aaaaggacca 1140
gggagaatag aaccatgagg aagacatagg tggagtggga ggaaggtgtg gacaatggtt 1200
gctcagctgg gatcatgttg gtcaggttca gcatgtagcc caaagtccag ccggcgtcgc 1260
tgccctggat cttgccaatg aaatggatgt gctcccagga atcagctgtg aaatgatagc 1320
cttgcagaag gagggagaga atgtaggtac cagaaaagca gtattcactc aggtacttct 1380
cctttactcc agcgtaagat gtttttatct cctctcaagg ctgagcacag aactttttca 1440
tcatctcagt caccttttcc tgagagactt tctctgatgt caagtttaaa aacttcatca 1500
caaagtaaaa agctgaaaat gccccaaaat ccccctggag tggtggcaag aaaatcccat 1560
tgaaggcaca ctgggagtaa gggcagtaac tggtgttgaa gagctccagg atgctttgat 1620
ggcattgttg atagtttcca ataccctgga tttcaaactg ctggaatgga agagtcatct 1680
caaatctctt ggtgcagggg gtcttgtaaa ggtcacttac gttcactacc ttcttatatc 1740
caggatgaaa gcatgggtcc ctgagaattt cattacttgc aacctgaatg tccttggcca 1800
gtttctgcca ggtcgacccg ggcggccgct tccctttagt gagggttaat gcttcgagca 1860
gacatgataa gatacattga tgagtttgga caaaccacaa ctagaatgca gtgaaaaaaa 1920
tgctttattt gtgaaatttg tgatgctatt gctttatttg taaccattat aagctgcaat 1980
aaacaagtta acaacaacaa ttgcattcat tttatgtttc aggttcaggg ggagatgtgg 2040
gaggtttttt aaagcaagta aaacctctac aaatgtggta aaatccgata aggatcgatc 2100
cgggctggcg taatagcgaa gaggcccgca ccgatcgccc ttcccaacag ttgcgcagcc 2160

tgaatggcga atggacgcgc cctgtagcgg cgcattaagc gcggcgggtg tggtggttac 2220
gcgcagcgtg accgctacac ttgccagcgc cctagcgccc gctcctttcg ctttcttccc 2280
ttcctttctc gccacgttcg ccggctttcc ccgtcaagct ctaaatcggg ggctcccttt 2340
agggttccga tttagtgctt tacggcacct cgaccccaaa aaacttgatt agggtgatgg 2400
ttcacgtagt gggccatcgc cctgatagac ggtttttcgc cctttgacgt tggagtccac 2460
gttctttaat agtggactct tgttccaaac tggaacaaca ctcaacccta tctcggtcta 2520
ttcttttgat ttataaggga ttttgccgat ttcggcctat tggttaaaaa atgagctgat 2580
ttaacaaaaa tttaacgcga attttaacaa aatattaacg cttacaattt cctgatgcgg 2640
tattttctcc ttacgcatct gtgcggtatt tcacaccgca tacgcggatc tgcgcagcac 2700
catggcctga aataacctct gaaagaggaa cttggttagg taccttctga ggcggaaaga 2760
accagctgtg gaatgtgtgt cagttagggt gtggaaagtc cccaggctcc ccagcaggca 2820
gaagtatgca aagcatgcat ctcaattagt cagcaaccag gtgtggaaag tccccaggct 2880
ccccagcagg cagaagtatg caaagcatgc atctcaatta gtcagcaacc atagtcccgc 2940
ccctaactcc gcccatcccg cccctaactc cgcccagttc cgcccattct ccgccccatg 3000
gctgactaat tttttttatt tatgcagagg ccgaggccgc ctcggcctct gagctattcc 3060
agaagtagtg aggaggcttt tttggaggcc taggcttttg caaaaagctt gattcttctg 3120
acacaacagt ctcgaactta aggctagagc caccatgatt gaacaagatg gattgcacgc 3180
aggttctccg gccgcttggg tggagaggct attcggctat gactgggcac aacagacaat 3240
cggctgctct gatgccgccg tgttccggct gtcagcgcag gggcgcccgg ttctttttgt 3300
caagaccgac ctgtccggtg ccctgaatga actgcaggac gaggcagcgc ggctatcgtg 3360
gctggccacg acgggcgttc cttgcgcagc tgtgctcgac gttgtcactg aagcgggaag 3420
ggactggctg ctattgggcg aagtgccggg gcaggatctc ctgtcatctc accttgctcc 3480
tgccgagaaa gtatccatca tggctgatgc aatgcggcgg ctgcatacgc ttgatccggc 3540
tacctgccca ttcgaccacc aagcgaaaca tcgcatcgag cgagcacgta ctcggatgga 3600
agccggtctt gtcgatcagg atgatctgga cgaagagcat caggggctcg cgccagccga 3660
actgttcgcc aggctcaagg cgcgcatgcc cgacggcgag gatctcgtcg tgacccatgg 3720
cgatgcctgc ttgccgaata tcatggtgga aaatggccgc ttttctggat tcatcgactg 3780
tggccggctg ggtgtggcgg accgctatca ggacatagcg ttggctaccc gtgatattgc 3840
tgaagagctt ggcggcgaat gggctgaccg cttcctcgtg ctttacggta tcgccgctcc 3900
cgattcgcag cgcatcgcct tctatcgcct tcttgacgag ttcttctgag cgggactctg 3960
gggttcgaaa tgaccgacca agcgacgccc aacctgccat cacgatggcc gcaataaaat 4020
atctttattt tcattacatc tgtgtgttgg ttttttgtgt gaatcgatag cgataaggat 4080
ccgcgtatgg tgcactctca gtacaatctg ctctgatgcc gcatagttaa gccagccccg 4140
acacccgcca acacccgctg acgcgccctg acgggcttgt ctgctcccgg catccgctta 4200
cagacaagct gtgaccgtct ccgggagctg catgtgtcag aggttttcac cgtcatcacc 4260
gaaacgcgcg agacgaaagg gcctcgtgat acgcctattt ttataggtta atgtcatgat 4320
aataatggtt tcttagacgt caggtggcac ttttcgggga aatgtgcgcg gaacccctat 4380
ttgtttattt ttctaaatac attcaaatat gtatccgctc atgagacaat aaccctgata 4440
aatgcttcaa taatattgaa aaaggaagag tatgagtatt caacatttcc gtgtcgccct 4500
tattcccttt tttgcggcat tttgccttcc tgtttttgct cacccagaaa cgctggtgaa 4560
agtaaaagat gctgaagatc agttgggtgc acgagtgggt tacatcgaac tggatctcaa 4620
cagcggtaag atccttgaga gttttcgccc cgaagaacgt tttccaatga tgagcacttt 4680
taaagttctg ctatgtggcg cggtattatc ccgtattgac gccgggcaag agcaactcgg 4740
tcgccgcata cactattctc agaatgactt ggttgagtac tcaccagtca cagaaaagca 4800
tcttacggat ggcatgacag taagagaatt atgcagtgct gccataacca tgagtgataa 4860
cactgcggcc aacttacttc tgacaacgat cggaggaccg aaggagctaa ccgctttttt 4920
gcacaacatg ggggatcatg taactcgcct tgatcgttgg gaaccggagc tgaatgaagc 4980
cataccaaac gacgagcgtg acaccacgat gcctgtagca atggcaacaa cgttgcgcaa 5040
actattaact ggcgaactac ttactctagc ttcccggcaa caattaatag actggatgga 5100
ggcggataaa gttgcaggac cacttctgcg ctcggccctt ccggctggct ggtttattgc 5160
tgataaatct ggagccggtg agcgtgggtc tcgcggtatc attgcagcac tggggccaga 5220
tggtaagccc tcccgtatcg tagttatcta cacgacgggg agtcaggcaa ctatggatga 5280
acgaaataga cagatcgctg agataggtgc ctcactgatt aagcattggt aactgtcaga 5340
ccaagtttac tcatatatac tttagattga tttaaaactt catttttaat ttaaaaggat 5400
ctaggtgaag atcctttttg ataatctcat gaccaaaatc ccttaacgtg agttttcgtt 5460
ccactgagcg tcagaccccg tagaaaagat caaaggatct tcttgagatc ctttttttct 5520
gcgcgtaatc tgctgcttgc aaacaaaaaa accaccgcta ccagcggtgg tttgtttgcc 5580
ggatcaagag ctaccaactc tttttccgaa ggtaactggc ttcagcagag cgcagatacc 5640
aaatactgtt cttctagtgt agccgtagtt aggccaccac ttcaagaact ctgtagcacc 5700
gcctacatac ctcgctctgc taatcctgtt accagtggct gctgccagtg gcgataagtc 5760
gtgtcttacc gggttggact caagacgata gttaccggat aaggcgcagc ggtcgggctg 5820
aacggggggt tcgtgcacac agcccagctt ggagcgaacg acctacaccg aactgagata 5880
cctacagcgt gagctatgag aaagcgccac gcttcccgaa gggagaaagg cggacaggta 5940
tccggtaagc ggcagggtcg gaacaggaga gcgcacgagg gagcttccag ggggaaacgc 6000
ctggtatctt tatagtcctg tcgggtttcg ccacctctga cttgagcgtc gatttttgtg 6060
atgctcgtca ggggggcgga gcctatggaa aaacgccagc aacgcggcct ttttacggtt 6120
cctggccttt tgctggcctt ttgctcacat ggctcgacag atct 6164

<210> 194
<211> 5717
<212> DNA
<213> Homo sapiens
<220>

<221> misc_feature
<223> vecteur d'expression appelé GS-V46 contenant la séquence antisens
de GS-N46 ; antisens: 1108...1364

<400> 194
tcaatattgg ccattagcca tattattcat tggttatata gcataaatca atattggcta 60
ttggccattg catacgttgt atctatatca taatatgtac atttatattg gctcatgtcc 120
aatatgaccg ccatgttggc attgattatt gactagttat taatagtaat caattacggg 180
gtcattagtt catagcccat atatggagtt ccgcgttaca taacttacgg taaatggccc 240
gcctggctga ccgcccaacg acccccgccc attgacgtca ataatgacgt atgttcccat 300
agtaacgcca atagggactt tccattgacg tcaatgggtg gagtatttac ggtaaactgc 360
ccacttggca gtacatcaag tgtatcatat gccaagtccg ccccctattg acgtcaatga 420
cggtaaatgg cccgcctggc attatgccca gtacatgacc ttacgggact ttcctacttg 480
gcagtacatc tacgtattag tcatcgctat taccatggtg atgcggtttt ggcagtacac 540
caatgggcgt ggatagcggt ttgactcacg gggatttcca agtctccacc ccattgacgt 600
caatgggagt ttgttttggc accaaaatca acgggacttt ccaaaatgtc gtaacaactg 660
cgatcgcccg ccccgttgac gcaaatgggc ggtaggcgtg tacggtggga ggtctatata 720
agcagagctc gtttagtgaa ccgtcagatc actagaagct ttattgcggt agtttatcac 780
agttaaattg ctaacgcagt cagtgcttct gacacaacag tctcgaactt aagctgcagt 840
gactctctta aggtagcctt gcagaagttg gtcgtgaggc actgggcagg taagtatcaa 900
ggttacaaga caggtttaag gagaccaata gaaactgggc ttgtcgagac agagaagact 960
cttgcgtttc tgataggcac ctattggtct tactgacatc cactttgcct ttctctccac 1020
aggtgtccac tcccagttca attacagctc ttaaggctag agtacttaat acgactcact 1080
ataggctagc ctcgagaatt cacgcgtggc cgcgggaatt cgattattaa ccctcactaa 1140
atgctgggga tcatggatcc tctgaggact gtttttaaaa agctgaagag gagaaggaag 1200
agaggactac ttcttcccat aggtgcagct gcaaagacat caagctggac ccatcgagga 1260
tttcggattc agccatcatg gggctgctgc cggggaagcc actgcccctc ctttctcccc 1320
agcatttagt gagggttaat aatcactagt gaattcgcgg ccgcgtcgac ccgggcggcc 1380
gcttcccttt agtgagggtt aatgcttcga gcagacatga taagatacat tgatgagttt 1440
ggacaaacca caactagaat gcagtgaaaa aaatgcttta tttgtgaaat ttgtgatgct 1500
attgctttat ttgtaaccat tataagctgc aataaacaag ttaacaacaa caattgcatt 1560
cattttatgt ttcaggttca gggggagatg tgggaggttt tttaaagcaa gtaaaacctc 1620
tacaaatgtg gtaaaatccg ataaggatcg atccgggctg gcgtaatagc gaagaggccc 1680
gcaccgatcg cccttcccaa cagttgcgca gcctgaatgg cgaatggacg cgccctgtag 1740
cggcgcatta agcgcggcgg gtgtggtggt tacgcgcagc gtgaccgcta cacttgccag 1800
cgccctagcg cccgctcctt tcgctttctt cccttccttt ctcgccacgt tcgccggctt 1860
tccccgtcaa gctctaaatc gggggctccc tttagggttc cgatttagtg ctttacggca 1920
cctcgacccc aaaaaacttg attagggtga tggttcacgt agtgggccat cgccctgata 1980
gacggttttt cgccctttga cgttggagtc cacgttcttt aatagtggac tcttgttcca 2040
aactggaaca acactcaacc ctatctcggt ctattctttt gatttataag ggattttgcc 2100
gatttcggcc tattggttaa aaaatgagct gatttaacaa aaatttaacg cgaattttaa 2160
caaaatatta acgcttacaa tttcctgatg cggtattttc tccttacgca tctgtgcggt 2220
atttcacacc gcatacgcgg atctgcgcag caccatggcc tgaaataacc tctgaaagag 2280
gaacttggtt aggtaccttc tgaggcggaa agaaccagct gtggaatgtg tgtcagttag 2340
ggtgtggaaa gtccccaggc tccccagcag gcagaagtat gcaaagcatg catctcaatt 2400
agtcagcaac caggtgtgga aagtccccag gctccccagc aggcagaagt atgcaaagca 2460
tgcatctcaa ttagtcagca accatagtcc cgcccctaac tccgcccatc ccgcccctaa 2520
ctccgcccag ttccgcccat tctccgcccc atggctgact aatttttttt atttatgcag 2580
aggccgaggc cgcctcggcc tctgagctat tccagaagta gtgaggaggc ttttttggag 2640
gcctaggctt ttgcaaaaag cttgattctt ctgacacaac agtctcgaac ttaaggctag 2700
agccaccatg attgaacaag atggattgca cgcaggttct ccggccgctt gggtggagag 2760
gctattcggc tatgactggg cacaacagac aatcggctgc tctgatgccg ccgtgttccg 2820
gctgtcagcg caggggcgcc cggttctttt tgtcaagacc gacctgtccg gtgccctgaa 2880
tgaactgcag gacgaggcag cgcggctatc gtggctggcc acgacgggcg ttccttgcgc 2940
agctgtgctc gacgttgtca ctgaagcggg aagggactgg ctgctattgg gcgaagtgcc 3000
ggggcaggat ctcctgtcat ctcaccttgc tcctgccgag aaagtatcca tcatggctga 3060
tgcaatgcgg cggctgcata cgcttgatcc ggctacctgc ccattcgacc accaagcgaa 3120
acatcgcatc gagcgagcac gtactcggat ggaagccggt cttgtcgatc aggatgatct 3180
ggacgaagag catcaggggc tcgcgccagc cgaactgttc gccaggctca aggcgcgcat 3240
gcccgacggc gaggatctcg tcgtgaccca tggcgatgcc tgcttgccga atatcatggt 3300
ggaaaatggc cgcttttctg gattcatcga ctgtggccgg ctgggtgtgg cggaccgcta 3360
tcaggacata gcgttggcta cccgtgatat tgctgaagag cttggcggcg aatgggctga 3420
ccgcttcctc gtgctttacg gtatcgccgc tcccgattcg cagcgcatcg ccttctatcg 3480
ccttcttgac gagttcttct gagcgggact ctggggttcg aaatgaccga ccaagcgacg 3540
cccaacctgc catcacgatg gccgcaataa aatatcttta ttttcattac atctgtgtgt 3600
tggttttttg tgtgaatcga tagcgataag gatccgcgta tggtgcactc tcagtacaat 3660
ctgctctgat gccgcatagt taagccagcc ccgacacccg ccaacacccg ctgacgcgcc 3720
ctgacgggct tgtctgctcc cggcatccgc ttacagacaa gctgtgaccg tctccgggag 3780
ctgcatgtgt cagaggtttt caccgtcatc accgaaacgc gcgagacgaa agggcctcgt 3840
gatacgccta tttttatagg ttaatgtcat gataataatg gtttcttaga cgtcaggtgg 3900
cacttttcgg ggaaatgtgc gcggaacccc tatttgttta tttttctaaa tacattcaaa 3960
tatgtatccg ctcatgagac aataaccctg ataaatgctt caataatatt gaaaaaggaa 4020

gagtatgagt attcaacatt tccgtgtcgc ccttattccc ttttttgcgg cattttgcct 4080
tcctgttttt gctcacccag aaacgctggt gaaagtaaaa gatgctgaag atcagttggg 4140
tgcacgagtg ggttacatcg aactggatct caacagcggt aagatccttg agagttttcg 4200
ccccgaagaa cgttttccaa tgatgagcac ttttaaagtt ctgctatgtg gcgcggtatt 4260
atcccgtatt gacgccgggc aagagcaact cggtcgccgc atacactatt ctcagaatga 4320
cttggttgag tactcaccag tcacagaaaa gcatcttacg gatggcatga cagtaagaga 4380
attatgcagt gctgccataa ccatgagtga taacactgcg gccaacttac ttctgacaac 4440
gatcggagga ccgaaggagc taaccgcttt tttgcacaac atgggggatc atgtaactcg 4500
ccttgatcgt tgggaaccgg agctgaatga agccatacca aacgacgagc gtgacaccac 4560
gatgcctgta gcaatggcaa caacgttgcg caaactatta actggcgaac tacttactct 4620
agcttcccgg caacaattaa tagactggat ggaggcggat aaagttgcag gaccacttct 4680
gcgctcggcc cttccggctg gctggtttat tgctgataaa tctggagccg gtgagcgtgg 4740
gtctcgcggt atcattgcag cactggggcc agatggtaag ccctcccgta tcgtagttat 4800
ctacacgacg gggagtcagg caactatgga tgaacgaaat agacagatcg ctgagatagg 4860
tgcctcactg attaagcatt ggtaactgtc agaccaagtt tactcatata tactttagat 4920
tgatttaaaa cttcattttt aatttaaaag gatctaggtg aagatccttt ttgataatct 4980
catgaccaaa atcccttaac gtgagttttc gttccactga gcgtcagacc ccgtagaaaa 5040
gatcaaagga tcttcttgag atcctttttt tctgcgcgta atctgctgct tgcaaacaaa 5100
aaaaccaccg ctaccagcgg tggtttgttt gccggatcaa gagctaccaa ctctttttcc 5160
gaaggtaact ggcttcagca gagcgcagat accaaatact gttcttctag tgtagccgta 5220
gttaggccac cacttcaaga actctgtagc accgcctaca tacctcgctc tgctaatcct 5280
gttaccagtg gctgctgcca gtggcgataa gtcgtgtctt accgggttgg actcaagacg 5340
atagttaccg gataaggcgc agcggtcggg ctgaacgggg ggttcgtgca cacagcccag 5400
cttggagcga acgacctaca ccgaactgag atacctacag cgtgagctat gagaaagcgc 5460
cacgcttccc gaagggagaa aggcggacag gtatccggta agcggcaggg tcggaacagg 5520
agagcgcacg agggagcttc cagggggaaa cgcctggtat ctttatagtc ctgtcgggtt 5580
tcgccacctc tgacttgagc gtcgattttt gtgatgctcg tcaggggggc ggagcctatg 5640
gaaaaacgcc agcaacgcgg cctttttacg gttcctggcc ttttgctggc cttttgctca 5700
catggctcga cagatct 5717

<210> 195
<211> 29
<212> DNA
<213> Homo sapiens
<220>
<221> misc_feature
<223> amorce directe : GV1-1


<400> 195
cgggtcgaca tggcagaagg tggatttga 29

<210> 196
<211> 29
<212> DNA
<213> Homo sapiens
<220>
<221> misc_feature
<223> amorce indirecte:GV1-2


<400> 196
cgcacgcgtc tggttggctt tccaggtag 29

<210> 197
<211> 29
<212> DNA
<213> Homo sapiens
<220>
<221> misc_feature
<223> amorce directe:GV2-1


<400> 197
cgggtcgacg ggatagggat ggggtcagg 29

<210> 198
<211> 29
<212> DNA
<213> Homo sapiens
<220>
<221> misc_feature
<223> amorce indirecte: GV2-2

<400> 198
cgcacgcgtg accggggaat ggaaggaga 29

<210> 199
<211> 30
<212> DNA
<213> Homo sapiens
<220>
<221> misc_feature
<223> amorce directe:GV3-1

<400> 199
cgggtcgacg ctggaggaac cgatttcagg 30

<210> 200
<211> 29
<212> DNA
<213> Homo sapiens
<220>
<221> misc_feature
<223> amorce indirecte: GV3-2

<400> 200
cgcacgcgtt gggtggggaa ggcaatcta 29

<210> 201
<211> 29
<212> DNA
<213> Homo sapiens
<220>
<221> misc_feature
<223> amorce directe: GV4-1

<400> 201
cgggtcgact ggctcaggtg gttggagaa 29

<210> 202
<211> 29
<212> DNA
<213> Homo sapiens
<220>
<221> misc_feature
<223> amorce indirecte: GV4-2

<400> 202
cgcacgcgtg gggatgtccc atcttgtgc 29

<210> 203
<211> 29
<212> DNA
<213> Homo sapiens
<220>
<221> misc_feature
<223> amorce directe: GV5-1

<400> 203
cgggtcgaca cttgcggcag aagctggag 29

<210> 204
<211> 29
<212> DNA
<213> Homo sapiens
<220>
<221> misc_feature
<223> amorce indirecte: GV5-2

<400>  204
cgcacgcgtc cgacacacgt ccttggaga                                          29

<210>  205
<211>  29
<212>  DNA
<213>  Homo sapiens
<220>
<221>  misc_feature
<223>  amorce directe: GV6-1

<400>  205
cgggtcgaca cggaccagat gtggcgaat                                          29

<210>  206
<211>  29
<212>  DNA
<213>  Homo sapiens
<220>
<221>  misc_feature
<223>  amorce indirecte: GV6-2

<400>  206
cgcacgcgta cgagaatgga ggccacgac                                          29

<210>  207
<211>  29
<212>  DNA
<213>  Homo sapiens
<220>
<221>  misc_feature
<223>  amorce directe: GV7-1

<400>  207
cgggtcgaca gcgtcgaaag tggccagaa                                          29

<210>  208
<211>  29
<212>  DNA
<213>  Homo sapiens
<220>
<221>  misc_feature
<223>  amorce indirecte: GV7-2

<400>  208
cgcacgcgtt gcgtgcttgc tttctcagg                                          29

<210>  209
<211>  31
<212>  DNA
<213>  Homo sapiens
<220>
<221>  misc_feature
<223>  amorce directe: GV8-1

<400>  209
cgggtcgacg gagctggtcc tttcagccat a                                       31

<210>  210
<211>  33
<212>  DNA
<213>  Homo sapiens
<220>
<221>  misc_feature
<223>  amorce indirecte: GV8-2

<400>  210
cgcacgcgtc cgtccattct tatcctccat ctg                                    33

<210>  211
<211>  29
<212>  DNA
<213>  Homo sapiens
<220>
<221>  misc_feature
<223>  amorce directe: GV9-1


<400>  211
cgggtcgact gcctctgttc ggtctggtg                                         29

<210>  212
<211>  29
<212>  DNA
<213>  Homo sapiens
<220>
<221>  misc_feature
<223>  amorce indirecte: GV9-2


<400>  212
cgcacgcgtg gaggggagag aacccaaca                                         29

<210>  213
<211>  29
<212>  DNA
<213>  Homo sapiens
<220>
<221>  misc_feature
<223>  amorce directe: GV10-1


<400>  213
cgggtcgact tccagagggg tgacagcaa                                         29

<210>  214
<211>  29
<212>  DNA
<213>  Homo sapiens
<220>
<221>  misc_feature
<223>  amorce directe: GV10-2


<400>  214
cgcacgcgtg atggagctgt gcctgtgga                                         29

<210>  215
<211>  30
<212>  DNA
<213>  Homo sapiens
<220>
<221>  misc_feature
<223>  amorce directe: GV11-1


<400>  215
cgggtcgacg ctgggttcag ttccttgtgc                                        30

<210>  216
<211>  30
<212>  DNA
<213>  Homo sapiens
<220>
<221>  misc_feature
<223>  amorce indirecte: GV11-2


<400>  216

cgcacgcgtc gcatcatcag ggagatctgg                                30

<210>  217
<211>  31
<212>  DNA
<213>  Homo sapiens
<220>
<221>  misc_feature
<223>  amorce directe: GV12-1

<400>  217
cgggtcgacg gtggaacaac ttcgtgagag c                             31

<210>  218
<211>  29
<212>  DNA
<213>  Homo sapiens
<220>
<221>  misc_feature
<223>  amorce  indirecte: GV12-2

<400>  218
cgcacgcgta agtttgggaa ccgctggtg                                29

<210>  219
<211>  33
<212>  DNA
<213>  Homo sapiens
<220>
<221>  misc_feature
<223>  amorce directe: GV13-1

<400>  219
cgggtcgacg gttttctgac atccaatgac acc                           33

<210>  220
<211>  29
<212>  DNA
<213>  Homo sapiens
<220>
<221>  misc_feature
<223>  amorce indirecte: GV13-2

<400>  220
   cgcacgcgtg cgcttgttag gggctgaag                                 29

<210>  221
<211>  33
<212>  DNA
<213>  Homo sapiens
<220>
<221>  misc_feature
<223>  amorce directe: GV14-1

<400>  221
cgggtcgact ctgcgcaaga tctagtcatc tgg                           33

<210>  222
<211>  29
<212>  DNA
<213>  Homo sapiens
<220>
<221>  misc_feature
<223>  amorce indirecte: GV14-2

<400>  222
cgcacgcgtt tccacatcct tgtgggtca                                29

<210> 223
<211> 27
<212> DNA
<213> Homo sapiens
<220>
<221> misc_feature
<223> amorce directe: GS-PGS-F


<400> 223
cgggtcgacg gccgcgggaa ttcgatt 27

<210> 224
<211> 27
<212> DNA
<213> Homo sapiens
<220>
<221> misc_feature
<223> amorce indirecte: GS-PGM-R


<400> 224
cgcacgcgtg cggccgcgaa ttcacta 27

<210> 225
<211> 1874
<212> DNA
<213> Homo sapiens
<220>
<221> misc_feature
<223> séquence appelée GS-N50

<300>
<308> GENBANK/BC008372
<309> 2001-07-12
<313> (1)..(1874)

<400> 225
agttccgcgg ggggctgtcg gggaaccatg gctgccccga gagacaatgt cacttattat 60
tcaagttata ctgcttggca gtgatgaccc tgatggctgc agtctatacc atagctttaa 120
gatacacaag gacatcagac aaagaactct acttttcaac cacagccgtg tgtatcacag 180
aagttataaa gttattgcta agtgtgggaa ttttagctaa agaaactggt agtctgggta 240
gattcaaagc atctttaaga gaaaatgtct tggggagccc caaggaactg ttgaagttaa 300
gtgtgccatc gttagtgtat gctgttcaga acaacatggc tttcctagct cttagcaatc 360
tggatgcagc agtgtaccag gtgacctacc agttgaagat tccgtgtact gctttatgca 420
ctgttttaat gttaaaccgg acactcagca aattacagtg ggtttcagtt tttatgctgt 480
gtgctggagt tacgcttgta cagtggaaac cagcccaagc tacaaaagtg gtggtggaac 540
aaaatccatt attagggttt ggcgctatag ctattgctgt attgtgctca ggatttgcag 600
gagtatattt tgaaaaagtt ttaaagagtt cagatacttc tctttgggtg agaaacattc 660
aaatgtatct atcagggatt attgtgacat tagctggcgt ctacttgtca gatggagctg 720
aaattaaaga aaaaggattt ttctatggtt acacatatta tgtctggttt gtcatctttc 780
ttgcaagtgt tggtggcctc tacacttctg ttgtggttaa gtacacagac aacatcatga 840
aaggcttttc tgcagcagcg gccattgtcc tttccaccat tgcttcagta atgctgtttg 900
gattacagat aacactcacc tttgccctgg gtactcttct tgtatgtgtt tccatatatc 960
tctatggatt acccagacaa gacactacat ccatccaaca aggagaaaca gcttcaaagg 1020
agagagttat tggtgtgtga ttttagcctc acgtgagact ccttttaaga ctaaaccatt 1080
tgcattaaac tagagcctta agtcaatctc agaaggtagc ataaacaaat aaaaattaac 1140
tgtatggcat gatcagtgcg gttatgtgga aacaacaaca aacaaacgaa gctatctgag 1200
tgaactgcta atacagaaac ttaatgtaga cctgtttggg gtctactatt gttttagaat 1260
gaaggaattg tattattgtg tgtatatata atttgtaaat aaaaagtatg gagatgatac 1320
ggtgttaaaa aaaatcatgg taaggctaca atactcaagt aacaaggttt gggacaatgt 1380
ctaagggtta aagtgccaaa gccatttctg tactaactgt tctcttgttc cggtaccggg 1440
gagaaggatg acccctcctt attctccaat tcatgtacag tattttgtcc tagcagcata 1500
aagacctagc tcttttctta caagaggcag aaacaagaca ggctagttca taaacaaact 1560
gtgtaacttc tcaaaatgaa tctatttcat aactcggaca atttctgggt ggtgactgag 1620
taccccttta gtgagtaccc ctttagtgct atatttgtgc cattcattat ctggttcata 1680
tttcttttct gttagatgat acacatttct tcaaaaaaat ttctaatgtc acttttgtac 1740
ttttttaaat aaagtatgtt taactgttgg gctctcaata atttgtgaaa tttcagtgtt 1800
ttctataatg ttaatgggga aattcagcaa taaactttat ttgcaaaaaa aaaaaaaaaa 1860
aaaaaaaaaa aaaa 1874

<210> 226

<211>  30
<212>  DNA
<213>  Homo sapiens
<220>
<221>  misc_feature
<223>  amorce directe: GV16-1

<400>  226
cgggtcgaca acacaggccc aaaaggtgtg                                    30

<210>  227
<211>  29
<212>  DNA
<213>  Homo sapiens
<220>
<221>  misc_feature
<223>  amorce indirecte: GV16-2

<400>  227
cgcacgcgtc cctggaatgc catcctcat                                     29

<210>  228
<211>  29
<212>  DNA
<213>  Homo sapiens
<220>
<221>  misc_feature
<223>  amorce directe: GV17-1

<400>  228
cgggtcgacg ctgtgtggtg ggaaccaag                                     29

<210>  229
<211>  29
<212>  DNA
<213>  Homo sapiens
<220>
<221>  misc_feature
<223>  amorce indirecte: GV17-2

<400>  229
cgcacgcgtt gagaccccac caactgcaa                                     29

<210>  230
<211>  29
<212>  DNA
<213>  Homo sapiens
<220>
<221>  misc_feature
<223>  amorce directe: GV18-1

<400>  230
cgggtcgact ggcggcacaa tcactatca                                     29

<210>  231
<211>  29
<212>  DNA
<213>  Homo sapiens
<220>
<221>  misc_feature
<223>  amorce indirecte: GV18-2

<400>  231
cgcacgcgtg ctggtgggtt cctctggtg                                     29

<210>  232
<211>  29

<212>  DNA
<213>  Homo sapiens
<220>
<221>  misc_feature
<223>  amorce directe: GV19-1


<400>  232
cgggtcgact atatgcgtca ggcaggaga                                    29

<210>  233
<211>  29
<212>  DNA
<213>  Homo sapiens
<220>
<221>  misc_feature
<223>  amorce indirecte: GV19-2


<400>  233
cgcacgcgtg ctcttacggg aatgtctgc                                    29

<210>  234
<211>  32
<212>  DNA
<213>  Homo sapiens
<220>
<221>  misc_feature
<223>  amorce directe: GV20-1


<400>  234
cgggtcgacg gagctgggag agagatctag ca                                32

<210>  235
<211>  33
<212>  DNA
<213>  Homo sapiens
<220>
<221>  misc_feature
<223>  amorce indirecte: GV20-2


<400>  235
cgcacgcgtc agatcttgta gatggctggg tca                               33

<210>  236
<211>  29
<212>  DNA
<213>  Homo sapiens
<220>
<221>  misc_feature
<223>  amorce directe: GV21-1


<400>  236
cgggtcgact ggccaacgag cacaagaaa                                    29

<210>  237
<211>  29
<212>  DNA
<213>  Homo sapiens
<220>
<221>  misc_feature
<223>  amorce indirecte: GV21-2


<400>  237
cgcacgcgtt tccactgcag gcgggttat                                    29

<210>  238
<211>  33
<212>  DNA

<213>  Homo sapiens
<220>
<221>  misc_feature
<223>  amorce directe: GV22-1


<400>  238
cgggtcgacg agttcaattc cacaccagct gtc                                    33

<210>  239
<211>  29
<212>  DNA
<213>  Homo sapiens
<220>
<221>  misc_feature
<223>  amorce indirecte: GV22-2


<400>  239
cgcacgcgtg aaaggccgtc catcaaagc                                         29

<210>  240
<211>  29
<212>  DNA
<213>  Homo sapiens
<220>
<221>  misc_feature
<223>  amorce directe: GV23-1


<400>  240
cgggtcgacg ctttgggtga gagcccaga                                         29

<210>  241
<211>  29
<212>  DNA
<213>  Homo sapiens
<220>
<221>  misc_feature
<223>  amorce indirecte: GV23-2


<400>  241
cgcacgcgtc cgggtggtca agacagaca                                         29

<210>  242
<211>  29
<212>  DNA
<213>  Homo sapiens
<220>
<221>  misc_feature
<223>  amorce directe: GV24-1


<400>  242
cgggtcgacg gtgaggggaa gtggcttcg                                         29

<210>  243
<211>  30
<212>  DNA
<213>  Homo sapiens
<220>
<221>  misc_feature
<223>  amorce indirecte: GV24-2


<400>  243
cgcacgcgta aaatgacgct ccacctgctg                                        30

<210>  244
<211>  31
<212>  DNA
<213>  Homo sapiens

<220>
<221>  misc_feature
<223>  amorce directe: GV25-1


<400>  244
cgggtcgacc catccaccaa ggagaaacag c                                    31

<210>  245
<211>  31
<212>  DNA
<213>  Homo sapiens
<220>
<221>  misc_feature
<223>  amorce indirecte: GV25-2


<400>  245
cgcacgcgtc ccttagacat tgtcccaaac c                                    31

<210>  246
<211>  29
<212>  DNA
<213>  Homo sapiens
<220>
<221>  misc_feature
<223>  amorce directe: GV26-1


<400>  246
cgggtcgact gccttcatga ggtccacca                                       29

<210>  247
<211>  29
<212>  DNA
<213>  Homo sapiens
<220>
<221>  misc_feature
<223>  amorce indirecte: GV26-2


<400>  247
cgcacgcgtt tccctgctgc catcaatca                                       29

<210>  248
<211>  29
<212>  DNA
<213>  Homo sapiens
<220>
<221>  misc_feature
<223>  amorce directe: GV27-1


<400>  248
cgggtcgacg catggccctg tttcctgtt                                       29

<210>  249
<211>  29
<212>  DNA
<213>  Homo sapiens
<220>
<221>  misc_feature
<223>  amorce indirecte: GV27-2


<400>  249
cgcacgcgtc gagtgggcag gcctgtatc                                       29

<210>  250
<211>  29
<212>  DNA
<213>  Homo sapiens
<220>

<221>  misc_feature
<223>  amorce directe: GV28-1

<400>  250
cgggtcgact tccctacccc accccagtc                                    29

<210>  251
<211>  29
<212>  DNA
<213>  Homo sapiens
<220>
<221>  misc_feature
<223>  amorce indirecte: GV28-2

<400>  251
cgcacgcgtt gggaagcatt ggctggttc                                    29

<210>  252
<211>  29
<212>  DNA
<213>  Homo sapiens
<220>
<221>  misc_feature
<223>  amorce directe: GV29-1

<400>  252
cgggtcgacg gtcccgtcaa gggcactct                                    29

<210>  253
<211>  29
<212>  DNA
<213>  Homo sapiens
<220>
<221>  misc_feature
<223>  amorce indirecte: GV30-2

<400>  253
cgcacgcgtt cccacaactt tcgctgcat                                    29

<210>  254
<211>  29
<212>  DNA
<213>  Homo sapiens
<220>
<221>  misc_feature
<223>  amorce directe: GV30-1

<400>  254
cgggtcgacc tgagcctgct ctgggaagc                                    29

<210>  255
<211>  29
<212>  DNA
<213>  Homo sapiens
<220>
<221>  misc_feature
<223>  amorce indirecte: GV30-2

<400>  255
cgcacgcgtt gaggctgggt tctgggttc                                    29

<210>  256
<211>  29
<212>  DNA
<213>  Homo sapiens
<220>
<221>  misc_feature

<223>  amorce  directe: GV31-1

<400>  256
cgggtcgacg ggagctgagc cagtcatgg                                        29

<210>  257
<211>  29
<212>  DNA
<213>  Homo sapiens
<220>
<221>  misc_feature
<223>  amorce indirecte: GV31-2

<400>  257
   cgcacgcgta gctggccttg gggttgaag                                         29

<210>  258
<211>  30
<212>  DNA
<213>  Homo sapiens
<220>
<221>  misc_feature
<223>  amorce directe: GV32-1

<400>  258
cgggtcgact ccctgtggac cctgaagaaa                                       30

<210>  259
<211>  29
<212>  DNA
<213>  Homo sapiens
<220>
<221>  misc_feature
<223>  amorce indirecte: GV32-2

<400>  259
cgcacgcgtc ctctttcagc ctgggcact                                        29

<210>  260
<211>  31
<212>  DNA
<213>  Homo sapiens
<220>
<221>  misc_feature
<223>  amorce directe: GV33-1

<400>  260
cgggtcgact tgaagcactc aagccatcca c                                     31

<210>  261
<211>  29
<212>  DNA
<213>  Homo sapiens
<220>
<221>  misc_feature
<223>  amorce indirecte: GV33-2

<400>  261
cgcacgcgtt ggaagcgcta aggcgtgac                                        29

<210>  262
<211>  30
<212>  DNA
<213>  Homo sapiens
<220>
<221>  misc_feature
<223>  amorce directe: GV34-1

<400>  262
cgggtcgact ggagctggag gagatgttgg                                    30

<210>  263
<211>  29
<212>  DNA
<213>  Homo sapiens
<220>
<221>  misc_feature
<223>  amorce indirecte: GV34-2


<400>  263
cgcctcgagt tccgggcctg actctggta                                     29

<210>  264
<211>  29
<212>  DNA
<213>  Homo sapiens
<220>
<221>  misc_feature
<223>  amorce directe: GV35-1


<400>  264
cgggtcgacg agcaggccgt ggaacacat                                     29

<210>  265
<211>  29
<212>  DNA
<213>  Homo sapiens
<220>
<221>  misc_feature
<223>  amorce indirecte: GV35-2


<400>  265
cgcacgcgtt gcttggtgtg caggcttgt                                     29

<210>  266
<211>  33
<212>  DNA
<213>  Homo sapiens
<220>
<221>  misc_feature
<223>  amorce directe: GV36-1


<400>  266
cgggtcgacc agaggaacct acaacctctg atg                                33

<210>  267
<211>  29
<212>  DNA
<213>  Homo sapiens
<220>
<221>  misc_feature
<223>  amorce indirecte: GV36-2


<400>  267
cgcacgcgtt ggagctgggt gagttgatg                                     29

<210>  268
<211>  29
<212>  DNA
<213>  Homo sapiens
<220>
<221>  misc_feature
<223>  amorce directe: GV37-1

<400>  268
cgggtcgaca ggcctctcgg ggagtgaac                                29

<210>  269
<211>  29
<212>  DNA
<213>  Homo sapiens
<220>
<221>  misc_feature
<223>  amorce indirecte: GV37-2


<400>  269
cgcacgcgtt ccaaggattt ggcctgtgg                                29

<210>  270
<211>  33
<212>  DNA
<213>  Homo sapiens
<220>
<221>  misc_feature
<223>  amorce directe: GV38-1


<400>  270
cgggtcgacg ctggtgtaca tagtgttggg cta                           33

<210>  271
<211>  36
<212>  DNA
<213>  Homo sapiens
<220>
<221>  misc_feature
<223>  amorce indirecte: GV38-2


<400>  271
cgcacgcgtc tgtagagagt tcctgtaaac tatcct                        36

<210>  272
<211>  29
<212>  DNA
<213>  Homo sapiens
<220>
<221>  misc_feature
<223>  amorce directe: GV39-1


<400>  272
cgggtcgacc ctggcttttc ctccctcag                                29

<210>  273
<211>  27
<212>  DNA
<213>  Homo sapiens
<220>
<221>  misc_feature
<223>  amorce indirecte: GV40-2


<400>  273
cgcacgcgtg ctgggggaag ggtgcag                                  27

<210>  274
<211>  33
<212>  DNA
<213>  Homo sapiens
<220>
<221>  misc_feature
<223>  amorce directe: GV40-1

<400> 274
cgggtcgacg ggtcaccttt gatgatgatc ctg 33

<210> 275
<211> 31
<212> DNA
<213> Homo sapiens
<220>
<221> misc_feature
<223> amorce indirecte: GV40-2

<400> 275
cgcacgcgtt ggaggaagat ccactgggaa a 31

<210> 276
<211> 27
<212> DNA
<213> Homo sapiens
<220>
<221> misc_feature
<223> amorce directe: GV41-1

<400> 276
cgggtcgacg tggacagcgc ggaggag 27

<210> 277
<211> 29
<212> DNA
<213> Homo sapiens
<220>
<221> misc_feature
<223> amorce indirecte: GV41-2

<400> 277
cgcacgcgtc gagcttcatt ccgcatcag 29

<210> 278
<211> 33
<212> DNA
<213> Homo sapiens
<220>
<221> misc_feature
<223> amorce directe: GV42-1

<400> 278
cgggtcgact ggacgaatat ggttgtccca gag 33

<210> 279
<211> 31
<212> DNA
<213> Homo sapiens
<220>
<221> misc_feature
<223> amorce indirecte: GV42-2

<400> 279
cgcacgcgtt ggttccgttg ggttaacagt g 31

<210> 280
<211> 29
<212> DNA
<213> Homo sapiens
<220>
<221> misc_feature
<223> amorcedirecte: GV43-1

<400> 280

cgggtcgacc agcaaaagcc gggtttcat                                    29

<210>  281
<211>  31
<212>  DNA
<213>  Homo sapiens
<220>
<221>  misc_feature
<223>  amorce indirecte: GV43-2


<400>  281
cgcacgcgtg caaaaactgc cacaatcttg c                                31

<210>  282
<211>  29
<212>  DNA
<213>  Homo sapiens
<220>
<221>  misc_feature
<223>  amorce directe: GV44-1


<400>  282
cgggtcgacg ctggagtggc gtttggaat                                   29

<210>  283
<211>  31
<212>  DNA
<213>  Homo sapiens
<220>
<221>  misc_feature
<223>  amorce indirecte: GV44-23


<400>  283
cgcacgcgtg gcagcctgaa gaaggtagga a                                31

<210>  284
<211>  4197
<212>  DNA
<213>  Homo sapiens
<220>
<221>  misc_feature
<222>  (1951)..(1951)
<223>  n est un résidu inconnu

<220>
<221>  misc_feature
<222>  (1952)..(1952)
<223>  n est un résidu inconnu

<220>
<221>  misc_feature
<222>  (1953)..(1953)
<223>  n est un résidu inconnu

<220>
<221>  misc_feature
<222>  (1954)..(1954)
<223>  n est un résidu inconnu

<220>
<221>  misc_feature
<222>  (1955)..(1955)
<223>  n est un résidu inconnu

<220>
<221>  misc_feature
<222>  (1956)..(1956)
<223>  n est un résidu inconnu

<220>

<221>  misc_feature
<222>  (1957)..(1957)
<223>  n est un résidu inconnu

<220>
<221>  misc_feature
<222>  (1958)..(1958)
<223>  n est un résidu inconnu

<220>
<221>  misc_feature
<222>  (1959)..(1959)
<223>  n est un résidu inconnu

<300>
<308>  Genbank/XM_038751
<309>  2001-10-16

<400>  284
gatgaagagg aagaaatcaa actggagata aatatgctaa agaaatactc tcatcacaga      60
aacattgcaa catattatgg tgctttcatc aaaaagagcc ctccaggaca tgatgaccaa     120
ctctggcttg ttatggagtt ctgtggggct gggtccatta cagaccttgt gaagaacacc     180
aaagggaaca cactcaaaga agactggatc gcttacatct ccagagaaat cctgagggga     240
ctggcacatc ttcacattca tcatgtgatt caccgggata tcaagggcca gaatgtgttg     300
ctgactgaga atgcagaggt gaaacttgtt gactttggtg tgagtgctca gctggacagg     360
actgtggggc ggagaaatac gttcataggc actccctact ggatggctcc tgaggtcatc     420
gcctgtgatg agaacccaga tgccacctat gattacagaa gtgatctttg gtcttgtggc     480
attacagcca ttgagatggc agaaggtgct ccccctctct gtgacatgca tccaatgaga     540
gcactgtttc tcattcccag aaaccctcct ccccggctga agtcaaaaaa atggtcgaag     600
aagtttttta gttttataga agggtgcctg gtgaagaatt acatgcagcg gccctctaca     660
gagcagcttt tgaaacatcc ttttataagg gatcagccaa atgaaaggca agttagaatc     720
cagcttaagg atcatataga tcgtaccagg aagaagagag gcgagaaaga tgaaactgag     780
tatgagtaca gtgggagtga ggaagaagag gaggaagtgc ctgaacagga aggagagcca     840
agttccattg tgaacgtgcc tggtgagtct actcttcgcc gagatttcct gagactgcag     900
caggagaaca aggaacgttc cgaggctctt cggagacaac agttactaca ggagcaacag     960
ctccgggagc aggaagaata taaaaggcaa ctgctggcag agagacagaa gcggattgag    1020
cagcagaaag aacagaggcg acggctagaa gagcaacaaa ggagagagcg ggaagctaga    1080
aggcagcagg aacgtgaaca gcgaaggaga gaacaagaag aaaagaggcg tctagaggag    1140
ttggagagaa ggcgcaaaga agaagaggag aggagacggg cagaagaaga aaagaggaga    1200
gttgaaagag aacaggagta tatcaggcga cagctagaag aggagcagcg gcacttggaa    1260
gtccttcagc agcagctgct ccaggagcag gccatgttac tgcatgacca taggaggccg    1320
cacccgcagc actcgcagca gccgccacca ccgcagcagg aaaggagcaa gccaagcttc    1380
catgctcccg agcccaaagc ccactacgag cctgctgacc gagcgcgaga ggtggaagat    1440
agatttagga aaactaacca cagctcccct gaagcccagt ctaagcagac aggcagagta    1500
ttggagccac cagtgccttc ccgatcagag tctttttcca atggcaactc cgagtctgtg    1560
catcccgccc tgcagagacc agcggagcca caggttcctg tgagaacaac atctcgctcc    1620
cctgttctgt cccgtcgaga ttccccactg cagggcagtg ggcagcagaa tagccaggca    1680
ggacagagaa actccaccag cagtattgag cccaggcttc tgtgggagag agtggagaag    1740
ctggtgccca gacctggcag tggcagctcc tcagggtcca gcaactcagg atcccagccc    1800
gggtctcacc ctgggtctca gagtggctcc ggggaacgct tcagagtgag atcatcatcc    1860
aagtctgaag gctctccatc tcagcgcctg gaaaatgcag tgaaaaaacc tgaagataaa    1920
aaggaagttt tcagacccct caagcctgct nnnnnnnnng atctgaccgc actggccaaa    1980
gagcttcgag cagtggaaga tgtacggcca cctcacaaag taacggacta ctcctcatcc    2040
agtgaggagt cggggacgac ggatgaggag gacgacgatg tggagcagga aggggctgac    2100
gagtccacct caggaccaga ggacaccaga gcagcgtcat ctctgaattt gagcaatggt    2160
gaaacggaat ctgtgaaaac catgattgtc catgatgatg tagaaagtga gccggccatg    2220
accccatcca aggagggcac tctaatcgtc cgccagactc agtccgctag tagcacactc    2280
cagaaacaca aatcttcctc ctcctttaca ccttttatag accccagatt actacagatt    2340
tctccatcta gcggaacaac agtgacatct gtggtgggat tttcctgtga tgggatgaga    2400
ccagaagcca taaggcaaga tcctacccgg aaaggctcag tggtcaatgt gaatcctacc    2460
aacactaggc cacagagtga caccccggag attcgtaaat acaagaagag gtttaactct    2520
gagattctgt gtgctgcctt atggggagtg aatttgctag tgggtacaga gagtggcctg    2580
atgctgctgg acagaagtgg ccaagggaag gtctatcctc ttatcaaccg aagacgattt    2640
caacaaatgg acgtacttga gggcttgaat gtcttggtga caatatctgg caaaaaggat    2700
aagttacgtg tctactattt gtcctggtta agaaataaaa tacttcacaa tgatccagaa    2760
gttgagaaga agcagggatg gacaaccgta ggggatttgg aaggatgtgt acattataaa    2820
gttgtaaaat atgaaagaat caaatttctg gtgattgctt tgaagagttc tgtggaagtc    2880
tatgcgtggg caccaaagcc atatcacaaa tttatggcct ttaagtcatt tggagaattg    2940
gtacataagc cattactggt ggatctcact gttgaggaag gccagaggtt gaaagtgatc    3000
tatggatcct gtgctggatt ccatgctgtt gatgtggatt caggatcagt ctatgacatt    3060
tatctaccaa cacatatcca gtgtagcatc aaaccccatg caatcatcat cctccccaat    3120
acagatggaa tggagcttct ggtgtgctat gaagatgagg gggtttatgt aaacacatat    3180
ggaaggatca ccaaggatgt agttctacag tggggagaga tgcctacatc agtagcatat    3240

attcgatcca atcagacaat gggctgggga gagaaggcca tagagatccg atctgtggaa 3300
actggtcact tggatggtgt gttcatgcac aaaagggctc aaagactaaa attcttgtgt 3360
gaacgcaatg acaaggtgtt ctttgcctct gttcggtctg gtggcagcag tcaggtttat 3420
ttcatgacct taggcaggac ttctcttctg agctggtaga agcagtgtga tccagggatt 3480
actggcctcc agagtcttca agatcctgag aacttggaat tccttgtaac tggagctcgg 3540
agctgcaccg agggcaacca ggacagctgt gtgtgcagac ctcatgtgtt gggttctctc 3600
ccctccttcc tgttcctctt atataccagt ttatccccat tctttttttt tttcttactc 3660
caaaataaat caaggctgca atgcagctgg tgctgttcag attctaccat caggtgctat 3720
aagtgtttgg gattgagcat catactggaa agcaaacacc tttcctccag ctccagaatt 3780
ccttgtctct gaatgactct gtcttgtggg tgtctgacag tggcgacgat gaacatgccg 3840
ttggttttat tggcagtggg cacaaggagg tgagaagtgg tggtaaaagg agcggagtgc 3900
tgaagcagag agcagattta atatagtaac attaacagtg tatttaattg acatttcttt 3960
tttgtaatgt gacgatatgt ggacaaagaa gaagatgcag gtttaagaag ttaatattta 4020
taaaatgtga aagacacagt tactaggata acttttttgt gggtggggct tgggagatgg 4080
ggtggggtgg gttaaggggt cccattttgt ttctttggat ttggggtggg ggtcctggcc 4140
aagaactcag tcatttttct gtgtaccagg ttgcctaaat catgtgcaga tggttct 4197

<210> 285
<211> 1304
<212> DNA
<213> Homo sapiens
<300>
<308> Genbank/BC006483
<309> 2001-07-12

<400> 285
atttgatggc gtgatgtctc acagaaagtt ctccgctccc agacatgggt ccctcggctt 60
cctgcctcgg aagcgcagca gcaggcatcg tgggaaggtg aagagcttcc ctaaggatga 120
cccatccaag ccggtccacc tcacagcctt cctgggatac aaggctggca tgactcacat 180
cgtgcgggaa gtcgacaggc cgggatccaa ggtgaacaag aaggaggtgg tggaggctgt 240
gaccattgta gagacaccac ccatggtggt tgtgggcatt gtgggctacg tggaaacccc 300
tcgaggcctc cggaccttca agactgtctt tgctgagcac atcagtgatg aatgcaagag 360
gcgtttctat aagaattggc ataaatctaa gaagaaggcc tttaccaagt actgcaagaa 420
atggcaggat gaggatggca agaagcagct ggagaaggac ttcagcagca tgaagaagta 480
ctgccaagtc atccgtgtca ttgcccacac ccagatgcgc ctgcttcctc tgcgccagaa 540
gaaggcccac ctgatggaga tccaggtgaa cggaggcact gtggccgaga agctggactg 600
ggcccgcgag aggcttgagc agcaggtacc tgtgaaccaa gtgtttgggc aggatgagat 660
gatcgacgtc atcggggtga ccaagggcaa aggctacaaa ggggtcacca gtcgttggca 720
caccaagaag ctgccccgca agacccaccg aggcctgcgc aaggtggcct gtattggggc 780
atggcatcct gctcgtgtag ccttctctgt ggcacgcgct gggcagaaag gctaccatca 840
ccgcactgag atcaacaaga agatttataa gattggccag ggctacctta tcaaggacgg 900
caagctgatc aagaacaatg cctccactga ctatgaccta tctgacaaga gcatcaaccc 960
tctgggtggc tttgtccact atggtgaagt gaccaatgac tttgtcatgc tgaaaggctg 1020
tgtggtggga accaagaagc gggtgctcac cctccgcaag tccttgctgg tgcagacgaa 1080
gcggcgggct ctggagaaga ttgaccttaa gttcattgac accacctcca agtttggcca 1140
tggccgcttc cagaccatgg aggagaagaa agcattcatg ggaccactga agaaagaccg 1200
aattgcaaag gaagaaggag cttaatgcca ggaacagatt ttgcagttgg tggggtctca 1260
ataaaagtta ttttccactg aaaaaaaaaa aaaaaaaaaa aaag 1304

<210> 286
<211> 1332
<212> DNA
<213> Homo sapiens
<300>
<308> Genbank/BC012786
<309> 2001-08-20

<400> 286
ggcacgagga tttgatggcg tgatgtctca cagaaagttc tccgctccca gacatgggtc 60
cctcggcttc ctgcctcgga agcgcagcag caggcatcgt gggaaggtga agagcttccc 120
taaggatgac ccatccaagc cggtccacct cacagccttc ctgggataca aggctggcat 180
gactcacatc gtgcgggaag tcgacaggcc gggatccaag gtgaacaaga aggaggtggt 240
ggaggctgtg accattgtag agacaccacc catggtggtt gtgggcattg tgggctacgt 300
ggaaacccct cgaggcctcc ggaccttcaa gactgtcttt gctgagcaca tcagtgatga 360
atgcaagagg cgtttctata agaattggca taaatctaag aagaaggcct ttaccaagta 420
ctgcaagaaa tggcaggatg aggatggcaa gaagcagctg gagaaggact tcagcagcat 480
gaagaagtac tgccaagtca tccgtgtcat tgcccacacc cagatgcgcc tgcttcctct 540
gcgccagaag aaggcccacc tgatggagat ccaggtgaac ggaggcactg tggccgagaa 600
gctggactgg gcccgcgaga ggcttgagca gcaggtacct gtgaaccaag tgtttgggca 660
ggatgagatg atcgacgtca tcggggtgac caagggcaaa ggctacaaag gggtcaccag 720
tcgttggcac accaagaagc tgccccgcaa gacccaccga ggcctgcgca aggtggcctg 780
tattggggca tggcatcctg ctcgtgtagc cttctctgtg gcacgcgctg ggcagaaagg 840
ctaccatcac cgcactgaga tcaacaagaa gatttataag attggccagg gctaccttat 900

caaggacggc aagctgatca agaacaatgc ctccactgac tatgacctat ctgacaagag 960
catcaaccct ctgggtggct ttgtccacta tggtgaagtg accaatgact ttgtcatgct 1020
gaaaggctgt gtggtgggaa ccaagaagcg ggtgctcacc ctccgcaagt ccttgctggt 1080
gcagacgaag cggcgggctc tggagaagat tgaccttaag ttcattgaca ccacctccaa 1140
gtttggccat ggccgcttcc agaccatgga ggagaagaaa gcattcatgg gaccactgaa 1200
gaaagaccga attgcaaagg aagaaggagc ttaatgccag gaacagattt tgcagttggt 1260
ggggtctcaa taaaagttat tttccactga aaaaaaaaaa aaaaaaaaaa aaaaaaaaaa 1320
aaaaaaaaaa aa 1332

<210> 287
<211> 3936
<212> DNA
<213> Homo sapiens
<300>
<308> Genbank/AF265230
<309> 2001-06-02

<400> 287
ggaagttctg ccttgtctcc gccgcgggtc aggggtgaga gctggaatct ctgcacgggc 60
cttggaaaac gactgtcttc ttctgccaaa atgtcaggaa ttggaaataa aagagcagct 120
ggagaacctg gcacctccat gcctcctgag aagaaggcag ctgttgaaga ttcagggacc 180
acagtggaaa caattaagct aggaggtgtc tcttcaacgg aggaactaga cattagaaca 240
ctgcaaacca aaaatcgcaa gctggcagaa atgttggatc agcggcaggc cattgaagat 300
gaacttcgtg agcacattga aaaactggaa cgacgacagg ccactgatga tgcctcacta 360
ttgattgtca accgatactg gagtcagttt gatgaaaaca tccgtatcat ccttaaacgt 420
tatgatctgg agcagggctt gggagaccta ctcacagaac gaaaagccct tgttgtgcct 480
gaaccagaac cagactctga tagcaatcag gagcgtaaag atgaccgaga gagaggggaa 540
gggcaagagc cagctttctc tttccttgct actttggcca gcagttccag tgaagagatg 600
gagtctcagc tgcaggaacg tgtggagtct tcccgccgag ccgtgtccca gattgtgact 660
gtttatgata aattgcaaga aaaagtggag ctcttatccc ggaagctaaa cagtggagat 720
aatctgatag tggaggaagc agtgcaggag ctgaactctt tcctcgcaca ggagaatatg 780
aggctacagg aattgacaga tcttcttcag gaaaagcatc gcaccatgtc tcaggagttc 840
tccaagttgc agagtaaagt ggagacagcc gaatcacgag tgtctgtcct ggagtccatg 900
attgatgacc tgcagtggga tattgacaaa attcgaaaga gggaacagcg actcgaccga 960
cacttagcag aagtcctaga acgggtgaat tccaaaggtt ataaggtgta tggagcgggg 1020
agcagtctgt atggcggcac aatcactatc aatgctcgga agtttgagga aatgaatgca 1080
gagcttgagg agaacaaaga gttggctcag aaccgtctct gtgagctgga gaaacttcgg 1140
caagactttg aggaggtcac tacacaaaat gaaaagctga aggtggaatt gcggagtgca 1200
gtggagcaag tcgttaagga aactccagaa tatcgctgca tgcagtcaca gttctccgtc 1260
ttgtataatg agagcctaca gttgaaagca cacttggatg aggctcggac cctgcttcat 1320
ggcaccagag gaacccacca gcaccaggtt gagcttattg agcgagatga ggttagtctt 1380
cataagaagc tgaggactga agtaattcag ctagaagata cattggccca ggtccgcaag 1440
gagtatgaaa tgctgaggat agaatttgag cagacccttg ctgccaatga acaagcaggc 1500
cctataaaca gggagatgcg ccacctcatc agtagcctcc agaatcacaa tcaccagctg 1560
aaaggggagg tcctgagata taagcggaaa ttgagagaag cccagtctga cctgaacaag 1620
acacgcctgc gtagtggtag tgccctcctg cagtcccagt ctagtactga ggacccgaag 1680
gatgagcctg cggagctaaa accagattct gaggacttat cctcccagtc ctcagcttca 1740
aaggcatctc aggaggatgc caatgaaatc aagtctaaac gggatgaaga agaacgagaa 1800
cgagaaagga gggagaagga gagggaacga gaaagagaac gggagaagga gaaggagaga 1860
gaacgagaga agcagaagct aaaagagtca gaaaaagaga gagattctgc taaggataaa 1920
gagaaaggca aacatgatga tggacggaaa aaggaagcag aaattatcaa acaattgaag 1980
attgaactca agaaggcaca ggagagccaa aaggagatga aactattgct ggatatgtac 2040
cgttctgccc caaaggaaca gagagacaaa gttcagctga tggcagctga gaagaagtct 2100
aaggcagagt tggaagatct aaggcaaaga ctcaaggatc tggaagataa agagaagaaa 2160
gagaacacga aaatggctga tgaggatgcc ttgaggaaga tccgggcagt ggaggagcag 2220
atagaatacc tacagaagaa gctagccatg gccaagcagg aagaagaagc actcctctct 2280
gaaatggatg tcacaggcca ggcctttgaa gacatgcagg agcaaaatat ccgtttgatg 2340
cagcaattgc gggagaagga tgatgcaaat ttcaagctca tgtcagagcg tatcaagtcc 2400
aatcagatcc ataagttgct taaagaagag aaggaggagc tggcagacca ggtgttgact 2460
ctgaagactc aggttgatgc ccagctacag gtagtaagga aactggaaga gaaggagcat 2520
ctgttacaga gcaacattgg cacaggggag aaggagctgg gtcttaggac ccaagcctta 2580
gagatgaata aacgcaaggc aatggaggca gcccagcttg cagatgacct caaagcacaa 2640
ctggagttgg ctcagaagaa gctacatgat tttcaggatg agatcgtgga gaacagtgtt 2700
accaaagaaa aggacatgtt caatttcaaa cgagcccagg aggacatctc tagacttcgc 2760
aggaagctgg agaccacaaa gaaaccagac aatgtaccca agtgtgatga gattctgatg 2820
gaagagatta aggattacaa ggcacgcttg acctgtccat gctgtaacat gcgtaaaaag 2880
gatgctgttc ttactaagtg ttttcatgtc ttctgctttg agtgtgtgaa gacacgctat 2940
gacacccgcc agcgcaaatg tcccaagtgt aatgctgctt ttggtgccaa tgattttcat 3000
cgcatctaca ttggttgatc taagtcaaga gaagaagagg agctggctag tcaggaactt 3060
attcattaac caccaaacct ctacctcttc tctccttgac tgtcacctgt aggacagttt 3120
atcagtcaac tacctttcct ccagacttta cttccaggct ctcctcttca gtagctggat 3180
gactttagca gaaaggactg gtaaatacaa gccttgggtt tcagaatgaa ttagaaacaa 3240
ataactctta ctgtcttccc tcccagcttt gtttattttg tgcttttaga cttttcagtg 3300

ttttcttttt ccagcccact gtataaactt ggattgtcca ttcctcctga agaaatcaag 3360
ttggtatttt tgatgtggaa aagggaacaa aagtggaaac atggctactt ttggggagtg 3420
atattttaaa aaataagttg tctatgggca caaagttttc ttcatttgtg tagcaaactt 3480
cttgtgaatg tggattacaa aatggtataa ttgtgctact ctcccctggg tggtttgcag 3540
ccctaatgaa attatgtcta ggatgattca gtctatttcc catttactag cagagtaact 3600
tgttaagatc agctggcttt cttgttaaag ttatttaagt tttgaatgct ctactacttc 3660
aagtctttaa atttcttgag actagaataa ttttaaataa tatgaccctt tgtcttctaa 3720
tgaaataaag attgaagagg ttgagtcagg actgagctgg tgaagaaatc ttgtgggtat 3780
tctggaaatt tgatacggag agaacttggt gagctatgaa ttactctcag tctccttttt 3840
acagggttgt tgtgatccct cttttccaga aaattctgtg gaatgtttct gtaggacttt 3900
gttctccaca agcttgaatt aaagcaggat tcagtt 3936

<210> 288
<211> 2994
<212> DNA
<213> Homo sapiens
<300>
<308> Genbank/NM_006910
<309> 2001-03-06

<400> 288
ggcacgagga aacctctagg tccaccacct ccatcttaca cgtgtttccg ttgtggtaaa 60
cctggacatt atattaagaa ttgcccaaca aatggggata aaaactttga atctggtcct 120
aggattaaaa agagcactgg aattcccaga agttttcatg atggaagtga aagatcctaa 180
tatgaaaggt gcaatgctta ccaacactgg aaaatatgca ataccaacta tagatgcaga 240
agcatatgca attgggaaga aagagaaacc tcccttctta ccagaggagc catcttcttc 300
ctcagaagaa gatgatccta tcccagatga attgttgtgt ctcatctgca aggatattat 360
gactgatgct gttgtgattc cctgctgtgg aaacagttac tgtgatgaat gtataagaac 420
agcactcctg gaatcagatg agcacacatg tccgacgtgt catcaaaatg atgtttctcc 480
tgatgcttta attgccaata aatttttacg acaggctgta aataacttca aaaatgaaac 540
tggctataca aaaagactac gaaaacagtt acctcctcca ccacccccaa taccacctcc 600
gagaccactg attcagagga acctacaacc tctgatgaga tctccgatat caagacaaca 660
agatcctctt atgattccag tgacatcttc atcaactcac ccagctccgt ctatatcttc 720
attaacttct aatcagtctt ccttggcccc tcctgtgtct ggaaatccgt cttctgctcc 780
agctcctgta cctgatataa ctgcaacagt atccatatca gttcattcag aaaaatcaga 840
tggacctttt cgggattctg ataataaaat attgccagct gcagctcttg catcagagca 900
ctcaaaggga acctcctcaa ttgcaattac cgctcttatg gaagagaagg gttaccaggt 960
gcctgttctt ggaaccccat ctttgcttgg acagtcatta ttgcatggac agttgatccc 1020
cacaactggt ccagtaagaa taaatactgc tcgtccaggt ggtggtcgac caggctggga 1080
acattccaac aaacttggct atctggtttc tccaccacaa caaattagaa gaggggagag 1140
gagctgctac agaagtataa accgtgggcg acaccacagc gaaagatcac agaggactca 1200
aggcccgtca ctaccagcaa ctccagtctt tgtacctgtt ccaccacctc ctttgtatcc 1260
gcctcctccc catacacttc ctctccctcc gggtgttcct cctccacagt tttctcctca 1320
gtttcctcct ggccagccac cacccgctgg gtatagtgtc cctcctccag ggtttcctcc 1380
agctcctgcc aatttatcaa caccttgggt atcatcagga gtgcagacag ctcattcaaa 1440
taccatccca acaacacaag caccaccttt gtccagggaa gaattctata gagagcagcg 1500
acgactaaaa gaagaggaaa agaaaaagtc caagctagat gagtttacaa atgattttgc 1560
taaggaattg atggaataca aaaagattca aaaggagcgt aggcgctcat tttccaggtc 1620
taaatctccc tatagtggtt cttcgtattc aagaagttca tatacttatt ctaaatcaag 1680
atctgggtca acacgttcac gctcttattc tcgatcattc agccgctcac attctcgttc 1740
ctattcacgg tcacctccat accccagaag aggcagaggc aagagccgca attaccgttc 1800
acggtctaga tctcatggat atcatcgatc taggtcaagg tcaccccctt acagacgcta 1860
tcattcacga tcaagatctc ctcaagcgtt taggggacag tctcctaata aacgtaatgt 1920
acctcaaggg gaaacagaac gtgaatattt taatagatac agagaagttc caccaccata 1980
tgacatgaaa gcatattatg ggagaagtgt tgactttaga gacccatttg aaaaagaacg 2040
ctaccgagaa tgggagagaa aatatagaga gtggtatgaa aaatattata aaggttatgc 2100
tgctggagca cagcctagac cctcagcaaa tagagagaac ttttctccag agagattttt 2160
gccacttaac atcaggaatt ctcccttcac aagaggccgc agagaagact atgttggtgg 2220
gcaaagtcat agaagtcgaa acataggtag caactatcca gaaaagcttt cagcaagaga 2280
tggtcacaat cagaaggata atacaaagtc aaaagagaag gagagtgaaa acgctccagg 2340
agatggtaaa ggaaataagc ataagaaaca cagaaaaaga agaaaagggg aggaaagtga 2400
gggttttctg aacccagagt tattagagac ttctaggaaa tcaagagaac ctacaggtgt 2460
tgaagaaaat aaaacagact cattgtttgt tctcccaagt agagatgatg ccacacctgt 2520
tagagatgaa ccaatggatg cagaatcaat cacttttaaa tcagtgtctg aaaaagacaa 2580
gagagaaagg gataaaccaa aagcaaaggg tgataaaacc aaacggaaga atgatggatc 2640
tgctgtgtcc aaaaaagaaa atattgtaaa acctgctaaa ggaccccaag aaaaagtaga 2700
tggagacgtg agagatctcc tcgatctgaa cctccaatta aaaaagccaa agaggagact 2760
ccgaagactg acaatactaa atcatcatct tcctctcaga aggatgaaaa aatcactgga 2820
acccccagaa aagctcactc taaatcagca aaagacacca agaaacaaaa ccagtcaaag 2880
aggaaaaagt gaagaaggac tattccaaag atgtcaaatc agaaaagcta acaactaagg 2940
aagaaaaggc caagaagcct aatgagaaaa acaaaccact tgataataag ggag 2994

<210> 289

<211>  2527
<212>  DNA
<213>  Homo sapiens
<300>
<308>  Genbank/M30448
<309>  1994-11-01

<400>  289
tccgggcctg gcctgtacgg tcgccgccgt tccctggaag tagcaacttc cctaccccac      60
cccagtcctg gtccccgtcc agccgctgac gtgaagatga gcagctcaga ggaggtgtcc     120
tggatttcct ggttctgtgg gctccgtggc aatgaattct tctgtgaagt ggatgaagac     180
tacatccagg acaaatttaa tcttactgga ctcaatgagc aggtccctca ctaccgacaa     240
gctctagaca tgatcttgga cctggagcct gatgaagaac tggaagacaa ccccaaccag     300
agtgacctga ttgagcaggc agccgagatg ctttatggat tgatccacgc ccgctacatc     360
cttaccaacc gtggcatcgc ccagatgttg gaaaagtacc agcaaggaga ctttggttac     420
tgtcctcgtg tgtactgtga gaaccagcca atgcttccca ttggcctttc agacatccca     480
ggtgaagcca tggtgaagct ctactgcccc aagtgcatgg atgtgtacac acccaagtca     540
tcaagacacc atcacacgga tggcgcctac ttcggcactg gtttccctca catgctcttc     600
atggtgcatc ccgagtaccg gcccaagaga cctgccaacc agtttgtgcc caggctctac     660
ggtttcaaga tccatgcgat ggcctaccag ctgcagctcc aagccgccag caacttcaag     720
agcccagtca agacgattcg ctgattccct cccccacctg tcctgcagtc tttgactttt     780
cctttctttt ttgccaccct ttcaggaacc ctgtatggtt tttagtttaa attaaaggag     840
tcgttattgt ggtgggaata tgaaataaag tagaagaaaa ggccatgaaa aaaaaaaaaa     900
aaaaaaaaaa aaaggtttgt gttctcgacg ccttggtagt tggcataggc taaagaaaag     960
ggatctcagc cccgaggaag ggtcaccctc ctagagatag ctactacccc gtctcaggag    1020
accctggtat ttctagagca cgctttgctt tcaccaaacc caaggaggtg acaggaggag    1080
cccccgcaca ggacctaaga atgctgtgac cagaagatgg gatcgcggaa cagcagcagt    1140
gcaggatccg gtccggagac cctccgaggg gcttgccccg aagaggggct ggcctgcgtc    1200
ggagtgagga agaggaagaa gaggatgaag atgtggatct ggcccaggta ctggcctatc    1260
tcctccgcag aggccaagtg aggttggtgc agggaggagg tgcagcaaat ttacaattca    1320
ttcaggccct cttggactca gaggaagaga atgacagagc ttgggatggt cgtcttgggg    1380
atcgatacaa cccacctgtg gatgctaccc ctgacacccg ggagctggaa ttcctggagt    1440
tgtgaacgcc gcggactccg gagccgcaca aaccagggct cgccatgaag ccaggattca    1500
gtcccccgtg gggtggcttt ggcggccgaa gggggctttg gtgaccgtgg tggtcgtgga    1560
ggccgagggg gctttggcgg gggccgaggt cgaggcggag gctttagagg tcgtggacga    1620
ggaggaggtg gaggcggcgg cggcggtgga ggaggaggaa gaggtggagg cttccattct    1680
ggtggcaacc ggggtcgtgg tcggggagga aaaagaggaa accagtcggg gaagaatgtg    1740
atggtggagc cgcatcggac tgagggtgtc ttcatttgtc gaggaaagga gatgcactgg    1800
tcaccaagaa cctggtcctg ggaatcaagt tatggagaga agagagtctc gattcggaag    1860
gagatgacaa aattgagtac cgagcctgga accccttccg ctccaagcta gcagcagcaa    1920
tcctgggtgg tgtggaccag atccacatca aaccgggggc taaggttctc tacctcgggg    1980
ctgcctcggg caccacggtc tcccatgtct ctgacatcgt tggtccggat ggtctagtct    2040
atgcagtcga gtttccccac cgctctggcc gtgacctcat taacttggcc aagaagagga    2100
ccaacatcat tcctgtgatc gaggatgctc gacacccaca caaataccgc atgctcatcg    2160
caatggtgga tgtgatcttt gctgatgtgg cccagccaga ccagacccgg aatgtggccc    2220
tgaaagccca caccttcctg cgctaatgga ggacactttg tgatttccat taaggccaac    2280
tgcattgact ccacagccta gcggtcaggc cgtgtttgcc tccgaagtga aaaagatgca    2340
acaggagaac atgaagccgc aggagcagtt gacccttgag ccatatgaaa gagaccatgc    2400
cgtggtcgtg ggagtgtaca ggccaccccc caaggtgaag aactgaagtt cagcgctgtc    2460
aggattgcga gagatgtgtg ttgatactgt tgcccgtgtg tttttctaat taaaagactc    2520
ctccgtc                                                              2527

<210>  290
<211>  1818
<212>  DNA
<213>  Homo sapiens
<300>
<308>  Genbank/S73813
<309>  1995-04-12

<400>  290
accacaccaa gcagcggctg ggggggggaa agacgaggaa agaggaggaa aacaaaagct      60
gctacttatg gaagatacaa aggagtctaa cgtgaagaca ttttgctcca agaatatcct     120
agccatcctt ggcttctcct ctatcatagc tgtgatagct ttgcttgctg tggggttgac     180
ccagaacaaa gcattgccag aaaacgttaa gtatgggatt gtgctggatg cgggttcttc     240
tcacacaagt ttatacatct ataagtggcc agcagaaaag gagaatgaca caggcgtggt     300
gcatcaagta gaagaatgca gggttaaagg tcctggaatc tcaaaatttg ttcagaaagt     360
aaatgaaata ggcatttacc tgactgattg catggaaaga gctagggaag tgattccaag     420
gtcccagcac caagagacac ccgtttacct gggagccacg gcaggcatgc ggttgctcag     480
gatggaaagt gaagagttgg cagacagggt tctggatgtg gtggagagga gcctcagcaa     540
ctaccccttt gacttccagg gtgccaggat cattactggc caagaggaag gtgcctatgg     600
ctggattact atcaactatc tgctgggcaa attcagtcag aaaacaaggt ggttcagcat     660
agtcccatat gaaaccaata atcaggaaac ctttggagct ttggaccttg ggggagcctc     720

tacacaagtc acttttgtac cccaaaacca gactatcgag tccccagata atgctctgca 780
atttcgcctc tatggcaagg actacaatgt ctacacacat agcttcttgt gctatgggaa 840
ggatcaggca ctctggcaga aactggccaa ggacattcag gttgcaagta atgaaattct 900
cagggaccca tgctttcatc ctggatataa gaaggtagtg aacgtaagtg acctttacaa 960
gaccccctgc accaagagat ttgagatgac tcttccattc cagcagtttg aaatccaggg 1020
tattggaaac tatcaacaat gccatcaaag catcctggag ctcttcaaca ccagttactg 1080
cccttactcc cagtgtgcct tcaatgggat ttcttgccca ccactccagg gggattttgg 1140
ggcattttca gctttttact ttgtgatgaa gtttttaaac ttgacatcag agaaagtctc 1200
tcaggaaaag gtgactgaga tgatgaaaaa gttctgtgct cagccttggg aggagataaa 1260
aacatcttac gctggagtaa aggagaagta cctgagtgaa tactgctttt ctggtaccta 1320
cattctctcc ctccttctgc aaggctatca tttcacagct gattcctggg agcacatcca 1380
tttcattggc aagatccagg gcagcgacgc cggctggact ttgggctaca tgctgaacct 1440
gaccaacatg atcccagctg agcaaccatt gtccacacct ctctcccact ccacctatgt 1500
cttcctcatg gttctattct ccctggtcct tttcacagtg gccatcatag gcttgcttat 1560
ctttcacaag ccttcatatt tctggaaaga tatggtatag caaaagcagc tgaaatatgc 1620
tggctggagt gaggaaaaaa tcgtccaggg agcattttcc tccatcgcag tgttcaaggc 1680
catccttccc tgtctgccag ggccagtctt gacgagtgtg aagcttcctt ggcttttact 1740
gaagcctttc ttttggaggt attcaatatc ctttgcctca aggacttcgg cagatactgt 1800
ctctttcatg agtttttc 1818

<210> 291
<211> 1141
<212> PRT
<213> Homo sapiens
<220>
<221> misc_feature
<222> (640)..(640)
<223> x est un résidu inconnu

<220>
<221> misc_feature
<223> CDS de la séquence Genbank/XM_038751 ( seq ID 284)

<220>
<221> misc_feature
<222> (641)..(641)
<223> x est un résidu inconnu

<220>
<221> misc_feature
<222> (642)..(642)
<223> x est un résidu inconnu


<400> 291
Met Leu Lys Lys Tyr Ser His His Arg Asn Ile Ala Thr Tyr Tyr Gly
1               5                   10                  15
Ala Phe Ile Lys Lys Ser Pro Pro Gly His Asp Asp Gln Leu Trp Leu
            20                  25                  30
Val Met Glu Phe Cys Gly Ala Gly Ser Ile Thr Asp Leu Val Lys Asn
        35                  40                  45
Thr Lys Gly Asn Thr Leu Lys Glu Asp Trp Ile Ala Tyr Ile Ser Arg
    50                  55                  60
Glu Ile Leu Arg Gly Leu Ala His Leu His Ile His His Val Ile His
65                  70                  75                  80
Arg Asp Ile Lys Gly Gln Asn Val Leu Leu Thr Glu Asn Ala Glu Val
                85                  90                  95
Lys Leu Val Asp Phe Gly Val Ser Ala Gln Leu Asp Arg Thr Val Gly
            100                 105                 110
Arg Arg Asn Thr Phe Ile Gly Thr Pro Tyr Trp Met Ala Pro Glu Val
        115                 120                 125
Ile Ala Cys Asp Glu Asn Pro Asp Ala Thr Tyr Asp Tyr Arg Ser Asp
    130                 135                 140
Leu Trp Ser Cys Gly Ile Thr Ala Ile Glu Met Ala Glu Gly Ala Pro
145                 150                 155                 160
Pro Leu Cys Asp Met His Pro Met Arg Ala Leu Phe Leu Ile Pro Arg
                165                 170                 175
Asn Pro Pro Pro Arg Leu Lys Ser Lys Lys Trp Ser Lys Lys Phe Phe
            180                 185                 190
Ser Phe Ile Glu Gly Cys Leu Val Lys Asn Tyr Met Gln Arg Pro Ser
        195                 200                 205
Thr Glu Gln Leu Leu Lys His Pro Phe Ile Arg Asp Gln Pro Asn Glu
    210                 215                 220

Arg Gln Val Arg Ile Gln Leu Lys Asp His Ile Asp Arg Thr Arg Lys
225                 230                 235                 240
Lys Arg Gly Glu Lys Asp Glu Thr Glu Tyr Glu Tyr Ser Gly Ser Glu
                245                 250                 255
Glu Glu Glu Glu Glu Val Pro Glu Gln Glu Gly Glu Pro Ser Ser Ile
            260                 265                 270
Val Asn Val Pro Gly Glu Ser Thr Leu Arg Arg Asp Phe Leu Arg Leu
        275                 280                 285
Gln Gln Glu Asn Lys Glu Arg Ser Glu Ala Leu Arg Arg Gln Gln Leu
    290                 295                 300
Leu Gln Glu Gln Gln Leu Arg Glu Gln Glu Glu Tyr Lys Arg Gln Leu
305                 310                 315                 320
Leu Ala Glu Arg Gln Lys Arg Ile Glu Gln Gln Lys Glu Gln Arg Arg
                325                 330                 335
Arg Leu Glu Glu Gln Gln Arg Arg Glu Arg Glu Ala Arg Arg Gln Gln
            340                 345                 350
Glu Arg Glu Gln Arg Arg Arg Glu Gln Glu Glu Lys Arg Arg Leu Glu
        355                 360                 365
Glu Leu Glu Arg Arg Arg Lys Glu Glu Glu Glu Arg Arg Arg Ala Glu
    370                 375                 380
Glu Glu Lys Arg Arg Val Glu Arg Glu Gln Glu Tyr Ile Arg Arg Gln
385                 390                 395                 400
Leu Glu Glu Glu Gln Arg His Leu Glu Val Leu Gln Gln Gln Leu Leu
                405                 410                 415
Gln Glu Gln Ala Met Leu Leu His Asp His Arg Arg Pro His Pro Gln
            420                 425                 430
His Ser Gln Gln Pro Pro Pro Pro Gln Gln Glu Arg Ser Lys Pro Ser
        435                 440                 445
Phe His Ala Pro Glu Pro Lys Ala His Tyr Glu Pro Ala Asp Arg Ala
    450                 455                 460
Arg Glu Val Glu Asp Arg Phe Arg Lys Thr Asn His Ser Ser Pro Glu
465                 470                 475                 480
Ala Gln Ser Lys Gln Thr Gly Arg Val Leu Glu Pro Pro Val Pro Ser
                485                 490                 495
Arg Ser Glu Ser Phe Ser Asn Gly Asn Ser Glu Ser Val His Pro Ala
            500                 505                 510
Leu Gln Arg Pro Ala Glu Pro Gln Val Pro Val Arg Thr Thr Ser Arg
        515                 520                 525
Ser Pro Val Leu Ser Arg Arg Asp Ser Pro Leu Gln Gly Ser Gly Gln
    530                 535                 540
Gln Asn Ser Gln Ala Gly Gln Arg Asn Ser Thr Ser Ser Ile Glu Pro
545                 550                 555                 560
Arg Leu Leu Trp Glu Arg Val Glu Lys Leu Val Pro Arg Pro Gly Ser
                565                 570                 575
Gly Ser Ser Ser Gly Ser Ser Asn Ser Gly Ser Gln Pro Gly Ser His
            580                 585                 590
Pro Gly Ser Gln Ser Gly Ser Gly Glu Arg Phe Arg Val Arg Ser Ser
        595                 600                 605
Ser Lys Ser Glu Gly Ser Pro Ser Gln Arg Leu Glu Asn Ala Val Lys
    610                 615                 620
Lys Pro Glu Asp Lys Lys Glu Val Phe Arg Pro Leu Lys Pro Ala Xaa
625                 630                 635                 640
Xaa Xaa Asp Leu Thr Ala Leu Ala Lys Glu Leu Arg Ala Val Glu Asp
                645                 650                 655
Val Arg Pro Pro His Lys Val Thr Asp Tyr Ser Ser Ser Ser Glu Glu
            660                 665                 670
Ser Gly Thr Thr Asp Glu Glu Asp Asp Asp Val Glu Gln Glu Gly Ala
        675                 680                 685
Asp Glu Ser Thr Ser Gly Pro Glu Asp Thr Arg Ala Ala Ser Ser Leu
    690                 695                 700
Asn Leu Ser Asn Gly Glu Thr Glu Ser Val Lys Thr Met Ile Val His
705                 710                 715                 720
Asp Asp Val Glu Ser Glu Pro Ala Met Thr Pro Ser Lys Glu Gly Thr
                725                 730                 735
Leu Ile Val Arg Gln Thr Gln Ser Ala Ser Ser Thr Leu Gln Lys His
            740                 745                 750
Lys Ser Ser Ser Ser Phe Thr Pro Phe Ile Asp Pro Arg Leu Leu Gln
        755                 760                 765
Ile Ser Pro Ser Ser Gly Thr Thr Val Thr Ser Val Val Gly Phe Ser
    770                 775                 780
Cys Asp Gly Met Arg Pro Glu Ala Ile Arg Gln Asp Pro Thr Arg Lys
785                 790                 795                 800
Gly Ser Val Val Asn Val Asn Pro Thr Asn Thr Arg Pro Gln Ser Asp

                805                 810                 815
Thr Pro Glu Ile Arg Lys Tyr Lys Lys Arg Phe Asn Ser Glu Ile Leu
                820                 825                 830
Cys Ala Ala Leu Trp Gly Val Asn Leu Leu Val Gly Thr Glu Ser Gly
        835                 840                 845
Leu Met Leu Leu Asp Arg Ser Gly Gln Gly Lys Val Tyr Pro Leu Ile
    850                 855                 860
Asn Arg Arg Arg Phe Gln Gln Met Asp Val Leu Glu Gly Leu Asn Val
865                 870                 875                 880
Leu Val Thr Ile Ser Gly Lys Lys Asp Lys Leu Arg Val Tyr Tyr Leu
                885                 890                 895
Ser Trp Leu Arg Asn Lys Ile Leu His Asn Asp Pro Glu Val Glu Lys
            900                 905                 910
Lys Gln Gly Trp Thr Thr Val Gly Asp Leu Glu Gly Cys Val His Tyr
        915                 920                 925
Lys Val Val Lys Tyr Glu Arg Ile Lys Phe Leu Val Ile Ala Leu Lys
    930                 935                 940
Ser Ser Val Glu Val Tyr Ala Trp Ala Pro Lys Pro Tyr His Lys Phe
945                 950                 955                 960
Met Ala Phe Lys Ser Phe Gly Glu Leu Val His Lys Pro Leu Leu Val
                965                 970                 975
Asp Leu Thr Val Glu Glu Gly Gln Arg Leu Lys Val Ile Tyr Gly Ser
            980                 985                 990
Cys Ala Gly Phe His Ala Val Asp  Val Asp Ser Gly Ser  Val Tyr Asp
        995                 1000                1005
Ile Tyr  Leu Pro Thr His Ile  Gln Cys Ser Ile Lys  Pro His Ala
    1010                1015                1020
Ile Ile  Ile Leu Pro Asn Thr  Asp Gly Met Glu Leu  Leu Val Cys
    1025                1030                1035
Tyr Glu  Asp Glu Gly Val Tyr  Val Asn Thr Tyr Gly  Arg Ile Thr
    1040                1045                1050
Lys Asp  Val Val Leu Gln Trp  Gly Glu Met Pro Thr  Ser Val Ala
    1055                1060                1065
Tyr Ile  Arg Ser Asn Gln Thr  Met Gly Trp Gly Glu  Lys Ala Ile
    1070                1075                1080
Glu Ile  Arg Ser Val Glu Thr  Gly His Leu Asp Gly  Val Phe Met
    1085                1090                1095
His Lys  Arg Ala Gln Arg Leu  Lys Phe Leu Cys Glu  Arg Asn Asp
    1100                1105                1110
Lys Val  Phe Phe Ala Ser Val  Arg Ser Gly Gly Ser  Ser Gln Val
    1115                1120                1125
Tyr Phe  Met Thr Leu Gly Arg  Thr Ser Leu Leu Ser  Trp
    1130                1135                1140


<210>  292
<211>  403
<212>  PRT
<213>  Homo sapiens
<220>
<221>  misc_feature
<223>  CDS de la séquence Genbank/BC006483 ( seq ID 285)


<400>  292
Met Ser His Arg Lys Phe Ser Ala Pro Arg His Gly Ser Leu Gly Phe
1               5                   10                  15
Leu Pro Arg Lys Arg Ser Ser Arg His Arg Gly Lys Val Lys Ser Phe
            20                  25                  30
Pro Lys Asp Asp Pro Ser Lys Pro Val His Leu Thr Ala Phe Leu Gly
        35                  40                  45
Tyr Lys Ala Gly Met Thr His Ile Val Arg Glu Val Asp Arg Pro Gly
    50                  55                  60
Ser Lys Val Asn Lys Lys Glu Val Val Glu Ala Val Thr Ile Val Glu
65                  70                  75                  80
Thr Pro Pro Met Val Val Val Gly Ile Val Gly Tyr Val Glu Thr Pro
                85                  90                  95
Arg Gly Leu Arg Thr Phe Lys Thr Val Phe Ala Glu His Ile Ser Asp
            100                 105                 110
Glu Cys Lys Arg Arg Phe Tyr Lys Asn Trp His Lys Ser Lys Lys Lys
        115                 120                 125
Ala Phe Thr Lys Tyr Cys Lys Lys Trp Gln Asp Glu Asp Gly Lys Lys
    130                 135                 140

Gln Leu Glu Lys Asp Phe Ser Ser Met Lys Lys Tyr Cys Gln Val Ile
145                 150                 155                 160
Arg Val Ile Ala His Thr Gln Met Arg Leu Leu Pro Leu Arg Gln Lys
                165                 170                 175
Lys Ala His Leu Met Glu Ile Gln Val Asn Gly Gly Thr Val Ala Glu
            180                 185                 190
Lys Leu Asp Trp Ala Arg Glu Arg Leu Glu Gln Gln Val Pro Val Asn
        195                 200                 205
Gln Val Phe Gly Gln Asp Glu Met Ile Asp Val Ile Gly Val Thr Lys
    210                 215                 220
Gly Lys Gly Tyr Lys Gly Val Thr Ser Arg Trp His Thr Lys Lys Leu
225                 230                 235                 240
Pro Arg Lys Thr His Arg Gly Leu Arg Lys Val Ala Cys Ile Gly Ala
                245                 250                 255
Trp His Pro Ala Arg Val Ala Phe Ser Val Ala Arg Ala Gly Gln Lys
            260                 265                 270
Gly Tyr His His Arg Thr Glu Ile Asn Lys Lys Ile Tyr Lys Ile Gly
        275                 280                 285
Gln Gly Tyr Leu Ile Lys Asp Gly Lys Leu Ile Lys Asn Asn Ala Ser
    290                 295                 300
Thr Asp Tyr Asp Leu Ser Asp Lys Ser Ile Asn Pro Leu Gly Gly Phe
305                 310                 315                 320
Val His Tyr Gly Glu Val Thr Asn Asp Phe Val Met Leu Lys Gly Cys
                325                 330                 335
Val Val Gly Thr Lys Lys Arg Val Leu Thr Leu Arg Lys Ser Leu Leu
            340                 345                 350
Val Gln Thr Lys Arg Arg Ala Leu Glu Lys Ile Asp Leu Lys Phe Ile
        355                 360                 365
Asp Thr Thr Ser Lys Phe Gly His Gly Arg Phe Gln Thr Met Glu Glu
    370                 375                 380
Lys Lys Ala Phe Met Gly Pro Leu Lys Lys Asp Arg Ile Ala Lys Glu
385                 390                 395                 400
Glu Gly Ala

<210>  293
<211>  403
<212>  PRT
<213>  Homo sapiens
<220>
<221>  misc_feature
<223>  CDS de la séquence Genbank/BC012786 ( seq ID 286)

<400>  293
Met Ser His Arg Lys Phe Ser Ala Pro Arg His Gly Ser Leu Gly Phe
1               5                   10                  15
Leu Pro Arg Lys Arg Ser Ser Arg His Arg Gly Lys Val Lys Ser Phe
            20                  25                  30
Pro Lys Asp Asp Pro Ser Lys Pro Val His Leu Thr Ala Phe Leu Gly
        35                  40                  45
Tyr Lys Ala Gly Met Thr His Ile Val Arg Glu Val Asp Arg Pro Gly
    50                  55                  60
Ser Lys Val Asn Lys Lys Glu Val Val Glu Ala Val Thr Ile Val Glu
65                  70                  75                  80
Thr Pro Pro Met Val Val Val Gly Ile Val Gly Tyr Val Glu Thr Pro
                85                  90                  95
Arg Gly Leu Arg Thr Phe Lys Thr Val Phe Ala Glu His Ile Ser Asp
            100                 105                 110
Glu Cys Lys Arg Arg Phe Tyr Lys Asn Trp His Lys Ser Lys Lys Lys
        115                 120                 125
Ala Phe Thr Lys Tyr Cys Lys Lys Trp Gln Asp Glu Asp Gly Lys Lys
    130                 135                 140
Gln Leu Glu Lys Asp Phe Ser Ser Met Lys Lys Tyr Cys Gln Val Ile
145                 150                 155                 160
Arg Val Ile Ala His Thr Gln Met Arg Leu Leu Pro Leu Arg Gln Lys
                165                 170                 175
Lys Ala His Leu Met Glu Ile Gln Val Asn Gly Gly Thr Val Ala Glu
            180                 185                 190
Lys Leu Asp Trp Ala Arg Glu Arg Leu Glu Gln Gln Val Pro Val Asn
        195                 200                 205
Gln Val Phe Gly Gln Asp Glu Met Ile Asp Val Ile Gly Val Thr Lys
    210                 215                 220

Gly Lys Gly Tyr Lys Gly Val Thr Ser Arg Trp His Thr Lys Lys Leu
225                 230                 235                 240
Pro Arg Lys Thr His Arg Gly Leu Arg Lys Val Ala Cys Ile Gly Ala
                245                 250                 255
Trp His Pro Ala Arg Val Ala Phe Ser Val Ala Arg Ala Gly Gln Lys
            260                 265                 270
Gly Tyr His His Arg Thr Glu Ile Asn Lys Lys Ile Tyr Lys Ile Gly
        275                 280                 285
Gln Gly Tyr Leu Ile Lys Asp Gly Lys Leu Ile Lys Asn Asn Ala Ser
    290                 295                 300
Thr Asp Tyr Asp Leu Ser Asp Lys Ser Ile Asn Pro Leu Gly Gly Phe
305                 310                 315                 320
Val His Tyr Gly Glu Val Thr Asn Asp Phe Val Met Leu Lys Gly Cys
                325                 330                 335
Val Val Gly Thr Lys Lys Arg Val Leu Thr Leu Arg Lys Ser Leu Leu
            340                 345                 350
Val Gln Thr Lys Arg Arg Ala Leu Glu Lys Ile Asp Leu Lys Phe Ile
        355                 360                 365
Asp Thr Thr Ser Lys Phe Gly His Gly Arg Phe Gln Thr Met Glu Glu
    370                 375                 380
Lys Lys Ala Phe Met Gly Pro Leu Lys Lys Asp Arg Ile Ala Lys Glu
385                 390                 395                 400
Glu Gly Ala


<210>  294
<211>  975
<212>  PRT
<213>  Homo sapiens
<220>
<221>  misc_feature
<223>  CDS de la séquence Genbank/AF265230 ( seq ID 287)


<400>  294
Met Ser Gly Ile Gly Asn Lys Arg Ala Ala Gly Glu Pro Gly Thr Ser
1               5                   10                  15
Met Pro Pro Glu Lys Lys Ala Ala Val Glu Asp Ser Gly Thr Thr Val
            20                  25                  30
Glu Thr Ile Lys Leu Gly Gly Val Ser Ser Thr Glu Glu Leu Asp Ile
        35                  40                  45
Arg Thr Leu Gln Thr Lys Asn Arg Lys Leu Ala Glu Met Leu Asp Gln
    50                  55                  60
Arg Gln Ala Ile Glu Asp Glu Leu Arg Glu His Ile Glu Lys Leu Glu
65                  70                  75                  80
Arg Arg Gln Ala Thr Asp Asp Ala Ser Leu Leu Ile Val Asn Arg Tyr
                85                  90                  95
Trp Ser Gln Phe Asp Glu Asn Ile Arg Ile Ile Leu Lys Arg Tyr Asp
            100                 105                 110
Leu Glu Gln Gly Leu Gly Asp Leu Leu Thr Glu Arg Lys Ala Leu Val
        115                 120                 125
Val Pro Glu Pro Glu Pro Asp Ser Asp Ser Asn Gln Glu Arg Lys Asp
    130                 135                 140
Asp Arg Glu Arg Gly Glu Gly Gln Glu Pro Ala Phe Ser Phe Leu Ala
145                 150                 155                 160
Thr Leu Ala Ser Ser Ser Ser Glu Glu Met Glu Ser Gln Leu Gln Glu
                165                 170                 175
Arg Val Glu Ser Ser Arg Arg Ala Val Ser Gln Ile Val Thr Val Tyr
            180                 185                 190
Asp Lys Leu Gln Glu Lys Val Glu Leu Leu Ser Arg Lys Leu Asn Ser
        195                 200                 205
Gly Asp Asn Leu Ile Val Glu Glu Ala Val Gln Glu Leu Asn Ser Phe
    210                 215                 220
Leu Ala Gln Glu Asn Met Arg Leu Gln Glu Leu Thr Asp Leu Leu Gln
225                 230                 235                 240
Glu Lys His Arg Thr Met Ser Gln Glu Phe Ser Lys Leu Gln Ser Lys
                245                 250                 255
Val Glu Thr Ala Glu Ser Arg Val Ser Val Leu Glu Ser Met Ile Asp
            260                 265                 270
Asp Leu Gln Trp Asp Ile Asp Lys Ile Arg Lys Arg Glu Gln Arg Leu
        275                 280                 285
Asp Arg His Leu Ala Glu Val Leu Glu Arg Val Asn Ser Lys Gly Tyr
    290                 295                 300

Lys Val Tyr Gly Ala Gly Ser Ser Leu Tyr Gly Gly Thr Ile Thr Ile
305                 310                 315                 320
Asn Ala Arg Lys Phe Glu Glu Met Asn Ala Glu Leu Glu Glu Asn Lys
                325                 330                 335
Glu Leu Ala Gln Asn Arg Leu Cys Glu Leu Glu Lys Leu Arg Gln Asp
            340                 345                 350
Phe Glu Glu Val Thr Thr Gln Asn Glu Lys Leu Lys Val Glu Leu Arg
        355                 360                 365
Ser Ala Val Glu Gln Val Val Lys Glu Thr Pro Glu Tyr Arg Cys Met
    370                 375                 380
Gln Ser Gln Phe Ser Val Leu Tyr Asn Glu Ser Leu Gln Leu Lys Ala
385                 390                 395                 400
His Leu Asp Glu Ala Arg Thr Leu Leu His Gly Thr Arg Gly Thr His
                405                 410                 415
Gln His Gln Val Glu Leu Ile Glu Arg Asp Glu Val Ser Leu His Lys
            420                 425                 430
Lys Leu Arg Thr Glu Val Ile Gln Leu Glu Asp Thr Leu Ala Gln Val
        435                 440                 445
Arg Lys Glu Tyr Glu Met Leu Arg Ile Glu Phe Glu Gln Thr Leu Ala
    450                 455                 460
Ala Asn Glu Gln Ala Gly Pro Ile Asn Arg Glu Met Arg His Leu Ile
465                 470                 475                 480
Ser Ser Leu Gln Asn His Asn His Gln Leu Lys Gly Glu Val Leu Arg
                485                 490                 495
Tyr Lys Arg Lys Leu Arg Glu Ala Gln Ser Asp Leu Asn Lys Thr Arg
            500                 505                 510
Leu Arg Ser Gly Ser Ala Leu Leu Gln Ser Gln Ser Ser Thr Glu Asp
        515                 520                 525
Pro Lys Asp Glu Pro Ala Glu Leu Lys Pro Asp Ser Glu Asp Leu Ser
    530                 535                 540
Ser Gln Ser Ser Ala Ser Lys Ala Ser Gln Glu Asp Ala Asn Glu Ile
545                 550                 555                 560
Lys Ser Lys Arg Asp Glu Glu Glu Arg Glu Arg Glu Arg Arg Glu Lys
                565                 570                 575
Glu Arg Glu Arg Glu Arg Glu Arg Glu Lys Glu Lys Glu Arg Glu Arg
            580                 585                 590
Glu Lys Gln Lys Leu Lys Glu Ser Glu Lys Glu Arg Asp Ser Ala Lys
        595                 600                 605
Asp Lys Glu Lys Gly Lys His Asp Asp Gly Arg Lys Lys Glu Ala Glu
    610                 615                 620
Ile Ile Lys Gln Leu Lys Ile Glu Leu Lys Lys Ala Gln Glu Ser Gln
625                 630                 635                 640
Lys Glu Met Lys Leu Leu Leu Asp Met Tyr Arg Ser Ala Pro Lys Glu
                645                 650                 655
Gln Arg Asp Lys Val Gln Leu Met Ala Ala Glu Lys Lys Ser Lys Ala
            660                 665                 670
Glu Leu Glu Asp Leu Arg Gln Arg Leu Lys Asp Leu Glu Asp Lys Glu
        675                 680                 685
Lys Lys Glu Asn Thr Lys Met Ala Asp Glu Asp Ala Leu Arg Lys Ile
    690                 695                 700
Arg Ala Val Glu Glu Gln Ile Glu Tyr Leu Gln Lys Lys Leu Ala Met
705                 710                 715                 720
Ala Lys Gln Glu Glu Glu Ala Leu Leu Ser Glu Met Asp Val Thr Gly
                725                 730                 735
Gln Ala Phe Glu Asp Met Gln Glu Gln Asn Ile Arg Leu Met Gln Gln
            740                 745                 750
Leu Arg Glu Lys Asp Asp Ala Asn Phe Lys Leu Met Ser Glu Arg Ile
        755                 760                 765
Lys Ser Asn Gln Ile His Lys Leu Leu Lys Glu Glu Lys Glu Glu Leu
    770                 775                 780
Ala Asp Gln Val Leu Thr Leu Lys Thr Gln Val Asp Ala Gln Leu Gln
785                 790                 795                 800
Val Val Arg Lys Leu Glu Glu Lys Glu His Leu Leu Gln Ser Asn Ile
                805                 810                 815
Gly Thr Gly Glu Lys Glu Leu Gly Leu Arg Thr Gln Ala Leu Glu Met
            820                 825                 830
Asn Lys Arg Lys Ala Met Glu Ala Ala Gln Leu Ala Asp Asp Leu Lys
        835                 840                 845
Ala Gln Leu Glu Leu Ala Gln Lys Lys Leu His Asp Phe Gln Asp Glu
    850                 855                 860
Ile Val Glu Asn Ser Val Thr Lys Glu Lys Asp Met Phe Asn Phe Lys
865                 870                 875                 880
Arg Ala Gln Glu Asp Ile Ser Arg Leu Arg Arg Lys Leu Glu Thr Thr

885                 890                 895
Lys Lys Pro Asp Asn Val Pro Lys Cys Asp Glu Ile Leu Met Glu Glu
            900                 905                 910
Ile Lys Asp Tyr Lys Ala Arg Leu Thr Cys Pro Cys Cys Asn Met Arg
        915                 920                 925
Lys Lys Asp Ala Val Leu Thr Lys Cys Phe His Val Phe Cys Phe Glu
    930                 935                 940
Cys Val Lys Thr Arg Tyr Asp Thr Arg Gln Arg Lys Cys Pro Lys Cys
945                 950                 955                 960
Asn Ala Ala Phe Gly Ala Asn Asp Phe His Arg Ile Tyr Ile Gly
                965                 970                 975

<210>  295
<211>  948
<212>  PRT
<213>  Homo sapiens
<220>
<221>  misc_feature
<223>  CDS de la séquence Genbank/NM_006910 ( seq ID 288)

<400>  295
   Met Gly Ile Lys Thr Leu Asn Leu Val Leu Gly Leu Lys Arg Ala Leu
   1               5                   10                  15
   Glu Phe Pro Glu Val Phe Met Met Glu Val Lys Asp Pro Asn Met Lys
               20                  25                  30
   Gly Ala Met Leu Thr Asn Thr Gly Lys Tyr Ala Ile Pro Thr Ile Asp
           35                  40                  45
   Ala Glu Ala Tyr Ala Ile Gly Lys Lys Glu Lys Pro Pro Phe Leu Pro
       50                  55                  60
   Glu Glu Pro Ser Ser Ser Ser Glu Glu Asp Asp Pro Ile Pro Asp Glu
   65                  70                  75                  80
   Leu Leu Cys Leu Ile Cys Lys Asp Ile Met Thr Asp Ala Val Val Ile
                   85                  90                  95
   Pro Cys Cys Gly Asn Ser Tyr Cys Asp Glu Cys Ile Arg Thr Ala Leu
               100                 105                 110
   Leu Glu Ser Asp Glu His Thr Cys Pro Thr Cys His Gln Asn Asp Val
           115                 120                 125
   Ser Pro Asp Ala Leu Ile Ala Asn Lys Phe Leu Arg Gln Ala Val Asn
       130                 135                 140
   Asn Phe Lys Asn Glu Thr Gly Tyr Thr Lys Arg Leu Arg Lys Gln Leu
   145                 150                 155                 160
   Pro Pro Pro Pro Pro Pro Ile Pro Pro Pro Arg Pro Leu Ile Gln Arg
                   165                 170                 175
   Asn Leu Gln Pro Leu Met Arg Ser Pro Ile Ser Arg Gln Gln Asp Pro
               180                 185                 190
   Leu Met Ile Pro Val Thr Ser Ser Ser Thr His Pro Ala Pro Ser Ile
           195                 200                 205
   Ser Ser Leu Thr Ser Asn Gln Ser Ser Leu Ala Pro Pro Val Ser Gly
       210                 215                 220
   Asn Pro Ser Ser Ala Pro Ala Pro Val Pro Asp Ile Thr Ala Thr Val
   225                 230                 235                 240
   Ser Ile Ser Val His Ser Glu Lys Ser Asp Gly Pro Phe Arg Asp Ser
                   245                 250                 255
   Asp Asn Lys Ile Leu Pro Ala Ala Ala Leu Ala Ser Glu His Ser Lys
               260                 265                 270
   Gly Thr Ser Ser Ile Ala Ile Thr Ala Leu Met Glu Glu Lys Gly Tyr
           275                 280                 285
   Gln Val Pro Val Leu Gly Thr Pro Ser Leu Leu Gly Gln Ser Leu Leu
       290                 295                 300
   His Gly Gln Leu Ile Pro Thr Thr Gly Pro Val Arg Ile Asn Thr Ala
   305                 310                 315                 320
   Arg Pro Gly Gly Gly Arg Pro Gly Trp Glu His Ser Asn Lys Leu Gly
                   325                 330                 335
   Tyr Leu Val Ser Pro Pro Gln Gln Ile Arg Arg Gly Glu Arg Ser Cys
               340                 345                 350
   Tyr Arg Ser Ile Asn Arg Gly Arg His His Ser Glu Arg Ser Gln Arg
           355                 360                 365
   Thr Gln Gly Pro Ser Leu Pro Ala Thr Pro Val Phe Val Pro Val Pro
       370                 375                 380
   Pro Pro Pro Leu Tyr Pro Pro Pro Pro His Thr Leu Pro Leu Pro Pro
   385                 390                 395                 400
   Gly Val Pro Pro Pro Gln Phe Ser Pro Gln Phe Pro Pro Gly Gln Pro
                   405                 410                 415
   Pro Pro Ala Gly Tyr Ser Val Pro Pro Pro Gly Phe Pro Pro Ala Pro
               420                 425                 430
   Ala Asn Leu Ser Thr Pro Trp Val Ser Ser Gly Val Gln Thr Ala His
           435                 440                 445
   Ser Asn Thr Ile Pro Thr Thr Gln Ala Pro Pro Leu Ser Arg Glu Glu
       450                 455                 460
   Phe Tyr Arg Glu Gln Arg Arg Leu Lys Glu Glu Glu Lys Lys Lys Ser
   465                 470                 475                 480
   Lys Leu Asp Glu Phe Thr Asn Asp Phe Ala Lys Glu Leu Met Glu Tyr
                   485                 490                 495
   Lys Lys Ile Gln Lys Glu Arg Arg Arg Ser Phe Ser Arg Ser Lys Ser
               500                 505                 510
   Pro Tyr Ser Gly Ser Ser Tyr Ser Arg Ser Ser Tyr Thr Tyr Ser Lys
           515                 520                 525
   Ser Arg Ser Gly Ser Thr Arg Ser Arg Ser Tyr Ser Arg Ser Phe Ser
       530                 535                 540
   Arg Ser His Ser Arg Ser Tyr Ser Arg Ser Pro Pro Tyr Pro Arg Arg
   545                 550                 555                 560
   Gly Arg Gly Lys Ser Arg Asn Tyr Arg Ser Arg Ser Arg Ser His Gly
                   565                 570                 575
   Tyr His Arg Ser Arg Ser Arg Ser Pro Pro Tyr Arg Arg Tyr His Ser
               580                 585                 590
   Arg Ser Arg Ser Pro Gln Ala Phe Arg Gly Gln Ser Pro Asn Lys Arg
           595                 600                 605
   Asn Val Pro Gln Gly Glu Thr Glu Arg Glu Tyr Phe Asn Arg Tyr Arg
       610                 615                 620
   Glu Val Pro Pro Pro Tyr Asp Met Lys Ala Tyr Tyr Gly Arg Ser Val
   625                 630                 635                 640
   Asp Phe Arg Asp Pro Phe Glu Lys Glu Arg Tyr Arg Glu Trp Glu Arg
                   645                 650                 655
   Lys Tyr Arg Glu Trp Tyr Glu Lys Tyr Tyr Lys Gly Tyr Ala Ala Gly
               660                 665                 670
   Ala Gln Pro Arg Pro Ser Ala Asn Arg Glu Asn Phe Ser Pro Glu Arg
           675                 680                 685
   Phe Leu Pro Leu Asn Ile Arg Asn Ser Pro Phe Thr Arg Gly Arg Arg
       690                 695                 700
   Glu Asp Tyr Val Gly Gly Gln Ser His Arg Ser Arg Asn Ile Gly Ser
   705                 710                 715                 720
   Asn Tyr Pro Glu Lys Leu Ser Ala Arg Asp Gly His Asn Gln Lys Asp
                   725                 730                 735
   Asn Thr Lys Ser Lys Glu Lys Glu Ser Glu Asn Ala Pro Gly Asp Gly
               740                 745                 750
   Lys Gly Asn Lys His Lys Lys His Arg Lys Arg Arg Lys Gly Glu Glu
           755                 760                 765
   Ser Glu Gly Phe Leu Asn Pro Glu Leu Leu Glu Thr Ser Arg Lys Ser
       770                 775                 780
   Arg Glu Pro Thr Gly Val Glu Glu Asn Lys Thr Asp Ser Leu Phe Val
   785                 790                 795                 800
   Leu Pro Ser Arg Asp Asp Ala Thr Pro Val Arg Asp Glu Pro Met Asp
                   805                 810                 815
   Ala Glu Ser Ile Thr Phe Lys Ser Val Ser Glu Lys Asp Lys Arg Glu
               820                 825                 830
   Arg Asp Lys Pro Lys Ala Lys Gly Asp Lys Thr Lys Arg Lys Asn Asp
           835                 840                 845
   Gly Ser Ala Val Ser Lys Lys Glu Asn Ile Val Lys Pro Ala Lys Gly
       850                 855                 860
   Pro Gln Glu Lys Val Asp Gly Asp Val Arg Asp Leu Leu Asp Leu Asn
   865                 870                 875                 880
   Leu Gln Leu Lys Lys Pro Lys Arg Arg Leu Arg Arg Leu Thr Ile Leu
                   885                 890                 895
   Asn His His Leu Pro Leu Arg Arg Met Lys Lys Ser Leu Glu Pro Pro
               900                 905                 910
   Glu Lys Leu Thr Leu Asn Gln Gln Lys Thr Pro Arg Asn Lys Thr Ser
           915                 920                 925
   Gln Arg Gly Lys Ser Glu Glu Gly Leu Phe Gln Arg Cys Gln Ile Arg
       930                 935                 940
   Lys Ala Asn Asn
   945

<210>  296

<211>  215
<212>  PRT
<213>  Homo sapiens
<220>
<221>  misc_feature
<223>  CDS de la séquence Genbank/M30448 ( seq ID 289)


<400>  296
Met Ser Ser Ser Glu Glu Val Ser Trp Ile Ser Trp Phe Cys Gly Leu
1               5                   10                  15
Arg Gly Asn Glu Phe Phe Cys Glu Val Asp Glu Asp Tyr Ile Gln Asp
            20                  25                  30
Lys Phe Asn Leu Thr Gly Leu Asn Glu Gln Val Pro His Tyr Arg Gln
        35                  40                  45
Ala Leu Asp Met Ile Leu Asp Leu Glu Pro Asp Glu Glu Leu Glu Asp
    50                  55                  60
Asn Pro Asn Gln Ser Asp Leu Ile Glu Gln Ala Ala Glu Met Leu Tyr
65                  70                  75                  80
Gly Leu Ile His Ala Arg Tyr Ile Leu Thr Asn Arg Gly Ile Ala Gln
                85                  90                  95
Met Leu Glu Lys Tyr Gln Gln Gly Asp Phe Gly Tyr Cys Pro Arg Val
            100                 105                 110
Tyr Cys Glu Asn Gln Pro Met Leu Pro Ile Gly Leu Ser Asp Ile Pro
        115                 120                 125
Gly Glu Ala Met Val Lys Leu Tyr Cys Pro Lys Cys Met Asp Val Tyr
    130                 135                 140
Thr Pro Lys Ser Ser Arg His His His Thr Asp Gly Ala Tyr Phe Gly
145                 150                 155                 160
Thr Gly Phe Pro His Met Leu Phe Met Val His Pro Glu Tyr Arg Pro
                165                 170                 175
Lys Arg Pro Ala Asn Gln Phe Val Pro Arg Leu Tyr Gly Phe Lys Ile
            180                 185                 190
His Ala Met Ala Tyr Gln Leu Gln Leu Gln Ala Ala Ser Asn Phe Lys
        195                 200                 205
Ser Pro Val Lys Thr Ile Arg
    210                 215


<210>  297
<211>  511
<212>  PRT
<213>  Homo sapiens
<220>
<221>  misc_feature
<223>  CDS de la séquence Genbank/S73813 ( seq ID 290)


<400>  297
Asp Met Glu Asp Thr Lys Glu Ser Asn Val Lys Thr Phe Cys Ser Lys
1               5                   10                  15
Asn Ile Leu Ala Ile Leu Gly Phe Ser Ser Ile Ile Ala Val Ile Ala
            20                  25                  30
Leu Leu Ala Val Gly Leu Thr Gln Asn Lys Ala Leu Pro Glu Asn Val
        35                  40                  45
Lys Tyr Gly Ile Val Leu Asp Ala Gly Ser Ser His Thr Ser Leu Tyr
    50                  55                  60
Ile Tyr Lys Trp Pro Ala Glu Lys Glu Asn Asp Thr Gly Val Val His
65                  70                  75                  80
Gln Val Glu Glu Cys Arg Val Lys Gly Pro Gly Ile Ser Lys Phe Val
                85                  90                  95
Gln Lys Val Asn Glu Ile Gly Ile Tyr Leu Thr Asp Cys Met Glu Arg
            100                 105                 110
Ala Arg Glu Val Ile Pro Arg Ser Gln His Gln Glu Thr Pro Val Tyr
        115                 120                 125
Leu Gly Ala Thr Ala Gly Met Arg Leu Leu Arg Met Glu Ser Glu Glu
    130                 135                 140
Leu Ala Asp Arg Val Leu Asp Val Val Glu Arg Ser Leu Ser Asn Tyr
145                 150                 155                 160
Pro Phe Asp Phe Gln Gly Ala Arg Ile Ile Thr Gly Gln Glu Glu Gly
                165                 170                 175
Ala Tyr Gly Trp Ile Thr Ile Asn Tyr Leu Leu Gly Lys Phe Ser Gln
            180                 185                 190

Lys Thr Arg Trp Phe Ser Ile Val Pro Tyr Glu Thr Asn Asn Gln Glu
        195                 200                 205
Thr Phe Gly Ala Leu Asp Leu Gly Gly Ala Ser Thr Gln Val Thr Phe
    210                 215                 220
Val Pro Gln Asn Gln Thr Ile Glu Ser Pro Asp Asn Ala Leu Gln Phe
225                 230                 235                 240
Arg Leu Tyr Gly Lys Asp Tyr Asn Val Tyr Thr His Ser Phe Leu Cys
                245                 250                 255
Tyr Gly Lys Asp Gln Ala Leu Trp Gln Lys Leu Ala Lys Asp Ile Gln
            260                 265                 270
Val Ala Ser Asn Glu Ile Leu Arg Asp Pro Cys Phe His Pro Gly Tyr
        275                 280                 285
Lys Lys Val Val Asn Val Ser Asp Leu Tyr Lys Thr Pro Cys Thr Lys
    290                 295                 300
Arg Phe Glu Met Thr Leu Pro Phe Gln Gln Phe Glu Ile Gln Gly Ile
305                 310                 315                 320
Gly Asn Tyr Gln Gln Cys His Gln Ser Ile Leu Glu Leu Phe Asn Thr
                325                 330                 335
Ser Tyr Cys Pro Tyr Ser Gln Cys Ala Phe Asn Gly Ile Phe Leu Pro
            340                 345                 350
Pro Leu Gln Gly Asp Phe Gly Ala Phe Ser Ala Phe Tyr Phe Val Met
        355                 360                 365
Lys Phe Leu Asn Leu Thr Ser Glu Lys Val Ser Gln Glu Lys Val Thr
    370                 375                 380
Glu Met Met Lys Lys Phe Cys Ala Gln Pro Trp Glu Glu Ile Lys Thr
385                 390                 395                 400
Ser Tyr Ala Gly Val Lys Glu Lys Tyr Leu Ser Glu Tyr Cys Phe Ser
                405                 410                 415
Gly Thr Tyr Ile Leu Ser Leu Leu Leu Gln Gly Tyr His Phe Thr Ala
            420                 425                 430
Asp Ser Trp Glu His Ile His Phe Ile Gly Lys Ile Gln Gly Ser Asp
        435                 440                 445
Ala Gly Trp Thr Leu Gly Tyr Met Leu Asn Leu Thr Asn Met Ile Pro
    450                 455                 460
Ala Glu Gln Pro Leu Ser Thr Pro Leu Ser His Ser Thr Tyr Val Phe
465                 470                 475                 480
Leu Met Val Leu Phe Ser Leu Val Leu Phe Thr Val Ala Ile Ile Gly
                485                 490                 495
Leu Leu Ile Phe His Lys Pro Ser Tyr Phe Trp Lys Asp Met Val
            500                 505                 510

<210>  298
<211>  29
<212>  DNA
<213>  Homo sapiens
<220>
<221>  misc_feature
<223>  amorce directe : GV45-1

<400>  298
cgggtcgacc tggcagaaac tggccaagg                                    29

<210>  299
<211>  30
<212>  DNA
<213>  Homo sapiens
<220>
<221>  misc_feature
<223>  amorce indirecte : GV45-2

<400>  299
cgcacgcgtg caagcctatg atggccactg                                   30

<210>  300
<211>  27
<212>  DNA
<213>  Homo sapiens
<220>
<221>  misc_feature
<223>  amorce directe : GS-PGM-F

<400>  300
cgcacgcgtg gccgcgggaa ttcgatt                                          27

<210>  301
<211>  27
<212>  DNA
<213>  Homo sapiens
<220>
<221>  misc_feature
<223>  amorce indirecte : GS-PGS-R

<400>  301
cgggtcgacg cggccgcgaa ttcacta                                          27

## Description

La présente invention appartient au domaine des compositions pharmaceutiques utiles pour le traitement de pathologies résultant d'une dérégulation du mécanisme de l'angiogénèse.

Elle concerne des compositions comprenant d'une part des séquences de nouveaux gènes, dont la fonction n'était pas identifiée à ce jour et dont l'implication dans le mécanisme de l'angiogenese a été mis en évidence pour la première fois par la demanderesse et d'autre part des séquences de gènes, dont au moins une de leurs fonctions a été préalablement identifiée, mais dont leur implication, en tant que gènes constitutifs des cellules endothéliales, dans le mécanisme de l'angiogenese a été mise en évidence pour la première fois lors des travaux réalisés par la demanderesse dans le cadre de la présente invention. Ces gènes sont identifiés par leurs séquences nucléotidiques dans le listage de séquences en annexe. La présente invention concerne également les séquences polypeptidiques des facteurs codés par lesdits gènes qui trouvent leur application dans l'étude clinique du processus de l'angiogénèse, le pronostic, le diagnostic et le traitement de pathologies liées à ce processus ainsi que dans la mise en oeuvre des essais pharmacologiques, pharmacogénomiques, ou pharmacosignalitiques.

L'angiogenèse est un processus fondamental par lequel de nouveaux vaisseaux sanguins se forment. Ce processus est essentiel dans plusieurs phénomènes physiologiques normaux tels que la reproduction, le développement ou encore la cicatrisation. Dans ces phénomènes biologiques normaux, l'angiogenèse est sous contrôle strict, c'est-à-dire qu'elle est déclenchée pendant une période courte, quelques jours, puis complètement inhibée. Cependant, plusieurs pathologies sont liées à une angiogenèse invasive et incontrôlée. L'arthrite, par exemple, est une pathologie due à l'endommagement des cartilages par les néovaisseaux invasifs. Dans la rétinopathie diabétique, l'invasion de la rétine par les néovaisseaux conduit à l'aveuglement des malades; la néovascularisation de l'appareil oculaire présente la cause majeure de l'aveuglement et cette néovascularisation domine une vingtaine de maladies de l'oeil. Enfin, la croissance et la métastase des tumeurs sont directement liées à la néovascularisation et sont dépendantes de l'angiogenèse. La tumeur stimule la croissance des néovaisseaux pour sa croissance elle-même. De plus, ces néovaisseaux présentent les voies d'échappement des tumeurs pour joindre la circulation sanguine et provoquer les métastases dans des sites à distance comme le foie, le poumon ou l'os.

Dans d'autres pathologies telles que les maladies cardio-vasculaires, les maladies d'artères périphériques, les lésions vasculaires ou cérébrales, l'angiogenèse peut présenter une base thérapeutique importante. En effet, la promotion de l'angiogenèse dans les endroits endommagés peut conduire à la formation de néovaisseaux sanguins latéraux et alternatifs aux vaisseaux endommagés fournissant ainsi le sang et par conséquence l'oxygène et d'autres facteurs nutritifs et biologiques nécessaires aux survies des tissus concernés.

La formation de néovaisseaux par les cellules endothéliales implique la migration, la croissance, et la différentiation des cellules endothéliales. La régulation de ces phénomènes biologiques est directement liée à l'expression génétique. En matière d'angiogénèse, un nombre sans cesse grandissant d'études montre que la régulation de l'angiogenèse se fait à travers un équilibre entre des facteurs agissant directement sur la cellule endothéliale. Ces facteurs peuvent être stimulants, d'une part, tels que, entre autres, le VEGF, le FGFs, l'IL-8, le HGF/SF, le PDGF. Ils peuvent aussi être inhibants, comme, entre autres, l'IL-10, l'IL-12, le gro-α et β, le facteur plaquettaire 4, l'angiostatine, l'inhibiteur dérivé de chondrocyte humaine, la thrombospondine, le facteur inhibiteur de la leucémie.(Jensen, Surg. Neural., 1998, 49, 189-195 ; Tamatani et al., Carcinogenesis, 1999, 20, 957-962 ; Tanaka et al., Cancer Res., 1998, 58, 3362-3369 ; Ghe et al., Cancer Res., 1997, 57, 3733-3740 ; Kawahara et al., Hepatology, 1998, 28, 1512-1517 ; Chandhuni et al., Cancer Res., 1997, 57, 1814-1819 ; Jendraschak et Sage, Semin. Cancer Biol., 1996, 7, 139-146 ; Majewski et al., J. Invest. Dermatol., 1996, 106, 1114-1119).

Le contrôle de l'angiogénèse représente donc un axe stratégique, à la fois de recherche fondamentale, afin d'améliorer notre compréhension des nombreux phénomènes pathologiques liés à l'angiogénèse, mais aussi une base pour l'élaboration des nouvelles thérapies destinées à traiter les pathologies liées à l'angiogénèse.

Afin de contrôler l'angiogenèse, plusieurs groupes pharmaceutiques ont développé des stratégies thérapeutiques basées directement sur l'utilisation de signaux paracrines, les facteurs stimulateurs et inhibiteurs, comme agents pour promouvoir ou inhiber l'angiogenèse. Ces stratégies sont basées essentiellement sur l'utilisation de ces facteurs sous leur forme polypeptidique comme agents stimulateurs ou inhibiteurs de l'angiogenèse, ou encore plus récemment sous la forme de vecteurs d'expression codant pour les facteurs choisis.

Un procédé permettant l'identification de nouveaux gènes impliqués dans la régulation de l'angiogenèse a été mis au point. Il a fait l'objet d'une demande de brevet français publiée sous le n° FR 2798674 et d'une demande de brevet internationale PCT publiée sous le n° WO 01/218312. Cette méthode a la particularité de traduire fidèlement le mécanisme intime régulant l'angiogenèse en prenant en compte tous les facteurs extracellulaires décrits comme agents régulateurs de l'angiogenèse, c'est-à-dire les facteurs angiogéniques, les facteurs angiostatiques, ainsi que les différents composants de la matrice extracellulaire. Cette méthodologie consiste à mettre en oeuvre ces différents facteurs extracellulaires à travers quatre conditions expérimentales bien définies. Les cellules endothéliales sont cultivées sur une composante et/ou un mélange bien défini de plusieurs composantes de la matrice extracellulaire et placées sous les quatre conditions expérimentales à savoir :

Une condition contrôle où les cellules endothéliales ne sont pas stimulées.

Une condition angiogénique où les cellules endothéliales sont stimulées par un ou plusieurs facteurs angiogéniques.

Une condition d'inhibition de l'angiogenèse où les cellules endothéliales sont stimulées par un ou plusieurs facteurs angiogéniques et mises en présence d'un ou plusieurs facteurs angiostatiques.

Une autre condition de contrôle où les cellules endothéliales sont stimulées par un ou plusieurs facteurs angiostatiques.

Ces quatre conditions permettent d'obtenir des préparations d'ARNm spécifiques de l'angiogenèse à savoir de l'état angiogénique et/ou l'inhibition de l'angiogenèse et permettent de déceler les gènes codant pour les constituants cellulaires impliqués dans la régulation de l'angiogénèse, incluant des régulateurs positifs et des régulateurs négatifs. Donc, le procédé ci-dessus décrit permet le criblage systématique de tous les facteurs angiogéniques et angiostatiques ainsi que des différentes composantes de la matrice extracellulaire dans le but de mettre en évidence et identifier les gènes codant pour les constituants cellulaires impliqués dans la régulation de l'angiogenèse. De plus, étant donné que l'expression génique peut être analysée tout au long de la cinétique de la formation des néovaisseaux par les cellules endothéliales, cette approche constitue une méthodologie *in vitro* permettant de relier l'expression génique aux paramètres fonctionnels biologiques de l'angiogenèse.

L'identification des cinquante-quatre gènes rapportés ci-dessous a été effectuée selon la méthodologie décrite ci-dessus, en utilisant les facteurs angiogéniques et angiostatiques, ainsi que le collagène type I comme composant de la matrice extracellulaire pour reproduire les quatre conditions expérimentales.

La Demanderesse a prouvé l'implication de ces cinquante-quatre nouveaux gènes identifiés par les séquences SEQ ID No. : 1 à SEQ ID No. : 53 et SEQ ID No. 225 dans la liste de séquences en annexe, dans le mécanisme de régulation de l'angiogènese.

Plus particulièrement l'invention concerne une composition pharmaceutique active sur les phénomènes d'angiogenèse comprenant à titre d'agent actif au moins une substance choisie parmi : (i) une molécule d'acide nucléique d'un gène d'une cellule endothéliale, dont l'expression est induite par un facteur angiogénique et inhibée par un agent angiostatique, d'une séquence complémentaire ou d'un fragment de celle-ci, (ii) une séquence polypeptidique codée par ladite molécule d'acide nucléique, ou un fragment de celle-ci (iii) une molécule capable d'inhiber l'expression d'une molécule d'acide nucléique selon (i) ou de se fixer sur une séquence polypeptidique selon (ii).

Selon un mode particulier de mise en oeuvre, la composition pharmaceutique de l'invention comprend à titre de composé actif au moins une séquence nucléotidique sélectionnée parmi l'ensemble des séquences nucléotidiques identifiées sous les numéros SEQ ID No. : 1 à SEQ ID No. : 53, SEQ ID No. 225 et SEQ ID No. 284 à SEQ ID No. 290 dans la liste de séquences en annexe, leurs séquences complémentaires et leurs séquences antisens correspondantes, ou un de leurs fragments.

Au sens de la présente invention, doivent être considérées comme des séquences équivalentes, des séquences nucléotidiques présentant des modifications structurelles, mineures, ne modifiant pas leur fonction, telles que délétions, mutations ou additions de bases, dont l'identité est d'au moins de 90% avec les séquences nucléotidiques identifiées sous les numéros SEQ ID No. : 1 à SEQ ID No. : 53, SEQ ID No. 225 et SEQ ID No. 284 à SEQ ID No. 290 dans la liste de séquences en annexe.

Selon un autre mode particulier de mise en oeuvre, la composition pharmaceutique, régulatrice de l'angiogénèse, comprend au moins une séquence inhibitrice de l'angiogènese.

Selon un mode particulier de mise en oeuvre, la composition pharmaceutique régulatrice de l'angiogenèse, comprend au moins une séquence stimulatrice de l'angiogenèse.

Selon un mode particulier de mise en oeuvre, la composition pharmaceutique de l'invention comprend une ou plusieurs séquences inhibitrices de l'angiogènese comprenant une séquence antisens d'au moins une séquence choisie parmi les SEQ ID N°1 à SEQ ID N°5, SEQ ID N°7, SEQ ID N°9, SEQ ID N°11 à SEQ ID N°15, SEQ ID N°17 à SEQ ID N°53, SEQ ID No. 225 et SEQ ID No. 284 à SEQ ID No. 290 dans la liste de séquences en annexe.

De préférence, la composition pharmaceutique de l'invention comprend une ou plusieurs séquences antisens choisies parmi les SEQ ID N°103 à SEQ ID SEQ ID No. 107, SEQ ID No. 109, SEQ ID No. 111 et SEQ ID No. 113 à SEQ ID No. 148 dans la liste de séquences en annexe.

Selon un deuxième mode de mise en oeuvre, la composition pharmaceutique de l'invention comprend une ou plusieurs séquences stimulatrice(s) de l'angiogènese comprenant une séquence antisens d'au moins une séquence choisie parmi les séquences SEQ ID N°6, SEQ ID N°8, SEQ ID N°10 et SEQ ID N°16 dans la liste de séquences en annexe.

De préférence la composition pharmaceutique de l'invention comprend des séquences antisens choisies parmi les séquences SEQ ID No. 108, SEQ ID No. 110 et SEQ ID No. 112 dans la liste de séquences en annexe.

L'invention a également pour objet une composition pharmaceutique destinée au diagnostic et/ou au traitement de pathologies liées à l'angiogenèse caractérisée en ce qu'elle comprend au moins une séquence polypeptidique, sélectionnée parmi de séquences polypeptidiques identifiées sous les numéros SEQ ID No. : 54 à SEQ ID No. : 102 ou parmi de séquences polypeptidiques identifiées sous les numéros SEQ ID No. : 291 à SEQ ID No. : 297 dans la liste de séquences en annexe.

Au sens de la présente invention, doivent être considérées comme des séquences équivalentes, des séquences polypeptidiques présentant des modifications structurelles, mineures, ne modifiant pas leur fonction, telles que délétions, mutations ou additions de résidus d'acides aminés, dont l'identité est d'au moins de 85%, de préférence d'au moins 90% avec les séquences polypeptidiques identifiées sous les numéros SEQ ID No. : 54 à SEQ ID No. : 102 ou avec les séquences polypeptidiques identifiées sous les numéros SEQ ID No. : 291 à SEQ ID No. : 297 dans la liste de séquences en annexe.

L'invention a également pour objet une composition pharmaceutique destinée au diagnostic et/ou au traitement de pathologies liées à l'angiogenèse comprenant au moins un antagoniste d'un ou plusieurs séquences polypeptidiques ci-dessus mentionnées.

On entend par antagoniste tout composé inhibiteur de l'activité biologique des dites séquences polypeptidiques, dans le mécanisme de l'angiogenèse.

A titre d'exemple de tels antagonistes, on peut citer un anticorps ayant une affinité pour ladite séquence polypeptidique.

L'invention concerne aussi des anticorps ayant une affinité pour chacune des séquences polypeptidiques identifiées sous les numéros SEQ ID No. : 54 à SEQ ID No. : 102 ou avec les séquences polypeptidiques identifiées sous les numéros SEQ ID No. : 291 à SEQ ID No. : 297 dans la liste de séquences en annexe, ainsi que les compositions thérapeutiques les comprenant.

Lesdits anticorps peuvent être obtenus par toute méthode d'immunisation *in vivo* ou *in vitro*, d'un animal, notamment d'un vertébré et de préférence d'un mammifère avec l'une quelconque des séquences polypeptidiques identifiées sous les numéros SEQ ID No. : 54 à SEQ ID No. : 102 ou avec les séquences polypeptidiques identifiées sous les numéros SEQ ID No. : 291 à SEQ ID No. : 297 dans la liste de séquences en annexe, ou l'un de leurs fragments conservant l'immunogénicité de la protéine totale.

Les anticorps peuvent être des anticorps polyclonaux ou monoclonaux. (Kohler G. and Milstein C. Nature. 1975 Aug 7;256(5517):495-7.)

L'invention concerne aussi une composition thérapeutique ou diagnostique comprenant un ou plusieurs anticorps ayant une affinité par une ou plusieurs des séquences polypeptidiques identifiées sous les numéros SEQ ID No. : 54 à SEQ ID No. : 102 ou avec les séquences polypeptidiques identifiées sous les numéros SEQ ID No. : 291 à SEQ ID No. : 297 ou par l'un de leurs fragments conservant cette affinité, ou préparés comme indiqué ci-dessus.

Un autre objet de l'invention concerne les séquences nucléotidiques antisens des séquences nucléotidiques identifiées sous les numéros SEQ ID No : 1 à SEQ ID No : 53, SEQ ID No. 225 et SEQ ID No. 284 à SEQ ID No. 290 dans la liste de séquences en annexe.

Dans le cadre de la présente invention on comprend par séquence antisens: Toute séquence d'ADN, d'au moins 10 mers, complémentaire d'un ARNm qui en s'hybridant avec une portion de la séquence d'ARNm initial inhibe son expression, c'est-à-dire sa traduction en une protéine.

Tout préférentiellement l'invention a pour objet les séquences antisens ayant une identité d'au moins 85%, de préférence de 95% avec une séquence choisie parmi les séquences identifiées sous les numéros SEQ ID N° 103 à SEQ ID N° 148 dans la liste de séquences en annexe.

L'invention a également pour objet un vecteur d'expression de mammifère comprenant au moins une séquence antisens définie ci-dessus.

Plus particulièrement ledit vecteur est choisi parmi le groupe de vecteurs GS-V1 à GS-V46, identifiés par leur séquence, portant les numéros SEQ ID N° 149 à SEQ ID N° 194 dans la liste de séquences an annexe.

L'introduction desdites séquences SEQ ID N° 103 à SEQ ID N° 148 ou de l'un de leurs homologues dans des vecteurs d'expression de mammifère, et l'insertion ultérieure desdits vecteurs dans des cellules de mammifère permet l'obtention de lignées cellulaires sous-exprimant les gènes intervenant dans le mécanisme de l'angiogènese.

Ces amorces particulièrement préférées sont indiquées sur le Tableau I ci-dessous et identifiées avec les numéros de séquences SEQ ID No. 195 à SEQ ID No. : 222, SEQ ID No. : 226 à SEQ ID No. : 283 et SEQ ID No. : 298 à SEQ ID No. : 299 dans la liste de séquences en annexe.

L'invention a également pour objet un vecteur d'expression de mammifère, ledit vecteur comprenant au moins une séquence antisens d'au moins une des séquences identifiées sous les numéros SEQ ID No 1 à SEQ ID No 53, SEQ ID No 225 et SEQ ID No 284 à SEQ ID No 290 dans la liste de séquences en annexe, ainsi qu'un promoteur qui permet l'expression dudit ADN antisens.

Pour la construction de ces vecteurs, des amorces spécifiques pour chacun des séquences identifiées, sont dessinées.

Avantageusement, l'amplification du fragment du plasmide bactérien comprenant le gène cloné est effectuée au moyen d'amorces s'hybridant aux régions du plasmide encadrant le gène cloné. Elles comprennent également dans leurs extrémités les sites de restriction non contenus dans le fragment cloné et présents dans la région multisite du vecteur d'expression.

Par exemple dans le cadre des fragments clonés mis en oeuvre dans la présente invention, les sites de restriction utilisés, avec le vecteur d'expression pCI, sont les sites salI et MluI. Ces deux sites de restriction peuvent être interchangés selon que le fragment ait été cloné dans le plasmide bactérien dans son orientation sens ou antisens.

A titre d'exemple, les amorces GS-PGS-F (SEQ ID No. 223) et GS-PGM-R (SEQ ID No. 224) sont utilisés pour des fragments clonés en orientation sens dans le plasmide bactérien.(GS-N15)

Ces amorces particulières peuvent être utilisées de façon universelle pour transférer tous les fragments clonés en orientation sens dans un vecteur bactérien pour intégrer le vecteur d'expression dans l'orientation antisens.

Les amorces GS-PGM-F (SEQ ID No. 300) et GS-PGS-R (SEQ ID No.301) sont utilisées pour des fragments clonés en orientation antisens dans le plasmide bactérien.(GS-N46).

Ces amorces particulières peuvent être utilisées de façon universelle pour transférer tous les fragments clonés en orientation antisens dans un vecteur bactérien pour intégrer le vecteur d'expression dans l'orientation antisens.

L'invention a également pour objet un vecteur d'expression de mammifère comprenant au moins une séquence nucléotidique choisie parmi l'ensemble de séquences identifiées sous les numéros SEQ ID. N° 1 à SEQ ID. N° 53, SEQ ID No. 225 et SEQ ID. N° 284 à SEQ ID. N° 290 dans la liste de séquences en annexe ou un de leurs fragments.

Ces constructions sont utiles d'une part pour préparer des compositions thérapeutiques destinées au traitement, par thérapie cellulaire, de désordres angiogéniques et d'autre part pour vérifier l'efficacité d'un traitement d'un désordre angiogénique chez un mammifère, notamment chez un être humain, ou encore pour vérifier la fonctionnalité des gènes éventuellement impliquées dans le mécanisme de l'angiogénèse, dans ledit mammifère .

Ainsi l'invention a également pour objet une cellule génétiquement modifiée, comprenant au moins l'un des vecteurs comprenant des séquences anti-sens, sous-exprimant au moins une séquence nucléotidique choisie parmi les séquences SEQ ID No 1 à SEQ ID No 53, SEQ ID No 225 et SEQ ID No 284 à SEQ ID No 290 dans la liste de séquences en annexe.

Un autre objet de l'invention concerne une méthode de préparation d'une lignée de cellules génétiquement modiofiées exprimant de manière stable un vecteur d'expression, ledit vecteur comprenant au moins une séquence antisens d'au moins une des séquences identifiées sous les numéros SEQ ID No 1 à SEQ ID No 53, SEQ ID No 225 et SEQ ID No 284 à SEQ ID No 290 dans la liste de séquences en annexe, ainsi qu'un promoteur qui permet l'expression dudit ADN antisens comprenant les étapes suivantes :
(a) on introduit un gène de résistance à au moins un antibiotique dans ladite cellule génétiquement modifiée,
b) on cultive les cellules obtenues à l'étape (a) en présence dudit antibiotique,
c) on sélectionne les cellules viables.

L'invention concerne également une composition pharmaceutique destinée au diagnostic et/ou au traitement de pathologies liées à l'angiogenèse comprenant, à titre de principe actif, ladite cellule génétiquement modifiée.

D'autre part, l'invention concerne également une cellule génétiquement modifiée comprenant au moins un vecteur comprenant une séquence nucléotidique choisie parmi l'ensemble de séquences identifiées sous les numéros SEQ ID. N° 1 à SEQ ID. N° 53, SEQ ID No. 225 et SEQ ID. N° 284 à SEQ ID. N° 290 dans la liste de séquences en annexe ou un de leurs fragments, surexprimant ladite séquence.

Aussi l'invention concerne une méthode de préparation d'une lignée de cellules génétiquement modifiée exprimant de manière stable un vecteur d'expression, ledit vecteur comprenant au moins une des séquences identifiées sous les numéros SEQ ID No 1 à SEQ ID No 53, SEQ ID No 225 et SEQ ID No 284 à SEQ ID No 290 dans la liste de séquences en annexe ou un de leurs fragments, ainsi qu'un promoteur qui permet l'expression dudit ADN antisens, comprenant les étapes suivantes :
(a) on introduit un gène de résistance à au moins un antibiotique dans ladite cellule génétiquement modifiée,
b) on cultive les cellules obtenues à l'étape (a) en présence dudit antibiotique,
c) on sélectionne les cellules viables.

On peut donc envisager d'isoler des cellules humaines et de les transfecter *in vitro* avec au moins un des vecteurs ci-dessus définis, codant pour au moins un des gènes dont les séquences sont identifiées sous les numéros SEQ ID No. : 1 à SEQ ID No. : 53, SEQ ID No ; 225 et SEQ ID No. 284 à SEQ ID No. 290 ou l'un de leurs fragments. Ces cellules génétiquement modifiées, pourront être ensuite administrées à un mammifère, de préférence un mammifère humain.

Des compositions thérapeutiques contenant de telles cellules peuvent se présenter sous forme de simples suspensions cellulaires, mais elles peuvent également être encapsulées dans un dispositif adéquat, en utilisant par exemple des membranes semi-perméables.

Un autre objet de l'invention est une méthode de préparation d'une protéine codée par au moins un des gènes dont les séquences sont identifiées sous les numéros SEQ ID No. : 1 à SEQ ID No. : 53, SEQ ID No : 225 et SEQ ID No. 284 à SEQ ID No. 290 dans la liste de séquences en annexe ou d'un de leurs fragments.

Ces protéines identifiées par les séquences SEQ ID No. 54 à SEQ ID No. 102 et SEQ ID No. 291 à SEQ ID No. 297 dans la liste de séquences en annexe, ou leurs fragments peuvent être produites, sous forme de protéines recombinantes *in vitro,* en introduisant dans un hôte adéquat un vecteur d'expression correspondant adéquat, puis purifiées et utilisées ensuite comme agent thérapeutique.

Une telle méthode de préparation d'une protéine recombinante comprend les étapes de :
a) la construction d'un vecteur d'expression comprenant au moins une séquence parmi celles identifiées sous les numéros SEQ ID No. : 1 à SEQ ID No. : 53, SEQ ID No : 225 et SEQ ID No. 284 à SEQ ID No. 290 dans la liste de séquences en annexe ou d'un de leurs fragments.
b) l'introduction dudit vecteur dans un hôte cellulaire,
c) la culture desdites cellules dans un milieu adéquat,
d) la purification des protéines exprimées ou d'un de leurs fragments.

L'invention concerne également une protéine recombinante obtenue par la méthode ci-dessus.

A titre d'exemple, des systèmes d'expression de protéines recombinantes chez les bactéries telles que E. Coli peuvent être utilisés pour exprimer de protéines ou polypeptides non glycosylés.

La séquence codante ou une séquence partielle du gène d'intérêt est amplifiée par PCR en utilisant des amorces spécifiques de ce gène avec aux extrémités des sites d'enzymes de restriction de préférence différents pour permettre l'orientation du gène dans le vecteur d'expression. L'ADN amplifié est purifié puis digéré par les enzymes de restriction ensuite insérée par ligation au vecteur d'expression préalablement digéré par ces mêmes enzymes de restriction. Un grand nombre de vecteurs peut-être utilisé comme le vecteur pBR322 (Bolivar et al., Gene 2 (1977) 95-113) ou ses dérivés, contenant par exemple le promoteur de l'ARN polymérase du bactériophage T7 pour un haut niveau d'expression, comme le plasmide pET3a (Studier et Moffatt,1986, J Mol Biol, 189(1):113-30), contenant de préférence les séquences qui codent pour des marqueurs de sélection (résistance à des antibiotiques), un site multiple de clonages contenant les sites d'enzymes de restrictions adéquats pour l'insertion de l'ADN, le système cellule/hôte est de préférence un système inductible comme celui utilisé pour le radiomarquage in vivo du facteur de croissance FGF2 (Colin et al, 1997, Eur.J.Biochem., 249, 473-480) et déjà décrit par Patry et al (1994, FEBS Lett, 349(1):23-8), il peut également contenir une région codant pour une queue poly histidine en extrémité du polypeptide d'intérêt pour faciliter la purification.

L'ADN amplifié est ligué dans le plasmide qui est transformé dans la bactérie selon la méthode décrite par Sambrook et al. (1989, Molecular Cloning: A Laboratory Manual, 2nd ed., Cold Spring Harbor Laboratory Press, Cold Spring Harbor, NY.). Les cellules transformées sont étalées sur milieu LB agar contenant les antibiotiques, les colonies résistantes aux antibiotiques sont alors contrôlées par PCR puis analyse sur gel. L'ADN plasmidique peut être isolé puis séquencé pour confirmer la construction. La production et la purification de la protéine recombinante peuvent être réalisées comme décrit ((Patry et al ,1994, FEBS Lett, 349(1):23-8),473-480).Brièvement, une colonie isolée est inoculée dans le milieu de culture liquide tel que le milieu LB broth additionné des antibiotiques. Après une nuit d'incubation, la pré-culture peut être utilisée pour ensemencer une culture d'un plus grand volume. L'expression du polypeptide est alors induite, les cellules se développent pendant quelques heures puis sont récoltées par centrifugation. Le culot cellulaire peut être lysé par agents chimiques connus dans l'art ou mécaniquement par sonication par exemple. La protéine peut-être purifiée grâce à ses propriétés physicochimiques comme décrit pour la purification du FGF2 recombinant (Colin et al, 1997, Eur.J.Biochem., 249, 473-480) ou si la protéine est étiquetée avec une queue poly histidine, elle peut être purifiée via cette queue par immobilisation sur un support chélateur d'ions métalliques comme décrit (Tang et al, Protein Expr Purif 1997 Dec;11(3):279-83).

A titre d'exemple, des systèmes d'expression de protéines recombinantes pour exprimer des polypeptides ayant des modifications post traductionnelles comme la glycosylation, tels que des systèmes eucaryotes (levures, plantes, insectes) peuvent être utilisés.

Ainsi, la protéine recombinante peut être produite par exemple dans la levure Pichia Pastoris comme décrit par Sreekrishna et al (1988, J Basic Microbiol,28(4):265-78). L'ADN amplifié sera introduit de la même façon après digestion et ligation dans un vecteur d'expression de Pichia Pastoris, contenant de préférence une séquence codant pour un marqueur de sélection (Scorer et al, Biotechnology (N Y), 1994 Feb;12(2):181-4). La protéine peut être soit intracellulaire soit sécrétée si le vecteur contient à l'extrémité du gène introduit une séquence codant pour une séquence signal de sécrétion comme par exemple le facteur prepropeptide de Saccharomyces cerevisiae (Cregg et al., 1993; Scorer et al., 1993). Une queue Histidine peut également être ajoutée à l'une des extrémités de la protéine recombinante pour faciliter la purification (Mozley et al, 1997, Photochem Photobiol, 66(5):710-5).

De préférence, ledit hôte est choisi parmi : une bactérie, une levure, une cellule d'insecte et une cellule d'un mammifère, une cellule végétale.

L'administration de compositions thérapeutiques comprenant de telles protéines peut se faire par exemple par voie topique, orale, intradermique, transdermique, intraveineuse.

Les fragments desdites protéines peuvent être utilisés en tant qu'antagonistes de la protéine dont ils sont issus. Ainsi l'administration adéquate à un animal d'une composition thérapeutique comprenant de tels fragments est préconisée pour induire une diminution de l'activité de ladite protéine dans le mécanisme de l'angiogenèse dans une pathologie donnée.

La présente invention concerne également une méthode de diagnostic d'une pathologie angiogénique chez un mammifère, notamment chez un être humain, consistant à détecter dans les cellules dudit mammifère la surexpression ou la sous-expression d'une ou plusieurs séquences nucléotidiques identifiées par les numéros SEQ ID N° 1 à SEQ ID N° 53, SEQ ID No. 225 et SEQ ID N° 284 à SEQ ID N° 290 dans la liste de séquences en annexe.

Une telle méthode de diagnostic comprend les étapes suivantes :
- la détection de l'expression d'une ou plusieurs desdites séquences nucléotidiques SEQ ID N° 1 à SEQ ID N° 53, SEQ ID No. 225 et SEQ ID N° 284 à SEQ ID N° 290 par une population cellulaire d'un mammifère,
- la détection de l'expression de celle(s) même(s) séquence(s) par une population cellulaire de référence dont l'état angiogénique est connu,
- l'identification des différences éventuelles du niveau d'expression de celle(s) même(s) séquence(s) par les deux populations cellulaires.

La présente invention concerne également une méthode de diagnostic et de pronostic d'une pathologie angiogénique chez un mammifère, notamment chez un être humain, consistant à détecter dans les cellules dudit mammifère la surexpression ou la sous-expression d'une ou plusieurs séquences polypeptidiques identifiées par les numéros SEQ ID No. : 54 à SEQ ID No. : 102 ou avec les séquences polypeptidiques identifiées sous les numéros SEQ ID No. : 291 à SEQ ID No. : 297 dans la liste de séquences en annexe.

Selon un mode de mise en oeuvre préféré, ladite méthode comporte les étapes suivantes :
a) la détection de l'expression d'une ou plusieurs desdites séquences polypeptidiques SEQ ID No. : 54 à SEQ ID No. : 102 ou avec les séquences polypeptidiques identifiées sous les numéros SEQ ID No. : 291 à SEQ ID No. : 297 par une population cellulaire d'un mammifère,
b) la détection de l'expression de celle(s) même(s) séquence(s) polypeptidique(s) par une population cellulaire de référence dont l'état angiogénique est connu,
c) l'identification des différences éventuelles du niveau d'expression de celle(s) même(s) séquence(s) polypeptidique(s) par les deux populations cellulaires.

Selon un mode particulier de mise en oeuvre, dans la méthode de diagnostic de l'invention la détection de l'expression des séquences est effectuée après avoir mis les cellules endothéliales en présence d'un fluide biologique issu d'un patient.

La présente invention concerne également une méthode de vérification de l'efficacité thérapeutique d'un traitement angiogénique chez un mammifère, notamment chez un être humain, par l'identification d'une population cellulaire chez ledit mammifère susceptible de surexprimer ou sous-exprimer une ou plusieurs séquences nucléotidiques identifiées par les numéros SEQ ID N° 1 à SEQ ID N° 53, SEQ ID No. 225 et SEQ ID N° 284 à SEQ ID N° 290 dans la liste de séquences en annexe.

Une telle méthode de vérification de l'efficacité thérapeutique comprend les étapes suivantes :
- la détection de l'expression d'une ou plusieurs desdites séquences nucléotidiques SEQ ID N° 1 à SEQ ID N° 53 et SEQ ID No. 225 par une population cellulaire d'un mammifère auquel est administrée une composition thérapeutique destinée à traiter un désordre angiogénique,
- la détection de l'expression de celle(s) même(s) séquence(s) nucléotidiques par une population cellulaire de référence dont l'état angiogénique est connu,
- l'identification des différences éventuelles du niveau d'expression de celle(s) même(s) séquence(s) nucléotidique(s) par les deux populations cellulaires.

Selon des modes de réalisation préférés, la méthode de vérification est effectuée sur une population cellulaire d'un mammifère *in vivo, ex-vivo,* ou bien sur une population cellulaire isolée dudit mammifère *in vitro*

Selon un mode particulier de mise en oeuvre, dans la méthode de vérification de l'invention la détection de l'expression des séquences est effectuée après avoir mis les cellules endothéliales en présence d'un fluide biologique issu d'un patient.

La présente invention concerne également une méthode de criblage de composés utiles pour le traitement angiogénique d'un mammifère, notamment d'un être humain.

Selon un mode de mise en oeuvre préféré, une telle méthode de criblage comprend les étapes suivantes :
- la détection de l'expression d'une ou plusieurs desdites séquences nucléotidiques SEQ ID N° 1 à SEQ ID N° 53, SEQ ID No. 225 et SEQ ID N° 284 à SEQ ID N° 290 par une population cellulaire mise en présence d'un composé susceptible d'avoir un effet thérapeutique sur un désordre angiogénique,
- la détection de l'expression celle(s) même(s) séquence(s) nucléotidique(s) par une population cellulaire de référence dont l'état angiogénique est connu,
- l'identification des différences éventuelles du niveau d'expression celle(s) même(s) séquence(s) nucléotidique(s) par les deux populations cellulaires.

Selon un autre mode de mise en oeuvre préféré, une telle méthode de criblage comprend également les étapes suivantes :
- la détection de l'expression d'une ou plusieurs desdites séquences polypeptidiques identifiées par les numéros SEQ ID No. : 54 à SEQ ID No. : 102 ou avec les séquences polypeptidiques identifiées sous les numéros SEQ ID No. : 291 à SEQ ID No. : 297 dans la liste de séquences en annexe par une population cellulaire mise en présence d'un composé susceptible d'avoir un effet thérapeutique sur un désordre angiogénique,
- la détection de l'expression de celle ou celles mêmes séquences polypeptidiques par une population cellulaire de référence dont l'état angiogénique est connu,
- l'identification des différences éventuelles du niveau d'expression de celle(s) même(s) séquence(s) polypeptidique(s) par les deux populations cellulaires.

Selon un mode particulier de mise en oeuvre, dans la méthode de criblage de l'invention la détection de l'expression des séquences est effectuée après avoir mis les cellules endothéliales en présence d'un fluide biologique issu d'un patient.

Parmi les désordres angiogéniques, susceptibles d'être diagnostiqués ou traités avec les compositions pharmaceutiques de l'invention on peut citer : la vascularisation de tumeurs, les rétinopathies, l'arthrite rhumatoïde, la maladie de Crohn, l'athérosclérose, l'hyperstimulation de l'ovaire, le psoriasis, l'endométrie associée à la néovascularisation, la resténose due à l'angioplastie du ballon, la superproduction tissulaire due à la cicatrisation, la maladie vasculaire périphérique, l'hypertension, l'inflammation vasculaire, la maladie et les phénomènes de Raynaud, l'anévrisme, la resténose artérielle, la thrombophlébite, la lymphagyte, le lymphodème, la cicatrisation et la réparation tissulaire, l'ischémie, l'angine, l'infarctus de myocarde, la maladie chronique du coeur, les insuffisances cardiaques telles que l'insuffisance cardiaque congestive, la dégénération maculaire liée à l'âge et l'ostéoporose.

L'invention a également pour objet un dispositif comprenant un support comprenant une ou plusieurs sondes spécifiques d'une ou plusieurs séquences nucléotidiques identifiées sous les numéros SEQ ID No : 1 à SEQ ID No: 53, SEQ ID No. 225 et SEQ ID No. 284 à SEQ ID No. 290, dans la liste de séquences en annexe pour la mise en oeuvre de la méthode de criblage de l'invention.

Dans le cadre de la présente invention on entend par sonde tout fragment d'ADN simple brin dont la séquence est complémentaire à une séquence recherchée : celle-ci pourra ainsi être décelée par hybridation avec la sonde marquée (par incorporation d'atomes radioactifs ou de groupements fluorescents), qui joue le rôle d'un "hameçon" moléculaire.

Selon des modes de mise en oeuvre préférés, le support dudit dispositif est choisi parmi une membrane de verre, une membrane métallique, une membrane polymère, une membrane de silice.

De tels dispositifs sont par exemple des puces à ADN comprenant une ou plusieurs séquences nucléotidiques identifiées sous les numéros SEQ ID No : 1 à SEQ ID No: 53, SEQ ID No. 225 et SEQ ID No. 284 à SEQ ID No. 290, dans la liste de séquences en annexe.

L'invention a aussi pour objet une trousse destinée à la mesure de l'affichage différentiel de gènes impliqués dans des désordres angiogéniques comprenant un dispositif tel que précédemment décrit, des amorces spécifiques et les accessoires nécessaires à l'amplification des séquences extraites d'un échantillon, leur hybridation avec les sondes du dispositif et la réalisation des mesures de l'affichage différentiel.

L'invention a aussi pour objet une trousse destinée à la mesure de l'affichage différentiel de gènes impliqués dans des désordres angiogéniques comprenant une lignée de cellules génétiquement modifiées exprimant de manière stable le vecteur exprimant au moins une des séquences nucléotidiques identifiées sous les numéros SEQ ID No : 1 à SEQ ID No: 53, SEQ ID No. 225 et SEQ ID No. 284 à SEQ ID No. 290, dans la liste de séquences en annexe, ou un de leurs fragments en tant que population cellulaire de référence et les moyens nécessaires pour la mesure dudit affichage différentiel.

L'invention a aussi pour objet une trousse destinée à la mesure de l'affichage différentiel de gènes impliqués dans des désordres angiogéniques comprenant une lignée de cellules génétiquement modifiées exprimant de manière stable le vecteur exprimant au moins une séquence antisens d'une des séquences nucléotidiques identifiées sous les numéros SEQ ID No : 1 à SEQ ID No: 53, SEQ ID No. 225 et SEQ ID No. 284 à SEQ ID No. 290, dans la liste de séquences en annexe, ou un de leurs fragments, en tant que population cellulaire de référence et les moyens nécessaires pour la mesure dudit affichage différentiel.

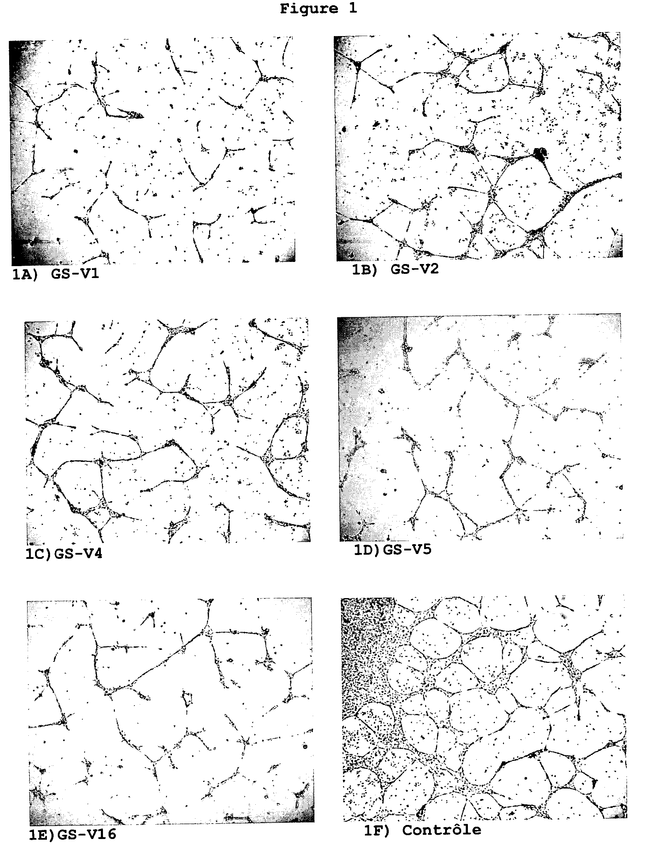

La vérification de l'implication des cinquante-quatre gènes identifiés et de leurs homologues dans le mécanisme de l'angiogènese a été effectuée selon la méthodologie décrite dans la section Matériel et méthodes.
Cette implication est illustrée à l'aide des figures 1 à 11 en annexe, dans lesquelles :
La figure 1 montre que l'expression de GS-V1, GS-V2, GS-V4, GS-V5, GS-V15 dans les cellules endothéliales humaines inhibe la formation des tubes capillaires : cellules endothéliales transfectées avec 1A)GS-V1 codant pour le transcrit antisens spécifique de GS-N1; 1B)GS-V2 codant pour le transcrit antisens spécifique de GS-N2; 1C)GS-V4 codant pour le transcrit antisens spécifique de GS-N4; 1D)GS-V5 codant pour le transcrit antisens spécifique de GS-N5 ; 1E)GS-V15 codant pour le transcrit antisens spécifique de GS-N15; 2F) le vecteur vide (Contrôle).

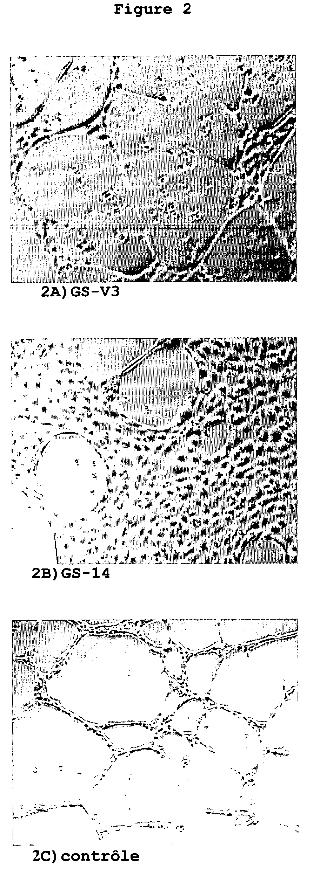

La figure 2 montre que l'expression de GS-V3, GS-V14 chez les cellules endothéliales humaines inhibe la formation des tubes capillaires : cellules endothéliales transfectées avec 2A)GS-V3 codant pour le transcrit antisens spécifique de GS-N3; 2B)GS-V13 codant pour le transcrit antisens spécifique de GS-N13; 2C) le vecteur vide (Contrôle).

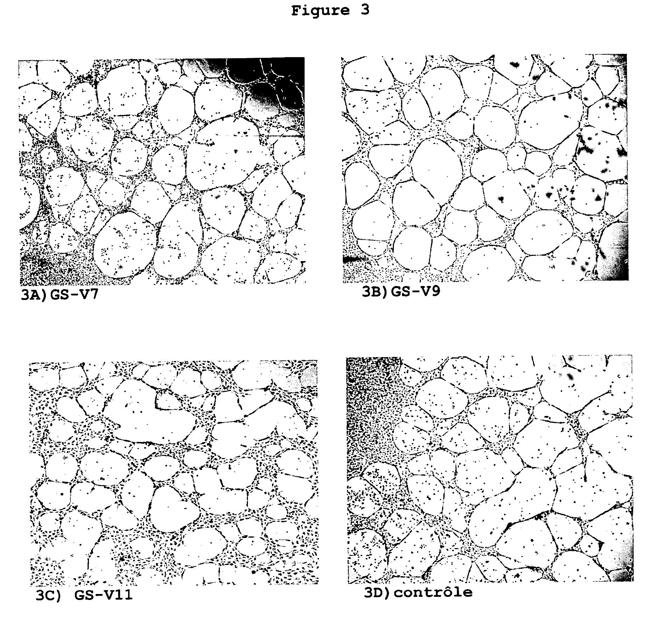

La figure 3 montre que l'expression de GS-V6, GS-V8, GS-V10 chez les cellules endothéliales humaines induit la formation des tubes capillaires : cellules endothéliales transfectées où 3A) GS-V6 codant pour le transcrit antisens spécifique de GS-N6; 3B) GS-V8 codant pour le transcrit antisens spécifique de GS-N8; 3C) GS-V10 codant pour le transcrit antisens spécifique de GS-N10 et de son homologue GS-N54; 3D) le vecteur vide (Contrôle).

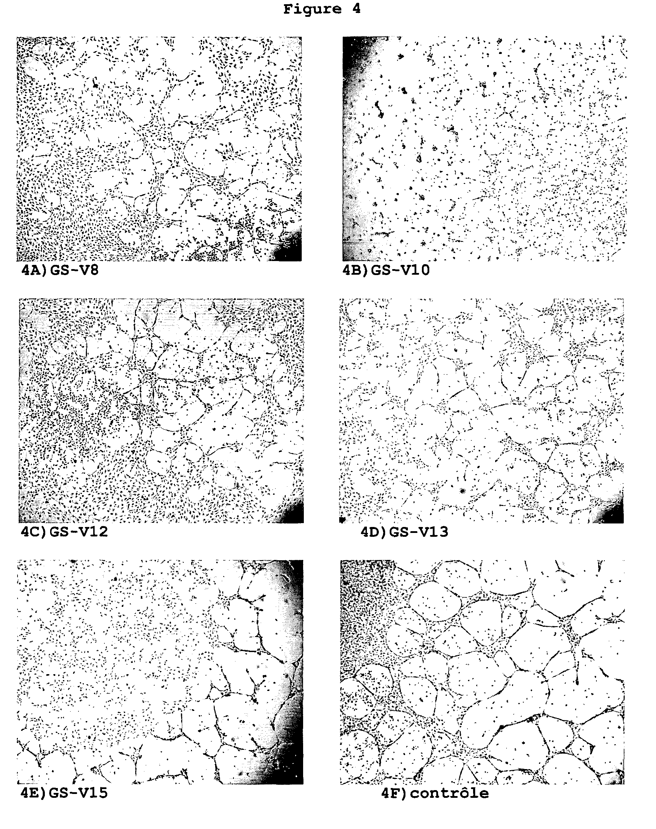

La figure 4 montre que l'expression de GS-V7, GS-V9, GS-V11, GS-V12, GS-V14 chez les cellules endothéliales humaines inhibe la formation des tubes capillaires : cellules endothéliales transfectées avec 4A)GS-V7 codant pour le transcrit antisens spécifique de GS-N7; 4B)GS-V9 codant pour le transcrit antisens spécifique de GS-N9; 4C)GS-V11 codant pour le transcrit antisens spécifique de GS-N11; 4D)GS-V12 codant pour le transcrit antisens spécifique de GS-N12; 4E)GS-V14 codant pour le transcrit antisens spécifique de GS-N14 et 4F) le vecteur vide (Contrôle).

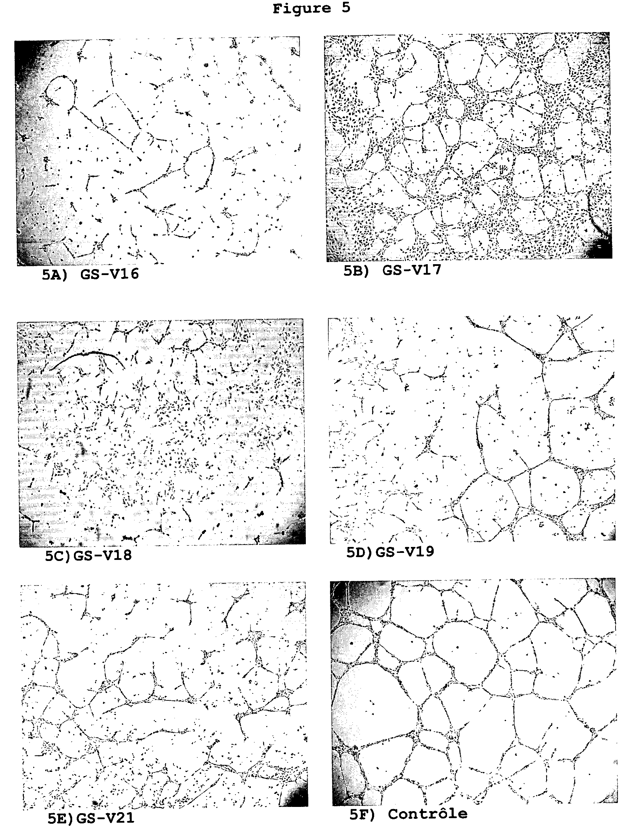

La figure 5 montre que l'expression de GS-V16, GS-V17, GS-V18, GS-V19, GS-V21 chez les cellules endothéliales humaines inhibe la formation des tubes capillaires : cellules endothéliales transfectées avec 5A)GS-V16 codant pour le transcrit antisens spécifique de GS-N16; 5B)GS-V17 codant pour le transcrit antisens spécifique de GS-N17; 5C)GS-V18 codant pour le transcrit antisens spécifique de GS-N18; 5D)GS-V19 codant pour le transcrit antisens spécifique de GS-N19 ; 5E)GS-V21 codant pour le transcrit antisens spécifique de GS-N21; 5F) le vecteur vide (Contrôle).

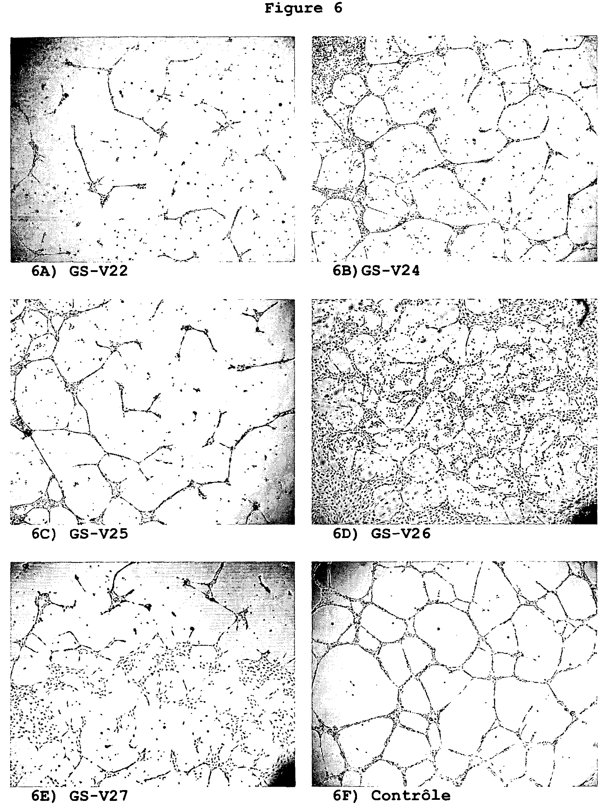

La figure 6 montre que l'expression de GS-V22, GS-V24, GS-V25, GS-V26, GS-V27 chez les cellules endothéliales humaines inhibe la formation des tubes capillaires : cellules endothéliales transfectées avec 6A)GS-V22 codant pour le transcrit antisens spécifique de GS-N22; 6B)GS-V24 codant pour le transcrit antisens spécifique de GS-N24 et de son homologue GS-N49; 6C)GS-V25 codant pour le transcrit antisens spécifique de GS-N25 et de son homologue GS-N50; 6D)GS-V26 codant pour le transcrit antisens spécifique de GS-N26 ; 6E)GS-V27 codant pour le transcrit antisens spécifique de GS-N27 et son homologue GS-N51; 6F) le vecteur vide (Contrôle).

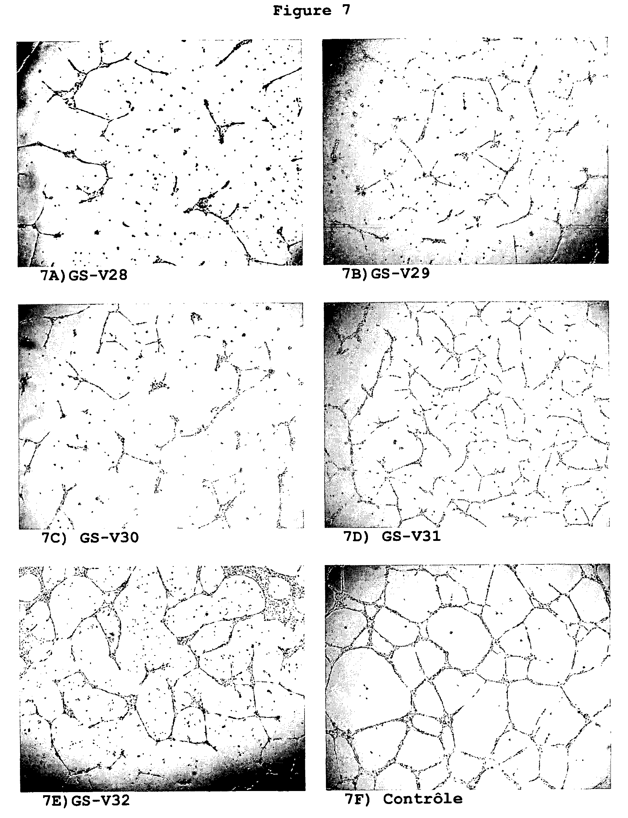

La figure 7 montre que l'expression de GS-V28, GS-V29, GS-V30, GS-V31, GS-V32 chez les cellules endothéliales humaines inhibe la formation des tubes capillaires : cellules endothéliales transfectées avec 7A)GS-V28 codant pour le transcrit antisens spécifique de GS-N28; 7B)GS-V29 codant pour le transcrit antisens spécifique de GS-N29 et son homologue GS-N52; 7C)GS-V30 codant pour le transcrit antisens spécifique de GS-N30; 7D)GS-V31 codant pour le transcrit antisens spécifique de GS-N31 et son homologue GS-N53 ; 7E)GS-V32 codant pour le transcrit antisens spécifique de GS-N32 ; 7F) le vecteur vide (Contrôle).

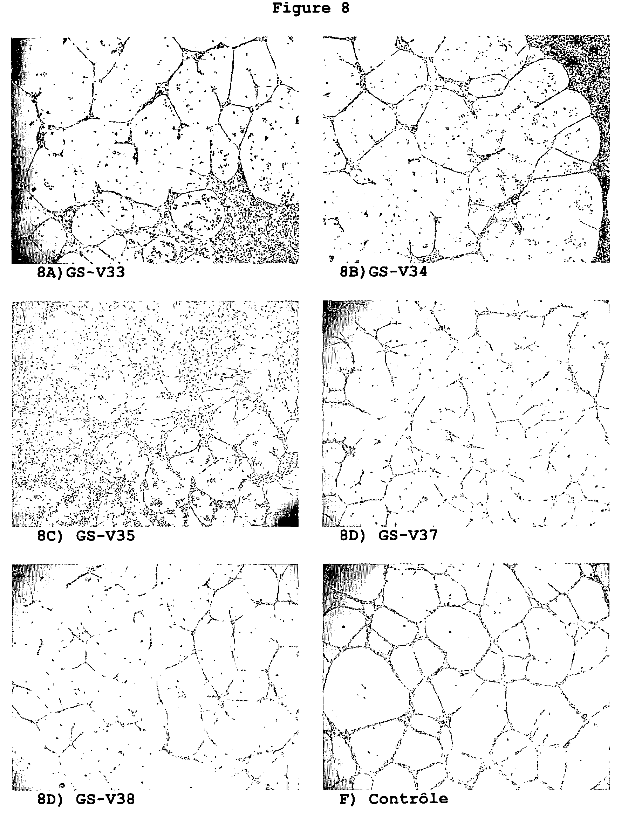

La figure 8 montre que l'expression de GS-V33, GS-V34, GS-V35, GS-V37, GS-V38 chez les cellules endothéliales humaines inhibe la formation des tubes capillaires : cellules endothéliales transfectées avec 8A)GS-V33 codant pour le transcrit antisens spécifique de GS-N33; 8B)GS-V34 codant pour le transcrit antisens spécifique de GS-N34; 8C)GS-V35 codant pour le transcrit antisens spécifique de GS-N35; 8D)GS-V37 codant pour le transcrit antisens spécifique de GS-N37 ; 8E)GS-V38 codant pour le transcrit antisens spécifique de GS-N38 ; 8F) le vecteur vide (Contrôle).

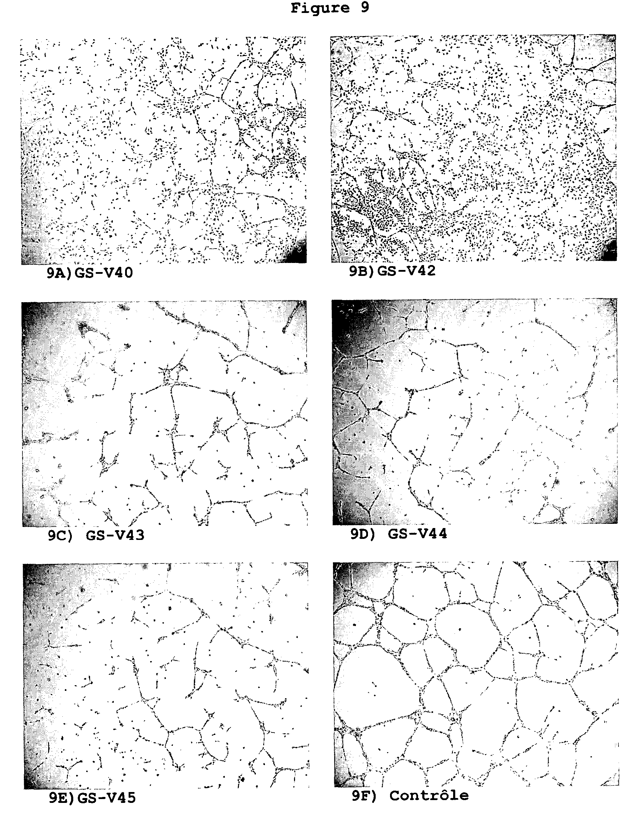

La figure 9 montre que l'expression de GS-V40, GS-V42, GS-V43, GS-V44, GS-V45 chez les cellules endothéliales humaines inhibe la formation des tubes capillaires : cellules endothéliales transfectées avec 9A)GS-V40 codant pour le transcrit antisens spécifique de GS-N40; 9B)GS-V42 codant pour le transcrit antisens spécifique de GS-N42; 9C)GS-V43 codant pour le transcrit antisens spécifique de GS-N43; 9D)GS-V44 codant pour le transcrit antisens spécifique de GS-N44 ; 9E)GS-V45 codant pour le transcrit antisens spécifique de GS-N45; F) le vecteur vide (Contrôle).

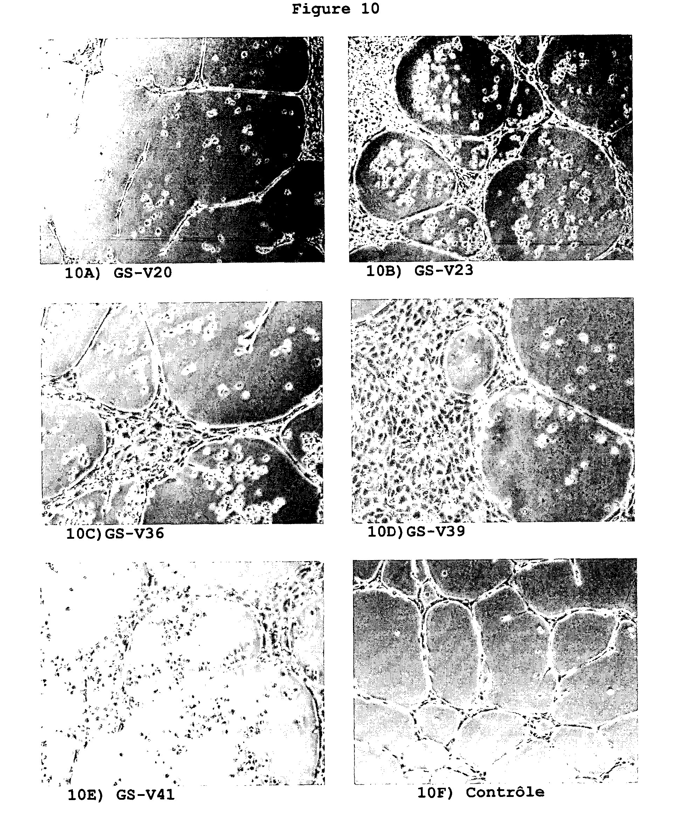

La figure 10 montre que l'expression de GS-V20, GS-V23, GS-V36 GS-V39 GS-V41 chez les cellules endothéliales humaines inhibe la formation des tubes capillaires : cellules endothéliales transfectées avec 10A)GS-V20 codant pour le transcrit antisens spécifique de GS-N20; 10B)GS-V23 codant pour le transcrit antisens spécifique de GS-N23 et ses homologues GS-N47 et GS-N48; 10C) GS-V36 codant pour le transcrit antisens spécifique de GS-N36; 10D)GS-V39 codant pour le transcrit antisens spécifique de GS-N39; 10E)GS-V41 codant pour le transcrit antisens spécifique de GS-N41; 10F) le vecteur vide (Contrôle).

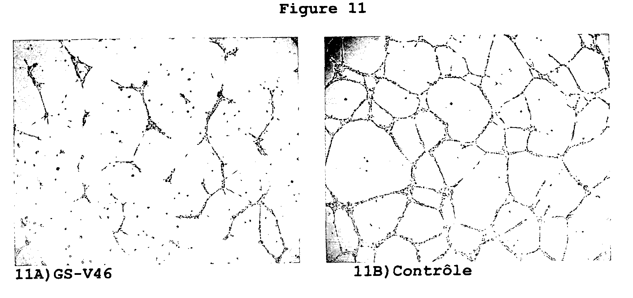

La figure 11 montre que l'expression de GS-V46, chez les cellules endothéliales humaines inhibe la formation des tubes capillaires : cellules endothéliales transfectées avec 11A)GS-V46 codant pour le transcrit antisens spécifique de GS-N46; 11B) le vecteur vide (Contrôle).

### MATÉRIEL ET METHODES

### 1.Culture des cellules et test d'angiogenèse

Des cellules endothéliales de veines ombilicales (HUVEC) sous lesdites quatre conditions de culture sont alors utilisées pour identifier les gènes codant pour les constituants cellulaires impliqués dans la régulation de l'angiogenèse.

Les cellules endothéliales sont maintenues en milieu complet (EGM-2 de Clonetics).

Pour l'identification des gènes impliqués dans l'angiogenèse dans les *test in vitro* de l'angiogenèse selon le modèle de Montesano et al.(1986, Proc Natl Acad Sci U S A, 83(19):7297-301). Les cellules sont tout d'abord ensemencées sur un gel de Collagène type I en milieu complet jusqu'à confluence. Puis, les cellules HUVEC de référence sont cultivées en milieu appauvri en sérum et sans facteurs de croissance : EBM-2 + 2% de sérum et différents facteurs sont ajoutés dans les conditions test.

Le FGF2 : à des concentrations comprises entre 5 ng/ml et 60 ng/ml, de préférence entre 10 et 40 ng/ml ; du VEGF : à des concentrations comprises entre 10 ng/ml et 60 ng/ml, de préférence comprises entre 30 ng/ml et 50 ng/ml ; du PF4 : à de concentrations comprises entre 0,1 et 5 µg/ml, de préférence comprises entre 0,5 µg/ml et 1 µg/ml ; du TNF-α à des concentrations comprises entre 20 ng/ml et 100 ng/ml, de préférence comprises entre 30 ng/ml et 60 ng/ml ; du IFN-γ :à des concentrations comprises entre 50 ng/ml et 200 ng/ml, de préférence comprises entre 80 ng/ml et 120 ng/ml.

Les cellules endothéliales humaines placées sous les quatre conditions de culture précitées sont alors utilisées pour identifier des gènes codant pour les constituants cellulaires impliqués dans la régulation de l'angiogenèse.

### 2. Facteurs angiogéniques et angiostatiques.

Des facteurs angiogéniques et angiostatiques, ayant un effet sur l'expression des gènes identifiés en corrélation avec la formation de néovaisseaux ou l'inhibition de néovaisseaux respectivement, utilisés, à titre d'exemple, dans le cadre de la présente invention, sont illustrés ci-dessous.
VEGF=Facteur de croissance endothélial vasculaire.
FGF2=Facteur de croissance basique du fibroblaste.
HGF=Facteur de croissance de l'hépatocyte.
PF4=Facteur plaquettaire 4.
IFN-γ=Interféron gamma.
TNF-α=Facteur de nécrose tumorale alpha

Le TNF-α qui est un régulateur de l'angiogénèse, il peut induire l'angiogénèse *in vivo* mais également inhiber la formation des vaisseaux *in vitro* (Frater-Schroder et al, 1987 , Proc Natl Acad Sci U S A,84(15):5277-81; Fajardo et al, 1992, Am J Pathol Mar,140(3):539-44; Niida et al, 1995, Neurol Med Chir (Tokyo),35(4):209-14. Dans notre modèle d'angiogénèse *in vitro,* le TNF-α est utilisé dans des conditions d'inhibition de l'angiogénèse.

### 3. Comparaison des expressions géniques.

Les expressions géniques peuvent être alors comparées en utilisant les puces d'ADN, le SAGE, une réaction d'amplification par PCR quantitative, les vecteurs viraux pour construire des banques soustractives, ou encore l'analyse par affichage différentiel.
Dans le cadre des travaux expérimentaux ayant conduit à la présente invention, la Demanderesse a utilisé préférentiellement la technique d'affichage différentiel pour l'identification desdits gènes.

### Affichage différentiel

Les ARN totaux sont préparés à partir des cellules HUVEC cultivées sur un gel de collagène en présence des différents facteurs utilisés, selon la méthode RNeasy Mini kit (Qiagen) en intégrant une étape de digestion à la DNase I selon le protocole préconisé par le fabricant.
L'affichage différentiel à partir des ARNs totaux est réalisé selon la méthode décrite par Liang et Pardee (1992, Science, 14;257(5072):967-7)en utilisant l'αP33-ATP en dilution isotopique au cours de l'amplification par PCR pour la visualisation des bandes par autoradiographie des gels d'électrophorèse.
Ainsi, les fragments d'ADN différentiellement présents sur le gel en fonction des conditions de culture analysées sont découpés, réamplifiés, clonés dans un plasmide PGEM easy vector, Promega), séquencés et identifiés par questionnement de la banque BLAST.

### 4. Vérification de l'implication des gènes identifiés dans le mécanisme de l'angiogènese.

### Test de fonctionnalité des gènes

Dans une deuxième étape, la fonctionnalité de chaque séquence identifiée est testée sur le modèle d'angiogenèse *in vitro* avec les cellules endothéliales transfectées avec un vecteur d'expression comprenant un oligonucléotide antisens de ladite séquence.

Pour la construction de ces vecteurs, des amorces spécifiques pour chacun des séquences identifiées, sont dessinées. Ces amorces sont indiquées sur le Tableau I ci-dessous et identifiées avec les numéros de séquences SEQ ID No. 195 à SEQ ID No. : 222, SEQ ID No. : 226 à SEQ ID No. : 283 et SEQ ID No. : 298 à SEQ ID No. : 299 dans la liste de séquences en annexe.

**Tableau I**

| **SÉQ.ID** | Nom amorce |
|---|---|
| SEQ ID No 1 (GS-N1) | GV1-1 GV1-2 |
| SEQ ID No 2 (GS-N2) | GV2-1 GV2-2 |
| SEQ ID No 3 (GS-N3) | GV3-1 GV3-2 |
| SEQ ID No 4 (GS-N4) | GV4-1 GV4-2 |
| SEQ ID No 5 (GS-N5) | GV5-1 GV5-2 |
| SEQ ID No 6 (GS-N6) | GV6-1 GV6-2 |
| SEQ ID No 7 (GS-N7) | GV7-1 GV7-2 |
| SEQ ID No 8 (GS-N8) | GV8-1 GV8-2 |
| SEQ ID No 9 (GS-N9) | GV9-1 GV9-2 |
| SEQ ID No 10 (GS-N10) | GV10-1 GV10-2 |
| SEQ ID No 11 (GS-N11) | GV11-1 GV11-2 |
| SEQ ID No 12 (GS-N12) | GV12-1 GV12-2 |
| SEQ ID No 13 (GS-N13) | GV13-1 GV13-2 |
| SEQ ID No 14 (GS-N14) | GV14-1 GV14-2 |
| SEQ ID No 15 (GS-N15) | GS-PGS-F GS-PGM-R |
| SEQ ID No 16 (GS-N54) | GV10-1 GV10-2 |
| SEQ ID No 17 (GS-N16) | GV16-1 GV16-2 |
| SEQ ID No 18 (GS-N17) | GV17-1 GV17-2 |
| SEQ ID No 19 (GS-N18) | GV18-1 GV18-2 |
| SEQ ID No 20 (GS-N19) | GV19-1 GV19-2 |
| SEQ ID No 21 (GS-N20) | GV20-1 GV20-2 |
| SEQ ID No 22 (GS-N21) | GV21-1 GV21-2 |
| SEQ ID No 23 (GS-N22) | GV22-1 GV22-2 |
| SEQ ID No 24 (GS-N23) | GV23-1 GV23-2 |
| SEQ ID No 25 (GS-N24) | GV24-1 GV24-2 |
| SEQ ID No 26 (GS-N25) | GV25-1 GV25-2 |
| SEQ ID No 27 (GS-N26) | GV26-1 GV26-2 |
| SEQ ID No 28 (GS-N27) | GV27-1 GV27-2 |
| SEQ ID No 29 (GS-N28) | GV28-1 GV28-2 |
| SEQ ID No 30 (GS-N29) | GV29-1 GV29-2 |
| SEQ ID No 31 (GS-N30) | GV30-1 GV30-2 |
| SEQ ID No 32 (GS-N31) | GV31-1 GV31-2 |
| SEQ ID No 33 (GS-N32) | GV32-1 GV32-2 |
| SEQ ID No 34 (GS-N33) | GV33-1 GV33-2 |
| SEQ ID No 35 (GS-N34) | GV34-1 GV34-2 |
| SEQ ID No 36 GS-N35) | GV35-1 GV35-2 |
| SEQ ID No 37 (GS-N36) | GV36-1 GV36-2 |
| SEQ ID No 38 (GS-N37) | GV37-1 GV37-2 |
| SEQ ID No 39 (GS-N38) | GV38-1 GV38-2 |
| SEQ ID N°40 (GS-N39) | GV39-1 GV39-2 |
| SEQ ID No 41 (GS-N40) | GV40-1 GV40-2 |
| SEQ ID No 42 (GS-N41) | GV41-1 GV41-2 |
| SEQ ID No 43 (GS-N42) | GV42-1 GV42-2 |
| SEQ ID No 44 (GS-N43) | GV43-1 GV43-2 |
| SEQ ID No 45 (GS-N44) | GV44-1 GV44-2 |
| SEQ ID No 46 (GS-N45) | GV45-1 GV45-2 |
| SEQ ID No 47 (GS-N46) | GS-PGM-F GS-PGS-R |
| SEQ ID No 48 (GS-N47) | GV23-1 GV23-2 |
| SEQ ID No 49 (GS-N48) | GV23-1 GV23-2 |
| SEQ ID No 50 (GS-N49) | GV24-1 GV24-2 |
| SEQ ID No 51 (GS-N51) | GV27-1 GV27-2 |
| SEQ ID No 52 (GS-N52) | GV29-1 GV29-2 |
| SEQ ID No 53 (GS-N53) | GV31-1 GV31-2 |
| SEQ ID No 225 (GS-N50) | GV50-1 GV50-2 |

Ces amorces contiennent à chacune de leurs extrémités, un site d'une enzyme de restriction différente (SalI : GTCGAC ou MluI : ACGCGT).

Des fragments amplifiés de chaque gène sont obtenus par PCR à partir des plasmides bactériens contenant le fragment du gène identifié en utilisant lesdites amorces.

Ces fragments sont purifiés, digérés par les enzymes de restriction SalI et MluI et insérés dans un vecteur d'expression chez les mammifères du type pCi-neo vector, (Promega), lui-même digéré par ces deux enzymes de restriction.
Chaque fragment est introduit dans l'orientation antisens.

Dans le cas particulier des séquences GS-N15 et GS-N46, l'amplification du fragment cloné dans le plasmide bactérien est effectuée au moyen d'amorces particulières, choisies parmi les séquences GS-PGS-F, GS-PGM-R ou GS-PGM-F et GS-PGS-R s'hybridant aux régions du plasmide encadrant le gène cloné et comprenant également dans leurs extrémités les sites de restriction (sites SalI et MluI), non contenus dans le fragment cloné, et présents dans la région multisite du vecteur d'expression.

Ces deux sites de restriction pouvant être interchangés selon que le fragment ait été cloné dans le plasmide bactérien dans son orientation sens ou antisens.

Des contrôles effectués avec ces amorces, que l'on peut considérer comme des amorces universelles, en absence du gène cloné, (plasmide vide) ont montré que le fragment amplifié du plasmide (40 pb) lorsqu'il est intégré dans le vecteur d'expression n'altère pas la formation des néovaisseaux dans le test de fonctionnalité in vitro. Les résultats obtenus avec ce vecteur ainsi construit sont identiques à ceux obtenus avec le vecteur vide (Résultats non montrés) et montrent que ces paires de bases supplémentaires n'altèrent pas l'effet des fragments antisens spécifiques de la séquence identifiée.

D'une façon générale, les vecteurs susceptibles d'être utilisés pour la mise en évidence de la fonctionnalité des gènes identifiés dans la présente invention dans le mécanisme de l'angiogénèse comprennent tout système de vecteurs d'expression chez les mammifères comprenant un promoteur qui permet l'expression d'un gène cloné, à titre d'exemple on peut citer le promoteur « fort » du cytomégalovirus humain (CMV).

D'autres vecteurs d'expression constitutifs ou inductibles susceptibles d'être également utilisés, sont indiqués dans la liste non-exhaustive ci-après indiquée :
Vecteurs commercialisés par la Société Promega ; des vecteurs avec un promoteur « fort » pour un haut niveau d'expression constitutif des gènes chez les cellules de mammifères (pCI Mammalian Expression vector, Expression Vector System cloning vector pALTER(R)*-MAX), des vecteurs commercialisés par la société Invitrogen : (pcDNA3 .1, -/hygro, -/Zeo, pcDNA4/HisMAx, -E, pBudCE4, pRcRSV, pRcCMV2, pSecTag2, - /hygro secretion vectors, les vecteurs pEBVHis A, B et C), des vecteurs d'expression chez le mammifère commercialisés par la société Clontech (pIRES, pIRES-EYFP pIRES2-EGFP, pCMV-Myc et pCMV-HA), Epitope-Tagged pTRE, les vecteurs VP16 Minimal Domain (ptTA 2, ptTA 3 et ptTA 4), les vecteurs d'expresion Tet bidirectionnels (pBI, pBI-EGFP, pBI-G, pBI-L), pRevTRE, pTRE2, pLEGFP-N1 Vecteur Retroviral pLEGFP-C1, les systèmes d'expression adenoviraux Adeno-X , pCMS-EGFP, pd1EGFP-N1, pd2ECFP-N1, pd2EYFP-N1, pEGFP(-C1, -C2, -C3, -N1, -N2, -N3), pEYFP-C1, -N1.
Chaque vecteur comprenant ledit fragment anti-sens est alors produit chez E.Coli, extrait, purifié et quantifié. Un µg de chaque vecteur est incubé en présence d'un agent transfectant (effectene, Qiagen) en suivant le protocole préconisé par le fabricant avec les cellules endothéliales. Vingt-quatre heures après la transfection, les cellules endothéliales sont trypsinées et étalées sur la matrice extracellulaire contenant les facteurs angiogéniques en l'occurrence le matrigel selon le modèle décrit par Grant et al, (1989,Cell,58(5):933-43). Après 24h d'incubation, la formation de vaisseaux est observée et comparée aux cellules témoins transfectées avec le vecteur d'expression de mammifère vide.

### 5. Etablissement de la banque de lignées stables exprimant les vecteurs d'expression contenant les séquences des gènes ou leurs fragments ou les séquences antisens.

Les systèmes d'expression peuvent comprendre un marqueur de sélection à un antibiotique (un gène de résistance à un antibiotique), pour sélectionner les cellules transfectées exprimant de façon stable le vecteur comprenant l'acide nucléique cloné dans ledit vecteur et ce, soit dans le même vecteur, soit dans un 2^{ème} vecteur co-transfecté.
Ce vecteur d'expression peut être un système d'expression constitutif ou inductible.

Dans l'exemple particulier ci-dessous décrit, les lignées stables pour l'expression de l'oligonucléotide antisens correspondant à chaque gène identifié ont été obtenues avec un vecteur d'expression constitutif et après sélection en présence d'antibiotique.

Pour ce faire, 24h après la transfection effectuée dans les conditions décrites ci-dessus, les cellules endothéliales BAEC sont trypsinées et ensemencées à raison de 80 000 cellules/puits en plaque de six puits en présence de 700 µg/ml de l'antibiotique G418 (Promega). Un puit contrôle est ensemencé avec des cellules non transfectées. Le milieu est changé tous les trois jours avec une recharge de l'antibiotique. Les cellules contrôles sont éliminées après 8 à 10 jours, les cellules résistantes à l'antibiotique sont récoltées à confluence (après 2 à trois semaines) puis transférées en flacons de culture toujours en présence de l'antibiotique. Les lignées stables sont ensuite testées pour leur capacité à former ou non des vaisseaux dans le test d'angiogenèse *in vitro.*

### 6. Résultats

### 6.1 Identification des gènes.

Les séquences d'acide nucléiques nommés GS-N1, GS-N2, GS-N3, GS-N4, GS-N5, GS-N6, GS-N7, GS-N8, GS-N9, GS-N10 (ou GS-N54), GS-N11, GS-N12, GS-N13, GN-14 et GS-N15, et respectivement les protéines codées par lesdits acides nucléiques GS-N1 à GS-N13 et GS-N15 nommées : angioinducine, angiodockine, angioblastine, angioreceptine, angiodensine, angiopartnerine, vassoserpentine, angiosulfatine, vassoreceptine, angiokinasine, vassosubstratine, angiosignaline, angiofoculine, angiohélicine, angioacyline, n'ont pas été identifiées auparavant comme ayant un rôle biologique quelconque, et encore moins un dans le processus de l'angiogenèse ou la différentiation des cellules endothéliales en tubes capillaires. Ces protéines sont décrites ci-dessous.
La méthode d'affichage différentielle ci-dessus décrite a permis l'identification des ARNm suivants :
- GS-N1 : un ARNm de 1683pb identifié par la séquence SEQ ID No : 1 dans la liste de séquences en annexe. Une recherche BLAST sur la base de séquences GENBANK permet de l'identifier sous le numéro d'accession BC008502.
   La séquence de cet ARNm a une séquence codante du nucléotide 159 au nucléotide 458. Nous avons donc identifié une protéine GS-P1, résultante de la traduction de cet ARNm. Cette protéine est composée de 99aa , identifiée sous le numéro SEQ ID No 54 dans la liste de séquences en annexe, appelée Angioinducine
- GS-N2 ; un ARNm de 1649 pb, identifié par la séquence SEQ ID No : 2 dans la liste de séquences en annexe. Une recherche BLAST sur la base de séquences GENBANK permet de l'identifier sous le numéro d'accession NM_022823.
   La séquence de cet ARNm a une séquence codante du nucléotide 367 au nucléotide 1071. Nous avons donc identifié une protéine GS-P2, résultante de la traduction de cet ARNm. Cette protéine est composée de 234 aa , identifiée sous le numéro SEQ ID No 55 dans la liste de séquences en annexe, appelée Angiodockine.
- GS-N3 : un ARNm de 5766 pb, identifié par la séquence SEQ ID No : 3 dans la liste de séquences en annexe. Une recherche BLAST sur la base de séquences GENBANK permet de l'identifier sous le numéro d'accession AB007963.
   La séquence de cet ARNm a une séquence codante du nucléotide 978 au nucléotide 2465. Nous avons donc identifié une protéine GS-P3, résultante de la traduction de cet ARNm. Cette protéine est composée de 495 aa , identifiée sous le numéro SEQ ID No 56 dans la liste de séquences en annexe, appelée Angioblastine.
- GS-N4 : un ARNm de 5242 pb identifié par la séquence SEQ ID No . 4 dans la liste de séquences en annexe. Une recherche BLAST sur la base de séquences GENBANK permet de l'identifier sous le numéro d'accession AB037835.
   La séquence de cet ARNm a une séquence codante partielle du nucléotide 1 au nucléotide 4762. Nous avons donc identifié une protéine GS-P4, résultante de la traduction de cet ARNm. Cette protéine est composée de 1586 aa, identifiée sous le numéro SEQ ID No 57 dans la liste de séquences en annexe, appelée Angioreceptine
- GS-N5 : un ARNm de 2153 pb identifié par la séquence SEQ ID No : 5 dans la liste de séquences en annexe. Une recherche BLAST sur la base de séquences GENBANK permet de l'identifier sous le numéro d'accession AK025682.
   La séquence de cet ARNm a une séquence codante du nucléotide 38 au nucléotide 691. Nous avons donc identifié une protéine GS-P5, résultante de la traduction de cet ARNm. Cette protéine est composée de 217 aa, identifiée sous le numéro SEQ ID No 58 dans la liste de séquences en annexe, appelée Angiodensine.
- GS-N6 : un ARNm de 3005 pb identifié par la séquence SEQ ID No : 6 dans la liste de séquences en annexe. Une recherche BLAST sur la base de séquences GENBANK permet de l'identifier sous le numéro d'accession AK023284.
   La séquence de cet ARNm a une séquence codante du nucléotide 90 au nucléotide 773. Nous avons donc identifié une protéine GS-P6, résultante de la traduction de cet ARNm. Cette protéine est composée de 227 aa, identifiée sous le numéro SEQ ID No 59 dans la liste de séquences en annexe, appelée Vassoserpentine.
- GS-N7: un ARNm de 4397 identifié par la séquence SEQ ID No : 7 dans la liste de séquences en annexe. Une recherche BLAST sur la base de séquences GENBANK permet de l'identifier sous le numéro d'accession AB033073.
   La séquence de cet ARNm a une séquence codante partielle du nucléotide 286 au nucléotide 2943. Nous avons donc identifié une protéine GS-P7, résultante de la traduction de cet ARNm. Cette protéine est composée de 885 aa, identifiée sous le numéro SEQ ID No 60 dans la liste de séquences en annexe, appelée Angiosulfatine.
- GS-N8 : un ARNm de 5844 pb identifié par la séquence SEQ ID No : 8 dans la liste de séquences en annexe. Une recherche BLAST sur la base de séquences GENBANK permet de l'identifier sous le numéro d'accession AB023187:.
   La séquence de cet ARNm a une séquence codante partielle du nucléotide 1 au nucléotide 3456. Nous avons donc identifié une protéine GS-P8, résultante de la traduction de cet ARNm. Cette protéine est composée de 1151 aa, identifiée sous le numéro SEQ ID No 61 dans la liste de séquences en annexe, appelée Vassoreceptine.
- GS-N9 : un ARNm de 4266 pb identifié par la séquence SEQ ID No : 9 dans la liste de séquences en annexe. Une recherche BLAST sur la base de séquences GENBANK permet de l'identifier sous le numéro d'accession AB014587.
   La séquence de cet ARNm a une séquence codante partielle du nucléotide 1 au nucléotide 3528. Nous avons donc identifié une protéine GS-P9, résultante de la traduction de cet ARNm. Cette protéine est composée de 1175 aa, identifiée sous le numéro SEQ ID No 62 dans la liste de séquences en annexe, appelée Angiokinasine.
   L'angiokinasine est homologue avec la MAP4K4 (SEQ ID No. : 224 dans la liste de séquences en annexe), n° accession: XM_038751 (Séquence nucléique:4197 bp, séquence protéique: 1141aa).
   L'un des mécanismes par lequel les cellules répondent au stimuli externe est le recrutement des chaînes constituées d'un ensemble de protéines qui assurent le relais du signal externe vers l'intérieur des cellules. En assurant la transduction du signal, cette chaîne change l'environnement intracellulaire contrôlant ainsi la transcription génique (revues: Avruch, 1998, Mol. Cell. Biochem., 182, 31-48; Karin, 1998, Ann. N. Y. Acad. Sci. 851, 139-146). Un nombre important de ces chaînes et par conséquent les voies de signalisation qui sont hautement conservées à travers l'évolution sont nommées collectivement les voix des protéines kinases activées par des agents mitogènes (MAPK)(Gupta et al, 1996, EMBO J., 15, 2760-2770; Madhani et Fink, 1998, Trends Genet. 14, 151-155). La voix MAPK classique est déclenchée par la liaison des facteurs de croissance à leur récepteur de la surface cellulaire conduisant à l'activation de la protéine Ras, GTPase. Cette voix résulte en l'activation des protéines kinases régulées par les signaux extracellulaires (ERKs) conduisant à la transcription génique et la prolifération cellulaire. Une voix parallèle de MAPK est stimulée par des facteurs de stress tels que le choc osmotique, les produits cytotoxiques, les radiations UV ou encore les cytokines inflammatoires. Cette voix résulte en l'activation des protéines kinase activées par le stress, connues sous le nom des kinases agissant sur le N-terminal de c-Jun (SAPK/JNKs) (Karin, 1998, Ann. N. Y. Acad. Sci. 851, 139-146). Une seconde voix activée par le stress conduit à l'activation de la MAPK p38. L'effet de l'activation par le stress couvre la prolifération, la différentiation ou encore la transcription génique conduisant à l'arrêt du cycle cellulaire et/ou l'apoptose tout dépend du type cellulaire et le stimulus (Karin, 1998, Ann. N. Y. Acad. Sci. 851, 139-146).
   Plusieurs études ont rapporté un rôle pour les MAPK 1 et 2 ainsi que la MAPK p38 dans les voix de transduction du signal provoquées par des facteurs angiogéniques ou anti-angiogéniques durant l'angiogenèse, cependant aucun rôle n'a été signalé pour la MAKP4 dans ce processus (Tanaka et al, 1999, Jpn J Cancer Res,90: 647-654; Erdreich-Epstein et al, 2000, Cancer Res, 60:712-721; Gupta et al, 1999, Exp Cell Res, 247:495-504; Bais et al, 1998, Nature, 391:24-25; Rousseau et al, Oncogene,1997, 15: 2169-2177; Shore et al, 1997, Placenta, 18:657-665).
   La MAPK 4 est un des membres de la famille des MAPK. Cette kinase phosphoryle directement et par conséquent active les kinases agissant directement sur le N-terminal c-Jun (JNK), en réponse aux stress et/ou aux cytokines inflammatoires. La MAPK 4 est exprimée dans différents tissus, cependant on note une abondance de l'expression de cette kinase dans les muscles squelettiques et le cerveau. Les souris déficientes du gène de la MAPK 4 développent des hépathogénèses anormales et meurent à l'état embryogénique au quatorzième jour. Cependant des lignées cellulaires déficientes en MAPK 4 ont été obtenues. Ces lignées se caractérisent par l'absence de transcription génique dépendante de JNK et du facteur de transcription AP-1. De plus, les lymphocytes T, déficients en MAPK 4 montrent un découplage de la production de l' IL-2 suite à l'activation des récepteurs des cellules T suggérant un rôle clé pour MAPK4/JNK dans le processus inflammatoire. La mutation de la MAPK4 chez certains carcinomes indique qu'elle peut jouer un rôle de suppresseur de tumeurs. Bien que le contrôle de l'expression et l'activité de la MAPK4 fassent l'objet à l'heure actuelle d'intenses analyses et études, celles-ci sont dans une approche pour élaborer une thérapie anti-inflammatoire et anticancéreuse. Cependant, le rôle de la MAPK4 dans l'angiogenèse n'a pas été démontré jusqu'à nos jours.
   Pedram et al (Endocrinology, 2001, 142:1578-86) ont montré que le peptide natriuretique supprime ou inhibe l'angiogenèse induite par le VEGF, et ils ont également montré que l'activation des kinases agissant directement sur le N-terminal de c-Jun est une étape importante dans l'induction de l'angiogenèse par le VEGF. A l'encontre, Jimenez et al, Oncogene, 2001, 20: 3443-3448) ont rapporté que l'activation des kinases agissant sur le N-terminal de c-Jun est nécessaire pour l'inhibition de la néovascularisation par la thrombospondine 1.
   Dans un cas comme dans l'autre, ces deux récentes études ne se rapportent pas à un rôle spécifique de la MAP4K4 dans la régulation de l'angiogenèse.
- GS-N10: un ARNm de 2034 bp identifié par la séquence SEQ ID No : 10 dans la liste de séquences en annexe. Une recherche BLAST sur la base de séquences GENBANK permet de l'identifier sous le numéro d'accession XM_035658.
   Cette séquence est homologue de la séquence GS-N54.
   GS-N54: un ARNm de 4749 pb identifié par la séquence SEQ ID No : 16 dans la liste de séquences en annexe. Une recherche BLAST sur la base de séquences GENBANK permet de l'identifier sous le numéro d'accession AK024248. Cet ARNm n'a pas de séquence codante.
   La séquence de ARNm GS-N10 a une séquence codante du nucléotide 618 au nucléotide 1787. Nous avons donc identifié une protéine GS-P10, résultante de la traduction de cet ARNm. Cette protéine est composée de 389 aa, identifiée sous le numéro SEQ ID No 63 dans la liste de séquences en annexe, appelée Vassosubstratine.
- GS-N11: un ARNm de 1817pb appelé identifié par la séquence SEQ ID No : 11 dans la liste de séquences en annexe. Une recherche BLAST sur la base de séquences GENBANK permet de l'identifier sous le numéro d'accession NM_032181.
   La séquence de cet ARNm a une séquence codante du nucléotide 439 au nucléotide 897. Nous avons donc identifié une protéine GS-P11, résultante de la traduction de cet ARNm. Cette protéine est composée de 152 aa, identifiée sous le numéro SEQ ID No 64 dans la liste de séquences en annexe, appelée Angiosignaline.
- GS-N12: un ARNm de 4131pb identifié par la séquence SEQ ID No : 12 dans la liste de séquences en annexe. Une recherche BLAST sur la base de séquences GENBANK permet de l'identifier sous le numéro d'accession AB023233.
   La séquence de cet ARNm a une séquence codante partielle du nucléotide 1 au nucléotide 2384. Nous avons donc identifié une protéine GS-P12, résultante de la traduction de cet ARNm. Cette protéine est composée de 793 aa, identifiée sous le numéro SEQ ID No 65 dans la liste de séquences en annexe, appelée Angiofoculine.
- GS-N13: un ARNm de 2566pb identifié par la séquence SEQ ID No : 13 dans la liste de séquences en annexe. Une recherche BLAST sur la base de séquences GENBANK permet de l'identifier sous le numéro XM_018273.
   La séquence de cet ARNm a une séquence codante du nucléotide 426 au nucléotide 2345. Nous avons donc identifié une protéine GS-P13, résultante de la traduction de cet ARNm. Cette protéine est composée de 639 aa, identifiée sous le numéro SEQ ID No 66 dans la liste de séquences en annexe, appelée Angiohélicine.
- GS-N14: un ARNm de 1830pb identifié par la séquence SEQ ID No : 14 dans la liste de séquences en annexe. Une recherche BLAST sur la base de séquences GENBANK permet de l'identifier sous le numéro d'accession AK022109.
   Cette séquence ne contient pas de séquence codante.
- GS-N15: un ARNm de 6253 pb identifié par la séquence SEQ ID No : 15 dans la liste de séquences en annexe. Une recherche BLAST sur la base de séquences GENBANK permet de l'identifier sous le numéro d'accession NM_014873.
   La séquence de cet ARNm a une séquence codante du nucléotide 228 au nucléotide 1340. Il a été donc identifié une protéine GS-P15, résultante de la traduction de cet ARNm. Cette protéine est composée de 370 aa, identifiée sous le numéro SEQ ID No 67 dans la liste de séquences en annexe, appelée angioacyline.
   Les séquences d'acide nucléiques nommés GS-N16 à GS-N53 identifiées par les numéros SEQ ID No : 17 à SEQ ID No : 53, et SEQ ID No. : 225 dans la liste de séquences en annexe et respectivement les protéines codées par lesdits acides nucléiques, identifiées sous les numéros SEQ ID No. : 68 à SEQ ID No. : 102 dans la liste de séquences en annexe n'ont pas été identifiées auparavant comme ayant un rôle biologique dans le processus de l'angiogènese ou la différentiation des cellules endothéliales en tubes capillaires. Ces séquences sont décrites ci-dessous.
- GS-N16: un ARNm de 3139 pb identifié sous le numéro de séquence SEQ ID No : 17 dans la liste de séquences en annexe. Une recherche BLAST permet de l'identifier sous le numéro d'accession XM_011833 (Homo sapiens phosducin-like (PDCL) dans la base de séquences GENBANK.
   La séquence de cet ARNm a une région codante du nucléotide 167 au nucléotide 1072. Une protéine GS-P16 résultante de la traduction de cet ARNm a donc été identifiée.
   Cette protéine dont la séquence est identifiée sous le numéro SEQ ID No : 68 dans la liste de séquences en annexe est un analogue de la phosducine appelée PDCL, composée de 301 aa.
   Les protéines G (protéines liant la guanine) jouent un rôle majeur dans les voies de signalisations transmembranaires par transmission de signaux extracellulaires à travers des récepteurs transmembranaires vers leurs effecteurs intracellulaires appropriés (Gilman, 1987, Annu. Rev. Biochem., 56, 615-649; Simon et al, 1991, Science, 252, 802-808). Après liaison du ligand, le récepteur catalyse l'échange du GDP pour un GTP sur la sous unité alpha de la protéine G hétérotrimérique qui provoque son activation et la dissociation de la sous unité alpha-GTP des sous unités béta et gamma (Gilman, 1987, Annu. Rev. Biochem., .56, 615-649). Les voies de signalisations dépendantes de la protéine G sont désignées pour amplifier et intégrer une multiplicité de réponses à la fois stimulatrices et inhibitrices et leur importance dans la fonction cellulaire est telle, qu'elles sont étroitement régulées. La PhLP (phosducin-like protein) est un de ces éléments régulateurs, elle appartient à la famille des phosducines et ses isoformes, protéines qui lient les sous unités béta/gamma de la protéine G, bloquant ainsi leur fonction (Lee et al, 1987, Biochemistry 26, 3983-3990; Miles et al., 1993, Proc. Natl. Acad. Sci. U. S. A.,90, 10831-10835; Craft et al, 1998, Biochemistry 37, 15758-15772). La phosducine, fortement exprimée dans les cellules photoréceptrices de la rétine (.(Lee et al, 1987, Biochemistry 26, 3983-3990; Wilkins et al, 1996, J. Biol. Chem., 271, 19232-19237) a été proposée intervenir dans l'adaptation à la lumière (Willardson et al, 1996, Proc. Natl. Acad. Sci. U. S. A., 93, 1475-1479). Par contre, la fonction de PhLP est moins connue, cette protéine est plus largement exprimée (Miles et al, 1993, Proc. Natl. Acad. Sci. U. S. A. 90, 10831-10835) et lie également les sous unités beta/gamma de la protéine G avec une haute affinité (Schröder et Lohse, 1996, Proc. Natl. Acad. Sci. U. S. A., 93, 2100-2104; Thibault et al, 1997, J. Biol. Chem., 272, 12253-12256). Cette protéine est proposée représenter un homologue de la phosducine qui régule un certain nombre de voies dépendantes de la protéine G dans beaucoup de types cellulaires (Savage et al, 2000, J. Biol. Chem., Vol. 275, 39, 30399-30407).
   Mais à ce jour, aucun rôle de la PhLP n'a été décrit dans la régulation de l'angiogenèse.
- GS-N17: un ARNm de 2326pb identifié sous le numéro de séquence SEQ ID No 18 dans la liste de séquences en annexe. Une recherche BLAST permet de l'identifier sous le numéro d'accession BC011860 (Protéine Ribosomale L3 (RPL3)), dans la base de séquences GENBANK.
   La séquence de cet ARNm a une séquence codante du nucléotide 1030 au nucléotide 2241, identifiée sous le numéro SEQ ID No 69 dans la liste de séquences en annexe. Cette séquence polypeptidique de 403 aa est identique (100% d'identité) à la protéine ribosomale L3 humaine (RPL3) dont la séquence nucléique est plus courte, no accession BC006483 (SEQ ID N° 285), BC012786 (SEQ ID N° 286).
   La protéine ribosome L3 (RPL3) est une protéine hautement conservée (Herwig et al, 1992, Eur J Biochem, 207(3):877-85; Van Raay et al, 1996, Genomics:37(2):172-6) localisée dans la grosse sous unité ribosomale. Chez E.Coli, cette protéine est connue lier l'ARN ribosomal 23S et participer dans la formation du centre peptidyltransférase du ribosome (Noller, 1993, Bacteriol. 175:5297-530039; Noller, 1997, Annu. Rev. Biochem. 66:679-716).
   Bien que l'expression différentielle de la protéine ribosomale L3 a été démontrée dans plusieurs études : dans le muscle squelettique dans un modèle d'études de l'obésité chez la souris (Vicent et al, 1998 , Diabetes 47:1451-8), dans l'hypothalamus et le tissus adipeux brun chez la souris impliquant RLP3 dans la régulation de l'équilibre énergétique (Allan et al, 2000, Physiological Genomics, 3:149-156), aucune expression différentielle n'a été décrite durant l'angiogènese ni dans la régulation de l'angiogènese.
- GS-N18:un ARNm de 3937 pb identifié sous le numéro de séquence SEQ ID No 19 dans la liste de séquences en annexe. Une recherche BLAST permet de l'identifier sous le numéro d'accession XM_042798 (Protéine 20 RING finger protein 20 (RNF20)) dans la base de séquences GENBANK.
   Cet ARNm présente une séquence codante du nucléotide 89 au nucléotide 3016, sa traduction directe permet d'identifier une protéine homologue de la protéine RNF20 composée de 975 aa, (GS-P18), identifiée sous le numéro SEQ ID No 70 dans la liste de séquences en annexe.
   Cette séquence GS-P18 présente une identité totale avec la séquence protéique déduite de la séquence nucléotidique identifiée sous le numéro d'accession AF265230 dans la base GENBANK et sous le numéro SEQ ID No. 287 dans la liste de séquences en annexe. Les séquences nucléiques correspondant aux deux protéines présentent entre elles une homologie de 99%.
   la protéine RNF20 est encore peu connue, mais elle se caractérise par la présence du domaine RING FINGER. Les protéines Ring finger sont susceptibles de jouer un rôle dans la formation et l'architecture de larges complexes protéiques qui contribuent à divers processus cellulaires tels que la transduction du signal, l'oncogènese, l'apoptose, le développement, la différentiation, la régulation des gènes, l'ubiquination (Saurin et al, 1996, Trends Biochem. Sci. 21, 208-214;Borden, 2000, J. Mol. Biol., 295, 1103-1112 ; Topcu et al, 1999, Oncogene 18, 7091-7100).
   A ce jour, il n'a pas été décrit que la protéine RNF20 soit impliquée dans la régulation de l'angiogènese.
- GS-N19: un ARNm de 2167 pb identifié sous le numéro SEQ ID No 20 dans la liste de séquences en annexe. Une recherche BLAST permet de l'identifier sous le numéro d'accession BC002781 (Protéine analogue du facteur d'épissage, arginine/serine-rich 4), dans la base de séquences GENBANK.
   La séquence de cet ARNm présente une région codante, du nucléotide 107 au nucléotide 1591. Une protéine GS-P19 résultante de la traduction de cet ARNm, présentée sous le numéro SEQ ID No 71 dans la liste de séquences en annexe a donc été identifiée. Cette protéine composée de 494aa est homologue du facteur d'épissage, arginine/serine-rich 4 (SFRS4) également appelé SRp75 et présente les mêmes domaines caractéristiques.
   La SRp75 appartient à la famille des protéines SR du fait qu'elle contient en N-terminale un domaine conservé, RRM (motif de reconnaissance de l'ARN), une région riche en glycine, une région interne homologue au RRM et un long (315aa) domaine C-terminal composé essentiellement de résidus serine et arginine en alternance (domaine RS) (Zahler et al, Mol Cell Biol 1993 Jul;13(7):4023-8). Les protéines SR constituent une famille de phosphoprotéines nucléaires qui sont nécessaires pour l'épissage constitutif mais également influencent la régulation de l'épissage alternatif. Les protéines SR ont une structure en modules (un ou deux domaines RRM et un domaine RS). Chaque domaine dans les protéines SR est un module fonctionnel. L'action coordonnée des domaines RRM détermine leur spécificité de liaison aux ARNs, tandis que les domaines RS fonctionnent comme les activateurs d'épissage (Cáceres et al., 1997, J. Cell Biol., 138, 225-238 ; Chandler et al., 1997, Proc. Natl Acad. Sci. USA, 94, 3596-3601 ; Mayeda et al., 1999, Mol. Cell. Biol., 19, 1853-1863 ; Graveley et Maniatis, 1998, Mol. Cell, 1, 765-771). Différents travaux ont permis de suggérer des fonctions uniques dans l'épissage alternatif des pré-ARNm pour des protéines SR particulières, d'autant qu'elles sont exprimées différentiellement dans une variété de tissus. Ces protéines SR sont ainsi présentées comme cruciales dans la régulation de l'épissage au cours du développement et de la différentiation des cellules (Zahler et al, Science 1993 Apr 9;260(5105):219-222 ; Fu, 1993, Nature, 365(6441):82-8 ; Cáceres et Krainer,1997, (ed. Krainer), Oxford University Press, Oxford, UK, pp. 174-212; Valcarcel et Green, 1996, Trends Biochem Sci, 21(8):296-301). Une étude récente montre un niveau d'expression variable de SRp75 dans différentes lignées cellulaires lymphoïdes (Dam et al, 1999, Biochim Biophys Acta ; 1446(3):317-33.
   A ce jour, aucun rôle dans la régulation de l'angiogènese n'est décrit ni pour SFRS4 ou SRp75 ni pour la protéine homologue de ce facteur.
- GS-N20: un ARNm de 5801 pb identifié sous le numéro SEQ ID No 21 dans la liste de séquences en annexe. Une recherche BLAST permet de l'identifier sous le numéro d'accession U65090 (Carboxypeptidase D) dans la base de séquences GENBANK
   La séquence de cet ARNm a une région codante du nucléotide 36 au nucléotide 4169. Une protéine GS-P21, issue de la traduction directe de cet ARNm, présentée sous le numéro SEQ ID No 72 dans la liste de séquences en annexe a donc été identifiée. Cette protéine appelée Carboxypeptidase D est composée de 1377 aa.
   La carboxypeptidase D (CPD de la famille S10 des sérines peptidases) est une protéine transmembranaire (180 kDa) qui mature les protéines dans le réseau trans-golgien et notamment les protéines sécrétées via la voie constitutive comme les facteurs de croissance et leurs récepteurs : récepteur de l'insuline, récepteur insuline-like des facteurs de croissance (Reznik et al, 1998, J ; Histochem. Cytochem., 46, 1359). C'est une carboxypeptidase avec une activité identique à la carboxypeptidase E (CPE-like) qui est largement distribuée dans les tissus. Les carboxypeptidases interviennent en éliminant les acides aminés basiques de la partie C-terminale du peptide pour générer soit le produit bioactif soit le précurseur pour la formation du groupement amide C-terminal (Fricker, 1988, Annu. Rev. Physiol., 50, 309-321; Fricker, 1991, (ed) *Peptide Biosynthesis and Processing,* pp. 199-230, CRC Press, Boca Raton, FL).
   LA CPD est constituée, chez l'homme, de trois domaines carboxypoeptidase-like, d'un domaine transmembranaire et d'une petite queue cytosolique de 58 résidus (Novikova et al, 1999, J. Biol. Chem., 274, 28887) capable de lier la Protéine Phosphatase A (PP2A) (Varlamov et al, 2001, J. Cell Science, 114, 311). C'est une protéine hautement conservée entre les espèces avec des propriétés enzymatiques similaires.
   La CPD est grandement exprimée dans le placenta humain. On la trouve notamment dans les cellules endothéliales, les trophoblastes, les cellules épithéliales amniotiques, les cellules à villosités endothéliales chorioniques ou les cellules vasculaires de muscle lisse des cordons ombilicaux (Reznik et al, 1998, J ; Histochem. Cytochem., 46, 1359). CPE et CPD sont également impliquées dans l'élaboration du précurseur de l'endothéline 1 (ET-1). Ceci suggère que la CPE et la CPD sont impliquées dans l'élaboration de certains peptides ombilicaux et du placenta ayant des fonctions autocrine et/ou paracrine.
   A ce jour, aucun rôle régulateur de la protéine CPD dans l'angiogènese n'a été décrit.
- GS-N21: un ARNm de 8171 pb identifié sous le numéro SEQ ID No 22 dans la liste de séquences en annexe. Une recherche BLAST permet de l'identifier sous le numéro d'accession NM_004652 (Protéase 9 spécifique de l'ubiquitine) dans la base de séquences GENBANK
   La séquence de cet ARNm a une séquence codante du nucléotide 60 au nucléotide 7751. Il a été ainsi identifié une protéine résultante de la traduction de cet ARNm. Cette protéine appelée GS-P21, Protéase 9 spécifique de l'ubiquitine, est composée de 2563 aa. Elle est identifiée sous le numéro SEQ ID No 73 dans la liste de séquences en annexe
   La protéine USP9X appartient à la famille des UBPs (Ubiquitine protéases), groupe d'enzymes dont la fonction est d'inverser la réaction d'ubiquitination en enlevant le résidu ubiquitine de nombreux substrats impliqués dans la division cellulaire, la croissance, la différentiation, la signalisation ou l'activation de la transcription (Liu et al, 1999, Mol Cell Biol, 4, 3029-38 ;Zhu et al, 1996, Proc. Natl. Acad. Sci. USA 93:3275-3279 ;Verma et al, 1995, Genes Dev. 9:2723-2735) L'ubiquitination des protéines est un important phénomène de régulation des voies biologiques telles que la transduction activée par les cytokines *(Baek et al, 2001, Blood, 98, 636-642).* Les UBPs sont caractérisées par un domaine coeur conservé avec des séquences divergentes environnantes et plus particulièrement à la partie N-terminale permettant la spécificité de substrat. Ces divergences en N terminale peuvent altérer la localisation et conférer des fonctions multiples aux différents membres de la grande famille des UBPs (Lin et al, 2001, J Biol Chem, 276(23):20357-63). Certaines protéases spécifiques de l'ubiquitine ont déjà été décrites ou suggérées impliquées dans certains processus biologiques, tels que l'UBP109 dans le développement embryonnaire (Parket al, 2000, Biochem J, ;349, 443-53), l'UBP43 dans la différentiation des cellules hématopoïétiques (Liu et al, 1999, Mol Cell Biol, 4, 3029-38). La voie de l'ubiquitination est impliquée dans l'angiogènese (Ravi et al, 2000, 14, 34-44 ; Sutter et al, Proc. Natl. Acad. Sci. USA, 97, Issue 9, 4748-4753) mais l'USP9X n'a jamais décrit à ce jour comme régulateur de l'angiogènese.
- GS-N22: un ARNm de 3851 pb identifié sous le numéro SEQ ID No 23 dans la liste de séquences en annexe. Une recherche BLAST permet de l'identifier sous le numéro d'accession NM_002525 (Nardilysine (N-arginine dibasic convertase) (NRD1)) dans la base de séquences GENBANK.
   La séquence de cet ARNm a une séquence codante du nucléotide 136 au nucléotide 3795. Il a été ainsi identifié une protéine GS-P22 résultante de la traduction de cet ARNm. Cette protéine Nardilysine est composée de 1219aa. Elle est identifiée sous le numéro SEQ ID No 74 dans la liste de séquences en annexe.
   La N-arginine dibasic convertase (NRD convertase; nardilysine ; EC 3.4.24.61) est une métalloendopeptidase de la famille des insulinases qui clive spécifiquement les peptides (particulièrement des peptides neuroendocrines tels que la somatosatine-28, la dynorphine-A, le facteur atrial natriuretique) du côté N-terminal d'un résidu Arginine au niveau des sites dibasiques *in vitro* (Cohen et al, 1995, Methods Enzymol., 248, 703-716; Hospital et al, 1997, Biochem. J., 327, 773-779). *In vivo,* sa fonction exacte demeure encore mal connue mais beaucoup d'enzymes de la même famille sont impliquées dans la maturation des prohormones et des proprotéines (Winter et al, 2000, Biochem. J., 351, 755-764). L'activité NRD convertase est présente principalement dans les tissus endocrines et majoritairement dans les testicules (Chesneau, et al, 1994, J. Biol. Chem., 269, 2056-2061). Elle peut être localisée à la fois dans le cytoplasme et à la surface cellulaire (Hospital et al, 2000, Biochem. J., 349, 587-597).
   Actuellement peu de choses sont connues sur la régulation de l'expression et de l'activité de la NRD convertase. L'activité semble être régulée par les amines qui lient le domaine acide (stretch) de l'enzyme (Csuhai et al, 1998, Biochemistry,37(11):3787-94). L'acide rétinoique a également été montré comme pouvant moduler l'expression de l'enzyme dans les lignées neuroblastiques humaines (Draoui et al, 1997, J. Neurooncol., 31, 99-106).
   Chez le rat adulte, l'expression régulée de la NRD convertase durant la spermatogénèse et sa concentration dans le flagelle suggère un rôle de cette enzyme dans la différentiation des cellules germinales mâles (Chesneau, et al, 1996, J. Cell Sci. 109, 2737-2745). Cette enzyme est également proposée jouer un rôle spécifique dans le développement neuronal (Fumagalli et al, 1998,Genomics 47, 238-245). Récemment, la NRD convertase a été décrite comme un nouveau récepteur spécifique du facteur de croissance type EGF liant l'héparine (HB-EGF) qui contrôle la migration cellulaire (Nishi et al, 2001, EMBO J,20(13):3342-50).
   Par contre, aucun rôle ne lui a été découvert, à ce jour, dans la régulation de l'angiogènese
- GS-N23: un ARNm de 13107 pb identifié sous le numéro SEQ ID No 24 dans la liste de séquences en annexe. Une recherche BLAST permet de l'identifier sous le numéro d'accession XM_016303(ARNm de myeloid/lymphoid or mixed-lineage leukemia(MLL)) dans la base de séquences GENBANK.

La séquence d'ARNm identifiée par le numéro SEQ ID No 24 a une séquence codante du nucléotide 1872 au nucléotide 10001. Il a été ainsi identifié une protéine GS-P23 résultante de la traduction de cet ARNm. Cette protéine appelée MLL est composée de 2709aa. Elle est identifiée sous le numéro SEQ ID No 75 dans la liste de séquences en annexe.
Cette séquence GS-N23 présente au moins 86% d'homologie avec les séquences suivantes :
- GS-N47: un ARNm de 14255 bp identifié sous le numéro SEQ ID No 48 dans la liste de séquences en annexe. Une recherche BLAST permet de l'identifier sous le numéro d'accession L04731 dans la base de séquences GENBANK.
   Cette séquence d'ARNm n'a pas de séquence codante. Elle comprend la séquence GS-N23, avec une homologie de 90%.
- GS-N48: un ARNm de 11910 bp identifié sous le numéro SEQ ID No 49 dans la liste de séquences en annexe. Une recherche BLAST permet de l'identifier sous le numéro d'accession NM_005933 dans la base de séquences GENBANK.
   La séquence d'ARNm identifiée par le numéro SEQ ID No 48 a une séquence codante du nucléotide 1 au nucléotide 11910. Il a été ainsi identifié une protéine GS-P48 résultante de la traduction de cet ARNm. Cette protéine appelée MLL est composée de 3969aa. Elle est identifiée sous le numéro SEQ ID No 99 dans la liste de séquences en annexe.
   Le gène de la leucémie lymphoblastique aiguë-1 (ALL)-1 ou Myeloid-lymphoid or Mixed-lineage Leukemia (MLL) ou encore appelé human tri-thorax (HRX) sur le chromosome humain11, bande q23 est le site de beaucoup d'altérations chromosomales regroupées localement (délétions, duplications partielles, translocations) associées avec plusieurs types de leucémies. Les protéines structurellement variantes dérivées du gène altéré est présumé causer la transformation maligne des cellules hématopoiétiques progénitrices (Nilson, Br J Haematol,1996, 93(4):966-72; Kobayashi et al, 1995, Leuk Lymphoma, 17(5-6):391-9).
   La protéine MLL est une grosse protéine nucléaire avec des motifs en doigt de zinc et domaine SET, hautement conservé de 200aa localisé dans sa partie C-terminale. Cette protéine est largement exprimée pendant l'embryogenèse ; des études ont montré que cette protéine est un régulateur positif des gènes homeobox *Hox* (Yu, et al, 1995, *Nature (London)* 378, 505-508). La protéine MLL est décrite comme étant impliquée dans la maintenance transcriptionnelle dans le développement qui fonctionne dans des processus morphogénétiques multiples (Yu et al, 1998, Vol. 95, Issue 18, 10632-10636) . La protéine MLL est suggérée jouer un rôle dans la régulation à la fois de la prolifération cellulaire et la survie dans l'embryon en développement (Hanson et al, 1999, Proc. Natl. Acad. Sci. USA, 96, Issue 25, 14372-14377).
   A ce jour aucun rôle n'a été décrit pour la protéine MLL dans la régulation de l'angiogènese.
- GS-N24: un ARNm de 10330 identifié sous le numéro SEQ ID No 25 dans la liste de séquences en annexe. Une recherche BLAST permet de l'identifier sous le numéro d'accession U72937 (Helicase et ATPase dépendante de l'ADN (ATRX), produit 2 de l'épissage alternatif) dans la base de séquences GENBANK.
   Cette séquence est homoloque, avec au moins 95% d'identité de la séquence:

- GS-N49: un ARNm de 10452 bp identifié sous le numéro SEQ ID No 50 dans la liste de séquences en annexe. Une recherche BLAST permet de l'identifier sous le numéro d'accession U72936 dans la base de séquences GENBANK.
   La séquence d'ARNm identifiée sous le numéro SEQ ID No 25 a une séquence codante du nucléotide 216 au nucléotide 7694. Il a été ainsi identifié une protéine GS-P24 résultante de la traduction de cet ARNm. Cette protéine appelée ATRX produit 2 est composée de 2492 aa. Elle est identifiée sous le numéro SEQ ID No 76 dans la liste de séquences en annexe.
   La séquence d'ARNm identifiée par le numéro SEQ ID No 50 a une séquence codante du nucléotide 950 au nucléotide 7816. Il a été ainsi identifié une protéine GS-P49 résultante de la traduction de cet ARNm. Cette protéine appelée ATRX produit 1 est composée de 2288aa. Elle est identifiée sous le numéro SEQ ID No 100 dans la liste de séquences en annexe.
   Les protéines identifiées par les numéros SEQ ID No 76 et 100 sont homologues à 90%.
   Depuis son identification en 1995, le gène ATRX (synonymes XNP, XH2) a été montré être le gène de nombreuses formes de maladies, différents syndromes de retard mental associé au chromosome X sont liés à des mutations de ce gène.(Revue: Gibbons et Higgs, 2000, Am J Med Genet,97(3):204-12). Ce gène code pour une protéine du sous-groupe SNF2 de la superfamille des hélicases et ATPases (Picketts et al, 1996, Hum Mol Genet 5 (12):1899-907), ces domaines suggèrent que la protéine ATRX a un rôle dans la régulation transcriptionnelle via un effet sur la structure et/ou la fonction de la chromatine, mais son rôle exacte n'est pas encore connu. Aucun rôle dans la régulation de l'angiogènese n' a été décrit à ce jour.
- GS-N25: un ARNm de 1777 pb identifié sous le numéro SEQ ID No 26 dans la liste de séquences en annexe. Une recherche BLAST permet de l'identifier sous le numéro d'accession NM_006476 (Transporteur de l'acide sialique-CMP, membre 1 (SLC35A1))dans la base de séquences GENBANK.
   La séquence de cet ARNm a une séquence codante du nucléotide 28 au nucléotide 1041. Il a été ainsi identifié une protéine GS-P25 résultante de la traduction de cet ARNm. Cette protéine appelée Transporteur de l'acide sialique-CMP est composée de 337a. Elle est identifiée sous le numéro SEQ ID No 77 dans la liste de séquences en annexe.
- GS-N50: un ARNm de 1874 bp identifié sous le numéro SEQ ID No 225 dans la liste de séquences en annexe. Une recherche BLAST permet de l'identifier sous le numéro d'accession BC008372 dans la base de séquences GENBANK. La séquence d'ARNm identifiée par le numéro SEQ ID No 225 n'a pas de séquence codante.
   Le transporteur de CMP-acide sialique est impliqué dans le processus de maturation de la glycosylation et plus particulièrement la sialylation ; il permet la translocation de l'acide CMP-sialique cytosolique à travers la membrane de l'appareil de Golgi nécessaire pour la sialylation des protéines membranaires ou sécrétées aussi bien que les lipides dans ce compartiment. (Hirschberg et Snider, 1987, Annu. Rev. Biochem., 56, 63-88; Hirschberg, 1996, in *Organellar Ion Channels and Transporters Society of General Physiologist, 49th Annual Symposium* (Clapham, D. E., and Ehrlich, B. E., eds), pp. 105-120, Rockefeller University Press, New York). La régulation du transport du CMP- acide sialic est encore mal connue si ce n'est qu'une augmentation de sialylation a été observée à la surface des cellules tumorales (Santer et al, 1989, Eur. J. Biochem., 181, 249-260; Saitoh et al, 1992, J. Biol. Chem., 267, 5700-5711; Bresalier et al, 1996, Gastroenterology, 110, 1354-1367; Gorelik et al, 1997, Cancer Res. 57, 332-336) et que l'inhibition du transporteur de CMP-acide sialic réduit la croissance et les métastases des cellules tumorales (Harvey, B. E., and Thomas, P. (1993) Biochem. Biophys. Res. Commun. 190, 571-575 ). Mais à ce jour, aucune implication de ce transporteur n'a été rapportée dans la régulation de l'angiogènese.
- GS-N26: un ARNm de 3982 pb identifié sous le numéro SEQ ID No 27 dans la liste de séquences en annexe. Une recherche BLAST permet de l'identifier sous le numéro d'accession U26710 dans la base de séquences GENBANK.
   La séquence de cet ARNm a une séquence codante du nucléotide 323 au nucléotide 3271. Il a été ainsi identifié une protéine GS-P26 résultante de la traduction de cet ARNm. Cette protéine appelée cbl-b est composée de 982aa.
   Elle est identifiée sous le numéro SEQ ID No 78 dans la liste de séquences en annexe.
   Cbl-b appartient à la famille des Cbl, hautement conservée à travers les espèces. Les protéines Cbl sont caractérisées dans leur partie N-terminale par un domaine putatif liant les phosphotyrosines et un motif doigt de RING dans la partie C-terminale, les protéines Cbl de mammifères contiennent une région riche en proline, des résidus tyrosines conservés et un motif leucine zipper. Les protéines Cbl participent à la signalisation des protéines des récepteurs à tyrosine kinase, aussi bien que les antigènes et les récepteurs des cytokines en s'associant à leur queue cytoplasmique assurant la continuité du signal de ces récepteurs. La protéine Cbl est recrutée par le module tyrosine kinase de ces récepteurs et les tyrosines phosphorylées après l'activation cellulaire. Cbl fonctionne comme une protéine d'arrimage et s'associe avec les molécules contenant les domaines SH2 et SH3, incluant la famille des adaptateurs Crk et Vav. Les protéines Cbl sont proposées aussi bien comme des régulateurs négatifs de la signalisation des récepteurs tyrosines kinases (Smit et al, Crit Rev Oncog 1997;8:359-79) que des modulateurs positifs de la signalisation de récepteurs tels que la superfamille des récepteurs de TNF (Arron et al, J Biol Chem 2001 Aug 10;276:30011-7).
   La protéine Cbl-b, largement exprimée dans beaucoup de tissus et cellules incluant les cellules hématopoïétiques (Keane et al, Oncogene 1995 Jun 15;10(12):2367-77), est impliquée dans la mise en place du seuil d'activation des lymphocytes (Bachmaier et al, Nature 2000, 403:211-6; Fang et al, J Biol Chem 2001;276:4872-8). La sous unité régulatrice P85 de la phosphatidylinositol 3-kinase (PI3K) a été identifiée comme étant son substrat. La Cbl-b, par son activité ligase de la protéine ubiquitine E3 régule négativement cette sous unité régulatrice P85 (Fang D, Nat Immunol 2001 Sep;2(9):870-5). Cbl-b est également un régulateur négatif de la signalisation du récepteur du facteur de croissance épidermal, EGFR (Ettenberg et al, Oncogene 1999;18:1855-66; Ettenberg et al, J Biol Chem 2001;276:27677-84).
   Par contre à ce jour, il n'a pas été décrit que Cbl-b joue un rôle dans la régulation de l'angiogènese.
- GS-N27: un ARNm de 3385 pb identifié sous le numéro SEQ ID No 28 dans la liste de séquences en annexe. Une recherche BLAST permet de l'identifier sous le numéro d'accession XM_39529 dans la base de séquences GENBANK.

Cette séquence présente 86% d'homologie avec la séquence suivante :
- un ADNc (DKFZp564D173) de 4461 pb identifié sous le numéro SEQ ID No 51 dans la liste de séquences en annexe. Une recherche BLAST permet de l'identifier sous le numéro d'accession AL110212 dans la base de séquences GENBANK. Il n'y a pas de séquence codante correspondante.
   La séquence d'ARNm identifiée sous le numéro SEQ ID No 27 a une séquence codante du nucléotide 107 au nucléotide 451. Il a été ainsi identifié une protéine GS-P27 résultante de la traduction de cet ARNm. Cette protéine appelée Histone H2A.F/Z variant (H2AV) est composée de 114aa. Elle est identifiée sous le numéro SEQ ID No 79 dans la liste de séquences en annexe.
   L'unité de base de la chromatine chez les eucaryotes est le nucléosome. Un nucléosome est constitué d'une séquence d'ADN de 146pb enroulée autour d'un octamère de protéines, les histones H2A, H2B, H3, H4 (Lugeret al, 1997, Nature, 389, 251-260). L'hétérogénéité dans la structure des nucléosomes peut être un mécanisme de régulation transcriptionnelle. Cette hétérogénéité est créée soit par modifications post-traductionnelles des histones telles que l'acétylation, la phosphorylation, la méthylation, l'ubiquitination (Mizzen et al, Cold Spring Harb Symp Quant Biol 1998;63:469-81 ; Workman et Kingston, 1998, Annu. Rev. Biochem., 67:545-579) soit l'incorporation de variants d'histones dans le nucléosome. Les différentes variantes d'histones permettent la spécialisation de la structure du nucléosome dans des buts spécifiques ; les variantes d'histones spécifiques du sperme, par exemple, facilitent la compaction dramatique de l'ADN qui se produit lors de la spermatogénèse (Wolffe, 1998, *Chromatin:* *Structure and Function ,* 3rd Ed. , Academic Press, San Diego ; Doenecke et al, 1997, Adv. Exp. Med. Biol., 424, 37-48).
   L'histone H2A.F/Z est une famille de variants de l'histone H2A qui est hautement conservée à travers les espèces et substantiellement divergente de l'histone H2A de la phase S dans des espèces données (Jackson et al, 1996, Trends Biochem. Sci., 221, 466-467 ; Jiang et al, 1998, Biochem. Biophys. Res. Commun., 245, 613-617 ). La fonction exacte de H2A.F/Z n'est pas encore connue, mais cette histone peut jouer un rôle dans la régulation transcriptionnelle car chez *Tetrahymena,* son expression est associée avec le macronoyau actif transcriptionnellement et chez *Drosophila* son incorporation dans la chromatine pendant le développement coïncide avec le début de l'expression du gène zygotique (Clarkson et al, 1999, DNA Cell Biol., 18, 457-462 ; Stargell et al, 1993, Genes Dev., 7, 2641-2651).
   Par contre, à ce jour, aucun rôle n'est connu pour l'histone H2A.F/Z dans la régulation de l'angiogènese.
- GS-N28: un ARNm de 1128 pb identifié sous le numéro SEQ ID No 29 dans la liste de séquences en annexe . Une recherche BLAST permet de l'identifier sous le numéro d'accession NM_001320 dans la base de séquences GENBANK.
   Cette séquence a 77% d'homologie avec la séquence identifiée sous le numéro d'accession M30448 dans la base GENBANK et identifiée sous le numéro SEQ ID No. 289 dans la liste de séquences en annexe.
   La séquence de cet ARNm a une séquence codante du nucléotide 97 au nucléotide 744. Il a été ainsi identifié une protéine GS-P28 résultante de la traduction de cet ARNm. Cette protéine appelée Caseine kinase II, sous unité Beta est composée de 215 aa. Elle est identifiée sous le numéro SEQ ID No 80 dans la liste de séquences en annexe.
   La caséine kinase II (CKII) est une serine/thréonine kinase ubiquitaire qui est localisée aussi bien dans le cytosol que dans le noyau des cellules eucaryotes. La CKII phosphoryle plus de cent substrats, parmi lesquels beaucoup sont impliqués dans le contrôle de la division cellulaire et dans la transduction du signal (revue : Allende et Allende, 1995, The FASEB Journal, Vol 9, 313-323). La CKII existe sous forme d'un tétramère composé de deux sous unités catalytiques alpha et/ou alpha' et deux sous unités régulatrices (béta). La sous unité béta semble agir pour stabiliser les sous unités alpha et/ou alpha' et influencer également la spécificité du substrat et les propriétés de cinétique de l'enzyme (Dobrowolska et al, 1999, Mol Cell Biochem, 191(1-2):3-12). Certaines études ont montré que l'activité de la caséine kinase est stimulée par des hormones ou facteurs de croissance tels que l'insuline, l'IGF-I, l'EGF (Sommercorn et al, 1987, Proc. Natl Acad Sci USA, 84, 8834-8838; Klarlund et al, 1988, J.Biol. Chem, 263, 1872-15875 ; Ackerman et Osheroff, 1989, J. Biol. Chem., 264, 11958- 11965), cette activation résultant d'une augmentation de la phosphorylation de la sous unité béta de la caséine kinase (Ackerman et al, 1990, Proc. Natl Acad Sci USA, 87, 821-825). Différentes études ont montré une expression dérégulée de la CKII dans les tumeurs. Des récentes études ont mis en évidence que la surexpression de la CKII dans les cellules de tumeurs n'est pas seulement un reflet de la prolifération des cellules de tumeurs mais aussi elle peut refléter les caractéristiques pathobiologiques de la tumeur. La dérégulation de la CKII pourrait influencer l'activité apoptotique de ces cellules (revue : Tawfic et al, 2001, Histol Histopathol, 16(2):573-82). Cette enzyme est décrite comme ayant un rôle possible dans l'oncogènese (Yu et al, J Cell Biochem, 1999,74(1):127-34).
   Par contre, aucune expression différentielle de la CKII ou de sa sous unité béta au cours de l'angiogènese n'a été décrite ni un rôle dans la régulation de l'angiogènese n'a été démontré.
- GS-N29: un ARNm de 18207 bp identifié sous le numéro SEQ ID No 30 dans la liste de séquences en annexe . Une recherche BLAST permet de l'identifier sous le numéro d'accession AF156100 dans la base de séquences GENBANK.
   La séquence de cet ARNm a une séquence codante du nucléotide 230 au nucléotide 17140. Il a été ainsi identifié une protéine GS-P29 résultante de la traduction de cet ARNm. Cette protéine appelée Hemicentine est composée de 5636aa. Elle est identifiée sous le numéro SEQ ID No 81 dans la liste de séquences en annexe.
   Cette séquence GS-29 comprend la séquence GS-N52 ci-dessous, présentant une homologie de 99% avec celle-ci.
   GS-N52: un ARNm de 8546 bp identifié sous le numéro SEQ ID No 52 dans la liste de séquences en annexe . Une recherche BLAST permet de l'identifier sous le numéro d'accession AJ306906 dans la base de séquences GENBANK.
   La séquence de cet ARNm a une séquence codante pour une protéine GS-P52 de 2673aa identifiée sous le numéro SEQ ID No 101 dans la liste de séquences en annexe .
   Décrite récemment, l'hémicentine est une protéine de la matrice extracellulaire de la superfamille des Immunoglobulines, qui est impliquée dans l'attachement et la migration cellulaire sur la membrane basale (Vogel et Hedgecock, 2001,Development, 128(6):883-94). Son rôle dans la régulation de l'angiogènese n'est pas décrit à l'heure actuelle. Cette protéine contient la séquence de la fibulin-6 qui appartient à la famille des fibulines, protéines de la matrice extracellulaire et du sang dont les deux membres les plus étudiés sont la fibuline 1 et la fibulin 2 (Alexande et Detwiler, 1984, Biochem. J., 217,67-71 ; Argraves, et al, 1990, J. Cell Biol., 111,3155-3164; Kluge et al, 1990, Eur. J. Biochem., 193,651-659; Pan et al, 1993, J. Cell Biol., 123,1269-1277). Elles interagissent avec des protéines impliquées dans l'adhésion cellulaire comme la fibronectine, la laminine, le fibrinogène (Brown et al, 1994, J Cell Sci,107 ( Pt 1), 329-38 ; Tran et al, 1995, J Biol Chem, 270(33):19458-64 ; Godyna et al, 1995, Matrix Biol, 14(6):467-77) ou encore l'endostatine qui est un inhibiteur de l'angiogènese (Sasaki et al, 1998, EMBO J,17(15):4249-56) ce qui leur confère une fonction régulatrice dans divers processus biologiques. La fibuline 1, par exemple, a été décrite pouvant jouer un rôle dans la régulation de l'activité neurotrophique de la protéine précurseur amyloïde béta (Ohsawa et al, 2001, J Neurochem; 76(5):1411-20), dans l'homéostase et la thrombose (Tran et al, 1995, J Biol Chem, 270(33):19458-64).
   Par contre, la fonction de la fibuline 6 n'est pas encore connu, et en particulier, aucun rôle de cette protéine n'a été décrit jusqu'à nos jours dans la régulation de l'angiogènese.
- GS-N30: un ARNm de 4325 pb identifié sous le numéro SEQ ID No 31 dans la liste de séquences en annexe . Une recherche BLAST permet de l'identifier sous le numéro d'accession NM_015180 dans la base de séquences GENBANK.
   La séquence de cet ARNm a une séquence codante du nucléotide 123 au nucléotide 3041. Il a été ainsi identifié une protéine GS-P30 résultante de la traduction de cet ARNm. Cette protéine appelée SYNE-2 est composée de 1092aa.Elle est identifiée sous le numéro SEQ ID No 82 dans la liste de séquences en annexe.
   La protéine Syne-2 est mal connue, elle a été décrite récemment avec une protéine homologue, la protéine Syne-1 (synaptic nuclear envelope-1). La protéine Syne-1 est associée avec les enveloppes nucléaires dans les cellules du muscle lisse, cardiaque et squelettique. Syn-1 est décrite comme la première protéine trouvée être associée sélectivement avec le noyau synaptique et est suggérée être impliquée dans la formation ou la maintenance des agrégats nucléaires dans la jonction musculaire (Appel et al, 2000, J. Biol. Chem., Vol. 275, Issue 41, 31986-31995). Syn-2 diffère de Syn-1 dans la distribution et par son niveau d'expression qui est plus faible. Les deux protéines homologues montrent des domaines répétés de la spectrine qui sont présents dans différentes protéines impliquées dans la structure du cytosquelette (Yan et al, Science 1993 Dec 24;262(5142):2027-30).
   A ce jour, aucun rôle de Syn-2 n'est décrit dans la régulation de l'angiogènese.
- GS-N31: un ARNm de 4248 bp identifié sous le numéro SEQ ID No 32 dans la liste de séquences en annexe. Une recherche BLAST permet de l'identifier sous le numéro d'accession AF261758 dans la base de séquences GENBANK.
   La séquence de cet ARNm a une séquence codante du nucléotide 100 au nucléotide 1650. Il a été ainsi identifié une protéine GS-P31 résultante de la traduction de cet ARNm. Cette protéine appelée Seladine-1 est composée de 516aa. Elle est identifiée sous le numéro SEQ ID No 83 dans la liste de séquences en annexe.
   Cette séquence d'ARNm identifiée sous le numéro SEQ ID No 32 dans la liste de séquences en annexe est homologue d'une séquence de 4187 bp. Une recherche BLAST permet de l'identifier sous le numéro d'accession D13643 dans la base de séquences GENBANK. Elle est identifiée sous le numéro SEQ ID No 53 dans la liste de séquences en annexe.
   Cet ARNm présente une séquence codante partielle. Une protéine GS-P53 de 528aa résultante de la traduction de cet ARNm est ainsi identifiée. Elle est identifiée sous le numéro SEQ ID No 102 dans la liste de séquences en annexe.
   Le gène seladin-1 a récemment été identifié dans le cerveau humain. La séladine-1 semble être un important facteur pour la protection des cellules contre la toxicité du peptide béta-amyloide et le stress oxydatif (Greeve et al, 2000, J Neuroscience, 20(19):7345-7352). Ces auteurs suggèrent que la séladine-1 peut être impliquée dans la régulation de la survie et la mort cellulaire et que l'expression diminuée de cette protéine dans des neurones spécifiques peut être une cause pour la vulnérabilité sélective dans la maladie D'Alzheimer.
   Par contre, à ce jour aucun rôle dans la régulation de l'angiogènese n'a été décrit pour la séladine-1.
- GS-N32: un ARNm de 7764 bp identifié sous le numéro SEQ ID No 33 dans la liste de séquences en annexe . Une recherche BLAST permet de l'identifier sous le numéro d'accession NM_001271 dans la base de séquences GENBANK.
   La séquence de cet ARNm a une séquence codante du nucléotide 708 au nucléotide 5927. Il a été ainsi identifié une protéine GS-P32 résultante de la traduction de cet ARNm. Cette protéine appelée CHD2 est composée de 1739aa. Elle est identifiée sous le numéro SEQ ID No 84 dans la liste de séquences en annexe.
   La protéine CHD2 appartient à la famille des CHD protéines caractérisées par un chromodomaine. Le "chromo" (CHRromatin Organization Modifier) domaine est une région conservée d'environ 60 acides aminés trouvée dans une variété de protéines incluant les protéines HP1 de *Drosophila melanogaster*, qui se lie à l'hétérochromatine; 4 gènes de cette famille ont été identifiés dans le génome humain: CHD1, CHD2, CHD3 and CHD4 (Woodage et al., 1997, 94, 11472-11477). Le chromodomaine confère à la protéine un rôle dans la compaction de la chromatine (Paro, 1990, Trends Genet. 6:416-421; Singh et al, 1991, Nucleic Acids Res. 19:789-794; Aasland et Stewart, 1995,Nucleic Acids Res. 23:3168-3173; Koonin et al, 1995, Nucleic Acids Res. 23:4229-4233, Messner et al, 1992, Genes dev.,6,1241-1254; James et Elgin, 1986, Mol. Cell.Biol,6,3862-3872). Les CHD contient un deuxième domaine conservé, le domaine Myb qui est impliqué dans la liaison avec l'ADN (Klempnauer et Sippel, 1987, EMBO J., 6:2719-2725). En plus de ces domaines, CHD2 contient le domaine SNF2, trouvé dans des protéines impliquées dans une variété de processus tels que la régulation de la transcription (ex: SNF2, STH1, brahma, MOT1), réparation de l'ADN (ex: ERCC6, RAD16, RAD5), recombinaison de l'ADN (e.g., RAD54)(revue: Eisen et al, 1995, Nucleic Acids Res , 23(14):2715-23) et enfin un domaine conservé de la superfamille des hélicases, les "DEAH box hélicases". Les hélicases sont impliquées dans le débobinement des acides nucléiques (Matson et Kaiser-Roger,.1990, *Annu. Rev. Biochem.* 59, 289-329). Des « DEAH box » hélicases ont été proposées comme étant impliquées dans l'épissage des ARNm et dans la progression du cycle cellulaire (Ludgren et al , 1996, *Mol. Biol. Cell* 7, 1083-1094; Imamura et al, 1998, Nucleic Acids Res , 26(9):2063-8).
   Les CHD peuvent jouer un rôle important dans la régulation de la transcription des gènes. (Delmas et al, 1993, Proc. Natl. Acad. Sci., 90, 2414-2418; Woodage et al, 1997, *Proc. Natl. Acad. Sci. USA,* 94, 11472-11477; Tran et al, 2000, The EMBO Journal, 19,2323-2331)
   Une récente étude a montré que CHD4 est induit lorsque les cellules endothéliales sont stimulées par le TNF-alpha (Murakami et al, 2000, J Atheroscler Thromb;7(1):39-44).
   Aucune étude ne montre une implication de CHD2 dans la régulation de l'angiogènese.
- GS-N33 : un ARNm de 4693 pb identifié sous le numéro SEQ ID No 34 dans la liste de séquences en annexe . Une recherche BLAST permet de l'identifier sous le numéro d'accession XM_042555 dans la base de séquences GENBANK.
   La séquence de cet ARNm a une séquence codante du nucléotide 1702 au nucléotide 4107. Il a été ainsi identifié une protéine GS-P33 résultante de la traduction de cet ARNm. Cette protéine appelée BRD2 est composée de 801aa. Elle est identifiée sous le numéro SEQ ID No 85 dans la liste de séquences en annexe.
   Le rôle de la protéine BRD2 n'est pas connu. Cette protéine se caractérise par deux bromodomaines. Le bromodomaine est une région conservée, identifiée la première fois comme une signature de 61-63 acides aminés, sa fonction n'étant pas connue (Haynes et al, Nucl. Acids Res., 1992, 20, 2603). Par la suite, ce domaine a été identifié dans des facteurs de transcription, des co-activateurs et autres protéines sont impliquées dans la transcription ou le remodelage de la chromatine et ses bornes ont été étendues jusqu'à 110 acides aminés. Le nombre grandissant de protéines contenant ce domaine est plus d'une quarantaine (Jeanmougin et al, 1997, Trends Biochem. Sci. 22, 151 - 153; Winston et Allis,1999, Nature Struct. Biol. 6, 601 - 604). Certaines protéines ont une seule copie du domaine d'autres présentent deux copies du motif. La protéine BRD2 se caractérise par deux bromodomaines comme c'est le cas du facteur de transcription BDF1 (Tamkun , 1995, Curr. Opin. Genet. Dev, 5:473-477) ou encore la protéine TAFII250, la plus large sous-unité du complexe multiprotéique, TFIID impliqué dans l'initiation de la transcription (Jacobson et al, 2000, Science,288(5470):1422-5). La fonction exacte de ce domaine n'est pas encore connue mais il est pensé être impliqué dans les interactions protéine-protéines et peut être important pour l'assemblage ou l'activité des complexes multiples composants impliqués dans la modification de la chromatine et le contrôle transcriptionnel d'une large variété de gènes eucaryotes incluant ceux qui contrôlent la croissance. Une large variété de fonctions dirigées sur la chromatine, incluant mais ne se limitant pas à la phosphorylation, acétylation, méthylation, co-activation ou recrutement transcriptionnelle caractérise les complexes qui contiennent des motifs bromodomaines. Leur versatilité et ubiquité assurent des réponses transcriptionnelles diverses, rapide et flexible (revue:Denis, 2001, Frontiers in Bioscience 6, d849-852).
   L'expression différentielle de protéines contenant un bromodomaine a déjà été montré notamment celle d'une protéine homologue à la RING3 kinase dont l'expression est induite par le VEGF ou le bFGF chez les cellules endothéliales, cette protéine est suggérée comme une nouvelle cible de la voix de signalisation activée par le VEGF et le bFGF qui permet aux cellules endothéliales d'entrer dans la phase proliférative du processus d'angiogènese (BelAiba et al, 2001, Eur J Biochem, 268(16):4398-407).
   Par contre, la protéine BRD2 n'a jamais été décrite impliquée dans la régulation de l'angiogènese.
- GS-N34: un ARNm de 2983 pb identifié sous le numéro SEQ ID No 35 dans la liste de séquences en annexe . Une recherche BLAST permet de l'identifier sous le numéro d'accession BC007429 dans la base de séquences GENBANK.
   La séquence de cet ARNm a une séquence codante du nucléotide 200 au nucléotide 1069. Il a été ainsi identifié une protéine GS-P34 résultante de la traduction de cet ARNm. Cette protéine appelée Syntaxine 3A est composée de 289aa. Elle est identifiée sous le numéro SEQ ID No 86 dans la liste de séquences en annexe.
   La syntaxine 3A appartient à la famille des syntaxines/epimorphines qui se caractérise par une taille entre 30 et 40 kDa, une extrémité C-terminale hautement hydrophobe qui est probablement impliquée dans l'ancrage de la protéine dans la membrane et une région centrale bien conservée qui semble être dans une conformation " coiled-coil" . Le profil spécifique de cette famille est basé sur la région la plus conservée du domaine "coiled-coil". Les syntaxines sont impliquées dans le transport intracellulaire de vésicules et leur arrimage à la membrane plasmique. Des récents travaux suggèrent que différentes isoformes de syntaxines peuvent interagir avec un groupe défini de protéines de transport membranaires et ainsi réguler leur activité de transport (revue: Saxena et al, 2000, Curr Opin Nephrol Hypertens, 9(5):523-7).
   La syntaxine 3A est une des deux isoformes (3A et 3B) identifiées chez l'homme, issues d'un épissage alternatif d'un même gène. L'augmentation de l'expression de la syntaxine 3A a déjà été mise en évidence au cours de différents processus biologiques comme la différentiation neutrophile des cellules HL-60 ou dans les cellules granulaires de la Dentée (*dentate granule cells*) au cours de la propagation de la plasticité synaptique dans le système nerveux (Rodger et al, 1998, J Neurochem, 71(2):666-675; Martin-Martin et al, 1999, J Leukoc Biol, 65(3):397-406).
   Par contre, à ce jour aucune expression augmentée de la syntaxine 3A n'a été décrite au cours de l'angiogènese et donc aucune implication dans la régulation de l'angiogènese n'a été rapportée.
- GS-N35: un ARNm de 12227pb identifié sous le numéro SEQ ID No 36 dans la liste de séquences en annexe. Une recherche BLAST permet de l'identifier sous le numéro d'accession NM_015001 dans la base de séquences GENBANK.
   La séquence de cet ARNm a une séquence codante du nucléotide 205 au nucléotide 11199. Il a été ainsi identifié une protéine GS-P35 résultante de la traduction de cet ARNm. Cette protéine appelée SHARP est composée de 3664aa. Elle est identifiée sous le numéro SEQ ID No 87 dans la liste de séquences en annexe.
   Sharp (SMART/HDAC1 Associated Repressor Protein) est un gène récemment isolé (Nagase et al, 1999, DNA Res, 6(1):63-70). SHARP est un répresseur potentiel de la transcription dont le domaine de répression (RD) interagit directement avec SMRT et au moins 5 membres du complexe NuRD (« nucleosome remodeling and histone deacetylase activities ») incluant les histones déacétylases HDAC1 et HDAC2. De plus, SHARP se lie au coactivateur ARS de l'ARN du récepteur des stéroïdes par un domaine intrinsèque liant l'ARN et supprime l'activité de transcription du récepteur de stéroïde. De cette façon, SHARP a la capacité de moduler à la fois les récepteurs nucléaires ligués et non ligués. L'expression de SHARP est elle-même inductible par les stéroïdes suggérant un simple mécanisme de retour (« feedback ») pour l'atténuation de la réponse hormonale (Shi et al, Genes Dev 2001 May 1;15(9):1140-51). Les histones déacétylases (HDAC) ont été montrées impliquées dans l'induction de l'angiogènese en supprimant l'expression des gènes suppresseurs de tumeurs (Kim et al, 2001, Nat Med, 7(4):437-43).
   Cependant, aucun rôle de la protéine SHARP n'a été décrit dans la régulation dans l'angiogènese.
- GS-N36: un ARNm de 5376 pb identifié sous le numéro SEQ ID No 37 dans la liste de séquences en annexe. Une recherche BLAST permet de l'identifier sous le numéro d'accession AF352051 dans la base de séquences GENBANK.
   La séquence de cet ARNm a une séquence codante du nucléotide 92 au nucléotide 4962. Il a été ainsi identifié une protéine GS-P36 résultante de la traduction de cet ARNm. Cette protéine appelée Protéine liée au potentiel de prolifération est composée de 1616aa. Elle est identifiée sous le numéro SEQ ID No 88 dans la liste de séquences en annexe.
   Cette séquence identifiée sous le numéro SEQ ID No 37 présente 92% homologie avec la séquence codant pour la protéine RPBB6 (retinoblastoma-binding protein 6) de 948aa identifiée sous le numéro SEQ ID No 288 dans la liste de séquences en annexe. La séquence nucléotidique correspondant à cette protéine comporte 2994bp et le numéro d'accession NM_006910 sur la base de séquences GENBANK.
   Le rôle exact de la Protéine liée au potentiel de prolifération (proliferation potential-related protein) identifiée n'est pas encore connu. Elle présente comme ses protéines homologues des domaines doigt de Zinc connus pour être impliqués dans l'interaction protéines-protéines. Cette protéine est homologue à un membre de la famille des protéines liant les protéines retinoblastoma (pRb), la « retinoblastoma-binding protein 6 (RPBB6) », également appelée RBQ-1 (Sakai et al, 1995, Genomics, 30(1):98-101). La protéine pRb (suppresseur de la tumeur appelée rétinoblastome) agit pour contrôler la proliferation cellulaire, inhiber l'apoptose et induire la différentiation cellulaire et ce en s'associant à un grand nombre de protéines (revue : Morris et Dyson, 2001, Adv Cancer Res;82:1-54).
   Jusqu'à ce jour la protéine liée au potentiel de prolifération n'a jamais été décrite comme étant impliquée dans la régulation de l'angiogènese.
- GS-N37: un ARNm de 6626 bp identifié sous le numéro SEQ ID No 38 dans la liste de séquences en annexe. Une recherche BLAST permet de l'identifier sous le numéro d'accession XM_005338 dans la base de séquences GENBANK.
   La séquence de cet ARNm a une séquence codante du nucléotide 245 au nucléotide 2989. Il a été ainsi identifié une protéine GS-P37 résultante de la traduction de cet ARNm. Cette protéine appelée Protéine HIP1 est composée de 914aa. Elle est identifiée sous le numéro SEQ ID No 89 dans la liste de séquences en annexe.
   La protéine HIP1 (« Huntingtin interacting protein 1 ») est une protéine de 116 kDa qui lie la protéine
   Huntingtine, produit du gène muté dans la maladie de Huntington (Kalchman et al, 1997, Nat. Genet.,16, 44-53; The Huntington's Disease Collaborative Research Group, 1993, Cell 72, 971-983). HIP1 est de façon prédominante exprimée dans le cerveau, mais est également détectée dans d'autres tissus (Wanker et al, 1997, Human Molecular Genetics, Vol 6, 487-495). La fonction de HIP1 n'est pas encore bien connue mais elle partage des caractéristiques biochimiques et domaines conservés avec Sla2p, protéine essentielle pour la fonction du cytosquelette chez Saccharomyces cerevisiae (Kalchman et al.,1997, Nat. Genet. 16, 44-53; Holtzman et al, 1993, J. Cell Biol. 122, 635-644). HIP1 contient également un domaine leucine Zipper et est homologue dans sa partie C-terminale à la taline, protéine du cytosquelette impliquée dans les interactions cellules-substratum et cellules-cellules (Rees et al, 1990, Nature; 347(6294):685-9).Récemment, on a montré que la protéine HIP1 est impliquée dans l'apoptose (Hackam et al, 2000, J. Biol. Chem., Vol. 275, Issue 52, 41299-41308).
   A ce jour, aucun rôle n'a été décrit pour l'HIP1 dans l'angiogènese.
- GS-N38: un ARNm de 2366 bp identifié sous le numéro SEQ ID No 39 dans la liste de séquences en annexe. Une recherche BLAST permet de l'identifier sous le numéro d'accession BC000335 dans la base de séquences GENBANK.
   La séquence de cet ARNm a une séquence codante du nucléotide 12 au nucléotide 2237. Il a été ainsi identifié une protéine GS-P38 résultante de la traduction de cet ARNm. Cette protéine appelée Nucleoporine 88kDa est composée de 741aa. Elle est identifiée sous le numéro SEQ ID No 90 dans la liste de séquences en annexe.
   La nucléoporine 88 (Nup88) est une protéine de la membrane nucléaire probablement impliquée dans le transport nucléocytoplasmique (Fornerod et al, 1997, EMBO J, 16:807-816; Fornerod et al, Oncogene 1996, 13:1801-1808). La Nup88 a été trouvée être associée avec le domaine central de CAN/Nup214, un composant du complexe d'un pore nucléaire impliqué probablement dans l'importation de protéines nucléaires, l'exportation d'ARNm nucléaires et la régulation du cycle cellulaire (van Deursen et al, 1996, EMBO J, 15:5574-5583). La Nup 88 a été montrée largement distribuée et surexprimée dans les cellules et tissus cancéreux et foetaux (Martinez et al, 1999, Cancer Research 59, 5408-5411; Gould et al, 2000, American Journal of Pathology, 157:1605-1613).
   A ce jour, la Nup88 n'a jamais été décrite comme étant impliquée dans la régulation de l'angiogènese.
- GS-N39 :un ARNm de 1543 bp identifié sous le numéro SEQ ID No 40 dans la liste de séquences en annexe. Une recherche BLAST permet de l'identifier sous le numéro d'accession XM_049486 dans la base de séquences GENBANK.
   La séquence de cet ARNm a une séquence codante du nucléotide 86 au nucléotide 412. Il a été ainsi identifié une protéine GS-P39 résultante de la traduction de cet ARNm. Cette protéine appelée Proteine 1A liant FK506 est composée de 108aa. Elle est identifiée sous le numéro SEQ ID No 91 dans la liste de séquences en annexe.
   Les FKBPs (FK506 Binding Protein) sont des protéines majeures liant avec une haute affinité, chez les vertébrés, le médicament immunosuppresseur FK506 (1990, Tropschug et al, Nature 346:674-677; Stein, 1991,Curr. Biol. 1:234-236; Siekierka et al, 1990, J. Biol. Chem. 265:21011-21015). Plusieurs membres de la famille FKBP ont été identifiés dans beaucoup de tissus et dans divers compartiments cellulaires, la plus connue étant la FKBP12, une isoforme cytoplasmique (Galat et Metcalf, 1995, Prog. Biophys. Mol. Biol. 63, 67-118; Kay, 1996, Biochem. J. 314, 361-385). Les FKBPs appartiennent à une grande famille de peptidyl propyl isomérases cis-trans, (PPIase or rotamase). La PPIase est une enzyme qui accélère le repliement de la protéine en catalysant l'isomérisation cis-trans des liaisons peptidiques impliquant le résidu proline (Fischer et Schmid, 1990, Biochemistry 29:2205-2212). Les FKBPs sont connues être impliquées dans beaucoup de processus cellulaires, tels que la signalisation cellulaire, le transport protéique et la transcription. Des études d'interruption du gène codant pour les FKBPs chez les plantes et les animaux ont souligné l'importance de cette famille de protéines dans la régulation de la division cellulaire et la différentiation (revue:Harrar et al , 2001 Trends Plant Sci;6:426-31). Cependant, malgré qu'elles lient des récepteurs de surfaces et malgré leur activité PPiase, leur fonction physiologique reste encore à définir. Récemment, les PPiases ont été proposées jouer un rôle dans le réarrangement fonctionnel des composants dans les hétérocomplexes récepteurs (Schiene-Fischer et Yu, 2001, FEBS Lett, 495(1-2):1-6).
   A ce jour, il n'a pas été constaté que les protéines FKBP soient impliquées dans la régulation de l'angiogènese.
- GS-N40: un ARNm de 3824 pb identifié sous le numéro SEQ ID No 41 dans la liste de séquences en annexe. Une recherche BLAST permet de l'identifier sous le numéro d'accession XM_042827 dans la base de séquences GENBANK.
   La séquence de cet ARNm a une séquence codante du nucléotide 115 au nucléotide 3663. Il a été ainsi identifié une protéine GS-P40 résultante de la traduction de cet ARNm. Cette protéine appelée Proteine SALF est composée de 1182 aa. Elle est identifiée sous le numéro SEQ ID No 92 dans la liste de séquences en annexe.
   L'ADN complémentaire (ADNc) de la protéine SALF (stoned B/TFIIA-alpha/beta-like factor) a été récemment identifié à partir d'une banque d'ADNc issue du placenta humain (Ashok et al, 1999, J Biol Chem, Vol. 274, Issue 25, 18040-18048). Le rôle de SALF n'est pas encore identifié cependant cet ADNc, qualifié de rare est identique à la séquence de la protéine ALF avec une séquence N-terminale plus étendue contenant un domaine homologue à la protéine Stone B de la Drosophile (Andrewe et al , .1996, *Genetics* 143, 1699-1711) et aux protéines adaptateurs de la Clathrine, µ₁ (AP47) et µ₂ (AP50) (Thurieau et al, 1988, *DNA (N. Y.)* 7, 663-669; Nakayama et al,1991, *Eur. J. Biochem.* 202, 569-574). La protéine ALF (TFIIA-alpha/beta-like factor) est également un nouveau facteur, qui est un homologue fonctionnel du facteur de transcription TFIIA alpha/beta qui, avec le facteur TFIIA gamma, peut stabiliser les interactions de l'élément TBP (TATA-binding protein )-TATA et maintenir la transcription *in vitro* dépendante de l'ARN polymérase II.. La protéine ALF est décrite comme un facteur de transcription général spécifiques des testicules (Ashok et al, 1999, J Biol Chem, Vol. 274, Issue 25, 18040-18048).
   Le domaine d'homologie "Stoned B/clathrin AP-like" de SALF révèle également une fonction potentielle dans le transport dépendant de la Clathrine des protéines membranaires.
   Chen et al (2001, Biochem Biophys Res Commun 23;281(2):475-82) ont montré que le gène du facteur SALF est induit par l'acide rétinoique (ou les rétinoides) chez les cellules du muscle lisse cultivées.
   Par contre, aucune implication de SALF dans la régulation de l'angiogènese n'a été décrite à ce jour.
- GS-N41: un ARNm de 1365 pb identifié sous le numéro SEQ ID No 42 dans la liste de séquences en annexe. Une recherche BLAST permet de l'identifier sous le numéro d'accession BC010862 dans la base de séquences GENBANK.
   La séquence de cet ARNm a une séquence codante du nucléotide 27 au nucléotide 758. Il a été ainsi identifié une protéine GS-P41 résultante de la traduction de cet ARNm. Cette protéine est composée de 243 aa et elle est identifiée sous le numéro SEQ ID No 93 dans la liste de séquences en annexe.
   La protéine P29, récemment décrite, est une protéine qui interagit avec la protéine GCIP (Grap2 cyclin-D interacting protein), elle-même interagissant avec la cycline D et la protéine Grap2. (Chang et al, Biochem Biophys Res Commun 2000 Dec 20;279(2):732-7). Grap2 est une protéine adaptatrice, spécifique des leucocytes des tissus immuns (Qiu et al, 1998, Biochem. Biophys. Res. Commun. 253, 443-447). Les protéines adaptatrices jouent un rôle essentiel dans la formation de complexes de signalisation intracellulaire relayant les signaux extracellulaires de la membrane plasmique au noyau de la cellule, Grap2 est une protéine centrale de liaison dans la signalisation et l'activation des cellules immunitaires. Les cyclines de types D, elles, répondent à des signaux mitogéniques extracellulaires (Sherr, 1993, *Cell* 73, 1059-1065). La protéine GCIP, qui est exprimée de façon ubiquitaire dans tous les tissus humains, interagit avec Grap2 et les cyclines D, son expression régule la phosphorylation de la protéine de rétinoblastome (Rb) et par voie de conséquence la voie de transcription contrôlée par le facteur de transcription E2F1, appartenant à une famille de facteurs qui joue un rôle majeur dans la prolifération, la différentiation, l'apoptose et la progression du cycle cellulaire (Nevins, 1998, Cell Growth Differ 9, 585-593; Chellappan et al ,1998, Curr. Top. Microbiol. Immunol. 227, 57-103; Dyson, 1998, Genes Dev. 12, 2245-2262). Ainsi, La GCIP est suggérée jouer un rôle dans le contrôle de la prolifération et la différentiation cellulaire à travers les voies de signalisation contrôlées par les protéines Grap2 et cycline D (Xia et al, 2000, J Biol Chem, 275(27):20942-8). La protéine, P29 qui a pu être localisée dans le noyau et présente dans de multiples tissus, a été trouvée être associée avec la GCIP et de ce fait, peut être impliquée dans la régulation fonctionnelle de GCIP (Chang et al, Biochem Biophys Res Commun 2000 Dec 20;279(2):732-7).
   La protéine P29 semble donc jouer un rôle dans les voies de signalisation impliquées dans la prolifération et la différentiation cellulaire. Cependant aucun rôle dans la régulation de l'angiogènese n'a été mise en évidence à ce jour ni pour la protéine P29 ni pour la protéine homologue identifiée.
- GS-N42: un ARNm de 6147 pb identifié sous le numéro SEQ ID No 43 dans la liste de séquences en annexe . Une recherche BLAST permet de l'identifier sous le numéro d'accession NM_013390 dans la base de séquences GENBANK.
   La séquence de cet ARNm a une séquence codante du nucléotide 149 au nucléotide 4300. Il a été ainsi identifié une protéine GS-P42 résultante de la traduction de cet ARNm. Cette protéine appelée TMEM2 est composée de 1383aa. Elle est identifiée sous le numéro SEQ ID No 94 dans la liste de séquences en annexe.
   Le gène de TMEM2 a été décrit récemment (Scott et al , Gene 2000;246(1-2):265-74); de part la structure, la protéine codée TMEM2 a été suggérée être transmembranaire. La présence du motif RGD suggérant un rôle dans l'adhésion cellulaire est une implication dans des voies de signalisation cellulaire.
   A l'heure actuelle aucun rôle pour cette protéine n'a été décrit et en particulier aucun rôle dans la régulation de l'angiogènese n'a été mise en évidence.
- GS-N43: un ARNm de 4357 bp identifié sous le numéro SEQ ID No 44 dans la liste de séquences en annexe . Une recherche BLAST permet de l'identifier sous le numéro d'accession AB029316 dans la base de séquences GENBANK.
   La séquence de cet ARNm a une séquence codante du nucléotide 318 au nucléotide 2834. Il a été ainsi identifié une protéine GS-P43 résultante de la traduction de cet ARNm. Cette protéine appelée Dorfine est composée de 838aa. Elle est identifiée sous le numéro SEQ ID No 95 dans la liste de séquences en annexe.
   La protéine appelée Dorfine est encore très peu étudiée, son gène a été récemment identifié. Cette protéine est ubiquitairement exprimée dans beaucoup d'organes.Elle se lie spécifiquement à des enzymes conjuguant l'ubiquitine, UbcH7 et UbcH8 à travers son domaine RING-finger/IBR. La Dorfine est proposée comme un nouveau membre des ubiquitine ligases type RING Finger . Cette protéine est localisée dans le centrosome et agit probablement dans les centres d'organisations des microtubules (Niwa et al, 2001, Biochem Biophys Res Commun, 281(3):706-13). A ce jour, aucune expression différentielle de la Dorfine n'a été décrite ni un rôle dans la régulation de l'angiogénèse.
- GS-N44: un ARNm de 1801pb identifié sous le numéro SEQ ID No 45 dans la liste de séquences en annexe . Une recherche BLAST permet de l'identifier sous le numéro d'accession XM_032382 (membre 2 de la superfamille 4 transmembranaire) dans la base de séquences GENBANK.
   La séquence de cet ARNm a une séquence codante du nucléotide 81 au nucléotide 815. Il a été ainsi identifié une protéine GS-P44 résultante de la traduction de cet ARNm. Cette protéine appelée TM4SF2 est composée de 244aa. Elle est identifiée sous le numéro SEQ ID No 96 dans la liste de séquences en annexe.
   La protéine TM4SF2 appartient à la superfamille transmembranaire de type 4 dont certains membres sont connus pour être impliqués dans l'angiogènese comme CD9 ou encore PETA-3/CD151(Klein-Soyer et al, 2000, Arterioscler Thromb Vasc Biol., 20:360-9 ;Sincock et al,1999, J.Cell. Science,112, 833-844). Par contre, la fonction de TM4SF2 n'est pas encore connue. Cette protéine est homologue à la protéine TALLA-1, proposée comme un marqueur spécifique de surface de neuroblastomes et leucémie neuroblastiques (Takagi et al, Int J Cancer, 1995, 61(5):706-15).
   Aucun rôle pour la protéine TM4SF2 dans l'angiogènese n'a été décrit à ce jour.
- GS-N45: Un ARNm de 2081 bp identifié sous le numéro SEQ ID No 46 dans la liste de séquences en annexe . Une recherche BLAST permet de l'identifier sous le numéro d'accession HSA133133 dans la base de séquences GENBANK.
   La séquence de cet ARNm a une séquence codante du nucléotide 184 au nucléotide 1737. Il a été ainsi identifié une protéine GS-P45 résultante de la traduction de cet ARNm. Cette protéine appelée Ecto-ATP diphosphohydrolase I est composée de 517aa.
   Elle est identifiée sous le numéro SEQ ID No 97 dans la liste de séquences en annexe.
   Les ecto-ATPases (ATPases de la surface cellulaire) sont des enzymes ubiquitaires chez les cellules qui hydrolysent l'ATP et l'ADP extracellulaires en AMP (revue: Plesner, 1995, Int Rev Cytol,158:141-214). La présence des ATPDases a été démontrée sur les cellules endothéliales aortiques et les cellules du muscle lisse et décrites comme pouvant jouer un rôle régulateur dans l'homéostase et la réactivité plaquettaire en hydrolysant l'ATP et l'ADP (Robson et al, 1997, J Exp Med, 185(1):153-63). L'ATPDase vasculaire est étroitement homologue à la glycoprotéine CD39 dont le numéro d'accession dans la base GENBANK est S73813 et est identifiée sous le numéro SEQ ID No. 290 dans la liste de séquences en annexe, antigène de l'activation des cellules lymphoïdes, également exprimée par les cellules endothéliales humaines (Kaczmarek et al, 1996, J. Biol.Chem, 271, 51, 33116-33122). Récemment, Goepfert et al, (2001, Circulation, n104 (25):3109-3115) en implantant des corps contenant du Matrigel chez des souris mutants caractérisées par la déficience de l'expression de CD39 ont montré l'absence de formation des néovaisseaux autour des corps implantés. Ces observations ont conduit ces auteurs à suspecter un rôle pour le CD39 dans le phénomène de l'angiogènese. Cependant, l'expérimentation décrite plaide plutôt pour un rôle mal défini de CD39 dans l'angiogènese que pour un rôle direct et incontestable. En effet, du fait que des souris mutantes déficientes en CD39 existent, il est possible que des embryons des souris déficientes en CD39 puissent se développer. Toutefois, sans angiogènese, il n'y a aucun développement embryonnaire possible. Par conséquent, soit le CD39 a un rôle dans l'angiogènese et de ce fait il n'y aura pas de souris mutantes déficientes en CD39 viables, soit le CD39 n'a aucun rôle dans l'angiogènese. Dans un autre article, Goepfert et al, (2001, Circulation,104 (25):3109-15) n'ont pas montré l'absence d'expression de CD39 chez les cellules endothéliales des souris mutantes utilisées.
   En somme, l'étude de Goepfert et al, (2001, Circulation,104(25):3109-15) n'apporte pas la preuve d'un quelconque rôle du CD39 dans l'angiogènese.
   Le CD39 et l'ecto-ATPDase I peuvent être deux formes résultant d'un épissage alternative d'un même gène. Le vecteur codant pour l'oligonucléotide antisens utilisé dans notre étude peut à la fois inhiber l'expression de CD39 et l'ecto-ATPDase I.
   Les résultats obtenus avec le vecteur GS-V45, dans le cadre de la présente invention, prouvent en revanche que le CD39 joue un rôle direct dans l'angiogènese.
   Il est à noter par ailleurs qu'aucun rôle n'a été décrit à ce jour pour l'ecto-ATPDase I dans la régulation de l'angiogènese.
- GS-N46: Un ARNm de 4332 bp identifié sous le numéro SEQ ID No 47 dans la liste de séquences en annexe . Une recherche BLAST permet de l'identifier sous le numéro d'accession AJ306399 dans la base de séquences GENBANK.
   La séquence de cet ARNm a une séquence codante du nucléotide 56 au nucléotide 1828. Il a été ainsi identifié une protéine GS-P46 résultante de la traduction de cet ARNm. Cette protéine est composée de 590aa. Elle est identifiée sous le numéro SEQ ID No 98 dans la liste de séquences en annexe.

L'expression des ARNms ci-dessus identifiés est observée dans des cellules endothéliales humaines qui forment des tubes capillaires. La Demanderesse a mis donc en évidence que l'expression différentielle du gène correspondant à chacun de ces ARNms accompagne la formation de néovaisseaux par les cellules endothéliales.

De plus, il est montré que l'induction de l'expression dudit gène durant l'angiogenèse est sensible à la présence de différents inhibiteurs. En effet, lorsque les cellules endothéliales humaines formant des néovaisseaux sont stimulées par un facteur angiogénique, (indiqués colonne 2 du tableau II) on observe une expression élevée de cet ARNm, tandis que lorsque les mêmes cellules endothéliales humaines sont stimulées par le même facteur angiogénique et mises en présence d'un facteur anti-angiogénique (indiqués colonne 3 du tableau II) (où l'angiogenèse est inhibée) on observe que l'expression de ce gène est également inhibée.

**Tableau II**

| SÉQ. ID | **Inducteurs de l'expression** | **Inhibiteurs de l'expression** |
|---|---|---|
| SEQ ID No 1 (GS-N1) | VEGF | PF4 |
| SEQ ID No 2 (GS-N2) | VEGF | PF4 |
| SEQ ID No 3 (GS-N3) | FGF2 | IFN-γ |
| SEQ ID No 4 (GS-N4) | VEGF | IFN-γ |
| SEQ ID No 5 (GS-N5) | VEGF | IFN-γ |
| SEQ ID No 6 (GS-N6) | VEGF | IFN-γ |
| SEQ ID No 7 (GS-N7) | VEGF | TNF-α |
| SEQ ID No 8 (GS-N8) | VEGF | TNF-α |
| SEQ ID No 9 (GS-N9) | VEGF | IFN-γ |
| SEQ ID No 10 (GS-N10) | VEGF | IFN-γ |
| SEQ ID No 11 (GS-N11) | VEGF | IFN-γ |
| SEQ ID No 12 (GS-N12) | VEGF | IFN-γ |
| SEQ ID No 13 (GS-N13) | VEGF | IFN-γ |
| SEQ ID No 14 (GS-N14) | VEGF | IFN-γ |
| SEQ ID No 15 (GS-N15) | VEGF | IFN-γ |
| SEQ ID No 17 (GS-N16) | VEGF | PF4 |
| SEQ ID No 18 (GS-N17) | VEGF | TSP-1 |
| SEQ ID No 19 (GS-N18) | VEGF | PF4 |
| SEQ ID No 20 (GS-N19) | VEGF | PF4 |
| SEQ ID No 21 (GS-N20) | VEGF | PF4 |
| SEQ ID No 22 (GS-N21) | VEGF | PF4 |
| SEQ ID No 23 (GS-N22) | VEGF | PF4 |
| SEQ ID No 24 (GS-N23) | FGF2 | TNF-α |
| SEQ ID No 25 (GS-N24) | VEGF | IFN-γ |
| SEQ ID No 26 (GS-N25) | VEGF | IFN-γ |
| SEQ ID No 27 (GS-N26) | VEGF | IFN-γ |
| SEQ ID No 28 (GS-N27) | VEGF | IFN-γ |
| SEQ ID No 29 (GS-N28) | VEGF | IFN-γ |
| SEQ ID No 30 (GS-N29) | FGF2 | Ang-2 |
| SEQ ID No 31 (GS-N30) | VEGF | TNF-α |
| SEQ ID No 32 (GS-N31) | VEGF | IFN-γ |
| SEQ ID No 33 (GS-N32) | VEGF | TNF-α |
| SEQ ID No 34 (GS-N33) | VEGF | IFN-γ |
| SEQ ID No 35 (GS-N34) | VEGF | TNF-α |
| SEQ ID No 36 GS-N35) | VEGF | IFN-γ |
| SEQ ID No 37 (GS-N36) | VEGF | IFN-γ |
| SEQ ID No 38 (GS-N37) | FGF2 | Ang-2 |
| SEQ ID No 39 (GS-N38) | VEGF | IFN-γ |
| SEQ ID N°40 (GS-N39) | VEGF | IFN-γ |
| SEQ ID No 41 (GS-N40) | VEGF | IFN-γ |
| SEQ ID No 42 (GS-N41) | VEGF | IFN-γ |
| SEQ ID No 43 (GS-N42) | VEGF | Ang-2 |
| SEQ ID No 44 (GS-N43) | FGF2 | PF4 |
| SEQ ID No 45 (GS-N44) | FGF2 | IFN-γ |
| SEQ ID No 46 (GS-N45) | VEGF | IFN-γ |
| SEQ ID No 47 (GS-N46) | FGF2 | TNF-α |
| SEQ ID No 48 (GS-N47) | FGF2 | TNF-α |
| SEQ ID No 49 (GS-N48) | FGF2 | TNF-α |
| SEQ ID No 50 (GS-N49) | VEGF | IFN-γ |
| SEQ ID No 51 (GS-N51) | VEGF | IFN-γ |
| SEQ ID No 52 (GS-N52) | FGF2 | Ang-2 |
| SEQ ID No 53 (GS-N53) | VEGF | TNF-α |
| SEQ ID No 225 (GS-N50) | VEGF | IFN-γ |
| SEQ ID No 16 (GS-N54) | VEGF | IFN-γ |

Il apparaît ainsi qu'il existe une correlation directe entre l'expression de chacun des gènes GS-N1 à GS-N54 et l'éta angiogénique des cellules endothéliales humaines.

### 6.2 Vérification du rôle des gènes identifiés dans la régulation de l'angiogénèse.

De plus, il a été également montré le rôle fonctionnel de ces gènes dans la formation de néovaisseaux par les cellules endothéliales humaines.
En effet, un oligonucléotide spécifique de chacun des gènes identifiés, choisi parmi les oligonucléotides identifiés par les sequences SEQ ID No. : 103 à SEQ ID. : 148 dans la liste de séquences en annexe a été introduit dans le vecteur d'expression pCI-neo Vector dans l'orientation antisens.
Les vecteurs résultants appelés GS-V1 à GS-V46 identifiés par leurs séquence SEQ ID No : 149 à SEQ ID No : 194 dans la liste de séquences en annexe ont été utilisés pour réprimer l'expression du gène codant pour cet ARNm chez les cellules endothéliales humaines suite à la transfection de ces dernières par ce vecteur.

Les cellules endothéliales humaines ont été stimulées alors par des facteurs angiogéniques. Les résultats obtenus, pour chacune des séquences illustrées ci-dessous, en utilisant les séquences antisens et les vecteurs correspondants, indiqués dans le tableau III, montrent que :
- la répression de l'expression des gènes SEQ ID N. 1 à SEQ ID No. 5, SEQ ID No. 7, SEQ ID No. 9, SEQ ID No. 11 à SEQ ID No. 15, SEQ ID No. 17 à SEQ ID No. 53 et SEQ ID No. 225 inhibe la formation de néovaisseaux par les cellules endothéliales humaines et que
- la répression des gènes SEQ ID No. 6, SEQ ID No. 8, SEQ ID No. 10 et SEQ ID No. 16 stimule la formation la formation de néovaisseaux par les cellules endothéliales humaines
   et cela malgré la présence des différents facteurs angiogéniques.

Ces résultats sont illustrés dans les figures correspondantes 1 à 11 en annexe.

**TABLEAU III**

| **NOM** | **Gènes SÉQ.ID** | **Protéine SEQ. ID** | **Séquences antisens** | **Vecteur avec l'antisens insere** | **Fig** | **Fig contrôle** |
|---|---|---|---|---|---|---|
| 1 | SEQ ID No 1 (GS-N1) | SEQ ID No 54 (GS-P1) Angioinducine | SEQ ID No 103 (257pb) | SEQ ID No 149 (GS-V1) | 1A | 1F |
| 2 | SEQ ID No 2 (GS-N2) | SEQ ID No 55 (GS-P2) Angiodockine | SEQ ID No 104 (202pb) | SEQ ID No 150 (GS-V2) | 1B | 1F |
| 3 | SEQ ID No 3 (GS-N3) | SEQ ID No 56 (GS-P3) Angioblastine | SEQ ID No 105 (242 pb) | SEQ ID No 151 (GS-V3) | 2A | 2C |
| 4 | SEQ ID No 4 (GS-N4) | SEQ ID No 57 (GS-P4) Angioreceptine | SEQ ID No 106 (211 pb) | SEQ ID No 152 (GS-V4) | 1C | 1F |
| 5 | SEQ ID No 5 (GS-N5) | SEQ ID No 58 (GS-P5) Angiodensine | SEQ ID No 107 (191 pb) | SEQ ID No 153 (GS-V5) | 1D | 1F |
| 6 | SEQ ID No 6 (GS-N6) | SEQ ID No 59 (GS-P6) Vassoserpentine | SEQ ID No 108 (238 pb) | SEQ ID No 154 (GS-V6) | 3A | 3D |
| 7 | SEQ ID No 7 (GS-N7) | SEQ ID No 60 (GS-P7) Angiosulfatine | SEQ ID No 109 (205 pb) | SEQ ID No 155 (GS-V7) | 4A | 4F |
| 8 | SEQ ID No 8 (GS-N8) | SEQ ID No 61 (GS-P8) Vassoreceptine | SEQ ID No 110 (186 pb) | SEQ ID No 156 (GS-V8) | 3B | 3D |
| 9 | SEQ ID No 9 (GS-N9) | SEQ ID No 62 (GS-P9) Angiokinasine | SEQ ID No 111 (223pb) | SEQ ID No 157 (GS-V9) (GS-V9) | 4B | 4F |
| 10 | SEQ ID No 10 (GS-N10) | SEQ ID No 63 (GS-P10) Vassosubstratin e | SEQ ID No 112 (247pb) | SEQ ID No 158 (GS-V10) | 3C | 3D |
| 11 | SEQ ID No 11 (GS-N11) | SEQ ID No 64 (GS-P11) Angiosignaline | SEQ ID No 113 (162pb) | SEQ ID No 159 (GS-V11) | 4C | 4F |
| 12 | SEQ ID No 12 (GS-N12) | SEQ ID No 65 (GS-P12) Angiofoculine | SEQ ID No 114 (166 pb) | SEQ ID No 160 (GS-V12) | 4D | 4F |
| 13 | SEQ ID No 13 (GS-N13) | SEQ ID No 66 (GS-P13) Angiohélicine | SEQ ID No 115 (135 pb) | SEQ ID No 161 (GS-V13) | 2B | 2C |
| 14 | SEQ ID No 14 (GS-N14) | - | SEQ ID No 116 (136) | SEQ ID No 162 (GS-V14) | 4E | 4F |
| 15 | SEQ ID No 15 (GS-N15) | SEQ ID No 67 (GS-P15) Angioacyline | SEQ ID No 117 (152) | SEQ ID No 163 (GS-V15) | 1A | 1F |
| 16 | SEQ ID No 16 (GS-N54) | | SEQ ID No 112 (247 pb) | SEQ ID No 158 (GS-V10) | 3C | 3D |
| 17 | SEQ ID No 17 (GS-N16) | SEQ ID No 68 (GS-P16) PDL | SEQ ID No 118 (417 pb) | SEQ ID No 164 (GS-V16) | 5A | 5F |
| 18 | SEQ ID No 18 (GS-N17) | SEQ ID No 69 (GS-P17) RPL3 | SEQ ID No 119 (244 pb) | SEQ ID No 165 (GS-V17) | 5B | 5F |
| 19 | SEQ ID No 19 (GS-N18) | SEQ ID No 70 (GS-P18) homol.RNF20 | SEQ ID No 120 (311 pb) | SEQ ID No 166 (GS-V18) | 5C | 5F |
| 20 | SEQ ID No 20 (GS-N19) | SEQ ID No 71 (GS-P19) homol.SFRS4 | SEQ ID No 121 (246 pb) | SEQ ID No 167 (GS-V19) | 5D | 5F |
| 21 | SEQ ID No 21 (GS-N20) | SEQ ID No 72 (GS-P20) CPD | SEQ ID No 122 (203 pb) | SEQ ID No 168 (GS-V20) | 10A | 10F |
| 22 | SEQ ID No 22 (GS-N21) | SEQ ID No 73 (GS-P21) USP9X | SEQ ID No 123 (253 pb) | SEQ ID No 169 (GS-V21) | 5E | 5F |
| 23 | SEQ ID No 23 (GS-N22) | SEQ ID No 74 (GS-P22) NRD1 | SEQ ID No 124 (173 pb) | SEQ ID No 170 (GS-V22) | 6A | 6F |
| 24 | SEQ ID No 24 (GS-N23) | SEQ ID No 75 (GS-P23) Homol. HRX , ALL-1, MLL | SEQ ID No 125 (228 pb) | SEQ ID No 171 (GS-V23) | 10B | 10F |
| 25 | SEQ ID No 25 (GS-N24) | SEQ ID No 76 (GS-P24) ATRX | SEQ ID No 126 (381 pb) | SEQ ID No 172 (GS-V24) | 6B | 6F |
| 26 | SEQ ID No 26 (GS-N25) | SEQ ID No 77 (GS-P25) transp.ac sial.-CMP1 | SEQ ID No 127 (395 pb) | SEQ ID No 173 (GS-V25) | 6C | 6F |
| 27 | SEQ ID No 27 (GS-N26) | SEQ ID No 78 (GS-P26) CBL-b | SEQ ID No 128 (381 pb) | SEQ ID No 174 (GS-V26) | 6D | 6F |
| 28 | SEQ ID No 28 (GS-N27) | SEQ ID No 79 (GS-P27) H2AV | SEQ ID No 129 (298 pb) | SEQ ID No175 (GS-V27) | 6E | 6F |
| 29 | SEQ ID No 29 (GS-N28) | SEQ ID No 80 (GS-P28) CSNK2B | SEQ ID No 130 (413 pb) | SEQ ID No 176 (GS-V28) (GS-V28) | 7A | 7F |
| 30 | SEQ ID No 30 (GS-N29) | SEQ ID No 81 (GS-P29) Hémicentine | SEQ ID No 131 (564 pb) | SEQ ID No 177 (GS-V29) | 7B | 7F |
| 31 | SEQ ID No 31 (GS-N30) | SEQ ID No 82 (GS-P30) SYNE-2 | SEQ ID No 132 (414 pb) | SEQ ID No 178 (GS-V30) | 7C | 7F |
| 32 | SEQ ID No 32 (GS-N31) | SEQ ID No 83 (GS-P31) Séladine-1 | SEQ ID No 133 (298 pb) | SEQ ID No 179 (GS-V31) | 7D | 7F |
| 33 | SEQ ID No 33 (GS-N32) | SEQ ID No 84 (GS-P32) CHD2 | SEQ ID No 134 (365 pb) | SEQ ID No 180 (GS-V32) | 7E | 7F |
| 34 | SEQ ID No 34 (GS-N33) | SEQ ID No 85 (GS-P33) BRD2 | SEQ ID No 135 (270 pb) | SEQ ID No 181 (GS-V33) | 8A | 8F |
| 35 | SEQ ID No 35 (GS-N34) | SEQ ID No 86 (GS-P34) Syntaxine 3A | SEQ ID No 136 (298 pb) | SEQ ID No 182 (GS-V34) | 8B | 8F |
| 36 | SEQ ID No 36 GS-N35) | SEQ ID No 87 (GS-P35) SHARP | SEQ ID No 137 (117 pb) | SEQ ID No 183 (GS-V35) | C | 8F |
| 37 | SEQ ID No 37 (GS-N36) | SEQ ID No 88 (GS-P36) PLPP | SEQ ID No 138 (96 pb) | SEQ ID No 184 (GS-V36) | 10C | 10F |
| 38 | SEQ ID No 38 (GS-N37) | SEQ ID No 89 (GS-P37) HIP1 | SEQ ID No 139 (393 pb) | SEQ ID No 185 (GS-V37) | 8D | 8F |
| 39 | SEQ ID No 39 (GS-N38) | SEQ ID No 90 (GS-P38) NUP88 | SEQ ID No 140 (100 pb) | SEQ ID No 186 (GS-V38) | 8E | 8F |
| 40 | SEQ ID N°40 (GS-N39) | SEQ ID N°91 (GS-P39) FKPB1A | SEQ ID No 141 (90 pb) | SEQ ID No 187 (GS-V39) | 10D | 10F |
| 41 | SEQ ID No 41 (GS-N40) | SEQ ID N°92 (GS-P40) SALF | SEQ ID No 142 (144 pb) | SEQ ID No 188 (GS-V40) | 9A | 9F |
| 42 | SEQ ID No 42 (GS-N41) | SEQ ID N°93 (GS-P41) Homol.P29 | SEQ ID No 143 (113 pb) | SEQ ID No 189 (GS-V41) | 1DE | 10F |
| 43 | SEQ ID No 43 (GS-N42) | SEQ ID N°94 (GS-P42) TMEM2 | SEQ ID No 144 (180 pb) | SEQ ID No 190 (GS-V42) | 9B | 9F |
| 44 | SEQ ID No 44 (GS-N43) | SEQ ID N°95 (GS-P43) Dorfine | SEQ ID No 145 (507 pb) ID | SEQ ID No 191 (GS-V43) | 9C | 9F |
| 45 | SEQ ID No 45 (GS-N44) | SEQ ID N°96 (GS-P44) TM4SF2 | SEQ No 146 (632 pb) | SEQ ID No 192 (GS-V44) | 9D | 9F |
| 46 | SEQ ID No 46 (GS-N45) | SEQ ID N°97 (GS-P45) Ecto-ATPase I | SEQ ID No 147 (704 pb) | SEQ ID No 193 (GS-V45) | 9E | 9F |
| 47 | SEQ ID No 47 | SEQ ID N°98 (GS-P46) Sélénoprotéine N | SEQ ID No 148 (257 pb) | SEQ ID No 194 (GS-V46) | 11A | 11B |
| 48 | SEQ ID No 48 (GS-N47) | - | SEQ ID No 125 (228 pb) | SEQ ID No 171 (GS-V23) | 10B | 10F |
| 49 | SEQ ID No 49 | SEQ ID N°99 (GS-P48) MLL | SEQ ID No 125 (228 pb) | SEQ ID No 171 (GS-V23) | 10B | 10F |
| 50 | SEQ ID No 50 (GS-N49) | SEQ ID N°100 (GS-P49) ATRX | SEQ ID No 126 (381 pb) | SEQ ID No 172 (GS-V24) | 6B | 6F |
| 51 | SEQ ID No 51 (GS-N51) | - | SEQ ID No 129 (298 pb) | SEQ ID No175 (GS-V27) | 6E | 6F |
| 52 | SEQ ID No 52 (GS-N52) | SEQ ID N°101 (GS-P52) Fibuline 6 | SEQ ID No 131 (564 pb) | SEQ ID No 177 (GS-V29) | 7B | 7F |
| 53 | SEQ ID No 53 (GS-N53) Séladine | SEQ ID N°102 (GS-P53) 1 | SEQ ID No 133 (298 pb) | SEQ ID No 179 (GS-V31) | 7D | 7F |
| 54 | SEQ ID No 225 (GS-N50) (GS-N50) | - | SEQ ID No 127 (395 pb) | SEQ ID No 173 (GS-V25) | 6C | 6F |

## Revendications

1. Séquence d'acides nucléiques choisie parmi
• les séquences de gènes identifiées dans la liste de séquences en annexe par les numéros SEQ ID N°1 à SEQ ID N°5, SEQ ID N°7, SEQ ID N°9 et SEQ ID N°11 à SEQ ID N°15, lesdits gènes codant pour un polypeptide impliqué dans l'activation de l'angiogenèse ;
• les séquences d'acides nucléiques antisens de gènes, identifiées dans la liste de séquences en annexe par les numéros SEQ ID N°108, SEQ ID N°110
ou SEQ ID N°112, lesdits gènes codant pour un polypeptide impliqué dans l'inhibition de l'angiogenèse ;
ou un fragment de celles-ci.

2. Séquence d'acides nucléiques selon la revendication 1, **caractérisée en ce qu'**elle comprend au moins 10 mers ou qu'elle présente une identité d'au moins 85%, de préférence de 95%, avec une séquence choisie parmi les séquences identifiées sous les numéros SEQ ID N°1 à SEQ ID N°5, SEQ ID N°7, SEQ ID N°9 et SEQ ID N°11 à SEQ ID N°15, SEQ ID N°108, SEQ ID N°110 ou SEQ ID N°112 dans la liste de séquences en annexe.

3. Vecteur d'expression de mammifère comprenant au moins une séquence en acides nucléiques telle que définie dans l'une quelconque des revendications 1 ou 2.

4. Vecteur selon la revendication 3, **caractérisé en ce qu'**il est choisi parmi le groupe de vecteurs GS-V6, GS-V8 et GS-V10 identifiés par leur séquence portant les numéros SEQ ID N°154, SEQ ID N°156 ou SEQ ID N°158 dans la liste de séquences en annexe.

5. Méthode de préparation d'une cellule génétiquement modifiée, sur-exprimant ou sous-exprimant un gène impliqué dans un désordre angiogénique, **caractérisée en ce qu'**elle comprend l'insertion, dans une cellule de mammifère,
- d'un vecteur défini à l'une quelconque des revendications 3 ou 4 ; ou
- d'un vecteur comprenant au moins une séquences en acides nucléiques identifiée dans la liste de séquence en annexe sous les numéros SEQ ID N°17 à SEQ ID N°53, SEQ ID N°225 et SEQ ID N°284 à SEQ ID N°290.

6. Cellule génétiquement modifiée, **caractérisée en ce qu'**elle sur-exprime au moins un gène impliqué dans l'activation de l'angiogenèse identifiée dans la liste de séquences en annexe par les numéros SEQ ID N°1 à SEQ ID N°5, SEQ ID N°7, SEQ ID N°9, SEQ ID N°11 à SEQ ID N°15, SEQ ID N°17 à SEQ ID N°53, SEQ ID N°225 et SEQ ID N°284 à SEQ ID N°290 ou un de leurs fragments.

7. Cellule génétiquement modifiée, **caractérisée en ce qu'**elle sous-exprime au moins un gène impliqué dans l'inhibition de l'angiogenèse identifiée dans la liste de séquences en annexe par numéros SEQ ID N°6, SEQ ID N°8, SEQ ID N°10 et SEQ ID N°16.

8. Méthode de préparation d'une lignée de cellules exprimant de manière stable un vecteur d'expression comprenant au moins une séquence en acides nucléiques
- telle que définie à la revendication 1 ou 2 ; ou
- identifiée dans la liste de séquences en annexe par les numéros SEQ ID N°17 à SEQ ID N°53, SEQ ID N°225 et SEQ ID N°284 à SEQ ID N°290,
**caractérisée en ce que**
- on introduit un gène de résistance à au moins un antibiotique dans une cellule,
- on cultive les cellules obtenues à l'étape (a) en présence dudit antibiotique,
- on sélectionne les cellules viables.

9. Séquence polypeptidique impliquée dans l'activation de l'angiogenèse choisie parmi les séquences identifiées dans la liste de séquences en annexe par les numéros SEQ ID N°54 à SEQ ID N°58, SEQ ID N°60, SEQ ID N°62, SEQ ID N°:64 à SEQ ID N°67, SEQ ID N°68 à SEQ ID N°102 et SEQ ID N°291 à SEQ ID N°297

10. Méthode de préparation d'une protéine recombinante activatrice de l'angiogenèse, comprenant les étapes de :
- la construction d'un vecteur d'expression comprenant une séquence d'un gène identifié dans la liste de séquences en annexe par les numéros SEQ ID N°1 à SEQ ID N°5, SEQ ID N°7, SEQ ID N°9, SEQ ID N°11 à SEQ ID N°15, SEQ ID N°17 à SEQ ID N°53, SEQ ID N°225 et SEQ ID N°284 à SEQ ID N°290;
- l'introduction dudit vecteur dans un hôte cellulaire,
- la culture desdites cellules dans un milieu adéquat,
- la purification des protéines exprimées ou d'un de leurs fragments.

11. Protéine recombinante ou fragment peptidique obtenu par le procédé selon la revendication 10.

12. Anticorps capable de se fixer sur, ou ayant une affinité pour, une séquence polypeptidique impliquée dans l'inhibition de l'angiogenèse identifiée sous les numéros SEQ ID N°59, SEQ ID N°61 et SEQ ID N°63 dans la liste de séquences en annexe.

13. Anticorps selon la revendication 12, **caractérisé en ce qu'**il est un anticorps monoclonal.

14. Anticorps selon la revendication 12, **caractérisé en ce qu'**il est un anticorps polyclonal.

15. Méthode de préparation d'un anticorps selon l'une quelconque des revendications 12 à 14, **caractérisée en ce qu'**elle comprend l'immunisation *in vivo* ou *in vitro*, d'une cellule immunocompétente d'un animal, notamment d'un vertébré et de préférence d'un mammifère, avec au moins une des séquences polypeptidiques impliquées dans l'inhibition de l'angiogenèse et identifiées sous les numéros SEQ ID N°59, SEQ ID N°61 et SEQ ID N°63 dans la liste de séquences en annexe.

16. Composition pharmaceutique activatrice de l'angiogenèse **caractérisée en ce qu'**elle comprend à titre d'agent actif au moins une substance choisie parmi :
- une séquence nucléotidique selon l'une quelconque des revendications 1 ou 2 ;
- une cellule génétiquement modifiée selon l'une quelconque des revendications 6 ou 7 ;
- une séquence polypeptidique l'une quelconque des revendications 9 ou 11 ;
- un anticorps selon l'une quelconque des revendications 12 à 14.

17. Composition pharmaceutique selon la revendication 16 destinée au diagnostic et/ou au traitement de pathologies liées à l'angiogenèse.

18. Composition pharmaceutique ou thérapeutique selon l'une quelconque des revendications 16 ou 17, **caractérisée en ce que** le désordre angiogénique est choisi parmi: la vascularisation de tumeurs, les rétinopathies, l'arthrite rhumatoïde, la maladie de Crohn, l'athérosclérose, l'hyperstimulation de l'ovaire, le psoriasis, l'endométrie associée à la néovascularisation, la resténose due à l'angioplastie du ballon, la superproduction tissulaire due à la cicatrisation, la maladie vasculaire périphérique, l'hypertension, l'inflammation vasculaire, la maladie et les phénomènes de Raynaud, l'anévrisme, la resténose artérielle, la thrombophlébite, la lymphagyte, le lymphodème, la cicatrisation et la réparation tissulaire, l'ischémie, l'angine, l'infarctus de myocarde, la maladie chronique du coeur, les insuffisances cardiaques telle que l'insuffisance cardiaque congestive, la dégénération maculaire liée à l'âge et l'ostéoporose.

19. Méthode de diagnostic et/ou de pronostic d'une pathologie angiogénique chez un mammifère, notamment chez un être humain, **caractérisée en ce que** l'on détecte *in vitro* dans les cellules dudit mammifère, la sur-expression ou la sous-expression d'une ou plusieurs séquence(s) d'acides nucléiques d'un gène activateur de l'angiogenèse, identifiée(s) dans la liste de séquences en annexe par les numéros SEQ ID N°1 à SEQ ID N°5, SEQ ID N°7, SEQ ID N°9, SEQ ID N°11 à SEQ ID N°15, SEQ ID N°17 à SEQ ID N°53, SEQ ID N°225 et SEQ ID N°284 à SEQ ID N°290.

20. Méthode selon la revendication 19, **caractérisée en ce qu'**elle comporte les étapes suivantes :
- la détection de l'expression par une population cellulaire d'un mammifère, d'une ou plusieurs desdites séquences d'acides nucléiques, identifiées dans la liste de séquences en annexe par les numéros SEQ ID N°1 à SEQ ID N°5, SEQ ID N°7, SEQ ID N°9, SEQ ID N°11 à SEQ ID N°15, SEQ ID N°17 à SEQ ID N°53, SEQ ID N°225 et SEQ ID N°284 à SEQ ID N°290 ;
- la détection de l'expression par une population cellulaire de référence dont l'état angiogénique est connu, d'une ou plusieurs desdites séquences d'acides nucléiques, identifiées dans la liste de séquences en annexe par les numéros SEQ ID N°1 à SEQ ID N°5, SEQ ID N°7, SEQ ID N°9, SEQ ID N°11 à SEQ ID N°15, SEQ ID N°17 à SEQ ID N°53, SEQ ID N°225 et SEQ ID N°284 à SEQ ID N°290 ;
- l'identification des différences éventuelles du niveau d'expression d'une ou plusieurs desdites séquences d'acides nucléiques, par les deux populations cellulaires.

21. Méthode de diagnostic et/ou de pronostic d'une pathologie angiogénique chez un mammifère, notamment chez un être humain, consistant à détecter *in vitro* dans les cellules dudit mammifère la surexpression ou la sous-expression d'une ou plusieurs séquence(s) polypeptidique(s) activatrice(s) de l'angiogenèse, identifiée(s) dans la liste de séquences en annexe par les numéros SEQ ID N°54 à SEQ ID N°58, SEQ ID N°60, SEQ ID N°62, SEQ ID N°:64 à SEQ ID N°67, SEQ ID N°68 à SEQ ID N°102 et SEQ ID N°291 à SEQ ID N°297.

22. Méthode selon la revendication 21, **caractérisée en ce qu'**elle comprend :
- la détection de l'expression par une première population cellulaire d'un mammifère, d'une ou plusieurs séquence(s) polypeptidique(s) activatrice(s) de l'angiogenèse, identifiée(s) dans la liste de séquences en annexe par les numéros SEQ ID N°54 à SEQ ID N°58, SEQ ID N°60, SEQ ID N°62, SEQ ID N°:64 à SEQ ID N°67, SEQ ID N°68 à SEQ ID N°102 et SEQ ID N°291 à SEQ ID N°297 ;
- la détection de l'expression par une deuxième population cellulaire de référence dont l'état angiogénique est connu, d'une plusieurs séquence(s) polypeptidique(s) activatrice(s) de l'angiogenèse, identifiée(s) dans la liste de séquences en annexe par les numéros SEQ ID N°54 à SEQ ID N°58, SEQ ID N°60, SEQ ID N°62, SEQ ID N°:64 à SEQ ID N°67, SEQ ID N°68 à SEQ ID N°102 et SEQ ID N°291 à SEQ ID N°297 ;
- l'identification des différences éventuelles du niveau d'expression par lesdites première et deuxième populations cellulaires, d'une ou plusieurs desdites séquences polypeptidiques.

23. Méthode de vérification de l'efficacité thérapeutique d'un traitement angiogénique chez un mammifère, notamment chez un être humain, **caractérisée en ce qu'**elle comporte l'identification *in vitro* dans une population cellulaire dudit mammifère, de la sur-expression ou de la sous-expression d'une séquence d'acides nucléiques d'au moins un gène impliqué dans un désordre angiogénique, identifiée dans la liste de séquences en annexe par les numéros SEQ ID N°1 à SEQ ID N°5, SEQ ID N°7, SEQ ID N°9, SEQ ID N°11 à SEQ ID N°15, SEQ ID N°17 à SEQ ID N°53, SEQ ID N°225 et SEQ ID N°284 à SEQ ID N°290.

24. Méthode de vérification de l'efficacité thérapeutique selon la revendication 23 **caractérisée en ce qu'**elle comporte les étapes suivantes :
- la détection de l'expression par une première population cellulaire d'un mammifère auquel est administrée une composition thérapeutique destinée à traiter un désordre angiogénique, d'une ou plusieurs séquence(s) d'acides nucléiques d'au moins un gène impliqué dans un désordre angiogénique, identifiée(s) dans la liste de séquences en annexe par les numéros SEQ ID N°1 à SEQ ID N°5, SEQ ID N°7, SEQ ID N°9, SEQ ID N°11 à SEQ ID N°15, SEQ ID N°17 à SEQ ID N°53, SEQ ID N°225 et SEQ ID N°284 à SEQ ID N°290;
- la détection de l'expression par une deuxième population cellulaire de référence dont l'état angiogénique est connu, d'une ou plusieurs desdites séquences d'acides nucléiques d'au moins un gène impliqué dans un désordre angiogénique, identifiées dans la liste de séquences en annexe par les numéros SEQ ID N°1 à SEQ ID N°5, SEQ ID N°7, SEQ ID N°9, SEQ ID N°11 à SEQ ID N°15, SEQ ID N°17 à SEQ ID N°53, SEQ ID N°225 et SEQ ID N°284 à SEQ ID N°290;
- l'identification des différences éventuelles du niveau d'expression par lesdites première et deuxième populations cellulaires, d'une, ou plusieurs, desdites séquences d'acides nucléiques.

25. Méthode de criblage de composés utiles pour le traitement d'un désordre angiogénique d'un mammifère, notamment d'un être humain, **caractérisée en ce qu'**elle comporte les étapes suivantes :
- la détection de l'expression par une première population cellulaire d'un mammifère mise en présence d'un composé susceptible d'avoir un effet thérapeutique sur un désordre angiogénique, d'une ou plusieurs séquences d'acides nucléiques d'au moins un gène impliqué dans un désordre angiogénique, identifiées dans la liste de séquences en annexe par les numéros SEQ ID N°1 à SEQ ID N°5, SEQ ID N°7, SEQ ID N°9, SEQ ID N°11 à SEQ ID N°15, SEQ ID N°17 à SEQ ID N°53, SEQ ID N°225 et SEQ ID N°284 à SEQ ID N°290;
- la détection de l'expression par une deuxième population cellulaire de référence dont l'état angiogénique est connu, d'une ou plusieurs séquences d'acides nucléiques d'au moins un gène impliqué dans un désordre angiogénique, identifiées dans la liste de séquences en annexe par les numéros SEQ ID N°1 à SEQ ID N°5, SEQ ID N°7, SEQ ID N°9, SEQ ID N°11 à SEQ ID N°15, SEQ ID N°17 à SEQ ID N°53, SEQ ID N°225 et SEQ ID N°284 à SEQ ID N°290;
- l'identification des différences éventuelles du niveau d'expression par lesdites première et deuxième populations cellulaires, d'une ou plusieurs desdites séquences d'acides nucléiques.

26. Méthode selon l'une quelconque des revendications 19 à 25, **caractérisée en ce que** la détection de l'expression desdites séquences est effectuée après avoir mis les cellules, particulièrement les cellules endothéliales, en présence d'un fluide biologique issu d'un patient.

27. Méthode de diagnostic et/ou de pronostic selon une quelconque des revendications 19 à 22 ou 26, **caractérisée en ce que** le désordre angiogénique est choisi parmi: la vascularisation de tumeurs, les rétinopathies, l'arthrite rhumatoïde, la maladie de Crohn, l'athérosclérose, l'hyperstimulation de l'ovaire, le psoriasis, l'endométrie associée à la néovascularisation, la resténose due à l'angioplastie du ballon, la superproduction tissulaire due à la cicatrisation, la maladie vasculaire périphérique, l'hypertension, l'inflammation vasculaire, la maladie et les phénomènes de Raynaud, l'anévrisme, la resténose artérielle, la thrombophlébite, la lymphagyte, le lymphodème, la cicatrisation et la réparation tissulaire, l'ischémie, l'angine, l'infarctus de myocarde, la maladie chronique du coeur, les insuffisances cardiaques tel que l'insuffisance cardiaque congestive, la dégénération maculaire liée à l'âge et l'ostéoporose.

28. Dispositif pour la mise en oeuvre d'une méthode de criblage selon les revendications 25 ou 26, comprenant un support comprenant une ou plusieurs sondes spécifiques d'une ou plusieurs séquences d'acides nucléiques d'au moins un gène impliqué dans un désordre angiogénique, identifiées dans la liste de séquences en annexe par les numéros SEQ ID N°1 à SEQ ID N°5, SEQ ID N°7, SEQ ID N°9, SEQ ID N°11 à SEQ ID N°15, SEQ ID N°17 à SEQ ID N°53, SEQ ID N°225 et SEQ ID N°284 à SEQ ID N°290.

29. Dispositif selon la revendication 28, **caractérisé en ce que** le support est choisi parmi une membrane de verre, une membrane métallique, une membrane polymère, une membrane de silice.

30. Trousse destinée à la mesure de l'affichage différentiel de gènes impliqués dans des désordres angiogéniques comprenant
- un dispositif selon l'une quelconque des revendications 28 ou 29,
- des amorces spécifiques et
- les accessoires nécessaires à l'amplification des séquences extraites d'un échantillon, leur hybridation avec les sondes du dispositif et la réalisation des mesures de l'affichage différentiel.

31. Trousse destinée à la mesure de l'affichage différentiel de gènes impliqués dans des désordres angiogéniques comprenant :
- une lignée stable de cellules génétiquement modifiées, selon les revendications 6 ou 7, en tant que population cellulaire de référence,
- une ou plusieurs sondes spécifiques des séquences d'acides nucléiques d'au moins un gène impliqué dans un désordre angiogénique, identifiées dans la liste de séquences en annexe par les numéros SEQ ID N°1 à SEQ ID N°5, SEQ ID N°7, SEQ ID N°9, SEQ ID N°11 à SEQ ID N°15, SEQ ID N°17 à SEQ ID N°53, SEQ ID N°225 et SEQ ID N°284 à SEQ ID N°290, et
- les moyens nécessaires pour la détection et la mesure dudit affichage différentiel.
